# EUROPEAN PATENT APPLICATION

(11) **EP 1 953 147 A1**
(43) Date of publication of application: **06.08.2008**
(21) Application number: 06833441.6
(22) Date of filing: 21.11.2006
(51) Int. Cl.: C07D 237/26, A61K 31/502, A61K 31/5025, A61K 31/55, A61K 38/21, A61K 45/00, A61P 1/16, A61P 31/14, C07D 401/14, C07D 403/10, C07D 403/12, C07D 405/14, C07D 409/04, C07D 409/10, C07D 409/14, C07D 413/10, C07D 417/10, C07D 471/04, C07D 487/04, C07D 491/048, C07D 495/04

(54) **HETEROCYCLIC COMPOUND AND MEDICINAL APPLICATION THEREOF**

(30) Priority: 21.11.2005 JP 2005336429; 05.12.2005 US 742308 P; 29.03.2006 JP 2006092163; 10.04.2006 US 790837 P
(71) Applicant: Japan Tobacco, Inc., Tokyo 105-8422 (JP)
(72) Inventor: UENO, Hiroshi, Takatsuki-shi Osaka 569-1125 (JP); SHIMADA, Takashi, Takatsuki-shi Osaka 569-1125 (JP); AOYAGI, Kouichi, Takatsuki-shi Osaka 569-1125 (JP); KATOH, Susumu, Takatsuki-shi Osaka 569-1125 (JP); SHINKAI, Hisashi, Takatsuki-shi Osaka 569-1125 (JP); MOTOMURA, Takahisa, Takatsuki-shi Osaka 569-1125 (JP); KOMODA, Yasumasa, Takatsuki-shi Osaka 569-1125 (JP); OTSUBAKI, Tomoko, Takatsuki-shi Osaka 569-1125 (JP); SOEJIMA, Yuki, Takatsuki-shi Osaka 569-1125 (JP); KAWAHARA, Iichiro, Takatsuki-shi Osaka 569-1125 (JP)
(74) Representative: Vossius & Partner
(86) International application number: PCT/JP2006/323637
(87) International publication number: WO 2007/058392

(57) **Abstract**

The present invention aims at providing a novel heterocyclic compound having HCV entry inhibitory activity and the pharmaceutical use thereof. The present invention provides a therapeutic agent for hepatitis C comprising a heterocyclic compound represented by the following formula [1] or a pharmaceutically acceptable salt thereof as an active ingredient: 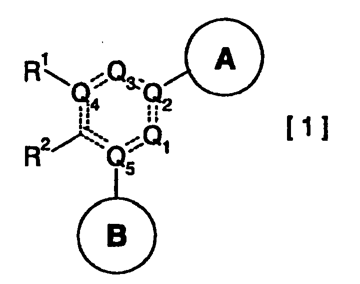 wherein Q₁ is -N=, etc., Q₂ is -N-, etc., Q₃ is -N=, etc., Q₄ is -N-, etc., Q₅ is -N-, etc., R¹ is a hydrogen atom, etc., R² is a hydrogen atom, etc., ring A is a monocyclic aryl group optionally having substituent(s), etc., and ring B is a monocyclic aryl group optionally having substituent(s), etc.

## Description

### Technical Field

The present invention relates to a novel heterocyclic compound and a pharmaceutical use thereof. More specifically, the present invention relates to a heterocyclic compound having an HCV entry inhibitory (HCV cell infection inhibitory) activity or a pharmaceutically acceptable salt thereof, a pharmaceutical composition containing same, and pharmaceutical use thereof.

### Background Art

In 1989, a main causative virus of non-A, non-B hepatitis after blood transfusion was found and named as hepatitis C virus (HCV). At present, type A, type B, type C and several other kinds of hepatitis viruses have been found, and hepatitis caused by HCV is called hepatitis C. Patients with HCV infection are estimated to occupy several % of the world's population, and they tend to suffer from long-term chronic infection.

HCV is an enveloped RNA virus whose genome is a single-stranded plus chain RNA, and classified in the genus Hepacivirus of the Flaviviridae family (classified according to The International Committee on Taxonomy of Viruses, the International Union of Microbiological Societies). Being the same hepatitis virus, for example, Hepatitis B virus (HBV), which is a DNA virus, is, in most cases, removed by the immune system except for neonates and infants having immature immunity, and some people develop acute hepatitis. In contrast, since HCV escapes a host's immune system by an unknown mechanism, many adults having developed immunie system often develop persistent infection when infected with HCV.

Once chronic hepatitis is developed along with the persistent infection with HCV, it progresses to cirrhosis and hepatic cancer with high probability, and even if the cancer can be removed by operation, many patients suffer from recurrence of hepatic cancer due to the inflammation continuously developed in the non-cancer part. There is also a report on the involvement of HCV infection in dermatic diseases such as chronic urticaria, lichen planus, cryoglobulinemic purpura and the like (Minami et al., J Dermatol, vol. 111(7), pp. 1075-81, 2001).

Under the circumstances, the needs for an effective treatment method of hepatitis C are extremely high. Particularly, aside from the symptomatic therapy suppressing inflammation with an anti-inflammatory agent, a pharmaceutical agent to reduce HCV to the extent inflammation does not occur or eradicate HCV is strongly demanded.

Currently, as an existing treatment method effective for eradication of HCV, a treatment by interferon administration and a treatment by a combination of interferon and Ribavirin are known. In addition, the development of various other therapeutic agents has been tried. for the inhibition of intracellular virus growth,for example, pharmaceutical agents targeting RNA-dependent RNA polymerase, serine protease, RNA helicase and the like, which are an HCV-derived non-structural protein, can be recited. However, such conventional approaches are not sufficient to expect a radical effect, since the presence of non-responder and insufficient effectiveness are feared. As a unique phenomenon common to virus infections, acquired resistance to pharmaceutical agents is also problematic. Accordingly, the development of a pharmaceutical agent based on a novel approach replacing such conventional approaches or used concurrently therewith has been desired.

HCV-derived protein includes the aforementioned non-structural protein, as well as structure proteins, E1 and E2 proteins. E1 and E2 are present on the virus envelope, and considered to be intervening molecules on the HCV side in the infection of HCV to the cell, i.e., from adhesion of HCV to the cell surface to its invasion into the cell (K. Watashi et al., Cancer Science, vol.94(11), pp.937-943, 2003). In recent years, a report has been documented that, for the analysis of initial process of cell infection with HCV, pseudotype HCV containing virus particles enveloped by HCV-derived E1 and E2 proteins, which is designed to express reporter gene on cell infection, was prepared and a highly sensitive cell infection assay system was constructed (Y. Matsuura et al., Virology, vol.286, pp. 263-275, 2001). This publication also reports that, using this assay system, it has been found that HCV invades into the cell by endocytosis via a receptor protein on the cell surface and cell infection essentially requires both E1 and E2 proteins.

Since cell infection with HCV via E1 and E2 proteins can be detected based on the expression of a reporter gene in this assay system, a compound that inhibits cell infection with HCV can be obtained by screening for or identifying, utilizing the above detection, a compound that lowers the expression level of the gene. Such compound can inhibit the initial process of HCV infection starting from the first contact with the cell, and potentially inhibits HCV growth resulting from the repeated process of cell infection, replication, budding and infection again.

Thus, the compound obtained as mentioned above is expected to be promising as a pharmaceutical agent to eradicate HCV based on a completely novel approach of inhibiting, in the HCV growth process, cell infection dependent on virus envelope.

Known compounds having a pyridazinone structure are described in the following.

DE-A-1913265 and US Patent No. 3671525 describe compounds represented by the formula 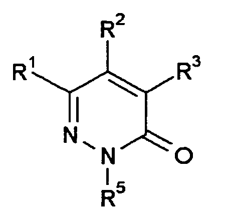 wherein R¹, R² and R³ are each independently a halogen atom, an aliphatic group, group-N(R⁶)(R⁶), an alkoxy group, a hydroxyl group, a phenoxy group, an alkylthio group or a phenylthio group, R¹ is optionally a hydrogen atom or an aromatic group and/or R² is optionally a hydrogen atom, R² and R³ optionally form a ring together with the carbon atom they are bonded to; and R⁵ is an aliphatic group, a cyclic aliphatic group, an aromatic substituted aliphatic group or an aromatic group.

GB Patent 858792 describes compounds represented the formula 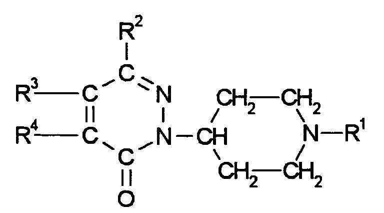 wherein R¹ is an alkyl group having 4 or less carbon atoms; R² is an alkyl group having 4 or less carbon atoms or an optionally substituted phenyl or thienyl group; R³ is a hydrogen atom or an alkyl group having 4 or less carbon atoms, or R² and R³ optionally form an alkylene group in combination; and R⁴ is a hydrogen atom, an alkyl group, an aryl group or a cyano group.

WO99/31090 describes a phosphodiesterase IV inhibitor or a compound useful as a therapeutic agent for asthma, which is represented by the formula 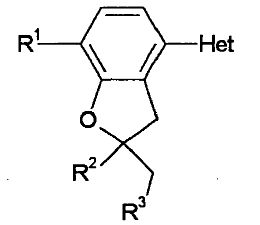 wherein Het is 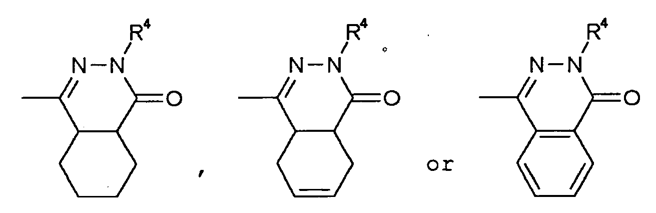 R⁴ is R⁵, -CₘH₂ₘ-R⁶ or -CₚH₂ₚ-Y-Ar; R⁵ is a hydrogen atom, a C₁₋₈ alkyl group, a C₃₋₁₀ cycloalkyl group, a C₃₋₇ cycloalkylmethyl group, a C₇₋₁₀ polycycloalkyl group, an unsubstituted phenyl group, a pyridyl group or a phenyl group substituted by R⁵¹ and/or R⁵²; m is an integer of 1-4; and p is an integer of 1-4.

WO02/22587 describes a compound useful as a non-NMDA receptor inhibitor, which is represented by the formula 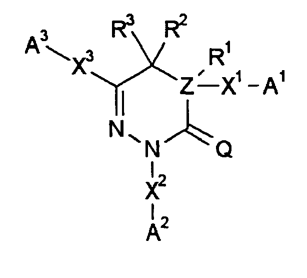 wherein A¹, A² and A³ are each independently an optionally substituted C₃₋₈ cycloalkyl group, a C₃₋₈ cycloalkenyl group, a 5- to 14-membered non-aromatic heterocyclic group, a C₆₋₁₄ aromatic hydrocarboncyclic group or a 5- to 14-membered aromatic heterocyclic group; Q is O, S or NH; Z is C or N; X¹, X² and X³ are each independently a single bond, an optionally substituted C₁₋₆ alkylene group, an optionally substituted C₂₋₆ alkenylene group, an optionally substituted C₂₋₆ alkynylene group, -NH-, -O-, -N(R⁴)CO-, -CON(R⁵)-, -N(R⁶)CH₂-, -CH₂N(R⁷)-, -CH₂CO-, -COCH₂-, - N(R⁸)SO₀₋₂-, -SO₀₋₂N(R⁹)-, -CH₂SO₀₋₂-, -SO₀₋₂CH₂-, -CH₂O-, - OCH₂-, -N(R¹⁰)CON(R¹¹)-, -N(R¹²)CS-N(R¹³)- or -SO₀₋₂-; R¹ and R² are each independently a hydrogen atom, an optionally substituted C₁₋₆ alkyl group, an optionally substituted C₂₋₆ alkenyl group or C₂₋₆ alkynyl group, or R¹ and R² are optionally bonded to each other such that CR²-ZR¹ forms C=C double bond; R³ is a hydrogen atom, an optionally substituted C₁₋₆ alkyl group, an optionally substituted C₂₋₆ alkenyl group or a C₂₋₆ alkynyl group, or optionally bonded to any atom on A¹ and A² to form an optionally substituted C₅₋₈ hydrocarbon ring or a 5- to 8-membered heterocycle.

JP-A-9-59255 and JP-A-10-226647 describe a compound useful as a phosphodiesterase IV inhibitor or a therapeutic agent for asthma, which is represented by the formula 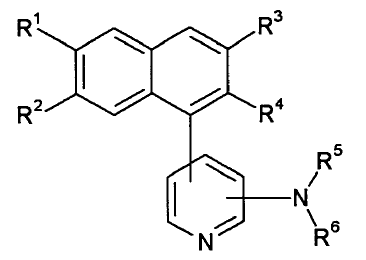 wherein R⁵ and R⁶ are each independently a hydrogen atom, an optionally substituted lower alkyl group, an optionally substituted phenyl group or an optionally protected amino group, or bonded at the terminals to form, together with the adjacent nitrogen atom, an optionally substituted heterocyclic group, and as one of the preferable examples of the "heterocyclic group substituted by oxo group" of the "heterocyclic group substituted by at least oxo group" when group-N(R⁵)(R⁶) of the compound is a "substituted heterocyclic group", 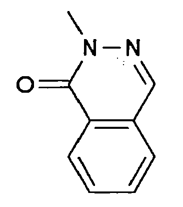 is recited.

JP-A-10-226685 and JP-A-2000-63275 describe a compound useful as a phosphodiesterase IV inhibitor or a therapeutic agent for asthma, which is represented by the formula 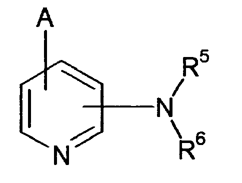 wherein A is 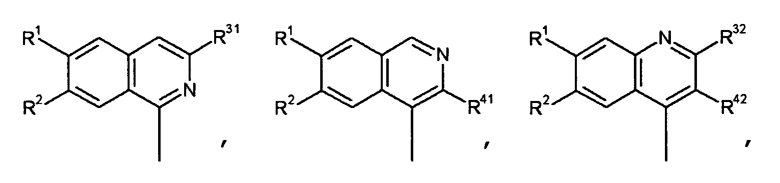 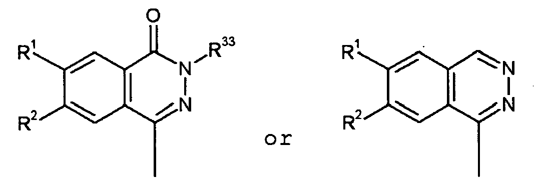 R⁵ and R⁶ are each independently a hydrogen atom or an optionally protected amino group, or bonded at the terminals to form, together with the adjacent nitrogen atom, an optionally substituted heterocyclic group, and as one of the preferable examples of the "heterocyclic group substituted by oxo group" of the "heterocyclic group substituted by at least oxo group" when group-N(R⁵)(R⁶) of the compound is a "substituted heterocyclic group", 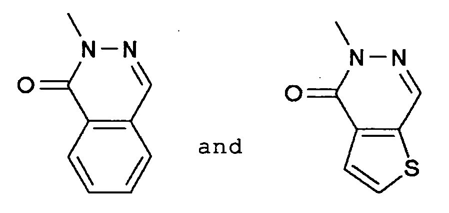 are recited.

WO02/28853 and JP-A-2003-277263 describe a compound useful as a tachykinin receptor antagonist, which is represented by the formula 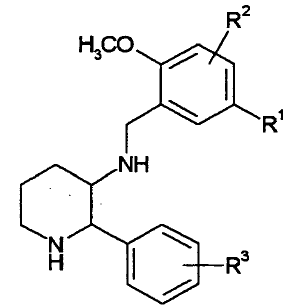 wherein R¹ is a condensed aromatic heterocyclic group containing, as hetero atom, 1 to 4 atoms selected from a nitrogen atom, an oxygen atom and a sulfur atom,
wherein the condensed aromatic heterocyclic group may be substituted by 1 to 3 groups selected from a halogen atom, an oxo group, a nitro group, a cyano group, a lower alkyl group, a halogenolower alkyl group, a lower alkoxy group, a lower alkoxy group substituted by a morpholinyl group or a pyridyl group, and a pyridyl group, and the sulfur atom contained in the condensed aromatic heterocyclic group may be oxidized, and as one of the examples of the compound, 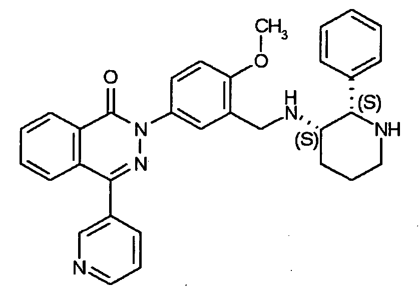 is recited.

US Patent No. 3014034 and GB Patent No. 889317 describe a compound useful for lowering of fever, analgesic action, and enhancing resistance of animal tissue to bacteria and viral infection, which is represented by the formula 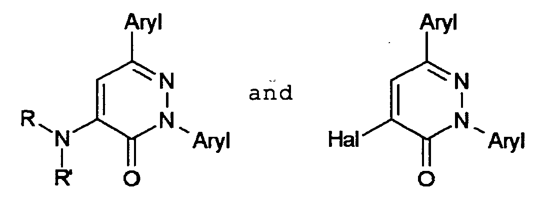 wherein R and R' are each independently selected from the group consisting of a hydrogen atom, a lower alkyl group and the like, or form a butylene group in combination, and the like; Hal is a halogen atom; and Aryl is a phenyl group optionally substituted by alkyl group etc.

WO2004/072243 describes a compound useful as an anti-HCV agent, which is represented by the formula 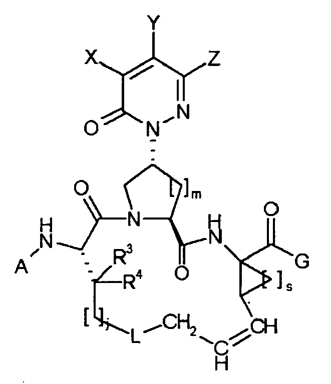 wherein X, Y and Z are each independently a hydrogen atom, N3, a halogen atom, a C₁₋₆ alkyl group, a C₃₋₁₂ cycloalkyl group, an alkylamino group, a di-alkylamino group, a C₁₋₆ alkynyl group, a substituted alkynyl group, an aryl group, a substituted aryl group, -S- aryl, -S-substituted aryl, -O- aryl, -O-substituted aryl, NH-aryl, NH-substituted aryl, a diarylamino group, a diheteroarylamino group, an arylalkyl group, a substituted arylalkyl group, a heteroaryl group, a substituted heteroaryl group, -S-heteroaryl, -S-substituted heteroaryl, -O-heteroaryl, -O-substituted heteroaryl, NH-heteroaryl, NH-substituted heteroaryl, a heteroaryl alkyl group, a substituted heteroaryl alkyl group, a heterocycloalkyl group or a substituted heterocycloalkyl group, or X and Y or Y and Z optionally form, together with the carbon atom bonded thereto, aryl, substituted aryl, heteroaryl or a substituted heteroaryl ring.

Journal of Medicinal Chemistry (2001), 44(16), 2511-2522 describes

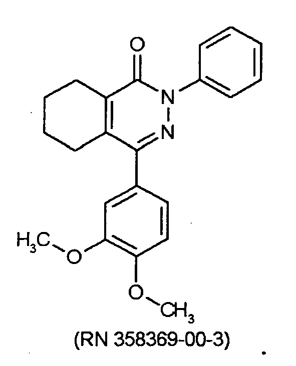

Journal of Chemical Research, Synopses (1999), (11), 648-649, 2801-2810, describes

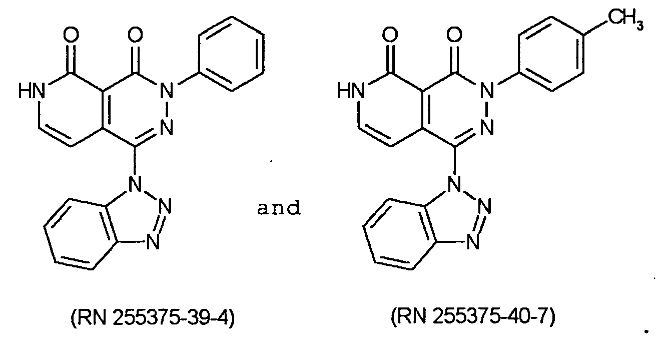

Journal fuer Praktische Chemie (Weinheim, Germany) (1999), 341(2), 147-151, describes

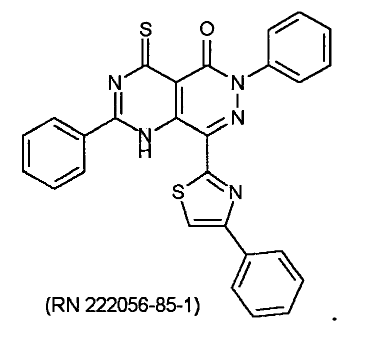

Zhurnal Organicheskoi Khimii (1989), 25(5), 1045-53, describes 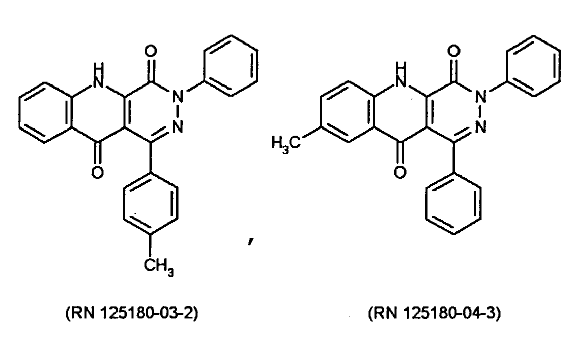 and

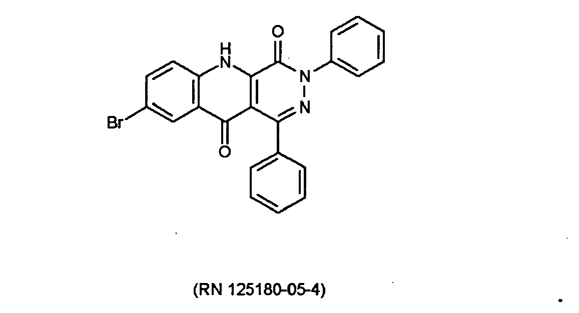

Heterocycles (1988), 27(2), 423-35, describes

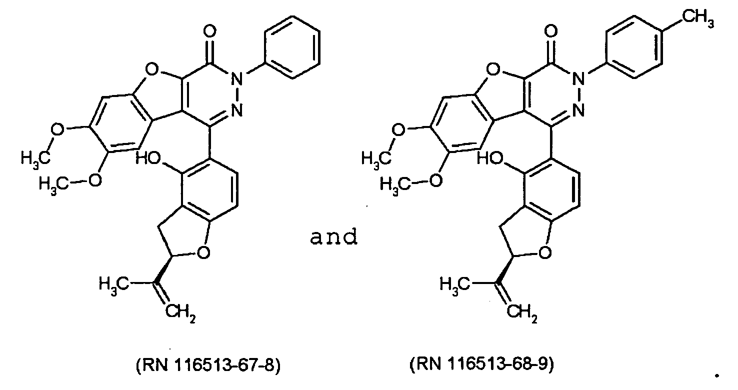

Khimiya Geterotsiklicheskikh Soedinenii (1974), (6), 784-7, describes

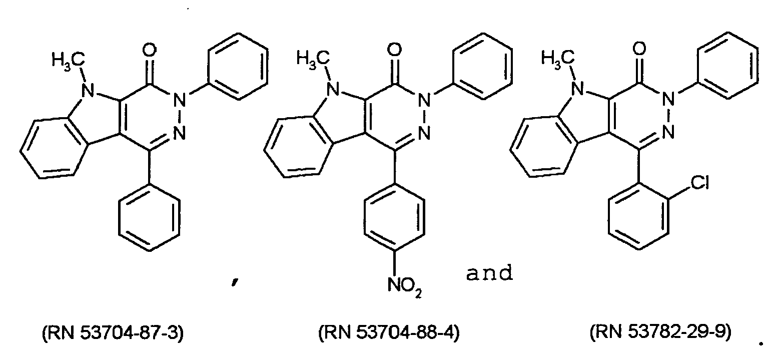

Helvetica Chimica Acta (1959), 42, 2506-14, describes

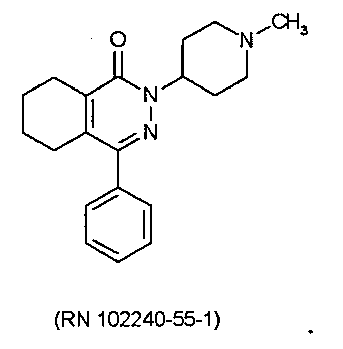

Of other compounds wherein a 5- or 6-membered aromatic ring has a condensed pyridazinone structure, a number of compounds wherein a benzene ring has a condensed pyridazinone structure (phthalazinone structure) are known and there are many publications that describe such compounds.

### Disclosure of the Invention

The problem of the present invention is provision of a novel heterocyclic compound having an HCV entry inhibitory (HCV cell infection inhibitory) activity or a pharmaceutically acceptable salt thereof, and a pharmaceutical composition containing same.

The present inventors conducted intensive studies and found that a heterocyclic compound having the following particular structure can solve the problem, and completed the present invention based on such finding.

### Disclosure of the Invention

The problem of the present invention is provision of a novel heterocyclic compound having an HCV entry inhibitory (HCV cell infection inhibitory) activity or a pharmaceutically acceptable salt thereof, and a pharmaceutical composition containing same.

The present inventors conducted intensive studies and found that a heterocyclic compound having the following particular structure can solve the problem, and completed the present invention based on such finding.

Accordingly, the present invention provides the following.

[1] Use of a heterocyclic compound represented by the following formula [1] or a pharmaceutically acceptable salt thereof for the production of a therapeutic agent for hepatitis C: 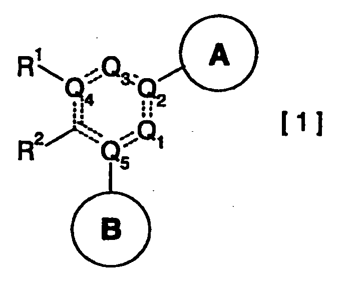 wherein
Q₁ is:
(1) -N=,
(2) -C(R⁵⁰)= wherein R⁵⁰ is a hydrogen atom, a hydroxyl group, a C₁₋₆ alkyl group or a C₁₋₆ alkoxy group, or
(3) -CO-;
Q₂ is:
(1) -N-, or
(2) -C=;
Q₃ is:
(1) -N=,
(2) -C(R⁴⁹)= wherein R⁴⁹ is a hydrogen atom, a hydroxyl group, a C₁₋₆ alkyl group or a C₁₋₆ alkoxy group, or
(3) -CO-;
Q₄ is:
(1) -N-,
(2) -C=, or
(3) -CH-;
Q₅ is:
(1) -N-, or
(2) -C=;
R¹ is:
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₁-R¹⁰¹
   wherein
   J₁ is
   (1) -N(R¹⁰²)- wherein R¹⁰² is a hydrogen atom or a C₁₋₆ alkyl group,
   (2) -O-,
   (3) -S-,
   (4) -CO-,
   (5) -CO-O-,
   (6) -O-CO-,
   (7) -CO-N(R¹⁰²)- wherein R¹⁰² is as defined above, or
   (8) -SO₂-N(R¹⁰²)- wherein R¹⁰² is as defined above,
   R¹⁰¹ is
   (1) a hydrogen atom,
   (2) -L₂-J₂-R¹⁰³
      wherein
      L₂ is
      (1) a bond, or
      (2) a C₁₋₆ alkylene group,
      J₂ is
      (1) -N(R¹⁰⁴)- wherein R¹⁰⁴ is a hydrogen atom or a C₁₋₆ alkyl group,
      (2) -O-,
      (3) -S-,
      (4)-CO-,
      (5) -SO₂-,
      (6) -CO-O-,
      (7) -CO-N(R¹⁰⁹)- wherein R¹⁰⁴ is as defined above, or
      (8) -SO₂-N(R¹⁰⁴)- wherein R¹⁰⁴ is as defined above,
      R¹⁰³ is
      (1) a hydrogen atom,
      (2) a C₁₋₆ alkyl group (said C₁₋₆ alkyl group is optionally substituted by 1 to 3 substituents selected from Group F),
      (3) a C₁₋₆ alkyl-carbonyl group (said C₁₋₆ alkyl-carbonyl group is optionally substituted by 1 to 3 substituents selected from Group F), or
      (4) a C₁₋₆ alkylsulfonyl group (said C₁₋₆ alkylsulfonyl group is optionally substituted by 1 to 3 substituents selected from Group F),
   (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
   (4) a substituent selected from a halogen atom, a cyano group and a nitro group,
   (5) a C₆₋₁₄ aryl group (said C₆₋₁₄ aryl group is optionally substituted by 1 to 5 substituents selected from Group E),
   (6) a heteroaryl group (said heteroaryl group is optionally substituted by 1 to 5 substituents selected from Group E), or
   (7) a C₃₋₈ cycloalkyl group (said C₃₋₈ cycloalkyl group is optionally substituted by 1 to 5 substituents selected from Group E), or
   R¹⁰¹ and R¹⁰² may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(7) a C₆₋₁₄ aryl group (said C₆₋₁₄ aryl group is optionally substituted by 1 to 5 substituents selected from Group E), or
(8) a heteroaryl group (said heteroaryl group is optionally substituted by 1 to 5 substituents selected from Group E);
   R² is:
   (1) a hydrogen atom, or
   (2) a C₁₋₆ alkyl group; or
   R¹ and R² may form, together with Q₄ and the carbon atom to which they are bonded respectively: 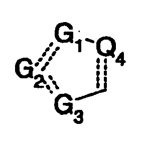 wherein
   G₁ is
   (1) -N(R³)-
      wherein Q₄ is as defined above,
      R³ is
      (1) a hydrogen atom,
      (2) -L₃-J₃-R¹¹³
         wherein
         L₃ is
         (1) a bond, or
         (2) a C₁₋₆ alkylene group,
         J₃ is
         (1) -N(R¹¹⁴)- wherein R¹¹⁴ is a hydrogen atom or a C₁₋₆ alkyl group,
         (2) -O-,
         (3) -S-,
         (4) -CO-,
         (5) -SO₂-,
         (6) -CO-O-,
         (7) -CO-N(R¹¹⁴)- wherein R¹¹⁴ is as defined above, or
         (8) -SO₂-N(R¹¹⁴)- wherein R¹¹⁴ is as defined above,
         R¹¹³ is
         (1) a hydrogen atom,
         (2) -L₄-J₄-R¹¹⁵
         wherein
         L₄ is
         (1) a bond, or
         (2) a C₁₋₆ alkylene group,
         J₄ is
         (1) -N(R¹¹⁶)- wherein R¹¹⁶ is a hydrogen atom or a C₁₋₆ alkyl group,
         (2) -O-,
         (3) -S-,
         (4) -CO-,
         (5) -SO₂-,
         (6) -CO-O-,
         (7) -CO-N(R¹¹⁶)- wherein R¹¹⁶ is as defined above, or
         (8) -SO₂-N(R¹¹⁶)- wherein R¹¹⁶ is as defined above,
         R¹¹⁵ is
         (1) a hydrogen atom,
         (2) a C₁₋₆ alkyl group (said C₁₋₆ alkyl group is optionally substituted by 1 to 3 substituents selected from Group F),
         (3) a C₁₋₆ alkyl-carbonyl group (said C₁₋₆ alkyl-carbonyl group is optionally substituted by 1 to 3 substituents selected from Group F), or
         (4) a C₁₋₆ alkylsulfonyl group (said C₁₋₆ alkylsulfonyl group is optionally substituted by 1 to 3 substituents selected from Group F),
         or
         R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
      (3) a C₁₋₆ alkyl group [said C₁₋₆ alkyl group is optionally substituted by 1 to 3 substituents selected from a halogen atom, a cyano group, a nitro group, C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
      (4) a substituent selected from a halogen atom, a cyano group and a nitro group,
      (5) a C₆₋₁₄ aryl group (said C₆₋₁₄ aryl group is optionally substituted by 1 to 5 substituents selected from Group E),
      (6) a heteroaryl group (said heteroaryl group is optionally substituted by 1 to 5 substituents selected from Group E), or
      (7) a C₃₋₈ cycloalkyl group (said C₃₋₈ cycloalkyl group is optionally substituted by 1 to 5 substituents selected from Group E), or
      R¹¹³ and R¹¹⁴ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
   (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
   (2) -C(R³)(R⁴)-
      wherein
      R³ is as defined above,
      R⁴ is
      (1) a hydrogen atom,
      (2) a hydroxyl group,
      (3) a C₁₋₆ alkyl group, or
      (4) a C₁₋₆ alkoxy group,
   (3) -C(R³)(R⁴)-C(R⁵)(R⁶)-
      wherein
      R³ and R⁴ are as defined above,
      R⁵ and R⁶ are the same or different and each is
      (1) a hydrogen atom,
      (2) a hydroxyl group,
      (3) a C₁₋₆ alkyl group, or
      (4) a C₁₋₆ alkoxy group,
   (4) -S-,
   (5) -O-,
   (6) -C(R³)=
      wherein R³ is as defined above, or
   (7) -N=;
      G₂ is
      (1) -N(R^{3'})-
         wherein
         R^{3'} is
         (1) a hydrogen atom,
         (2) a hydroxyl group,
         (3) a C₁₋₆ alkyl group, or
         (4) a C₁₋₆ alkoxy group,
      (2) -C(R^{3'})(R^{4'})-
         wherein
         R^{3'} is as defined above,
         R^{4'} is
         (1) a hydrogen atom,
         (2) a hydroxyl group,
         (3) a C₁₋₆ alkyl group, or
         (4) a C₁₋₆ alkoxy group,
         (3) -C(R^{3'})(R^{4'})-C(R^{5'})(R^{6'})-
            wherein
            R^{3'} and R^{4'} are each as defined above,
            R^{5'} and R^{6'} are the same or different and each is
            (1) a hydrogen atom,
            (2) a hydroxyl group,
            (3) a C₁₋₆ alkyl group, or
            (4) a C₁₋₆ alkoxy group,
         (4) -S-,
         (5) -O-,
         (6) -C(R^{4'})=
            wherein R^{4'} is as defined above,
         (7) =C(R^{4'})-C(R^{6'})=
            wherein R^{4'} and R^{6'} are each as defined above,
         (8) =C(R^{4'})-N=
            wherein R^{4'} is as defined above,
         (9) =N-C(R^{6'})=
            wherein R^{6'} is as defined above, or
         (10) =N-N=;

G₃ is
(1) -N(R^{3''})-
   wherein
   R^{3''} is
   (1) a hydrogen atom,
   (2) a hydroxyl group,
   (3) a C₁₋₆ alkyl group, or
   (4) a C₁₋₆ alkoxy group,
(2) -C(R^{3''})(R^{4''})-
   wherein
   R^{3''} is as defined above,
   R^{4''} is
   (1) a hydrogen atom,
   (2) a hydroxyl group,
   (3) a C₁₋₆ alkyl group, or
   (4) a C₁₋₆ alkoxy group],
(3) -C(R^{3''})(R^{4''})-C(R^{5''})(R^{6''})-
   wherein
   R^{3''} and R^{4''} are each as defined above,
   R^{5''} and R^{6''} are the same or different and each is
   (1) a hydrogen atom,
   (2) a hydroxyl group,
   (3) a C₁₋₆ alkyl group, or
   (4) a C₁₋₆ alkoxy group,
(4) -S-,
(5) -O-,
(6) =C(R^{4''})-
   wherein R^{4''} is as defined above, or
(7) =N-; 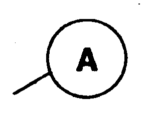 is 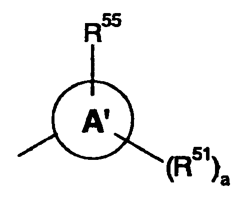 wherein
   ring A' is
   (1) a monocyclic aryl group, or
   (2) a monocyclic heteroaryl group;

R⁵⁵ is
J₅ is

(1) -N(R¹²²)- wherein R¹²² is a hydrogen atom or a C₁₋₆ alkyl group,
(2) -O-,
(3) -S-,
(4) -CO-,
(5) -CO-O-,
(6) -O-CO-,
(7) -CO-N(R¹²²)- wherein R¹²² is as defined above,
(8) -SO₂-N(R¹²²)- wherein R¹²² is as defined above,
(9) -N(R¹²³)-CO- wherein R¹²³ is a hydrogen atom or a C₁₋₆ alkyl group, or
(10) -N(R¹²³)-SO₂- wherein R¹²³ is as defined above,
   R¹²¹ is
   (1) a hydrogen atom,
   (2) -L₆-J₆-R¹²⁴
      wherein
      L₆ is
      (1) a bond, or
      (2) a C₁₋₆ alkylene group,
         J₆ is
         (1) -N(R¹²⁵)- wherein R¹²⁵ is a hydrogen atom or a C₁₋₆ alkyl group,
         (2) -O-,
         (3) -S-,
         (4) -CO-,
         (5) -CO-O-,
         (6) -O-CO-,
         (7) -CO-N(R¹²⁵)- wherein R¹²⁵ is as defined above,
         (8) -SO₂-N(R¹²⁵)- wherein R¹²⁵ is as defined above,
         (9) -N(R¹²⁶)-CO- wherein R¹²⁶ is a hydrogen atom or a C₁₋₆ alkyl group,
         (10) -N(R¹²⁶)-SO₂- wherein R¹²⁶ is as defined above,
         (11) -N(R¹²⁶)-CO-O- wherein R¹²⁶ is as defined above, or
         (12) -N(R¹²⁶)-CO-N(R¹²⁵)- wherein R¹²⁵ and R¹²⁶ are as defined above,
         R¹²⁴ is
         (1) a hydrogen atom, or
         (2) a C₁₋₆ alkyl group (said C₁₋₆ alkyl group is optionally substituted by 1 to 3 substituents selected from Group G, or
            R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
         (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
         (4) a substituent selected from a halogen atom, a cyano group and a nitro group,
         (5) a C₆₋₁₄ aryl group (said C₆₋₁₄ aryl group is optionally substituted by 1 to 5 substituents selected from Group E),
         (6) a heteroaryl group (said heteroaryl group is optionally substituted by 1 to 5 substituents selected from Group E), or
         (7) a C₃₋₈ cycloalkyl group (said C₃₋₈ cycloalkyl group is optionally substituted by 1 to 5 substituents selected from Group E), or
      R¹²¹ and R¹²² may form, together with the nitrogen atom to which they are bonded, a nitrogen-containing 4-to 7-membered heterocyclic group, wherein said nitrogen-containing 4- to 7-membered heterocyclic group is optionally substituted by 1 to 5 substituents selected from
      (1) Group F,
      (2) a C₁₋₆ alkyl group (said C₁₋₆ alkyl group is optionally substituted by 1 to 3 substituents selected from Group F),
      (3) a C₁₋₆ alkoxy group (said C₁₋₆ alkoxy group is optionally substituted by 1 to 3 substituents selected from Group F), and
      (4) an oxo group,
      (7) a monocyclic aryl group (said monocyclic aryl group is optionally substituted by 1 to 5 substituents selected from Group E),
      (8) a monocyclic heteroaryl group (said monocyclic heteroaryl group is optionally substituted by 1 to 5 substituents selected from Group E),
      (9) a C₃₋₈ cycloalkyl group (said C₃₋₈ cycloalkyl group is optionally substituted by 1 to 5 substituents selected from Group E), or
      (10) a heterocycloalkyl group (said heterocycloalkyl group is optionally substituted by 1 to 5 substituents selected from Group E);
         a is 1 or 2;
         R⁵¹ are the same or different and each is

      (1) a hydrogen atom,
      (2) a halogen atom,
      (3) a cyano group,
      (4) a nitro group,
      (5) a hydroxyl group,
      (6) a mercapto group,
      (7) a formyl group,
      (8) a carboxyl group,
      (9) a C₁₋₆ alkyl group (said C₁₋₆ alkyl group is optionally substituted by 1 to 3 substituents selected from Group G),
      (10) a C₂₋₆ alkenyl group (said C₂₋₆ alkenyl group is optionally substituted by 1 to 3 substituents selected from Group G),
      (11) a C₂₋₆ alkynyl group (said C₂₋₆ alkynyl group is optionally substituted by 1 to 3 substituents selected from Group G),
      (12) a C₁₋₆ alkoxy group (said C₁₋₆ alkoxy group is optionally substituted by 1 to 3 substituents selected from Group G),
      (13) a C₁₋₆ alkylthio group (said C₁₋₆ alkylthio group is optionally substituted by 1 to 3 substituents selected from Group G),
      (14) a C₁₋₆ alkylsulfinyl group (said C₁₋₆ alkylsulfinyl group is optionally substituted by 1 to 3 substituents selected from Group G),
      (15) a C₁₋₆ alkylsulfonyl group (said C₁₋₆ alkylsulfonyl group is optionally substituted by 1 to 3 substituents selected from Group G),
      (16) a C₁₋₆ alkyl-carbonyl group (said C₁₋₆ alkyl-carbonyl group is optionally substituted by 1 to 3 substituents selected from Group G),
      (17) a C₁₋₆ alkoxy-carbonyl group (said C₁₋₆ alkoxy-carbonyl group is optionally substituted by 1 to 3 substituents selected from Group G),
      (18) -N(R⁵⁷)(R⁵⁸)
         wherein
         R⁵⁷ and R⁵⁸ are the same or different and each is
         (1) a hydrogen atom,
         (2) a C₁₋₆ alkyl group (said C₁₋₆ alkyl group is optionally substituted by 1 to 3 substituents selected from Group G),
         (3) a C₁₋₆ alkylsulfonyl group (said C₁₋₆ alkylsulfonyl group is optionally substituted by 1 to 3 substituents selected from Group G),
         (4) a C₁₋₆ alkyl-carbonyl group (said C₁₋₆ alkyl-carbonyl group is optionally substituted by 1 to 3 substituents selected from Group G),
         (5) a C₁₋₆ alkoxy-carbonyl group (said C₁₋₆ alkoxy-carbonyl group is optionally substituted by 1 to 3 substituents selected from Group G),
         (6) a carbamoyl group,
         (7) a C₁₋₆ alkylamino-carbonyl group (said C₁₋₆ alkylamino-carbonyl group is optionally substituted by 1 to 3 substituents selected from Group G),
         (8) a di(C₁₋₆ alkyl)amino-carbonyl group (said di(C₁₋₆ alkyl)amino-carbonyl group is optionally substituted by 1 to 3 substituents selected from Group G),
         (9) a hydroxyl group,
         (10) a C₁₋₆ alkoxy group (said C₁₋₆ alkoxy group is optionally substituted by 1 to 3 substituents selected from Group G),
         (11) an amino group,
         (12) a C₁₋₆ alkylamino group (said C₁₋₆ alkylamino group is optionally substituted by 1 to 3 substituents selected from Group G),
         (13) a di(C₁₋₆ alkyl)amino group (said di(C₁₋₆ alkyl)amino group is optionally substituted by 1 to 3 substituents selected from Group G), or
         (14) a C₁₋₆ alkyl-carbonyl-amino group (said C₁₋₆ alkyl-carbonyl-amino group is optionally substituted by 1 to 3 substituents selected from Group G), or
            R⁵⁷ and R⁵⁸ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
      (19) -CO-N(R⁵⁷)(R⁵⁸) wherein R⁵⁷ and R⁵⁸ are as defined above,
      (20) -C(R⁵⁹)=N(R⁶⁰)
         wherein
         R⁵⁹ is
         (1) a hydrogen atom,
         (2) a C₁₋₆ alkyl group, or
         (3) an amino group,
         R⁶⁰ is
         (1) a hydroxyl group,
         (2) a C₁₋₆ alkoxy group (said C₁₋₆ alkoxy group is optionally substituted by 1 to 3 substituents selected from Group G),
         (3) a C₁₋₆ alkyl-carbonyl-oxy group (said C₁₋₆ alkyl-carbonyl-oxy group is optionally substituted by 1 to 3 substituents selected from Group G), or
         (4) -N(R⁶¹)(R⁶²) wherein R⁶¹ and R⁶² are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
      (21) a monocyclic aryl group (said monocyclic aryl group is optionally substituted by 1 to 5 substituents selected from Group E),
      (22) a monocyclic heteroaryl group (said monocyclic heteroaryl group is optionally substituted by 1 to 5 substituents selected from Group E), or
      (23) a heterocycloalkyl group (said heterocycloalkyl group is optionally substituted by 1 to 5 substituents selected from Group E), or
         two adjacent R⁵¹s may form, together with the ring to which they are bonded, a fused ring (said fused ring is optionally further substituted by one substituent selected from Group E); 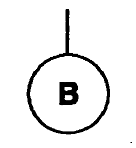 is 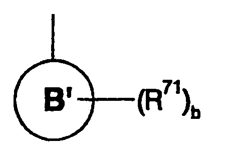
      wherein
      ring B' is
      (1) a monocyclic aryl group,
      (2) a monocyclic heteroaryl group,
      (3) a C₃₋₈ cycloalkyl group, or
      (4) a C₃₋₈ cycloalkenyl group;
         b is an integer of 1-3;
         R⁷¹ are the same or different and each is

      (1) a hydrogen atom,
      (2) a halogen atom,
      (3) a cyano group,
      (4) a nitro group,
      (5) a hydroxyl group,
      (6) a mercapto group,
      (7) a formyl group,
      (8) a carboxyl group,
      (9) a C₁₋₆ alkyl group (said C₁₋₆ alkyl group is optionally substituted by 1 to 3 substituents selected from Group G),
      (10) a C₂₋₆ alkenyl group (said C₂₋₆ alkenyl group is optionally substituted by 1 to 3 substituents selected from Group G),
      (11) a C₂₋₆ alkynyl group (said C₂₋₆ alkynyl group is optionally substituted by 1 to 3 substituents selected from Group G),
      (12) a C₁₋₆ alkoxy group (said C₁₋₆ alkoxy group is optionally substituted by 1 to 3 substituents selected from Group G),
      (13) a C₁₋₆ alkylthio group (said C₁₋₆ alkylthio group is optionally substituted by 1 to 3 substituents selected from Group G),
      (14) a C₁₋₆ alkylsulfinyl group (said C₁₋₆ alkylsulfinyl group is optionally substituted by 1 to 3 substituents selected from Group G),
      (15) a C₁₋₆ alkylsulfonyl group (said C₁₋₆ alkylsulfonyl group is optionally substituted by 1 to 3 substituents selected from Group G),
      (16) a C₁₋₆ alkyl-carbonyl group (said C₁₋₆ alkyl-carbonyl group is optionally substituted by 1 to 3 substituents selected from Group G),
      (17) a C₁₋₆ alkoxy-carbonyl group (said C₁₋₆ alkoxy-carbonyl group is optionally substituted by 1 to 3 substituents selected from Group G),
      (18) -N(R⁷²)(R⁷³)
         wherein
         (1) a hydrogen atom,
         (2) a C₁₋₆ alkyl group (said C₁₋₆ alkyl group is optionally substituted by 1 to 3 substituents selected from Group G),
         (3) a C₁₋₆ alkylsulfonyl group (said C₁₋₆ alkylsulfonyl group is optionally substituted by 1 to 3 substituents selected from Group G),
         (4) a C₁₋₆ alkyl-carbonyl group (said C₁₋₆ alkyl-carbonyl group is optionally substituted by 1 to 3 substituents selected from Group G),
         (5) a C₁₋₆ alkoxy-carbonyl group (said C₁₋₆ alkoxy-carbonyl group is optionally substituted by 1 to 3 substituents selected from Group G),
         (6) a carbamoyl group,
         (7) a C₁₋₆ alkylamino-carbonyl group (said C₁₋₆ alkylamino-carbonyl group is optionally substituted by 1 to 3 substituents selected from Group G),
         (8) a di(C₁₋₆ alkyl)amino-carbonyl group (said di(C₁₋₆ alkyl)amino-carbonyl group is optionally substituted by 1 to 3 substituents selected from Group G),
         (9) a hydroxyl group,
         (10) a C₁₋₆ alkoxy group (said C₁₋₆ alkoxy group is optionally substituted by 1 to 3 substituents selected from Group G),
         (11) an amino group,
         (12) a C₁₋₆ alkylamino group (said C₁₋₆ alkylamino group is optionally substituted by 1 to 3 substituents selected from Group G),
         (13) a di(C₁₋₆ alkyl) amino group (said di(C₁₋₆ alkyl)amino group is optionally substituted by 1 to 3 substituents selected from Group G), or (14) a C₁₋₆ alkyl-carbonyl-amino group (said C₁₋₆ alkyl-carbonyl-amino group is optionally substituted by 1 to 3 substituents selected from Group G), or
            R⁷² and R⁷³ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
      (19) -CO-N(R⁷²)(R⁷³) wherein R⁷² and R⁷³ are as defined above,
      (20) -C(R⁷⁴)=N(R⁷⁵)
         wherein
         R⁷⁴ is
         (1) a hydrogen atom,
         (2) a C₁₋₆ alkyl group, or
         (3) an amino group,
         R⁷⁵ is
         (1) a hydroxyl group,
         (2) a C₁₋₆ alkoxy group (said C₁₋₆ alkoxy group is optionally substituted by 1 to 3 substituents selected from Group G),
         (3) a C₁₋₆ alkyl-carbonyl-oxy group (said C₁₋₆ alkyl-carbonyl-oxy group is optionally substituted by 1 to 3 substituents selected from Group G), or
         (4) -N(R⁷⁶)(R⁷⁷) wherein R⁷⁶ and R⁷⁷ are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
      (21) a monocyclic aryl group (said monocyclic aryl group is optionally substituted by 1 to 5 substituents selected from Group E),
      (22) a monocyclic heteroaryl group (said monocyclic heteroaryl group is optionally substituted by 1 to 5 substituents selected from Group E), or
      (23) a heterocycloalkyl group (said heterocycloalkyl group is optionally substituted by 1 to 5 substituents selected from Group E), or
         two adjacent R⁷¹s may form, together with the ring to which they are bonded, a fused ring (said fused ring is optionally further substituted by one substituent selected from Group E):
      R⁷² and R⁷³ are the same or different and each is
      wherein
      Group E consists of
      (1) a halogen atom,
      (2) a cyano group,
      (3) a nitro group,
      (4) a hydroxyl group,
      (5) a mercapto group,
      (6) a formyl group,
      (7) a carboxyl group,
      (8) a carbamoyl group,
      (9) a sulfamoyl group,
      (10) an amino group,
      (11) a C₁₋₆ alkyl group (said C₁₋₆ alkyl group is optionally substituted by 1 to 3 substituents selected from Group G),
      (12) a C₂₋₆ alkenyl group (said C₂₋₆ alkenyl group is optionally substituted by 1 to 3 substituents selected from Group G),
      (13) a C₂₋₆ alkynyl group (said C₂₋₆ alkynyl group is optionally substituted by 1 to 3 substituents selected from Group G),
      (14) a C₁₋₆ alkoxy group (said C₁₋₆ alkoxy group is optionally substituted by 1 to 3 substituents selected from Group G),
      (15) a C₁₋₆ alkylthio group,
      (16) a C₁₋₆ alkylsulfinyl group,
      (17) a C₁₋₆ alkylsulfonyl group,
      (18) a C₁₋₆ alkyl-carbonyl group,
      (19) a C₁₋₆ alkoxy-carbonyl group,
      (20) a C₁₋₆ alkylamino group,
      (21) a C₆₋₁₄ aryl group,
      (22) a heteroaryl group, and
      (23) an oxo group;
         Group F consists of

      (1) a halogen atom,
      (2) a cyano group,
      (3) a nitro group,
      (4) a hydroxyl group,
      (5) a mercapto group,
      (6) a formyl group,
      (7) a carboxyl group,
      (8) a C₁₋₆ alkoxy group,
      (9) a C₁₋₆ alkylthio group,
      (10) a C₁₋₆ alkylsulfinyl group,
      (11) a C₁₋₆ alkylsulfonyl group,
      (12) a C₁₋₆ alkyl-carbonyl group,
      (13) a C₁₋₆ alkoxy-carbonyl group,
      (14) -N(R⁹¹)(R⁹²) wherein R⁹¹ and R⁹² are the same or different and each is a hydrogen atom, a C₁₋₆ alkyl group, a C₁₋₆ alkyl-carbonyl group, a C₁₋₆ alkylsulfonyl group, a C₁₋₆ alkoxy-carbonyl group, a carbamoyl group, a C₁₋₆ alkylamino-carbonyl group, or a di(C₁₋₆
         alkyl)amino-carbonyl group, or R⁹¹ and R⁹² may form, together with the nitrogen atom to which they are bonded, a nitrogen-containing 4- to 7-membered heterocyclic group,
      (15) -CO-N(R⁹¹)(R⁹²) wherein R⁹¹ and R⁹² are as defined above,
      (16) -SO₂-N(R⁹¹)(R⁹²) wherein R⁹¹ and R⁹² are as defined above,
      (17) -O-CO-N(R⁹¹)(R⁹²) wherein R⁹¹ and R⁹² are as defined above,
      (18) a C₆₋₁₄ aryl group,
      (19) a C₆₋₁₄ aryl-oxy group,
      (20) a C₆₋₁₄ aryl-carbonyl group, and
      (21) a heteroaryl group;
      Group G consists of
      (1) a halogen atom,
      (2) a cyano group,
      (3) a nitro group,
      (4) a hydroxyl group,
      (5) a mercapto group,
      (6) a formyl group,
      (7) a carboxyl group,
      (8) a carbamoyl group,
      (9) a sulfamoyl group,
      (10) an amino group,
      (11) a C₁₋₆ alkoxy group,
      (12) a C₁₋₆ alkylthio group,
      (13) a C₁₋₆ alkylsulfinyl group,
      (14) a C₁₋₆ alkylsulfonyl group,
      (15) a C₁₋₆ alkyl-carbonyl group,
      (16) a C₁₋₆ alkoxy-carbonyl group,
      (17) a C₁₋₆ alkylamino group,
      (18) a C₁₋₆ alkyl-carbonyl-amino group,
      (19) a C₆₋₁₄ aryl group, and
      (20) a heteroaryl group.

[2] Use of a heterocyclic compound represented by the formula [1] of the aforementioned [1] or a pharmaceutically acceptable salt thereof for the production of an anti-hepatitis virus agent.

[3] Use of a heterocyclic compound represented by the formula [1] of the aforementioned [1] or a pharmaceutically acceptable salt thereof for the production of an HCV entry inhibitor.

[4] The use of the aforementioned [1], which uses a heterocyclic compound as represented by the following formula [1] or a pharmaceutically acceptable salt thereof: 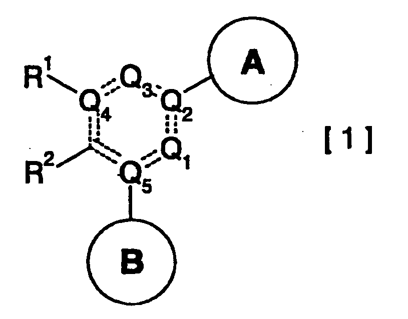 wherein 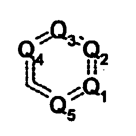 is 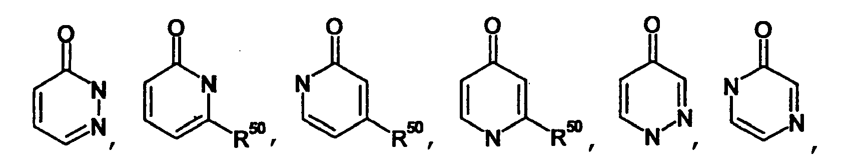 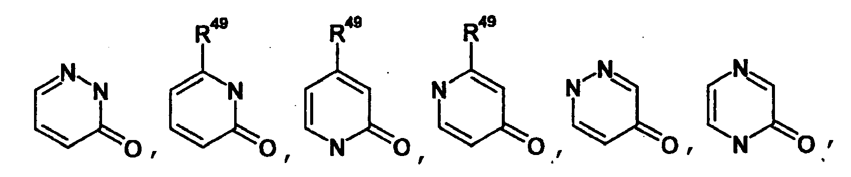 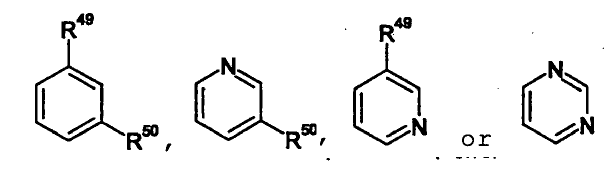 wherein
R⁴⁹ and R⁵⁰ are the same or different and each is
(1) a hydrogen atom,
(2) a hydroxyl group,
(3) a C₁₋₆ alkyl group, or
(4) a C₁₋₆ alkoxy group;
R¹ is:
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₁-R¹⁰¹
   wherein
   J₁ is
   (1) -N(R¹⁰²)- wherein R¹⁰² is a hydrogen atom or a C₁₋₆ alkyl group,
   (2) -O-,
   (3) -S-,
   (4) -CO-,
   (5) -CO-O-,
   (6) -O-CO-,
   (7) -CO-N(R¹⁰²)- wherein R¹⁰² is as defined above, or
   (8) -SO₂-N(R¹⁰²)- wherein R¹⁰² is as defined above,
   R¹⁰¹ is
   (1) a hydrogen atom,
   (2) -L₂-J₂-R¹⁰³
      wherein
      L₂ is
      (1) a bond, or
      (2) a C₁₋₆ alkylene group,
      J₂ is
      (1) -N(R¹⁰⁴)- wherein R¹⁰⁴ is a hydrogen atom or a C₁₋₆ alkyl group,
      (2) -O-,
      (3) -S-,
      (4) -CO-,
      (5) -SO₂-,
      (6) -CO-O-,
      (7) -CO-N(R¹⁰⁴)- wherein R¹⁰⁴ is as defined above, or
      (8) -SO₂-N(R¹⁰⁴)- wherein R¹⁰⁴ is as defined above,
      R¹⁰³ is
      (1) a hydrogen atom,
      (2) a C₁₋₆ alkyl group (said C₁₋₆ alkyl group is optionally substituted by 1 to 3 substituents selected from Group F),
      (3) a C₁₋₆ alkyl-carbonyl group (said C₁₋₆ alkyl-carbonyl group is optionally substituted by 1 to 3 substituents selected from Group F), or
      (4) a C₁₋₆ alkylsulfonyl group (said C₁₋₆ alkylsulfonyl group is optionally substituted by 1 to 3 substituents selected from Group F),
   (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
   (4) a substituent selected from a halogen atom, a cyano group and a nitro group,
   (5) a C₆₋₁₄ aryl group (said C₆₋₁₄ aryl group is optionally substituted by 1 to 5 substituents selected from Group E),
   (6) a heteroaryl group (said heteroaryl group is optionally substituted by 1 to 5 substituents selected from Group E), or
   (7) a C₃₋₈ cycloalkyl group (said C₃₋₈ cycloalkyl group is optionally substituted by 1 to 5 substituents selected from Group E), or
   R¹⁰¹ and R¹⁰² may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(7) a C₆₋₁₄ aryl group (said C₆₋₁₄ aryl group is optionally substituted by 1 to 5 substituents selected from Group E), or
(8) a heteroaryl group (said heteroaryl group is optionally substituted by 1 to 5 substituents selected from Group E);
R² is:
(1) a hydrogen atom, or
(2) a C₁₋₆ alkyl group; or
R¹ and R² may form, together with Q₄ and the carbon atom to which they are bonded respectively: 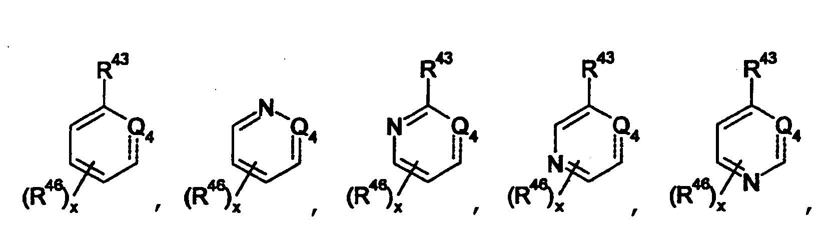 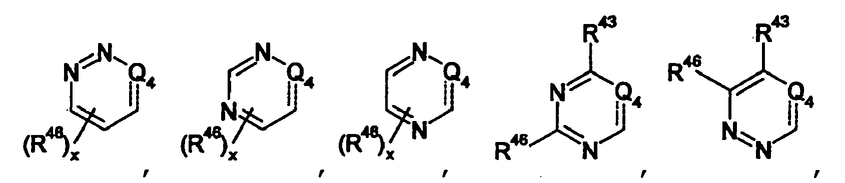 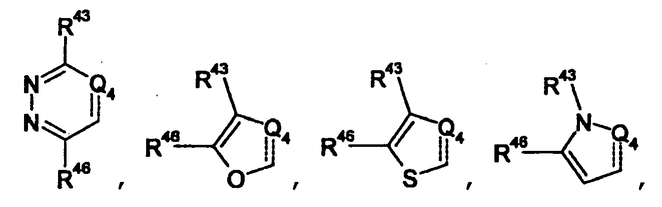 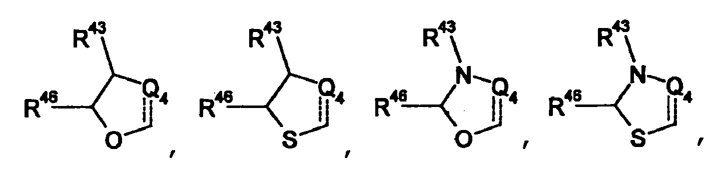 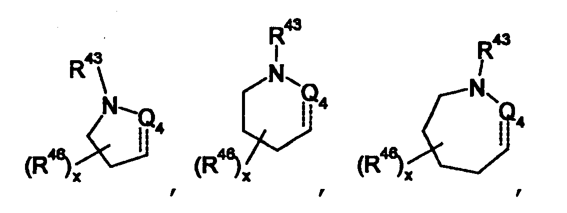 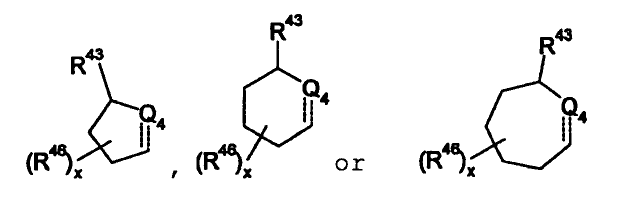 wherein
Q₄ is as defined above,
R⁴³ is
(1) a hydrogen atom,
(2) -L₃-J₃-R¹¹³
   wherein
   L₃ is
   (1) a bond, or
   (2) a C₁₋₆ alkylene group,
   J₃ is
   (1) -N(R¹¹⁴)- wherein R¹¹⁴ is a hydrogen atom or a C₁₋₆ alkyl group,
   (2) -O-,
   (3) -S-,
   (4) -CO-,
   (5) -SO₂-,
   (6) -CO-O-,
   (7) -CO-N(R¹¹⁴)- wherein R¹¹⁴ is as defined above, or
   (8) -SO₂-N(R¹¹⁴)- wherein R¹¹⁴ is as defined above,
   R¹¹³ is
   (1) a hydrogen atom,
   (2) -L₄-J₄-R¹¹⁵
      wherein
      L₄ is
      (1) a bond, or
      (2) a C₁₋₆ alkylene group,
         J₄ is
         (1) -N(R¹¹⁶)- wherein R¹¹⁶ is a hydrogen atom or a C₁₋₆ alkyl group,
         (2) -O-,
         (3) -S-,
         (4) -CO-,
         (5) -SO₂-,
         (6) -CO-O-,
         (7) -CO-N(R¹¹⁶)- wherein R¹¹⁶ is as defined above, or
         (8) -SO₂-N(R¹¹⁶)- wherein R¹¹⁶ is as defined above,
         R¹¹⁵ is
         (1) a hydrogen atom,
         (2) a C₁₋₆ alkyl group (said C₁₋₆ alkyl group is optionally substituted by 1 to 3 substituents selected from Group G),
         (3) a C₁₋₆ alkyl-carbonyl group (said C₁₋₆ alkyl-carbonyl group is optionally substituted by 1 to 3 substituents selected from Group G), or
         (4) a C₁₋₆ alkylsulfonyl group (said C₁₋₆ alkylsulfonyl group is optionally substituted by 1 to 3 substituents selected from Group G), or
         R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
      (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
      (4) a substituent selected from a halogen atom, a cyano group and a nitro group,
      (5) a C₆₋₁₄ aryl group (said C₆₋₁₄ aryl group is optionally substituted by 1 to 5 substituents selected from Group E),
      (6) a heteroaryl group (said heteroaryl group is optionally substituted by 1 to 5 substituents selected from Group E), or
      (7) a C₃₋₈ cycloalkyl group (said C₃₋₈ cycloalkyl group is optionally substituted by 1 to 5 substituents selected from Group E), or
      R¹¹³ and R¹¹⁴ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
   (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E);
   x is 1 or 2;

R⁴⁶ are the same or different and each is
(1) a hydrogen atom,
(2) a hydroxyl group,
(3) a C₁₋₆ alkyl group, or
(4) a C₁₋₆ alkoxy group; 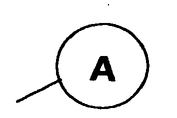 is 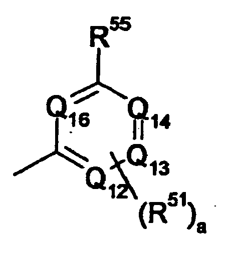 wherein
   Q₁₂, Q₁₃, Q₁₄, and Q₁₆ are the same or different and each is
   (1) -N=, or
   (2) -CH=;
   R⁵⁵ is
   (1) a hydrogen atom,
   (2) a substituent selected from a halogen atom, a cyano group and a nitro group,
   (3) a C₁₋₆ alkyl group (said C₁₋₆ alkyl group is optionally substituted by 1 to 3 substituents selected from Group F),
   (4) a C₂₋₆ alkenyl group (said C₂₋₆ alkenyl group is optionally substituted by 1 to 3 substituents selected from Group F),
   (5) a C₂₋₆ alkynyl group (said C₂₋₆ alkynyl group is optionally substituted by 1 to 3 substituents selected from Group F),
   (6) -J₅-R¹²¹
      wherein
      J₅ is
      (1) -N(R¹²²)- wherein _{R}¹²² is a hydrogen atom or a C₁₋₆ alkyl group,
      (2) -O-,
      (3) -S-,
      (4) -CO-,
      (5) -CO-O-,
      (6) -O-CO-,
      (7) -CO-N(R¹²²)- wherein R¹²² is as defined above,
      (8) -SO₂-N(R¹²²)- wherein R¹²² is as defined above,
      (9) -N(R¹²³)-CO- wherein R¹²³ is a hydrogen atom or a C₁₋₆ alkyl group, or
      (10) -N(R¹²³)-SO₂- wherein R¹²³ is as defined above,
         R¹²¹ is
         (1) a hydrogen atom,
         (2) -L₆-J₆-R¹²⁴
         wherein
         L₆ is
         (1) a bond, or
         (2) a C₁₋₆ alkylene group,
         J₆ is
         (1) -N(R¹²⁵)- wherein R¹²⁵ is a hydrogen atom or a C₁₋₆ alkyl group,
         (2) -O-,
         (3) -S-,
         (4) -CO-,
         (5) -CO-O-,
         (6) -O-CO-,
         (7) -CO-N(R¹²⁵)- wherein R¹²⁵ is as defined above,
         (8) -SO₂-N(R¹²⁵)- wherein R¹²⁵ is as defined above,
         (9) -N(R¹²⁶)-CO- wherein R¹²⁶ is a hydrogen atom or a C₁₋₆ alkyl group,
         (10) -N(R¹²⁶)-SO₂- wherein R¹²⁶ is as defined above,
         (11) -N(R¹²⁶)-CO-O- wherein R¹²⁶ is as defined above, or
         (12) -N(R¹²⁶)-CO-N(R¹²⁵)- wherein R¹²⁵ and R¹²⁶ are as defined above,
         R¹²⁴ is
         (1) a hydrogen atom, or
         (2) a C₁₋₆ alkyl group (said C₁₋₆ alkyl group is optionally substituted by 1 to 3 substituents selected from Group G), or
         R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),

(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
   R¹²¹ and R¹²² may form, together with the nitrogen atom to which they are bonded, a nitrogen-containing 4-to 7-membered heterocyclic group, wherein said nitrogen-containing 4- to 7-membered heterocyclic group is optionally substituted by 1 to 5 substituents selected from

(1) Group F,
(2) a C₁₋₆ alkyl group (said C₁₋₆ alkyl group is optionally substituted by 1 to 3 substituents selected from Group F),
(3) a C₁₋₆ alkoxy group (said C₁₋₆ alkoxy group is optionally substituted by 1 to 3 substituents selected from Group F), and
(4) an oxo group,
   (7) a monocyclic aryl group (said monocyclic aryl group is optionally substituted by 1 to 5 substituents selected from Group E),
   (8) a monocyclic heteroaryl group (said monocyclic heteroaryl group is optionally substituted by 1 to 5 substituents selected from Group E),
   (9) a C₃₋₈ cycloalkyl group (said C₃₋₈ cycloalkyl group is optionally substituted by 1 to 5 substituents selected from Group E), or
   (10) a heterocycloalkyl group (said heterocycloalkyl group is optionally substituted by 1 to 5 substituents selected from Group E);
   a is 1 or 2;
   R⁵¹ are the same or different and each is
   (1) a hydrogen atom,
   (2) a halogen atom,
   (3) a cyano group,
   (4) a nitro group,
   (5) a hydroxyl group,
   (6) a mercapto group,
   (7) a formyl group,
   (8) a carboxyl group,
   (9) a C₁₋₆ alkyl group (said C₁₋₆ alkyl group is optionally substituted by 1 to 3 substituents selected from Group G),
   (10) a C₂₋₆ alkenyl group (said C₂₋₆ alkenyl group is optionally substituted by 1 to 3 substituents selected from Group G),
   (11) a C₂₋₆ alkynyl group (said C₂₋₆ alkynyl group is optionally substituted by 1 to 3 substituents selected from Group G),
   (12) a C₁₋₆ alkoxy group (said C₁₋₆ alkoxy group is optionally substituted by 1 to 3 substituents selected from Group G),
   (13) a C₁₋₆ alkylthio group (said C₁₋₆ alkylthio group is optionally substituted by 1 to 3 substituents selected from Group G),
   (14) a C₁₋₆ alkylsulfinyl group (said C₁₋₆ alkylsulfinyl group is optionally substituted by 1 to 3 substituents selected from Group G),
   (15) a C₁₋₆ alkylsulfonyl group (said C₁₋₆ alkylsulfonyl group is optionally substituted by 1 to 3 substituents selected from Group G),
   (16) a C₁₋₆ alkyl-carbonyl group (said C₁₋₆ alkyl-carbonyl group is optionally substituted by 1 to 3 substituents selected from Group G),
   (17) a C₁₋₆ alkoxy-carbonyl group (said C₁₋₆ alkoxy-carbonyl group is optionally substituted by 1 to 3 substituents selected from Group G),
   (18) -N(R⁵⁷)(R⁵⁸)
      wherein
      R⁵⁷ and R⁵⁸ are the same or different and each is
      (1) a hydrogen atom,
      (2) a C₁₋₆ alkyl group (said C₁₋₆ alkyl group is optionally substituted by 1 to 3 substituents selected from Group G),
      (3) a C₁₋₆ alkylsulfonyl group (said C₁₋₆ alkylsulfonyl group is optionally substituted by 1 to 3 substituents selected from Group G),
      (4) a C₁₋₆ alkyl-carbonyl group (said C₁₋₆ alkyl-carbonyl group is optionally substituted by 1 to 3 substituents selected from Group G),
      (5) a C₁₋₆ alkoxy-carbonyl group (said C₁₋₆ alkoxy-carbonyl group is optionally substituted by 1 to 3 substituents selected from Group G),
      (6) a carbamoyl group,
      (7) a C₁₋₆ alkylamino-carbonyl group (said C₁₋₆ alkylamino-carbonyl group is optionally substituted by 1 to 3 substituents selected from Group G),
      (8) a di(C₁₋₆ alkyl)amino-carbonyl group (said di(C₁₋₆ alkyl)amino-carbonyl group is optionally substituted by 1 to 3 substituents selected from Group G),
      (9) a hydroxyl group,
      (10) a C₁₋₆ alkoxy group (said C₁₋₆ alkoxy group is optionally substituted by 1 to 3 substituents selected from Group G),
      (11) an amino group,
      (12) a C₁₋₆ alkylamino group (said C₁₋₆ alkylamino group is optionally substituted by 1 to 3 substituents selected from Group G),
      (13) a di(C₁₋₆ alkyl)amino group (said di(C₁₋₆ alkyl)amino group is optionally substituted by 1 to 3 substituents selected from Group G), or
      (14) a C₁₋₆ alkyl-carbonyl-amino group (said C₁₋₆ alkyl-carbonyl-amino group is optionally substituted by 1 to 3 substituents selected from Group G), or
         R⁵⁷ and R⁵⁸ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
   (19) -CO-N(R⁵⁷)(R⁵⁸) wherein R⁵⁷ and R⁵⁸ is as defined above,
   (20) -C(R⁵⁹)=N(R⁶⁰)
      wherein
      R⁵⁹ is
      (1) a hydrogen atom,
      (2) a C₁₋₆ alkyl group, or
      (3) an amino group,
      R⁶⁰ is
      (1) a hydroxyl group,
      (2) a C₁₋₆ alkoxy group (said C₁₋₆ alkoxy group is optionally substituted by 1 to 3 substituents selected from Group G),
      (3) a C₁₋₆ alkyl-carbonyl-oxy group (said C₁₋₆ alkyl-carbonyl-oxy group is optionally substituted by 1 to 3 substituents selected from Group G), or
      (4) -N(R⁶¹)(R⁶²) wherein R⁶¹ and R⁶² are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
   (21) a monocyclic aryl group (said monocyclic aryl group is optionally substituted by 1 to 5 substituents selected from Group E),
   (22) a monocyclic heteroaryl group (said monocyclic heteroaryl group is optionally substituted by 1 to 5 substituents selected from Group E), or
   (23) a heterocycloalkyl group (said heterocycloalkyl group is optionally substituted by 1 to 5 substituents selected from Group E), or
      two adjacent R⁵¹s may form, together with the ring to which they are bonded, a fused ring (said fused ring is optionally further substituted by one substituent selected from Group E); 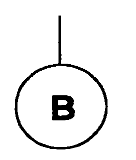 is 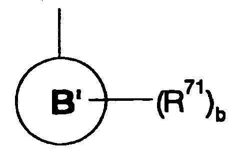 wherein
      ring B' is
      (1) a monocyclic aryl group, or
      (2) a monocyclic heteroaryl group;
      b is an integer of 1-3;
      R⁷¹ are the same or different and each is
      (1) a hydrogen atom,
      (2) a halogen atom,
      (3) a cyano group,
      (4) a nitro group,
      (5) a hydroxyl group,
      (6) a mercapto group,
      (7) a formyl group,
      (8) a carboxyl group,
      (9) a C₁₋₆ alkyl group (said C₁₋₆ alkyl group is optionally substituted by 1 to 3 substituents selected from Group G),
      (10) a C₂₋₆ alkenyl group (said C₂₋₆ alkenyl group is optionally substituted by 1 to 3 substituents selected from Group G),
      (11) a C₂₋₆ alkynyl group (said C₂₋₆ alkynyl group is optionally substituted by 1 to 3 substituents selected from Group G),
      (12) a C₁₋₆ alkoxy group (said C₁₋₆ alkoxy group is optionally substituted by 1 to 3 substituents selected from Group G),
      (13) a C₁₋₆ alkylthio group (said C₁₋₆ alkylthio group is optionally substituted by 1 to 3 substituents selected from Group G),
      (14) a C₁₋₆ alkylsulfinyl group (said C₁₋₆ alkylsulfinyl group is optionally substituted by 1 to 3 substituents selected from Group G),
      (15) a C₁₋₆ alkylsulfonyl group (said C₁₋₆ alkylsulfonyl group is optionally substituted by 1 to 3 substituents selected from Group G),
      (16) a C₁₋₆ alkyl-carbonyl group (said C₁₋₆ alkyl-carbonyl group is optionally substituted by 1 to 3 substituents selected from Group G),
      (17) a C₁₋₆ alkoxy-carbonyl group (said C₁₋₆ alkoxy-carbonyl group is optionally substituted by 1 to 3 substituents selected from Group G),
      (18) -N(R⁷²)(R⁷³)
         wherein
         R⁷² and R⁷³ are the same or different and each is
         (1) a hydrogen atom,
         (2) a C₁₋₆ alkyl group (said C₁₋₆ alkyl group is optionally substituted by 1 to 3 substituents selected from Group G),
         (3) a C₁₋₆ alkylsulfonyl group (said C₁₋₆ alkylsulfonyl group is optionally substituted by 1 to 3 substituents selected from Group G),
         (4) a C₁₋₆ alkyl-carbonyl group (said C₁₋₆ alkyl-carbonyl group is optionally substituted by 1 to 3 substituents selected from Group G),
         (5) a C₁₋₆ alkoxy-carbonyl group (said C₁₋₆ alkoxy-carbonyl group is optionally substituted by 1 to 3 substituents selected from Group G),
         (6) a carbamoyl group,
         (7) a C₁₋₆ alkylamino-carbonyl group (said C₁₋₆ alkylamino-carbonyl group is optionally substituted by 1 to 3 substituents selected from Group G),
         (8) a di(C₁₋₆ alkyl)amino-carbonyl group (said di(C₁₋₆ alkyl)amino-carbonyl group is optionally substituted by 1 to 3 substituents selected from Group G),
         (9) a hydroxyl group,
         (10) a C₁₋₆ alkoxy group (said C₁₋₆ alkoxy group is optionally substituted by 1 to 3 substituents selected from Group G),
         (11) an amino group,
         (12) a C₁₋₆ alkylamino group (said C₁₋₆ alkylamino group is optionally substituted by 1 to 3 substituents selected from Group G),
         (13) a di(C₁₋₆ alkyl)amino group (said di(C₁₋₆ alkyl)amino group is optionally substituted by 1 to 3 substituents selected from Group G), or
         (14) a C₁₋₆ alkyl-carbonyl-amino group (said C₁₋₆ alkyl-carbonyl-amino group is optionally substituted by 1 to 3 substituents selected from Group G), or
            R⁷² and R⁷³ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
   (19) -CO-N(R⁷²)(R⁷³) wherein R⁷² and R⁷³ is as defined above,
   (20) -C(R⁷⁴)=N(R⁷⁵)
      wherein
      R⁷⁴ is
      (1) a hydrogen atom,
      (2) a C₁₋₆ alkyl group, or
      (3) an amino group,
      R⁷⁵ is
      (1) a hydroxyl group,
      (2) a C₁₋₆ alkoxy group (said C₁₋₆ alkoxy group is optionally substituted by 1 to 3 substituents selected from Group G),
      (3) a C₁₋₆ alkyl-carbonyl-oxy group (said C₁₋₆ alkyl-carbonyl-oxy group is optionally substituted by 1 to 3 substituents selected from Group G), or
      (4) -N(R⁷⁶)(R⁷⁷) wherein R⁷⁶ and R⁷⁷ are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
   (21) a monocyclic aryl group (said monocyclic aryl group is optionally substituted by 1 to 5 substituents selected from Group E),
   (22) a monocyclic heteroaryl group (said monocyclic heteroaryl group is optionally substituted by 1 to 5 substituents selected from Group E), or
   (23) a heterocycloalkyl group (said heterocycloalkyl group is optionally substituted by 1 to 5 substituents selected from Group E), or
      two adjacent R⁷¹s may form, together with the ring to which they are bonded, a fused ring (said fused ring is optionally further substituted by one substituent selected from Group E):
      wherein
      Group E consists of
      (1) a halogen atom,
      (2) a cyano group,
      (3) a nitro group,
      (4) a hydroxyl group,
      (5) a mercapto group,
      (6) a formyl group,
      (7) a carboxyl group,
      (8) a carbamoyl group,
      (9) a sulfamoyl group,
      (10) an amino group,
      (11) a C₁₋₆ alkyl group (said C₁₋₆ alkyl group is optionally substituted by 1 to 3 substituents selected from Group G),
      (12) a C₂₋₆ alkenyl group (said C₂₋₆ alkenyl group is optionally substituted by 1 to 3 substituents selected from Group G),
      (13) a C₂₋₆ alkynyl group (said C₂₋₆ alkynyl group is optionally substituted by 1 to 3 substituents selected from Group G),
      (14) a C₁₋₆ alkoxy group (said C₁₋₆ alkoxy group is optionally substituted by 1 to 3 substituents selected from Group G),
      (15) a C₁₋₆ alkylthio group,
      (16) a C₁₋₆ alkylsulfinyl group,
      (17) a C₁₋₆ alkylsulfonyl group,
      (18) a C₁₋₆ alkyl-carbonyl group,
      (19) a C₁₋₆ alkoxy-carbonyl group,
      (20) a C₁₋₆ alkylamino group,
      (21) a C₆₋₁₄ aryl group,
      (22) a heteroaryl group, and
      (23) an oxo group;
         Group F consists of
         (1) a halogen atom,
         (2) a cyano group,
         (3) a nitro group,
         (4) a hydroxyl group,
         (5) a mercapto group,
         (6) a formyl group,
         (7) a carboxyl group,
         (8) a C₁₋₆ alkoxy group,
         (9) a C₁₋₆ alkylthio group,
         (10) a C₁₋₆ alkylsulfinyl group,
         (11) a C₁₋₆ alkylsulfonyl group,
         (12) a C₁₋₆ alkyl-carbonyl group,
         (13) a C₁₋₆ alkoxy-carbonyl group,
         (14) -N(R⁹¹)(R⁹²) wherein R⁹¹ and R⁹² are the same or different and each is a hydrogen atom, a C₁₋₆ alkyl group, a C₁₋₆ alkyl-carbonyl group, a C₁₋₆ alkylsulfonyl group, a C₁₋₆ alkoxycarbonyl group, a carbamoyl group, a C₁₋₆ alkylamino-carbonyl group, or a di(C₁₋₆ alkyl) amino-carbonyl group, or R⁹¹ and R⁹² may form, together with the nitrogen atom to which they are bonded, a nitrogen-containing 4- to 7-membered heterocyclic group,
         (15) -CO-N(R⁹¹)(R⁹²) wherein R⁹¹ and R⁹² are as defined above,
         (16) -SO₂-N(R⁹¹)(R⁹²) wherein R⁹¹ and R⁹² are as defined above,
         (17) -O-CO-N(R⁹¹)(R⁹²) wherein R⁹¹ and R⁹² are as defined above,
         (18) a C₆₋₁₄ aryl group,
         (19) a C₆₋₁₄ aryl-oxy group,
         (20) a C₆₋₁₄ aryl-carbonyl group, and
         (21) a heteroaryl group;
         Group G consists of
         (1) a halogen atom,
         (2) a cyano group,
         (3) a nitro group,
         (4) a hydroxyl group,
         (5) a mercapto group,
         (6) a formyl group,
         (7) a carboxyl group,
         (8) a carbamoyl group,
         (9) a sulfamoyl group,
         (10) an amino group,
         (11) a C₁₋₆ alkoxy group,
         (12) a C₁₋₆ alkylthio group,
         (13) a C₁₋₆ alkylsulfinyl group,
         (14) a C₁₋₆ alkylsulfonyl group,
         (15) a C₁₋₆ alkyl-carbonyl group,
         (16) a C₁₋₆ alkoxy-carbonyl group,
         (17) a C₁₋₆ alkylamino group,
         (18) a C₁₋₆ alkyl-carbonyl-amino group,
         (19) a C₆₋₁₄ aryl group, and
         (20) a heteroaryl group.

[5] The use of the aforementioned [4], which uses a heterocyclic compound as represented by the following formula [1] or a pharmaceutically acceptable salt thereof: 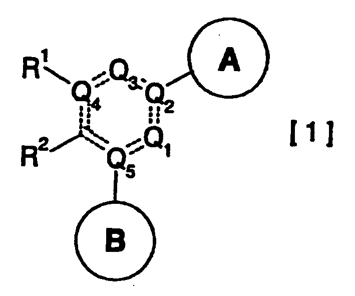 wherein 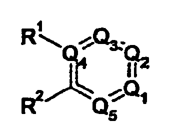 is 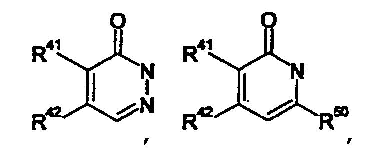 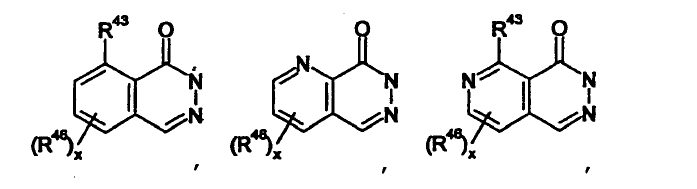 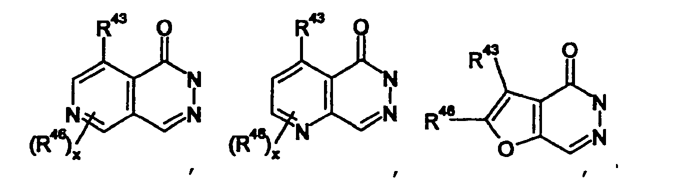 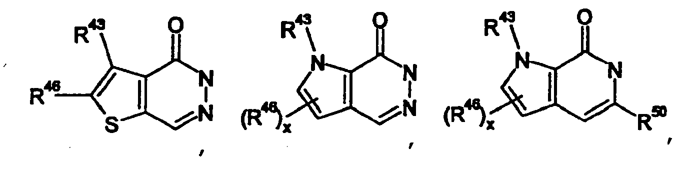 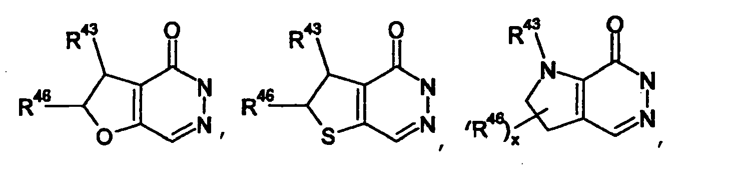 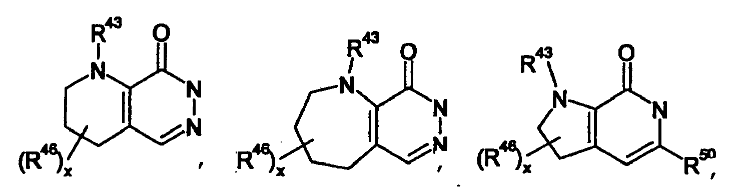 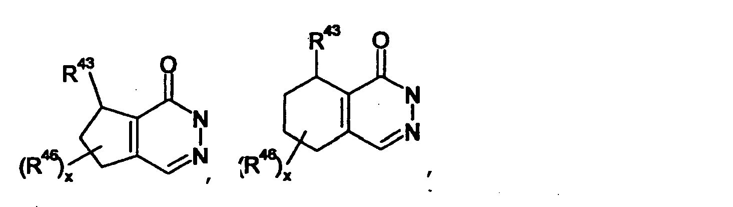 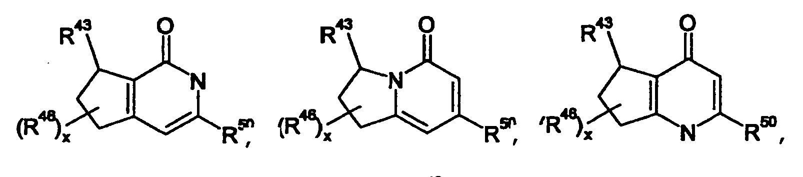 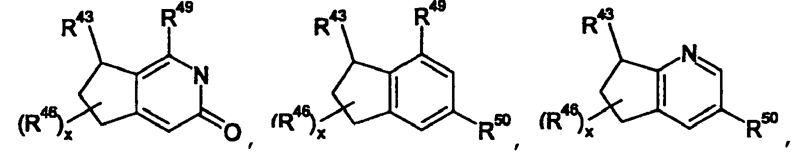 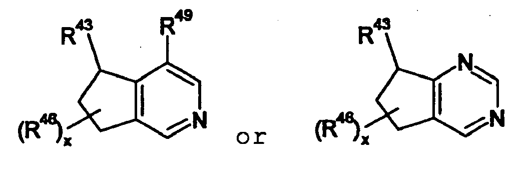 wherein
R⁴¹ is
(1) a hydrogen atom,
(2) a C₁₋₆ alkyl group (said C₁₋₆ alkyl group is optionally substituted by 1 to 3 substituents selected from Group F),
(3) a C₂₋₆ alkenyl group,
(4) -J₁-R¹⁰¹
   wherein
   J₁ is
   (1) -N(R¹⁰²)- wherein R¹⁰² is a hydrogen atom or a C₁₋₆ alkyl group,
   (2) -O-,
   (3) -S-,
   (4) -CO-,
   (5) -CO-O-, or
   (6) -CO-N(R¹⁰²)- wherein R¹⁰² is as defined above,
   R¹⁰¹ is
   (1) a hydrogen atom,
   (2)-L₂-J₂-R¹⁰³
      wherein
      L₂ is
      (1) a bond, or
      (2) a C₁₋₆ alkylene group,
      J₂ is
      (1) -N(R¹⁰⁴)- wherein R¹⁰⁴ is a hydrogen atom or a C₁₋₆ alkyl group,
      (2) -O-,
      (3) -CO-,
      (4) -SO₂-,
      (5) -CO-O-,
      (6) -CO-N(R¹⁰⁴)- wherein R¹⁰⁴ is as defined above, or
      (7) -SO₂-N(R¹⁰⁴)- wherein R¹⁰⁴ is as defined above,
      R¹⁰³ is
      (1) a hydrogen atom,
      (2) a C₁₋₆ alkyl group, or
      (3) a C₁₋₆ alkyl-carbonyl group, or
   (3) a C₁₋₆ alkyl group, or
   R¹⁰¹ and R¹⁰² may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E), or
(5) a C₆₋₁₄ aryl group;
   R⁴² is
   (1) a hydrogen atom, or
   (2) a C₁₋₆ alkyl group;
   R⁴³ is
   (1) a hydrogen atom,
   (2) -L₃-J₃-R¹¹³
      wherein
      L₃ is
      (1) a bond, or
      (2) a C₁₋₆ alkylene group,
      J₃ is
      (1) -N(R¹¹⁴)- wherein R¹¹⁴ is a hydrogen atom or a C₁₋₆ alkyl group,
      (2) -O-,
      (3) -CO-,
      (4) -SO₂-,
      (5) -CO-O-, or
      (6) -CO-N(R¹¹⁴)- wherein R¹¹⁴ is as defined above,
      R¹¹³ is
      (1) a hydrogen atom,
      (2) -L₄-J₄-R¹¹⁵
      wherein
      L₄ is
      (1) a bond, or
      (2) a C₁₋₆ alkylene group,
      J₄ is
      (1) -N(R¹¹⁶)- wherein R¹¹⁶ is a hydrogen atom or a C₁₋₆ alkyl group,
      (2) -O-, or
      (3) -CO-,
      R¹¹⁵ is
      (1) a hydrogen atom,
      (2) a C₁₋₆ alkyl group (said C₁₋₆ alkyl group is optionally substituted by 1 to 3 substituents selected from Group G), or
      (3) a C₁₋₆ alkyl-carbonyl group, or
      R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
   (3) a C₁₋₆ alkyl group, or
      R¹¹³ and R¹¹⁴ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈cycloalkyl group is optionally substituted by 1 to 5 substituents selected from Group E);
x is 1 or 2;
R⁴⁶ are the same or different and each is
(1) a hydrogen atom,
(2) a hydroxyl group,
(3) a C₁₋₆ alkyl group, or
(4) a C₁₋₆ alkoxy group;
R⁴⁹ and R⁵⁰ are the same or different and each is
(1) a hydrogen atom,
(2) a hydroxyl group, or
(3) a C₁₋₆ alkoxy group; 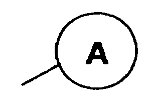 is 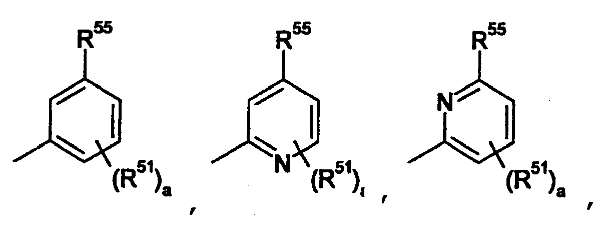 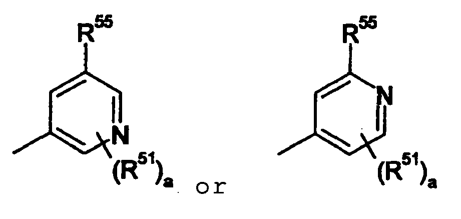
wherein
R⁵⁵ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkynyl group (said C₂₋₆ alkynyl group is optionally substituted by 1 to 3 substituents selected from Group F),
(5) -J₅-R¹²¹
   wherein
   J₅ is
   (1) -N(R¹²²)- wherein R¹²² is a hydrogen atom or a C₁₋₆ alkyl group,
   (2) -O-,
   (3) -CO-,
   (4) -CO-O-,
   (5) -CO-N(R¹²²)- wherein R¹²² is as defined above, or
   (6) -N(R¹²³)-CO- wherein R¹²³ is a hydrogen atom or a C₁₋₆ alkyl group,
   R¹²¹ is
   (1) a hydrogen atom,
   (2) -L₆-J₆-R¹²⁴
      wherein
      L₆ is
      (1) a bond, or
      (2) a C₁₋₆ alkylene group,
      J₆ is
      (1) -N(R¹²⁵)- wherein R¹²⁵ is a hydrogen atom or a C₁₋₆ alkyl group,
      (2) -O-,
      (3) -CO-,
      (4) -CO-O-,
      (5) -CO-N(R¹²⁵)- wherein R¹²⁵ is as defined above,
      (6) -N(R¹²⁶)-CO- wherein R¹²⁶ is a hydrogen atom or a C₁₋₆ alkyl group,
      (7) -N(R¹²⁶)-SO₂- wherein R¹²⁶ is as defined above,
      (8) -N(R¹²⁶)-CO-O- wherein R¹²⁶ is as defined above, or
      (9)-N(R¹²⁶)-CO-N(R¹²⁵)- wherein R¹²⁵ and R¹²⁶ are as defined above,
      R¹²⁴ is
      (1) a hydrogen atom, or
      (2) a C₁₋₆ alkyl group (said C₁₋₆ alkyl group is optionally substituted by 1 to 3 substituents selected from Group G), or
      R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
   (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
   (4) a substituent selected from a halogen atom, a cyano group and a nitro group,
   (5) a C₆₋₁₄ aryl group (said C₆₋₁₄ aryl group is optionally substituted by 1 to 5 substituents selected from Group E),
   (6) a heteroaryl group (said heteroaryl group is optionally substituted by 1 to 5 substituents selected from Group E), or
   (7) a C₃₋₈ cycloalkyl group (said C₃₋₈ cycloalkyl group is optionally substituted by 1 to 5 substituents selected from Group E), or
   R¹²¹ and R¹²² may form, together with the nitrogen atom to which they are bonded, a nitrogen-containing 4-to 7-membered heterocyclic group, wherein said nitrogen-containing 4- to 7-membered heterocyclic group is optionally substituted by 1 to 5 substituents selected from
   (1) Group F,
   (2) a C₁₋₆ alkyl group (said C₁₋₆ alkyl group is optionally substituted by 1 to 3 substituents selected from Group F),
   (3) a C₁₋₆ alkoxy group (said C₁₋₆ alkoxy group is optionally substituted by 1 to 3 substituents selected from Group F), and
   (4) an oxo group,
(6) a monocyclic aryl group,
(7) a monocyclic heteroaryl group (said monocyclic heteroaryl group is optionally substituted by 1 to 5 substituents selected from Group E), or
(8) a heterocycloalkyl group;
a is 1 or 2;
R⁵¹ are the same or different and each is
(1) a hydrogen atom,
(2) a halogen atom,
(3) a cyano group,
(4) a nitro group,
(5) a hydroxyl group,
(6) a formyl group,
(7) a carboxyl group,
(8) a C₁₋₆ alkyl group (said C₁₋₆ alkyl group is optionally substituted by 1 to 3 substituents selected from Group G),
(9) a C₂₋₆alkenyl group,
(10) a C₁₋₆ alkoxy group (said C₁₋₆ alkoxy group is optionally substituted by 1 to 3 substituents selected from Group G),
(11) a C₁₋₆ alkylthio group,
(12) a C₁₋₆ alkylsulfinyl group,
(13) a C₁₋₆ alkylsulfonyl group,
(14) a C₁₋₆ alkyl-carbonyl group (said C₁₋₆ alkyl-carbonyl group is optionally substituted by 1 to 3 substituents selected from Group G),
(15) a C₁₋₆ alkoxy-carbonyl group,
(16) -N(R⁵⁷)(R⁵⁸)
   wherein
   R⁵⁷ and R⁵⁸ are the same or different and each is
   (1) a hydrogen atom,
   (2) a C₁₋₆ alkyl group,
   (3) a C₁₋₆ alkylsulfonyl group,
   (4) a C₁₋₆ alkyl-carbonyl group, or
   (5) a C₁₋₆ alkoxy-carbonyl group, or
   R⁵⁷ and R⁵⁸ may form, together with the nitrogen atom to which they are bonded, a nitrogen-containing 4-to 7-membered heterocyclic group,
(17) -CO-N(R⁵⁷)(R⁵⁸) wherein R⁵⁷ and R⁵⁸ are as defined above, or
(18) -C(R⁵⁹)=N(R⁶⁰)
   wherein
   R⁵⁹ is
   (1) a hydrogen atom,
   R⁶⁰ is
   (1) a hydroxyl group, or
   (2) a C₁₋₆ alkyl-carbonyl-oxy group, or
   two adjacent R⁵¹s may form, together with the ring to which they are bonded, a fused ring (said fused ring is optionally further substituted by one substituent selected from Group E); 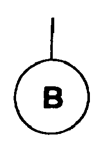 is 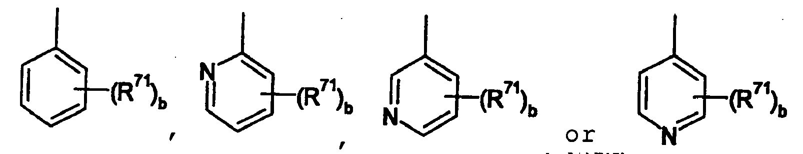
wherein
b is 1 or 2;
R⁷¹ are the same or different and each is
(1) a hydrogen atom,
(2) a halogen atom,
(3) a cyano group,
(4) a hydroxyl group,
(5) a formyl group,
(6) a carboxyl group,
(7) a C₁₋₆ alkyl group (said C₁₋₆ alkyl group is optionally substituted by 1 to 3 substituents selected from Group G),
(8) a C₂₋₆ alkenyl group (said C₂₋₆ alkenyl group is optionally substituted by 1 to 3 substituents selected from Group G),
(9) a C₁₋₆ alkoxy group (said C₁₋₆ alkoxy group is optionally substituted by 1 to 3 substituents selected from Group G),
(10) a C₁₋₆ alkyl-carbonyl group,
(11) a C₁₋₆ alkoxy-carbonyl group,
(12) -N(R⁷²)(R⁷³)
   wherein
   R⁷² and R⁷³ are the same or different and each is
   (1) a hydrogen atom,
   (2) a C₁₋₆ alkyl group (said C₁₋₆ alkyl group is optionally substituted by 1 to 3 substituents selected from Group G),
   (3) a C₁₋₆ alkylsulfonyl group,
   (4) a C₁₋₆ alkyl-carbonyl group,
   (5) a C₁₋₆ alkoxy-carbonyl group,
   (6) a carbamoyl group,
   (7) a hydroxyl group,
   (8) an amino group, or
   (9) a C₁₋₆ alkyl-carbonyl-amino group, or
      R⁷² and R⁷³ may form, together with the nitrogen atom to which they are bonded, a nitrogen-containing 4-to 7-membered heterocyclic group,
(13) -CO-N(R⁷²)(R⁷³) wherein R⁷² and R⁷³ are as defined above,
(14) -C(R⁷⁴)=N(R⁷⁵)
   wherein
   R⁷⁴ is
   (1) a hydrogen atom,
   (2) a C₁₋₆ alkyl group, or
   (3) an amino group,
      R⁷⁵ is

   (1) a hydroxyl group,
   (2) a C₁₋₆ alkoxy group (said C₁₋₆ alkoxy group is optionally substituted by 1 to 3 substituents selected from Group G), or
   (3) -N(R⁷⁶)(R⁷⁷) wherein R⁷⁶ and R⁷⁷ are the same or different and each is a hydrogen atom, or a C₁₋₆ alkyl-carbonyl group,
(15) a monocyclic aryl group,
(16) a monocyclic heteroaryl group (said monocyclic heteroaryl group is optionally substituted by 1 to 5 substituents selected from Group E), or
(17) a heterocycloalkyl group, or
   two adjacent R⁷¹s may form, together with the ring to which they are bonded, a fused ring (said fused ring is optionally further substituted by one substituent selected from Group E):
wherein
Group E consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkyl group (said C₁₋₆ alkyl group is optionally substituted by 1 to 3 substituents selected from Group G),
(12) a C₂-₆ alkenyl group (said C₂₋₆ alkenyl group is optionally substituted by 1 to 3 substituents selected from Group G),
(13) a C₂₋₆ alkynyl group (said C₂₋₆ alkynyl group is optionally substituted by 1 to 3 substituents selected from Group G),
(14) a C₁₋₆ alkoxy group (said C₁₋₆ alkoxy group is optionally substituted by 1 to 3 substituents selected from Group G),
(15) a C₁₋₆ alkylthio group,
(16) a C₁₋₆ alkylsulfinyl group,
(17) a C₁₋₆ alkylsulfonyl group,
(18) a C₁₋₆ alkyl-carbonyl group,
(19) a C₁₋₆ alkoxy-carbonyl group,
(20) a C₁₋₆ alkylamino group,
(21) a C₆₋₁₄ aryl group,
(22) a heteroaryl group, and
(23) an oxo group;
Group F consists of
(1) a halogen atom,
(2) a cyano group,
(3) a nitro group,
(4) a hydroxyl group,
(5) a mercapto group,
(6) a formyl group,
(7) a carboxyl group,
(8) a C₁₋₆ alkoxy group,
(9) a C₁₋₆ alkylthio group,
(10) a C₁₋₆ alkylsulfinyl group,
(11) a C₁₋₆ alkylsulfonyl group,
(12) a C₁₋₆ alkyl-carbonyl group,
(13) a C₁₋₆ alkoxy-carbonyl group,
(14) -N(R⁹¹)(R⁹²) wherein R⁹¹ and R⁹² are the same or different and each is a hydrogen atom, a C₁₋₆ alkyl group, a C₁₋₆ alkyl-carbonyl group, a C₁₋₆ alkylsulfonyl group, a C₁₋₆ alkoxy-carbonyl group, a carbamoyl group, a Cₗ-₆ alkylamino-carbonyl group, or a di(C₁₋₆ alkyl) amino-carbonyl group, or R⁹¹ and R⁹² may form, together with the nitrogen atom to which they are bonded, a nitrogen-containing 4- to 7-membered heterocyclic group,
(15) -CO-N(R⁹¹)(R⁹²) wherein R⁹¹ and R⁹² are as defined above,
(16) -SO₂-N(R⁹¹)(R⁹²) wherein R⁹¹ and R⁹² are as defined above,
(17) -O-CO-N(R⁹¹)(R⁹²) wherein R⁹¹ and R⁹² are as defined above,
(18) a C₆₋₁₄ aryl group,
(19) a C₆₋₁₄ aryl-oxy group,
(20) a C₆₋₁₄ aryl-carbonyl group, and
(21) a heteroaryl group;
Group G consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkoxy group,
(12) a C₁₋₆ alkylthio group,
(13) a C₁₋₆ alkylsulfinyl group,
(14) a C₁₋₆ alkylsulfonyl group,
(15) a C₁₋₆ alkyl-carbonyl group,
(16) a C₁₋₆ alkoxy-carbonyl group,
(17) a C₁₋₆ alkylamino group,
(18) a C₁₋₆ alkyl-carbonyl-amino group,
(19) a C₆₋₁₄ aryl group, and
(20) a heteroaryl group.

[6] A heterocyclic compound represented by the following formula [1], or a pharmaceutically acceptable salt thereof: 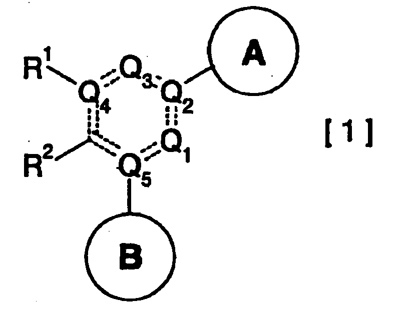 wherein 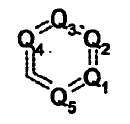 is 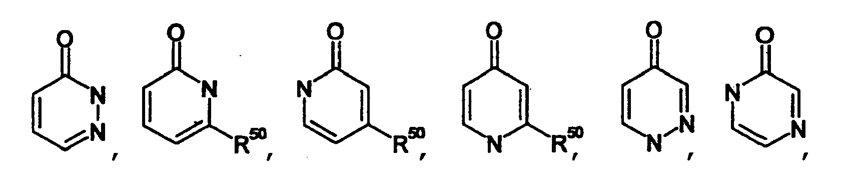 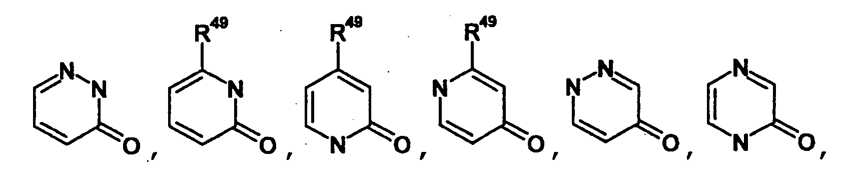 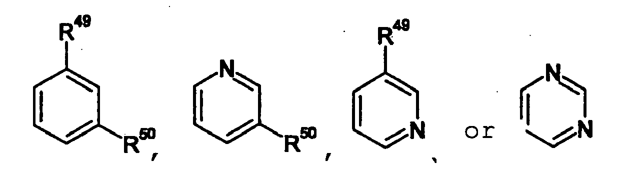 wherein
R⁴⁹ and R⁵⁰ are the same or different and each is
(1) a hydrogen atom,
(2) a hydroxyl group,
(3) a C₁₋₆ alkyl group, or
(4) a C₁₋₆ alkoxy group;
   R¹ is:

(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₁-R¹⁰¹
   wherein
   J₁ is
   (1) -N(R¹⁰²)- wherein R¹⁰² is a hydrogen atom or a C₁₋₆ alkyl group,
   (2) -O-,
   (3) -S-,
   (4) -CO-,
   (5) -CO-O-,
   (6) -O-CO-,
   (7) -CO-N(R¹⁰²)- wherein R¹⁰² is as defined above, or
   (8) -SO₂-N(R¹⁰²)- wherein R¹⁰² is as defined above,
   R¹⁰¹ is
   (1) a hydrogen atom,
   (2) -L₂-J₂-R¹⁰³
      wherein
      L₂ is
      (1) a bond, or
      (2) a C₁₋₆ alkylene group,
      J₂ is
      (1) -N(R¹⁰⁴)- wherein R¹⁰⁴ is a hydrogen atom or a C₁₋₆ alkyl group,
      (2) -O-,
      (3) -S-,
      (4) -CO-,
      (5) -SO₂-,
      (6) -CO-O-,
      (7) -CO-N(R¹⁰⁴)- wherein R¹⁰⁴ is as defined above, or
      (8) -SO₂-N(R¹⁰⁴)- wherein R¹⁰⁴ is as defined above,
      R¹⁰³ is
      (1) a hydrogen atom,
      (2) a C₁₋₆ alkyl group (said C₁₋₆ alkyl group is optionally substituted by 1 to 3 substituents selected from Group F),
      (3) a C₁₋₆ alkyl-carbonyl group (said C₁₋₆ alkyl-carbonyl group is optionally substituted by 1 to 3 substituents selected from Group F), or
      (4) a C₁₋₆ alkylsulfonyl group (said C₁₋₆ alkylsulfonyl group is optionally substituted by 1 to 3 substituents selected from Group F),
   (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
   (4) a substituent selected from a halogen atom, a cyano group and a nitro group,
   (5) a C₆₋₁₄ aryl group (said C₆₋₁₄ aryl group is optionally substituted by 1 to 5 substituents selected from Group E),
   (6) a heteroaryl group (said heteroaryl group is optionally substituted by 1 to 5 substituents selected from Group E), or
   (7) a C₃₋₈ cycloalkyl group (said C₃₋₈ cycloalkyl group is optionally substituted by 1 to 5 substituents selected from Group E), or
      R¹⁰¹ and R¹⁰² may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(7) a C₆₋₁₄ aryl group (said C₆₋₁₄ aryl group is optionally substituted by 1 to 5 substituents selected from Group E), or
(8) heteroaryl group (said heteroaryl group is optionally substituted by 1 to 5 substituents selected from Group E);
R² is:
(1) a hydrogen atom, or
(2) a C₁₋₆ alkyl group; or
   R¹ and R² may form, together with Q₄ and the carbon atom to which they are bonded respectively: 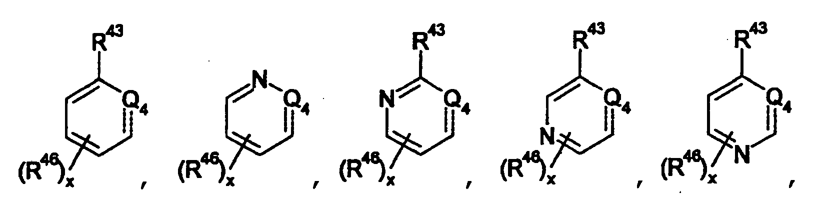 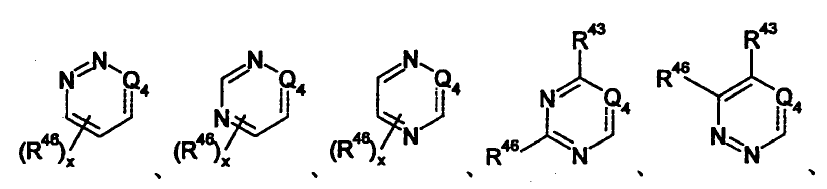 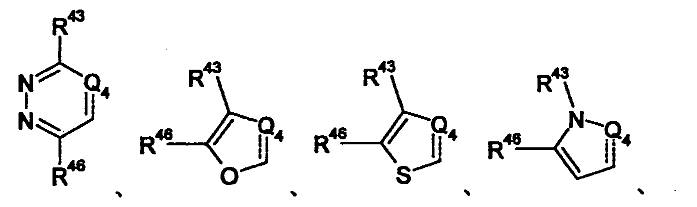 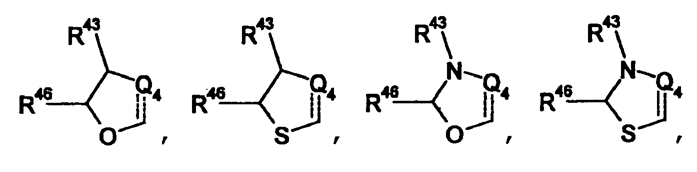 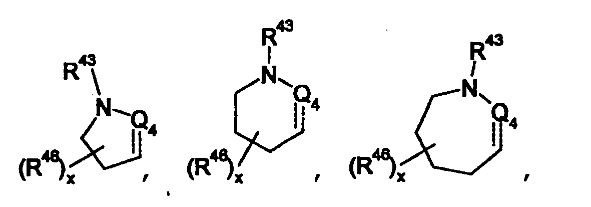 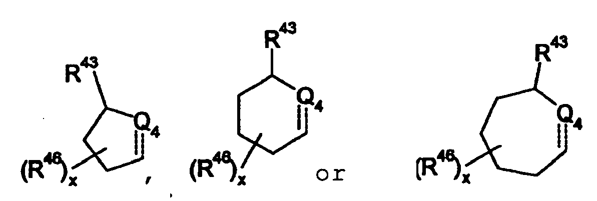
wherein
Q₄ is as defined above,
R⁴³ is
(1) a hydrogen atom,
(2) -L₃-J₃-R¹¹³
   wherein
   L₃ is
   (1) a bond, or
   (2) a C₁₋₆ alkylene group,
   J₃ is
   (1) -N(R¹¹⁴)- wherein R¹¹⁴ is a hydrogen atom or a C₁₋₆ alkyl group,
   (2) -O-,
   (3) -S-,
   (4) -CO-,
   (5) -SO₂-,
   (6) -CO-O-,
   (7) -CO-N(R¹¹⁴)- wherein R¹¹⁴ is as defined above, or
   (8) -SO₂-N(R¹¹⁴)- wherein R¹¹⁴ is as defined above,
   R¹¹³ is
   (1) a hydrogen atom,
   (2) -L₄-J₄-R¹¹⁵
      wherein
      L₄ is
      (1) a bond, or
      (2) a C₁₋₆ alkylene group,
      J₄ is
      (1) -N(R¹¹⁶)- wherein R¹¹⁶ is a hydrogen atom or a C₁₋₆ alkyl group,
      (2) -O-,
      (3) -S-,
      (4) -CO-,
      (5) -SO₂-,
      (6) -CO-O-,
      (7) -CO-N(R¹¹⁶)- wherein R¹¹⁶ is as defined above, or
      (8) -SO₂-N(R¹¹⁶)- wherein R¹¹⁶ is as defined above,
      R¹¹⁵ is
      (1) a hydrogen atom,
      (2) a C₁₋₆ alkyl group (said C₁₋₆ alkyl group is optionally substituted by 1 to 3 substituents selected from Group G),
      (3) a C₁₋₆ alkyl-carbonyl group (said C₁₋₆ alkyl-carbonyl group is optionally substituted by 1 to 3 substituents selected from Group G), or
      (4) a C₁₋₆ alkylsulfonyl group (said C₁₋₆ alkylsulfonyl group is optionally substituted by 1 to 3 substituents selected from Group G), or
      R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
   (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
   (4) a substituent selected from a halogen atom, a cyano group and a nitro group,
   (5) a C₆₋₁₄ aryl group (said C₆₋₁₄ aryl group is optionally substituted by 1 to 5 substituents selected from Group E),
   (6) a heteroaryl group (said heteroaryl group is optionally substituted by 1 to 5 substituents selected from Group E), or
   (7) a C₃₋₈ cycloalkyl group (said C₃₋₈ cycloalkyl group is optionally substituted by 1 to 5 substituents selected from Group E), or
   R¹¹³ and R¹¹⁴ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E);
x is 1 or 2;
R⁴⁶ are the same or different and each is
(1) a hydrogen atom,
(2) a hydroxyl group,
(3) a C₁₋₆ alkyl group, or
(4) a C₁₋₆ alkoxy group; 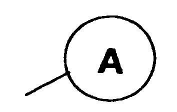 is 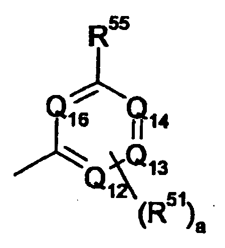
wherein
Q₁₂, Q₁₃, Q₁₄, and Q₁₆ are the same or different and each is
(1) -N=, or
(2) -CH=;
R⁵⁵ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₅-R¹²¹
   wherein
   J₅ is
   (1) -N(R¹²²)- wherein R¹²² is a hydrogen atom or a C₁₋₆ alkyl group,
   (2) -O-,
   (3) -S-,
   (4) -CO-,
   (5) -CO-O-,
   (6) -O-CO-,
   (7) -CO-N(R¹²²)- wherein R¹²² is as defined above,
   (8) -SO₂-N(R¹²²)- wherein R¹²² is as defined above,
   (9) -N(R¹²³)-CO- wherein R¹²³ is a hydrogen atom or a C₁₋₆ alkyl group, or
   (10) -N(R¹²³)-SO₂- wherein R¹²³ is as defined above,
   R¹²¹ is
   (1) a hydrogen atom,
   (2) -L₆-J₆-R¹²⁴
      wherein
      L₆ is
      (1) a bond, or
      (2) a C₁₋₆ alkylene group,
      J₆ is
      (1) -N(R¹²⁵)- wherein R¹²⁵ is a hydrogen atom or a C₁₋₆ alkyl group,
      (2) -O-,
      (3) -S-,
      (4) -CO-,
      (5) -CO-O-,
      (6) -O-CO-,
      (7) -CO-N(R¹²⁵)- wherein R¹²⁵ is as defined above,
      (8) -SO₂-N(R¹²⁵)- wherein R¹²⁵ is as defined above,
      (9) -N(R¹²⁶)-CO- wherein R¹²⁶ is a hydrogen atom or a C₁₋₆ alkyl group,
      (10) -N(R¹²⁶)-SO₂- wherein R¹²⁶ is as defined above,
      (11) -N(R¹²⁶)-CO-O- wherein R¹²⁶ is as defined above, or
      (12) -N(R¹²⁶)-CO-N(R¹²⁵)- wherein R¹²⁵ and R¹²⁶ is as defined above,
      R¹²⁴ is
      (1) a hydrogen atom, or
      (2) a C₁₋₆ alkyl group (said C₁₋₆ alkyl group is optionally substituted by 1 to 3 substituents selected from Group G), or
      R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
   (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
   (4) a substituent selected from a halogen atom, a cyano group and a nitro group,
   (5) a C₆₋₁₄ aryl group (said C₆₋₁₄ aryl group is optionally substituted by 1 to 5 substituents selected from Group E),
   (6) a heteroaryl group (said heteroaryl group is optionally substituted by 1 to 5 substituents selected from Group E), or
   (7) a C₃₋₈ cycloalkyl group (said C₃₋₈ cycloalkyl group is optionally substituted by 1 to 5 substituents selected from Group E), or
   R¹²¹ and R¹²² may form, together with the nitrogen atom to which they are bonded, a nitrogen-containing 4-to 7-membered heterocyclic group, wherein said nitrogen-containing 4- to 7-membered heterocyclic group is optionally substituted by 1 to 5 substituents selected from
   (1) Group F,
   (2) a C₁₋₆ alkyl group (said C₁₋₆ alkyl group is optionally substituted by 1 to 3 substituents selected from Group F),
   (3) a C₁₋₆ alkoxy group (said C₁₋₆ alkoxy group is optionally substituted by 1 to 3 substituents selected from Group F), and
   (4) an oxo group,
(7) a monocyclic aryl group (said monocyclic aryl group is optionally substituted by 1 to 5 substituents selected from Group E),
(8) a monocyclic heteroaryl group (said monocyclic heteroaryl group is optionally substituted by 1 to 5 substituents selected from Group E),
(9) a C₃₋₈ cycloalkyl group (said C₃₋₈ cycloalkyl group is optionally substituted by 1 to 5 substituents selected from Group E), or
(10) a heterocycloalkyl group (said heterocycloalkyl group is optionally substituted by 1 to 5 substituents selected from Group E);
a is 1 or 2;

R⁵¹ are the same or different and each is
(1) a hydrogen atom,
(2) a halogen atom,
(3) a cyano group,
(4) a nitro group,
(5) a hydroxyl group,
(6) a mercapto group,
(7) a formyl group,
(8) a carboxyl group,
(9) a C₁₋₆ alkyl group (said C₁₋₆ alkyl group is optionally substituted by 1 to 3 substituents selected from Group G),
(10) a C₂₋₆ alkenyl group (said C₂₋₆ alkenyl group is optionally substituted by 1 to 3 substituents selected from Group G),
(11) a C₂₋₆ alkynyl group (said C₂₋₆ alkynyl group is optionally substituted by 1 to 3 substituents selected from Group G),
(12) a C₁₋₆ alkoxy group (said C₁₋₆ alkoxy group is optionally substituted by 1 to 3 substituents selected from Group G),
(13) a C₁₋₆ alkylthio group (said C₁₋₆ alkylthio group is optionally substituted by 1 to 3 substituents selected from Group G),
(14) a C₁₋₆ alkylsulfinyl group (said C₁₋₆ alkylsulfinyl group is optionally substituted by 1 to 3 substituents selected from Group G),
(15) a C₁₋₆ alkylsulfonyl group (said C₁₋₆ alkylsulfonyl group is optionally substituted by 1 to 3 substituents selected from Group G),
(16) a C₁₋₆ alkyl-carbonyl group (said C₁₋₆ alkyl-carbonyl group is optionally substituted by 1 to 3 substituents selected from Group G),
(17) a C₁₋₆ alkoxy-carbonyl group (said C₁₋₆ alkoxy-carbonyl group is optionally substituted by 1 to 3 substituents selected from Group G),
(18) -N(R⁵⁷)(R⁵⁸)
   wherein
   R⁵⁷ and R⁵⁸ are the same or different and each is
   (1) a hydrogen atom,
   (2) a C₁₋₆ alkyl group (said C₁₋₆ alkyl group is optionally substituted by 1 to 3 substituents selected from Group G),
   (3) a C₁₋₆ alkylsulfonyl group (said C₁₋₆ alkylsulfonyl group is optionally substituted by 1 to 3 substituents selected from Group G),
   (4) a C₁₋₆ alkyl-carbonyl group (said C₁₋₆ alkyl-carbonyl group is optionally substituted by 1 to 3 substituents selected from Group G),
   (5) a C₁₋₆ alkoxy-carbonyl group (said C₁₋₆ alkoxy-carbonyl group is optionally substituted by 1 to 3 substituents selected from Group G),
   (6) a carbamoyl group,
   (7) a C₁₋₆ alkylamino-carbonyl group (said C₁₋₆ alkylamino-carbonyl group is optionally substituted by 1 to 3 substituents selected from Group G),
   (8) a di(C₁₋₆ alkyl)amino-carbonyl group (said di(C₁₋₆ alkyl)amino-carbonyl group is optionally substituted by 1 to 3 substituents selected from Group G),
   (9) a hydroxyl group,
   (10) a C₁₋₆ alkoxy group (said C₁₋₆ alkoxy group is optionally substituted by 1 to 3 substituents selected from Group G),
   (11) an amino group,
   (12) a C₁₋₆ alkylamino group (said C₁₋₆ alkylamino group is optionally substituted by 1 to 3 substituents selected from Group G),
   (13) a di(C₁₋₆ alkyl)amino group (said di(C₁₋₆ alkyl)amino group is optionally substituted by 1 to 3 substituents selected from Group G), or
   (14) a C₁₋₆ alkyl-carbonyl-amino group (said C₁₋₆ alkyl-carbonyl-amino group is optionally substituted by 1 to 3 substituents selected from Group G), or
   R⁵⁷ and R⁵⁸ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(19) -CO-N(R⁵⁷)(R⁵⁸) wherein R⁵⁷ and R⁵⁸ is as defined above,
(20) -C(R⁵⁹)=N(R⁶⁰)
   wherein
   R⁵⁹ is
   (1) a hydrogen atom,
   (2) a C₁₋₆ alkyl group, or
   (3) an amino group,
      R⁶⁰ is

   (1) a hydroxyl group,
   (2) a C₁₋₆ alkoxy group (said C₁₋₆ alkoxy group is optionally substituted by 1 to 3 substituents selected from Group G),
   (3) a C₁₋₆ alkyl-carbonyl-oxy group (said C₁₋₆ alkyl-carbonyl-oxy group is optionally substituted by 1 to 3 substituents selected from Group G), or
   (4) -N(R⁶¹)(R⁶²) wherein R⁶¹ and R⁶² are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
(21) a monocyclic aryl group (said monocyclic aryl group is optionally substituted by 1 to 5 substituents selected from Group E),
(22) a monocyclic heteroaryl group (said monocyclic heteroaryl group is optionally substituted by 1 to 5 substituents selected from Group E), or
(23) a heterocycloalkyl group (said heterocycloalkyl group is optionally substituted by 1 to 5 substituents selected from Group E), or
   two adjacent R⁵¹s may form, together with the ring to which they are bonded, a fused ring (said fused ring is optionally further substituted by one substituent selected from Group E); 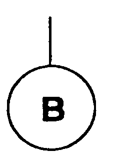 is 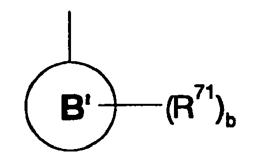
wherein
ring B' is
(1) a monocyclic aryl group, or
(2) a monocyclic heteroaryl group;
   b is an integer of 1-3;
   R⁷¹ are the same or different and each is

(1) a hydrogen atom,
(2) a halogen atom,
(3) a cyano group,
(4) a nitro group,
(5) a hydroxyl group,
(6) a mercapto group,
(7) a formyl group,
(8) a carboxyl group,
(9) a C₁₋₆ alkyl group (said C₁₋₆ alkyl group is optionally substituted by 1 to 3 substituents selected from Group G),
(10) a C₂₋₆ alkenyl group (said C₂₋₆ alkenyl group is optionally substituted by 1 to 3 substituents selected from Group G),
(11) a C₂₋₆ alkynyl group (said C₂₋₆ alkynyl group is optionally substituted by 1 to 3 substituents selected from Group G),
(12) a C₁₋₆ alkoxy group (said C₁₋₆ alkoxy group is optionally substituted by 1 to 3 substituents selected from Group G),
(13) a C₁₋₆ alkylthio group (said C₁₋₆ alkylthio group is optionally substituted by 1 to 3 substituents selected from Group G),
(14) a C₁₋₆ alkylsulfinyl group (said C₁₋₆ alkylsulfinyl group is optionally substituted by 1 to 3 substituents selected from Group G),
(15) a C₁₋₆ alkylsulfonyl group (said C₁₋₆ alkylsulfonyl group is optionally substituted by 1 to 3 substituents selected from Group G),
(16) a C₁₋₆ alkyl-carbonyl group (said C₁₋₆ alkyl-carbonyl group is optionally substituted by 1 to 3 substituents selected from Group G),
(17) a C₁₋₆ alkoxy-carbonyl group (said C₁₋₆ alkoxy-carbonyl group is optionally substituted by 1 to 3 substituents selected from Group G),
(18) -N(R⁷²)(R⁷³)
   wherein
   R⁷² and R⁷³ are the same or different and each is
   (1) a hydrogen atom,
   (2) a C₁₋₆ alkyl group (said C₁₋₆ alkyl group is optionally substituted by 1 to 3 substituents selected from Group G),
   (3) a C₁₋₆ alkylsulfonyl group (said C₁₋₆ alkylsulfonyl group is optionally substituted by 1 to 3 substituents selected from Group G),
   (4) a C₁₋₆ alkyl-carbonyl group (said C₁₋₆ alkyl-carbonyl group is optionally substituted by 1 to 3 substituents selected from Group G),
   (5) a C₁₋₆ alkoxy-carbonyl group (said C₁₋₆ alkoxy-carbonyl group is optionally substituted by 1 to 3 substituents selected from Group G),
   (6) a carbamoyl group,
   (7) a C₁₋₆ alkylamino-carbonyl group (said C₁₋₆ alkylamino-carbonyl group is optionally substituted by 1 to 3 substituents selected from Group G),
   (8) a di(C₁₋₆ alkyl)amino-carbonyl group (said di(C₁₋₆ alkyl)amino-carbonyl group is optionally substituted by 1 to 3 substituents selected from Group G),
   (9) a hydroxyl group,
   (10) a C₁₋₆ alkoxy group (said C₁₋₆ alkoxy group is optionally substituted by 1 to 3 substituents selected from Group G),
   (11) an amino group,
   (12) a C₁₋₆ alkylamino group (said C₁₋₆ alkylamino group is optionally substituted by 1 to 3 substituents selected from Group G),
   (13) a di(C₁₋₆ alkyl) amino group (said di(C₁₋₆ alkyl)amino group is optionally substituted by 1 to 3 substituents selected from Group G), or
   (14) a C₁₋₆ alkyl-carbonyl-amino group (said C₁₋₆ alkyl-carbonyl-amino group is optionally substituted by 1 to 3 substituents selected from Group G), or
   R⁷² and R⁷³ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(19) -CO-N(R⁷²)(R⁷³) wherein R⁷² and R⁷³ is as defined above,
(20) -C(R⁷⁴)=N(R⁷⁵)
   wherein
   R⁷⁴ is
   (1) a hydrogen atom,
   (2) a C₁₋₆ alkyl group, or
   (3) an amino group,
   R⁷⁵ is
   (1) a hydroxyl group,
   (2) a C₁₋₆ alkoxy group (said C₁₋₆ alkoxy group is optionally substituted by 1 to 3 substituents selected from Group G),
   (3) a C₁₋₆ alkyl-carbonyl-oxy group (said C₁₋₆ alkyl-carbonyl-oxy group is optionally substituted by 1 to 3 substituents selected from Group G), or
   (4) -N(R⁷⁶)(R⁷⁷) wherein R⁷⁶ and R⁷⁷ are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
(21) a monocyclic aryl group (said monocyclic aryl group is optionally substituted by 1 to 5 substituents selected from Group E),
(22) a monocyclic heteroaryl group (said monocyclic heteroaryl group is optionally substituted by 1 to 5 substituents selected from Group E), or
(23) a heterocycloalkyl group (said heterocycloalkyl group is optionally substituted by 1 to 5 substituents selected from Group E), or
   two adjacent R⁷¹s may form, together with the ring to which they are bonded, a fused ring (said fused ring is optionally further substituted by one substituent selected from Group E):
wherein
Group E consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkyl group (said C₁₋₆ alkyl group is optionally substituted by 1 to 3 substituents selected from Group G),
(12) a C₂₋₆ alkenyl group (said C₂₋₆ alkenyl group is optionally substituted by 1 to 3 substituents selected from Group G),
(13) a C₂₋₆ alkynyl group (said C₂₋₆ alkynyl group is optionally substituted by 1 to 3 substituents selected from Group G),
(14) a C₁₋₆ alkoxy group (said C₁₋₆ alkoxy group is optionally substituted by 1 to 3 substituents selected from Group G),
(15) a C₁₋₆ alkylthio group,
(16) a C₁₋₆ alkylsulfinyl group,
(17) a C₁₋₆ alkylsulfonyl group,
(18) a C₁₋₆ alkyl-carbonyl group,
(19) a C₁₋₆ alkoxy-carbonyl group,
(20) a C₁₋₆ alkylamino group,
(21) a C₆₋₁₄ aryl group,
(22) a heteroaryl group, and
(23) an oxo group;
Group F consists of
(1) a halogen atom,
(2) a cyano group,
(3) a nitro group,
(4) a hydroxyl group,
(5) a mercapto group,
(6) a formyl group,
(7) a carboxyl group,
(8) a C₁₋₆ alkoxy group,
(9) a C₁₋₆ alkylthio group,
(10) a C₁₋₆ alkylsulfinyl group,
(11) a C₁₋₆ alkylsulfonyl group,
(12) a C₁₋₆ alkyl-carbonyl group,
(13) a C₁₋₆ alkoxy-carbonyl group,
(14) -N(R⁹¹)(R⁹²) wherein R⁹¹ and R⁹² are the same or different and each is a hydrogen atom, a C₁₋₆ alkyl group, a C₁₋₆ alkyl-carbonyl group, a C₁₋₆ alkylsulfonyl group, a C₁₋₆ alkoxycarbonyl group, a carbamoyl group, a C₁₋₆ alkylamino-carbonyl group, or a di(C₁₋₆ alkyl)amino-carbonyl group, or R⁹¹ and R⁹² may form, together with the nitrogen atom to which they are bonded, a nitrogen-containing 4- to 7-membered heterocyclic group,
(15) -CO-N(R⁹¹)(R⁹²) wherein R⁹¹ and R⁹² is as defined above,
(16) -SO₂-N(R⁹¹)(R⁹²) wherein R⁹¹ and R⁹² is as defined above,
(17) -O-CO-N(R⁹¹)(R⁹²) wherein R⁹¹ and R⁹² is as defined above,
(18) a C₆₋₁₄ aryl group,
(19) a C₆₋₁₄ aryl-oxy group,
(20) a C₆₋₁₄ aryl-carbonyl group, and
(21) a heteroaryl group;
Group G consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkoxy group,
(12) a C₁₋₆ alkylthio group,
(13) a C₁₋₆ alkylsulfinyl group,
(14) a C₁₋₆ alkylsulfonyl group,
(15) a C₁₋₆ alkyl-carbonyl group,
(16) a C₁₋₆ alkoxy-carbonyl group,
(17) a C₁₋₆ alkylamino group,
(18) a C₁₋₆ alkyl-carbonyl-amino group,
(19) a C₆₋₁₄ aryl group, and
(20) a heteroaryl group,
   provided that the compound of the formula [1] satisfies the the following provisions 1 to 12:
   provision 1:
when 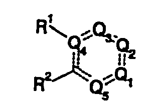 wherein R¹, R², Q₁, Q₂, Q₃, Q₄ and Q₅ are as defined above,
is 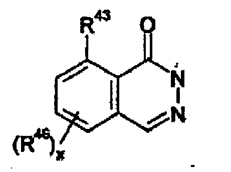 wherein R⁴³, R⁴⁶ and x are as defined above, 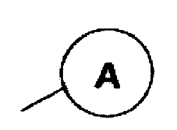 is 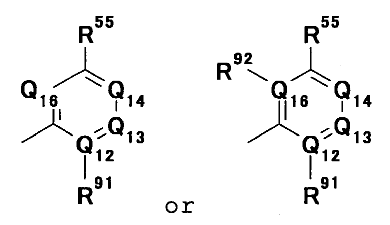 wherein
Q₁₂, Q₁₃, Q₁₄, Q₁₆ and R⁵⁵ are as defined above,
R⁹¹ is as defined for R⁵¹ (provided that a hydrogen atom is excluded), and
R⁹² is as defined for R⁵¹, and
the compounds satisfying the following provisions 1-1 to 1-3 are excluded:
provision 1-1:
ring A is 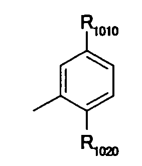 wherein R₁₀₁₀ is a chlorine atom, a piperidinyl group, a pyrrolidinyl group, an azepanylgroup or a morpholinyl group, and R₁₀₂₀ is a nitro group or an amino group, and ring B is 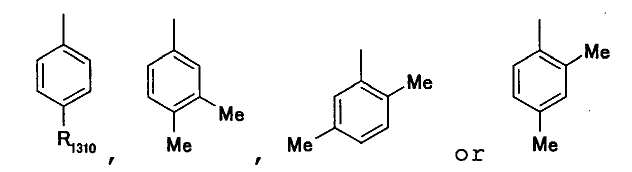 wherein R₁₃₁₀ is a hydrogen atom, a fluorine atom, a chlorine atom, a methyl group or an ethyl group;
provision 1-2:
ring A is 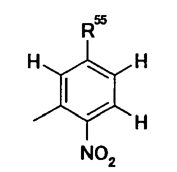
wherein
R⁵⁵ is an unsubstituted or substituted piperazinyl group optionally substituted by 1 to 5 substituents selected from Group E, Group F, a C₁₋₆ alkyl group (said C₁₋₆ alkyl group is optionally substituted by 1 to 3 substituents selected from Group F) and a C₁₋₆ alkoxy group (said C₁₋₆ alkoxy group is optionally substituted by 1 to 3 substituents selected from Group F); and
provision 1-3:
ring A is 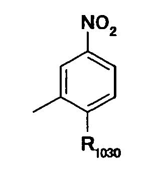 wherein R₁₀₃₀ is a chlorine atom, a pyrrolidinyl group, a morpholinyl group or a N-methylpiperazinyl group, and ring B is 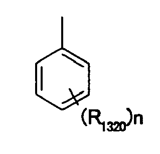 wherein n is 1 or 2, R₁₃₂₀ is a hydrogen atom, a chlorine atom or a methyl group;
provision 2:
when 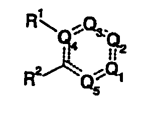 wherein R¹, R², Q₁, Q₂, Q₃, Q₄ and Q₅ are as defined above, is 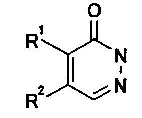 wherein R¹ and R² are as defined above, provided that the case where R¹ and R² form, together with Q₄ and the carbon atom to which they are respectively bonded, 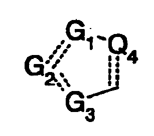 wherein G₁, G₂, G₃ and Q₄ are as defined above, is excluded, 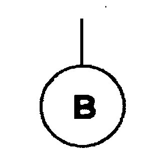 is 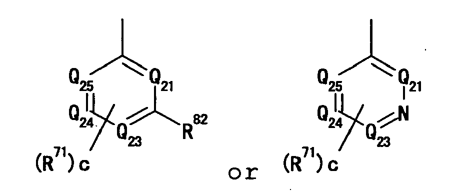 wherein Q₂₁, Q₂₃, Q₂₄ and Q₂₅ are the same or different and each is a carbon atom or a nitrogen atom, R⁷¹ is as defined above, R⁸² is as defined for R⁷¹ (provided that a hydrogen atom is excluded), c is 0, 1 or 2, or two adjacent R⁷¹ s may form, together with the ring to which they are bonded, a fused ring (said fused ring is optionally further substituted by one substituent selected from Group E), and the following compounds are excluded:
6-(2,5-dimethylphenyl)-2-phenyl-3(2H)-pyridazinone,
6-(3,4-dimethylphenyl)-2-phenyl-3(2H)-pyridazinone,
6-(4-chloro-3-methylphenyl)-2-phenyl-3(2H)-pyridazinone,
2-(2-aminophenyl)-6-(3-chlorophenyl)-3(2H)-pyridazinone,
2-(2-aminophenyl)-6-[3-(trifluoromethyl)phenyl]-3(2H)-pyridazinone,
6-(3-chlorophenyl)-2-(2-nitrophenyl)-3(2H)-pyridazinone,
2-(2-nitrophenyl)-6-[3-(trifluoromethyl)phenyl]-3(2H)-pyridazinone,
6-(3,4-dichlorophenyl)-2-phenyl-4-(2,4,6-trimethylphenyl)-3(2H)-pyridazinone,
2-(2,4-dinitrophenyl)-6-(3-nitrophenyl)-3(2H)-pyridazinone,
2-(4-chlorophenyl)-6-(3-nitrophenyl)-3(2H)-pyridazinone,
2-(3-chlorophenyl)-6-(3-nitrophenyl)-3(2H)-pyridazinone,
2-(4-methylphenyl)-6-(3-nitrophenyl)-3(2H)-pyridazinone,
2-(3-methylphenyl)-6-(3-nitrophenyl)-3(2H)-pyridazinone,
2-(2-methylphenyl)-6-(3-nitrophenyl)-3(2H)-pyridazinone,
6-(3-nitrophenyl)-2-(4-nitrophenyl)-3(2H)-pyridazinone,
2,6-bis(3-nitrophenyl)-3(2H)-pyridazinone,
2-(2-nitrophenyl)-6-(3-nitrophenyl)-3(2H)-pyridazinone,
6-(3-nitrophenyl)-2-phenyl-3(2H)-pyridazinone,
6-(2-naphthyl)-2-phenyl-3(2H)-pyridazinone,
6-(1-naphthalenyl)-2-phenyl-3(2H)-pyridazinone,
2-(2-cyanophenyl)-4-(2-methoxyphenyl)-6-(3-pyridazinyl)-2H-pyridazin-3-one,
2-(2-cyanophenyl)-4-phenyl-6-(2-pyrazyl)-2H-pyridazin-3-one,
2-(2-aminophenyl)-6-(3-pyridazinyl)-3(2H)-pyridazinone,
2-(2-nitrophenyl)-6-(3-pyridazinyl)-3(2H)-pyridazinone,
6,6''-dichloro-[3,1'(6'H):3',3''-terpyridazine]-6'-one,
3-[1,6-dihydro-1-(4-methylphenyl)-6-oxo-3-pyridazinyl]-2(1H)-quinoxalinone,
3-[1,6-dihydro-1-(2-methylphenyl)-6-oxo-3-pyridazinyl]-2(1H)-quinoxalinone,
3-(1,6-dihydro-6-oxo-1-phenyl-3-pyridazinyl)-1-methyl-2(1H)-quinoxalinone,
3-[1-(4-chlorophenyl)-1,6-dihydro-6-oxo-3-pyridazinyl]-2(1H)-quinoxalinone, and
3-(1,6-dihydro-6-oxo-1-phenyl-3-pyridazinyl)-2(1H)-quinoxalinone;
provision 3:
when 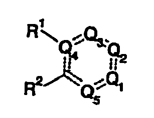 wherein R¹, R², Q₁, Q₂, Q₃, Q₄ and Q₅ are as defined above,
is 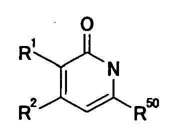 wherein R¹ and R² are as defined above, provided that the case where R¹ and R² form, together with Q₄ and the carbon atom to which they are respectively bonded, 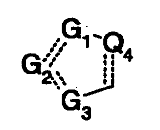 wherein G₁, G₂, G₃ and Q₄ are as defined above, is excluded, and R⁵⁰ is as defined above, 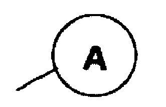 is 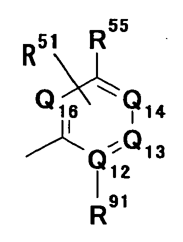 wherein Q₁₂, Q₁₃, Q₁₄, Q₁₆, R⁵¹ and R⁵⁵ are as defined above, R⁹¹ is as defined for R⁵¹ (provided that a hydrogen atom is excluded), 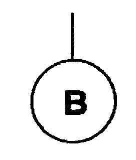 is 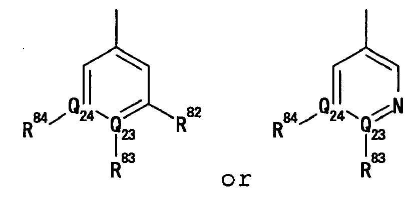 wherein
Q₂₃ and Q₂₄ are the same or different and each is a carbon atom or a nitrogen atom,
R⁸² is as defined for R⁷¹ (provided that a hydrogen atom is excluded),
R⁸³ and R⁸⁴ are the same or different and each is as defined for R⁷¹, or R⁸³ and R⁸⁴ may form, together with the ring to which they are bonded, a fused ring (said fused ring is optionally further substituted by one substituent selected from Group E);
provision 4:
when 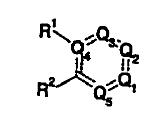 wherein R¹, R², Q₁, Q₂, Q₃, Q₄ and Q₅ are as defined above,
is 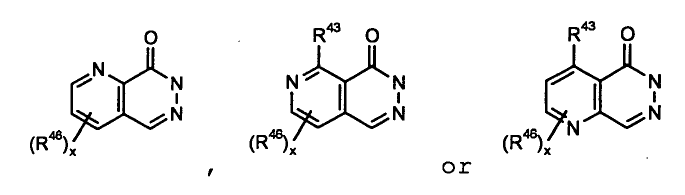 wherein R⁴³ and R⁴⁶ are the same or different and each is a hydrogen atom, a halogen atom, a hydroxyl group or a C₁₋₆ alkyl group, and x is as defined above,
the compounds satisfying the following provisions 4-1 and 4-2 are excluded:
provision 4-1: 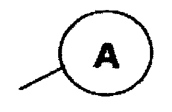 is 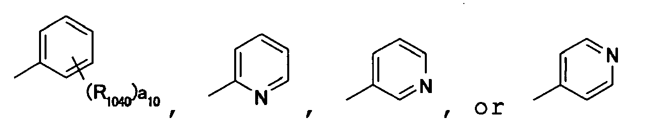 wherein when a₁₀ is 1, R₁₀₄₀ is an amino group, a hydrogen atom, a trifluoromethyloxy group, a bromine atom, a sulfamoyl group, a methanesulfonyl group, a hydroxyl group, a C₁₋₆ alkyl group, a C₁₋₆ alkoxy group, a chlorine atom, a fluorine atom, a trifluoromethyl group, a carbamoyl group, a nitro group or a cyano group, and a₁₀ is an integer of 2 or 3, R₁₀₄₀ are the same or different and each is a hydroxyl group, a C₁₋₆ alkyl group, a C₁₋₆ alkoxy group, a chlorine atom, a fluorine atom, a trifluoromethyl group, a carbamoyl group, a nitro group or a cyano group, and
ring B is a benzotriazole group, or 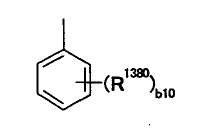 wherein b10 is an integer of 0 or 1 to 5, R¹³⁸⁰ are the same or different and each is a hydrogen atom, a halogen atom, a C₁₋₆ alkyl group, -COOH, a C₁₋₆ alkoxycarbonyl group, a carbamoyl group, a C₁₋₆ alkylcarbonyl group, a halocarbonyl group, a methylthio group, a trifluoromethyl group, a cyano group, a nitro group, a 2,2-difluorodioxolanyl group, a methanesulfonyl group or a trifluoromethyloxy group;
provision 4-2: 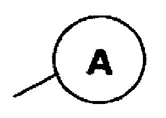 is 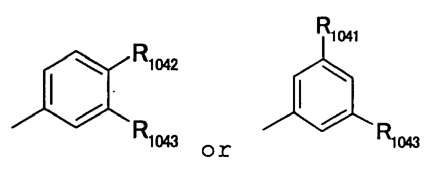 wherein R₁₀₄₁ is a fluorine atom or a trifluoromethyl group, R₁₀₄₂ is a fluorine atom or chlorine atom, R₁₀₄₃ is a fluorine atom, a chlorine atom, a cyano group or a trifluoromethyl group, or R₁₀₄₂ and R₁₀₄₃ form a ring represented by 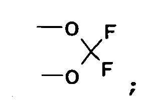 provision 5:
when 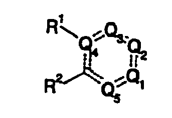 is 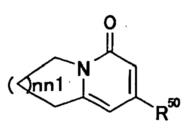 wherein R⁵⁰ is a hydrogen atom or a hydroxyl group, and nn1 is an integer of 1 to 3,
the compounds satisfying the following provisions 5-1 and 5-2 are excluded:
provision 5-1:
ring A is a phenyl group or a 3,4-dimethoxyphenyl group, and ring B is a phenyl group or a 4-chlorophenyl group, and nn1 is 1;
provision 5-2:
ring A is a phenyl group or a pyridyl group, which is optionally substituted by a halogen atom, a trifluoromethyl group, a nitro group, a C₁₋₆ alkyl group or a C₁₋₆ alkoxy group, and nn1 is 2 or 3;
provision 6:
when 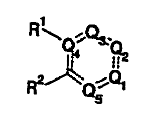 wherein R¹, R², Q₁, Q₂, Q₃, Q₄ and Q₅ are as defined above,
is 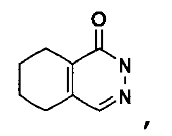 compounds in which ring A is a phenyl group and ring B is a 3,4-dimethoxyphenyl group are excluded;
provision 7:
when 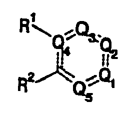 wherein R¹, R², Q₁, Q₂, Q₃, Q₄ and Q₅ are as defined above,
is 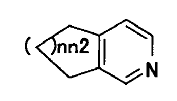 wherein nn2 is an integer of 1 to 3,
compounds in which ring A is a phenyl group or 4-chlorophenyl group, and nn2 is an integer of 1 to 3 are excluded;
provision 8:
when 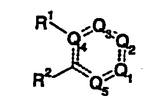 wherein R¹, R², Q₁, Q₂, Q₃, Q₄ and Q₅ are as defined above,
is 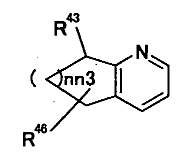 wherein R⁴³ is a hydrogen atom, a hydroxyl group, an acetyloxy group, a methyl group, a benzyl group, a 4-methylbenzyl group, -CH₂-NMe₂ or -CH₂-S-nBu, R⁴⁶ is a hydrogen atom or a methyl group, and nn3 is an integer of 1 to 3,
the following compounds are excluded:
compounds in which ring A is 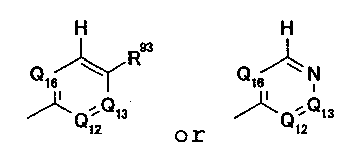 wherein Q₁₂, Q₁₃ and Q₁₆ are as described above, and R⁹³ is an amino group, a nitro group, a hydrogen atom, a trifluoromethyl group, a methoxy group, a methyl group, a chlorine atom or an acetamide group, and
ring B is 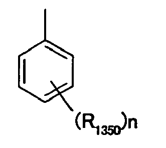 wherein R₁₃₅₀ is a hydrogen atom, -COOMe, a cyano group, -COOEt, t-Bu, a dimethylamino group, a methyl group, a chlorine atom, a dioxolanyl group, a 2-pyridyl group or a 2-pyrimidinyl group, and n is an integer of 1 to 3;
provision 9:
when 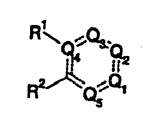 wherein R¹, R², Q₁, Q₂, Q₃, Q₄ and Q₅ are as defined above,
is 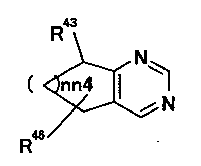 wherein R⁴³ and R⁴⁶ are as defined above, and nn4 is an integer of 1 to 3,
compounds in which ring A is a phenyl group, a 3,4-dimethoxyphenyl group, a 4-methylphenyl group, a 4-chlorophenyl group or a 4-fluorophenyl group, ring B is a phenyl group, a 3,4-dimethoxyphenyl group, a 4-methylphenyl group, a 4-chlorophenyl group, a 4-fluorophenyl group or a 3-chlorophenyl group, R⁴³ and R⁴⁶ are each a hydrogen atom or a methyl group, and nn4 is an integer of 1 to 3 are excluded;
provision 10:
when 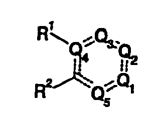 wherein R¹, R², Q₁, Q₂, Q₃, Q₄ and Q₅ are as defined above,
is 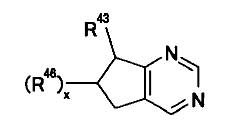 wherein R⁴³ and R⁴⁶ are the same or different and each is a hydrogen atom or a C₁₋₆ alkyl group,
compounds in which ring A is a phenyl group substituted, at the ortho- or meta- position, by a halogen atom, a C₁₋₆ alkyl group, a C₁₋₆ haloalkyl group, a C₁₋₆ alkoxy group or a C₁₋₆ haloalkyloxy group, and ring B is a phenyl group substituted by a hydrogen atom, a halogen atom, a C₁₋₆ alkyl group, a C₁₋₆ haloalkyl group, a C₁₋₆ alkoxy group or a C₁₋₆ haloalkyloxy group are excluded;
provision 11:
when 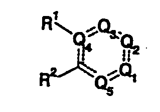 wherein R¹, R², Q₁, Q₂, Q₃, Q₄ and Q₅ are as defined above,
is 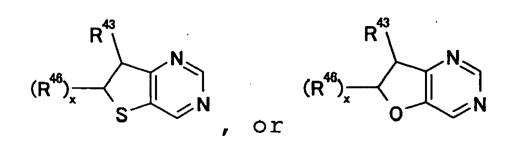 wherein R⁴³ and R⁴⁶ are the same or different and each is a hydrogen or a C₁₋₆ alkyl group,
compounds in which
ring A is a phenyl group substituted at the ortho- or meta- position by a halogen atom, a C₁₋₆ alkyl group, a C₁₋₆ haloalkyl group, a C₁₋₆ alkoxy group or a C₁₋₆ haloalkyloxy group, and ring B is a phenyl group substituted by a hydrogen atom, a halogen atom, a C₁₋₆ alkyl group, a C₁₋₆ haloalkyl group, a C₁₋₆ alkoxy group or a C₁₋₆ haloalkyloxy group are excluded; and
provision 12:
when 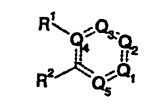 wherein R¹, R², Q₁, Q₂, Q₃, Q₄ and Q₅ are as defined above,
is 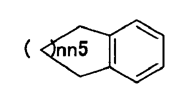 wherein nn5 is an integer of 1 to 3,
compounds in which ring A and ring B are each a 3-nitrophenyl group or a 4-nitrophenyl group, and nn5 is 2 are excluded.

[7] The heterocyclic compound of the aforementioned [6],
wherein, in the formula [1], 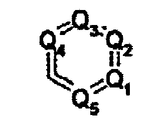 wherein Q₁, Q₂, Q₃, Q₄ and Q₅ are as defined above, is 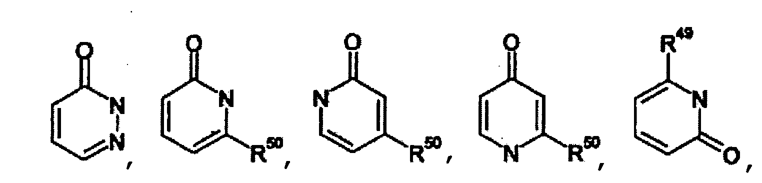 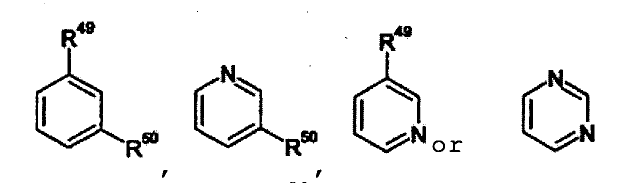 wherein R⁴⁹ and R⁵⁰ are as defined above, or a pharmaceutically acceptable salt thereof.

[8] The heterocyclic compound of the aforementioned [6],
wherein, in the formula [1], the ring formed by R¹ and R², together with Q₄ and the carbon atom to which they are bonded, is 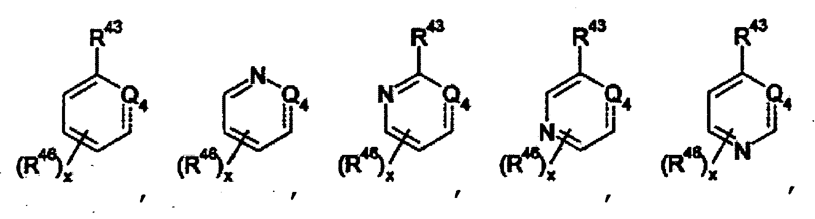 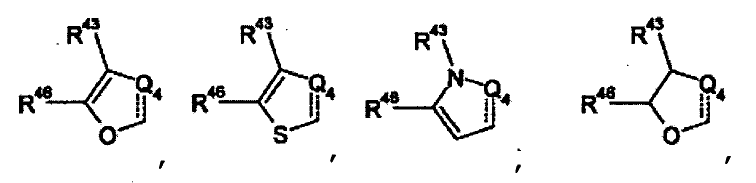 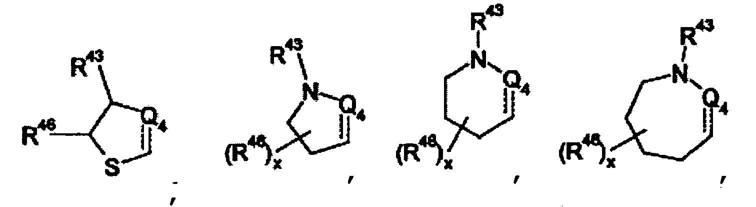 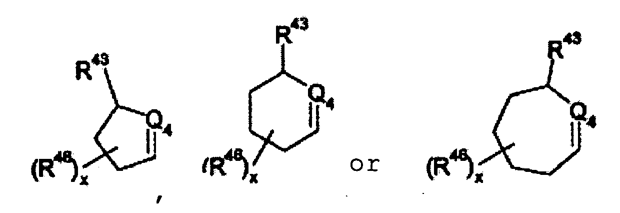 wherein Q₄, R⁴³, R⁴⁶ and x are as defined above, or a pharmaceutically acceptable salt thereof.

[9] The heterocyclic compound of the aforementioned [6],
wherein
R¹ is
(1) a hydrogen atom,
(2) a C₁₋₆ alkyl group (said C₁₋₆ alkyl group is optionally substituted by 1 to 3 substituents selected from Group F),
(3) a C₂₋₆ alkenyl group (said C₂₋₆ alkenyl group is optionally substituted by 1 to 3 substituents selected from Group F),
(4) -J₁-R¹⁰¹ wherein J₁ and R¹⁰¹ are as defined in the aforementioned [6], or
(5) a C₆₋₁₄ aryl group (said C₆₋₁₄ aryl group is optionally substituted by 1 to 5 substituents selected from Group E), or a pharmaceutically acceptable salt thereof.

[10] The heterocyclic compound of the aforementioned [6], wherein R² is a hydrogen atom, or a pharmaceutically acceptable salt thereof.

[11] The heterocyclic compound of the aforementioned [6], wherein R⁴⁹ and R⁵⁰ are the same or different and each is a hydrogen atom, a hydroxyl group or a C₁₋₆ alkoxy group, or a pharmaceutically acceptable salt thereof.

[12] The heterocyclic compound of the aforementioned [6], wherein R⁴³ is -L₃-J₃-R¹¹³ wherein L₂, J₃ and R¹¹³ are as defined in the aforementioned [6], or a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E), or a pharmaceutically acceptable salt thereof.

[13] The heterocyclic compound of the aforementioned [6], wherein 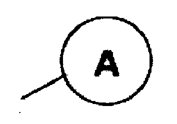 is 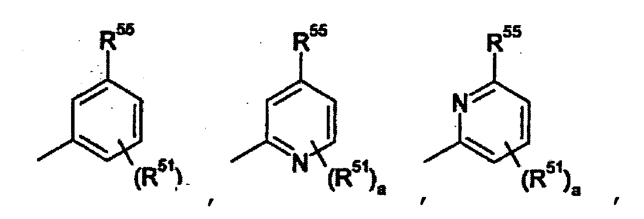 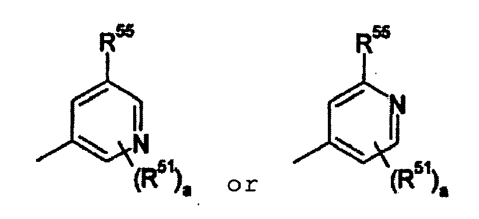 wherein
R⁵⁵ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkynyl group (said C₂₋₆ alkynyl group is optionally substituted by 1 to 3 substituents selected from Group F),
(5) -J₅-R¹²¹ wherein J₅ and R¹²¹ are as defined in the aforementioned [6],
(6) a monocyclic aryl group,
(7) a monocyclic heteroaryl group (said monocyclic heteroaryl group is optionally substituted by 1 to 5 substituents selected from Group E), or
(8) a heterocycloalkyl group,
a is 1 or 2,
R⁵¹ are the same or different and each is
(1) a hydrogen atom,
(2) a halogen atom,
(3) a cyano group,
(4) a nitro group,
(5) a hydroxyl group,
(6) a formyl group,
(7) a carboxyl group,
(8) a C₁₋₆ alkyl group (said C₁₋₆ alkyl group is optionally substituted by 1 to 3 substituents selected from Group G),
(9) a C₂₋₆ alkenyl group,
(10) a C₁₋₆ alkoxy group (said C₁₋₆ alkoxy group is optionally substituted by 1 to 3 substituents selected from Group G),
(11) a C₁₋₆ alkylthio group,
(12) a C₁₋₆ alkylsulfinyl group,
(13) a C₁₋₆ alkylsulfonyl group,
(14) a C₁₋₆ alkyl-carbonyl group (said C₁₋₆ alkyl-carbonyl group is optionally substituted by 1 to 3 substituents selected from Group G),
(15) a C₁₋₆ alkoxy-carbonyl group,
(16) -N(R⁵⁷)(R⁵⁸) wherein R⁵⁷ and R⁵⁸ are as defined in the aforementioned [6],
(17) -CO-N(R⁵⁷)(R⁵⁸) wherein R⁵⁷ and R⁵⁸ are as defined in the aforementioned [6], or
(18) -C(R⁵⁹)=N(R⁶⁰)
   wherein
   R⁵⁹ is
   (1) a hydrogen atom,
   R⁶⁰ is
   (1) a hydroxyl group, or
   (2) a C₁₋₆ alkyl-carbonyl-oxy group,
or
two adjacent R⁵¹s may form, together with the ring to which they are bonded, a fused ring (said fused ring is optionally further substituted by one substituent selected from Group E, or a pharmaceutically acceptable salt thereof.

[14] The heterocyclic compound of the aforementioned [6], wherein 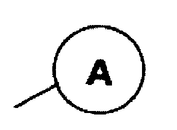 is 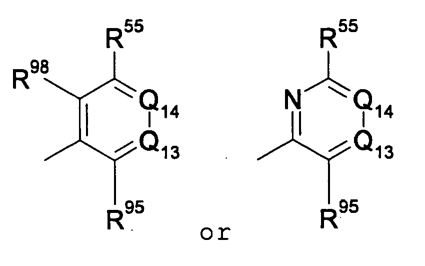 wherein
R⁹⁵ is as defined for R⁵¹ (provided that a hydrogen atom is excluded),
R⁹⁸ is as defined for R⁵¹, and
Q₁₃, Q₁₄, Q₁₆ and R⁵⁵ are as defined in the aforementioned [6], or a pharmaceutically acceptable salt thereof.

[15] The heterocyclic compound of the aforementioned

[14], wherein
R⁹⁵ is
(1) a halogen atom,
(2) a cyano group,
(3) a nitro group,
(4) a formyl group,
(5) a C₁₋₆ alkyl group (said C₁₋₆ alkyl group is optionally substituted by 1 to 3 substituents selected from Group G),
(6) a C₁₋₆ alkylsulfinyl group (said C₁₋₆ alkylsulfinyl group is optionally substituted by 1 to 3 substituents selected from Group G), or
(7) a C₁₋₆ alkylsulfonyl group (said C₁₋₆ alkylsulfonyl group is optionally substituted by 1 to 3 substituents selected from Group G), or a pharmaceutically acceptable salt thereof.

[16] The heterocyclic compound of the aforementioned [6], wherein 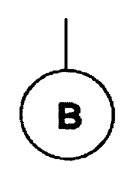 is 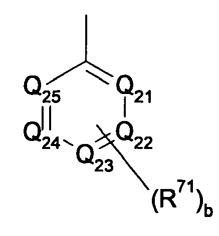 wherein
Q₂₁, Q₂₂, Q₂₃, Q₂₄, and Q₂₅ are the same or different and each is -N= or -CH=, and other symbols are as defined in the aforementioned [6], or two adjacent R⁷¹s may form, together with the ring to which they are bonded, a fused ring (said fused ring is optionally further substituted by one substituent selected from Group E), or a pharmaceutically acceptable salt thereof.

[17] The heterocyclic compound of the aforementioned

[16], wherein 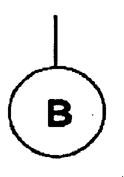 is 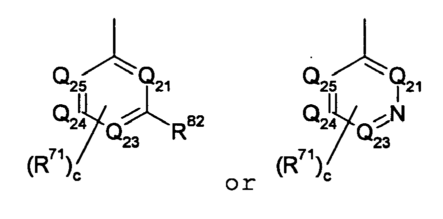 wherein
R⁸² is as defined for R⁷¹ (provided that a hydrogen atom is excluded),
c is 0, 1 or 2,
R⁸² and the adjacent R⁷¹ may form, together with the ring to which they are bonded, a fused ring (said fused ring is optionally substituted by one substituent selected from Group E), and
other symbols are as defined in the aforementioned [16], or a pharmaceutically acceptable salt thereof.

[18]. The heterocyclic compound of the aforementioned

[16], wherein
R⁷¹ are the same or different and each is
(1) a hydrogen atom,
(2) a halogen atom,
(3) a cyano group,
(4) a hydroxyl group,
(5) a formyl group,
(6) a carboxyl group,
(7) a C₁₋₆ alkyl group (said C₁₋₆ alkyl group is optionally substituted by 1 to 3 substituents selected from Group G),
(8) a C₂₋₆ alkenyl group (said C₂₋₆ alkenyl group is optionally substituted by 1 to 3 substituents selected from Group G),
(9) a C₁₋₆ alkoxy group (said C₁₋₆ alkoxy group is optionally substituted by 1 to 3 substituents selected from Group G),
(10) a C₁₋₆ alkyl-carbonyl group,
(11) a C₁₋₆ alkoxy-carbonyl group,
(12) -N(R⁷²)(R⁷³) wherein R⁷² and R⁷³ are as defined in the aforementioned [6])
(13) -CO-N(R⁷²)(R⁷³) wherein R⁷² and R⁷³ are as defined in the aforementioned [6],
(14) -C(R⁷⁴)=N(R⁷⁵) wherein R⁷⁴ and R⁷⁵ are as defined in the aforementioned [6],
(15) a monocyclic aryl group,
(16) a monocyclic heteroaryl group (said monocyclic heteroaryl group is optionally substituted by 1 to 5 substituents selected from Group E), or
(17) a heterocycloalkyl group, or
   two adjacent R⁷¹s may form, together with the ring to which they are bonded, a fused ring (said fused ring is optionally further substituted by one substituent selected from Group E), or a pharmaceutically acceptable salt thereof.

[19] The heterocyclic compound of the aforementioned [6] represented by the following formula [1], or a pharmaceutically acceptable salt thereof: 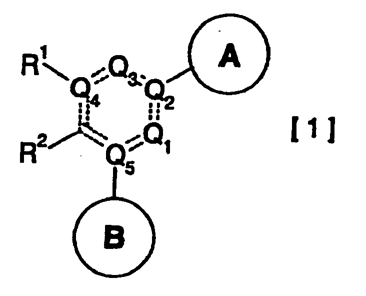 wherein 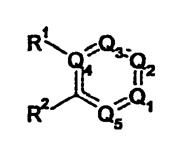 is 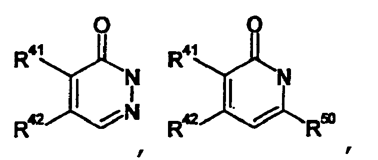 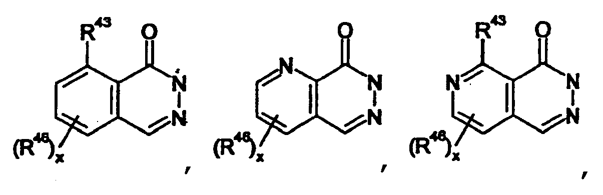 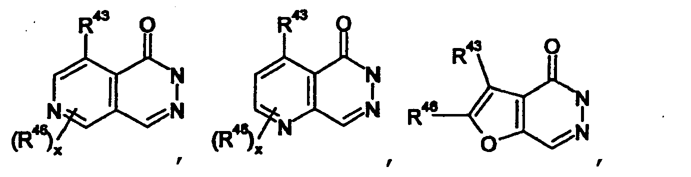 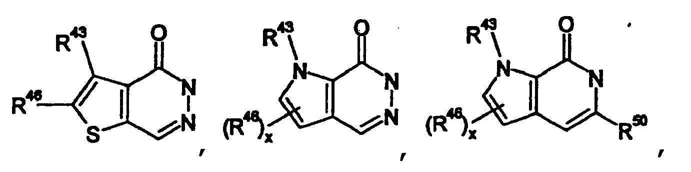 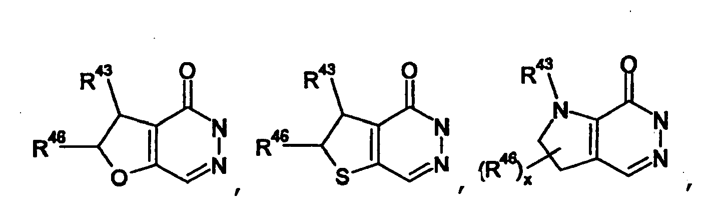 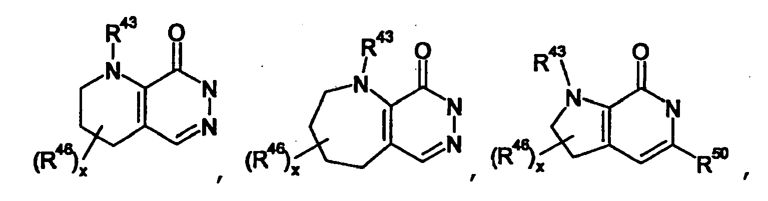 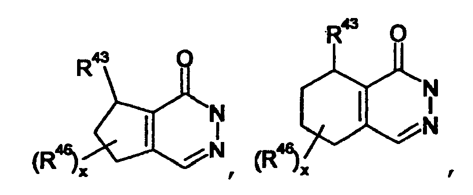 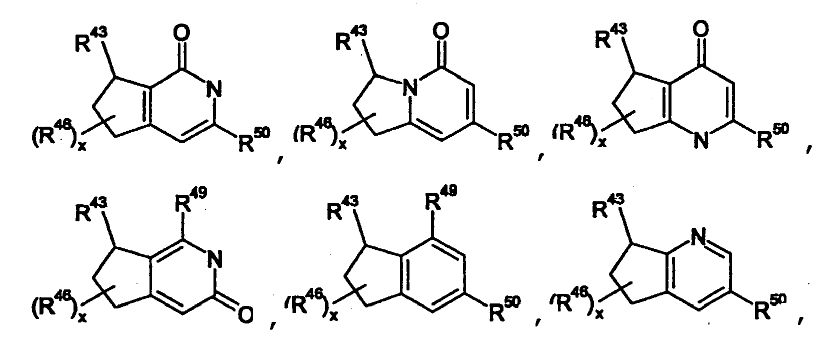 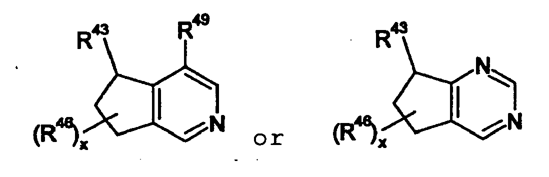 wherein
R⁴¹ is
(1) a hydrogen atom,
(2) a C₁₋₆ alkyl group (said C₁₋₆ alkyl group is optionally substituted by 1 to 3 substituents selected from Group F),
(3) a C₂₋₆ alkenyl group,
(4) -J₁-R¹⁰¹
wherein
J₁ is
(1) -N(R¹⁰²)- wherein R¹⁰² is a hydrogen atom or a C₁₋₆ alkyl group,
(2) -O-,
(3) -S-,
(4) -CO-,
(5) -CO-O-, or
(6) -CO-N(R¹⁰²)- wherein R¹⁰² is as defined above,
R¹⁰¹ is
(1) a hydrogen atom,
(2) -L₂-J₂-R¹⁰³
   wherein
   L₂ is
   (1) a bond, or
   (2) a C₁₋₆ alkylene group,
   J₂ is
   (1) -N(R¹⁰⁴)- wherein R¹⁰⁴ is a hydrogen atom or a C₁₋₆ alkyl group,
   (2) -O-,
   (3) -CO-,
   (4) -SO₂-,
   (5) -CO-O-,
   (6) -CO-N(R¹⁰⁴)- wherein R¹⁰⁴ is as defined above, or
   (7) -SO₂-N(R¹⁰⁴)- wherein R¹⁰⁴ is as defined above,
   R¹⁰³ is
   (1) a hydrogen atom,
   (2) a C₁₋₆ alkyl group, or
   (3) a C₁₋₆ alkyl-carbonyl group, or
   (3) a C₁₋₆ alkyl group, or
   R¹⁰¹ and R¹⁰² may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E), or
(5) a C₆₋₁₄ aryl group;
R⁴² is
(1) a hydrogen atom, or
(2) a C₁₋₆ alkyl group;
R⁴³ is
(1) a hydrogen atom,
(2) -L₃- ₃-R¹¹³
   wherein
   L₃ is
   (1) a bond, or
   (2) a C₁₋₆ alkylene group,
   J₃ is
   (1) -N(R¹¹⁴)- wherein R¹¹⁴ is a hydrogen atom or a C₁₋₆ alkyl group,
   (2) -O-,
   (3) -CO-,
   (4) -SO₂-,
   (5)-CO-O-, or
   (6) -CO-N(R¹¹⁴)- wherein R¹¹⁴ is as defined above,
   R¹¹³ is
   (1) a hydrogen atom,
   (2) -L₄-J₄-R¹¹⁵
      wherein
      L₄ is
      (1) a bond, or
      (2) a C₁₋₆ alkylene group,
      J₄ is
      (1) -N(R¹¹⁶)- wherein R¹¹⁶ is a hydrogen atom or a C₁₋₆ alkyl group,
      (2) -O-, or
      (3) -CO-,
      R¹¹⁵ is
      (1) a hydrogen atom,
      (2) a C₁₋₆ alkyl group (said C₁₋₆ alkyl group is optionally substituted by 1 to 3 substituents selected from Group G), or
      (3) a C₁₋₆ alkyl-carbonyl group, or
      R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
   (3) a C₁₋₆ alkyl group, or
   R¹¹³ and R¹¹⁴ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E);
x is 1 or 2;
R⁴⁶ are the same or different and each is
(1) a hydrogen atom,
(2) a hydroxyl group,
(3) a C₁₋₆ alkyl group, or
(4) a C₁₋₆ alkoxy group;
R⁴⁹ and R⁵⁰ are the same or different and each is
(1) a hydrogen atom,
(2) a hydroxyl group, or
(3) a C₁₋₆ alkoxy group; 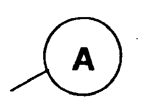 is 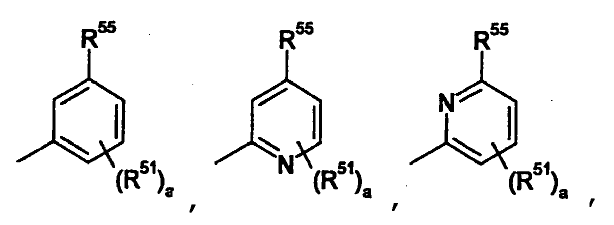 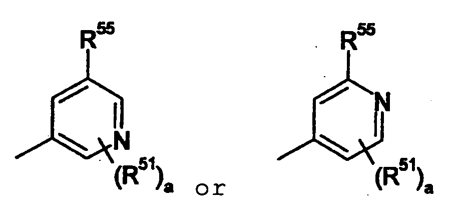
wherein
R⁵⁵ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkynyl group (said C₂₋₆ alkynyl group is optionally substituted by 1 to 3 substituents selected from Group F),
(5) -J₅-R¹²¹
   wherein
   J₅ is
   (1) -N(R¹²²)- wherein R¹²² is a hydrogen atom or a C₁₋₆ alkyl group,
   (2) -O-,
   (3) -CO-,
   (4) -CO-O-,
   (5) -CO-N(R¹²²)- wherein R¹²² is as defined above, or
   (6) -N(R¹²³)-CO- wherein R¹²³ is a hydrogen atom or a C₁₋₆ alkyl group,
   R¹²¹ is
   (1) a hydrogen atom,
   (2) -L₆-J₆-R¹²⁴
      wherein
      L₆ is
      (1) a bond, or
      (2) a C₁₋₆ alkylene group,
      J₆ is
      (1) -N(R¹²⁵)- wherein R¹²¹ is a hydrogen atom or a C₁₋₆ alkyl group,
      (2) -O-,
      (3) -CO-,
      (4) -CO-O-,
      (5) -CO-N(R¹²⁵)- wherein R¹²⁵ is as defined above,
      (6) -N(R¹²⁶)-CO- wherein R¹²⁶ is a hydrogen atom or a C₁₋₆ alkyl group,
      (7) -N(R¹²⁶)-SO₂- wherein R¹²⁶ is as defined above,
      (8) -N(R¹²⁶)-CO-O- wherein R¹²⁶ is as defined above, or
      (9) -N(R¹²⁶)-CO-N(R¹²⁵)- wherein R¹²⁵ and R¹²⁶ are as defined above,
      R¹²⁴ is
      (1) a hydrogen atom, or
      (2) a C₁₋₆ alkyl group (said C₁₋₆ alkyl group is optionally substituted by 1 to 3 substituents selected from Group G), or
      R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
   (3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
   (4) a substituent selected from a halogen atom, a cyano group and a nitro group,
   (5) a C₆₋₁₄ aryl group (said C₆₋₁₄ aryl group is optionally substituted by 1 to 5 substituents selected from Group E),
   (6) a heteroaryl group (said heteroaryl group is optionally substituted by 1 to 5 substituents selected from Group E), or
   (7) a C₃₋₈ cycloalkyl group (said C₃₋₈ cycloalkyl group is optionally substituted by 1 to 5 substituents selected from Group E), or
   R¹²¹ and R¹²² may form, together with the nitrogen atom to which they are bonded, a nitrogen-containing 4-to 7-membered heterocyclic group, wherein said nitrogen-containing 4- to 7-membered heterocyclic group is optionally substituted by 1 to 5 substituents selected from
   (1) Group F,
   (2) a C₁₋₆ alkyl group (said C₁₋₆ alkyl group is optionally substituted by 1 to 3 substituents selected from Group F),
   (3) a C₁₋₆ alkoxy group (said C₁₋₆ alkoxy group is optionally substituted by 1 to 3 substituents selected from Group F), and
   (4) an oxo group,
(6) a monocyclic aryl group,
(7) a monocyclic heteroaryl group (said monocyclic heteroaryl group is optionally substituted by 1 to 5 substituents selected from Group E), or
(8) a heterocycloalkyl group;
a is 1 or 2;
R⁵¹ are the same or different and each is
(1) a hydrogen atom,
(2) a halogen atom,
(3) a cyano group,
(4) a nitro group,
(5) a hydroxyl group,
(6) a formyl group,
(7) a carboxyl group,
(8) a C₁₋₆ alkyl group (said C₁₋₆ alkyl group is optionally substituted by 1 to 3 substituents selected from Group G),
(9) a C₂₋₆ alkenyl group,
(10) a C₁₋₆ alkoxy group (said C₁₋₆ alkoxy group is optionally substituted by 1 to 3 substituents selected from Group G),
(11) a C₁₋₆ alkylthio group,
(12) a C₁₋₆ alkylsulfinyl group,
(13) a C₁₋₆ alkylsulfonyl group,
(14) a C₁₋₆ alkyl-carbonyl group (said C₁₋₆ alkyl-carbonyl group is optionally substituted by 1 to 3 substituents selected from Group G),
(15) a C₁₋₆ alkoxy-carbonyl group,
(16) -N(R⁵⁷)(R⁵⁸)
   wherein
   R⁵⁷ and R⁵⁸ are the same or different and each is
   (1) a hydrogen atom,
   (2) a C₁₋₆ alkyl group,
   (3) a C₁₋₆ alkylsulfonyl group,
   (4) a C₁₋₆ alkyl-carbonyl group, or
   (5) a C₁₋₆ alkoxy-carbonyl group, or
   R⁵⁷ and R⁵⁸ may form, together with the nitrogen atom to which they are bonded, a nitrogen-containing 4-to 7-membered heterocyclic group,
(17) -CO-N(R⁵⁷)(R⁵⁸) wherein R⁵⁷ and R⁵⁸ are as defined above, or
(18) -C(R⁵⁹)=N(R⁶⁰)
   wherein
   R⁵⁹ is
   (1) a hydrogen atom,
   R⁶⁰ is
   (1) a hydroxyl group, or
   (2) a C₁₋₆ alkyl-carbonyl-oxy group, or
      two adjacent R⁵¹s may form, together with the ring to which they are bonded, a fused ring (said fused ring is optionally further substituted by one substituent selected from Group E); 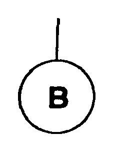 is 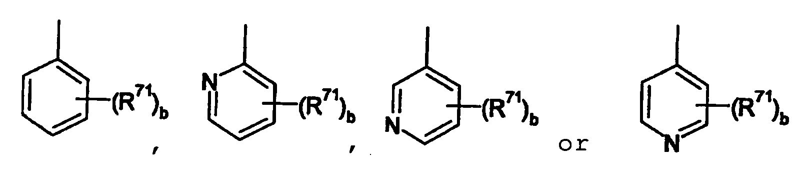
wherein
b is 1 or 2;
R⁷¹ are the same or different and each is
(1) a hydrogen atom,
(2) a halogen atom,
(3) a cyano group,
(4) a hydroxyl group,
(5) a formyl group,
(6) a carboxyl group,
(7) a C₁₋₆ alkyl group (said C₁₋₆ alkyl group is optionally substituted by 1 to 3 substituents selected from Group G),
(8) a C₂₋₆ alkenyl group (said C₂₋₆ alkenyl group is optionally substituted by 1 to 3 substituents selected from Group G),
(9) a C₁₋₆ alkoxy group (said C₁₋₆ alkoxy group is optionally substituted by 1 to 3 substituents selected from Group G),
(10) a C₁₋₆ alkyl-carbonyl group,
(11) a C₁₋₆ alkoxy-carbonyl group,
(12) -N(R⁷²)(R⁷³)
   wherein
   R⁷² and R⁷³ are the same or different and each is
   (1) a hydrogen atom,
   (2) a C₁₋₆ alkyl group (said C₁₋₆ alkyl group is optionally substituted by 1 to 3 substituents selected from Group G),
   (3) a C₁₋₆ alkylsulfonyl group,
   (4) a C₁₋₆ alkyl-carbonyl group,
   (5) a C₁₋₆ alkoxy-carbonyl group,
   (6) a carbamoyl group,
   (7) a hydroxyl group,
   (8) an amino group, or
   (9) a C₁₋₆ alkyl-carbonyl-amino group, or
      R⁷² and R⁷³ may form, together with the nitrogen atom to which they are bonded, a nitrogen-containing 4-to 7-membered heterocyclic group,
(13) -CO-N(R⁷²)(R⁷³) wherein R⁷² and R⁷³ are as defined above,
(14) -C(R⁷⁴)=N(R⁷⁵)
   wherein
   R⁷⁴ is
   (1) a hydrogen atom,
   (2) a C₁₋₆ alkyl group, or
   (3) an amino group,
   R⁷⁵ is
   (1) a hydroxyl group,
   (2) a C₁₋₆ alkoxy group (said C₁₋₆ alkoxy group is optionally substituted by 1 to 3 substituents selected from Group G), or
   (3)-N(R⁷⁶)(R⁷⁷) wherein R⁷⁶ and R⁷⁷ are the same or different and each is a hydrogen atom, or a C₁₋₆ alkyl-carbonyl group,
(15) a monocyclic aryl group,
(16) a monocyclic heteroaryl group (said monocyclic heteroaryl group is optionally substituted by 1 to 5 substituents selected from Group E), or
(17) a heterocycloalkyl group, or
   two adjacent R⁷¹s may form, together with the ring to which they are bonded, a fused ring (said fused ring is optionally further substituted by one substituent selected from Group E):
wherein
Group E consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkyl group (said C₁₋₆ alkyl group is optionally substituted by 1 to 3 substituents selected from Group G),
(12) a C₂₋₆ alkenyl group (said C₂₋₆alkenyl group is optionally substituted by 1 to 3 substituents selected from Group G),
(13) a C₂₋₆ alkynyl group (said C₂₋₆ alkynyl group is optionally substituted by 1 to 3 substituents selected from Group G),
(14) a C₁₋₆ alkoxy group (said C₁₋₆ alkoxy group is optionally substituted by 1 to 3 substituents selected from Group G),
(15) a C₁₋₆ alkylthio group,
(16) a C₁₋₆ alkylsulfinyl group,
(17) a C₁₋₆ alkylsulfonyl group,
(18) a C₁₋₆ alkyl-carbonyl group,
(19) a C₁₋₆ alkoxy-carbonyl group,
(20) a C₁₋₆ alkylamino group,
(21) a C₆₋₁₄aryl group,
(22) a heteroaryl group, and
(23) an oxo group;
Group F consists of
(1) a halogen atom,
(2) a cyano group,
(3) a nitro group,
(4) a hydroxyl group,
(5) a mercapto group,
(6) a formyl group,
(7) a carboxyl group,
(8) a C₁₋₆ alkoxy group,
(9) a C₁₋₆ alkylthio group,
(10) a C₁₋₆ alkylsulfinyl group,
(11) a C₁₋₆ alkylsulfonyl group,
(12) a C₁₋₆ alkyl-carbonyl group,
(13) a C₁₋₆ alkoxy-carbonyl group,
(14) -N(R⁹¹)(R⁹²) wherein R⁹¹ and R⁹² are the same or different and each is a hydrogen atom, a C₁₋₆ alkyl group, a C₁₋₆ alkyl-carbonyl group, a C₁₋₆ alkylsulfonyl group, a C₁₋₆ alkoxy-carbonyl group, a carbamoyl group, a C₁₋₆ alkylamino-carbonyl group, or a di(C₁₋₆ alkyl)amino-carbonyl group, or R⁹¹ and R⁹² may form, together with the nitrogen atom to which they are bonded, a nitrogen-containing 4- to 7-membered heterocyclic group,
(15) -CO-N(R⁹¹)(R⁹²) wherein R⁹¹ and R⁹² are as defined above,
(16) -SO₂-N(R⁹¹)(R⁹²) wherein R⁹¹ and R⁹² are as defined above,
(17) -O-CO-N(R⁹¹)(R⁹²) wherein R⁹¹ and R⁹² are as defined above,
(18) a C₆₋₁₄ aryl group,
(19) a C₆₋₁₄ aryl-oxy group,
(20) a C₆₋₁₄ aryl-carbonyl group, and
(21) a heteroaryl group;
Group G consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkoxy group,
(12) a C₁₋₆ alkylthio group,
(13) a C₁₋₆ alkylsulfinyl group,
(14) a C₁₋₆ alkylsulfonyl group,
(15) a C₁₋₆ alkyl-carbonyl group,
(16) a C₁₋₆ alkoxy-carbonyl group,
(17) a C₁₋₆ alkylamino group,
(18) a C₁₋₆ alkyl-carbonyl-amino group,
(19) a C₆₋₁₄ aryl group, and
(20) heteroaryl group,
   provided that the compound of the formula [1] satisfies the the following provisions 1 to 12:

### provision 1:

when 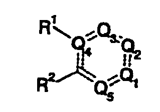 wherein R¹, R², Q₁, Q₂, Q₃, Q₄ and Q₅ are as defined above,
is 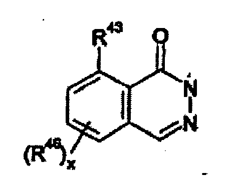 wherein R⁴³, R⁴⁶ and x are as defined above, 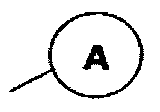 is 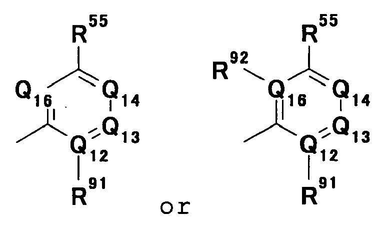 wherein
Q₁₂, Q₁₃, Q₁₄, Q₁₆ and R⁵⁵ are as defined above,
R⁹¹ is as defined for R⁵¹ (provided that a hydrogen atom is excluded), and
R⁹² is as defined for R⁵¹, and
the compounds satisfying the following provisions 1-1 to 1-3 are excluded:
provision 1-1:
ring A is 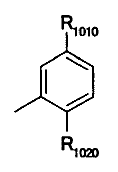 wherein R₁₀₁₀ is a chlorine atom, a piperidinyl group, a pyrrolidinyl group, an azepanylgroup or a morpholinyl group, and R₁₀₂₀ is a nitro group or an amino group, and ring B is 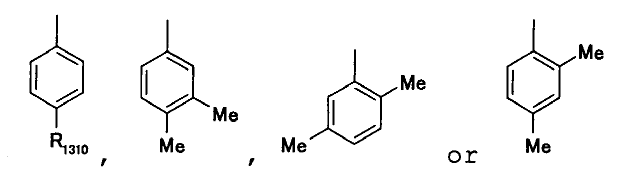 wherein R₁₃₁₀ is a hydrogen atom, a fluorine atom, a chlorine atom, a methyl group or an ethyl group;
provision 1-2:
ring A is 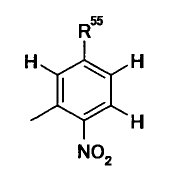 wherein
R⁵⁵ is an unsubstituted or substituted piperazinyl group optionally substituted by 1 to 5 substituents selected from Group E, Group F, a C₁₋₆ alkyl group (said C₁₋₆ alkyl group is optionally substituted by 1 to 3 substituents selected from Group F) and a C₁₋₆ alkoxy group (said C₁₋₆ alkoxy group is optionally substituted by 1 to 3 substituents selected from Group F); and
provision 1-3:
ring A is 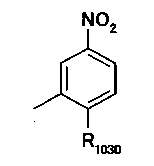 wherein R₁₀₃₀ is a chlorine atom, a pyrrolidinyl group, a morpholinyl group or a N-methylpiperazinyl group, and ring B is 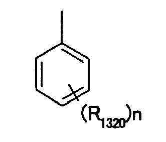 wherein n is 1 or 2, R₁₃₂₀ is a hydrogen atom, a chlorine atom or a methyl group;
provision 2:
when 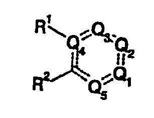 wherein R¹, R², Q₁, Q₂, Q₃, Q₄ and Q₅ are as defined above, is 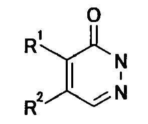 wherein R¹ and R² are as defined above, provided that the case where R¹ and R² form, together with Q₄ and the carbon atom to which they are respectively bonded, 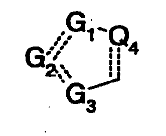 wherein G₁, G₂, G₃ and Q₄ are as defined above, is excluded, 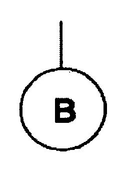 is 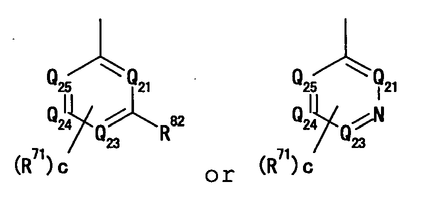 wherein Q₂₁, Q₂₃, Q₂₄ and Q₂₅ are the same or different and each is a carbon atom or a nitrogen atom, R⁷¹ is as defined above, R⁸² is as defined for R⁷¹ (provided that a hydrogen atom is excluded), c is 0, 1 or 2, or two adjacent R⁷¹s may form, together with the ring to which they are bonded, a fused ring (said fused ring is optionally further substituted by one substituent selected from Group E), and the following compounds are excluded:
6-(2,5-dimethylphenyl)-2-phenyl-3(2H)-pyridazinone,
6-(3,4-dimethylphenyl)-2-phenyl-3(2H)-pyridazinone,
6-(4-chloro-3-methylphenyl)-2-phenyl-3(2H)-pyridazinone,
2-(2-aminophenyl)-6-(3-chlorophenyl)-3(2H)-pyridazinone,
2-(2-aminophenyl)-6-[3-(trifluoromethyl)phenyl]-3(2H)-pyridazinone,
6-(3-chlorophenyl)-2-(2-nitrophenyl)-3(2H)-pyridazinone,
2-(2-nitrophenyl)-6-[3-(trifluoromethyl)phenyl]-3(2H)-
pyridazinone,
6-(3,4-dichlorophenyl)-2-phenyl-4-(2,4,6-trimethylphenyl)-3(2H)-pyridazinone,
2-(2,4-dinitrophenyl)-6-(3-nitrophenyl)-3(2H)-pyridazinone,
2-(4-chlorophenyl)-6-(3-nitrophenyl)-3(2H)-pyridazinone,
2-(3-chlorophenyl)-6-(3-nitrophenyl)-3(2H)-pyridazinone,
2-(4-methylphenyl)-6-(3-nitrophenyl)-3(2H)-pyridazinone,
2-(3-methylphenyl)-6-(3-nitrophenyl)-3(2H)-pyridazinone,
2-(2-methylphenyl)-6-(3-nitrophenyl)-3(2H)-pyridazinone,
6-(3-nitrophenyl)-2-(4-nitrophenyl)-3(2H)-pyridazinone,
2,6-bis(3-nitrophenyl)-3(2H)-pyridazinone,
2-(2-nitrophenyl)-6-(3-nitrophenyl)-3(2H)-pyridazinone,
6-(3-nitrophenyl)-2-phenyl-3(2H)-pyridazinone,
6-(2-naphthyl)-2-phenyl-3(2H)-pyridazinone,
6-(1-naphthalenyl)-2-phenyl-3(2H)-pyridazinone,
2-(2-cyanophenyl)-4-(2-methoxyphenyl)-6-(3-pyridazinyl)-2H-pyridazin-3-one,
2-(2-cyanophenyl)-4-phenyl-6-(2-pyrazyl)-2H-pyridazin-3-one,
2-(2-aminophenyl)-6-(3-pyridazinyl)-3(2H)-pyridazinone, 2-(2-nitrophenyl)-6-(3-pyridazinyl)-3(2H)-pyridazinone,
6,6''-dichloro-[3,1'(6'H):3',3''-terpyridazine]-6'-one,
3-[1,6-dihydro-1-(4-methylphenyl)-6-oxo-3-pyridazinyl]-2(1H)-quinoxalinone,
3-[1,6-dihydro-1-(2-methylphenyl)-6-oxo-3-pyridazinyl]-2(1H)-quinoxalinone,
3-(1,6-dihydro-6-oxo-1-phenyl-3-pyridazinyl)-1-methyl-2(1H)-quinoxalinone,
3-[1-(4-chlorophenyl)-1,6-dihydro-6-oxo-3-pyridazinyl]-2(1H)-quinoxalinone, and
3-(1,6-dihydro-6-oxo-1-phenyl-3-pyridazinyl)-2(1H)-quinoxalinone;
provision 3:
when 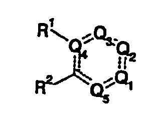 wherein R¹, R², Q₁, Q₂, Q₃, Q₄ and Q₅ are as defined above,
is 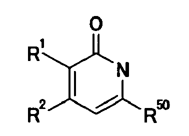 wherein R¹ and R² are as defined above, provided that the case where R¹ and R² form, together with Q₄ and the carbon atom to which they are respectively bonded, 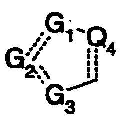 wherein G₁, G₂, G₃ and Q₄ are as defined above, is excluded, and R⁵⁰ is as defined above, 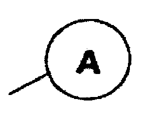 is 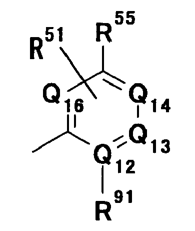 wherein Q₁₂, Q₁₃, Q₁₄, Q₁₆, R⁵¹ and R⁵⁵are as defined above, R⁹¹ is as defined for R⁵¹ (provided that a hydrogen atom is excluded), 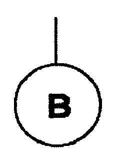 is 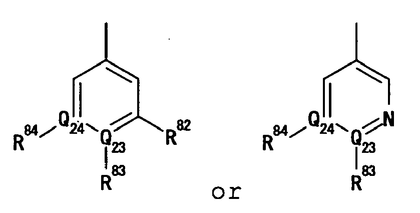 wherein
Q₂₃ and Q₂₄ are the same or different and each is a carbon atom or a nitrogen atom,
R⁸² is as defined for R⁷¹ (provided that a hydrogen atom is excluded),
R⁸³ and R⁸⁴ are the same or different and each is as defined for R⁷¹, or R⁸³ and R⁸⁴ may form, together with the ring to which they are bonded, a fused ring (said fused ring is optionally further substituted by one substituent selected from Group E);
provision 4:
when 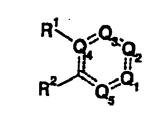
wherein R¹, R², Q₁, Q₂, Q₃, Q₄ and Q₅ are as defined above,
is 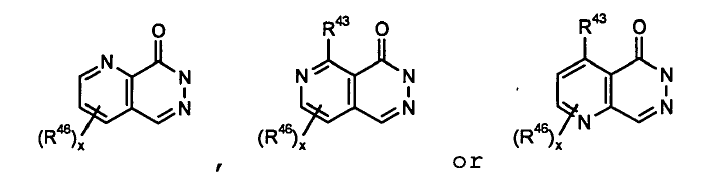 wherein R⁴³ and R⁴⁶ are the same or different and each is a hydrogen atom, a halogen atom, a hydroxyl group or a C₁₋₆ alkyl group, and x is as defined above,
the compounds satisfying the following provisions 4-1 and 4-2 are excluded:
provision 4-1: 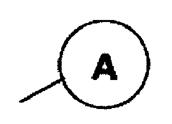 is 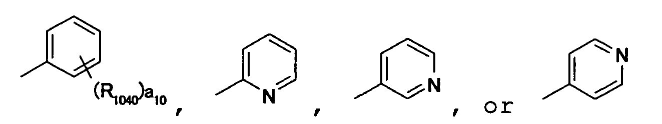 wherein when a₁₀ is 1, R₁₀₄₀ is an amino group, a hydrogen atom, a trifluoromethyloxy group, a bromine atom, a sulfamoyl group, a methanesulfonyl group, a hydroxyl group, a C₁₋₆ alkyl group, a C₁₋₆ alkoxy group, a chlorine atom, a fluorine atom, a trifluoromethyl group, a carbamoyl group, a nitro group or a cyano group, and a₁₀ is an integer of 2 or 3, R₁₀₄₀ are the same or different and each is a hydroxyl group, a C₁₋₆ alkyl group, a C₁₋₆ alkoxy group, a chlorine atom, a fluorine atom, a trifluoromethyl group, a carbamoyl group, a nitro group or a cyano group, and
ring B is a benzotriazole group, or 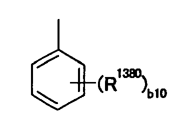 wherein b10 is an integer of 0 or 1 to 5, R¹³⁸⁰ are the same or different and each is a hydrogen atom, a halogen atom, a C₁₋₆ alkyl group, -COOH, a C₁₋₆ alkoxycarbonyl group, a carbamoyl group, a C₁₋₆ alkylcarbonyl group, a halocarbonyl group, a methylthio group, a trifluoromethyl group, a cyano group, a nitro group, a 2,2-difluorodioxolanyl group, a methanesulfonyl group or a trifluoromethyloxy group;
provision 4-2: 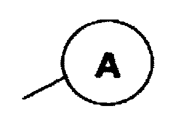 is 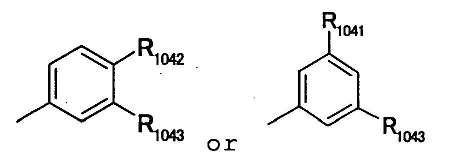 wherein R₁₀₄₁ is a fluorine atom or a trifluoromethyl group, R₁₀₄₂ is a fluorine atom or chlorine atom, R₁₀₄₃ is a fluorine atom, a chlorine atom, a cyano group or a trifluoromethyl group, or R₁₀₄₂ and R₁₀₄₃ form a ring represented by 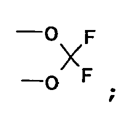 provision 5:
when 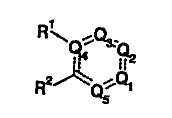
is 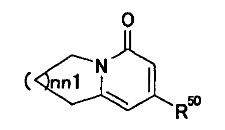
wherein R⁵⁰ is a hydrogen atom or a hydroxyl group, and nn1 is an integer of 1 to 3,
the compounds satisfying the following provisions 5-1 and 5-2 are excluded:
provision 5-1:
ring A is a phenyl group or a 3,4-dimethoxyphenyl group, and ring B is a phenyl group or a 4-chlorophenyl group, and nn1 is 1;
provision 5-2:
ring A is a phenyl group or a pyridyl group, which is optionally substituted by a halogen atom, a trifluoromethyl group, a nitro group, a C₁₋₆ alkyl group or a C₁₋₆ alkoxy group, and nn1 is 2 or 3;
provision 6:
when 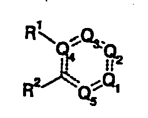 wherein R¹, R², Q₁, Q₂, Q₃, Q₄ and Q₅ are as defined above,
is 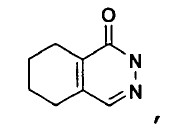 compounds in which ring A is a phenyl group and ring B is a 3,4-dimethoxyphenyl group are excluded;
provision 7:
when 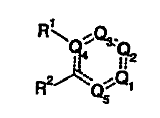 wherein R¹, R², Q₁, Q₂, Q₃, Q₄ and Q₅ are as defined above,
is 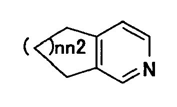 wherein nn2 is an integer of 1 to 3,
compounds in which ring A is a phenyl group or 4-chlorophenyl group, and nn2 is an integer of 1 to 3 are excluded;
provision 8:
when 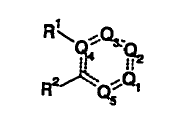 wherein R¹, R², Q₁, Q₂, Q₃, Q₄ and Q₅ are as defined above,
is 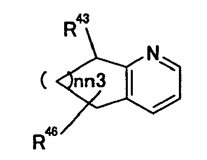 wherein R⁴³ is a hydrogen atom, a hydroxyl group, an acetyloxy group, a methyl group, a benzyl group, a 4-methylbenzyl group, -CH₂-NMe₂ or -CH₂-S-nBu, R⁴⁶ is a hydrogen atom or a methyl group, and nn3 is an integer of 1 to 3,
the following compounds are excluded:
compounds in which ring A is 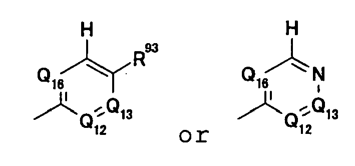 wherein Q₁₂, Q₁₃ and Q₁₆ are as described above, and R⁹³ is an amino group, a nitro group, a hydrogen atom, a trifluoromethyl group, a methoxy group, a methyl group, a chlorine atom or an acetamide group, and
ring B is 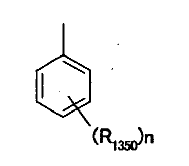 wherein R₁₃₅₀ is a hydrogen atom, -COOMe, a cyano group, -COOEt, t-Bu, a dimethylamino group, a methyl group, a chlorine atom, a dioxolanyl group, a 2-pyridyl group or a 2-pyrimidinyl group, and n is an integer of 1 to 3;
provision 9:
when 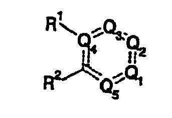 wherein R¹, R², Q₁, Q₂, Q₃, Q₄ and Q₅ are as defined above,
is 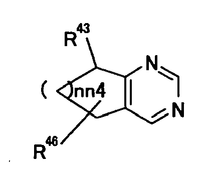 wherein R⁴³ and R⁴⁶ are as defined above, and nn4 is an integer of 1 to 3,
compounds in which ring A is a phenyl group, a 3,4-dimethoxyphenyl group, a 4-methylphenyl group, a 4-chlorophenyl group or a 4-fluorophenyl group, ring B is a phenyl group, a 3,4-dimethoxyphenyl group, a 4-methylphenyl group, a 4-chlorophenyl group, a 4-fluorophenyl group or a 3-chlorophenyl group, R⁴³ and R⁴⁶ are each a hydrogen atom or a methyl group, and nn4 is an integer of 1 to 3 are excluded;
provision 10:
when 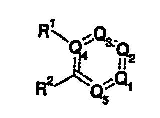 wherein R¹, R², Q₁, Q₂, Q₃, Q₄ and Q₅ are as defined above,
is 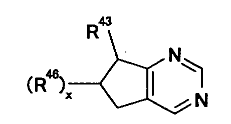 wherein R⁴³ and R⁴⁶ are the same or different and each is a hydrogen atom or a C₁₋₆ alkyl group,
compounds in which ring A is a phenyl group substituted, at the ortho- or meta- position, by a halogen atom, a C₁₋₆ alkyl group, a C₁₋₆ haloalkyl group, a C₁₋₆ alkoxy group or a C₁₋₆ haloalkyloxy group, and ring B is a phenyl group substituted by a hydrogen atom, a halogen atom, a C₁₋₆ alkyl group, a C₁₋₆ haloalkyl group, a C₁₋₆ alkoxy group or a C₁₋₆ haloalkyloxy group are excluded;
provision 11:
when 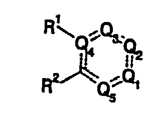 wherein R¹, R², Q₁, Q₂, Q₃, Q₄ and Q₅ are as defined above,
is 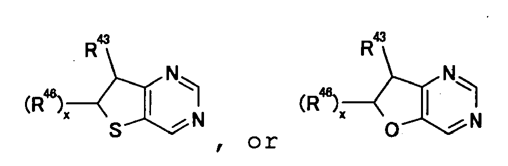 wherein R⁴³ and R⁴⁶ are the same or different and each is a hydrogen or a C₁₋₆ alkyl group,
compounds in which ring A is a phenyl group substituted at the ortho- or meta- position by a halogen atom, a C₁₋₆ alkyl group, a C₁₋₆ haloalkyl group, a C₁₋₆ alkoxy group or a C₁₋₆ haloalkyloxy group, and ring B is a phenyl group substituted by a hydrogen atom, a halogen atom, a C₁₋₆ alkyl group, a C₁₋₆ haloalkyl group, a C₁₋₆ alkoxy group or a C₁₋₆ haloalkyloxy group are excluded; and
provision 12:
when 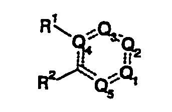 wherein R¹, R², Q₁, Q₂, Q₃, Q₄ and Q₅ are as defined above,
is 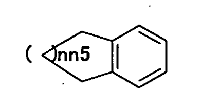 wherein nn5 is an integer of 1 to 3,
compounds in which ring A and ring B are each a 3-nitrophenyl group or a 4-nitrophenyl group, and nn5 is 2 are excluded.

20. A heterocyclic compound represented by the following formula: 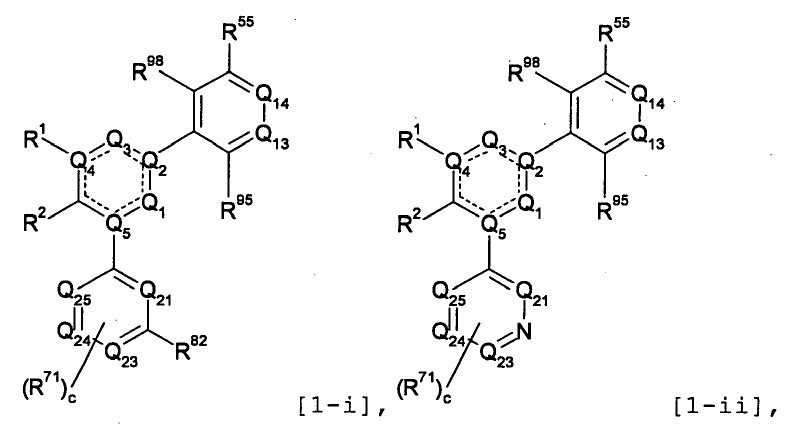 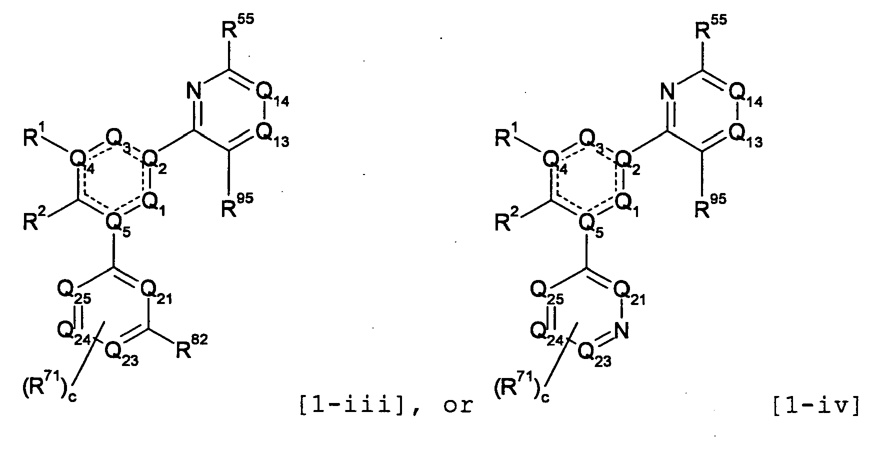 wherein Q₁, Q₂, Q₃, Q₄, Q₅, R¹, R², R⁵⁵ and R⁷¹ are as defined in the aforementioned [1], Q₁₃ and Q₁₄ are as defined in the aforementioned [6], R⁹⁵ and R⁹⁸ are as defined in the aforementioned [12], Q₂₁, Q₂₂, Q₂₄ and Q₂₅ are as defined in the aforementioned [14], and R⁸² is as defined in the aforementioned [17], or a pharmaceutically acceptable salt thereof.

[21] A pharmaceutical composition comprising the compound of any one of the aforementioned [6]-[20] or a pharmaceutically acceptable salt thereof and a pharmaceutically acceptable carrier.

[22] An HCV entry inhibitor comprising the compound of any one of the aforementioned [6]-[20] or a pharmaceutically acceptable salt thereof as an active ingredient.

[23] An anti-hepatitis virus agent comprising the compound of any one of the aforementioned [6]-[20] or a pharmaceutically acceptable salt thereof as an active ingredient.

[24] A therapeutic agent for hepatitis C comprising the compound of any one of the aforementioned [6]-[20] or a pharmaceutically acceptable salt thereof as an active ingredient.

[25] A therapeutic agent for hepatitis C comprising a combination of (a) the therapeutic agent for hepatitis C of the aforementioned [24], and (b) at least one pharmaceutical agent selected from other antiviral agent, anti-inflammatory agent and immunity enhancing agent.

[26] A therapeutic agent for hepatitis C comprising a combination of (a) the therapeutic agent for hepatitis C of the aforementioned [24], and (b) an interferon.

[27] An anti-hepatitis virus agent comprising a combination of (a) the anti-hepatitis virus agent of the aforementioned [23], and (b) at least one pharmaceutical agent selected from other antiviral agent, anti-inflammatory agent and immunity enhancing agent.

[28] An anti-hepatitis virus agent comprising a combination of (a) the anti-hepatitis virus agent of the aforementioned [23], and (b) an interferon.

[29] A pharmaceutical composition comprising a combination of (a) the compound of any one of the aforementioned [6]-[20] or a pharmaceutically acceptable salt thereof, and (b) at least one pharmaceutical agent selected from other antiviral agent, anti-inflammatory agent and immunity enhancing agent.

[30] A pharmaceutical composition comprising a combination of (a) the compound of any one of the aforementioned [6]-[20] or a pharmaceutically acceptable salt thereof, and (b) an interferon.

[31] Use of the compound of any one of the aforementioned [6]-[20] or a pharmaceutically acceptable salt thereof for the production of an HCV entry inhibitor.

[32] Use of the compound of any one of the aforementioned [6]-[20] or a pharmaceutically acceptable salt thereof for the production of an anti-hepatitis C virus agent.

[33] Use of the compound of any one of the aforementioned [6]-[20] or a pharmaceutically acceptable salt thereof for the production of a therapeutic agent for hepatitis C.

[34] Use of a combination of (a) the therapeutic agent for hepatitis C of the aforementioned [24] and (b) at least one pharmaceutical agent selected from other antiviral agent, anti-inflammatory agent and immunity enhancing agent for the production of a therapeutic agent for hepatitis C.

[35] Use of a combination of (a) the therapeutic agent for hepatitis C of the aforementioned [24] and (b) an interferon for the production of a therapeutic agent forf hepatitis C.

[36] Use of a combination of (a) the anti-hepatitis virus agent of the aforementioned [23] and (b) at least one pharmaceutical agent selected from other antiviral agent, anti-inflammatory agent and immunity enhancing agent for the production of an anti-hepatitis C virus agent.

[37] Use of a combination of (a) the anti-hepatitis virus agent of the aforementioned [23] and (b) an interferon for the production of an anti-hepatitis C virus agent.

The present invention also provides the following.

[1'] A condensed pyridazinone compound represented by the following formula [10], or a pharmaceutically acceptable salt thereof: 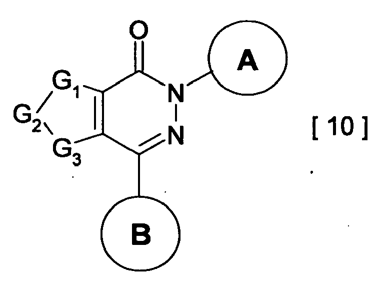 wherein
G₁ is
(1) -N(R³)- wherein R³ is a hydrogen atom, a substituent selected from (Ca)-(Cq) of the following Group C, or a C₁₋₆ alkyl group optionally substituted by 1 to 5 substituents selected from (Ca)-(Cq) in the following Group C,
(2) -C(R³) (R⁴)- wherein R³ is as defined above, R⁴ is a hydrogen atom, a substituent selected from (Da)-(Dx) in the following Group D, or a C₁₋₆ alkyl group optionally substituted by 1 to 5 substituents selected from (Da)-(Dx) in the following Group D,
(3) -C(R³)(R⁴)-C(R⁵)(R⁶)- wherein R³ and R⁴ are as defined above, and R⁵ and R⁶ are as defined for R³ and R⁴, respectively,
(4) -S-, or
(5) -O-;
G₂ is
(1) -N(R^{3'})- wherein R^{3'} is as defined for R³,
(2) -C(R^{3'})(R^{4'})- wherein R^{3'} and R^{4'} are as defined for R³ and R⁴, respectively,
(3) -C(R^{3'})(R^{4'})-C(R^{5'})(R^{6'})- wherein R^{3'}, R^{4'}, R^{5'} and R^{6'} are as defined for R³, R⁴, R⁵ and R⁶, respectively,
(4) -S-, or
(5) -O-;
G₃ is
(1) -N(R^{3''})- wherein R^{3''} is as defined for R³,
(2) -C(R^{3''})(R^{4''})- wherein R^{3''} and R^{4''} are as defined for R³ and R⁴, respectively,
(3) -C(R^{3''})(R^{4''})-C(R^{5''})(R^{6''})- wherein R^{3''}, R^{4''}, R^{5''}, and R^{6''} are as defined for R³, R⁴, R⁵ and R⁶, respectively,
(4) -S-, or
(5) -O-;
ring A and ring B are the same or different and each is
(1) a C₆₋₁₄ aryl group optionally substituted by 1 to 5 substituents selected from (Da)-(Dx) in the following Group D,
(2) a C₃₋₈ cycloalkyl group optionally substituted by 1 to 5 substituents selected from (Da)-(Dx) in the following Group D,
(3) a C₃₋₈ cycloalkenyl group optionally substituted by 1 to 5 substituents selected from (Da)-(Dx) in the following Group D,
(4) a 4- to 7-membered heterocyclic group optionally substituted by 1 to 5 substituents selected from (Da)-(Dx) in the following Group D, or
(5) a 5- to 14-membered aromatic condensed heterocyclic group,
Group C consists of:
(Ca) a halogen atom,
(Cb) a hydroxyl group,
(Cc) a cyano group,
(Cd) a C₁₋₆ alkyl group (said C₁₋₆ alkyl group is optionally substituted by substituent(s) selected from a hydroxyl group, a C₁₋₇ acylamino group, a (C₁₋₇
acyl)(C₁₋₆ alkyl) amino group, a halogen atom, a C₁₋₆ alkylamino-carbonyl group and a di(C₁₋₆ alkyl)amino-carbonyl group),
(Ce) a C₁₋₆ alkoxy group (said C₁₋₆ alkoxy group is optionally substituted by substituent(s) selected from a hydroxyl group, a C₁₋₇ acylamino group, a (C₁₋₇ acyl)(C₁₋₆ alkyl) amino group, a C₁₋₆ alkylamino-carbonyl group and a di(C₁₋₆ alkyl)amino-carbonyl group),
(Cf) -CO(R⁷) wherein R⁷ is a hydroxyl group or a C₁₋₆ alkyl group (said C₁₋₆ alkyl group is optionally substituted by substituent(s) selected from a hydroxyl group, a C₁₋₇ acyloxy group, an amino group and a C₇₋₂₀ aralkyloxy group (the aryl moiety of said C₇₋₂₀ aralkyloxy group is optionally substituted by substituent(s) selected from (Da)-(Dx) in the following Group D)),
(Cg) a C₁₋₆ alkoxy-carbonyl group,
(Ch) a C₁₋₇ acyl group,
(Ci) -CON(R⁸)(R⁹) {wherein R⁸ and R⁹ are the same or different and each is a hydrogen atom or a C₁₋₆ alkyl group [said C₁₋₆ alkyl group is optionally substituted by substituent(s) selected from a hydroxyl group, a C₁₋₆ alkoxy group, a C₁₋₇ acylamino group and -N(R¹⁰)(R¹¹)
   wherein R¹⁰ and R¹¹ are the same or different and each is a hydrogen atom, a C₁₋₆ alkyl group or a C₁₋₇ acyl group, or R¹⁰ and R¹¹ may form, together with the nitrogen atom to which they are bonded, a nitrogen-containing 5- or 6-membered heterocycle (said nitrogen-containing 5- or 6-membered heterocycle is optionally substituted by substituent(s) selected from a C₁₋₆ alkyl group (said C₁₋₆ alkyl group is optionally substituted by a C₁₋₇ acylamino group), a C₁₋₇ acyl group and a hydroxyl group)], or R⁸ and R⁹ may form, together with the nitrogen atom to which they are bonded, a nitrogen-containing 5- or 6-membered heterocycle (said nitrogen-containing 5- or 6-membered heterocycle is optionally substituted by substituent(s) selected from a C₁₋₆ alkyl group (said C₁₋₆ alkyl group is optionally substituted by a C₁₋₇ acylamino group), a C₁₋₇ acyl group, a hydroxyl group and an oxo group)},
(Cj) -N(R⁸)(R⁹) wherein R⁸ and R⁹ are as defined above, (Ck) -N(R⁸)CO(R⁷) wherein R⁸ and R⁷ are as defined above,
(Cl) -N(R⁸)CON(R¹⁰)(R¹¹) wherein R⁸, R¹⁰ and R¹¹ are as defined above,
(Cm) -N(R⁸)SO₂(R⁷) wherein R⁷ and R⁸ are as defined above,
(Cn) -SO₂(R⁷) wherein R⁷ is as defined above,
(Co) a C₁₋₆ alkylamino-carbonyloxy group or a di(C₁₋₆ alkyl)amino-carbonyloxy group,
(Cp) a C₆₋₁₄ aryl group optionally substituted by 1 to 5 substituents selected from (Da)-(Dx) in the following Group D, and
(Cq) an oxo group,
Group D consists of:
(Da) a halogen atom,
(Db) a hydroxyl group,
(Dc) a C₁₋₆ alkoxy group optionally substituted by 1 to 5 substituents selected from (Ca)-(Cq) in the aforementioned Group C,
(Dd) a C₁₋₆ alkyl group optionally substituted by 1 to 5 substituents selected from (Ca)-(Cq) in the aforementioned Group C,
(De) -CON(R¹²)(R¹³) wherein R¹² and R¹³ are as defined for R⁸ and R⁹, respectively,
(Df) -N(R¹⁴)-(CH₂)m-(R¹⁵) [wherein R¹⁴ is a hydrogen atom, a C₁₋₆ alkyl group (said C₁₋₆ alkyl group is optionally substituted by substituent(s) selected from a hydroxyl group, a C₁₋₇ acyloxy group, an amino group, a C₆₋₁₄ aryl group, a C₃₋₈ cycloalkyl group and a C₇₋₂₀ aralkyloxy group (the aryl moiety of said C₇₋₂₀ aralkyloxy group is optionally substituted by 1 to 5 substituents selected from (Ca)-(Cq) in the aforementioned Group C), a C₁₋₆ alkoxy-carbonyl group, a C₁₋₇ acyl group, or a C₆₋₁₄ aryl group; R¹⁵ is a hydrogen atom, a hydroxyl group, a C₁₋₆ alkyl group (said C₁₋₆ alkyl group is optionally substituted by 1 to 5 substituents selected from a hydroxyl group, a C₁₋₇ acyloxy group, an amino group, a C₆₋₁₄ aryl group, a C₃₋₈ cycloalkyl group and a C₇₋₂₀ aralkyloxy group (the aryl moiety of said C₇₋₂₀ aralkyloxy group is optionally substituted by 1 to 5 substituents selected from (Ca)-(Cq) in the aforementioned Group C)), a C₁₋₆ alkoxy-carbonyl group, a C₁₋₆ alkoxy group, a C₁₋₇ acyl group, - N(R¹⁶)(R¹⁷) wherein R¹⁶ and R¹⁷ are as defined for R⁸ and R⁹, respectively, or a C₆₋₁₄ aryl group; and m is an integer of 0 or 1-6,
(Dg) -N(R¹⁸)-(CH₂)n-CO(R¹⁹) wherein R¹⁸ is as defined for R¹⁴, R¹⁹ is as defined for R¹⁵, and n is an integer of 0 or 1-6,
(Dh) -N(R²⁰)-(CH₂)q-N(R²¹)(R²²) wherein R²⁰ is as defined for R¹⁴, R²¹ and R²¹ are as defined for R⁸ and R⁹, respectively, and q is an integer of 0 or 1-6),
(Di) -N(R²³)-(CH₂)r-N(R²⁴)-SO₂(R²⁵) wherein R²³ and R²⁴ are each as defined for R¹⁴, R²⁵ is as defined for R¹⁵, and r is an integer of 0 or 1-6,
(Dj) -N(R²⁶)-(CH₂)s-CON(R²⁷)(R²⁸) wherein R²⁶ is as defined for R¹⁴, R²⁷ and R²⁸ are as defined for R⁸ and R⁹, respectively, and s is an integer of 0 or 1-6,
(Dk) -S(R²⁹) wherein R²⁹ is as defined for R¹⁴,
(Dl) -SO(R³⁰) wherein R³⁰ is as defined for R¹⁴,
(Dm) -SO₂(R³¹) wherein R³¹ is as defined for R¹⁴,
(Dn) -N(R³²)SO₂(R³³) wherein R³² is as defined for R¹⁴ and R³³ is as defined for R¹⁵,
(Do) -SO₂N(R³⁴)(R³⁵) wherein R³⁴ and R³⁵ are as defined for R⁸ and R⁹, respectively,
(Dp) -CO(R³⁶) wherein R³⁶ is as defined for R¹⁵,
(Dq) a nitro group,
(Dr) a cyano group,
(Ds) a C₆₋₁₄ aryl group optionally substituted by 1 to 5 substituents selected from (Ca)-(Cq) in the aforementioned Group C,
(Dt) a C₃₋₈ cycloalkyl group optionally substituted by 1 to 5 substituents selected from (Ca)-(Cq) in the aforementioned Group C,
(Du) a C₃₋₈ cycloalkenyl group optionally substituted by 1 to 5 substituents selected from (Ca)-(Cq) in the aforementioned Group C,
(Dv) a 4- to 7-membered heterocyclic group optionally substituted by 1 to 5 substituents selected from (Ca)-(Cq) in the aforementioned Group C,
(Dw) -N(R³⁷)-(CH₂)ₜ-N(R³⁸)COR³⁹ wherein R³⁷ and R³⁸ are each as defined for R¹⁴, R³⁹ is as defined for R¹⁵, and t is an integer of 1-6), and
(Dx) a C₂₋₆ alkynyl group (said C₂₋₆ alkynyl group is optionally substituted by a hydroxyl group).

[2'] The condensed pyridazinone compound of the aforementioned [1'], wherein
G₁ is
(1) -N(R³)- wherein R³ is as defined above, or
(2) -C(R³) (R⁴)- wherein R³ and R⁴ are each as defined above;
G₂ is
(2) -C(R^{3'})(R^{4'})- wherein R^{3'} and R^{4'} are each as defined above; and
G₃ is
(2) -C(R^{3''})(R^{4''})- wherein R^{3''} and R^{4''} are each as defined above,
(4) -S-, or
(5) -O-,
   or a pharmaceutically acceptable salt thereof.

[3'] The condensed pyridazinone compound of the aforementioned [1'], wherein
G₁ is
(1) -N(R³)- wherein R³ is as defined above, or
(2) -C(R³)(R⁴)- wherein R³ and R⁴ are each as defined above;
G₂ is
(3) -C(R^{3'})(R^{4'})-C(R^{5'})(R^{6'})- wherein R^{3'}, R^{4'}, R^{5'} and R^{6'} are as defined for R³, R⁴, R⁵ and R⁶, respectively; and G₃ is
(2) -C(R^{3''})(R^{4''})- wherein R^{3''} and R^{4''} are as defined for R³ and R⁴, respectively,
or a pharmaceutically acceptable salt thereof.

[4'] The condensed pyridazinone compound of the aforementioned [1'], wherein
G₁ is
(1) -N(R³)- wherein R³ is as defined above, or
(2) -C(R³)(R⁴)- wherein R³ and R⁴ are each as defined above;
G₂ is
(3) -C(R^{3'})(R^{4'})-C(R^{5'})(R^{6'})- wherein R^{3'}, R^{4'}, R^{5'} and R^{6'} are as defined for R³, R⁴, R⁵ and R⁶, respectively; and
G₃ is
(3) -C(R^{3''})(R^{4''})-C(R^{5''})(R^{6''})- wherein R^{3''}, R^{4''}, R^{5''} and R^{6''} are as defined for R³, R⁴, R⁵ and R⁶, respectively,
or a pharmaceutically acceptable salt thereof.

[5'] A pharmaceutical composition comprising the condensed pyridazinone compound of any one of the aforementioned [1']-[4'] or a pharmaceutically acceptable salt thereof and a pharmaceutically acceptable carrier.

[6'] A HCV cell infection inhibitor comprising the compound of any one of the aforementioned [1']-[4'] or a pharmaceutically acceptable salt thereof as an active ingredient.

[7'] An anti-hepatitis virus agent comprising the compound of any one of the aforementioned [1']-[4'] or a pharmaceutically acceptable salt thereof as an active ingredient.

[8'] A therapeutic agent for hepatitis C comprising the compound of any one of the aforementioned [1']-[4'] or a pharmaceutically acceptable salt thereof as an active ingredient.

[9'] A therapeutic agent for hepatitis C comprising a combination of (a) the therapeutic agent of hepatitis C of the aforementioned [8'], and (b) at least one pharmaceutical agent selected from other antiviral agent, anti-inflammatory agent and immunity enhancing agent.

[10'] A therapeutic agent for hepatitis C comprising a combination of (a) the therapeutic agent of hepatitis C of the aforementioned [8'], and (b) an interferon. [11'] An anti-hepatitis virus agent comprising a combination of (a) the anti-hepatitis virus agent of the aforementioned [7'], and (b) at least one pharmaceutical agent selected from other antiviral agent, anti-inflammatory agent and immunity enhancing agent.

[12'] An anti-hepatitis virus agent comprising a combination of (a) the anti-hepatitis virus agent of the aforementioned [7'], and (b) an interferon.

[13'] A pharmaceutical composition comprising (a) the compound of any one of the aforementioned [1']-[4'] or a pharmaceutically acceptable salt thereof, and (b) at least one pharmaceutical agent selected from other antiviral agent, anti-inflammatory agent and immunity enhancing agent.

[14'] A pharmaceutical composition comprising (a) the compound of any one of the aforementioned [1']-[4'] or a pharmaceutically acceptable salt thereof, and (b) interferon.

### Effect of the Invention

The novel heterocyclic compound of the present invention or a pharmaceutically acceptable salt thereof has an HCV entry inhibitory (HCV cell infection inhibitory) activity. Therefore, it is useful as an HCV entry inhibitor (HCV cell infection inhibitor), an anti-hepatitis virus agent, a therapeutic agent for hepatitis C and the like.

Furthermore, the hepatitis C virus agent and therapeutic agent for hepatitis C can also be used as a concomitant agent in combination with at least one pharmaceutical agent selected from other antiviral agent, anti-inflammatory agent and immunity enhancing agent, or with an interferon.

### Best Mode for Embodying the Invention

In the present specification, the definitions of the substituents used are as follows.

In the present specification, the "halogen atom" refers to a fluorine atom, a chlorine atom, a bromine atom or an iodine atom, preferably a fluorine atom, a chlorine atom or a bromine atom.

In the present specification, the "C₁₋₆ alkyl group" refers to a straight chain or branched chain alkyl group having 1 to 6 carbon atoms, and examples thereof include methyl group, ethyl group, propyl group, isopropyl group, butyl group, isobutyl group, sec-butyl group, tert-butyl group, pentyl group, 1-methylbutyl group, 2-methylbutyl group, 3-methylbutyl group, 1,1-dimethylpropyl group, 1,2-dimethylpropyl group, 2,2-dimethylpropyl group, 1-ethylpropyl group, 2-methylpropyl group, hexyl group and the like.

In the present specification, the "C₁₋₆ alkoxy group" refers to a group formed by bonding the aforementioned "C₁₋₆ alkyl group" to an oxygen atom, and examples thereof include methoxy group, ethoxy group, propoxy group, isopropoxy group, butoxy group, isobutoxy group, tert-butoxy group, pentyloxy group, 1-methylbutyloxy group, 2-methylbutyloxy group, 3-methylbutyloxy group, 1,1-dimethylpropyloxy group, 1,2-dimethylpropyloxy group, 2,2-dimethylpropyloxy group, 1-ethylpropoxy group, hexyloxy group and the like.

In the present specification, the "C₁₋₆ alkoxy-carbonyl group" refers to a group formed by bonding the aforementioned "C₁₋₆ alkoxy group" to a carbonyl group, and examples thereof include methoxycarbonyl group, ethoxycarbonyl group, propoxycarbonyl group, isopropoxycarbonyl group, butoxycarbonyl group, isobutoxycarbonyl group, tert-butoxycarbonyl group, pentyloxycarbonyl group, 1-methylbutoxycarbonyl group, 2-methylbutoxycarbonyl group, 3-methylbutoxycarbonyl group, 1,1-dimethylpropoxycarbonyl group, 1,2-dimethylpropoxycarbonyl group, 2,2-dimethylpropoxycarbonyl group, 1-ethylpropoxycarbonyl group, hexyloxycarbonyl group and the like.

In the present specification, the "C₁₋₆ alkylamino-carbonyl group" refers to a group formed by bonding the aforementioned "'C₁₋₆ alkyl group" to an aminocarbonyl group, and examples thereof include methylaminocarbonyl group, ethylaminocarbonyl group, propylaminocarbonyl group, isopropylaminocarbonyl group, butylaminocarbonyl group, isobutylaminocarbonyl group, sec-butylaminocarbonyl group, tert-butylaminocarbonyl group, pentylaminocarbonyl group, 1-methylbutylaminocarbonyl group, 2-methylbutylaminocarbonyl group, 3-methylbutylaminocarbonyl group, 1,1-dimethylpropylaminocarbonyl group, 1,2-dimethylpropylaminocarbonyl group, 2,2-dimethylpropylaminocarbonyl group, 1-ethylpropylaminocarbonyl group, hexylaminocarbonyl group and the like.

In the present specification, the "di(C₁₋₆ alkyl)amino-carbonyl group" refers to a group formed by bonding the same or different two "C₁₋₆ alkyl groups" mentioned above to an aminocarbonyl group, and examples thereof include dimethylaminocarbonyl group, diethylaminocarbonyl group, dipropylaminocarbonyl group, diisopropylaminocarbonyl group, dibutylaminocarbonyl group, diisobutylaminocarbonyl group, di-sec-butylaminocarbonyl group, di-tert-butylaminocarbonyl group, dipentylaminocarbonyl group, di-(1-methylbutyl)aminocarbonyl group, di-(2-methylbutyl)aminocarbonyl group, di-(3-methylbutyl)aminocarbonyl group, di-(1,1-dimethylpropyl)aminocarbonyl group, di-(1,2-dimethylpropyl)aminocarbonyl group, di-(2,2-dimethylpropyl)aminocarbonyl group, di-(1-ethylpropyl)aminocarbonyl group, dihexylaminocarbonyl group, ethylmethylaminocarbonyl group, methylpropylaminocarbonyl group, butylmethylaminocarbonyl group and the like.

In the present specification, the "C₁₋₆ alkylamino-carbonyloxy group" refers to a group formed by bonding the aforementioned "C₁₋₆ alkylamino-carbonyl group" to an oxygen atom, and examples thereof include methylaminocarbonyloxy group, ethylaminocarbonyloxy group, propylaminocarbonyloxy group, isopropylaminocarbonyloxy group, butylaminocarbonyloxy group, isobutylaminocarbonyloxy group, sec-butylaminocarbonyloxy group, tert-butylaminocarbonyloxy group, pentylaminocarbonyloxy group, 1-methylbutylaminocarbonyloxy group, 2-methylbutylaminocarbonyloxy group, 3-methylbutylaminocarbonyloxy group, 1,1-dimethylpropylaminocarbonyloxy group, 1,2-dimethylpropylaminocarbonyloxy group, 2,2-dimethylpropylaminocarbonyloxy group, 1-ethylpropylaminocarbonyloxy group, hexylaminocarbonyloxy group and the like.

In the present specification, the "di(C₁₋₆ alkyl)amino-carbonyloxy group" is a group formed by bonding the same or different two "C₁₋₆ alkyl groups" mentioned above to an aminocarbonyloxy group, and examples thereof include dimethylaminocarbonyloxy group, diethylaminocarbonyloxy group, dipropylaminocarbonyloxy group, diisopropylaminocarbonyloxy group, dibutylaminocarbonyloxy group, diisobutylaminocarbonyloxy group, di-sec-butylaminocarbonyloxy group, di-tert-butylaminocarbonyloxy group, dipentylaminocarbonyloxy group, di-(1-methylbutyl)aminocarbonyloxy group, di-(2-methylbutyl)aminocarbonyloxy group, di-(3-methylbutyl)aminocarbonyloxy group, di-(1,1-dimethylpropyl)aminocarbonyloxy group, di-(1,2-dimethylpropyl)aminocarbonyloxy group, di-(2,2-dimethylpropyl)aminocarbonyloxy group, di-(1-ethylpropyl)aminocarbonyloxy group, dihexylaminocarbonyloxy group, ethylmethylaminocarbonyloxy group, methylpropylaminocarbonyloxy group, butylmethylaminocarbonyloxy group and the like.

In the present specification, the "C₁₋₆ alkylthio group" refers to a group formed by bonding the aforementioned "C₁₋₆ alkyl group" to a sulfur atom, and examples thereof include methylthio group, ethylthio group, propylthio group, isopropylthio group, butylthio group, isobutylthio group, sec-butylthio group, tert-butylthio group, pentylthio group, 1-methylbutylthio group, 2-methylbutylthio group, 3-methylbutylthio group, 1,1-dimethylpropylthio group, 1,2-dimethylpropylthio group, 2,2-dimethylpropylthio group, 1-ethylpropylthio group, hexylthio group and the like.

In the present specification, the "C₁₋₆ alkylsulfinyl group" refers to a group formed by bonding the aforementioned "C₁₋₆ alkyl group" to a sulfinyl group, and examples thereof include methylsulfinyl group, ethylsulfinyl group, propylsulfinyl group, isopropylsulfinyl group, butylsulfinyl group, isobutylsulfinyl group, sec-butylsulfinyl group, tert-butylsulfinyl group, pentylsulfinyl group, 1-methylbutylsulfinyl group, 2-methylbutylsulfinyl group, 3-methylbutylsulfinyl group, 1,1-dimethylpropylsulfinyl group, 1,2-dimethylpropylsulfinyl group, 2,2-dimethylpropylsulfinyl group, 1-ethylpropylsulfinyl group, hexylsulfinyl group and the like.

In the present specification, the "C₁₋₆ alkylsulfonyl group" refers to a group formed by bonding the aforementioned "C₁₋₆ alkyl group" to a sulfonyl group, and examples thereof include methylsulfonyl group, ethylsulfonyl group, propylsulfonyl group, isopropylsulfonyl group, butylsulfonyl group, isobutylsulfonyl group, sec-butylsulfonyl group, tert-butylsulfonyl group, pentylsulfonyl group, 1-methylbutylsulfonyl group, 2-methylbutylsulfonyl group, 3-methylbutylsulfonyl group, 1,1-dimethylpropylsulfonyl group, 1,2-dimethylpropylsulfonyl group, 2,2-dimethylpropylsulfonyl group, 1-ethylpropylsulfonyl group, hexylsulfonyl group and the like.

In the present specification, the "C₁₋₆ alkyl-carbonyl group" refers to a group formed by bonding the aforementioned "C₁₋₆ alkyl,group" to a carbonyl group, and examples thereof include methylcarbonyl group, ethylcarbonyl group, propylcarbonyl group, isopropylcarbonyl group, butylcarbonyl group, isobutylcarbonyl group, sec-butylcarbonyl group, tert-butylcarbonyl group, pentylcarbonyl group, 1-methylbutylcarbonyl group, 2-methylbutylcarbonyl group, 3-methylbutylcarbonyl group, 1,1-dimethylpropylcarbonyl group, 1,2-dimethylpropylcarbonyl group, 2,2-dimethylpropylcarbonyl group, 1-ethylpropylcarbonyl group, hexylcarbonyl group and the like.

In the present specification, the "C₁₋₆ alkyl-carbonyl-oxy group" refers to a group formed by bonding the aforementioned "C₁₋₆ alkylcarbonyl group" to an oxygen atom, and examples thereof include methylcarbonyloxy group, ethylcarbonyloxy group, propylcarbonyloxy group, isopropylcarbonyloxy group, butylcarbonyloxy group, isobutylcarbonyloxy group, sec-butylcarbonyloxy group, tert-butylcarbonyloxy group, pentylcarbonyloxy group, 1-methylbutylcarbonyloxy group, 2-methylbutylcarbonyloxy group, 3-methylbutylcarbonyloxy group, 1,1-dimethylpropylcarbonyloxy group, 1,2-dimethylpropylcarbonyloxy group, 2,2-dimethylpropylcarbonyloxy group, 1-ethylpropylcarbonyloxy group, hexylcarbonyloxy group and the like.

In the present specification, the "C₁₋₆ alkyl-carbonyl-amino group" refers to a group formed by bonding the aforementioned "C₁₋₆ alkylcarbonyl group" to an amino group, and examples thereof include methylcarbonylamino group, ethylcarbonylamino group, propylcarbonylamino group, isopropylcarbonylamino group, butylcarbonylamino group, isobutylcarbonylamino group, sec-butylcarbonylamino group, tert-butylcarbonylamino group, pentylcarbonylamino group, 1-methylbutylcarbonylamino group, 2-methylbutylcarbonylamino group, 3-methylbutylcarbonylamino group, 1,1-dimethylpropylcarbonylamino group, 1,2-dimethylpropylcarbonylamino group, 2,2-dimethylpropylcarbonylamino group, 1-ethylpropylcarbonylamino group, hexylcarbonylamino group and the like.

In the present specification, the "C₁₋₆ alkylamino group" refers to a group formed by bonding the aforementioned "C₁₋₆ alkyl group" to an amino group, and examples thereof include methylamino group, ethylamino group, propylamino group, isopropylamino group, butylamino group, isobutylamino group, sec-butylamino group, tert-butylamino group, pentylamino group, 1-methylbutylamino group, 2-methylbutylamino group, 3-methylbutylamino group, 1,1-dimethylpropylamino group, 1,2-dimethylpropylamino group, 2,2-dimethylpropylamino group, 1-ethylpropylamino group, hexylamino group and the like.

In the present specification, the "di(C₁₋₆ alkyl)amino group" refers to a group formed by bonding same or different two "C₁₋₆ alkyl groups" mentioned above to an amino group, and examples thereof include dimethylamino group, diethylamino group, dipropylamino group, diisopropylamino group, dibutylamino group, diisobutylamino group, di-sec-butylamino group, di-tert-butylamino group, dipentylamino group, di-(1-methylbutyl)amino group, di-(2-methylbutyl)amino group, di-(3-methylbutyl)amino group, di-(1,1-dimethylpropyl)amino group, di-(1,2-dimethylpropyl)amino group, di-(2,2-dimethylpropyl)amino group, di-(1-ethylpropyl)amino group, dihexylamino group, ethylmethylamino group, ethylpropylamino group, butylmethylamino group and the like.

The preferable examples of the aforementioned "C₁₋₆ alkyl group" and the "C₁₋₆ alkyl" portion of each substituent comprising the "C₁₋₆ alkyl group" as defined above are, but are not limited to, methyl group, ethyl group, propyl group, isopropyl group, butyl group, isobutyl group, tert-butyl group, 1,1-dimethylpropyl group and 1-ethylpropyl group for R¹ and R⁴¹; methyl group, ethyl group, propyl group, isopropyl group, butyl group, isobutyl group and tert-butyl group for R¹⁰¹, R¹⁰², R¹⁰³ and R¹⁰⁴; methyl group and ethyl group for R³ and R⁴³; methyl group, ethyl group, propyl group, isopropyl group, butyl group, isobutyl group and tert-butyl group, pentyl group and hexyl group for R¹¹³ and R¹¹⁵; methyl group and ethyl group for R¹¹⁴ and R¹¹⁶; methyl group and ethyl group for R⁴, R⁵, R⁶, R^{3'}, R^{4'}, R^{5'}, R^{6'}, R^{3''}, R^{4''}, R^{5''}, R^{6''}, R⁴⁴ and R⁴⁶; methyl group for R⁴⁹ and R⁵⁰; methyl group, ethyl group and propyl group for R⁵⁵; methyl group, ethyl group, propyl group, isopropyl group, butyl group and tert-butyl group for R¹²¹ and R¹²⁴; methyl group and ethyl group for R¹²², R¹²³, R¹²⁵ and R¹²⁶; methyl group, ethyl group, propyl group, isopropyl group, butyl group, isobutyl group, tert-butyl group, pentyl group, 3-methylbutyl group and 1-ethylpropyl group for R⁵¹, R⁵⁷, R⁵⁸, R⁵⁹, R⁶⁰, R⁶¹ and R⁶²; and methyl group, ethyl group, propyl group, isopropyl group, butyl group, isobutyl group, tert-butyl group, pentyl group, 3-methylbutyl group and 1-ethylpropyl group for R⁷¹, R⁷², R⁷³, R⁷⁴, R⁷⁵, R⁷⁶ and R⁷⁷.

When the aforementioned "C₁₋₆ alkyl group" and the "C₁₋₆ alkyl" portion in the substituents comprising the "C₁₋₆ alkyl group" as defined above are optionally substituted, preferable examples of the substituted "C₁₋₆ alkyl" include, but are not limited to, trifluoromethyl group, cyanomethyl group, hydroxymethyl group, 1-hydroxyethyl group, 2-hydroxyethyl group, 1,2-dihydroxyethyl group, (2,2-dihydroxyethyl group, )3-hydroxypropyl group, 1-hydroxy-1-methyl-ethyl group, methoxymethyl group, 2,2-dimethoxyethyl group, methylaminomethyl group, dimethylaminomethyl group, 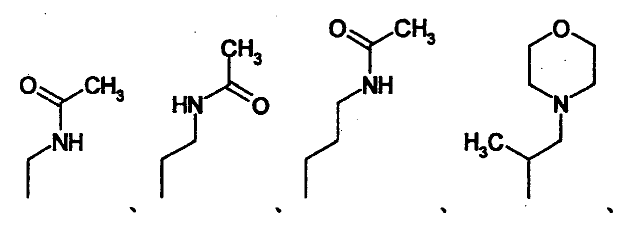 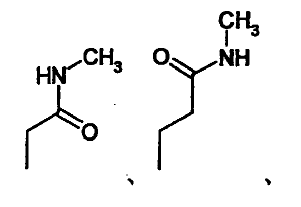 benzyl group, 4-methoxybenzyl group, phenethyl group, 3-phenylpropyl group, cyclopropylmethyl group, 2-cyclopropylethyl group, cyclopentylmethyl group, 2-cyclopentylethyl group and the like.

In the present specification, the "C₁₋₆ alkylene group" refers to a straight chain or branched chain alkylene group having 1 to 6 carbon atoms, and examples thereof include, but are not limited to, methylene group, ethylene group, trimethylene group, tetramethylene group, pentamethylene group, hexamethylene group, 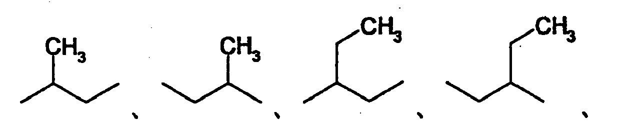 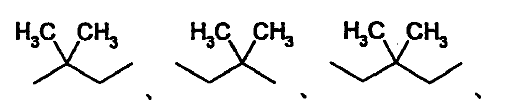 and the like, preferably methylene group, ethylene group, trimethylene group, tetramethylene group, pentamethylene group, hexamethylene group,

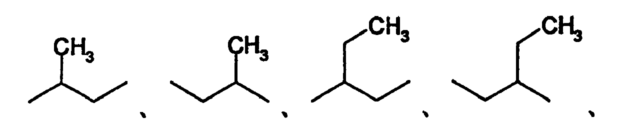

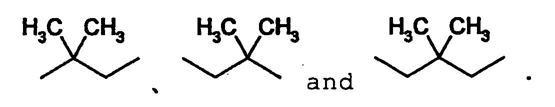

In the present specification, the "C₂₋₆ alkenyl group" refers to a straight chain or branched chain alkenyl group having 2 to 6 carbon atoms, and examples thereof include, but are not limited to, ethenyl group, propenyl group, isopropenyl group, butenyl group, isobutenyl group, sec-butenyl group, pentenyl group, isopentenyl group, neopentenyl group, tert-pentenyl group, 1-ethylpropenyl group, hexenyl group and the like can be mentioned, preferably C₂₋₄ alkenyl group such as ethenyl group, propenyl group, isopropenyl group, butenyl group, isobutenyl group and sec-butenyl group.

In the present specification, the "C₂₋₆ alkynyl group" refers to a straight chain or branched chain alkynyl group having 2 to 6 carbon atoms, and examples thereof include, but are not limited to, ethynyl group, propynyl group, butynyl group, sec-butynyl group, pentynyl group, isopentynyl group, tert-pentynyl group, 1-ethylpropynyl group, hexynyl group and the like, preferably C₂₋₄ alkynyl group such as ethynyl group, propynyl group, butynyl group and sec-butynyl group.

In the present specification, the "C₃₋₈ cycloalkyl group" refers to a cycloalkyl group having 3 to 8 carbon atoms, and examples thereof include, but are not limited to, cyclopropyl group, cyclobutyl group, cyclopentyl group, cyclohexyl group, cycloheptyl group, cyclooctyl group and the like, preferably a cycloalkyl group having 5 or 6 carbon atoms such as cyclopentyl group and cyclohexylgroup and the like.

In the present specification, the "C₃₋₈ cycloalkenyl group" is a cycloalkenyl group having 3 to 8 carbon atoms, and examples thereof include, but are not limited to, cyclopropenyl group, cyclobutenyl group, cyclopentenyl group, cyclohexenyl group, cycloheptenyl group, cyclooctenyl group and the like, preferably a cycloalkenyl group having 5 or 6 carbon atoms such as cyclopentenyl group, cyclohexenyl group and the like.

In the present specification, the "C₆₋₁₄ aryl group" refers to an aromatic hydrocarbon group having 6 to 14 carbon atoms, and examples thereof include, but are not limited to, phenyl group, naphthyl group, biphenylyl group (e.g., 2-biphenylyl group, 3-biphenylyl group, 4-biphenylyl group and the like), anthryl group and the like, preferably phenyl group.

In the present specification, the "C₇₋₂₀ aralkyl group" refers to a group formed by bonding the aforementioned "C₆₋₁₄ aryl group" to the aforementioned "C₁₋₆ alkyl group", and examples thereof include, but are not limited to, benzyl group, phenethyl group, phenylbutyl group, phenylpropyl group, phenylpentyl group, phenylhexyl group, naphthylmethyl group, anthrylmethyl group, biphenylylmethyl group and the like, preferably benzyl group.

In the present specification, the "C₇₋₂₀ aralkyloxy group" refers to a group formed by bonding the aforementioned "C₇₋₂₀ aralkyl group" to an oxygen atom, and examples thereof include, but are not limited to, benzyloxy group, phenethyloxy group, phenylbutyloxy group, phenylpropyloxy group, phenylpentyloxy group, phenylhexyloxy group, naphthylmethyloxy group, anthrylmethyloxy group, biphenylylmethyloxy group and the like, preferably benzyloxy group.

In the present specification, the "C₆₋₁₄ aryl-oxy group" refers to a group formed by the aforementioned "C₆₋₁₄ aryl group" to an oxygen atom, and examples thereof include, but are not limited to, phenoxy group, naphthyloxy group, anthryloxy group, biphenylyloxy group and the like, preferably phenoxy group.

In the present specification, the "C₆₋₁₄ aryl-carbonyl group" refers to a group formed by bonding the aforementioned "C₆₋₁₄ aryl group" to a carbonyl group, and examples thereof include, but are not limited to, benzoyl group, naphthalenecarbonyl group, anthracenecarbonyl group, biphenylcarbonyl group and the like, preferably benzoyl group.

In the present specification, the "monocyclic aryl group" refers to a monocyclic aromatic hydrocarbon group having 6 to 14 carbon atoms, and examples thereof include, but are not limited to, phenyl group and the like.

In the present specification, examples of the "C₁₋₇ acyl group" are formyl group; C₁₋₆ alkyl-carbonyl group such as acetyl group, propionyl group, butyryl group and pivaloyl group; C₂₋₆ alkenyl-carbonyl group such as ethenoyl group, propenoyl group and butenoyl group; aroyl group such as benzoyl group, and the like, preferably acetyl group.

In the present specification, the "C₁₋₇ acyloxy group" refers to a group formed by bonding the aforementioned "C₁₋₇ acyl group" to an oxygen atom, and examples thereof include formyloxy group; C₁₋₆ alkyl-carbonyloxy group such as acetyloxy group, propionyloxy group, butyryloxy group and pivaloyloxy group; C₂₋₆ alkenyl-carbonyloxy group such as ethenoyloxy group, propenoyloxy group and butenoyloxy group; aroyloxy group such as benzoyloxy group, and the like, preferably acetyloxy group.

In the present specification, the "C₁₋₇ acylamino group" refers to a group formed by bonding the aforementioned "C₁₋₇ acyl group" to an amino group, and examples thereof include formylamino group; C₁₋₆ alkylcarbonylamino group such as acetylamino group, propionylamino group, butyrylamino group and pivaloylamino group; C₂₋₆ alkenyl-carbonylamino group such as ethenoylamino group, propenoylamino group and butenoylamino group; aroylamino group such as benzoylamino group, and the like, preferably acetylamino group.

In the present specification, the "(C₁₋₇ acyl)(C₁₋₆ alkyl)amino group" refers to a group formed by bonding the the aforementioned "C₁₋₇ acyl group" and "C₁₋₆ alkyl group" to an amino group, and examples thereof include formylmethylamino group and ethylformylamino group; (C₁₋₆ alkyl)(C₂₋₆ alkenyl-carbonyl)amino group such as acetylmethylamino group, acetylethylamino group, methylpropionylamino group, ethylpropionylamino group, butyrylmethylamino group, butyrylethylamino group, methylpivaloylamino group, ethylpivaloylamino group and the like;ethenoylmethylamino group, ethenoylethylamino group, methylpropenoylamino group, ethylpropenoylamino group, butenoylmethylamino group and butenoylethylamino group; (C₁₋₆ alkyl)aroylamino group such as benzoylmethylamino group and benzoylethylamino, and the like.

In the present specification, the "4- to 7-membered heterocyclic group" refers to a saturated or unsaturated 4- to 7-membered heterocyclic group containing 1 to 4 hetero atoms selected from the group consisting of a nitrogen atom, an oxygen atom and a sulfur atom, and examples thereof include azetidinyl group, pyrrolidinyl group, piperidinyl group, tetrahydrofuryl group, tetrahydropyranyl group, tetrahydrothienyl group, oxazolidinyl group, thiazolidinyl group, imidazolidinyl group, pyrazolidinyl group, piperazinyl group, morpholinyl group, dioxanyl group, furyl group, thienyl group, pyrrolyl group, pyridyl group, oxazolyl group, isoxazolyl group, thiazolyl group, isothiazolyl group, imidazolyl group, pyrazolyl group, oxadiazolyl group, thiadiazolyl group, triazolyl group, pyridazinyl group, pyrimidinyl group, pyrazinyl group, and the like, preferably azetidinyl group, pyrrolidinyl group, piperidinyl group, piperazinyl group, morpholinyl group, pyrrolyl group, thienyl group, oxazolyl group, oxadiazolyl group, pyridyl group, pyridazinyl group and pyrazinyl group.

In the present specification, the "5- to 14-membered aromatic condensed heterocyclic group" refers to a 5- to 14-membered aromatic condensed heterocyclic group containing 1 to 4 hetero atoms selected from the group consisting of a nitrogen atom, an oxygen atom and a sulfur atom", and examples thereof include indolyl group, isoindolyl group, benzofuranyl group, benzodioxolyl group, benzodioxinyl group, benzothienyl group, benzimidazolyl group, benzothiazolyl group, benzoxazolyl group, indolizinyl group, quinolyl group, isoquinolyl group, quinazolinyl group, cinnolinyl group, quinoxalinyl group, phthalazinyl group, acrydinyl group, phenazinyl group, naphthyridinyl group and the like, preferably benzofuranyl group, benzodioxolyl group and benzodioxinyl group.

In the present specification, the "nitrogen-containing 5- or 6-membered heterocyclic group" refers to a 5- or 6-membered heterocyclic group containing one nitrogen atom, which optionally further contains 1 to 3 hetero atoms selected from the group consisting of a nitrogen atom, an oxygen atom and a sulfur atom, and examples thereof include pyrrolidino group, piperidinyl group, piperazinyl group and morpholinyl group and the like, preferably pyrrolidino group, piperidinyl group and morpholinyl group.

In the present specification, the "nitrogen-containing 4- to 7-membered heterocyclic group" refers to a 4- to 7-membered heterocyclic group containing one nitrogen atom, which optionally further contains 1 to 3 hetero atoms selected from the group consisting of a nitrogen atom, an oxygen atom and a sulfur atom, and examples thereof include azetidinyl group, oxazatidinylgroup, thiazetidinyl group, pyrrolidinyl group, oxazolidinyl group, thiazolidinyl group, isoxazolidinyl group, isothiazolidinyl group, piperidinyl group, piperazinyl group, morpholinyl group, thiomorpholinyl group, oxazinanyl group, thiazinanyl group, azepanyl group and the like, preferably azetidin-1-yl group, pyrrolidin-1-yl group, piperidin-1-yl group, azepan-1-yl group, piperazin-1-yl group, morpholin-4-yl group, thiomorpholin-4-yl group and [1,3]oxazinan-3-yl group, more preferably azetidin-1-yl group and pyrrolidin-1-yl group.

When the "nitrogen-containing 4- to 7-membered heterocyclic group " is optionally substituted by an oxo group, examples of the nitrogen-containing 4- to 7-membered heterocyclic group substituted by an oxo group include, but are not limited to, 2-oxo-azetidinyl group, 3-oxo-azetidinyl group, 2-oxo-pyrrolidinyl group, 2-oxo-pyrrolidinyl group, 2-oxo-piperidinyl group, 3-oxo-piperidinyl group, 4-oxo-piperidinyl group, 2-oxo-piperazinyl group, 3-oxo-piperazinyl group, 3-oxo-morpholinyl group, 2-oxo-morpholinyl group, 1-oxo-1λ⁴-thiomorpholinyl group, 1,1-dioxo-1λ⁶-thiomorpholinyl group, 2-oxo-[1,3]oxazinanyl group and the like, preferably

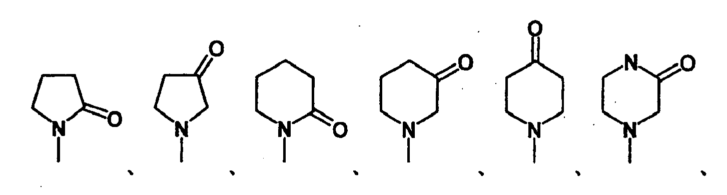

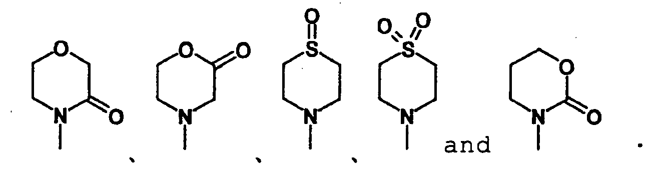

In the present specification, the "heteroaryl group" refers to a monocycle or condensed aromatic heterocyclic group containing 1 to 4 hetero atoms selected from the group consisting of a nitrogen atom, an oxygen atom and a sulfur atom, preferably a 4- to 7-membered heterocyclic group or a fused ring formed by a 4- to 7-membered heterocyclic group and a benzene ring, and examples thereof include furyl group, thienyl group, pyrrolyl group, oxazolyl group, isoxazolyl group, thiazolyl group, isothiazolyl group, imidazolyl group, pyrazolyl group, oxadiazolyl group, thiadiazolyl group, triazolyl group, pyranyl group, pyridyl group, pyridazinyl group, pyrimidinyl group, pyrazinyl group, benzofuranyl group, benzothienyl group, indolyl group, isoindolyl group, benzoxazolyl group, benzothiazolyl group, benzimidazolyl group, indolizinyl group, quinolyl group, isoquinolyl group, quinazolinyl group, cinnolinyl group, phthalazinyl group, quinoxalinyl group, naphthyridinyl group, acrydinyl group, phenazinyl group and the like, preferably furyl group, thienyl group, pyrrolyl group, oxazolyl group, isoxazolyl group, thiazolyl group, pyrazolyl group, oxadiazolyl group, thiadiazolyl group, triazolyl group, pyridyl group, pyridazinyl group, pyrazinyl group, benzofuranyl group and benzoxazolyl group.

When the "heteroaryl group" is optionally substituted by an oxo group, examples of the heteroaryl group optionally substituted by an oxo group include 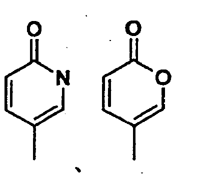 and the like.

In the present specification, the "monocyclic heteroaryl group" refers to a monocyclic aromatic heterocyclic group containing 1 to 4 hetero atoms selected from the group consisting of a nitrogen atom, an oxygen atom and a sulfur atom monocyclic aromatic heterocyclic group, preferably a 4- to 7-membered heterocyclic group, more preferably a 5- or 6-membered heterocyclic group, and examples thereof include furyl group, thienyl group, pyrrolyl group, oxazolyl group, isoxazolyl group, thiazolyl group, isothiazolyl group, imidazolyl group, pyrazolyl group, oxadiazolyl group, thiadiazolyl group, triazolyl group, pyranyl group, pyridyl group, pyridazinyl group, pyrimidinyl group, pyrazinyl group and the like, preferably furyl group, thienyl group, pyrrolyl group, oxazolyl group, isoxazolyl group, thiazolyl group, pyrazolyl group, oxadiazolyl group, thiadiazolyl group, triazolyl group, pyridyl group, pyridazinyl group and pyrazinyl group.

When the "monocyclic heteroaryl group" is optionally substituted by an oxo group, examples of the monocyclic heteroaryl group substituted by an oxo group include 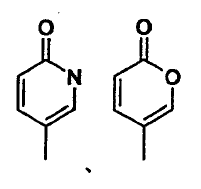 and the like.

In the present specification, the "fused ring" refers to a condensed type hydrocarbon group or heterocyclic group optionally containing 1 to 4 hetero atoms selected from the group consisting of a nitrogen atom, an oxygen atom and a sulfur atom, preferably a fused ring group formed by a 4- to 7-membered ring and benzene, more preferably a fused ring group formed by a 5- or 6-membered heterocyclic group ring and benzene, and examples thereof include naphthyl group, indolyl group, isoindolyl group, benzofuranyl group, benzodioxolyl group, benzodioxinyl group, dihydrobenzodioxinyl group, benzothienyl group, benzimidazolyl group, benzothiazolyl group, benzoxazolyl group, indolizinyl group, quinolyl group, isoquinolyl group, quinazolinyl group, cinnolinyl group, quinoxalinyl group, phthalazinyl group, acrydinyl group, phenazinyl group, naphthyridinyl group and the like, preferably benzofuranyl group, benzodioxolyl group and dihydrobenzodioxinyl group.

In the present specification, the "heterocycloalkyl group" refers to a saturated 4- to 7-membered heterocyclic group containing 1 to 4 hetero atoms selected from the group consisting of a nitrogen atom, an oxygen atom and a sulfur atom, and examples thereof include azetidinyl group, pyrrolidinyl group, piperidinyl group, tetrahydrofuryl group, tetrahydropyranyl group, tetrahydrothienyl group, oxazolidinyl group, thiazolidinyl group, imidazolidinyl group, pyrazolidinyl group, dioxolanyl group, piperazinyl group, morpholinyl group, dioxanyl group and the like, preferably [1,3]dioxoran-2-yl group and tetrahydropyran-4-yl group.

In the present specification, the "bond" refers to a direct connection. For example, when L₂ is a "bond" in the group -J₁-R¹⁰¹ wherein R¹⁰¹ is -L₂-J₂-R¹⁰³, the group represents -J₁-J₂-R¹⁰³. Namely, in said case, where J₁ is -CO-, L₂ is a "bond", J₂ is -O- and R¹⁰³ is a hydrogen atom, the group represents -COOH (carboxyl group).

In the present specification, the "C₁₋₆ haloalkyl group" refers to a straight chain or branched chain alkyl group having 1 to 6 carbon atoms substituted by the "halogen atom" as defined above, and specific examples thereof include fluoromethyl group, difluoromethyl group, trifluoromethyl group, bromomethyl group, chloromethyl group, 1,2-dichloroethyl group, 2,2-dichloroethyl group, 2,2,2-trifluoroethyl group and the like.

In the present specification, the "C₁₋₆ haloalkyloxy group" refers to a group formed by bonding the aforementioned "C₁₋₆ haloalkyl group" to an oxygen atom, and specific examples include fluoromethyloxy group, difluoromethyloxy group, trifluoromethyloxy group, bromomethyloxy group, chloromethyloxy group, 1,2-dichloroethyloxy group, 2,2-dichloroethyloxy group, 2,2,2-trifluoroethyloxy group and the like.

In the present specification, the symbol 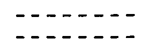
in the formulas 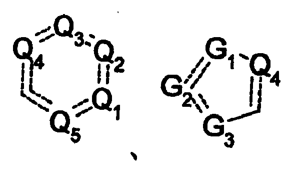 and the like, means a single bond or a double bond, and either of them is appropriately selected in accordance with the substituent selected for each of the symbols. Furthermore, when the substituent selected for each of the symbols is -N=, -C= or the like, the order of single bond and double may be from a single bond to a double bond, or from a double bond to a single bond. Namely, for example, when Q₁ is -N=,

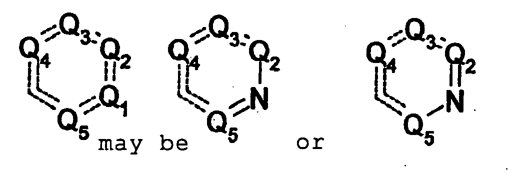

Furthermore, from a chemical viewpoint, the following partial structures 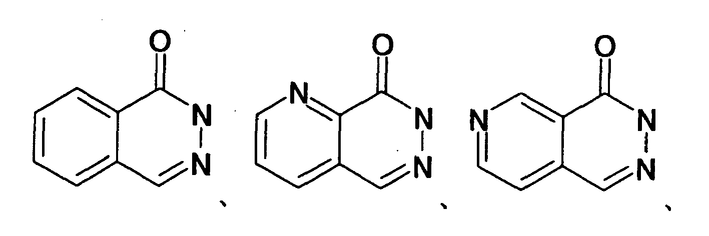 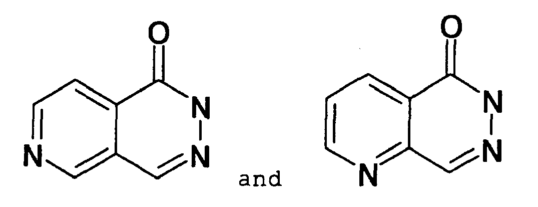 are similar to the following strucutural formulas: 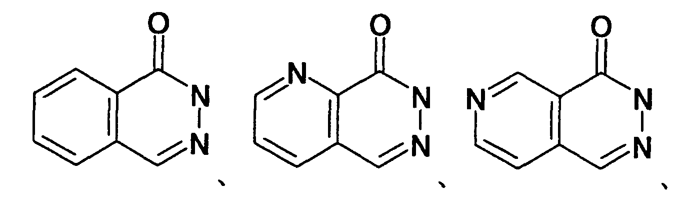 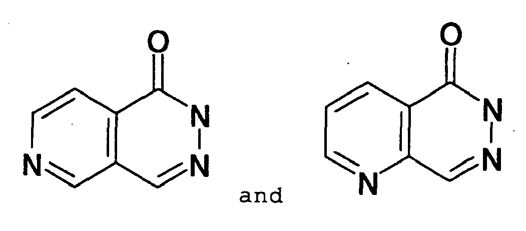 , respectively.

Accordingly, all the compounds having any of the aforementioned partial structures and having other portions as defined in the formula [1] are encompassed in the heterocyclic compound of the present invention.

The "salt acceptable for pharmaceutical agent" or "pharmaceutically acceptable salt" may be any salt so long as it forms a non-toxic salt with the aforementioned compound [1], and examples thereof can be obtained by reaction with an inorganic acid such as hydrochloric acid, sulfuric acid, phosphoric acid and hydrobromic acid; or with an organic acid such as oxalic acid, malonic acid, citric acid, fumaric acid, lactic acid, malic acid, succinic acid, tartaric acid, acetic acid, trifluoroacetic acid, gluconic acid, ascorbic acid, methylsulfonic acid and benzylsulfonic acid; or with an inorganic base such as sodium hydroxide, potassium hydroxide, calcium hydroxide, magnesium hydroxide and ammonium hydroxide; or with an organic base such as methylamine, diethylamine, triethylamine, triethanolamine, ethylenediamine, tris(hydroxymethyl)methylamine, guanidine, chorine, cinchonine and N-methyl-D-glucamine; or an amino acid such as lysine, histidine, arginine and alanine. Hydrous forms or hydrates and solvates of the compounds are also encompassed in the present invention.

Furthermore, there are various isomers of the compound [1]. For example, E-form and Z-form exist as geometric isomers, and when an asymmetric carbon atom exists, enantioisomer and diastereomer exist as stereoisomers based on the aforementioned forms. When axis-asymmetry exists, stereoisomers based on axis-asymmetry also exist. In some case, tautomers may exist. Accordingly, all of these isomers and mixtures thereof are encompassed in the scope of the present invention.

Prodrugs and metabolites of the compounds are also encompassed in the present invention.

The "prodrug" refers to a derivative of the compound [1] of the present invention having a group that can be decomposed chemically or metabolically, which is administrated to a living organism and converted to the original compound [1] to show its efficacy, and comprises complexes and salts formed not by a covalent bond.

The prodrug is used, for example, for improving absorption during oral administration, or for targeting a target moiety. Examples of the moiety for modification include highly reactive functional groups such as hydroxyl group, carboxyl group, amino group and thiol group in the compound [1] of the present invention.

When the compound [1] of the present invention is used for a pharmaceutical preparation, it can be administered systemically or topically, and orally or parenterally in the form of tablet, pill, powder, glanule, suppository, injection, eye drop, liquid, capsule, troche, aerosol, elixir, suspension, emulsion, syrup and the like, which are prepared by admixing the compound with a pharmaceutically acceptable carrier, an excipient, a diluent, an extending agent, a disintegrant, a stabilizer, a preservative, a buffer, an emulsifier, a flavoring agent, a coloring agent, a sweetening agent, a viscous agent, a corrigent, a dissolution aids, other additives, specifically water, vegetable oil, alcohol such as ethanol and benzyl alcohol, polyethylene glycol, glyceroltriacetate, gelatin, lactose, carbon hydride such as starch, magnesium stearate, talc, lanorin, vaserine, etc. according to a conventional method.

While the dose varies depending on age, body weight, condition, treatment effect, administration method and the like, a dose in the range of 0.01 mg to 3 g per one dose is generally administered once or several times a day per one adult.

The "prophylaxis of hepatitis C" refers to, for example, administration of a pharmaceutical agent to a person who has been examined to have HCV but has not developed the condition of hepatitis C yet, or administration of a pharmaceutical agent to a person who had a treatment of hepatitis C and the condition of hepatitis was remedied, but HCV has not been eradicated and recurrence of hepatitis is foreseen.

The "inhibition of HCV entry" refers to inhibition of any of the processes of HCV infection, i.e., adhesion of HCV on cell surface (also referred to as adsorption), invasion of HCV into cell and uncapsidation of HCV.

By the inhibition, growth of virus is suppressed. Therefore, it is expected that the amount of virus in a living organism (in tissue cell, blood) can be decreased.

The therapeutic agent for hepatitis C of the present invention can be expected to exhibit a synergistic effect by concomitant use with at least one pharmaceutical agent selected from antiviral agents, anti-inflammatory agents and immunity enhancing agents. Examples of such pharmaceutical agent that can be expected to show a synergistic effect include interferons (e.g., interferon-α, interferon-β, interferon-γ), interleukins (e.g., interleukin-2, interleukin-8, interleukin-10, interleukin-12), TNFα or recombinant, modified form or agonist thereof, and various antibodies, vaccines, ribozymes, antisense nucleotides and the like.

As shown in the concomitant use therapy (also referred to as cocktail therapy) of anti-HIV agents, and the like, concomitant use of various antiviral agents against virus containing various mutant genes can be expected to show effect to suppress emergence and increase of drug resistance virus.

Examples of such concomitant use include concomitant use of two or three or more agents, such as a combination of the compound [1] of the present invention and HCV-IRES inhibitor, HCV-NS3 protease inhibitor, HCV-NS2NS3 protease inhibitor, HCV-NS5A inhibitor, HCV polymerase inhibitor and the like, for example, concomitant use with Ribavirin (R), interferon-α (IFN-α, Roferon (R), Intron A (R), Sumiferon (R), MultiFeron (R), Infergen (R), Omniferon (R), Pegasys (R), PEG-Intron A (R)), interferon-β (Frone (R), Rebif (R), AvoneX (R), IFNβMOCHIDA (R)), interferon-ω, 1-β-L-ribofuranosyl-1H-1,2,4-triazole-3-carboxamide, 16α-bromo-3β-hydroxy-5α-androstan-17-one, 1H-imidazole-4-ethanamidedihydrochloride, HCVribozymeHeptazyme(R), polyclonalantibodyCivacir(R), lactoferrin GPX-400, (1S,2R,8R,8aR)-1,2,8-trihydroxyoctahydroindolizinium chloride, HCV vaccine (MTH-68/B, Innivax C(R), Engerix B (R)), antisense oligonucleotide ISIS-14803, HCV-RNA transcription enzymeinhibitor VP-50406, tetrachlorodecaoxide (high concentration Oxoferin (R)), (S)-N-3-[3-(3-methoxy-4-oxazol-5-ylphenyl)ureido]benzylcarbamic acid tetrahydrofuran-3-yl ester, 4-amino-2-ethoxymethyl-α,α-dimethyl-1H-imidazo[4,5-c]quinoline-1-ethanol, interleukin-2 (Proleukin (R)), thymosinα1 and the like. (R) refers to a trade name.

Further examples of such concomitant use include concomitant use of the compound [1] of the present invention with the compounds disclosed in JP-A-8-268890, JP-A-10-101591, JP-A-7-69899 and WO99/61613 as HCV-IRES inhibitors; with the compounds disclosed in WO98/22496, WO99/7733, WO99/7734, WO00/9543, WO00/9558, WO01/59929, WO98/17679, EP932617, WO99/50230, WO00/74768, WO97/43310, US5990276, WO01/58929, WO01/77113, WO02/8198, WO02/8187, WO02/8244, WO02/8256, WO01/7407, WO01/40262, WO01/64678, WO98/46630, JP-A-11-292840, JP-A-10-298151, JP-A-11-127861, JP-A-2001-103993, WO98/46597, WO99/64442, WO00/31129, WO01/32961, WO93/15730, US 7832236, WO00/200400, WO02/8251, WO01/16379 and WO02/7761 as HCV protease inhibitors; with the compounds disclosed in WO97/36554, US5830905, WO97/36866, US 5633388, WO01/7027 and WO00/24725 as HCV helicase inhibitors; with the compounds disclosed in WO00/10573, WO00/13708, WO00/18231, WO00/6529, WO02/6246, WO01/32153, WO01/60315, WO01/77091, WO02/4425, WO02/20497 and WO00/4141 as HCV polymerase inhibitors; and with the compounds disclosed in WO01/58877, JP-A-11-180981 and WO01/12214 as interferon agonists or enhancers, and the like.

In the case of concomitant administration, the compound [1] of the present invention can be administered simultaneously with a pharmaceutical agent used in combination (hereinafter referred to be a concomitant drug) or at time intervals. The compound [1] of the present invention can be administered in the form of a pharmaceutical composition containing a concomitant drug. Alternatively, a pharmaceutical composition containing the compound [1] of the present invention and a pharmaceutical composition containing a concomitant drug can be administered separately. The administration route of the compound [1] of the present invention and that of the concomitant drug may be the same or different.

In the case of concomitant administration, the compound [1] of the present invention can be administered by an amount of 0.1 mg to 1 g per a dose once or several times a day. Alternatively, a smaller amount may be administered. The concomitant drug can be administered at a dose generally used for the prophylaxis or treatment of hepatitis C, for example, in an amount of of 0.1 mg to 1 g per dose. Alternatively, a smaller dose may be administered.

Hereinafter the preferable embodiments of the present invention are explained in detail.

In the following structure of the compound of the formula [1] 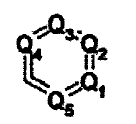 wherein Q₁-Q₅ are each as defined in the aforementioned [1] (hereinafter referred to as "ring 1"),
Q₁ is preferably -N=, -CH= or -CO-, more preferably -N= or -CH=,
Q₃ is preferably -N=, -C(R⁴⁹)= wherein R⁴⁹ is preferably a hydrogen atom, a hydroxyl group or a C₁₋₆ alkoxy group, or -CO-, more preferably -CH= or -CO-, more preferably -CO-, and
Q₄ is preferably -N- or -CH=.

The number of nitrogen atoms in ring 1 is preferably 1 or 2, more preferably 2.

Examples of Ring 1 are preferably 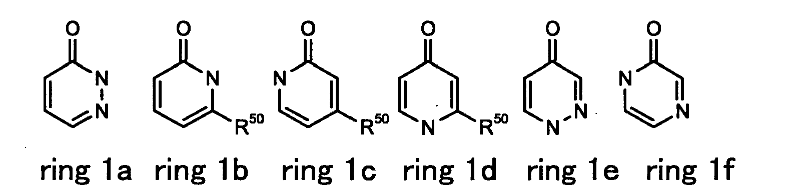 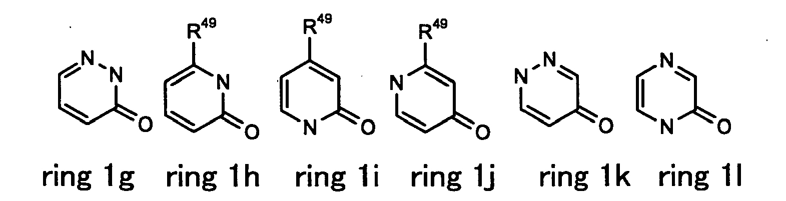 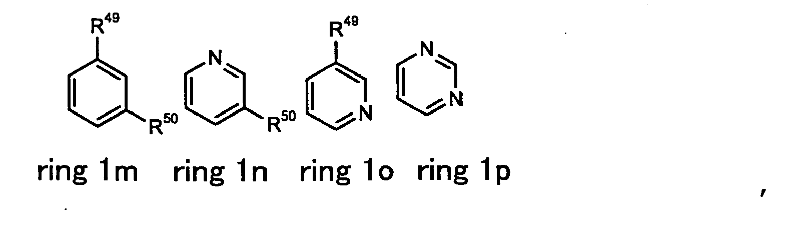 more preferably

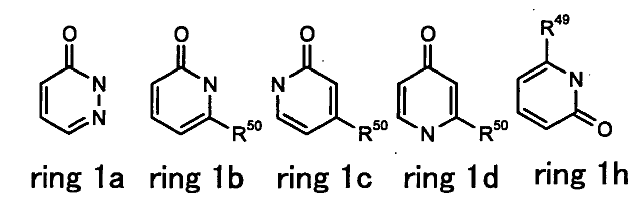

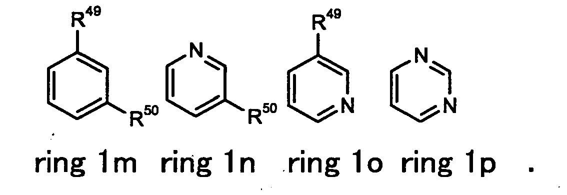

More preferable equivalent embodiments are 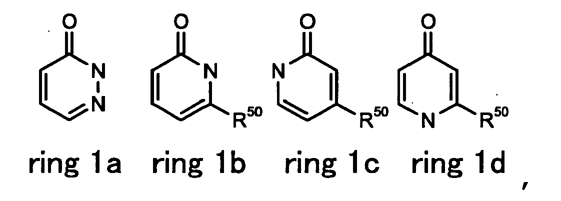 more preferable equivalent embodiments are 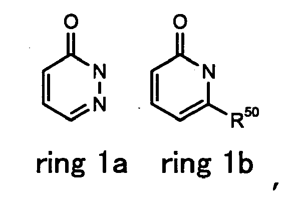 and
more preferable equivalent embodiments are

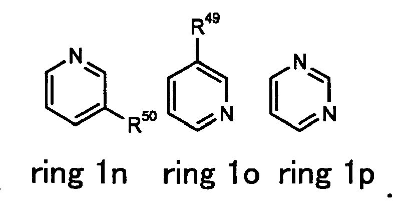

In the aforementioned rings 1a-1p, R⁴⁹ and R⁵⁰ are each as defined in the aforementioned [1].

In the following ring, which is formed by R¹ and R² and Q₄ and the carbon atom to which they are bonded, 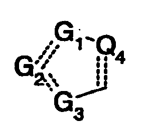 wherein G₁-G₃ are as defined in the aforementioned [1] (hereinafter referred to as ring 2),
G₁ is preferably -N(R³)-, -C(R³)(R⁴)-, -C(R³)= or -N=, wherein R³ and R⁴ are as defined in the aforementioned [1],
G₂ is preferably -C(R^{3'})(R^{4'})-, -C(R^{3'})(R^{4'})-C(R^{5'})(R^{6'})-, -C(R^{4'})=, =C(R^{4'})-C(R^{6'})=, =C(R^{4'})-N= or =N-C(R^{6'})=, wherein R^{3'}, R^{4'}, R^{5'} and R^{6'} are as defined in the aforementioned [1],
G₃ is preferably -C(R^{3''})(R^{4''})-, -C(R^{3''})(R^{4''})-C(R^{5''})(R^{6''})-, -S-, -O-, =C(R^{4''})- or =N-, wherein R^{3''}, R^{4''}, R^{5''} and R^{6''} are as defined in the aforementioned [1]).

In ring 2, G₁, G₂ and G₃ are preferably all carbon atoms, or one of them is an oxygen atom, a sulfur atom or a nitrogen atom, more preferably a nitrogen atom.

Examples of Ring 2 are preferably 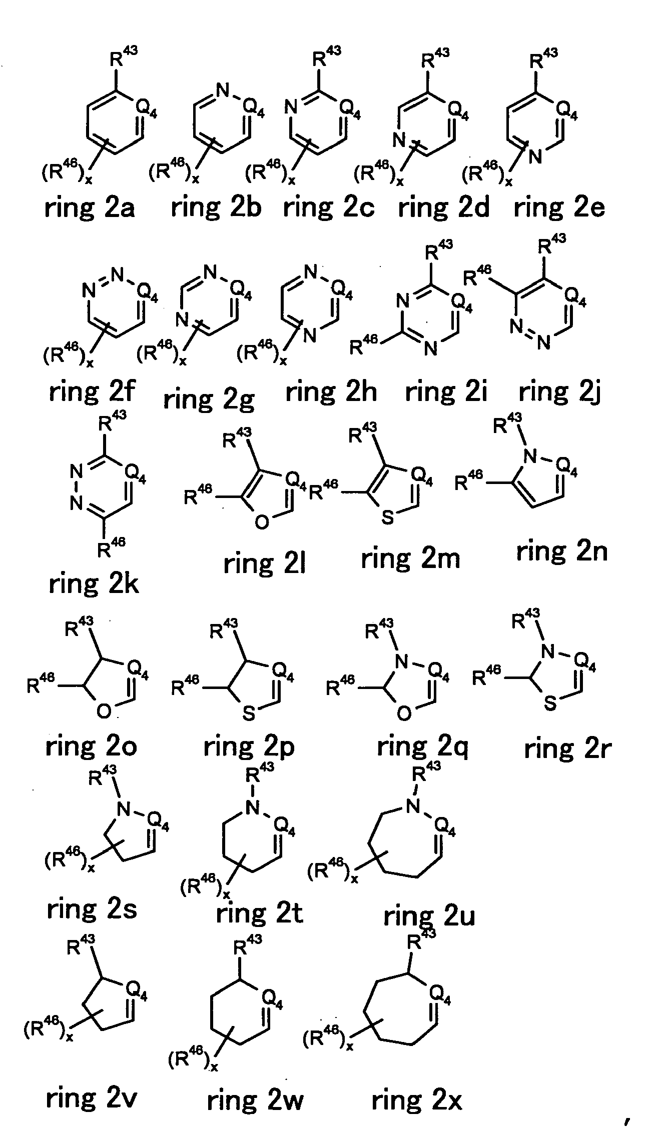 more preferably 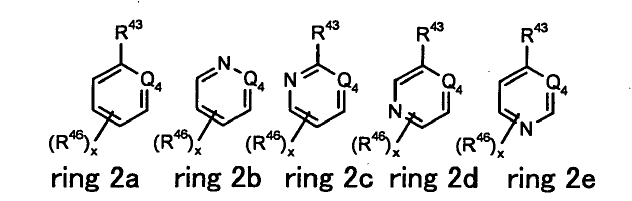 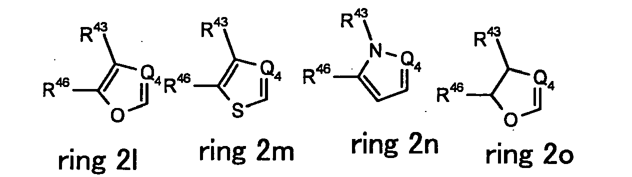 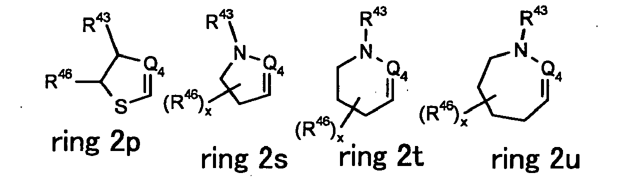 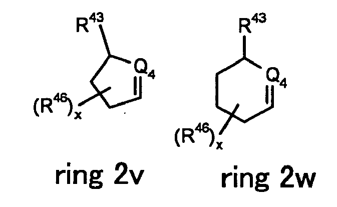 further more preferably

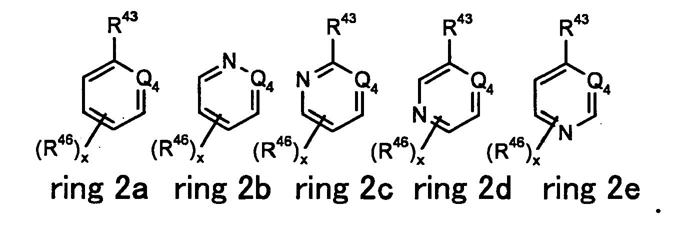

More preferable equivalent embodiments are 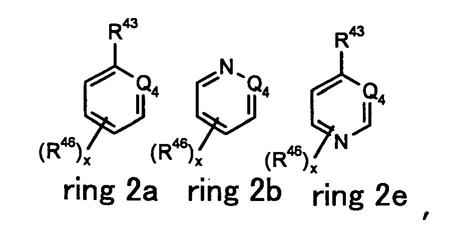 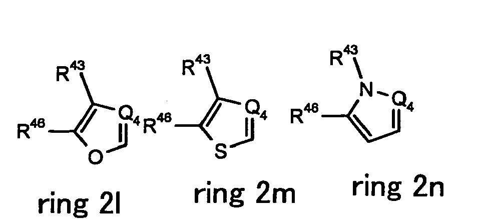 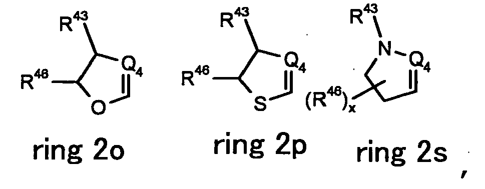 more preferable equivalent embodiments are 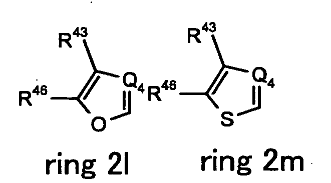 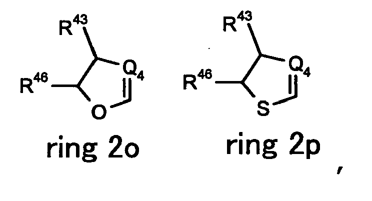 more preferable equivalent embodiments are 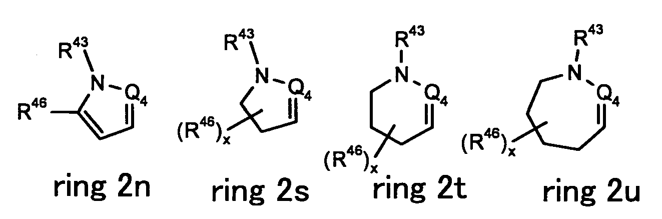 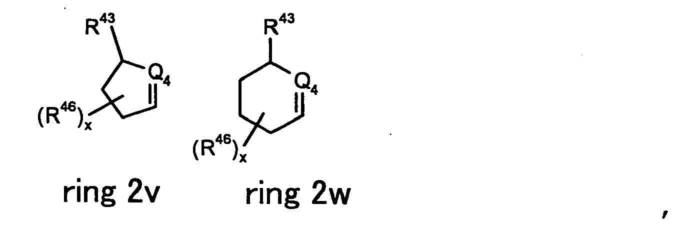 more preferable equivalent embodiments are

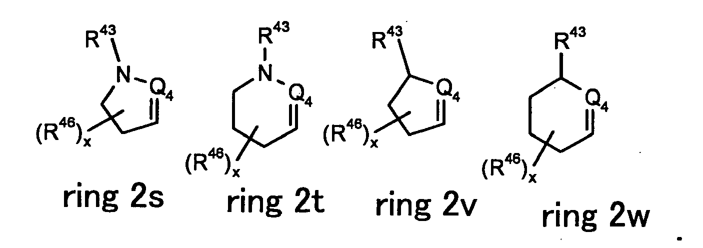

In the aforementioned rings 2a-2w, Q⁴, R⁴³, R⁴⁶ and x are as defined in the aforementioned [6].

In the following structure in the formula [1] 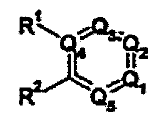 wherein R¹, R² and Q₁-Q₅ are as defined in the aforementioned [1],
preferable combinations of rings 1 and 2 include the following:
rings 1a and 2a, rings 1a and 2b, rings 1a and 2c,
rings 1a and 2d, rings 1a and 2e, rings 1a and 2f,
rings 1a and 2g, rings 1a and 2h, rings 1a and 2i,
rings 1a and 2j, rings 1a and 2k, rings 1a and 21,
rings 1a and 2m, rings 1a and 2n, rings 1a and 2o,
rings 1a and 2p, rings 1a and 2q, rings 1a and 2r,
rings 1a and 2s, rings 1a and 2t, rings 1a and 2u,
rings 1a and 2v, rings 1a and 2w, rings 1a and 2x,
rings 1b and 2a, rings 1b and 2b, rings 1b and 2c,
rings 1b and 2d, rings 1b and 2e, rings 1b and 2f,
rings 1b and 2g, rings 1b and 2h, rings 1b and 2i,
rings 1b and 2j, rings 1b and 2k, rings 1b and 21,
rings 1b and 2m, rings 1b and 2n, rings 1b and 2o,
rings 1b and 2p, rings 1b and 2q, rings 1b and 2r,
rings 1b and 2s, rings 1b and 2t, rings 1b and 2u,
rings 1b and 2v, rings 1b and 2w, rings 1b and 2x,
rings 1c and 2a, rings 1c and 2b, rings 1c and 2c,
rings 1c and 2d, rings 1c and 2e, rings 1c and 2f,
rings 1c and 2g, rings 1c and 2h, rings 1c and 2i,
rings 1c and 2j, rings 1c and 2k, rings 1c and 21,
rings 1c and 2m, rings 1c and 2n, rings 1c and 2o,
rings 1c and 2p, rings 1c and 2q, rings 1c and 2r,
rings 1c and 2s, rings 1c and 2t, rings 1c and 2u,
rings 1c and 2v, rings 1c and 2w, rings 1c and 2x,
rings 1d and 2a, rings 1d and 2b, rings 1d and 2c,
rings 1d and 2d, rings 1d and 2e, rings 1d and 2f,
rings 1d and 2g, rings 1d and 2h, rings 1d and 2i,
rings 1d and 2j, rings 1d and 2k, rings 1d and 21,
rings 1d and 2m, rings 1d and 2n, rings 1d and 2o,
rings 1d and 2p, rings 1d and 2q, rings 1d and 2r,
rings 1d and 2s, rings 1d and 2t, rings 1d and 2u,
rings 1d and 2v, rings 1d and 2w, rings 1d and 2x,
rings 1e and 2a, rings 1e and 2b, rings 1e and 2c,
rings 1e and 2d, rings 1e and 2e, rings 1e and 2f,
rings 1e and 2g, rings 1e and 2h, rings 1e and 2i,
rings 1e and 2j, rings 1e and 2k, rings 1e and 21,
rings 1e and 2m, rings 1e and 2n, rings 1e and 2o,
rings 1e and 2p, rings 1e and 2q, rings 1e and 2r,
rings 1e and 2s, rings 1e and 2t, rings 1e and 2u,
rings 1e and 2v, rings 1e and 2w, rings 1e and 2x,
rings 1f and 2a, rings 1f and 2b, rings 1f and 2c,
rings 1f and 2d, rings 1f and 2e, rings 1f and 2f,
rings 1f and 2g, rings 1f and 2h, rings 1f and 2i,
rings 1f and 2j, rings 1f and 2k, rings 1f and 21,
rings 1f and 2m, rings 1f and 2n, rings 1f and 2o,
rings 1f and 2p, rings 1f and 2q, rings 1f and 2r,
rings 1f and 2s, rings 1f and 2t, rings 1f and 2u,
rings 1f and 2v, rings 1f and 2w, rings 1f and 2x,
rings 1g and 2a, rings 1g and 2b, rings 1g and 2c,
rings 1g and 2d, rings 1g and 2e, rings 1g and 2f,
rings 1g and 2g, rings 1g and 2h, rings 1g and 2i,
rings 1g and 2j, rings 1g and 2k, rings 1g and 21,
rings 1g and 2m, rings 1g and 2n, rings 1g and 2o,
rings 1g and 2p, rings 1g and 2q, rings 1g and 2r,
rings 1g and 2s, rings 1g and 2t, rings 1g and 2u,
rings 1g and 2v, rings 1g and 2w, rings 1g and 2x,
rings 1h and 2a, rings 1h and 2b, rings 1h and 2c,
rings 1h and 2d, rings 1h and 2e, rings 1h and 2f,
rings 1h and 2g, rings 1h and 2h, rings 1h and 2i,
rings 1h and 2j, rings 1h and 2k, rings 1h and 21,
rings 1h and 2m, rings 1h and 2n, rings 1h and 2o,
rings 1h and 2p, rings 1h and 2q, rings 1h and 2r,
rings 1h and 2s, rings 1h and 2t, rings 1h and 2u,
rings 1h and 2v, rings 1h and 2w, rings 1h and 2x,
rings 1i and 2a, rings 1i and 2b, rings 1i and 2c,
rings 1i and 2d, rings 1i and 2e, rings 1i and 2f,
rings 1i and 2g, rings 1i and 2h, rings 1i and 2i,
rings 1i and 2j, rings 1i and 2k, rings 1i and 21,
rings 1i and 2m, rings 1i and 2n, rings 1i and 2o,
rings 1i and 2p, rings 1i and 2q, rings 1i and 2r,
rings 1i and 2s, rings 1i and 2t, rings 1i and 2u,
rings 1i and 2v, rings 1i and 2w, rings 1i and 2x,
rings 1j and 2a, rings 1j and 2b, rings 1j and 2c,
rings 1j and 2d, rings 1j and 2e, rings 1j and 2f,
rings 1j and 2g, rings 1j and 2h, rings 1j and 2i,
rings 1j and 2j, rings 1j and 2k, rings 1j and 21,
rings 1j and 2m, rings 1j and 2n, rings 1j and 2o,
rings 1j and 2p, rings 1j and 2q, rings 1j and 2r,
rings 1j and 2s, rings 1j and 2t, rings 1j and 2u,
rings 1j and 2v, rings 1j and 2w, rings 1j and 2x,
rings 1k and 2a, rings 1k and 2b, rings 1k and 2c,
rings 1k and 2d, rings 1k and 2e, rings 1k and 2f,
rings 1k and 2g, rings 1k and 2h, rings 1k and 2i,
rings 1k and 2j, rings 1k and 2k, rings 1k and 21,
rings 1k and 2m, rings 1k and 2n, rings 1k and 2o,
rings 1k and 2p, rings 1k and 2q, rings 1k and 2r,
rings 1k and 2s, rings 1k and 2t, rings 1k and 2u,
rings 1k and 2v, rings 1k and 2w, rings 1k and 2x,
rings 11 and 2a, rings 11 and 2b, rings 11 and 2c,
rings 11 and 2d, rings 11 and 2e, rings 11 and 2f,
rings 11 and 2g, rings 11 and 2h, rings 11 and 2i,
rings 11 and 2j, rings 11 and 2k, rings 11 and 21,
rings 11 and 2m, rings 11 and 2n, rings 11 and 2o,
rings 11 and 2p, rings 11 and 2q, rings 11 and 2r,
rings 11 and 2s, rings 11 and 2t, rings 11 and 2u,
rings 11 and 2v, rings 11 and 2w, rings 11 and 2x,
rings 1m and 2a, rings 1m and 2b, rings 1m and 2c,
rings 1m and 2d, rings 1m and 2e, rings 1m and 2f,
rings 1m and 2g, rings 1m and 2h, rings 1m and 2i,
rings 1m and 2j, rings 1m and 2k, rings 1m and 21,
rings 1m and 2m, rings 1m and 2n, rings 1m and 2o,
rings 1m and 2p, rings 1m and 2q, rings 1m and 2r,
rings 1m and 2s, rings 1m and 2t, rings 1m and 2u,
rings 1m and 2v, rings 1m and 2w, rings 1m and 2x,
rings 1o and 2a, rings 1o and 2b, rings 1o and 2c,
rings 1o and 2d, rings 1o and 2e, rings 1o and 2f,
rings 1o and 2g, rings 1o and 2h, rings 1o and 2i,
rings 1o and 2j, rings 1o and 2k, rings 1o and 21,
rings 1o and 2m, rings 1o and 2n, rings 1o and 2o,
rings 1o and 2p, rings 1o and 2q, rings 1o and 2r,
rings 1o and 2s, rings 1o and 2t, rings 1o and 2u,
rings 1o and 2v, rings 1o and 2w, rings 1o and 2x,
rings 1p and 2a, rings 1p and 2b, rings 1p and 2c,
rings 1p and 2d, rings 1p and 2e, rings 1p and 2f,
rings 1p and 2g, rings 1p and 2h, rings 1p and 2i,
rings 1p and 2j, rings 1p and 2k, rings 1p and 21,
rings 1p and 2m, rings 1p and 2n, rings 1p and 2o,
rings 1p and 2p, rings 1p and 2q, rings 1p and 2r,
rings 1p and 2s, rings 1p and 2t, rings 1p and 2u,
rings 1p and 2v, rings 1p and 2w, ands 1p and 2x;
more preferable combinations are:
rings 1a and 2a, rings 1a and 2b, rings 1a and 2c,
rings 1a and 2d, rings 1a and 2e, rings 1a and 21,
rings 1a and 2m, rings 1a and 2n, rings 1a and 2o,
rings 1a and 2p, rings 1a and 2s, rings 1a and 2t,
rings 1a and 2u, rings 1a and 2v, rings 1a and 2w,
rings 1b and 2a, rings 1b and 2b, rings 1b and 2c,
rings 1b and 2d, rings 1b and 2e, rings 1b and 21,
rings 1b and 2m, rings 1b and 2n, rings 1b and 2o,
rings 1b and 2p, rings 1b and 2s, rings 1b and 2t,
rings 1b and 2u, rings 1b and 2v, rings 1b and 2w,
rings 1c and 2a, rings 1c and 2b, rings 1c and 2c,
rings 1c and 2d, rings 1c and 2e, rings 1c and 21,
rings 1c and 2m, rings 1c and 2n, rings 1c and 2o,
rings 1c and 2p, rings 1c and 2s, rings 1c and 2t,
rings 1c and 2u, rings 1c and 2v, rings 1c and 2w,
rings 1d and 2a, rings 1d and 2b, rings 1d and 2c,
rings 1d and 2d, rings 1d and 2e, rings 1d and 21,
rings 1d and 2m, rings 1d and 2n, rings 1d and 2o,
rings 1d and 2p, rings 1d and 2s, rings 1d and 2t,
rings 1d and 2u, rings 1d and 2v, rings 1d and 2w,
rings 1h and 2a, rings 1h and 2b, rings 1h and 2c,
rings 1h and 2d, rings 1h and 2e, rings 1h and 21,
rings 1h and 2m, rings 1h and 2n, rings 1h and 2o,
rings 1h and 2p, rings 1h and 2s, rings 1h and 2t,
rings 1h and 2u, rings 1h and 2v, rings 1h and 2w,
rings 1m and 2a, rings 1m and 2b, rings 1m and 2c,
rings 1m and 2d, rings 1m and 2e, rings 1m and 21,
rings 1m and 2m, rings 1m and 2n, rings 1m and 2o,
rings 1m and 2p, rings 1m and 2s, rings 1m and 2t,
rings 1m and 2u, rings 1m and 2v, rings 1m and 2w,
rings 1n and 2a, rings 1n and 2b, rings 1n and 2c,
rings 1n and 2d, rings 1n and 2e, rings 1n and 21,
rings 1n and 2m, rings 1n and 2n, rings 1n and 2o,
rings 1n and 2p, rings 1n and 2s, rings 1n and 2t,
rings 1n and 2u, rings 1n and 2v, rings 1n and 2w,
rings 1o and 2a, rings 1o and 2b, rings 1o and 2c,
rings 1o and 2d, rings 1o and 2e, rings 1o and 21,
rings 1o and 2m, rings 1o and 2n, rings 1o and 2o,
rings 1o and 2p, rings 1o and 2s, rings 1o and 2t,
rings 1o and 2u, rings 1o and 2v, rings 1o and 2w,
rings 1p and 2a, rings 1p and 2b, rings 1p and 2c,
rings 1p and 2d, rings 1p and 2e, rings 1p and 21,
rings 1p and 2m, rings 1p and 2n, rings 1p and 2o,
rings 1p and 2p, rings 1p and 2s, rings 1p and 2t,
rings 1p and 2u, rings 1p and 2v, ands 1p and 2w;
further more preferable combinations are:
rings 1m and 2a, rings 1m and 2b, rings 1m and 2c,
rings 1m and 2d, rings 1m and 2e, rings 1m and 21,
rings 1m and 2m, rings 1m and 2n, rings 1m and 2o,
rings 1m and 2p, rings 1m and 2s, rings 1m and 2t,
rings 1m and 2u, rings 1m and 2v, rings 1m and 2w,
rings 1n and 2a, rings 1n and 2b, rings 1n and 2c,
rings 1n and 2d, rings 1n and 2e, rings 1n and 21,
rings 1n and 2m, rings 1n and 2n, rings 1n and 2o,
rings 1n and 2p, rings 1n and 2s, rings 1n and 2t,
rings 1n and 2u, rings 1n and 2v, rings 1n and 2w,
rings 1o and 2a, rings 1o and 2b, rings 1o and 2c,
rings 1o and 2d, rings 1o and 2e, rings 1o and 21,
rings 1o and 2m, rings 1o and 2n, rings 1o and 2o,
rings 1o and 2p, rings 1o and 2s, rings 1o and 2t,
rings 1o and 2u, rings 1o and 2v, rings 1o and 2w,
rings 1p and 2a, rings 1p and 2b, rings 1p and 2c,
rings 1p and 2d, rings 1p and 2e, rings 1p and 21,
rings 1p and 2m, rings 1p and 2n, rings 1p and 2o,
rings 1p and 2p, rings 1p and 2s, rings 1p and 2t,
rings 1p and 2u, rings 1p and 2v, ands 1p and 2w.

More preferable equivalent embodiments are as follows:
combinations of rings 1m and 2a, rings 1m and 2b, rings 1m and 2c, rings 1m and 2d, rings 1m and 2e, rings 1m and 21, rings 1m and 2m, rings 1m and 2n, rings 1m and 2o, rings 1m and 2p, rings 1m and 2s, rings 1m and 2t, rings 1m and 2u, rings 1m and 2v, rings 1m and 2w, rings 1o and 2a, rings 1o and 2b, rings 1o and 2c, rings 1o and 2d, rings 1o and 2e, rings 1o and 21, rings 1o and 2m, rings 1o and 2n, rings 1o and 2o, rings 1o and 2p, rings 1o and 2s, rings 1o and 2t, rings 1o and 2u, rings 1o and 2v, ands 1o and 2w;
combinations of rings 1n and 2a, rings 1n and 2b, rings 1n and 2c, rings 1n and 2d, rings 1n and 2e, rings 1n and 21, rings 1n and 2m, rings 1n and 2n, rings 1n and 2o, rings 1n and 2p, rings 1n and 2s, rings 1n and 2t, rings 1n and 2u, rings 1n and 2v, rings 1n and 2w, rings 1p and 2a, rings 1p and 2b, rings 1p and 2c, rings 1p and 2d, rings 1p and 2e, rings 1p and 21, rings 1p and 2m, rings 1p and 2n, rings 1p and 2o, rings 1p and 2p, rings 1p and 2s, rings 1p and 2t, rings 1p and 2u, rings 1p and 2v, ands 1p and 2w;
combinations of rings 1m and 2b, rings 1m and 2c, rings 1m and 2d, rings 1m and 2e, rings 1m and 21, rings 1m and 2m, rings 1m and 2n, rings 1n and 2b, rings 1n and 2c, rings 1n and 2d, rings 1n and 2e, rings 1n and 21, rings 1n and 2m, rings 1n and 2n, rings 1o and 2b, rings 1o and 2c, rings 1o and 2d, rings 1o and 2e, rings 1o and 21, rings 1o and 2m, rings 1o and 2n, rings 1p and 2b, rings 1p and 2c, rings 1p and 2d, rings 1p and 2e, rings 1p and 21, rings 1p and 2m, ands 1p and 2n;
combinations of rings 1m and 2o, rings 1m and 2p, rings 1m and 2s, rings 1m and 2t, rings 1n and 2o, rings 1n and 2p, rings 1n and 2s, rings 1n and 2t, rings 1o and 2o, rings 1o and 2p, rings 1o and 2s, rings 1o and 2t, rings 1p and 2o, rings 1p and 2p, rings 1p and 2s, ands 1p and 2t;
combinations of rings 1m and 2u, rings 1m and 2v, rings 1m and 2w, rings 1n and 2u, rings 1n and 2v, rings 1n and 2w, rings 1o and 2u, rings 1o and 2v, rings 1o and 2w, rings 1p and 2u, rings 1p and 2v, and rings 1p and 2w;
combinations of rings 1a and 2a, rings 1a and 2b, rings 1a and 2c, rings 1a and 2d, rings 1a and 2e, rings 1a and 21, rings 1a and 2m, rings 1a and 2n, rings 1a and 2o, rings 1a and 2p, rings 1a and 2s, rings 1a and 2t, rings 1a and 2u, rings 1a and 2v, rings 1a and 2w, rings 1b and 2a, rings 1b and 2b, rings 1b and 2c, rings 1b and 2d, rings 1b and 2e, rings 1b and 21, rings 1b and 2m, rings 1b and 2n, rings 1b and 2o, rings 1b and 2p, rings 1b and 2s, rings 1b and 2t, rings 1b and 2u, rings 1b and 2v, rings 1b and 2w, rings 1c and 2a, rings 1c and 2b, rings 1c and 2c, rings 1c and 2d, rings 1c and 2e, rings 1c and 21, rings 1c and 2m, rings 1c and 2n, rings 1c and 2o, rings 1c and 2p, rings 1c and 2s, rings 1c and 2t, rings 1c and 2u, rings 1c and 2v, rings 1c and 2w, rings 1d and 2a, rings 1d and 2b, rings 1d and 2c, rings 1d and 2d, rings 1d and 2e, rings 1d and 21, rings 1d and 2m, rings 1d and 2n, rings 1d and 2o, rings 1d and 2p, rings 1d and 2s, rings 1d and 2t, rings 1d and 2u, rings 1d and 2v, and rings 1d and 2w;
combinations of rings 1a and 2a, rings 1a and 2b, rings 1a and 2c, rings 1a and 2d, rings 1a and 2e, rings 1a and 21, rings 1a and 2m, rings 1a and 2n, rings 1a and 2o, rings 1a and 2p, rings 1a and 2s, rings 1a and 2t, rings 1a and 2u, rings 1a and 2v, rings 1a and 2w, rings 1b and 2a, rings 1b and 2b, rings 1b and 2c, rings 1b and 2d, rings 1b and 2e, rings 1b and 21, rings 1b and 2m, rings 1b and 2n, rings 1b and 2o, rings 1b and 2p, rings 1b and 2s, rings 1b and 2t, rings 1b and 2u, rings 1b and 2v, and rings 1b and 2w;
combinations of rings 1c and 2a, rings 1c and 2b, rings 1c and 2c, rings 1c and 2d, rings 1c and 2e, rings 1c and 21, rings 1c and 2m, rings 1c and 2n, rings 1c and 2o, rings 1c and 2p, rings 1c and 2s, rings 1c and 2t, rings 1c and 2u, rings 1c and 2v, rings 1c and 2w, rings 1d and 2a, rings 1d and 2b, rings 1d and 2c, rings 1d and 2d, rings 1d and 2e, rings 1d and 21, rings 1d and 2m, rings 1d and 2n, rings 1d and 2o, rings 1d and 2p, rings 1d and 2s, rings 1d and 2t, rings 1d and 2u, rings 1d and 2v, and rings 1d and 2w;
combinations of rings 1a and 2b, rings 1a and 2c, rings 1a and 2d, rings 1a and 2e, rings 1a and 21, rings 1a and 2m, rings 1a and 2n, rings 1b and 2b, rings 1b and 2c, rings 1b and 2d, rings 1b and 2e, rings 1b and 21, rings 1b and 2m, rings 1b and 2n, rings 1c and 2b, rings 1c and 2c, rings 1c and 2d, rings 1c and 2e, rings 1c and 21, rings 1c and 2m, rings 1c and 2n, rings 1d and 2b, rings 1d and 2c, rings 1d and 2d, rings 1d and 2e, rings 1d and 21, rings 1d and 2m, and rings 1d and 2n;
combinations of rings 1a and 2o, rings 1a and 2p, rings 1a and 2s, rings 1a and 2t, rings 1b and 2o, rings 1b and 2p, rings 1b and 2s, rings 1b and 2t, rings 1c and 2o, rings 1c and 2p, rings 1c and 2s, rings 1c and 2t, rings 1d and 2o, rings 1d and 2p, rings 1d and 2s, and rings 1d and 2t;
combinations of rings 1a and 2u, rings 1a and 2v, rings 1a and 2w, rings 1b and 2u, rings 1b and 2v, rings 1b and 2w, rings 1c and 2u, rings 1c and 2v, rings 1c and 2w, rings 1d and 2u, rings 1d and 2v, and rings 1d and 2w;
combinations of rings 1a and 2a, rings 1b and 2a, rings 1c and 2a, rings 1d and 2a, rings 1m and 2a, rings 1n and 2a, rings 1o and 2a, and rings 1p and 2a; combinations of rings 1a and 2a, rings 1b and 2a, rings 1c and 2a, and rings 1d and 2a;
combinations of rings 1m and 2a, rings 1n and 2a, rings 1o and 2a, and rings 1p and 2a; and
combinations of rings 1a and 2a, rings 1a and 2b, rings 1a and 2c, rings 1a and 2d, rings 1a and 2e, rings 1a and 21, rings 1a and 2m, rings 1a and 2n, rings 1a and 2o, rings 1a and 2p, rings 1a and 2s, rings 1a and 2t, rings 1a and 2u, rings 1a and 2v, rings 1a and 2w, rings 1b and 2n, rings 1b and 2s, rings 1b and 2v, rings 1c and 2v, rings 1d and 2v, rings 1h and 2v, rings 1m and 2v, rings 1n and 2v, rings 1o and 2v, and rings 1p and 2v.

Still more preferable combinations include rings 1a and 2s, rings 1a and 2t, rings 1a and 2u, rings 1a and 2o, rings 1a and 2p, rings 1a and 2v, and rings 1a and 2w.

Still more preferable equivalent embodiments are as follows:
combinations of rings 1a and 2a, rings 1a and 2b, rings 1a and 2c, rings 1a and 2d, and rings 1a and 2e; combinations of rings 1a and 2a, rings 1a and 2b, and rings 1a and 2e;
combinations of rings 1a and 2c, and rings 1a and 2d;
combinations of rings 1a and 21, rings 1a and 2m, rings 1a and 2o, and rings 1a and 2p;
combinations of rings 1a and 21, and rings 1a and 2m; combinations of rings 1a and 2o, and rings 1a and 2p;
combinations of rings 1a and 2n, rings 1a and 2s, rings 1a and 2t, rings 1a and 2u, rings 1a and 2v, rings 1a and 2w, rings 1b and 2n, rings 1b and 2s, and rings 1b and 2v;
combinations of rings 1a and 2n, and rings 1b and 2n; combinations of rings 1a and 2s, rings 1a and 2t, rings 1a and 2u, and rings 1b and 2s;
combinations of rings 1a and 2v, rings 1a and 2w, rings 1b and 2s, and rings 1b and 2v;
combinations of rings 1c and 2v, rings 1d and 2v, and rings 1h and 2v;
combinations of rings 1c and 2v, and rings 1d and 2v;
combinations of rings 1m and 2v, rings 1n and 2v, rings 1o and 2v, and rings 1p and 2v;
combinations of rings 1m and 2v, and rings 1o and 2v; and
combinations of rings 1n and 2v, and rings 1p and 2v.

Preferable position of substitution by R⁴⁶ on ring 2 is the carbon atom to which R⁴⁶ can be bonded, which is apart from the carbon or nitrogen atom to which R⁴³ is bonded, by 1 or 2 atom(s), more preferably 1 atom.

In the formula [1], when the formula 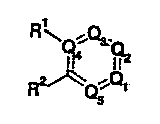 represents a monocycle, preferable embodiments for R¹ (and R⁴¹) and R² (and R⁴²) may be the following.

R¹ is preferably
(1) a hydrogen atom,
(2) a C₁₋₆ alkyl group (said C₁₋₆ alkyl group is optionally substituted by 1 to 3 substituents selected from Group F),
(3) a C₂₋₆ alkenyl group (said C₂₋₆ alkenyl group is optionally substituted by 1 to 3 substituents selected from Group F),
(4) -J₁-R¹⁰¹ wherein J₁ and R¹⁰¹ are as defined for the aforementioned [1]), or
(5) a C₆₋₁₄ aryl group (said C₆₋₁₄ aryl group is optionally substituted by 1 to 5 substituents selected from Group E).

As preferable embodiments of R¹, substituents other than a hydrogen atom can be mentioned.

R¹ is more preferably
(1) a C₁₋₆ alkyl group (said C₁₋₆ alkyl group is optionally substituted by 1 to 3 substituents selected from Group F), and preferable examples of said alkyl moiety include methyl group, ethyl group, propyl group, isopropyl group, n-butyl group, isobutyl group and t-butyl group,
(2) a C₂₋₆ alkenyl group (said C₂₋₆ alkenyl group is optionally substituted by 1 to 3 substituents selected from Group F),
(3) -J₁-R¹⁰¹ wherein J₁ and R¹⁰¹ are as defined for the aforementioned [1], or
(4) a C₆₋₁₄ aryl group (said C₆₋₁₄ aryl group is optionally substituted by 1 to 5 substituents selected from Group E).

R¹ is more preferably -J₁-R¹⁰¹,
wherein
J₁ is preferably
(1) -N(R¹⁰²)- wherein R¹⁰² is a hydrogen atom or a C₁₋₆ alkyl group,
(2) -O-,
(3) -S-,
(4) -CO-,
(5) -CO-O-, or
(6) -CO-N(R¹⁰²)- wherein R¹⁰² is defined for the aforementioned [1],
J₁ is more preferably -N(R¹⁰²)-, -O- or -S-, R¹⁰¹ is preferably
(1) a hydrogen atom,
(2) -L₂-J₂-R¹⁰³, or
(3) a C₁₋₆ alkyl group,
   L₂ is preferably a bond or a C₁₋₆ alkylene group, more preferably a C₁₋₆ alkylene group, more preferably methylene, ethylene or propylene,
J₂ is preferably
(1) -N(R¹⁰⁴)- wherein R¹⁰⁴ is a hydrogen atom or a C₁₋₆ alkyl group,
(2) -O-,
(3) -CO-,
(4) -SO₂-,
(5) -CO-O-,
(6) -CO-N(R¹⁰⁴)- wherein R¹⁰⁴ is as defined for the aforementioned [1], or
(7) -SO₂-N(R¹⁰⁴)- wherein R¹⁰⁴ is as defined for the aforementioned [1],
R¹⁰³ is preferably
(1) a hydrogen atom,
(2) a C₁₋₆ alkyl group, or
(3) a C₁₋₆ alkyl-carbonyl group, or
   R¹⁰¹ and R¹⁰² may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E).

In other preferable embodiment,
R¹ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₁-R¹⁰¹
   wherein
   J₁ is
   (1) -N(R¹⁰²)- wherein R¹⁰² is a hydrogen atom or a C₁₋₆ alkyl group,
   (2) -O-,
   (3) -S-,
   (4) -CO-,
   (5) -CO-O-,
   (6) -O-CO-,
   (7) -CO-N(R¹⁰²)- wherein R¹⁰² is as defined for the aforementioned [1], or
   (8) -SO₂-N(R¹⁰²)- wherein R¹⁰² is as defined for the aforementioned [1],
   R¹⁰¹ is
   (1) a hydrogen atom,
   (2) -L₂-J₂-R¹⁰³
      wherein
      L₂ is
      (1) a bond, or
      (2) a C₁₋₆ alkylene group,
      J₂ is
      (1) -N(R¹⁰⁴)- wherein R¹⁰⁴ is a hydrogen atom or a C₁₋₆ alkyl group,
      (2) -O-,
      (3) -S-,
      (4) -CO-,
      (5) -SO₂-,
      (6) -CO-O-,
      (7) -CO-N(R¹⁰⁴)- wherein R¹⁰⁴ is as defined above, or
      (8) -SO₂-N(R¹⁰⁴)- wherein R¹⁰⁴ is as defined above,
      R¹⁰³ is
      (1) a hydrogen atom,
      (2) a C₁₋₆ alkyl group (said C₁₋₆ alkyl group is optionally substituted by 1 to 3 substituents selected from Group F),
      (3) a C₁₋₆ alkyl-carbonyl group (said C₁₋₆ alkyl-carbonyl group is optionally substituted by 1 to 3 substituents selected from Group F), or
      (4) a C₁₋₆ alkylsulfonyl group (said C₁₋₆ alkylsulfonyl group is optionally substituted by 1 to 3 substituents selected from Group F),
   (3) a C₁₋₆ alkyl group [said C₁₋₆ alkyl group is optionally substituted by 1 to 3 substituents selected from a halogen atom, a cyano group and a nitro group],
   (4) a substituent selected from a halogen atom, a cyano group and a nitro group, provided that Group F does not include a C₆₋₁₄ aryl group, a C₆₋₁₄ aryl-oxy group, a C₆₋₁₄ aryl-carbonyl group, a heteroaryl group, and a nitrogen-containing 4-to 7-membered heterocyclic group formed by R⁹¹ and R⁹² together with the nitrogen atom to which they are bonded, i.e., Group F does not include all cyclic substituents as R¹.

In the formula [1], R⁴⁹ and R⁵⁰ are preferably the same or different and each is a hydrogen atom, a hydroxyl group or a methoxy group.

R⁵⁰ is more preferably a hydrogen atom.

R⁴³ is preferably -L₃-J₃-R¹¹³ wherein each symbol are as defined in the aforementioned [1], or a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E), and a C₃₋₈ cycloalkyl group (said C₃₋₈ cycloalkyl group is optionally substituted by 1 to 5 substituents selected from Group E)].

R⁴³ is more preferably -L₃-J₃-R¹¹³ wherein the symbols in the formula are as defined in the aforementioned [1],
wherein L₃ is preferably
(1) a bond, or
(2) a C₁₋₆ alkylene group,
J₃ is preferably
(1) -N(R¹¹⁴)- wherein R¹¹⁴ is a hydrogen atom or a C₁₋₆ alkyl group,
(2) -O-,
(4) -CO-,
(5) -SO₂-,
(6) -CO-O-, or
(7) -CO-N(R¹¹⁴)- wherein R¹¹⁴ is as defined for the aforementioned [1],
R¹¹³ is preferably
(1) a hydrogen atom,
(2) -L₄-J₄-R¹¹⁵ wherein the symbols are each as defined for the aforementioned [1], or
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E), and a C₃₋₈ cycloalkyl group (said C₃₋₈ cycloalkyl group is optionally substituted by 1 to 5 substituents selected from Group E)], or R¹¹³ and R¹¹⁴ may form, together with the nitrogen atom to which they are bonded, anitrogen-containing 4- to 7-membered heterocyclic group (said nitrogen-containing 4- to 7-membered heterocyclic group is optionally substituted by 1 to 5 substituents selected from Group E).
L₄ is preferably a bond or a C₁₋₆ alkylene group,
J₄ is preferably
(1) -N(R¹¹⁶)- wherein R¹¹⁶ is a hydrogen atom or a C₁₋₆ alkyl group,
(2) -O-, or
(3) -CO-,
R¹¹⁵ is preferably
(1) a hydrogen atom,
(2) a C₁₋₆ alkyl group (said C₁₋₆ alkyl group is optionally substituted by 1 to 3 substituents selected from Group G), or
(3) a C₁₋₆ alkyl-carbonyl group (said C₁₋₆ alkyl-carbonyl group is optionally substituted by 1 to 3 substituents selected from Group G), or
R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E).

In further preferable equivalent, a C₁₋₆ alkyl group for R⁴³ is a methyl group, an ethyl group, a cyanomethyl group or a unsubstituted benzyl group.

x is preferably 1.

R⁴⁶ is preferably a hydrogen atom, a hydroxyl group, a methyl group, an ethyl group or a methoxy group.

Ring A is preferably 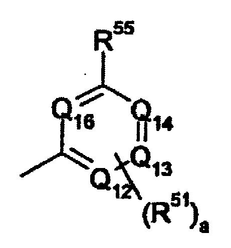 wherein Q₁₂, Q₁₃, Q₁₄ and Q₁₆ are the same or different and each is -N= or -CH=, and other symbols are as defined in the aforementioned [6].

Specific examples of ring A include 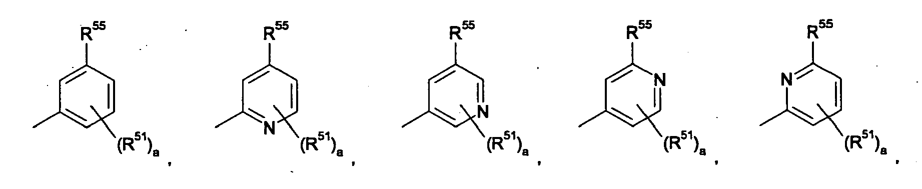 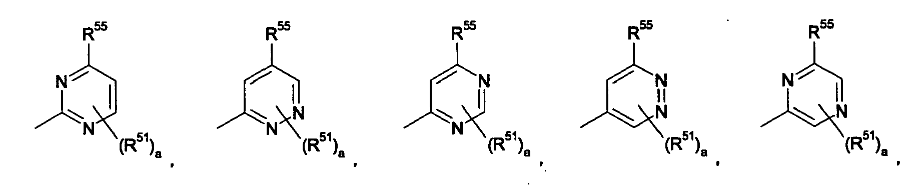 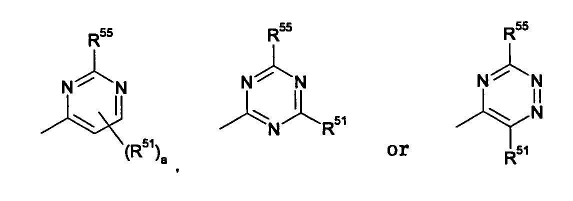 wherein R⁵¹, R⁵⁵ and a are as defined in the aforementioned [6].

Ring A is preferably 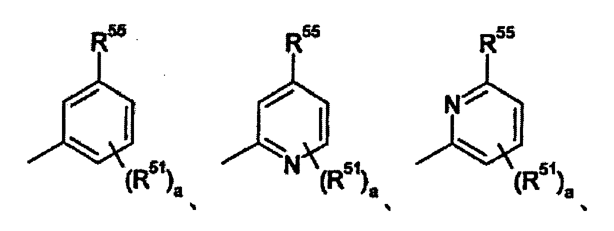 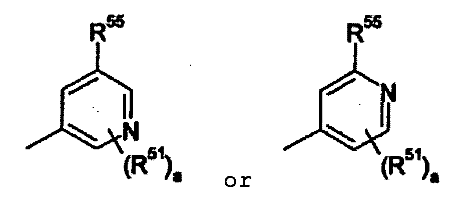 wherein R⁵¹, R⁵⁵ and a are as defined in the aforementioned [6],
more preferably ring A is 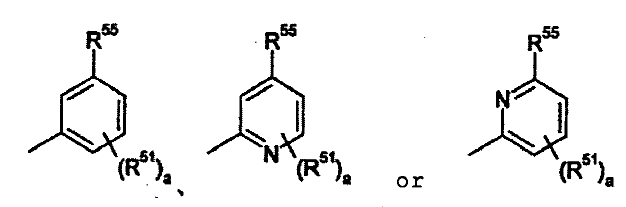 wherein R⁵¹, R⁵⁵ and a are as defined in the aforementioned [6], and
the most preferably, ring A is 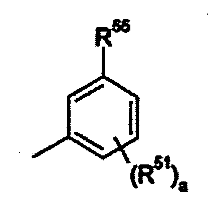 wherein R⁵¹, R⁵⁵ and a are as defined in the aforementioned [6].

In these formulas, R⁵⁵ is preferably
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkynyl group (said C₂₋₆ alkynyl group is optionally substituted by 1 to 3 substituents selected from Group F),
(5) -J₅-R¹²¹ wherein J₅ and R¹²¹ are as defined in the aforementioned [6],
(6) a monocyclic aryl group,
(7) a monocyclic heteroaryl group (said monocyclic heteroaryl group is optionally substituted by 1 to 5 substituents selected from Group E), or
(8) a heterocycloalkyl group.

Preferable embodiments for R⁵⁵ may include substituents other than a hydrogen atom.

R⁵⁵ is more preferably -J₅-R¹²¹ wherein the symbols are each as defined in the aforementioned [6],
wherein
J₅ is preferably
(1) -N(R¹²²)- wherein R¹²² is a hydrogen atom or a C₁₋₆ alkyl group,
(2) -O-,
(3) -CO-,
(4) -CO-O-,
(5) -CO-N(R¹²²)- wherein R¹²² is as defined above, or (6) -N(R¹²³)-CO- wherein R¹²³ is a hydrogen atom or a C₁₋₆ alkyl group,

J₅ is more preferably -N(R¹²²)-, -O- or -N(R¹²³)-CO-, R¹²¹ is preferably a hydrogen atom or -L₆-J₆-R¹²⁴ wherein the symbols are each as defined in the aforementioned [6], or R¹²¹ and R¹²² may form, together with the nitrogen atom to which they are bonded, a nitrogen-containing 4- to 7-membered heterocyclic group (said nitrogen-containing 4- to 7-membered heterocyclic group is optionally substituted with 1 to 5 substituents selected from
(1) Group F,
(2) a C₁₋₆ alkyl group (said C₁₋₆ alkyl group is optionally substituted by 1 to 3 substituents selected from Group F,
(3) a C₁₋₆ alkoxy group (said C₁₋₆ alkoxy group is optionally substituted by 1 to 3 substituents selected from Group F, and
(4) an oxo group),
wherein
L₆ is preferably a bond or a C₁₋₆ alkylene group,
J₆ is preferably
(1) -N(R¹²⁵)- wherein R¹²⁵ is a hydrogen atom or a C₁₋₆ alkyl group,
(2) -O-,
(3) -CO-,
(4) -CO-O-,
(5) -CO-N(R¹²⁵)- wherein R¹²⁵ is as defined above,
(6) -N(R¹²⁶)-CO- wherein R¹²⁶ is a hydrogen atom or a C₁₋₆ alkyl group,
(7) -N(R¹²⁶)-SO₂- wherein R¹²⁶ is as defined above,
(8) -N(R¹²⁶)-CO-O- wherein R¹²⁶ is as defined above, or
(9) -N(R¹²⁶)-CO-N(R¹²⁵)- wherein R¹²⁵ and R¹²⁶ are as defined above,
R¹²⁴ is preferably a hydrogen atom or a C₁₋₆ alkyl group (said C₁₋₆ alkyl group is optionally substituted by 1 to 3 substituents selected from Group G), or R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E).
a is preferably, 1 or 2, more preferably 1.
R⁵¹ are preferably, the same or different,
(1) a hydrogen atom,
(2) a halogen atom,
(3) a cyano group,
(4) a nitro group,
(5) a hydroxyl group,
(6) a formyl group,
(7) a carboxyl group,
(8) a C₁₋₆ alkyl group (said C₁₋₆ alkyl group is optionally substituted by 1 to 3 substituents selected from Group G),
(9) a C₂₋₆ alkenyl group,
(10) a C₁₋₆ alkoxy group (said C₁₋₆ alkoxy group is optionally substituted by 1 to 3 substituents selected from Group G),
(11) a C₁₋₆ alkylthio group,
(12) a C₁₋₆ alkylsulfinyl group,
(13) a C₁₋₆ alkylsulfonyl group,
(14) a C₁₋₆ alkyl-carbonyl group (said C₁₋₆ alkyl-carbonyl group is optionally substituted by 1 to 3 substituents selected from Group G),
(15) a C₁₋₆ alkoxy-carbonyl group,
(16) -N(R⁵⁷)(R⁵⁸) wherein R⁵⁷ and R⁵⁸ are as defined for the aforementioned [6],
(17) -CO-N(R⁵⁷)(R⁵⁸) wherein R⁵⁷ and R⁵⁸ are as defined for the aforementioned [6], or
(18) -C(R⁵⁹)=N(R⁶⁰) wherein R⁵⁹ and R⁶⁰ are as defined for the aforementioned [6], or
   the two adjacent R⁵¹s may form, together with the ring to which they are bonded, a fused ring (said fused ring is optionally further substituted by one substituent selected from Group E.

Preferable embodiments for R⁵¹ may include substituents other than a hydrogen atom.

Preferable embodiments for R⁵¹ may also include the two adjacent R⁵¹s which do not form, together with the ring to which they are bonded, a fused ring.

R⁵⁷ and R⁵⁸ are preferably
(1) a hydrogen atom,
(2) a C₁₋₆ alkyl group,
(3) a C₁₋₆ alkylsulfonyl group,
(4) a C₁₋₆ alkyl-carbonyl group, or
(5) a C₁₋₆ alkoxy-carbonyl group, or
   R⁵⁷ and R⁵⁸ may form, together with the ring to which they are bonded, a nitrogen-containing 4- to 7-membered heterocyclic group.

R⁵⁹ is preferably a hydrogen atom.

R⁶⁰ is preferably a hydroxyl group or a C₁₋₆ alkyl-carbonyl-oxy group.

Ring A is preferably

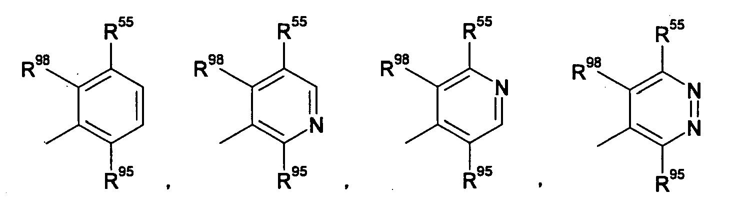

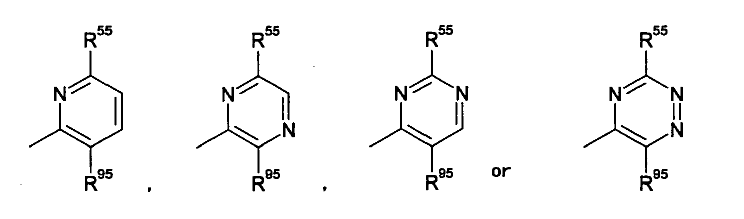

Preferable R⁹⁵ and R⁹⁸ may be the substituents as exemplified for R⁵¹.

R⁹⁵ is more preferably
(1) a halogen atom,
(2) a cyano group,
(3) a nitro group,
(4) a formyl group,
(5) a C₁₋₆ alkyl group (said C₁₋₆ alkyl group is optionally substituted by 1 to 3 substituents selected from Group G),
(6) a C₁₋₆ alkylsulfinyl group (said C₁₋₆ alkylsulfinyl group is optionally substituted by 1 to 3 substituents selected from Group G), or
(7) a C₁₋₆ alkylsulfonyl group (said C₁₋₆ alkylsulfonyl group is optionally substituted by 1 to 3 substituents selected from Group G).

R⁹⁵ is most preferably a halogen atom, a cyano group, a nitro group, a formyl group, a trifluoromethyl group, a methanesulfinyl group, a methanesulfonyl group or an ethanesulfonyl group.

R⁹⁸ is preferably
(1) a halogen atom,
(2) a nitro group,
(3) a formyl group,
(4) a C₁₋₆ alkyl group (said C₁₋₆ alkyl group is optionally substituted by 1 to 3 substituents selected from Group G),
(5) a C₂₋₆ alkenyl group (said C₂₋₆ alkenyl group is optionally substituted by 1 to 3 substituents selected from Group G),
(6) a C₁₋₆ alkylsulfonyl group (said C₁₋₆ alkylsulfonyl group is optionally substituted by 1 to 3 substituents selected from Group G).

R⁹⁸ is more preferably a halogen atom, a methyl group, an ethyl group, an n-propyl group, an n-butyl group, a 1-hydroxyethyl group, a trifluoromethyl group, a vinyl group, a methoxy group or a methanesulfonyl group.

Ring B is preferably 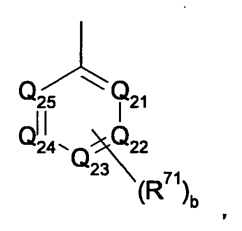 wherein Q₂₁, Q₂₂, Q₂₃, Q₂₄ and Q₂₅ are the same or different and each is -N= or -CH=, wherein the adjacent two R⁷¹s may form, together with the ring to which they are bonded, a fused ring (said fused ring is optionally further substituted by one substituent selected from Group E), and other symbols are as defined above.

It is preferable that all of Q₂₁, Q₂₂, Q₂₃, Q₂₄ and Q₂₅ are -CH=, or any one of Q₂₁, Q₂₂, Q₂₃, Q₂₄ and Q₂₅ is - N=.

Ring B is more preferably 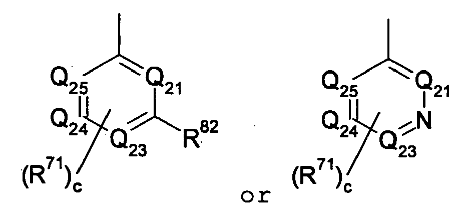 wherein R⁷¹ and b are as defined in the aforementioned [6], R⁸² is as defined for R⁷¹ (provided that a hydrogen atom is excluded), c is 0, 1 or 2, or R⁸² and the adjacent R⁷¹ may form, together with the ring to which they are bonded, a fused ring (said fused ring is optionally further substituted by one substituent selected from Group E).

Specific examples of Ring B include 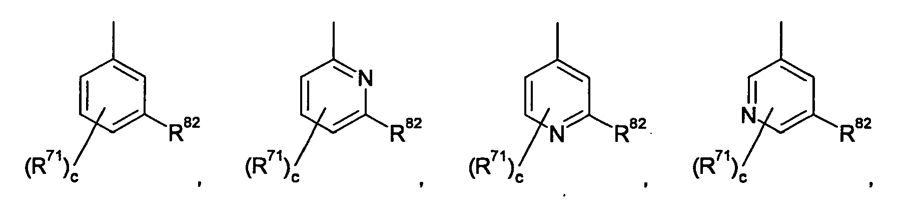 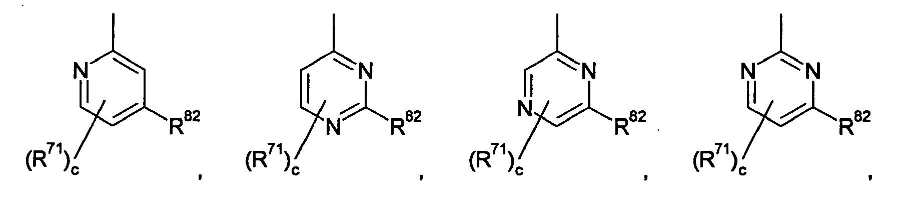 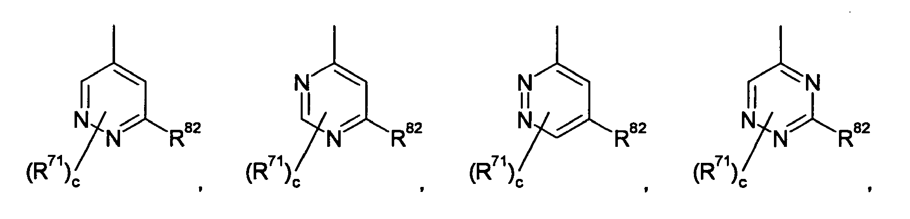 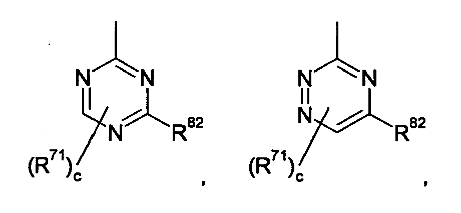 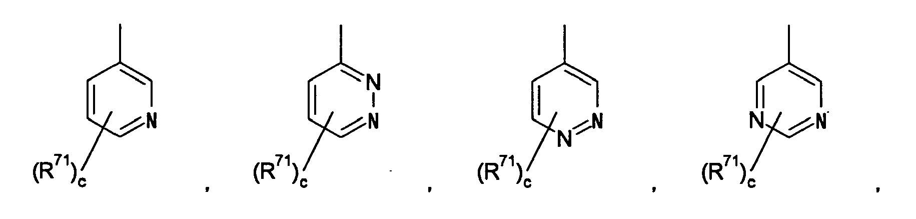 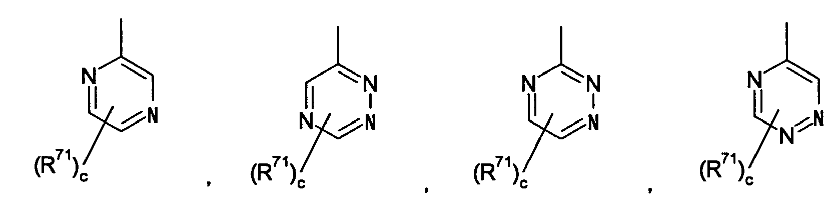 or 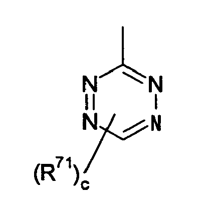 wherein the symbols are each as defined above.

Ring B is more preferably 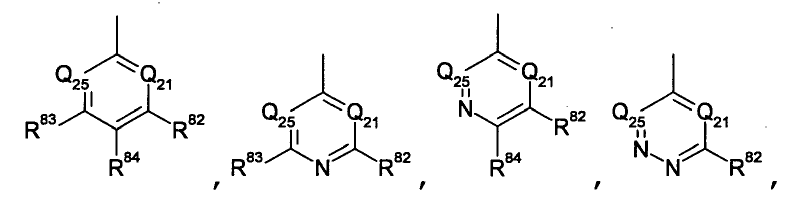 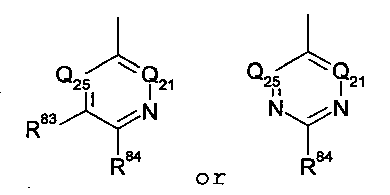 wherein R⁸³ and R⁸⁴ are the same or different and each is as defined for R⁷¹ in the aforementioned [6], or R⁸² and R⁸⁴ may form, together with the ring to which they are bonded, a fused ring (said fused ring is optionally further substituted by one substituent selected from Group E), and other symbols are each as defined above.

Q₂₁ and Q₂₅ are preferably -CH=.

Specific examples of the preferable ring B include 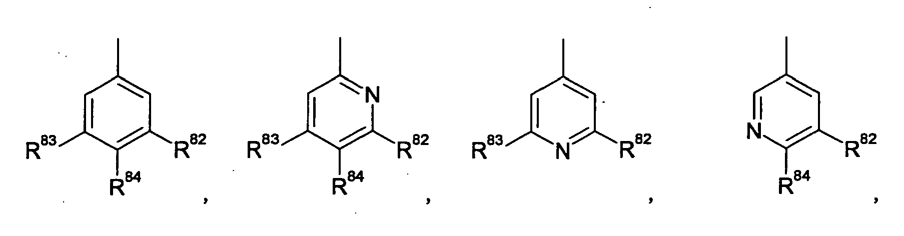 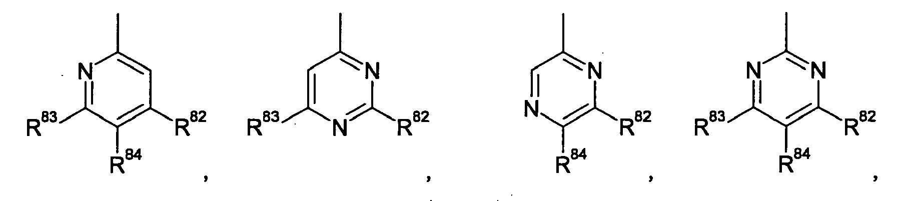 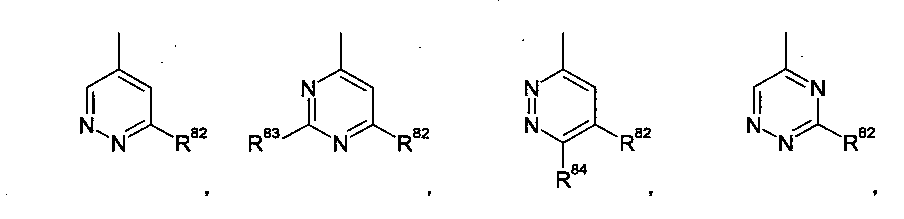 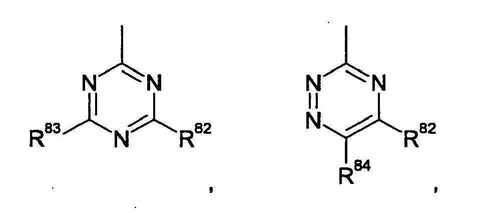 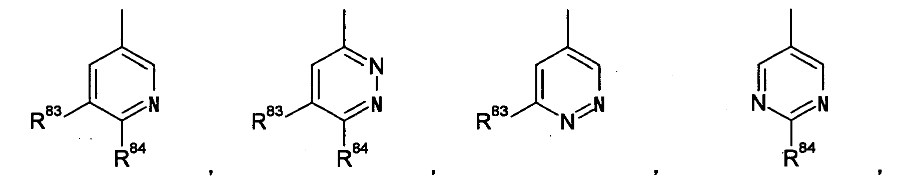 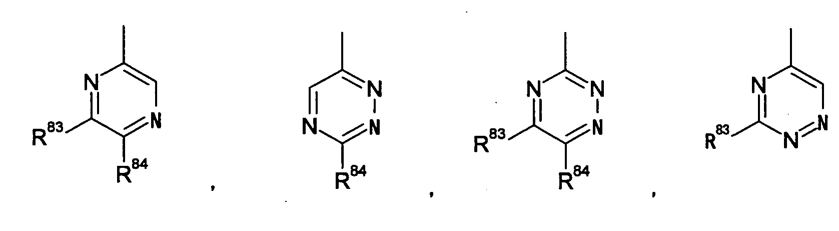 or 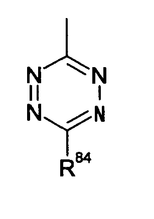 wherein
ring B' is preferably
(1) a monocyclic aryl group, or,
(2) a monocyclic heteroaryl group,
   b is preferably 1 or 2,
   R⁷¹ are preferably the same or different and each is

(1) a hydrogen atom,
(2) a halogen atom,
(3) a cyano group,
(4) a hydroxyl group,
(5) a formyl group,
(6) a carboxyl group,
(7) a C₁₋₆ alkyl group (said C₁₋₆ alkyl group is optionally substituted by 1 to 3 substituents selected from Group G),
(8) a C₂₋₆ alkenyl group (said C₂₋₆ alkenyl group is optionally substituted by 1 to 3 substituents selected from Group G),
(9) a C₁₋₆ alkoxy group (said C₁₋₆ alkoxy group is optionally substituted by 1 to 3 substituents selected from Group G),
(10) a C₁₋₆ alkyl-carbonyl group,
(11) a C₁₋₆ alkoxy-carbonyl group,
(12) -N(R⁷²)(R⁷³) wherein R⁷² and R⁷³ are each as defined above,
(13) -CO-N(R⁷²)(R⁷³) wherein R⁷² and R⁷³ are each as defined above,
(14) -C(R⁷⁴)=N(R⁷⁵) wherein R⁷⁴ and R⁷⁵ are each as defined above,
(15) a monocyclic aryl group,
(16) a monocyclic heteroaryl group (said monocyclic heteroaryl group is optionally substituted by 1 to 5 substituents selected from Group E), or
(17) a heterocycloalkyl group, or
   two adjacent R⁷¹s may form, together with the ring to which they are bonded, a fused ring (said fused ring is optionally further substituted by one substituent selected from Group E).

Preferable embodiments for R⁸² include
(1) a halogen atom,
(2) a formyl group,
(3) a C₁₋₆ alkyl group (said C₁₋₆ alkyl group is optionally substituted by 1 to 3 substituents selected from Group G), more preferably a methyl group or an ethyl group,
(4) a C₂₋₆ alkenyl group (said C₂₋₆ alkenyl group is optionally substituted by 1 to 3 substituents selected from Group G), more preferably a vinyl group, a 1-methylpropenyl group or a propenyl group,
(5) a C₁₋₆ alkoxy group (said C₁₋₆ alkoxy group is optionally substituted by 1 to 3 substituents selected from Group G), more preferably a methoxy group or an ethoxy group,
(6) -C(R⁷⁴)=N(R⁷⁵) wherein R⁷⁴ and R⁷⁵ are each as defined above, more preferably -CH=NOH, or one of the groups represented by the following chemical formulas: 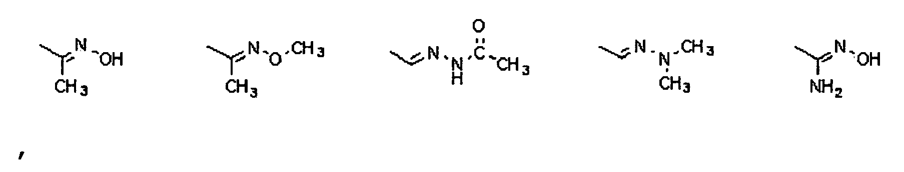
(7) a monocyclic heteroaryl group (said monocyclic heteroaryl group is optionally substituted by 1 to 5 substituents selected from Group E), and
(8) a heterocycloalkyl group.

Preferable embodiments for R⁸³ include
(1) a halogen atom,
(2) a formyl group,
(3) a C₁₋₆ alkyl group (said C₁₋₆ alkyl group is optionally substituted by 1 to 3 substituents selected from Group G), more preferably a methyl group or an ethyl group,
(4) a C₂₋₆ alkenyl group (said C₂₋₆ alkenyl group is optionally substituted by 1 to 3 substituents selected from Group G), more preferably a vinyl group, a 1-methylpropenyl group or a propenyl group,
(5) a C₁₋₆ alkoxy group (said C₁₋₆ alkoxy group is optionally substituted by 1 to 3 substituents selected from Group G), more preferably a methoxy group or an ethoxy group,
(6) -C(R⁷⁴)=N(R⁷⁵) wherein R⁷⁴ and R⁷⁵ are each as defined above, more preferably -CH=NOH, or one of the groups represented by the following chemical formulas 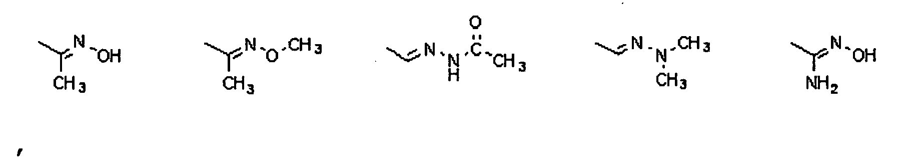
(7) a monocyclic heteroaryl group (said monocyclic heteroaryl group is optionally substituted by 1 to 5 substituents selected from Group E), and
(8) a heterocycloalkyl group.

Preferable embodiments for R⁸⁴ include
(1) a hydrogen atom,
(2) a halogen atom,
(3) a hydroxyl group,
(4) a C₁₋₆ alkyl group (said C₁₋₆ alkyl group is optionally substituted by 1 to 3 substituents selected from Group G), more preferably a methyl group, an ethyl group or a trifluoromethyl group,
(5) a C₁₋₆ alkoxy group (said C₁₋₆ alkoxy group is optionally substituted by 1 to 3 substituents selected from Group G), more preferably a methoxy group, and
(6) -N(R⁷²)(R⁷³) wherein R⁷² and R⁷³ are as defined in the aforementioned [6], more preferably a dimethylamino group.

R⁷² and R⁷³ are preferably the same or different and each is
(1) a hydrogen atom,
(2) a C₁₋₆ alkyl group (said C₁₋₆ alkyl group is optionally substituted by 1 to 3 substituents selected from Group G),
(3) a C₁₋₆ alkylsulfonyl group,
(4) a C₁₋₆ alkyl-carbonyl group,
(5) a C₁₋₆ alkoxy-carbonyl group,
(6) carbamoyl group,
(7) a hydroxyl group,
(8) an amino group, or
(9) a C₁₋₆ alkyl-carbonyl-amino group, or
   R⁷² and R⁷³ may form, together with the nitrogen atom to which they are bonded, to form a nitrogen-containing 4-to 7-membered heterocyclic group.

R⁷⁴ is preferably
(1) a hydrogen atom,
(2) a C₁₋₆ alkyl group, or
(3) an amino group.

R⁷⁵ is preferably
(1) a hydroxyl group,
(2) a C₁₋₆ alkoxy group (said C₁₋₆ alkoxy group is optionally substituted by 1 to 3 substituents selected from Group G), or (3) -N(R⁷⁶)(R⁷⁷) wherein R⁷⁶ and R⁷⁷ are the same or different and each is a hydrogen atom, a C₁₋₆ alkyl group or a C₁₋₆ alkyl-carbonyl group.

In the formula [1], the compounds represented by the following formulas are also preferable embodiments:
a compound represented by: 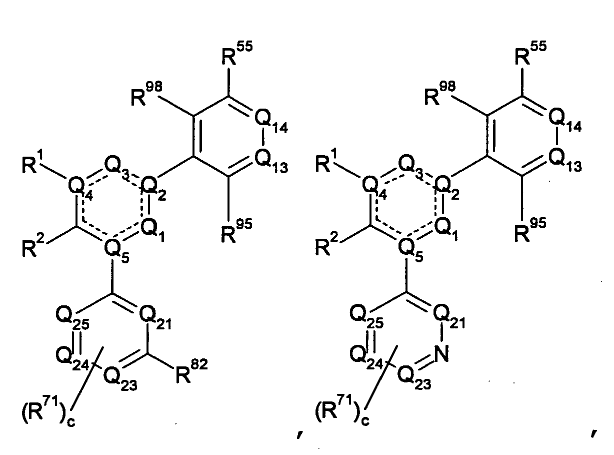 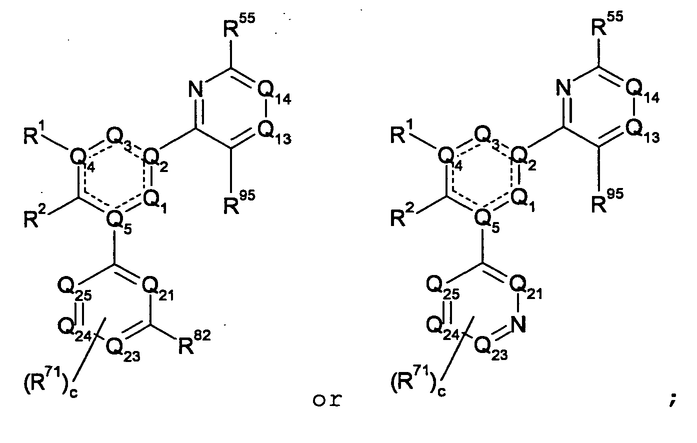
a compound represented by: 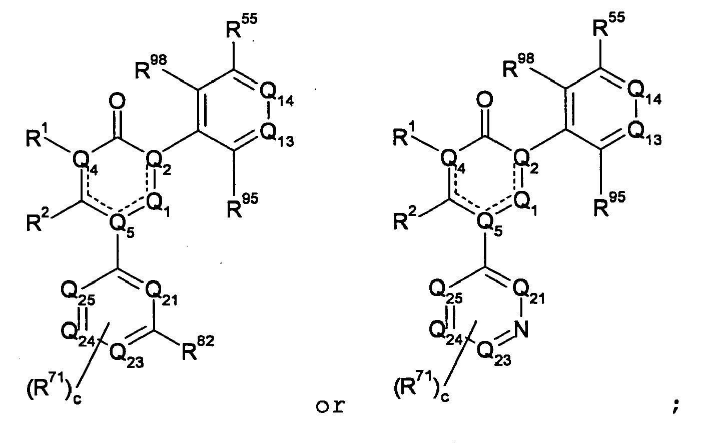
a compound represented by: 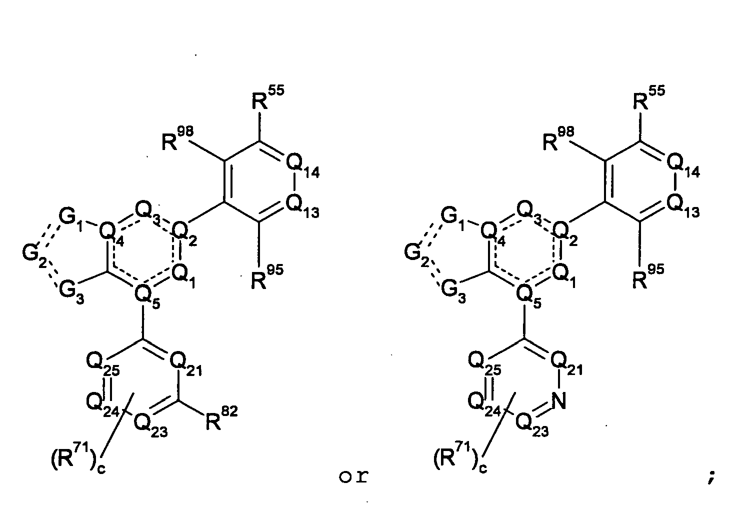
a compound represented by: 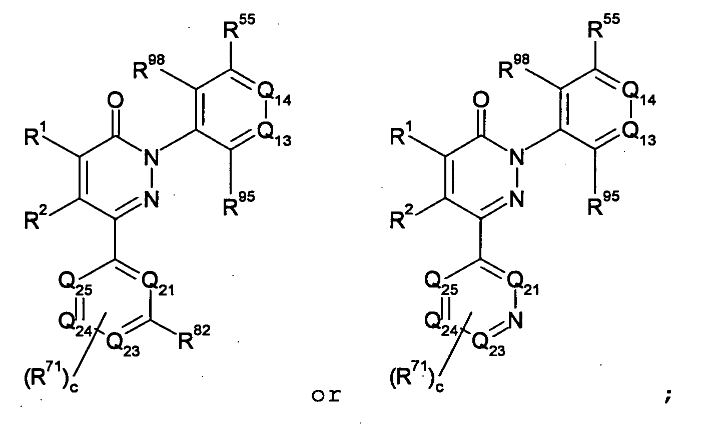
a compound represented by: 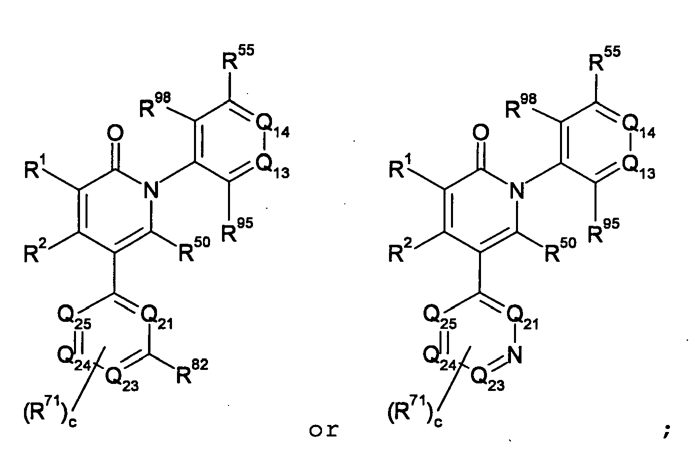
a compound represented by: 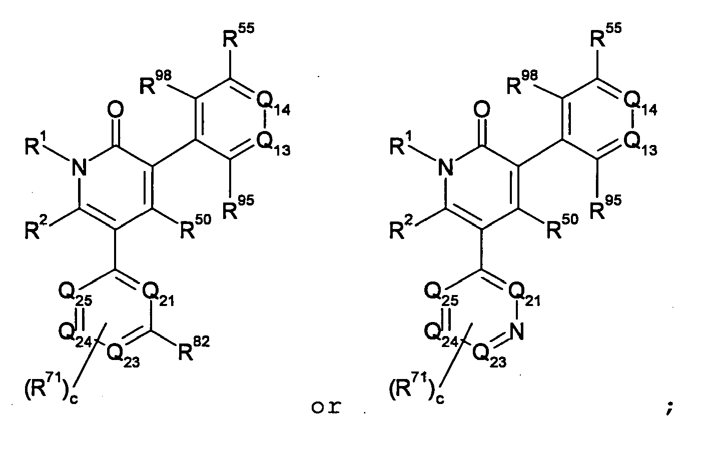
a compound represented by: 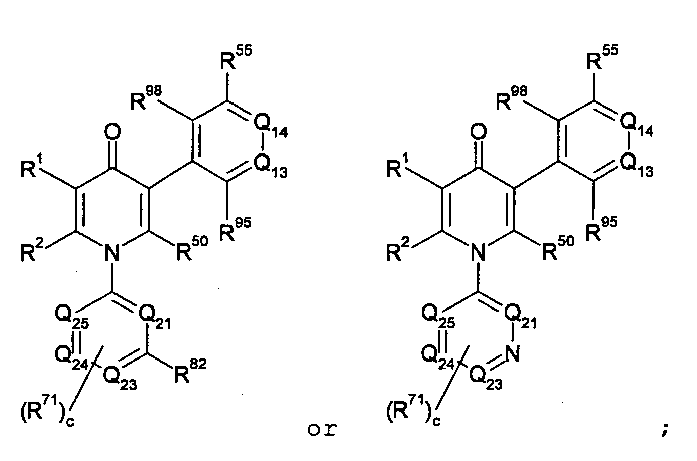
a compound represented by: 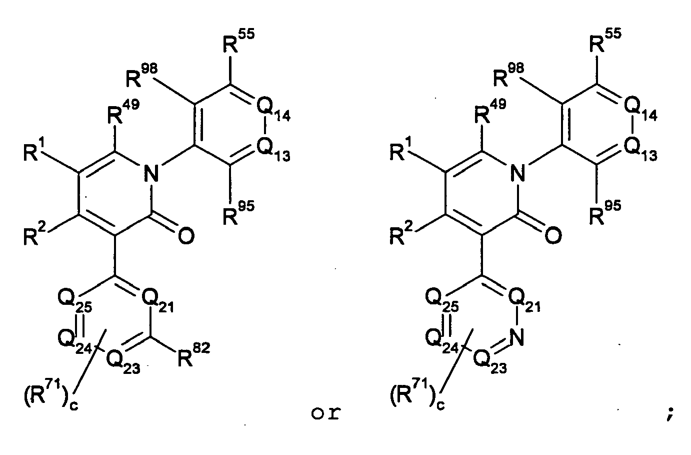
a compound represented by: 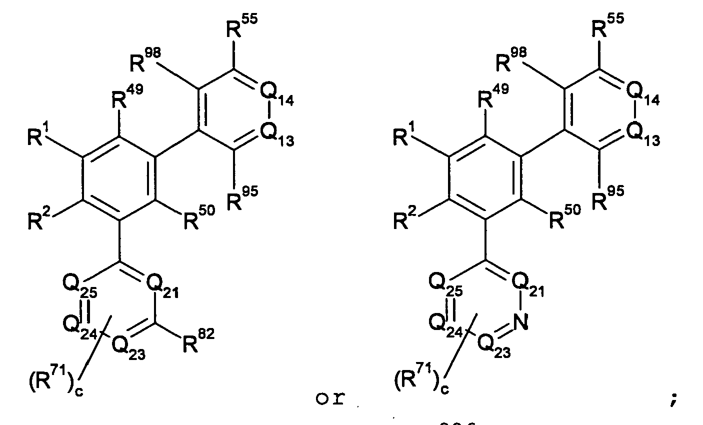
a compound represented by: 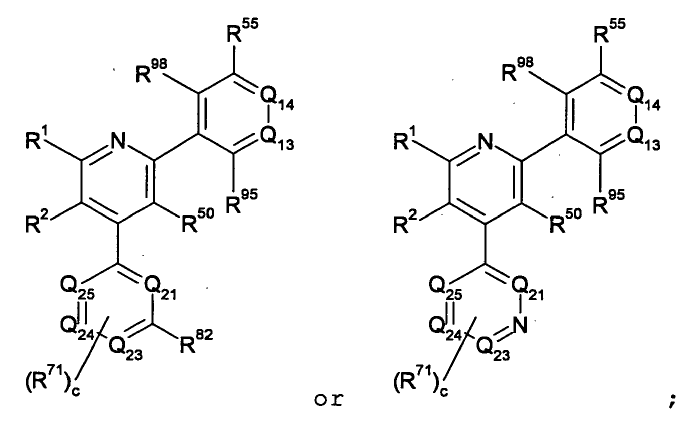
a compound represented by: 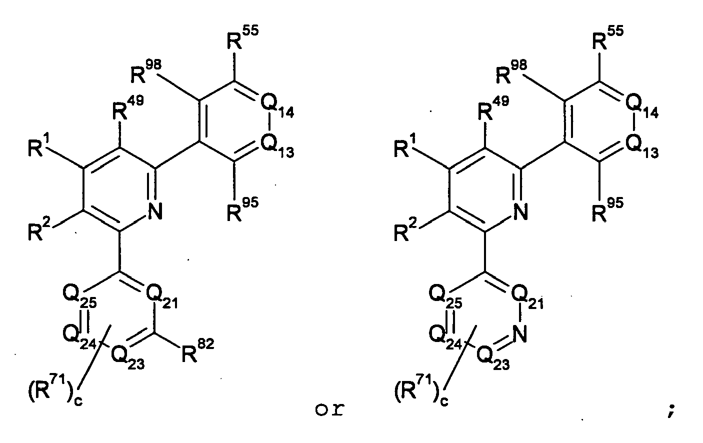
a compound represented by: 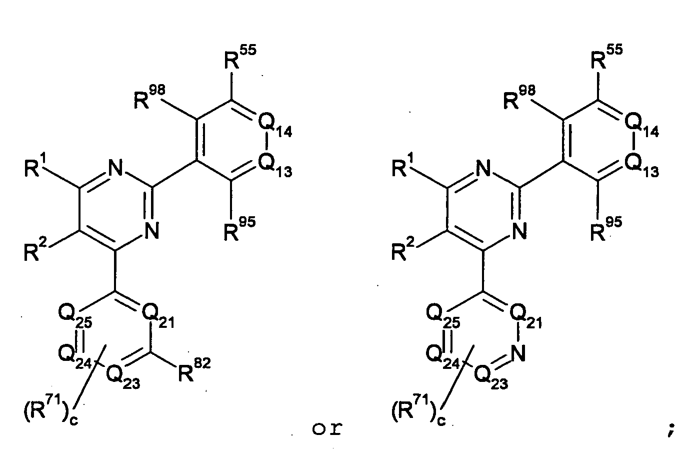
a compound represented by: 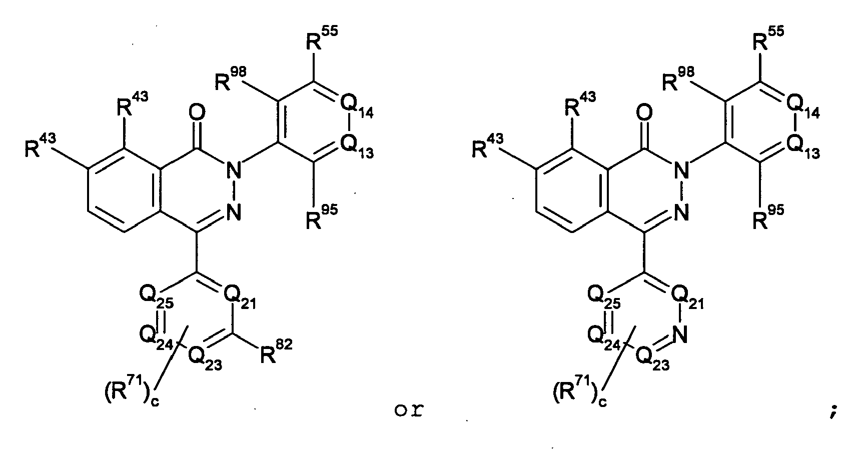
a compound represented by: 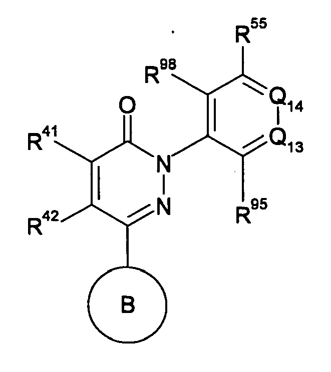 wherein ring B is 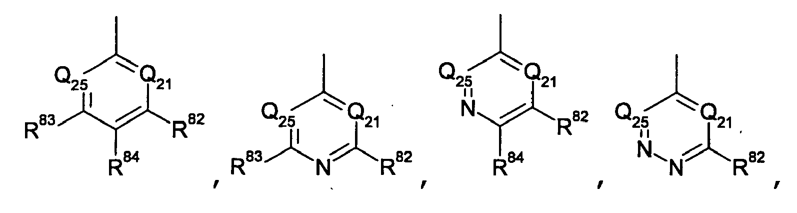 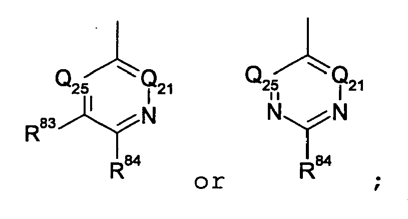
a compound represented by: 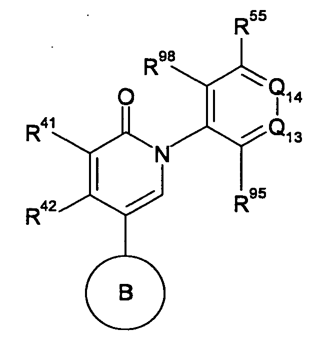 wherein ring B is 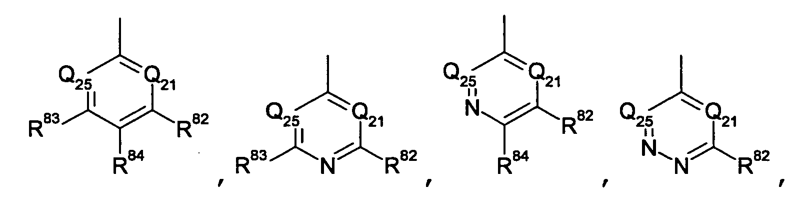 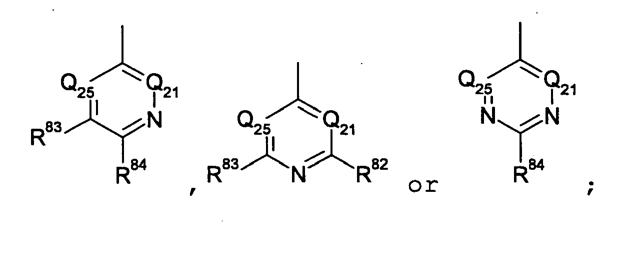 in the aforementioned formulas, Q₁, Q₂, Q₃, Q₄, Q₅, R¹, R², R⁵⁵ and R⁷¹ are each as defined in the aforementioned [1], Q₁₃ and Q₁₄ are each as defined in the aforementioned [6], R⁹⁵ and R⁹⁸ are each as defined in the aforementioned [12], Q₂₁, Q₂₂, Q₂₃ and Q₂₄ are each as defined in the aforementioned [14], and R⁸² is as defined in the aforementioned [17].

Hereinafter the preferable examples for each substituents are specifically listed.

Preferable examples of 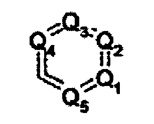 wherein Q₁ to Q₅ are each as defined in the aforementioned [1], include

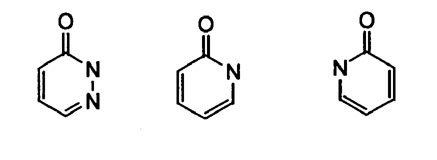

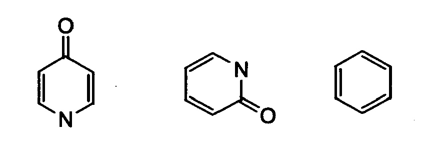

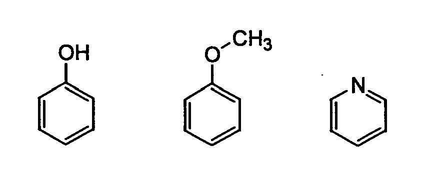

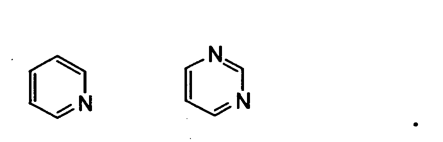

In ring A, preferable examples of 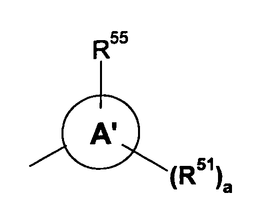 wherein A', R⁵¹ and R⁵⁵ are each as defined in the aforementioned [1], include 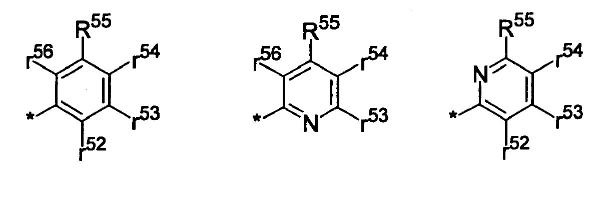 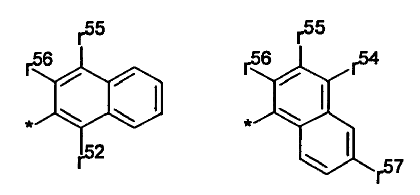 wherein R⁵⁵ is as defined in the aforementioned [1], and other symbols are explained below.

In the aforementioned formulas, preferable examples of r⁵² include a hydrogen atom, a chlorine atom, a fluorine atom, a bromine atom, a methyl group, an ethyl group, an n-butyl group, an isopropyl group, a trifluoromethyl group, a vinyl group, a 1-methylvinyl group, a 2-methylpropenyl group, a propenyl group, a hydroxymethyl group, a 1-hydroxyethyl group, a 1-hydroxyisopropyl group, a benzyloxy group, a cyano group, a nitro group, a hydroxyiminomethyl group, an acetyloxyiminomethyl group, a formyl group, a trifluoromethylcarbonyl group, an acetyl group, a methoxycarbonyl group, a carboxyl group, a N-ethylcarbamoyl group, an N,N-dimethylcarbamoyl group, methylthio group, methylsulfonyl group, methylsulfinyl group, an ethylsulfonyl group, an amino group, a methylamino group, an ethylamino group, a dimethylamino group, an acetylamino group, a methylsulfonyl group, an amino group, a hydroxyl group and a methoxy group.

In the aforementioned formulas, preferable examples of r⁵³ include a hydrogen atom, a chlorine atom, a hydroxyl group, a methoxy group, an ethoxy group, an n-propylamino group, a morpholino-group and a benzyloxy group.

In the aforementioned formulas, preferable examples of r⁵⁴ include a hydrogen atom, a chlorine atom, a fluorine atom, a bromine atom, a hydroxyl group, a methyl group, a methoxy group, a cyano group, a nitro group, an amino group, a methylamino group, an ethylamino group, a formyl group, an ethoxycarbonyl group, a methoxycarbonylamino group, an acetylamino group, a t-butoxycarbonylamino group, a methylsulfonylamino group, a carbamoyl group, a carboxyl group, an N,N-dimethylcarbamoyl group and a dimethylamino group.

In the aforementioned formulas, preferable examples of r⁵⁶ include a hydrogen atom, a chlorine atom, a fluorine atom, a bromine atom, a hydroxyl group, a methyl group, an ethyl group, a vinyl group, a methoxy group and a benzyloxy group.

In the aforementioned formulas, preferable examples of r⁵⁷ include a hydrogen atom, a hydroxyl group, an isopropoxy group and a benzyloxy group.

In the aforementioned formulas, preferable examples of R⁵⁵ include a hydrogen atom, a chlorine atom, a fluorine atom, a bromine atom, an iodine atom, a nitro group, an amino group, a cyano group, a methyl group, an ethyl group, an n-propyl group, a methylaminomethyl group, an N,N-dimethylaminomethyl group, a hydroxymethyl group, a 3-hydroxypropyl group, a 3-hydroxypropynyl group, a hydroxyl group, a methoxy group, an ethoxy group, an n-propoxy group, a carboxyl group, a methoxycarbonyl group, an ethoxycarbonyl group, an ethylamino group, an n-propylamino group, an n-butylamino group, an isopropylamino group, an N-ethyl-N-methylamino group, a 1-ethylpropylamino group, a 3-methylpropylamino group, a dimethylamino group, an N-methyl-N-propylamino group, a diethylamino group, a carbamoyl group, a methylcarbamoyl group, an N,N-dimethylcarbamoyl group, and the substituents represented by following chemical formulas

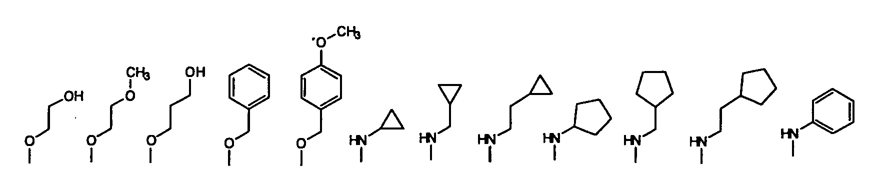

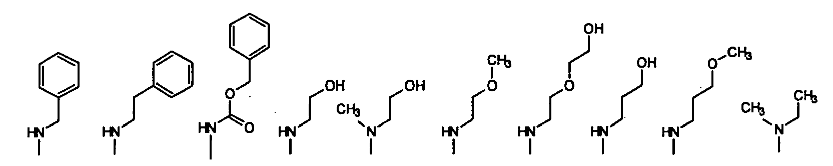

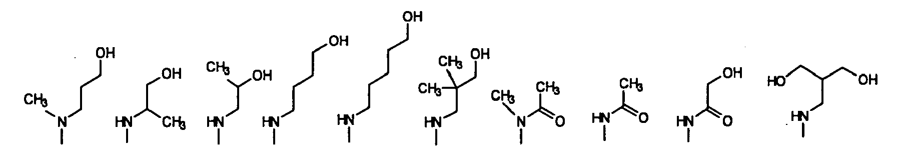

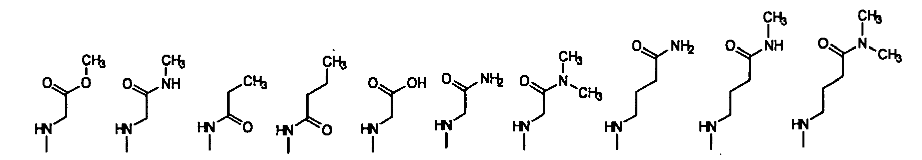

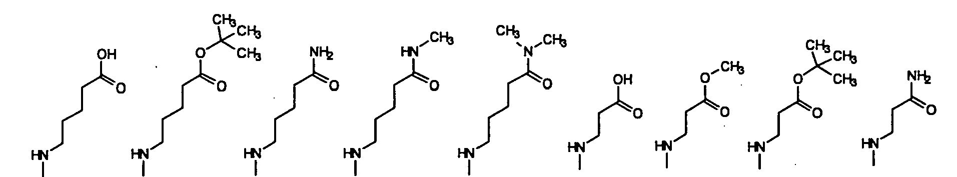

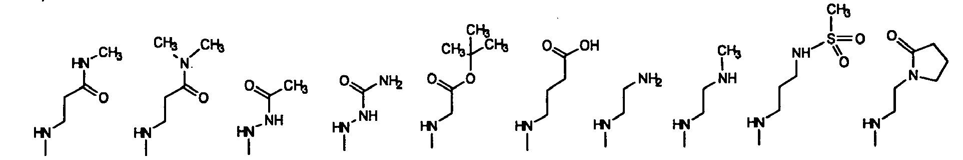

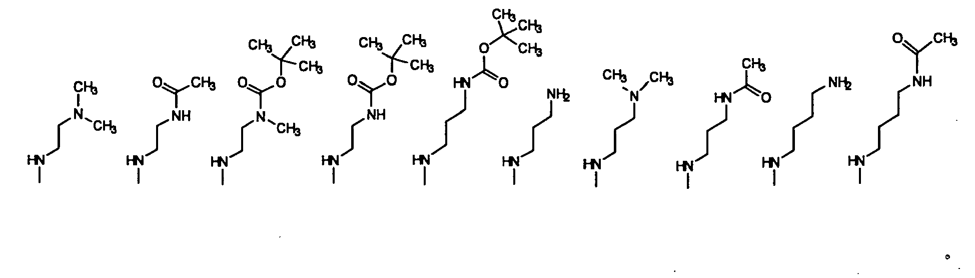

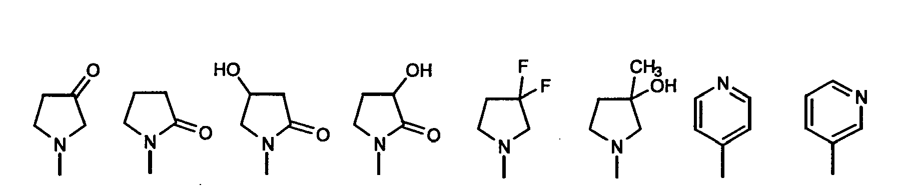

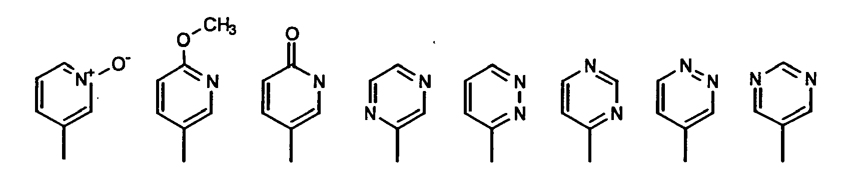

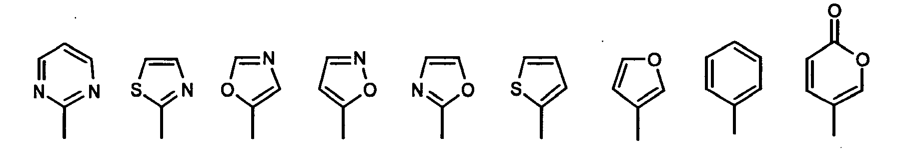

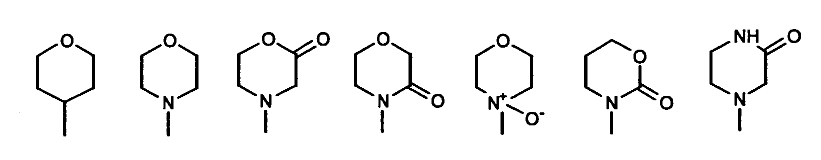

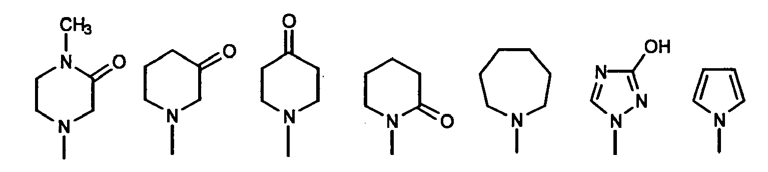

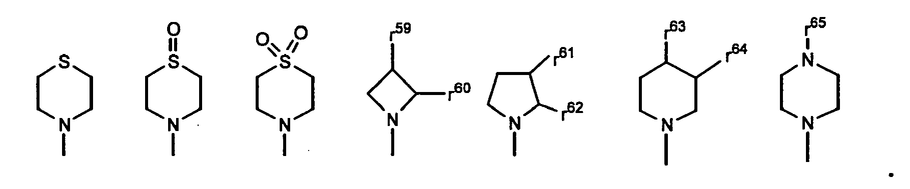

In the aforementioned formulas, preferable examples of r⁵⁹ include a hydrogen atom, a hydroxymethyl group, an N-methylcarbamoylmethyl group, an N-acetylaminomethyl group, an N-methylcarbamoyl group, a hydroxyl group, a carbamoyloxy group, an N-methylcarbamoylmethoxy group and an acetylamino group.

In the aforementioned formulas, preferable examples of r⁶⁰ include a hydrogen atom and a hydroxymethyl group.

In the aforementioned formulas, preferable examples of R⁶¹ include a hydrogen atom, a methyl group, a trifluoromethyl group, a hydroxymethyl group, a methylcarbamoylmethyl group, a carboxyl group, a methoxycarbonyl group, a carbamoyl group, an N-methylcarbamoyl group, an N,N-dimethylcarbamoyl group, an N-ethylcarbamoyl group, an N-isopropylcarbamoyl group, an N-(t-butoxycarbonyl)-N-methylamino group, an amino group, a methylamino group, a dimethylamino group, an acetylamino group, an N-acetyl-N-methylamino group, an ethylcarbonylamino group, an isopropylcarbonylamino group, an n-propylcarbonylamino group, a methylsulfonylamino group, a hydroxyl group, a methoxy group, a 2-acetylaminoethoxy group, a 3-acetylaminopropoxy group, an N-methylcarbamoylmethoxy group, a 2-hydroxyethoxy group, a 3-hydroxypropoxy group, a 2-(N-methylcarbamoyl)ethoxy group and an N-methylcarbamoyloxy group.

In the aforementioned formulas, preferable examples of r⁶² include a hydrogen atom, a methyl group and a hydroxymethyl group.

In the aforementioned formulas, preferable examples of r⁶³ include a hydrogen atom, a hydroxyl group, a methoxy group, a dimethylamino group, an ethoxycarbonyl group, a carboxyl group, a hydroxymethyl group, a methoxymethyl group and a phenoxymethyl group.

In the aforementioned formulas, preferable examples of r⁶⁴ include a hydrogen atom, a hydroxyl group and a methoxy group.

In the aforementioned formulas, preferable examples of r⁶⁵ include a hydrogen atom, a methyl group, a n acetyl group, an N-methylcarbamoyl group and a methylsulfonyl group.

Preferable examples of R⁵¹ include those exemplified for r⁵², r⁵³, r⁵⁴ and r⁵⁶. Examples of the fused ring formed by the two adjacent R⁵¹s, together with the ring to which they are bonded, include

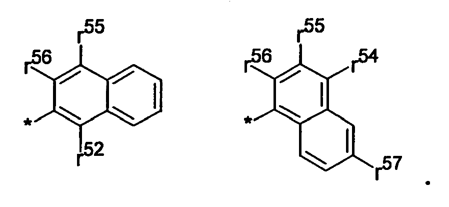

Preferable examples of R⁹⁵ include those exemplied for r⁵² (provided that a hydrogen atom is excluded). Preferable examples of R⁹⁸ include those exemplied for r⁵⁶.

In ring B, preferable examples of 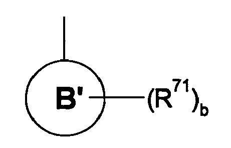 wherein B', R⁷¹ and b are as defined in the aforementioned [1], include

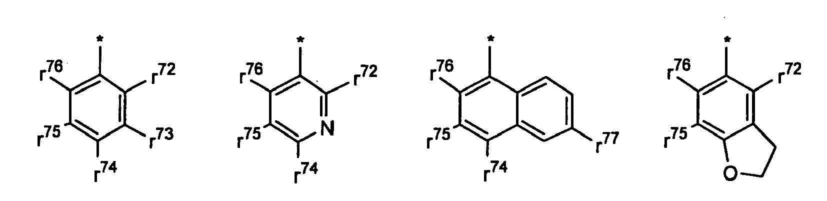

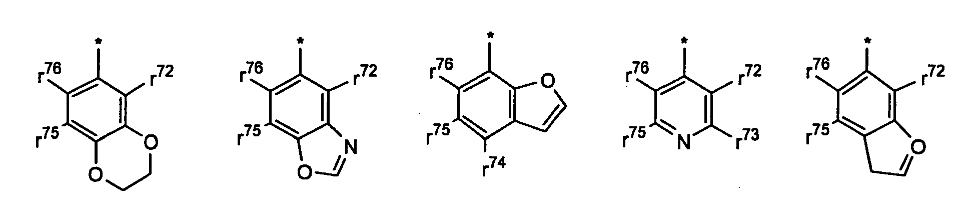

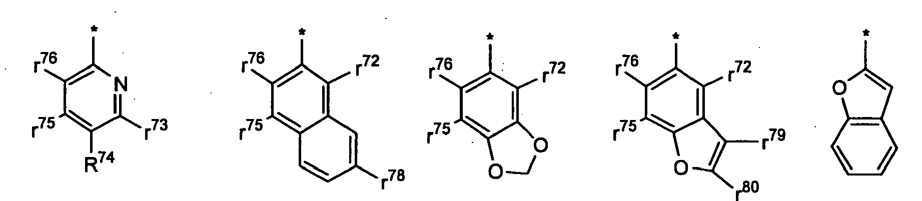

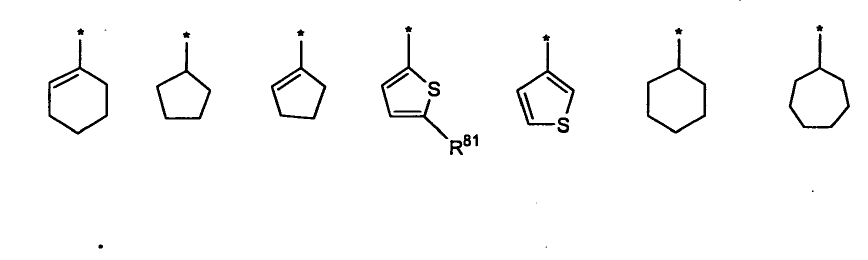

Preferable examples of r⁷² include a hydrogen atom and a methyl group.

Preferable examples of r⁷³ include a hydrogen atom, a chlorine atom, a fluorine atom, a bromine atom, a methyl group, an ethyl group, an isopropyl group, a trifluoromethyl group, a hydroxymethyl group, a 1-hydroxyethyl group, a vinyl group, a 1-methylvinyl group, a 1,2-dihydroxyethyl group, a cyano group, a hydroxyiminomethyl group, a methoxyiminomethyl group, a t-butoxy iminomethyl group, a carboxyl group, a methoxycarbonyl group, an acetyl group, a formyl group, a carbamoyl group, an N-methylcarbamoyl group, an N,N-dimethylcarbamoyl group, an N-(t-butyl)carbamoyl group, an N-hydroxycarbamoyl group, a methylsulfonylamino group, a hydroxyl group, a methoxy group, an ethoxy group, an isopropoxy group, a t-butoxy group, an n-propoxy group, an n-butoxy group, a trifluoromethoxy group, an amino group, a dimethylamino group, a trimethylammonium group, an acetylamino group, an acetylmethylamino group, an ureido group, a methoxycarbonylamino group, a t-butoxycarbonylamino group, and the substituents represented by the following structural formulas

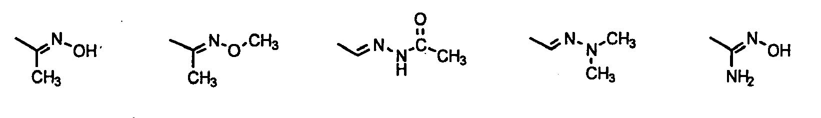

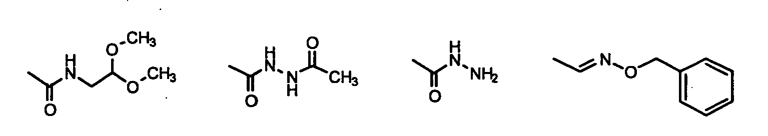

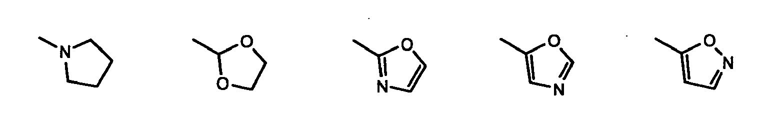

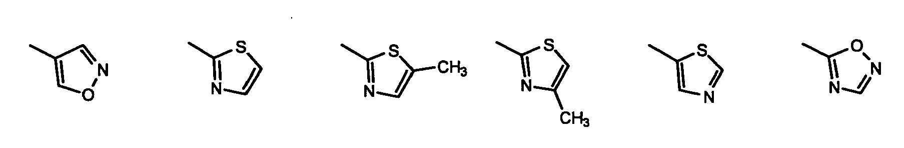

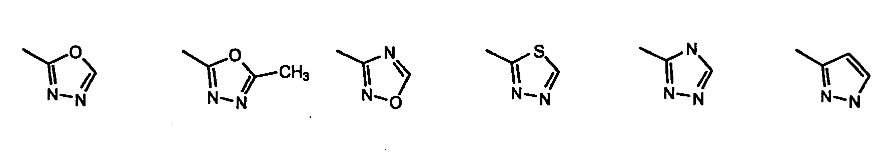

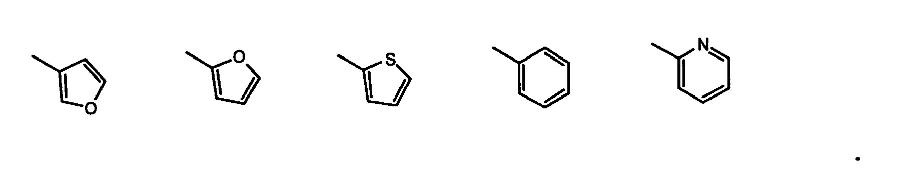

Preferable examples of r⁷⁴ include a hydrogen atom, a fluorine atom, a chlorine atom, a bromine atom, a methyl group, an ethyl group, a trifluoromethyl group, a cyano group, a hydroxyl group, a methoxy group, a dimethylamino group, a carboxyl group and a carbamoyl group.

Preferable examples of r⁷⁵ include a hydrogen atom, a fluorine atom, a chlorine atom, a bromine atom, a vinyl group, a propenyl group, a 1-methylvinyl group, a 2-(methoxycarbonyl)vinyl group, a formyl group, an acetyl group, a cyano group, a hydroxyiminomethyl group, a hydroxymethyliminomethyl group, a methyl group, a trifluoromethyl group, a methoxy group, an ethoxy group, a hydroxyl group and an oxazolyl group.

Preferable examples of r⁷⁶ include a hydrogen atom, a fluorine atom, a chlorine atom, a bromine atom, a hydroxyl group and a methoxy group.

Preferable examples of r⁷⁷ and r⁷⁸ may each include a hydrogen atom, a hydroxyl group and a methoxy group.

Preferable examples of r⁷⁹, r⁸⁰ and r⁸¹ may each include a hydrogen atom and a methyl group.

Preferable examples of R⁷¹ include those exemplified for r⁷², r⁷³, r⁷⁴, r⁷⁵ and r⁷⁶. Preferable examples of the fused ring formed by the two adjacent R⁷¹s, together with the ring to which they are bonded, include

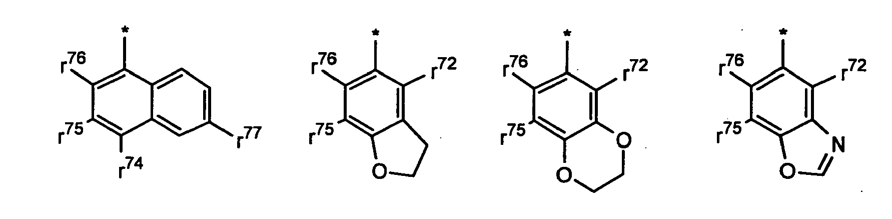

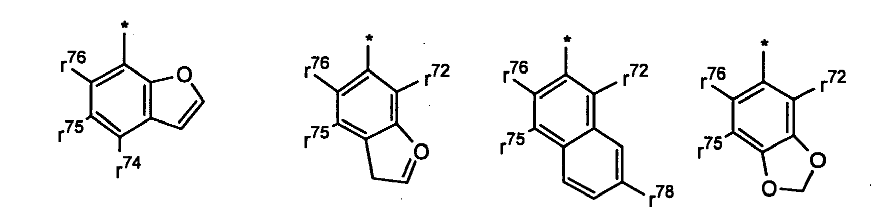

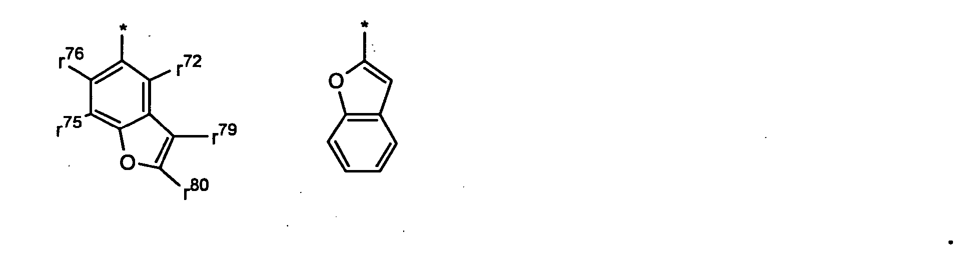

Preferable examples of R⁸² include those exemplified for r⁷³ (provided that a hydrogen atom is excluded). Preferable examples of R⁸⁴ include those exemplified for r⁷⁴. Preferable examples of R⁸³ include those exemplified for ⁷⁵.

Examples of the fused ring formed by R⁸² and R⁸⁴, toghther with the ring to which they are bonded, include

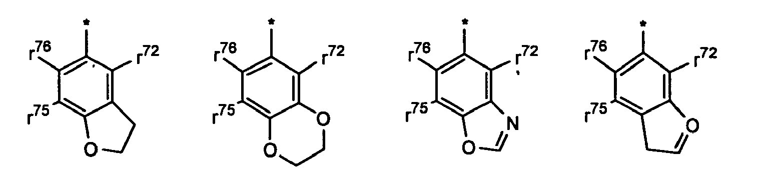

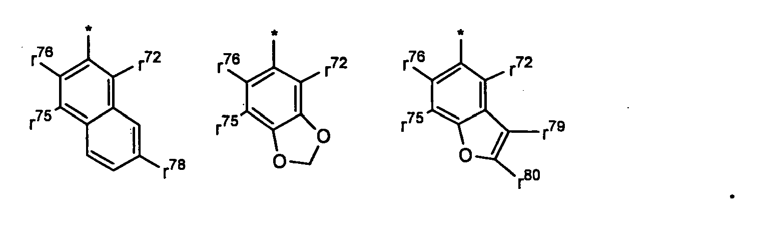

Preferable examples of R¹ include a hydrogen atom, an amino group, an ethylamino group, a dimethylamino group, an ethylamino group, an N-ethyl-N-methylamino group, a diethylamino group, an N-methyl-N-(n-propyl)amino group, an N-isopropyl-N-amino group, an N-isopropyl-N-methylamino group, a diisopropylamino group, an N-(sec-butyl)-N-methylamino group, a t-butylamino group, an N-(t-butylmethyl)-N-amino group, a methyl group, an ethyl group, an n-propyl group, an isopropyl group, an n-butyl group, a sec-butyl group, a t-butyl group, a 1-ethylpropyl group, a 1,1-dimethylpropyl group, a 1-methylvinyl group, an acetyl group, a carboxyl group, an ethoxycarbonyl group, a 2-methylpropyloxycarbonyl group, an N-methylcarbamoyl group, an N,N-dimethylcarbamoyl group, an ethylthio group, an ethoxy group, an isopropyloxy group, an n-propyloxy group, an n-butyloxy group, a methoxyethoxy group, a phenyl group, a benzyl group, a phenethyl group and a 3-phenylpropyl group, and the substitients represented by the following structural formulas:

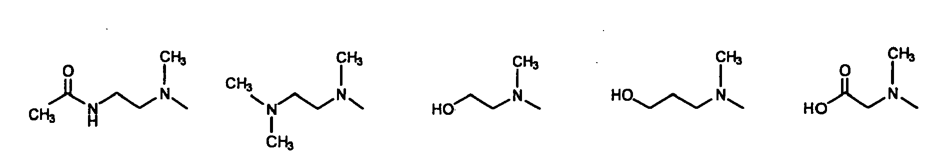

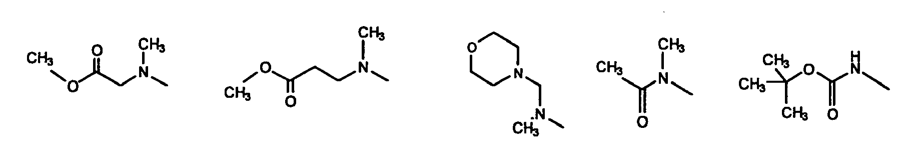

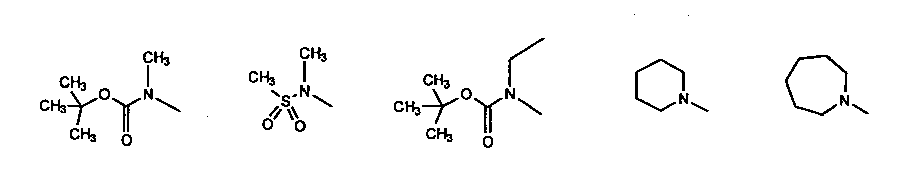

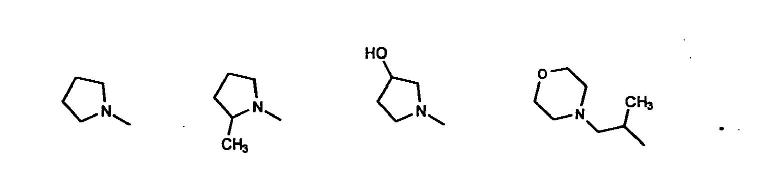

Preferable examples of R² include a hydrogen atom, a methyl group and an ethyl group.

In the aforementioned [1], preferable examples of 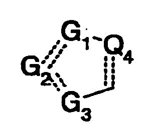 which is formed by R¹ and R², together with Q₄ (Q₄ is as defined in the aforementioned [1]) and the carbon atom to which they are bonded (wherein G₁-G₃ and Q₄ are as defined in the aforementioned [1]), include the following:
(1) a cyclic structure represented by the following chemical formula: 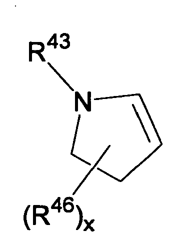 wherein R⁴³ is preferably, a hydrogen atom, a methyl group, an ethyl group, a 2-hydroxyethyl group, a 3-hydroxypropyl group, a 2-hydroxy-2-methylpropyl group, a 1-hydroxy-N-butyl group, a 2-methoxyethyl group, a methylcyanomethyl group, a 2-aminoethyl group, a 2-acetylaminoethyl group, a benzyl group, an acetylmethyl group, a carboxymethyl group, a methoxycarbonylmethyl group, a carbamoylmethyl group, an N-methylcarbamoylmethyl group, an N,N-dimethylcarbamoylmethyl group, an N-ethylcarbamoylmethyl group, an N,N-diethylcarbamoylmethyl group, an N,N-butylcarbamoylmethyl group, an N,N-hexylcarbamoylmethyl group, a methylsulfonyl group, an aminoacetyl group, an acetyl group, an ethylcarbonyl group, a hydroxyacetyl group, an acetyloxyacetyl group, a methoxycarbonyl group, an ethoxycarbonyl group, an n-propoxycarbonyl group, an isopropoxycarbonyl group, a t-butoxycarbonyl group, a carbamoyl group, an N-methylcarbamoyl group, an N,N-dimethylcarbamoyl group or an acetylcarbonyl group, or the substituent represented by one of the following structural formulas 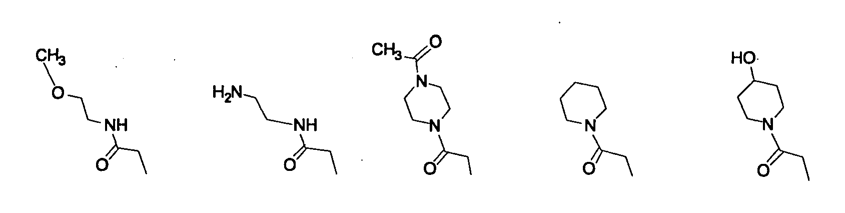 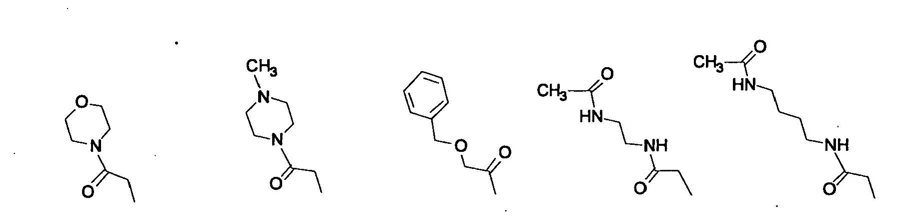 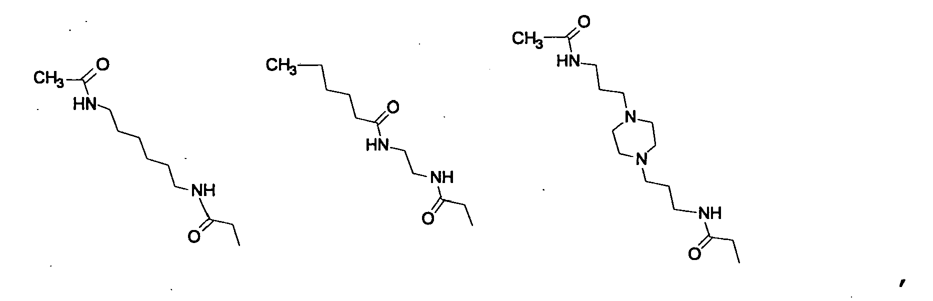 R⁴⁶ are preferably the same or different and each is a hydrogen atom or a methyl group, and x is 1 or 2;
(2) a ring structure represented by the following chemical formula: 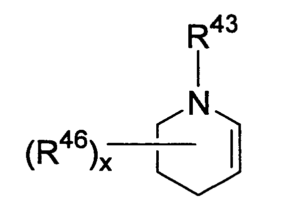 wherein
   R⁴³ is preferably a hydrogen atom, a methyl group, an ethyl group, an n-butyl group, a hydroxyethyl group, a carboxylmethyl group, a methoxycarbonylmethyl group, an ethoxycarbonylmethyl group, an acetyl group or a methylsulfonyl group,
   R⁴⁶ are preferably the same or different and each is a hydrogen atom, a methyl group or an ethyl group, and X is 1 or 2;
(3) a ring structure represented by the following chemical formula: 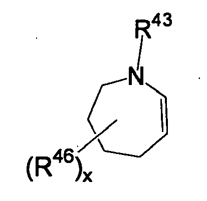 wherein R⁴³ and R⁴⁶ are preferably each a hydrogen atom, and
   x is preferably 1 or 2;
(4) a ring structure represented by the following chemical formula: 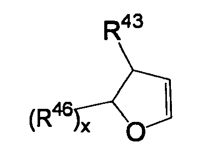 wherein
   R⁴³ and R⁴⁶ are preferably each a hydrogen atom, and X is preferably 1 or 2;
(5) a ring structure represented by the following chemical formula: 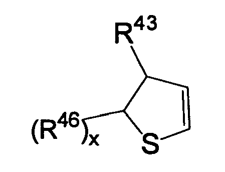 wherein
   R⁴³ and R⁴⁶ are preferably each a hydrogen atom, and
   x is preferably 1 or 2;
(6) a ring structure represented by the following chemical formula: 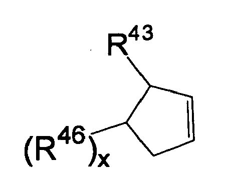 wherein
   R⁴³ is preferably a hydrogen atom,
   R⁴⁶ are preferably each a hydrogen atom or a methyl group, and
   x is preferably 1 or 2;
(7) a cyclic structure represented by the following chemical formula: 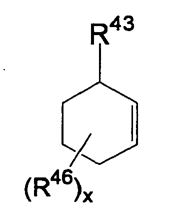 wherein R⁴³ and R⁴⁶ are preferably each a hydrogen atom, and
   x is preferably 1 or 2;
(8) a cyclic structure represented by the following chemical formula: 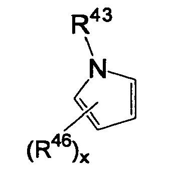 wherein
   R⁴³ is preferably a hydrogen atom, a hydroxymethyl group or 2-hydroxyethyl group, R⁴⁶ are preferably each a hydrogen atom, a methyl group or an ethyl group, and
   X is preferably 1 or 2;
(9) a cyclic structure represented by the following chemical formula: 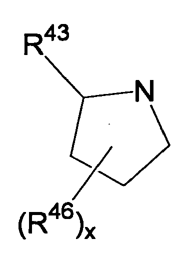 wherein
   R⁴³ is preferably a hydrogen atom,
   R⁴⁶ is preferably a hydrogen atom, and
   X is preferably 1 or 2;
(10) a cyclic structure represented by the following chemical formula: 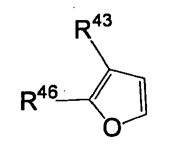 wherein
   R⁴³ is preferably a hydrogen atom or a methoxycarbonyl group, and
   R⁴⁶ is preferably a hydrogen atom;
(11) a cyclic structure represented by the following chemical formula: 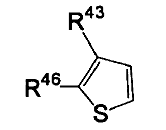 wherein
   R⁴³ is preferably a hydrogen atom or a methoxycarbonyl group, and
   R⁴⁶ is preferably a hydrogen atom;
(12) a cyclic structure represented by the following chemical formula: 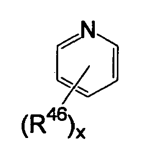 wherein R⁴⁶ are preferably each a hydrogen atom, a methyl group, an ethyl group, a methoxy group or a hydroxyl group, and
   x is preferably 1 or 2;
(13) a cyclic structure represented by the following chemical formula: 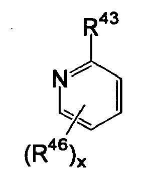 wherein
   R⁴³ is preferably a hydrogen atom, a methyl group, an ethyl group, a methoxy group or a hydroxyl group,
   R⁴⁶ are preferably each a hydrogen atom, a methyl group, an ethyl group, a methoxy group or a hydroxyl group, and
   x is preferably 1 or 2;
(14) a cyclic structure represented by the following chemical formula: 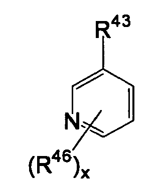 wherein
   R⁴³ is preferably a hydrogen atom, a methyl group, an ethyl group, a methoxy group or a hydroxyl group,
   R⁴⁶ are preferably each a hydrogen atom, a methyl group, an ethyl group, a methoxy group or a hydroxyl group, and
   x is preferably 1 or 2;
(15) a cyclic structure represented by the following chemical formula: 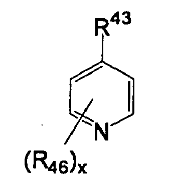 wherein
   R⁴³ is preferably a hydrogen atom, a methyl group, an ethyl group, a methoxy group or a hydroxyl group,
   R⁴⁶ are preferably each a hydrogen atom, a methyl group, an ethyl group, a methoxy group and a hydroxyl group, and
   x is preferably 1 or 2; and
(16) a cyclic structure represented by the following chemical formula: 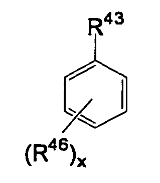 wherein
   R⁴³ is preferably a hydrogen atom, a methyl group, an ethyl group, a methoxy group or a hydroxyl group, R⁴⁶ are preferably each a hydrogen atom, a methyl group, an ethyl group, a methoxy group or a hydroxyl group, and x is preferably 1 or 2.

The specific examples of the preferable compounds of the present invention are listed below. The numbers in the brackets attached to the head of the compound names correspond to the Example numbers, respectively.
(1-1) 4-(3-methoxy-5-methyl-phenyl)-2-(5-nitro-2-trifluoromethyl-phenyl)-2,5,6,7-tetrahydro-cyclopenta[d]pyridazin-1-one,
(1-2) 2-(5-amino-2-trifluoromethyl-phenyl)-4-(3-methoxy-5-methyl-phenyl)-2,5,6,7-tetrahydro-cyclopenta[d]pyridazin-1-one,
(1-3) 2-(5-bromo-2-trifluoromethyl-phenyl)-4-(3-methoxy-5-methyl-phenyl)-2,5,6,7-tetrahydro-cyclopenta[d]pyridazin-1-one,
(1-4) 4-(3-methoxy-5-methyl-phenyl)-2-(5-pyridazin-3-yl-2-trifluoromethyl-phenyl)-2,5,6,7-tetrahydro-cyclopenta[d]pyridazin-1-one,
(1-5) 4-(3,5-dimethoxy-phenyl)-2-[5-((S)-3-hydroxy-pyrrolidin-1-yl)-2-nitro-phenyl]-2,5,6,7-tetrahydro-cyclopenta[d]pyridazin-1-one,
(1-6) 4-(3,5-dimethoxy-phenyl)-2-[2-dimethylamino-5-((S)-3-hydroxy-pyrrolidin-1-yl)-phenyl]-2,5,6,7-tetrahydro-cyclopenta[d]pyridazin-1-one,
(1-7) 4-(3,5-dimethoxy-phenyl)-2-[6-((S)-3-hydroxy-pyrrolidin-1-yl)-pyridin-2-yl]-2,5,6,7-tetrahydro-cyclopenta[d]pyridazin-1-one,
(1-8) 2-(5-bromo-2-trifluoromethyl-phenyl)-4-(3,5-dimethoxy-phenyl),-2,5,6,7-tetrahydro-cyclopenta[d]pyridazin-1-one,
(1-9) 3-[4-(3,5-dimethoxy-phenyl)-1-oxo-1,5,6,7-tetrahydro-cyclopenta[d]pyridazin-2-yl]-4-trifluoromethyl-benzoic acid methyl ester,
(1-10) 3-[4-(3,5-dimethoxy-phenyl)-1-oxo-1,5,6,7-tetrahydro-cyclopenta[d]pyridazin-2-yl]-4-trifluoromethyl-benzoic acid,
(1-11) 3-[4-(3,5-dimethoxy-phenyl)-1-oxo-1,5,6,7-tetrahydro-cyclopenta[d]pyridazin-2-yl]-N-methyl-4-trifluoromethyl-benzamide,
(1-12) 4-(3,5-dimethoxy-phenyl)-2-(5-fluoro-2-trifluoromethyl-phenyl)-2,5,6,7-tetrahydro-cyclopenta[d]pyridazin-1-one,
(1-13) 4-(3,5-dimethoxy-phenyl)-2-[5-(3-hydroxy-propoxy)-2-trifluoromethyl-phenyl]-2,5,6,7-tetrahydro-cyclopenta[d]pyridazin-1-one,
(1-14) 2-(5-bromo-2-methoxy-phenyl)-4-(3,5-dimethoxy-phenyl)-2,5,6,7-tetrahydro-cyclopenta[d]pyridazin-1-one,
(1-15) 4-(3,5-dimethoxy-phenyl)-2-[5-((S)-3-hydroxy-pyrrolidin-1-yl)-2-methoxy-phenyl]-2,5,6,7-tetrahydro-cyclopenta[d]pyridazin-1-one,
(1-16) 2-(5-bromo-2-trifluoromethyl-phenyl)-4-(thiophen-2-yl)-2,5,6,7-tetrahydro-cyclopenta[d]pyridazin-1-one,
(1-17) 2-[5-((S)-3-hydroxy-pyrrolidin-1-yl)-2-trifluoromethyl-phenyl]-4-thiophen-2-yl-2,5,6,7-tetrahydro-cyclopenta[d]pyridazin-1-one,
(1-18) 4-cyclopent-1-enyl-2-[5-((S)-3-hydroxy-pyrrolidin-1-yl)-2-trifluoromethyl-phenyl]-2,5,6,7-tetrahydro-cyclopenta[d]pyridazin-1-one,
(1-19) 4-cyclopentyl-2-[5-((S)-3-hydroxy-pyrrolidin-1-yl)-2-trifluoromethyl-phenyl]-2,5,6,7-tetrahydro-cyclopenta[d]pyridazin-1-one,
(1-20) 4-(4-fluoro-3-methyl-phenyl)-6-(5-fluoro-2-trifluoromethyl-phenyl)-1,2,3,6-tetrahydro-pyrrolo[2,3-d]pyridazin-7-one,
(1-21) methyl [4-(4-fluoro-3-methyl-phenyl)-6-(5-fluoro-2-trifluoromethyl-phenyl)-7-oxo-2,3,6,7-tetrahydro-pyrrolo[2,3-d]pyridazin-1-yl]-acetate,
(1-22) 4-(4-fluoro-3-methyl-phenyl)-6-(5-fluoro-2-trifluoromethyl-phenyl)-1-(2-hydroxy-ethyl)-1,2,3,6-tetrahydro-pyrrolo[2,3-d]pyridazin-7-one,
(1-23) 4-(4-fluoro-3-methyl-phenyl)-1-(2-hydroxyethyl)-6-[5-(3-hydroxymethyl-azetidin-1-yl)-2-trifluoromethyl-phenyl]-1,2,3,6-tetrahydro-pyrrolo[2,3-d]pyridazin-7-one,
(1-24) 6-(5-bromo-2-trifluoromethyl-phenyl)-4-(3,5-dimethyl-phenyl)-1,2,3,6-tetrahydro-pyrrolo[2,3-d]pyridazin-7-one,
(1-25) methyl [6-(5-bromo-2-trifluoromethyl-phenyl)-4-(3,5-dimethyl-phenyl)-7-oxo-2,3,6,7-tetrahydro-pyrrolo[2,3-d]pyridazin-1-yl]-acetate,
(1-26) methyl [6-[5-((R)-3-acetylamino-pyrrolidin-1-yl)-2-trifluoromethyl-phenyl]-4-(3,5-dimethyl-phenyl)-7-oxo-2,3,6,7-tetrahydro-pyrrolo[2,3-d]pyridazin-1-yl]-acetate,
(1-27) 2-[6-[5-((R)-3-acetylamino-pyrrolidin-1-yl)-2-trifluoromethyl-phenyl]-4-(3,5-dimethyl-phenyl)-7-oxo-2,3,6,7-tetrahydro-pyrrolo[2,3-d]pyridazin-1-yl]-acetamide,
(1-28) 6-(5-fluoro-2-trifluoromethyl-phenyl)-4-(5-methoxy-pyridin-3-yl)-1,2,3,6-tetrahydro-pyrrolo[2,3-d]pyridazin-7-one,
(1-29) 6-[5-((S)-3-hydroxy-pyrrolidin-1-yl)-2-trifluoromethyl-phenyl]-4-(5-methoxy-pyridin-3-yl)-7-oxo-2,3,6,7-tetrahydro-pyrrolo[2,3-d]pyridazine-1-carboxylic acid amide,
(1-30) 6-(5-bromo-2-trifluoromethyl-phenyl)-4-(3,5-dimethyl-phenyl)-2-methyl-1,2,3,6-tetrahydro-pyrrolo[2,3-d]pyridazin-7-one,
(1-31) 1-acetyl-6-(5-bromo-2-trifluoromethyl-phenyl)-4-(3,5-dimethyl-phenyl)-2-methyl-1,2,3,6-tetrahydro-pyrrolo[2,3-d]pyridazin-7-one,
(1-32) 1-acetyl-4-(3,5-dimethyl-phenyl)-6-[5-((S)-3-hydroxy-pyrrolidin-1-yl)-2-trifluoromethyl-phenyl]-2-methyl-1,2,3,6-tetrahydro-pyrrolo[2,3-d]pyridazin-7-one,
(1-33) 4-(3,5-dimethyl-phenyl)-6-[5-((S)-3-hydroxy-pyrrolidin-1-yl)-2-trifluoromethyl-phenyl]-2-methyl-1,2,3,6-tetrahydro-pyrrolo[2,3-d]pyridazin-7-one,
(1-34) 5-(5-bromo-2-trifluoromethyl-phenyl)-7-(3,5-dimethoxy-phenyl)-3,5-dihydro-2H-furo[2,3-d]pyridazin-4-one,
(1-35) 7-(3,5-dimethoxy-phenyl)-5-[5-((S)-3-hydroxy-pyrrolidin-1-yl)-2-trifluoromethyl-phenyl]-3,5-dihydro-2H-furo[2,3-d]pyridazin-4-one,
(1-36) 7-(3,5-dimethoxy-phenyl)-5-(5-fluoro-2-trifluoromethyl-phenyl)-3,5-dihydro-2H-thieno[2,3-d]pyridazin-4-one,
(1-37) 7-(3,5-dimethoxy-phenyl)-5-[5-((S)-3-hydroxy-pyrrolidin-1-yl)-2-trifluoromethyl-phenyl]-3,5-dihydro-2H-thieno[2,3-d]pyridazin-4-one,
(1-38) 7-[5-((S)-3-hydroxy-pyrrolidin-1-yl)-2-trifluoromethyl-phenyl]-5-(3-methyl-5-oxazol-5-yl-phenyl)-1,3,4,7-tetrahydro-2H-pyrido[2,3-d]pyridazin-8-one,
(1-39) 5-(3,5-dimethoxy-phenyl)-1-(2-hydroxy-ethyl)-7-[5-((S)-3-hydroxy-pyrrolidin-1-yl)-2-trifluoromethylphenyl]-1,3,4,7-tetrahydro-2H-pyrido[2,3-d]pyridazin-8-one,
(1-40) 5-(3,5-dimethoxy-phenyl)-1-(2-hydroxy-ethyl)-7-[5-(3-hydroxymethyl-azetidin-1-yl)-2-trifluoromethylphenyl]-1,3,4,7-tetrahydro-2H-pyrido[2,3-d]pyridazin-8-one,
(1-41) 6-(5-amino-2-trifluoromethyl-phenyl)-4-(3,5-dimethoxy-phenyl)-1-(2-hydroxy-ethyl)-1,2,3,6-tetrahydro-pyrrolo[2,3-d]pyridazin-7-one,
(1-42) 4-(3,5-dimethoxy-phenyl)-6-[5-(3-hydroxymethyl-azetidin-1-yl)-2-trifluoromethyl-phenyl]-1,2,3,6-tetrahydro-pyrrolo[2,3-d]pyridazin-7-one,
(1-43) 4-(3,5-dimethoxy-phenyl)-1-(2-hydroxy-ethyl)-6-[5-(3-hydroxy-2-hydroxymethyl-propylamino)-2-trifluoromethyl-phenyl]-1,2,3,6-tetrahydro-pyrrolo[2,3-d]pyridazin-7-one,
(1-44) 1-(2-hydroxy-ethyl)-4-(3-hydroxy-5-methoxyphenyl)-6-[5-(3-hydroxymethyl-azetidin-1-yl)-2-trifluoromethyl-phenyl]-1,2,3,6-tetrahydro-pyrrolo[2,3-d]pyridazin-7-one,
(1-45) 1-{3-[4-(3,5-Dimethoxy-phenyl)-1-(2-hydroxyethyl)-7-oxo-1,2,3,7-tetrahydro-pyrrolo[2,3-d]pyridazin-6-yl]-4-trifluoromethyl-phenyl}-azetidine-3-carboxylic acid,
(1-46) {4-(3,5-Dimethoxy-phenyl)-6-[5-(3-hydroxymethyl-azetidin-1-yl)-2-trifluoromethyl-phenyl]-7-oxo-2,3,6,7-tetrahydro-pyrrolo[2,3-d]pyridazin-1-yl}-acetic acid,
(2) 2-[5-((S)-3-hydroxy-pyrrolidin-1-yl)-2-trifluoromethyl-phenyl]-4-(3-methyl-5-oxazol-5-yl-phenyl)-2,5,6,7-tetrahydro-cyclopenta[d]pyridazin-1-one,
(3) 4-(3-methyl-5-oxazol-5-yl-phenyl)-2-(5-pyridazin-3-yl-2-trifluoromethyl-phenyl)-2,5,6,7-tetrahydro-cyclopenta[d]pyridazin-1-one,
(4) 4-(3,5-dimethyl-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(5) 2-methyl-4-(3-methyl-5-oxazol-5-yl-phenyl)-6-(5-pyridazin-3-yl-2-trifluoromethyl-phenyl)-1,2,3,6-tetrahydro-pyrrolo[2,3-d]pyridazin-7-one,
(6) 2-[5-((S)-3-hydroxy-pyrrolidin-1-yl)-2-trifluoromethyl-phenyl]-6,6-dimethyl-4-(3-methyl-5-oxazol-5-yl-phenyl)-2,5,6,7-tetrahydro-cyclopenta[d]pyridazin-1-one,
(7) 2-[5-((S)-3-hydroxy-pyrrolidin-1-yl)-2-trifluoromethyl-phenyl]-4-(3-methoxy-5-methyl-phenyl)-2,5,6,7-tetrahydro-cyclopenta[d]pyridazin-1-one,
(8) 4-(3,5-dimethyl-phenyl)-2-(5-pyridazin-3-yl-2-trifluoromethyl-phenyl)-2,5,6,7-tetrahydro-cyclopenta[d]pyridazin-1-one,
(9) 4-(3,5-dimethoxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(10) 4-(3,5-dimethyl-phenyl)-6-(5-pyridazin-3-yl-2-trifluoromethyl-phenyl)-1,2,3,6-tetrahydro-pyrrolo[2,3-d]pyridazin-7-one,
(11) 4-(3,5-dimethyl-phenyl)-6-[5-((S)-3-hydroxy-pyrrolidin-1-yl)-2-trifluoromethyl-phenyl]-1,2,3,6-tetrahydro-pyrrolo[2,3-d]pyridazin-7-one,
(12) 4-(3,5-dimethyl-phenyl)-2-methyl-6-(5-pyridazin-3-yl-2-trifluoromethyl-phenyl)-1,2,3,6-tetrahydro-pyrrolo[2,3-d]pyridazin-7-one,
(13) 4-(3-methoxy-5-methyl-phenyl)-6-(5-pyridazin-3-yl-2-trifluoromethyl-phenyl)-1,2,3,6-tetrahydro-pyrrolo[2,3-d]pyridazin-7-one,
(14) 6-[5-((S)-3-hydroxy-pyrrolidin-1-yl)-2-trifluoromethyl-phenyl]-4-(3-methoxy-5-methyl-phenyl)-1,2,3,6-tetrahydro-pyrrolo[2,3-d]pyridazin-7-one,
(15) 4-(3-methoxy-5-methyl-phenyl)-2-methyl-6-(5-pyridazin-3-yl-2-trifluoromethyl-phenyl)-1,2,3,6-tetrahydro-pyrrolo[2,3-d]pyridazin-7-one,
(16) 6-[5-((S)-3-hydroxy-pyrrolidin-1-yl)-2-trifluoromethyl-phenyl]-4-(3-methoxy-5-methyl-phenyl)-2-methyl-1,2,3,6-tetrahydro-pyrrolo[2,3-d]pyridazin-7-one,
(17) 4-(3,5-dimethoxy-phenyl)-6-(5-pyridazin-3-yl-2-trifluoromethyl-phenyl)-1,2,3,6-tetrahydro-pyrrolo[2,3-d]pyridazin-7-one,
(18) 4-(3,5-dimethoxy-phenyl)-6-[5-((S)-3-hydroxy-pyrrolidin-1-yl)-2-trifluoromethyl-phenyl]-1,2,3,6-tetrahydro-pyrrolo[2,3-d]pyridazin-7-one,
(19) 4-(3,5-dimethoxy-phenyl)-2-methyl-6-(5-pyridazin-3-yl-2-trifluoromethyl-phenyl)-1,2,3,6-tetrahydro-pyrrolo[2,3-d]pyridazin-7-one,
(20) 4-(3,5-dimethoxy-phenyl)-6-[5-((S)-3-hydroxy-pyrrolidin-1-yl)-2-trifluoromethyl-phenyl]-2-methyl-1,2,3,6-tetrahydro-pyrrolo[2,3-d]pyridazin-7-one,
(21) 4-(3,5-dimethoxy-phenyl)-2-[5-((R)-3-hydroxy-pyrrolidin-1-yl)-2-trifluoromethyl-phenyl]-2,5,6,7-tetrahydro-cyclopenta[d]pyridazin-1-one,
(22) 6-[5-((R)-3-hydroxy-pyrrolidin-1-yl)-2-trifluoromethyl-phenyl]-4-(3-methoxy-5-methyl-phenyl)-1,2,3,6-tetrahydro-pyrrolo[2,3-d]pyridazin-7-one,
(23) 2-[5-((R)-3-hydroxy-pyrrolidin-1-yl)-2-trifluoromethyl-phenyl]-4-(3-methoxy-5-methyl-phenyl)-2,5,6,7-tetrahydro-cyclopenta[d]pyridazin-1-one,
(24) 4-(3,5-dimethoxy-phenyl)-6-[5-((R)-3-hydroxy-pyrrolidin-1-yl)-2-trifluoromethyl-phenyl]-1,2,3,6-tetrahydro-pyrrolo[2,3-d]pyridazin-7-one,
(25) 4-(3,5-dimethoxy-phenyl)-6-[5-(3-oxo-pyrrolidin-1-yl)-2-trifluoromethyl-phenyl]-1,2,3,6-tetrahydro-pyrrolo[2,3-d]pyridazin-7-one,
(26) {4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-acetic acid methyl ester,
(27) {4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-acetic acid,
(28) 2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-acetamide,
(29) 4-(3,5-dimethoxy-phenyl)-1-(2-hydroxy-ethyl)-6-[5-((S)-3-hydroxy-pyrrolidin-1-yl)-2-trifluoromethylphenyl]-1,2,3,6-tetrahydro-pyrrolo[2,3-d]pyridazin-7-one,
(30) 4-(3,5-dimethoxy-phenyl)-1-(2-hydroxy-butyl)-6-[5-((S)-3-hydroxy-pyrrolidin-1-yl)-2-trifluoromethylphenyl]-1,2,3,6-tetrahydro-pyrrolo[2,3-d]pyridazin-7-one,
(31) 2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-N-methyl-acetamide,
(32) 4-(3,5-dimethoxy-phenyl)-6-(2-trifluoromethylphenyl)-1,2,3,6-tetrahydro-pyrrolo[2,3-d]pyridazin-7-one,
(33) 2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-N-ethyl-acetamide,
(34) N-butyl-2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-acetamide,
(35) 2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-N-hexyl-acetamide,
(36) 2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-N-(2-methoxy-ethyl)-acetamide,
(37) 4-(3,5-dimethoxy-phenyl)-6-(5-pyrrolidin-1-yl-2-trifluoromethyl-phenyl)-1,2,3,6-tetrahydro-pyrrolo[2,3-d]pyridazin-7-one,
(38) 4-(3,5-dimethoxy-phenyl)-2-[5-((S)-3-hydroxy-pyrrolidin-1-yl)-2-methanesulfonyl-phenyl]-2,5,6,7-tetrahydro-cyclopenta[d]pyridazin-1-one,
(39) N-(2-acetylamino-ethyl)-2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-acetamide,
(40) N-(6-acetylamino-hexyl)-2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-acetamide,
(41) 1-[2-(4-acetyl-piperazin-1-yl)-2-oxo-ethyl]-4-(3,5-dimethoxy-phenyl)-6-[5-((S)-3-hydroxy-pyrrolidin-1-yl)-2-trifluoromethyl-phenyl]-1,2,3,6-tetrahydro-pyrrolo[2,3-d]pyridazin-7-one,
(42) 4-(3,5-dimethoxy-phenyl)-2-[2-fluoro-5-((S)-3-hydroxy-pyrrolidin-1-yl)-phenyl]-2,5,6,7-tetrahydro-cyclopenta[d]pyridazin-1-one,
(43) 6-[5-((S)-3-hydroxy-pyrrolidin-1-yl)-2-trifluoromethyl-phenyl]-4-(3-methoxy-phenyl)-1,2,3,6-tetrahydro-pyrrolo[2,3-d]pyridazin-7-one,
(44) N-(4-acetylamino-butyl)-2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-acetamide,
(45) 2-[5-((S)-3-hydroxy-pyrrolidin-1-yl)-2-trifluoromethyl-phenyl]-4-(5-methoxy-pyridin-3-yl)-2,5,6,7-tetrahydro-cyclopenta[d]pyridazin-1-one,
(46) hexanoic acid [2-(2-{4-(3,5-dimethoxy-phenyl)-6-[5-((S)-3-hydroxy-pyrrolidin-1-yl)-2-trifluoromethylphenyl]-7-oxo-2,3,6,7-tetrahydro-pyrrolo[2,3-d]pyridazin-1-yl}-acetylamino)-ethyl]-amide,
(47) 3-{3-[4-(3,5-dimethoxy-phenyl)-7-oxo-1,2,3,7-tetrahydro-pyrrolo[2,3-d]pyridazin-6-yl]-4-trifluoromethyl-phenylamino}-propionic acid methyl ester,
(48) {4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-acetonitrile,
(49) 1-(2-amino-ethyl)-4-(3,5-dimethoxy-phenyl)-6-[5-((S)-3-hydroxy-pyrrolidin-1-yl)-2-trifluoromethylphenyl]-1,2,3,6-tetrahydro-pyrrolo[2,3-d]pyridazin-7-one,
(50) 4-(3,5-dimethoxy-phenyl)-6-(5-fluoro-2-trifluoromethyl-phenyl)-1,2,3,6-tetrahydro-pyrrolo[2,3-d]pyridazin-7-one,
(51) 6-(5-chloro-2-trifluoromethyl-phenyl)-4-(3,5-dimethoxy-phenyl)-1,2,3,6-tetrahydro-pyrrolo[2,3-d]pyridazin-7-one,
(52) 4-(3,5-dimethoxy-phenyl)-6-[3-((S)-3-hydroxy-pyrrolidin-1-yl)-phenyl]-1,2,3,6-tetrahydro-pyrrolo[2,3-d]pyridazin-7-one,
(53) 4-(3,5-dimethoxy-phenyl)-2-(5-propoxy-2-trifluoromethyl-phenyl)-2,5,6,7-tetrahydro-cyclopenta[d]pyridazin-1-one,
(54) 4-(3,5-dimethoxy-phenyl)-6-[5-((S)-3-hydroxy-pyrrolidin-1-yl)-2-trifluoromethyl-phenyl]-1-methyl-1,2,3,6-tetrahydro-pyrrolo[2,3-d]pyridazin-7-one,
(55) 4-(3,5-dimethoxy-phenyl)-1-ethyl-6-[5-((S)-3-hydroxy-pyrrolidin-1-yl)-2-trifluoromethyl-phenyl]-1,2,3,6-tetrahydro-pyrrolo[2,3-d]pyridazin-7-one,
(56) N-{3-[4-(3-acetylamino-propyl)-piperazin-1-yl]-propyl}-2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-acetamide,
(57) N-{3-[4-(3,5-dimethoxy-phenyl)-1-oxo-1,5,6,7-tetrahydro-cyclopenta[d]pyridazin-2-yl]-4-trifluoromethyl-phenyl}-propionamide,
(58) 2-[5-((S)-3-hydroxy-pyrrolidin-1-yl)-2-trifluoromethyl-phenyl]-4-(3-methoxy-phenyl)-2,5,6,7-tetrahydro-cyclopenta[d]pyridazin-1-one,
(59) 4-(3,5-dimethoxy-phenyl)-6-[5-((R)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid tert-butyl ester,
(60) 4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid ethyl ester,
(61) 4-(3,5-dihydroxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid ethyl ester,
(62) 4-(3,5-dimethoxy-phenyl)-2-(5-ethoxy-2-trifluoromethyl-phenyl)-2,5,6,7-tetrahydro-cyclopenta[d]pyridazin-1-one,
(63) 4-(3,5-dimethoxy-phenyl)-2-[5-(2-hydroxy-ethoxy)-2-trifluoromethyl-phenyl]-2,5,6,7-tetrahydro-cyclopenta[d]pyridazin-1-one,
(64) 2-[5-((S)-3-hydroxy-pyrrolidin-1-yl)-2-trifluoromethyl-phenyl]-4-m-tolyl-2,5,6,7-tetrahydro-cyclopenta[d]pyridazin-1-one,
(65) 4-(3-fluoro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(66) 2-[5-((S)-3-hydroxy-pyrrolidin-1-yl)-2-trifluoromethyl-phenyl]-4-phenyl-2,5,6,7-tetrahydro-cyclopenta[d]pyridazin-1-one,
(67) 4-(3,5-dimethoxy-phenyl)-2-(5-ethylamino-2-trifluoromethyl-phenyl)-2,5,6,7-tetrahydro-cyclopenta[d]pyridazin-1-one,
(68) 4-(3,5-dimethoxy-phenyl)-2-[5-(ethyl-methylamino)-2-trifluoromethyl-phenyl]-2,5,6,7-tetrahydro-cyclopenta[d]pyridazin-1-one,
(69) 4-(3,5-dimethoxy-phenyl)-2-(5-methoxy-2-trifluoromethyl-phenyl)-2,5,6,7-tetrahydro-cyclopenta[d]pyridazin-1-one,
(70) 4-(3,5-dimethoxy-phenyl)-2-(5-morpholin-4-yl-2-trifluoromethyl-phenyl)-2,5,6,7-tetrahydro-cyclopenta[d]pyridazin-1-one,
(71) 2-(5-azetidin-1-yl-2-trifluoromethyl-phenyl)-4-(3,5-dimethoxy-phenyl)-2,5,6,7-tetrahydro-cyclopenta[d]pyridazin-1-one,
(72) 4-(3-chloro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(73) 4-(3-bromo-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(74) 2-[5-((S)-3-hydroxy-pyrrolidin-1-yl)-2-trifluoromethyl-phenyl]-4-(3-trifluoromethyl-phenyl)-2,5,6,7-tetrahydro-cyclopenta[d]pyridazin-1-one,
(75) 2-[5-((S)-3-hydroxy-pyrrolidin-1-yl)-2-trifluoromethyl-phenyl]-4-(4-trifluoromethyl-phenyl)-2,5,6,7-tetrahydro-cyclopenta[d]pyridazin-1-one,
(76) 4-(4-fluoro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(77) 2-[5-((S)-3-hydroxy-pyrrolidin-1-yl)-2-trifluoromethyl-phenyl]-4-(4-methoxy-phenyl)-2,5,6,7-tetrahydro-cyclopenta[d]pyridazin-1-one,
(78) 4-(4-chloro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(79) 3-{3-[5-((S)-3-hydroxy-pyrrolidin-1-yl)-2-trifluoromethyl-phenyl]-4-oxo-4,5,6,7-tetrahydro-3H-cyclopenta[d]pyridazin-1-yl}-benzoic acid,
(80) 4-{3-[5-((S)-3-hydroxy-pyrrolidin-1-yl)-2-trifluoromethyl-phenyl]-4-oxo-4,5,6,7-tetrahydro-3H-cyclopenta[d]pyridazin-1-yl}-benzoic acid,
(81) 4-(3,5-dimethoxy-phenyl)-2-(5-dimethylamino-2-trifluoromethyl-phenyl)-2,5,6,7-tetrahydro-cyclopenta[d]pyridazin-1-one,
(82) 4-(3,5-dimethoxy-phenyl)-2-(5-methylamino-2-trifluoromethyl-phenyl)-2,5,6,7-tetrahydro-cyclopenta[d]pyridazin-1-one,
(83) N-{3-[4-(3,5-dimethoxy-phenyl)-1-oxo-1,5,6,7-tetrahydro-cyclopenta[d]pyridazin-2-yl]-4-trifluoromethyl-phenyl}-2-hydroxy-acetamide,
(84) N-{3-[4-(3,5-dimethoxy-phenyl)-1-oxo-1,5,6,7-tetrahydro-cyclopenta[d]pyridazin-2-yl]-4-trifluoromethyl-phenyl}-butyramide,
(85) 4-(3-dimethylamino-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(86) 2-(2-chloro-phenyl)-4-(3,5-dimethoxy-phenyl)-2,5,6,7-tetrahydro-cyclopenta[d]pyridazin-1-one,
(87) 2-(2,6-dichloro-phenyl)-4-(3,5-dimethoxy-phenyl)-2,5,6,7-tetrahydro-cyclopenta[d]pyridazin-1-one,
(88) 4-(3,5-dimethoxy-phenyl)-2-(5-methyl-2-trifluoromethyl-phenyl)-2,5,6,7-tetrahydro-cyclopenta[d]pyridazin-1-one,
(89) 4-(3,5-dimethoxy-phenyl)-2-(5-ethyl-2-trifluoromethyl-phenyl)-2,5,6,7-tetrahydro-cyclopenta[d]pyridazin-1-one,
(90) 4-(4-bromo-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(91) 4-(3,5-dimethoxy-phenyl)-2-[5-(2-hydroxy-ethylamino)-2-trifluoromethyl-phenyl]-2,5,6,7-tetrahydro-cyclopenta[d]pyridazin-1-one,
(92) 4-(3,5-dimethoxy-phenyl)-2-{5-[(2-hydroxy-ethyl)-methyl-amino]-2-trifluoromethyl-phenyl}-2,5,6,7-tetrahydro-cyclopenta[d]pyridazin-1-one,
(93) 2-[5-((S)-3-hydroxy-pyrrolidin-1-yl)-2-trifluoromethyl-phenyl]-4-(2-methoxy-pyridin-4-yl)-2,5,6,7-tetrahydro-cyclopenta[d]pyridazin-1-one,
(94) {3-[4-(3,5-dimethoxy-phenyl)-1-oxo-1,5,6,7-tetrahydro-cyclopenta[d]pyridazin-2-yl]-4-trifluoromethyl-phenylamino}-acetic acid tert-butyl ester,
(95) 2-[5-((S)-3-hydroxy-pyrrolidin-1-yl)-2-trifluoromethyl-phenyl]-4-pyridin-4-yl-2,5,6,7-tetrahydro-cyclopenta[d]pyridazin-1-one,
(96) 4-(2-chloro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(97) {3-[4-(3,5-dimethoxy-phenyl)-1-oxo-1,5,6,7-tetrahydro-cyclopenta[d]pyridazin-2-yl]-4-trifluoromethyl-phenylamino}-acetic acid,
(98) 3-{3-[5-((S)-3-hydroxy-pyrrolidin-1-yl)-2-trifluoromethyl-phenyl]-4-oxo-4,5,6,7-tetrahydro-3H-cyclopenta[d]pyridazin-1-yl}-benzamide,
(99) 4-{3-[5-((S)-3-hydroxy-pyrrolidin-1-yl)-2-trifluoromethyl-phenyl]-4-oxo-4,5,6,7-tetrahydro-3H-cyclopenta[d]pyridazin-1-yl}-benzamide,
(100) 4-(3,5-dimethoxy-phenyl)-2-(5-propylamino-2-trifluoromethyl-phenyl)-2,5,6,7-tetrahydro-cyclopenta[d]pyridazin-1-one,
(101) 4-(3,5-dimethoxy-phenyl)-2-[5-(methyl-propylamino)-2-trifluoromethyl-phenyl]-2,5,6,7-tetrahydro-cyclopenta[d]pyridazin-1-one,
(102) 1-benzyl-4-(3,5-dimethoxy-phenyl)-6-[5-((S)-3-hydroxy-pyrrolidin-1-yl)-2-trifluoromethyl-phenyl]-1,2,3,6-tetrahydro-pyrrolo[2,3-d]pyridazin-7-one,
(103) 2-[5-((S)-3-hydroxy-pyrrolidin-1-yl)-2-trifluoromethyl-phenyl]-4-pyridin-3-yl-2,5,6,7-tetrahydro-cyclopenta[d]pyridazin-1-one,
(104) 4-(2-bromo-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(105) 4-(2-fluoro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(106) 2-(4-bromo-2-trifluoromethyl-phenyl)-4-(3,5-dimethoxy-phenyl)-2,5,6,7-tetrahydro-cyclopenta[d]pyridazin-1-one,
(107) 4-(3,5-dimethoxy-phenyl)-2-(2-trifluoromethylphenyl)-2,5,6,7-tetrahydro-cyclopenta[d]pyridazin-1-one,
(108) 4-(3,5-dimethoxy-phenyl)-2-(4-dimethylamino-2-trifluoromethyl-phenyl)-2,5,6,7-tetrahydro-cyclopenta[d]pyridazin-1-one,
(109) 2-[5-((S)-3-hydroxy-pyrrolidin-1-yl)-2-trifluoromethyl-phenyl]-4-(2-methoxy-phenyl)-2,5,6,7-tetrahydro-cyclopenta[d]pyridazin-1-one,
(110) 2-[5-((S)-3-hydroxy-pyrrolidin-1-yl)-2-trifluoromethyl-phenyl]-4-pyridin-2-yl-2,5,6,7-tetrahydro-cyclopenta[d]pyridazin-1-one,
(111) 4-(2-hydroxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(112) 4-(4-dimethylamino-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(113) 4-cyclohex-1-enyl-2-[5-((S)-3-hydroxy-pyrrolidin-1-yl)-2-trifluoromethyl-phenyl]-2,5,6,7-tetrahydro-cyclopenta[d]pyridazin-1-one,
(114) 2-[5-((S)-3-hydroxy-pyrrolidin-1-yl)-2-trifluoromethyl-phenyl]-4-p-tolyl-2,5,6,7-tetrahydro-cyclopenta[d]pyridazin-1-one,
(115) 3-{3-[4-(3,5-dimethoxy-phenyl)-1-oxo-1,5,6,7-tetrahydro-cyclopenta[d]pyridazin-2-yl]-4-trifluoromethyl-phenylamino}-propionic acid tert-butyl ester,
(116) 4-(3,5-dimethoxy-phenyl)-2-[5-(2-dimethylamino-ethylamino)-2-trifluoromethyl-phenyl]-2,5,6,7-tetrahydro-cyclopenta[d]pyridazin-1-one,
(117) N-(2-{3-[4-(3,5-dimethoxy-phenyl)-1-oxo-1,5,6,7-tetrahydro-cyclopenta[d]pyridazin-2-yl]-4-trifluoromethyl-phenylamino}-ethyl)-acetamide,
(118) (2-{3-[4-(3,5-dimethoxy-phenyl)-1-oxo-1,5,6,7-tetrahydro-cyclopenta[d]pyridazin-2-yl]-4-trifluoromethyl-phenylamino}-ethyl)-carbamic acid tert-butyl ester,
(119) 2-[5-(2-amino-ethylamino)-2-trifluoromethylphenyl]-4-(3,5-dimethoxy-phenyl)-2,5,6,7-tetrahydro-cyclopenta[d]pyridazin-1-one hydrochloride,
(120) 4-(3,5-dimethoxy-phenyl)-2-[5-(3-hydroxy-prop-1-ynyl)-2-trifluoromethyl-phenyl]-2,5,6,7-tetrahydro-cyclopenta[d]pyridazin-1-one,
(121) 4-(3,5-dimethoxy-phenyl)-2-[5-(3-hydroxy-propyl)-2-trifluoromethyl-phenyl]-2,5,6,7-tetrahydro-cyclopenta[d]pyridazin-1-one,
(122) 3-{3-[4-(3,5-dimethoxy-phenyl)-1-oxo-1,5,6,7-tetrahydro-cyclopenta[d]pyridazin-2-yl]-4-trifluoromethyl-phenylamino}-propionic acid,
(123) 2-{3-[4-(3,5-dimethoxy-phenyl)-1-oxo-1,5,6,7-tetrahydro-cyclopenta[d]pyridazin-2-yl]-4-trifluoromethyl-phenylamino}-acetamide,
(124) 2-{3-[4-(3,5-dimethoxy-phenyl)-1-oxo-1,5,6,7-tetrahydro-cyclopenta[d]pyridazin-2-yl]-4-trifluoromethyl-phenylamino}-N-methyl-acetamide,
(125) 2-{3-[4-(3,5-dimethoxy-phenyl)-1-oxo-1,5,6,7-tetrahydro-cyclopenta[d]pyridazin-2-yl]-4-trifluoromethyl-phenylamino}-N,N-dimethyl-acetamide,
(126) {3-[4-(3,5-dimethoxy-phenyl)-1-oxo-1,5,6,7-tetrahydro-cyclopenta[d]pyridazin-2-yl]-4-trifluoromethyl-phenylamino}-acetic acid methyl ester,
(127) 2-[5-(3-amino-propylamino)-2-trifluoromethylphenyl]-4-(3,5-dimethoxy-phenyl)-2,5,6,7-tetrahydro-cyclopenta[d]pyridazin-1-one hydrochloride,
(128) 4-(3,5-dimethoxy-phenyl)-2-[5-(3-dimethylamino-propylamino)-2-trifluoromethyl-phenyl]-2,5,6,7-tetrahydro-cyclopenta[d]pyridazin-1-one,
(129) (3-{3-[4-(3,5-dimethoxy-phenyl)-1-oxo-1,5,6,7-tetrahydro-cyclopenta[d]pyridazin-2-yl]-4-trifluoromethyl-phenylamino}-propyl)-carbamic acid tert-butyl ester,
(130) 4-(3,5-dimethoxy-phenyl)-2-[5-(3-hydroxy-propylamino)-2-trifluoromethyl-phenyl]-2,5,6,7-tetrahydro-cyclopenta[d]pyridazin-1-one,
(131) 4-(3,5-dimethoxy-phenyl)-2-[5-(2-hydroxy-1-methyl-ethylamino)-2-trifluoromethyl-phenyl]-2,5,6,7-tetrahydro-cyclopenta[d]pyridazin-1-one,
(132) 4-(3,5-dimethoxy-phenyl)-2-[5-(2-hydroxy-propylamino)-2-trifluoromethyl-phenyl]-2,5,6,7-tetrahydro-cyclopenta[d]pyridazin-1-one,
(133) 2-[5-((S)-3-hydroxy-pyrrolidin-1-yl)-2-trifluoromethyl-phenyl]-4-o-tolyl-2,5,6,7-tetrahydro-cyclopenta[d]pyridazin-1-one,
(134) 4-(3,5-dimethoxy-phenyl)-2-(5-phenylamino-2-trifluoromethyl-phenyl)-2,5,6,7-tetrahydro-cyclopenta[d]pyridazin-1-one,
(135) 2-(5-benzylamino-2-trifluoromethyl-phenyl)-4-(3,5-dimethoxy-phenyl)-2,5,6,7-tetrahydro-cyclopenta[d]pyridazin-1-one,
(136) 4-(3,5-dimethoxy-phenyl)-2-(5-phenethylamino-2-trifluoromethyl-phenyl)-2,5,6,7-tetrahydro-cyclopenta[d]pyridazin-1-one,
(137) N-(2-{3-[4-(3,5-dimethoxy-phenyl)-1-oxo-1,5,6,7-tetrahydro-cyclopenta[d]pyridazin-2-yl]-4-trifluoromethyl-phenylamino}-ethyl)-methanesulfonamide,
(138) 3-{3-[4-(3,5-dimethoxy-phenyl)-1-oxo-1,5,6,7-tetrahydro-cyclopenta[d]pyridazin-2-yl]-4-trifluoromethyl-phenylamino}-N-methyl-propionamide,
(139) 3-{3-[4-(3,5-dimethoxy-phenyl)-1-oxo-1,5,6,7-tetrahydro-cyclopenta[d]pyridazin-2-yl]-4-trifluoromethyl-phenylamino}-N,N-dimethyl-propionamide,
(140) 2-(5-cyclopropylamino-2-trifluoromethyl-phenyl)-4-(3,5-dimethoxy-phenyl)-2,5,6,7-tetrahydro-cyclopenta[d]pyridazin-1-one,
(141) 2-[5-(cyclopropylmethyl-amino)-2-trifluoromethylphenyl]-4-(3,5-dimethoxy-phenyl)-2,5,6,7-tetrahydro-cyclopenta[d]pyridazin-1-one,
(142) 3-{3-[4-(3,5-dimethoxy-phenyl)-1-oxo-1,5,6,7-tetrahydro-cyclopenta[d]pyridazin-2-yl]-4-trifluoromethyl-phenylamino}-propionamide,
(143) 3-{3-[4-(3,5-dimethoxy-phenyl)-1-oxo-1,5,6,7-tetrahydro-cyclopenta[d]pyridazin-2-yl]-4-trifluoromethyl-phenylamino}-propionic acid methyl ester,
(144) 2-[5-((S)-3-hydroxy-pyrrolidin-1-yl)-2-trifluoromethyl-phenyl]-4-isobutyl-2,5,6,7-tetrahydro-cyclopenta[d]pyridazin-1-one,
(145) 4-(3,5-dimethoxy-phenyl)-2-{5-[(3-hydroxypropyl)-methyl-amino]-2-trifluoromethyl-phenyl}-2,5,6,7-tetrahydro-cyclopenta[d]pyridazin-1-one,
(146) 2-(5-cyclopentylamino-2-trifluoromethyl-phenyl)-4-(3,5-dimethoxy-phenyl)-2,5,6,7-tetrahydro-cyclopenta[d]pyridazin-1-one,
(147) 2-[5-(2-cyclopropyl-ethylamino)-2-trifluoromethyl-phenyl]-4-(3,5-dimethoxy-phenyl)-2,5,6,7-tetrahydro-cyclopenta[d]pyridazin-1-one,
(148) 2-[5-(cyclopentylmethyl-amino)-2-trifluoromethylphenyl]-4-(3,5-dimethoxy-phenyl)-2,5,6,7-tetrahydro-cyclopenta[d]pyridazin-1-one,
(149) 4-(3,5-dimethoxy-phenyl)-2-(5-hydroxymethyl-2-trifluoromethyl-phenyl)-2,5,6,7-tetrahydro-cyclopenta[d]pyridazin-1-one,
(150) 4-(3,5-dimethoxy-phenyl)-2-[5-(2-oxo-pyrrolidin-1-yl)-2-trifluoromethyl-phenyl]-2,5,6,7-tetrahydro-cyclopenta[d]pyridazin-1-one,
(151) 4-{3-[4-(3,5-dimethoxy-phenyl)-1-oxo-1,5,6,7-tetrahydro-cyclopenta[d]pyridazin-2-yl]-4-trifluoromethyl-phenylamino}-butyric acid,
(152) 4-{3-[4-(3,5-dimethoxy-phenyl)-1-oxo-1,5,6,7-tetrahydro-cyclopenta[d]pyridazin-2-yl]-4-trifluoromethyl-phenylamino}-butyramide,
(153) 4-{3-[4-(3,5-dimethoxy-phenyl)-1-oxo-1,5,6,7-tetrahydro-cyclopenta[d]pyridazin-2-yl]-4-trifluoromethyl-phenylamino}-N-methyl-butyramide,
(154) 4-{3-[4-(3,5-dimethoxy-phenyl)-1-oxo-1,5,6,7-tetrahydro-cyclopenta[d]pyridazin-2-yl]-4-trifluoromethyl-phenylamino}-N,N-dimethyl-butyramide,
(155) 4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid isopropyl ester,
(156) (2-{3-[4-(3,5-dimethoxy-phenyl)-1-oxo-1,5,6,7-tetrahydro-cyclopenta[d]pyridazin-2-yl]-4-trifluoromethyl-phenylamino}-ethyl)-methyl-carbamic acid tert-butyl ester,
(157) 4-(3,5-dimethoxy-phenyl)-2-[5-(2-methylamino-ethylamino)-2-trifluoromethyl-phenyl]-2,5,6,7-tetrahydro-cyclopenta[d]pyridazin-1-one hydrochloride,
(158) 2-[5-((S)-3-hydroxy-pyrrolidin-1-yl)-2-trifluoromethyl-phenyl]-4-thiophen-3-yl-2,5,6,7-tetrahydro-cyclopenta[d]pyridazin-1-one,
(159) N-{3-[4-(3,5-dimethoxy-phenyl)-1-oxo-1,5,6,7-tetrahydro-cyclopenta[d]pyridazin-2-yl]-4-trifluoromethyl-phenyl}-acetamide,
(160) 4-(3,5-dimethoxy-phenyl)-2-(5-dimethylaminomethyl-2-trifluoromethyl-phenyl)-2,5,6,7-tetrahydro-cyclopenta[d]pyridazin-1-one hydrochloride,
(161) 2-(5-amino-2-trifluoromethyl-phenyl)-4-(3,5-dimethoxy-phenyl)-2,5,6,7-tetrahydro-cyclopenta[d]pyridazin-1-one,
(162) 1-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-propane-1,2-dione,
(163) 2-[5-(2-cyclopentyl-ethylamino)-2-trifluoromethyl-phenyl]-4-(3,5-dimethoxy-phenyl)-2,5,6,7-tetrahydro-cyclopenta[d]pyridazin-1-one,
(164) 4-(3,5-dimethoxy-phenyl)-2-[5-(3-hydroxy-3-methyl-pyrrolidin-1-yl)-2-trifluoromethyl-phenyl]-2,5,6,7-tetrahydro-cyclopenta[d]pyridazin-1-one,
(165) 2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-N,N-dimethyl-acetamide,
(166) 4-(3,5-dimethoxy-phenyl)-1-[2-(4-hydroxy-piperidin-1-yl)-2-oxo-ethyl]-6-[5-((S)-3-hydroxy-pyrrolidin-1-yl)-2-trifluoromethyl-phenyl]-1,2,3,6-tetrahydro-pyrrolo[2,3-d]pyridazin-7-one,
(167) 4-(3,5-dimethoxy-phenyl)-6-[5-((S)-3-hydroxy-pyrrolidin-1-yl)-2-trifluoromethyl-phenyl]-1-(2-oxo-2-piperidin-1-yl-ethyl)-1,2,3,6-tetrahydro-pyrrolo[2,3-d]pyridazin-7-one,
(168) N-(3-{3-[4-(3,5-dimethoxy-phenyl)-1-oxo-1,5,6,7-tetrahydro-cyclopenta[d]pyridazin-2-yl]-4-trifluoromethyl-phenylamino}-propyl)-acetamide,
(169) N-(3-{3-[4-(3,5-dimethoxy-phenyl)-1-oxo-1,5,6,7-tetrahydro-cyclopenta[d]pyridazin-2-yl]-4-trifluoromethyl-phenylamino}-propyl)-methanesulfonamide,
(170) 4-(3,5-dimethoxy-phenyl)-6-[5-((S)-3-hydroxy-pyrrolidin-1-yl)-2-trifluoromethyl-phenyl]-1-[2-(4-methyl-piperazin-1-yl)-2-oxo-ethyl]-1,2,3,6-tetrahydro-pyrrolo[2,3-d]pyridazin-7-one,
(171) 4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid methyl ester,
(172) 4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid propyl ester,
(173) N-(2-amino-ethyl)-2-{4-(3,5-dimethoxy-phenyl)-6-[5-((S)-3-hydroxy-pyrrolidin-1-yl)-2-trifluoromethylphenyl]-7-oxo-2,3,6,7-tetrahydro-pyrrolo[2,3-d]pyridazin-1-yl}-acetamide,
(174) 4-(3,5-dimethoxy-phenyl)-6-[5-((S)-3-hydroxy-pyrrolidin-1-yl)-2-trifluoromethyl-phenyl]-1-(2-morpholin-4-yl-2-oxo-ethyl)-1,2,3,6-tetrahydro-pyrrolo[2,3-d]pyridazin-7-one,
(175) 2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-N,N-diethyl-acetamide,
(176) 2-[5-(3,3-difluoro-pyrrolidin-1-yl)-2-trifluoromethyl-phenyl]-4-(3,5-dimethoxy-phenyl)-2,5,6,7-tetrahydro-cyclopenta[d]pyridazin-1-one,
(177) N-(1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-acetamide,
(178) N-(1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-N-methyl-acetamide,
(179) 2-[2-amino-5-((S)-3-hydroxy-pyrrolidin-1-yl)-phenyl]-4-(3,5-dimethoxy-phenyl)-2,5,6,7-tetrahydro-cyclopenta[d]pyridazin-1-one,
(180) 3-[4-(3,5-dimethoxy-phenyl)-1-oxo-1,5,6,7-tetrahydro-cyclopenta[d]pyridazin-2-yl]-4-trifluoromethyl-benzamide,
(181) 3-[4-(3,5-dimethoxy-phenyl)-1-oxo-1,5,6,7-tetrahydro-cyclopenta[d]pyridazin-2-yl]-N,N-dimethyl-4-trifluoromethyl-benzamide,
(182) N-{3-[4-(3,5-dimethoxy-phenyl)-1-oxo-1,5,6,7-tetrahydro-cyclopenta[d]pyridazin-2-yl]-4-trifluoromethyl-phenyl}-N-methyl-acetamide,
(183) 4-(3,5-dimethoxy-phenyl)-2-(5-pyrrolidin-1-yl-2-trifluoromethyl-phenyl)-2,5,6,7-tetrahydro-cyclopenta[d]pyridazin-1-one,
(184) 4-(3,5-dimethoxy-phenyl)-2-(5-piperidin-1-yl-2-trifluoromethyl-phenyl)-2,5,6,7-tetrahydro-cyclopenta[d]pyridazin-1-one,
(185) N-(2-{4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-ethyl)-acetamide,
(186) 4-(3,5-dimethoxy-phenyl)-6-[5-((S)-3-hydroxy-pyrrolidin-1-yl)-2-trifluoromethyl-phenyl]-1-methanesulfonyl-1,2,3,6-tetrahydro-pyrrolo[2,3-d]pyridazin-7-one,
(187) 4-(3,5-dimethoxy-phenyl)-1-(2-hydroxy-2-methylpropyl)-6-[5-((S)-3-hydroxy-pyrrolidin-1-yl)-2-trifluoromethyl-phenyl]-1,2,3,6-tetrahydro-pyrrolo[2,3-d]pyridazin-7-one,
(188) 4-(3,5-dimethoxy-phenyl)-2-[5-(4-hydroxy-butylamino)-2-trifluoromethyl-phenyl]-2,5,6,7-tetrahydro-cyclopenta[d]pyridazin-1-one,
(189) 4-(3,5-dimethoxy-phenyl)-2-[5-(3-hydroxymethyl-pyrrolidin-1-yl)-2-trifluoromethyl-phenyl]-2,5,6,7-tetrahydro-cyclopenta[d]pyridazin-1-one,
(190) 4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid dimethylamide,
(191) acetic acid 2-{4-(3,5-dimethoxy-phenyl)-6-[5-((S)-3-hydroxy-pyrrolidin-1-yl)-2-trifluoromethylphenyl]-7-oxo-2,3,6,7-tetrahydro-pyrrolo[2,3-d]pyridazin-1-yl}-2-oxo-ethyl ester,
(192) 2-[5-(4-acetyl-piperazin-1-yl)-2-trifluoromethylphenyl]-4-(3,5-dimethoxy-phenyl)-2,5,6,7-tetrahydro-cyclopenta[d]pyridazin-1-one,
(193) 2-[5-(4-amino-butylamino)-2-trifluoromethylphenyl]-4-(3,5-dimethoxy-phenyl)-2,5,6,7-tetrahydro-cyclopenta[d]pyridazin-1-one hydrochloride,
(194) 4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid amide,
(195) 4-(3,5-dimethoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid methylamide,
(196) 1-acetyl-4-(3,5-dimethoxy-phenyl)-6-[5-((S)-3-hydroxy-pyrrolidin-1-yl)-2-trifluoromethyl-phenyl]-1,2,3,6-tetrahydro-pyrrolo[2,3-d]pyridazin-7-one,
(197) 4-(3,5-dimethoxy-phenyl)-2-(5-methylaminomethyl-2-trifluoromethyl-phenyl)-2,5,6,7-tetrahydro-cyclopenta[d]pyridazin-1-one,
(198) 4-(3,5-dimethoxy-phenyl)-2-(5-isopropylamino-2-trifluoromethyl-phenyl)-2,5,6,7-tetrahydro-cyclopenta[d]pyridazin-1-one,
(199) 4-(3,5-dimethoxy-phenyl)-2-[5-(5-hydroxy-pentylamino)-2-trifluoromethyl-phenyl]-2,5,6,7-tetrahydro-cyclopenta[d]pyridazin-1-one,
(200) N-(1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-ylmethyl)-acetamide,
(201) 4-(3,5-dimethoxy-phenyl)-2-[5-((S)-2-hydroxymethyl-pyrrolidin-1-yl)-2-trifluoromethylphenyl]-2,5,6,7-tetrahydro-cyclopenta[d]pyridazin-1-one,
(202) 4-(3,5-dimethoxy-phenyl)-2-[5-(4-hydroxy-piperidin-1-yl)-2-trifluoromethyl-phenyl]-2,5,6,7-tetrahydro-cyclopenta[d]pyridazin-1-one,
(203) 1-(2-benzyloxy-acetyl)-4-(3,5-dimethoxy-phenyl)-6-[5-((S)-3-hydroxy-pyrrolidin-1-yl)-2-trifluoromethylphenyl]-1,2,3,6-tetrahydro-pyrrolo[2,3-d]pyridazin-7-one,
(204) 4-(3,5-dimethoxy-phenyl)-6-[5-((S)-3-hydroxy-pyrrolidin-1-yl)-2-trifluoromethyl-phenyl]-1-propionyl-1,2,3,6-tetrahydro-pyrrolo[2,3-d]pyridazin-7-one,
(205) 4-(3,5-dimethoxy-phenyl)-1-(2-hydroxy-acetyl)-6-[5-((S)-3-hydroxy-pyrrolidin-1-yl)-2-trifluoromethylphenyl]-1,2,3,6-tetrahydro-pyrrolo[2,3-d]pyridazin-7-one,
(206) 4-(3,5-dimethoxy-phenyl)-2-(5-thiophen-2-yl-2-trifluoromethyl-phenyl)-2,5,6,7-tetrahydro-cyclopenta[d]pyridazin-1-one,
(207) 4-(3,5-dimethoxy-phenyl)-2-(5-pyrrol-1-yl-2-trifluoromethyl-phenyl)-2,5,6,7-tetrahydro-cyclopenta[d]pyridazin-1-one,
(208) 4-(3,5-dimethoxy-phenyl)-2-[5-((S)-3-hydroxy-pyrrolidin-1-yl)-2-methylamino-phenyl]-2,5,6,7-tetrahydro-cyclopenta[d]pyridazin-1-one,
(209) methyl-carbamic acid (S)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl ester,
(210) 4-(3,5-dimethoxy-phenyl)-1-(2-hydroxy-ethyl)-6-(5-pyrrolidin-1-yl-2-trifluoromethyl-phenyl)-1,2,3,6-tetrahydro-pyrrolo[2,3-d]pyridazin-7-one,
(211) 4-(4-fluoro-3-methoxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(212) 4-(3,4-dimethoxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(213) 4-(3,5-dimethoxy-phenyl)-2-[2-trifluoromethyl-5-(3-trifluoromethyl-pyrrolidin-1-yl)-phenyl]-2,5,6,7-tetrahydro-cyclopenta[d]pyridazin-1-one,
(214) 4-(3,5-dimethoxy-phenyl)-2-[5-(3-methoxy-pyrrolidin-1-yl)-2-trifluoromethyl-phenyl]-2,5,6,7-tetrahydro-cyclopenta[d]pyridazin-1-one,
(215) 4-(3,5-dimethoxy-phenyl)-2-{5-[2-(2-oxopyrrolidin-1-yl)-ethylamino]-2-trifluoromethyl-phenyl}-2,5,6,7-tetrahydro-cyclopenta[d]pyridazin-1-one,
(216) 4-(3,5-dimethoxy-phenyl)-6-[5-((S)-3-hydroxy-pyrrolidin-1-yl)-2-trifluoromethyl-phenyl]-2,2-dimethyl-7-oxo-2,3,6,7-tetrahydro-pyrrolo[2,3-d]pyridazine-1-carboxylic acid methyl ester,
(217) 4-(3,5-dimethoxy-phenyl)-2-(4-trifluoromethyl-biphenyl-3-yl)-2,5,6,7-tetrahydro-cyclopenta[d]pyridazin-1-one,
(218) 4-(3,5-dimethoxy-phenyl)-2-(5-oxazol-5-yl-2-trifluoromethyl-phenyl)-2,5,6,7-tetrahydro-cyclopenta[d]pyridazin-1-one,
(219) 4-(2,3-dihydro-benzofuran-5-yl)-2-[5-((S)-3-hydroxy-pyrrolidin-1-yl)-2-trifluoromethyl-phenyl]-2,5,6,7-tetrahydro-cyclopenta[d]pyridazin-1-one,
(220) 4-benzo[1,3]dioxol-5-yl-2-[5-((S)-3-hydroxy-pyrrolidin-1-yl)-2-trifluoromethyl-phenyl]-2,5,6,7-tetrahydro-cyclopenta[d]pyridazin-1-one,
(221) 4-(2,3-dihydro-benzo[1,4]dioxin-6-yl)-2-[5-((S)-3-hydroxy-pyrrolidin-1-yl)-2-trifluoromethyl-phenyl]-2,5,6,7-tetrahydro-cyclopenta[d]pyridazin-1-one,
(222) 4-(3,4-dichloro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(223) 4-(3-chloro-4-methoxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(224) 4-(3-chloro-4-fluoro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(225) 4-(3,5-dimethoxy-phenyl)-6-[5-((S)-3-hydroxy-pyrrolidin-1-yl)-2-trifluoromethyl-phenyl]-2,2-dimethyl-1,2,3,6-tetrahydro-pyrrolo[2,3-d]pyridazin-7-one,
(226) 6-[5-(3,3-difluoro-pyrrolidin-1-yl)-2-trifluoromethyl-phenyl]-4-(3,5-dimethoxy-phenyl)-1-(2-hydroxy-ethyl)-1,2,3,6-tetrahydro-pyrrolo[2,3-d]pyridazin-7-one,
(227) 4-(3,5-dimethoxy-phenyl)-2-[5-(3-oxo-piperazin-1-yl)-2-trifluoromethyl-phenyl]-2,5,6,7-tetrahydro-cyclopenta[d]pyridazin-1-one,
(228) 4-(3-fluoro-4-methoxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(229) 4-(4-chloro-3-methoxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(230) 1-{3-[4-(3,5-dimethoxy-phenyl)-1-oxo-1,5,6,7-tetrahydro-cyclopenta[d]pyridazin-2-yl]-4-trifluoromethyl-phenyl}-azetidine-3-carboxylic acid methylamide,
(231) 4-(3,5-dimethoxy-phenyl)-2-[5-(3-hydroxymethyl-azetidin-1-yl)-2-trifluoromethyl-phenyl]-2,5,6,7-tetrahydro-cyclopenta[d]pyridazin-1-one,
(232) 4-{3-[4-(3,5-dimethoxy-phenyl)-1-oxo-1,5,6,7-tetrahydro-cyclopenta[d]pyridazin-2-yl]-4-trifluoromethyl-phenyl}-piperazine-1-carboxylic acid methylamide,
(233) N-(1-{3-[4-(3,5-dimethoxy-phenyl)-1-oxo-1,5,6,7-tetrahydro-cyclopenta[d]pyridazin-2-yl]-4-trifluoromethyl-phenyl}-azetidin-3-ylmethyl)-acetamide,
(234) 4-(3,5-dimethoxy-phenyl)-6-[5-((S)-3-hydroxy-pyrrolidin-1-yl)-2-trifluoromethyl-phenyl]-1-(2-methoxy-ethyl)-1,2,3,6-tetrahydro-pyrrolo[2,3-d]pyridazin-7-one,
(235) 1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidine-3-carboxylic acid methyl ester,
(236) 1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidine-3-carboxylic acid,
(237) 4-(3,5-dimethoxy-phenyl)-6-[5-((S)-3-hydroxy-pyrrolidin-1-yl)-2-trifluoromethyl-phenyl]-1-(2-oxopropyl)-1,2,3,6-tetrahydro-pyrrolo[2,3-d]pyridazin-7-one,
(238) 1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidine-3-carboxylic acid methylamide,
(239) 5-{3-[4-(3,5-dimethoxy-phenyl)-1-oxo-1,5,6,7-tetrahydro-cyclopenta[d]pyridazin-2-yl]-4-trifluoromethyl-phenylamino}-pentanoic acid tert-butyl ester,
(240) 4-(4-fluoro-3-methyl-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(241) 1-(2-amino-acetyl)-4-(3,5-dimethoxy-phenyl)-6-[5-((S)-3-hydroxy-pyrrolidin-1-yl)-2-trifluoromethylphenyl]-1,2,3,6-tetrahydro-pyrrolo[2,3-d]pyridazin-7-one,
(242) 4-(3,4-difluoro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(243) 4-(4-chloro-3-fluoro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(244) 5-{3-[4-(3,5-dimethoxy-phenyl)-1-oxo-1,5,6,7-tetrahydro-cyclopenta[d]pyridazin-2-yl]-4-trifluoromethyl-phenylamino}-pentanoic acid amide,
(245) 4-(3-bromo-4-fluoro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(246) 4-(3-bromo-4-methoxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(247) 4-(3,5-dimethoxy-phenyl)-2-[5-(3-dimethylamino-pyrrolidin-1-yl)-2-trifluoromethyl-phenyl]-2,5,6,7-tetrahydro-cyclopenta[d]pyridazin-1-one,
(248) 4-(3,5-dimethoxy-phenyl)-2-[5-(2-oxo-piperidin-1-yl)-2-trifluoromethyl-phenyl]-2,5,6,7-tetrahydro-cyclopenta[d]pyridazin-1-one,
(249) 4-(4-chloro-3-dimethylamino-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(250) 4-(3,5-dimethoxy-phenyl)-1-(2-hydroxy-ethyl)-6-[5-(3-hydroxy-3-methyl-pyrrolidin-1-yl)-2-trifluoromethyl-phenyl]-1,2,3,6-tetrahydro-pyrrolo[2,3-d]pyridazin-7-one,
(251) 4-(3,5-dimethoxy-phenyl)-2-[5-(4-methyl-3-oxo-piperazin-1-yl)-2-trifluoromethyl-phenyl]-2,5,6,7-tetrahydro-cyclopenta[d]pyridazin-1-one,
(252) 4-(3,5-dimethoxy-phenyl)-1-(3-hydroxy-propyl)-6-[5-((S)-3-hydroxy-pyrrolidin-1-yl)-2-trifluoromethylphenyl]-1,2,3,6-tetrahydro-pyrrolo[2,3-d]pyridazin-7-one,
(253) N-(1-{3-[4-(3,5-dimethoxy-phenyl)-1-(2-hydroxyethyl)-7-oxo-1,2,3,7-tetrahydro-pyrrolo[2,3-d]pyridazin-6-yl]-4-trifluoromethyl-phenyl}-pyrrolidin-3-yl)-acetamide,
(254) 2-[5-((S)-3-hydroxy-pyrrolidin-1-yl)-2-trifluoromethyl-phenyl]-4-(4-methoxy-3-methyl-phenyl)-2,5,6,7-tetrahydro-cyclopenta[d]pyridazin-1-one,
(255) 4-(4-chloro-3-methyl-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(256) (1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-methyl-carbamic acid tert-butyl ester,
(257) 4-(3,5-dimethoxy-phenyl)-2-[5-(3-methylamino-pyrrolidin-1-yl)-2-trifluoromethyl-phenyl]-2,5,6,7-tetrahydro-cyclopenta[d]pyridazin-1-one hydrochloride,
(258) 2-[6-[5-(3,3-difluoro-pyrrolidin-1-yl)-2-trifluoromethyl-phenyl]-4-(3,5-dimethoxy-phenyl)-7-oxo-2,3,6,7-tetrahydro-pyrrolo[2,3-d]pyridazin-1-yl]-acetamide,
(259) N-((S)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-acetamide,
(260) 2-[2-chloro-5-((S)-3-hydroxy-pyrrolidin-1-yl)-phenyl]-4-(3,5-dimethoxy-phenyl)-2,5,6,7-tetrahydro-cyclopenta[d]pyridazin-1-one,
(261) 5-{3-[4-(3,5-dimethoxy-phenyl)-1-oxo-1,5,6,7-tetrahydro-cyclopenta[d]pyridazin-2-yl]-4-trifluoromethyl-phenylamino}-pentanoic acid,
(262) 4-(3-bromo-4-chloro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(263) N-((R)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-acetamide,
(264) N-((R)-1-{3-[4-(3,5-dimethoxy-phenyl)-7-oxo-1,2,3,7-tetrahydro-pyrrolo[2,3-d]pyridazin-6-yl]-4-trifluoromethyl-phenyl}-pyrrolidin-3-yl)-acetamide,
(265) 2-(1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-N-methyl-acetamide,
(266) N-(4-{3-[4-(3,5-dimethoxy-phenyl)-1-oxo-1,5,6,7-tetrahydro-cyclopenta[d]pyridazin-2-yl]-4-trifluoromethyl-phenylamino}-butyl)-acetamide,
(267) 4-(3-dimethylamino-4-fluoro-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(268) 4-(3,5-dimethoxy-phenyl)-2-[5-(3-hydroxy-azetidin-1-yl)-2-trifluoromethyl-phenyl]-2,5,6,7-tetrahydro-cyclopenta[d]pyridazin-1-one,
(269) 5-{3-[4-(3,5-dimethoxy-phenyl)-1-oxo-1,5,6,7-tetrahydro-cyclopenta[d]pyridazin-2-yl]-4-trifluoromethyl-phenylamino}-pentanoic acid dimethylamide,
(270) 6-[5-((S)-3-acetylamino-pyrrolidin-1-yl)-2-trifluoromethyl-phenyl]-4-(3,5-dimethoxy-phenyl)-7-oxo-2,3,6,7-tetrahydro-pyrrolo[2,3-d]pyridazine-1-carboxylic acid ethyl ester,
(271) N-((S)-1-{3-[4-(3,5-dimethoxy-phenyl)-7-oxo-1,2,3,7-tetrahydro-pyrrolo[2,3-d]pyridazin-6-yl]-4-trifluoromethyl-phenyl}-pyrrolidin-3-yl)-acetamide,
(272) 4-{3-[4-(3,5-dimethoxy-phenyl)-1-(2-hydroxyethyl)-7-oxo-1,2,3,7-tetrahydro-pyrrolo[2,3-d]pyridazin-6-yl]-4-trifluoromethyl-phenylamino}-N-methyl-butyramide,
(273) 4-(3,5-dimethoxy-phenyl)-6-[5-(3-hydroxy-3-methyl-pyrrolidin-1-yl)-2-trifluoromethyl-phenyl]-7-oxo-2,3,6,7-tetrahydro-pyrrolo[2,3-d]pyridazine-1-carboxylic acid amide,
(274) 3-(1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yloxy)-N-methyl-propionamide,
(275) N-(1-{3-[4-(3,5-dimethoxy-phenyl)-1-oxo-1,5,6,7-tetrahydro-cyclopenta[d]pyridazin-2-yl]-4-trifluoromethyl-phenyl}-azetidin-3-yl)-acetamide,
(276) 4-(3-dimethylamino-4-methoxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(277) 6-[5-(3,3-difluoro-pyrrolidin-1-yl)-2-trifluoromethyl-phenyl]-4-(3,5-dimethoxy-phenyl)-7-oxo-2,3,6,7-tetrahydro-pyrrolo[2,3-d]pyridazine-1-carboxylic acid amide,
(278) 4-(3,5-dimethoxy-phenyl)-2-[5-(2-hydroxymethyl-azetidin-1-yl)-2-trifluoromethyl-phenyl]-2,5,6,7-tetrahydro-cyclopenta[d]pyridazin-1-one,
(279) 4-(3,5-dimethoxy-phenyl)-2-[5-(2-methylpyrrolidin-1-yl)-2-trifluoromethyl-phenyl]-2,5,6,7-tetrahydro-cyclopenta[d]pyridazin-1-one,
(280) 4-(3,5-dimethoxy-phenyl)-2-{5-[3-(3-hydroxy-propoxy)-pyrrolidin-1-yl]-2-trifluoromethyl-phenyl}-2,5,6,7-tetrahydro-cyclopenta[d]pyridazin-1-one,
(281) N-((R)-1-{3-[4-(3,5-dimethoxy-phenyl)-1-(2-hydroxy-ethyl)-7-oxo-1,2,3,7-tetrahydro-pyrrolo[2,3-d]pyridazin-6-yl]-4-trifluoromethyl-phenyl}-pyrrolidin-3-yl)-acetamide,
(282) N-((S)-1-{3-[4-(3,5-dimethoxy-phenyl)-1-(2-hydroxy-ethyl)-7-oxo-1,2,3,7-tetrahydro-pyrrolo[2,3-d]pyridazin-6-yl]-4-trifluoromethyl-phenyl}-pyrrolidin-3-yl)-acetamide,
(283) 4-(3,5-dimethoxy-phenyl)-2-[5-((S)-3-hydroxy-pyrrolidin-1-yl)-2-methyl-phenyl]-2,5,6,7-tetrahydro-cyclopenta[d]pyridazin-1-one,
(284) 4-(3,5-dimethoxy-phenyl)-2-{5-[3-(2-hydroxyethoxy)-pyrrolidin-1-yl]-2-trifluoromethyl-phenyl}-2,5,6,7-tetrahydro-cyclopenta[d]pyridazin-1-one,
(285) N-[3-(1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yloxy)-propyl]-acetamide,
(286) 2-(1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yloxy)-N-methyl-acetamide,
(287) N-((R)-1-{3-[4-(5-methoxy-pyridin-3-yl)-1-oxo-1,5,6,7-tetrahydro-cyclopenta[d]pyridazin-2-yl]-4-trifluoromethyl-phenyl}-pyrrolidin-3-yl)-acetamide,
(288) 6-[5-((R)-3-acetylamino-pyrrolidin-1-yl)-2-trifluoromethyl-phenyl]-4-(3,5-dimethoxy-phenyl)-7-oxo-2,3,6,7-tetrahydro-pyrrolo[2,3-d]pyridazine-1-carboxylic acid amide,
(289) 5-{3-[4-(3,5-dimethoxy-phenyl)-1-oxo-1,5,6,7-tetrahydro-cyclopenta[d]pyridazin-2-yl]-4-trifluoromethyl-phenylamino}-pentanoic acid methylamide,
(290) 4-(4-fluoro-3-methyl-phenyl)-1-(2-hydroxy-ethyl)-6-[5-((S)-3-hydroxy-pyrrolidin-1-yl)-2-trifluoromethylphenyl]-1,2,3,6-tetrahydro-pyrrolo[2,3-d]pyridazin-7-one,
(291) 4-(4-fluoro-3-methoxy-phenyl)-1-(2-hydroxyethyl)-6-[5-((S)-3-hydroxy-pyrrolidin-1-yl)-2-trifluoromethyl-phenyl]-1,2,3,6-tetrahydro-pyrrolo[2,3-d]pyridazin-7-one,
(292) N-[2-(1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yloxy)-ethyl]-acetamide,
(293) 2-(1-{3-[4-(3,5-dimethoxy-phenyl)-1-oxo-1,5,6,7-tetrahydro-cyclopenta[d]pyridazin-2-yl]-4-trifluoromethyl-phenyl}-azetidin-3-yloxy)-N-methyl-acetamide,
(294) 1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidine-3-carboxylic acid ethylamide,
(295) 1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidine-3-carboxylic acid isopropylamide,
(296) 4-{3-[4-(3,5-dimethoxy-phenyl)-7-oxo-1,2,3,7-tetrahydro-pyrrolo[2,3-d]pyridazin-6-yl]-4-trifluoromethyl-phenylamino}-N-methyl-butyramide,
(297) 4-(3-bromo-5-methoxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(298) 4-(3,5-dimethoxy-phenyl)-6-[5-(3-hydroxy-azetidin-1-yl)-2-trifluoromethyl-phenyl]-1-(2-hydroxyethyl)-1,2,3,6-tetrahydro-pyrrolo[2,3-d]pyridazin-7-one,
(299) 4-(3-fluoro-5-methoxy-phenyl)-2-[5-((S)-3-hydroxy-pyrrolidin-1-yl)-2-trifluoromethyl-phenyl]-2,5,6,7-tetrahydro-cyclopenta[d]pyridazin-1-one,
(300) 1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidine-3-carboxylic acid amide,
(301) 4-(4-fluoro-3-methoxy-phenyl)-7-oxo-6-(5-pyrrolidin-1-yl-2-trifluoromethyl-phenyl)-2,3,6,7-tetrahydro-pyrrolo[2,3-d]pyridazine-1-carboxylic acid amide,
(302) 4-(3-methoxy-phenyl)-7-oxo-6-(5-pyrrolidin-1-yl-2-trifluoromethyl-phenyl)-2,3,6,7-tetrahydro-pyrrolo[2,3-d]pyridazine-1-carboxylic acid amide,
(303) 2-[5-((R)-3-amino-pyrrolidin-1-yl)-2-trifluoromethyl-phenyl]-4-(3,5-dimethoxy-phenyl)-2,5,6,7-tetrahydro-cyclopenta[d]pyridazin-1-one,
(304) 2-[4-(3,5-dimethoxy-phenyl)-7-oxo-6-(5-pyrrolidin-1-yl-2-trifluoromethyl-phenyl)-2,3,6,7-tetrahydro-pyrrolo[2,3-d]pyridazin-1-yl]-acetamide,
(305) 4-(3,5-dimethoxy-phenyl)-7-oxo-6-(5-pyrrolidin-1-yl-2-trifluoromethyl-phenyl)-2,3,6,7-tetrahydro-pyrrolo[2,3-d]pyridazine-1-carboxylic acid amide,
(306) 1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidine-3-carboxylic acid dimethylamide,
(307) 2-[4-(3,5-dimethyl-phenyl)-7-oxo-6-(5-pyrrolidin-1-yl-2-trifluoromethyl-phenyl)-2,3,6,7-tetrahydro-pyrrolo[2,3-d]pyridazin-1-yl]-acetamide,
(308) 2-{4-(3,5-dimethyl-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazin-1-yl}-acetamide,
(309) 4-(3,5-dimethyl-phenyl)-7-oxo-6-(5-pyrrolidin-1-yl-2-trifluoromethyl-phenyl)-2,3,6,7-tetrahydro-pyrrolo[2,3-d]pyridazine-1-carboxylic acid amide,
(310) 4-(3,5-dimethyl-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid amide,
(311) N-((R)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-propionamide,
(312) N-((R)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-butyramide,
(313) N-((R)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-isobutyramide,
(314) N-((R)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-methanesulfonamide,
(315) 6-[5-((R)-3-acetylamino-pyrrolidin-1-yl)-2-trifluoromethyl-phenyl]-4-(3-methoxy-phenyl)-7-oxo-2,3,6,7-tetrahydro-pyrrolo[2,3-d]pyridazine-1-carboxylic acid amide,
(316) 6-[5-((S)-3-hydroxy-pyrrolidin-1-yl)-2-trifluoromethyl-phenyl]-4-(3-methoxy-phenyl)-7-oxo-2,3,6,7-tetrahydro-pyrrolo[2,3-d]pyridazine-1-carboxylic acid amide,
(317) N-((R)-1-{3-[4-(4-fluoro-3-methoxy-phenyl)-1-(2-hydroxy-ethyl)-7-oxo-1,2,3,7-tetrahydro-pyrrolo[2,3-d]pyridazin-6-yl]-4-trifluoromethyl-phenyl}-pyrrolidin-3-yl)-acetamide,
(318) N-((R)-1-{3-[1-(2-hydroxy-ethyl)-4-(3-methoxyphenyl)-7-oxo-1,2,3,7-tetrahydro-pyrrolo[2,3-d]pyridazin-6-yl]-4-trifluoromethyl-phenyl}-pyrrolidin-3-yl)-acetamide,
(319) 2-[6-[5-((S)-3-hydroxy-pyrrolidin-1-yl)-2-trifluoromethyl-phenyl]-4-(3-methoxy-phenyl)-7-oxo-2,3,6,7-tetrahydro-pyrrolo[2,3-d]pyridazin-1-yl]-acetamide,
(320) 2-[4-(3-methoxy-phenyl)-7-oxo-6-(5-pyrrolidin-1-yl-2-trifluoromethyl-phenyl)-2,3,6,7-tetrahydro-pyrrolo[2,3-d]pyridazin-1-yl]-acetamide,
(321) 2-[6-[5-((R)-3-acetylamino-pyrrolidin-1-yl)-2-trifluoromethyl-phenyl]-4-(3-methoxy-phenyl)-7-oxo-2,3,6,7-tetrahydro-pyrrolo[2,3-d]pyridazin-1-yl]-acetamide,
(322) 4-(3,5-dimethoxy-phenyl)-1-(2-hydroxy-ethyl)-6-[5-((S)-3-methoxy-pyrrolidin-1-yl)-2-trifluoromethylphenyl]-1,2,3,6-tetrahydro-pyrrolo[2,3-d]pyridazin-7-one,
(323) N-((R)-1-{3-[4-(4-fluoro-3-methoxy-phenyl)-7-oxo-1,2,3,7-tetrahydro-pyrrolo[2,3-d]pyridazin-6-yl]-4-trifluoromethyl-phenyl}-pyrrolidin-3-yl)-acetamide,
(324) 6-[5-((R)-3-acetylamino-pyrrolidin-1-yl)-2-trifluoromethyl-phenyl]-4-(4-fluoro-3-methoxy-phenyl)-7-oxo-2,3,6,7-tetrahydro-pyrrolo[2,3-d]pyridazine-1-carboxylic acid amide,
(325) 4-(4-fluoro-3-methoxy-phenyl)-6-[5-((S)-3-hydroxy-pyrrolidin-1-yl)-2-trifluoromethyl-phenyl]-7-oxo-2,3,6,7-tetrahydro-pyrrolo[2,3-d]pyridazine-1-carboxylic acid amide,
(326) 2-{4-(3,5-dimethyl-phenyl)-6-[5-(3-hydroxymethyl-azetidin-1-yl)-2-trifluoromethyl-phenyl]-7-oxo-2,3,6,7-tetrahydro-pyrrolo[2,3-d]pyridazin-1-yl}-acetamide,
(327) 6-[5-((R)-3-acetylamino-pyrrolidin-1-yl)-2-trifluoromethyl-phenyl]-4-(3,5-dimethyl-phenyl)-7-oxo-2,3,6,7-tetrahydro-pyrrolo[2,3-d]pyridazine-1-carboxylic acid amide,
(328) 4-(3,5-dimethyl-phenyl)-6-[5-(3-hydroxymethyl-azetidin-1-yl)-2-trifluoromethyl-phenyl]-7-oxo-2,3,6,7-tetrahydro-pyrrolo[2,3-d]pyridazine-1-carboxylic acid amide,
(329) 4-(3,5-dimethoxy-phenyl)-1-(2-hydroxy-ethyl)-6-[5-(3-hydroxymethyl-azetidin-1-yl)-2-trifluoromethylphenyl]-1,2,3,6-tetrahydro-pyrrolo[2,3-d]pyridazin-7-one,
(330) carbamic acid 1-{3-[4-(3,5-dimethoxy-phenyl)-1-oxo-1,5,6,7-tetrahydro-cyclopenta[d]pyridazin-2-yl]-4-trifluoromethyl-phenyl}-azetidin-3-yl ester,
(331) methyl-carbamic acid 1-{3-[4-(3,5-dimethoxy-phenyl)-1-oxo-1,5,6,7-tetrahydro-cyclopenta[d]pyridazin-2-yl]-4-trifluoromethyl-phenyl}-azetidin-3-yl ester,
(332) 6-[5-(3-hydroxymethyl-azetidin-1-yl)-2-trifluoromethyl-phenyl]-4-(3-methoxy-phenyl)-1,2,3,6-tetrahydro-pyrrolo[2,3-d]pyridazin-7-one,
(333) 6-[5-(3-hydroxymethyl-azetidin-1-yl)-2-trifluoromethyl-phenyl]-4-(3-methoxy-phenyl)-7-oxo-2,3,6,7-tetrahydro-pyrrolo[2,3-d]pyridazine-1-carboxylic acid amide,
(334) 2-(1-{3-[4-(3,5-dimethoxy-phenyl)-7-oxo-1,2,3,7-tetrahydro-pyrrolo[2,3-d]pyridazin-6-yl]-4-trifluoromethyl-phenyl}-pyrrolidin-3-yl)-N-methyl-acetamide,
(335) 2-[6-[5-(3,3-difluoro-pyrrolidin-1-yl)-2-trifluoromethyl-phenyl]-4-(3,5-dimethyl-phenyl)-7-oxo-2,3,6,7-tetrahydro-pyrrolo[2,3-d]pyridazin-1-yl]-acetamide,
(336) 2-[6-[5-(3-hydroxymethyl-azetidin-1-yl)-2-trifluoromethyl-phenyl]-4-(3-methoxy-phenyl)-7-oxo-2,3,6,7-tetrahydro-pyrrolo[2,3-d]pyridazin-1-yl]-acetamide,
(337) N-((R)-1-{3-[4-(3,5-dimethyl-phenyl)-1-(2-hydroxy-ethyl)-7-oxo-1,2,3,7-tetrahydro-pyrrolo[2,3-d]pyridazin-6-yl]-4-trifluoromethyl-phenyl}-pyrrolidin-3-yl)-acetamide,
(338) (S)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidine-3-carboxylic acid methylamide,
(339) (R)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidine-3-carboxylic acid methylamide,
(340) 4-(3,5-dimethoxy-phenyl)-6-[5-(3-hydroxymethyl-azetidin-1-yl)-2-trifluoromethyl-phenyl]-7-oxo-2,3,6,7-tetrahydro-pyrrolo[2,3-d]pyridazine-1-carboxylic acid amide,
(341) 1-(2-hydroxy-ethyl)-6-[5-((S)-3-hydroxy-pyrrolidin-1-yl)-2-trifluoromethyl-phenyl]-4-(5-methoxy-pyridin-3-yl)-1,2,3,6-tetrahydro-pyrrolo[2,3-d]pyridazin-7-one,
(342) 2-((S)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-N-methyl-acetamide,
(343) 2-(1-{3-[4-(3,5-dimethoxy-phenyl)-1-oxo-1,5,6,7-tetrahydro-cyclopenta[d]pyridazin-2-yl]-4-trifluoromethyl-phenyl}-azetidin-3-yl)-N-methyl-acetamide,
(344) 4-(3,5-dimethoxy-phenyl)-6-{5-[(S)-3-(2-hydroxyethoxy)-pyrrolidin-1-yl]-2-trifluoromethyl-phenyl}-7-oxo-2,3,6,7-tetrahydro-pyrrolo[2,3-d]pyridazine-1-carboxylic acid amide,
(345) 4-(3,5-dimethoxy-phenyl)-6-{5-[(R)-3-(2-hydroxyethoxy)-pyrrolidin-1-yl]-2-trifluoromethyl-phenyl}-7-oxo-2,3,6,7-tetrahydro-pyrrolo[2,3-d]pyridazine-1-carboxylic acid amide,
(346) 2-((R)-1-{3-[4-(3,5-dimethoxy-phenyl)-1-oxo-1,5,6,7-tetrahydro-cyclopenta[d]pyridazin-2-yl]-4-trifluoromethyl-phenyl}-pyrrolidin-3-yl)-N-methyl-acetamide,
(347) 2-((R)-1-{3-[4-(3,5-dimethoxy-phenyl)-7-oxo-1,2,3,7-tetrahydro-pyrrolo[2,3-d]pyridazin-6-yl]-4-trifluoromethyl-phenyl}-pyrrolidin-3-yl)-N-methyl-acetamide,
(348) 4-(4-fluoro-3-methoxy-phenyl)-6-[5-(3-hydroxymethyl-azetidin-1-yl)-2-trifluoromethyl-phenyl]-7-oxo-2,3,6,7-tetrahydro-pyrrolo[2,3-d]pyridazine-1-carboxylic acid amide,
(349) 2-((S)-1-{3-[4-(3,5-dimethoxy-phenyl)-7-oxo-1,2,3,7-tetrahydro-pyrrolo[2,3-d]pyridazin-6-yl]-4-trifluoromethyl-phenyl}-pyrrolidin-3-yl)-N-methyl-acetamide,
(350) 4-(3,5-dimethoxy-phenyl)-6-{5-[(R)-3-(2-hydroxyethoxy)-pyrrolidin-1-yl]-2-trifluoromethyl-phenyl}-1,2,3,6-tetrahydro-pyrrolo[2,3-d]pyridazin-7-one,
(351) 4-(3,5-dimethoxy-phenyl)-1-(2-hydroxy-ethyl)-6-[5-((R)-3-hydroxy-pyrrolidin-1-yl)-2-trifluoromethylphenyl]-1,2,3,6-tetrahydro-pyrrolo[2,3-d]pyridazin-7-one,
(352) 4-(4-fluoro-3-methoxy-phenyl)-1-(2-hydroxyethyl)-6-[5-(3-hydroxymethyl-azetidin-1-yl)-2-trifluoromethyl-phenyl]-1,2,3,6-tetrahydro-pyrrolo[2,3-d]pyridazin-7-one,
(353) N-((R)-1-{3-[1-(2-hydroxy-ethyl)-4-(5-methoxy-pyridin-3-yl)-7-oxo-1,2,3,7-tetrahydro-pyrrolo[2,3-d]pyridazin-6-yl]-4-trifluoromethyl-phenyl}-pyrrolidin-3-yl)-acetamide,
(354) 1-(2-hydroxy-ethyl)-6-[5-((S)-3-hydroxy-pyrrolidin-1-yl)-2-trifluoromethyl-phenyl]-4-(3-methoxy-phenyl)-1,2,3,6-tetrahydro-pyrrolo[2,3-d]pyridazin-7-one,
(355) 1-(2-hydroxy-ethyl)-6-[5-(3-hydroxymethyl-azetidin-1-yl)-2-trifluoromethyl-phenyl]-4-(3-methoxyphenyl)-1,2,3,6-tetrahydro-pyrrolo[2,3-d]pyridazin-7-one,
(356) 2-((S)-1-{3-[4-(3,5-dimethoxy-phenyl)-1-(2-hydroxy-ethyl)-7-oxo-1,2,3,7-tetrahydro-pyrrolo[2,3-d]pyridazin-6-yl]-4-trifluoromethyl-phenyl}-pyrrolidin-3-yl)-N-methyl-acetamide,
(357) 4-(3,5-dimethoxy-phenyl)-6-{5-[(S)-3-(2-hydroxyethoxy)-pyrrolidin-1-yl]-2-trifluoromethyl-phenyl}-1,2,3,6-tetrahydro-pyrrolo[2,3-d]pyridazin-7-one,
(358) 1-(2-hydroxy-ethyl)-6-[5-((S)-3-hydroxy-pyrrolidin-1-yl)-2-trifluoromethyl-phenyl]-4-m-tolyl-1,2,3,6-tetrahydro-pyrrolo[2,3-d]pyridazin-7-one,
(359) 1-(2-hydroxy-ethyl)-6-[5-(3-hydroxymethyl-azetidin-1-yl)-2-trifluoromethyl-phenyl]-4-m-tolyl-1,2,3,6-tetrahydro-pyrrolo[2,3-d]pyridazin-7-one,
(360) 4-(4-fluoro-3-methyl-phenyl)-1-(2-hydroxy-ethyl)-6-[5-((R)-3-hydroxy-pyrrolidin-1-yl)-2-trifluoromethylphenyl]-1,2,3,6-tetrahydro-pyrrolo[2,3-d]pyridazin-7-one,
(361) 4-(3-methoxy-phenyl)-2-(5-morpholin-4-yl-2-nitrophenyl)-5,6,7,8-tetrahydro-2H-phthalazin-1-one,
(362) 7-(2,6-dichloro-phenyl)-5-(3-methyl-5-oxazol-5-yl-phenyl)-1,3,4,7-tetrahydro-2H-pyrido[2,3-d]pyridazin-8-one,
(363) 7-[5-(3-hydroxy-propylamino)-2-nitro-phenyl]-5-(3-methyl-5-oxazol-5-yl-phenyl)-1,3,4,7-tetrahydro-2H-pyrido[2,3-d]pyridazin-8-one,
(364) 7-(2,6-dichloro-phenyl)-1-methyl-5-(3-methyl-5-oxazol-5-yl-phenyl)-1,3,4,7-tetrahydro-2H-pyrido[2,3-d]pyridazin-8-one,
(365) 7-(2,6-dichloro-phenyl)-5-(3-methyl-5-[1,3,4]oxadiazol-2-yl-phenyl)-1,3,4,7-tetrahydro-2H-pyrido[2,3-d]pyridazin-8-one,
(366) 7-(2,6-dichloro-phenyl)-1-ethyl-5-(3-methyl-5-oxazol-5-yl-phenyl)-1,3,4,7-tetrahydro-2H-pyrido[2,3-d]pyridazin-8-one,
(367) 1-butyl-7-(2,6-dichloro-phenyl)-5-(3-methyl-5-oxazol-5-yl-phenyl)-1,3,4,7-tetrahydro-2H-pyrido[2,3-d]pyridazin-8-one,
(368) [7-(2,6-dichloro-phenyl)-5-(3-methyl-5-oxazol-5-yl-phenyl)-8-oxo-3,4,7,8-tetrahydro-2H-pyrido[2,3-d]pyridazin-1-yl]-acetic acid ethyl ester,
(369) 7-(2,6-dichloro-phenyl)-1-(2-hydroxy-ethyl)-5-(3-methyl-5-oxazol-5-yl-phenyl)-1,3,4,7-tetrahydro-2H-pyrido[2,3-d]pyridazin-8-one,
(370) 7-(2-chloro-6-methanesulfonyl-phenyl)-5-(3-methyl-5-oxazol-5-yl-phenyl)-1,3,4,7-tetrahydro-2H-pyrido[2,3-d]pyridazin-8-one,
(371) [7-(2,6-dichloro-phenyl)-5-(3-methyl-5-oxazol-5-yl-phenyl)-8-oxo-3,4,7,8-tetrahydro-2H-pyrido[2,3-d]pyridazin-1-yl]-acetic acid,
(372) [7-(2,6-dichloro-phenyl)-5-(3-methyl-5-oxazol-5-yl-phenyl)-8-oxo-3,4,7,8-tetrahydro-2H-pyrido[2,3-d]pyridazin-1-yl]-acetic acid methyl ester,
(373) 1-acetyl-7-(2,6-dichloro-phenyl)-5-(3-methyl-5-oxazol-5-yl-phenyl)-1,3,4,7-tetrahydro-2H-pyrido[2,3-d]pyridazin-8-one,
(374) 7-(2,6-dichloro-phenyl)-1-methanesulfonyl-5-(3-methyl-5-oxazol-5-yl-phenyl)-1,3,4,7-tetrahydro-2H-pyrido[2,3-d]pyridazin-8-one,
(375) 7-(2-methanesulfonyl-phenyl)-5-(3-methyl-5-oxazol-5-yl-phenyl)-1,3,4,7-tetrahydro-2H-pyrido[2,3-d]pyridazin-8-one,
(376) 7-[5-((S)-3-hydroxy-pyrrolidin-1-yl)-2-methanesulfonyl-phenyl]-5-(3-methyl-5-oxazol-5-yl-phenyl)-1,3,4,7-tetrahydro-2H-pyrido[2,3-d]pyridazin-8-one,
(377) 7-[2-methanesulfonyl-5-(3-oxo-morpholin-4-yl)-phenyl]-5-(3-methyl-5-oxazol-5-yl-phenyl)-1,3,4,7-tetrahydro-2H-pyrido[2,3-d]pyridazin-8-one,
(378) 7-[2-methanesulfonyl-5-(6-oxo-1,6-dihydro-pyridin-3-yl)-phenyl]-5-(3-methyl-5-oxazol-5-yl-phenyl)-1,3,4,7-tetrahydro-2H-pyrido[2,3-d]pyridazin-8-one,
(379) 7-(2-chloro-6-trifluoromethyl-phenyl)-5-(3-methyl-5-oxazol-5-yl-phenyl)-1,3,4,7-tetrahydro-2H-pyrido[2,3-d]pyridazin-8-one,
(380) N-{4-methanesulfonyl-3-[5-(3-methyl-5-oxazol-5-yl-phenyl)-8-oxo-2,3,4,8-tetrahydro-1H-pyrido[2,3-d]pyridazin-7-yl]-phenyl}-acetamide,
(381) 7-[2-methanesulfonyl-5-(3-oxo-pyrrolidin-1-yl)-phenyl]-5-(3-methyl-5-oxazol-5-yl-phenyl)-1,3,4,7-tetrahydro-2H-pyrido[2,3-d]pyridazin-8-one,
(382) 7-[2-ethanesulfonyl-5-((S)-3-hydroxy-pyrrolidin-1-yl)-phenyl]-5-(3-methyl-5-oxazol-5-yl-phenyl)-1,3,4,7-tetrahydro-2H-pyrido[2,3-d]pyridazin-8-one,
(383) 5-(3-methyl-5-oxazol-5-yl-phenyl)-7-(2-trifluoromethyl-phenyl)-1,3,4,7-tetrahydro-2H-pyrido[2,3-d]pyridazin-8-one,
(384) 5-(3-methyl-5-oxazol-5-yl-phenyl)-7-[5-(2-oxopyrrolidin-1-yl)-2-trifluoromethyl-phenyl]-1,3,4,7-tetrahydro-2H-pyrido[2,3-d]pyridazin-8-one,
(385) 7-[5-((R)-3-hydroxy-pyrrolidin-1-yl)-2-trifluoromethyl-phenyl]-5-(3-methyl-5-oxazol-5-yl-phenyl)-1,3,4,7-tetrahydro-2H-pyrido[2,3-d]pyridazin-8-one,
(386) 5-(3-methyl-5-oxazol-5-yl-phenyl)-7-[5-(6-oxo-1,6-dihydro-pyridin-3-yl)-2-trifluoromethyl-phenyl]-1,3,4,7-tetrahydro-2H-pyrido[2,3-d]pyridazin-8-one,
(387) 7-[5-(2-hydroxy-ethylamino)-2-trifluoromethylphenyl]-5-(3-methyl-5-oxazol-5-yl-phenyl)-1,3,4,7-tetrahydro-2H-pyrido[2,3-d]pyridazin-8-one,
(388) 7-[5-((S)-3-hydroxy-pyrrolidin-1-yl)-2-trifluoromethyl-phenyl]-2-methyl-5-(3-methyl-5-oxazol-5-yl-phenyl)-1,3,4,7-tetrahydro-2H-pyrido[2,3-d]pyridazin-8-one,
(389) [7-[5-((S)-3-hydroxy-pyrrolidin-1-yl)-2-trifluoromethyl-phenyl]-5-(3-methyl-5-oxazol-5-yl-phenyl)-8-oxo-3,4,7,8-tetrahydro-2H-pyrido[2,3-d]pyridazin-1-yl]-acetic acid,
(390) 2-methyl-5-(3-methyl-5-oxazol-5-yl-phenyl)-7-(5-pyridazin-3-yl-2-trifluoromethyl-phenyl)-1,3,4,7-tetrahydro-2H-pyrido[2,3-d]pyridazin-8-one,
(391) 4-(3,5-dimethyl-phenyl)-2-(5-pyridazin-3-yl-2-trifluoromethyl-phenyl)-5,6,7,8-tetrahydro-2H-phthalazin-1-one,
(392) 4-(3,5-dimethyl-phenyl)-2-[5-((S)-3-hydroxy-pyrrolidin-1-yl)-2-trifluoromethyl-phenyl]-5,6,7,8-tetrahydro-2H-phthalazin-1-one,
(393) 7-[5-((S)-3-hydroxy-pyrrolidin-1-yl)-2-trifluoromethyl-phenyl]-3-methyl-5-(3-methyl-5-oxazol-5-yl-phenyl)-1,3,4,7-tetrahydro-2H-pyrido[2,3-d]pyridazin-8-one,
(394) 5-(3,5-dimethyl-phenyl)-2-methyl-7-(5-pyridazin-3-yl-2-trifluoromethyl-phenyl)-1,3,4,7-tetrahydro-2H-pyrido[2,3-d]pyridazin-8-one,
(395) 5-(3,5-dimethyl-phenyl)-7-[5-((S)-3-hydroxy-pyrrolidin-1-yl)-2-trifluoromethyl-phenyl]-2-methyl-1,3,4,7-tetrahydro-2H-pyrido[2,3-d]pyridazin-8-one,
(396) 5-(3,5-dimethoxy-phenyl)-7-[5-((S)-3-hydroxy-pyrrolidin-1-yl)-2-trifluoromethyl-phenyl]-1,3,4,7-,tetrahydro-2H-pyrido[2,3-d]pyridazin-8-one,
(397) 5-(3,5-dimethoxy-phenyl)-7-[5-((S)-3-hydroxy-pyrrolidin-1-yl)-2-trifluoromethyl-phenyl]-1-methyl-1,3,4,7-tetrahydro-2H-pyrido[2,3-d]pyridazin-8-one,
(398) 5-(3,5-dimethoxy-phenyl)-7-[5-((S)-3-hydroxy-pyrrolidin-1-yl)-2-trifluoromethyl-phenyl]-2-methyl-1,3,4,7-tetrahydro-2H-pyrido[2,3-d]pyridazin-8-one,
(399) 5-(3,5-dimethoxy-phenyl)-2-ethyl-7-[5-((S)-3-hydroxy-pyrrolidin-1-yl)-2-trifluoromethyl-phenyl]-1,3,4,7-tetrahydro-2H-pyrido[2,3-d]pyridazin-8-one,
(400) 6-(3,5-dimethyl-phenyl)-8-(5-pyridazin-3-yl-2-trifluoromethyl-phenyl)-1,2,3,4,5,8-hexahydro-pyridazino[4,5-b]azepin-9-one,
(401) 6-(3,5-dimethyl-phenyl)-8-[5-((S)-3-hydroxy-pyrrolidin-1-yl)-2-trifluoromethyl-phenyl]-1,2,3,4,5,8-hexahydro-pyridazino[4,5-b]azepin-9-one,
(402-1) 5-[5-(3-hydroxy-propylamino)-2-nitro-phenyl]-7-(3-methyl-5-oxazol-5-yl-phenyl)-5H-furo[2,3-d]pyridazin-4-one,
(402-3) N-((R)-1-{3-[4-(3,5-dimethoxy-phenyl)-1-(2-hydroxy-ethyl)-7-oxo-1,7-dihydro-pyrrolo[2,3-d]pyridazin-6-yl]-4-trifluoromethyl-phenyl}-pyrrolidin-3-yl)-acetamide,
(402-4) 4-(3,5-dimethoxy-phenyl)-1-(2-hydroxy-ethyl)-6-[5-(3-hydroxymethyl-azetidin-1-yl)-2-trifluoromethylphenyl]-1,6-dihydro-pyrrolo[2,3-d]pyridazin-7-one,
(402-5) 4-(4-fluoro-3-methyl-phenyl)-1-(2-hydroxyethyl)-6-[5-((S)-3-hydroxy-pyrrolidin-1-yl)-2-trifluoromethyl-phenyl]-1,6-dihydro-pyrrolo[2,3-d]pyridazin-7-one,
(402-6) 4-(4-fluoro-3-methyl-phenyl)-1-(2-hydroxyethyl)-6-[5-(3-hydroxymethyl-azetidin-1-yl)-2-trifluoromethyl-phenyl]-1,6-dihydro-pyrrolo[2,3-d]pyridazin-7-one,
(402-7) 7-(4-fluoro-3-methyl-phenyl)-5-[5-(3-hydroxymethyl-azetidin-1-yl)-2-trifluoromethyl-phenyl]-4-oxo-4,5-dihydro-thieno[2,3-d]pyridazine-3-carboxylic acid,
(402-8) 7-(4-fluoro-3-methyl-phenyl)-5-[5-(3-hydroxymethyl-azetidin-1-yl)-2-trifluoromethyl-phenyl]-4-oxo-4,5-dihydro-thieno[2,3-d]pyridazine-3-carboxylic acid methyl ester,
(403) 4-(3,5-dimethyl-phenyl)-6-[5-((S)-3-hydroxy-pyrrolidin-1-yl)-2-trifluoromethyl-phenyl]-2-methyl-1,6-dihydro-pyrrolo[2,3-d]pyridazin-7-one,
(404) 4-(3,5-dimethoxy-phenyl)-6-[5-((S)-3-hydroxy-pyrrolidin-1-yl)-2-trifluoromethyl-phenyl]-1,6-dihydro-pyrrolo[2,3-d]pyridazin-7-one,
(405) 4-(3,5-dimethoxy-phenyl)-6-[5-((S)-3-hydroxy-pyrrolidin-1-yl)-2-trifluoromethyl-phenyl]-2-methyl-1,6-dihydro-pyrrolo[2,3-d]pyridazin-7-one,
(406) 4-(3,5-dimethoxy-phenyl)-2-ethyl-6-[5-((S)-3-hydroxy-pyrrolidin-1-yl)-2-trifluoromethyl-phenyl]-1,6-dihydro-pyrrolo[2,3-d]pyridazin-7-one,
(407) 4-(3,5-dimethoxy-phenyl)-1-(2-hydroxy-ethyl)-6-[5-((S)-3-hydroxy-pyrrolidin-1-yl)-2-trifluoromethylphenyl]-2-methyl-1,6-dihydro-pyrrolo[2,3-d]pyridazin-7-one,
(408) N-((S)-1-{3-[4-(3,5-dimethoxy-phenyl)-2-methyl-7-oxo-1,7-dihydro-pyrrolo[2,3-d]pyridazin-6-yl]-4-trifluoromethyl-phenyl}-pyrrolidin-3-yl)-acetamide,
(409) 4-{3-[4-(3,5-dimethoxy-phenyl)-1-hydroxymethyl-2-methyl-7-oxo-1,7-dihydro-pyrrolo[2,3-d]pyridazin-6-yl]-4-trifluoromethyl-phenylamino}-N-methyl-butyramide,
(410) N-((R)-1-{3-[4-(3,5-dimethoxy-phenyl)-2-methyl-7-oxo-1,7-dihydro-pyrrolo[2,3-d]pyridazin-6-yl]-4-trifluoromethyl-phenyl}-pyrrolidin-3-yl)-acetamide,
(411) 4-{3-[4-(3,5-dimethoxy-phenyl)-2-methyl-7-oxo-1,7-dihydro-pyrrolo[2,3-d]pyridazin-6-yl]-4-trifluoromethyl-phenylamino}-N-methyl-butyramide,
(412) 4-(3,5-dimethoxy-phenyl)-1-(2-hydroxy-ethyl)-6-[5-((S)-3-hydroxy-pyrrolidin-1-yl)-2-trifluoromethylphenyl]-1,6-dihydro-pyrrolo[2,3-d]pyridazin-7-one,
(413) N-((S)-1-{3-[4-(3,5-dimethoxy-phenyl)-1-(2-hydroxy-ethyl)-7-oxo-1,7-dihydro-pyrrolo[2,3-d]pyridazin-6-yl]-4-trifluoromethyl-phenyl}-pyrrolidin-3-yl)-acetamide,
(414) 4-(3,5-dimethoxy-phenyl)-1-(2-hydroxy-ethyl)-6-[5-(3-hydroxy-3-methyl-pyrrolidin-1-yl)-2-trifluoromethyl-phenyl]-1,6-dihydro-pyrrolo[2,3-d]pyridazin-7-one,
(415) 6-[5-(3,3-difluoro-pyrrolidin-1-yl)-2-trifluoromethyl-phenyl]-4-(3,5-dimethoxy-phenyl)-1-(2-hydroxy-ethyl)-1,6-dihydro-pyrrolo[2,3-d]pyridazin-7-one,
(416) 4-(3,5-dimethoxy-phenyl)-6-[5-(3-hydroxy-3-methyl-pyrrolidin-1-yl)-2-trifluoromethyl-phenyl]-1,6-dihydro-pyrrolo[2,3-d]pyridazin-7-one,
(417) 6-[5-(3,3-difluoro-pyrrolidin-1-yl)-2-trifluoromethyl-phenyl]-4-(3,5-dimethoxy-phenyl)-1,6-dihydro-pyrrolo[2,3-d]pyridazin-7-one,
(418) N-((R)-1-{3-[4-(3,5-dimethoxy-phenyl)-7-oxo-1,7-dihydro-pyrrolo[2,3-d]pyridazin-6-yl]-4-trifluoromethyl-phenyl}-pyrrolidin-3-yl)-acetamide,
(419) 4-{3-[4-(3,5-dimethoxy-phenyl)-1-(2-hydroxyethyl)-7-oxo-1,7-dihydro-pyrrolo[2,3-d]pyridazin-6-yl]-4-trifluoromethyl-phenylamino}-N-methyl-butyramide,
(420) 4-(4-fluoro-3-methoxy-phenyl)-6-[5-((S)-3-hydroxy-pyrrolidin-1-yl)-2-trifluoromethyl-phenyl]-1,6-dihydro-pyrrolo[2,3-d]pyridazin-7-one,
(421) 4-{3-[4-(3,5-dimethoxy-phenyl)-7-oxo-1,7-dihydro-pyrrolo[2,3-d]pyridazin-6-yl]-4-trifluoromethyl-phenylamino}-N-methyl-butyramide,
(422) 4-(4-fluoro-3-methyl-phenyl)-6-[5-((S)-3-hydroxy-pyrrolidin-1-yl)-2-trifluoromethyl-phenyl]-1,6-dihydro-pyrrolo[2,3-d]pyridazin-7-one,
(423) 4-(3,5-dimethoxy-phenyl)-6-(5-pyrrolidin-1-yl-2-trifluoromethyl-phenyl)-1,6-dihydro-pyrrolo[2,3-d]pyridazin-7-one,
(424) N-((R)-1-{3-[4-(3,5-dimethyl-phenyl)-1-(2-hydroxy-ethyl)-7-oxo-1,7-dihydro-pyrrolo[2,3-d]pyridazin-6-yl]-4-trifluoromethyl-phenyl}-pyrrolidin-3-yl)-acetamide,
(425) 6-[5-((S)-3-hydroxy-pyrrolidin-1-yl)-2-trifluoromethyl-phenyl]-4-(5-methoxy-pyridin-3-yl)-1,6-dihydro-pyrrolo[2,3-d]pyridazin-7-one,
(426) 7-(3,5-dimethoxy-phenyl)-5-[5-((S)-3-hydroxy-pyrrolidin-1-yl)-2-trifluoromethyl-phenyl]-5H-thieno[2,3-d]pyridazin-4-one,
(427) 5-(3-bromo-5-oxazol-5-yl-phenyl)-7-(2,6-dichloro-4-nitro-phenyl)-7H-pyrido[2,3-d]pyridazin-8-one,
(428) 7-(4-amino-2,6-dichloro-phenyl)-5-(3-bromo-5-oxazol-5-yl-phenyl)-7H-pyrido[2,3-d]pyridazin-8-one,
(429) 5-(3-bromo-5-oxazol-5-yl-phenyl)-7-(2,6-dichlorophenyl)-7H-pyrido[2,3-d]pyridazin-8-one,
(430) N-{4-[5-(3-bromo-5-oxazol-5-yl-phenyl)-8-oxo-8H-pyrido[2,3-d]pyridazin-7-yl]-3,5-dichloro-phenyl}-acetamide,
(431) N-{4-[5-(3-bromo-5-oxazol-5-yl-phenyl)-8-oxo-8H-pyrido[2,3-d]pyridazin-7-yl]-3,5-dichloro-phenyl}-methanesulfonamide,
(432) 5-(3-bromo-5-oxazol-5-yl-phenyl)-7-(2,6-dichloro-4-methylamino-phenyl)-7H-pyrido[2,3-d]pyridazin-8-one,
(433) 7-[5-(2-hydroxy-ethylamino)-2-nitro-phenyl]-5-(3-methyl-5-oxazol-5-yl-phenyl)-7H-pyrido[2,3-d]pyridazin-8-one,
(434) 7-(2,6-dichloro-phenyl)-5-(3-methyl-5-oxazol-5-yl-phenyl)-7H-pyrido[2,3-d]pyridazin-8-one,
(435) 7-(2,6-dimethyl-phenyl)-5-(3-methyl-5-oxazol-5-yl-phenyl)-7H-pyrido[2,3-d]pyridazin-8-one,
(436) 7-(2,6-difluoro-phenyl)-5-(3-methyl-5-oxazol-5-yl-phenyl)-7H-pyrido[2,3-d]pyridazin-8-one,
(437) 7-(4-bromo-2,6-dichloro-phenyl)-5-(3-methyl-5-oxazol-5-yl-phenyl)-7H-pyrido[2,3-d]pyridazin-8-one,
(438) 6-[5-(3-hydroxy-propylamino)-2-nitro-phenyl]-8-(3-methyl-5-oxazol-5-yl-phenyl)-6H-pyrido[2,3-d]pyridazin-5-one,
(439) 3-[5-(3-hydroxy-propylamino)-2-nitro-phenyl]-1-(3-methyl-5-oxazol-5-yl-phenyl)-3H-pyrido[3,4-d]pyridazin-4-one,
(440) 2-[5-(3-hydroxy-propylamino)-2-nitro-phenyl]-4-(3-methyl-5-oxazol-5-yl-phenyl)-2H-pyrido[3,4-d]pyridazin-1-one,
(441) 7-(2,6-dichloro-4-methyl-phenyl)-5-(3-methyl-5-oxazol-5-yl-phenyl)-7H-pyrido[2,3-d]pyridazin-8-one,
(442) 7-[5-((R)-3-hydroxy-pyrrolidin-1-yl)-2-methanesulfonyl-phenyl]-5-(3-methyl-5-oxazol-5-yl-phenyl)-7H-pyrido[2,3-d]pyridazin-8-one,
(443) {4-methanesulfonyl-3-[5-(3-methyl-5-oxazol-5-yl-phenyl)-8-oxo-8H-pyrido[2,3-d]pyridazin-7-yl]-phenylamino}-acetic acid methyl ester,
(444) {4-methanesulfonyl-3-[5-(3-methyl-5-oxazol-5-yl-phenyl)-8-oxo-8H-pyrido[2,3-d]pyridazin-7-yl]-phenylamino}-acetic acid,
(445) 7-[5-(3-hydroxy-pyrrolidin-1-yl)-2-methanesulfonyl-phenyl]-5-(3-methoxy-5-oxazol-5-yl-phenyl)-7H-pyrido[2,3-d]pyridazin-8-one,
(446) 7-(2-methanesulfonyl-phenyl)-5-(3-methyl-5-oxazol-5-yl-phenyl)-7H-pyrido[2,3-d]pyridazin-8-one,
(447) 5-(3-chloro-5-oxazol-5-yl-phenyl)-7-[5-((S)-3-hydroxy-pyrrolidin-1-yl)-2-methanesulfonyl-phenyl]-7H-pyrido[2,3-d]pyridazin-8-one,
(448) 5-(3-bromo-5-oxazol-5-yl-phenyl)-7-[5-((S)-3-hydroxy-pyrrolidin-1-yl)-2-methanesulfonyl-phenyl]-7H-pyrido[2,3-d]pyridazin-8-one,
(449) 7-[2-methanesulfonyl-5-(6-methoxy-pyridin-3-yl)-phenyl]-5-(3-methyl-5-oxazol-5-yl-phenyl)-7H-pyrido[2,3-d]pyridazin-8-one,
(450) 7-(2-methanesulfonyl-5-piperazin-1-yl-phenyl)-5-(3-methyl-5-oxazol-5-yl-phenyl)-7H-pyrido[2,3-d]pyridazin-8-one,
(451) 7-[5-(4-acetyl-piperazin-1-yl)-2-methanesulfonylphenyl]-5-(3-methyl-5-oxazol-5-yl-phenyl)-7H-pyrido[2,3-d]pyridazin-8-one,
(452) 5-(3-bromo-5-oxazol-5-yl-phenyl)-7-(2-chloro-6-trifluoromethyl-phenyl)-7H-pyrido[2,3-d]pyridazin-8-one,
(453) 7-[2-methanesulfonyl-5-(4-methanesulfonyl-piperazin-1-yl)-phenyl]-5-(3-methyl-5-oxazol-5-yl-phenyl)-7H-pyrido[2,3-d]pyridazin-8-one,
(454) 7-[2-methanesulfonyl-5-(6-oxo-1,6-dihydro-pyridin-3-yl)-phenyl]-5-(3-methyl-5-oxazol-5-yl-phenyl)-7H-pyrido[2,3-d]pyridazin-8-one,
(455) 5-(3-bromo-5-oxazol-5-yl-phenyl)-7-[5-(3-hydroxy-2,2-dimethyl-propylamino)-2-methanesulfonyl-phenyl]-7H-pyrido[2,3-d]pyridazin-8-one,
(456) 7-(2,6-dibromo-phenyl)-5-(3-methyl-5-oxazol-5-yl-phenyl)-7H-pyrido[2,3-d]pyridazin-8-one,
(457) 7-(2,6-divinyl-phenyl)-5-(3-methyl-5-oxazol-5-yl-phenyl)-7H-pyrido[2,3-d]pyridazin-8-one,
(458) 5-(3-methyl-5-oxazol-5-yl-phenyl)-7-[2-nitro-5-(3-oxo-piperazin-1-yl)-phenyl]-7H-pyrido[2,3-d]pyridazin-8-one,
(459) 7-(2,6-diethyl-phenyl)-5-(3-methyl-5-oxazol-5-yl-phenyl)-7H-pyrido[2,3-d]pyridazin-8-one,
(460) 7-(2,6-dichloro-phenyl)-5-(3-methyl-5-[1,3,4]oxadiazol-2-yl-phenyl)-7H-pyrido[2,3-d]pyridazin-8-one,
(461) 7-(2-chloro-6-methanesulfonyl-phenyl)-5-(3-methyl-5-oxazol-5-yl-phenyl)-7H-pyrido[2,3-d]pyridazin-8-one,
(462) 7-(2,6-dichloro-phenyl)-5-(3-methyl-5-[1,3,4]thiadiazol-2-yl-phenyl)-7H-pyrido[2,3-d]pyridazin-8-one,
(463) N-{4-methanesulfonyl-3-[5-(3-methyl-5-oxazol-5-yl-phenyl)-8-oxo-8H-pyrido[2,3-d]pyridazin-7-yl]-phenyl}-acetamide,
(464) 7-[2-methanesulfonyl-5-(3-oxo-morpholin-4-yl)-phenyl]-5-(3-methyl-5-oxazol-5-yl-phenyl)-7H-pyrido[2,3-d]pyridazin-8-one,
(465) 7-[2-methanesulfonyl-5-(2-oxo-pyrrolidin-1-yl)-phenyl]-5-(3-methyl-5-oxazol-5-yl-phenyl)-7H-pyrido[2,3-d]pyridazin-8-one,
(466) 7-[5-((S)-3-hydroxy-pyrrolidin-1-yl)-2-methanesulfonyl-phenyl]-5-(3-methyl-5-oxazol-5-yl-phenyl)-7H-pyrido[2,3-d]pyridazin-8-one,
(467) 3-{4-methanesulfonyl-3-[5-(3-methyl-5-oxazol-5-yl-phenyl)-8-oxo-8H-pyrido[2,3-d]pyridazin-7-yl]-phenylamino}-propionic acid methyl ester,
(468) 7-(2,6-dichloro-4-fluoro-phenyl)-5-(3-methyl-5-oxazol-5-yl-phenyl)-7H-pyrido[2,3-d]pyridazin-8-one,
(469) 3-{4-methanesulfonyl-3-[5-(3-methyl-5-oxazol-5-yl-phenyl)-8-oxo-8H-pyrido[2,3-d]pyridazin-7-yl]-phenylamino}-propionic acid,
(470) 7-[2-methanesulfonyl-5-(3-oxo-pyrrolidin-1-yl)-phenyl]-5-(3-methyl-5-oxazol-5-yl-phenyl)-7H-pyrido[2,3-d]pyridazin-8-one,
(471) 7-[5-((S)-3-hydroxy-pyrrolidin-1-yl)-2-trifluoromethyl-phenyl]-5-(3-methyl-5-oxazol-5-yl-phenyl)-7H-pyrido[2,3-d]pyridazin-8-one,
(472) 5-(3-methyl-5-oxazol-5-yl-phenyl)-7-(2-trifluoromethyl-phenyl)-7H-pyrido[2,3-d]pyridazin-8-one,
(473) 7-[5-(6-methoxy-pyridin-3-yl)-2-trifluoromethylphenyl]-5-(3-methyl-5-oxazol-5-yl-phenyl)-7H-pyrido[2,3-d]pyridazin-8-one,
(474) 7-[5-((R)-3-hydroxy-pyrrolidin-1-yl)-2-trifluoromethyl-phenyl]-5-(3-methyl-5-oxazol-5-yl-phenyl)-7H-pyrido[2,3-d]pyridazin-8-one,
(475) 5-(3-methyl-5-oxazol-5-yl-phenyl)-7-[5-(3-oxopyrrolidin-1-yl)-2-trifluoromethyl-phenyl]-7H-pyrido[2,3-d]pyridazin-8-one,
(476) 5-(3-methyl-5-oxazol-5-yl-phenyl)-7-[5-(2-oxopyrrolidin-1-yl)-2-trifluoromethyl-phenyl]-7H-pyrido[2,3-d]pyridazin-8-one,
(477) 5-(3-methyl-5-oxazol-5-yl-phenyl)-7-[5-(6-oxo-1,6-dihydro-pyridin-3-yl)-2-trifluoromethyl-phenyl]-7H-pyrido[2,3-d]pyridazin-8-one,
(478) 7-[5-(2-hydroxy-ethylamino)-2-trifluoromethylphenyl]-5-(3-methyl-5-oxazol-5-yl-phenyl)-7H-pyrido[2,3-d]pyridazin-8-one,
(479) 7-[5-((S)-3-hydroxy-pyrrolidin-1-yl)-2-trifluoromethyl-phenyl]-2-methyl-5-(3-methyl-5-oxazol-5-yl-phenyl)-7H-pyrido[2,3-d]pyridazin-8-one,
(480) 2-methyl-5-(3-methyl-5-oxazol-5-yl-phenyl)-7-(5-pyridazin-3-yl-2-trifluoromethyl-phenyl)-7H-pyrido[2,3-d]pyridazin-8-one,
(481) 3-methyl-5-(3-methyl-5-oxazol-5-yl-phenyl)-7-(5-pyridazin-3-yl-2-trifluoromethyl-phenyl)-7H-pyrido[2,3-d]pyridazin-8-one,
(482) 7-[5-((S)-3-hydroxy-pyrrolidin-1-yl)-2-trifluoromethyl-phenyl]-3-methyl-5-(3-methyl-5-oxazol-5-yl-phenyl)-7H-pyrido[2,3-d]pyridazin-8-one,
(483) 7-[2-ethanesulfonyl-5-((S)-3-hydroxy-pyrrolidin-1-yl)-phenyl]-5-(3-methyl-5-oxazol-5-yl-phenyl)-7H-pyrido[2,3-d]pyridazin-8-one,
(484) 5-(3,5-dimethoxy-phenyl)-7-[5-((S)-3-hydroxy-pyrrolidin-1-yl)-2-trifluoromethyl-phenyl]-7H-pyrido[2,3-d]pyridazin-8-one,
(485) 5-(3,5-dimethoxy-phenyl)-7-[5-((S)-3-hydroxy-pyrrolidin-1-yl)-2-trifluoromethyl-phenyl]-2-methyl-7H-pyrido[2,3-d]pyridazin-8-one,
(486) 5-(3,5-dimethoxy-phenyl)-2-ethyl-7-[5-((S)-3-hydroxy-pyrrolidin-1-yl)-2-trifluoromethyl-phenyl]-7H-pyrido[2,3-d]pyridazin-8-one,
(487) 6-methyl-2-(2-nitro-5-piperidin-1-yl-phenyl)-4-phenyl-2H-phthalazin-1-one,
(488) 7-methoxy-2-(2-nitro-5-piperidin-1-yl-phenyl)-4-phenyl-2H-phthalazin-1-one,
(489) 5-methoxy-2-(2-nitro-5-piperidin-1-yl-phenyl)-4-phenyl-2H-phthalazin-1-one,
(490) 2-(2-nitro-5-propylamino-phenyl)-4-phenyl-2H-phthalazin-1-one,
(491) 2-[5-(1-ethyl-propylamino)-2-nitro-phenyl]-4-phenyl-2H-phthalazin-1-one,
(492) 2-(5-isobutylamino-2-nitro-phenyl)-4-phenyl-2H-phthalazin-1-one,
(493) 2-[5-(methyl-propyl-amino)-2-nitro-phenyl]-4-phenyl-2H-phthalazin-1-one,
(494) 2-(5-diethylamino-2-nitro-phenyl)-4-phenyl-2H-phthalazin-1-one,
(495) 2-(5-amino-2-nitro-phenyl)-4-phenyl-2H-phthalazin-1-one,
(496) N-[4-nitro-3-(1-oxo-4-phenyl-1H-phthalazin-2-yl)-phenyl]-acetamide,
(497) 2-(5-butylamino-2-nitro-phenyl)-4-phenyl-2H-phthalazin-1-one,
(498) 2-[5-(2-methoxy-ethylamino)-2-nitro-phenyl]-4-phenyl-2H-phthalazin-1-one,
(499) 2-[5-(4-hydroxy-piperidin-1-yl)-2-nitro-phenyl]-4-phenyl-2H-phthalazin-1-one,
(500) 2-[5-(3-methyl-butylamino)-2-nitro-phenyl]-4-phenyl-2H-phthalazin-1-one,
(501) 2-(5-morpholin-4-yl-2-nitro-phenyl)-4-p-tolyl-2H-phthalazin-1-one,
(502) 4-(3-methoxy-phenyl)-2-(5-morpholin-4-yl-2-nitrophenyl)-2H-phthalazin-1-one,
(503) 2-(5-morpholin-4-yl-2-nitro-phenyl)-4-o-tolyl-2H-phthalazin-1-one,
(504) 4-(3-hydroxy-phenyl)-2-(5-morpholin-4-yl-2-nitrophenyl)-2H-phthalazin-1-one,
(505) 4-(3-chloro-phenyl)-2-(5-morpholin-4-yl-2-nitrophenyl)-2H-phthalazin-1-one,
(506) 4-(4-bromo-phenyl)-2-(5-morpholin-4-yl-2-nitrophenyl)-2H-phthalazin-1-one,
(507) 2-(4-bromo-5-morpholin-4-yl-2-nitro-phenyl)-4-m-tolyl-2H-phthalazin-1-one,
(508) 4-(3-ethyl-phenyl)-2-(5-morpholin-4-yl-2-nitrophenyl)-2H-phthalazin-1-one,
(509) 4-[3-(1,2-dihydroxy-ethyl)-phenyl]-2-(5-morpholin-4-yl-2-nitro-phenyl)-2H-phthalazin-1-one,
(510) 4-(3-isopropyl-phenyl)-2-(5-morpholin-4-yl-2-nitro-phenyl)-2H-phthalazin-1-one,
(511) 4-(4-ethyl-phenyl)-2-(5-morpholin-4-yl-2-nitrophenyl)-2H-phthalazin-1-one,
(512) 3-[3-(5-morpholin-4-yl-2-nitro-phenyl)-4-oxo-3,4-dihydro-phthalazin-1-yl]-benzaldehyde,
(513) 4-(3-ethoxy-phenyl)-2-(5-morpholin-4-yl-2-nitrophenyl)-2H-phthalazin-1-one,
(514) 4-(3-isopropoxy-phenyl)-2-(5-morpholin-4-yl-2-nitro-phenyl)-2H-phthalazin-1-one,
(515) 2-(5-morpholin-4-yl-2-nitro-phenyl)-4-[((E)-3-propenyl)-phenyl]-2H-phthalazin-1-one,
(516) 3-[3-(5-morpholin-4-yl-2-nitro-phenyl)-4-oxo-3,4-dihydro-phthalazin-1-yl]-benzaldehyde o-methyl-oxime,
(517) 4-(4-methoxy-phenyl)-2-(5-morpholin-4-yl-2-nitrophenyl)-2H-phthalazin-1-one,
(518) 4-(4-hydroxy-phenyl)-2-(5-morpholin-4-yl-2-nitrophenyl)-2H-phthalazin-1-one,
(519) 2-(5-morpholin-4-yl-2-nitro-phenyl)-4-(3-trifluoromethoxy-phenyl)-2H-phthalazin-1-one,
(520) 2-(5-morpholin-4-yl-2-nitro-phenyl)-4-(3-trifluoromethyl-phenyl)-2H-phthalazin-1-one,
(521) 4-(3,5-dimethyl-phenyl)-2-(5-morpholin-4-yl-2-nitro-phenyl)-2H-phthalazin-1-one,
(522) 4-(3-bromo-phenyl)-2-(5-morpholin-4-yl-2-nitrophenyl)-2H-phthalazin-1-one,
(523) 3-[3-(5-dimethylamino-2-nitro-phenyl)-4-oxo-3,4-dihydro-phthalazin-1-yl]-benzaldehyde,
(524) 4-[3-(1-hydroxy-ethyl)-phenyl]-2-(5-morpholin-4-yl-2-nitro-phenyl)-2H-phthalazin-1-one,
(525) 4-(3-acetyl-phenyl)-2-(5-morpholin-4-yl-2-nitrophenyl)-2H-phthalazin-1-one,
(526) 4-(3-isopropenyl-phenyl)-2-(5-morpholin-4-yl-2-nitro-phenyl)-2H-phthalazin-1-one,
(527) 4-(3-{1-[(E)-hydroxyimino]-ethyl}-phenyl)-2-(5-morpholin-4-yl-2-nitro-phenyl)-2H-phthalazin-1-one,
(528) 4-(3-{1-[(E)-methoxyimino]-ethyl}-phenyl)-2-(5-morpholin-4-yl-2-nitro-phenyl)-2H-phthalazin-1-one,
(529) N-{3-[3-(5-morpholin-4-yl-2-nitro-phenyl)-4-oxo-3,4-dihydro-phthalazin-1-yl]-phenyl}-acetamide,
(530) 4-(3-hydroxy-5-methoxy-phenyl)-2-(5-morpholin-4-yl-2-nitro-phenyl)-2H-phthalazin-1-one,
(531) 4-(3-ethoxy-5-methoxy-phenyl)-2-(5-morpholin-4-yl-2-nitro-phenyl)-2H-phthalazin-1-one,
(532) 4-(3-methoxy-5-propoxy-phenyl)-2-(5-morpholin-4-yl-2-nitro-phenyl)-2H-phthalazin-1-one,
(533) 4-(3-butoxy-5-methoxy-phenyl)-2-(5-morpholin-4-yl-2-nitro-phenyl)-2H-phthalazin-1-one,
(534) 3-bromo-5-[3-(5-morpholin-4-yl-2-nitro-phenyl)-4-oxo-3,4-dihydro-phthalazin-1-yl]-benzaldehyde,
(535) 4-(3-bromo-5-vinyl-phenyl)-2-(5-morpholin-4-yl-2-nitro-phenyl)-2H-phthalazin-1-one,
(536) 3-bromo-5-[3-(5-morpholin-4-yl-2-nitro-phenyl)-4-oxo-3,4-dihydro-phthalazin-1-yl]-benzaldehyde oxime,
(537) 3-[3-(5-morpholin-4-yl-2-nitro-phenyl)-4-oxo-3,4-dihydro-phthalazin-1-yl]-5-vinyl-benzaldehyde,
(538) 3-[3-(5-morpholin-4-yl-2-nitro-phenyl)-4-oxo-3,4-dihydro-phthalazin-1-yl]-5-vinyl-benzaldehyde oxime,
(539) 4-(3,5-dichloro-4-methoxy-phenyl)-2-(5-morpholin-4-yl-2-nitro-phenyl)-2H-phthalazin-1-one,
(540) 5-[3-(5-morpholin-4-yl-2-nitro-phenyl)-4-oxo-3,4-dihydro-phthalazin-1-yl]-benzene-1,3-dicarbaldehyde,
(541) 5-[3-(5-morpholin-4-yl-2-nitro-phenyl)-4-oxo-3,4-dihydro-phthalazin-1-yl]-benzene-1,3-dicarbaldehyde dioxime,
(542) 3-methoxy-5-[3-(5-morpholin-4-yl-2-nitro-phenyl)-4-oxo-3,4-dihydro-phthalazin-1-yl]-benzaldehyde oxime,
(543) 4-(2-methyl-benzofuran-5-yl)-2-(5-morpholin-4-yl-2-nitro-phenyl)-2H-phthalazin-1-one,
(544) 4-benzofuran-6-yl-2-(5-morpholin-4-yl-2-nitrophenyl)-2H-phthalazin-1-one,
(545) 3-methoxy-5-[3-(5-morpholin-4-yl-2-nitro-phenyl)-4-oxo-3,4-dihydro-phthalazin-1-yl]-benzaldehyde o-tert-butyl-oxime,
(546) 3-methoxy-5-[3-(5-morpholin-4-yl-2-nitro-phenyl)-4-oxo-3,4-dihydro-phthalazin-1-yl]-benzaldehyde o-benzyl-oxime,
(547) 3-methoxy-5-[3-(5-morpholin-4-yl-2-nitro-phenyl)-4-oxo-3,4-dihydro-phthalazin-1-yl]-benzoic acid,
(548) 4-[3-(dimethyl-hydrazonomethyl)-5-methoxyphenyl]-2-(5-morpholin-4-yl-2-nitro-phenyl)-2H-phthalazin-1-one,
(549) acetic acid [1-{3-methoxy-5-[3-(5-morpholin-4-yl-2-nitro-phenyl)-4-oxo-3,4-dihydro-phthalazin-1-yl]-phenyl}-meth-(E)-ylidene]-hydrazide,
(550) 4-(3-methyl-5-vinyl-phenyl)-2-(5-morpholin-4-yl-2-nitro-phenyl)-2H-phthalazin-1-one,
(551) 4-(3-acetyl-5-methyl-phenyl)-2-(5-morpholin-4-yl-2-nitro-phenyl)-2H-phthalazin-1-one,
(552) 2-morpholin-4-yl-5-nitro-4-(1-oxo-4-m-tolyl-1H-phthalazin-2-yl)-benzonitrile,
(553) 4-[3-bromo-5-(1-hydroxy-ethyl)-phenyl]-2-(5-morpholin-4-yl-2-nitro-phenyl)-2H-phthalazin-1-one,
(554) 4-(3-acetyl-5-bromo-phenyl)-2-(5-morpholin-4-yl-2-nitro-phenyl)-2H-phthalazin-1-one,
(555) 4-benzoxazol-5-yl-2-(5-morpholin-4-yl-2-nitrophenyl)-2H-phthalazin-1-one,
(556) 3-[3-(5-morpholin-4-yl-2-nitro-phenyl)-4-oxo-3,4-dihydro-phthalazin-1-yl]-5-vinyl-benzoic acid,
(557) 3-[3-(5-morpholin-4-yl-2-nitro-phenyl)-4-oxo-3,4-dihydro-phthalazin-1-yl]-5-vinyl-benzonitrile,
(558) (E)-3-{3-[3-(5-morpholin-4-yl-2-nitro-phenyl)-4-oxo-3,4-dihydro-phthalazin-1-yl]-5-vinyl-phenyl}-acrylic acid methyl ester,
(559) 4-(3-acetyl-5-isopropenyl-phenyl)-2-(5-morpholin-4-yl-2-nitro-phenyl)-2H-phthalazin-1-one,
(560) 4-(3-amino-5-vinyl-phenyl)-2-(5-morpholin-4-yl-2-nitro-phenyl)-2H-phthalazin-1-one,
(561) 2-(5-morpholin-4-yl-2-nitro-phenyl)-4-(3-propenyl-5-vinyl-phenyl)-2H-phthalazin-1-one,
(562) 4-(3-dimethylamino-5-vinyl-phenyl)-2-(5-morpholin-4-yl-2-nitro-phenyl)-2H-phthalazin-1-one,
(563) {3-[3-(5-morpholin-4-yl-2-nitro-phenyl)-4-oxo-3,4-dihydro-phthalazin-1-yl]-5-vinyl-phenyl}-carbamic acid methyl ester,
(564) 4-(3,5-diisopropenyl-phenyl)-2-(5-morpholin-4-yl-2-nitro-phenyl)-2H-phthalazin-1-one,
(565) 4-(3-{1-[(E)-hydroxyimino]-ethyl}-5-isopropenyl-phenyl)-2-(5-morpholin-4-yl-2-nitro-phenyl)-2H-phthalazin-1-one,
(566) N-{3-[3-(5-morpholin-4-yl-2-nitro-phenyl)-4-oxo-3,4-dihydro-phthalazin-1-yl]-5-vinyl-phenyl}-methanesulfonamide,
(567) N-{3-[3-(5-morpholin-4-yl-2-nitro-phenyl)-4-oxo-3,4-dihydro-phthalazin-1-yl]-5-vinyl-phenyl}-acetamide,
(568) N-{3-[3-(5-morpholin-4-yl-2-nitro-phenyl)-4-oxo-3,4-dihydro-phthalazin-1-yl]-5-vinyl-phenyl}-urea,
(569) N-methyl-N-{3-[3-(5-morpholin-4-yl-2-nitrophenyl)-4-oxo-3,4-dihydro-phthalazin-1-yl]-5-vinyl-phenyl}-acetamide,
(570) 3-bromo-5-[3-(5-morpholin-4-yl-2-nitro-phenyl)-4-oxo-3,4-dihydro-phthalazin-1-yl]-benzamide,
(571) 2-[5-(4-methoxy-piperidin-1-yl)-2-nitro-phenyl]-4-phenyl-2H-phthalazin-1-one,
(572) 1-[4-nitro-3-(1-oxo-4-phenyl-1H-phthalazin-2-yl)-phenyl]-piperidine-4-carboxylic acid ethyl ester,
(573) 1-[4-nitro-3-(1-oxo-4-phenyl-1H-phthalazin-2-yl)-phenyl]-piperidine-4-carboxylic acid,
(574) 2-[5-(4-methyl-3-oxo-piperazin-1-yl)-2-nitrophenyl]-4-phenyl-2H-phthalazin-1-one,
(575) 2-[5-(3-methoxy-piperidin-1-yl)-2-nitro-phenyl]-4-phenyl-2H-phthalazin-1-one,
(576) 2-[5-(3-hydroxy-piperidin-1-yl)-2-nitro-phenyl]-4-phenyl-2H-phthalazin-1-one,
(577) 2-[5-(4-dimethylamino-piperidin-1-yl)-2-nitrophenyl]-4-phenyl-2H-phthalazin-1-one,
(578) 2-[5-(4-hydroxymethyl-piperidin-1-yl)-2-nitrophenyl]-4-phenyl-2H-phthalazin-1-one,
(579) 2-[5-(4-methoxymethyl-piperidin-1-yl)-2-nitrophenyl]-4-phenyl-2H-phthalazin-1-one,
(580) 2-[2-nitro-5-(4-phenoxymethyl-piperidin-1-yl)-phenyl]-4-phenyl-2H-phthalazin-1-one,
(581) 2-(5-azepan-1-yl-2-nitro-phenyl)-4-phenyl-2H-phthalazin-1-one,
(582) 2-(2-nitro-5-pyrrolidin-1-yl-phenyl)-4-phenyl-2H-phthalazin-1-one,
(583) 2-(2-chloro-3-morpholin-4-yl-6-nitro-phenyl)-4-phenyl-2H-phthalazin-1-one,
(584) 2-(5-morpholin-4-yl-2-nitro-phenyl)-4-m-tolyl-2H-phthalazin-1-one,
(585) 4-(3-hydroxymethyl-phenyl)-2-(5-morpholin-4-yl-2-nitro-phenyl)-2H-phthalazin-1-one,
(586) 3-[3-(5-morpholin-4-yl-2-nitro-phenyl)-4-oxo-3,4-dihydro-phthalazin-1-yl]-benzoic acid,
(587) 4-[3-(5-morpholin-4-yl-2-nitro-phenyl)-4-oxo-3,4-dihydro-phthalazin-1-yl]-benzonitrile,
(588) 3-[3-(5-morpholin-4-yl-2-nitro-phenyl)-4-oxo-3,4-dihydro-phthalazin-1-yl]-benzaldehyde oxime,
(589) 3-[3-(5-morpholin-4-yl-2-nitro-phenyl)-4-oxo-3,4-dihydro-phthalazin-1-yl]-benzonitrile,
(590) 3-[3-(5-morpholin-4-yl-2-nitro-phenyl)-4-oxo-3,4-dihydro-phthalazin-1-yl]-benzoic acid methyl ester,
(591) N,N-dimethyl-3-[3-(5-morpholin-4-yl-2-nitrophenyl)-4-oxo-3,4-dihydro-phthalazin-1-yl]-benzamide,
(592) {3-[3-(5-morpholin-4-yl-2-nitro-phenyl)-4-oxo-3,4-dihydro-phthalazin-1-yl]-phenyl}-carbamic acid tert-butyl ester,
(593) 4-(3-amino-phenyl)-2-(5-morpholin-4-yl-2-nitrophenyl)-2H-phthalazin-1-one,
(594) trimethyl-{3-[3-(5-morpholin-4-yl-2-nitrophenyl)-4-oxo-3,4-dihydro-phthalazin-1-yl]-phenyl}-ammonium iodide,
(595) 4-(3-dimethylamino-phenyl)-2-(5-morpholin-4-yl-2-nitro-phenyl)-2H-phthalazin-1-one,
(596) 4-(3,5-dimethoxy-phenyl)-2-(5-morpholin-4-yl-2-nitro-phenyl)-2H-phthalazin-1-one,
(597) 2-(5-morpholin-4-yl-2-nitro-phenyl)-4-(3-propoxyphenyl)-2H-phthalazin-1-one,
(598) 2-(5-morpholin-4-yl-2-nitro-phenyl)-4-(3-vinyl-phenyl)-2H-phthalazin-1-one,
(599) 4-(3-methoxy-phenyl)-8-methyl-2-(5-morpholin-4-yl-2-nitro-phenyl)-2H-phthalazin-1-one,
(600) 4-(3-methoxy-phenyl)-5-methyl-2-(5-morpholin-4-yl-2-nitro-phenyl)-2H-phthalazin-1-one,
(601) 3-[3-(5-morpholin-4-yl-2-nitro-phenyl)-4-oxo-3,4-dihydro-phthalazin-1-yl]-benzoic acid n'-acetyl-hydrazide,
(602) N-hydroxy-3-[3-(5-morpholin-4-yl-2-nitro-phenyl)-4-oxo-3,4-dihydro-phthalazin-1-yl]-benzamide,
(603) 4-(3,5-bis-trifluoromethyl-phenyl)-2-(5-morpholin-4-yl-2-nitro-phenyl)-2H-phthalazin-1-one,
(604) 4-(3,5-diethoxy-phenyl)-2-(5-morpholin-4-yl-2-nitro-phenyl)-2H-phthalazin-1-one,
(605) 4-(3,5-dibromo-phenyl)-2-(5-morpholin-4-yl-2-nitro-phenyl)-2H-phthalazin-1-one,
(606) 4-(3,5-dichloro-phenyl)-2-(5-morpholin-4-yl-2-nitro-phenyl)-2H-phthalazin-1-one,
(607) 7-hydroxy-2-(2-nitro-5-piperidin-1-yl-phenyl)-4-phenyl-2H-phthalazin-1-one,
(608) 5-hydroxy-2-(2-nitro-5-piperidin-1-yl-phenyl)-4-phenyl-2H-phthalazin-1-one,
(609) 4-(3-bromo-5-methyl-phenyl)-2-(5-morpholin-4-yl-2-nitro-phenyl)-2H-phthalazin-1-one,
(610) 4-(3,5-divinyl-phenyl)-2-(5-morpholin-4-yl-2-nitro-phenyl)-2H-phthalazin-1-one,
(611) 4-(3-tert-butoxy-5-methoxy-phenyl)-2-(5-morpholin-4-yl-2-nitro-phenyl)-2H-phthalazin-1-one,
(612) {3-[3-(5-morpholin-4-yl-2-nitro-phenyl)-4-oxo-3,4-dihydro-phthalazin-1-yl]-phenyl}-urea,
(613) 3-methoxy-5-[3-(5-morpholin-4-yl-2-nitro-phenyl)-4-oxo-3,4-dihydro-phthalazin-1-yl]-benzaldehyde,
(614) 4-(3-methoxy-5-vinyl-phenyl)-2-(5-morpholin-4-yl-2-nitro-phenyl)-2H-phthalazin-1-one,
(615) 4-(3,5-dichloro-4-hydroxy-phenyl)-2-(5-morpholin-4-yl-2-nitro-phenyl)-2H-phthalazin-1-one,
(616) N-hydroxy-3-[3-(5-morpholin-4-yl-2-nitro-phenyl)-4-oxo-3,4-dihydro-phthalazin-1-yl]-benzamidine,
(617) 4-benzofuran-5-yl-2-(5-morpholin-4-yl-2-nitrophenyl)-2H-phthalazin-1-one,
(618) 4-benzofuran-7-yl-2-(5-morpholin-4-yl-2-nitrophenyl)-2H-phthalazin-1-one,
(619) 2-(5-morpholin-4-yl-2-nitro-phenyl)-4-naphthalen-2-yl-2H-phthalazin-1-one,
(620) 2-(5-morpholin-4-yl-2-nitro-phenyl)-4-naphthalen-1-yl-2H-phthalazin-1-one,
(621) 4-(6-methoxy-naphthalen-1-yl)-2-(5-morpholin-4-yl-2-nitro-phenyl)-2H-phthalazin-1-one,
(622) 4-(7-methoxy-naphthalen-2-yl)-2-(5-morpholin-4-yl-2-nitro-phenyl)-2H-phthalazin-1-one,
(623) 4-(6-hydroxy-naphthalen-1-yl)-2-(5-morpholin-4-yl-2-nitro-phenyl)-2H-phthalazin-1-one,
(624) 4-(7-hydroxy-naphthalen-2-yl)-2-(5-morpholin-4-yl-2-nitro-phenyl)-2H-phthalazin-1-one,
(625) 4-(7-methyl-benzofuran-5-yl)-2-(5-morpholin-4-yl-2-nitro-phenyl)-2H-phthalazin-1-one,
(626) 4-(3-methyl-benzofuran-5-yl)-2-(5-morpholin-4-yl-2-nitro-phenyl)-2H-phthalazin-1-one,
(627) 3-[3-(5-morpholin-4-yl-2-nitro-phenyl)-4-oxo-3,4-dihydro-phthalazin-1-yl]-5-vinyl-benzoic acid methyl ester,
(628) 3-methoxy-N,N-dimethyl-5-[3-(5-morpholin-4-yl-2-nitro-phenyl)-4-oxo-3,4-dihydro-phthalazin-1-yl]-benzamide,
(629) N-tert-butyl-3-methoxy-5-[3-(5-morpholin-4-yl-2-nitro-phenyl)-4-oxo-3,4-dihydro-phthalazin-1-yl]-benzamide,
(630) 4-(3-acetyl-5-vinyl-phenyl)-2-(5-morpholin-4-yl-2-nitro-phenyl)-2H-phthalazin-1-one,
(631) 4-(3,5-diacetyl-phenyl)-2-(5-morpholin-4-yl-2-nitro-phenyl)-2H-phthalazin-1-one,
(632) 4-(3-bromo-5-trifluoromethyl-phenyl)-2-(5-morpholin-4-yl-2-nitro-phenyl)-2H-phthalazin-1-one,
(633) 2-(5-morpholin-4-yl-2-nitro-phenyl)-4-(3-trifluoromethyl-5-vinyl-phenyl)-2H-phthalazin-1-one,
(634) 4-(3-{1-[(E)-hydroxyimino]-ethyl}-5-vinyl-phenyl)-2-(5-morpholin-4-yl-2-nitro-phenyl)-2H-phthalazin-1-one,
(635) 4-(3,5-bis-{1-[(E)-hydroxyimino]-ethyl}-phenyl)-2-(5-morpholin-4-yl-2-nitro-phenyl)-2H-phthalazin-1-one,
(636) 4-(3-bromo-5-ethyl-phenyl)-2-(5-morpholin-4-yl-2-nitro-phenyl)-2H-phthalazin-1-one,
(637) 4-(3-ethyl-5-vinyl-phenyl)-2-(5-morpholin-4-yl-2-nitro-phenyl)-2H-phthalazin-1-one,
(638) 3-[3-(5-morpholin-4-yl-2-nitro-phenyl)-4-oxo-3,4-dihydro-phthalazin-1-yl]-5-vinyl-benzamide,
(639) N-methyl-3-[3-(5-morpholin-4-yl-2-nitro-phenyl)-4-oxo-3,4-dihydro-phthalazin-1-yl]-5-vinyl-benzamide,
(640) N,N-dimethyl-3-[3-(5-morpholin-4-yl-2-nitrophenyl)-4-oxo-3,4-dihydro-phthalazin-1-yl]-5-vinyl-benzamide,
(641) 3-bromo-5-[3-(5-morpholin-4-yl-2-nitro-phenyl)-4-oxo-3,4-dihydro-phthalazin-1-yl]-benzoic acid hydrazide,
(642) N-(2,2-dimethoxy-ethyl)-3-methoxy-5-[3-(5-morpholin-4-yl-2-nitro-phenyl)-4-oxo-3,4-dihydro-phthalazin-1-yl]-benzamide,
(643) 5-[3-(5-morpholin-4-yl-2-nitro-phenyl)-4-oxo-3,4-dihydro-phthalazin-1-yl]-isophthalonitrile,
(644) 3-bromo-N-hydroxy-5-[3-(5-morpholin-4-yl-2-nitrophenyl)-4-oxo-3,4-dihydro-phthalazin-1-yl]-benzamidine,
(645) 3-hydroxy-5-[3-(5-morpholin-4-yl-2-nitro-phenyl)-4-oxo-3,4-dihydro-phthalazin-1-yl]-benzaldehyde,
(646) 2-(3-nitro-phenyl)-4-phenyl-2H-phthalazin-1-one,
(647) 4-phenyl-2-(3-piperidin-1-yl-phenyl)-2H-phthalazin-1-one,
(648) 2-(2-nitro-phenyl)-4-phenyl-2H-phthalazin-1-one,
(649) 2-(2-chloro-phenyl)-4-phenyl-2H-phthalazin-1-one,
(650) 2-(2,6-dichloro-phenyl)-4-phenyl-2H-phthalazin-1-one,
(651) 2-(5-chloro-2-nitro-phenyl)-4-phenyl-2H-phthalazin-1-one,
(652) 2-(2-amino-phenyl)-4-phenyl-2H-phthalazin-1-one,
(653) 2-(2-bromo-phenyl)-4-phenyl-2H-phthalazin-1-one,
(654) 4-phenyl-2-(2-vinyl-phenyl)-2H-phthalazin-1-one,
(655) 2-(5-methoxy-2-nitro-phenyl)-4-phenyl-2H-phthalazin-1-one,
(656) 2-(2-ethyl-phenyl)-4-phenyl-2H-phthalazin-1-one,
(657) 4-(4-fluoro-phenyl)-2-(2-nitro-phenyl)-2H-phthalazin-1-one,
(658) 2-(4-chloro-2-nitro-phenyl)-4-phenyl-2H-phthalazin-1-one,
(659) 4-nitro-3-(1-oxo-4-phenyl-1H-phthalazin-2-yl)-benzoic acid ethyl ester,
(660) 2-(3-morpholin-4-yl-phenyl)-4-phenyl-2H-phthalazin-1-one,
(661) 2-morpholin-4-yl-4-(1-oxo-4-phenyl-1H-phthalazin-2-yl)-benzaldehyde,
(662) 4-morpholin-4-yl-2-(1-oxo-4-phenyl-1H-phthalazin-2-yl)-benzaldehyde,
(663) 4-morpholin-4-yl-2-(1-oxo-4-phenyl-1H-phthalazin-2-yl)-benzonitrile,
(664) 2-(5-morpholin-4-yl-2-vinyl-phenyl)-4-phenyl-2H-phthalazin-1-one,
(665) 2-[5-(4-methoxy-benzyloxy)-2-nitro-phenyl]-4-phenyl-2H-phthalazin-1-one,
(666) 2-(5-hydroxy-2-nitro-phenyl)-4-phenyl-2H-phthalazin-1-one,
(667) 2-naphthalen-1-yl-4-phenyl-2H-phthalazin-1-one,
(668) 2-[2-(2-methyl-propenyl)-5-morpholin-4-yl-phenyl]-4-phenyl-2H-phthalazin-1-one,
(669) N-[4-morpholin-4-yl-2-(1-oxo-4-phenyl-1H-phthalazin-2-yl)-phenyl]-acetamide,
(670) 2-(2-dimethylamino-5-morpholin-4-yl-phenyl)-4-phenyl-2H-phthalazin-1-one,
(671) N-[4-morpholin-4-yl-2-(1-oxo-4-phenyl-1H-phthalazin-2-yl)-phenyl]-methanesulfonamide,
(672) 4-morpholin-4-yl-2-(1-oxo-4-phenyl-1H-phthalazin-2-yl)-benzoic acid,
(673) 4-morpholin-4-yl-2-(1-oxo-4-phenyl-1H-phthalazin-2-yl)-benzoic acid methyl ester,
(674) 3-chloro-N,N-dimethyl-2-(1-oxo-4-phenyl-1H-phthalazin-2-yl)-benzamide,
(675) 2-(2-chloro-6-ethyl-phenyl)-4-phenyl-2H-phthalazin-1-one,
(676) 2-(6-isopropoxy-naphthalen-1-yl)-4-phenyl-2H-phthalazin-1-one,
(677) 3-chloro-2-(1-oxo-4-phenyl-1H-phthalazin-2-yl)-benzoic acid methyl ester,
(678) 3-chloro-2-(1-oxo-4-phenyl-1H-phthalazin-2-yl)-benzoic acid,
(679) 2-[2-(1-hydroxy-ethyl)-5-morpholin-4-yl-phenyl]-4-phenyl-2H-phthalazin-1-one,
(680) 2-(2-chloro-6-nitro-phenyl)-4-phenyl-2H-phthalazin-1-one,
(681) 2-(2-acetyl-5-morpholin-4-yl-phenyl)-4-phenyl-2H-phthalazin-1-one,
(682) 2-[2-(1-hydroxy-1-methyl-ethyl)-5-morpholin-4-yl-phenyl]-4-phenyl-2H-phthalazin-1-one,
(683) 2-(2-isopropenyl-5-morpholin-4-yl-phenyl)-4-phenyl-2H-phthalazin-1-one,
(684) 2-(2-butyl-6-chloro-phenyl)-4-phenyl-2H-phthalazin-1-one,
(685) 2-[2-chloro-6-(1-hydroxy-ethyl)-phenyl]-4-phenyl-2H-phthalazin-1-one,
(686) 2-(2-acetyl-6-chloro-phenyl)-4-phenyl-2H-phthalazin-1-one,
(687) 2-(3-morpholin-4-yl-phenyl)-4-m-tolyl-2H-phthalazin-1-one,
(688) 3-acetyl-5-[3-(2,6-dichloro-phenyl)-4-oxo-3,4-dihydro-phthalazin-1-yl]-benzaldehyde,
(689) 2-(2,6-dichloro-phenyl)-4-(3-methyl-5-vinyl-phenyl)-2H-phthalazin-1-one,
(690) 5-amino-2-morpholin-4-yl-4-(1-oxo-4-m-tolyl-1H-phthalazin-2-yl)-benzonitrile,
(691) 3-[3-(2,6-dichloro-phenyl)-4-oxo-3,4-dihydro-phthalazin-1-yl]-5-{1-[(E)-hydroxyimino]-ethyl}-benzaldehyde oxime,
(692) 4-(3,5-bis-{1-[(E)-hydroxyimino]-ethyl}-phenyl)-2-(2,6-dichloro-phenyl)-2H-phthalazin-1-one,
(693) 4-(3-acetyl-5-methyl-phenyl)-2-(2,6-dichlorophenyl)-2H-phthalazin-1-one,
(694) 4-(3-bromo-5-{1-[(E)-hydroxyimino]-ethyl}-phenyl)-2-(2,6-dichloro-phenyl)-2H-phthalazin-1-one,
(695) 2-morpholin-4-yl-4-(1-oxo-4-m-tolyl-1H-phthalazin-2-yl)-benzonitrile,
(696) 5-bromo-2-morpholin-4-yl-4-(1-oxo-4-m-tolyl-1H-phthalazin-2-yl)-benzonitrile,
(697) 5-isopropenyl-2-morpholin-4-yl-4-(1-oxo-4-m-tolyl-1H-phthalazin-2-yl)-benzonitrile,
(698) 4-morpholin-4-yl-6-(1-oxo-4-m-tolyl-1H-phthalazin-2-yl)-isophthalonitrile,
(699) 4-(3-bromo-5-oxazol-2-yl-phenyl)-2-(2,6-dichlorophenyl)-2H-phthalazin-1-one,
(700) 4-(3,5-bis-oxazol-2-yl-phenyl)-2-(2,6-dichlorophenyl)-2H-phthalazin-1-one,
(701) 2-(2,6-dichloro-phenyl)-4-(3-methyl-5-oxazol-2-yl-phenyl)-2H-phthalazin-1-one,
(702) 4-(3-bromo-5-oxazol-5-yl-phenyl)-2-(2,4-dichlorophenyl)-2H-phthalazin-l-one,
(703) 2-(2,6-dichloro-phenyl)-4-(3-methyl-5-oxazol-5-yl-phenyl)-2H-phthalazin-l-one,
(704) 2-[2-amino-5-(2-hydroxy-ethylamino)-phenyl]-4-(3-bromo-5-oxazol-5-yl-phenyl)-2H-phthalazin-1-one,
(705) 2-[2-amino-5-(3-hydroxy-propylamino)-phenyl]-4-(3-bromo-5-oxazol-5-yl-phenyl)-2H-phthalazin-1-one,
(706) 2-[6-amino-2-chloro-3-(2-hydroxy-ethylamino)-phenyl]-4-(3-bromo-5-oxazol-5-yl-phenyl)-2H-phthalazin-1-one,
(707) 4-(3-bromo-5-oxazol-5-yl-phenyl)-2-[2,6-dichloro-3-(2-hydroxy-ethylamino)-phenyl]-2H-phthalazin-1-one,
(708) 4-(3-bromo-5-oxazol-5-yl-phenyl)-2-[2,6-dichloro-3-(3-hydroxy-propylamino)-phenyl]-2H-phthalazin-1-one,
(709) 4-(3-bromo-5-oxazol-5-yl-phenyl)-2-[2-chloro-5-(2-hydroxy-ethylamino)-phenyl]-2H-phthalazin-1-one,
(710) 4-(3-bromo-5-oxazol-5-yl-phenyl)-2-[2-chloro-5-(3-hydroxy-propylamino)-phenyl]-2H-phthalazin-1-one,
(711) 2-[3-(3-hydroxy-propylamino)-phenyl]-4-(3-methyl-5-oxazol-5-yl-phenyl)-2H-phthalazin-1-one,
(712) 4-(3-bromo-5-oxazol-5-yl-phenyl)-2-[5-(2-hydroxyethoxy)-2-nitro-phenyl]-2H-phthalazin-1-one,
(713) 4-(3-bromo-5-oxazol-5-yl-phenyl)-2-[5-(3-hydroxy-propoxy)-2-nitro-phenyl]-2H-phthalazin-1-one,
(714) 2-[2-bromo-5-(2-hydroxy-ethylamino)-phenyl]-4-(3-methyl-5-oxazol-5-yl-phenyl)-2H-phthalazin-1-one,
(715) 4-(3-bromo-5-oxazol-5-yl-phenyl)-2-[2-chloro-5-(2-hydroxy-ethoxy)-phenyl]-2H-phthalazin-1-one,
(716) 2-(2,6-dichloro-4-hydroxy-phenyl)-4-(3-methyl-5-oxazol-5-yl-phenyl)-2H-phthalazin-1-one,
(717) 4-bromo-2-[4-(3-methyl-5-oxazol-5-yl-phenyl)-1-oxo-1H-phthalazin-2-yl]-benzonitrile,
(718) N-[3-[4-(3-methyl-5-oxazol-5-yl-phenyl)-1-oxo-1H-phthalazin-2-yl]-4-(2,2,2-trifluoro-acetyl)-phenyl]-acetamide,
(719) 2-[5-(3-hydroxy-propylamino)-2-methoxy-phenyl]-4-(3-methyl-5-oxazol-5-yl-phenyl)-2H-phthalazin-1-one,
(720) N-{4-cyano-3-[4-(3-methyl-5-oxazol-5-yl-phenyl)-1-oxo-1H-phthalazin-2-yl]-phenyl}-acetamide,
(721) 4-(3-hydroxy-propylamino)-2-[4-(3-methyl-5-oxazol-5-yl-phenyl)-1-oxo-1H-phthalazin-2-yl]-benzonitrile,
(722) 2-[5-(3-hydroxy-propylamino)-2-(2,2,2-trifluoroacetyl)-phenyl]-4-(3-methyl-5-oxazol-5-yl-phenyl)-2H-phthalazin-1-one,
(723) 4-nitro-3-(1-oxo-4-phenyl-1H-phthalazin-2-yl)-benzoic acid,
(724) 2-(2-amino-5-morpholin-4-yl-phenyl)-4-phenyl-2H-phthalazin-1-one,
(725) 2-(2-chloro-5-morpholin-4-yl-phenyl)-4-phenyl-2H-phthalazin-1-one,
(726) 3-nitro-4-(1-oxo-4-phenyl-1H-phthalazin-2-yl)-benzoic acid ethyl ester,
(727) 3-nitro-4-(1-oxo-4-phenyl-1H-phthalazin-2-yl)-benzoic acid,
(728) 4-nitro-3-(1-oxo-4-phenyl-1H-phthalazin-2-yl)-benzamide,
(729) 4-nitro-3-(1-oxo-4-phenyl-1H-phthalazin-2-yl)-benzonitrile,
(730) N-[3-nitro-4-(1-oxo-4-phenyl-1H-phthalazin-2-yl)-phenyl]-carbamic acid tert-butyl ester,
(731) 2-(4-amino-2-nitro-phenyl)-4-phenyl-2H-phthalazin-1-one,
(732) N-[3-nitro-4-(1-oxo-4-phenyl-1H-phthalazin-2-yl)-phenyl]-acetamide,
(733) N,N-dimethyl-4-nitro-3-(1-oxo-4-phenyl-1H-phthalazin₋2-yl)-benzamide,
(734) N,N-dimethyl-3-nitro-4-(1-oxo-4-phenyl-1H-phthalazin-2-yl)-benzamide,
(735) 3-nitro-4-(1-oxo-4-phenyl-1H-phthalazin-2-yl)-benzamide,
(736) 3-nitro-4-(1-oxo-4-phenyl-1H-phthalazin-2-yl)-benzonitrile,
(737) 2-[3-(2-methoxy-ethylamino)-phenyl]-4-phenyl-2H-phthalazin-1-one,
(738) 2-(3-diethylamino-phenyl)-4-phenyl-2H-phthalazin-1-one,
(739) 2-(3-amino-phenyl)-4-phenyl-2H-phthalazin-1-one,
(740) 2-(3-butylamino-phenyl)-4-phenyl-2H-phthalazin-1-one,
(741) 2-[3-(4-methyl-3-oxo-piperazin-1-yl)-phenyl]-4-phenyl-2H-phthalazin-1-one,
(742) 2-[3-(4-dimethylamino-piperidin-1-yl)-phenyl]-4-phenyl-2H-phthalazin-1-one,
(743) 2-[5-morpholin-4-yl-2-((Z)-propenyl)-phenyl]-4-phenyl-2H-phthalazin-1-one,
(744) 2-[5-morpholin-4-yl-2-((E)-propenyl)-phenyl]-4-phenyl-2H-phthalazin-1-one,
(745) 2-(6-benzyloxy-naphthalen-1-yl)-4-phenyl-2H-phthalazin-1-one,
(746) 3-chloro-2-(1-oxo-4-phenyl-1H-phthalazin-2-yl)-benzaldehyde,
(747) 2-(2-chloro-6-hydroxymethyl-phenyl)-4-phenyl-2H-phthalazin-1-one,
(748) 2-(2-chloro-6-vinyl-phenyl)-4-phenyl-2H-phthalazin-1-one,
(749) 2-(6-hydroxy-naphthalen-1-yl)-4-phenyl-2H-phthalazin-1-one,
(750) 3-chloro-N-ethyl-2-(1-oxo-4-phenyl-1H-phthalazin-2-yl)-benzamide,
(751) 2-(2-chloro-5-morpholin-4-yl-phenyl)-4-m-tolyl-2H-phthalazin-1-one,
(752) 2-(2-amino-5-morpholin-4-yl-phenyl)-4-(3,5-dimethyl-phenyl)-2H-phthalazin-1-one,
(753) 4-(3,5-dimethyl-phenyl)-2-(3-morpholin-4-yl-phenyl)-2H-phthalazin-1-one,
(754) 2-(2-chloro-5-morpholin-4-yl-phenyl)-4-(3,5-dimethyl-phenyl)-2H-phthalazin-1-one,
(755) 3-[3-(2,6-dichloro-phenyl)-4-oxo-3,4-dihydro-phthalazin-1-yl]-5-vinyl-benzaldehyde oxime,
(756) 2-(2-nitro-5-thiophen-2-yl-phenyl)-4-phenyl-2H-phthalazin-1-one,
(757) 4-(3,5-dibromo-phenyl)-2-(2,6-dichloro-phenyl)-2H-phthalazin-1-one,
(758) 4-(3-acetyl-phenyl)-2-(2,6-dichloro-phenyl)-2H-phthalazin-1-one,
(759) 4-(3,5-diacetyl-phenyl)-2-(2,6-dichloro-phenyl)-2H-phthalazin-1-one,
(760) 4-(3-acetyl-5-bromo-phenyl)-2-(2,6-dichlorophenyl)-2H-phthalazin-1-one,
(761) 3-bromo-5-[3-(2,6-dichloro-phenyl)-4-oxo-3,4-dihydro-phthalazin-1-yl]-benzaldehyde,
(762) 4-(3-acetyl-5-vinyl-phenyl)-2-(2,6-dichlorophenyl)-2H-phthalazin-1-one,
(763) 3-bromo-5-[3-(2,6-dichloro-phenyl)-4-oxo-3,4-dihydro-phthalazin-1-yl]-benzaldehyde oxime,
(764) 4-(3-bromo-5-methyl-phenyl)-2-(2,6-dichlorophenyl)-2H-phthalazin-1-one,
(765) 3-(4-oxo-3-phenyl-3,4-dihydro-phthalazin-1-yl)-5-vinyl-benzaldehyde oxime,
(766) 3-[3-(2-nitro-phenyl)-4-oxo-3,4-dihydro-phthalazin-1-yl]-5-vinyl-benzaldehyde oxime,
(767) 3-[3-(2-chloro-phenyl)-4-oxo-3,4-dihydro-phthalazin-1-yl]-5-vinyl-benzaldehyde oxime,
(768) 4-(3-bromo-5-oxazol-5-yl-phenyl)-2-phenyl-2H-phthalazin-1-one,
(769) 4-(3-bromo-5-oxazol-5-yl-phenyl)-2-(2,6-dichlorophenyl)-2H-phthalazin-1-one,
(770) 4-(3-bromo-5-oxazol-5-yl-phenyl)-2-(2-nitrophenyl)-2H-phthalazin-1-one,
(771) 2-(2-amino-phenyl)-4-(3-bromo-5-oxazol-5-yl-phenyl)-2H-phthalazin-1-one,
(772) 4-(3-bromo-5-oxazol-5-yl-phenyl)-2-(2-chlorophenyl)-2H-phthalazin-1-one,
(773) 4-(3-bromo-5-oxazol-5-yl-phenyl)-2-(4-chlorophenyl)-2H-phthalazin-1-one,
(774) 4-(3-bromo-5-oxazol-5-yl-phenyl)-2-(3-chlorophenyl)-2H-phthalazin-1-one,
(775) 4-(3-bromo-5-oxazol-5-yl-phenyl)-2-naphthalen-1-yl-2H-phthalazin-1-one,
(776) 4-(3-bromo-5-oxazol-5-yl-phenyl)-2-naphthalen-2-yl-2H-phthalazin-1-one,
(777) 4-(3-bromo-5-oxazol-5-yl-phenyl)-2-(5-chloro-2-nitro-phenyl)-2H-phthalazin-1-one,
(778) 4-(3-bromo-5-oxazol-5-yl-phenyl)-2-(2,3-dichlorophenyl)-2H-phthalazin-1-one,
(779) 4-(3-bromo-5-oxazol-5-yl-phenyl)-2-[2-chloro-5-(3-hydroxy-propoxy)-phenyl]-2H-phthalazin-1-one,
(780) 4-(3-bromo-5-oxazol-5-yl-phenyl)-2-[3-(2-hydroxyethoxy)-phenyl]₋2H-phthalazin-1-one,
(781) 4-(3-bromo-5-oxazol-5-yl-phenyl)-2-[3-(3-hydroxy-propoxy)-phenyl]-2H-phthalazin-1-one,
(782) 2-(5-bromo-2-fluoro-phenyl)-4-(3-methyl-5-oxazol-5-yl-phenyl)-2H-phthalazin-1-one,
(783) 2-[2-bromo-5-(3-hydroxy-propylamino)-phenyl]-4-(3-methyl-5-oxazol-5-yl-phenyl)-2H-phthalazin-1-one,
(784) 2-(5-bromo-2-methanesulfonyl-phenyl)-4-(3-methyl-5-oxazol-5-yl-phenyl)-2H-phthalazin-1-one,
(785) N-{4-fluoro-3-[4-(3-methyl-5-oxazol-5-yl-phenyl)-1-oxo-1H-phthalazin-2-yl]-phenyl}-acetamide,
(786) N-{4-methanesulfonyl-3-[4-(3-methyl-5-oxazol-5-yl-phenyl)-1-oxo-1H-phthalazin-2-yl]-phenyl}-acetamide,
(787) 2-[2-fluoro-5-(3-hydroxy-propylamino)-phenyl]-4-(3-methyl-5-oxazol-5-yl-phenyl)-2H-phthalazin-1-one,
(788) 2-[2-ethyl-5-(3-hydroxy-propylamino)-phenyl]-4-(3-methyl-5-oxazol-5-yl-phenyl)-2H-phthalazin-1-one,
(789) 2-[5-(3-hydroxy-propylamino)-2-methyl-phenyl]-4-(3-methyl-5-oxazol-5-yl-phenyl)-2H-phthalazin-1-one,
(790) 2-[5-(3-hydroxy-propylamino)-2-methanesulfonylphenyl]-4-(3-methyl-5-oxazol-5-yl-phenyl)-2H-phthalazin-1-one,
(791) 2-[5-(3-hydroxy-propylamino)-2-trifluoromethylphenyl]-4-(3-methyl-5-oxazol-5-yl-phenyl)-2H-phthalazin-1-one,
(792) 2-(2,6-dichloro-4-methoxy-phenyl)-4-(3-methyl-5-oxazol-5-yl-phenyl)-2H-phthalazin-1-one,
(794) 4-(3-[1,3]dioxolan-2-yl-phenyl)-2-(5-morpholin-4-yl-2-nitro-phenyl)-2H-phthalazin-1-one,
(795) 4-(3-bromo-5-[1,3]dioxolan-2-yl-phenyl)-2-(5-morpholin-4-yl-2-nitro-phenyl)-2H-phthalazin-1-one,
(796) 4-[3-bromo-5-(1H-pyrazol-3-yl)-phenyl]-2-(5-morpholin-4-yl-2-nitro-phenyl)-2H-phthalazin-1-one,
(797) 2-(5-morpholin-4-yl-2-nitro-phenyl)-4-[3-(1H-pyrazol-3-yl)-5-vinyl-phenyl]-2H-phthalazin-1-one,
(798) 2-(5-morpholin-4-yl-2-nitro-phenyl)-4-(3-oxazol-2-yl-5-vinyl-phenyl)-2H-phthalazin-1-one,
(799) 2-(5-morpholin-4-yl-2-nitro-phenyl)-4-(3-pyrrolidin-1-yl-5-vinyl-phenyl)-2H-phthalazin-1-one,
(800) 2-(5-morpholin-4-yl-2-nitro-phenyl)-4-(3-pyridin-2-yl-5-vinyl-phenyl)-2H-phthalazin-1-one hydrochloride,
(801) 4-(3-methyl-5-oxazol-2-yl-phenyl)-2-(5-morpholin-4-yl-2-nitro-phenyl)-2H-phthalazin-1-one,
(802) 2-(5-morpholin-4-yl-2-nitro-phenyl)-4-(3-thiazol-2-yl-5-vinyl-phenyl)-2H-phthalazin-1-one,
(803) 4-(3-furan-2-yl-5-vinyl-phenyl)-2-(5-morpholin-4-yl-2-nitro-phenyl)-2H-phthalazin-1-one,
(804) 4-(3-[1,3]dioxolan-2-yl-5-methoxy-phenyl)-2-(5-morpholin-4-yl-2-nitro-phenyl)-2H-phthalazin-1-one,
(805) 2-(5-morpholin-4-yl-2-nitro-phenyl)-4-(5-vinyl-biphenyl-3-yl)-2H-phthalazin-1-one,
(806) 4-[3-bromo-5-(4H-[1,2,4]triazol-3-yl)-phenyl]-2-(5-morpholin-4-yl-2-nitro-phenyl)-2H-phthalazin-1-one,
(807) 4-(3-bromo-5-[1,3,4]thiadiazol-2-yl-phenyl)-2-(5-morpholin-4-yl-2-nitro-phenyl)-2H-phthalazin-1-one,
(808) 4-[3-bromo-5-(4-methyl-thiazol-2-yl)-phenyl]-2-(5-morpholin-4-yl-2-nitro-phenyl)-2H-phthalazin-1-one,
(809) 4-[3-bromo-5-(5-methyl-thiazol-2-yl)-phenyl]-2-(5-morpholin-4-yl-2-nitro-phenyl)-2H-phthalazin-1-one,
(810) 4-(3-bromo-5-oxazol-5-yl-phenyl)-2-[5-(2-hydroxy-ethylamino)-2-nitro-phenyl]-2H-phthalazin-1-one,
(811) 4-(3-bromo-5-oxazol-5-yl-phenyl)-2-[5-(3-hydroxy-propylamino)-2-nitro-phenyl]-2H-phthalazin-1-one,
(812) 4-(3-bromo-5-isoxazol-4-yl-phenyl)-2-(5-morpholin-4-yl-2-nitro-phenyl)-2H-phthalazin-1-one,
(813) 4-(3-bromo-5-oxazol-5-yl-phenyl)-2-[2-chloro-3-(2-hydroxy-ethylamino)-6-nitro-phenyl]-2H-phthalazin-1-one,
(814) 4-(3-bromo-5-oxazol-5-yl-phenyl)-2-(2-nitro-5-piperidin-1-yl-phenyl)-2H-phthalazin-1-one,
(815) 4-(3-bromo-5-oxazol-5-yl-phenyl)-2-[2-chloro-3-(3-hydroxy-propylamino)-6-nitro-phenyl]-2H-phthalazin-1-one,
(816) 4-(3-methoxy-5-oxazol-5-yl-phenyl)-2-(5-morpholin-4-yl-2-nitro-phenyl)-2H-phthalazin-1-one,
(817) 4-(3-bromo-5-[1,2,4]oxadiazol-5-yl-phenyl)-2-(5-morpholin-4-yl-2-nitro-phenyl)-2H-phthalazin-1-one,
(818) 4-(3-bromo-5-[1,3,4]oxadiazol-2-yl-phenyl)-2-(5-morpholin-4-yl-2-nitro-phenyl)-2H-phthalazin-1-one,
(819) 4-(3-methoxy-5-oxazol-2-yl-phenyl)-2-(5-morpholin-4-yl-2-nitro-phenyl)-2H-phthalazin-1-one,
(820) 4-(3-furan-3-yl-5-vinyl-phenyl)-2-(5-morpholin-4-yl-2-nitro-phenyl)-2H-phthalazin-1-one,
(821) 4-(3-bromo-5-oxazol-5-yl-phenyl)-2-(5-morpholin-4-yl-2-nitro-phenyl)-2H-phthalazin-1-one,
(822) 4-(3,5-bis-oxazol-2-yl-phenyl)-2-(5-morpholin-4-yl-2-nitro-phenyl)-2H-phthalazin-1-one,
(823) 4-(3-bromo-5-oxazol-2-yl-phenyl)-2-(5-morpholin-4-yl-2-nitro-phenyl)-2H-phthalazin-1-one,
(824) 4-(3-bromo-5-isoxazol-5-yl-phenyl)-2-(5-morpholin-4-yl-2-nitro-phenyl)-2H-phthalazin-1-one,
(825) 4-[3-bromo-5-(5-methyl-[1,3,4]oxadiazol-2-yl)-phenyl]-2-(5-morpholin-4-yl-2-nitro-phenyl)-2H-phthalazin-1-one,
(826) 4-(3-methyl-5-oxazol-5-yl-phenyl)-2-(5-morpholin-4-yl-2-nitro-phenyl)-2H-phthalazin-1-one,
(827) 2-(5-morpholin-4-yl-2-nitro-phenyl)-4-(3-oxazol-5-yl-5-vinyl-phenyl)-2H-phthalazin-1-one,
(828) 4-(3-bromo-5-thiazol-5-yl-phenyl)-2-(5-morpholin-4-yl-2-nitro-phenyl)-2H-phthalazin-1-one,
(829) 4-(3-bromo-5-thiophen-2-yl-phenyl)-2-(5-morpholin-4-yl-2-nitro-phenyl)-2H-phthalazin-1-one,
(830) 4-(3-bromo-5-[1,2,4]oxadiazol-3-yl-phenyl)-2-(5-morpholin-4-yl-2-nitro-phenyl)-2H-phthalazin-1-one,
(831) 4-(5-methyl-thiophen-2-yl)-2-(5-morpholin-4-yl-2-nitro-phenyl)-2H-phthalazin-1-one,
(832) 2-(2-nitro-phenyl)-4-pyridin-4-yl-2H-phthalazin-1-one,
(833) 4-benzofuran-2-yl-2-(5-morpholin-4-yl-2-nitrophenyl)-2H-phthalazin-1-one,
(834) 4-cyclohexyl-2-(2-nitro-5-piperidin-1-yl-phenyl)-2H-phthalazin-1-one,
(835) 4-cycloheptyl-2-(2-nitro-phenyl)-2H-phthalazin-1-one,
(836-1) 4-isopropyl-6-(3-methoxy-phenyl)-2-(5-morpholin-4-yl-2-nitro-phenyl)-2H-pyridazin-3-one,
(836-2) 2-(5-chloro-2-nitro-phenyl)-6-(3-methoxyphenyl)-4-propoxy-2H-pyridazin-3-one,
(836-3) 6-(3-methoxy-phenyl)-2-(5-morpholin-4-yl-2-nitro-phenyl)-4-propoxy-2H-pyridazin-3-one,
(836-4) 6-(2-chloro-5-methoxy-phenyl)-4-ethylsulfanyl-2-(5-morpholin-4-yl-2-nitro-phenyl)-2H-pyridazin-3-one,
(836-5) ethyl 6-(3-hydroxymethyl-5-methyl-phenyl)-2-(5-nitro-2-trifluoromethyl-phenyl)-3-oxo-2,3-dihydro-pyridazin-4-carboxylate,
(836-6) methyl 2-(5-bromo-2-trifluoromethyl-phenyl)-6-(3-hydroxymethyl-5-methyl-phenyl)-3-oxo-2,3-dihydro-pyridazin-4-carboxylate,
(836-7) tert-butyl {2-(5-bromo-2-trifluoromethylphenyl)-6-[3-methyl-5-(oxazol-5-yl)-phenyl]-3-oxo-2,3-dihydro-pyridazin-4-yl}-carbamate,
(836-8) tert-butyl {2-(5-bromo-2-trifluoromethylphenyl)-6-[3-methyl-5-(oxazol-5-yl)-phenyl]-3-oxo-2,3-dihydro-pyridazin-4-yl}-ethyl-carbamate,
(836-9) 4-ethylamino-2-[5-((S)-3-hydroxy-pyrrolidin-1-yl)-2-trifluoromethyl-phenyl]-6-(3-methyl-5-oxazol-5-yl-phenyl)-2H-pyridazin-3-one,
(836-10) ethyl 2-(2-chloro-6-trifluoromethyl-phenyl)-6-(3-hydroxymethyl-5-methyl-phenyl)-3-oxo-2,3-dihydro-pyridazin-4-carboxylate,
(836-11) tert-butyl {2-(2-chloro-6-trifluoromethylphenyl)-6-[3-methyl-5-(oxazol-5-yl)-phenyl]-3-oxo-2,3-dihydro-pyridazin-4-yl}-carbamate,
(836-12) 2-(2-chloro-6-trifluoromethyl-phenyl)-4-ethylamino-6-(3-methyl-5-oxazol-5-yl-phenyl)-2H-pyridazin-3-one,
(836-13) ethyl 6-(3-methoxy-phenyl)-2-[4-(morpholin-4-yl)-pyridin-2-yl]-3-oxo-2,3-dihydro-pyridazin-4-carboxylate,
(836-14) tert-butyl {6-(3-methoxy-phenyl)-2-[4-(morpholin-4-yl)-pyridin-2-yl]-3-oxo-2,3-dihydro-pyridazin-4-yl}-carbamate,
(836-15) 4-amino-6-(3-methoxy-phenyl)-2-[4-(morpholin-4-yl)-pyridin-2-yl]-2H-pyridazin-3-one,
(836-16) 4-isopropylamino-6-(3-methoxy-phenyl)-2-(4-morpholin-4-yl-pyridin-2-yl)-2H-pyridazin-3-one,
(837) 6-(2-chloro-5-methoxy-phenyl)-4-dimethylamino-2-(5-morpholin-4-yl-2-nitro-phenyl)-2H-pyridazin-3-one,
(838) 6-(2-chloro-5-methoxy-phenyl)-2-(5-morpholin-4-yl-2-nitro-phenyl)-4-pyrrolidin-1-yl-2H-pyridazin-3-one,
(839) 4-dimethylamino-6-(3-methoxy-phenyl)-2-(5-morpholin-4-yl-2-nitro-phenyl)-2H-pyridazin-3-one,
(840) 6-(3-methoxy-phenyl)-2-(5-morpholin-4-yl-2-nitrophenyl)-4-pyrrolidin-1-yl-2H-pyridazin-3-one,
(841) [6-(3-methoxy-phenyl)-2-(5-morpholin-4-yl-2-nitro-phenyl)-3-oxo-2,3-dihydro-pyridazin-4-yl]-carbamic acid tert-butyl ester,
(842) [6-(3-methoxy-phenyl)-2-(5-morpholin-4-yl-2-nitro-phenyl)-3-oxo-2,3-dihydro-pyridazin-4-yl]-methyl-carbamic acid tert-butyl ester,
(843) 6-(3-methoxy-phenyl)-4-methylamino-2-(5-morpholin-4-yl-2-nitro-phenyl)-2H-pyridazin-3-one,
(844) N-[6-(3-methoxy-phenyl)-2-(5-morpholin-4-yl-2-nitro-phenyl)-3-oxo-2,3-dihydro-pyridazin-4-yl]-N-methyl-acetamide,
(845) N-[6-(3-methoxy-phenyl)-2-(5-morpholin-4-yl-2-nitro-phenyl)-3-oxo-2,3-dihydro-pyridazin-4-yl]-N-methyl-methanesulfonamide,
(846) 6-(3-methoxy-phenyl)-4-(methyl-propyl-amino)-2-(5-morpholin-4-yl-2-nitro-phenyl)-2H-pyridazin-3-one,
(847) 4-(isobutyl-methyl-amino)-6-(3-methoxy-phenyl)-2-(5-morpholin-4-yl-2-nitro-phenyl)-2H-pyridazin-3-one,
(848) 4-(ethyl-methyl-amino)-6-(3-methoxy-phenyl)-2-(5-morpholin-4-yl-2-nitro-phenyl)-2H-pyridazin-3-one,
(849) 4-[(2-hydroxy-ethyl)-methyl-amino]-6-(3-methoxyphenyl)-2-(5-morpholin-4-yl-2-nitro-phenyl)-2H-pyridazin-3-one,
(850) 4-[(3-hydroxy-propyl)-methyl-amino]-6-(3-methoxyphenyl)-2-(5-morpholin-4-yl-2-nitro-phenyl)-2H-pyridazin-3-one,
(851) 4-(isopropyl-methyl-amino)-6-(3-methoxy-phenyl)-2-(5-morpholin-4-yl-2-nitro-phenyl)-2H-pyridazin-3-one,
(852) 4-(ethyl-methyl-amino)-6-(3-methoxy-phenyl)-2-(2-nitro-phenyl)-2H-pyridazin-3-one,
(853) 2-(2-amino-phenyl)-4-(ethyl-methyl-amino)-6-(3-methoxy-phenyl)-2H-pyridazin-3-one,
(854) 4-(ethyl-methyl-amino)-6-(3-methoxy-phenyl)-2-phenyl-2H-pyridazin-3-one,
(855) 2-(2-chloro-phenyl)-4-(ethyl-methyl-amino)-6-(3-methoxy-phenyl)-2H-pyridazin-3-one,
(856) 4-[(2-dimethylamino-ethyl)-methyl-amino]-6-(3-methoxy-phenyl)-2-(5-morpholin-4-yl-2-nitro-phenyl)-2H-pyridazin-3-one,
(857) 2-(2-bromo-phenyl)-4-(ethyl-methyl-amino)-6-(3-methoxy-phenyl)-2H-pyridazin-3-one,
(858) 4-(ethyl-methyl-amino)-2-(2-ethyl-phenyl)-6-(3-methoxy-phenyl)-2H-pyridazin-3-one,
(859) 4-diisopropylamino-6-(3-methoxy-phenyl)-2-(5-morpholin-4-yl-2-nitro-phenyl)-2H-pyridazin-3-one,
(860) 2-(2,6-dimethyl-phenyl)-4-(ethyl-methyl-amino)-6-(3-methoxy-phenyl)-2H-pyridazin-3-one,
(861) 2-(2,6-dichloro-4-nitro-phenyl)-4-(ethyl-methylamino)-6-(3-methoxy-phenyl)-2H-pyridazin-3-one,
(862) 2-(4-amino-2,6-dichloro-phenyl)-4-(ethyl-methylamino)-6-(3-methoxy-phenyl)-2H-pyridazin-3-one,
(863) 4-(ethyl-methyl-amino)-6-(3-methoxy-phenyl)-2-(3-nitro-phenyl)-2H-pyridazin-3-one,
(864) 2-(3-amino-phenyl)-4-(ethyl-methyl-amino)-6-(3-methoxy-phenyl)-2H-pyridazin-3-one,
(865) 2-(2,6-dichloro-phenyl)-4-(ethyl-methyl-amino)-6-(3-methoxy-phenyl)-2H-pyridazin-3-one,
(866) 3-bromo-5-[1-(2-chloro-phenyl)-6-oxo-5-pyrrolidin-1-yl-1,6-dihydro-pyridazin-3-yl]-benzaldehyde oxime,
(867) 2-(3,5-di-morpholin-4-yl-phenyl)-4-(ethyl-methylamino)-6-(3-methoxy-phenyl)-2H-pyridazin-3-one,
(868) 4-(ethyl-methyl-amino)-6-(3-methoxy-phenyl)-2-(2-nitro-5-piperidin-1-yl-phenyl)-2H-pyridazin-3-one,
(869) 4-(ethyl-methyl-amino)-2-[3-(2-methoxy-ethylamino)-phenyl]-6-(3-methoxy-phenyl)-2H-pyridazin-3-one,
(870) 2-(3,5-bis-propylamino-phenyl)-4-(ethyl-methylamino)-6-(3-methoxy-phenyl)-2H-pyridazin-3-one,
(871) 2-(2-chloro-5-piperidin-1-yl-phenyl)-4-(ethylmethyl-amino)-6-(3-methoxy-phenyl)-2H-pyridazin-3-one,
(872) 4-(ethyl-methyl-amino)-6-(3-methoxy-phenyl)-2-(3-propylamino-phenyl)-2H-pyridazin-3-one,
(873) 4-(ethyl-methyl-amino)-6-(3-methoxy-phenyl)-2-(3-propyl-phenyl)-2H-pyridazin-3-one,
(874) 2-(2-amino-5-morpholin-4-yl-phenyl)-4-(ethylmethyl-amino)-6-(3-methoxy-phenyl)-2H-pyridazin-3-one,
(875) 2-(2-chloro-5-morpholin-4-yl-phenyl)-4-(ethylmethyl-amino)-6-(3-methoxy-phenyl)-2H-pyridazin-3-one,
(876) 2-(2-chloro-3-morpholin-4-yl-6-nitro-phenyl)-4-(ethyl-methyl-amino)-6-(3-methoxy-phenyl)-2H-pyridazin-3-one,
(877) 2-(3,5-bis-benzyloxy-phenyl)-4-(ethyl-methylamino)-6-(3-methoxy-phenyl)-2H-pyridazin-3-one,
(878) 2-(2,6-bis-benzyloxy-phenyl)-4-(ethyl-methylamino)-6-(3-methoxy-phenyl)-2H-pyridazin-3-one,
(879) 2-(3,5-dihydroxy-phenyl)-4-(ethyl-methyl-amino)-6-(3-methoxy-phenyl)-2H-pyridazin-3-one,
(880) 2-(2,6-dihydroxy-phenyl)-4-(ethyl-methyl-amino)-6-(3-methoxy-phenyl)-2H-pyridazin-3-one,
(881) 3-[1-(2,6-dichloro-phenyl)-6-oxo-5-pyrrolidin-1-yl-1,6-dihydro-pyridazin-3-yl]-5-vinyl-benzaldehyde oxime,
(882) 4-(ethyl-methyl-amino)-6-(3-methoxy-phenyl)-2-[5-(4-methoxy-piperidin-1-yl)-2-nitro-phenyl]-2H-pyridazin-3-one,
(883) 4-(ethyl-methyl-amino)-2-[5-(2-methoxy-ethylamino)-2-nitro-phenyl]-6-(3-methoxy-phenyl)-2H-pyridazin-3-one,
(884) 4-(ethyl-methyl-amino)-2-[5-(2-hydroxy-ethylamino)-2-nitro-phenyl]-6-(3-methoxy-phenyl)-2H-pyridazin-3-one,
(885) 2-(2-dimethylamino-5-morpholin-4-yl-phenyl)-4-(ethyl-methyl-amino)-6-(3-methoxy-phenyl)-2H-pyridazin-3-one,
(886) 2-[5-(1,1-dioxo-1λ⁶-thiomorpholin-4-yl)-2-nitrophenyl]-4-(ethyl-methyl-amino)-6-(3-methoxy-phenyl)-2H-pyridazin-3-one,
(887) 4-(ethyl-methyl-amino)-6-(3-methoxy-phenyl)-2-[5-(4-methyl-piperazin-1-yl)-2-nitro-phenyl]-2H-pyridazin-3-one,
(888) 2-[5-(4-acetyl-piperazin-1-yl)-2-nitro-phenyl]-4-(ethyl-methyl-amino)-6-(3-methoxy-phenyl)-2H-pyridazin-3-one,
(889) 4-(ethyl-methyl-amino)-2-[5-(3-hydroxy-pyrrolidin-1-yl)-2-nitro-phenyl]-6-(3-methoxy-phenyl)-2H-pyridazin-3-one,
(890) 4-(ethyl-methyl-amino)-6-(3-methoxy-phenyl)-2-(2-nitro-5-thiophen-2-yl-phenyl)-2H-pyridazin-3-one,
(891) 2-[5-(ethyl-methyl-amino)-3-(3-methoxy-phenyl)-6-oxo-6H-pyridazin-1-yl]-4-morpholin-4-yl-benzaldehyde,
(892) 2-[5-(ethyl-methyl-amino)-3-(3-methoxy-phenyl)-6-oxo-6H-pyridazin-1-yl]-4-morpholin-4-yl-benzaldehyde oxime,
(893) 4-(ethyl-methyl-amino)-2-[2-(1-hydroxy-ethyl)-5-morpholin-4-yl-phenyl]-6-(3-methoxy-phenyl)-2H-pyridazin-3-one,
(894) 2-(2-acetyl-5-morpholin-4-yl-phenyl)-4-(ethylmethyl-amino)-6-(3-methoxy-phenyl)-2H-pyridazin-3-one,
(895) 2-(2-acetoxyiminomethyl-5-morpholin-4-yl-phenyl)-4-(ethyl-methyl-amino)-6-(3-methoxy-phenyl)-2H-pyridazin-3-one,
(896) 2-[5-(ethyl-methyl-amino)-3-(3-methoxy-phenyl)-6-oxo-6H-pyridazin-1-yl]-4-morpholin-4-yl-benzonitrile,
(897) 4-(ethyl-methyl-amino)-2-[5-(3-hydroxy-piperidin-1-yl)-2-nitro-phenyl]-6-(3-methoxy-phenyl)-2H-pyridazin-3-one,
(898) 4-(ethyl-methyl-amino)-2-[5-((R)-2-hydroxymethyl-pyrrolidin-1-yl)-2-nitro-phenyl]-6-(3-methoxy-phenyl)-2H-pyridazin-3-one,
(899) 4-(ethyl-methyl-amino)-2-[5-((S)-2-hydroxymethyl-pyrrolidin-1-yl)-2-nitro-phenyl]-6-(3-methoxy-phenyl)-2H-pyridazin-3-one,
(900) 4-(ethyl-methyl-amino)-6-(3-methoxy-phenyl)-2-[2-nitro-5-(3-oxo-piperidin-1-yl)-phenyl]-2H-pyridazin-3-one,
(901) 2-[5-((R)-3-hydroxy-pyrrolidin-1-yl)-2-nitrophenyl]-4-isopropylamino-6-(3-methoxy-phenyl)-2H-pyridazin-3-one,
(902) 2-[5-((S)-3-hydroxy-pyrrolidin-1-yl)-2-nitrophenyl]-4-isopropylamino-6-(3-methoxy-phenyl)-2H-pyridazin-3-one,
(903) 4-isopropylamino-6-(3-methoxy-phenyl)-2-[2-nitro-5-(3-oxo-pyrrolidin-1-yl)-phenyl]-2H-pyridazin-3-one,
(904) 4-(ethyl-methyl-amino)-6-(3-methoxy-phenyl)-2-[2-nitro-5-(3-oxo-piperazin-1-yl)-phenyl]-2H-pyridazin-3-one,
(905) 4-(ethyl-methyl-amino)-2-[5-(3-hydroxy-propylamino)-2-nitro-phenyl]-6-(3-methoxy-phenyl)-2H-pyridazin-3-one,
(906) 4-(ethyl-methyl-amino)-6-(3-methoxy-phenyl)-2-[5-(3-methoxy-propylamino)-2-nitro-phenyl]-2H-pyridazin-3-one,
(907) 4-(ethyl-methyl-amino)-6-(3-methoxy-phenyl)-2-[5-(4-methyl-3-oxo-piperazin-1-yl)-2-nitro-phenyl]-2H-pyridazin-3-one,
(908) 4-(ethyl-methyl-amino)-2-{5-[2-(2-hydroxyethoxy)-ethylamino]-2-nitro-phenyl}-6-(3-methoxyphenyl)-2H-pyridazin-3-one,
(909) acetic acid n'-{3-[5-(ethyl-methyl-amino)-3-(3-methoxy-phenyl)-6-oxo-6H-pyridazin-1-yl]-4-nitrophenyl}-hydrazide,
(910) (2-{3-[5-(ethyl-methyl-amino)-3-(3-methoxyphenyl)-6-oxo-6H-pyridazin-1-yl]-4-nitro-phenyl}-hydrazine)-carboxamide,
(911) 4-(ethyl-methyl-amino)-2-[5-(3-hydroxy-[1,2,4]triazol-1-yl)-2-nitro-phenyl]-6-(3-methoxyphenyl)-2H-pyridazin-3-one,
(912) 4-(ethyl-methyl-amino)-6-(3-methoxy-phenyl)-2-(2-methyl-5-morpholin-4-yl-phenyl)-2H-pyridazin-3-one,
(913) 6-(3-bromo-5-oxazol-5-yl-phenyl)-4-ethylamino-2-phenyl-2H-pyridazin-3-one,
(914) 6-(3-bromo-5-oxazol-5-yl-phenyl)-2-(2,6-dichlorophenyl)-4-ethylamino-2H-pyridazin-3-one,
(915) 6-(3-bromo-5-oxazol-5-yl-phenyl)-4-ethylamino-2-(5-morpholin-4-yl-2-nitro-phenyl)-2H-pyridazin-3-one,
(916) 2-[5-((S)-3-hydroxy-pyrrolidin-1-yl)-2-trifluoromethyl-phenyl]-4-isopropylamino-6-(3-methoxyphenyl)-2H-pyridazin-3-one,
(917) 2-[5-((S)-3-hydroxy-pyrrolidin-1-yl)-2-trifluoromethyl-phenyl]-4-isopropylamino-6-(3-methyl-5-oxazol-5-yl-phenyl)-2H-pyridazin-3-one,
(918) 2-[5-(2-hydroxy-ethylamino)-2-trifluoromethylphenyl]-4-isopropylamino-6-(3-methyl-5-oxazol-5-yl-phenyl)-2H-pyridazin-3-one,
(919) 6-(3,5-dimethyl-phenyl)-2-[5-((S)-3-hydroxy-pyrrolidin-1-yl)-2-trifluoromethyl-phenyl]-4-isopropylamino-2H-pyridazin-3-one,
(920) 2-[5-((S)-3-hydroxy-pyrrolidin-1-yl)-2-trifluoromethyl-phenyl]-4-methylamino-6-(3-methyl-5-oxazol-5-yl-phenyl)-2H-pyridazin-3-one,
(921) 4-ethylamino-2-[5-(2-hydroxy-ethylamino)-2-trifluoromethyl-phenyl]-6-(3-methyl-5-oxazol-5-yl-phenyl)-2H-pyridazin-3-one,
(922) 2-[5-((S)-3-hydroxy-pyrrolidin-1-yl)-2-trifluoromethyl-phenyl]-6-(3-methyl-5-oxazol-5-yl-phenyl)-4-pyrrolidin-1-yl-2H-pyridazin-3-one,
(923) 4-(2-hydroxy-ethylamino)-2-[5-((S)-3-hydroxy-pyrrolidin-1-yl)-2-trifluoromethyl-phenyl]-6-(3-methyl-5-oxazol-5-yl-phenyl)-2H-pyridazin-3-one,
(924) 4-{3-[5-ethylamino-3-(3-methyl-5-oxazol-5-yl-phenyl)-6-oxo-6H-pyridazin-1-yl]-4-trifluoromethylphenyl}-morpholin-3-one,
(925) {3-[5-ethylamino-3-(3-methyl-5-oxazol-5-yl-phenyl)-6-oxo-6H-pyridazin-1-yl]-4-trifluoromethylphenyl}-carbamic acid benzyl ester,
(926) 4-ethylamino-6-(3-methyl-5-oxazol-5-yl-phenyl)-2-(5-oxazol-2-yl-2-trifluoromethyl-phenyl)-2H-pyridazin-3-one,
(927) 4-ethylamino-6-(3-methyl-5-oxazol-5-yl-phenyl)-2-(5-pyrrolidin-1-yl-2-trifluoromethyl-phenyl)-2H-pyridazin-3-one,
(928) 4-ethylamino-2-[5-(3-hydroxy-prop-1-ynyl)-2-trifluoromethyl-phenyl]-6-(3-methyl-5-oxazol-5-yl-phenyl)-2H-pyridazin-3-one,
(929) 4-ethylamino-2-[5-(3-hydroxy-propyl)-2-trifluoromethyl-phenyl]-6-(3-methyl-5-oxazol-5-yl-phenyl)-2H-pyridazin-3-one,
(930) 4-ethylamino-2-(5-ethylamino-2-trifluoromethylphenyl)-6-(3-methyl-5-oxazol-5-yl-phenyl)-2H-pyridazin-3-one,
(931) 4-ethylamino-6-(3-methyl-5-oxazol-5-yl-phenyl)-2-(5-pyrimidin-5-yl-2-trifluoromethyl-phenyl)-2H-pyridazin-3-one,
(932) 4-ethylamino-2-[5-(2-hydroxy-ethoxy)-2-trifluoromethyl-phenyl]-6-(3-methyl-5-oxazol-5-yl-phenyl)-2H-pyridazin-3-one,
(933) 4-ethylamino-6-(3-methyl-5-oxazol-5-yl-phenyl)-2-(5-pyrimidin-2-yl-2-trifluoromethyl-phenyl)-2H-pyridazin-3-one,
(934) 4-ethylamino-2-[5-(3-hydroxymethyl-pyrrolidin-1-yl)-2-trifluoromethyl-phenyl]-6-(3-methyl-5-oxazol-5-yl-phenyl)-2H-pyridazin-3-one,
(935) 4-ethylamino-6-(3-methyl-5-oxazol-5-yl-phenyl)-2-(5-pyridazin-4-yl-2-trifluoromethyl-phenyl)-2H-pyridazin-3-one,
(936) 4-ethylamino-6-(3-methyl-5-oxazol-5-yl-phenyl)-2-(5-morpholin-4-yl-2-trifluoromethyl-phenyl)-2H-pyridazin-3-one,
(937) 6-(3,5-dimethyl-phenyl)-4-ethylamino-2-[5-((S)-3-hydroxy-pyrrolidin-1-yl)-2-trifluoromethyl-phenyl]-2H-pyridazin-3-one,
(938) 4-ethylamino-6-(3-methyl-5-oxazol-5-yl-phenyl)-2-[5-(3-methyl-pyrrolidin-1-yl)-2-trifluoromethylphenyl]-2H-pyridazin-3-one,
(939) 3-{5-ethylamino-1-[5-((S)-3-hydroxy-pyrrolidin-1-yl)-2-trifluoromethyl-phenyl]-6-oxo-1,6-dihydro-pyridazin-3-yl}-5-methyl-benzaldehyde oxime,
(940) 4-isopropyl-6-(3-methoxy-phenyl)-2-(3-morpholin-4-yl-phenyl)-2H-pyridazin-3-one,
(941) 2-(2-chloro-5-morpholin-4-yl-phenyl)-4-isopropyl-6-(3-methoxy-phenyl)-2H-pyridazin-3-one,
(942) 6-(3-methoxy-phenyl)-2-(5-morpholin-4-yl-2-nitrophenyl)-3-oxo-2,3-dihydro-pyridazine-4-carboxylic acid ethyl ester,
(943) 6-(3-methoxy-phenyl)-2-(5-morpholin-4-yl-2-nitrophenyl)-3-oxo-2,3-dihydro-pyridazine-4-carboxylic acid,
(944) 4-(1,1-dimethyl-propyl)-6-(3-methoxy-phenyl)-2-(5-morpholin-4-yl-2-nitro-phenyl)-2H-pyridazin-3-one,
(945) 6-(3-methoxy-phenyl)-2-(5-morpholin-4-yl-2-nitrophenyl)-3-oxo-2,3-dihydro-pyridazine-4-carboxylic acid methylamide,
(946) 6-(3-methoxy-phenyl)-2-(5-morpholin-4-yl-2-nitrophenyl)-3-oxo-2,3-dihydro-pyridazine-4-carboxylic acid dimethylamide,
(947) 6-(3-methoxy-phenyl)-4-(1-methyl-2-morpholin-4-yl-ethyl)-2-(5-morpholin-4-yl-2-nitro-phenyl)-2H-pyridazin-3-one,
(948) 4-isopropenyl-6-(3-methoxy-phenyl)-2-(5-morpholin-4-yl-2-nitro-phenyl)-2H-pyridazin-3-one,
(949) 4-acetyl-6-(3-methoxy-phenyl)-2-(5-morpholin-4-yl-2-nitro-phenyl)-2H-pyridazin-3-one,
(950) 4-ethoxy-6-(3-methoxy-phenyl)-2-(5-morpholin-4-yl-2-nitro-phenyl)-2H-pyridazin-3-one,
(951) 6-(3-methoxy-phenyl)-2-(5-morpholin-4-yl-2-nitrophenyl)-4-phenyl-2H-pyridazin-3-one,
(952) 6-(3-methoxy-phenyl)-2-(5-morpholin-4-yl-2-nitrophenyl)-3-oxo-2,3-dihydro-pyridazine-4-carboxylic acid isobutyl ester,
(953) 4-isopropoxy-6-(3-methoxy-phenyl)-2-(5-morpholin-4-yl-2-nitro-phenyl)-2H-pyridazin-3-one,
(954) 4-azepan-1-yl-6-(3-methoxy-phenyl)-2-(5-morpholin-4-yl-2-nitro-phenyl)-2H-pyridazin-3-one,
(955) 4-(3-hydroxy-pyrrolidin-1-yl)-6-(3-methoxyphenyl)-2-(5-morpholin-4-yl-2-nitro-phenyl)-2H-pyridazin-3-one,
(956) 4-diethylamino-6-(3-methoxy-phenyl)-2-(5-morpholin-4-yl-2-nitro-phenyl)-2H-pyridazin-3-one,
(957) 6-(3-methoxy-phenyl)-2-(5-morpholin-4-yl-2-nitrophenyl)-4-piperidin-1-yl-2H-pyridazin-3-one,
(958) 3-{[6-(3-methoxy-phenyl)-2-(5-morpholin-4-yl-2-nitro-phenyl)-3-oxo-2,3-dihydro-pyridazin-4-yl]-methylamino}-propionic acid methyl ester,
(959) 4-tert-butylamino-6-(3-methoxy-phenyl)-2-(5-morpholin-4-yl-2-nitro-phenyl)-2H-pyridazin-3-one,
(960) 4-(tert-butyl-methyl-amino)-6-(3-methoxy-phenyl)-2-(5-morpholin-4-yl-2-nitro-phenyl)-2H-pyridazin-3-one,
(961) {[6-(3-methoxy-phenyl)-2-(5-morpholin-4-yl-2-nitro-phenyl)-3-oxo-2,3-dihydro-pyridazin-4-yl]-methylamino}-acetic acid methyl ester,
(962) {[6-(3-methoxy-phenyl)-2-(5-morpholin-4-yl-2-nitro-phenyl)-3-oxo-2,3-dihydro-pyridazin-4-yl]-methylamino}-acetic acid,
(963) 4-(ethyl-methyl-amino)-6-(3-methoxy-phenyl)-2-(3-morpholin-4-yl-phenyl)-2H-pyridazin-3-one,
(964) 6-(3-methoxy-phenyl)-4-methylamino-2-(2-nitro-5-piperidin-1-yl-phenyl)-2H-pyridazin-3-one hydrochloride,
(965) 2-(2-amino-5-piperidin-1-yl-phenyl)-4-(ethylmethyl-amino)-6-(3-methoxy-phenyl)-2H-pyridazin-3-one,
(966) 4-(ethyl-methyl-amino)-6-(3-methoxy-phenyl)-2-(3-piperidin-1-yl-phenyl)-2H-pyridazin-3-one,
(967) N-(2-{[6-(3-methoxy-phenyl)-2-(5-morpholin-4-yl-2-nitro-phenyl)-3-oxo-2,3-dihydro-pyridazin-4-yl]-methyl-amino}-ethyl)-acetamide,
(968) 6-(3-methoxy-phenyl)-4-methylamino-2-(2-nitrophenyl)-2H-pyridazin-3-one,
(969) 3-[1-(2-chloro-phenyl)-6-oxo-5-pyrrolidin-1-yl-1,6-dihydro-pyridazin-3-yl]-5-vinyl-benzaldehyde oxime,
(970) 4-(ethyl-methyl-amino)-6-(3-methoxy-phenyl)-2-(4-nitro-phenyl)-2H-pyridazin-3-one,
(971) 4-(ethyl-methyl-amino)-6-(3-methoxy-phenyl)-2-(4-methoxy-phenyl)-2H-pyridazin-3-one,
(972) 2-(4-amino-phenyl)-4-(ethyl-methyl-amino)-6-(3-methoxy-phenyl)-2H-pyridazin-3-one,
(973) 2-(4-ethylamino-phenyl)-4-(ethyl-methyl-amino)-6-(3-methoxy-phenyl)-2H-pyridazin-3-one,
(974) N-{4-[5-(ethyl-methyl-amino)-3-(3-methoxyphenyl)-6-oxo-6H-pyridazin-1-yl]-phenyl}-acetamide,
(975) {4-[5-(ethyl-methyl-amino)-3-(3-methoxy-phenyl)-6-oxo-6H-pyridazin-1-yl]-phenyl}-carbamic acid methyl ester,
(976) 6-(3-bromo-phenyl)-2-(2,6-dichloro-phenyl)-4-diethylamino-2H-pyridazin-3-one,
(977) 6-(3-bromo-phenyl)-2-(2,6-dichloro-phenyl)-4-pyrrolidin-1-yl-2H-pyridazin-3-one,
(978) 2-(2-ethylamino-phenyl)-4-(ethyl-methyl-amino)-6-(3-methoxy-phenyl)-2H-pyridazin-3-one,
(979) 2-(3-dimethylamino-phenyl)-4-(ethyl-methylamino)-6-(3-methoxy-phenyl)-2H-pyridazin-3-one,
(980) 2-(3-diethylamino-phenyl)-4-(ethyl-methyl-amino)-6-(3-methoxy-phenyl)-2H-pyridazin-3-one,
(981) 4-(ethyl-methyl-amino)-6-(3-methoxy-phenyl)-2-(3-pyrrolidin-1-yl-phenyl)-2H-pyridazin-3-one
(982) 2-(2-dimethylamino-phenyl)-4-(ethyl-methylamino)-6-(3-methoxy-phenyl)-2H-pyridazin-3-one,
(983) 2-(3,5-dichloro-phenyl)-4-(ethyl-methyl-amino)-6-(3-methoxy-phenyl)-2H-pyridazin-3-one,
(984) 2-(3-benzyloxy-5-hydroxy-phenyl)-4-(ethyl-methylamino)-6-(3-methoxy-phenyl)-2H-pyridazin-3-one,
(985) 2-(3,5-dimethoxy-phenyl)-4-(ethyl-methyl-amino)-6-(3-methoxy-phenyl)-2H-pyridazin-3-one,
(986) 2-(2,6-dimethoxy-phenyl)-4-(ethyl-methyl-amino)-6-(3-methoxy-phenyl)-2H-pyridazin-3-one,
(987) 2-(3,5-diethoxy-phenyl)-4-(ethyl-methyl-amino)-6-(3-methoxy-phenyl)-2H-pyridazin-3-one,
(988) 2-(2-chloro-6-nitro-3-piperidin-1-yl-phenyl)-4-(ethyl-methyl-amino)-6-(3-methoxy-phenyl)-2H-pyridazin-3-one,
(989) 2-(6-amino-2-chloro-3-piperidin-1-yl-phenyl)-4-(ethyl-methyl-amino)-6-(3-methoxy-phenyl)-2H-pyridazin-3-one,
(990) 2-(2,6-dichloro-3-piperidin-1-yl-phenyl)-4-(ethyl-methyl-amino)-6-(3-methoxy-phenyl)-2H-pyridazin-3-one,
(991) 2-(2-chloro-3-piperidin-1-yl-phenyl)-4-(ethylmethyl-amino)-6-(3-methoxy-phenyl)-2H-pyridazin-3-one,
(992) 2-(6-amino-2-chloro-3-morpholin-4-yl-phenyl)-4-(ethyl-methyl-amino)-6-(3-methoxy-phenyl)-2H-pyridazin-3-one,
(993) 2-(2,6-dichloro-3-morpholin-4-yl-phenyl)-4-(ethyl-methyl-amino)-6-(3-methoxy-phenyl)-2H-pyridazin-3-one,
(994) 2-(2-chloro-3-morpholin-4-yl-phenyl)-4-(ethylmethyl-amino)-6-(3-methoxy-phenyl)-2H-pyridazin-3-one,
(995) 4-(ethyl-methyl-amino)-6-(3-methoxy-phenyl)-2-(2-nitro-5-thiomorpholin-4-yl-phenyl)-2H-pyridazin-3-one,
(996) 4-(ethyl-methyl-amino)-2-[5-(4-hydroxy-piperidin-1-yl)-2-nitro-phenyl]-6-(3-methoxy-phenyl)-2H-pyridazin-3-one,
(997) 4-(ethyl-methyl-amino)-6-(3-methoxy-phenyl)-2-[2-nitro-5-(1-oxo-1λ4-thiomorpholin-4-yl)-phenyl]-2H-pyridazin-3-one,
(998) 4-(ethyl-methyl-amino)-6-(3-methoxy-phenyl)-2-[2-nitro-5-(4-oxo-piperidin-1-yl)-phenyl]-2H-pyridazin-3-one,
(999) 4-(ethyl-methyl-amino)-2-[2-(1-hydroxy-1-methylethyl)-5-morpholin-4-yl-phenyl]-6-(3-methoxy-phenyl)-2H-pyridazin-3-one,
(1000) 4-(ethyl-methyl-amino)-2-(2-isopropenyl-5-morpholin-4-yl-phenyl)-6-(3-methoxy-phenyl)-2H-pyridazin-3-one,
(1001) 4-(ethyl-methyl-amino)-6-(3-methoxy-phenyl)-2-[2-nitro-5-(3-oxo-pyrrolidin-1-yl)-phenyl]-2H-pyridazin-3-one,
(1002) 2-(2-bromo-5-morpholin-4-yl-phenyl)-4-(ethylmethyl-amino)-6-(3-methoxy-phenyl)-2H-pyridazin-3-one,
(1003) 4-{3-[5-(ethyl-methyl-amino)-3-(3-methoxy-phenyl)-6-oxo-6H-pyridazin-1-yl]-4-nitro-phenyl}-morpholin-2-one,
(1004) 4-(ethyl-methyl-amino)-2-(2-isopropyl-5-morpholin-4-yl-phenyl)-6-(3-methoxy-phenyl)-2H-pyridazin-3-one,
(1005) 4-(ethyl-methyl-amino)-6-(3-methoxy-phenyl)-2-(2-nitro-5-thiazol-2-yl-phenyl)-2H-pyridazin-3-one,
(1006) 4-ethylamino-6-(3-methoxy-phenyl)-2-(5-morpholin-4-yl-2-nitro-phenyl)-2H-pyridazin-3-one,
(1007) 4-isopropylamino-6-(3-methoxy-phenyl)-2-(5-morpholin-4-yl-2-nitro-phenyl)-2H-pyridazin-3-one,
(1008) 4-(ethyl-methyl-amino)-6-(3-methoxy-phenyl)-2-[2-nitro-5-(tetrahydro-pyran-4-yl)-phenyl]-2H-pyridazin-3-one,
(1009) 4-(ethyl-methyl-amino)-2-[5-((R)-3-hydroxy-pyrrolidin-1-yl)-2-nitro-phenyl]-6-(3-methoxy-phenyl)-2H-pyridazin-3-one,
(1010) 4-(ethyl-methyl-amino)-2-[5-((S)-3-hydroxy-pyrrolidin-1-yl)-2-nitro-phenyl]-6-(3-methoxy-phenyl)-2H-pyridazin-3-one,
(1011) 4-(ethyl-methyl-amino)-2-{5-[(2-hydroxy-ethyl)-methyl-amino]-2-nitro-phenyl}-6-(3-methoxy-phenyl)-2H-pyridazin-3-one,
(1012) 4-(ethyl-methyl-amino)-2-[5-(2-hydroxy-ethoxy)-2-nitro-phenyl]-6-(3-methoxy-phenyl)-2H-pyridazin-3-one,
(1013) 4-(ethyl-methyl-amino)-2-[5-(2-methoxy-ethoxy)-2-nitro-phenyl]-6-(3-methoxy-phenyl)-2H-pyridazin-3-one,
(1014) 4-(ethyl-methyl-amino)-6-(3-methoxy-phenyl)-2-(2-nitro-5-pyridin-4-yl-phenyl)-2H-pyridazin-3-one,
(1015) 4-(ethyl-methyl-amino)-6-(3-methoxy-phenyl)-2-(2-methylsulfanyl-5-morpholin-4-yl-phenyl)-2H-pyridazin-3-one,
(1016) 4-(ethyl-methyl-amino)-2-(2-methanesulfinyl-5-morpholin-4-yl-phenyl)-6-(3-methoxy-phenyl)-2H-pyridazin-3-one,
(1017) 4-(ethyl-methyl-amino)-2-[2-methanesulfinyl-5-(4-oxy-morpholin-4-yl)-phenyl]-6-(3-methoxy-phenyl)-2H-pyridazin-3-one,
(1018) 4-isopropylamino-6-(3-methoxy-phenyl)-2-[2-nitro-5-(3-oxo-piperazin-1-yl)-phenyl]-2H-pyridazin-3-one,
(1019) 4-ethylamino-6-(3-methoxy-phenyl)-2-[2-nitro-5-(3-oxo-piperazin-1-yl)-phenyl]-2H-pyridazin-3-one,
(1020) 6-(3,5-dibromo-phenyl)-4-ethylamino-2-(5-morpholin-4-yl-2-nitro-phenyl)-2H-pyridazin-3-one,
(1021) 4-ethylamino-6-(3-methoxy-phenyl)-2-(2-nitro-5-pyridin-3-yl-phenyl)-2H-pyridazin-3-one,
(1022) 4-ethylamino-6-(3-methoxy-phenyl)-2-[2-nitro-5-(1-oxy-pyridin-3-yl)-phenyl]-2H-pyridazin-3-one,
(1023) 4-ethylamino-6-(3-methoxy-phenyl)-2-[5-(6-methoxy-pyridin-3-yl)-2-nitro-phenyl]-2H-pyridazin-3-one,
(1024) 4-ethylamino-6-(3-methoxy-phenyl)-2-[2-nitro-5-(6-oxo-1,6-dihydro-pyridin-3-yl)-phenyl]-2H-pyridazin-3-one,
(1025) 6-(3-bromo-5-oxazol-2-yl-phenyl)-4-ethylamino-2-(5-morpholin-4-yl-2-nitro-phenyl)-2H-pyridazin-3-one,
(1026) 4-ethylamino-2-(5-morpholin-4-yl-2-nitrophenyl)-6-(3-oxazol-2-yl-5-vinyl-phenyl)-2H-pyridazin-3-one,
(1027) 3-bromo-5-[5-ethylamino-1-(5-morpholin-4-yl-2-nitro-phenyl)-6-oxo-1,6-dihydro-pyridazin-3-yl]-benzoic acid,
(1028) 3-[5-ethylamino-1-(5-morpholin-4-yl-2-nitrophenyl)-6-oxo-1,6-dihydro-pyridazin-3-yl]-5-vinyl-benzaldehyde oxime,
(1029) 2-[5-(4-acetyl-piperazin-1-yl)-2-trifluoromethyl-phenyl]-4-ethylamino-6-(3-methyl-5-oxazol-5-yl-phenyl)-2H-pyridazin-3-one,
(1030) 2-[5-((S)-3-hydroxy-pyrrolidin-1-yl)-2-trifluoromethyl-phenyl]-6-(3-methyl-5-oxazol-5-yl-phenyl)-4-piperidin-1-yl-2H-pyridazin-3-one,
(1031) 4-ethylamino-6-(3-methyl-5-oxazol-5-yl-phenyl)-2-[5-(2-oxo-pyrrolidin-1-yl)-2-trifluoromethyl-phenyl]-2H-pyridazin-3-one,
(1032) 4-ethylamino-2-(5-furan-3-yl-2-trifluoromethylphenyl)-6-(3-methyl-5-oxazol-5-yl-phenyl)-2H-pyridazin-3-one,
(1033) 4-ethylamino-6-(3-methyl-5-oxazol-5-yl-phenyl)-2-[5-(6-oxo-6H-pyran-3-yl)-2-trifluoromethyl-phenyl]-2H-pyridazin-3-one,
(1034) 4-ethylamino-6-(3-methyl-5-oxazol-5-yl-phenyl)-2-[5-(2-oxo-piperidin-1-yl)-2-trifluoromethyl-phenyl]-2H-pyridazin-3-one,
(1035) 4-ethylamino-6-(3-methyl-5-oxazol-5-yl-phenyl)-2-(5-pyrazin-2-yl-2-trifluoromethyl-phenyl)-2H-pyridazin-3-one,
(1036) 4-ethylamino-2-[5-(4-hydroxy-2-oxo-pyrrolidin-1-yl)-2-trifluoromethyl-phenyl]-6-(3-methyl-5-oxazol-5-yl-phenyl)-2H-pyridazin-3-one,
(1037) 4-ethylamino-6-(3-methyl-5-oxazol-5-yl-phenyl)-2-(5-pyridazin-3-yl-2-trifluoromethyl-phenyl)-2H-pyridazin-3-one,
(1038) 4-ethylamino-2-[5-((S)-3-hydroxy-2-oxopyrrolidin-1-yl)-2-trifluoromethyl-phenyl]-6-(3-methyl-5-oxazol-5-yl-phenyl)-2H-pyridazin-3-one,
(1039) 4-ethylamino-2-(5-isoxazol-5-yl-2-trifluoromethyl-phenyl)-6-(3-methyl-5-oxazol-5-yl-phenyl)-2H-pyridazin-3-one,
(1040) 4-ethylamino-6-(3-methyl-5-oxazol-5-yl-phenyl)-2-(5-oxazol-5-yl-2-trifluoromethyl-phenyl)-2H-pyridazin-3-one,
(1041) 4-ethylamino-6-(3-methyl-5-oxazol-5-yl-phenyl)-2-(5-pyrimidin-4-yl-2-trifluoromethyl-phenyl)-2H-pyridazin-3-one,
(1042) 2-[5-((S)-3-hydroxy-pyrrolidin-1-yl)-2-trifluoromethyl-phenyl]-6-(3-methyl-5-oxazol-5-yl-phenyl)-4-(2-methyl-pyrrolidin-1-yl)-2H-pyridazin-3-one,
(1043) 3-{3-[5-ethylamino-3-(3-methyl-5-oxazol-5-yl-phenyl)-6-oxo-6H-pyridazin-1-yl]-4-trifluoromethylphenyl}-[1,3]oxazinan-2-one,
(1044) 3-[5-ethylamino-6-oxo-1-(5-pyridazin-3-yl-2-trifluoromethyl-phenyl)-1,6-dihydro-pyridazin-3-yl]-5-methyl-benzaldehyde oxime,
(1045) 6-(3-methoxy-phenyl)-2-(5-morpholin-4-yl-2-nitro-phenyl)-2H-pyridazin-3-one,
(1046) 4-ethyl-6-(3-methoxy-phenyl)-2-(5-morpholin-4-yl-2-nitro-phenyl)-2H-pyridazin-3-one,
(1047) 5-ethyl-6-(3-methoxy-phenyl)-2-(5-morpholin-4-yl-2-nitro-phenyl)-2H-pyridazin-3-one,
(1048) 6-(3-methoxy-phenyl)-2-(5-morpholin-4-yl-2-nitro-phenyl)-4-propyl-2H-pyridazin-3-one,
(1049) 4-benzyl-6-(3-methoxy-phenyl)-2-(5-morpholin-4-yl-2-nitro-phenyl)-2H-pyridazin-3-one,
(1050) 4-butyl-6-(3-methoxy-phenyl)-2-(5-morpholin-4-yl-2-nitro-phenyl)-2H-pyridazin-3-one,
(1051) 4-isobutyl-6-(3-methoxy-phenyl)-2-(5-morpholin-4-yl-2-nitro-phenyl)-2H-pyridazin-3-one,
(1052) 6-(3-methoxy-phenyl)-2-(5-morpholin-4-yl-2-nitro-phenyl)-4-phenethyl-2H-pyridazin-3-one,
(1053) 4-ethyl-6-(3-methoxy-phenyl)-5-methyl-2-(5-morpholin-4-yl-2-nitro-phenyl)-2H-pyridazin-3-one,
(1054) 6-(3-methoxy-phenyl)-2-(5-morpholin-4-yl-2-nitro-phenyl)-4-(3-phenyl-propyl)-2H-pyridazin-3-one,
(1055) 4-tert-butyl-6-(3-methoxy-phenyl)-2-(5-morpholin-4-yl-2-nitro-phenyl)-2H-pyridazin-3-one, .
(1056) 4-(1-ethyl-propyl)-6-(3-methoxy-phenyl)-2-(5-morpholin-4-yl-2-nitro-phenyl)-2H-pyridazin-3-one,
(1057) 2-(2-amino-5-morpholin-4-yl-phenyl)-4-isopropyl-6-(3-methoxy-phenyl)-2H-pyridazin-3-one,
(1058) 4-(2-methoxy-ethoxy)-6-(3-methoxy-phenyl)-2-(5-morpholin-4-yl-2-nitro-phenyl)-2H-pyridazin-3-one,
(1059) 4-butoxy-6-(3-methoxy-phenyl)-2-(5-morpholin-4-yl-2-nitro-phenyl)-2H-pyridazin-3-one,
(1060) 2-[5-((S)-3-hydroxy-pyrrolidin-1-yl)-2-trifluoromethyl-phenyl]-4-isopropyl-6-(3-methyl-5-oxazol-5-yl-phenyl)-2H-pyridazin-3-one,
(1061) 2-[5-((S)-3-hydroxy-pyrrolidin-1-yl)-2-trifluoromethyl-phenyl]-6-(3-methyl-5-oxazol-5-yl-phenyl)-4-propyl-2H-pyridazin-3-one,
(1062) 4-ethoxy-2-[5-((S)-3-hydroxy-pyrrolidin-1-yl)-2-trifluoromethyl-phenyl]-6-(3-methyl-5-oxazol-5-yl-phenyl)-2H-pyridazin-3-one,
(1063) 6-(3,5-dimethoxy-phenyl)-4-dimethylamino-2-[5-((S)-3-hydroxy-pyrrolidin-1-yl)-2-trifluoromethylphenyl]-5-methyl-2H-pyridazin-3-one,
(1064) 6-(3,5-dimethoxy-phenyl)-2-[5-((S)-3-hydroxy-pyrrolidin-1-yl)-2-trifluoromethyl-phenyl]-5-methyl-4-methylamino-2H-pyridazin-3-one,
(1065) 6-(3,5-dimethoxy-phenyl)-2-[5-((S)-3-hydroxy-pyrrolidin-1-yl)-2-trifluoromethyl-phenyl]-4,5-dimethyl-2H-pyridazin-3-one,
(1066-1) 4-(3,5-dimethoxy-phenyl)-2-(5-nitro-2-trifluoromethyl-phenyl)-2,5,6,7-tetrahydro-cyclopenta[c]pyridin-1-one,
(1066-2) 4-(3,5-dimethoxy-phenyl)-2-(5-iodo-2-trifluoromethyl-phenyl)-2,5,6,7-tetrahydro-cyclopenta[c]pyridin-1-one,
(1066-3) 4-(3,5-dimethoxy-phenyl)-2-[5-((S)-3-hydroxy-pyrrolidin-1-yl)-2-trifluoromethyl-phenyl]-2,5,6,7-tetrahydro-cyclopenta[c]pyridin-1-one,
(1066-4) 1-benzoyl-6-(5-bromo-2-trifluoromethylphenyl)-4-(3,5-dimethoxy-phenyl)-1,2,3,6-tetrahydro-pyrrolo[2,3-c]pyridin-7-one,
(1066-5) 4-(3,5-dimethoxy-phenyl)-6-[5-(3-hydroxymethyl-azetidin-1-yl)-2-trifluoromethyl-phenyl]-7-oxo-2,3,6,7-tetrahydro-pyrrolo[2,3-c]pyridine-1-carboxylic acid methyl ester,
(1066-6) 6-(5-bromo-2-trifluoromethyl-phenyl)-4-(3,5-dimethoxy-phenyl)-1,2,3,6-tetrahydro-pyrrolo[2,3-c]pyridin-7-one,
(1066-7) 6-(5-bromo-2-trifluoromethyl-phenyl)-4-(3,5-dimethoxy-phenyl)-1,6-tetrahydro-pyrrolo[2,3-c]pyridin-7-one,
(1066-8) 6-(5-bromo-2-trifluoromethyl-phenyl)-4-(3,5-dimethoxy-phenyl)-1-(2-hydroxy-ethyl)-1,6-dihydro-pyrrolo[2,3-c]pyridin-7-one,
(1066-9) 4-(3,5-dimethoxy-phenyl)-1-(2-hydroxy-ethyl)-6-[5-((S)-3-hydroxy-pyrrolidin-1-yl)-2-trifluoromethylphenyl]-1,6-dihydro-pyrrolo[2,3-c]pyridin-7-one,
(1068) 3-ethylamino-1-[5-((S)-3-hydroxy-pyrrolidin-1-yl)-2-trifluoromethyl-phenyl]-5-(3-methyl-5-oxazol-5-yl-phenyl)-1H-pyridin-2-one,
(1069) 4-(3,5-Dimethoxy-phenyl)-2-[5-((S)-3-hydroxy-pyrrolidin-1-yl)-2-trifluoromethyl-phenyl]-2,5,6,7-tetrahydro-cyclopenta[c]pyridin-1-one,
(1070) N-((R)-1-{3-[4-(3,5-dimethoxy-phenyl)-7-oxo-1,2,3,7-tetrahydro-pyrrolo[2,3-c]pyridin-6-yl]-4-trifluoromethyl-phenyl}-pyrrolidin-3-yl)-acetamide,
(1071) 4-(3,5-dimethoxy-phenyl)-6-{5-[(S)-3-(2-hydroxyethoxy)-pyrrolidin-1-yl]-2-trifluoromethyl-phenyl}-7-oxo-2,3,6,7-tetrahydro-pyrrolo[2,3-c]pyridine-1-carboxylic acid amide,
(1072) N-((R)-1-{3-[1-(2-hydroxy-ethyl)-4-(3-methoxyphenyl)-7-oxo-1,2,3,7-tetrahydro-pyrrolo[2,3-c]pyridin-6-yl]-4-trifluoromethyl-phenyl}-pyrrolidin-3-yl)-acetamide,
(1073) 4-(3,5-dimethoxy-phenyl)-1-(2-hydroxy-ethyl)-6-[5-(3-hydroxymethyl-azetidin-1-yl)-2-trifluoromethylphenyl]-1,6-dihydro-pyrrolo[2,3-c]pyridin-7-one,
(1074) 4-(3,5-dimethoxy-phenyl)-6-{5-[(S)-3-(2-hydroxyethoxy)-pyrrolidin-1-yl]-2-trifluoromethyl-phenyl}-1-(2-hydroxy-ethyl)-1,6-dihydro-pyrrolo[2,3-c]pyridin-7-one,
(1075) 4-(3,5-dimethoxy-phenyl)-6-[5-(3-hydroxymethyl-azetidin-1-yl)-2-trifluoromethyl-phenyl]-7-oxo-2,3,6,7-tetrahydro-pyrrolo[2,3-c]pyridine-1-carboxylic acid amide,
(1076) N-((R)-1-{3-[4-(3,5-dimethoxy-phenyl)-1-(2-hydroxy-ethyl)-7-oxo-1,2,3,7-tetrahydro-pyrrolo[2,3-c]pyridin-6-yl]-4-trifluoromethyl-phenyl}-pyrrolidin-3-yl)-acetamide,
(1077) 4-(3,5-dimethoxy-phenyl)-1-(2-hydroxy-ethyl)-6-[5-(3-hydroxymethyl-azetidin-1-yl)-2-trifluoromethylphenyl]-1,2,3,6-tetrahydro-pyrrolo[2,3-c]pyridin-7-one,
(1078) 2-{4-(3,5-dimethoxy-phenyl)-6-[5-(3-hydroxymethyl-azetidin-1-yl)-2-trifluoromethyl-phenyl]-7-oxo-2,3,6,7-tetrahydro-pyrrolo[2,3-c]pyridin-1-yl}-acetamide,
(1079) 6-[5-(3-hydroxymethyl-azetidin-1-yl)-2-trifluoromethyl-phenyl]-4-(3-methoxy-phenyl)-7-oxo-2,3,6,7-tetrahydro-pyrrolo[2,3-c]pyridine-1-carboxylic acid amide,
(1080) N-((R)-1-{3-[4-(3,5-Dimethoxy-phenyl)-1-oxo-1,5,6,7-tetrahydro-cyclopenta[c]pyridine-2-yl]-4-trifluoromethyl-phenyl}-pyrrolidin-3-yl)-acetamide,
(1081) 2-((S)-1-{3-[4-(3,5-Dimethoxy-phenyl)-1-oxo-1,5,6,7-tetrahydro-cyclopenta[c]pyridine-2-yl]-4-trifluoromethyl-phenyl}-pyrrolidin-3-yl)-N-methyl-acetamide,
(1082) 2-((R)-1-{3-[4-(3,5-Dimethoxy-phenyl)-1-oxo-1,5,6,7-tetrahydro-cyclopenta[c]pyridine-2-yl]-4-trifluoromethyl-phenyl}-pyrrolidin-3-yl)-N-methyl-acetamide,
(1083) 2-[6-[5-(3-hydroxymethyl-azetidin-1-yl)-2-trifluoromethyl-phenyl]-4-(3-methoxy-phenyl)-7-oxo-2,3,6,7-tetrahydro-pyrrolo[2,3-c]pyridin-1-yl]-acetamide,
(1084) 4-(3,5-dimethoxy-phenyl)-6-[5-((S)-3-hydroxy-pyrrolidin-1-yl)-2-trifluoromethyl-phenyl]-1,6-dihydro-pyrrolo[2,3-c]pyridin-7-one,
(1085-1) 8-(3,5-dimethoxy-phenyl)-6-(5-fluoro-2-trifluoromethyl-phenyl)-2,3-dihydro-1H-indolizin-5-one,
(1085-2) 8-(3,5-dimethoxy-phenyl)-6-[5-((S)-3-hydroxy-pyrrolidin-1-yl)-2-trifluoromethyl-phenyl]-2,3-dihydro-1H-indolizin-5-one,
(1086) N-((R)-1-{3-[8-(3,5-dimethoxy-phenyl)-5-oxo-1,2,3,5-tetrahydro-indolizin-6-yl]-4-trifluoromethylphenyl}-pyrrolidin-3-yl)-acetamide,
(1087) 2-((R)-1-{3-[8-(3,5-dimethoxy-phenyl)-5-oxo-1,2,3,5-tetrahydro-indolizin-6-yl]-4-trifluoromethylphenyl}-pyrrolidin-3-yl)-N-methyl-acetamide,
(1088) 2-((S)-1-{3-[8-(3,5-dimethoxy-phenyl)-5-oxo-1,2,3,5-tetrahydro-indolizin-6-yl]-4-trifluoromethylphenyl}-pyrrolidin-3-yl)-N-methyl-acetamide,
(1089) 8-(3,5-dimethoxy-phenyl)-6-{5-[(S)-3-(2-hydroxyethoxy)-pyrrolidin-1-yl]-2-trifluoromethyl-phenyl}-2,3-dihydro-1H-indolizin-5-one,
(1090-1) 1-(3,5-dimethoxy-phenyl)-3-(5-fluoro-2-trifluoromethyl-phenyl)-1,5,6,7-tetrahydro-cyclopenta[b]pyridin-4-one,
(1090-2) 1-(3,5-Dimethoxy-phenyl)-3-[5-((S)-3-hydroxy-pyrrolidin-1-yl)-2-trifluoromethyl-phenyl]-1,5,6,7-tetrahydro-cyclopenta[b]pyridin-4-one,
(1091-1) 4-(3,5-dimethoxy-phenyl)-2-(5-nitro-2-trifluoromethyl-phenyl)-2,5,6,7-tetrahydro-cyclopenta[c]pyridin-3-one,
(1091-2) 2-(5-amino-2-trifluoromethyl-phenyl)-4-(3,5-dimethoxy-phenyl)-2,5,6,7-tetrahydro-cyclopenta[c]pyridin-3-one,
(1091-3) 2-(5-bromo-2-trifluoromethyl-phenyl)-4-(3,5-dimethoxy-phenyl)-2,5,6,7-tetrahydro-cyclopenta[c]pyridin-3-one,
(1091-4) 4-(3,5-Dimethoxy-phenyl)-2-[5-((S)-3-hydroxy-pyrrolidin-1-yl)-2-trifluoromethyl-phenyl]-2,5,6,7-tetrahydro-cyclopenta[c]pyridine-3-one,
(1092-1) 4-(3,5-dimethoxy-phenyl)-2-(5-fluoro-2-trifluoromethyl-phenyl)-6,7-dihydro-5H-cyclopenta[b]pyridine,
(1092-2) (S)-1-{3-[4-(3,5-Dimethoxy-phenyl)-6,7-dihydro-5H-cyclopenta[b]pyridine-2-yl]-4-trifluoromethyl-phenyl}-pyrrolidin-3-ol,
(1093-1) 1-(3,5-dimethoxy-phenyl)-3-(5-fluoro-2-trifluoromethyl-phenyl)-6,7-dihydro-5H-cyclopenta[c]pyridine,
(1093-2) (S)-1-{3-[1-(3,5-dimethoxy-phenyl)-6,7-dihydro-5H-cyclopenta[c]pyridine-3-yl]-4-trifluoromethyl-phenyl}-pyrrolidin-3-ol,
(1094-1) (S)-1-{3-[4-(3,5-dimethoxy-phenyl)-6,7-dihydro-5H-cyclopentapyrimidin-2-yl]-4-trifluoromethylphenyl}-pyrrolidin-3-yl acetate,
(1094-2) (S)-1-{3-[4-(3,5-dimethoxy-phenyl)-6,7-dihydro-5H-cyclopentapyrimidin-2-yl]-4-trifluoromethylphenyl}-pyrrolidin-3-ol,
(1095-1) (S)-1-{3-[7-(3,5-dimethoxy-phenyl)-indan-5-yl]-4-trifluoromethyl-phenyl}-pyrrolidin-3-ol,
(1095-2) (S)-1-{3-[7-(3,5-dimethoxy-phenyl)-4-hydroxy-indan-5-yl]-4-trifluoromethyl-phenyl}-pyrrolidin-3-ol, and
(1096) (S)-1-{3-[7-(3,5-dimethoxy-phenyl)-4-methoxy-indan-5-yl]-4-trifluoromethyl-phenyl}-pyrrolidin-3-ol.

Of the aforementioned compounds, those obtained in the following Examples are specifically preferable: Examples 1-4, 1-6, 1-7, 1-9, 1-10, 1-11, 1-12, 1-13, 1-15, 1-17, 1-18, 1-19, 1-23, 1-27, 1-29, 1-33, 1-35, 1-37, 1-38, 1-39, 1-40, 1-41, 1-42, 1-43, 1-44, 1-45, 1-46, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198,
199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402-1, 402-3, 402-4, 402-5, 402-6, 402-7, 402-8, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 490, 491, 492, 493, 494, 495, 496, 497, 498, 499, 500, 502, 503, 504, 505, 506, 507, 508, 509, 510, 512, 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568,
569, 570, 571, 572, 573, 575, 576, 577, 578, 579, 580, 583, 584, 585, 586, 587, 588, 589, 590, 591, 592, 593, 594, 595, 596, 597; 598, 599, 600, 601, 602, 603, 604, 605, 606, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 650, 655, 659, 662, 663, 664, 665, 666, 668, 669, 670, 671, 672, 673, 674, 675, 677, 678, 679, 680, 681, 682, 683, 684, 685, 686, 688, 689, 690, 691, 692, 693, 694, 696, 697, 698, 699, 700, 701, 703, 704, 705, 706, 707, 708, 709, 710, 712, 713, 714, 715, 716, 717, 718, 719, 720, 721, 722, 723, 725, 728, 729, 733, 743, 744, 746, 747, 748, 750, 751, 752, 754, 755, 756, 757, 758, 759, 760, 761, 762, 763, 764, 769, 777, 779, 782, 783, 784, 785, 786, 787, 788, 789, 790, 791, 792, 794, 795, 796, 797, 798, 799, 800, 801, 802, 803, 804, 805, 806, 807, 808, 809, 810, 811, 812, 813, 814, 815, 816, 817, 818, 819, 820, 821, 822, 823, 824, 825, 826, 827, 828, 829, 830, 831, 833, 834, 836-1, 836-3, 836-4, 836-9, 836-12, 836-16, 837, 838, 839, 840, 841, 842, 843, 844, 845, 846, 847, 848, 849, 850, 851, 852, 853, 854, 855, 856, 857, 858, 859, 860, 861, 862, 863, 864, 865, 866, 867, 868, 869, 870, 871, 872, 873, 874, 875, 876, 877, 878, 879, 880, 881, 882, 883, 884, 885, 886, 887, 888, 889, 890, 891, 892, 893, 894, 895, 896, 897, 898, 899, 900, 901, 902, 903, 904, 905, 906, 907, 908, 909, 910, 911, 912, 913, 914, 915, 916, 917, 918, 919, 920, 921, 922, 923, 924, 925, 926, 927, 928, 929, 930, 931, 932, 933, 934, 935, 936, 937, 938, 939, 940, 941, 942, 943, 944, 945, 946, 947, 948, 949, 950, 951, 952, 953, 954, 955, 956, 957, 958, 959, 960, 961, 962, 963, 964, 965, 966, 967, 968, 969, 970, 971, 972, 973, 974, 975, 976, 977, 978, 979, 980, 981, 982, 983, 984, 985, 986, 987, 988, 989, 990, 991, 992, 993,
994, 995, 996, 997, 998, 999, 1000, 1001, 1002, 1003, 1004, 1005, 1006, 1007, 1008, 1009, 1010, 1011, 1012, 1013, 1014, 1015, 1016, 1017, 1018, 1019, 1020, 1021, 1022, 1023, 1024, 1025, 1026, 1027, 1028, 1029, 1030, 1031, 1032, 1033, 1034, 1035, 1036, 1037, 1038, 1039, 1040, 1041, 1042, 1043, 1044, 1045, 1046, 1047, 1048, 1049, 1050, 1051, 1052, 1053, 1054, 1055, 1056, 1057, 1058, 1059, 1060, 1061, 1062, 1063, 1064, 1065, 1066-5, 1066-9, 1068, 1069, 1070, 1071, 1072, 1073, 1074, 1075, 1076, 1077, 1078, 1079, 1080, 1081, 1082, 1083, 1084, 1085-2, 1086, 1087, 1088, 1089, 1090-2, 1091-4, 1092-2, 1093-2, 1094-2, 1095-1, 1095-2 and 1096.

The compounds obtained in the following Examples are also preferable:
Examples 487, 488, 489, 501, 511, 574, 581, 582, 607, 608, 646, 647, 648, 649, 651, 652, 653, 654, 656, 657, 658, 660, 661, 667, 676, 687, 695, 702, 711, 724, 726, 727, 730, 731, 732, 734, 735, 736, 737, 738, 739, 740, 741, 742, 745, 749, 753, 765, 766, 767, 768, 770, 771, 772, 773, 774, 775, 776, 778, 780, 781, 832 and 835.

While the production methods of the compound of the present invention are explained in the following, it is needless to say that the production methods of the compound of the present invention are not limited thereto.

Even in the absence of description in the production method, efficient production can be afforded, where necessary, by introducing a protecting group into a functional group followed by deprotection in a subsequent step, using a functional group as a precursor in each step, and converting the precursor to a desired functional group at a suitable stage, exchanging the order of respective steps and the like.

The workup after reaction in each step can be applied by a typical method, wherein isolation and purification is performed by selecting or combining conventional methods as necessary, such as crystallization, recrystallization, silica gel chromatography, preparative HPLC and the like.

The abbreviations mean the following. *n*-Bu shows a butyl group, and OTf shows a trifluoromethanesulfonyloxy group.

While the production methods of the compound of the present invention are explained in the following, it is needless to say that the production methods of the compound of the present invention are not limited thereto.

The starting materials and intermediates in these production methods are commercially available or can be produced by a known art. Even in the absence of description in the production method, efficient production can be afforded, where necessary, by introducing a protecting group into a functional group followed by deprotection in a subsequent step, using a functional group as a precursor in each step, and converting the precursor to a desired functional group at a suitable stage, exchanging the order of production methods and respective steps and the like.

The workup after reaction in each step can be applied by a typical method, wherein isolation and purification is performed by selecting or combining conventional methods as necessary, such as crystallization, recrystallization, evaporation, column chromatography, thin layer chromatography, preparative HPLC and the like.

The abbreviations mean the following. *n*-Bu shows a butyl group, and OTf shows a trifluoromethanesulfonyloxy group.

### Production method 1

Of the compounds represented by the formula [1], the production method shown here is suitable for the synthesis of compound [1-1] wherein the center ring has the following structure.

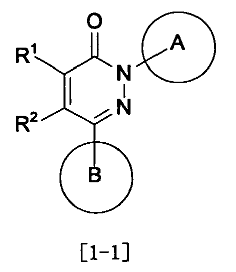

### Production method 1-1

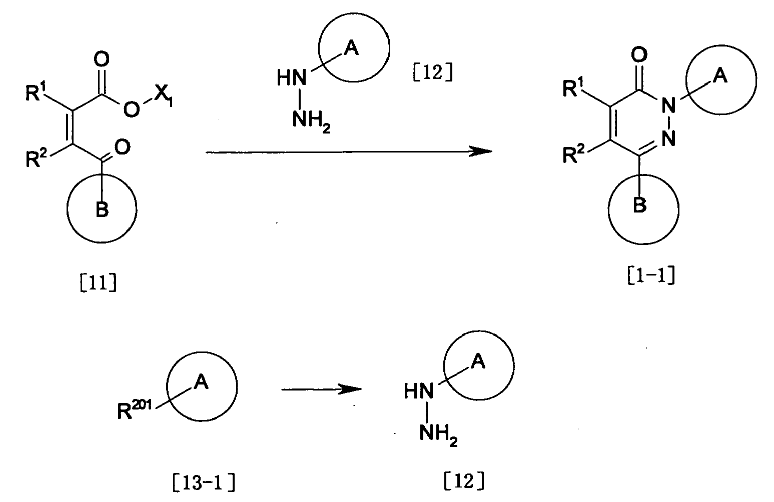

wherein X₁ is a C₁₋₆ alkyl group, R²⁰¹ is a fluorine atom, a chlorine atom, a bromine atom, an iodine atom or an NH₂ group, and other symbols are as defined in the aforementioned formula [1].

The corresponding compound [1-1] can be obtained by heating compound [11] and compound [12] in the presence or absence of an acid in an alcohol solvent such as ethanol, 1-propanol, 1-butanol and the like. As the acid to be used, hydrochloric acid, methanesulfonic acid, trifluoroacetic acid, acetic acid and the like can be mentioned. While the reaction proceeds in two steps of formation of hydrazone and subsequent ring-closing reaction, good results are often obtained by adding, after confirmation of formation of hydrazone, a base such as sodium carbonate, cesium carbonate, triethylamine, diazabicycloundecene and the like and heating the mixture again.

Compound [12] can be synthesized by heating compound [13-1] wherein R²⁰¹ is a fluorine atom or chlorine atom with hydrazine hydrate in the presence of potassium carbonate and the like in an alcohol solvent such as ethanol, 1-propanol and the like, or an amide solvent such as N,N-dimethylformamide, N,N-dimethylacetamide, 1,3-dimethyl-2-imidazolidone and the like. Compound [12] can also be synthesized by reacting compound [13-1] wherein R²⁰¹ is a bromine atom or an iodine atom with di-tert-butyl azodicarboxylate in the presence of a metal reagent such as n-butyllithium, magnesium and the like in a solvent such as tetrahydrofuran and the like, and treating the *tert-*butoxycarbonyl group with an acid such as trifluoroacetic acid, hydrogen chloride-dioxane solution and the like according to a conventional method. In addition, compound [12] can also be synthesized by converting compound [13-1] wherein R²⁰¹ is NH₂ to a diazonium salt using sodium nitrite and the like in an aqueous solution containing an acid such as hydrochloric acid, sulfuric acid, trifluoroacetic acid and the like, and reducing the compound with tin chloride (II).

### Production method 1-2

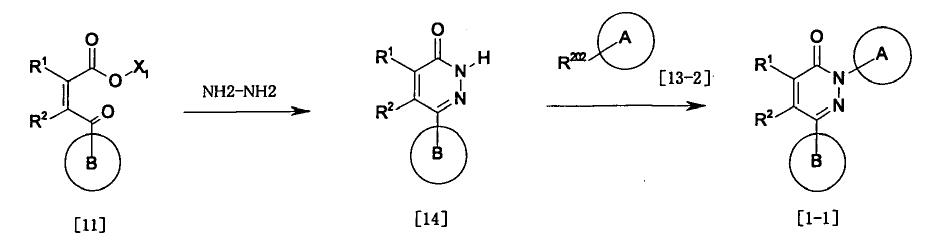

wherein R²⁰² is a fluorine atom or a chlorine atom, and other symbols are each as defined in the formula [1] and/or the aforementioned production method.

The corresponding compound [1-1] can also be obtained by reacting compound [11] with hydrazine hydrate by a method according to production method 1-1 or a method analogous thereto to give compound [14], and heating the compound with compound [13-2] under basic conditions. As the base to be used, inorganic bases such as potassium carbonate, cesium carbonate and the like can be mentioned. As the solvent to be used, amide solvents such as N,N-dimethylformamide, N,N-dimethylacetamide, 1,3-dimethyl-2-imidazolidone and the like can be mentioned.

### Production method 1-3a

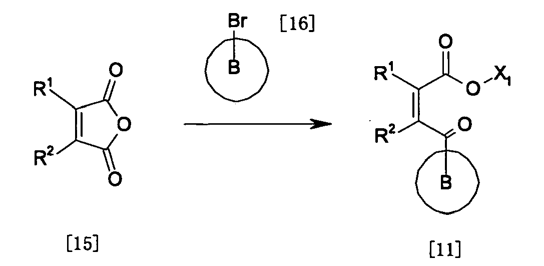

wherein the symbols in the formula are each as defined in the formula [1] and/or the aforementioned production methods.

Compound [11] can be synthesized by reacting with an active species obtained from compound [16] and a metal reagent such as *n*-butyllithium, magnesium and the like and compound [15] in a solvent such as tetrahydrofuran and the like from under cooling to room temperature.

### Production method 1-3b

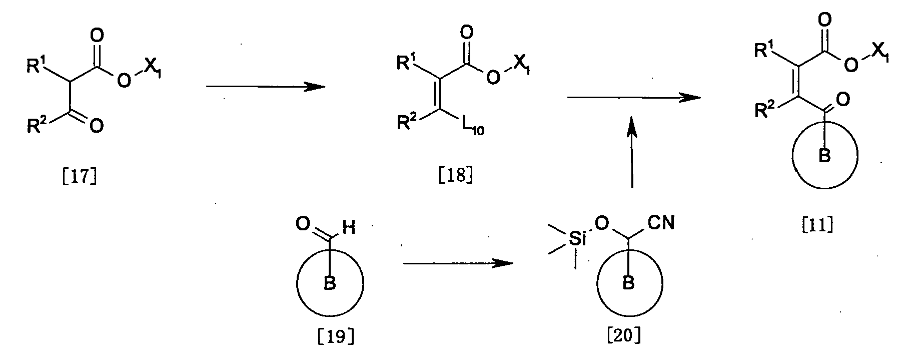

wherein L₁₀ is a chlorine atom or an OTf group, and other symbols are each as defined in the formula [1] and/or the aforementioned production methods.

Compound [20] can be synthesized by reacting compound [19] with trimethylsilyl cyanide under a triethylamine catalyst in chloroform. Compound [11] can be obtained by reacting the obtained compound [20] with an organic metal reagent such as lithium diisopropylamide, lithium bis(trimethylsilyl)amide and the like, and then reacting the compound with compound [18] in tetrahydrofuran from under cooling to room temperature. Compound [18] can be synthesized by reacting compound [17] with a chlorination reagent such as phosphorous pentachloride, or a sulfonylation reagent such as trifluorometanesulfonic anhydride and the like.

### Production method 1-3c

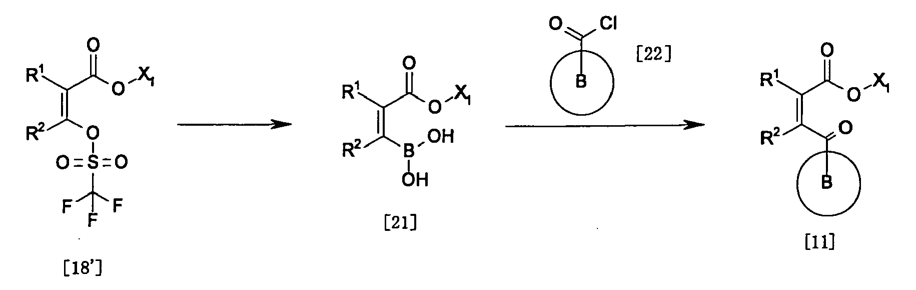

wherein the symbols in the formula are each as defined in the formula [1] and/or the aforementioned production methods.

Compound [21] can be synthesized by reacting compound [18'] prepared by the method described in Production method 1-3b with bis(pinacolate)diborane in the presence of a palladium catalyst such as bis(triphenylphosphine)palladium(II) dichloride and the like and a base such as potassium carbonate and the like in a solvent such as dioxane and the like under heating, and then hydrolyzing in the presence of sodium periodate in a mixed solvent of acetone and water. The corresponding compound [11] can be obtained by subjecting the obtained compound [21] and compound [22] to a coupling reaction as mentioned above using palladium as a catalyst.

### Production method 1-3d

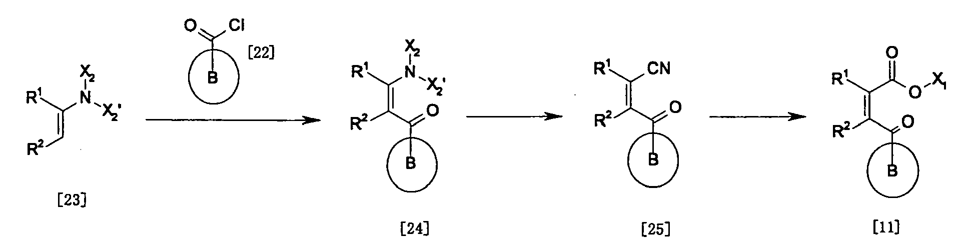

wherein X₂ and X₂' are each a C₁₋₆ alkyl group or form, together with the nitrogen atom bonded thereto, a nitrogen-containing 4- to 7-membered heterocyclic group, and other symbols are as defined in the formula [1] and/or the aforementioned Production methods.

Compound [24] can be synthesized by reacting compound [23] with compound [22] in the presence of triethylamine in chloroform. Compound [25] can be obtained by reacting the obtained compound [24] with trimethylsilyl cyanide, and treating the compound with an acid such as trifluoroacetic acid and the like. Thereafter, the corresponding compound [11] can be obtained by heating the compound [25] in the presence of an acid such as sulfuric acid, hydrochloric acid and the like in an alcohol solvent such as methanol, ethanol, 1-butanol and the like.

### Production method 1-3e

Of the compounds represented by the formula [1], the method shown here is suitable for the synthesis of compound [11-1] wherein particularly R¹ is -N(R¹⁰²)-R¹⁰¹ wherein R¹, R¹⁰¹ and R¹⁰² are as defined for the formula [1], or compound [11-2], wherein R¹ and R² in combination form 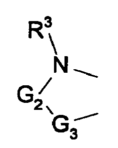 wherein R³, G₂ and G₃ are as defined for the formula [1]. 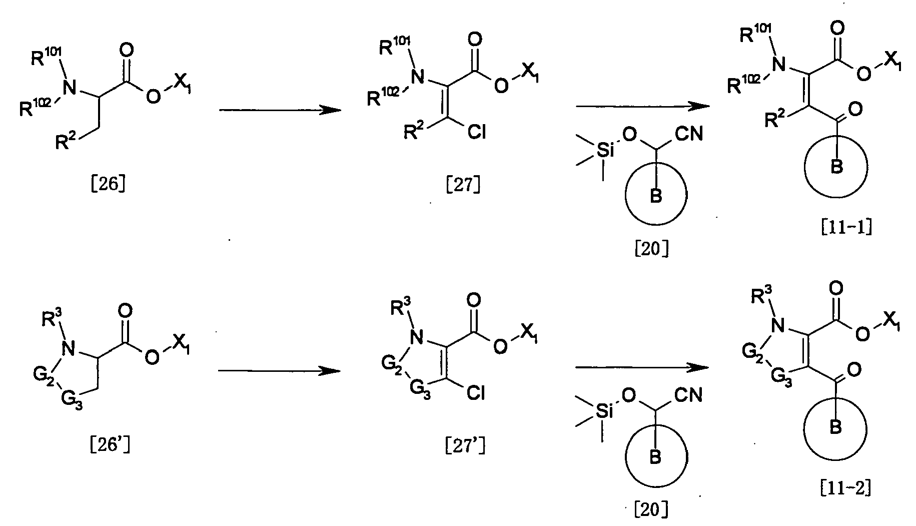 wherein the symbols in the formula are each as defined in the formula [1] and/or the aforementioned Production methods.

Compound [11-1] can be obtained by reacting a chlorination agent such as N-chlorosuccinimide, tert-butyl hypochlorite and the like with compound [26] in the presence of triethylamine in a solvent such as chloroform and the like from under cooling to room temperature to give compound [27], and reacting the compound with compound [20] by a method similar to Production method 1-3b.

In addition, compound [11-2] can be synthesized from compound [26'] by a method similar to the above-mentioned method.

### Production method 1-3f

Of the compounds represented by the formula [1], the method shown here is suitable for the synthesis of compound [11-3] wherein R¹ and R² (R¹ and R² are as defined for the formula [1]) in combination form the following ring structure. 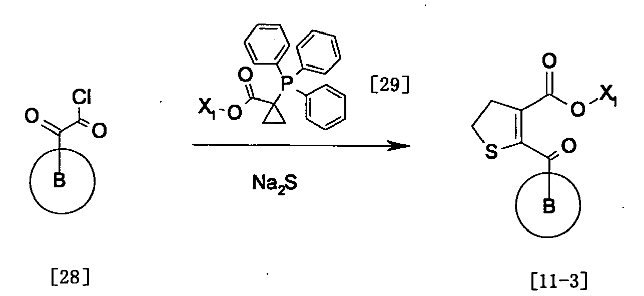 wherein X₁ is as defined in Production method 1-1.

The corresponding compound [11-3] can be obtained by reacting the active species obtained by reacting compound [29] with sodium sulfide in tetrahydrofuran at room temperature with compound [28] in tetrahydrofuran from room temperature to under heating.

### Production method 1-3g

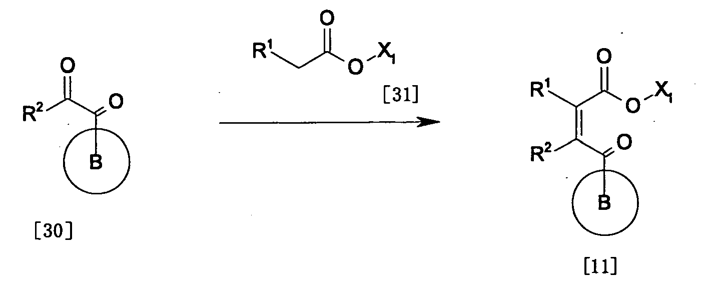

wherein the symbols in the formula are each as defined in the formula [1] and/or the aforementioned Production methods.

The corresponding compound [11] can be obtained by reacting compound [31] with an organic metal reagent such as lithium diisopropylamide, lithium bis(trimethylsilyl)amide and the like, and reacting with compound [30] in tetrahydrofuran from under cooling to room temperature. In addition, a method comprising heating compound [30] and compound [31] in the presence of a base such as sodium hydroxide, potassium hydroxide and the like in an alcohol solvent such as methanol, ethanol and the like and a mixed solvent of them and water sometimes affords good results.

### Production method 1-3h

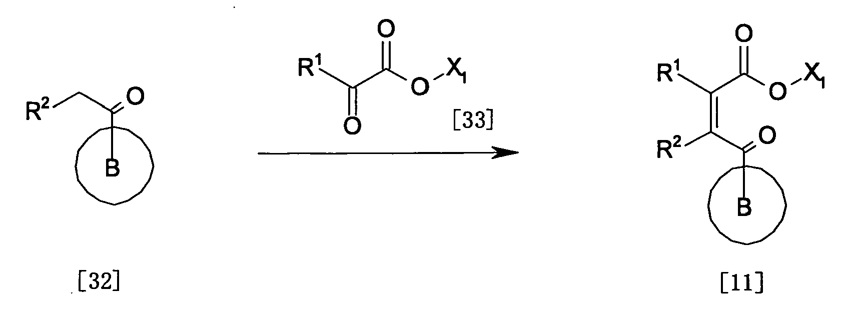

wherein the symbols in the formula are each as defined in the formula [1] and/or the aforementioned Production methods.

Compound [11] can be obtained by reacting compound [32] with compound [33] by a method similar to Production method 1-3g.

### Production method 2

Of the compounds represented by the formula [1], the production method shown here is suitable for the synthesis of compound [1-2] wherein the center ring has the following structure.

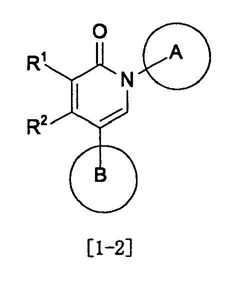

### Production method 2-1

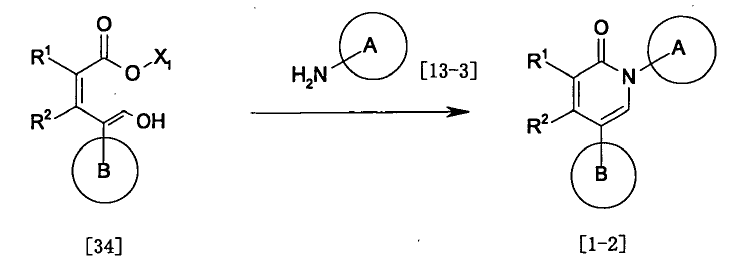

wherein the symbols in the formula are each as defined in the formula [1] and/or the aforementioned Production methods.

Compound [1-2] can be synthesized by reacting compound [34] with compound [13-3] in the presence of an acid such as hydrochloric acid and the like in an alcohol solvent such as ethanol and the like at room temperature or under heating. While the reaction proceeds in two steps of enamine formation and subsequent ring-closing reaction, good results are often afforded by adding, after confirmation of enamine formation, a base such as sodium carbonate, cesium carbonate, triethylamine, diazabicycloundecene and the like and carrying out the reaction again.

### Production method 2-2

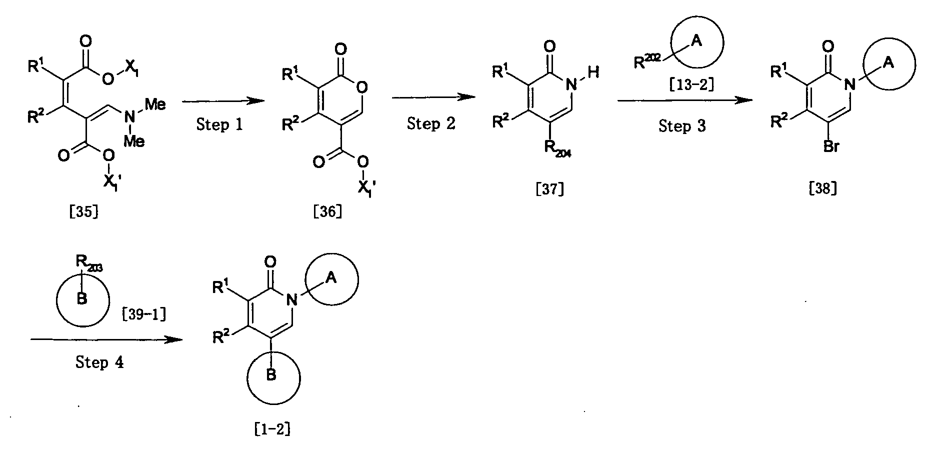

wherein R²⁰³ is B(OH)₂ or B(OX₃)(OX₄) (wherein X₃ and X₄ are the same or different and each is a C₁₋₆ alkyl group, or X₃ and X₄ may form a ring of -CH₂-CH₂- or - CH₂-CH₂-CH₂-), R²⁰⁴ is a hydrogen atom or a bromine atom, X₁' is a C₁₋₆ alkyl group, and other symbols are each as defined in the formula [1] and/or the aforementioned Production methods.

### step 1

Compound [36] can be synthesized by converting the enamine moiety to enol by reacting compound [35] in an aqueous acidic solution such as hydrochloric acid and the like at room temperature, and performing ring-closing in the presence of an acid such as concentrated sulfuric acid and the like in an alcohol solvent such as ethanol and the like.

### step 2

Compound [37] wherein R²⁰⁴ is a bromine atom can be obtained by heating compound [36] with an aqueous ammonia solution in an alcohol solvent such as ethanol and the like to convert the compound to compound [37] wherein R²⁰⁴ is a hydrogen atom, and reacting the compound with a bromination agent such as bromine, N-bromosuccinimide, benzyltrimethylammonium tribromide and the like in a solvent such as chloroform, acetic acid and the like or a mixed solvent thereof at room temperature or under heating.

### step 3

Compound [38] can be obtained by reacting compound [37] wherein R²⁰⁴ is a bromine atom with compound [13-2] by a method according to Production method 1-2 or a method analogous thereto.

### step 4

Compound [1-2] can be obtained from compound [38] and compound [39-1] by a coupling reaction using palladium as a catalyst according to the method described in
a) Handbook of Palladium-Catalysed Organic Reactions: Synthetic Aspects and Catalystic Cycles. Jean-Luc Malleron et al. (1997) Academic Pr.
b) Handbook of Organopalladium Chemistry for Organic Synthesis. Eiichi Negishi et al. (2002) John Wiley & Sons Inc.
and the like.

As the palladium catalyst, a combination of tris (dibenzylideneacetone) dipalladium (0) -chloroformate and tris(o-tolyl)phosphine, and 1,1'-bis(diphenylphosphino)ferrocene palladium(II) dichloride-dichloromethane complex affords preferable results. As the base to be used, inorganic and organic bases such as sodium carbonate, potassium carbonate, triethylamine and the like can be mentioned.

As the solvent, benzene solvents such as toluene, xylene and the like, ether solvents such as dioxane, dimethoxyethane and the like, alcohol solvents such as ethanol, *tert*-butanol and the like, amide solvents such as N,N-dimethylformamide and the like, and mixed solvents thereof can be mentioned.

### Production method 2-3a

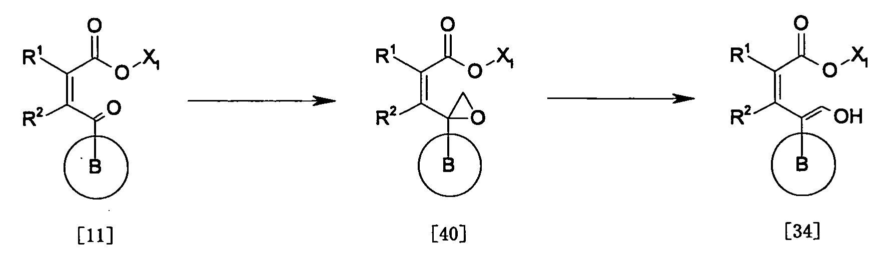

wherein the symbols in the formula are each as defined in the formula [1] and/or the aforementioned Production methods.

Compound [34] can be synthesized by reacting compound [11] that can be synthesized by the method described in Production method 1-3 with trimethylsulfonium iodide in the presence of a base sodium hydride and the like in dimethyl sulfoxide to convert the compound to compound [40], and opening the oxirane ring by reacting the compound in the presence of an acid such as hydrochloric acid and the like in a solvent such as ethyl acetate, dioxane and the like at room temperature.

### Production method 2-3b

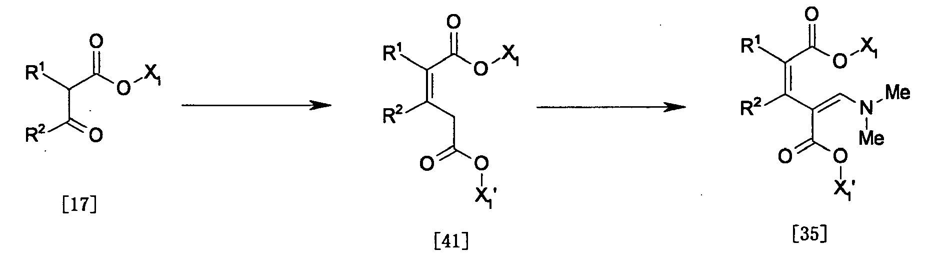

wherein the symbols in the formula are each as defined in the formula [1] and/or the aforementioned Production methods.

Compound [35] can be synthesized by reacting compound [17] with a Honor-Emmons reagent according to a conventional method to convert the compound to compound [41], and heating the compound with N,N-dimethylformamide dimethylacetal and N,N-dimethylformamide.

### Production method 3

Of the compounds represented by the formula [1], the production method shown here is suitable for the synthesis of compound [1-3] wherein the center ring has the following structure.

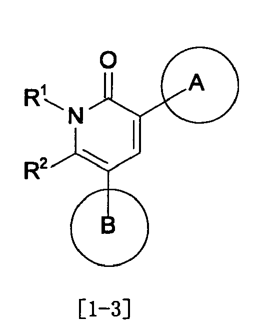

### Production method 3-1

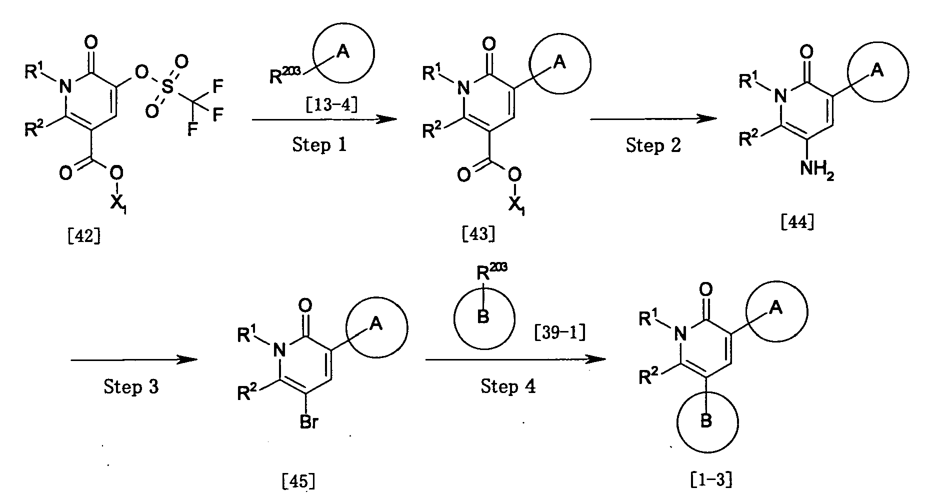

wherein the symbols in the formula are each as defined in the formula [1] and/or the aforementioned Production methods.

### step 1

Compound [43] can be obtained by reacting compound [42] that can be synthesized by the method described in Journal of Organic Chemistry, 1999, 64, 8648 or a method analogous thereto, with compound [13-4] according to the method described in Production method 2-2, step 4.

### step 2

Compound [44] can be obtained by hydrolyzing compound [43] with a base such as aqueous sodium hydroxide solution and the like, and heating the compound with diphenylphosphoryl azide in the presence of a base such as triethylamine and the like in a solvent such as tetrahydrofuran, toluene and the like.

### step 3

Compound [45] can be obtained by converting compound [44] using sodium nitrite and the like in an aqueous solution containing an acid such as hydrochloric acid, sulfuric acid, trifluoroacetic acid and the like to give a diazonium salt, reducing the diazonium salt with an aqueous phosphorous acid solution to give a deaminated derivative, and then bromating the deaminated derivative by a method according to Production method 2-2, step 2, or a method analogous thereto.

### step 4

Compound [1-3] can be obtained by reacting compound [45] with compound [39-1] by a method according to Production method 2-2, step 4 or a method analogous thereto.

### Production method 4

Of the compounds represented by the formula [1], the production method shown here is suitable for the synthesis of compound [1-4] wherein the center ring has the following structure.

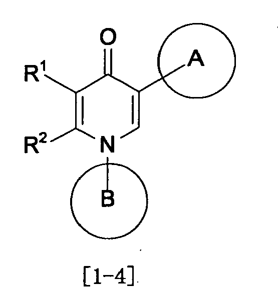

### Production method 4-1

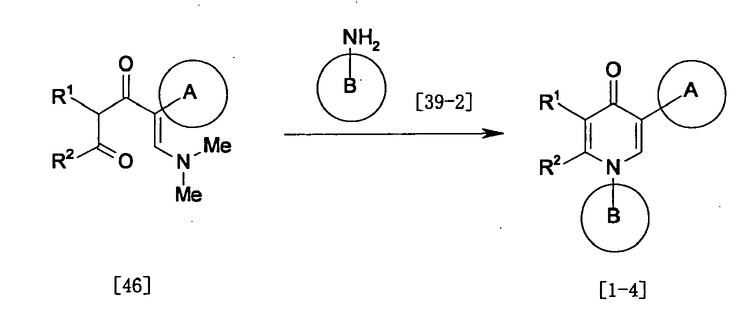

wherein the symbols in the formula are as defined for the formula [1].

Compound [1-4] can be obtained by heating compound [46] with compound [39-2] in a solvent such as toluene and the like.

### Production method 4-2

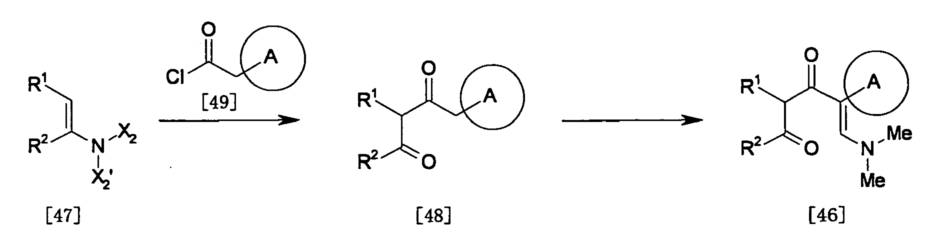

wherein the symbols in the formula are each as defined in the formula [1] and/or the aforementioned Production methods.

Compound [46] can be obtained by reacting compound [47] with compound [49] according to the method described in Production method 1-3d, compounds [23] to [24] to give compound [48], and reacting the compound with N,N-dimethylformamide dimethylacetal according to the method described in Production method 2-3b.

### Production method 5

Of the compounds represented by the formula [1], the production method shown here is suitable for the synthesis of compound [1-5] wherein the center ring has the following structure. 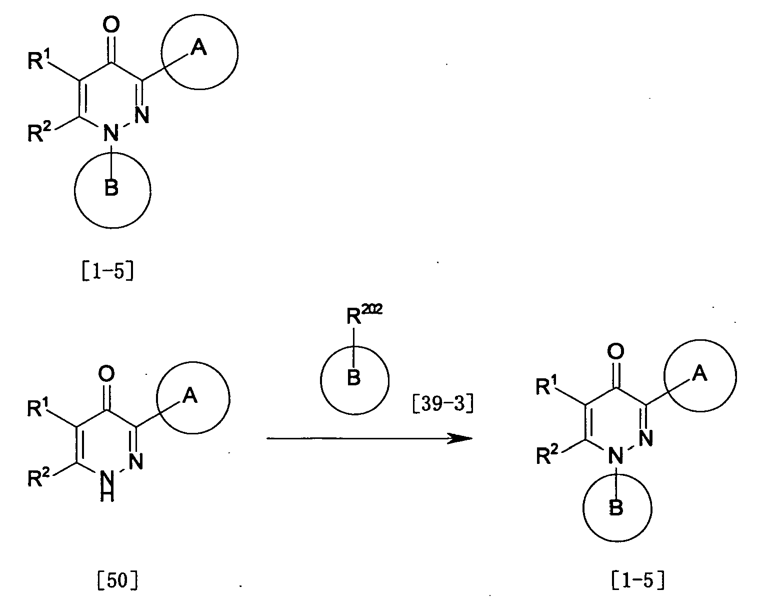 wherein the symbols in the formula are each as defined in the formula [1] and/or the aforementioned Production methods.

Compound [1-5] can be obtained by reacting compound [50] that can be synthesized according to the method described in Synthesis, 1979, 385 with compound [39-3] by a method similar to Production method 1-2.

### Production method 6

Of the compounds represented by the formula [1], the production method shown here is suitable for the synthesis of compound [1-6] wherein the center ring has the following structure. 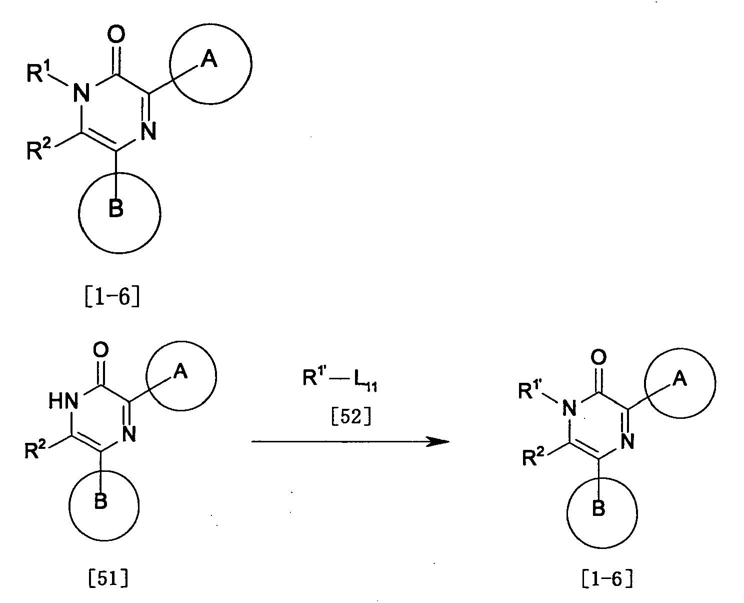 wherein R¹' is a C₁₋₆ alkyl group optionally substituted by 1 to 3 substituents selected from Group F, or a C₆₋₁₄ aryl group optionally substituted by 1 to 5 substituents selected from Group E, L₁₁ is a leaving group such as a chlorine atom, a bromine atom, an iodine atom, a methanesulfonyloxy group and the like, and other symbols are as defined for the formula [1].

Compound [1-6] can be obtained by reacting compound [51] that can be synthesized according to the method described in Synthesis, 1991, 10, 861 with compound [52] in the presence of a base such as potassium carbonate, sodium hydride and the like in a solvent such as N,N-dimethylformamide, tetrahydrofuran and the like at room temperature or under heating.

### Production method 7

Of the compounds represented by the formula [1], the production method shown here is suitable for the synthesis of compound [1-7] wherein the center ring has the following structure. 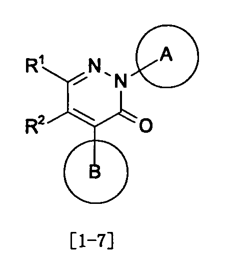 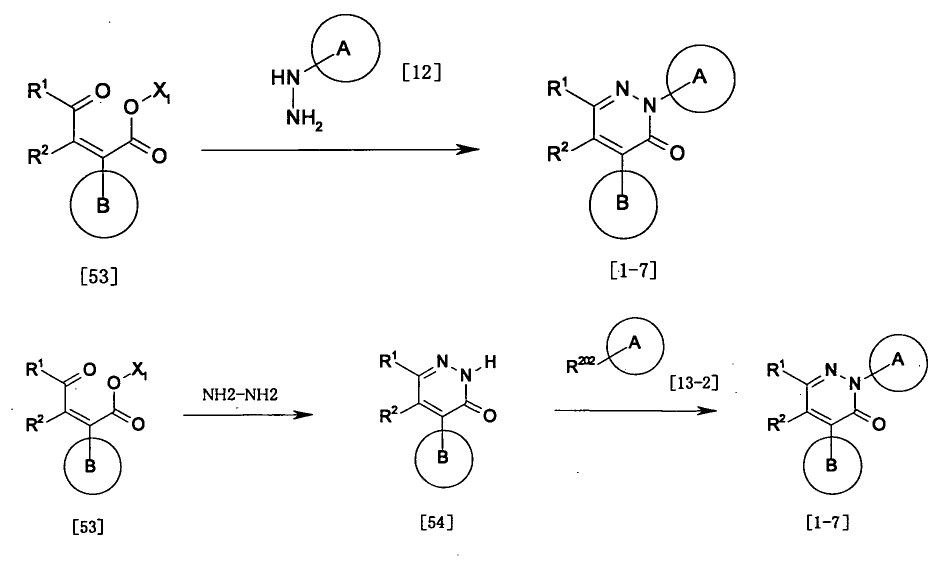 wherein the symbols in the formula are each as defined in the formula [1] and/or the aforementioned Production methods.

Compound [1-7] can be obtained from compound [53] by a method according to Production method 1-1 or Production method 1-2. Here, compound [53] to be the starting material can be synthesized by applying a method of any of Production methods 1-3.

### Production method 8

Of the compounds represented by the formula [1], the production method shown here is suitable for the synthesis of compound [1-8] wherein the center ring has the following structure. 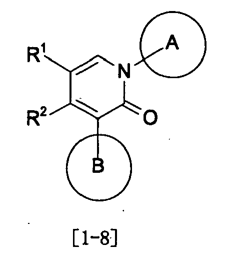 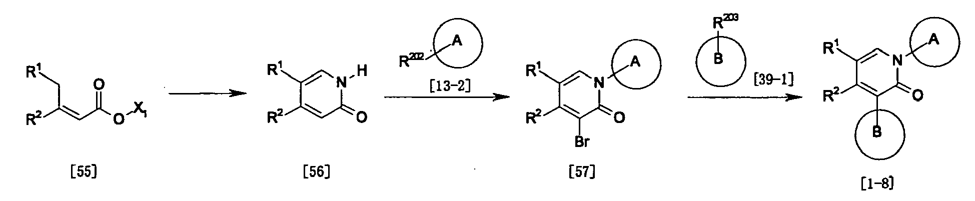 wherein the symbols in the formula are each as defined in the formula [1] and/or the aforementioned Production methods.

Compound [56] can be obtained by heating compound [55] with 1,3,5-triazine in the presence of a base such as sodium hydride and the like in a solvent such as N,N-dimethylformamide and the like. Compound [57] can be obtained by reacting compound [56] with compound [13-2] by a method similar to Production method 1-2 and bromating the compound by a method similar to Production method 2-2, step 2. Compound [1-8] can be synthesized by reacting compound [57] with compound [39-1] under similar conditions as in Production method 2-2, step 4.

### Production method 9

Of the compounds represented by the formula [1], the production method shown here is suitable for the synthesis of compound [1-9] wherein the center ring has the following structure. 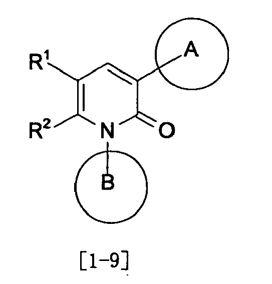 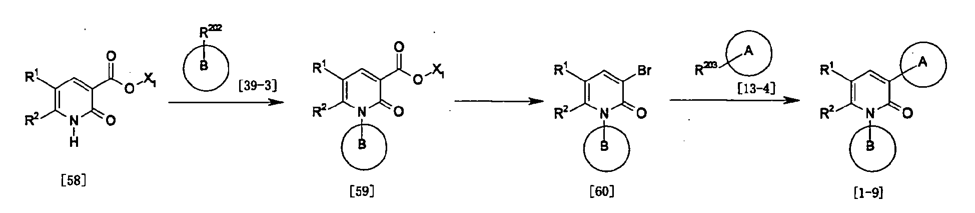 wherein the symbols in the formula are each as defined in the formula [1] and/or the aforementioned Production methods.

Compound [59] can be obtained by reacting compound [58] that can be synthesized according to the method described in Synthesis, 1999, 7, 1169 with compound [39-3] by a method according to Production method 1-2 or a method analogous thereto. Compound [60] is obtained by reacting compound [59] by a method similar to Production method 3-1, steps 2 and 3, and Production method 2-2, step 2. And compound [1-9] can be obtained by a coupling reaction using palladium as a catalyst in the same manner as in Production method 2-2, step 4

### Production method 10

Of the compounds represented by the formula [1], the production method shown here is suitable for the synthesis of compound [1-10] wherein the center ring has the following structure. 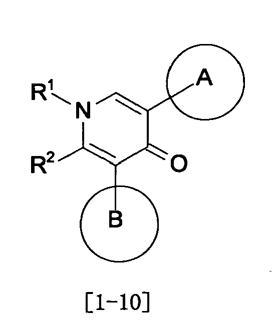 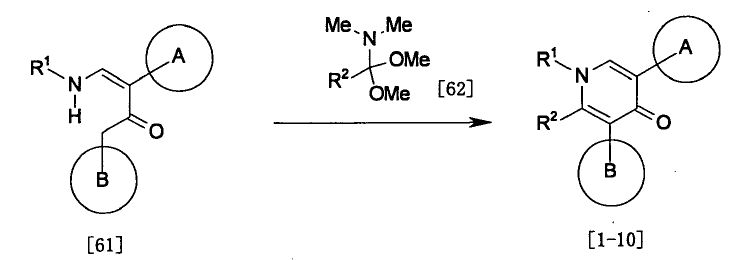 wherein the symbols in the formula are each as defined in the formula [1] and/or the aforementioned Production methods.

Compound [1-10] can be synthesized by reacting compound [61] with compound [62] according to the method described in Synthetic Communication, 1977, 7, 305, or a method analogous thereto.

### Production method 11

Of the compounds represented by the formula [1], the production method shown here is suitable for the synthesis of compound [1-11] wherein the center ring has the following structure. 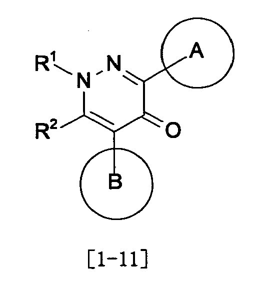 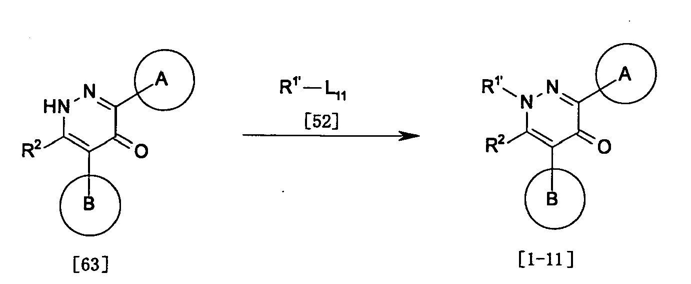 wherein the symbols in the formula are each as defined in the formula [1] and/or the aforementioned Production methods.

Compound [1-11] can be obtained by reacting compound [63] that can be synthesized according to the method described in Synthesis, **1979,** 385 with compound [52] by a method similar to Production method 6.

### Production method 12

Of the compounds represented by the formula [1], the production method shown here is suitable for the synthesis of compound [1-12] wherein the center ring has the following structure. 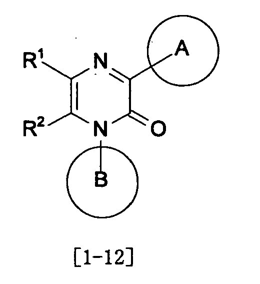 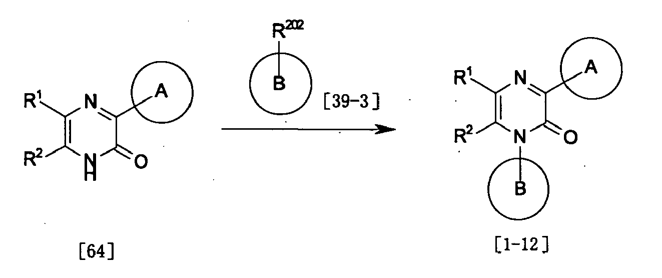 wherein the symbols in the formula are each as defined in the formula [1] and/or the aforementioned Production methods.

Compound [1-12] can be obtained by reacting compound [64] that can be synthesized according to the method described in Synthesis, 1991, 10, 861 with compound [39-3] by a method similar to Production method 1-2.

### Production method 13

Of the compounds represented by the formula [1], the production method shown here is suitable for the synthesis of compound [1-13] wherein the center ring has the following structure. 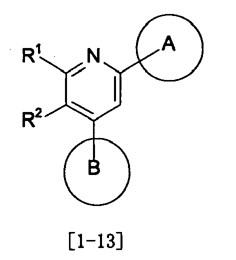 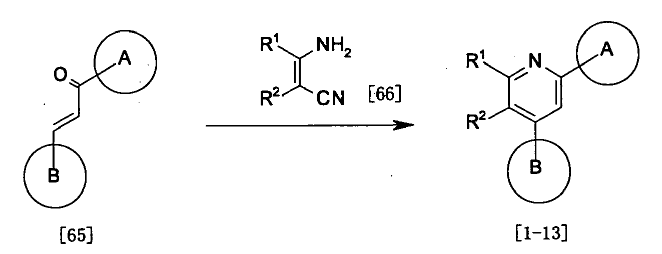 wherein the symbols in the formula are as defined for the formula [1].

Compound [1-13] can be obtained by heating compound [65] that can be synthesized from the corresponding aldehyde and ketone according to a conventional method with compound [66] in the presence of acetic acid ammonium in acetic acid.

### Production method 14

Of the compounds represented by the formula [1], the production method shown here is suitable for the synthesis of compound [1-14] wherein the center ring has the following structure.

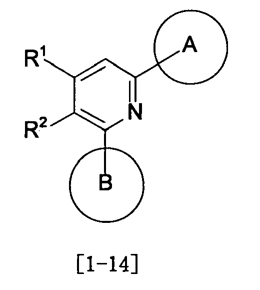

### Production method 14-1

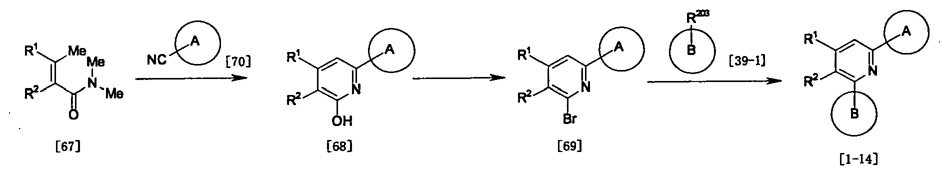

wherein the symbols in the formula are each as defined in the formula [1] and/or the aforementioned Production methods.

Compound [68] can be synthesized by reacting with an active species obtained from compound [67] and an organic metal reagent such as lithium diisopropylamide, lithium bis(trimethylsilyl)amide and the like and compound [70] in a solvent such as tetrahydrofuran and the like under cooling to room temperature. Compound [1-14] can be synthesized by bromating compound [68] using phosphorus oxybromide and the like to give compound [69] and subjecting the obtained compound [69] to a coupling reaction using palladium as a catalyst with compound [39-1] in the same manner as in Production method 2-2, step.

### Production method 14-2

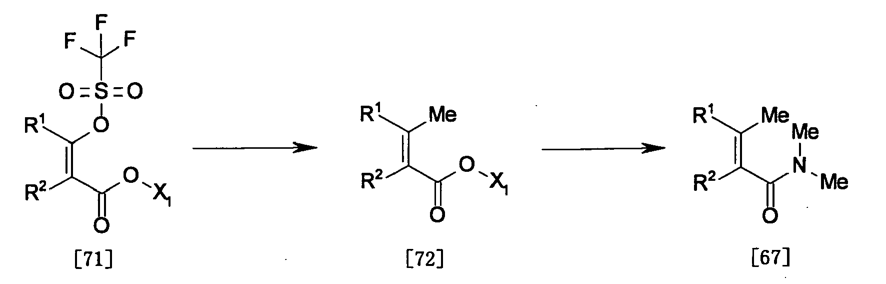

wherein the symbols in the formula are each as defined in the formula [1] and/or the aforementioned Production methods.

Compound [72] can be obtained by reacting compound [71] that can be synthesized according to a method similar to Production method 1-3b with an organic copper reagent that can be prepared from copper(I) iodide and methyl lithium according to a conventional method in an ether solvent such as diethyl lether and the like under cooling to room temperature. Compound [67] can be obtained by hydrolyzing compound [72] with a base such as an aqueous sodium hydroxide solution and the like, and reacting the compound with dimethylamine and a condensing agent such as dicyclohexylcarbodiimide, 1-ethyl-3-(3-dimethylaminopropyl)carbodiimide and the like in the presence or absence of an additive such as 1-hydroxybenzotriazole, N-hydroxysuccinimide and the like in an ether solvent such as tetrahydrofuran, dioxane and the like, a halogen solvent such as chloroform and the like, an amide solvent such as N,N-dimethylformamide, N,N-dimethylacetamide, 1,3-dimethyl-2-imidazolidone and the like.

### Production method 15

Of the compounds represented by the formula [1], the production method shown here is suitable for the synthesis of compound [1-15] wherein the center ring has the following structure. 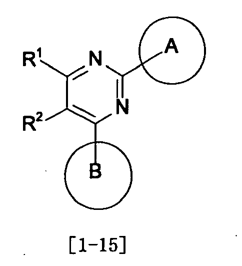 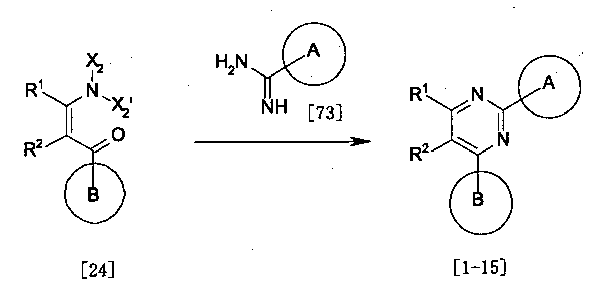 wherein the symbols in the formula are each as defined in the formula [1] and/or the aforementioned Production methods.

Compound [1-15] can be obtained by reacting compound [24] described in Production method 1-3d with compound [73] in pyridine from room temperature to under heating.

### Production method 16

Of the compounds represented by the formula [1], the production method shown here is suitable for the synthesis of compound [1-16] wherein the center ring has the following structure.

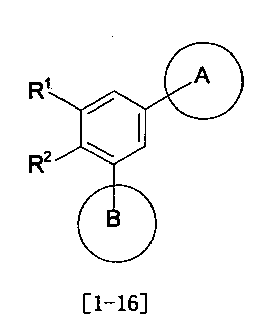

### Production method 16-1

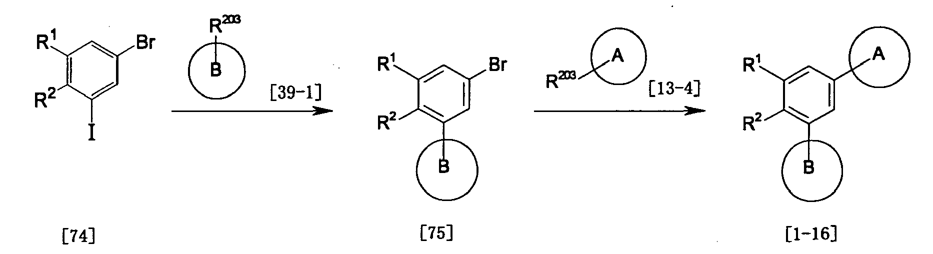

wherein the symbols in the formula are each as defined in the formula [1] and/or the aforementioned Production methods.

Compound [1-16] can be synthesized by a coupling reaction of compound [74] and compound [39-1], and a subsequent coupling reaction of compound [75] and compound [13-4] using palladium as a catalyst in the same manner as in Production method 2-2, step 4.

### Production method 16-2

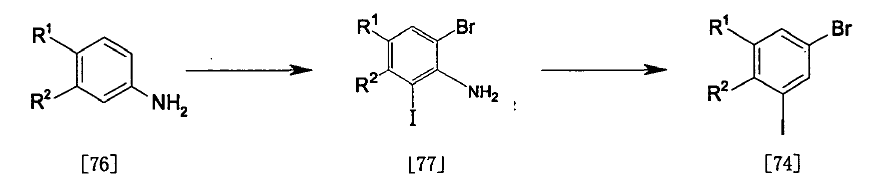

wherein the symbols in the formula are as defined for the formula [1].

Compound [74] can be synthesized by bromation and then iodination of compound [76] according to a method similar to Production method 2-2, step 2 to give compound [77] and deamination thereof according to a method similar to Production method 3-1, step 3.

### Production method 17

Of the compounds represented by the formula [1], compounds [1'-1], [1'-2] and [1'-3], wherein R¹ and R² (R¹ and R² are as defined for the formula [1]) in combination form the following ring structure, sometimes afford good results by construction of a center ring, followed by ring-closure. 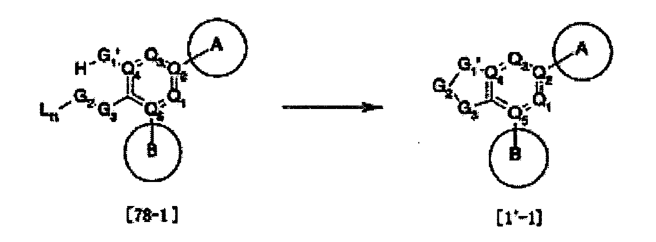 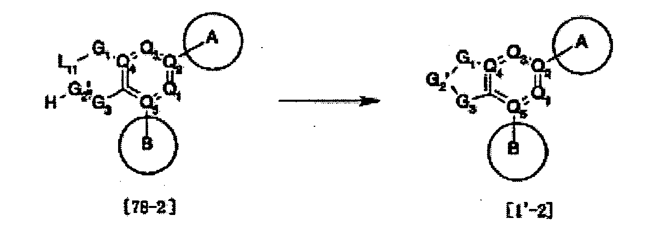 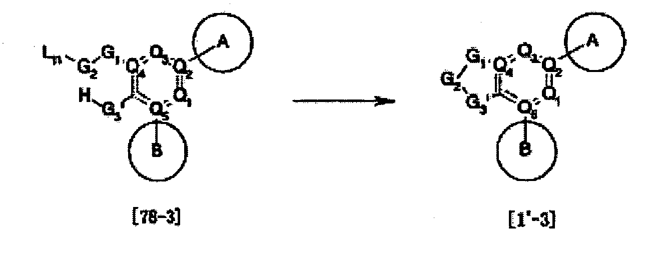 wherein G₁', G₂' and G₃' are each -N(R³)- wherein R³ is as defined for the formula [1], -O- or -S-, and other symbols are as defined for the formula [1].

Compound [1'-1] can be obtained by subjecting compound [78-1] to a ring-closing reaction in the presence or absence of a base in a polar aprotic solvent such as N,N-dimethylformamide, dioxane and the like at room temperature or under heating. As the base to be used, potassium carbonate, *tert*-butoxy potassium, sodium hydride and the like can be mentioned.

Compound [1'-2] can be obtained by reacting compound [78-2] under the conditions similar to those mentioned above, and compound [1'-3] can be obtained from compound [78-3]. Here, compound [78-1], [78-2] and [78-3] can be synthesized by a method according to the aforementioned Production method 1-16.

When carrying out this reaction, a conventional method usually employed in general organic reaction, such as protection of other reactive moiety in advance, followed by deprotection after the reaction, or a ring-closing reaction carried out with a stable precursor, followed by conversion to a desired form and the like often affords good results.

### Production method 18

A replacement or insertion of substituents on ring A represented by the formula [1] can be synthesized by the following methods. 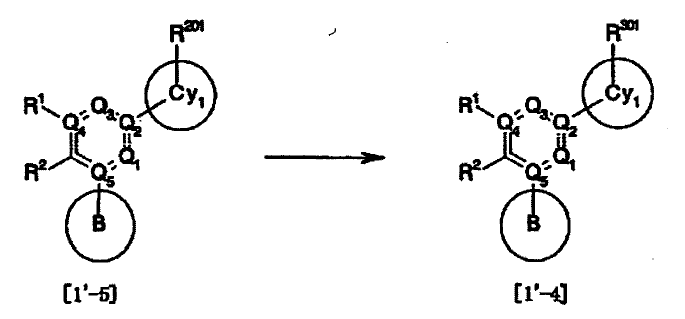 wherein Cy¹ is a monocyclic aryl group or a monocyclic heteroaryl group, R³⁰¹ is -J₅^{'}-R¹²¹ wherein J^{5'} is - N(R¹²²)-, -O-, -S-, -CO-, -CO₂-, -CON(R¹²²)-, -N(R¹²³)CO- or -N(R¹²³)SO₂-, R¹²¹, R¹²² and R¹²³ are as defined for the formula [1], a C₁₋₆ alkyl group optionally substituted by 1 to 3 substituents selected from Group F, a C₂₋₆ alkenyl group optionally substituted by 1 to 3 substituents selected from Group F, a C₂₋₆ alkynyl group optionally substituted by 1 to 3 substituents selected from Group F, a monocyclic aryl group optionally substituted by 1 to 5 substituents selected from Group E, a monocyclic heteroaryl group optionally substituted by 1 to 5 substituents selected from Group E, or a cyano group (Groups E, F and G are each as defined for the formula [1]), and other symbols are as defined for the formula [1].

### Production method 18-1

Synthesis of compound [1'-4] wherein R³⁰¹ is -J₅'-R¹²¹ wherein J₅' is -N(R¹²²)-

Compound [1'-4] wherein R³⁰¹ is -N(R¹²²)-R¹²¹ can be obtained from compound [1'-5] wherein R²⁰¹ is a bromine atom or an iodine atom by a coupling reaction using palladium as a catalyst with NH(R¹²²)-R¹²¹ under the conditions according to Production method 2-2, step 4 or analogous.

As the palladium catalyst and ligand to be used, a combination of tris(dibenzylideneacetone)dipalladium(0)-chloroform adduct and 4,5-bis(diphenylphosphino)-9,9-dimethylxanthene, or a combination of palladium(II) acetate and 2,2'-bis(diphenylphosphino)-1,1'-biphenyl often afford good results. As the base to be used, inorganic and organic bases such as sodium carbonate, potassium carbonate, triethylamine and the like can be mentioned. As the solvent, benzene solvents such as toluene, xylene and the like, ether solvents such as dioxane, dimethoxyethane and the like, alcohol solvents such as ethanol, *tert*-butanol and the like, amide solvents such as N,N-dimethylformamide and the like, and mixed solvents thereof can be mentioned.

Compound [1'-4] wherein R³⁰¹ is -N(R¹²²)-R¹²¹ can also be obtained by heating compound [1'-5] wherein R²⁰¹ is a fluorine atom or a chlorine atom and NH(R¹²²)-R¹²¹ in the presence of a base such as potassium carbonate and the like in an aprotic polar solvent such as N,N-dimethylformamide, dioxane and the like.

### Production method 18-2

### Synthesis of compound [1'-4] wherein R³⁰¹ is -J₅'-R¹²¹ wherein J₅' is -N(R¹²³)-CO- or -N(R¹²³)-SO₂-

Compound [1'-4] wherein R³⁰¹ is -N(R¹²³)-CO-R¹²¹ or -N(R¹²³)-SO₂-R¹²¹ can be obtained by subjecting compound [1'-5] wherein R²⁰¹ is a bromine atom or an iodine atom and H-N(R¹²³)-CO-R¹²¹ or H-N(R¹²³)-SO₂-R¹²¹ to a coupling reaction in the same manner as in Production method 18-1 and using palladium as a catalyst.

In addition, the compound [1'-4] can also be synthesized by reacting compound [1'-5] wherein R²⁰¹ is NH₂ with the corresponding acid chloride (e.g., Cl-COR¹²¹) or sulfonyl chloride (e.g., Cl-SO₂R¹²¹) in the presence of a base such as triethylamine, pyridine and the like in a solvent such as chloroform, tetrahydrofuran and the like.

### Production method 18-3

### Synthesis of compound [1'-4] wherein R³⁰¹ is -J₅'-R¹²¹ wherein J₅' is -O- or -S-

Compound [1'-4] wherein R³⁰¹ is -O-R¹²¹ or -S-R¹²¹ can be obtained by heating compound [1'-5] wherein R²⁰¹ is a fluorine atom or a chlorine atom with the corresponding R¹²¹-ONa or R¹²¹-SNa in an aprotic polar solvent such as N,N-dimethylformamide, dioxane and the like.

### Production method 18-4

### Synthesis of compound [1'-4] wherein R³⁰¹ is -J₅'-R¹²¹ wherein J₅' is -CO₂- or -CON(R¹²²)-

Compound [1'-4] wherein R³⁰¹ is -CO₂R¹²¹ can be obtained by heating compound [1'-5] wherein R²⁰¹ is a bromine atom or an iodine atom under carbon monooxide in the presence of the corresponding R¹²¹-OH, palladium(II) acetate, 1,3-bis(diphenylphosphino)propane and triethylamine. Compound [1'-4] wherein R³⁰¹ is -CO₂H can be obtained by hydrolyzing the obtained compound [1'-4] wherein R³⁰¹ is -CO₂R¹²¹ with sodium hydroxide in a solvent such as methanol and the like.

Further, compound [1'-4] wherein R³⁰¹ is - CON(R¹²²)-R¹²¹ can be obtained by reacting compound [1'-4] wherein R³⁰¹ is -CO₂H with NH(R¹²²)-R¹²¹ using a condensing agent such as dicyclohexylcarbodiimide, 1-ethyl-3-(3-dimethylaminopropyl)carbodiimide and the like in the presence or absence of an additive such as 1-hydroxybenzotriazole, N-hydroxysuccinimide and the like in an ether solvent such as tetrahydrofuran, dioxane and the like, or in a halogen solvent such as chloroform and the like, an amide solvent such as N,N-dimethylformamide, N,N-dimethylacetamide, 1,3-dimethyl-2-imidazolidone and the like.

### Production method 18-5

### Synthesis of compound [1'-4] wherein R³⁰¹ is -J₅'-R¹²¹ wherein J₅' is -CO-

Compound [1'-4] wherein R³⁰¹ is -CO-R¹²¹ can be obtained by subjecting compound [1'-5] wherein R²⁰¹ is a bromine atom or an iodine atom and the corresponding 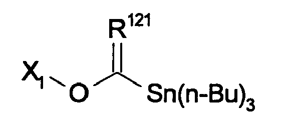 to a coupling reaction in the same manner as in Production method 18-1 and using palladium as a catalyst, and then to an acid treatment.

### Production method 18-6

### Synthesis of compound [1'-4] wherein R³⁰¹ is a C₂₋₆ alkenyl group optionally substituted by 1 to 3 substituents selected from Group F or a C₂₋₆ alkynyl group optionally substituted by 1 to 3 substituents selected from Group F

Compound [1'-4] wherein R³⁰¹ is a C₂₋₆ alkenyl group optionally substituted by 1 to 3 substituents selected from Group F or a C₂₋₆ alkynyl group optionally substituted by 1 to 3 substituents selected from Group F can be obtained by subjecting compound [1'-5] wherein R²⁰¹ is a bromine atom or an iodine atom and the corresponding R³⁰¹-Sn(*n*-Bu)₃ to a coupling reaction in the same manner as in Production method 18-1 and using palladium as a catalyst. In this reaction, a palladium catalyst such as tetrakis(triphenylphosphine)palladium(0) often affords preferable results.

### Production method 18-7

### Synthesis of compound [1'-4] wherein R³⁰¹ is a C₁₋₆ alkyl group optionally substituted by 1 to 3 substituents selected from Group F

Compound [1'-4] wherein R³⁰¹ is a C₁₋₆ alkyl group optionally substituted by 1 to 3 substituents selected from Group F can be obtained by subjecting compound [1'-4] wherein R³⁰¹ is a C₂₋₆ alkenyl group optionally substituted by 1 to 3 substituents selected from Group F or a C₂₋₆ alkynyl group optionally substituted by 1 to 3 substituents selected from Group F, which can be obtained in Production method 18-6, to a catalytic hydrogenation in the presence of a catalyst such as palladium carbon, platinum oxide and the like under hydrogen in an alcohol solvent such as methanol, ethanol and the like, an ether solvent such as tetrahydrofuran and the like, acetic acid or a mixed solvent thereof. In addition, compound [1'-4] wherein R³⁰¹ is a methyl group can also be obtained by heating compound [1'-5] wherein R²⁰¹ is a bromine atom or an iodine atom and trimethylboroxin in the presence of a palladium catalyst such as tetrakis(triphenylphosphine)palladium(0) and the like and a base such as potassium carbonate and the like in a solvent such as toluene, dioxane, N,N-dimethylformamide and the like.

### Production method 18-8

### Synthesis of compound [1'-4] wherein R³⁰¹ is a cyano group

Compound [1'-4] wherein R³⁰¹ is a cyano group can be obtained by heating compound [1'-5] wherein R²⁰¹ is a bromine atom or an iodine atom and dicyanozinc in the presence of tetrakis(triphenylphosphine)palladium(0) in N,N-dimethylformamide.

### Production method 18-9

### Synthesis of compound [1'-4] wherein R³⁰¹ is a monocyclic aryl group optionally substituted by 1 to 5 substituents selected from Group E or a monocyclic heteroaryl group optionally substituted by 1 to 5 substituents selected from Group E

Compound [1'-4] wherein R³⁰¹ is a monocyclic aryl group optionally substituted by 1 to 5 substituents selected from Group E, or a monocyclic heteroaryl group optionally substituted by 1 to 5 substituents selected from Group E can be obtained by subjecting compound [1'-5] wherein R²⁰¹ is a bromine atom or an iodine atom and R³⁰¹-Sn(*n*-Bu)₃ to a coupling reaction using palladium as a catalyst described in Production method 18-1. As the palladium catalyst, a combination of tris(dibenzylideneacetone)dipalladium(0)-chloroform adduct and tris(o-tolyl)phosphine, and 1,1'-bis(diphenylphosphino)ferrocene palladium(II) dichloride-dichloromethane complex afford preferable results.

In addition, a reaction under the same conditions as above and using R³⁰¹-B(OH)₂ instead of R³⁰¹-Sn(*n*-Bu)₃ often affords good results.

Compound [1'-4] wherein R³⁰¹ is a monocyclic heteroaryl group optionally substituted by 1 to 5 substituents selected from Group E can also be obtained using compound [1'-4] wherein R³⁰¹ is -CO₂H, which is synthesized by the method of Production method 18-4, and applying a method according to the method described in
a) Journal of Organic Chemistr, 1976, 41, 711
b) Journal of Heterocyclic Chemistry, 1977, 14, 345
c) Journal of Heterocyclic Chemistry, 1966, 3, 470
d) Journal of Organic Chemistry. 2000, 65, 2246 and the like, or a method analogous thereto.

### Production method 19

A replacement or insertion of substituents on ring B represented by the formula [1] can be synthesized by the following methods. 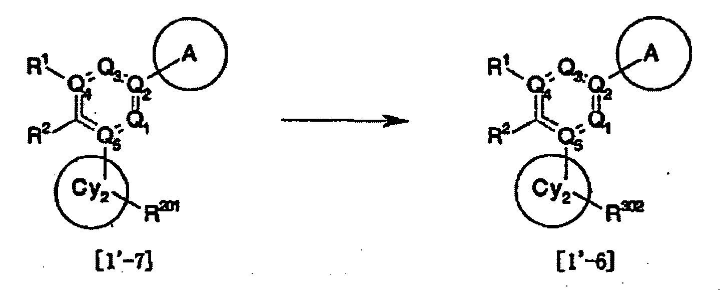 wherein Cy² is a monocyclic aryl group, a monocyclic heteroaryl group, a C₃₋₈ cycloalkyl group or a C₃₋₈ cycloalkenyl group, R³⁰² is a C₁₋₆ alkyl group, a C₂₋₆ alkenyl group, a C₂₋₆ alkynyl group, a C₁₋₆ alkoxy group, a C₁₋₆ alkylthio group, a C₁₋₆ alkylsulfinyl group, a C₁₋₆ alkylsulfonyl group, a C₁₋₆ alkyl-carbonyl group or a C₁₋₆ alkoxy-carbonyl group, each of which is optionally substituted by 1 to 3 substituents selected from Group G; -N(R⁷²)(R⁷³) or -CON(R⁷²)(R⁷³) wherein R⁷² and R⁷³ are as defined for the formula [1]); a monocyclic aryl group optionally substituted by 1 to 5 substituents selected from Group E or a monocyclic heteroaryl group optionally substituted by 1 to 5 substituents selected from Group E; or a cyano group, and other symbols are as defined for the formula [1].

Compound [1'-6] can be synthesized by reacting compound [1'-7] under the conditions similar to those in Production method 18.

Particularly, compound [1'-6] wherein R³⁰² is a C₁₋₆ alkylsulfinyl group or a C₁₋₆ alkylsulfonyl group can be synthesized by reacting compound [1'-6] wherein the corresponding R³⁰² is a C₁₋₆ alkylthio group with an oxidant such as methachloroperbenzoic acid and the like in a solvent such as chloroform and the like at room temperature or under heating.

### Production method 20

Of the compounds represented by formula [1], an insertion of substituent R³⁰³ on compounds [1'-8], [1'-9] and [1'-10] wherein R¹ and R² (R¹ and R² are as defined for the formula [1]) in combination form the following ring structure can be synthesized by the following methods. 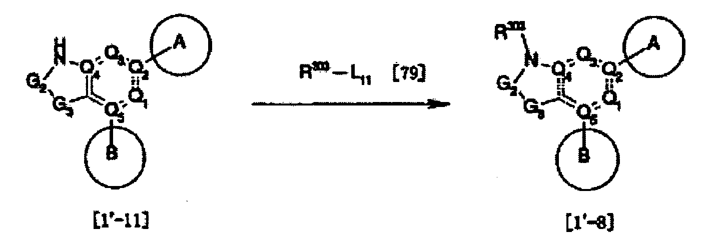 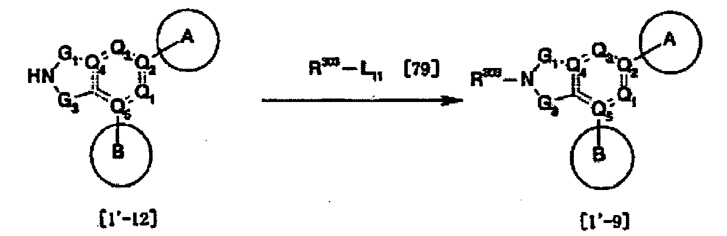 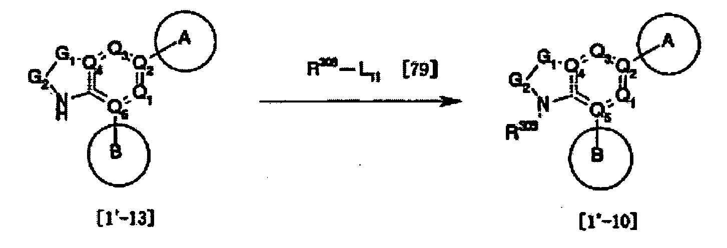 wherein R³⁰³ is a C₁₋₆ alkyl group, -L₃^{'}-J₃^{'}-R¹¹³ or -L₃^{''}-J₃-R¹¹³ wherein L₃^{'} is a bond, L^{3''} is an alkylene, J₃^{'} is -CO-, -SO₂-, -CON(R¹¹⁴)- or -SO₂N(R¹¹⁴)-, and other symbols are as defined for the formula [1].

When compound [1'-8] is desired, it can be synthesized from compound [1'-11] by introducing substituent R³⁰³. That is, the corresponding compound [1'-8] can be obtained by reacting compound [1'-11] with compound [79] wherein R³⁰³ is an alkyl group or - L₃"-J₃-R¹¹³ in the presence of a base such as sodium hydride and the like in a polar aprotic solvent such as N,N-dimethylformamide and the like at room temperature or under heating.

The corresponding compound [1'-8] can be obtained by reacting compound [1'-11] with compound [79] wherein R³⁰³ is -L₃'-J₃'-R¹¹³ in the presence of a base such as triethylamine, pyridine and the like in a solvent such as chloroform, tetrahydrofuran, pyridine and the like.

Particularly, when compound [1'-8] wherein R³⁰³ is -L₃'-J₃'-R¹¹³ wherein J₃' is -CON(R¹¹⁴)- is desired, it can be synthesized by reacting compound [1'-11] with triphosgene in the presence of a base such as triethylamine and the like in tetrahydrofuran and reacting the compound with the corresponding NHR¹¹⁴R¹¹³.

When carrying out this reaction, a conventional method usually employed in general organic reaction, such as protection of other reactive moiety in advance, followed by deprotection after the reaction, and the like often affords good results.

Compound [1'-9] can be obtained by reacting compound [1'-12] with compound [79] by a method similar to the above.

Similarly, compound [1'-10] can be obtained from compound [1'-13] and compound [79].

### Production method 21

When compound [1'-14] wherein R¹ is -N(R¹⁰²)-R¹⁰¹ wherein R¹⁰¹ and R¹⁰² are as defined for the formula [1] or compound [1'-17] wherein R¹ is -J₁'-R¹⁰¹ wherein J₁' is -N(R¹⁰²)-, -O- or -S- and R¹⁰¹ and R¹⁰² are as defined for the formula [1] is desired, a method comprising converting or introducing the substituent R¹ at the final step sometimes affords good results.

### Production method 21-1

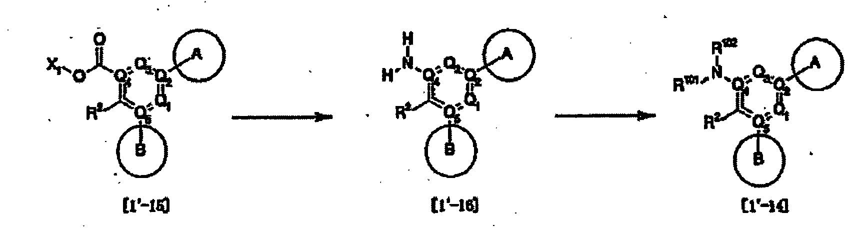

wherein the symbols in the formula are each as defined in the formula [1] and/or the aforementioned Production methods.

compound [1'-14] can be obtained by converting compound [1'-15] to compound [1'-16] by a method according to or similar to Production method 3-1, step 2 and, when R₁₀₁ and R₁₀₂ are the same, reacting the obtained compound [1'-16] with R₁₀₁-L₁₁ and R₁₀₂-L₁₁ in the presence of a base such as sodium hydride and the like in a polar aprotic solvent such as N,N-dimethylformamide, dioxane and the like from room temperature to under heating, and when R₁₀₁ and R₁₀₂ are different, reacting the obtained compound [1'-16] with R₁₀₁-L₁₁ or R₁₀₂-L₁₁ in the presence of a base such as sodium hydride and the like in a polar aprotic solvent such as N,N-dimethylformamide, dioxane and the like from room temperature to under heating to give a compound and repeating the same reaction with the compound. Here, compound [1'-15] can be synthesized according to the method described in the above-mentioned Production methods 1 to 16.

### Production method 21-2

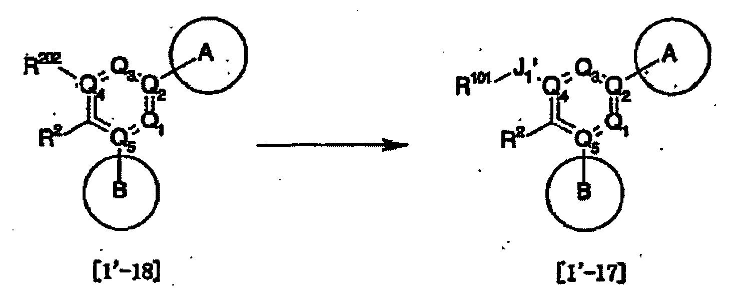

wherein the symbols in the formula are each as defined in the formula [1] and/or the aforementioned Production methods.

compound [1'-17] can be obtained by reacting compound [1'-18] that can be synthesized according to the method described in the above-mentioned Production methods 1 to 16 with H-J₁'-R¹⁰¹ in the presence of a base such as potassium carbonate and the like in a solvent such as N,N-dimethylformamide and the like at room temperature or under heating.

### Example 1-1

### 4-(3-methoxy-5-methyl-phenyl)-2-(5-nitro-2-trifluoromethyl-phenyl)-2,5,6,7-tetrahydro-cyclopenta[d]pyridazin-1-one

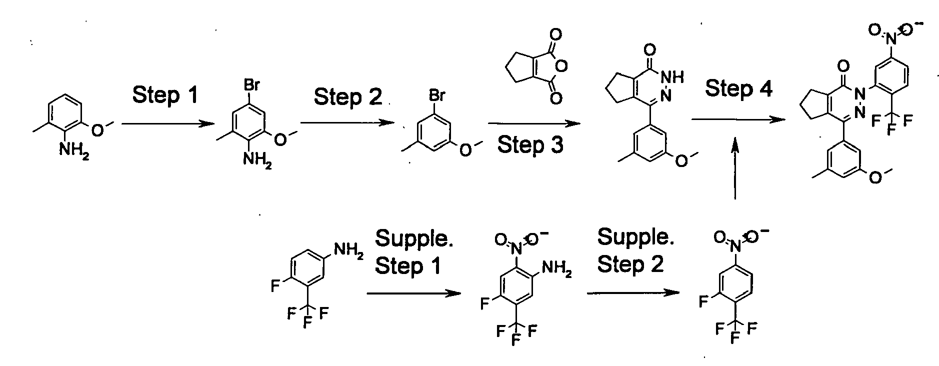

### Step 1

To a mixed solution of 2-methoxy-6-methyl-aniline (61.7 g) in acetic acid (50 mL) and methanol (150 mL) was added dropwise a solution of bromine (23 mL) in acetic acid (150 mL) under ice-cooling. After stirring at the same temperature for 2 hr, the reaction mixture was concentrated, 1N aqueous sodium hydroxide solution (300 mL) was added, and the mixture was extracted with ethyl acetate. The organic layer was washed with water and brine, dried, concentrated, and purified by silica gel column chromatography (chloroform:hexane=9:1) to give 4-bromo-2-methoxy-6-methyl-aniline (81.1 g).
¹H-NMR(CDCl₃) δ: 6.85(dd,1H,J=0.7,2.1Hz), 6.80(d,1H,J=2.1Hz), 3.79(s,3H), 3.73(brs,2H), 2.15(s,3H).

### Step 2

4-Bromo-2-methoxy-6-methyl-aniline (81.1 g) obtained in step 1 was suspended in a mixed solvent of acetic acid (570 mL), water (240 mL) and concentrated hydrochloric acid (65 mL) and a solution of sodium nitrite (31.1 g) in water (81 mL) was added dropwise under ice-cooling. After stirring at the same temperature for 30 min, the mixture was slowly added to ice-cooled 50% phosphorous acid aqueous solution (650 mL). The mixture was allowed to return to room temperature and, after stirring for 12 hr, water (400 mL) was added and the mixture was extracted 3 times with ethyl acetate. The combined organic layer was dried, concentrated and purified by silica gel column chromatography (hexane) to give 1-bromo-3-methoxy-5-methyl-benzene (69.6 g).
¹H-NMR(CDCl₃) δ: 6.93(brs,1H), 6.87(t,1H,J=2.1Hz), 6.65(brs,1H), 3.78(s,3H), 2.31(brs,3H).

### Step 3

Tetrahydrofuran (1 mL) and a small amount of iodine were added to magnesium (0.46 g), and a solution of 1-bromo-3-methoxy-5-methyl-benzene (3.2 g) obtained in step 2 in tetrahydrofuran (20 mL) was added dropwise under argon at room temperature. After stirring at room temperature for 1 hr, the mixture was added dropwise over 30 min to a solution of 5,6-dihydro-4H-cyclopenta[c]furan-1,3-dione (2.0 g) in tetrahydrofuran (20 mL) cooled to -70°C. The cooling bath was removed, and after stirring for 2 hr, 10% aqueous citric acid solution and ethyl acetate were added to separate the organic layer. The organic layer was washed with water and brine, concentrated, and ethanol (30 mL) and hydrazine monohydrate (0.75 mL) were added to the obtained residue. After stirring for 30 min under refluxing, hydrazine monohydrate (0.7 mL) was added again, and the mixture was stirred at the same temperature for 30 min. The mixture was allowed to return to room temperature, the insoluble material was filtered off, and the residue was concentrated and purified by silica gel column chromatography (ethyl acetate:hexane=2:1) to give 4-(3-methoxy-5-methylphenyl)-2,5,6,7-tetrahydro-cyclopenta[d]pyridazin-1-one (0.95 g).
¹H-NMR(CDCl₃)δ: 11.17(brs,1H), 7.00(s,1H), 6.93(s,1H), 6.80(s,1H), 3.84(s,3H), 3.06(tt,2H,J=2.1,7.4Hz), 2.99(tt,2H,J=1.6,7.4Hz), 2.39(s,3H), 2.15(quint,2H,J=7.4Hz).

### Step 4

To a solution of 4-(3-methoxy-5-methyl-phenyl)-2,5,6,7-tetrahydro-cyclopenta[d]pyridazin-1-one (0.95 g) obtained in step 3 in 1,3-dimethyl-2-imidazolidone (10 mL) were added potassium carbonate (1.53 g) and 2-fluoro-4-nitro-1-trifluoromethyl-benzene (3.31 g) obtained in Example 1-1, supplementary step 2, and the mixture was stirred at 80°C for 20 hr. The mixture was allowed to return to room temperature, ethyl acetate was added, and the mixture was washed with water and brine. The organic layer was dried, concentrated and purified by silica gel column chromatography (ethyl acetate:hexane=1:4-1:2) to give 4-(3-methoxy-5-methylphenyl)-2-(5-nitro-2-trifluoromethyl-phenyl)-2,5,6,7-tetrahydro-cyclopenta[d]pyridazin-1-one (1.27 g).
¹H-NMR(CDCl₃)δ: 8.43(dd,1H,J=2.1,8.6Hz), 8.40(d,1H,J=2.1Hz), 8.04(d,1H,J=8.6Hz), 6.97(s,1H), 6.91(brs,1H), 6.80(s,1H), 3.82(s,3H), 3.12(t,2H,J=6.7Hz), 3.04(tt,2H,J=1.6,7.7Hz), 2.37(s,3H), 2.22(quint,2H,J=7.4Hz).

### Supplementary step 1

4-Fluoro-3-trifluoromethyl-aniline (100 g) was added dropwise to trifluoroacetic anhydride (1000 mL) over 45 min under ice-cooling. After completion of the dropwise addition, the mixture was stirred for 30 min at the same temperature, and then potassium nitrate (62.1 g) was added over 1.5 hr by small portions to make the inside temperature 20°C or below. The mixture was allowed to return to room temperature, and after stirring for 12 hr, water (240 ml) was added by small portions under ice-cooling. The precipitated crystals were collected by filtration, washed with water, and the obtained resultant product was suspended in methanol (800 mL). A 7% aqueous potassium carbonate solution (570 mL) was added and the mixture was stirred at room temperature. After 1.5 hr, water (2 L) was added, and the precipitated crystals were collected by filtration and washed with water to give 4-fluoro-2-nitro-5-trifluoromethyl-aniline (74.2 g).
¹H-NMR(CDCl₃)δ: 7.98(d,1H,J=10.2Hz), 7.11(d,1H,J=5.7Hz), 6.08(brs,1H).

### Supplementary step 2

A solution of 4-fluoro-2-nitro-5-trifluoromethylaniline (183 g) produced by the method of supplementary step 1 in N,N-dimethylformamide (600 mL) was added dropwise over 1.5 hr to a solution of tert-butyl nitrite (145 mL) in N,N-dimethylformamide (300 mL), which had been heated to 60°C. After stirring for 2.5 hr at the same temperature, the reaction mixture was added to ice-cooled 6 N hydrochloric acid (1 L) by small portions. The mixture was extracted with hexane, the organic layer was washed with water, dried, and directly purified by silica gel column chromatography (hexane) to give 2-fluoro-4-nitro-1-trifluoromethylbenzene (47.7 g).
¹H-NMR(CDCl₃)δ: 8.16(d,1H,J=9.0Hz), 8.10(d,1H,J=9.3Hz), 7.86(dd,1H,J=7.2,8.1Hz).

### Example 1-2

### 2-(5-amino-2-trifluoromethyl-phenyl)-4-(3-methoxy-5-methyl-phenyl)-2,5,6,7-tetrahydro-cyclopenta[d]pyridazin-1-one

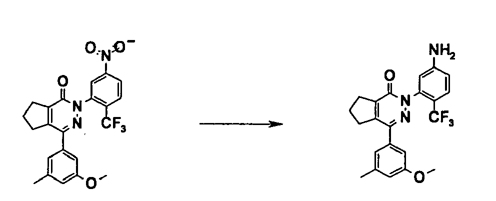

To a mixed solution of 4-(3-methoxy-5-methyl-phenyl)-2-(5-nitro-2-trifluoromethyl-phenyl)-2,5,6,7-tetrahydro-cyclopenta[d]pyridazin-1-one (1.27 g) obtained in Example 1-1 in ethanol (16 mL), tetrahydrofuran (8 mL) and water (4 mL) were added ammonium chloride (1.52 g) and reduced iron (1.59 g) and the mixture was stirred under refluxing for 2 hr. The mixture was allowed to return to room temperature, ethyl acetate was added, and the insoluble material was filtered through celite. The filtrate was washed with an aqueous sodium hydrogencarbonate solution, water and brine. The organic layer was dried and concentrated to give 2-(5-amino-2-trifluoromethyl-phenyl)-4-(3-methoxy-5-methyl-phenyl)-2,5,6,7-tetrahydro-cyclopenta[d]pyridazin-1-one (1.10 g).
¹H-NMR(CDCl₃)δ: 7.55(d,1H,J=8.7Hz), 6.97(s,1H), 6.92(brs,1H), 6.76(brs,1H), 6.74(dd,1H,J=2.3,8.7Hz), 6.67(d,1H,J=2.3Hz), 3.80(s,3H), 3.08(brs,2H), 3.01(t,2H,J=7.9Hz), 2.35(s,3H), 2.18(quint,2H,J=7.5,7.5Hz).

### Example 1-3

### 2-(5-bromo-2-trifluoromethyl-phenyl)-4-(3-methoxy-5-methyl-phenyl)-2,5,6,7-tetrahydro-cyclopenta[d]pyridazin-1-one

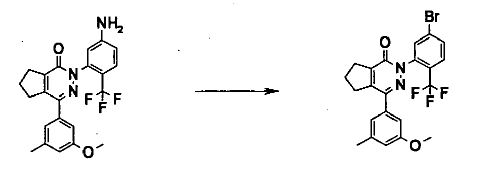

To a mixed solution of 2-(5-amino-2-trifluoromethyl-phenyl)-4-(3-methoxy-5-methyl-phenyl)-2,5,6,7-tetrahydro-cyclopenta[d]pyridazin-1-one (1.10 g) obtained in Example 1-2 in acetic acid (8 mL)-aqueous hydrobromic acid solution (2 mL) was added dropwise under ice-cooling a solution of sodium nitrite (0.20 g) in water (2 mL). After stirring at the same temperature for 20 min, a solution of copper(I) bromide (0.76 g) in aqueous hydrobromic acid (1 mL) was added dropwise. After stirring from under ice-cooling to room temperature for 2 hr, water was added, and the mixture was extracted with ethyl acetate, and washed with water, an aqueous sodium hydrogencarbonate solution and brine. The organic layer was dried, concentrated, the obtained crude crystals were washed with a mixed solvent (10 mL) of ethyl acetate-hexane (1:4) to give 2-(5-bromo-2-trifluoromethyl-phenyl)-4-(3-methoxy-5-methyl-phenyl)-2,5,6,7-tetrahydro-cyclopenta[d]pyridazin-1-one (0.85 g).
¹H-NMR(CDCl₃)δ: 7.74-7.65(m,3H), 6.96(s,1H), 6.90(brs,1H), 6.78(s,1H), 3.79(s,3H), 3.09(brs,2H), 3.01(tt,2H,J=1.5,7.5Hz), 2.36(s,3H), 2.19(quint,2H,J=7.5,7.5Hz).

### Example 1-4

### 4-(3-methoxy-5-methyl-phenyl)-2-[5-(pyridazin-3-yl)-2-trifluoromethyl-phenyl]-2,5,6,7-tetrahydro-cyclopenta[d]pyridazin-1-one

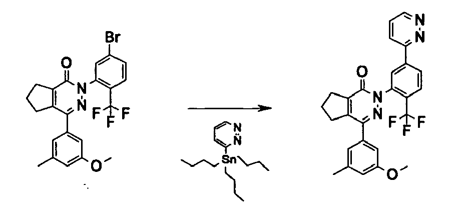

To a solution of 2-(5-bromo-2-trifluoromethylphenyl)-4-(3-methoxy-5-methyl-phenyl)-2,5,6,7-tetrahydro-cyclopenta[d]pyridazin-1-one (100 mg) obtained in Example 1-3 in toluene (1 mL) were added 3-tributylstannyl-pyridazine (154 mg; described in WO-0123383) and tetrakis(triphenylphosphine)palladium(0) (48 mg) under argon. After stirring under refluxing for 27 hr, the mixture was diluted with ethyl acetate and the insoluble material was filtered off. The filtrate was washed with water and brine, dried and concentrated. The residue purified by silica gel thin layer chromatography (ethyl acetate:hexane=1:1) to give 4-(3-methoxy-5-methyl-phenyl)-2-[5-(pyridazin-3-yl)-2-trifluoromethyl-phenyl]-2,5,6,7-tetrahydro-cyclopenta[d]pyridazin-1-one (38 mg).
¹H-NMR(CDCl₃)δ: 9.22(dd,1H,J=5.3,1.5Hz), 8.37(d,1H,J=8.3Hz), 8.22(brs,1H), 7.99(d,1H,J=8.3Hz), 7.91(dd,1H,J=8.7,1.5Hz), 7.58(dd,1H,J=8.7,5.3Hz), 6.99(s,1H), 6.93(brs,1H), 6.77(s,1H), 3.81(s,3H), 3.11(brs,2H), 3.04(t,2H,J=7.5Hz), 2.36(s,3H), 2.21(quint,2H,J=7.5,7.5Hz).

### Example 1-5

### 4-(3,5-dimethoxy-phenyl)-2-[5-((S)-3-hydroxy-pyrrolidin-1-yl)-2-nitro-phenyl]-2,5,6,7-tetrahydro-cyclopenta[d]pyridazin-1-one

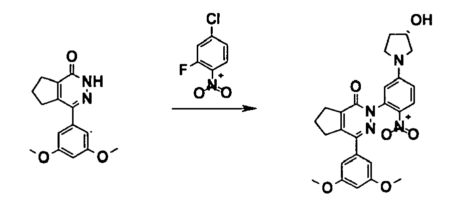

To a solution of 4-(3,5-dimethoxy-phenyl)-2,5,6,7-tetrahydro-cyclopenta[d]pyridazin-1-one (3.2 g) produced by a method similar to the method of Example 1-1 in N,N-dimethylformamide (35 mL) were added 4-chloro-2-fluoro-1-nitrobenzene (2.27 g) and potassium carbonate (1.95 g). After stirring at 80°C for 16 hr, (S)-3-hydroxy-pyrrolidine (1.41 mL) and potassium carbonate (2.89 g) were added. After further stirring at 80°C for 9 hr, water and 2N hydrochloric acid were added and the mixture was extracted with ethyl acetate. The organic layer was washed with water, dried, concentrated and purified by silica gel column chromatography (ethyl acetate:hexane=1:1-ethyl acetate) to give 4-(3,5-dimethoxy-phenyl)-2-[5-((S)-3-hydroxy-pyrrolidin-1-yl)-2-nitro-phenyl]-2,5,6,7-tetrahydro-cyclopenta[d]pyridazin-1-one (4.2 g).
¹H-NMR(CDCl₃)δ: 8.18(d,1H,J=9.8Hz), 6.70(d,2H,J=2.3Hz), 6.48-6.56(m,3H), 4.58-4.65(m,1H), 3.80(s,6H), 3.43-3.64(m,3H), 3.33-3.39(m,1H), 3.09(t,2H,J=7.5Hz), 3.01(t,2H,J=7.7Hz), 2.07-2.26(m,4H).

### Example 1-6

### 4-(3,5-dimethoxy-phenyl)-2-[2-dimethylamino-5-((S)-3-hydroxy-pyrrolidin-1-yl)-phenyl]-2,5,6,7-tetrahydro-cyclopenta[d]pyridazin-1-one

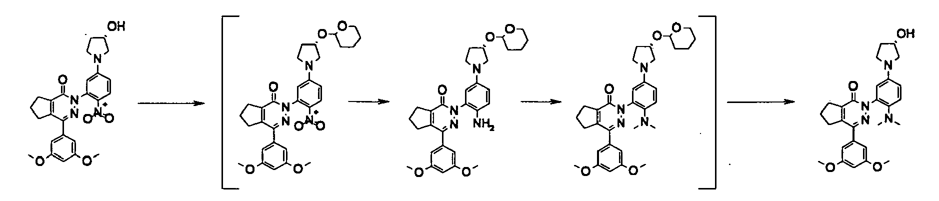

To a solution of 4-(3,5-dimethoxy-phenyl)-2-[5-((S)-3-hydroxy-pyrrolidin-1-yl)-2-nitro-phenyl]-2,5,6,7-tetrahydro-cyclopenta[d]pyridazin-1-one (300 mg) obtained in Example 1-5 in chloroform (3 mL) were added dihydropyran (0.068 mL) and camphorsulfonic acid (15 mg). After stirring at room temperature for 20 hr, a saturated aqueous sodium hydrogencarbonate solution was added, and the mixture was extracted with ethyl acetate. The organic layer was washed with water, dried and concentrated to give a crude product (326 mg). To a solution of the resultant product (326 mg) in tetrahydrofuran (18 mL)-ethanol (9 mL)-water (9 mL) were added ammonium chloride (248 mg) and reduced iron (163 mg). After stirring under refluxing for 1.5 hr, an insoluble material was filtered off through celite, and the filtrate was concentrated. Water-ethyl acetate was added to the residue. The organic layer was washed with water, dried and concentrated. Silica gel column chromatography (ethyl acetate:hexane=1:1-ethyl acetate) gave an amino derivative (250 mg). To a solution of the amino derivative (50 mg) in tetrahydrofuran (0.3 mL)-methanol (0.7 mL) were added 36% formalin (0.024 mL) and decaborane (7 mg). After stirring at room temperature for 26 hr, the reaction mixture was concentrated. The obtained residue was subjected to silica gel thin layer chromatography (ethyl acetate) and the obtained resultant product was dissolved in methanol (1 mL). Camphorsulfonic acid was added by small portions. After stirring at room temperature for 18 hr, 2N hydrochloric acid (0.04 mL) was added. After further stirring for 4 hrs, a saturated aqueous sodium hydrogencarbonate solution was added, and the mixture was extracted with ethyl acetate. The organic layer was washed with water, dried, concentrated and purified by silica gel thin layer chromatography (ethyl acetate) to give 4-(3,5-dimethoxy-phenyl)-2-[2-dimethylamino-5-((S)-3-hydroxy-pyrrolidin-1-yl)-phenyl]-2,5,6,7-tetrahydro-cyclopenta[d]pyridazin-1-one (32 mg).
¹H-NMR(CDCl₃+TFA-d)δ: 7.47(d,1H,J=9.1Hz), 6.86(dd,1H,J=9.1,2.8Hz), 6.68-6.71(m,3H), 6.60(dd,1H,J=1.9,2.3Hz), 4.74-4.81(m,1H), 3.83(s,6H), 3.39-3.67(m,4H), 3.31(s,6H), 3.14(t,2H,J=7.4Hz), 3.06(t,2H,J=7.4Hz), 2.20-2.34(m,4H).

### Example 1-7

### 4-(3,5-dimethoxy-phenyl)-2-[6-((S)-3-hydroxy-pyrrolidin-1-yl)-pyridin-2-yl]-2,5,6,7-tetrahydro-cyclopenta[d]pyridazin-1-one

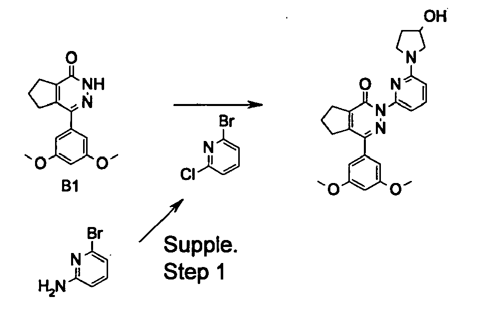

To a solution of 4-(3,5-dimethoxy-phenyl)-2,5,6,7-tetrahydro-cyclopenta[d]pyridazin-1-one (93 mg) obtained by a method similar to the method in Example 1-1 in N,N-dimethylformamide (1.3 mL) were added 2-bromo-6-chloropyridine (79 mg) obtained in supplementary step 1 and potassium carbonate (71 mg) and the mixture was stirred at 120°C for 16 hr. The mixture was allowed to return to room temperature, water was added, and the mixture was extracted with ethyl acetate. The organic layer was washed with water, dried and concentrated. The obtained residue was dissolved in N,N-dimethylacetamide (1 mL). (S)-3-hydroxy-pyrrolidine (56 mg) was added, and the mixture was stirred at 120°C for 5.5 hr. The reaction mixture was diluted with ethyl acetate and washed 4 times with brine. The organic layer was dried, concentrated and purified by silica gel column chromatography (chloroform:acetone=2:1-chloroform:methanol=10:1) to give 4-(3,5-dimethoxy-phenyl)-2-[6-((S)-3-hydroxy-pyrrolidin-1-yl)-pyridin-2-yl]-2,5,6,7-tetrahydro-cyclopenta[d]pyridazin-1-one (47 mg).
¹H-NMR(CDCl₃) δ: 7.58(dd,1H,J=8.3,7.4Hz), 6.77-6.75(m,3H), 6.50(t,1H,J=2.3Hz), 6.41(d,1H,J=8.3Hz), 4.59-4.55(m,1H), 3.81(s,6H), 3.67-3.53(m,4H), 3.06(t,2H,J=7.5Hz), 3.01(t,2H,J=7.7Hz), 2.20-2.03(m,4H), 1.81(br s,1H).

### Supplementary step 1

To a solution of 6-bromo-2-aminopyridine (500 mg) in concentrated hydrochloric acid (0.8 mL)-water (1.6 mL) was added dropwise a solution of sodium nitrite (219 mg) in water (3.2 mL) under ice-cooling. After stirring at the same temperature for 15 min, the mixture was allowed to return to room temperature. The mixture was neutralized with 1N aqueous sodium hydroxide solution and extracted with ethyl acetate. The organic layer was washed with brine, dried and concentrated. The obtained residue was purified by silica gel column chromatography (ethyl acetate:hexane=1:3) to give 2-bromo-6-chloropyridine (79 mg).
¹H-NMR(CDCl₃)δ: 7.52(t,1H,J=7.8Hz), 7.43(dd,1H,J=7.7,0.9Hz), 7.31(dd,1H,J=7.7,0.9Hz).

### Example 1-8

### 2-(5-bromo-2-trifluoromethyl-phenyl)-4-(3,5-dimethoxy-phenyl)-2,5,6,7-tetrahydro-cyclopenta[d]pyridazin-1-one

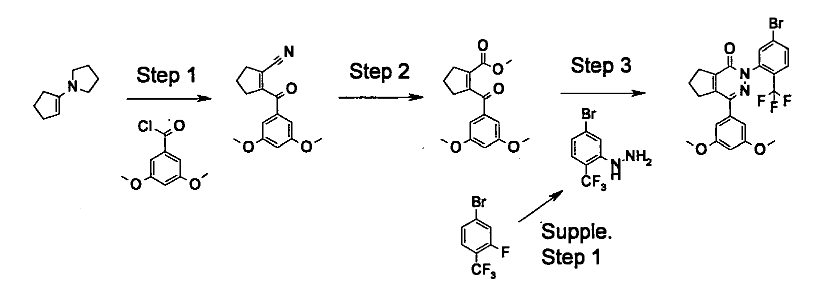

### Step 1

To a solution of triethylamine (38.5 mL) in chloroform (200 mL) was added 1-cyclopent-1-enyl-pyrrolidine (20.0 mL) under nitrogen atmosphere and under ice-cooling. At the same temperature, a solution of 3,5-dimethoxy-benzoylchloride (27.68 g) in chloroform (80 mL) was further added dropwise over 25 min. After stirring for 1 hr under ice-cooling, trimethylsilyl cyanide (28.8 mL) was added. The mixture was stirred at the same temperature for 30 min and at room temperature for 2 hr, and ice-cooled again. Trifluoroacetic acid (42.5 mL) was added dropwise over 20 min and the mixture was stirred at room temperature for 16 hr. Under ice-cooling, saturated aqueous sodium hydrogencarbonate solution (400 mL) was added, and the mixture was stirred at room temperature for 25 min to separate into two layers. The aqueous layer was extracted with chloroform (100 mL). The combined organic layer was washed with water, dried and concentrated. The obtained crude crystals were washed with diisopropyl ether to give 2-(3,5-dimethoxybenzoyl)-cyclopent-1-ene carbonitrile (20.7 g).
¹H-NMR(CDCl₃)δ: 6.97(d,2H,J=2.3Hz), 6.71(t,1H,J=2.3Hz), 3.85(s,6H),2.98-2.81(m,4H), 2.19-2.09(m,2H).

### Step 2

To a suspension of 2-(3,5-dimethoxy-benzoyl)-cyclopent-1-enecarbonitrile (40 g) produced by the method of step 1 in methanol (400 mL) was added concentrated sulfuric acid (40 mL) under ice-cooling. After stirring under refluxing for 5 days, the reaction mixture was poured into ice water. Methanol was evaporated and the residue was extracted with ethyl acetate, and washed with brine and an aqueous sodium hydrogencarbonate solution. The organic layer was dried, concentrated and purified by silica gel column chromatography (ethyl acetate:hexane=1:4) to give methyl 2-(3,5-dimethoxy-benzoyl)-cyclopent-1-enecarboxylate (26.2 g).
¹H-NMR(CDCl₃)δ: 7.01(d,2H,J=2.3Hz), 6.66(t,1H,J=2.3Hz), 3.83(s,6H), 3.52(s,3H), 2.87-2.79(m,4H), 2.16-2.06(m,2H).

### Step 3

To a suspension of methyl 2-(3,5-dimethoxybenzoyl)-cyclopent-1-enecarboxylate (6.6 g) obtained in step 2 in ethanol (50 mL) were added (5-bromo-2-trifluoromethyl-phenyl)-hydrazine (6.38 g) obtained in Example 1-8, supplementary step 1 and trifluoroacetic acid (2.62 mL) and the mixture was stirred at 80°C for 2 hr. (5-Bromo-2-trifluoromethyl-phenyl)-hydrazine (1.16 g) was added again and the mixture was continuously stirred at 80°C for 1 hr. The mixture was allowed to return to room temperature, and cesium carbonate (25.9 g) was added. After stirring at room temperature for 13 hr, 2NHCl (55 mL) was added under ice-cooling, and the mixture was extracted with chloroform and washed with brine. The organic layer was dried, concentrated, and the obtained crude crystals were recrystallized two times from a mixed solvent of ethanol and ethanol-chloroform to give 2-(5-bromo-2-trifluoromethyl-phenyl)-4-(3,5-dimethoxy-phenyl)-2,5,6,7-tetrahydro-cyclopenta[d]pyridazin-1-one (7.9 g).
¹H-NMR(CDCl₃)δ: 7.76-7.65(m,3H), 6.71(d,2H,J=2.3Hz), 6.51(t,1H,J=2.3Hz), 3.81(s,6H), 3.20-2.95(m,4H), 2.27-2.13(m,2H).

### Supplementary step 1

To a solution of 4-bromo-2-fluoro-1-trifluoromethyl-benzene (450 g) in 1,3-dimethyl-2-imidazolidone (900 mL) were added hydrazine monohydrate (180 mL) and potassium carbonate (255 g). After stirring at the inside temperature at 100°C for 4 hr, the mixture was allowed to return to room temperature, and water (2L)-diethyl ether (1 L) was added. The mixture was separated into two layers, and the aqueous layer was extracted with diethyl ether (1 L). The combined organic layer was washed with water, dried and concentrated. Hexane (720 mL) was added to the obtained crude crystals. The crystals were collected by filtration, and washed with hexane to give (5-bromo-2-trifluoromethyl-phenyl)-hydrazine (405 g).
¹H-NMR(CDCl₃)δ: 7.53(s,1H), 7.26(d,1H,J=8.4Hz), 6.91(d,1H,J=8.4Hz), 5.87(brs,1H), 3.63(brs,2H).

### Example 1-9

### methyl 3-[4-(3,5-dimethoxy-phenyl)-1-oxo-1,5,6,7-tetrahydro-cyclopenta[d]pyridazin-2-yl]-4-trifluoromethyl-benzoate

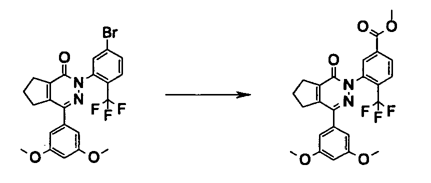

To a solution of 2-(5-bromo-2-trifluoromethylphenyl)-4-(3,5-dimethoxy-phenyl)-2,5,6,7-tetrahydro-cyclopenta[d]pyridazin-1-one (500 mg) obtained in Example 1-8 in dimethylsulfoxide (2 ml)-methanol (1.5 mL) were added palladium acetate (II)(23 mg), 1,3-bis(diphenylphosphino)propane (42 mg) and triethylamine (0.28 mL) and the mixture was stirred under carbon monooxide at 65°C for 19 hr. Palladium (II) acetate (46 mg), 1,3-bis(diphenylphosphino)propane (84 mg) and dimethyl sulfoxide (2 ml)-methanol (1.5 mL) were added and the mixture was stirred at 60°C for 30 hr. Further, 1,1'-bis(diphenylphosphino)ferrocene palladium (II) dichloride-dichloromethane complex (41 mg) and 1,1'-bis(diphenylphosphino)ferrocene (28 mg) were added and the mixture was continuously stirred at 60°C for 22 hr. Water was added to the reaction mixture and the mixture was extracted 3 times with ethyl acetate. The organic layer was washed with water, dried, concentrated and purified by silica gel column chromatography (ethyl acetate:hexane=2:3-1:1) to give methyl 3-[4-(3,5-dimethoxy-phenyl)-1-oxo-1,5,6,7-tetrahydro-cyclopenta[d]pyridazin-2-yl]-4-trifluoromethyl-benzoate (242 mg).
¹H-NMR (CDCl₃)δ: 8.23(d,1H,J=8.3Hz), 8.17(s,1H), 7.91(d,1H,J=8.3Hz), 6.71(d,1H,J=2.3Hz), 6.51(d,1H,J=2.3Hz), 3.94(s,3H), 3.80(s,6H), 3.20-2.90(m,4H), 2.30-2.10(m,2H).

### Example 1-10

### 3-[4-(3,5-dimethoxy-phenyl)-1-oxo-1,5,6,7-tetrahydro-cyclopenta[d]pyridazin-2-yl]-4-trifluoromethyl-benzoic acid

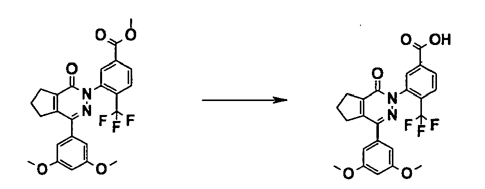

To a solution of methyl 3-[4-(3,5-dimethoxy-phenyl)-1-oxo-1,5,6,7-tetrahydro-cyclopenta[d]pyridazin-2-yl]-4-trifluoromethyl-benzoate (100 mg) obtained in Example 1-9 in methanol (1 mL) was added 4N aqueous sodium hydroxide solution (0.11 mL) and the mixture was stirred at 60°C for 30 min. The mixture was allowed to return to room temperature, and acetic acid (0.11 mL) and water (0.3 mL) were added. The precipitated solid was collected by filtration to give 3-[4-(3,5-dimethoxy-phenyl)-1-oxo-1,5,6,7-tetrahydro-cyclopenta[d]pyridazin-2-yl]-4-trifluoromethyl-benzoic acid (85 mg).
¹H-NMR(DMSO-d₆)δ: 14.0-13.3(brs,1H), 8.24(d,1H,J=8.3Hz), 8.11(s,1H), 8.09(d,1H,J=8.3Hz), 6.72(d,2H,J=2.1Hz), 6.60(t,1H,J=2.1Hz), 3.76(s,6H), 3.20-3.00(m,2H), 2.95-2.80(m,2H), 2.20-2.00(m,2H).

### Example 1-11

### 3-[4-(3,5-dimethoxy-phenyl)-1-oxo-1,5,6,7-tetrahydro-cyclopenta[d]pyridazin-2-yl]-N-methyl-4-trifluoromethyl-benzamide

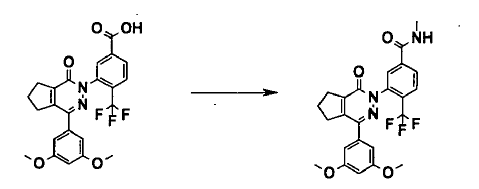

To a solution of 3-[4-(3,5-dimethoxy-phenyl)-1-oxo-1,5,6,7-tetrahydro-cyclopenta[d]pyridazin-2-yl]-4-trifluoromethyl-benzoic acid (15 mg) obtained in Example 1-10 in N,N-dimethylformamide (0.4 mL) were successively added 1-ethyl-3-(3-dimethylaminopropyl)carbodiimide hydrochloride (9 mg), 1-hydroxybenzotriazole hydrate (8 mg), methylamine hydrochloride (7 mg) and triethylamine (0.014 mL) and the mixture was stirred at room temperature for 2 hr. The mixture was diluted with ethyl acetate and washed with an aqueous sodium hydrogencarbonate solution, water and brine. The organic layer was dried and concentrated, and the obtained crude crystals were washed with chloroform-hexane to give 3-[4-(3,5-dimethoxy-phenyl)-1-oxo-1,5,6,7-tetrahydro-cyclopenta[d]pyridazin-2-yl]-N-methyl-4-trifluoromethyl-benzamide (9 mg).
¹H-NMR(DMSO-d₆)δ: 7.97(d,1H,J=8.3Hz), 7.89(d,1H,J=8.3Hz), 7.83(s,1H), 6.72(d,2H,J=2.3Hz), 6.51(t,1H,J=2.3Hz), 6.36(s,1H), 3.80(s,6H), 3.20-2.0(m,7H), 2.30-2.10(m,2H).

### Example 1-12

### 4-(3,5-dimethoxy-phenyl)-2-(5-fluoro-2-trifluoromethylphenyl)-2,5,6,7-tetrahydro-cyclopenta[d]pyridazin-1-one

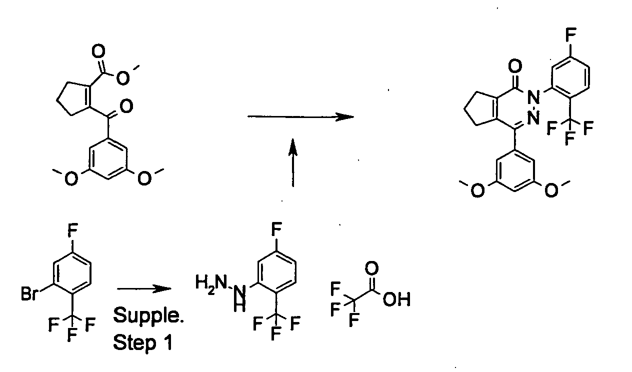

Methyl 2-(3,5-dimethoxy-benzoyl)-cyclopent-1-enecarboxylate (1.44 g) obtained in Example 1-8, step 2, and (5-fluoro-2-trifluoromethyl-phenyl)-hydrazine trifluoroacetate (1.68 g) obtained in supplementary step 1 were reacted in the same manner as in Example 1-8, step 3, to give 4-(3,5-dimethoxy-phenyl)-2-(5-fluoro-2-trifluoromethyl-phenyl)-2,5,6, 7-tetrahydro-cyclopenta[d]pyridazin-1-one (2.07 g).
¹H-NMR(CDCl₃)δ: 7.83(dd,1H,J=8.8,5.6Hz), 7.32-7.21(m,2H), 6.72(d,2H,J=2.3Hz), 6.51(t,1H,J=2.3Hz), 3.80(s,6H), 3.17-3.05(m,2H), 3.02(dd,2H,J=7.7,7.7Hz), 2.22(dd,1H,J=7.7,7.7Hz), 2.18(dd,1H,J=7.7,7.7Hz).

### Supplementary step 1

To a mixture of magnesium (1.83 g) and tetrahydrofuran (6 mL) were added dropwise a solution of 2-bromo-4-fluoro-1-trifluoromethyl-benzene (15.0 g) in tetrahydrofuran (60 mL) over 25 min under a nitrogen atmosphere. After completion of the dropwise addition, the mixture was stirred at room temperature for 1 hr, and added dropwise to an ice-cooled solution of di-tert-butyl azodicarboxylate (12.36 g) in tetrahydrofuran (120 mL). The cooling bath was removed, and after stirring for 1 hr, the mixture was ice-cooled again and acetic acid (3.53 mL) was added. Water was added and the mixture was extracted with diethyl ether. The organic layer was washed with water, dried and concentrated. The obtained residue was dissolved in chloroform (50 mL). Trifluoroacetic acid (50 mL) was added at room temperature, and after stirring for 5.5 hr, the reaction mixture was concentrated. The residue was crystallized from chloroform (150 mL) to give (5-fluoro-2-trifluoromethyl-phenyl)-hydrazine trifluoroacetate (9.46 g).
¹H-NMR(DMSO-d₆)δ: 8.00(brs,1H), 7.64(dd,1H,J=8.6,6.5Hz), 7.01(dd,1H,J=11.5,2.5Hz), 6.82(ddd,1H,J=8.6,8.6,2.5Hz).

### Example 1-13

### 4-(3,5-dimethoxy-phenyl)-2-[5-(3-hydroxy-propoxy)-2-trifluoromethyl-phenyl]-2,5,6,7-tetrahydro-cyclopenta[d]pyridazin-1-one

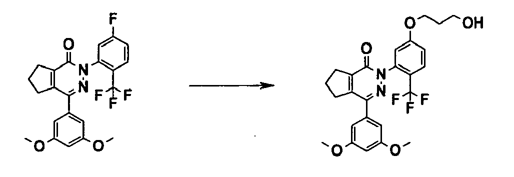

To a solution of 4-(3,5-dimethoxy-phenyl)-2-(5-fluoro-2-trifluoromethyl-phenyl)-2,5,6,7-tetrahydro-cyclopenta[d]pyridazin-1-one (50 mg) obtained in Example 1-12 in 1,3-propanediol (1 mL) was added cesium carbonate (78 mg). After stirring at 130°C for 3 hr, water was added, and the precipitated crystals were collected by filtration to give 4-(3,5-dimethoxy-phenyl)-2-[5-(3-hydroxy-propoxy)-2-trifluoromethylphenyl]-2,5,6,7-tetrahydro-cyclopenta[d]pyridazin-1-one (53.9 mg).
¹H-NMR(CDCl₃)δ: 7.71(d,1H,J=8.8Hz), 7.06(dd,1H,J=8.8,2.3Hz), 7.02(d,1H,J=2.3Hz), 6.73(d,2H,J=2.3Hz), 6.50(t,1H,J=2.3Hz), 4.18(dd,2H,J=6.0,6.0Hz), 3.87-3.74(m,2H), 3.80(s,6H),3.23-2.95(m,2H), 3.02(dd,2H,J=7.7,7.7Hz), 2.21(dd,1H,J=7.7,7.7Hz), 2.17(dd,1H,J=7.7,7.7Hz), 2.03(ddd,2H,J=12.1,6.0,6.0Hz), 1.66(t,1H,J=5.1Hz).

### Example 1-14

### 2-(5-bromo-2-methoxy-phenyl)-4-(3,5-dimethoxy-phenyl)-2,5,6,7-tetrahydro-cyclopenta[d]pyridazin-1-one

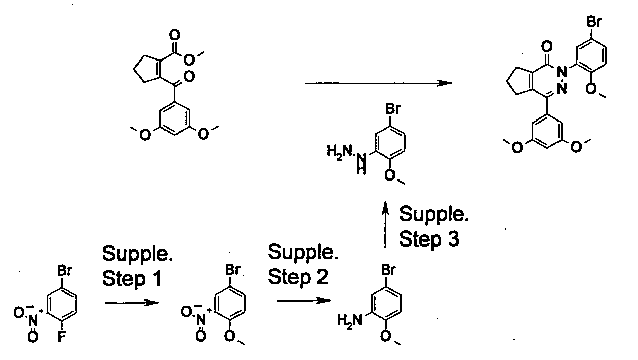

Methyl 2-(3,5-dimethoxy-benzoyl)-cyclopent-1-enecarboxylate (200 mg) obtained in Example 1-8, step 2, and (5-bromo-2-methoxy-phenyl)-hydrazine (179 mg) obtained in Example 1-14, supplementary step 3, were reacted in the same manner as in Example 1-8, step 3, and purified by silica gel column chromatography (ethyl acetate:hexane=2:1) to give 2-(5-bromo-2-methoxyphenyl)-4-(3,5-dimethoxy-phenyl)-2,5,6,7-tetrahydro-cyclopenta[d]pyridazin-1-one (249 mg).
¹H-NMR(CDCl₃)δ: 7.48-7.52(m,2H), 6.92(d,1H,J=8.4Hz), 6.72(d,2H,J=2.3Hz), 6.51(t,1H,J=2.3Hz), 3.82(s,3H), 3.82(s,6H), 2.97-3.11(m,4H), 2.12-2.23(m,2H).

### Supplementary step 1

To a solution of 4-bromo-1-fluoro-2-nitrobenzene (1.0 g) in methanol (10 mL) was added 28% sodium methoxide/methanol solution (1.39 mL) under ice-cooling, and the mixture was stirred at room temperature for 3 hr. Saturated brine was added to the reaction mixture and the mixture was extracted with ethyl acetate. The organic layer was dried, concentrated and purified by silica gel column chromatography (ethyl acetate:hexane=2:1) to give 4-bromo-1-methoxy-2-nitrobenzene (1.03 g).
¹H-NMR(CDCl₃)δ: 7.98(d,1H,J=2.6Hz), 7.64(dd,1H,J=9.0,2.6Hz), 6.99(d,1H,J=9.0Hz), 3.96(s,3H).

### Supplementary step 2

To a solution of 4-bromo-1-methoxy-2-nitrobenzene (1.03 g) obtained in supplementary step 1 in ethanol (8 mL)-tetrahydrofuran (4 mL)-water (2 mL) were added ammonium chloride (709 mg) and reduced iron (740 mg) and the mixture was stirred under refluxing for 2 hr. The mixture was allowed to return to room temperature, ethyl acetate was added and the insoluble material was filtered off. The filtrate was washed with an aqueous sodium hydrogencarbonate solution and brine and the organic layer was dried and concentrated to give 5-bromo-2-methoxy-aniline (888 mg).
¹H-NMR(CDCl₃)δ: 6.77-6.87(m,2H), 6.62(m,1H), 3.83(s,3H).

### Supplementary step 3

To a solution of 5-bromo-2-methoxy-aniline (888 mg) obtained in supplementary step 2 in trifluoroacetic acid (10 mL)-concentrated hydrochloric acid (2 mL) was added dropwise a solution of sodium nitrite (318 mg) in water (3 mL) under ice-cooling. After stirring at the same temperature for 10 min, a solution of tin (II) chloride dihydrate (1.98 g) in concentrated hydrochloric acid (3 mL) was added dropwise over 5 min. After stirring at the same temperature for 1 hr, a 4N aqueous sodium hydroxide solution (24 mL) was added, and the mixture was extracted with diethyl ether. The organic layer was washed with an aqueous sodium hydrogencarbonate solution and brine, dried and concentrated to give (5-bromo-2-methoxy-phenyl)-hydrazine (901 mg).
¹H-NMR(DMSO-d₆)δ: 7.08(d,1H,J=2.3Hz), 6.66-6.73(m,2H), 6.24(bs,1H), 4.01(bs,2H), 3.73(s,3H).

### Example 1-15

### 4-(3,5-dimethoxy-phenyl)-2-[5-((S)-3-hydroxy-pyrrolidin-1-yl)-2-methoxy-phenyl]-2,5,6,7-tetrahydro-cyclopenta[d]pyridazin-1-one

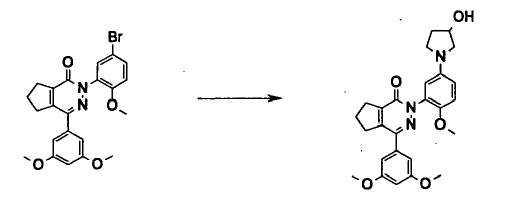

To a solution of 2-(5-bromo-2-methoxy-phenyl)-4-(3,5-dimethoxy-phenyl)-2,5,6,7-tetrahydro-cyclopenta[d]pyridazin-1-one (249 mg) obtained in Example 1-14 in dioxane (2.5 mL) were added (S)-3-hydroxy-pyrrolidine (142 mg), tris(dibenzylideneacetone)dipalladium(0)-chloroform adduct (56 mg), 4,5-bis(diphenylphosphino)-9,9-dimethylxanthene (94 mg) and cesium carbonate (266 mg) under argon, and the mixture was stirred at room temperature for 30 min and at 100°C for 8 hr. The reaction mixture was diluted with ethyl acetate, and washed with an aqueous ammonium chloride solution and brine. The organic layer was dried, concentrated and purified by silica gel column chromatography (chloroform:acetone=2:1) to give 4-(3,5-dimethoxy-phenyl)-2-[5-((S)-3-hydroxy-pyrrolidin-1-yl)-2-methoxyphenyl]-2,5,6,7-tetrahydro-cyclopenta[d]pyridazin-1-one (23 mg).
¹H-NMR(CDCl₃)δ: 6.98(d,1H,J=8.8Hz), 6.75(d,2H,J=2.3Hz), 6.56-6.64(m,2H), 6.49(t,1H,J=2.3Hz), 4.55(bs,1H), 3.81(s,6H), 3.77(s,3H), 3.42-3.52(m,2H), 3.21-3.34(m,2H), 2.95-3.12(m,4H), 2.11-2.23(m,3H), 2.03(m,1H), 1.72(bs,1H).

### Example 1-16

### 2-(5-bromo-2-trifluoromethyl-phenyl)-4-(thiophen-2-yl)-2,5,6,7-tetrahydro-cyclopenta[d]pyridazin-1-one

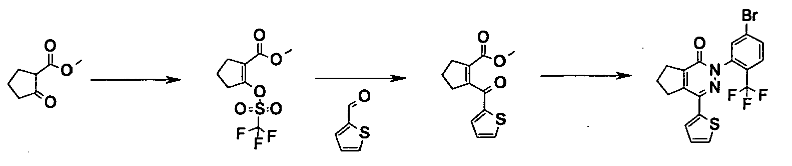

### Step 1

To a solution of methyl 2-oxo-cyclopentanecarboxylate (1.0 g) in methylene chloride (15 mL) was added diisopropylethylamine (5.58 mL) and the mixture was cooled to -70°C. Trifluoromethanesulfonic anhydride (1.29 mL) was added and the mixture was stirred for 15 hr while raising the temperature gradually to room temperature. Chloroform (30 mL) was added and the organic layer was washed with water and 10% aqueous citric acid solution, dried, concentrated and purified by silica gel column chromatography (ethyl acetate:hexane=1:6) to give methyl 2-trifluoromethanesulfonyloxy-cyclopent-1-enecarboxylate (1.77 g).
¹H-NMR(CDCl₃)δ: 3.79(3H,s), 2.81-2.66(4H,m), 2.02(2H,quin,J=7.9Hz).

### Step 2

To a solution of 2-thiophenealdehyde (150 mg) in chloroform (1.5 mL) were added trimethylsilyl cyanide (0.19 mL) and triethylamine (0.019 mL) and the mixture was stirred under argon at 50°C for 2.5 hr. The reaction mixture was concentrated, and an azeotropic operation was performed using toluene. The obtained residue was dissolved in tetrahydrofuran (5 mL), methyl 2-trifluoromethanesulfonyloxy-cyclopent-1-enecarboxylate (387 mg) obtained in step 1 was added, and potassium bis(trimethylsilyl)amide (0.5 M toluene solution; 2.68 mL) was added dropwise under argon at - 78°C. The mixture was stirred at the same temperature for 5 min, allowed to return to room temperature, and stirred for 1 hr. Tetrabutylammonium fluoride (1.0 M tetrahydrofuran solution; 1.34 mL) was added and the mixture was stirred at room temperature for 1 hr. An aqueous sodium hydrogencarbonate solution was added, and the mixture was extracted with ethyl acetate. The organic layer was dried, concentrated and purified by silica gel thin layer chromatography (ethyl acetate:hexane=1:4) to give methyl 2-(thiophene-2-carbonyl)-cyclopent-1-enecarboxylate (261 mg).
¹H-NMR(CDCl₃)δ: 7.69(dd,1H,J=4.9,1.1Hz), 7.56(dd,1H,J=3.8,1.1Hz), 7.12(dd,1H,J=4.9,3.8Hz), 3.54(s,3H), 2.94-2.77(m,4H), 2.38-2.25(m,1H), 2.19-2.05(m,1H).

### Step 3

Methyl 2-(thiophene-2-carbonyl)-cyclopent-1-enecarboxylate (260 mg) obtained in step 2 and (5-bromo-2-trifluoromethyl-phenyl)-hydrazine (310 mg) obtained in Example 1-8, supplementary step 1 were reacted in the same manner as in Example 1-8, step 3, and crystallized from a mixed solvent of ethyl acetate-hexane to give 2-(5-bromo-2-trifluoromethyl-phenyl)-4-(thiophen-2-yl)-2,5,6,7-tetrahydro-cyclopenta[d]pyridazin-1-one (212 mg).
¹H-NMR(CDCl₃)δ: 7.77-7.64(m,3H), 7.44(dd,1H,J=3.7,0.9Hz), 7.38(dd,1H,J=5.1,0.9Hz), 7.09(dd,1H,J=5.1,3.7Hz), 3.28(t,2H,J=7.5Hz), 3.02(t,2H,J=7.5Hz), 2.27(quin,2H,J=7.5Hz).

### Example 1-17

### 2-[5-((S)-3-hydroxy-pyrrolidin-1-yl)-2-trifluoromethylphenyl]-4-(thiophen-2-yl)-2,5,6,7-tetrahydro-cyclopenta[d]pyridazin-1-one

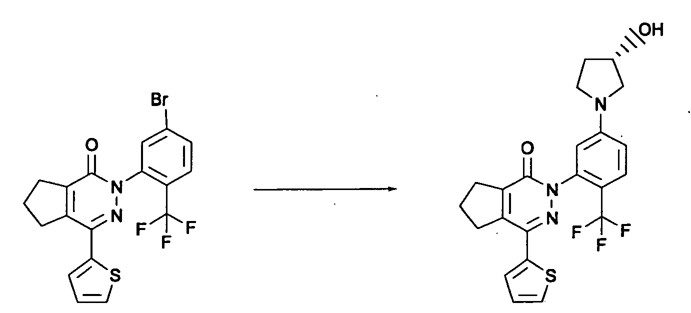

To a solution of 2-(5-bromo-2-trifluoromethylphenyl)-4-(thiophen-2-yl)-2,5,6,7-tetrahydro-cyclopenta[d]pyridazin-1-one (100 mg) obtained in Example 1-16 in dioxane (1.33 mL) were added palladium (II) acetate (5.1 mg), 2,2'-bis(diphenylphosphino)-1,1'-binaphthyl (16 mg) and cesium carbonate (104 mg) under argon, and the mixture was stirred at room temperature for 2 hr. tert-Butanol (0.67 mL) and (S)-3-hydroxy-pyrrolidine (0.023 mL) were added, and the mixture was stirred at 80°C for 3 hr. The mixture was allowed to return to room temperature, the insoluble material was filtered off, and the filtrate was concentrated. The obtained residue was purified by silica gel thin layer chromatography (chloroform:methanol=10:1), further crystallized from a mixed solution of ethyl acetate-hexane to give 2-[5-((S)-3-hydroxy-pyrrolidin-1-yl)-2-trifluoromethylphenyl]-4-(thiophen-2-yl)-2,5,6,7-tetrahydro-cyclopenta[d]pyridazin-1-one (82 mg).
¹H-NMR(DMSO-d₆)δ: 7.63(1H,dd,J=5.1,0.9Hz), 7.59(1H,d,J=8.8Hz), 7.54(1H,dd,J=3.7,0.9Hz), 7.16(1H,dd,J=5.1,3.7Hz), 6.71(1H,dd,J=8.8,2.3Hz), 6.60(1H,d,J=2.3Hz), 5.03(1H,d,J=3.7Hz), 4.40(1H,brs), 3.48-3.23(3H,m), 3.16(1H,d,J=10.7Hz), 2.84(2H,t,J=7.7Hz), 2.50(2H,quin,J=1.9Hz), 2.16(2H,quin,J=7.4Hz), 2.10-1.98(1H,m), 1.96-1.87(1H,m).

### Example 1-18

### 4-(cyclopent-1-enyl)-2-[5-((S)-3-hydroxy-pyrrolidin-1-yl)-2-trifluoromethyl-phenyl]-2,5,6,7-tetrahydro-cyclopenta[d]pyridazin-1-one

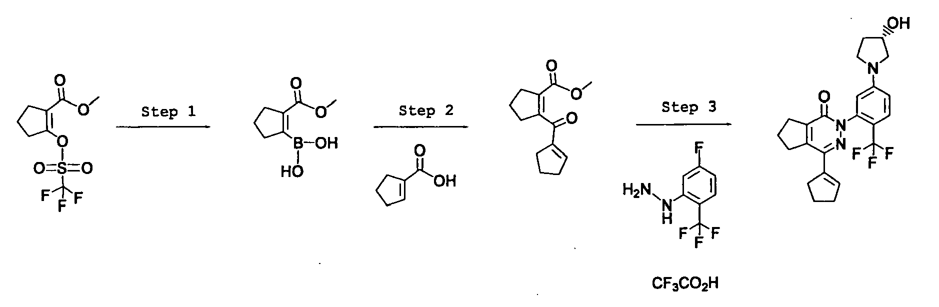

### Step 1

To a solution of methyl 2-trifluoromethanesulfonyloxy-cyclopent-1-enecarboxylate (2.43 g) obtained in Example 1-16 in dioxane (35 mL), step 1, were added bis(pinacolate)diborane (4.5 g), bis(triphenylphosphine)palladium(II) dichloride (311 mg), triphenylphosphine (232 mg) and potassium carbonate (2.45 g) under argon, and the mixture was stirred at 80°C for 20 hr. The mixture was allowed to return to room temperature, ethyl acetate was added, and the insoluble material was filtered off. The filtrate was concentrated, the obtained residue was dissolved in acetone (80 mL), and added to a solution of ammonium acetate (2.18 g) and sodium periodate (9.48 g) in water (80 mL). After stirring at room temperature for 21 hr, acetone was evaporated, and the mixture was extracted with ethyl acetate. The organic layer was dried, concentrated and purified by silica gel column chromatography (ethyl acetate:hexane=1:6-1:4) to give 2-methoxycarbonyl-cyclopent-1-ene boronic acid (1.12 g).
¹H-NMR(CDCl₃)δ: 7.53(s,2H), 3.82(s,3H), 2.79-2.68(m,4H), 1.86(tt,2H,J=7.4,7.4Hz).

### Step 2

To a solution of cyclopent-1-enecarboxylic acid (1.0 g) in chloroform (10 mL) was added oxalyl chloride (0.86 mL) under ice-cooling. The cooling bath was removed, a small amount of N,N-dimethylformamide was added, and the mixture was stirred at room temperature for 2 hr. The reaction mixture was concentrated, and an azeotropic operation was performed using toluene. To a solution of the obtained crude acid chloride (306 mg) in toluene (10 mL) were added 2-methoxycarbonyl-cyclopent-1-eneboronic acid (200 mg) obtained in step 1, tetrakis(triphenylphosphine)palladium(0) (68 mg) and cesium carbonate (1.9 g) and the mixture was stirred under refluxing. After 4 hr, a solution of the aforementioned acid chloride (31 mg) in toluene (0.5 mL) was added, and the mixture was further heated under reflux for 1.5 hr. The mixture was allowed to return to room temperature, the insoluble material was filtered through celite, and the filtrate was concentrated. The obtained residue was purified by silica gel column chromatography (ethyl acetate:hexane=1:6) to give methyl 2-(cyclopent-1-enecarbonyl)-cyclopent-1-ene carboxylate (235 mg).
¹H-NMR(CDCl₃)δ: 6.57-6.54(m,1H), 3.66(s,3H), 2.81-2.73(m,4H), 2.67-2.62(m,2H), 2.60-2.55(m,2H), 2.08-1.96(m,4H).

### Step 3

To a solution of methyl 2-(cyclopent-1-enecarbonyl)-cyclopent-1-ene carboxylate (235 mg) obtained in step 2 in dioxane (2.3 mL) was added (5-fluoro-2-trifluoromethyl-phenyl)-hydrazine trifluoroacetate (362 mg) obtained in Example 1-12, supplementary step 1, and the mixture was stirred at 100°C for 20 min. The mixture was allowed to return to room temperature, potassium carbonate (444 mg) and N,N-dimethylacetamide (2.3 mL) were added and the mixture was stirred at 80°C for 1 hr. At the same temperature, (S)-3-hydroxy-pyrrolidine (0.13 mL) was added, the temperature was raised to 100°C and stirred for 3.5 hr. (S)-3-hydroxy-pyrrolidine (0.043 mL) and cesium carbonate (348 mg) were added, and the mixture was continuously stirred with heating for 7 hr. The reaction mixture was allowed to return to room temperature, ethyl acetate was added, and the mixture was washed with 1N hydrochloric acid and brine. The organic layer was dried and concentrated, and the obtained crude crystals were recrystallized twice from ethyl acetate to give 4-(cyclopent-1-enyl)-2-[5-((S)-3-hydroxy-pyrrolidin-1-yl)-2-trifluoromethyl-phenyl]-2,5,6,7-tetrahydro-cyclopenta[d]pyridazin-1-one (188 mg).
¹H-NMR(CDCl₃)δ: 7.59(d,1H,J=8.8Hz), 6.61(dd,1H,J=8.8,2.2Hz), 6.50(d,1H,J=2.2Hz), 6.29(tt,1H,J=3.7,1.9Hz), 4.64-4.59(m,1H), 3.60-3.48(m,2H), 3.47-3.38(m,1H), 3.37-3.25(m,1H), 3.14(t,2H,J=7.4Hz), 3.02-2.91(m,2H), 2.75-2.65(m,2H), 2.62-2.54(m,2H), 2.24-2.04(m,4H), 1.90(tt,2H,J=7.4,7.4Hz).

### Example 1-19

### 4-cyclopentyl-2-[5-((S)-3-hydroxy-pyrrolidin-1-yl)-2-trifluoromethyl-phenyl]-2,5,6,7-tetrahydro-cyclopenta[d]pyridazin-1-one

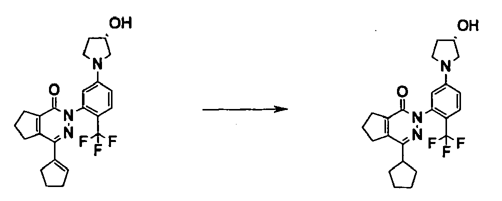

To a solution of 4-(cyclopent-1-enyl)-2-[5-((S)-3-hydroxy-pyrrolidin-1-yl)-2-trifluoromethyl-phenyl]-2,5,6,7-tetrahydro-cyclopenta[d]pyridazin-1-one (100 mg) obtained in Example 1-18 in tetrahydrofuran (7 mL) was added 10% palladium carbon (20 mg), and the mixture was stirred for 4 hr under hydrogen (3 atm). The insoluble material was filtered through celite, and the filtrate was concentrated. The obtained residue was crystallized from ethyl acetate to give 4-cyclopentyl-2-[5-((S)-3-hydroxy-pyrrolidin-1-yl)-2-trifluoromethylphenyl]-2,5,6,7-tetrahydro-cyclopenta[d]pyridazin-1-one (60 mg).
¹H-NMR(CDCl₃)δ: 7.56(d,1H,J=9.0Hz), 6.59(dd,1H,J=9.0,2.3Hz), 6.47(d,1H,J=2.3Hz), 4.63-4.56(m,1H), 3.59-3.35(m,3H), 3.35-3.25(m,1H), 3.10-2.87(m,5H), 2.26-2.02(m,4H), 2.00-1.60(m,8H).

### Example 1-20

### 4-(4-fluoro-3-methyl-phenyl)-6-(5-fluoro-2-trifluoromethyl-phenyl)-1,2,3,6-tetrahydro-pyrrolo[2,3-d]pyridazin-7-one

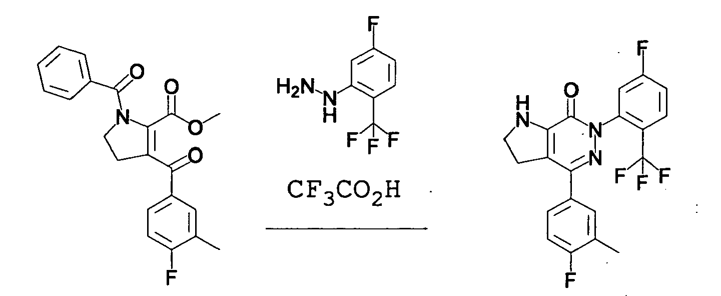

To a solution of methyl 1-benzoyl-3-(4-fluoro-3-methylbenzoyl)-4,5-dihydro-1H-pyrrole-2-carboxylate (720 mg) produced from 4-fluoro-3-methyl-benzaldehyde by a method similar to Example 1-24, steps 1 and 2, in 1-propanol (7 mL) was added (5-fluoro-2-trifluoromethyl-phenyl)-hydrazine trifluoroacetate (556 mg) obtained in Example 1-12, supplementary step 1, and the mixture was stirred at 80°C for 3 hr. At the same temperature, potassium carbonate (748 mg) was added, and the temperature was raised to 110°C. The mixture was continuously stirred with heating for 2 hr, and the reaction mixture was allowed to return to room temperature. Water (30 mL) and 1-propanol (2 mL) were added, and the mixture was stirred under ice-cooling for 2 hr. The precipitated crystals were collected by filtration to give 4-(4-fluoro-3-methyl-phenyl)-6-(5-fluoro-2-trifluoromethyl-phenyl)-1,2,3,6-tetrahydro-pyrrolo[2,3-d]pyridazin-7-one (491 mg).
¹H-NMR(DMSO-d₆)δ: 8.02(dd,1H,J=4.2,6.6Hz), 7.71(dd,1H,J=1.8,6.6Hz), 7.62(dt,1H,J=1.5,6.6Hz), 7.53(dd,1H,J=1.8,6.0Hz), 7.47(ddd,1H,J=1.8,4.2,6.0Hz), 7.21(t,1H,J=6.6.Hz), 7.12(s,1H), 3.71(t,2H,J=7.5Hz), 3.28(t,2H,J=7.5Hz), 2.27(d,3H,J=1.2Hz).

### Example 1-20a

### 6-(5-bromo-2-trifluoromethyl-phenyl)-4-(3,5-dimethoxy-phenyl)-1,2,3,6-tetrahydro-pyrrolo[2,3-d]pyridazin-7-one

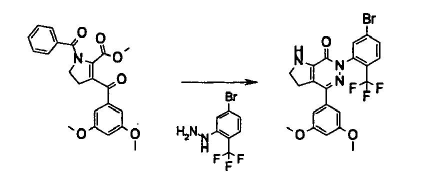

Methyl 1-benzoyl-3-(3,5-dimethoxy-benzoyl)-4,5-dihydro-1*H*-pyrrole-2-carboxylate (1.69 g) obtained by a method similar to the below-mentioned Example 1066-4, step 2, and (5-bromo-2-trifluoromethyl-phenyl)-hydrazine (1.20 g) obtained in Example 1-8, supplementary step 1 were reacted in the same manner as in Example 1-24, step 3, to give 6-(5-bromo-2-trifluoromethyl-phenyl)-4-(3,5-dimethoxy-phenyl)-1,2,3,6-tetrahydro-pyrrolo[2,3-d]pyridazin-7-one (1.90 g).
¹H-NMR(DMSO-d₆)δ: 8.01-7.93(2H,m), 7.87(1H,d,*J*=8.3Hz), 7.09(1H,s), 6.71(2H,d,*J*=2.1Hz), 6.55(1H,t,*J*=2.1Hz), 3.74(6H,s), 3.72-3.64(2H,m), 3.35-3.17(2H,m).

### Example 1-21

### methyl [4-(4-fluoro-3-methyl-phenyl)-6-(5-fluoro-2-trifluoromethyl-phenyl)-7-oxo-2,3,6,7-tetrahydro-pyrrolo[2,3-d]pyridazin-1-yl]-acetate

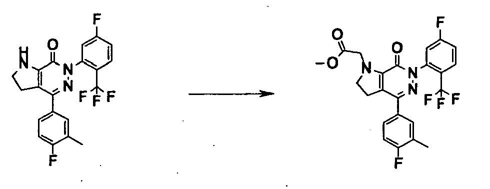

To a solution of 4-(4-fluoro-3-methyl-phenyl)-6-(5-fluoro-2-trifluoromethyl-phenyl)-1,2,3,6-tetrahydro-pyrrolo[2,3-d]pyridazin-7-one (289 mg) obtained in Example 1-20 in N,N-dimethylformamide (3 mL) were added methyl bromoacetate (0.34 mL) and cesium carbonate (1.15 g), and the mixture was stirred at 50°C for 1 hr. The mixture was allowed to return to room temperature, a 10% aqueous citric acid solution was added, and the mixture was extracted with ethyl acetate. The organic layer was washed with water, dried, concentrated and purified by silica gel column chromatography (ethyl acetate:hexane=1:2) to give methyl [4-(4-fluoro-3-methyl-phenyl)-6-(5-fluoro-2-trifluoromethyl-phenyl)-7-oxo-2,3,6,7-tetrahydro-pyrrolo[2,3-d]pyridazin-1-yl]-acetate (386 mg).
¹H-NMR(CDCl₃)δ: 7.81(dd,1H,J=4.2,6.0Hz), 7.41(dd,1H,J=1.5,6.0Hz), 7.35(ddd,1H,J=1.8,4.2,6.0Hz), 7.27(m,1H), 7.23(dd,1H,J=1.5,6.6Hz), 7.04(t,1H,J=6.6Hz), 4.58(s,2H), 3.77(m,7H), 2.31(d,3H,J=1.5Hz).

### Example 1-21a

### methyl [6-(5-bromo-2-trifluoromethyl-phenyl)-4-(3,5-dimethoxy-phenyl)-7-oxo-2,3,6,7-tetrahydro-pyrrolo[2,3-d]pyridazin-1-yl]-acetate

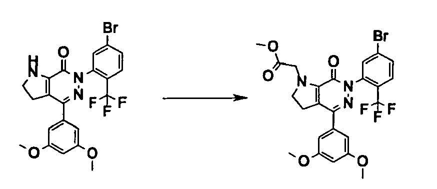

6-(5-Bromo-2-trifluoromethyl-phenyl)-4-(3,5-dimethoxy-phenyl)-1,2,3,6-tetrahydro-pyrrolo[2,3-d]pyridazin-7-one (1.72 g) obtained in Example 1-20a, methyl bromoacetate (1.63 mL) and cesium carbonate (5.65 g) were reacted in the same manner as in Example 1-25, and purified by silica gel column chromatography (ethyl acetate:hexane=1:2) to give methyl [6-(5-bromo-2-trifluoromethyl-phenyl)-4-(3,5-dimethoxy-phenyl)-7-oxo-2,3,6,7-tetrahydro-pyrrolo[2,3-d]pyridazin-1-yl]-acetate (2.45 g).
¹H-NMR(DMSO-d₆)δ: 7.97-7.93(2H,m), 7.88-7.84(1H,m), 6.71-6.68(2H,m), 6.58-6.55(1H,m), 4.68-4.33(2H,m), 3.80-3.55(2H,m), 3.75(6H,s), 3.61(3H,s), 3.33-3.08(2H,m).

### Example 1-22

### 4-(4-fluoro-3-methyl-phenyl)-6-(5-fluoro-2-trifluoromethyl-phenyl)-1-(2-hydroxy-ethyl)-1,2,3,6-tetrahydro-pyrrolo[2,3-d]pyridazin-7-one

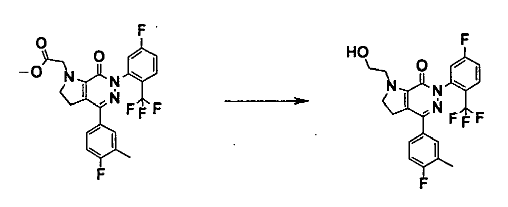

To a solution of methyl [4-(4-fluoro-3-methyl-phenyl)-6-(5-fluoro-2-trifluoromethyl-phenyl)-7-oxo-2,3,6,7-tetrahydro-pyrrolo[2,3-d]pyridazin-1-yl]-acetate (386 mg) obtained in Example 1-21 and lithium chloride (90 mg) in tetrahydrofuran (2 mL) were successively added sodium borohydride (80 mg) and methanol (2 mL) under ice-cooling, and the mixture was stirred at room temperature. After 8 hr, lithium chloride (90 mg) and sodium borohydride (80 mg) were added, and the mixture was continuously stirred at the same temperature for 2 hr. Lithium chloride (90 mg) and sodium borohydride (80 mg) were added again, and the mixture was stirred with heating at 50°C. After 4 hr, an aqueous potassium hydrogen sulfate solution was added dropwise under ice-cooling, and the mixture was extracted with ethyl acetate. The organic layer was washed with water, dried, concentrated and purified by silica gel column chromatography (ethyl acetate:hexane=1:2-1:1) to give 4-(4-fluoro-3-methylphenyl)-6-(5-fluoro-2-trifluoromethyl-phenyl)-1-(2-hydroxy-ethyl)-1,2,3,6-tetrahydro-pyrrolo[2,3-d]pyridazin-7-one (275 mg).
¹H-NMR(CDCl₃)δ: 7.82(dd,1H,J=4.2,6.6Hz), 7.38(dd,1H,J=1.5,6.0Hz), 7.32(ddd,1H,J=1.5,4.2,6.0Hz), 7.26(m,1H), 7.22(dd,1H,J=2.1,6.6Hz), 7.04(t,1H,J=6.6Hz), 3.91(brs,2H), 3.81(m,4H), 3.18(brs,2H), 2.30(d,3H,J=1.5Hz).

### Example 1-22a

### 6-(5-bromo-2-trifluoromethyl-phenyl)-4-(3,5-dimethoxy-phenyl)-1-(2-hydroxy-ethyl)-1,2,3,6-tetrahydro-pyrrolo[2,3-d]pyridazin-7-one

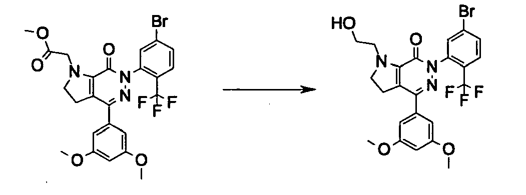

To a solution of methyl [6-(5-bromo-2-trifluoromethyl-phenyl)-4-(3,5-dimethoxy-phenyl)-7-oxo-2,3,6,7-tetrahydro-pyrrolo[2,3-d]pyridazin-1-yl]-acetate (2.45 g) obtained in Example 1-21a in tetrahydrofuran (30 mL) was added lithium borohydride (227 mg) under ice-cooling. The reaction mixture was allowed to return to room temperature and, after stirring for 22 hr, an aqueous ammonium chloride solution was added, and the mixture was extracted with ethyl acetate. The organic layer was washed with water, dried and concentrated to give 6-(5-bromo-2-trifluoromethyl-phenyl)-4-(3,5-dimethoxy-phenyl)-1-(2-hydroxy-ethyl)-1,2,3,6-tetrahydro-pyrrolo[2,3-d]pyridazin-7-one (1.70 g).
¹H-NMR(DMSO-d₆)δ: 7.96-7.92(2H,m), 7.86(1H,d,*J*=8.3Hz), 6.67(2H,d,*J*=1.9Hz), 6.56-6.54(1H,m), 4.71(1H,t,*J*=5.3Hz), 3.80-3.70(10H,m), 3.56(2H,q,*J*=5.3Hz), 3.25-3.03(2H,m).

### Example 1-23

### 4-(4-fluoro-3-methyl-phenyl)-1-(2-hydroxy-ethyl)-6-[5-(3-hydroxymethyl-azetidin-1-yl)-2-trifluoromethylphenyl]-1,2,3,6-tetrahydro-pyrrolo[2,3-d]pyridazin-7-one

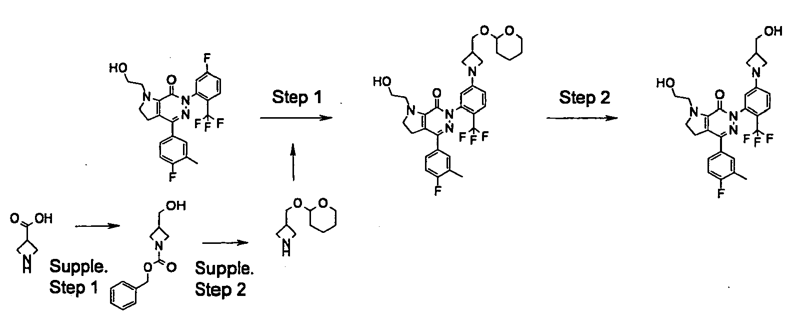

### Step 1

To a solution of 4-(4-fluoro-3-methyl-phenyl)-6-(5-fluoro-2-trifluoromethyl-phenyl)-1-(2-hydroxy-ethyl)-1,2,3,6-tetrahydro-pyrrolo[2,3-d]pyridazin-7-one (135 mg) obtained in Example 1-22 in N,N-dimethylacetamide (2 mL) were added potassium carbonate (62 mg) and 3-(tetrahydro-pyran-2-yloxymethyl)-azetidine (102 mg) obtained in Example 1-23, supplementary step 2, and the mixture was stirred at 100°C for 42 hr. The reaction mixture was allowed to return to room temperature, water was added, and the mixture was extracted with ethyl acetate. The organic layer was washed with water, dried, concentrated and purified by silica gel thin layer chromatography (ethyl acetate:hexane=4:3-2:1) to give 4-(4-fluoro-3-methylphenyl)-1-(2-hydroxy-ethyl)-6-{5-[3-(tetrahydro-pyran-2-yloxymethyl) -azetidin-1-yl]-2-trifluoromethylphenyl}-1,2,3,6-tetrahydro-pyrrolo[2,3-d]pyridazin-7-one (74 mg).
¹H-NMR(CDCl₃)δ: 7.55(d,1H,J=5.7Hz), 7.37(dd,1H,J=1.8,9.0Hz), 7.32(ddd,1H,J=2.7,5.7,9.0Hz), 7.01(t,1H,J=9.0Hz), 6.44(dd,1H,J=1.8,9.0Hz), 6.37(d,1H,J=2.7Hz), 4.60(t,1H,J=3.4Hz), 3.92(dd,1H,J=6.9,10.2Hz), 3.78(m,9H), 3.53(m,2H), 3.10(m,2H), 2.28(d,3H,J=1.5Hz), 1.61(m,7H).

### Step 2

To a solution of 4-(4-fluoro-3-methyl-phenyl)-1-(2-hydroxy-ethyl)-6-{5-[3-(tetrahydro-pyran-2-yloxymethyl) -azetidin-1-yl]-2-trifluoromethyl-phenyl}-1,2,3,6-tetrahydro-pyrrolo[2,3-d]pyridazin-7-one (74 mg) obtained in step 1 in methanol (1 mL) was added p-toluenesulfonic acid monohydrate (10.6 mg) and the mixture was stirred at room temperature for 4 hrs. An aqueous sodium hydrogencarbonate solution was added, and the mixture was extracted with ethyl acetate. The organic layer was washed with water, dried, concentrated and purified by silica gel thin layer chromatography (ethyl acetate:methanol=100:1), and further crystallized from a mixed solvent of methanol-water to give 4-(4-fluoro-3-methyl-phenyl)-1-(2-hydroxy-ethyl)-6-[5-(3-hydroxymethyl-azetidin-1-yl)-2-trifluoromethyl-phenyl]-1,2,3,6-tetrahydro-pyrrolo[2,3-d]pyridazin-7-one (43 mg).
¹H-NMR(DMSO-d₆)δ: 7.57(d,1H,J=6.6Hz), 7.48(dd,1H,J=1.9,5.7Hz), 7.42(ddd,1H,J=1.9,3.9,6.6Hz), 7.18(t,1H,J=9.3Hz), 6.55(dd,1H,J=1.5,6.6.Hz), 6.42(d,1H,J=1.8Hz), 4.80(t,1H,J=3.9Hz), 4.70(t,1H,J=3.9Hz), 3.95(t,2H,J=6.0Hz), 3.75(m,4H), 3.67(t,2H,J=6.0Hz), 3.58(m,4H), 3.13(m,2H), 2.84(m,1H), 2.27(d,3H,J=0.9Hz).

### Supplementary step 1

To a suspension of azetidine-3-carboxylic acid (6.25 g) in dioxane (62.5 mL) was added 4N aqueous sodium hydroxide solution (61.9 mL) and, under ice-cooling, benzyl chloroformate (17.6 mL) was added dropwise. The mixture was allowed to return to room temperature and, after stirring for 1 hr, a 8N aqueous sodium hydroxide solution (61.9 mL) was added, and the mixture was washed twice with diethyl ether. The aqueous layer was acidified with 6N hydrochloric acid, and extracted twice with ethyl acetate. The organic layer was washed with water, dried and concentrated, and the obtained residue was dissolved in tetrahydrofuran (100 mL). Triethylamine (11.8 mL) was added and, under ice-cooling, isobutyl chloroformate (8.09 mL) was added dropwise over 10 min. After stirring at the same temperature for 30 min, the insoluble material was filtered off, and the filtrate was added to a suspension of sodium borohydride (7.99 g) in water (33 mL) under ice-cooling. The mixture was allowed to return to room temperature, and after stirring for 1 hr, water was added, and the mixture was extracted with hexane. The organic layer was washed with water, dried, concentrated and purified by silica gel column chromatography (ethyl acetate:hexane=1:1-ethyl acetate) to give benzyl 3-hydroxymethyl-azetidine-1-carboxylate (7.62 g).
¹H-NMR(CDCl₃)δ: 7.36-7.29(m,5H), 5.09(s,2H), 4.08(t,2H,J=8.5Hz), 3.80-3.77(m,4H), 2.81-2.71(m,1H), 1.63(t,1H,J=5.1Hz).

### Supplementary step 2

To a solution of benzyl 3-hydroxymethyl-azetidine-1-carboxylate (7.62 g) obtained in supplementary step 1 in chloroform (80 mL) were added dihydropyran (3.45 mL) and pyridinium p-toluenesulfonate (434 mg) and the mixture was stirred at room temperature. After 1 hr, water was added, and the mixture was extracted with hexane. The mixture was washed with an aqueous sodium hydrogencarbonate solution and water. The organic layer was dried, concentrated and the obtained residue was dissolved in methanol (100 mL). 5% Palladium carbon (965 mg) was added and the mixture was stirred under ordinary hydrogen pressure for 13 hr. The catalyst was filtered off, and the obtained filtrate was concentrated to give 3-(tetrahydro-pyran-2-yloxymethyl)-azetidine (5.4 g).
¹H-NMR(CDCl₃)δ: 4.61(t,1H,J=3.6Hz), 3.99-3.77(m,6H), 3.57-3.48(m,2H), 3.15-3.01(m,1H), 1.89-1.50(m,6H).

### Example 1-24

### 6-(5-bromo-2-trifluoromethyl-phenyl)-4-(3,5-dimethyl-phenyl)-1,2,3,6-tetrahydro-pyrrolo[2,3-d]pyridazin-7-one

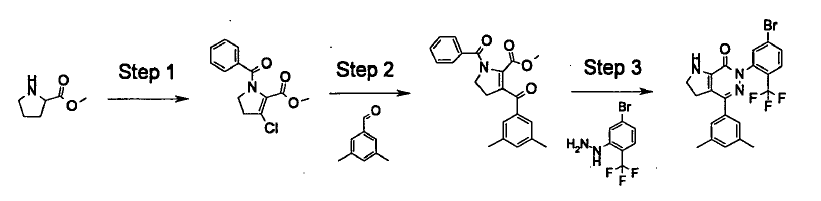

### Step 1

To a suspension of proline methyl ester hydrochloride (100 g) in chloroform (1 L) was added dropwise triethylamine (168 mL) under ice-cooling, and N-chloro-succinimide (84.6 g) was added by small portions over 30 min. After stirring at the same temperature for 1.5 hr, the inside temperature was raised to 45°C, and N-chloro-succinimide (84.6 g) was added over 50 min. After stirring at the same temperature for 20 min, benzoyl chloride (70.1 mL) was added. Then, the mixture was stirred under refluxing for 3 hr and allowed to return to room temperature, and water (500 mL) was added. The mixture was separated into two layers, and the aqueous layer was extracted with chloroform (300 mL). The combined organic layer was washed with an aqueous sodium hydrogencarbonate solution and brine, dried and concentrated. Diisopropyl ether was added and the mixture was crystallized to give methyl 1-benzoyl-3-chloro-4,5-dihydro-1H-pyrrole-2-carboxylate (90.0 g).
¹H-NMR(CDCl₃)δ: 7.62(m,2H), 7.53-7.48(m,1H), 7.43(m,2H), 4.11(t,2H,J=8.8Hz), 3.71(s,3H), 2.94(t,2H,J=8.8Hz). Step 2

To a solution of 3,5-dimethyl-benzaldehyde (5.0 g) in chloroform (60 mL) were added trimethylsilyl cyanide (5.96 mL) and triethylamine (0.519 mL). After stirring at 60°C for 2.5 hr, the reaction mixture was concentrated, and subjected to a toluene azeotropic operation. To a solution of the obtained resultant product and methyl 1-benzoyl-3-chloro-4,5-dihydro-1H-pyrrole-2-carboxylate (7.14 g) obtained in step 1 in tetrahydrofuran (70 mL) was added dropwise lithium diisopropylamide (2.0 M heptane-tetrahydrofuran-ethylbenzene solution; 18.7 mL) under argon at -18°C. After stirring at the same temperature for 30 min, acetic acid (4.6 mL) was added. After further stirring for 10 min, tetrabutylammonium fluoride (1 M tetrahydrofuran solution; 32 mL) was added. After stirring at room temperature for 1.5 hr, a saturated aqueous sodium hydrogencarbonate solution (40 mL) was added, and the mixture was extracted with ethyl acetate. The organic layer was washed with a 10% aqueous citric acid solution and brine, dried, concentrated and purified by silica gel column chromatography (ethyl acetate:hexane=1:4-1:2) to give methyl 1-benzoyl-3-(3,5-dimethyl-benzoyl)-4,5-dihydro-1H-pyrrole-2-carboxylate (7.61 g).
¹H-NMR(CDCl₃)δ: 7.64(d,2H,J=6.6Hz), 7.55-7.39(m,3H), 7.32(s,2H), 7.16(s,1H), 4.14(t,2H,J=8.9Hz), 3.22(s,3H), 3.11(t,2H,J=8.9Hz), 2.35(s,6H).

### Step 3

To a solution of methyl 1-benzoyl-3-(3,5-dimethylbenzoyl)-4,5-dihydro-1H-pyrrole-2-carboxylate (7.61 g) obtained in step 2 in 1-propanol (80 mL) were added (5-bromo-2-trifluoromethyl-phenyl)-hydrazine (5.88 g) obtained in Example 1-8, supplementary step 1 and trifluoroacetic acid (1.94 mL). After stirring at 85°C for 1.5 hr, the mixture was allowed to return to room temperature, potassium carbonate (6.96 g) was added. After stirring at 100°C for 19 hr, water (100 mL) was added, and the precipitated crystals were collected by filtration, and purified by silica gel column chromatography (ethyl acetate:hexane=1:4-2:1) to give 6-(5-bromo-2-trifluoromethyl-phenyl)-4-(3,5-dimethyl-phenyl)-1,2,3,6-tetrahydro-pyrrolo[2,3-d]pyridazin-7-one (6.47 g).
¹H-NMR(CDCl₃)δ: 7.72-7.65(m,3H), 7.18(s,2H), 7.03(s,1H), 4.85(s,1H), 3.85(t,2H,J=9.4Hz), 3.40-3.21(m,2H), 2.34(s,6H).

### Example 1-25

### methyl [6-(5-bromo-2-trifluoromethyl-phenyl)-4-(3,5-dimethyl-phenyl)-7-oxo-2,3,6,7-tetrahydro-pyrrolo[2,3-d]pyridazin-1-yl]-acetate

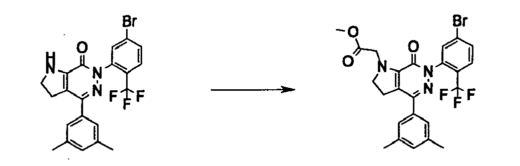

To a solution of 6-(5-bromo-2-trifluoromethylphenyl)-4-(3,5-dimethyl-phenyl)-1,2,3,6-tetrahydro-pyrrolo[2,3-d]pyridazin-7-one (1.0 g) obtained in Example 1-24 in N,N-dimethylformamide (10 mL) were added cesium carbonate (3.5 g) and methyl bromoacetate (0.98 mL), and the mixture was stirred at 50°C. After stirring for 3 hr, the mixture was allowed to return to room temperature, water was added, and the mixture was extracted with ethyl acetate. The organic layer was washed with water, dried, concentrated and purified by silica gel column chromatography (ethyl acetate:hexane=1:5-1:4) to give methyl [6-(5-bromo-2-trifluoromethyl-phenyl)-4-(3,5-dimethyl-phenyl)-7-oxo-2,3,6,7-tetrahydro-pyrrolo[2,3-d]pyridazin-1-yl]-acetate (1.15 g).
¹H-NMR(CDCl₃)δ: 7.71-7.61(m,3H), 7.14(s,2H), 7.02(s,1H), 4.57(s,2H), 3.78-3.70(m,2H), 3.72(s,3H), 3.27-3.08(m,2H), 2.33(s,6H)

### Example 1-26

### methyl [6-[5-((R)-3-acetylamino-pyrrolidin-1-yl)-2-trifluoromethyl-phenyl]-4-(3,5-dimethyl-phenyl)-7-oxo-2,3,6,7-tetrahydro-pyrrolo[2,3-d]pyridazin-1-yl]-acetate

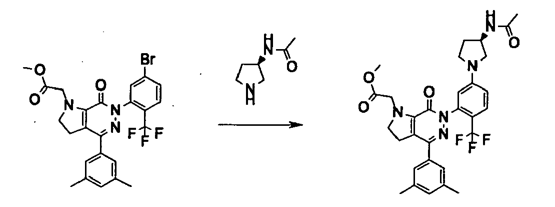

A suspension of tris(dibenzylideneacetone)dipalladium(0)-chloroform adduct (19 mg), 4,5-bis(diphenylphosphino)-9,9-dimethylxanthene (24 mg) and cesium carbonate (170 mg) in dioxane (1 mL) was stirred at room temperature under argon for 4 hr, then a solution of methyl [6-(5-bromo-2-trifluoromethyl-phenyl)-4-(3,5-dimethyl-phenyl)-7-oxo-2,3,6,7-tetrahydro-pyrrolo[2,3-d]pyridazin-1-yl]-acetate (200 mg) obtained in Example 1-25 and (R)-N-(pyrrolidin-3-yl)-acetamide (57 mg) in dioxane (1.6 mL)-tert-butanol (1.3 mL) were added, and the mixture was stirred at 100°C. After 2.5 hr, the insoluble material was filtered off, and the filtrate was concentrated. The obtained residue was purified by silica gel thin layer chromatography (chloroform:methanol=15:1) to give methyl [6-[5-((R)-3-acetylamino-pyrrolidin-1-yl)-2-trifluoromethyl-phenyl]-4-(3,5-dimethyl-phenyl)-7-oxo-2,3,6,7-tetrahydro-pyrrolo[2,3-d]pyridazin-1-yl]-acetate (199 mg).
¹H-NMR(CDCl₃)δ: 8.16(d,1H,J=7.0Hz), 7.57(d,1H,J=8.8Hz), 7.19(s,2H), 7.05(s,1H), 6.70(dd,1H,J=2.0,8.8Hz), 6.52(d,1H,J=2.0Hz), 4.53(m,2H), 4.33(m,1H), 3.67(m,2H), 3.62(s,3H), 3.53(m,2H), 3.43-3.34(m,2H), 3.21-3.11(m,2H), 2.29(s,6H), 2.16(m,1H), 1.90(m,1H), 1.80(s,3H)

### Example 1-27

### {6-[5-((R)-3-acetylamino-pyrrolidin-1-yl)-2-trifluoromethyl-phenyl]-4-(3,5-dimethyl-phenyl)-7-oxo-2,3,6,7-tetrahydro-pyrrolo[2,3-d]pyridazin-1-yl}-acetamide

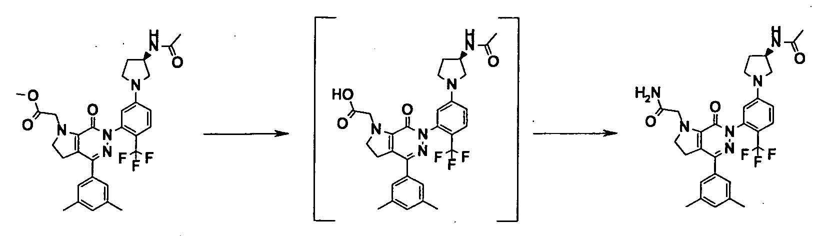

To a solution of methyl [6-[5-((R)-3-acetylamino-pyrrolidin-1-yl)-2-trifluoromethyl-phenyl]-4-(3,5-dimethyl-phenyl)-7-oxo-2,3,6,7-tetrahydro-pyrrolo[2,3-d]pyridazin-1-yl]-acetate (197 mg) obtained in Example 1-26 in methanol (2 mL)-tetrahydrofuran (1 mL) was added 1N aqueous sodium hydroxide solution (0.675 mL), and the mixture was stirred at room temperature for 1.5 hr. The mixture was neutralized with 1N hydrochloric acid, and extracted with ethyl acetate. The organic layer was washed with water, dried and concentrated to give a crude carboxylic acid (164 mg). To a solution of the carboxylic acid (100 mg) in N,N-dimethylformamide (1 mL) were successively added 1-ethyl-3-(3-dimethylaminopropyl)carbodiimide hydrochloride (44 mg), 1-hydroxybenzotriazole hydrate (31 mg), ammonium chloride (14 mg) and triethylamine (0.037 mL), and the mixture was stirred at room temperature for 20 hr. The mixture was diluted with ethyl acetate, and washed with an aqueous sodium hydrogencarbonate solution, water and brine. The organic layer was dried, concentrated, purified by silica gel thin layer chromatography (chloroform:methanol=10:1), and further crystallized from ethyl acetate to give {6-[5-((R)-3-acetylamino-pyrrolidin-1-yl)-2-trifluoromethyl-phenyl]-4-(3,5-dimethyl-phenyl)-7-oxo-2,3,6,7-tetrahydro-pyrrolo[2,3-d]pyridazin-1-yl}-acetamide (50 mg).
¹H-NMR(CDCl₃)δ: 7.60(d,1H,J=8.8Hz), 7.14(s,2H), 7.02(s,1H), 6.66(m,1H), 6.60(d,1H,J=9.0Hz), 6.53(d,1H,J=2.0Hz), 5.67(brs,1H), 5.30(brs,1H), 4.59(m,1H), 4.47(m,1H), 4.19(m,1H), 3.81(m,2H), 3.61(m,1H), 3.42(m,2H), 3.23(m,2H), 3.09(m,1H), 2.33(s,6H), 2.28(m,1H), 2.00(m,1H), 1.97(s,3H)

### Example 1-28

### 6-(5-fluoro-2-trifluoromethyl-phenyl)-4-(5-methoxy-pyridin-3-yl)-1,2,3,6-tetrahydro-pyrrolo[2,3-d]pyridazin-7-one

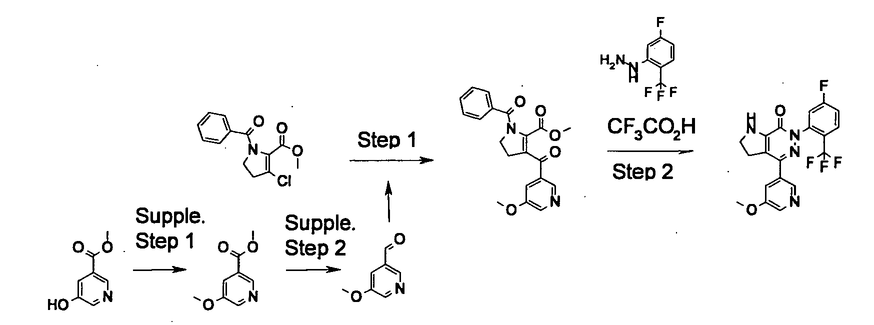

### Step 1

Methyl 1-benzoyl-3-chloro-4,5-dihydro-1H-pyrrole-2-carboxylate (718 mg) obtained in Example 1-24, step 1, and 5-methoxy-pyridine-3-carbaldehyde (371 mg) obtained in supplementary step 2 were reacted in the same manner as in Example 1-24, step 2, and purified by silica gel column chromatography (ethyl acetate:hexane=2:1) to give methyl 1-benzoyl-3-(5-methoxy-pyridine-3-carbonyl)-4,5-dihydro-1H-pyrrole-2-carboxylate (476 mg).
¹H-NMR(CDCl₃)δ: 8.48(d,1H,J=1.9Hz), 8.45(d,1H,J=3.0Hz), 7.65-7.63(m,2H), 7.53-7.51(m,1H), 7.47-7.43(m,3H), 4.16(t,2H,J=9.0Hz), 3.91(s,3H), 3.38(s,3H), 3.15(t,2H,J=8.9Hz).

### Step 2

Methyl 1-Benzoyl-3-(5-methoxy-pyridine-3-carbonyl)-4,5-dihydro-1H-pyrrole-2-carboxylate (476 mg) obtained in step 1 and (5-fluoro-2-trifluoromethyl-phenyl)-hydrazine trifluoroacetate (400 mg) obtained in Example 1-12, supplementary step 1, were reacted in the same manner as in Example 1-20 and purified by silica gel column chromatography (ethyl acetate:hexane=3:1-4:1) to give 6-(5-fluoro-2-trifluoromethyl-phenyl)-4-(5-methoxy-pyridin-3-yl)-1,2,3,6-tetrahydro-pyrrolo[2,3-d]pyridazin-7-one (336 mg).
¹H-NMR(DMSO-d₆)δ: 8.40(d,1H,J=1.5Hz), 8.35(d,1H,J=2.9Hz), 8.02(dd,1H,J=8.8,5.6Hz), 7.72(dd,1H,J=9.0,2.5Hz), 7.65-7.59(m,1H), 7.51(dd,1H,J=2.9,1.6Hz), 7.23(s,1H), 3.85(s,3H), 3.72(t,2H,J=10.3Hz), 3.31(t,2H,J=10.3Hz). Supplementary step 1

To a solution of methyl 5-hydroxy-3-pyridinecarboxylate (2.0 g) in N,N-dimethylformamide (20 mL) were added cesium carbonate (5.1 g) and methyl iodide (0.89 mL), and the mixture was stirred at room temperature. After 40 hr, the reaction mixture was concentrated, water was added, and the mixture was extracted with ethyl acetate. The organic layer was washed with water, dried, concentrated and purified by silica gel column chromatography (ethyl acetate:hexane=2:3-3:4) to give methyl 5-methoxy-3-pyridinecarboxylate (958 mg).
¹H-NMR(CDCl₃)δ: 8.83(d,1H,J=1.9Hz), 8.48(d,1H,J=3.0Hz), 7.77(dd,1H,J=2.9,1.7Hz), 3.96(s,3H), 3.91(s,3H). Supplementary step 2

To a solution of methyl 5-methoxy-3-pyridinecarboxylate (958 mg) obtained in supplementary step 1 in methanol (8 mL)-tetrahydrofuran (8 mL) were added lithium chloride (729 mg) and sodium borohydride (651 mg) under ice-cooling, and the mixture was stirred at room temperature. After 8.5 hr, an aqueous potassium hydrogen sulfate solution was added, and the mixture was extracted with ethyl acetate. The organic layer was washed with water, dried and concentrated and purified by silica gel column chromatography (ethyl acetate). The obtained resultant product (387 mg) was dissolved in chloroform (4 mL), 1,1,1-tris(acetyloxy)-1,1-dihydro-1,2-benzoiodoxol-3-(1H)-one (Dess-Martin reagent; 1.30 g) was added, and the mixture was stirred at room temperature. After 1.5 hr, ethanol (4 mL) was added. After stirring at room temperature for 40 min, the insoluble material was filtered off. The filtrate was concentrated and the obtained residue was purified by silica gel column chromatography (ethyl acetate:hexane=1:1) to give 5-methoxy-pyridine-3-carbaldehyde (371 mg).
¹H-NMR(CDCl₃)δ: 10.11(s,1H), 8.67(d,1H,J=1.9Hz), 8.56(d,1H,J=3.0Hz), 7.62(dd,1H,J=2.8,1.7Hz), 3.93(s,3H).

### Example 1-29

### 6-[5-((S)-3-hydroxy-pyrrolidin-1-yl)-2-trifluoromethylphenyl]-4-(5-methoxy-pyridin-3-yl)-7-oxo-2,3,6,7-tetrahydro-pyrrolo[2,3-d]pyridazine-1-carboxamide

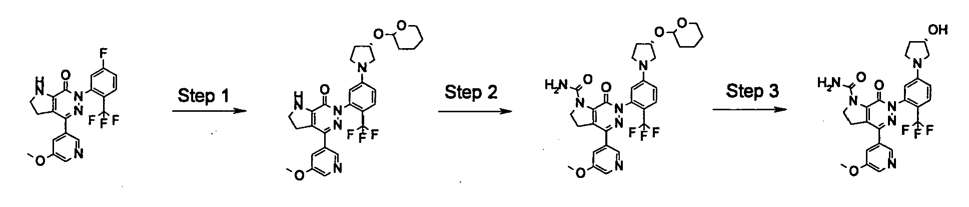

### Step 1

6-(5-Fluoro-2-trifluoromethyl-phenyl)-4-(5-methoxy-pyridin-3-yl)-1,2,3,6-tetrahydro-pyrrolo[2,3-d]pyridazin-7-one (100 mg) obtained in Example 1-28 and (S)-3-(tetrahydro-pyran-2-yloxy)-pyrrolidine (140 mg) were reacted in the same manner as in Example 1-23, step 1, and purified by silica gel thin layer chromatography (ethyl acetate:hexane=5:1) to give 4-(5-methoxy-pyridin-3-yl)-6-{5-[(S)-3-(tetrahydro-pyran-2-yloxy)-pyrrolidin-1-yl]-2-trifluoromethyl-phenyl}-1,2,3,6-tetrahydro-pyrrolo[2,3-d]pyridazin-7-one (52 mg).
¹H-NMR(CDCl₃)δ: 8.45(d,1H,J=1.6Hz), 8.32(d,1H,J=2.8Hz), 7.61-7.58(m,1H), 7.54-7.52(m,1H), 6.61(dd,1H,J=8.6,2.1Hz), 6.53-6.51(m,1H), 4.96(s,1H), 4.73-4.65(m,1H), 4.55-4.48(m,1H), 3.90-3.80(m,3H), 3.86(s,3H), 3.61-3.23(m,7H), 2.20-2.04(m,2H), 1.83-1.64(m,2H), 1.59-1.48(m,4H).

### Step 2

To a solution of 4-(5-methoxy-pyridin-3-yl)-6-{5-[(S)-3-(tetrahydro-pyran-2-yloxy)-pyrrolidin-1-yl]-2-trifluoromethyl-phenyl}-1,2,3,6-tetrahydro-pyrrolo[2,3-d]pyridazin-7-one (52 mg) obtained in step 1 in tetrahydrofuran (1 mL) were successively added triethylamine (0.018 mL), 4-dimethylamino-pyridine (1.1 mg) and triphosgene (11 mg) under nitrogen atmosphere and under ice-cooling, and the mixture was stirred under ice-cooling. After 30 min, triethylamine (0.018 mL) and triphosgene (11 mg) were added, and the mixture was further stirred at the same temperature for 30 min. Water was added, and the mixture was extracted with ethyl acetate. The organic layer was washed with water, dried, concentrated and purified by silica gel thin layer chromatography (ethyl acetate) to give 4-(5-methoxy-pyridin-3-yl)-7-oxo-6-{5-[(S)-3-(tetrahydropyran-2-yloxy)-pyrrolidin-1-yl]-2-trifluoromethylphenyl}-2,3,6,7-tetrahydro-pyrrolo[2,3-d]pyridazine-1-carboxamide (41 mg).
¹H-NMR(CDCl₃)δ: 8.42(d,1H,J=1.5Hz), 8.37(d,1H,J=2.6Hz), 7.61(d,1H,J=8.7Hz), 7.42-7.40(m,1H), 6.64(dd,1H,J=8.7,1.9Hz), 6.50(d,1H,J=2.3Hz), 4.73-4.65(m,1H), 4.57-4.49(m,1H), 4.43-4.23(m,2H), 3.90-3.82(m,1H), 3.88(s,3H), 3.64-3.03(m,7H), 2.29-2.06(m,2H), 1.89-1.68(m,2H), 1.60-1.47(m,4H).

### Step 3

To a solution of 4-(5-methoxy-pyridin-3-yl)-7-oxo-6-{5-[(S)-3-(tetrahydro-pyran-2-yloxy)-pyrrolidin-1-yl]-2-trifluoromethyl-phenyl}-2,3,6,7-tetrahydro-pyrrolo[2,3-d]pyridazine-1-carboxamide (41 mg) obtained in step 2 in methanol (1 mL) were added p-toluenesulfonic acid monohydrate (13 mg), and the mixture was stirred at room temperature for 5 hr. An aqueous sodium hydrogencarbonate solution was added, and the mixture was extracted with ethyl acetate. The organic layer was washed with water, dried and concentrated, and the obtained crude crystals were washed with a mixed solvent of ethyl acetate-hexane to give 6-[5-((S)-3-hydroxy-pyrrolidin-1-yl)-2-trifluoromethyl-phenyl]-4-(5-methoxy-pyridin-3-yl)-7-oxo-2,3,6,7-tetrahydro-pyrrolo[2,3-d]pyridazine-1-carboxamide (28 mg).
¹H-NMR(DMSO-d₆)δ: 8.40(d,1H,J=2.8Hz), 8.39(d,1H,J=1.9Hz), 7.62-7.59(m,1H), 7.55(dd,1H,J=2.6,1.9Hz), 6.74-6.71(m,2H), 5.05-4.98(m,1H), 4.43-4.39(m,1H), 4.15-4.08(m,2H), 3.87(s,3H), 3.47-3.16(m,6H), 2.10-1.89(m,2H).

### Example 1-30

### 6-(5-bromo-2-trifluoromethyl-phenyl)-4-(3,5-dimethyl-phenyl)-2-methyl-1,2,3,6-tetrahydro-pyrrolo[2,3-d]pyridazin-7-one

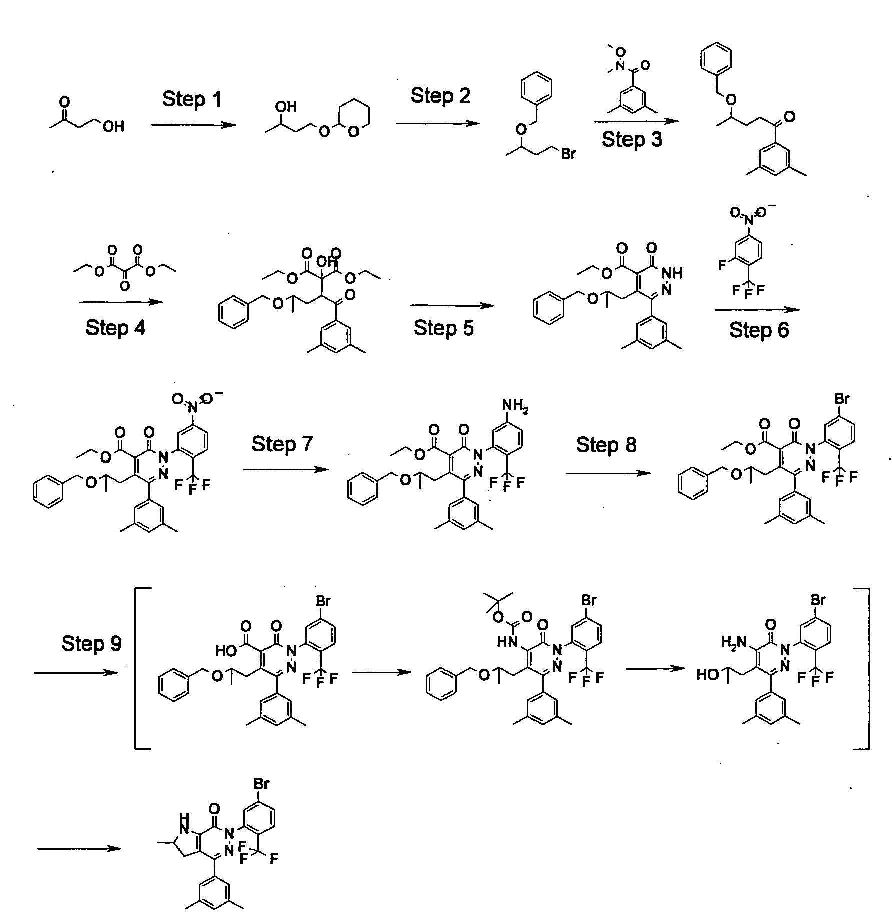

### Step 1

To a solution of 4-hydroxy-butane-2-one (14.7 g) in chloroform (150 mL) were added dihydropyran (16.7 mL) and camphorsulfonic acid (1.94 g), and the mixture was stirred at room temperature for 15 hr. An aqueous sodium hydrogencarbonate solution was added, and extracted with chloroform. The organic layer was washed with water, dried and concentrated. The obtained crude product was dissolved in ethanol (200 mL), sodium borohydride (3.16 g) was added under ice-cooling, and the mixture was stirred at the same temperature. After 20 min, a saturated aqueous ammonium chloride solution was added, and the mixture was extracted with ethyl acetate. The organic layer was washed with water, dried, concentrated and purified by silica gel column chromatography (ethyl acetate:hexane=1:4-1:1) to give 4-(tetrahydro-pyran-2-yloxy)-2-butanol (24.2 g).
¹H-NMR(CDCl₃)δ: 4.62-4.60(m,1H), 4.07-3.83(m,3H), 3.61-3.55(m,2H), 1.84-1.66(m,4H), 1.64-1.48(m,4H), 1.22(d,3H,J=6.5Hz).

### Step 2

To a solution of 4-(tetrahydro-pyran-2-yloxy)-2-butanol (24.2 g) obtained in step 1 in N,N-dimethylformamide (100 mL) was added 60% sodium hydride (6.7 g) under ice-cooling, and the mixture was stirred for 20 min. Benzyl bromide (18 mL) was added and, after stirring at room temperature for 1 hr, ice water was added, and the mixture was extracted with diethyl ether. The organic layer was washed with water, dried and concentrated. The obtained crude product was dissolved in methanol (100 mL)-tetrahydrofuran (10 mL), and camphorsulfonic acid (2.0 g) was added. After stirring at room temperature for 2 hr, an aqueous sodium hydrogencarbonate solution was added, and the mixture was extracted with ethyl acetate. The organic layer was washed with water, dried, concentrated and dissolved in N,N-dimethylformamide (70 mL) under ice-cooling, to which triphenylphosphine dibromide (61.6 g) was added by small portions. After stirring at room temperature for 1.5 hr, water (100 mL), ethyl acetate (100 mL) and hexane (60 mL) were added to separate the organic layer. The organic layer was washed with water, dried and concentrated. The obtained residue was purified by silica gel column chromatography (ethyl acetate:hexane=1:20) to give (3-bromo-1-methyl-propoxymethyl)-benzene (27 g).
¹H-NMR(CDCl₃)δ: 7.38-7.27(m,5H), 4.61(d,1H,J=11.7Hz), 4.45(d,1H,J=11.7Hz), 3.80-3.70(m,1H), 3.60-3.45(m,2H), 2.17-2.06(m,1H), 2.01-1.89(m,1H), 1.23(d,3H,J=6.0Hz).

### Step 3

A solution of (3-bromo-1-methyl-propoxymethyl)-benzene (22.4 g) obtained in step 2 in tetrahydrofuran (80 mL) was added dropwise to magnesium (2.17 g) under argon over 1 hr. The mixture was stirred at room temperature for 1 hr, and added dropwise to a solution of N-methoxy-3,5,N-trimethyl-benzamide (13.2 g) cooled to -70°C in tetrahydrofuran (200 mL) over 10 min. The mixture was allowed to return to room temperature and stirred for 1 hr. 1N hydrochloric acid (80 mL) was added, and the mixture was extracted with ethyl acetate. The organic layer was washed with water, dried, concentrated and purified by silica gel column chromatography (ethyl acetate:hexane=1:15-1:10) to give 4-benzyloxy-1-(3,5-dimethylphenyl)-1-pentanone (18.8 g).
¹H-NMR(CDCl₃)δ: 7.56(s,2H), 7.37-7.25(m,5H), 7.19(s,1H), 4.60(d,1H,J=12.1Hz), 4.44(d,1H,J=12.1Hz), 3.69-3.60(m,1H), 3.08-3.02(m,2H), 2.36(s,6H), 2.05-1.87(m,2H), 1.27(d,3H,J=5.6Hz).

### Step 4

To a solution of 4-benzyloxy-1-(3,5-dimethylphenyl)-1-pentanone (18.8 g) obtained in step 3 in tetrahydrofuran (100 mL) was added dropwise lithium diisopropylamide (2M heptane-tetrahydrofuran-ethylbenzene solution; 38 mL) under argon at -70°C. After stirring at the same temperature for 1 hr, the mixture was added to a solution of diethyl ketomalonate (11.6 mL) in tetrahydrofuran (200 mL) cooled to -70°C. After stirring at the same temperature for 1.5 hr, a saturated aqueous ammonium chloride solution was added, and the mixture was allowed to return to room temperature. The mixture was extracted with ethyl acetate and the organic layer was washed with water, dried, concentrated and purified by silica gel column chromatography (ethyl acetate:hexane=1:10-1:3) to give diethyl 2-[3-benzyloxy-1-(3,5-dimethyl-benzoyl)-butyl]-2-hydroxy-malonate (23.85 g).
¹H-NMR(CDCl₃)δ: 7.60(s,2H), 7.36-7.11(m,6H), 4.99(d,1H,J=22.3Hz), 4.86-4.62(m,1H), 4.47-4.24(m,3H), 4.16-3.87(m,3H), 3.55-3.27(m,1H), 2.21(s,6H*0.7), 2.19(s,6H*0.3), 2.06-2.03(m,1H), 1.36-1.31(m,3H), 1.18-1.09(m,6H).

### Step 5

To a solution of diethyl 2-[3-benzyloxy-1-(3,5-dimethyl-benzoyl)-butyl]-2-hydroxy-malonate (23.85 g) obtained in step 4 in ethanol (110 mL) was added hydrazine monohydrate (3.2 mL), and the mixture was stirred with heating at 55°C. After 12 hr, methanesulfonic acid (5.26 mL) was added, and the mixture was stirred under refluxing. After 10 hr, the mixture was allowed to return to room temperature, and neutralized with an aqueous sodium hydrogencarbonate solution. Ethanol was evaporated, and the mixture was extracted with ethyl acetate. The organic layer was washed with water, dried, concentrated and purified by silica gel column chromatography (ethyl acetate:hexane=1:2-2:1) to give ethyl 5-(2-benzyloxypropyl)-6-(3,5-dimethylphenyl)-3-oxo-2,3-dihydro-pyridazine-4-carboxylate (11.2 g).
¹H-NMR(CDCl₃)δ: 11.32(brs,1H), 7.31-7.27(m,3H), 7.13-7.12(m,2H), 7.03(s,1H), 6.81(s,2H), 4.41(dq,2H,J=1.5,7.2Hz), 4.26(d,1H,J=12.1Hz), 4.13(d,1H,J=12.1Hz), 3.33(ddq,1H,J=7.9,5.7,6.0Hz), 3.01(dd,1H,J=13.2,7.9Hz), 2.62(dd,1H,J=13.2,5.7Hz), 2.31(s,6H), 1.37(t,3H,J=7.2Hz), 0.99(d,3H,J=6.0Hz). Step 6

Ethyl 5-(2-benzyloxy-propyl)-6-(3,5-dimethylphenyl)-3-oxo-2,3-dihydro-pyridazine-4-carboxylate (11.2 g) obtained in step 5 and 2-fluoro-4-nitro-1-trifluoromethyl-benzene (10.8 g) obtained in Example 1-1, supplementary step 2 were reacted in the same manner as in Example 1-1, step 4, and purified by silica gel column chromatography (ethyl acetate:hexane=1:5-1:4) to give ethyl 5-(2-benzyloxypropyl)-6-(3,5-dimethylphenyl)-2-(5-nitro-2-trifluoromethyl-phenyl)-3-oxo-2,3-dihydro-pyridazine-4-carboxylate (11.2 g).
¹H-NMR(CDCl₃)δ: 8.45-8.41(m,2H), 8.02(d,1H,J=9.3Hz), 7.37-7.30(m,3H), 7.23-7.19(m,2H), 7.04(s,1H), 6.89(s,2H), 4.46-4.35(m,2H), 4.32(d,1H,J=12.1Hz), 4.22(d,1H,J=12.1Hz), 3.46(ddq,1H,J=8.3,5.3,6.0Hz), 3.08(dd,1H,J=13.4,8.3Hz), 2.71(dd,1H,J=13.4,5.3Hz), 1.37(t,3H,J=7.0Hz), 1.06(d,3H,J=6.0Hz).

### Step 7

Ethyl 5-(2-benzyloxy-propyl)-6-(3,5-dimethylphenyl)-2-(5-nitro-2-trifluoromethyl-phenyl)-3-oxo-2,3-dihydro-pyridazine-4-carboxylate (11.2 g) obtained in step 6 was reacted in the same manner as in Example 1-2 and purified by silica gel column chromatography (ethyl acetate:hexane=1:1-2:1) to give ethyl 2-(5-amino-2-trifluoromethyl-phenyl)-5-(2-benzyloxy-propyl)-6-(3,5-dimethylphenyl)-3-oxo-2,3-dihydro-pyridazine-4-carboxylate (10.65 g).
¹H-NMR(CDCl₃)δ: 7.51(d,1H,J=8.3Hz), 7.34-7.26(m,3H), 7.20-7.15(m,2H), 7.00(s,1H), 6.92(s,2H), 6.72-6.69(m,2H), 4.44-4.31(m,2H), 4.23(brs,2H), 3.43(ddq,1H,J=7.5,5.7,6.4Hz), 3.08(dd,1H,J=13.2,7.5Hz), 2.67(dd,1H,J=13.2,5.7Hz), 2.28(s,6H), 1.34(t,3H,J=7.2Hz), 1.03(d,3H,J=6.4Hz).

### Step 8

Ethyl 2-(5-amino-2-trifluoromethyl-phenyl)-5-(2-benzyloxy-propyl)-6-(3,5-dimethylphenyl)-3-oxo-2,3-dihydro-pyridazine-4-carboxylate (10.65 g) obtained in step 7 was reacted in the same manner as in Example 1-3 and purified by silica gel column chromatography (ethyl acetate:hexane=1:4) to give ethyl 5-(2-benzyloxypropyl)-2-(5-bromo-2-trifluoromethyl-phenyl)-6-(3,5-dimethylphenyl)-3-oxo-2,3-dihydro-pyridazine-4-carboxylate (4.51 g).
¹H-NMR(CDCl₃)δ: 7.73-7.63(m,3H), 7.38-7.27(m,3H), 7.19-7.18(m,2H), 7.02(s,1H), 6.90(s,2H), 4.40-4.38(m,2H), 4.24(q,2H,J=11.1Hz), 3.44(ddq,1H,J=7.9,5.6,6.0Hz), 3.07(dd,1H,J=13.4,7.9Hz), 2.68(dd,1H,J=13.4,5.6Hz), 2.27(s,6H), 1.36(t,3H,J=7.0Hz), 1.04(d,3H,J=6.0Hz). Step 9

To a solution of ethyl 5-(2-benzyloxy-propyl)-2-(5-bromo-2-trifluoromethyl-phenyl)-6-(3,5-dimethylphenyl)-3-oxo-2,3-dihydro-pyridazine-4-carboxylate (4.51 g) obtained in step 8 in tetrahydrofuran (20 mL)-ethanol (40 mL) was added a 1N aqueous sodium hydroxide solution (7.7 mL), and the mixture was stirred at room temperature for 30 min, and at 60°C for 3 hrs. The mixture was allowed to return to room temperature, and neutralized with 1N hydrochloric acid. Tetrahydrofuran-ethanol was evaporated and the mixture was extracted with ethyl acetate. The organic layer was washed with water, dried and concentrated. To a solution of the obtained residue (4.2 g) in dioxane (45 mL)-tert-butanol (20 mL) were successively added triethylamine (2.27 mL) and diphenylphosphoryl azide (1.76 mL). After stirring at room temperature for 30 min, the temperature was raised to 100°C, and the mixture was stirred with heating for 6 hr. The mixture was allowed to return to room temperature, water was added, and the mixture was extracted with ethyl acetate. The organic layer was washed with water, dried, concentrated, and purified by silica gel column chromatography (ethyl acetate:hexane=1:8-1:4). The obtained resultant product (4.9 g) was dissolved in chloroform (50 mL). Methanesulfonic acid (5.0 mL) was added and, after stirring at room temperature for 1.5 hr, methanesulfonic acid (5.0 mL) was further added. After stirring the reaction mixture at room temperature for 20 hr, a saturated aqueous sodium hydrogencarbonate solution was added by small portions, and the mixture was extracted with ethyl acetate. The organic layer was washed with water, dried, concentrated, and purified by silica gel column chromatography (ethyl acetate:hexane=1:4-1:1). The obtained resultant product (1.8 g) was dissolved in chloroform (30 mL). Thionyl chloride (0.31 mL) was added, and the mixture was stirred at 60°C. After 2 hr, the reaction mixture was concentrated and the obtained residue was purified by silica gel column chromatography (ethyl acetate:hexane=1:3). The obtained resultant product (1.71 g) was dissolved in N,N-dimethylformamide (20 mL), potassium tert-butoxide (410 mg) was added, and the mixture was stirred at room temperature. After 1 hr, water was added, and the mixture was extracted with ethyl acetate. The organic layer was washed with water, dried, concentrated and purified by silica gel column chromatography (toluene:ethyl acetate=8:1) to give 6-(5-bromo-2-trifluoromethyl-phenyl)-4-(3,5-dimethyl-phenyl)-2-methyl-1,2,3,6-tetrahydro-pyrrolo[2,3-d]pyridazin-7-one (375 mg).
¹H-NMR(CDCl₃)δ: 7.71-7.67(m,3H), 7.18(s,2H), 7.04(s,1H), 4.87(brs,1H), 4.32-4.28(m,1H), 3.57-3.34(m,1H), 2.98-2.81(m,1H), 2.35(s,6H), 1.38(brs,3H).

### Example 1-31

### 1-acetyl-6-(5-bromo-2-trifluoromethyl-phenyl)-4-(3,5-dimethyl-phenyl)-2-methyl-1,2,3,6-tetrahydro-pyrrolo[2,3-d]pyridazin-7-one

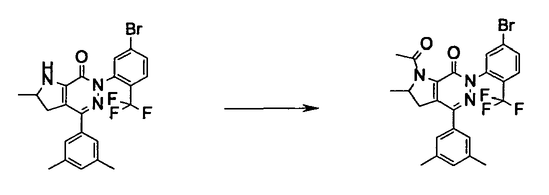

To a solution of 6-(5-bromo-2-trifluoromethylphenyl)-4-(3,5-dimethyl-phenyl)-2-methyl-1,2,3,6-tetrahydro-pyrrolo[2,3-d]pyridazin-7-one (285 mg) obtained in Example 1-30 in acetic acid (3.0 mL) was added acetyl chloride (0.13 mL), and the mixture was stirred at room temperature for 3 hrs. The reaction mixture was concentrated, and the obtained residue was purified by silica gel column chromatography (ethyl acetate:hexane=1:5) to give 1-acetyl-6-(5-bromo-2-trifluoromethyl-phenyl)-4-(3,5-dimethyl-phenyl)-2-methyl-1,2,3,6-tetrahydro-pyrrolo[2,3-d]pyridazin-7-one (250 mg).
¹H-NMR(CDCl₃)δ: 7.75-7.70(m,3H), 7.15(s,2H), 7.09(s,1H), 5.07-4.95(m,1H), 3.64-3.49(m,1H), 2.49-2.31(m,10H), 1.29(brs,3H).

### Example 1-32

### 1-acetyl-4-(3,5-dimethyl-phenyl)-6-[5-((S)-3-hydroxy-pyrrolidin-1-yl)-2-trifluoromethyl-phenyl]-2-methyl-1,2,3,6-tetrahydro-pyrrolo[2,3-d]pyridazin-7-one

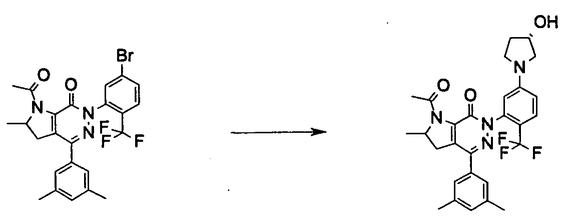

1-Acetyl-6-(5-bromo-2-trifluoromethyl-phenyl)-4-(3,5-dimethyl-phenyl)-2-methyl-1,2,3,6-tetrahydro-pyrrolo[2,3-d]pyridazin-7-one (130 mg) obtained in Example 1-31 and (S)-3-(tert-butyl-diphenyl-silanyloxy)-pyrrolidine (121 mg) were reacted in the same manner as in Example 1-26 and the obtained resultant product (115 mg) was dissolved in tetrahydrofuran (5 mL). Tetrabutylammonium fluoride hydrate (128 mg) was added and, after stirring at room temperature for 30 min, water was added, and the mixture was extracted with ethyl acetate. The organic layer was washed with water, dried, concentrated and purified by silica gel column chromatography (ethyl acetate:hexane=1:3-ethyl acetate) to give 1-acetyl-4-(3,5-dimethyl-phenyl)-6-[5-((S)-3-hydroxy-pyrrolidin-1-yl)-2-trifluoromethyl-phenyl]-2-methyl-1,2,3,6-tetrahydro-pyrrolo[2,3-d]pyridazin-7-one (46 mg).
¹H-NMR(CDCl₃)δ: 7.60(d,1H,J=6.4Hz), 7.17(s,2H), 7.06(s,1H), 6.66-6.51(m,2H), 5.05-4.92(m,1H), 4.60(s,1H), 3.59-3.30(m,5H), 2.59-2.26(m,10H), 2.22-2.00(m,2H), 1.85(brs,1H), 1.35-1.19(m,3H).

### Example 1-33

### 4-(3,5-dimethyl-phenyl)-6-[5-((S)-3-hydroxy-pyrrolidin-1-yl)-2-trifluoromethyl-phenyl]-2-methyl-1,2,3,6-tetrahydro-pyrrolo[2,3-d]pyridazin-7-one

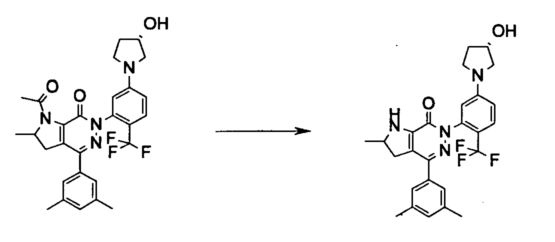

To a solution of 1-acetyl-4-(3,5-dimethyl-phenyl)-6-[5-((S)-3-hydroxy-pyrrolidin-1-yl)-2-trifluoromethylphenyl]-2-methyl-1,2,3,6-tetrahydro-pyrrolo[2,3-d]pyridazin-7-one (61 mg) obtained in Example 1-32 in ethanol (2 mL) was added hydrazine monohydrate (0,20 mL), and the mixture was stirred at room temperature. After 2 hr, water was added, and the mixture was extracted with ethyl acetate. The organic layer was washed with a aqueous potassium hydrogen sulfate solution, dried, concentrated and purified by silica gel thin layer chromatography (chloroform :methanol=10:1) to give 4-(3,5-dimethyl-phenyl)-6-[5-((S)-3-hydroxy-pyrrolidin-1-yl)-2-trifluoromethyl-phenyl]-2-methyl-1,2,3,6-tetrahydro-pyrrolo[2,3-d]pyridazin-7-one (29 mg).
¹H-NMR(CDCl₃)δ: 7.58(d,1H,J=9.0Hz), 7.21-7.18(m,2H), 7.01(s,1H), 6.58(dd,1H,J=9.0,1.9Hz), 6.52(d,1H,J=1.9Hz), 4.93(br,1H), 4.58-4.51(m,1H), 4.33-4.20(m,1H), 3.55-3.22(m,5H), 2.89(brs,1H), 2.33(s,6H), 2.17-2.01(m,2H), 1.36(br,3H).

### Example 1-34

### 5-(5-bromo-2-trifluoromethyl-phenyl)-7-(3,5-dimethoxy-phenyl)-3,5-dihydro-2H-furo[2,3-d]pyridazin-4-one

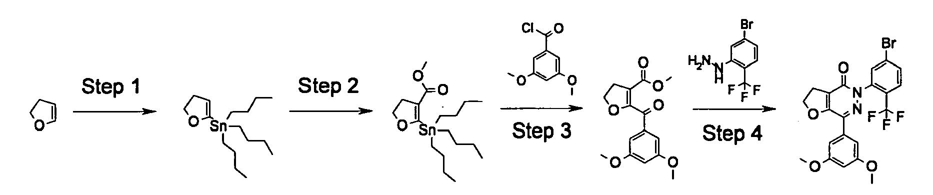

### Step 1

To a solution of 2,3-dihydrofuran (2.0 mL) in tetrahydrofuran (53 mL) was added dropwise tert-butyllithium (1.6M pentane solution; 18.2 mL) under argon at -78°C. After dropwise addition, the temperature was raised to 0°C over 40 min. The mixture was cooled to -78°C again, and tributyltin chloride (7.17 mL) was added. The cooling bath was removed, and the mixture was stirred for 1 hr. Water was added, and the mixture was extracted with diethyl ether. The organic layer was dried, concentrated and purified by silica gel column chromatography (ethyl acetate:hexane=1:100-1:1) to give tributyl-(4,5-dihydro-furan-2-yl)-stannane (5.48 g).
¹H-NMR(CDCl₃)δ: 5.06(t,1H,J=2.6Hz), 4.23(t,2H,J=9.5Hz), 2.57(td,2H,J=9.5,2.5Hz), 1.57-1.50(m,6H), 1.38-1.29(m,6H), 1.01-0.96(m,6H), 0.90(t,9H,J=7.3Hz).

### Step 2

To a solution of tributyl-(4,5-dihydro-furan-2-yl)-stannane (5.48 g) obtained in step 1 in methylene chloride (55 mL) were added diisopropylethylamine (0.266 mL) and trichloroacetyl chloride (1.82 mL) under ice-cooling. The cooling bath was removed and, after stirring for 3 hr, the reaction mixture was poured into an aqueous sodium hydrogencarbonate solution. The mixture was extracted with chloroform, and the organic layer was washed with water, dried, concentrated, and purified by silica gel column chromatography (ethyl acetate:hexane=1:20). The obtained resultant product (2.83 g) was dissolved in methanol (450 mL), and triethylamine (50 mL) was added. After stirring at room temperature for 16 hr, the reaction mixture was concentrated. Diluted hydrochloric acid was added to the obtained residue, and the mixture was extracted with ethyl acetate. The organic layer was dried and concentrated to give methyl 2-tributylstannyl-4,5-dihydro-furan-3-carboxylate (2.21 g).
¹H-NMR(CDCl₃)δ: 4.49(dd,2H,J=9.4,10.2Hz), 3.70(s,3H), 2.78(dd,2H,J=9.4,10.2Hz), 1.57-1.46(m,6H), 1.37-1.26(m,6H), 1.14-1.02(m,6H), 0.89(t,9H,J=7.2Hz).

### Step 3

To a solution of methyl 2-tributylstannyl-4,5-dihydro-furan-3-carboxylate (417 mg) obtained in step 2 in dioxane (2 mL) were added 3,5-dimethoxybenzoyl chloride (200 mg) and bis(triphenylphosphine)palladium (II) dichloride (35 mg) under argon. After stirring at 100°C for 40 min, copper(I) iodide (9.5 mg) was added, and the mixture was continuously stirred at the same temperature for 40 min. The mixture was allowed to return to room temperature, the insoluble material was filtered through celite, and the filtrate was concentrated. The residue was purified by silica gel column chromatography (ethyl acetate:hexane=1:3-1:2) to give methyl 2-(3,5-dimethoxy-benzoyl)-4,5-dihydrofuran-3-carboxylate (291 mg).
¹H-NMR(CDCl₃)δ: 7.09(d,2H,J=2.3Hz), 6.71(t,1H,J=2.3Hz), 4.73(t,2H,J=9.9Hz),3.85(s,6H), 3.56(s,3H), 3.12(t,2H,J=9.9Hz).

### Step 4

To a solution of methyl 2-(3,5-dimethoxy-benzoyl)-4,5-dihydro-furan-3-carboxylate (291 mg) obtained in step 3 and (5-bromo-2-trifluoromethyl-phenyl)-hydrazine (330 mg) obtained in Example 1-8, supplementary step 1, in 1-butanol (1.5 mL) was added trifluoroacetic acid (0.030 mL). After stirring at 70°C for 1.5 hr, 1-butanol (1.5 mL) and cesium carbonate (812 mg) were added, and the mixture was continuously stirred at the same temperature. After 1.5 hr, the mixture was allowed to return to room temperature, water was added, and the mixture was extracted with ethyl acetate. The organic layer was washed with water, dried, concentrated, and purified by silica gel column chromatography (ethyl acetate:hexane=1:9-2:3). The obtained crude crystals were washed with a mixed solvent of ethyl acetate-hexane was to give 5-(5-bromo-2-trifluoromethyl-phenyl)-7-(3,5-dimethoxy-phenyl)-3,5-dihydro-2H-furo[2,3-d]pyridazin-4-one (128 mg).
¹H-NMR(CDCl₃)δ: 7.77-7.65(m,3H), 7.10(d,2H,J=2.3Hz), 6.52(t,1H,J=2.3Hz), 4.90(t,2H,J=9.6Hz), 3.81(s,6H), 3.29(t,2H,J=9.6Hz).

### Example 1-35

### 7-(3,5-dimethoxy-phenyl)-5-[5-((S)-3-hydroxy-pyrrolidin-1-yl)-2-trifluoromethyl-phenyl]-3,5-dihydro-2H-furo[2,3-d]pyridazin-4-one

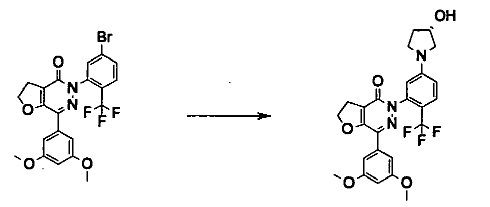

5-(5-Bromo-2-trifluoromethyl-phenyl)-7-(3,5-dimethoxy-phenyl)-3,5-dihydro-2H-furo[2,3-d]pyridazin-4-one (128 mg) obtained in Example 1-34 and (S)-3-hydroxy-pyrrolidine (0.026 mL) were reacted in the same manner as in Example 1-17 and crystallized from chloroform-ethyl acetate to give 7-(3,5-dimethoxy-phenyl)-5-[5-((S)-3-hydroxy-pyrrolidin-1-yl)-2-trifluoromethyl-phenyl]-3,5-dihydro-2H-furo[2,3-d]pyridazin-4-one (108 mg).
¹H-NMR(DMSO-d₆)δ: 7.61(d,1H,J=8.8Hz), 6.99(d,2H,J=2.1Hz), 6.73 (dd,1H,J=2.1,8.8Hz), 6.62(brs, 2H), 5.04(d,1H,J=3.7Hz), 4.90(t,2H,J=10.0Hz), 4.42(brs,1H), 3.76(s,6H), 3.49-3.34(m,3H), 3.20-3.15(m,1H), 3.16(t,2H,J=10.0Hz), 2.11-2.00(m,1H), 1.97-1.89(m,1H).

### Example 1-36

### 7-(3,5-dimethoxy-phenyl)-5-(5-fluoro-2-trifluoromethylphenyl)-3,5-dihydro-2H-thieno[2,3-d]pyridazin-4-one

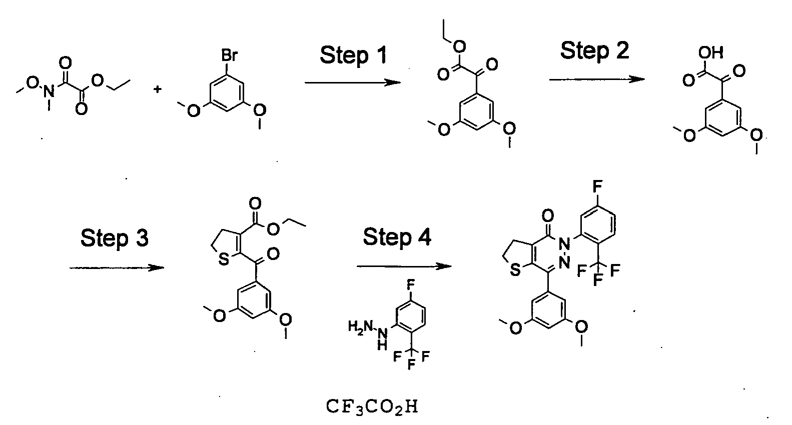

### Step 1

To a solution of ethyl chloroglyoxylate (10.0 g) in methylene chloride (120 mL) were added N,O-dimethylhydroxylamine hydrochloride and triethylamine (17.1 mL) under ice-cooling. After stirring at room temperature for 30 min, the reaction mixture was concentrated. Tetrahydrofuran (40 mL) was added to the residue, and the insoluble material was filtered off. The filtrate was concentrated to give ethyl 1-(N-methoxy-N-methyl-amino)-1-oxo-acetate (11.3 g). To a solution of 1-bromo-3,5-dimethoxybenzene (5.0 g) in tetrahydrofuran (50 mL) was added dropwise n-butyllithium (2.71 M hexane solution; 8.23 mL) under argon at -78°C. After stirring at the same temperature for 20 min, the mixture was added dropwise over 10 min to a solution of ethyl 1-(N-methoxy-N-methyl-amino)-1-oxo-acetate (11.3 g) in tetrahydrofuran (100 mL) cooled to -78°C. After stirring at the same temperature for 30 min, 1N hydrochloric acid (100 mL) was added, and the mixture was extracted with ethyl acetate. The organic layer was washed with water, dried, concentrated and purified by silica gel column chromatography (ethyl acetate:hexane=1:10-1:5) to give ethyl (3,5-dimethoxy-phenyl)-oxo-acetate (1.3 g). ¹H-NMR(CDCl₃)δ: 7.13(d,2H,J=2.2Hz), 6.73(t,1H,J=2.2Hz), 4.45(q,2H,J=7.2Hz), 3.84(s,6H), 1.42(t,3H,J=7.2Hz).

### Step 2

To a solution of ethyl (3,5-dimethoxy-phenyl)-oxo-acetate (1.2 g) obtained in step 1 in tetrahydrofuran (15 mL)-methanol (15 mL)-water (30 mL) was added dropwise a 0.5 M aqueous sodium hydroxide solution (5 mL) while maintaiing the conditions of pH of not more than 10.5 and 0°C or below. After completion of the dropwise addition, the mixture was extracted with diethyl ether. The aqueous layer was adjusted to pH 1 with 1N hydrochloric acid, and extracted twice with diethyl ether. The organic layer was washed with water, dried and concentrated to give (3,5-dimethoxy-phenyl)-oxo-acetic acid (900 mg).
¹H-NMR(CDCl₃)δ: 7.48(d,2H,J=2.3Hz), 6.79(t,1H,J=2.3Hz), 6.10(brs,1H), 3.86(s,6H).

### Step 3

To a suspension of (3,5-dimethoxy-phenyl)-oxo-acetic acid (450 mg) obtained in step 2 in toluene (5 mL) were added triethylamine (0.36 mL) and thionyl chloride (0.18 mL) under ice-cooling. After stirring at room temperature for 20 min, the insoluble material was filtered off, and the filtrate was concentrated. The residue was dissolved in tetrahydrofuran (3 mL) and added to a solution of 1-carboethoxycyclopropyltriphenylphosphonium tetrafluoroborate (970 mg) and sodium sulfide (161 mg) in tetrahydrofuran (8 mL) under ice-cooling. After stirring at room temperature for 30 min, the mixture was stirred with heating under refluxing for 48 hr. The insoluble material was filtered off, and purified by silica gel column chromatography (ethyl acetate:hexane=1:20-1:4) to give ethyl 2-(3,5-dimethoxy-benzoyl)-4,5-dihydro-thiophene-3-carboxylate (47 mg).
¹H-NMR(CDCl₃)δ: 7.11(d,2H,J=2.2Hz), 6.68(t,2H,J=2.2Hz), 3.96(q,2H,J=7.1Hz), 3.83(s,6H), 3.53-3.46(m,2H), 3.32-3.25(m,2H), 0.97(t,3H,J=7.1Hz).

### Step 4

Ethyl 2-(3,5-dimethoxy-benzoyl)-4,5-dihydrothiophene-3-carboxylate (45 mg) obtained in step 3 and (5-fluoro-2-trifluoromethyl-phenyl)-hydrazine trifluoroacetate (100 mg) obtained in Example 1-12, supplementary step 2, were reacted in the same manner as in Example 1-8, step 3, and purified by silica gel thin layer chromatography (ethyl acetate:hexane=1:1) to give 7-(3,5-dimethoxy-phenyl)-5-(5-fluoro-2-trifluoromethyl-phenyl)-3,5-dihydro-2H-thieno[2,3-d]pyridazin-4-one (25 mg).
¹H-NMR(CDCl₃)δ: 7.83(dd,1H,J=8.6,5.8Hz), 7.34-7.20(m,2H), 6.83(d,2H,J=2.0Hz), 6.52(t,1H,J=2.0Hz), 3.81(s,6H), 3.55-3.44(m,4H).

### Example 1-37

### 7-(3,5-dimethoxy-phenyl)-5-[5-((S)-3-hydroxy-pyrrolidin-1-yl)-2-trifluoromethyl-phenyl]-3,5-dihydro-2H-thieno[2,3-d]pyridazin-4-one

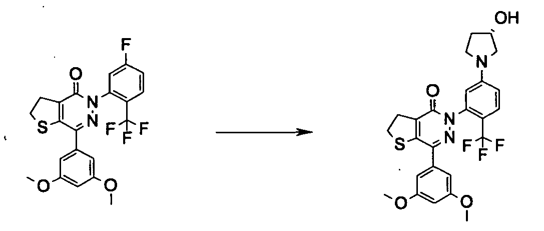

7-(3,5-Dimethoxy-phenyl)-5-(5-fluoro-2-trifluoromethyl-phenyl)-3,5-dihydro-2H-thieno[2,3-d]pyridazin-4-one (23 mg) obtained in Example 1-36 and (S)-3-hydroxy-pyrrolidine (0.006 mL) were reacted in the same manner as in Example 1-23, step 1, purified by silica gel thin layer chromatography (ethyl acetate) to give 7-(3,5-dimethoxy-phenyl)-5-[5-((S)-3-hydroxy-pyrrolidin-1-yl)-2-trifluoromethyl-phenyl]-3,5-dihydro-2H-thieno[2,3-d]pyridazin-4-one (5 mg), and 7-(3,5-dimethoxy-phenyl)-5-[5-((S)-3-hydroxy-pyrrolidin-1-yl)-2-trifluoromethyl-phenyl]-2H-thieno[2,3-d]pyridazin-4-one (3.7 mg).
¹H-NMR(CDCl₃)δ: 7.59(d,1H,J=8.8Hz), 6.86(d,2H,J=2.3Hz), 6.61(dd,1H,J=8.8,2.1Hz), 6.53(d,1H,J=2.1Hz), 6.50(t,1H,J=2.3Hz), 4.61(brs,1H), 3.81(s,6H), 3.58-3.37(m,7H), 3.34-3.26(m,1H), 2.22-2.01(m,2H).

### Example 1-38

### 7-[5-((S)-3-hydroxy-pyrrolidin-1-yl)-2-trifluoromethylphenyl]-5-[3-methyl-5-(oxazol-5-yl)-phenyl]-1,3,4,7-tetrahydro-2H-pyrido[2,3-d]pyridazin-8-one

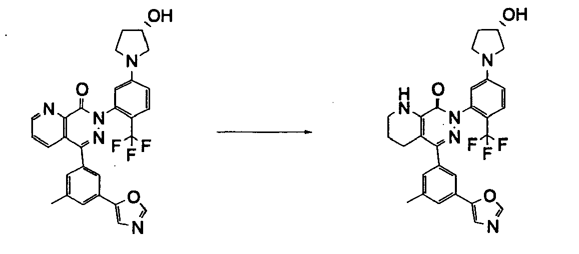

To a solution of 7-[5-((S)-3-hydroxy-pyrrolidin-1-yl)-2-trifluoromethyl-phenyl]-5-[3-methyl-5-(oxazol-5-yl)-phenyl]-7H-pyrido[2,3-d]pyridazin-8-one (52 mg) obtained in below-mentioned Example 471 in acetic acid (1.5 mL) was added platinum oxide (10 mg). After stirring under hydrogen (3 atm) at room temperature for 12 hr, the catalyst was exchanged, and the mixture was stirred under the same conditions for 8 hr. The insoluble material was filtered off, and the filtrate was concentrated. To the obtained residue was added an aqueous sodium hydrogencarbonate solution, and the mixture was extracted with ethyl acetate. The organic layer was washed with water, dried, concentrated and purified by silica gel column chromatography (ethyl acetate-chloroform:methanol=10:1) to give 7-[5-((S)-3-hydroxy-pyrrolidin-1-yl)-2-trifluoromethyl-phenyl]-5-[3-methyl-5-(oxazol-5-yl)-phenyl]-1,3,4,7-tetrahydro-2H-pyrido[2,3-d]pyridazin-8-one (22 mg).
¹H-NMR(CDCl₃)δ: 7.90(1H,s), 7.60-7.53(2H,m), 7.47(1H,s), 7.34(1H,s), 7.26(1H,s), 6.62-6.51(2H,m), 5.75(1H,s), 4.59(1H,brs), 3.59-3.22(6H,m), 2.57(2H,brs), 2.42(3H,s), 2.22-1.99(2H,m), 1.91(2H,brs).

### Example 18

### 4-(3,5-dimethoxyphenyl)-6-[5-(3-hydroxy-pyrrolidin-1-yl)-2-trifluoromethyl-phenyl]-1,2,3,6-tetrahydro-pyrrolo[2,3-d]pyridazin-7-one

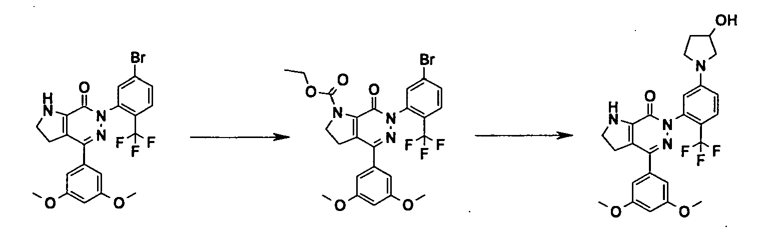

### Step 1

To a mixed solution of 6-(5-bromo-2-trifluoromethyl-phenyl)-4-(3,5-dimethoxy-phenyl)-1,2,3,6-tetrahydro-pyrrolo[2,3-d]pyridazin-7-one (8.93 g) obtained in Example 1-20a in chloroform (45 mL)-tetrahydrofuran (18 mL) were added pyridine (2.18 mL) and ethyl chloroformate (2.58 mL) under ice-cooling, and the mixture was stirred at room temperature for 12 hr. Ice water was added, and the mixture was extracted with chloroform. The organic layer was washed with water, dried, concentrated and purified by silica gel column chromatography (ethyl acetate:hexane=1:3-2:3) to give ethyl 6-(5-bromo-2-trifluoromethyl-phenyl)-4-(3,5-dimethoxyphenyl)-7-oxo-2,3,6,7-tetrahydro-pyrrolo[2,3-d]pyridazine-1-carboxylate (10.1 g).
¹H-NMR(CDCl₃)δ: 7.75-7.65(3H,m), 6.66(2H,d,*J*=2.3Hz), 6.51(1H,t,*J*=2.3Hz), 4.28(2H,q,*J*=7.0Hz), 4.19(2H,t,*J*=8.9Hz), 3.80(6H,s), 3.30-3.12(2H,m), 1.31(3H,t,*J*=7.0Hz).

### Step 2

Ethyl 6-(5-bromo-2-trifluoromethyl-phenyl)-4-(3,5-dimethoxyphenyl)-7-oxo-2,3,6,7-tetrahydro-pyrrolo[2,3-d]pyridazine-1-carboxylate (1.0 g) obtained in step 1 and (S)-3-hydroxy-pyrrolidine (160 mg) were reacted in the same manner as in Example 1-15 and purified by silica gel column chromatography (ethyl acetate:hexane=3:1-ethyl acetate). The obtained resultant product (700 mg) was dissolved in tetrahydrofuran (2 mL)-ethanol (3.5 mL), and a 2N aqueous sodium hydroxide solution (1.83 mL) was added. After stirring at room temperature for 12 hr, water was added, and the mixture was extracted with ethyl acetate. The organic layer was washed with water, dried, concentrated and purified by silica gel column chromatography (chloroform:acetone=2:1), and further crystallized from ethyl acetate to give 4-(3,5-dimethoxyphenyl)-6-[5-(3-hydroxy-pyrrolidin-1-yl)-2-trifluoromethyl-phenyl]-1,2,3,6-tetrahydro-pyrrolo[2,3-d]pyridazin-7-one (270 mg).
¹H-NMR(CDCl₃)δ: 7.60(1H,d,*J*=8.8Hz), 6.77(2H,d,*J*=2.3Hz), 6.60(1H,dd,*J*=8.6,2.3Hz), 6.54(1H,d,*J*=2.3Hz), 6.48(1H,t,*J*=2.3Hz), 4.90(1H,brs), 4.61-4.57(1H,m), 3.90-3.78(8H,m), 3.56-3.20(6H,m), 2.19-2.03(2H,m).

### Example 326

### 2-{4-(3,5-dimethyl-phenyl)-6-[5-(3-hydroxymethyl-azetidin-1-yl)-2-trifluoromethyl-phenyl]-7-oxo-2,3,6,7-tetrahydro-pyrrolo[2,3-d]pyridazin-1-yl}-acetamide

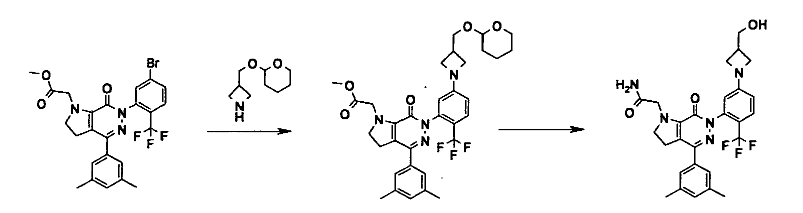

### Step 1

To a mixture of palladium acetate (9 mg), *rac-*2,2'-bis(diphenylphosphino)-1,1'-binaphthyl (27 mg) and cesium carbonate (170 mg) was added dioxane (2.7 mL) under nitrogen atmosphere, and the mixture was stirred at room temperature. After 1 hr, methyl [6-(5-bromo-2-trifluoromethyl-phenyl)-4-(3,5-dimethyl-phenyl)-7-oxo-2,3,6,7-tetrahydro-pyrrolo[2,3-d]pyridazin-1-yl]-acetate (200 mg) obtained in Example 1-25 and a solution of 3-(tetrahydro-pyran-2-yloxymethyl)-azetidine (77 mg) obtained in Example 1-23, supplementary step 2, in *tert*-butanol (1.3 mL) were added, and the mixture was stirred at 100°C for 2.5 hr. The reaction mixture was allowed to return to room temperature, and the insoluble material was filtered through celite. The filtrate was concentrated and the obtained residue was purified by silica gel column chromatography (ethyl acetate:hexane=1:4-1:2) to give methyl (4-(3,5-dimethyl-phenyl)-7-oxo-6-{5-[3-(tetrahydro-pyran-2-yloxymethyl)-azetidin-1-yl]-2-trifluoromethyl-phenyl}-2,3,6,7-tetrahydro-pyrrolo[2,3-d]pyridazin-1-yl)-acetate (193 mg).
¹H-NMR(CDCl₃)δ: 7.54(1H,d,*J*=8.6Hz), 7.16(2H,s), 7.00(1H,s), 6.43-6.37(2H,m), 4.62-4.58(3H,m), 4.05-3.96(2H,m), 3.92(1H,dd,*J*=10.0,6.9Hz), 3.87-3.80(1H,m), 3.78-3.62(7H,m), 3.59-3.48(2H,m), 3.30-3.18(1H,m), 3.13-2.95(2H,m), 2.33(6H,s), 1.85-1.66(2H,m), 1.62-1.48(4H,m).

### Step 2

Methyl (4-(3,5-dimethyl-phenyl)-7-oxo-6-{5-[3-(tetrahydro-pyran-2-yloxymethyl)-azetidin-1-yl]-2-trifluoromethyl-phenyl}-2,3,6,7-tetrahydro-pyrrolo[2,3-d]pyridazin-1-yl)-acetate (190 mg) obtained in step 1 was reacted in the same manner as in Example 1-27. The obtained crude product was dissolved in tetrahydrofuran (0.5 mL)-methanol (1.0 mL), and 2N hydrochloric acid (0.5 mL) was added. After stirring at room temperature for 1 hr, brine was added, and the mixture was extracted with ethyl acetate. The organic layer was washed with an aqueous sodium hydrogencarbonate solution and brine, dried, concentrated and purified by silica gel thin layer chromatography (chloroform:methanol=10:1), and further crystallized from ethyl acetate-hexane to give 2-{4-(3,5-dimethyl-phenyl)-6-[5-(3-hydroxymethyl-azetidin-1-yl)-2-trifluoromethyl-phenyl]-7-oxo-2,3,6,7-tetrahydro-pyrrolo[2,3-d]pyridazin-1-yl}-acetamide (121 mg).
¹H-NMR(CDCl₃) δ: 7.58(1H,d,*J*=8.81Hz), 7.15(2H,s), 7.03(1H,s), 6.69(1H,brs), 6.46(1H,d,*J*=8.58Hz), 6.39(1H,d,*J*=2.32Hz), 5.30(1H,brs), 4.51(1H,d,*J*=15.31Hz), 4.19(1H,d,*J*=15.54Hz), 4.05-4.00(2H,brm), 3.86-3.80(4H,m), 3.76-3.72(2H,brm), 3.30-3.20(1H,m), 3.14-3.07(1H,m), 2.97-2.90(1H,m), 2.34(6H,s).

### Example 329

### 4-(3,5-dimethoxy-phenyl)-1-(2-hydroxy-ethyl)-6-[5-(3-hydroxymethyl-azetidin-1-yl)-2-trifluoromethyl-phenyl]-1,2,3,6-tetrahydro-pyrrolo[2,3-d]pyridazin-7-one

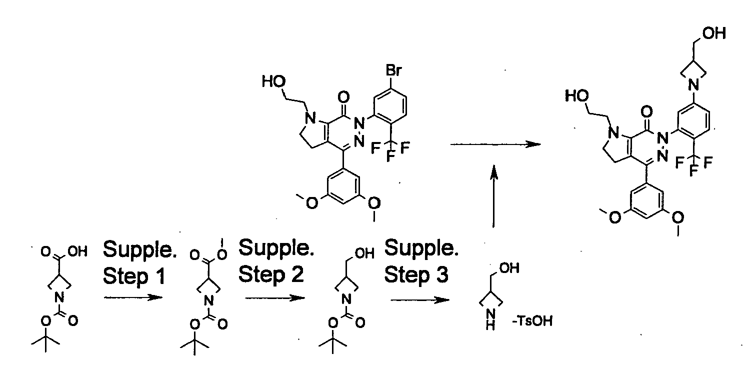

To a mixed solution of 6-(5-bromo-2-trifluoromethyl-phenyl)-4-(3,5-dimethoxy-phenyl)-1-(2-hydroxy-ethyl)-1,2,3,6-tetrahydro-pyrrolo[2,3-d]pyridazin-7-one (250 mg) obtained in Example 1-22a and azetidin-3-yl-methanol tosylate (179 mg) obtained in supplementary step 3 in N,N-dimethylacetamide (1.88 mL)-*tert*-butanol (0.63 mL) was added potassium carbonate (127 mg) under a nitrogen atmosphere. After stirring at 130°C for 32 hr, water was added to the reaction mixture at 50°C, and the mixture was stirred at room temperature for 12 hr. The precipitated crystals were collected by filtration, dried, recrystallized from methanol-toluene mixed solvent, and then slurry washed with acetone (50°C) to give 4-(3,5-dimethoxy-phenyl)-1-(2-hydroxy-ethyl)-6-[5-(3-hydroxymethyl-azetidin-1-yl)-2-trifluoromethyl-phenyl]-1,2,3,6-tetrahydro-pyrrolo[2,3-d]pyridazin-7-one (142 mg).
¹H-NMR(DMSO-d₆) δ: 7.56(1H,d,J=8.8Hz), 6.66(2H,d,J=2.3Hz), 6.55-6.53(2H,m), 6.40(1H,d,J=2.3 Hz), 4.79(1H,t,J=5.2Hz), 4.69(1H,t,J=5.2Hz), 3.93(2H,t,J=7.9Hz), 3.76-3.69(10H,m), 3.66-3.63(2H,m), 3.58-3.53(4H,m), 3.22-3.01(2H,m), 2.86-2.77(1H,m).

### Supplementary step 1

To a solution of 1-tert-butoxycarbonyl-azetidine-3-carboxylic acid (10 g) in dimethylsulfoxide (20 mL) were successively added potassium carbonate (9.60 g) and methyl iodide (3.7 mL) by small portions. After stirring at room temperature for 2 hr, an aqueous citric acid solution was added, and the mixture was extracted with toluene. The toluene layer was washed with water and brine and the whole aqueous layer was extracted again with toluene. The toluene layer were combined, dried, and concentrated to give methyl 1-tert-butoxycarbonyl-azetidine-3-carboxylate (10.5 g).
¹H-NMR(CDCl₃)δ: 4.11(4H,d,*J*=7.7Hz), 3.76(3H,s), 3.40-3.32(1H,m), 1.45(9H,s).

### Supplementary step 2

To a solution of methyl 1-tert-butoxycarbonyl-azetidine-3-carboxylate (5.0 g) obtained in supplementary step 1 in toluene (50 mL) was added dropwise sodium bis(2-methoxyethoxy)aluminum hydride (65 wt% toluene solution; 7.68 mL) at -17°C to 13°C over 40 min. After stirring at the same temperature for 20 min, an aqueous citric acid solution was added and the mixture was extracted with toluene. The toluene layer was successively washed with an aqueous potassium hydrogen sulfate solution, brine, and an aqueous sodium hydrogencarbonate solution, the whole aqueous layer was extracted again with toluene. The toluene layers were combined, dried, concentrated to give *tert*-butyl 3-hydroxymethyl-azetidine-1-carboxylate (3.89 g).
¹H-NMR(CDCl₃)δ: 4.01(2H,t,*J*=8.6Hz), 3.79(2H,dd,*J*=6.5,5.3Hz), 3.70(2H,dd,*J*=8.6,5.3Hz), 2.77-2.66(1H,m), 1.77(1H,t,*J*=5.3Hz), 1.45(9H,s).

### Supplementary step 3

*tert*-Butyl 3-hydroxymethyl-azetidine-1-carboxylate (3.70 g) obtained in supplementary step 2 were added methanol (12 mL) and p-toluenesulfonic acid monohydrate (4.52 g). After stirring at the inside temperature of 55°C for 7 hr, the reaction mixture was concentrated under reduced pressure. The residual water was azeotropically evaporated with toluene, and tetrahydrofuran was added. The precipitated crystals were collected by filtration, and dried to give azetidin-3-yl-methanol tosylate (4.97 g).
¹H-NMR(DMSO-d₆)δ: 8.46(2H,brs), 7.49(2H,d,*J*=7.9Hz), 7.13(2H,d,*J*=7.9Hz), 5.06(1H,brs), 3.98-3.90(2H,m), 3.80-3.72(2H,m), 3.50(2H,d,*J*=5.1Hz), 2.92-2.81(1H,m), 2.30(3H,s).

### Examples 2 to 401

The compounds of Examples 2 to 401 were obtained in the same manner as in a method selected from Examples 1-1 to 1-38. The compounds of Examples 1-1 to 1-46 and the compounds of Examples 2 to 401 obtained in the same manner are shown in the following Tables. In the tables, the left line shows Example numbers and the right line shows the structural formulas of the corresponding compounds.

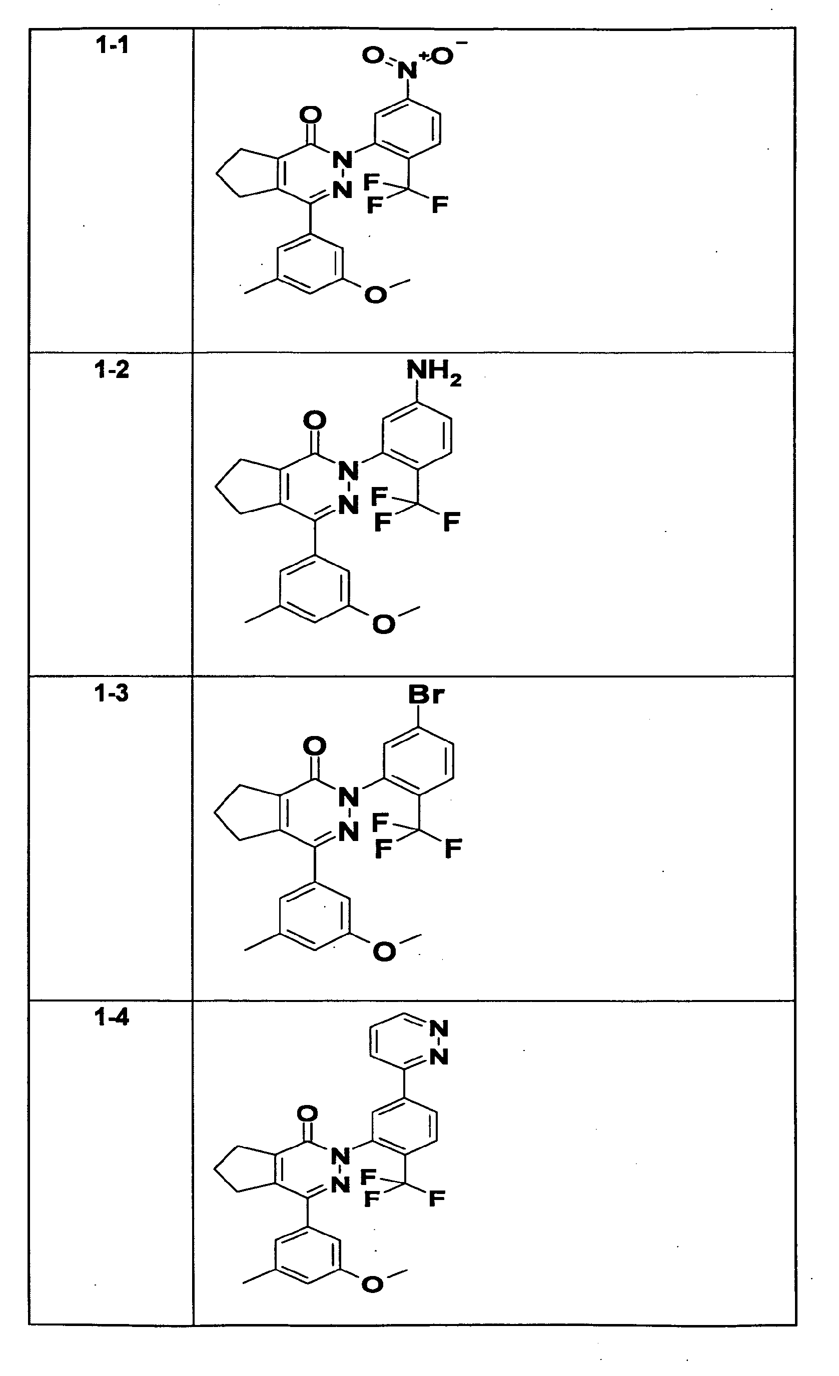

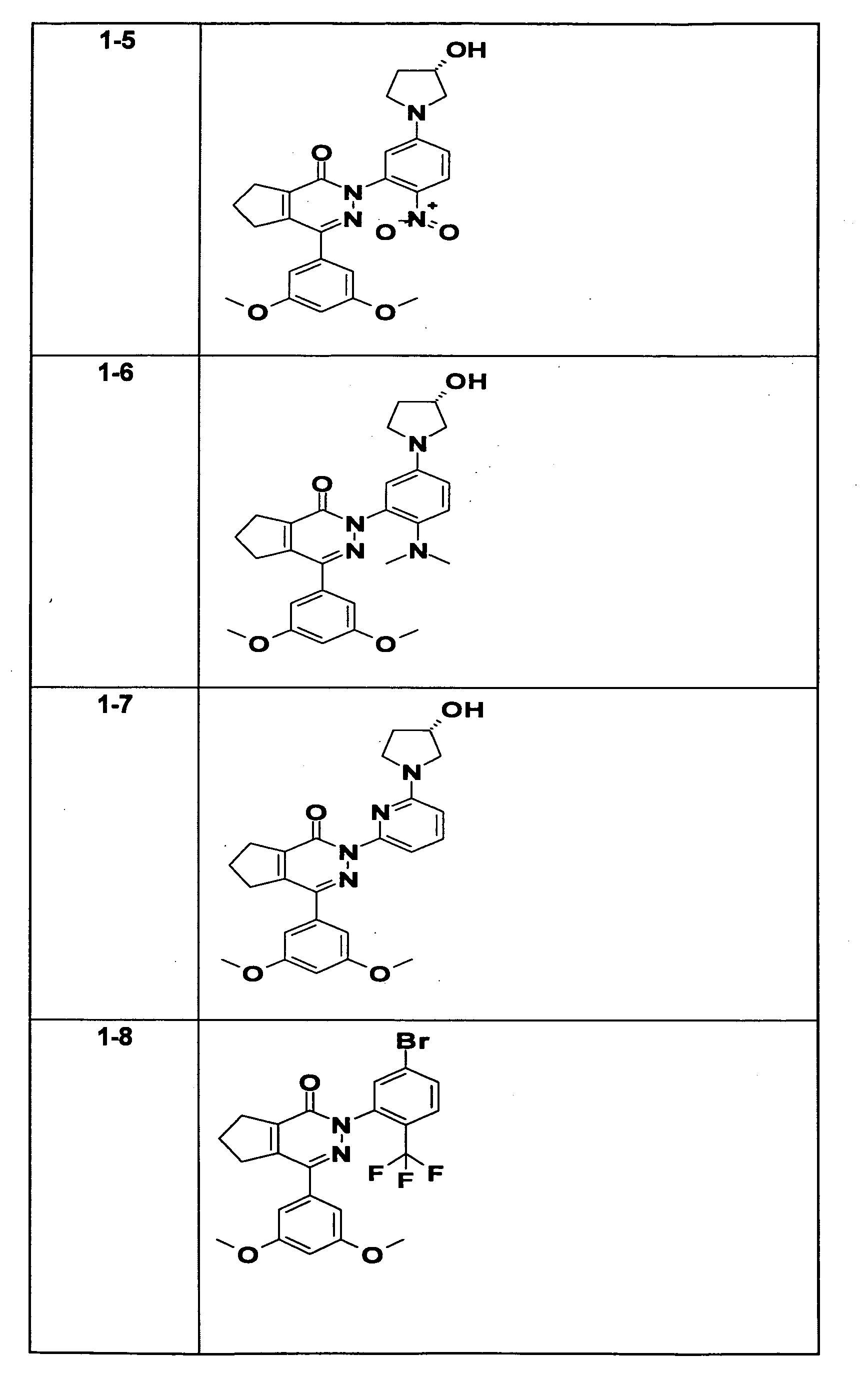

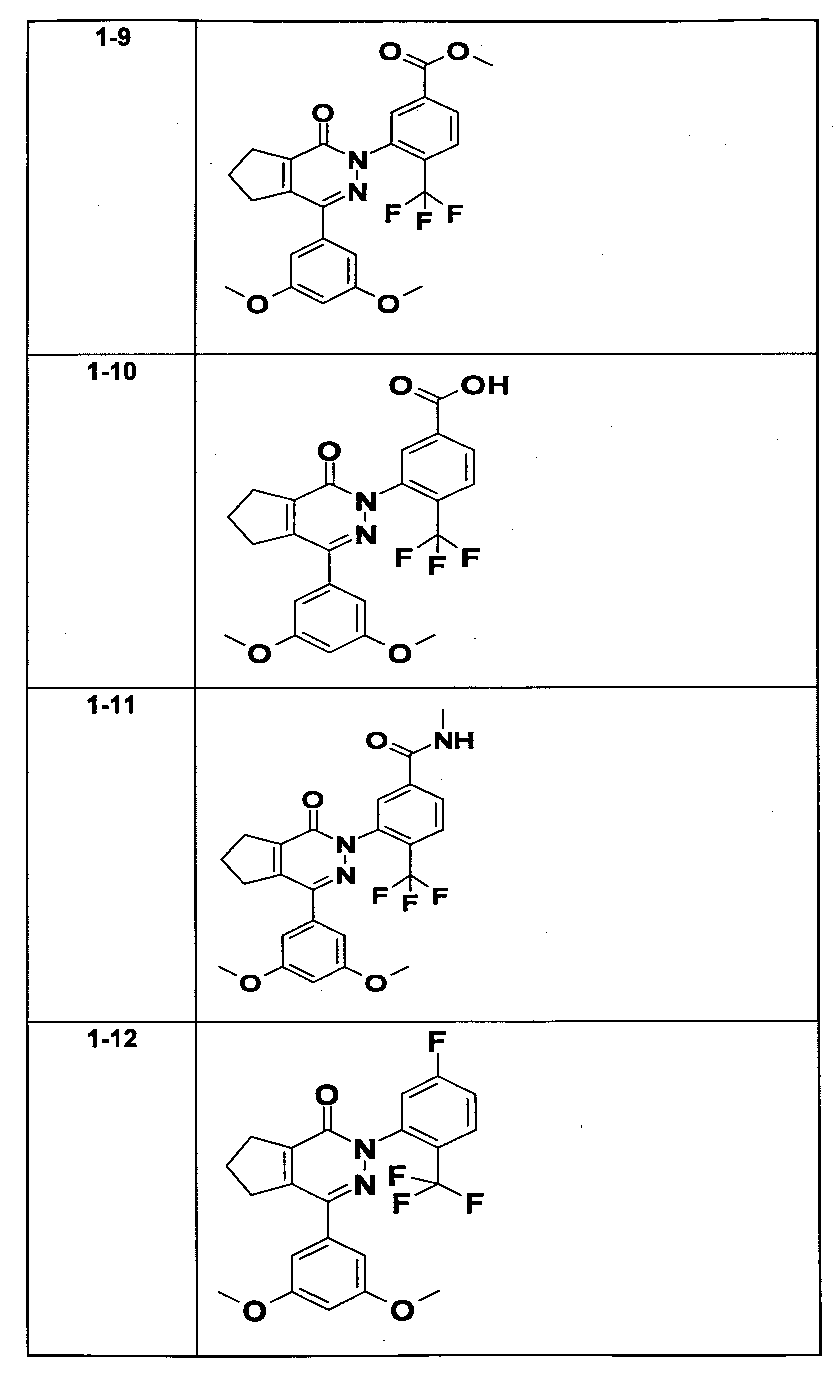

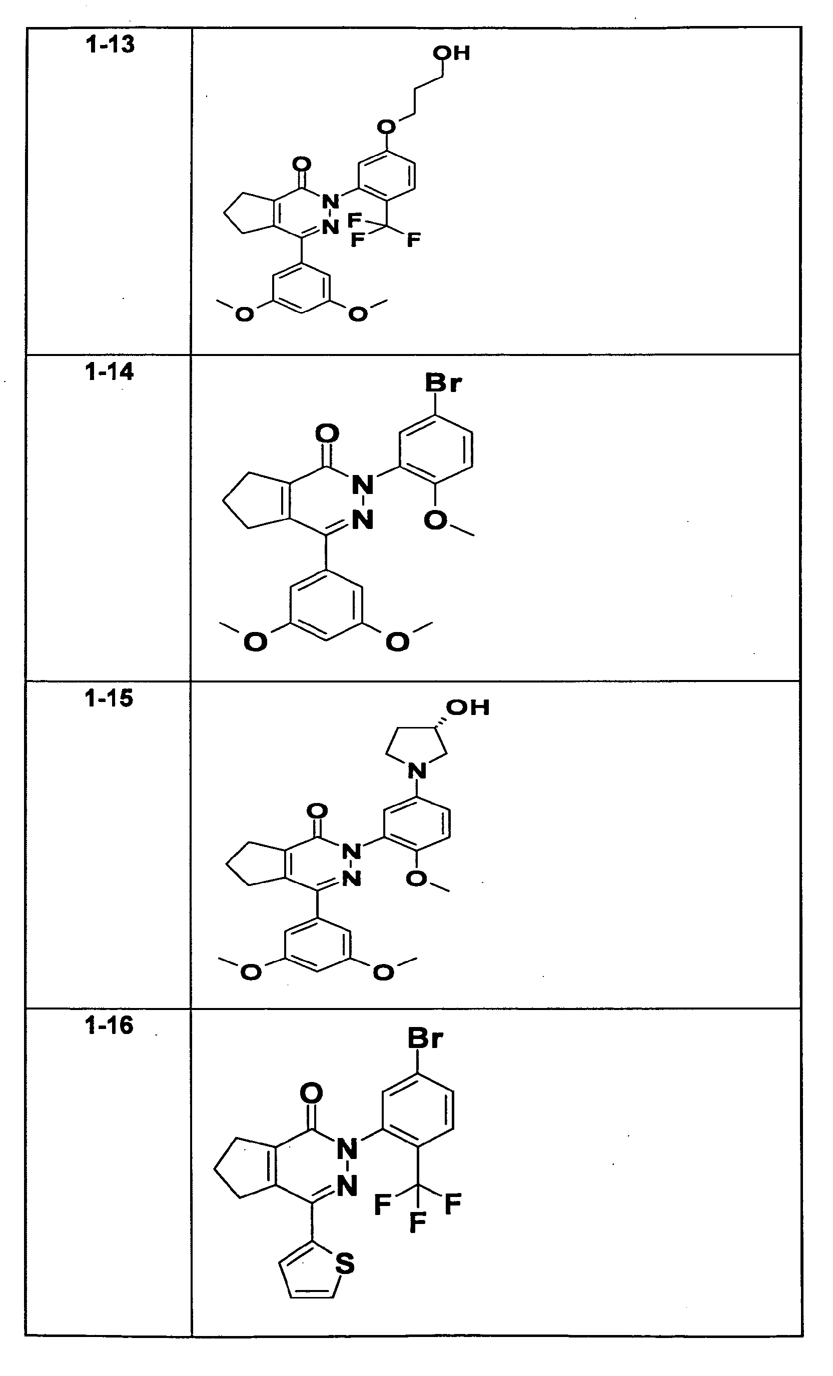

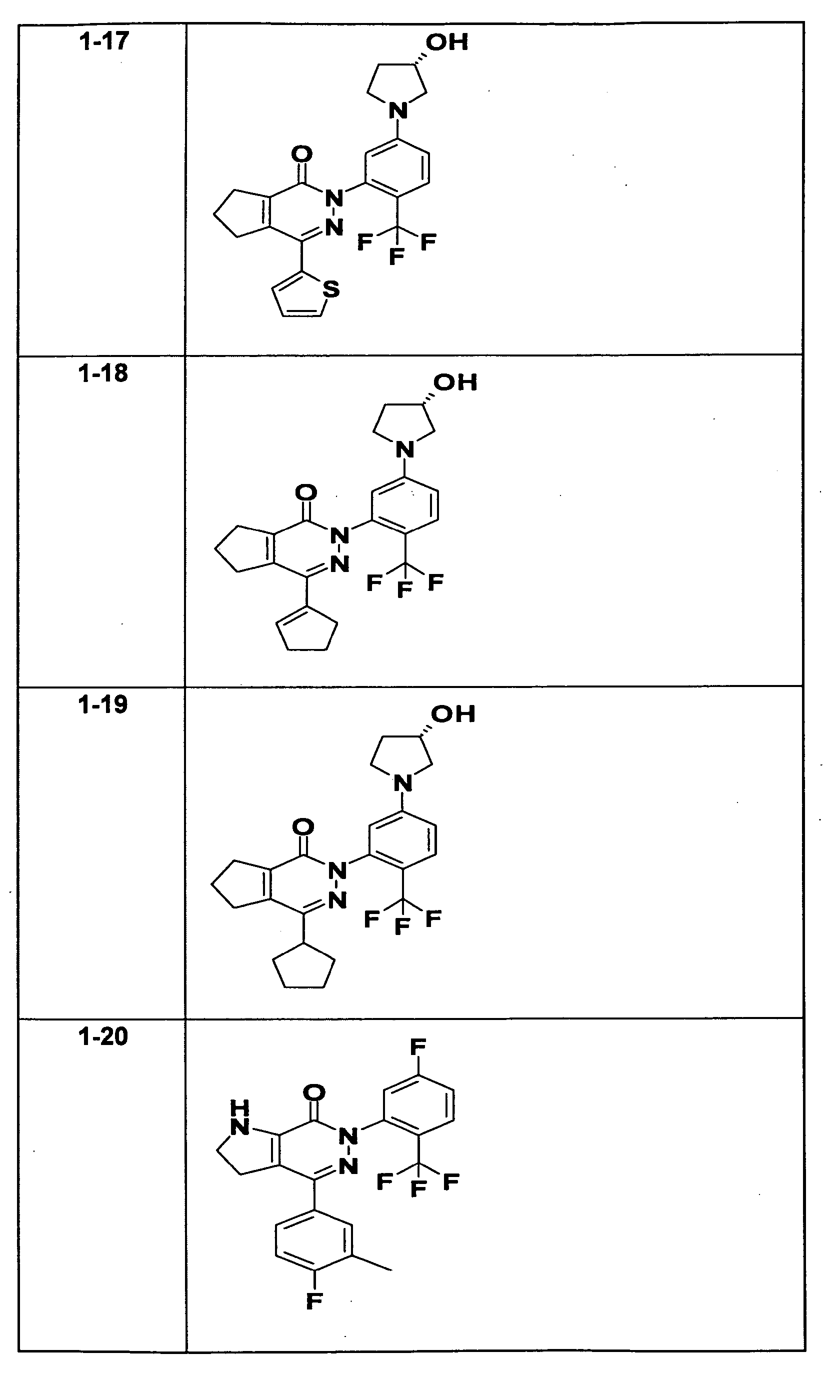

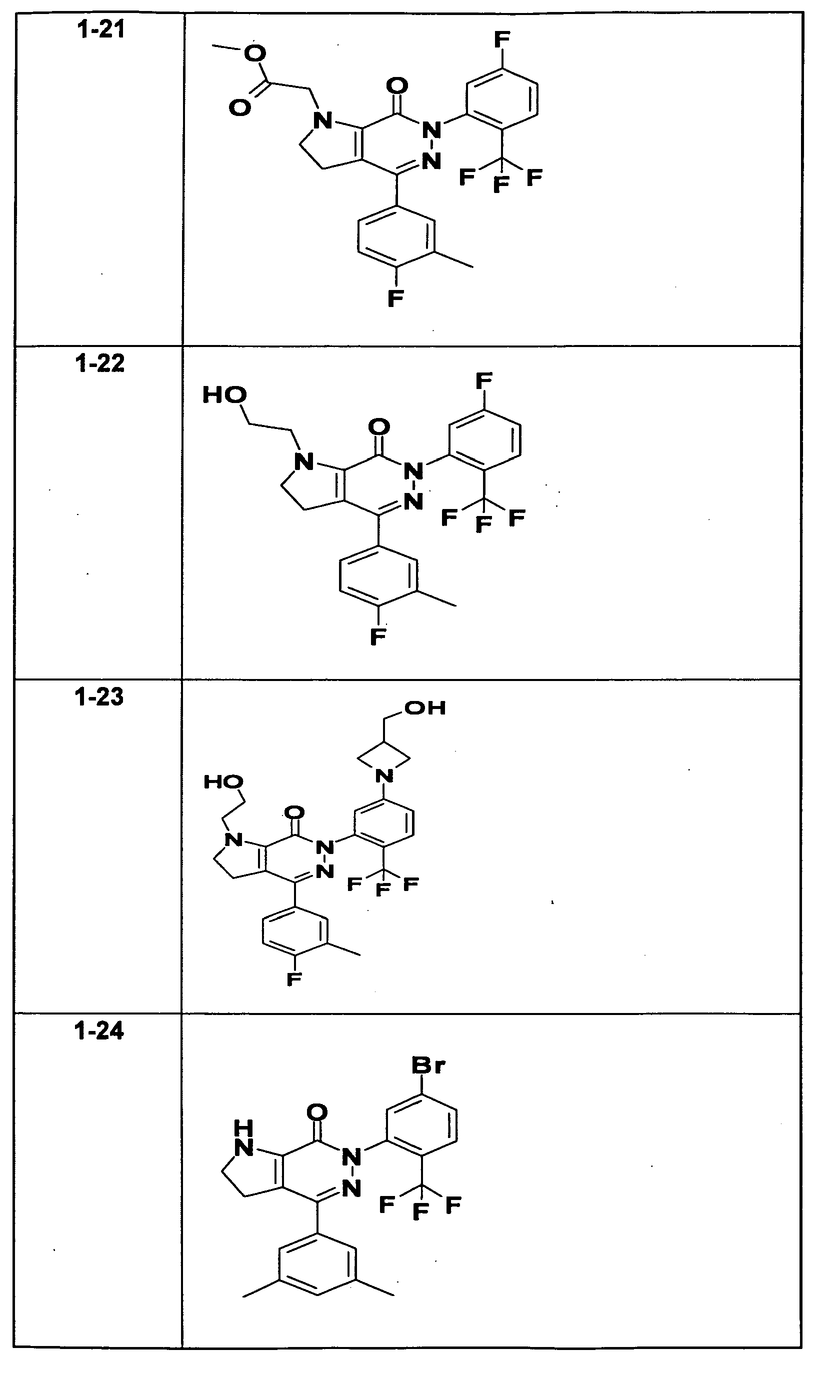

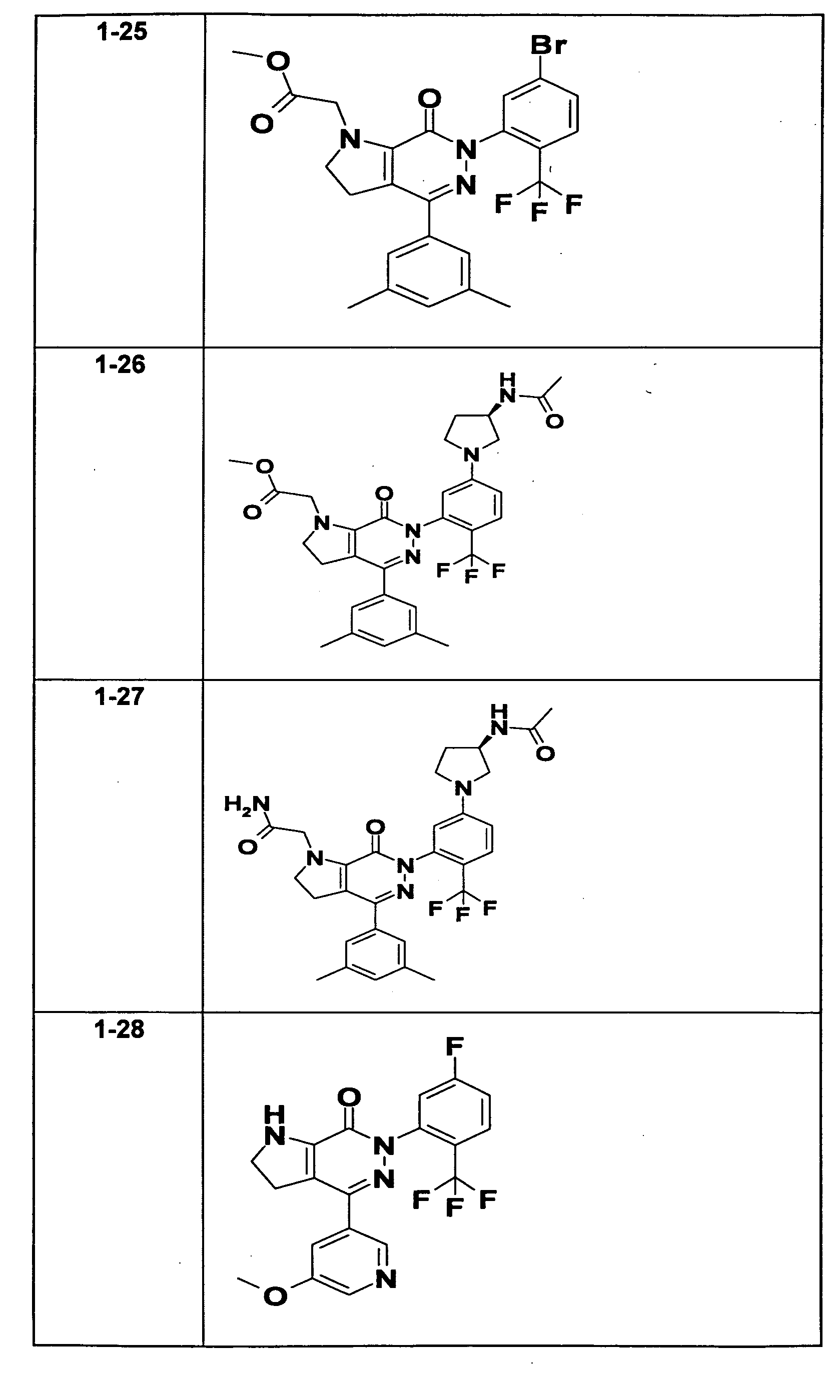

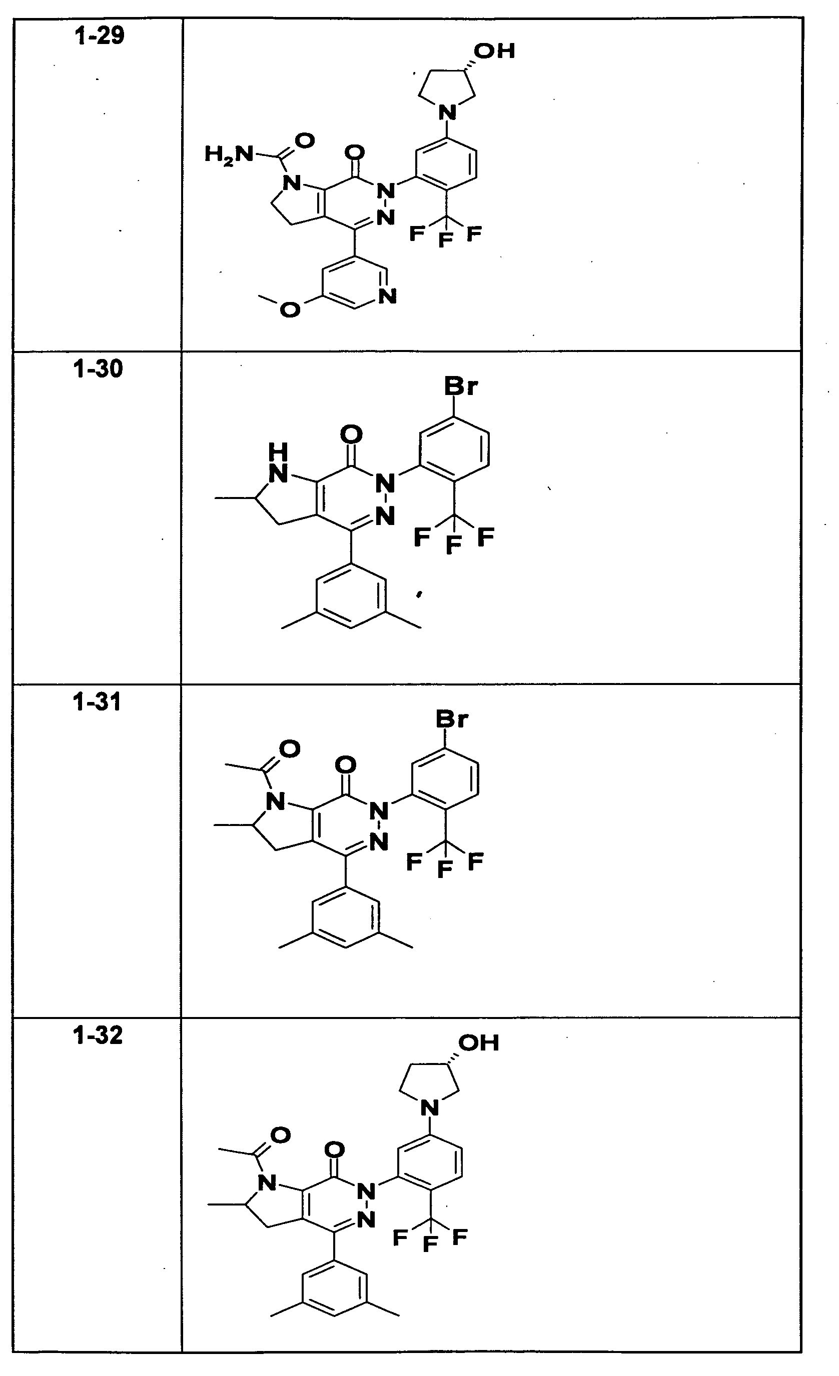

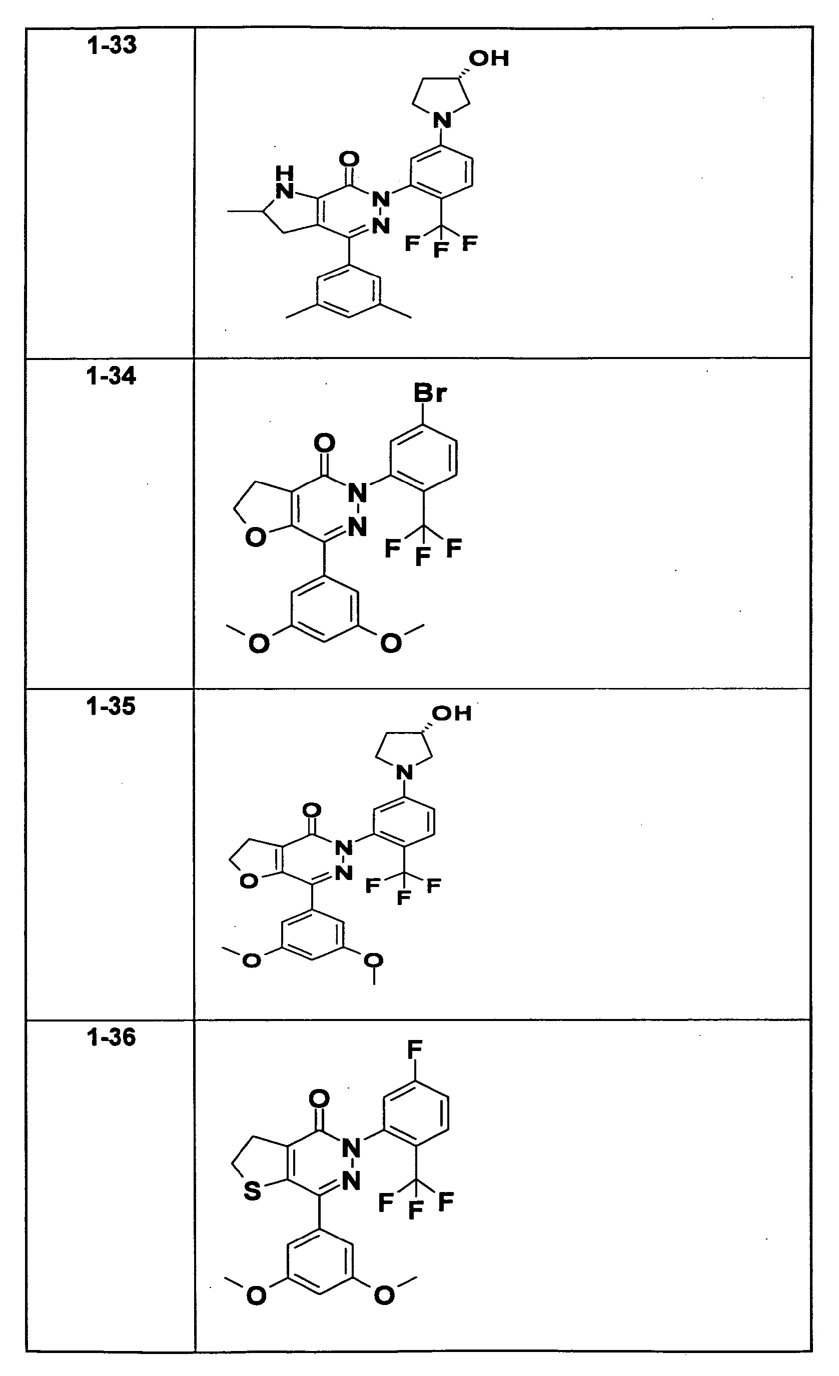

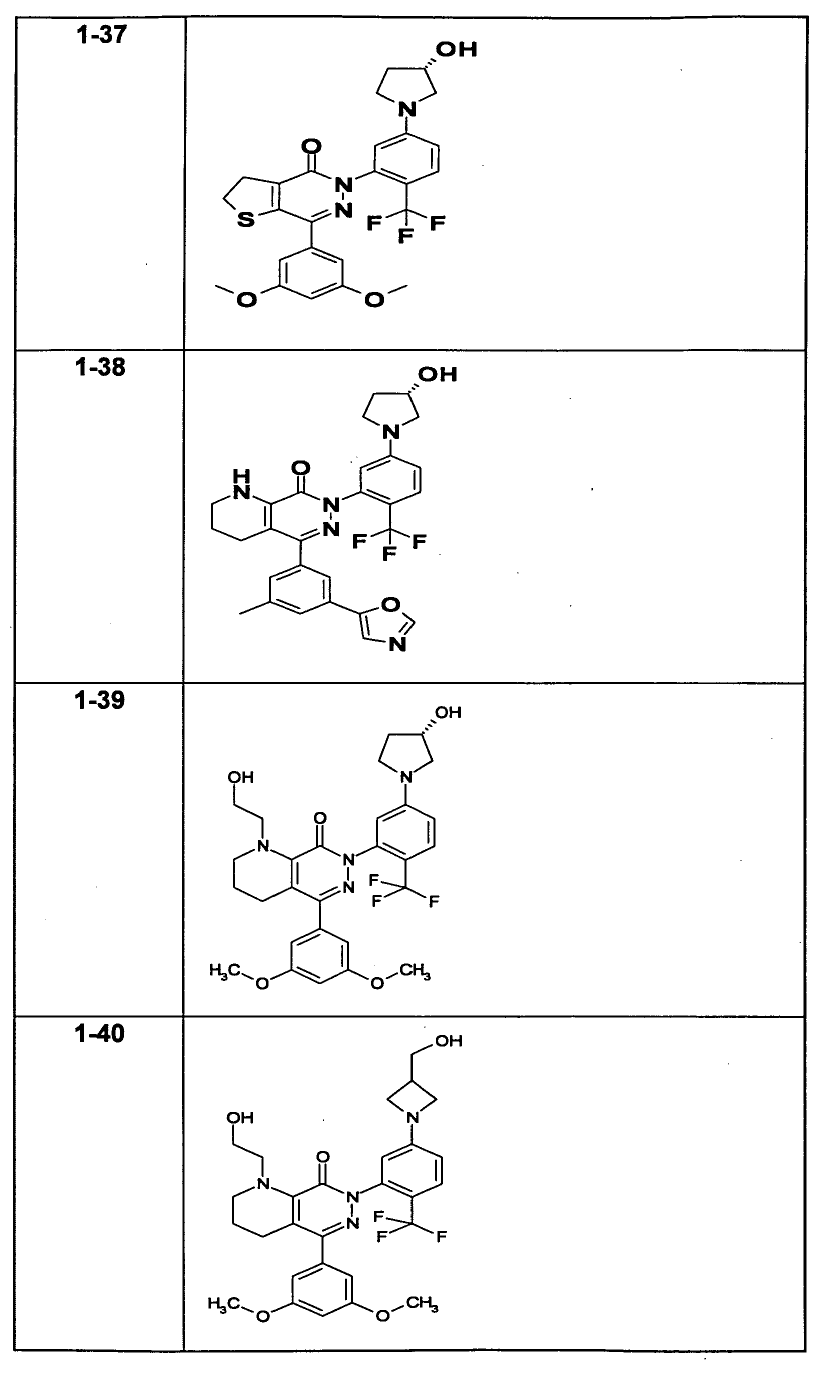

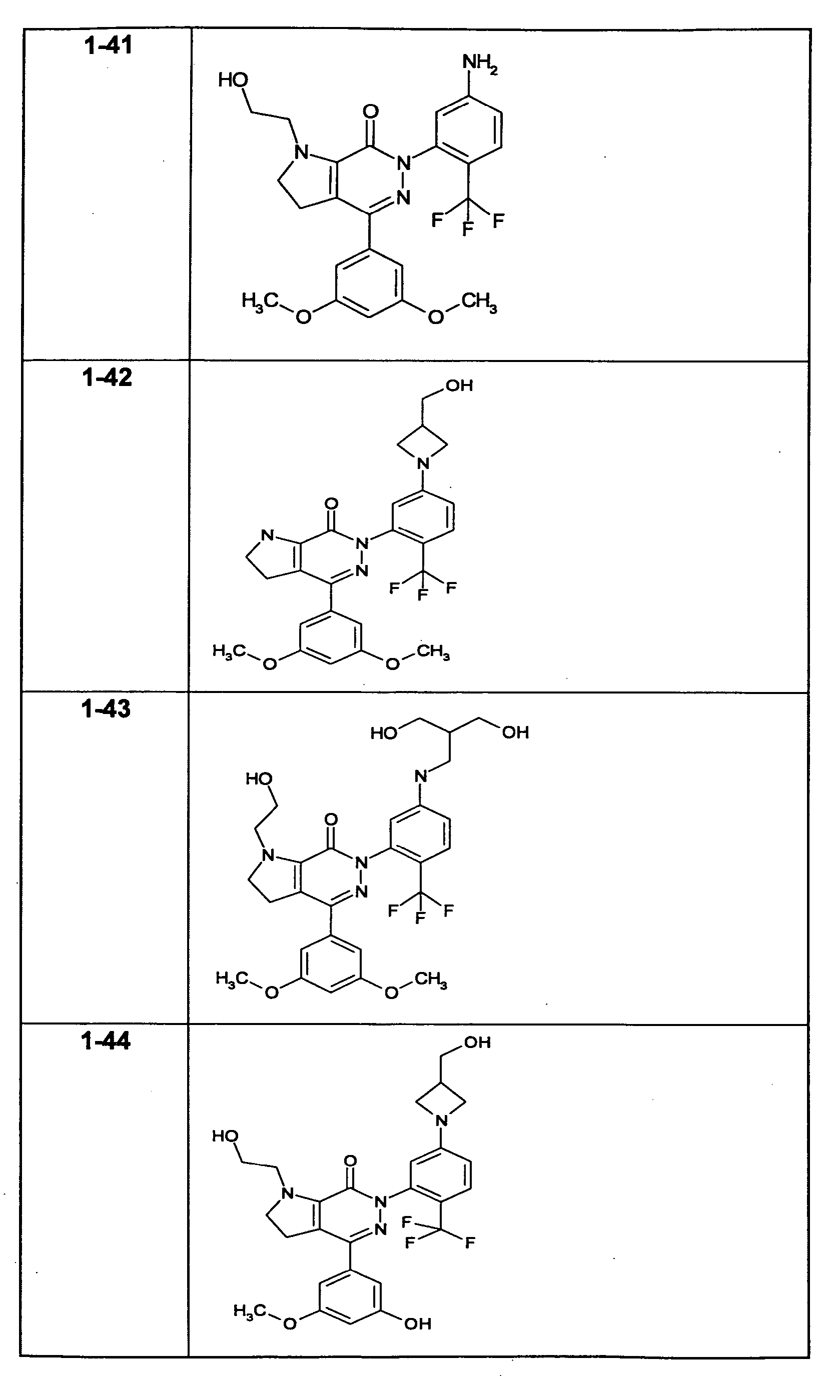

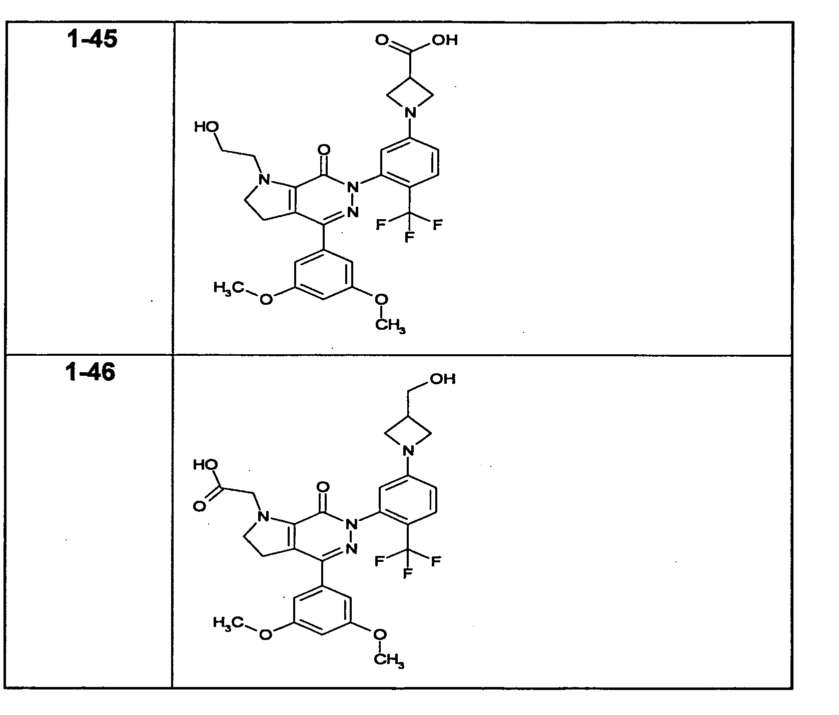

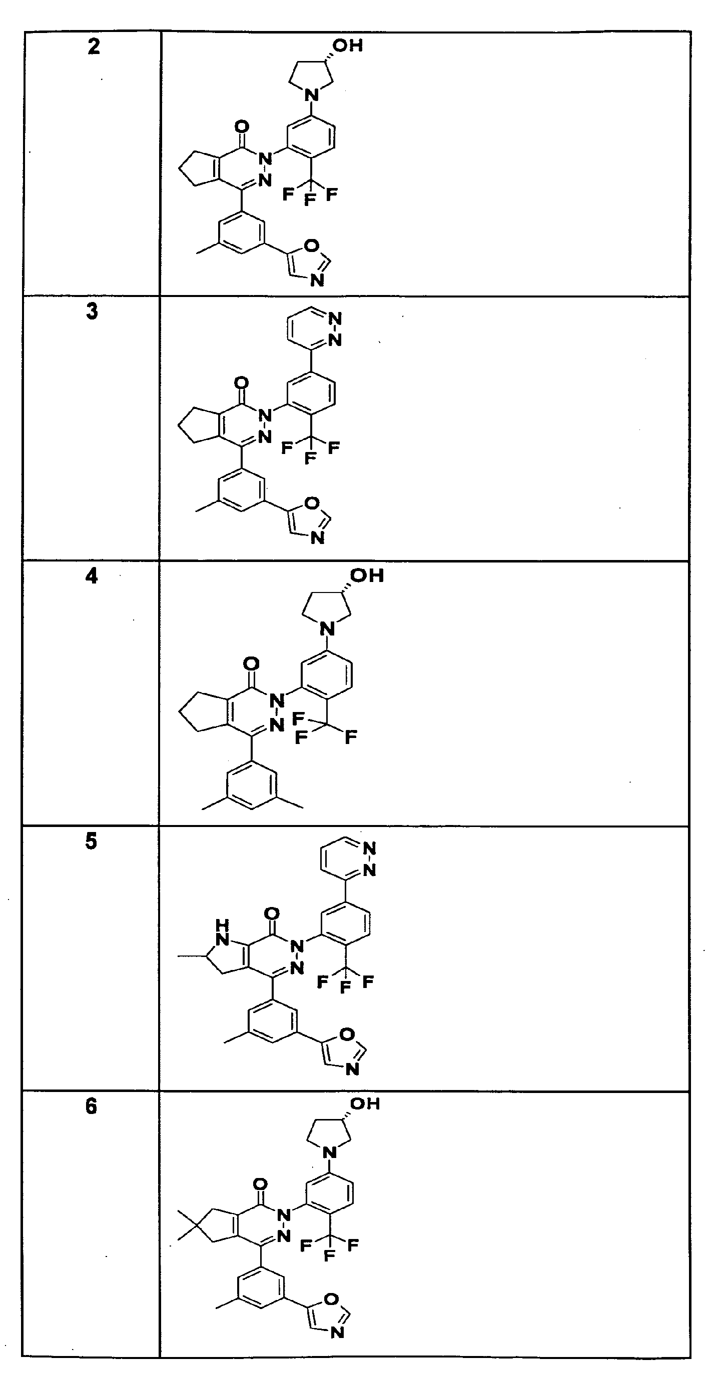

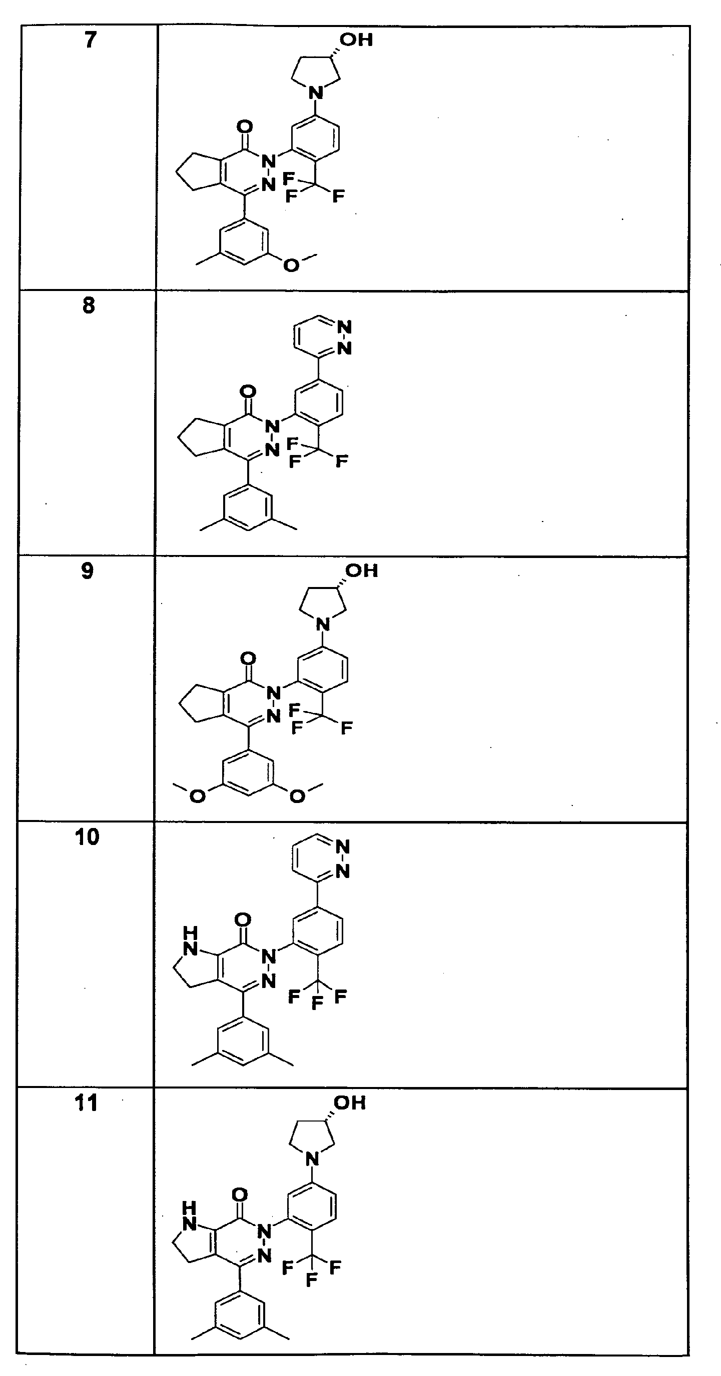

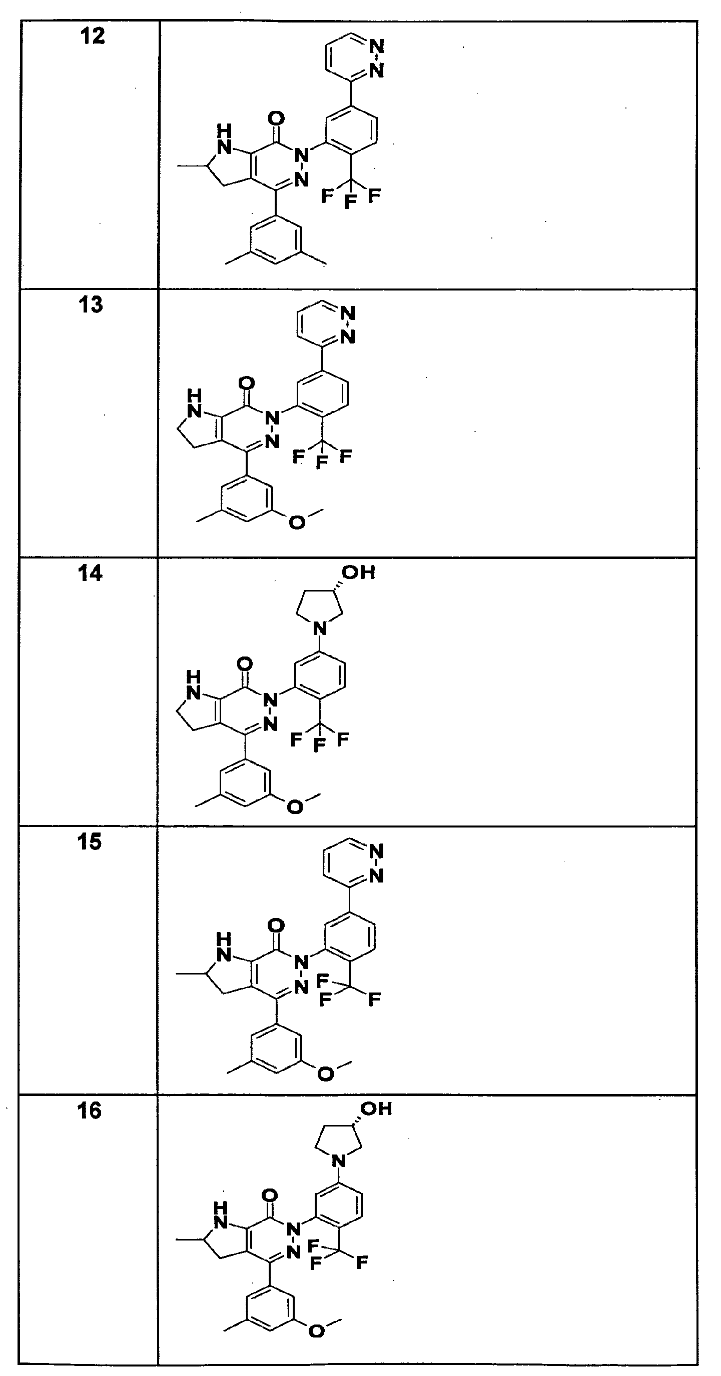

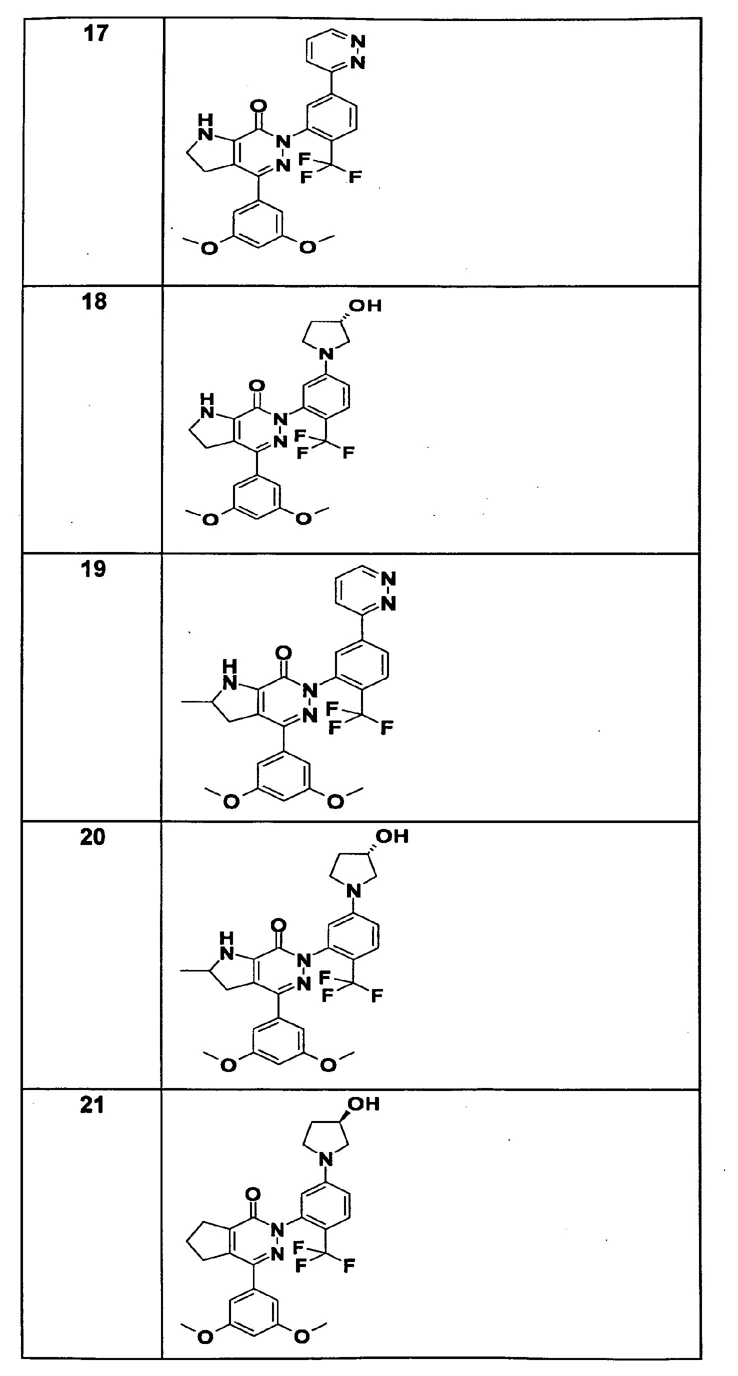

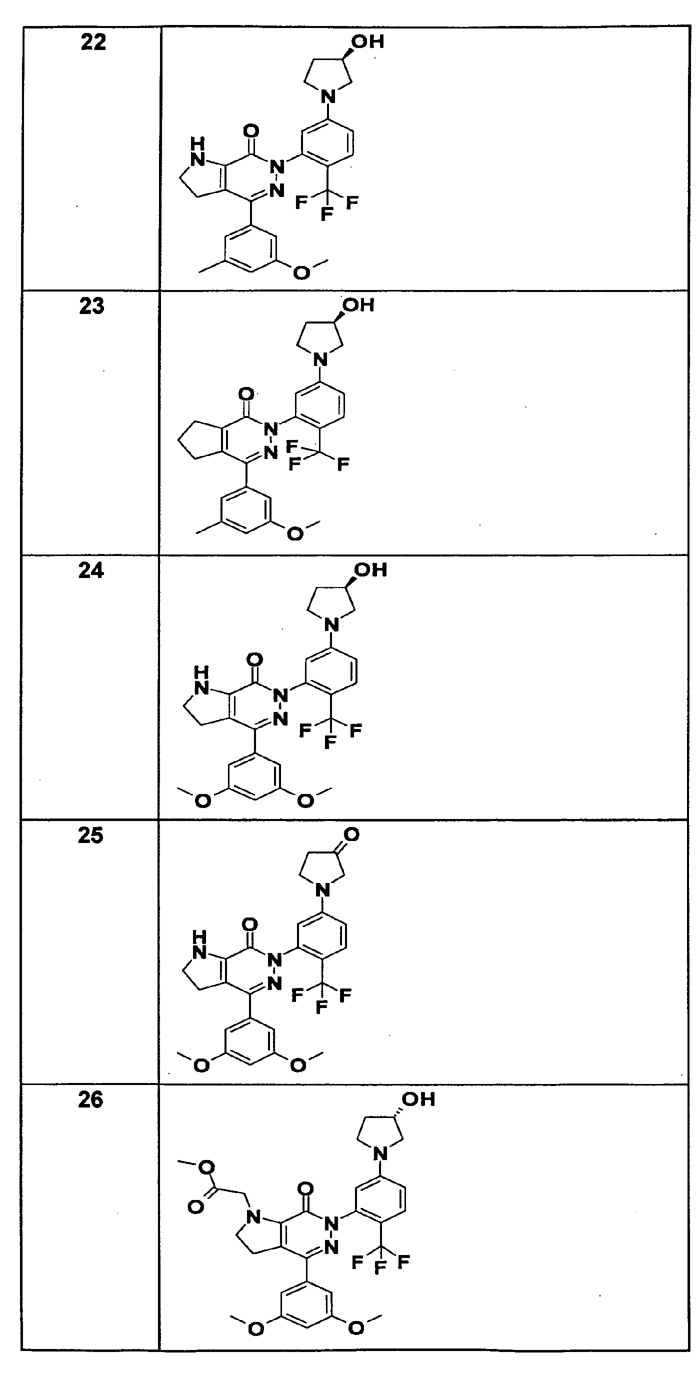

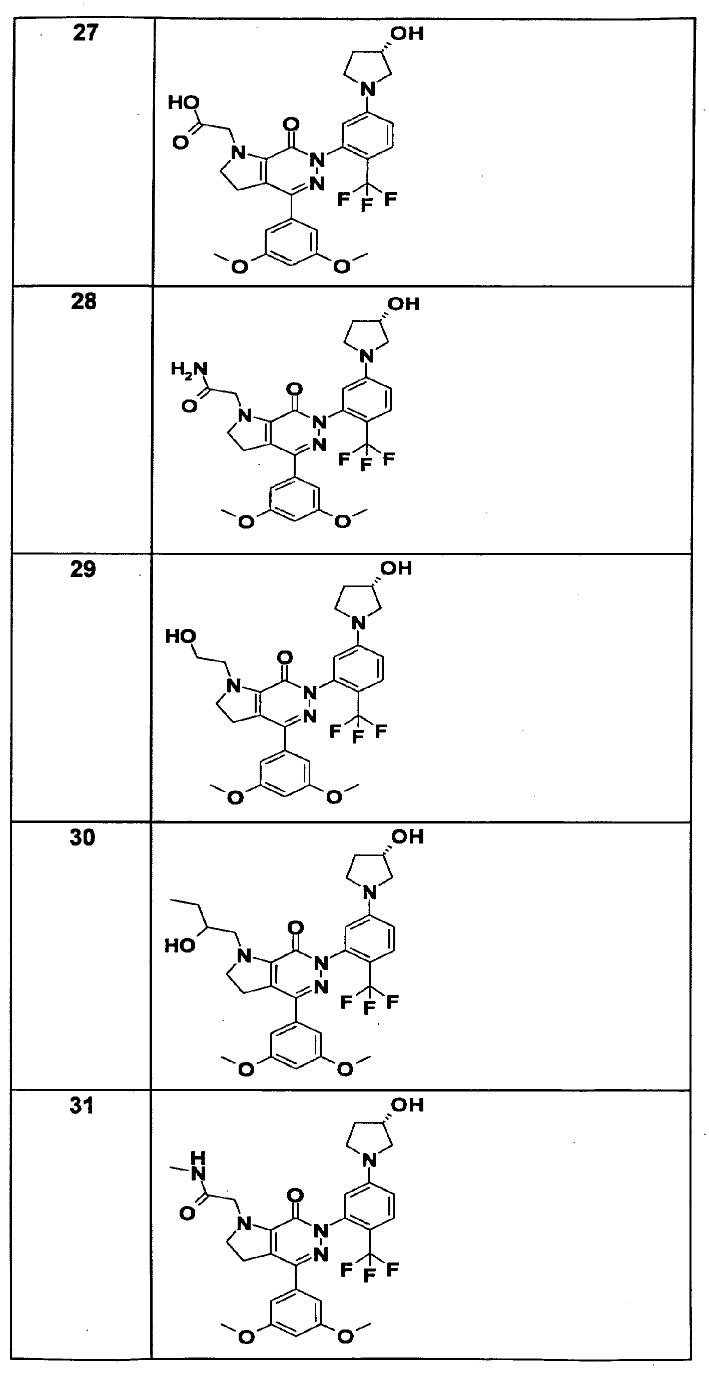

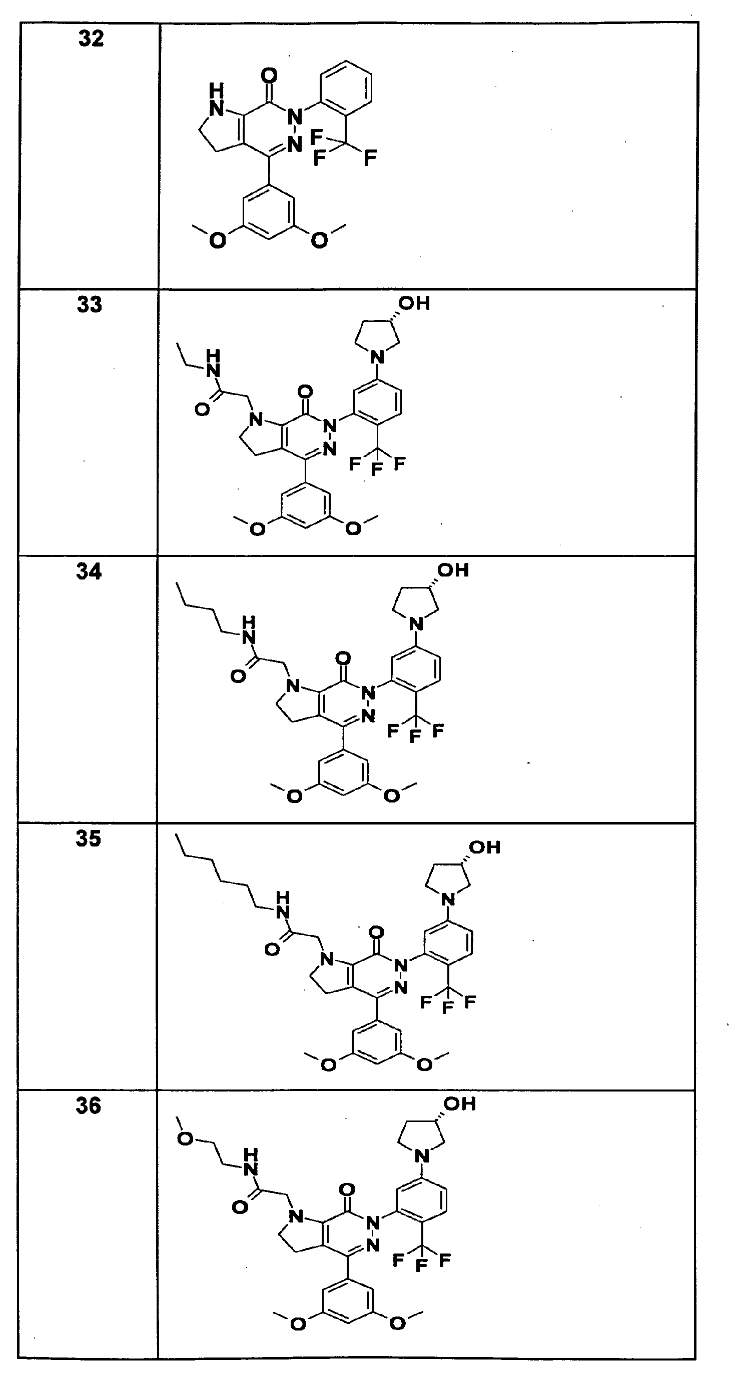

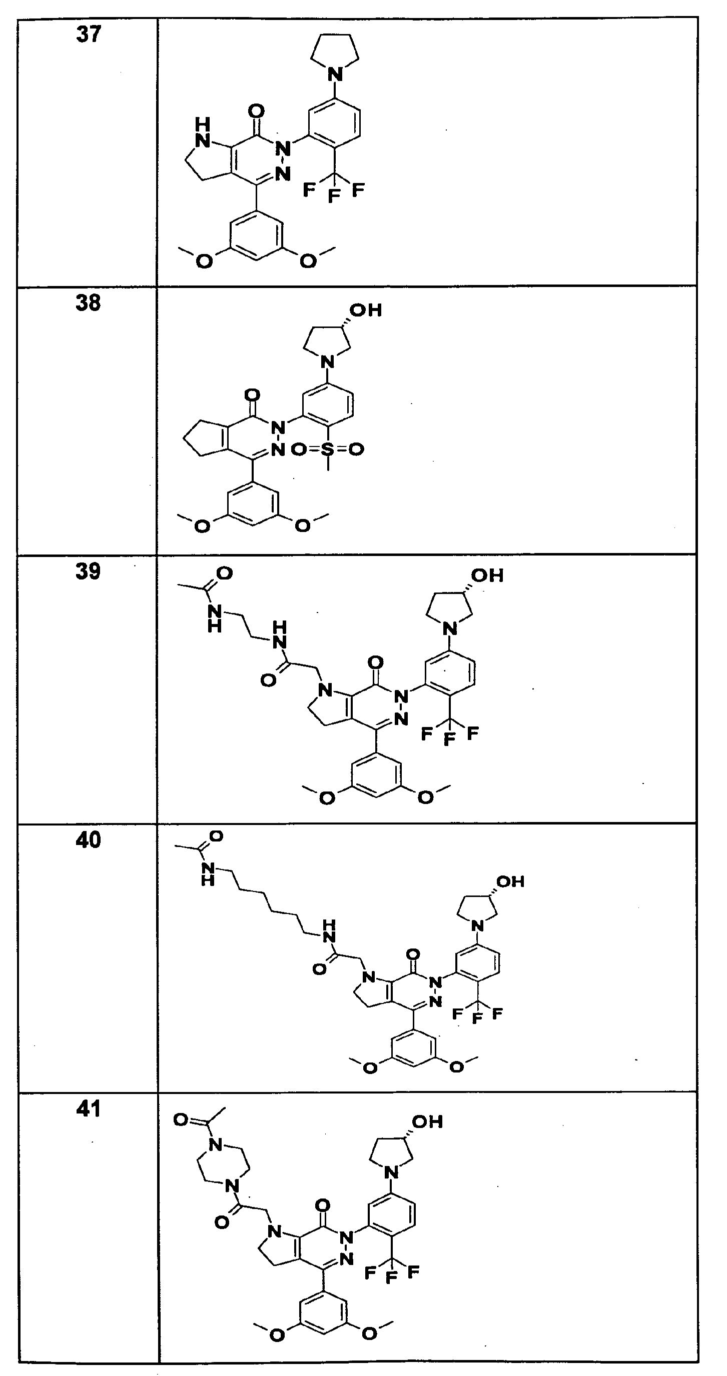

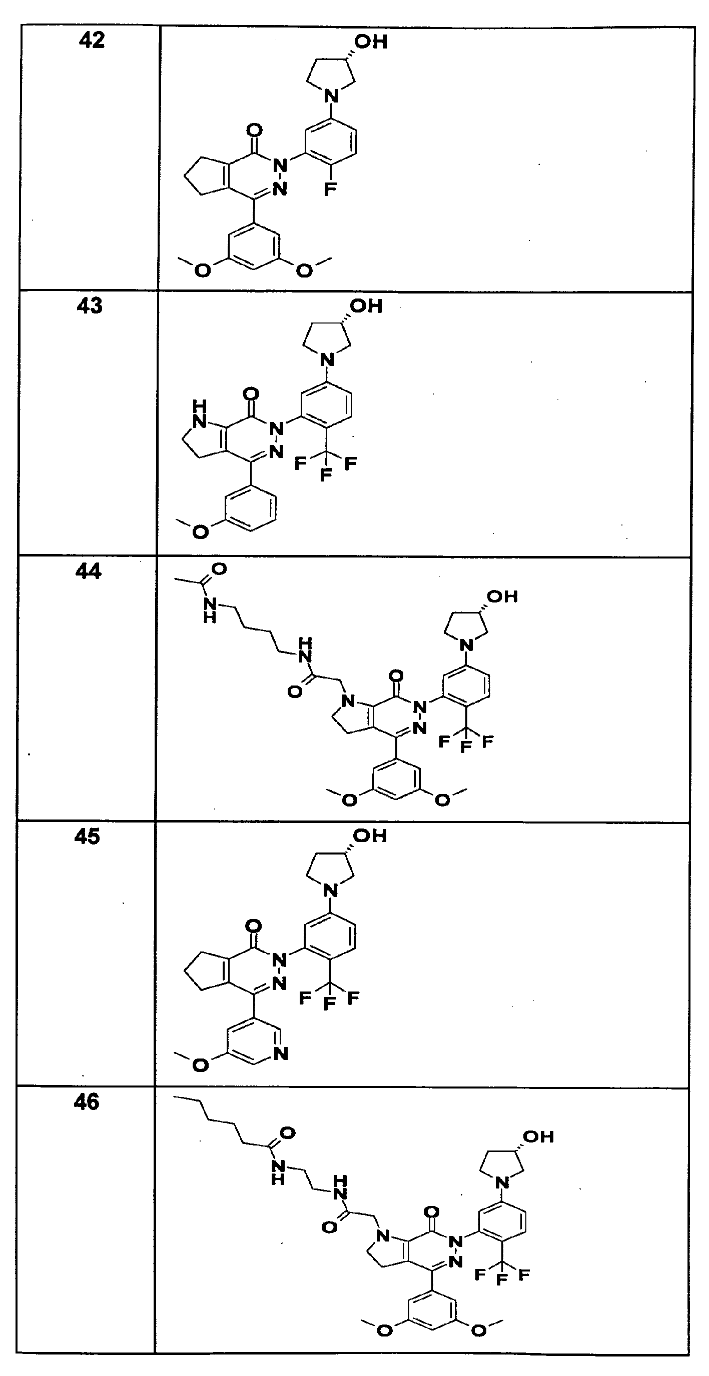

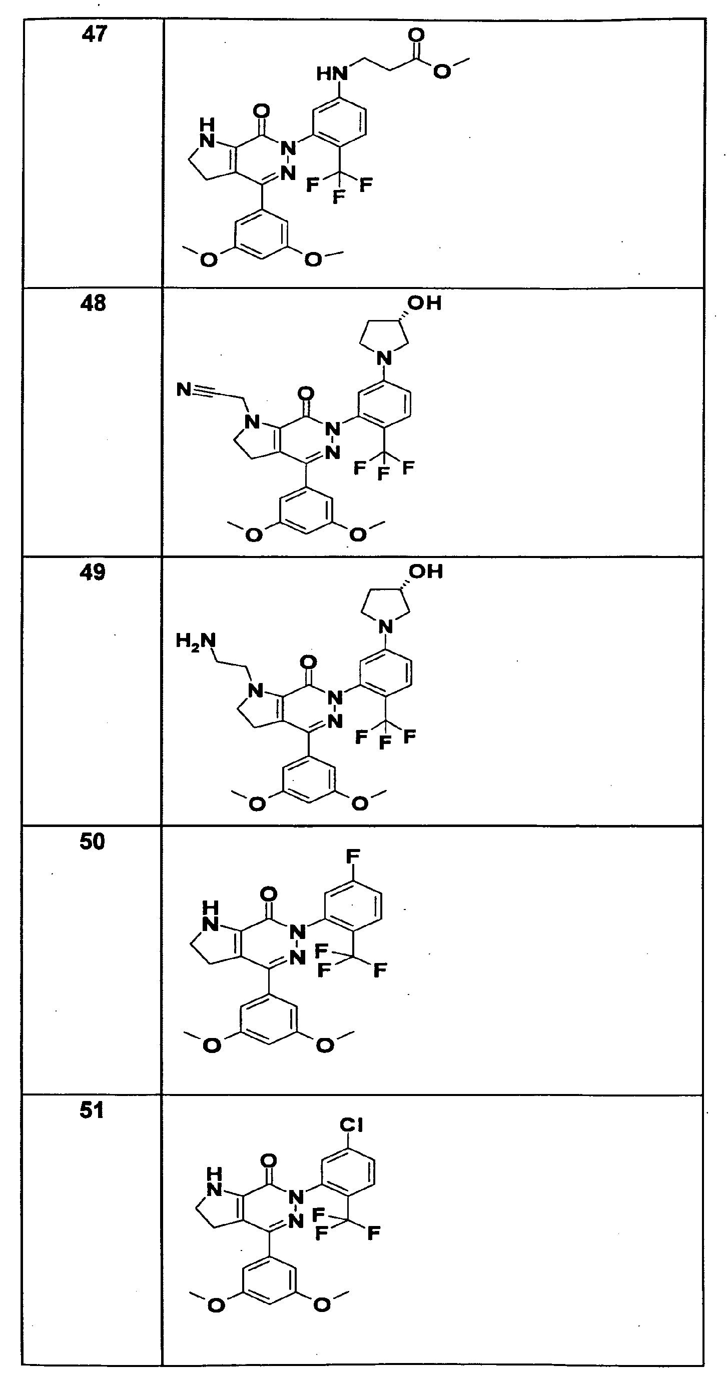

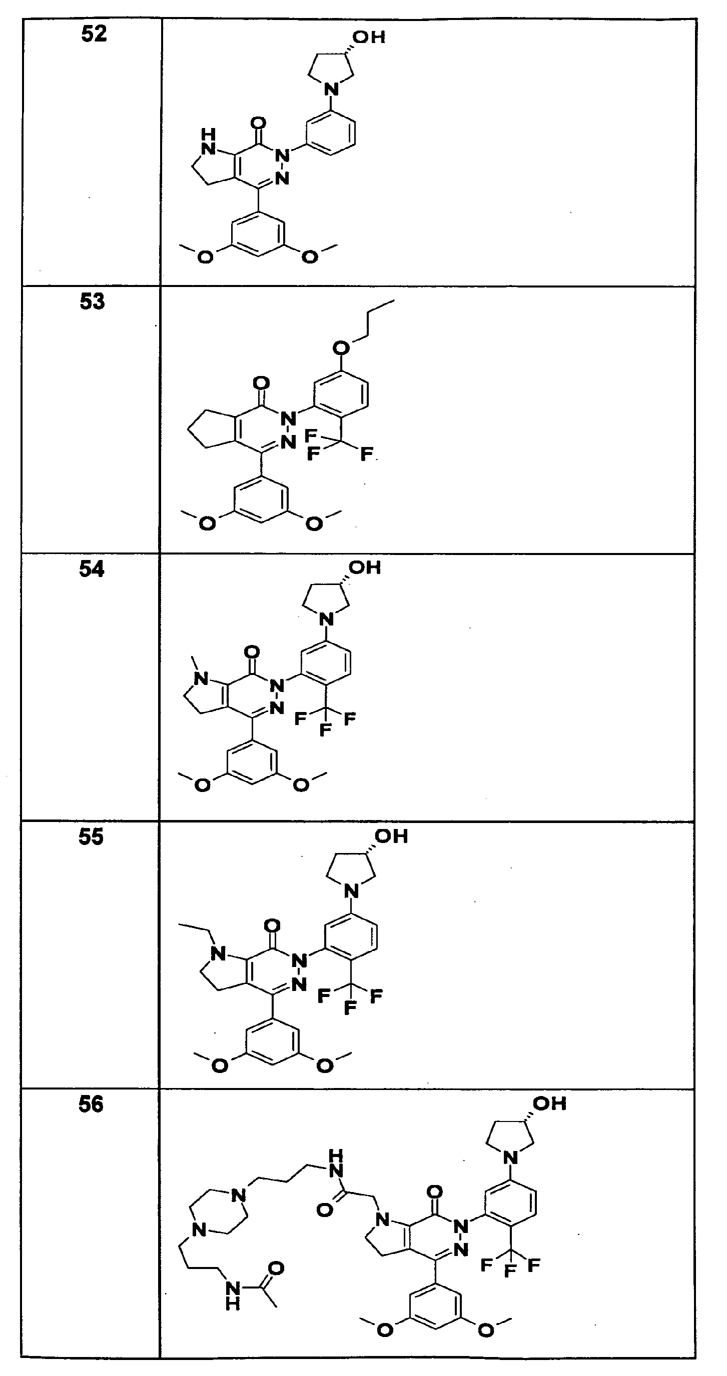

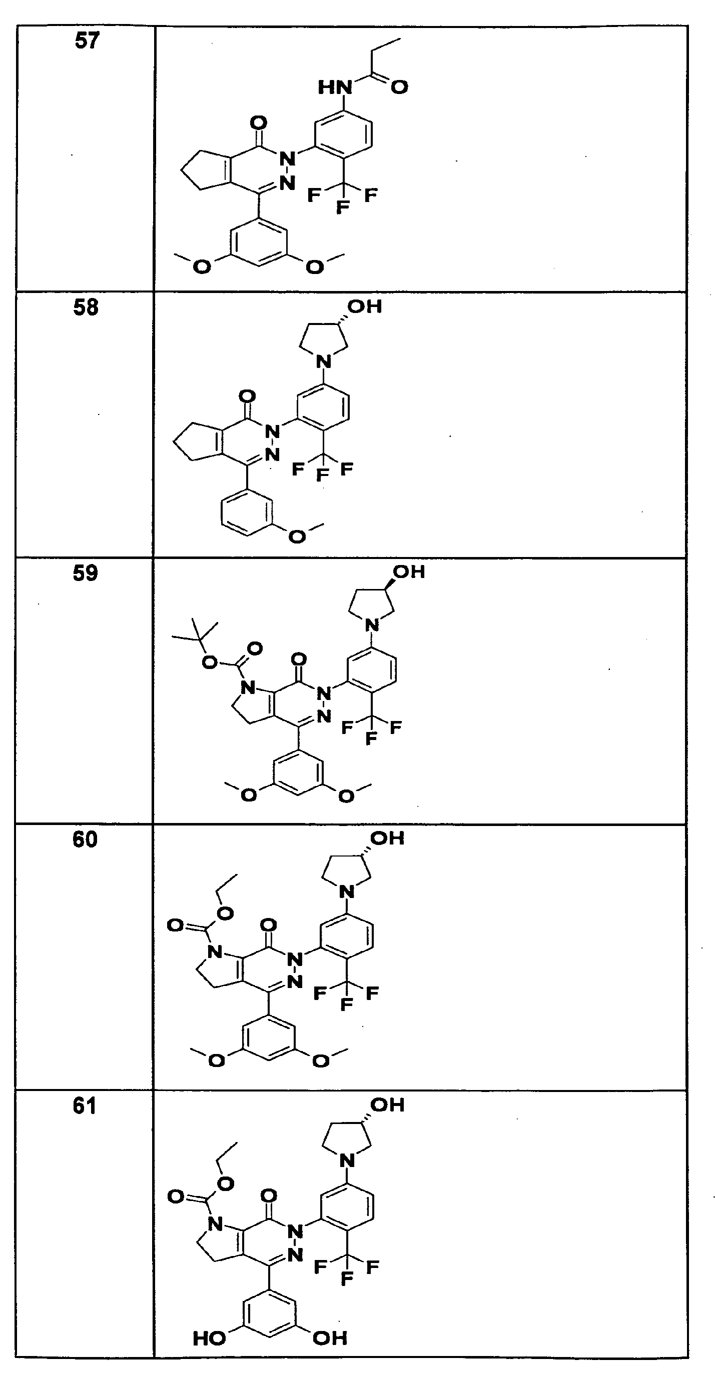

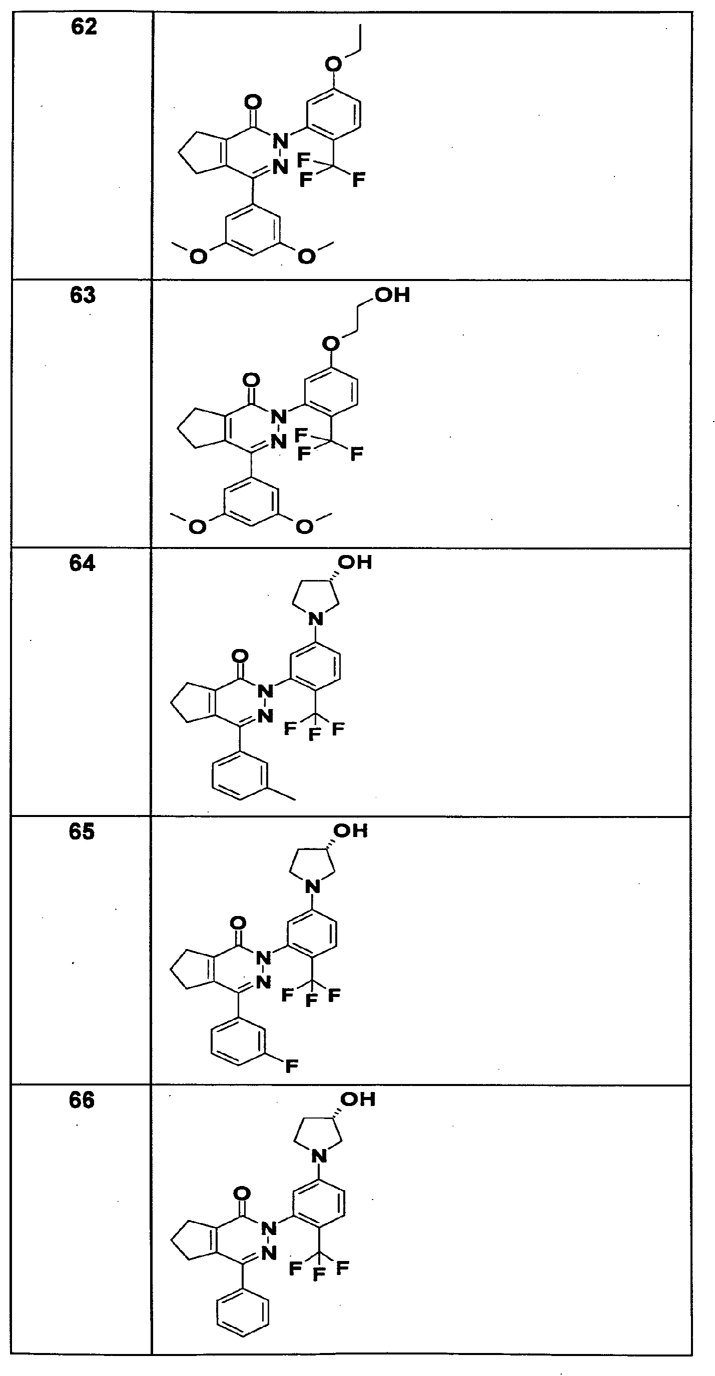

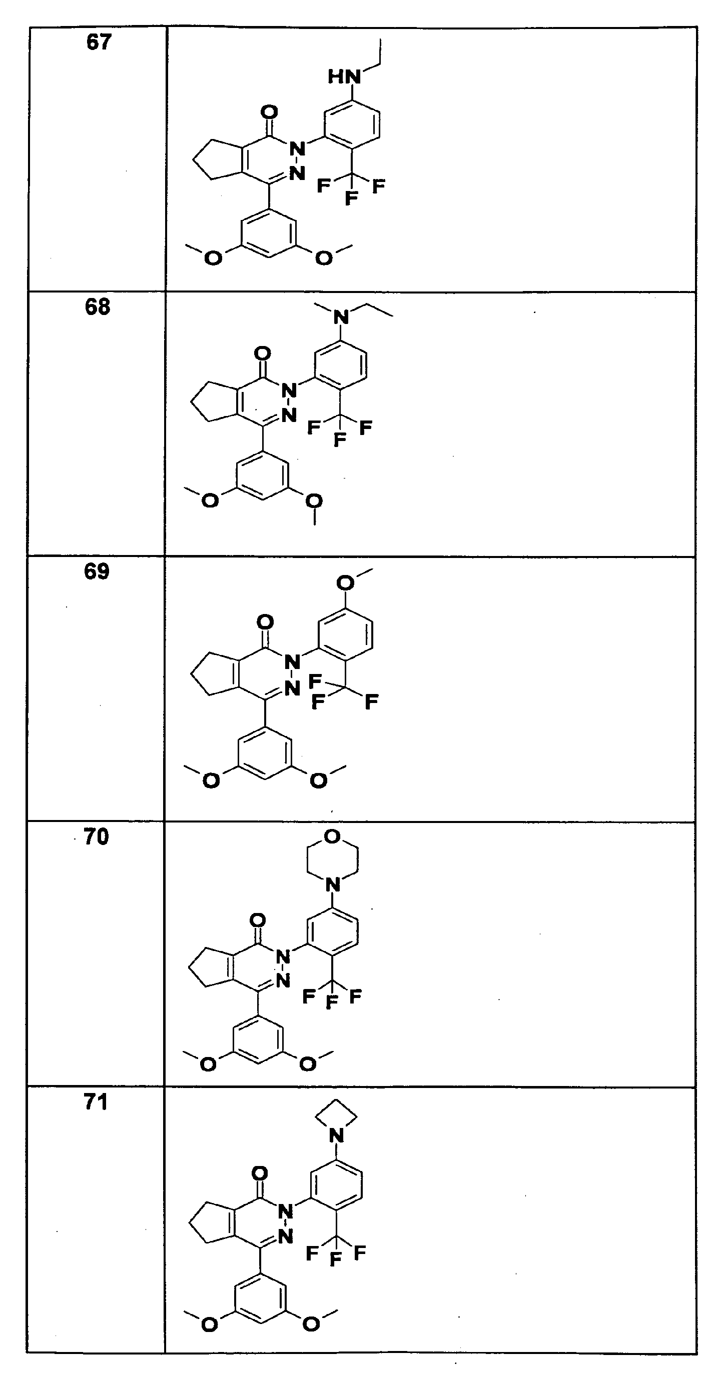

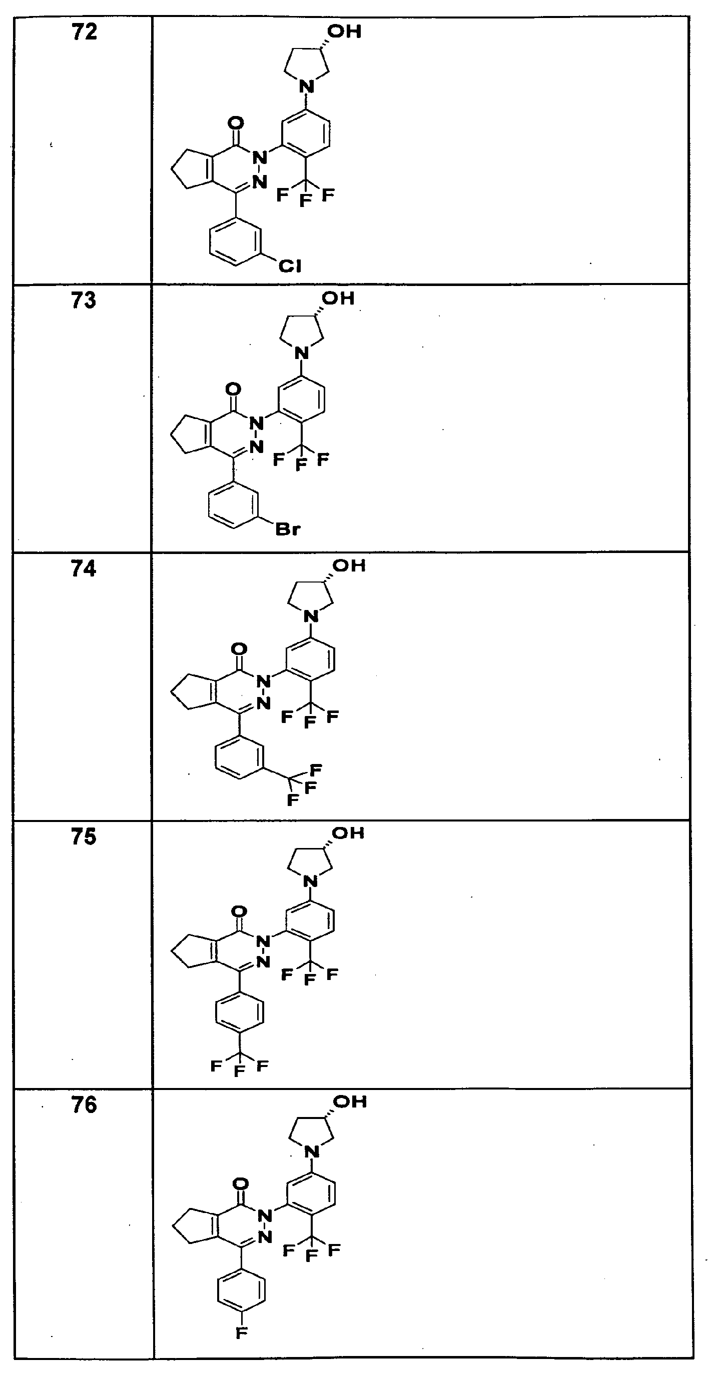

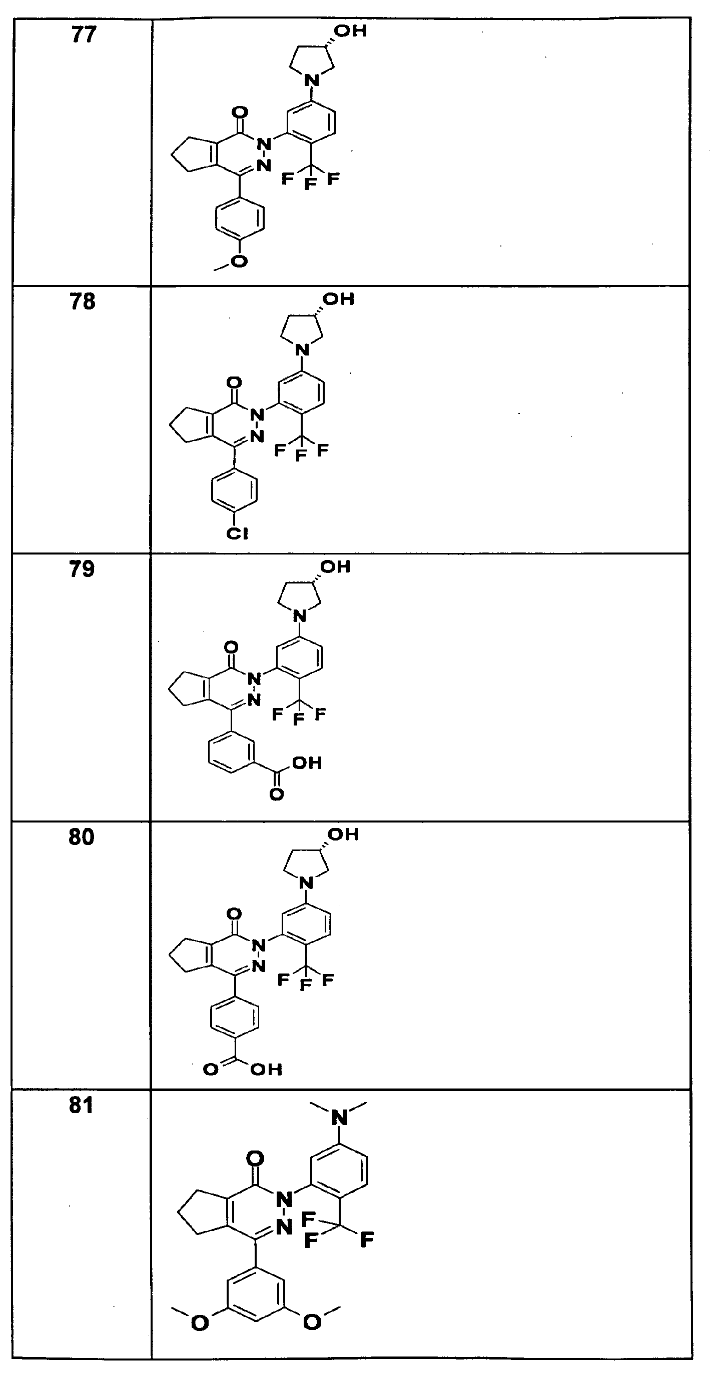

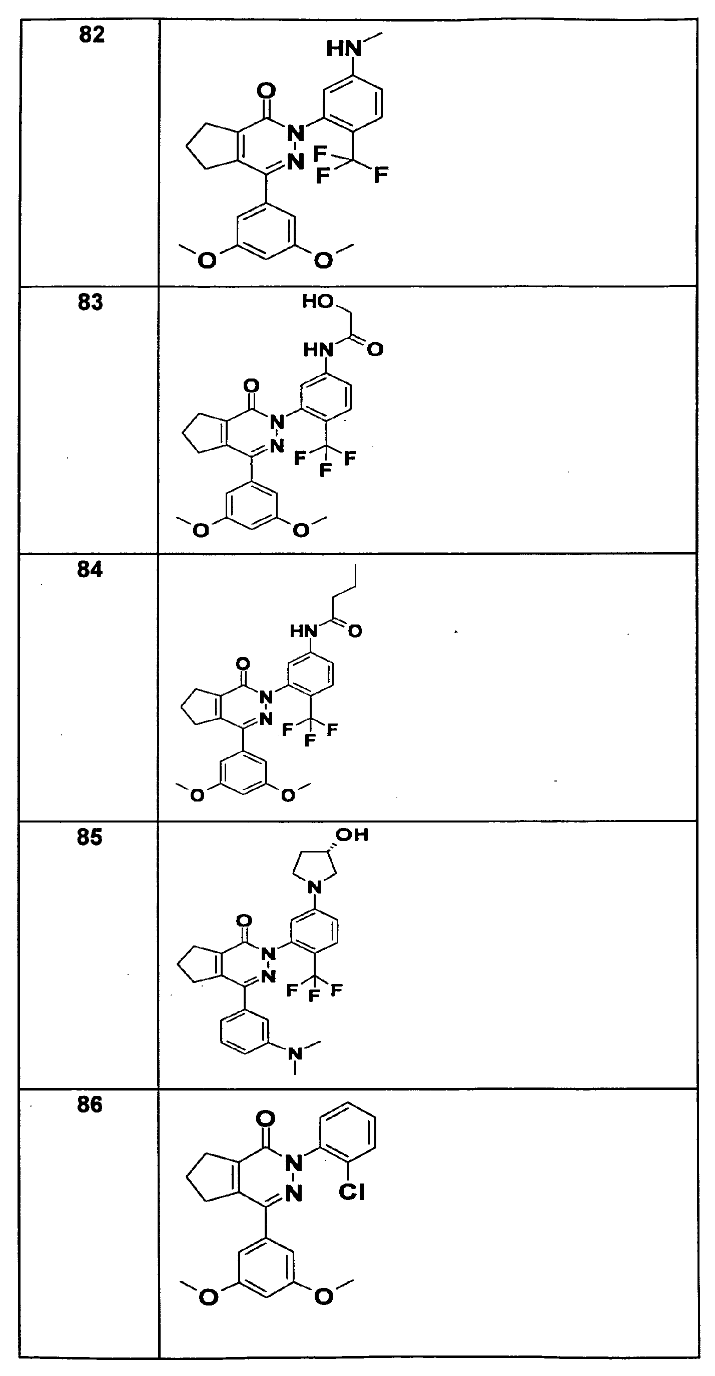

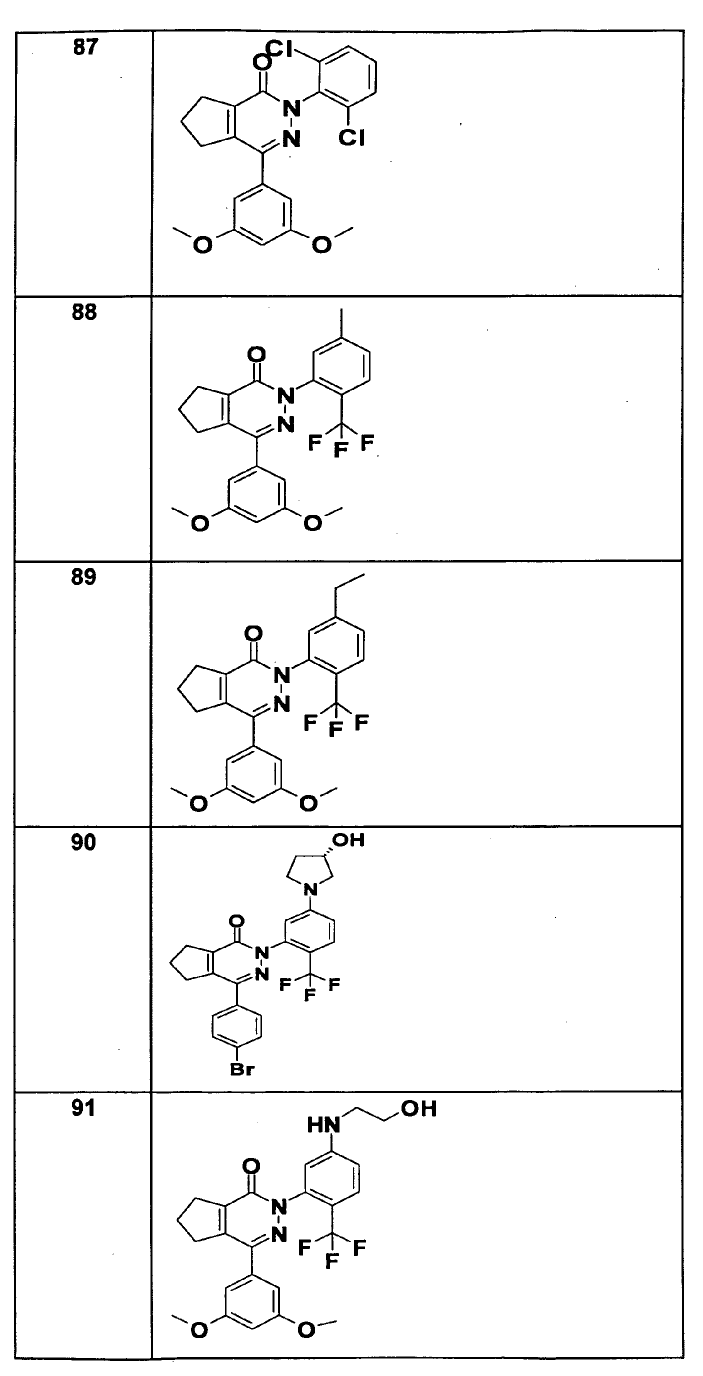

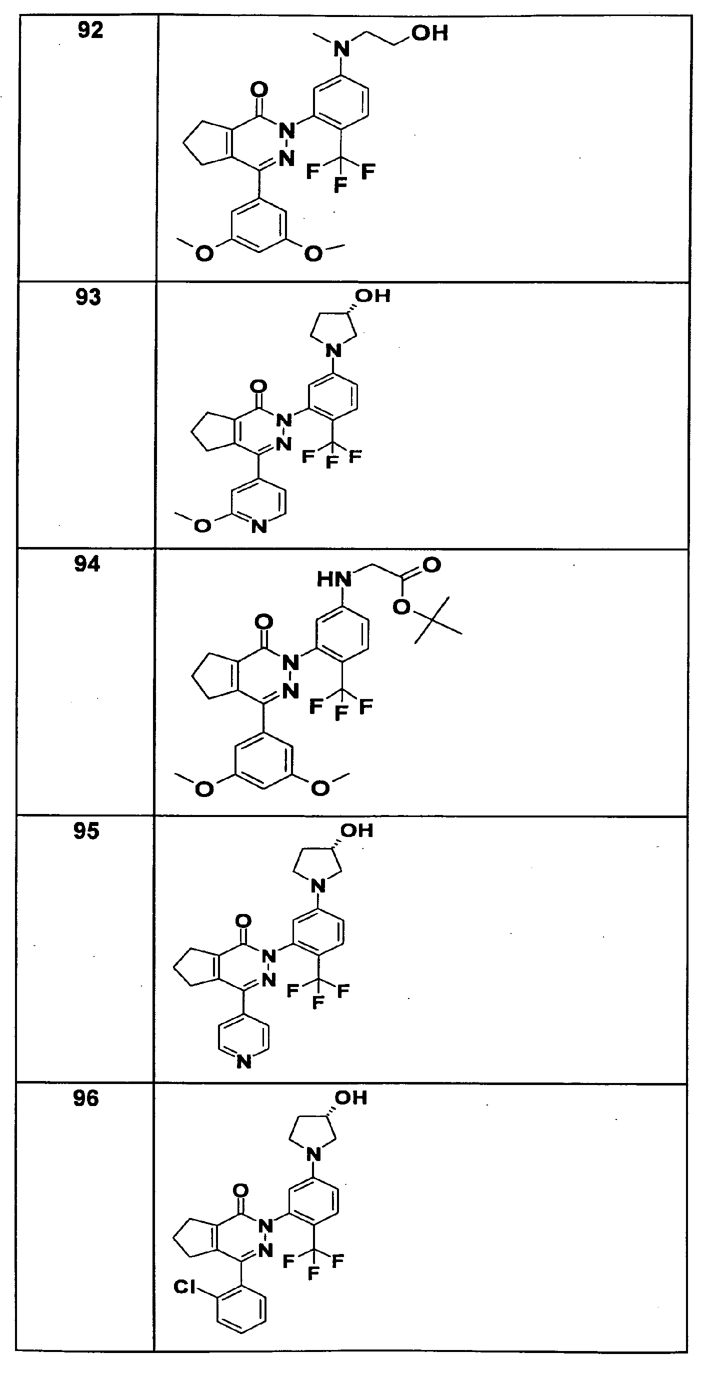

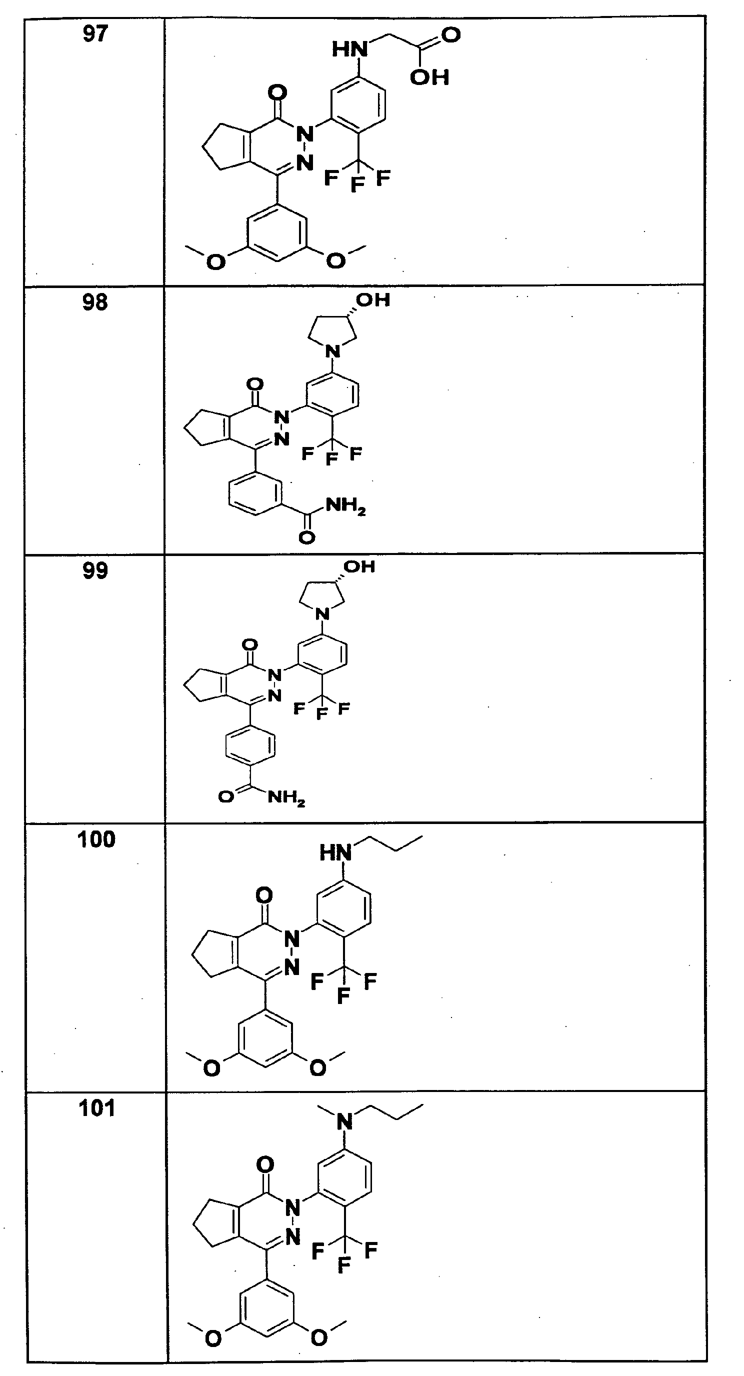

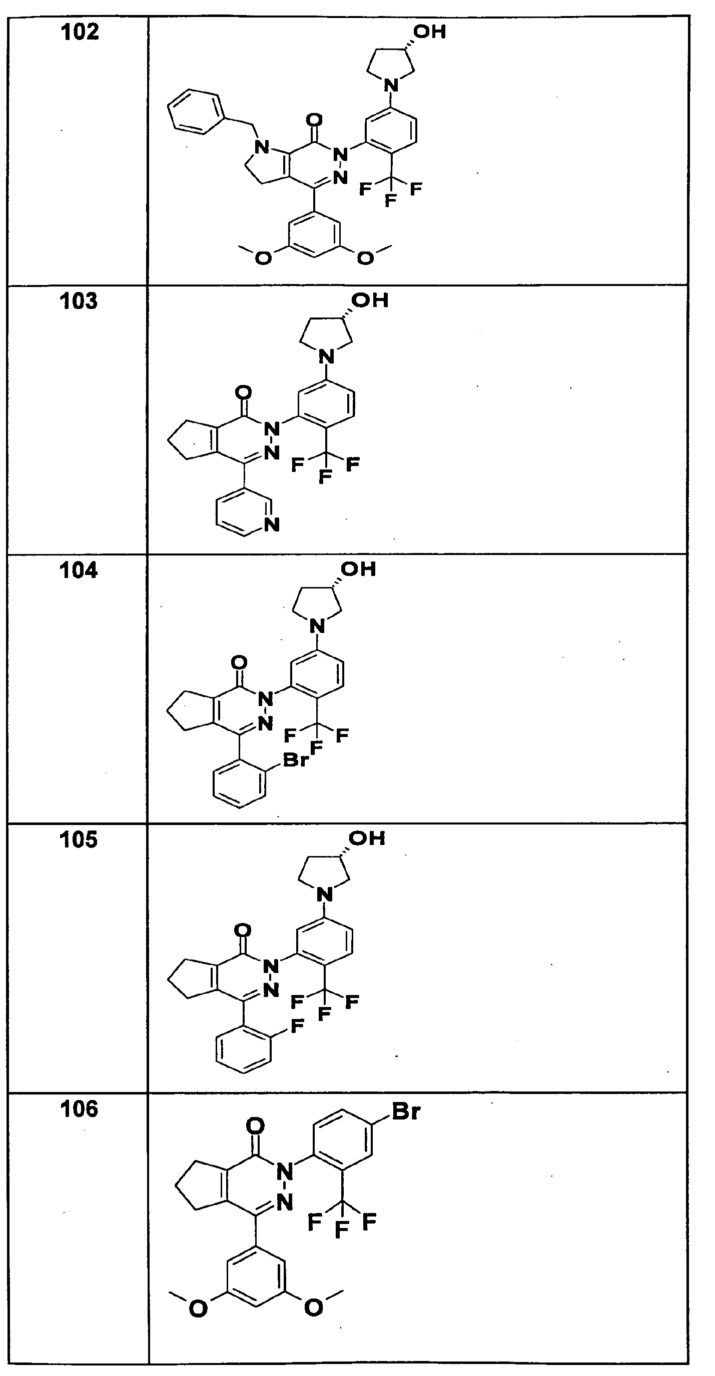

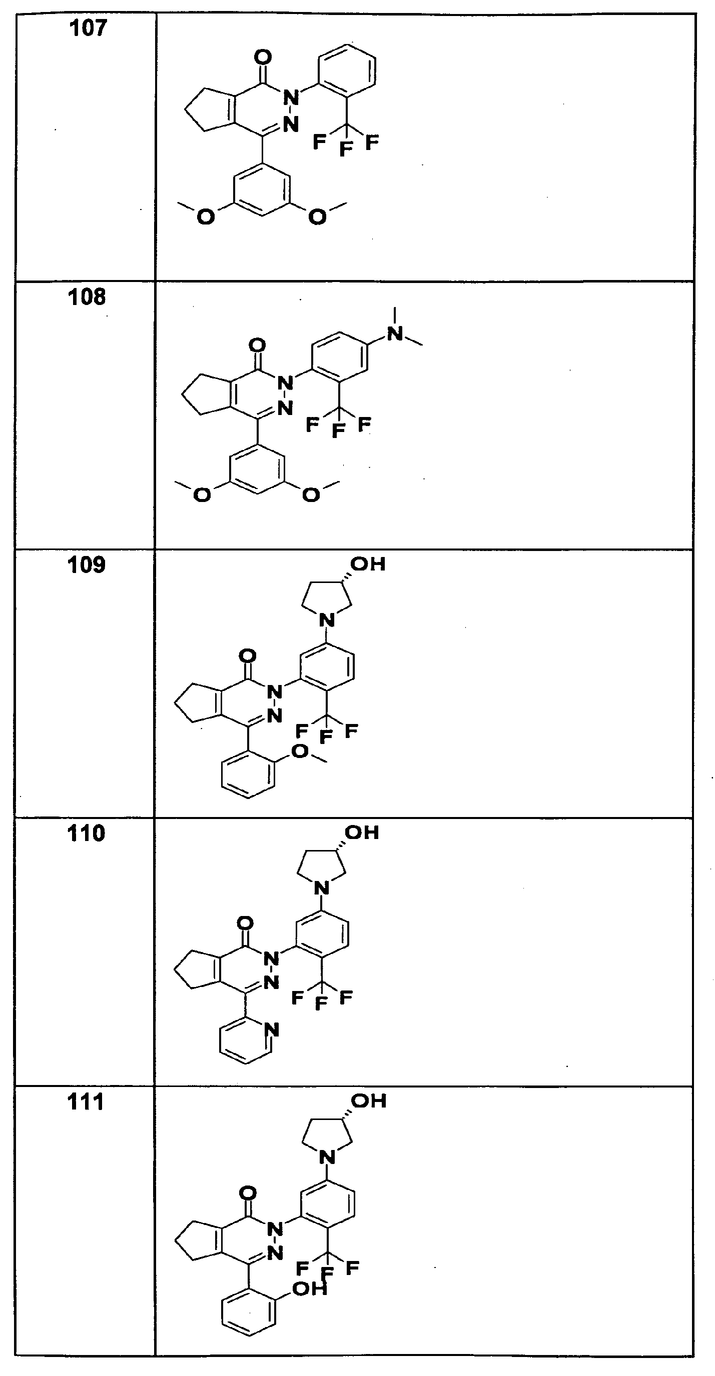

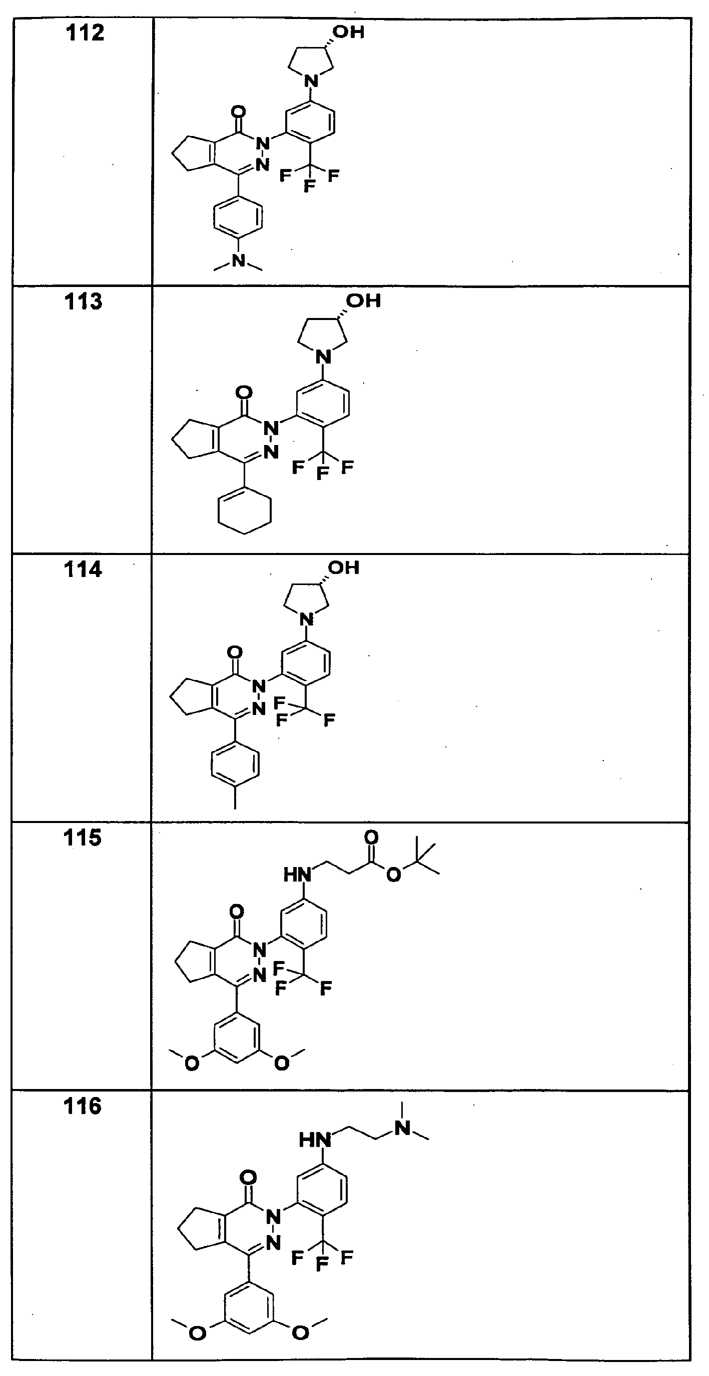

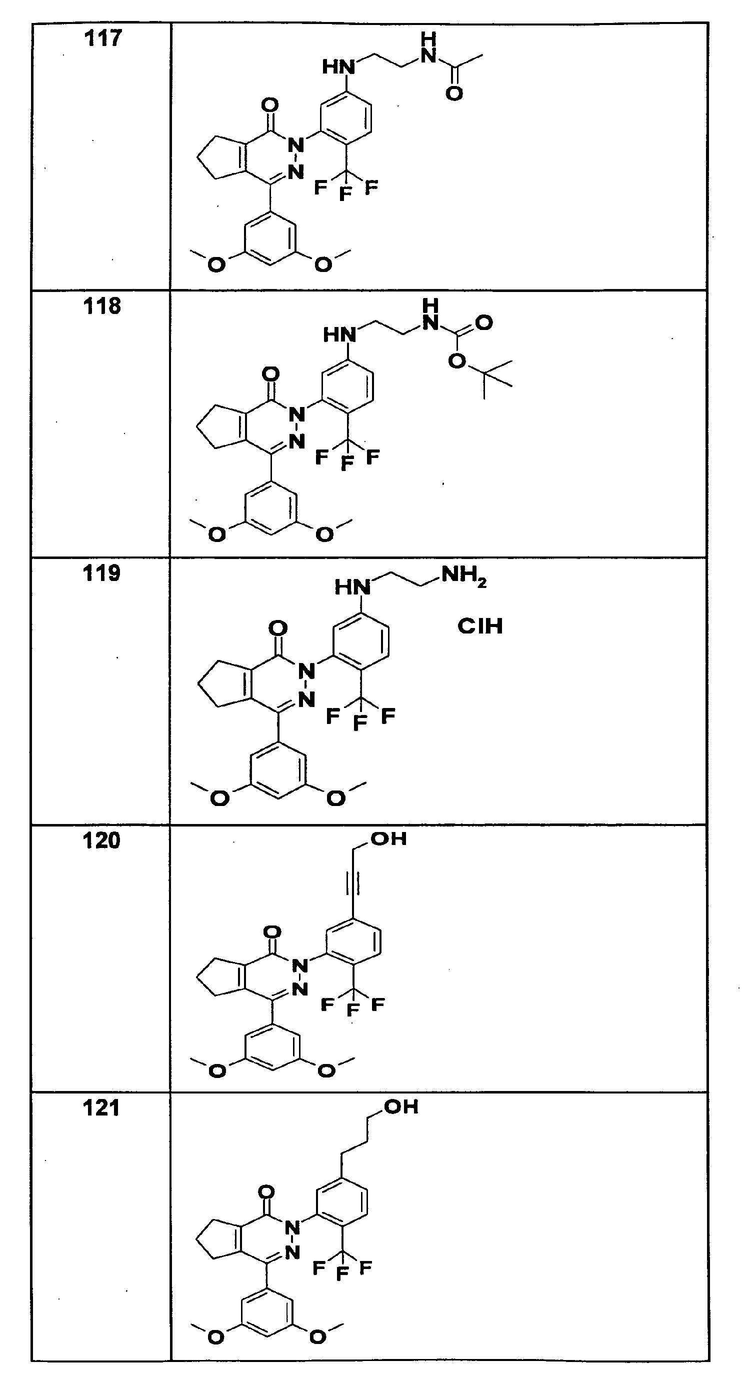

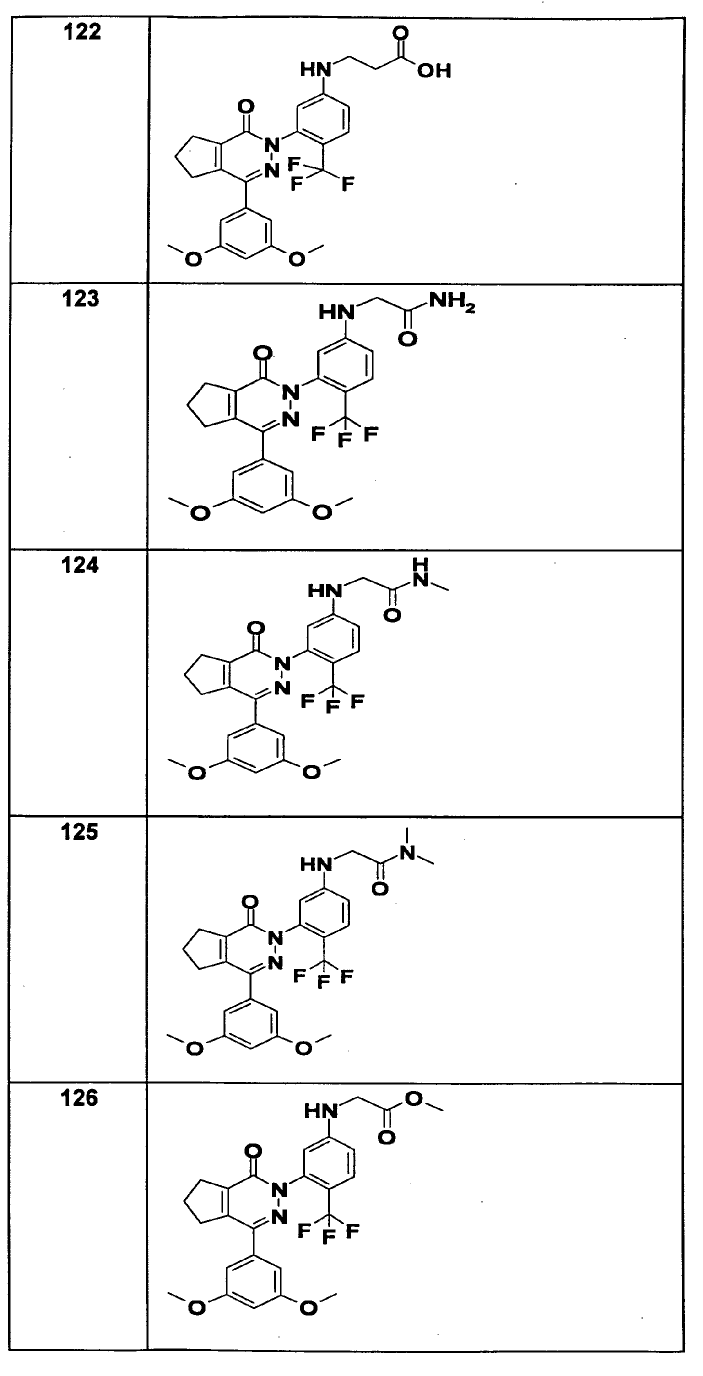

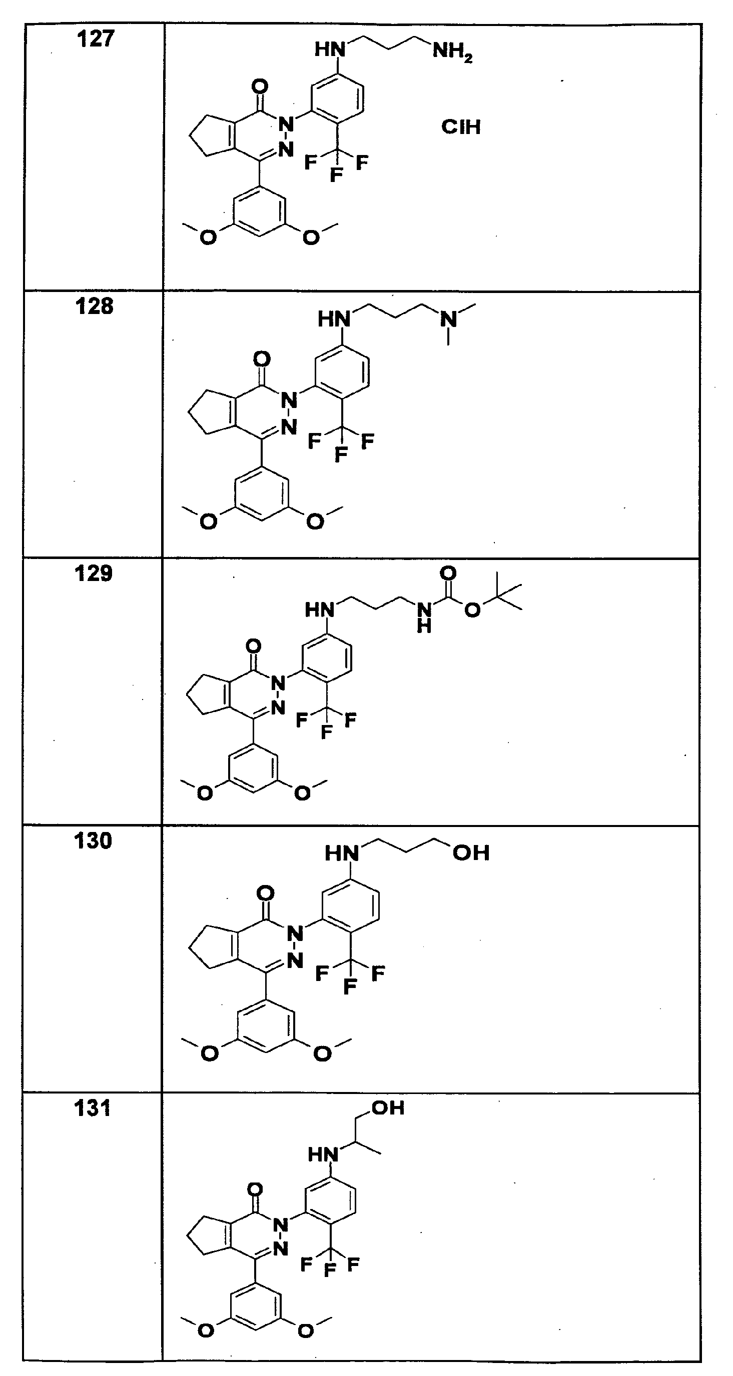

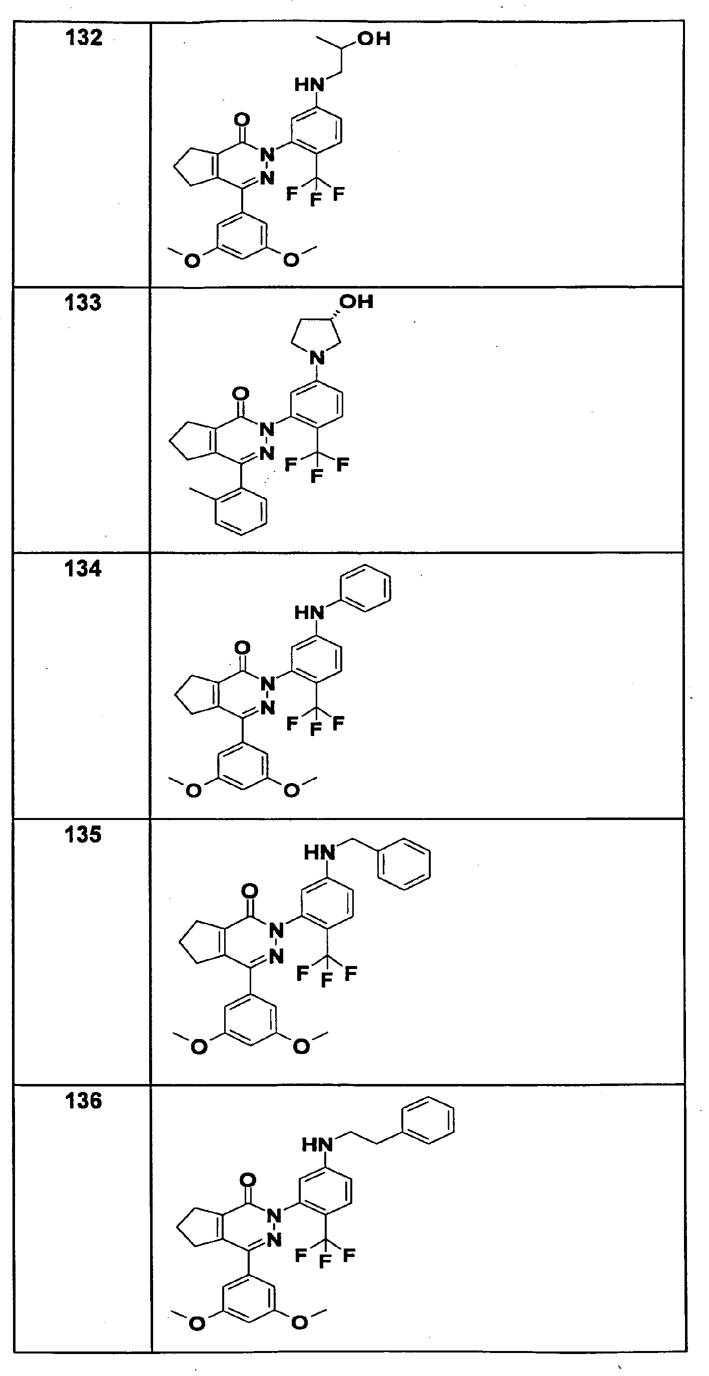

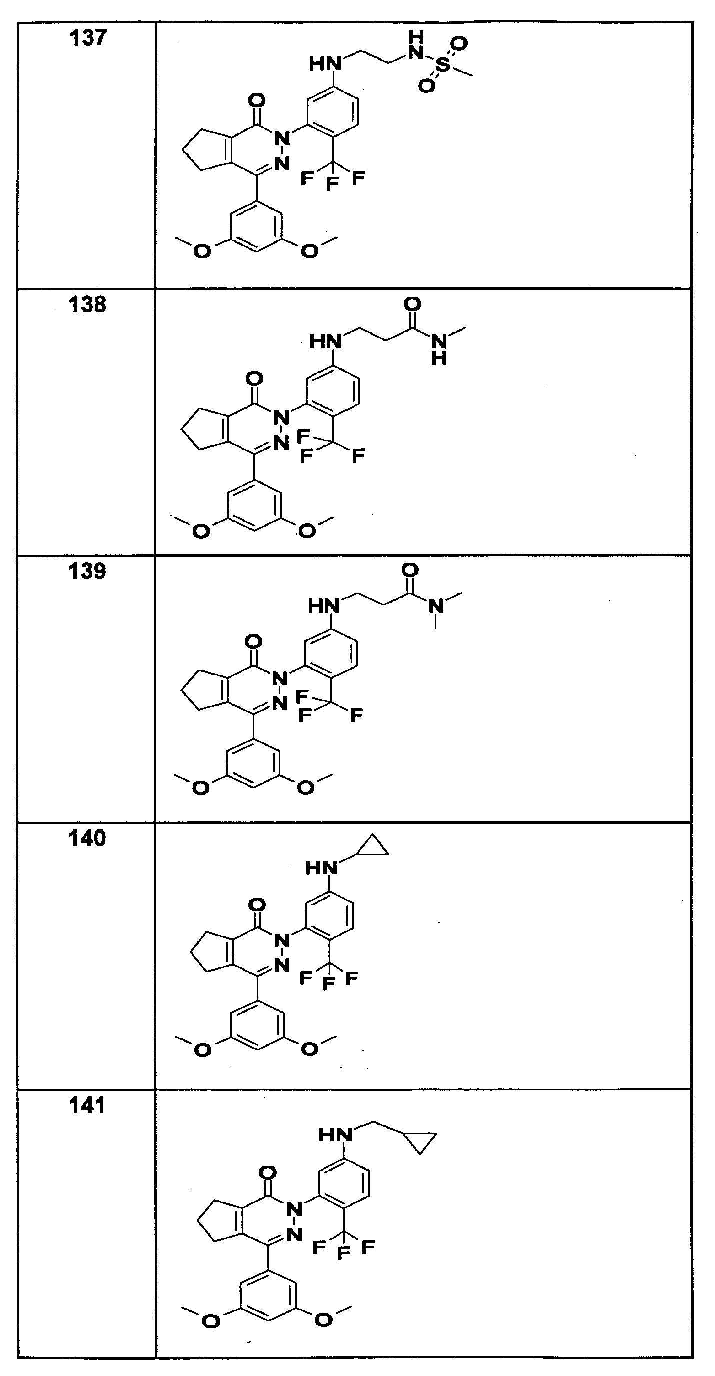

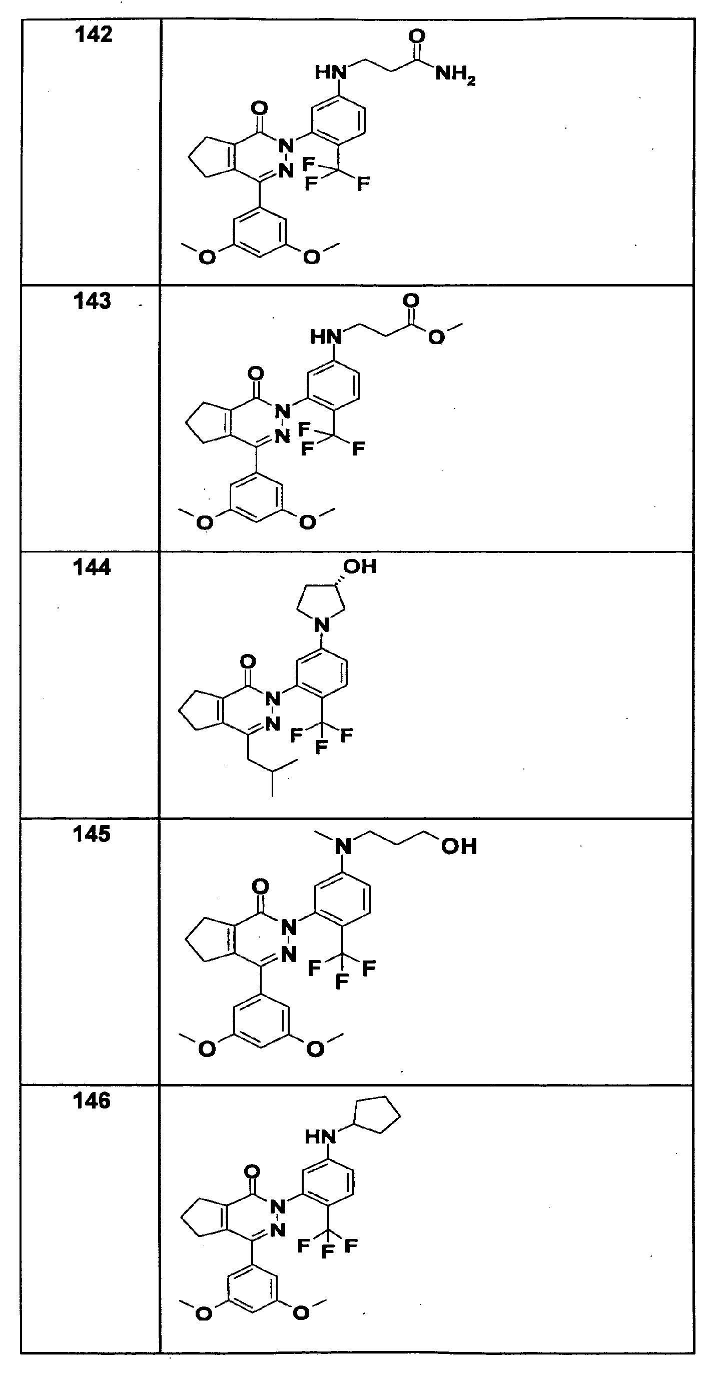

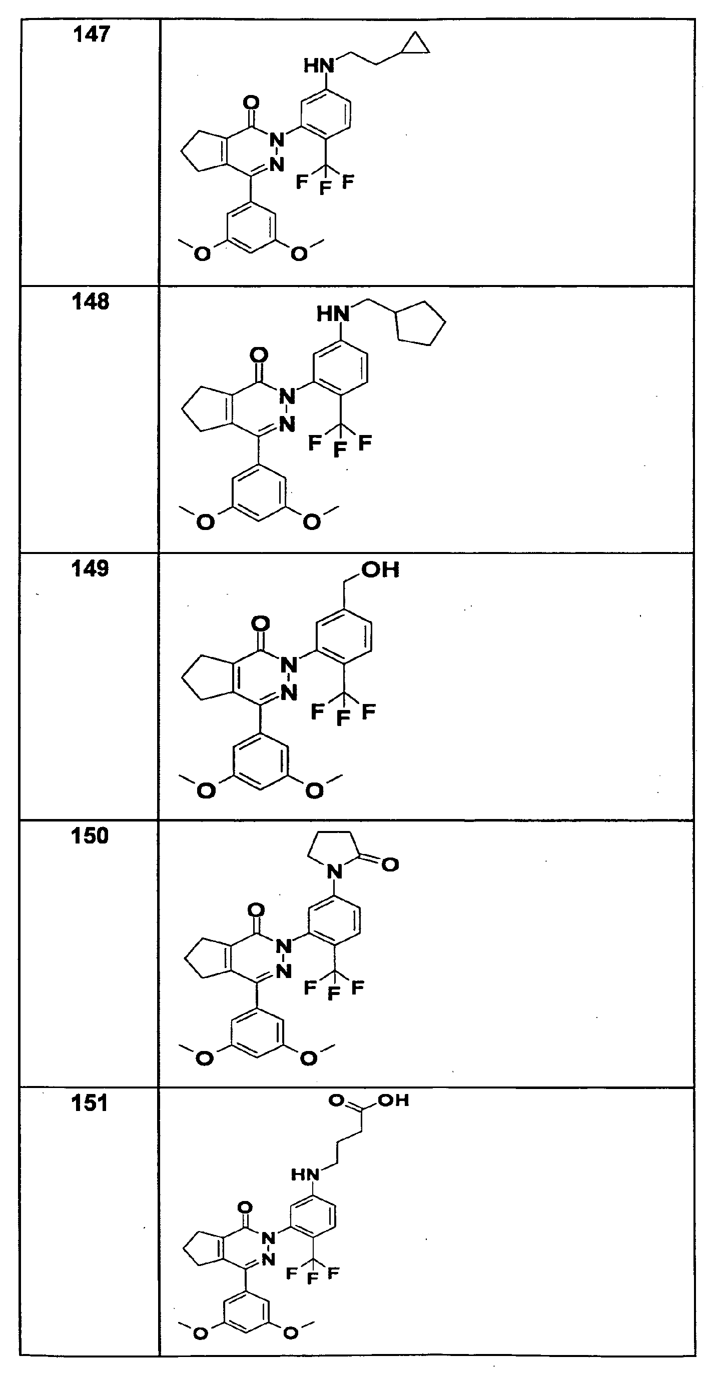

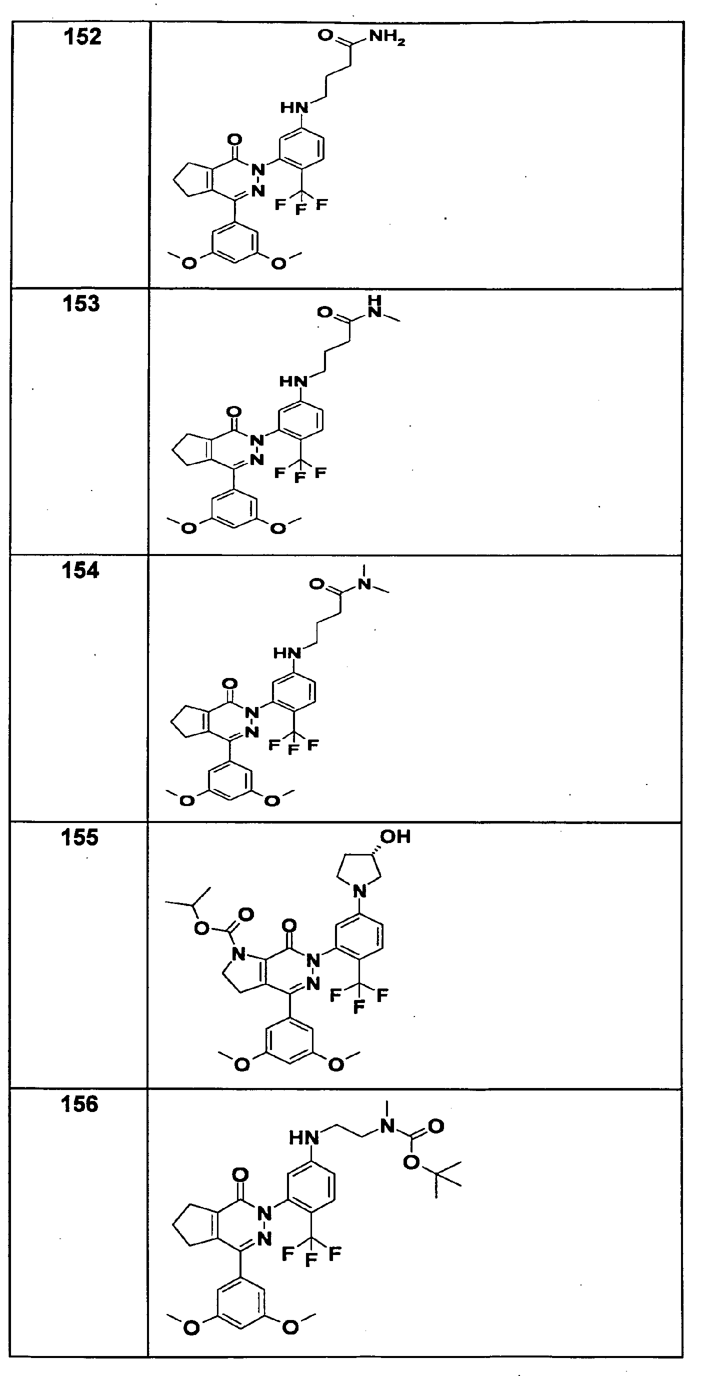

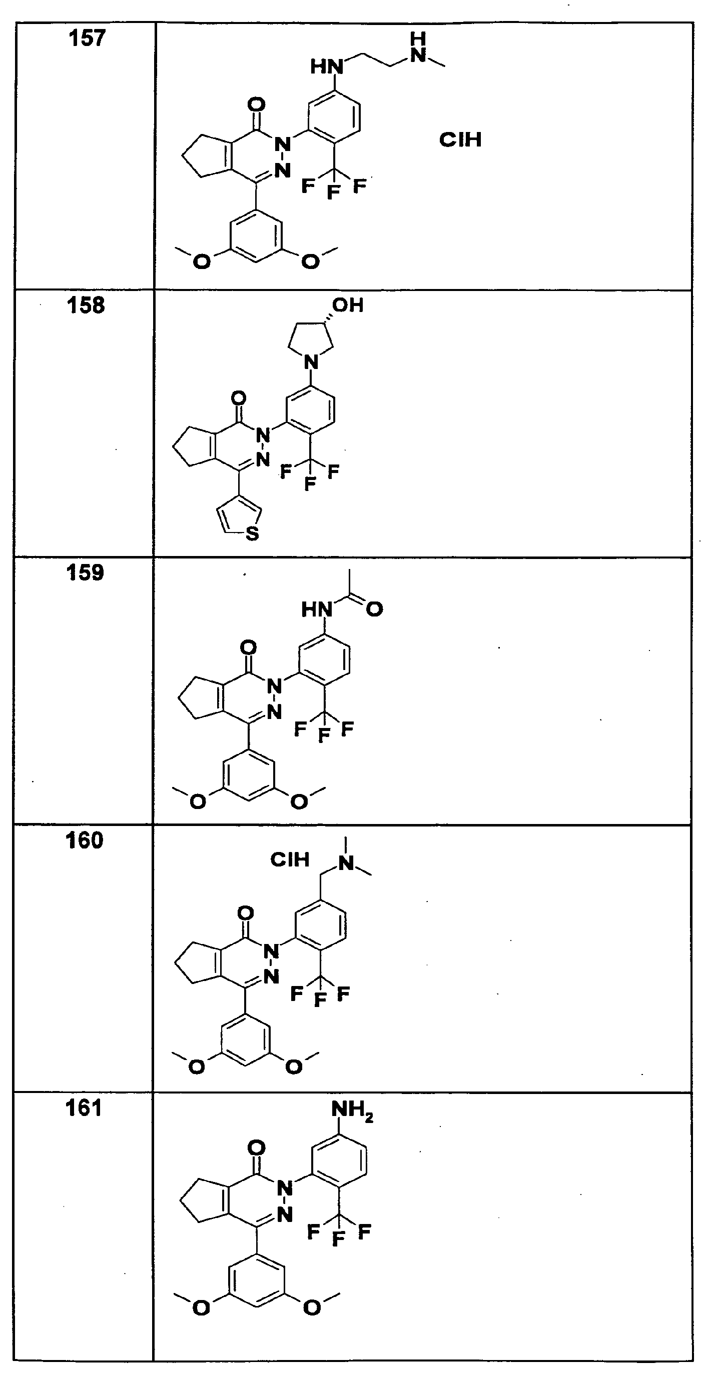

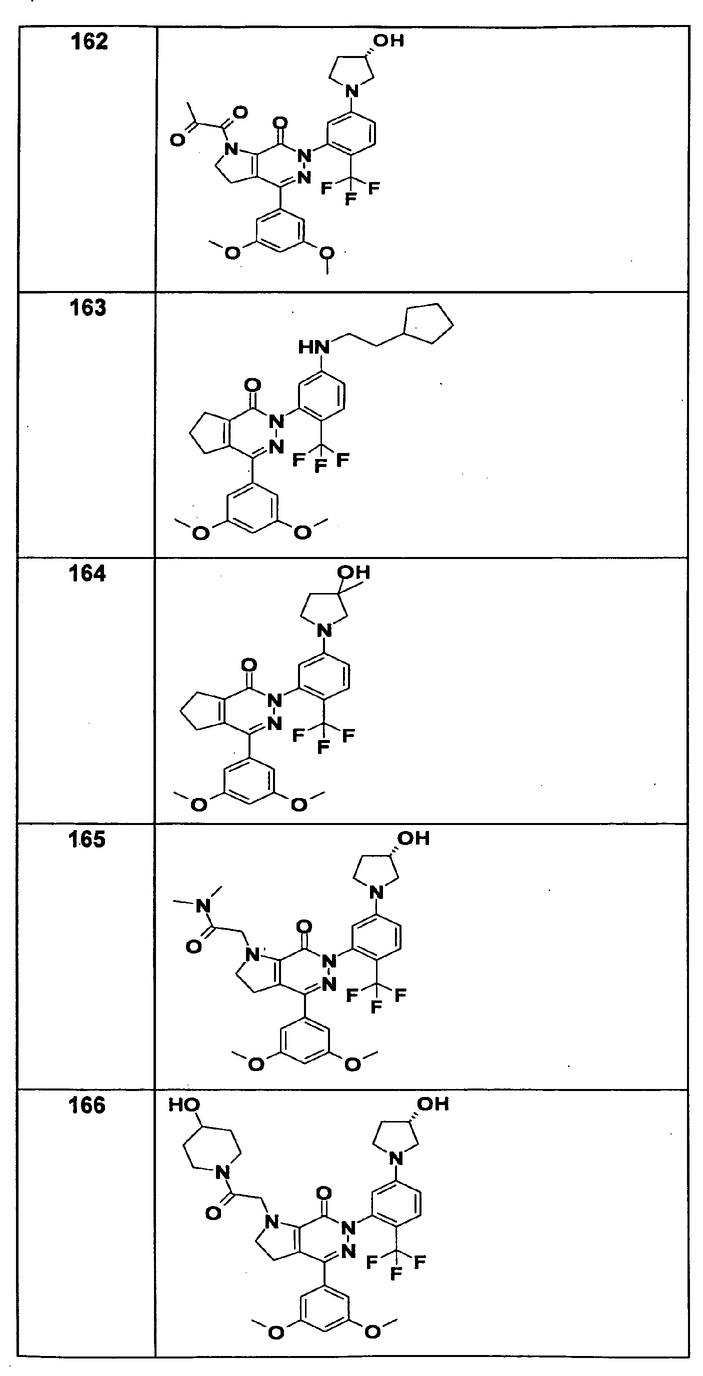

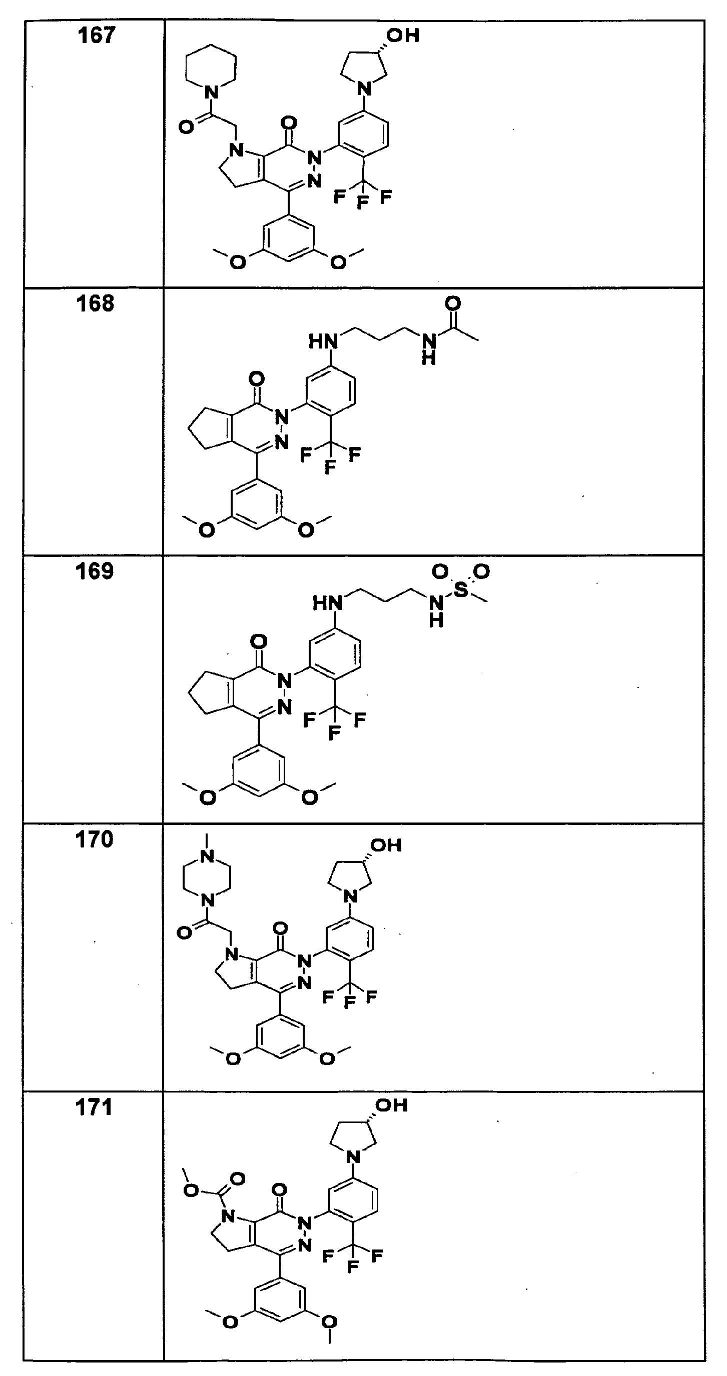

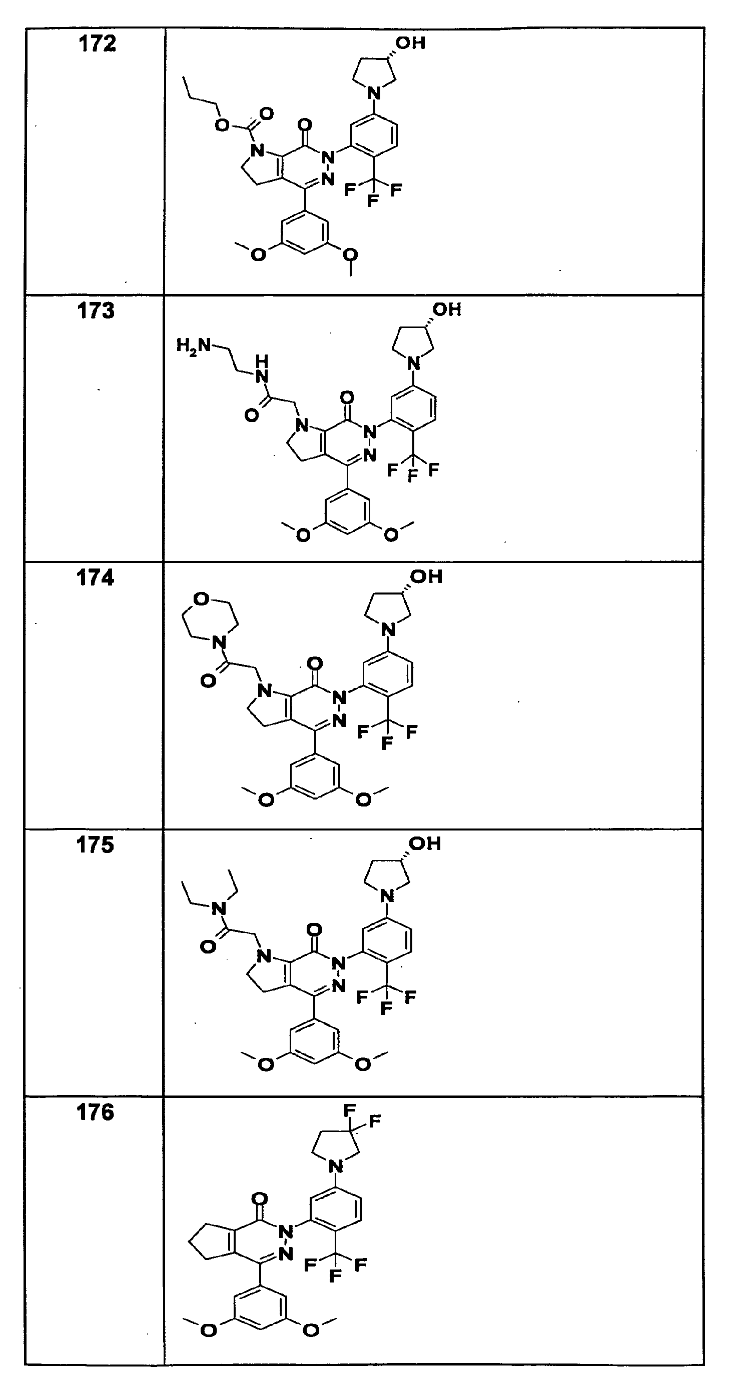

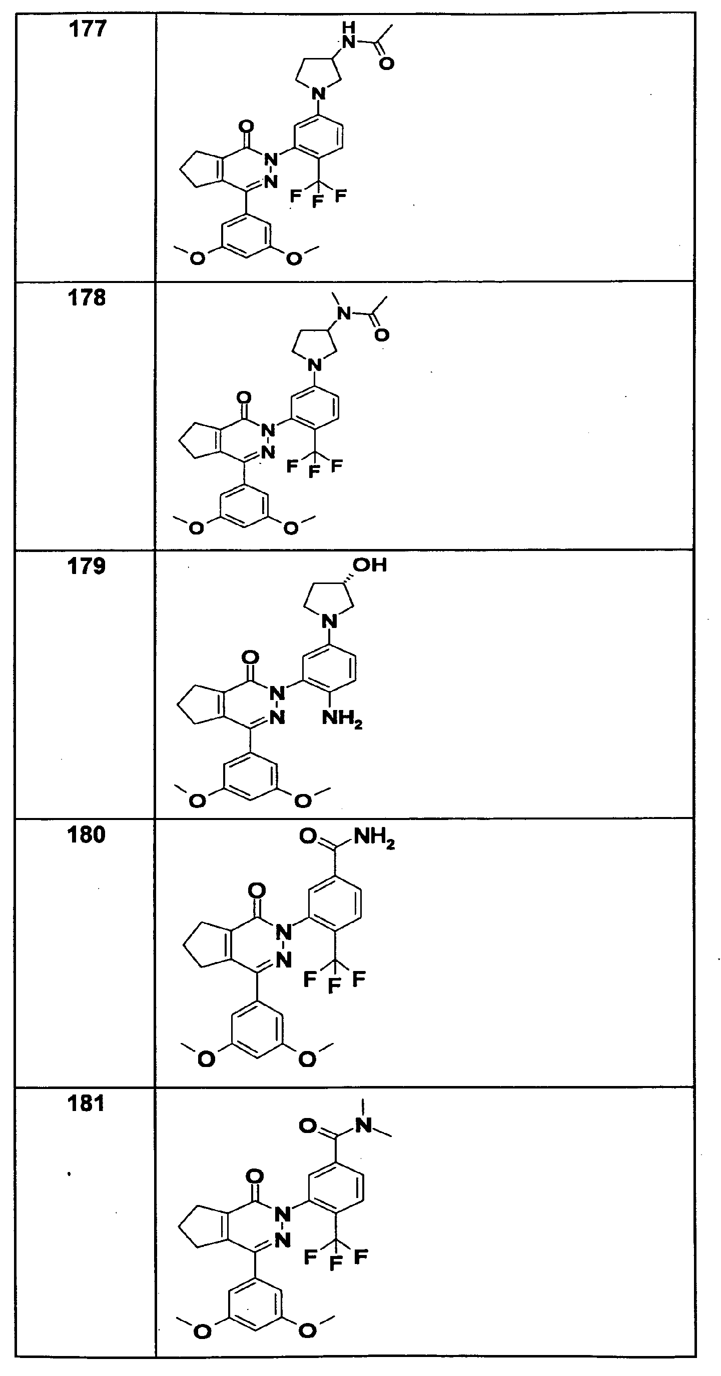

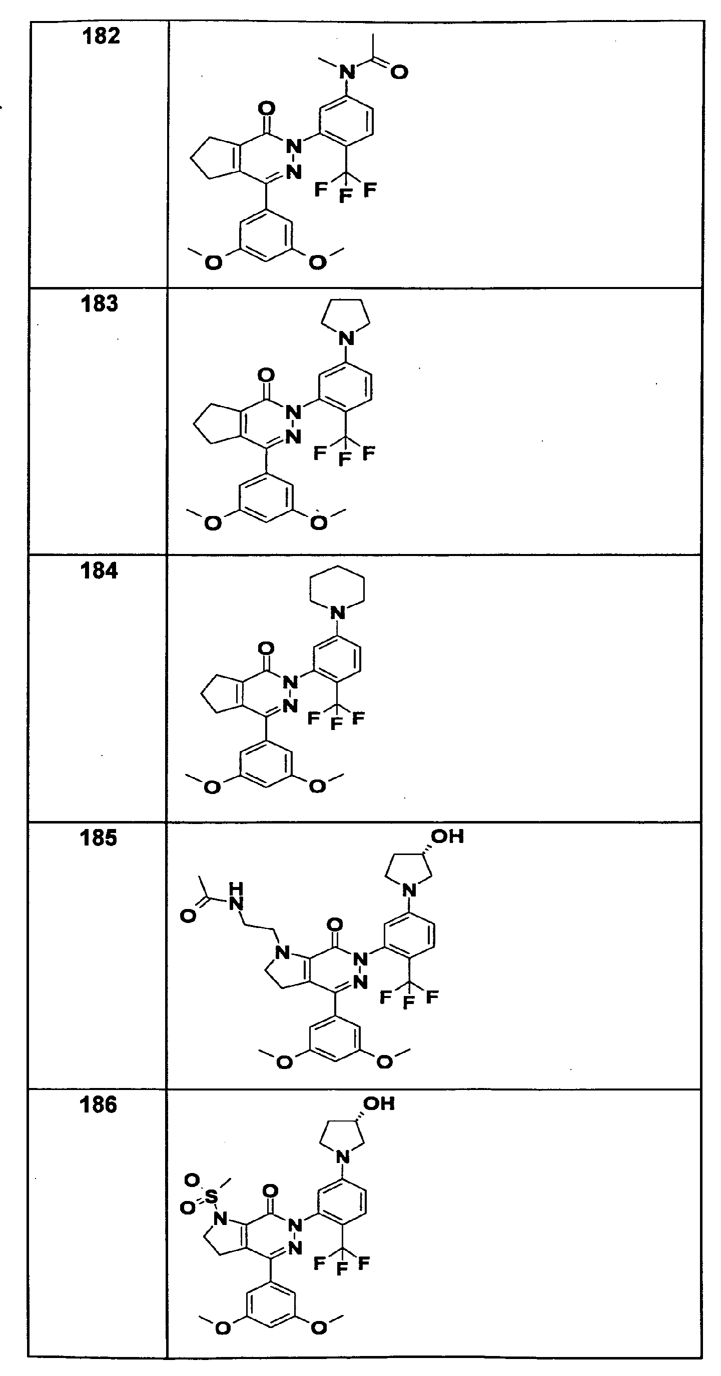

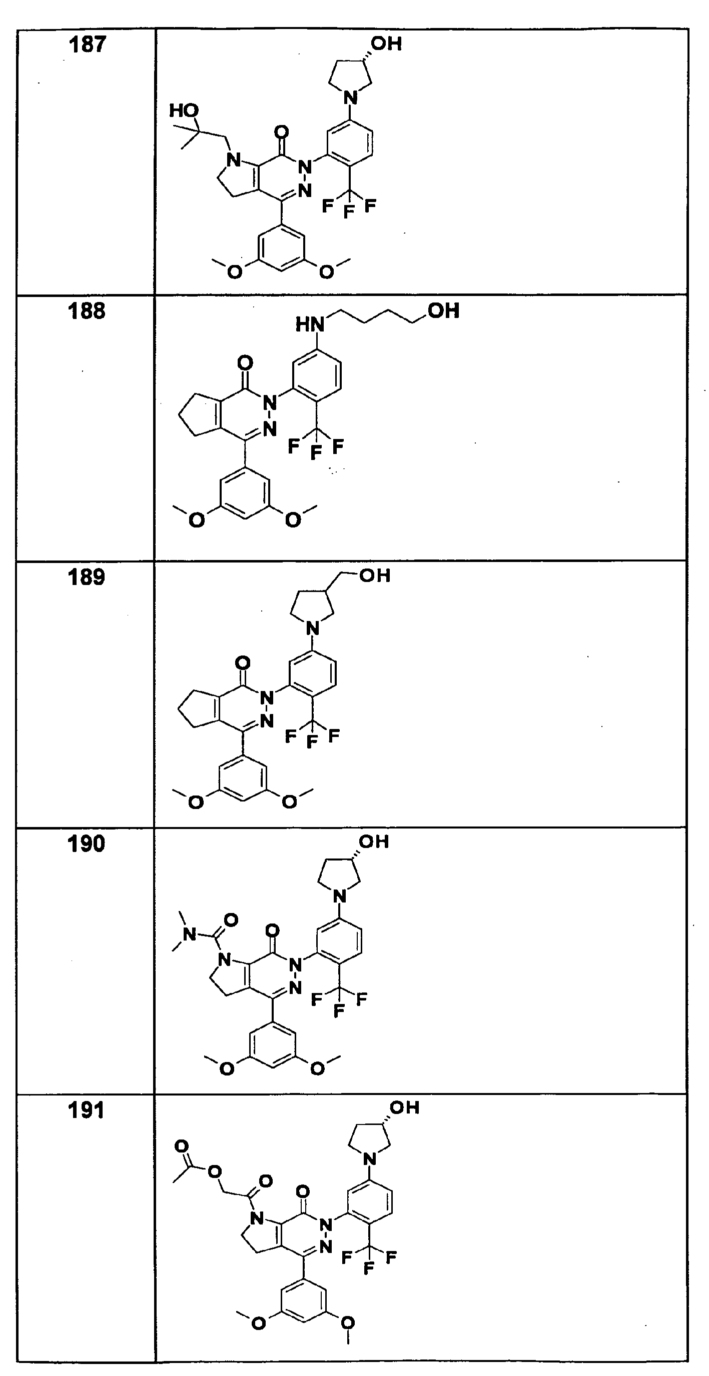

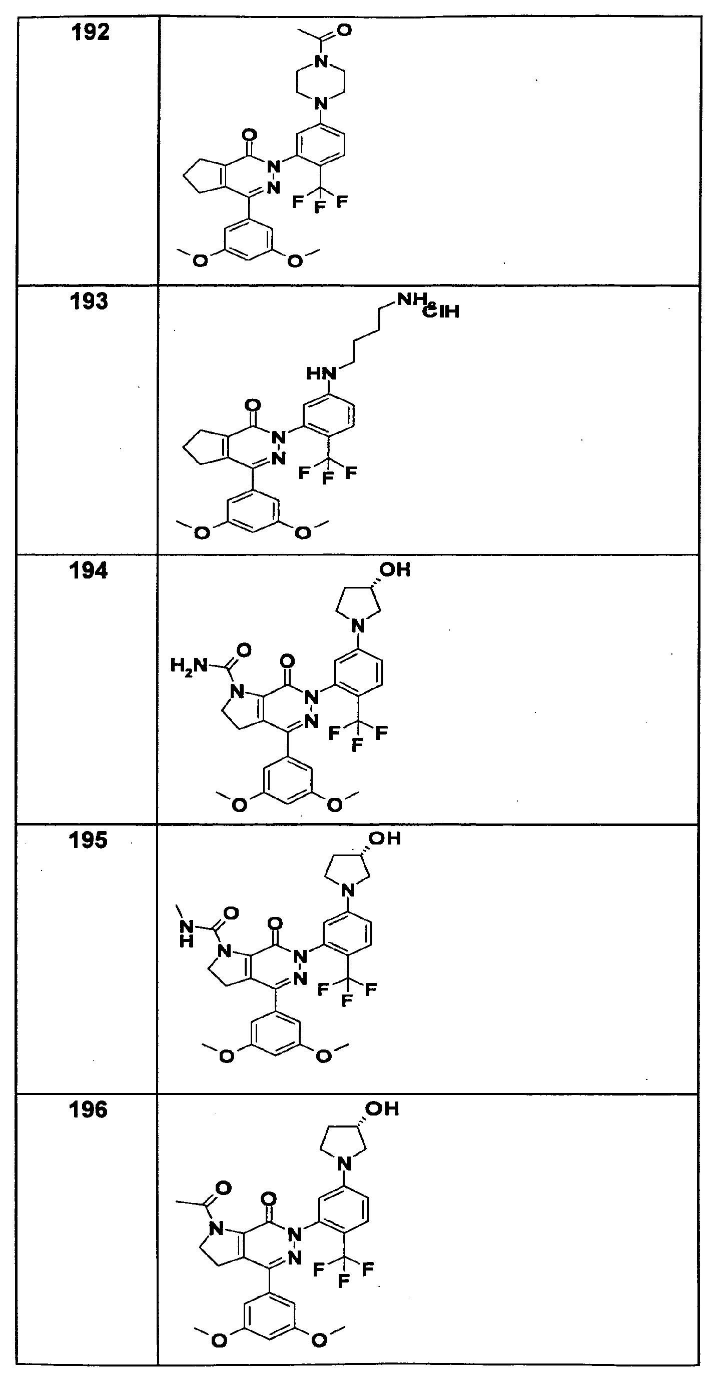

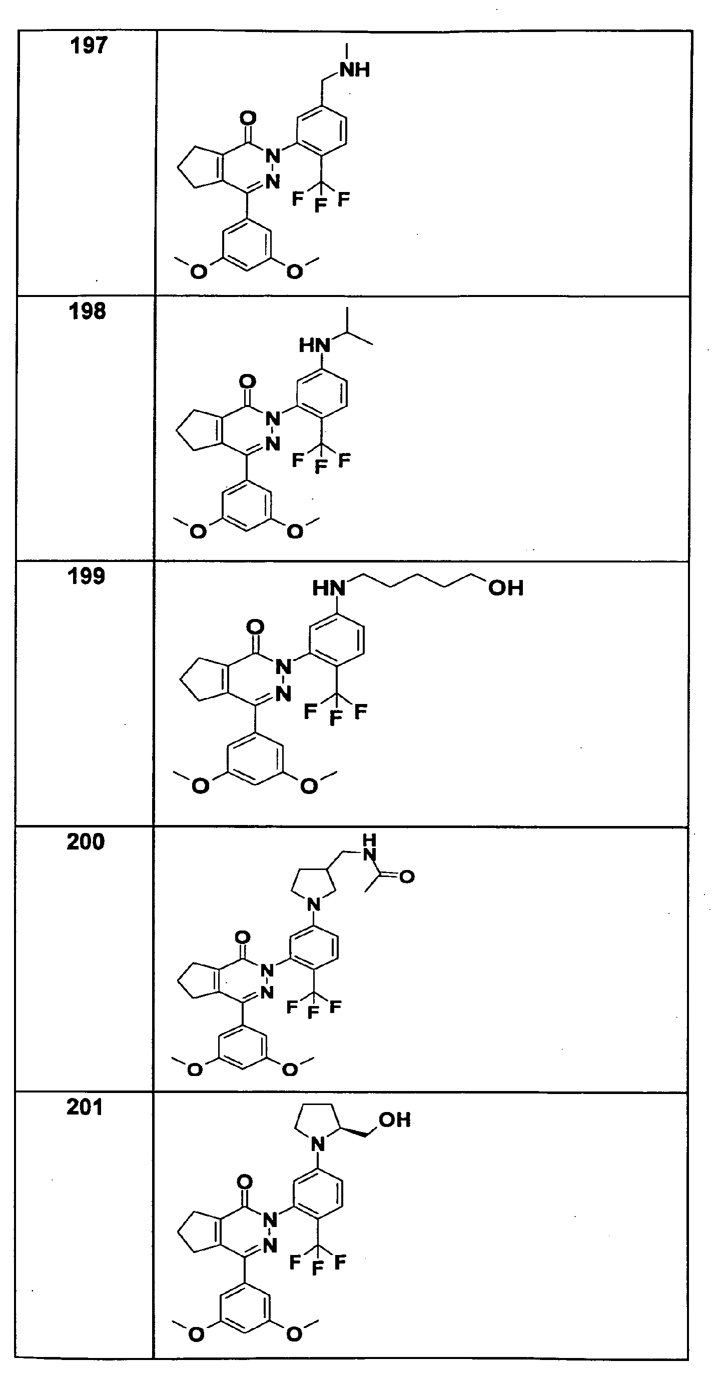

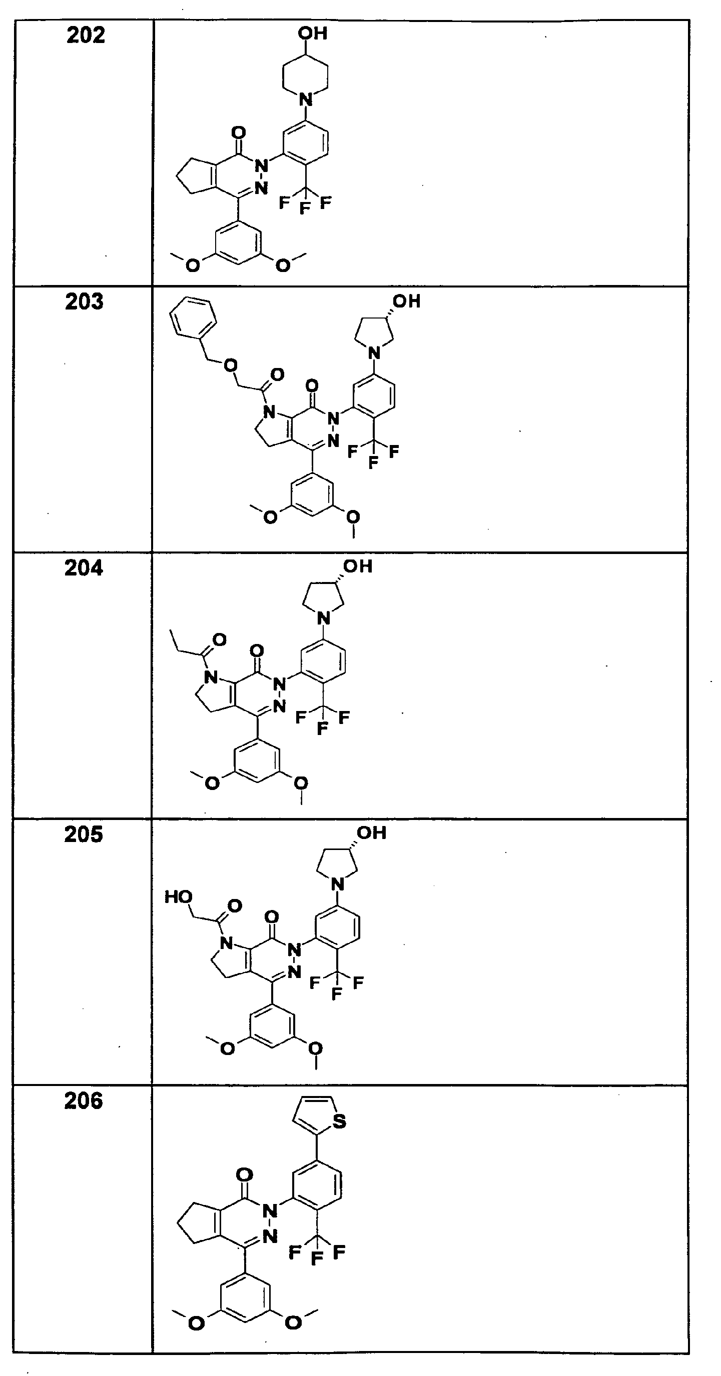

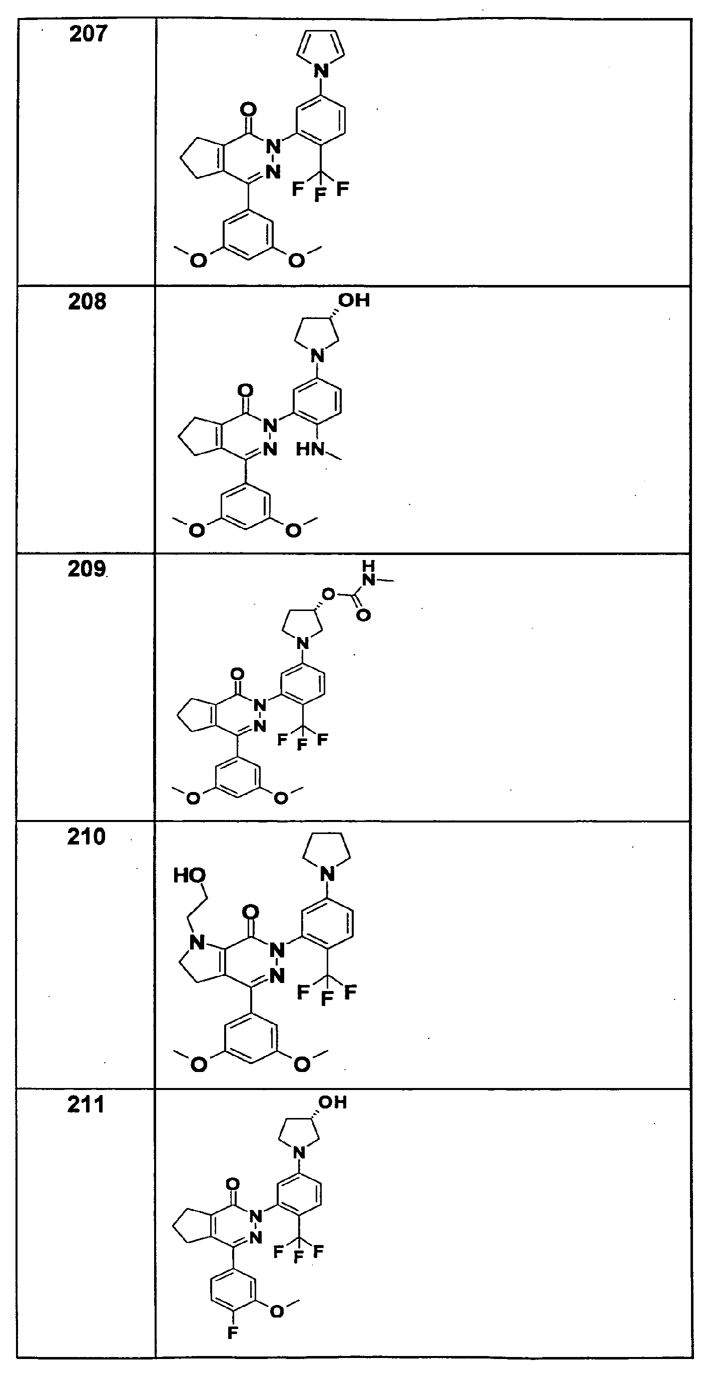

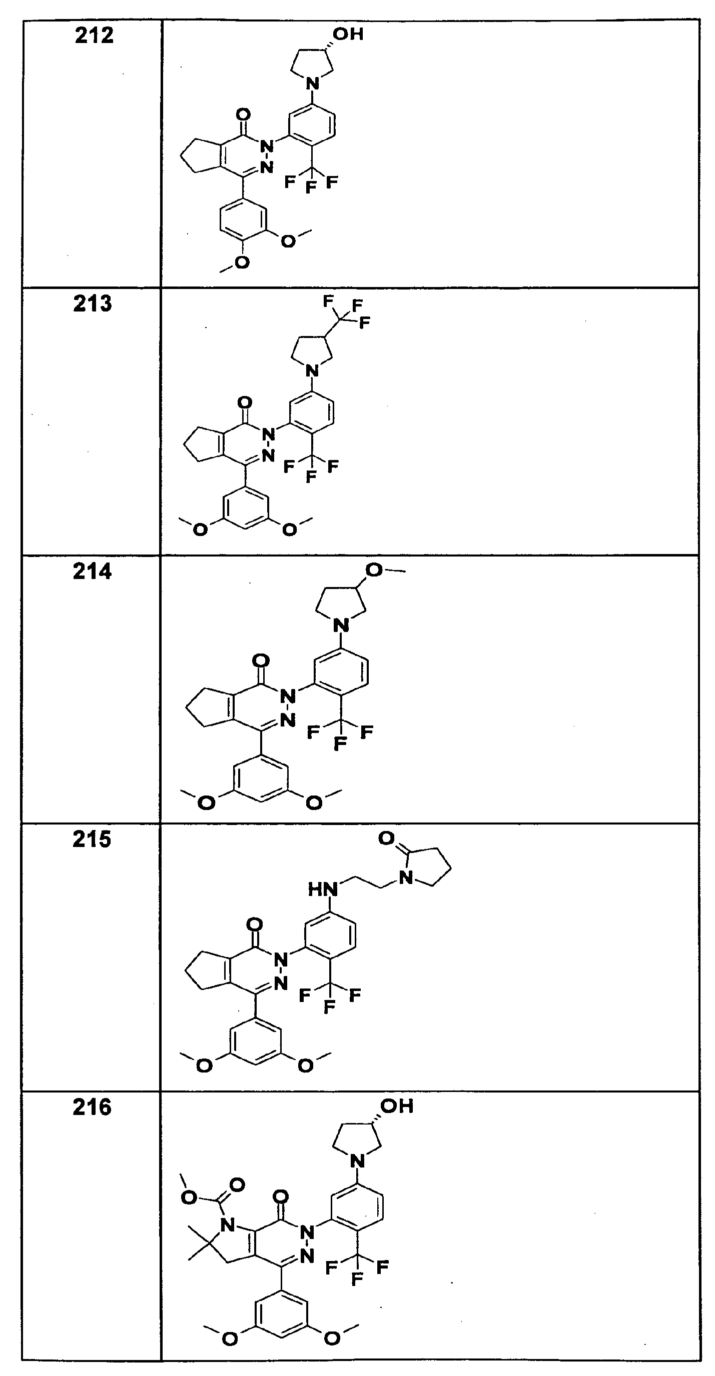

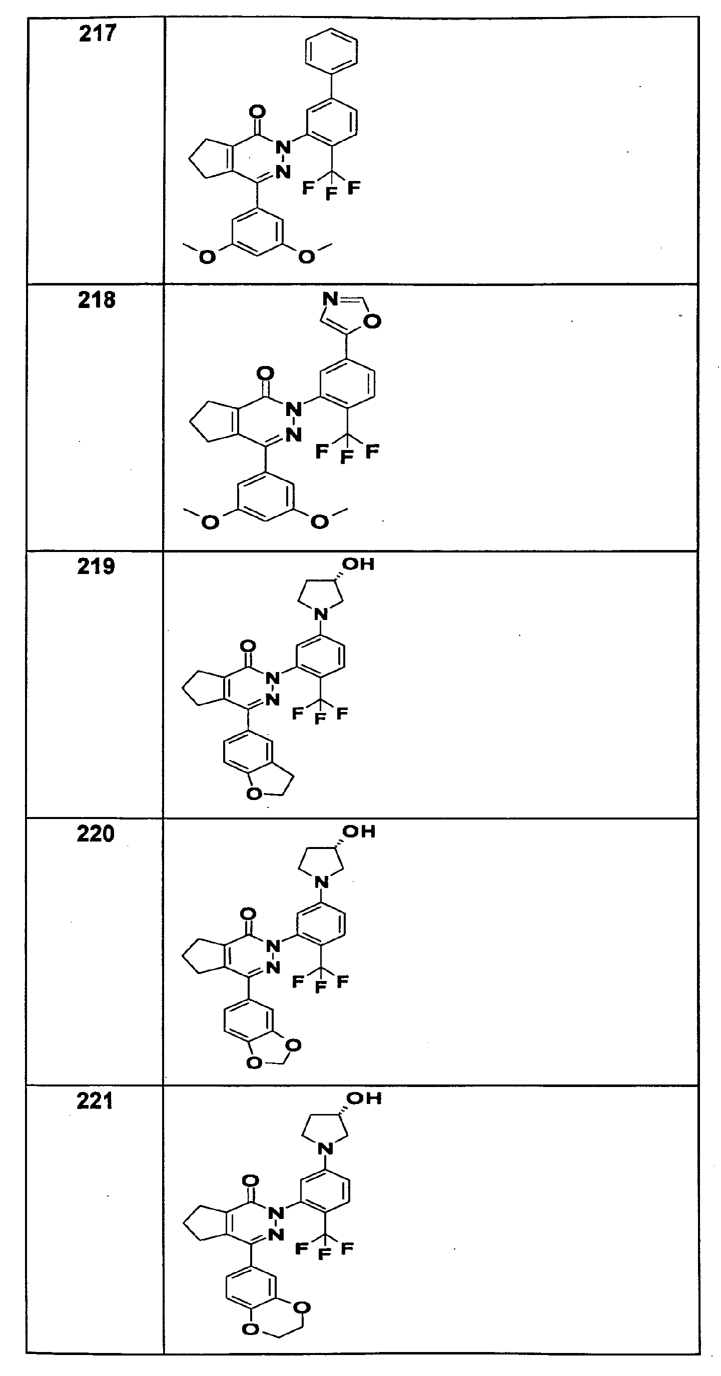

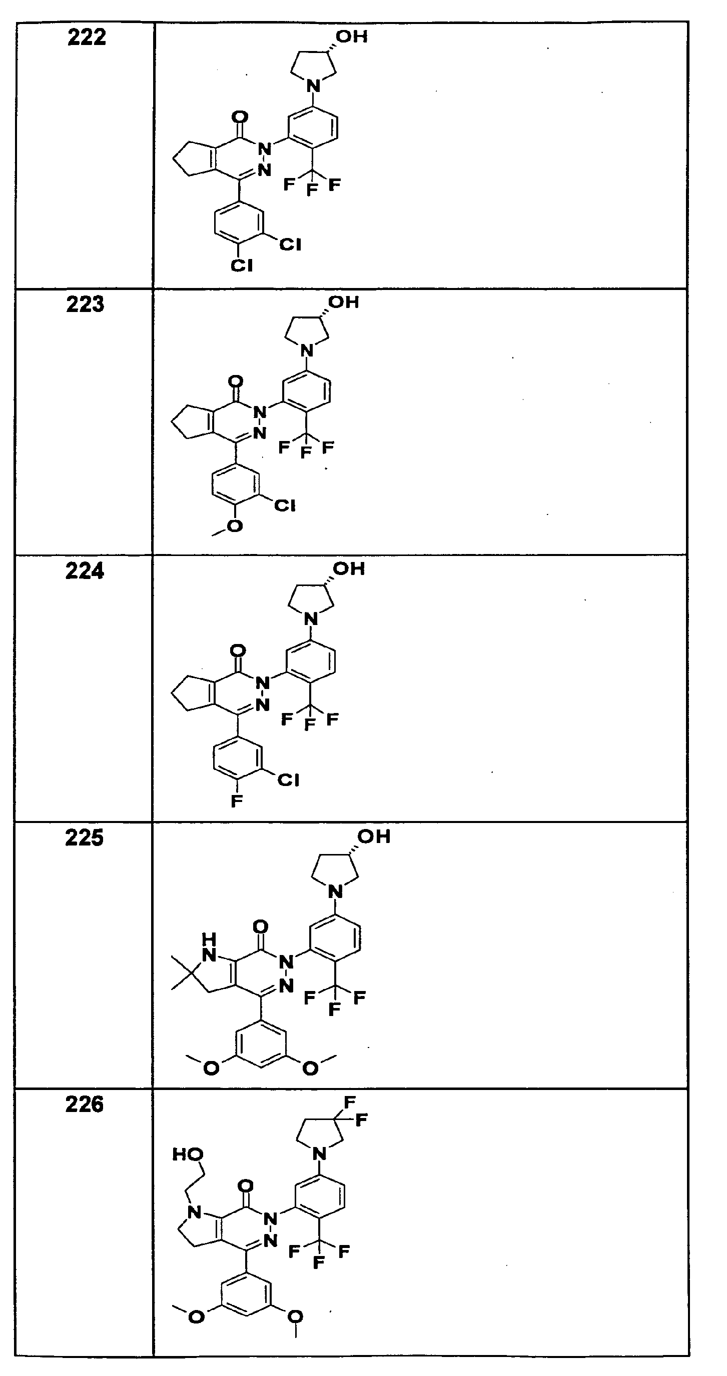

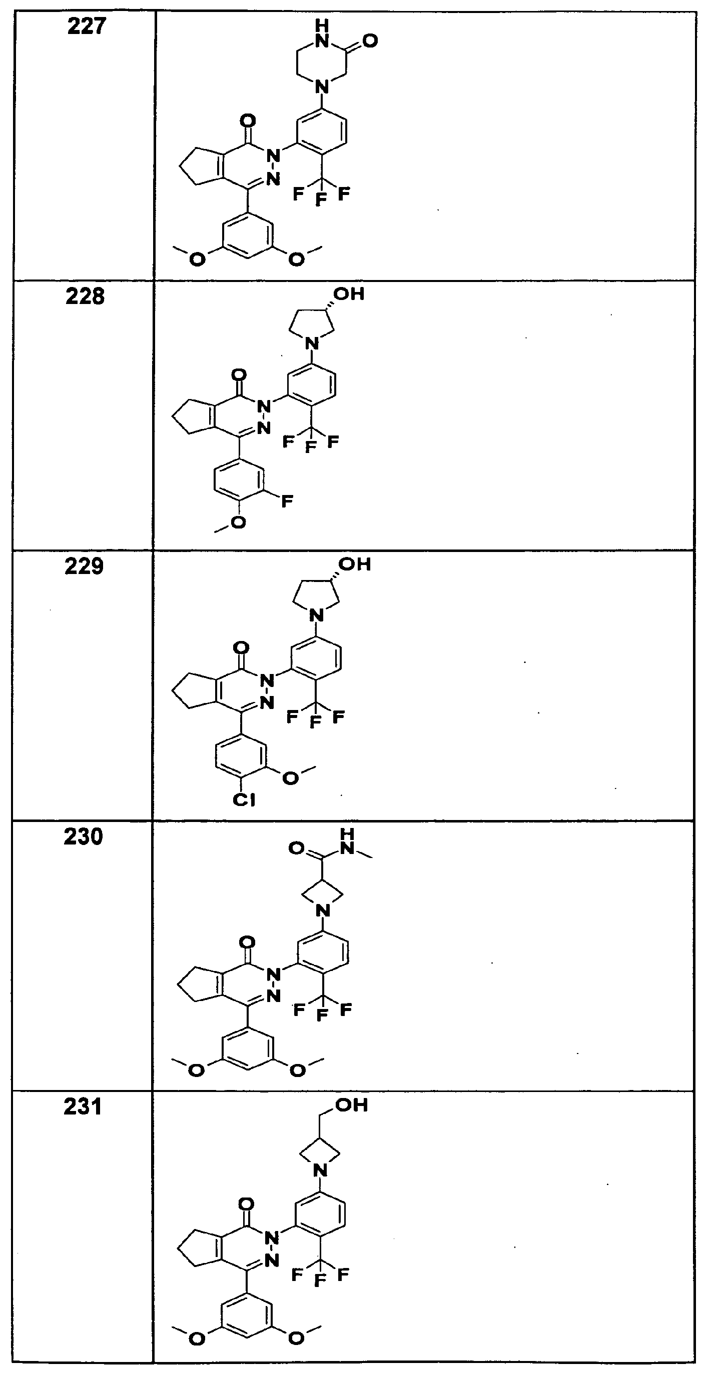

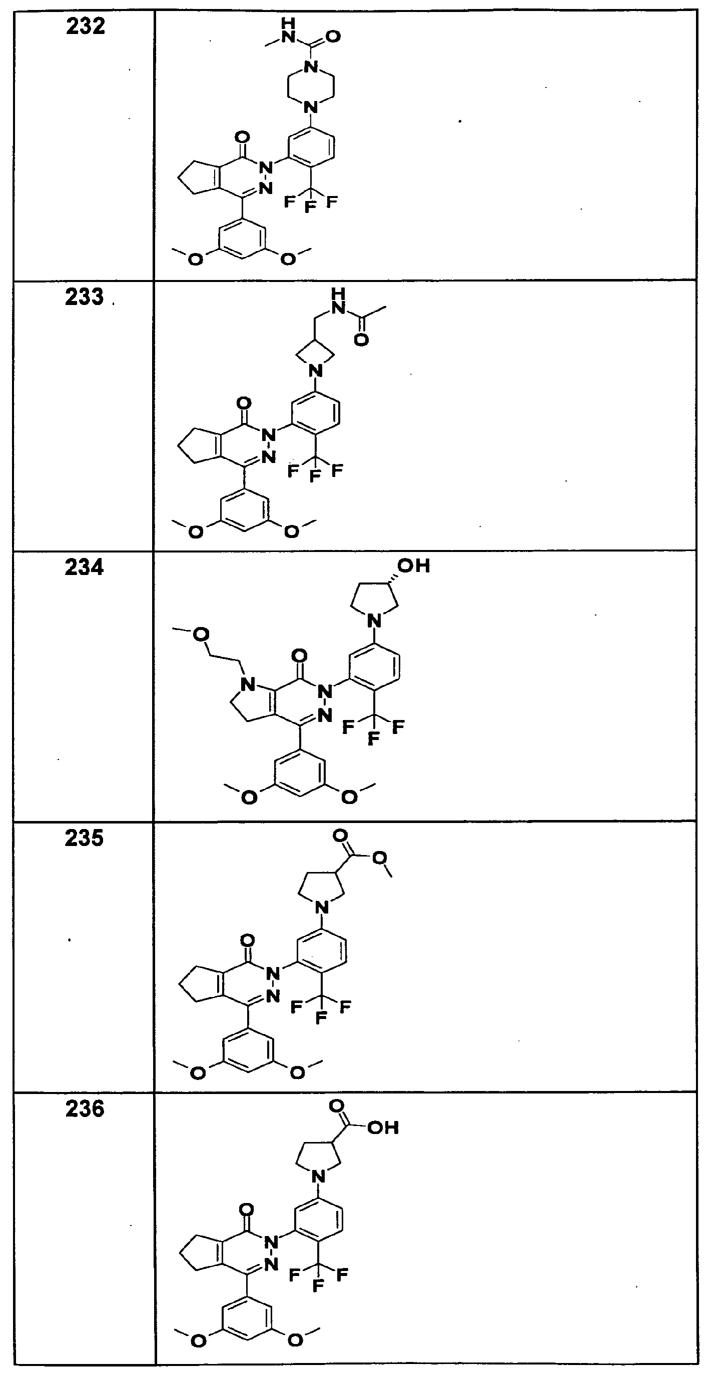

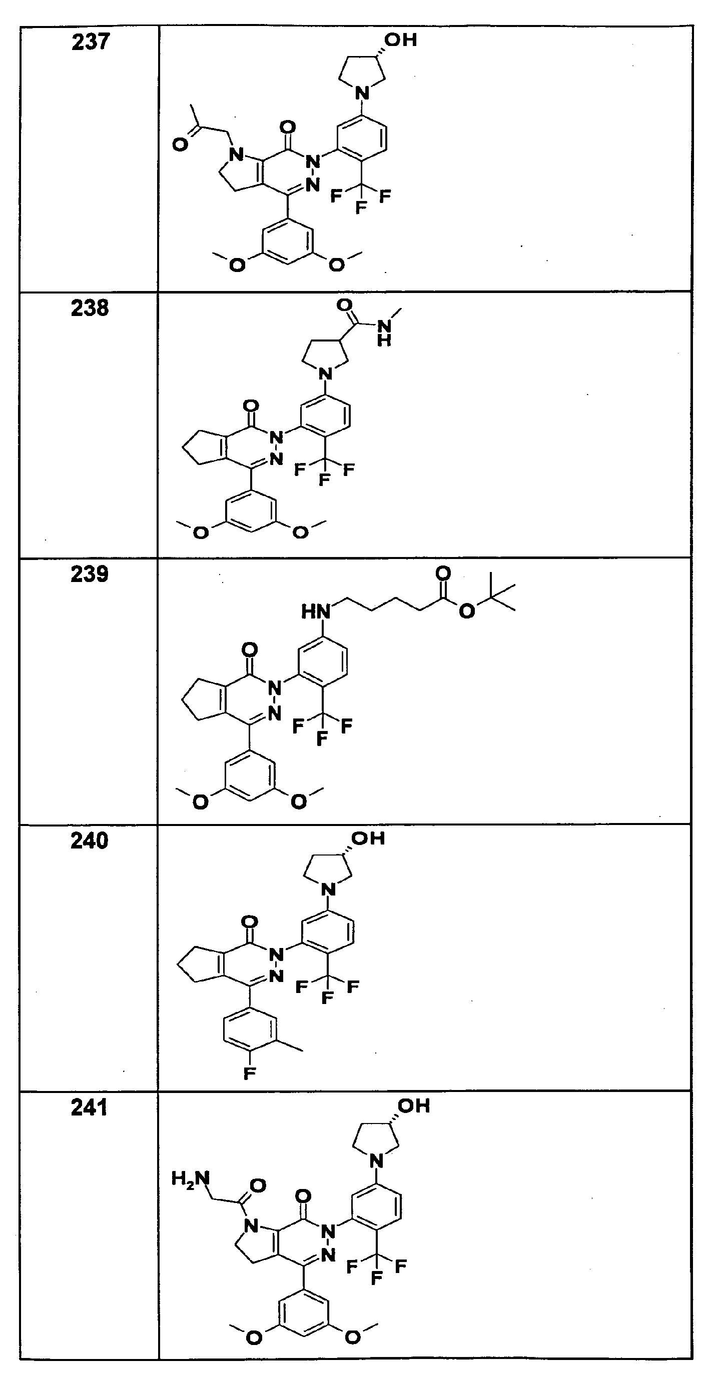

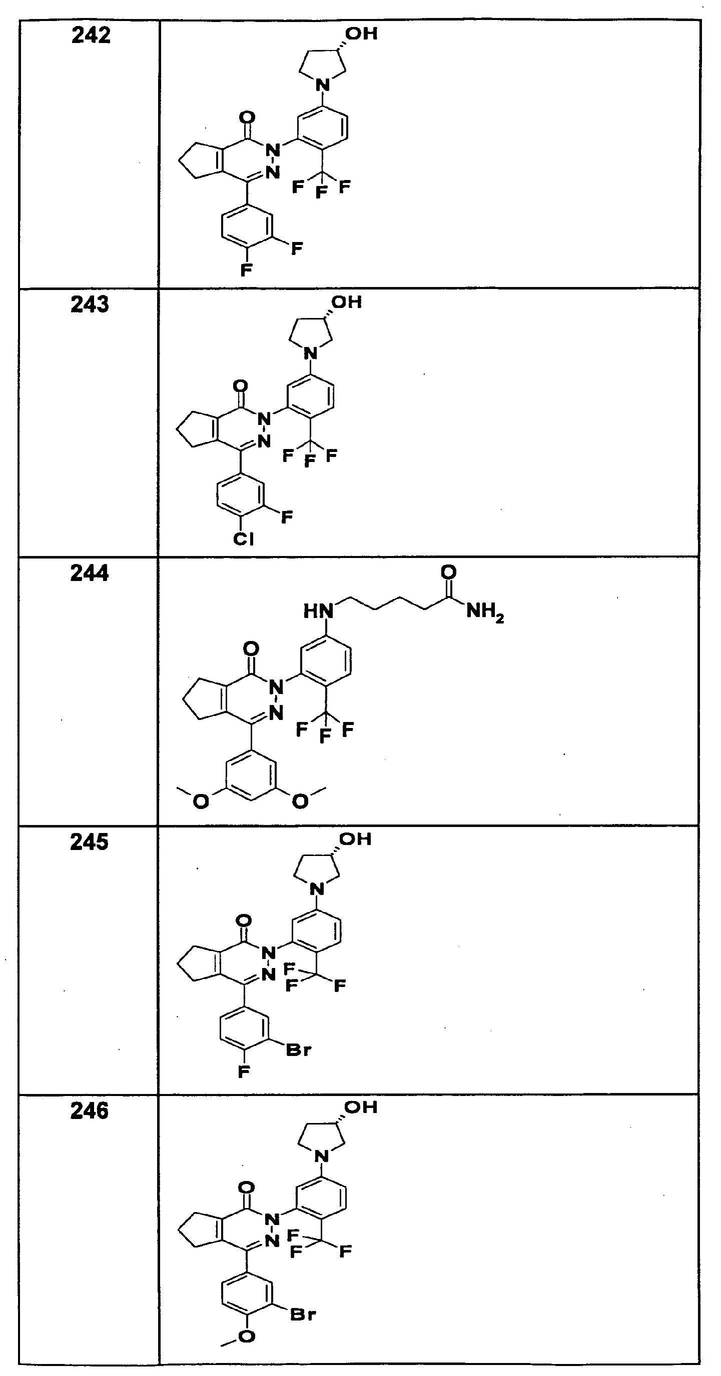

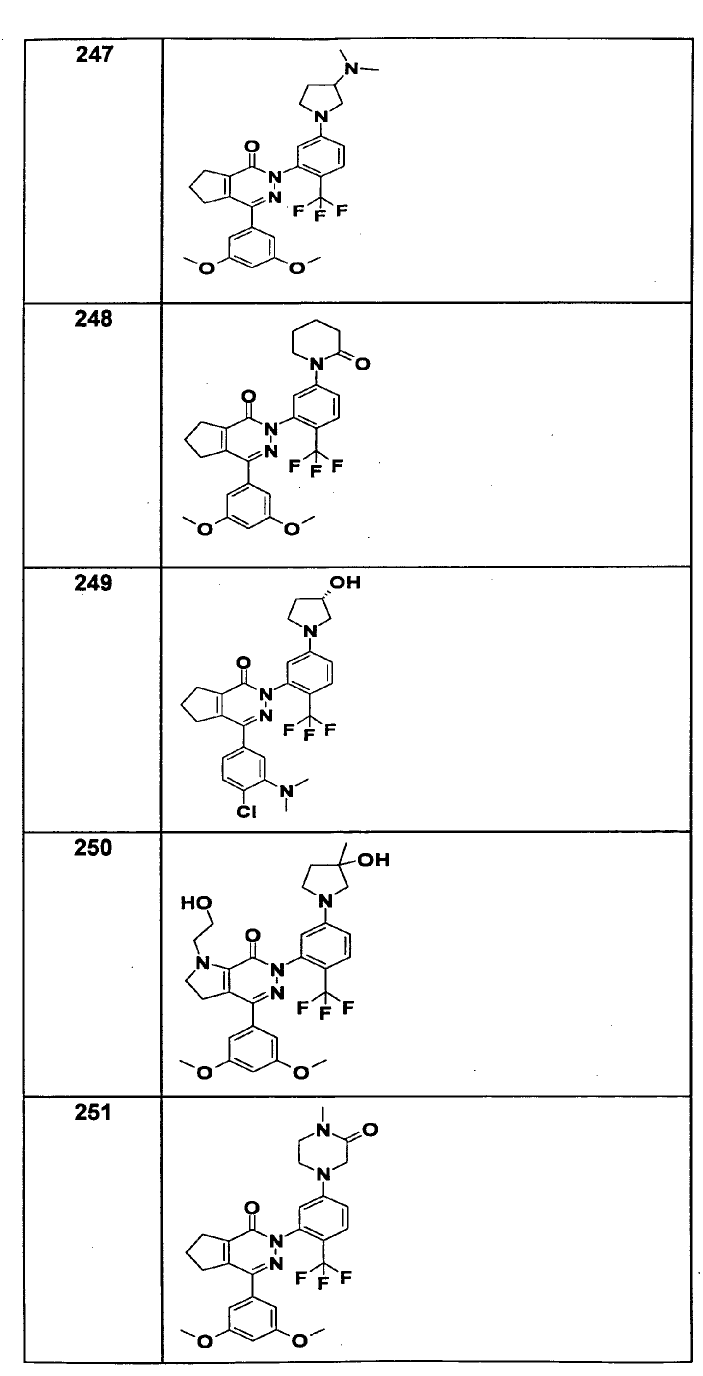

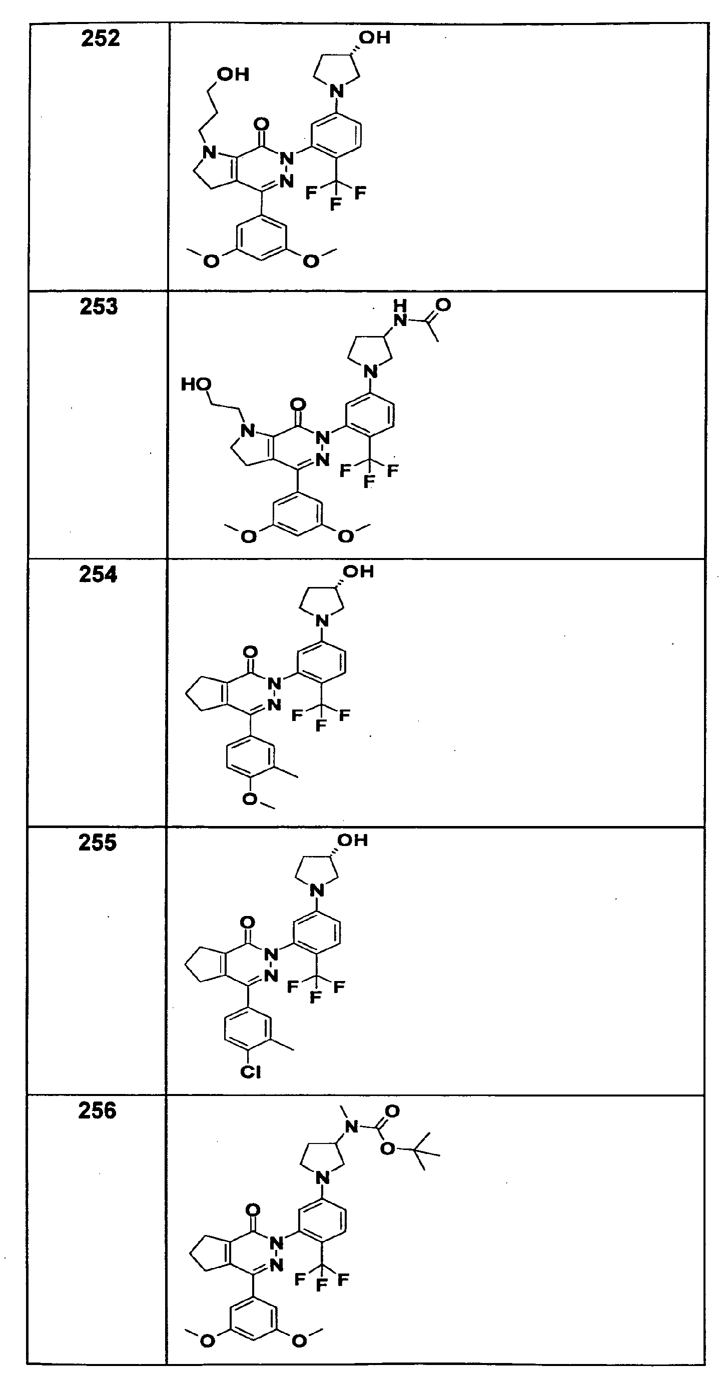

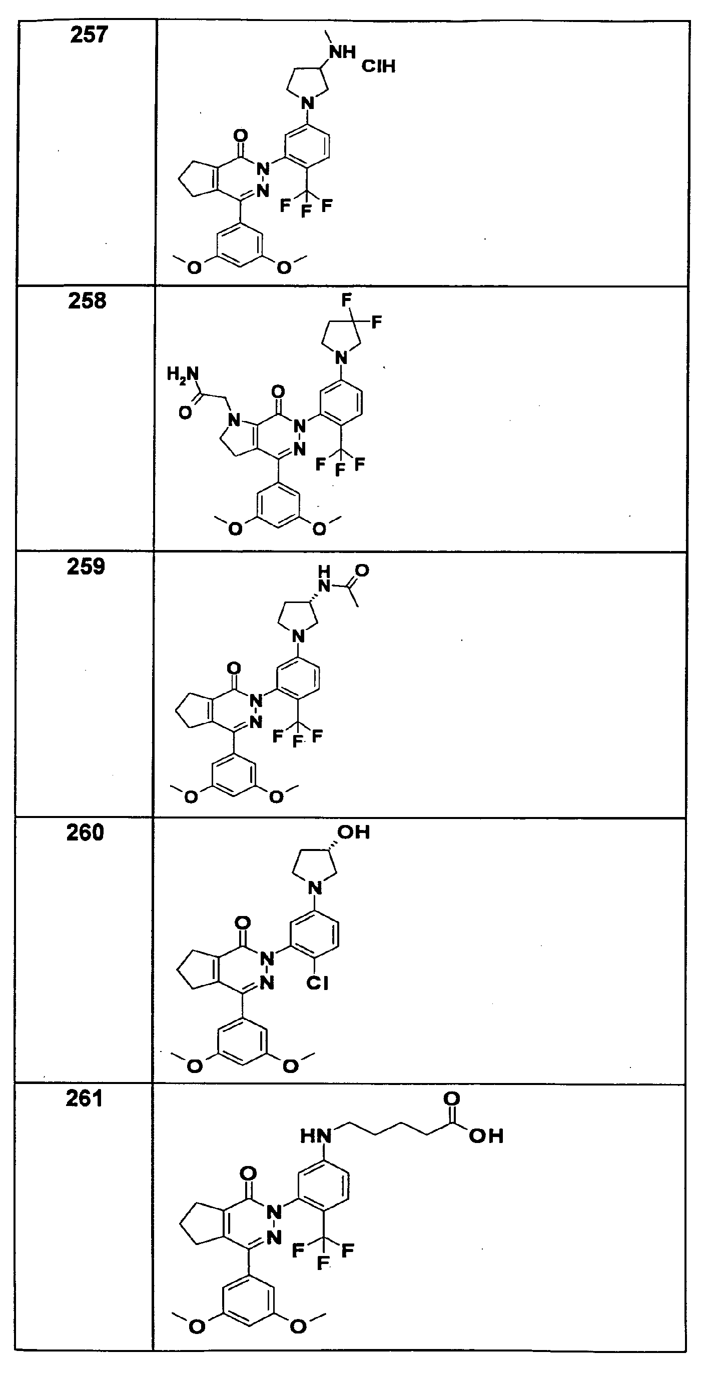

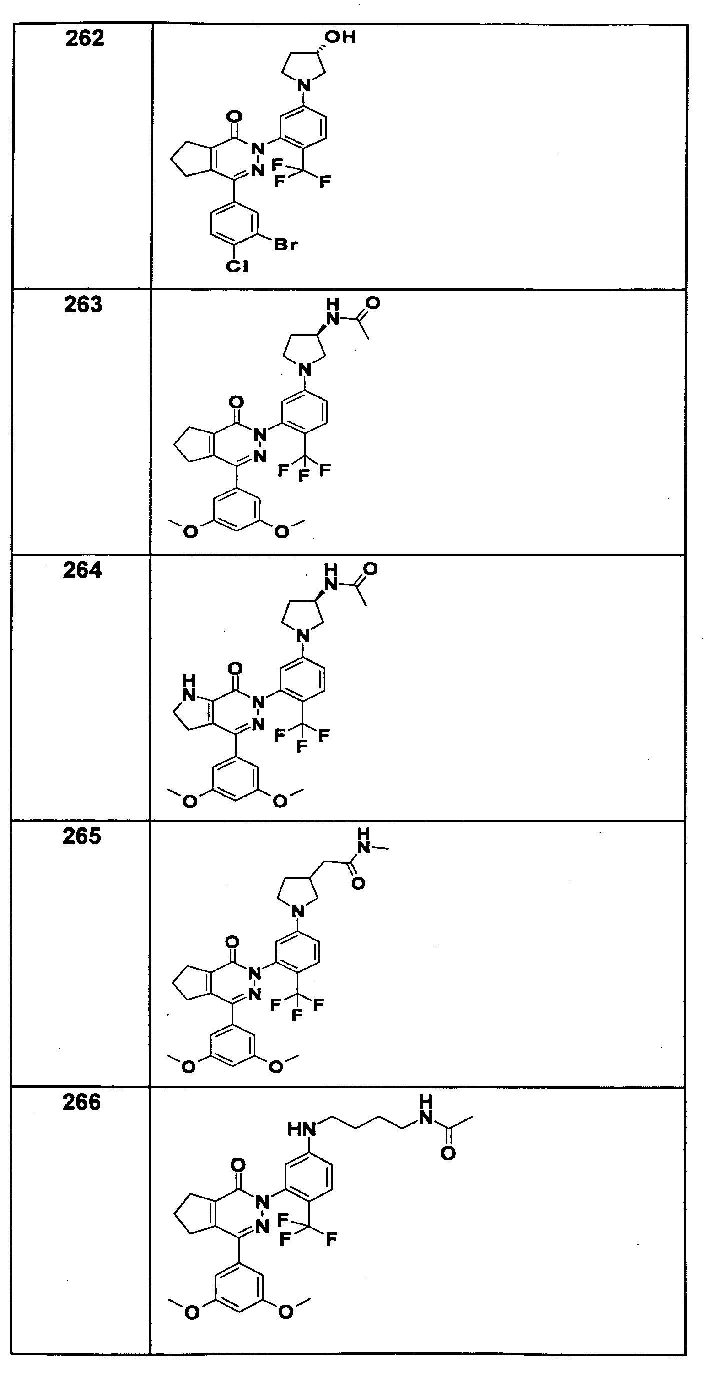

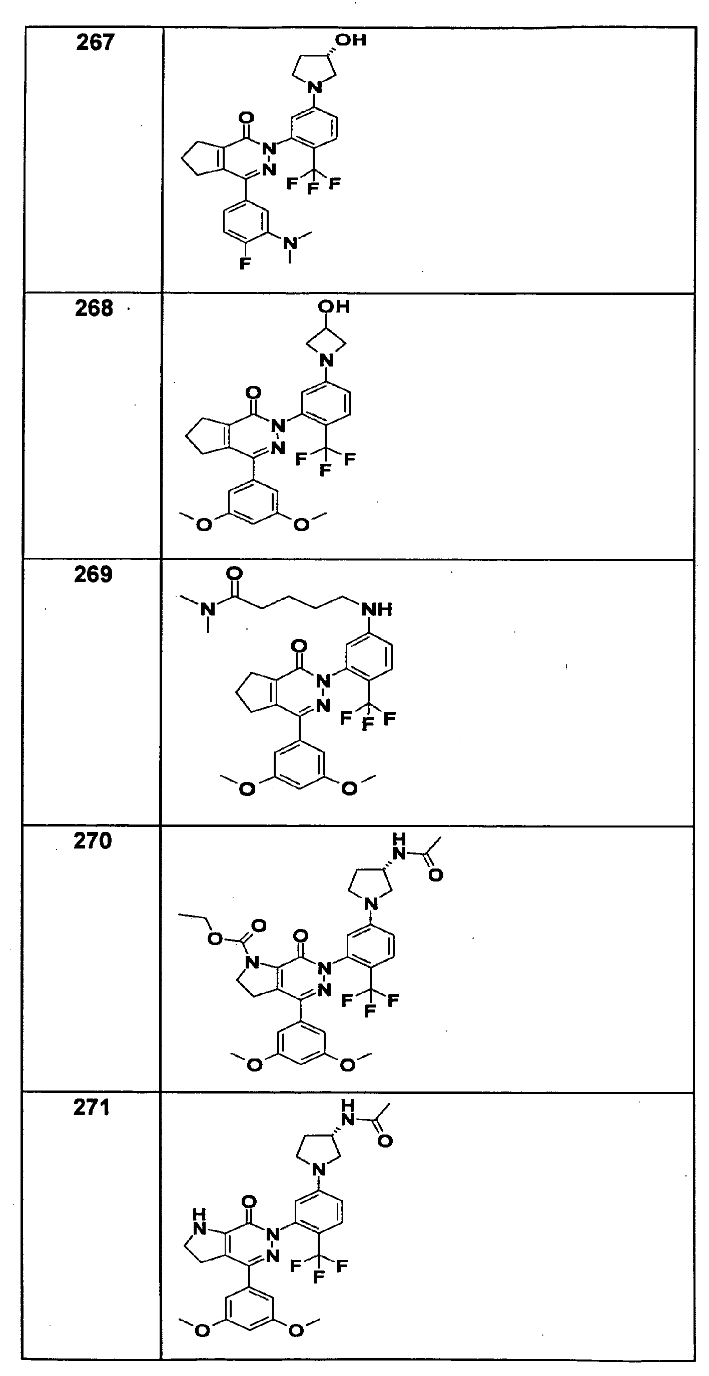

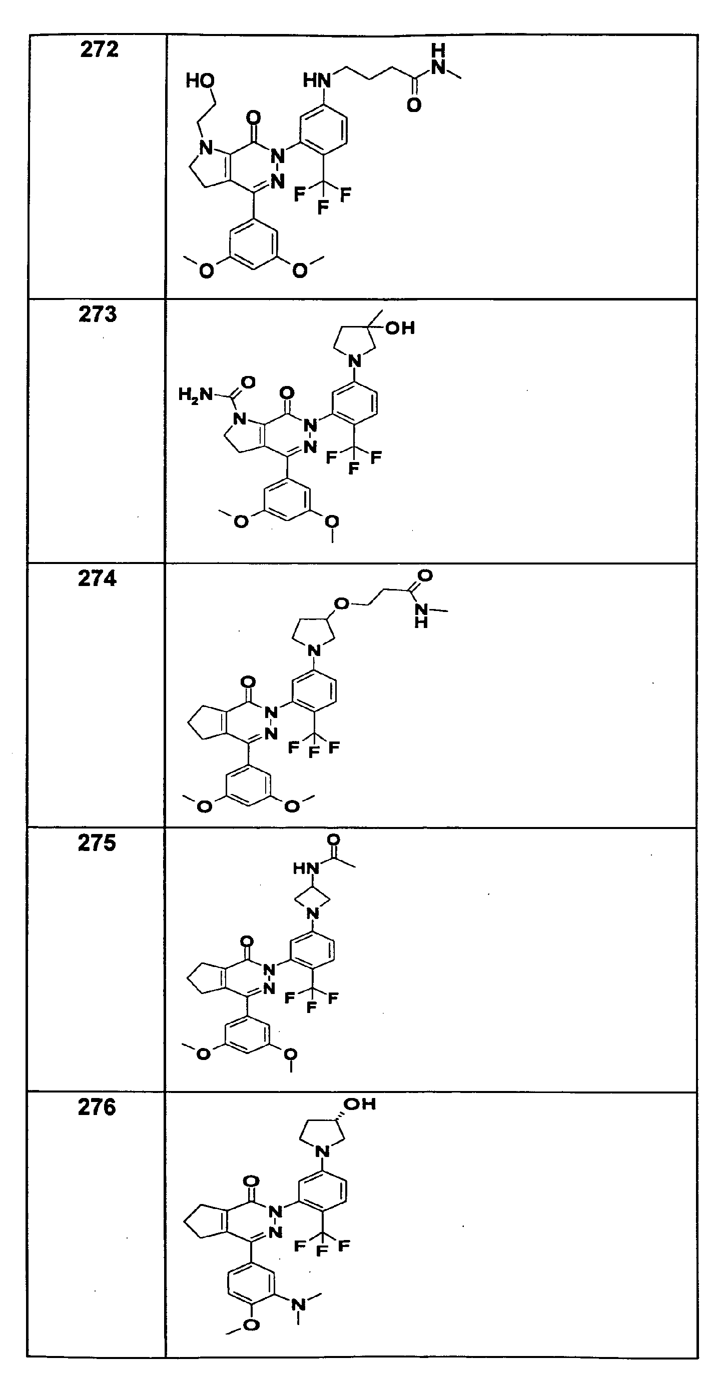

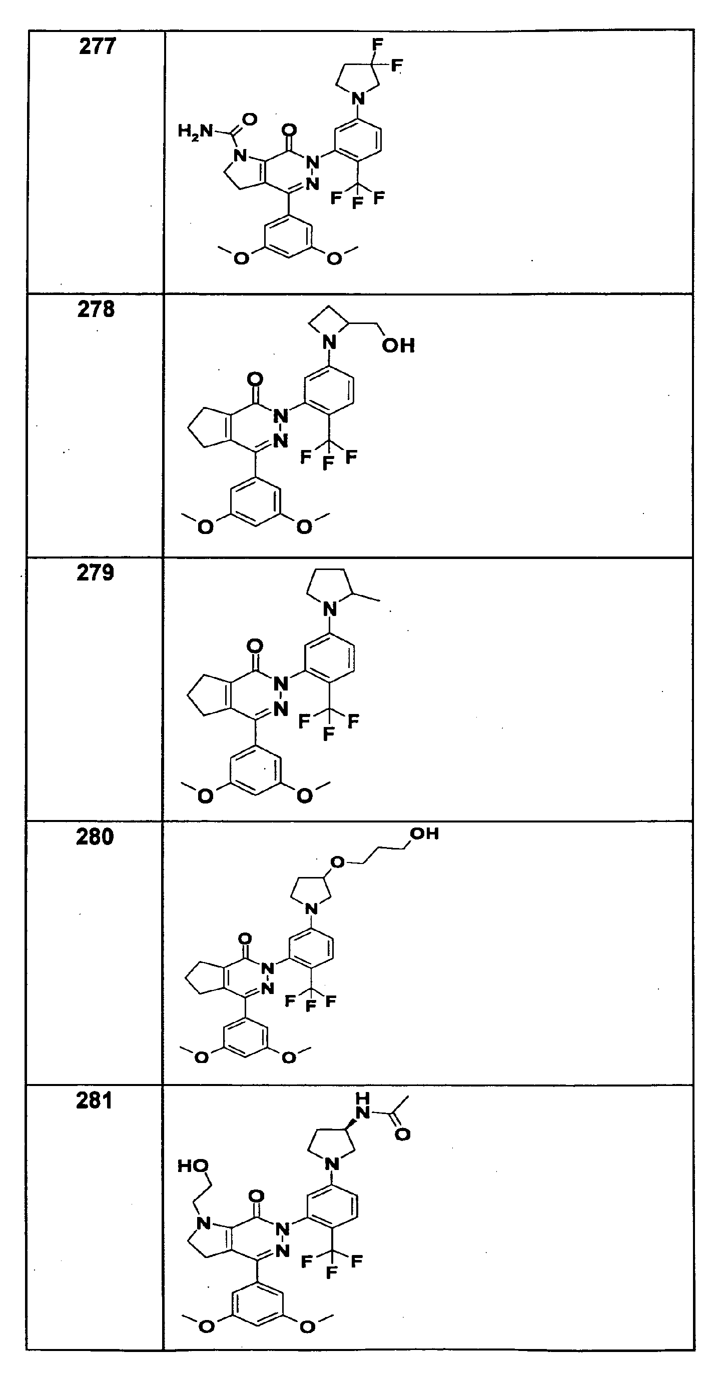

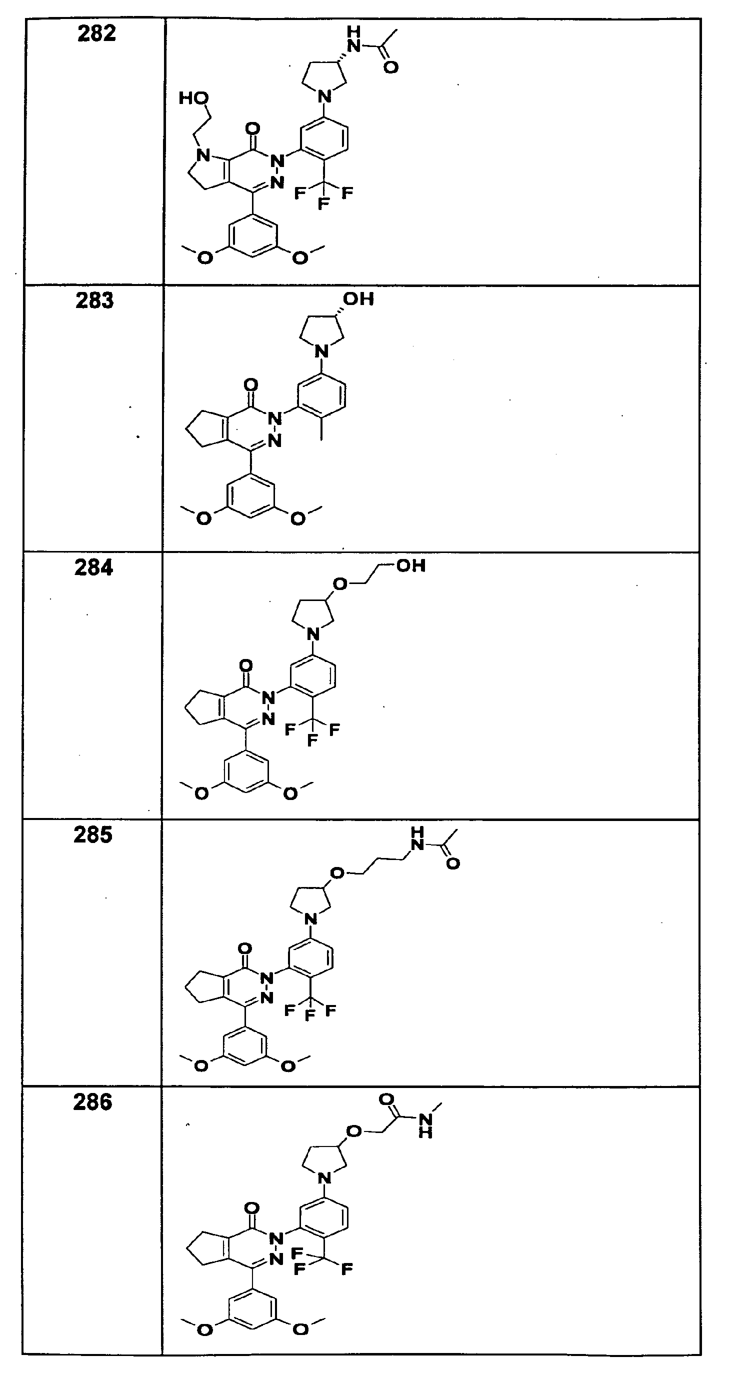

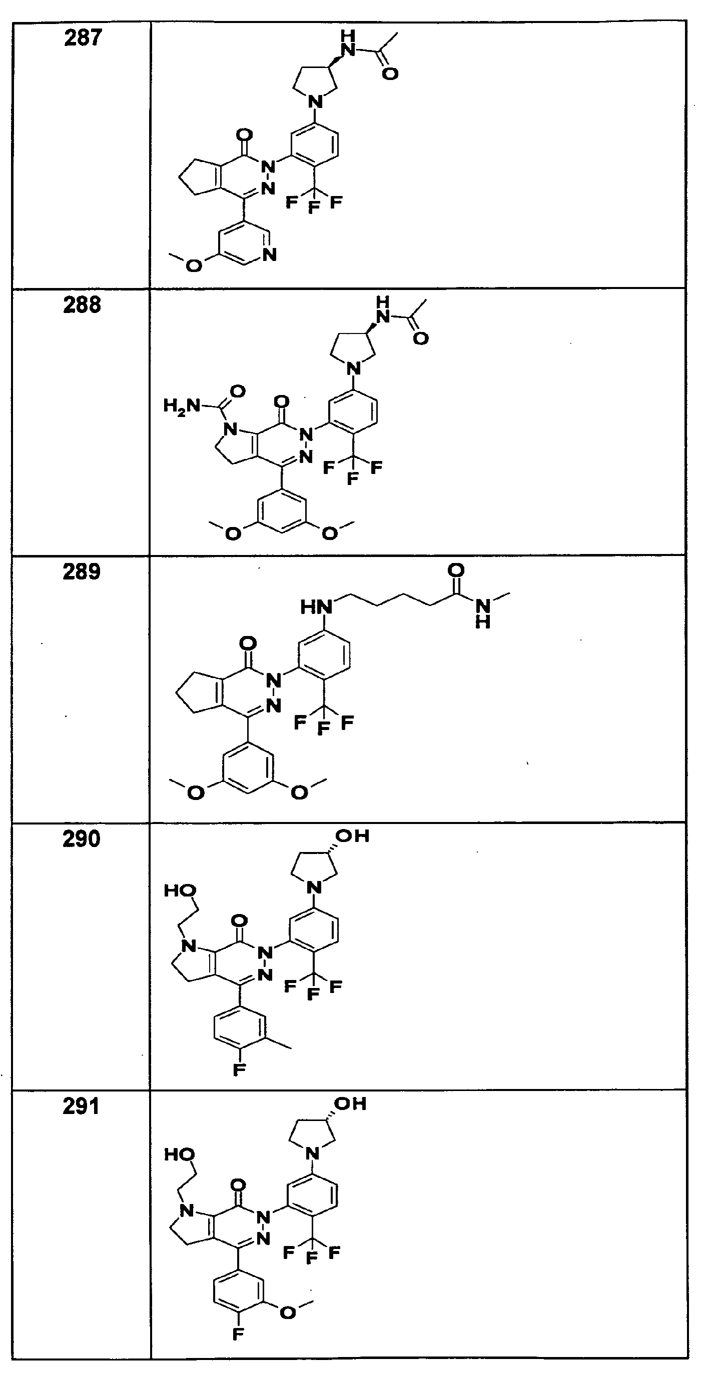

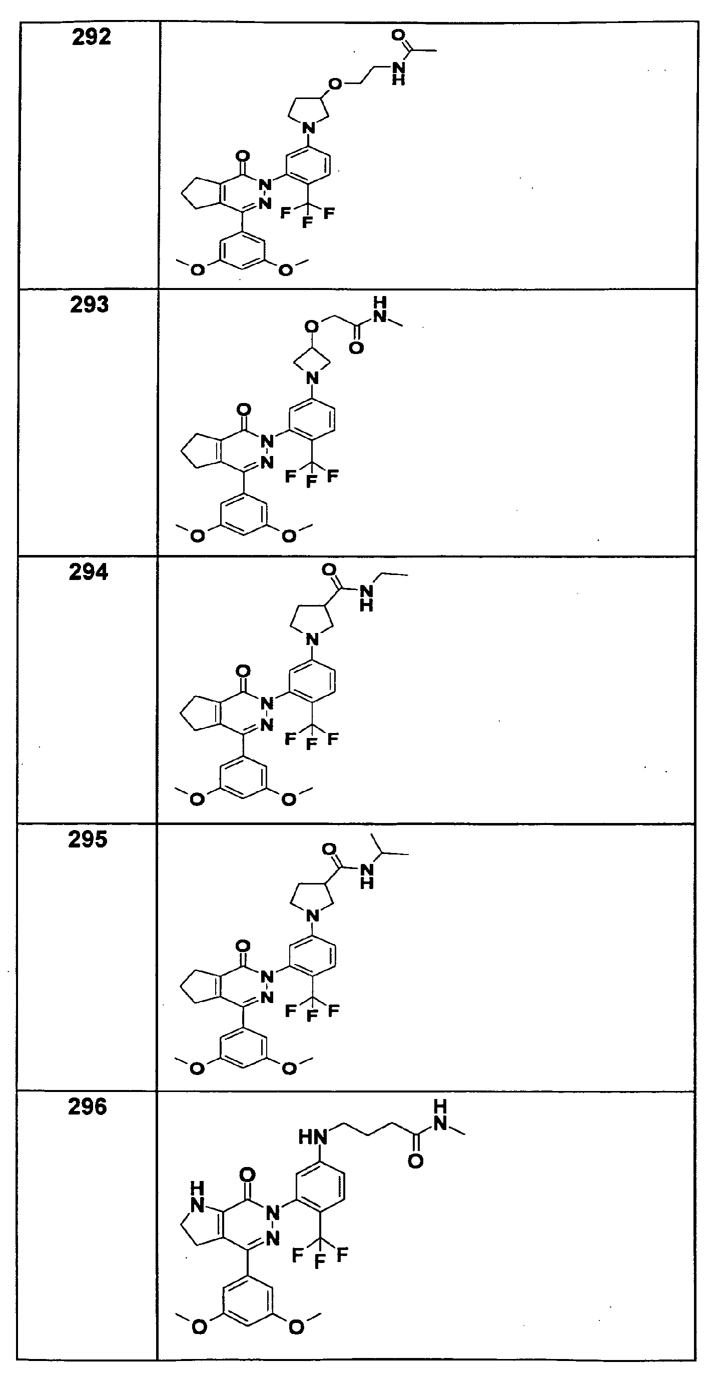

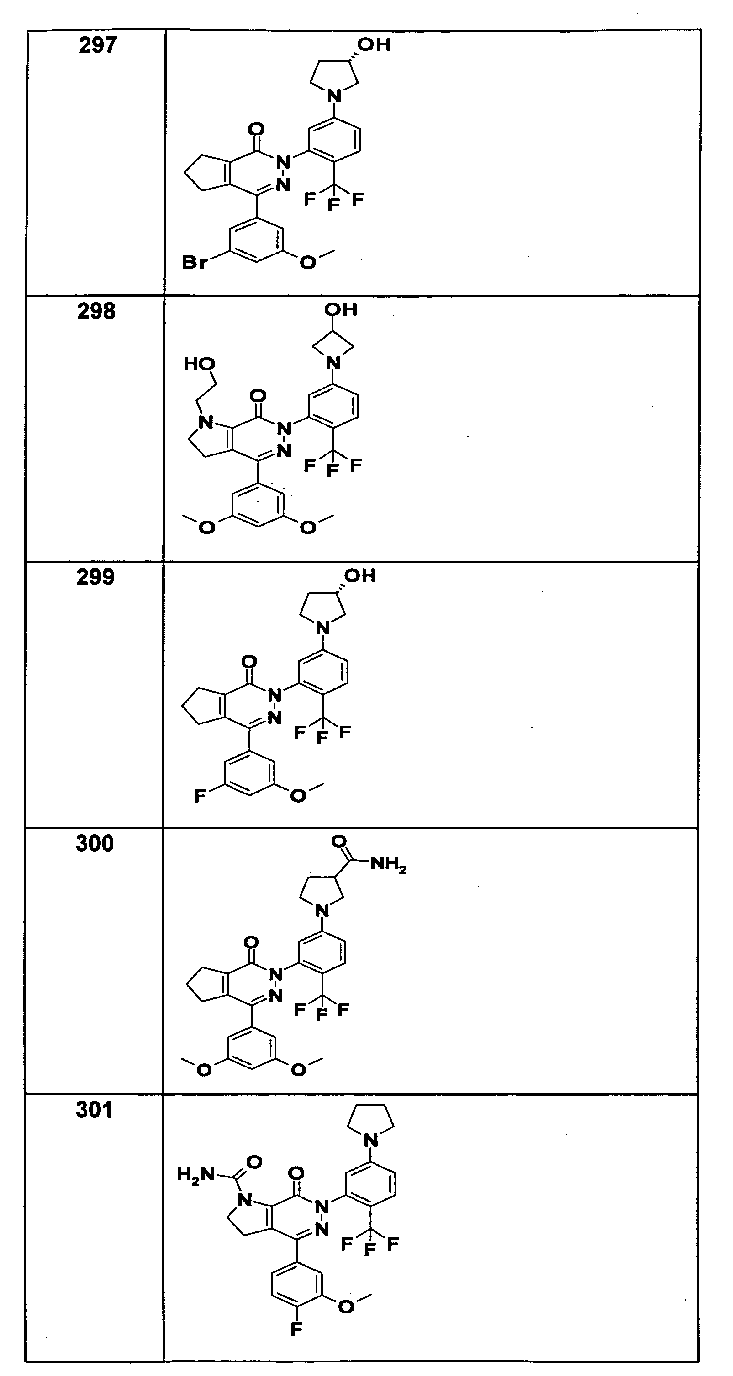

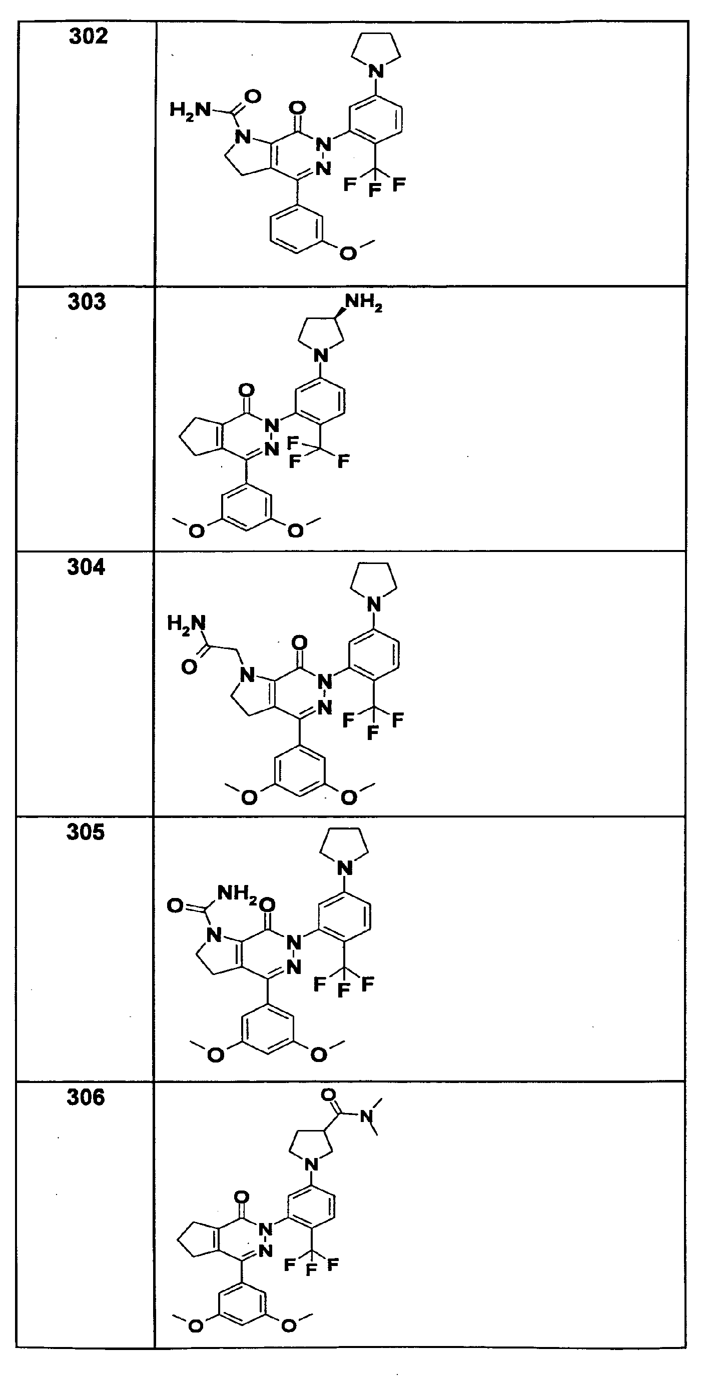

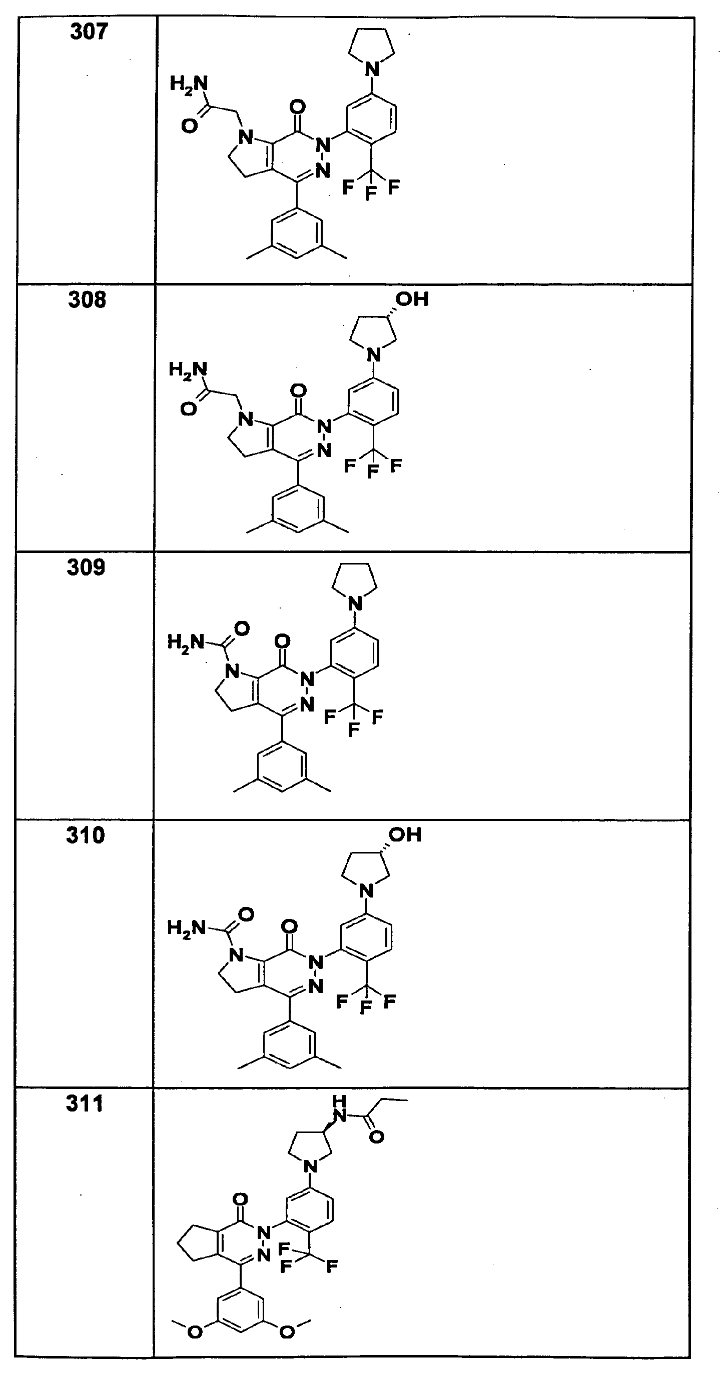

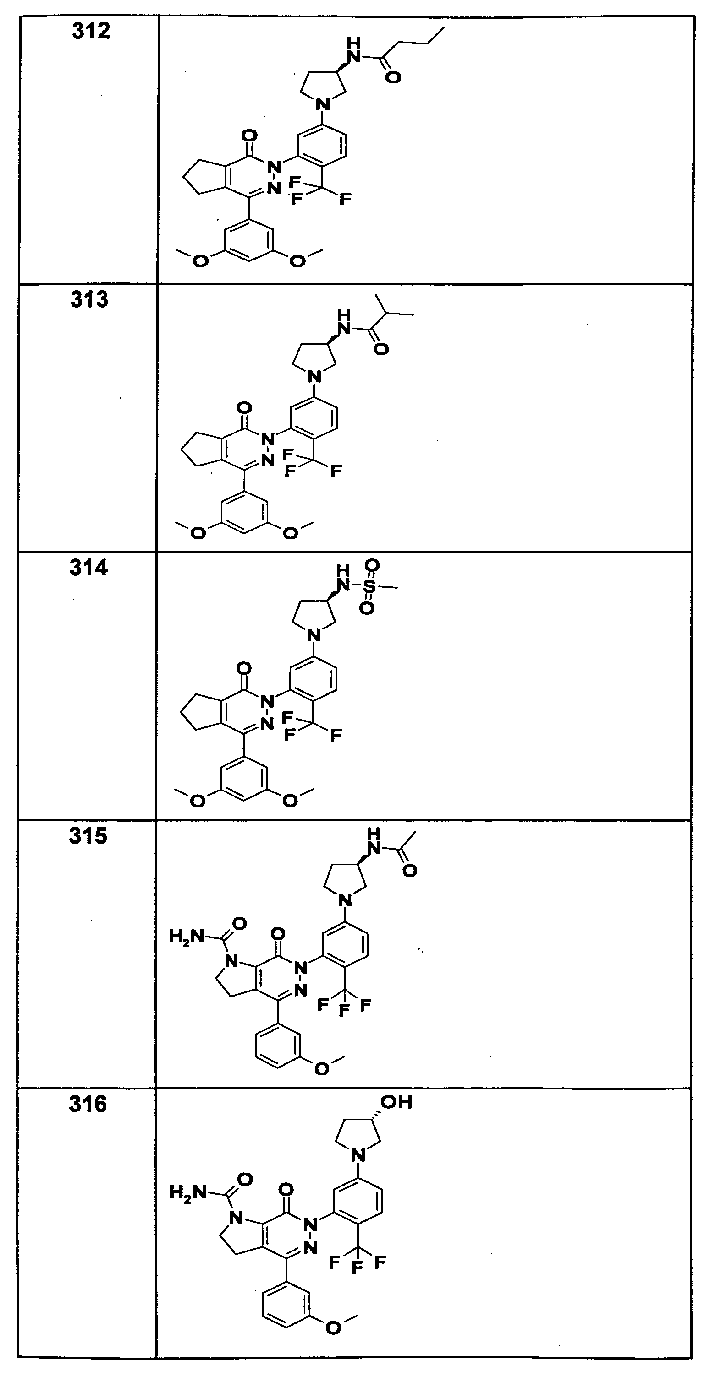

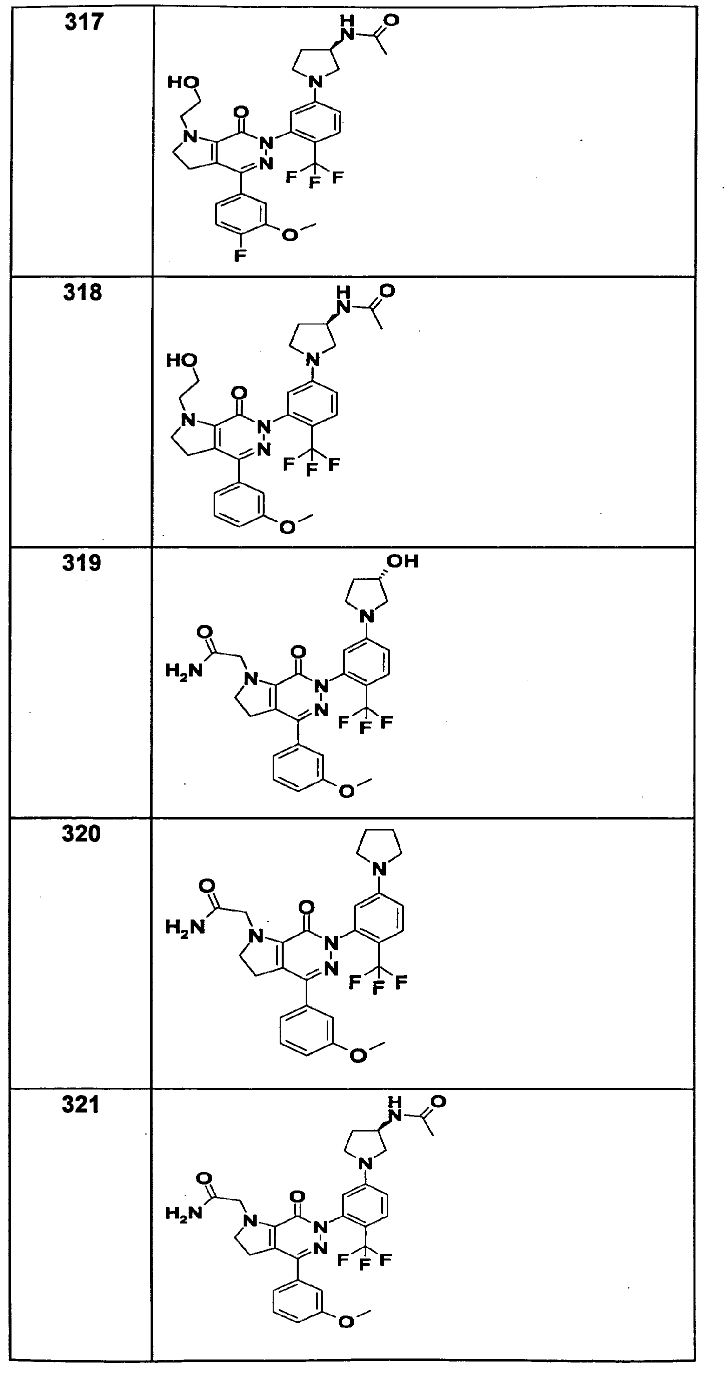

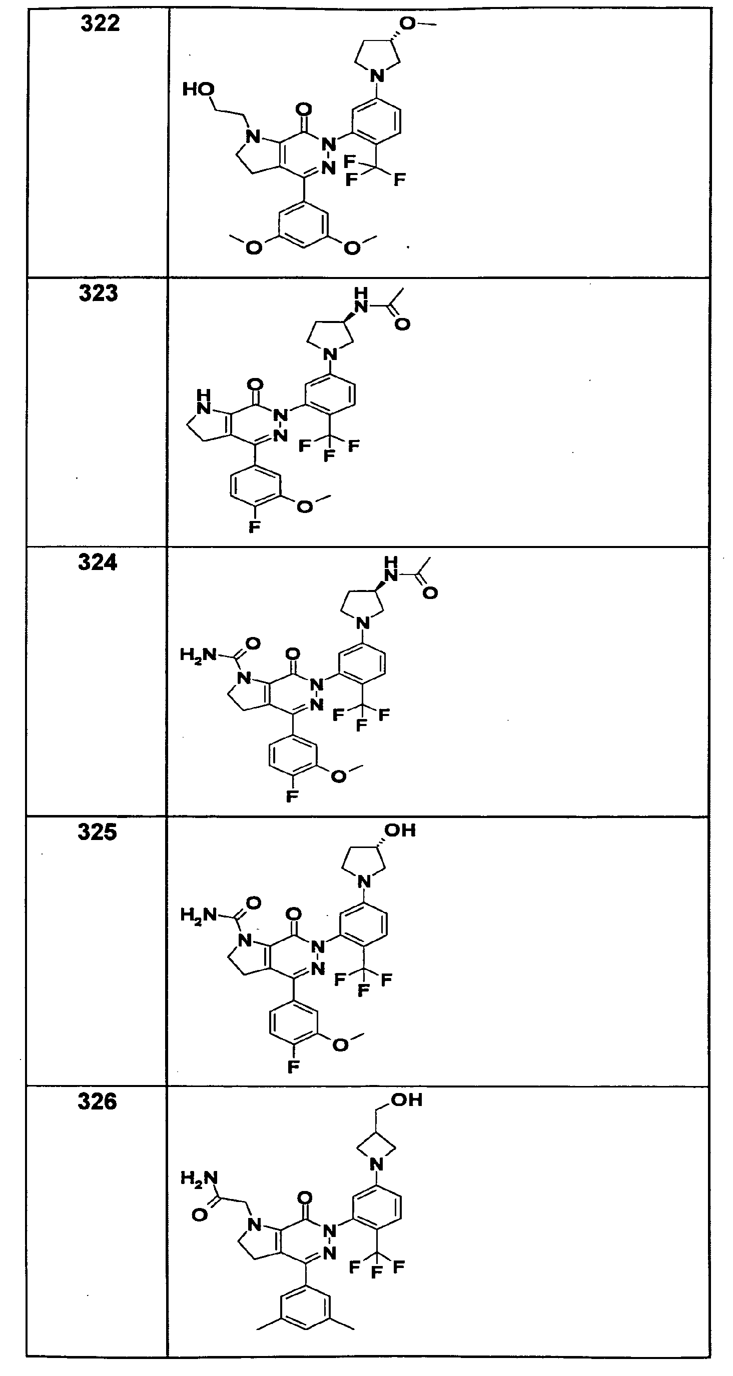

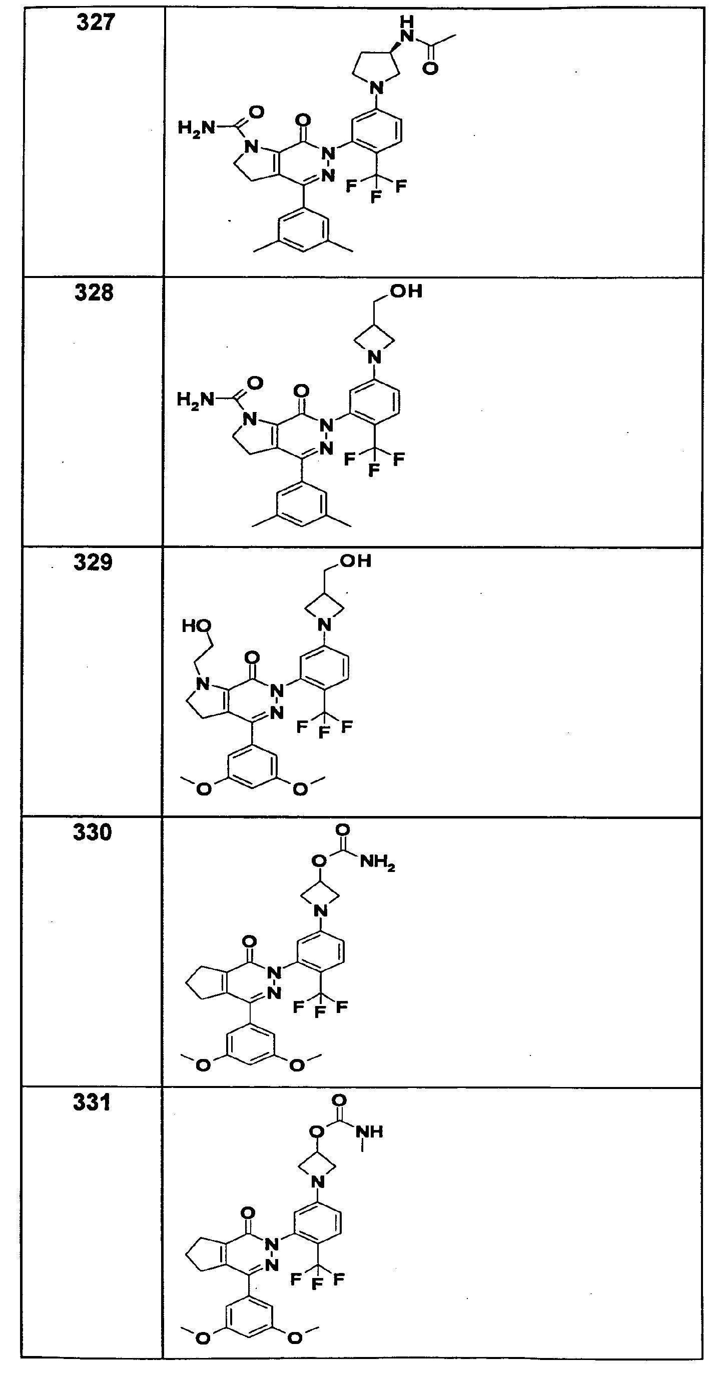

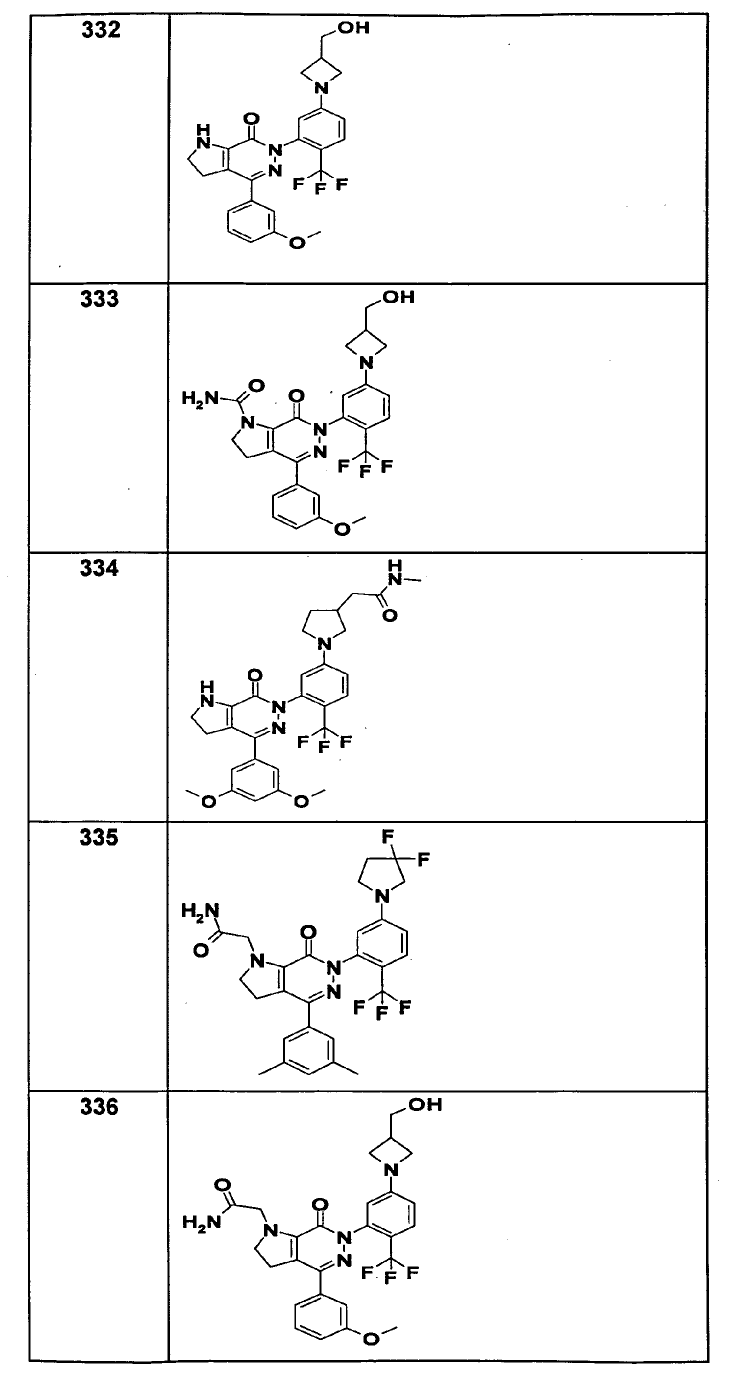

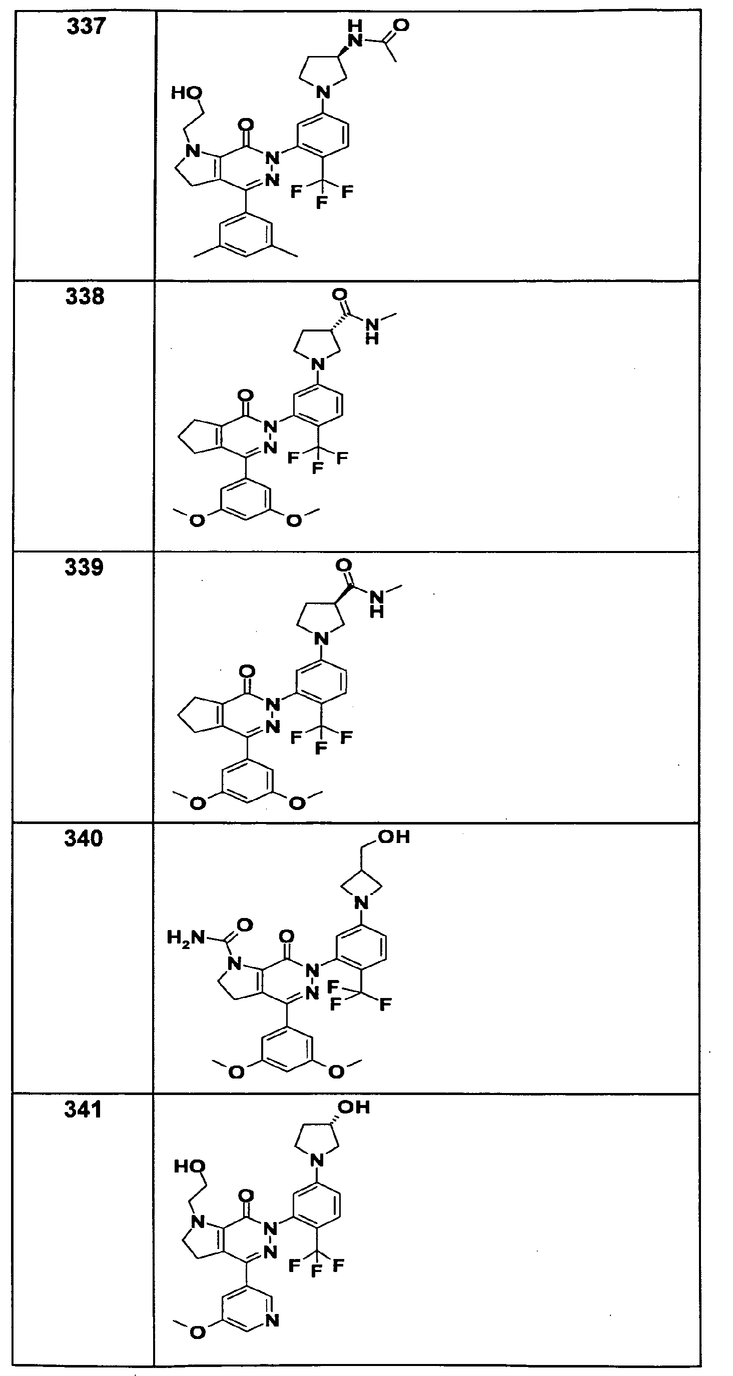

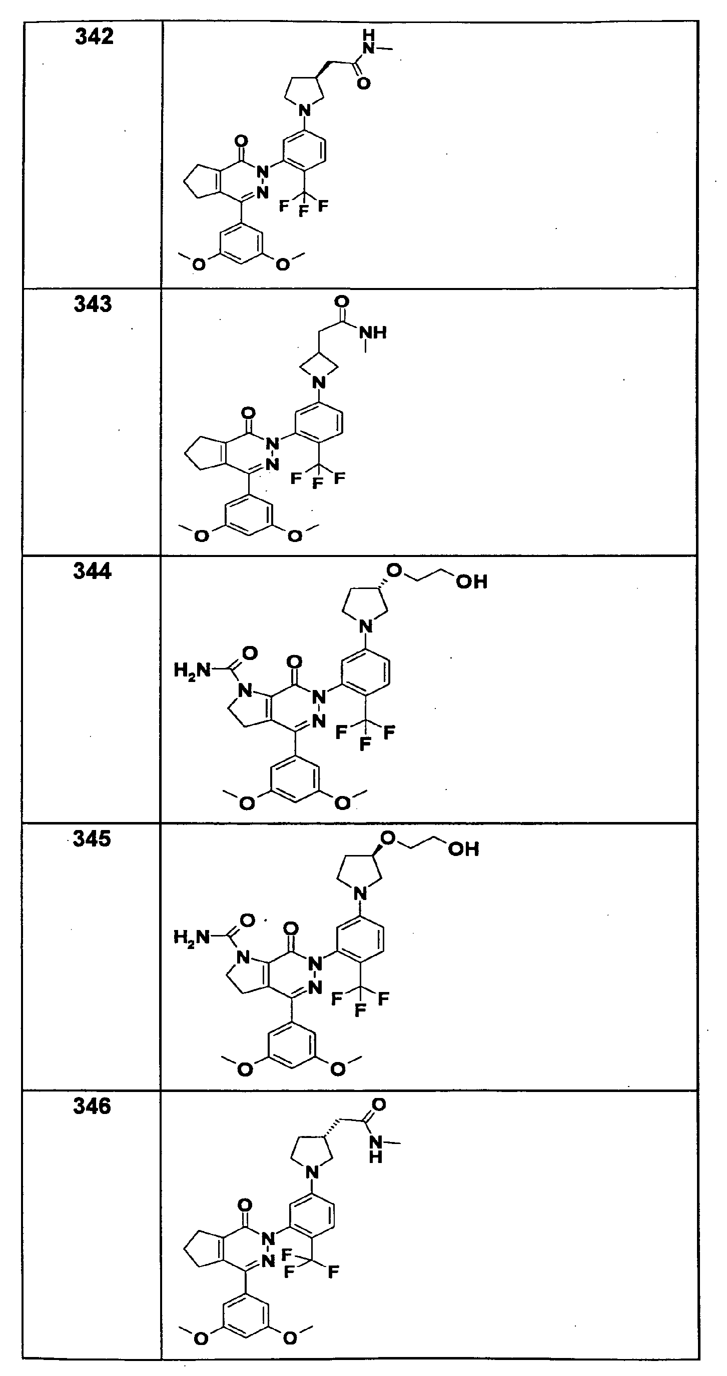

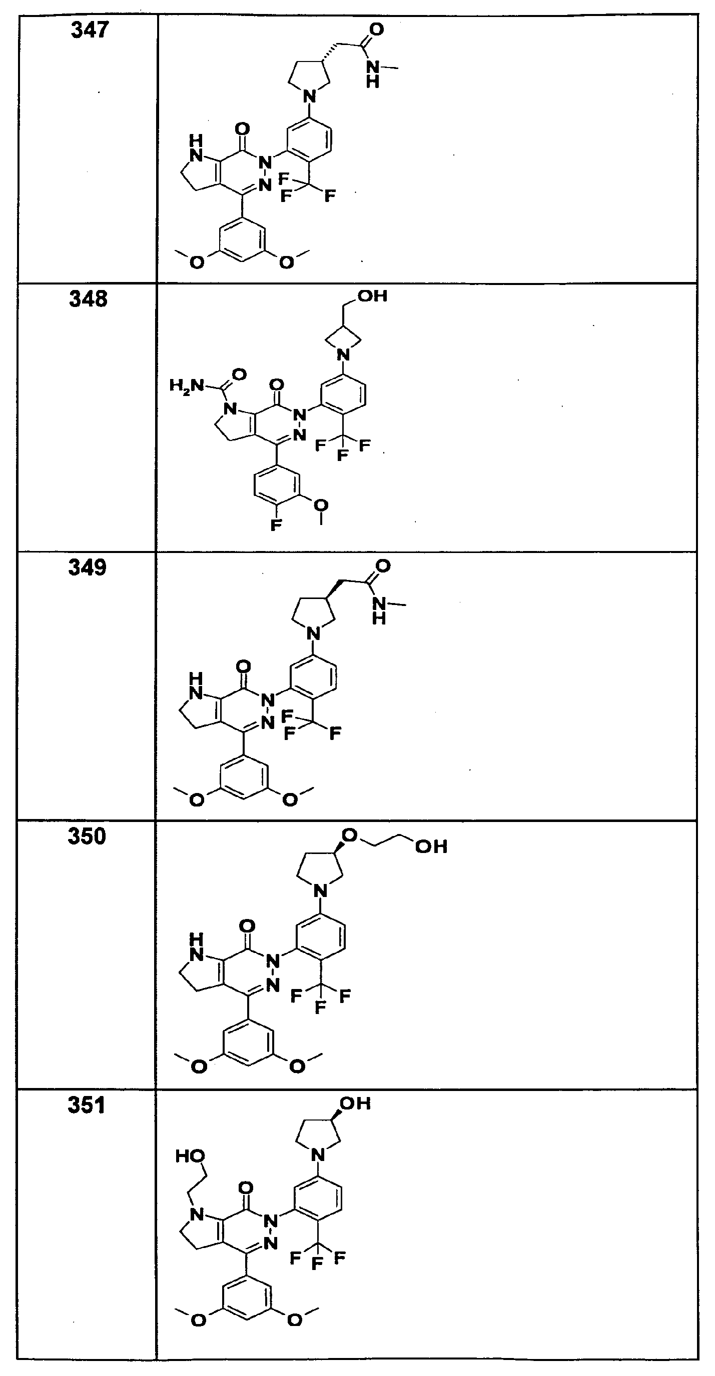

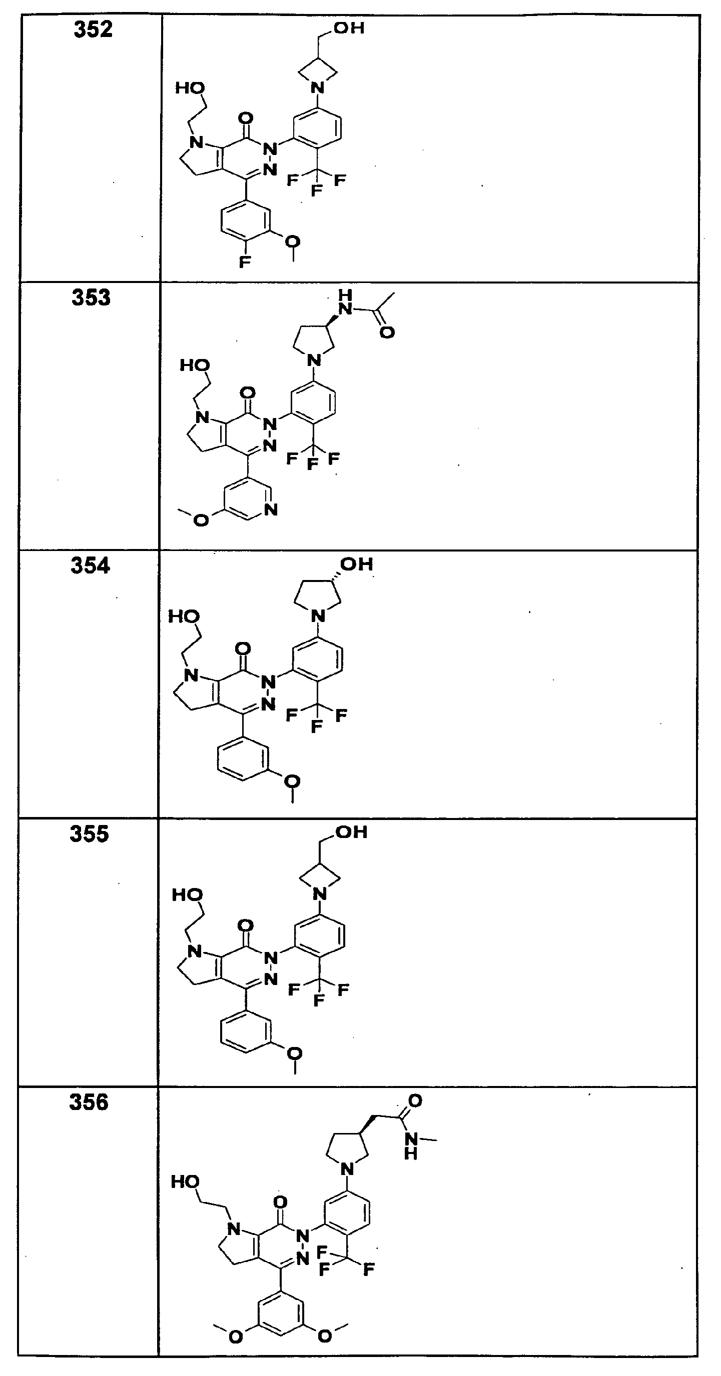

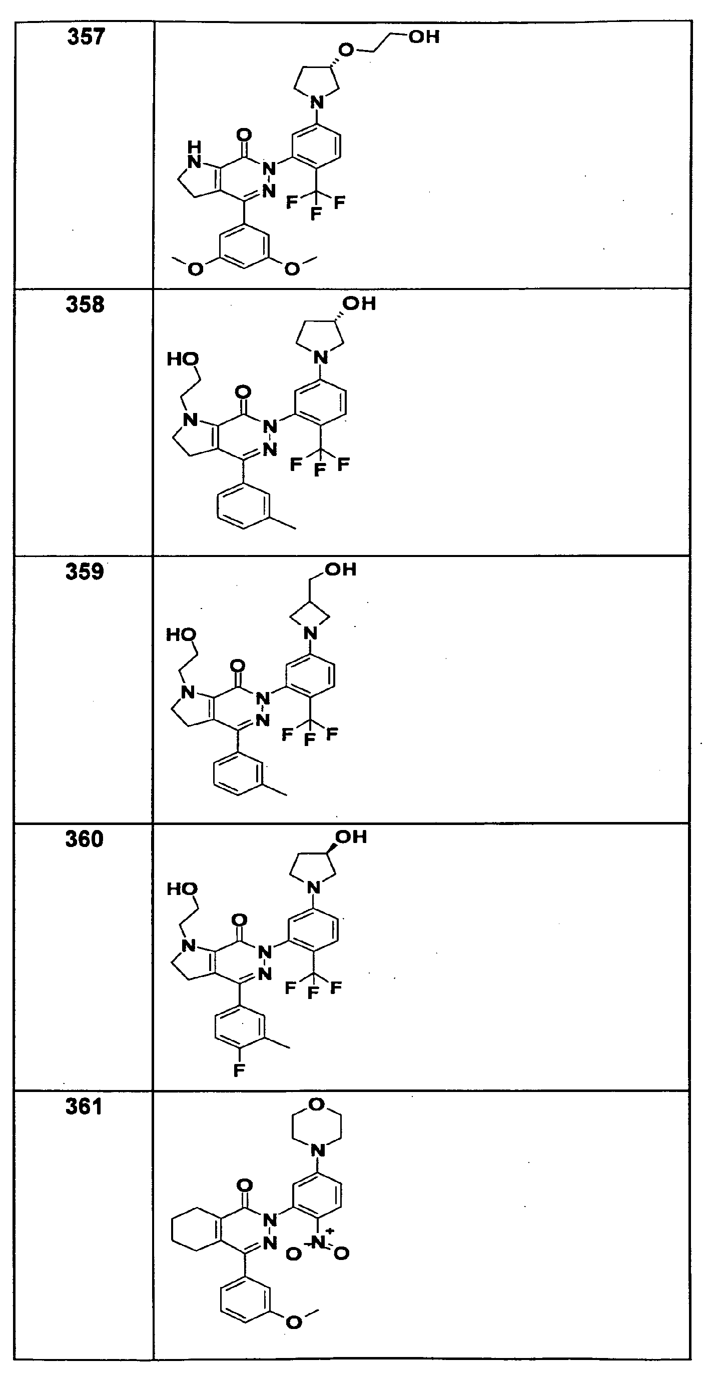

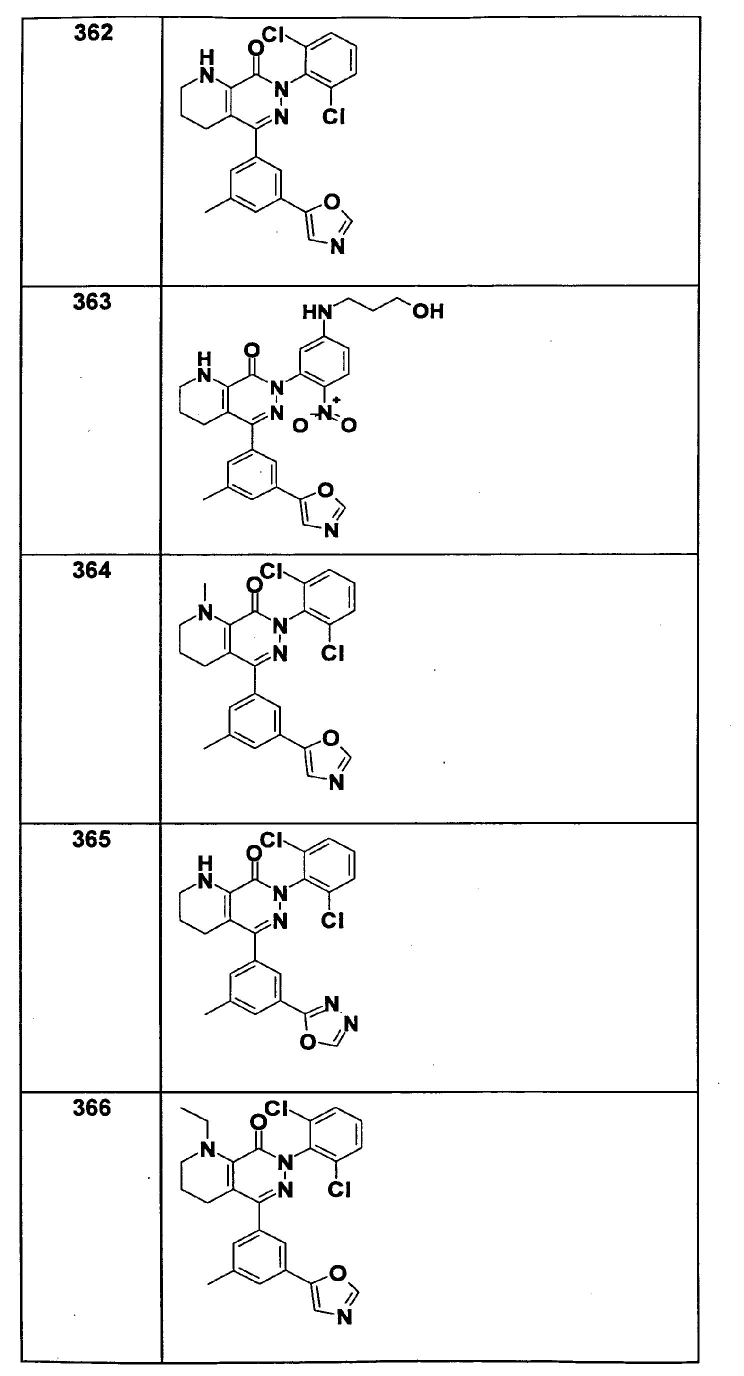

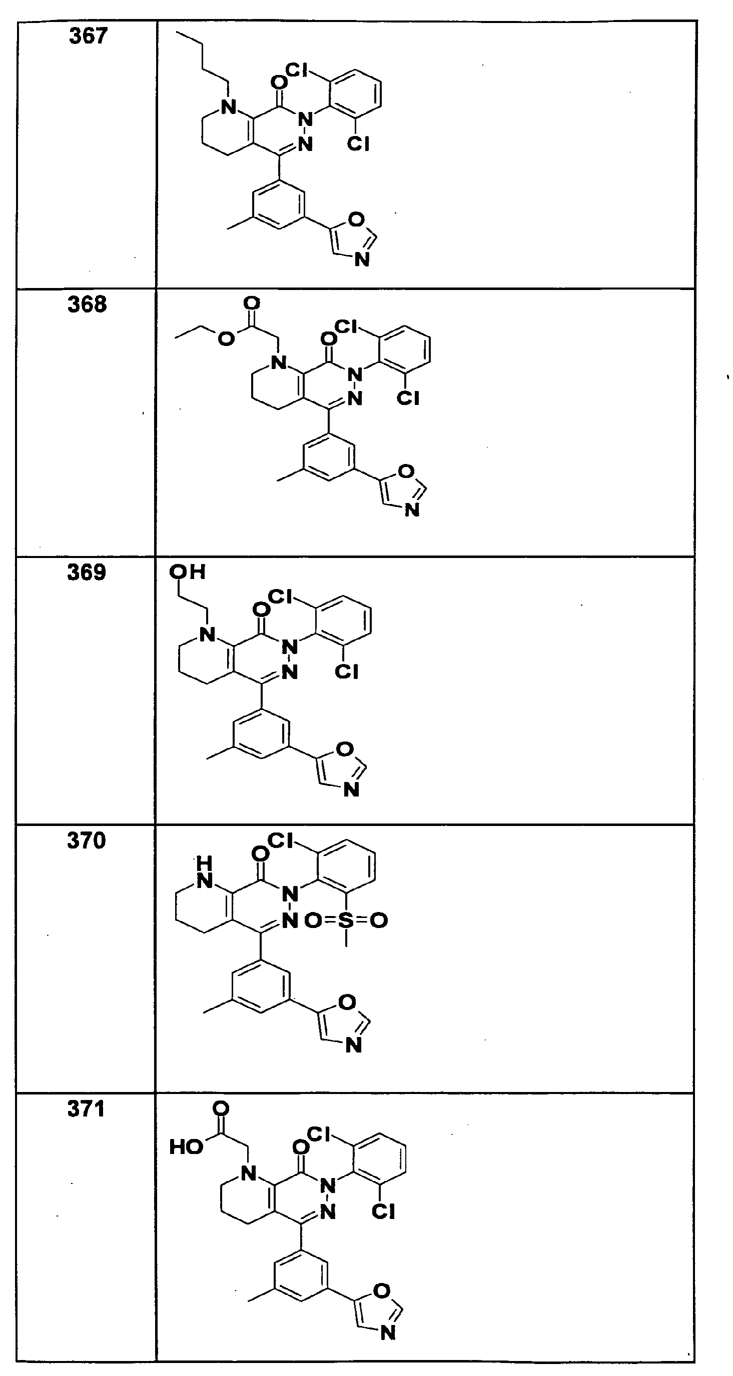

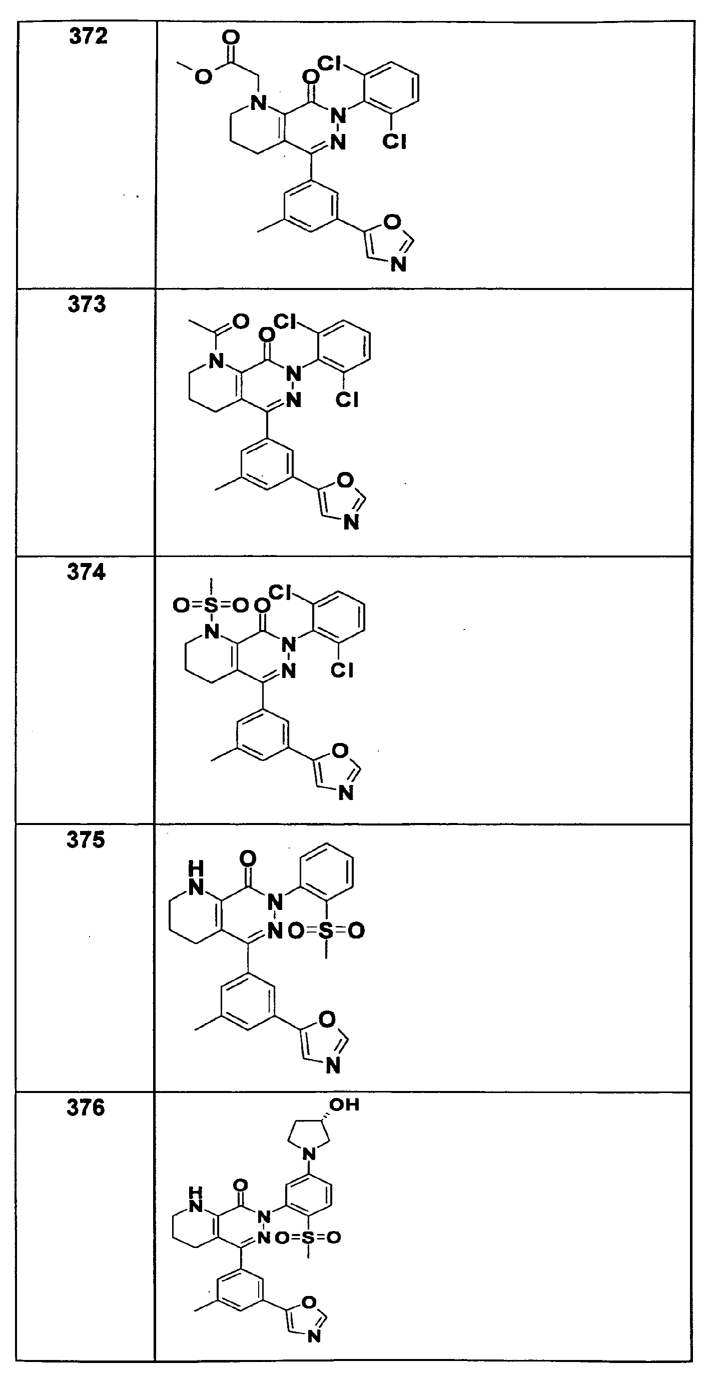

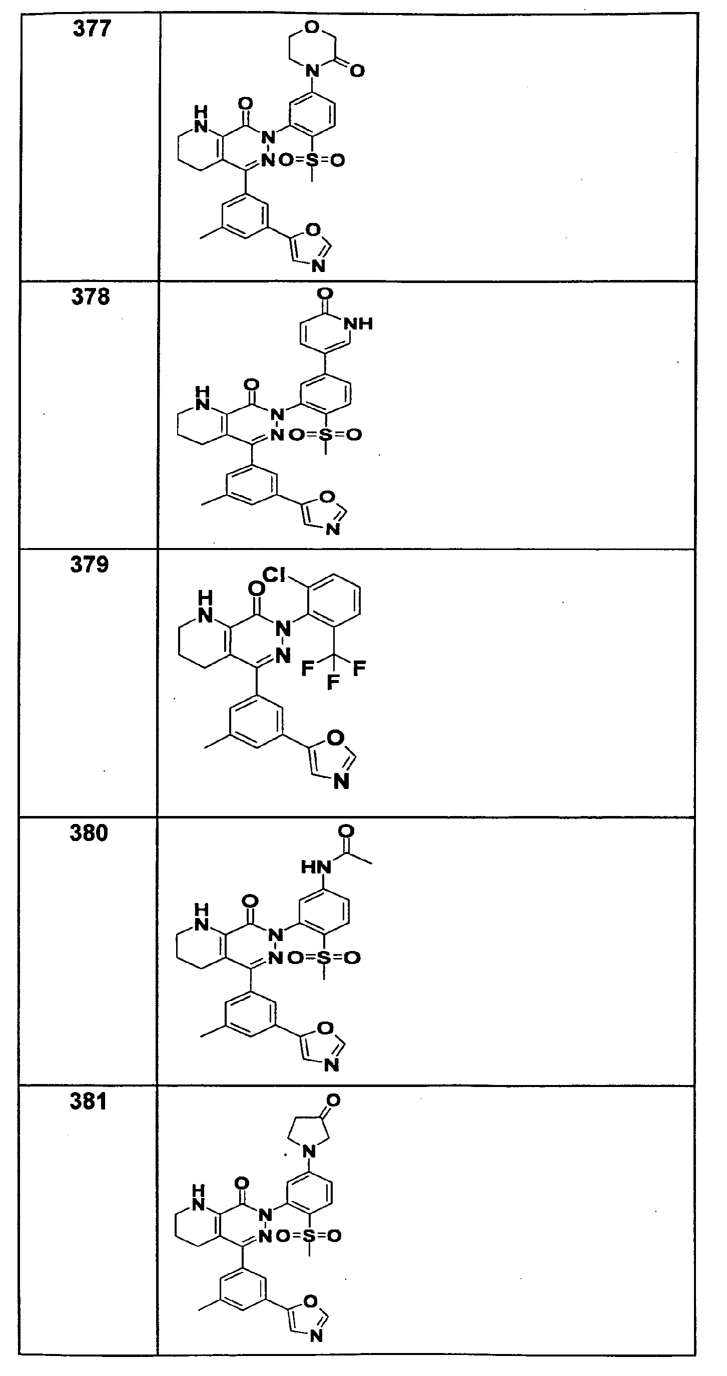

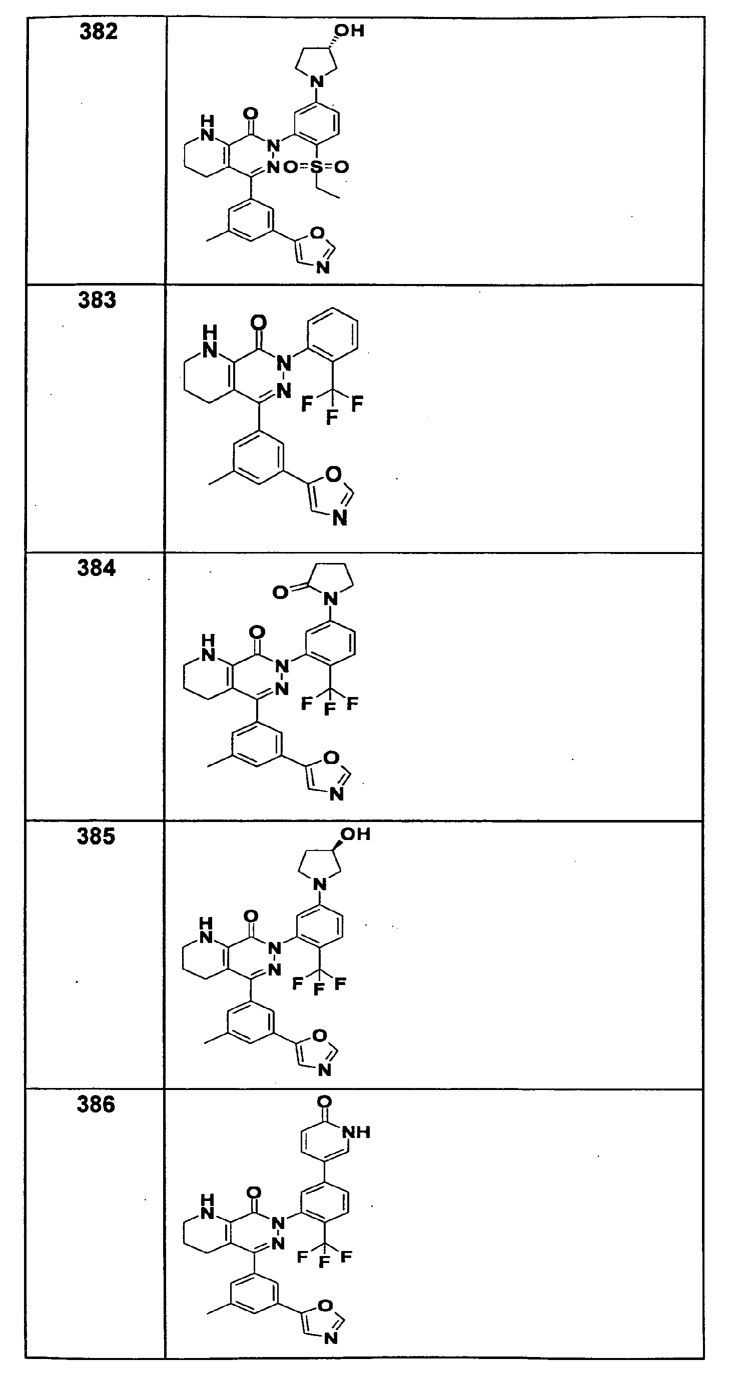

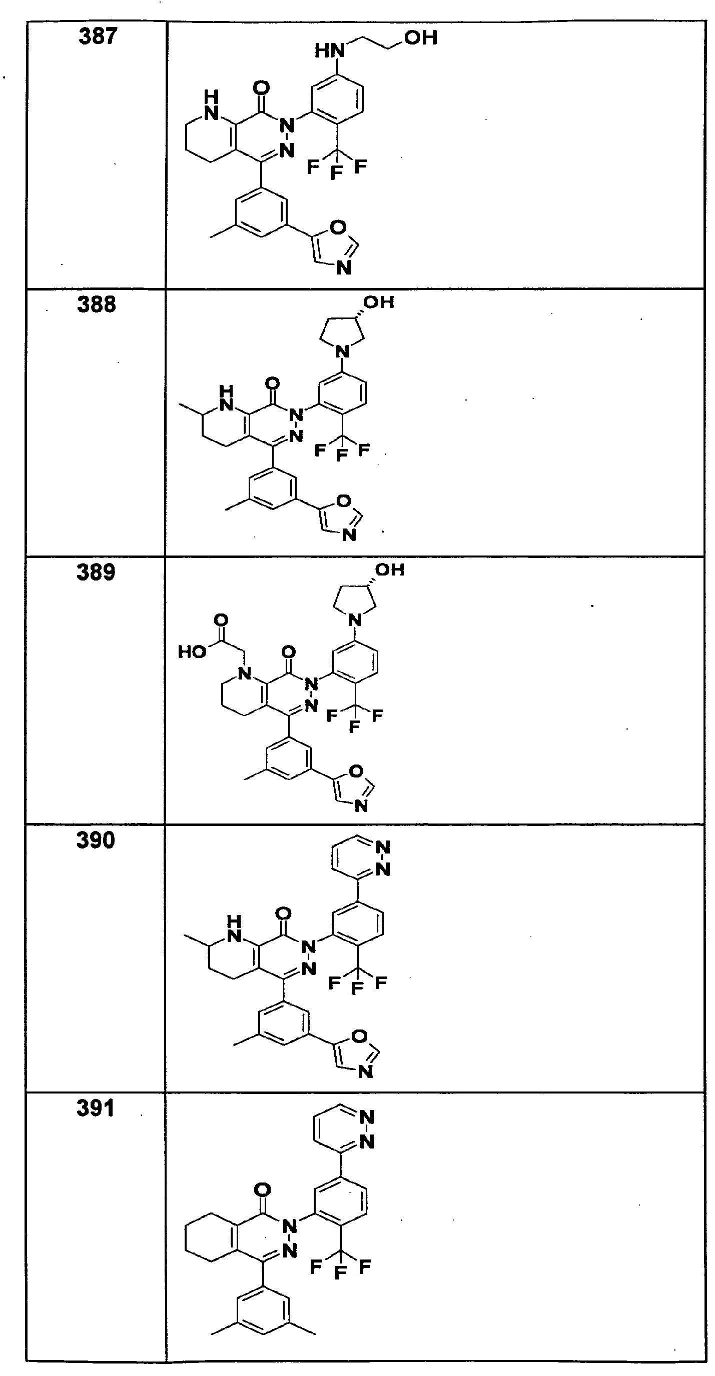

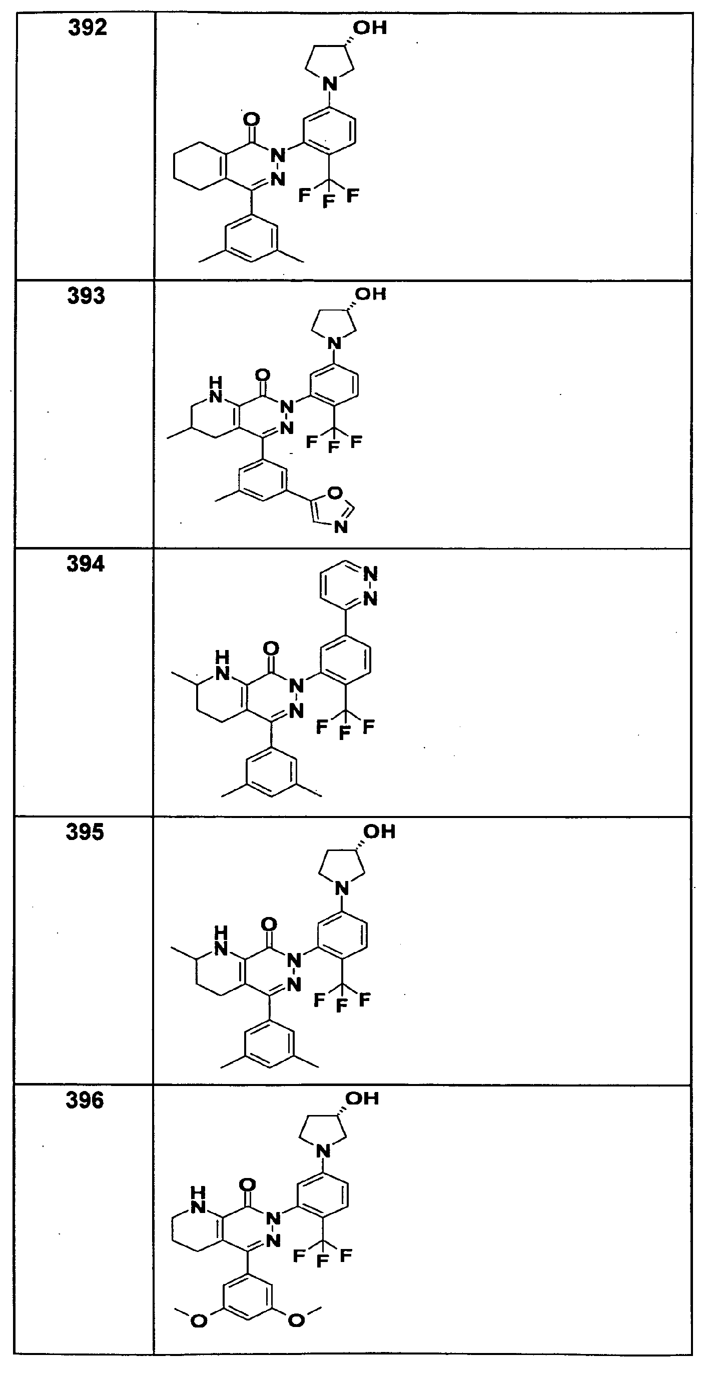

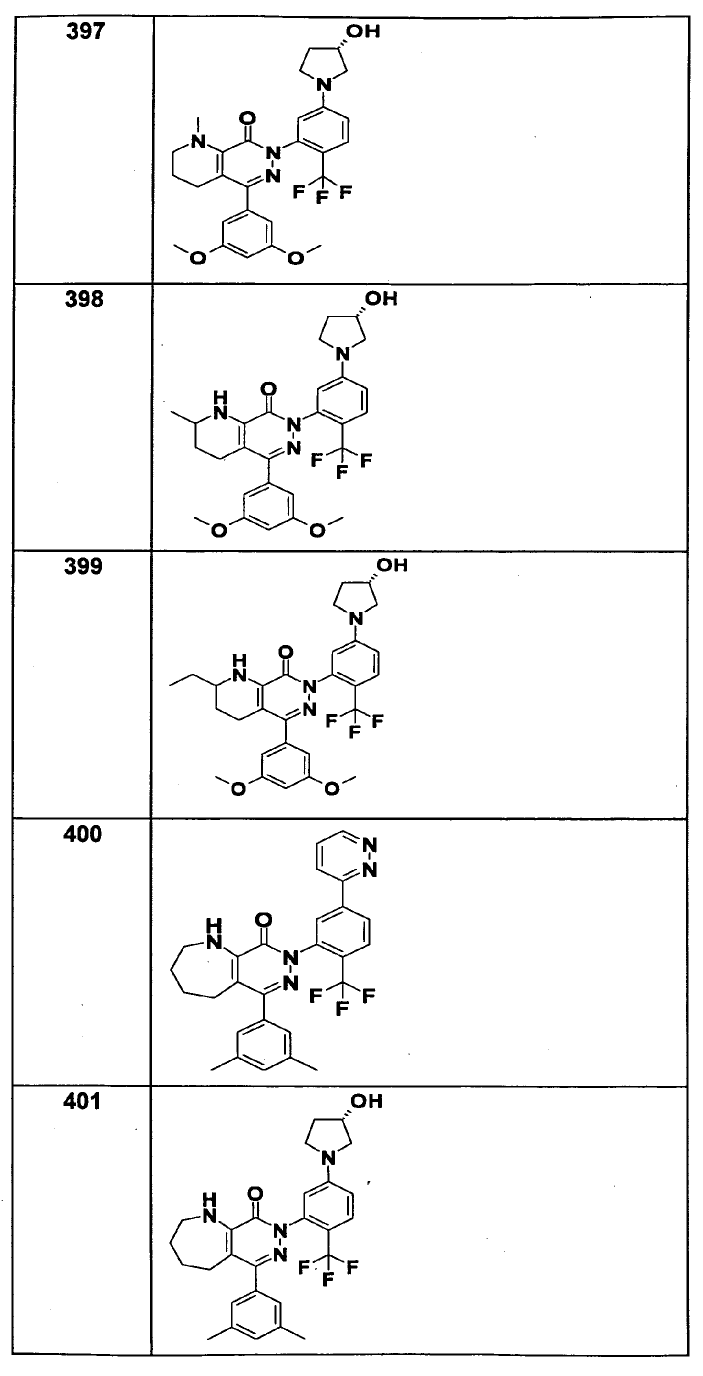

### Example 402-1

### 5-[5-(3-hydroxy-propylamino)-2-nitro-phenyl]-7-[3-methyl-5-(oxazol-5-yl)-phenyl]-5H-furo[2,3-d]pyridazin-4-one

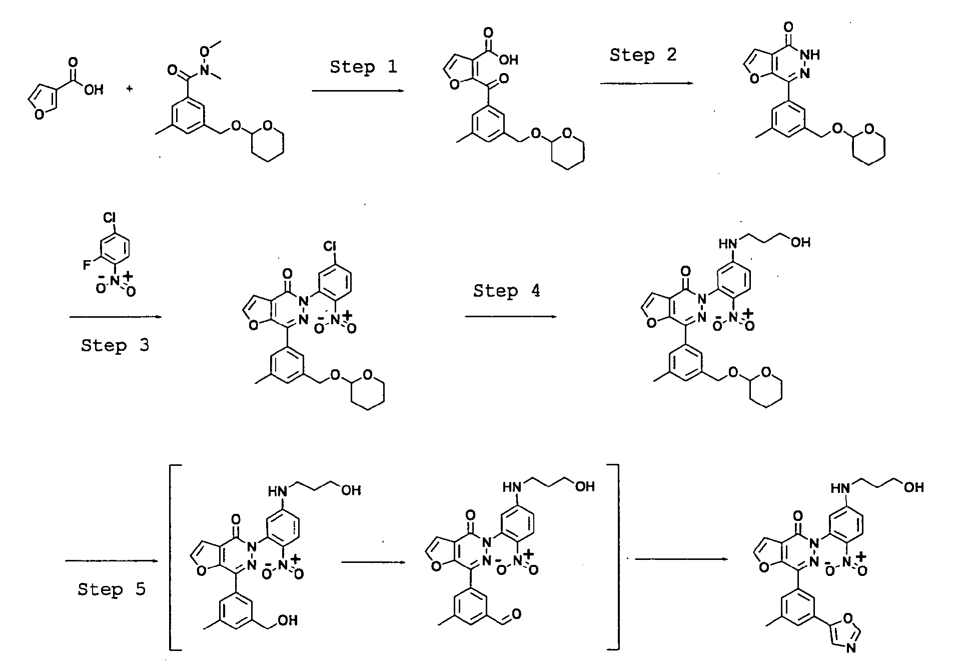

### Step 1

To a solution of furan-3-carboxylic acid (374 mg) in tetrahydrofuran (10 mL) was added dropwise lithium diisopropylamide (1.5 M cyclohexane solution; 4.45 mL) over 10 min under argon at -78°C. After stirring at the same temperature for 1 hr, a solution of N-methoxy-3,N-dimethyl-5-(tetrahydro-pyran-2-yloxymethyl)-benzamide (1.0 g) obtained in the below-mentioned Example 471, step 2, in tetrahydrofuran (3 mL) was added dropwise over 10 min at -78°C. The cooling bath was removed and, after stirring for 14 hr, a 10%
aqueous citric acid solution (20 mL) was added, and the mixture was extracted with ethyl acetate. The organic layer was washed with water, dried and concentrated to give 2-[3-methyl-5-(tetrahydro-pyran-2-yloxymethyl)-benzoyl]-furan-3-carboxylic acid (1.2 g).
¹H-NMR(CDCl₃)δ: 7.95(s,1H), 7.86(s,1H), 7.72(d,1H,J=1.9Hz), 7.53(s,1H), 7.28(d,1H,J=1.9Hz), 4.74(t,1H,J=3.7Hz), 4.87-4.56(AB,2H), 3.95-3.90(m,1H), 3.59-3.55(m,1H), 2.48(s,3H), 1.91-1.55(m,8H).

### Step 2

2-[3-Methyl-5-(tetrahydro-pyran-2-yloxymethyl)-benzoyl]-furan-3-carboxylic acid (1.2 g) obtained in step 1 was reacted in the same manner as in Example 1-1, step 3, to give 7-[3-methyl-5-(tetrahydro-pyran-2-yloxymethyl)-phenyl]-5H-furo[2,3-d]pyridazin-4-one (193 mg).
¹H-NMR(DMSO-d₆)δ: 13.11(s,1H), 8.33(d,1H,J=1.9Hz), 7.82(s,1H), 7.79(s,1H), 7.30(s,1H), 7.22(d,1H,J=1.9Hz), 4.75-4.50(m,3H), 3.85-3.79(m,1H), 3.53-3.47(m,1H), 2.42(s,3H), 1.79-1.64(m,2H), 1.58-1.48(m,4H).

### Step 3

7-[3-Methyl-5-(tetrahydro-pyran-2-yloxymethyl)-phenyl]-5H-furo[2,3-d]pyridazin-4-one (193 mg) obtained in step 2 and 4-chloro-2-fluoro-1-nitrobenzene (99 mg) were reacted in the same manner as in Example 1-5 to give 5-(5-chloro-2-nitro-phenyl)-7-[3-methyl-5-(tetrahydro-pyran-2-yloxymethyl)-phenyl]-5H-furo[2,3-d]pyridazin-4-one (269 mg).
¹H-NMR(CDCl₃)δ: 8.09(d,1H,J=8.8Hz), 7.86(d,1H,J=1.9Hz), 7.79(s,1H), 7.71(d,1H,J=2.3Hz), 7.58(dd,1H,J=8.8,2.3Hz), 7.34(s,1H), 7.18(d,1H,J=1.9Hz), 4.86-4.55(AB,2H), 4.74(t,1H,J=3.7Hz), 3.97-3.92(m,1H), 3.60-3.54(m,1H), 2.45(s,3H), 1.92-1.51(m,6H).

### Step 4

To a solution of 5-(5-chloro-2-nitro-phenyl)-7-[3-methyl-5-(tetrahydro-pyran-2-yloxymethyl)-phenyl]-5H-furo[2,3-d]pyridazin-4-one (269 mg) obtained in step 3 in 1,3-dimethyl-2-imidazolidone (2 mL) was added 3-hydroxy-propylamine (0.4 mL). Microwave (40 W, 130-140°C) was irradiated for 10 min, water was added, and the mixture was extracted with ethyl acetate. The organic layer was washed with water, dried and concentrated to give 5-[5-(3-hydroxy-propylamino)-2-nitro-phenyl]-7-[3-methyl-5-(tetrahydro-pyran-2-yloxymethyl)-phenyl]-5H-furo[2,3-d]pyridazin-4-one (296 mg).
¹H-NMR(CDCl₃)δ: 8.16(d,1H,J=8.8Hz), 7.86(s,1H), 7.82(d,1H,J=2.3Hz), 7.78(s,1H), 7.31(s,1H), 7.18(d,1H,J=2.3Hz), 6.66(d,1H,J=2.8Hz), 6.62(dd,1H,J=8.8,2.8Hz), 5.24(t,1H,J=5.3Hz), 4.84-4.54(AB,2H), 4.73(t,1H,J=3.7Hz), 3.96-3.91(m,1H), 3.84-3.80(m,2H), 3.58-3.53(m,1H), 3.40-3.36(m,2H), 2.43(s,3H), 1.92-1.53(m,8H).

### Step 5

To a solution of 5-[5-(3-hydroxy-propylamino)-2-nitro-phenyl]-7-[3-methyl-5-(tetrahydro-pyran-2-yloxymethyl)-phenyl]-5H-furo[2,3-d]pyridazin-4-one (296 mg) obtained in step 4 in ethanol (3 mL) was added p-toluenesulfonic acid monohydrate (19 mg). After stirring at room temperature for 3.5 hr, triethylamine (0.03 mL) was added, and the mixture was further stirred for 5 min. Ethyl acetate-water was added, and the mixture was separated into two layers. The aqueous layer was extracted with ethyl acetate. The organic layer was washed with water, dried and concentrated. The obtained residue (212 mg) was dissolved in N,N-dimethylformamide (2 mL). Imidazolium dichromate (384 mg) was added and, after stirring at room temperature for 4 hr, water was added, and the mixture was extracted with a mixed solvent of ethyl acetate and chloroform. The organic layer was washed with water, dried and concentrated. The obtained residue was dissolved in tetrahydrofuran (1 mL)-methanol (3 mL), and p-toluenesulfonylmethyl isocyanide (116 mg) and potassium carbonate (112 mg) were added. After stirring at 60°C for 4 hr, the mixture was allowed to return to room temperature, water was added, and the mixture was extracted with ethyl acetate. The organic layer was washed with water, dried, concentrated and purified by silica gel thin layer chromatography (chloroform:methanol=10:1) to give 5-[5-(3-hydroxy-propylamino)-2-nitro-phenyl]-7-[3-methyl-5-(oxazol-5-yl)-phenyl]-5H-furo[2,3-d]pyridazin-4-one (84 mg).
¹H-NMR(CDCl₃)δ: 8.18(d,1H,J=9.3Hz), 8.15(s,1H), 7.94(s,1H), 7.87(d,1H,J=1.9Hz), 7.85(s,1H), 7.58(s,1H), 7.42(s,1H), 7.20(d,1H,J=1.9Hz), 6.67(d,1H,J=2.8Hz), 6.63(dd,1H,J=9.3,2.8Hz), 5.31(s,1H), 3.82(t,2H,J=5.6Hz), 3.40-3.36(m,2H), 2.48(s,3H), 1.93-1.87(m,2H).

### Example 402-2

### 6-(5-bromo-2-trifluoromethyl-phenyl)-4-(3,5-dimethoxy-phenyl)-1,6-dihydro-pyrrolo[2,3-d]pyridazin-7-one

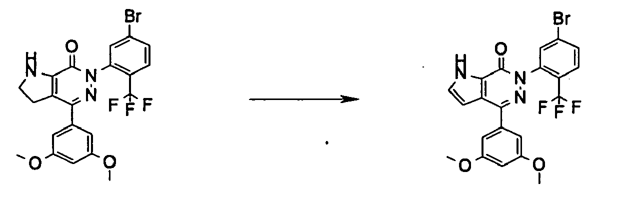

To a solution of 6-(5-bromo-2-trifluoromethylphenyl)-4-(3,5-dimethoxy-phenyl)-1,2,3,6-tetrahydro-pyrrolo[2,3-d]pyridazin-7-one (2.0 g) obtained in Example 1-20a in chloroform (60 mL) was added 2,3-dichloro-5,6-dicyano-p-benzoquinone (1.83 g). After stirring at 50°C for 18 hr, water (50 mL) was added, and the mixture was extracted twice with chloroform (50 mL). The organic layer was washed with water, dried, concentrated, and purified by silica gel column chromatography (ethyl acetate:hexane=1:3-1:2) to give a mixture (2.6 g) containing 6-(5-bromo-2-trifluoromethyl-phenyl)-4-(3,5-dimethoxy-phenyl)-1,6-dihydro-pyrrolo[2,3-d]pyridazin-7-one as a main component.

### Example 402-3

### N-((R)-1-{3-[4-(3,5-dimethoxy-phenyl)-1-(2-hydroxyethyl)-7-oxo-1,7-dihydro-pyrrolo[2,3-d]pyridazin-6-yl]-4-trifluoromethyl-phenyl}-pyrrolidin-3-yl)-acetamide

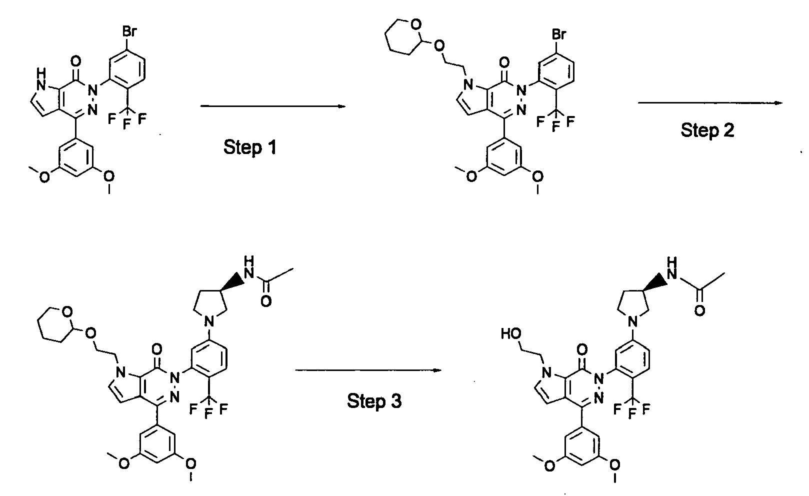

### Step 1

To a solution of 6-(5-bromo-2-trifluoromethylphenyl)-4-(3,5-dimethoxy-phenyl)-1,6-dihydro-pyrrolo[2,3-d]pyridazin-7-one (1.3 g) obtained in Example 402-2 in N,N-dimethylformamide (25 mL) were added 2-(2-bromoethoxy)-tetrahydro-2H-pyran (0.621 mL) and cesium carbonate (1.3 g). After stirring at 60°C for 15 hr, 2-(2-bromoethoxy)-tetrahydro-2H-pyran (0.207 mL) and cesium carbonate (0.43 g) were added. After stirring at 60°C for 4 hr, water (50 mL) was added, and the mixture was extracted twice with ethyl acetate (50 mL). The organic layer was washed with water, dried, concentrated and purified by silica gel column chromatography (ethyl acetate:hexane=1:4) to give 6-(5-bromo-2-trifluoromethyl-phenyl)-4-(3,5-dimethoxy-phenyl)-1-[2-(tetrahydro-pyran-2-yloxy)-ethyl]-1,6-dihydro-pyrrolo[2,3-d]pyridazin-7-one (1.0 g).
¹H-NMR(CDCl₃)δ: 7.76-7.66(m,3H), 7.37(d,1H,J=2.8Hz), 6.93(d,2H,J=2.3Hz), 6.63(d,1H,J=2.8Hz), 6.55(t,1H,J=2.3Hz), 4.88(ddd,1H,J=13.9,6.0,3.2Hz), 4.75(ddd,1H,J=13.9,7.0,3.2Hz), 4.54(brs,1H), 4.17-4.03(m,1H), 3.87-3.74(m,1H), 3.83(s,6H), 3.44(brs,1H), 1.83-1.71(m,1H), 1.71-1.62(m,1H), 1.60-1.41(m,5H).

### Step 2

6-(5-Bromo-2-trifluoromethyl-phenyl)-4-(3,5-dimethoxy-phenyl)-1-[2-(tetrahydro-pyran-2-yloxy)-ethyl]-1,6-dihydro-pyrrolo[2,3-d]pyridazin-7-one (100 mg) obtained in step 1 and (R)-N-(pyrrolidin-3-yl)-acetamide (24.7 mg) were reacted in the same manner as in Example 1-26 and purified by silica gel thin layer chromatography (chloroform:methanol=10:1) to give N-((R)-1-{3-[4-(3,5-dimethoxy-phenyl)-7-oxo-1-[2-(tetrahydro-pyran-2-yloxy)-ethyl]-1,7-dihydro-pyrrolo[2,3-d]pyridazin-6-yl]-4-trifluoromethylphenyl}-pyrrolidin-3-yl)-acetamide (110 mg).
¹H-NMR(CDCl₃)δ: 7.61(d,1H,J=8.8Hz), 7.34(d,1H,J=2.8Hz), 6.95(d,2H,J=2.3Hz), 6.62-6.59(m,3H), 6.53(t,1H,J=2.3Hz), 5.69(s,1H), 4.92-4.86(m,1H), 4.78-4.72(m,1H), 4.63-4.51(m,2H), 4.11-4.05(m,1H), 3.87-3.77(m,2H), 3.83(s,6H), 3.70-3.36(m,4H), 3.27-3.19(m,1H), 2.32-2.23(m,1H), 2.03-1.96(m,1H), 1.97(s,3H), 1.81-1.46(m,6H).

### Step 3

To a solution of N-((R)-1-{3-[4-(3,5-dimethoxy-phenyl)-7-oxo-1-[2-(tetrahydro-pyran-2-yloxy)-ethyl]-1,7-dihydro-pyrrolo[2,3-d]pyridazin-6-yl]-4-trifluoromethyl-phenyl}-pyrrolidin-3-yl)-acetamide (110 mg) obtained in step 2 in methanol (2 mL) was added p-toluenesulfonic acid monohydrate (3 mg). After stirring at room temperature for 4 hr, sodium hydrogencarbonate was added. The insoluble material was filtered off and the filtrate was purified by silica gel thin layer chromatography (chloroform:methanol=10:1) to give N-((R)-1-{3-[4-(3,5-dimethoxy-phenyl)-1-(2-hydroxy-ethyl)-7-oxo-1,7-dihydro-pyrrolo[2,3-d]pyridazin-6-yl]-4-trifluoromethyl-phenyl}-pyrrolidin-3-yl)-acetamide (89 mg).
¹H-NMR(DMSO-d₆)δ: 8.16(d,1H), 7.62-7.59(m,2H), 6.84(d,2H,J=1.9Hz), 6.74-6.62(m,4H), 4.99-4.85(m,2H), 4.43-4.32(m,1H), 3.79(s,6H), 3.78-3.69(m,2H), 3.58-3.11(m,4H), 2.24-2.11(m,1H), 1.97-1.84(m,1H), 1.80(s,3H).

### Example 402-7

### 7-(4-fluoro-3-methyl-phenyl)-5-[5-(3-hydroxymethyl-azetidin-1-yl)-2-trifluoromethyl-phenyl]-4-oxo-4,5-dihydro-thieno[2,3-d]pyridazine-3-carboxylic acid

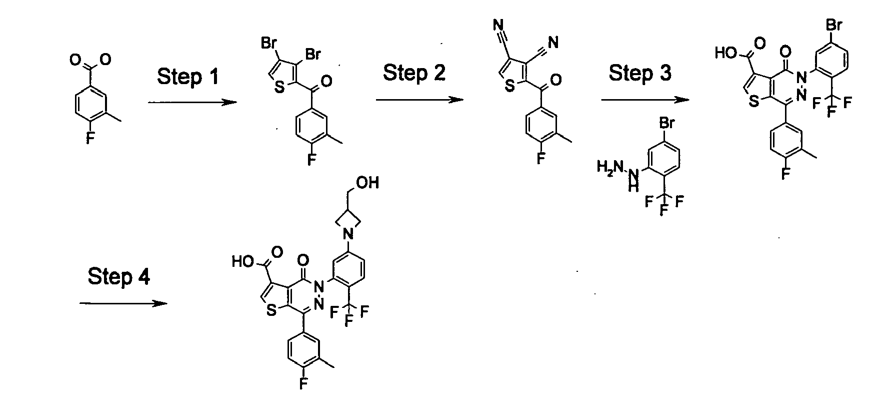

### Step 1

To a suspension of 4-fluoro-3-methyl-benzoic acid (10 g) in chloroform (100 mL) were added oxalylchloride (8 mL) and N,N-dimethylformamide(0.5 mL), and the mixture was stirred at room temperature for 1 hr. The reaction mixture was concentrated under reduced pressure and the obtained residue was added to a mixture of 3,4-dibromothiophene (7.2 mL) and carbon disulfide (60 mL). Further, aluminum chloride (8.7 g) was added, and the mixture was stirred at room temperature for 14 hrs. The reaction mixture was ice-cooled, 2N hydrochloric acid was added, and the mixture was extracted with diethyl ether. The organic layer was washed with water, dried and concentrated. The obtained crude crystals were washed with hexane to give (3,4-dibromo-thiophen-2-yl)-(4-fluoro-3-methyl-phenyl)-methanone (18.4 g).
¹H-NMR(CDCl₃)δ 7.73(dd,1H,*J*=2.3,7.2Hz), 7.67(m,1H),7.63(s,1H), 7.10(t,1H,*J*=8.8Hz), 2.35(d,3H,*J*=2.1Hz).

### Step 2

To a solution of (3,4-dibromo-thiophen-2-yl)-(4-fluoro-3-methyl-phenyl)-methanone (5 g) obtained in step 1 in N,N-dimethylformamide (15 mL) was added copper cyanide (3.5 g), and the mixture was stirred at 140°C for 5 hr. Ice water and aqueous ammonia were added to the reaction mixture, and the mixture was extracted with ethyl acetate. The organic layer was washed with aqueous ammonia, dried, concentrated and purified by silica gel column chromatography (ethyl acetate:hexane=1:3) to give 2-(4-fluoro-3-methylbenzoyl)-thiophene-3,4-dicarbonitrile (1.1 g).
¹H-NMR(CDCl₃)8.25(s,1H), δ 7.78(dd,1H,*J*=2.1,7.0Hz), 7.72(m,1H), 7.19(t,1H,*J*=8.6Hz), 2.38(d,3H,*J*=1.9Hz).

### Step 3

To a solution of 2-(4-fluoro-3-methyl-benzoyl)-thiophene-3,4-dicarbonitrile (1.05 g) obtained in step 2 in 1-propanol (20 mL) were added (5-bromo-2-trifluoromethyl-phenyl)-hydrazine (1.2 g) obtained in Example 1-8, supplementary step 1, and trifluoroacetic acid (0.36 mL), and the mixture was stirred at 80°C. After 8 hr, cesium carbonate (3.03 g) was added by small portions and the mixture was stirred at 100°C. After 12 hr, a 2N aqueous sodium hydroxide solution (2 mL) was added, and the mixture was further stirred at 100°C for 15 hr. The reaction mixture was allowed to return to room temperature, water was added and the mixture was extracted with ethyl acetate. The organic layer was washed with water, dried, concentrated and purified by silica gel column chromatography (chloroform:methanol=30:1) to give 5-(5-bromo-2-trifluoromethyl-phenyl)-7-(4-fluoro-3-methyl-phenyl)-4-oxo-4,5-dihydro-thieno[2,3-d]pyridazine-3-carboxylic acid (420 mg).
¹H-NMR(CDCl₃) δ 8.93(s,1H), 7.85(d,1H,*J*=8.6Hz), 7.77(m,2H), 7.64(m,2H), 7.20(t,1H,*J*=8.6Hz), 2.37(d,3H,*J*=2.0Hz).

### Step 4

To a mixture of palladium acetate (9 mg), rac-2,2'-bis(diphenylphosphino)-1,1'-binaphthyl (27 mg) and cesium carbonate (389 mg) was added dioxane (1.3 mL) under nitrogen atmosphere, and the mixture was stirred at room temperature. After 1 hr, a mixed solution of 5-(5-bromo-2-trifluoromethyl-phenyl)-7-(4-fluoro-3-methyl-phenyl)-4-oxo-4,5-dihydro-thieno[2,3-d]pyridazine-3-carboxylic acid (420 mg) obtained in step 3 and 3-hydroxymethyl-azetidine (90 mg) in dioxane (1.3 mL)-tert-butanol (1.3 mL) was added, and the mixture was stirred at 100°C for 15 hr. The reaction mixture was allowed to return to room temperature, and the insoluble material was filtered through celite. The filtrate was concentrated and the obtained residue was purified by silica gel column chromatography (chloroform:methanol=20:1) to give 7-(4-fluoro-3-methyl-phenyl)-5-[5-(3-hydroxymethyl-azetidin-1-yl)-2-trifluoromethyl-phenyl]-4-oxo-4,5-dihydro-thieno[2,3-d]pyridazine-3-carboxylic acid (74 mg).
¹H-NMR (CDCl₃) δ: 8.90(s,1H), 7.69-7.62(m,3H), 7.22-7.16(m,1H), 6.58-6.54(m,1H), 6.45-6.44(m,1H), 4.10-3.71(m,6H), 3.02-2.94(m,1H), 2.38(s,3H).

### Example 402-8

### methyl 7-(4-fluoro-3-methyl-phenyl)-5-[5-(3-hydroxymethyl-azetidin-1-yl)-2-trifluoromethyl-phenyl]-4-oxo-4,5-dihydro-thieno[2,3-d]pyridazine-3-carboxylate

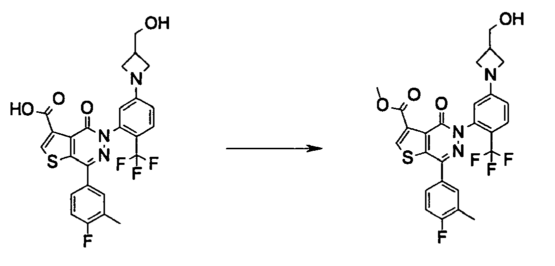

To a solution of 7-(4-fluoro-3-methyl-phenyl)-5-[5-(3-hydroxymethyl-azetidin-1-yl)-2-trifluoromethylphenyl]-4-oxo-4,5-dihydro-thieno[2,3-d]pyridazine-3-carboxylic acid (31 mg) obtained in Example 402-7 in N,N-dimethylformamide (0.3 mL) were added potassium carbonate (24 mg) and methyl iodide (0.005 mL) and the mixture was stirred at room temperature for 16 hr. Water was added to the reaction mixture, and the mixture was extracted with ethyl acetate. The organic layer was washed with water, dried, concentrated and purified by silica gel thin layer chromatography (ethyl acetate) to give methyl 7-(4-fluoro-3-methyl-phenyl)-5-[5-(3-hydroxymethyl-azetidin-1-yl)-2-trifluoromethylphenyl]-4-oxo-4,5-dihydro-thieno[2,3-d]pyridazine-3-carboxylate (9 mg).
¹H-NMR (CDCl₃) δ: 8.08(s,1H), 7.65-7.57(m,3H), 7.16-7.10(m,1H), 6.49-6.45(m,2H), 4.06-3.99(m,2H), 3.95(s,3H), 3.85(d,2H,J= 6.4Hz), 3.77-3.70(m,2H), 2.98-2.88(m,1H), 2.34 (d,3H, J = 1.9Hz).

### Example 417

### 6-[5-(3,3-difluoropyrrolidin-1-yl)-2-trifluoromethylphenyl]-4-(3,5-dimethoxyphenyl)-1,6-dihydro-pyrrolo[2,3-d]pyridazin-7-one

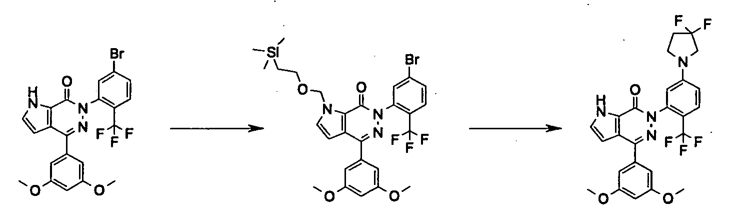

### Step 1

To a solution of 6-(5-bromo-2-trifluoromethylphenyl)-4-(3,5-dimethoxy-phenyl)-1,6-dihydro-pyrrolo[2,3-d]pyridazin-7-one (790 mg) obtained in Example 402-2 in N,N-dimethylformamide (8 mL) were added 2-(trimethylsilyl)-ethoxymethyl chloride (0.34 mL) and sodium hydride (60%; 77 mg). After stirring at room temperature for 1 hr, ice water was added, and the mixture was extracted with ethyl acetate. The organic layer was washed with water, dried, concentrated and purified by silica gel column chromatography (ethyl acetate:hexane=1:5-1:4) to give 6-(5-bromo-2-trifluoromethylphenyl)-4-(3,5-dimethoxyphenyl)-1-(2-trimethylsilylethoxymethyl)-1,6-dihydro-pyrrolo[2,3-d]pyridazin-7-one (940 mg).
¹H-NMR(CDCl₃)δ: 7.75-7.67(3H,m), 7.44(1H,d,*J*=3.2Hz), 6.93(2H,d,*J*=2.3Hz), 6.74(1H,d,*J*=3.2Hz), 6.56(1H,t,*J*=2.3Hz), 6.10-5.90(2H,m), 3.83(6H,s), 3.68-3.56(2H,m), 0.95-0.89(2H,m), -0.04(9H,s).

### Step 2

To a mixture of palladium acetate (4 mg), rac-2,2'-bis(diphenylphosphino)-1,1'-binaphthyl (11 mg) and cesium carbonate (156 mg) was added dioxane (1.4 mL) under nitrogen atmosphere, and the mixture was stirred at room temperature. After 1 hr, a solution of 6-(5-bromo-2-trifluoromethylphenyl)-4-(3,5-dimethoxyphenyl)-1-(2-trimethylsilylethoxymethyl)-1,6-dihydro-pyrrolo[2,3-d]pyridazin-7-one (100 mg) obtained in step 1 and 3,3-difluoropyrrolidine (28 mg) in *tert*-butanol (0.65 mL) were added and the mixture was stirred at 80°C for 2 hr. The reaction mixture was allowed to return to room temperature, and the insoluble material was filtered through celite. The filtrate was concentrated and the obtained residue was dissolved in chloroform (4 mL). Boron trifluoride-diethyl ether complex (0.066 mL) was added. After stirring at room temperature for 20 min, water was added, and the mixture was extracted with ethyl acetate. The organic layer was washed with water, dried and concentrated. To the residue were added acetonitrile (4 mL) and Triton B (40% aqueous solution; 0.167 mL). After stirring at room temperature for 1.5 hr, water was added, and the mixture was extracted with ethyl acetate. The organic layer was washed with water, dried, concentrated, purified by silica gel thin layer chromatography (chloroform:methanol=20:1), and further recrystallized from ethanol to give 6-[5-(3,3-difluoropyrrolidin-1-yl)-2-trifluoromethyl-phenyl]-4-(3,5-dimethoxyphenyl)-1,6-dihydro-pyrrolo[2,3-d]pyridazin-7-one (36 mg).
¹H-NMR(CDCl₃)δ: 11.38(1H,brs), 7.69(1H,d,*J*=8.3Hz), 7.35(1H,t,*J*=2.3Hz), 6.98(2H,d,*J*=2.3Hz), 6.72(1H,t,*J*=2.3Hz), 6.68-6.63(2H,m), 6.55(1H,t,*J*=2.3Hz), 3.83(6H,s), 3.78-3.68(2H,m), 3.60(2H,t,*J*=7.2Hz), 2.57-2.43(2H,m).

### Example 521

### 4-(3,5-dimethylphenyl)-2-(5-morpholin-4-yl-2-nitrophenyl)-2H-phthalazin-1-one

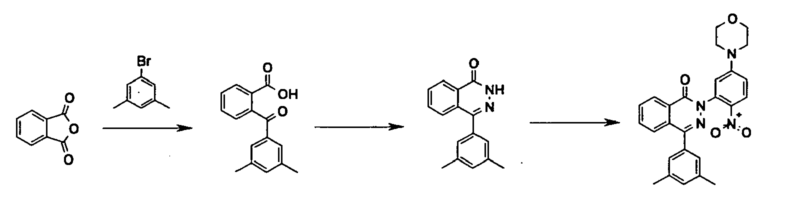

### Step 1

A solution of 3,5-dimethyl-bromobenzene (3.67 mL) in tetrahydrofuran (20 mL) was added dropwise over 30 min to magnesium (0.79 mg) under nitrogen atmosphere. After stirring at room temperature for 1.5 hr, it was added dropwise to a solution of phthalic anhydride (3.6 g) in tetrahydrofuran (35 mL) cooled to -70°C. The mixture was allowed to return to room temperature and stirred for 1.5 hr. 2N hydrochloric acid was added slowly, and the mixture was extracted with ethyl acetate. The organic layer was washed with water, dried and concentrated. The residue was adjusted to pH>10 by addition of 1N aqueous sodium hydroxide solution. The aqueous layer was washed with diethyl ether, acidified with 2N hydrochloric acid, and the precipitated crystals were collected by filtration to give 2-(3,5-dimethyl-benzoyl)benzoic acid (4.45 g).
¹H-NMR(CDCl₃)δ: 8.09(1H,d,*J*=7.7Hz), 7.67(1H,td,*J*=7.4,1.2Hz), 7.58(1H,td,*J*=7.7,1.2Hz), 7.38(1H,d,*J*=7.4Hz), 7.34(2H,s), 7,19(1H,s), 2.34(6H,s).

### Step 2

To a solution of 2-(3,5-dimethyl-benzoyl)benzoic acid (4.45 g) obtained in step 1 in ethanol (40 mL) was added hydrazine monohydrate (2.2 mL) and the mixture was stirred under refluxing for 4.5 hr. The reaction mixture was allowed to return to room temperature, ethanol (20 mL) was evaporated, and the precipitated crystal was collected by filtration to give 4-(3,5-dimethylphenyl)-2H-phthalazin-1-one (4.19 g).
¹H-NMR(CDCl₃)δ: 10.36(1H,brs), 8.55-8.50(1H,m), 7.83-7.78(3H,m), 7.20(2H,s), 7.16(1H,s), 2.42(6H,s).

### Step 3

4-(3,5-Dimethylphenyl)-2H-phthalazin-1-one (200 mg) obtained in step 2, potassium carbonate (332 mg), 4-chloro-2-fluoro-1-nitrobenzene (169 mg), and morpholine (0.35 mL) were reacted in the same manner as in Example 836-1, step 2. The mixture was purified by silica gel column chromatography (ethyl acetate:hexane=1:1-2:1), and further recrystallized from a mixed solvent of ethyl acetate-hexane to give 4-(3,5-dimethylphenyl)-2-(5-morpholin-4-yl-2-nitrophenyl)-2H-phthalazin-1-one (240 mg).
¹H-NMR(CDCl₃)δ: 8.58-8.54(1H,m), 8.22(1H,d,*J*=9.5Hz), 7.85-7.77(3H,m), 7.20(2H,s), 7.14(1H,s), 6.98(1H,d,*J*=2.8Hz), 6.90(1H,dd,*J=*9.5,2.8Hz), 3.86-3.84(4H,m), 3.42-3.39(4H,m), 2.40(6H,s).

### Example 471

### 7-[5-((S)-3-hydroxy-pyrrolidin-1-yl)-2-trifluoromethylphenyl]-5-(3-methyl-5-oxazol-5-yl-phenyl)-7H-pyrido[2,3-d]pyridazin-8-one

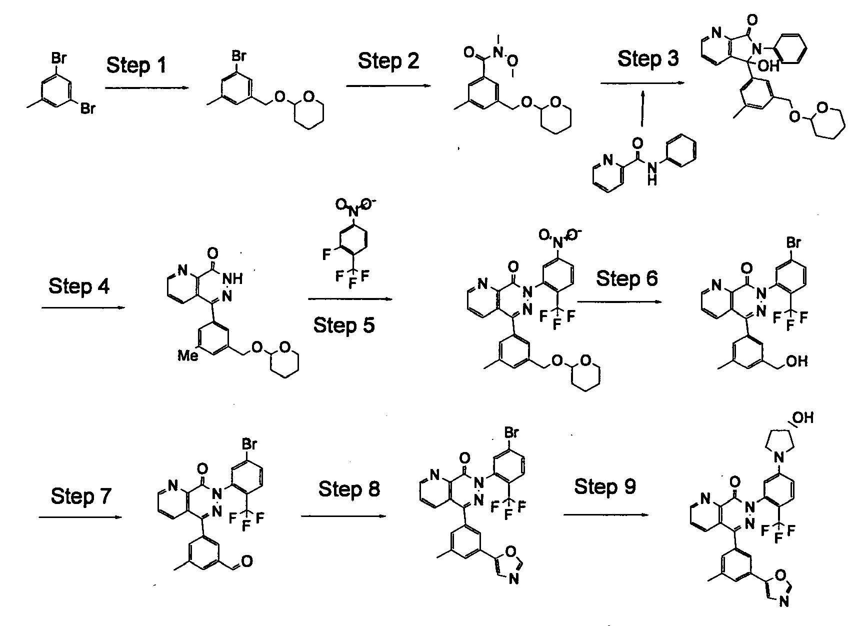

### Step 1

To a solution of 1,3-dibromo-5-methylbenzene (20 g) in tetrahydrofuran (200 mL) was added dropwise under argon n-butyllithium (1.59 M hexane solution; 52.8 mL) at -78°C over 10 min. After stirring at the same temperature for 1 hr, N,N-dimethylformamide (12.4 mL) was added. After stirring at -78°C for 1.5 hr, a saturated aqueous ammonium chloride solution was added, and the mixture was allowed to return to room temperature. After extraction with ethyl acetate, the organic layer was washed with water, dried and concentrated to give a crude product (16 g). This was dissolved in ethanol (100 mL), and sodium borohydride (1.51 g) was added under ice-cooling. After stirring at the same temperature for 3.5 hr, saturated brine was added, and ethanol was evaporated. The aqueous layer was extracted with ethyl acetate, and the organic layer was washed with water, dried and concentrated. The residue was dissolved in chloroform (100 mL), and dihydropyran (8.75 mL) and camphorsulfonic acid (930 mg) were added. After stirring at room temperature for 15 hr, the mixture was washed with an aqueous sodium hydrogencarbonate solution and brine. The organic layer was dried, concentrated and purified by silica gel column chromatography (ethyl acetate:hexane=1:20) to give 2-(3-bromo-5-methyl-benzyloxy)-tetrahydropyran (19.9 g).
¹H-NMR(CDCl₃)δ: 7.32(s,1H), 7.24(s,1H), 7.08(s,1H), 4.75-4.67(m,2H), 4.42(d,1H,J=12.1Hz), 3.99-3.84(m,1H), 3.60-3.50(m,1H), 2.32(s,3H), 1.91-1.53(m,6H).

### Step 2

To a solution of 2-(3-bromo-5-methyl-benzyloxy)-tetrahydropyran (13.5 g) obtained in step 1 in tetrahydrofuran (100 mL) was added dropwise n-butyllithium (1.59 M hexane solution; 32.7 mL) over 10 min under argon at -78°C. After stirring at the same temperature for 1 hr, N,N-dimethylformamide (7.4 mL) was added. After stirring at -78°C for 1 hr, a saturated aqueous ammonium chloride solution was added, and the mixture was allowed to return to room temperature. The mixture was extracted with ethyl acetate, and the organic layer was washed with water, dried and concentrated. The obtained residue was dissolved in tetrahydrofuran (120 mL)-tert-butanol (100 mL)-water (35 mL). The mixture was ice-cooled, and sodium chlorite (12.8 g), amylene (25 mL) and sodium dihydrogen phosphate dihydrate (9.6 g) were added. After stirring at the same temperature for 7 hr, the solvent was evaporated, and the residue was basified with a 1N aqueous sodium hydroxide solution. The mixture was washed with diethyl ether, and the aqueous layer was acidified with 1N hydrochloric acid and extracted with ethyl acetate. The organic layer was washed with water, dried and concentrated to give a crude product (9.8 g). The resultant product and N,O-dimethylhydroxylamine hydrochloride (4.6 g) were reacted in the same manner as in Example 1-27 and purified by silica gel column chromatography (ethyl acetate:hexane=1:2) to give N-methoxy-3,N-dimethyl-5-(tetrahydro-pyran-2-yloxymethyl)-benzamide (10.45 g).
¹H-NMR(CDCl₃)δ: 7.46(s,1H), 7.39(s,1H), 7.28(s,1H), 4.79(d,1H,J=12.1Hz), 4.71(t,1H,J=3.5Hz), 4.50(d,1H,J=12.1Hz), 3.96-3.89(m,1H), 3.59-3.53(m,4H), 3.35(s,3H), 2.39(s,3H), 1.90-1.85(m,1H), 1.79-1.54(m,5H).

### Step 3

To a solution of N-phenyl-2-pyridinecarboxamide (4.24 g) produced from 2-pyridinecarboxylic acid and aniline by a method similar to the method of Example 1-11 in tetrahydrofuran (130 mL) was added dropwise n-butyllithium (1.59 M hexane solution; 27 mL) over 10 min under argon at -78°C. After stirring from -78°C to under ice-cooling for 2 hr, the mixture was cooled again to -78°C, and a solution of N-methoxy-3,N-dimethyl-5-(tetrahydro-pyran-2-yloxymethyl)-benzamide (5.23 g) obtained in step 2 in tetrahydrofuran (10 mL) was added dropwise. After stirring at the same temperature for 45 min and under ice-cooling for 2 hr, water was added, and the mixture was extracted with ethyl acetate. The organic layer was washed with water, dried, concentrated and purified by silica gel column chromatography (ethyl acetate:hexane=1:2-1:1) to give 5-hydroxy-5-[3-methyl-5-(tetrahydro-pyran-2-yloxymethyl)-phenyl]-6-phenyl-5,6-dihydro-pyrrolo[3,4-b]pyridin-7-one (4.33 g).
¹H-NMR(CDCl₃)δ: 9.90(br,1H), 8.76-8.74(m,1H), 7.75(dt,1H,J=7.8,1.7Hz), 7.65-7.51(m,5H), 7.37-7.25(m,3H), 7.09(t,1H,J=7.3Hz), 4.72(d,1H,J=12.5Hz), 4.64(brs,1H), 4.46(d,1H,J=12.5Hz), 3.90-3.78(m,1H), 3.55-3.44(m,1H), 2.36(s,3H), 1.70-1.48(m,6H).

### Step 4

5-Hydroxy-5-[3-methyl-5-(tetrahydro-pyran-2-yloxymethyl)-phenyl]-6-phenyl-5,6-dihydro-pyrrolo[3,4-b]pyridin-7-one (4.6 g) obtained in step 3 and hydrazine monohydrate were reacted in the same manner as in Example 1-1, step 3, the latter half, to give 5-[3-methyl-5-(tetrahydro-pyran-2-yloxymethyl)-phenyl]-7H-pyrido[2,3-d]pyridazin-8-one (3.4 g).
¹H-NMR(CDCl₃)δ: 10.26(brs,1H), 9.15(dd,1H,J=4.4,1.5Hz), 8.15(dd,1H,J=8.4,1.5Hz), 7.72(dd,1H,J=8.4,4.4Hz), 7.36(s,2H), 7.29(s,1H), 4.86(d,1H,J=12.1Hz), 4.75(t,1H,J=3.5Hz), 4.55(d,1H,J=12.1Hz), 3.96-3.89(m,1H), 3.57-3.55(m,1H), 2.46(s,3H), 1.80-1.65(m,6H).

### Step 5

5-[3-Methyl-5-(tetrahydro-pyran-2-yloxymethyl)-phenyl]-7H-pyrido[2,3-d]pyridazin-8-one (500 mg) obtained in step 4 and 2-fluoro-4-nitro-1-trifluoromethyl-benzene (330 mg) obtained in Example 1-1, supplementary step 2, were reacted in the same manner as in Example 1-1, step 4, and purified by silica gel column chromatography (ethyl acetate:hexane=1:1-2:1) to give 5-[3-methyl-5-(tetrahydro-pyran-2-yloxymethyl)-phenyl-7-(5-nitro-2-trifluoromethyl-phenyl)-7H-pyrido[2,3-d]pyridazin-8-one (300 mg).
¹H-NMR(CDCl₃)δ: 9.22 (1H,dd, J=4.4,1.8Hz), 8.54-8.41(2H,m), 8.21(1H,dd,J=8.3,1.8Hz), 8.06(2H,d,J=8.8Hz), 7.79(1H,dd,J=8.3,4.4Hz), 7.36(1H,d,J=4.8Hz), 7.27(1H,d,J=7.0Hz), 7.26(1H,s), 4.84(1H,d,J=12.3Hz), 4.73(1H,t,J=3.3Hz), 4.54(1H,d,J=12.3Hz), 3.98-3.85(1H,m), 3.64-3.46(1H,m), 1.77-1.54(6H,m).

### Step 6

5-[3-Methyl-5-(tetrahydro-pyran-2-yloxymethyl)-phenyl-7-(5-nitro-2-trifluoromethyl-phenyl)-7H-pyrido[2,3-d]pyridazin-8-one (300 mg) obtained in step 5 were reacted in the same manner as in Examples 1-2 and 1-3, and purified by silica gel column chromatography (ethyl acetate:hexane=3:1) to give 7-(5-bromo-2-trifluoromethyl-phenyl)-5-(3-hydroxymethyl-5-methyl-phenyl)-7H-pyrido[2,3-d]pyridazin-8-one (150 mg).
¹H-NMR(CDCl₃)δ: 9.20(1H,dd,J=4.5,1.5Hz), 8.17(1H,dd,J=8.3,1.5Hz), 7.81-7.66(4H,m), 7.36(2H,d,J=4.5Hz), 7.30(1H,s), 4.76(2H,d,J=5.7Hz), 2.44(3H,s).

### Step 7

To a solution of 7-(5-bromo-2-trifluoromethylphenyl)-5-(3-hydroxymethyl-5-methyl-phenyl)-7H-pyrido[2,3-d]pyridazin-8-one (150 mg) obtained in step 6 in chloroform (3 mL) was added 1,1,1-tris(acetyloxy)-1,1-dihydro-1,2-benziodoxol-3-(1H)-one (143 mg) under ice-cooling. After stirring at the same temperature for 1 hr, an aqueous thiosodium sulfate solution was added, and the mixture was extracted with ethyl acetate. The organic layer was washed with an aqueous sodium hydrogencarbonate solution, dried, concentrated and purified by silica gel column chromatography (ethyl acetate:hexane=2:1-4:1) to give 3-[7-(5-bromo-2-trifluoromethyl-phenyl)-8-oxo-7,8-dihydro-pyrido[2,3-d]pyridazin-5-yl]-5-methyl-benzaldehyde (118 mg).
¹H-NMR(CDCl₃)δ: 10.07(1H,s), 9.23(1H,dd,J=4.6,1.9Hz), 8.13(1H,dd,J=8.3,1.9Hz), 7.89(1H,s), 7.86(1H,s), 7.83-7.69(4H,m), 7.66(1H,s), 2.53(3H,s).

### Step 8

To a solution of 3-[7-(5-bromo-2-trifluoromethylphenyl)-8-oxo-7,8-dihydro-pyrido[2,3-d]pyridazin-5-yl]-5-methyl-benzaldehyde (118 mg) obtained in step 7 in tetrahydrofuran (0.4 mL)-methanol (1.5 mL) were added potassium carbonate (40 mg) and p-toluenesulfonyl methylisocyanide (52 mg). After stirring at room temperature for 15 min, potassium carbonate (20 mg) and methanol (1.5 mL) were added, and the mixture was stirred under refluxing for 30 min. The mixture was allowed to return to room temperature, and water was added. Tetrahydrofuran-methanol was evaporated, and the mixture was extracted with ethyl acetate. The organic layer was washed with water, dried, concentrated and purified by silica gel column chromatography (ethyl acetate:hexane=3:1 to ethyl acetate) to give 7-(5-bromo-2-trifluoromethyl-phenyl)-5-[3-methyl-5-(oxazol-5-yl)-phenyl]-7H-pyrido[2,3-d]pyridazin-8-one (118 mg).
¹H-NMR(CDCl₃)δ: 9.22(1H,dd,J=4.5,1.6Hz), 8.18(1H,dd,J=8.1,1.6Hz), 7.93(1H,s), 7.83-7.69(4H,m), 7.65(2H,d,J=6.4Hz), 7.41(1H,s), 7.35(1H,s), 2.49(3H,s).

### Step 9

7-(5-Bromo-2-trifluoromethyl-phenyl)-5-[3-methyl-5-(oxazol-5-yl)-phenyl]-7H-pyrido[2,3-d]pyridazin-8-one (118 mg) obtained in step 8 and (S)-3-(tert-butyldiphenyl-silanyloxy)-pyrrolidine (106 mg) were reacted in the same manner as in Example 1-32 and purified by silica gel column chromatography (ethyl acetate-chloroform:methanol=20:1-10:1) to give 7-[5-((S)-3-hydroxy-pyrrolidin-1-yl)-2-trifluoromethyl-phenyl]-5-[3-methyl-5-(oxazol-5-yl)-phenyl]-7H-pyrido[2,3-d]pyridazin-8-one (81 mg).
¹H-NMR(CDCl₃)δ: 9.20(1H,dd,J=4.5,1.5Hz), 8.18(1H,dd,J=8.3,1.5Hz), 7.93(1H,s), 7.76(1H,dd,J=8.3,4.5Hz), 7.70-7.59(3H,m), 7.41-7.36(2H,m), 6.69-6.60(2H,m), 4.62(1H,brs), 3.62-3.37(3H,m), 3.32(1H,d,J=10.2Hz), 2.48(3H,s), 2.25-2.01(2H,m).

### Examples 403 to 835

The compounds of Examples 403 to 835 were obtained in the same manner as in a method selected from Examples 402-1 to 402-8. The compounds of Examples 402-1 to 402-8 and the compounds of Examples 403 to 835 obtained in the same manner are shown in the following Tables. In the tables, the left line shows Example numbers and the right line shows the structural formulas of the corresponding compounds.

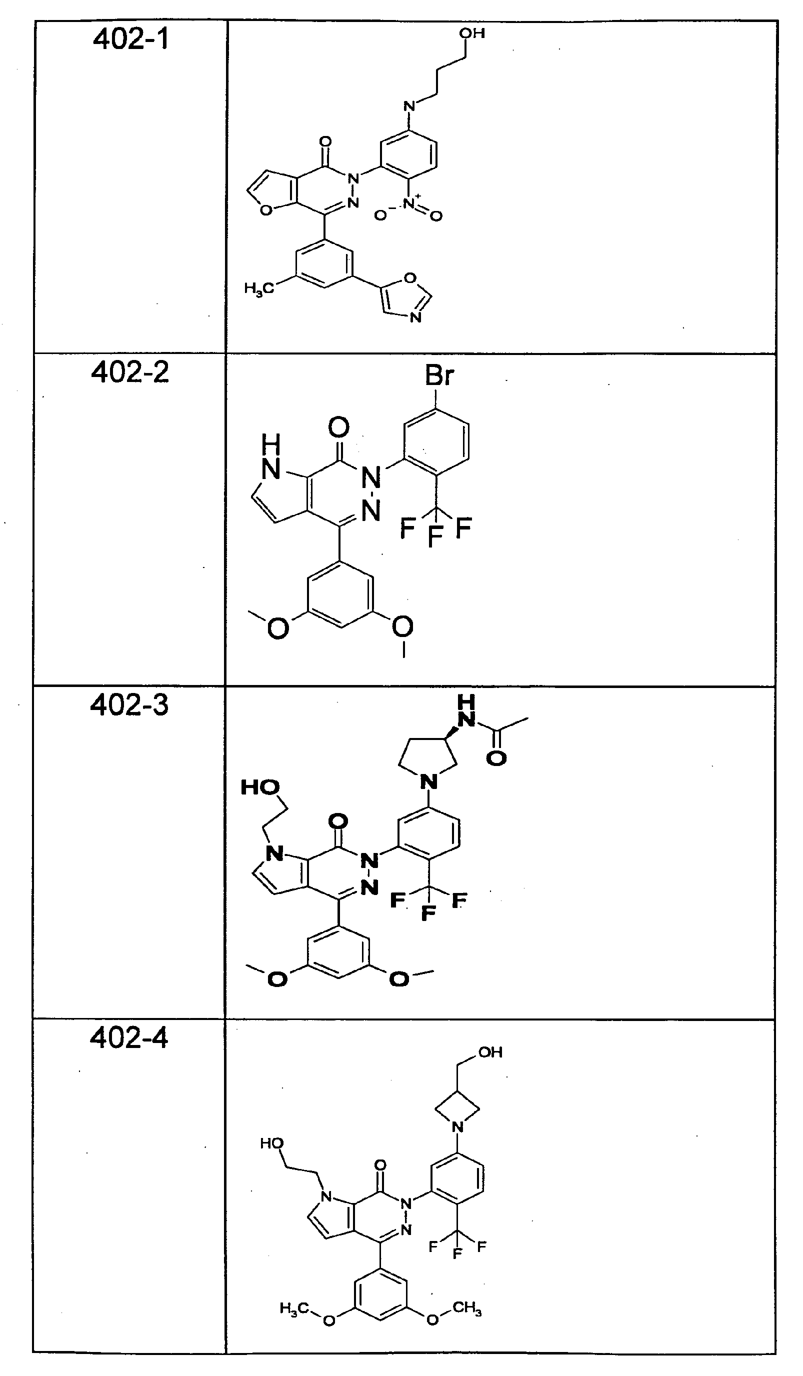

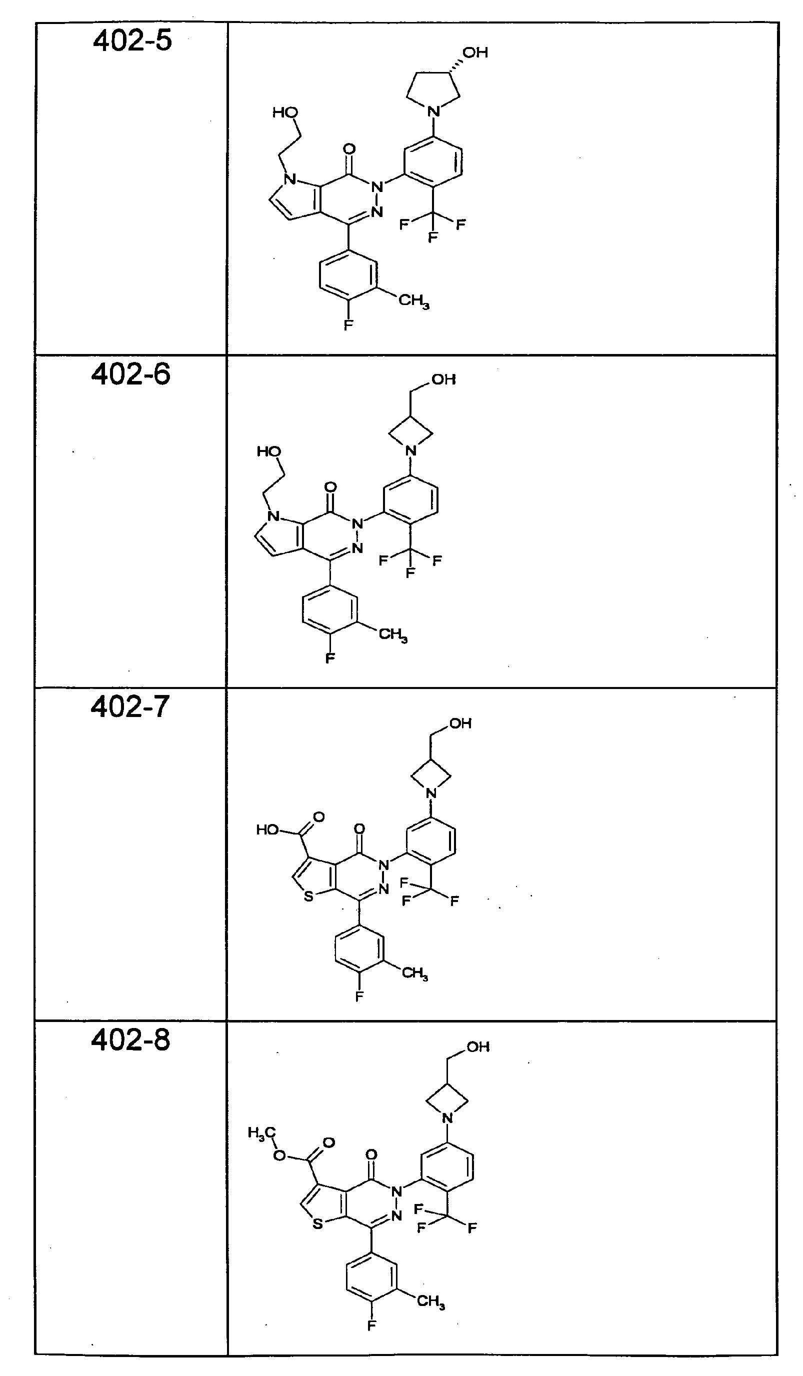

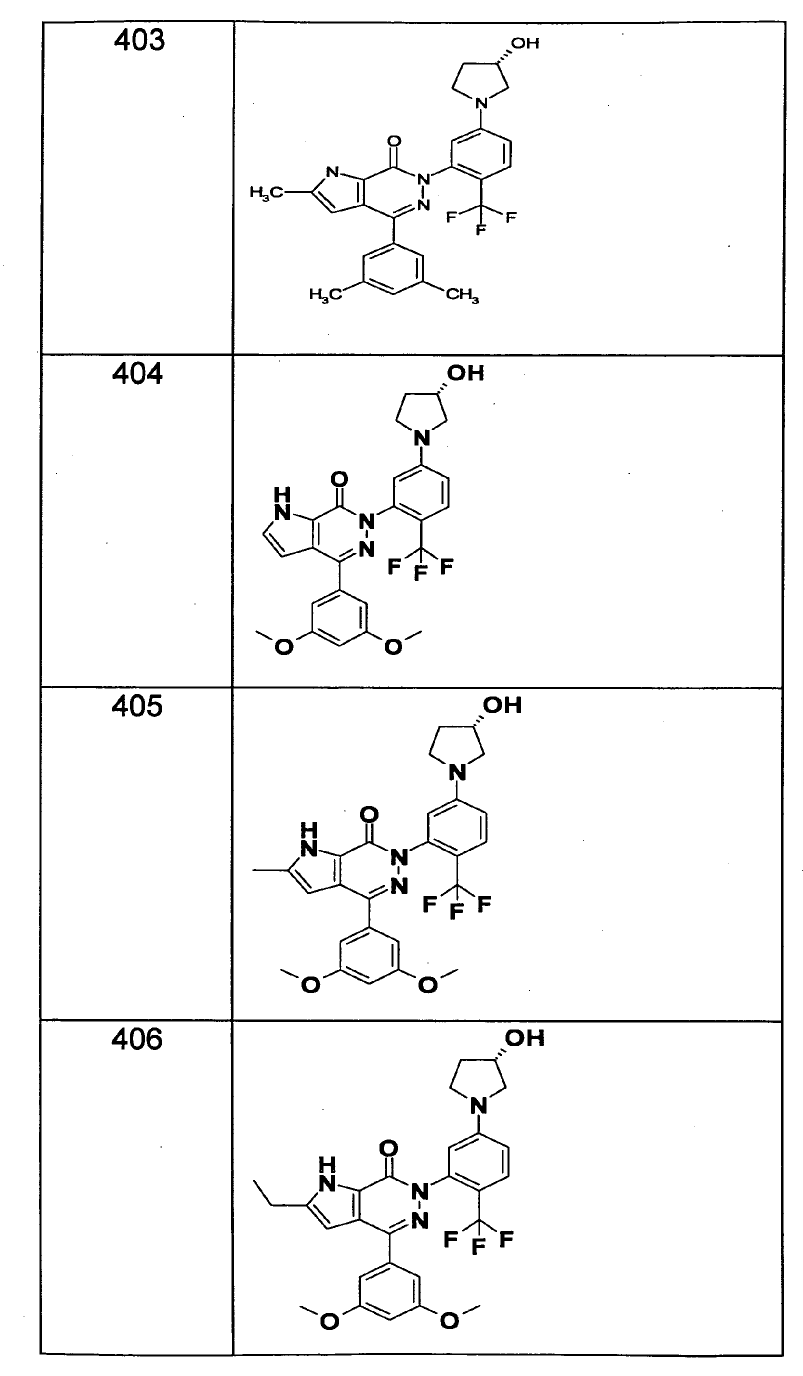

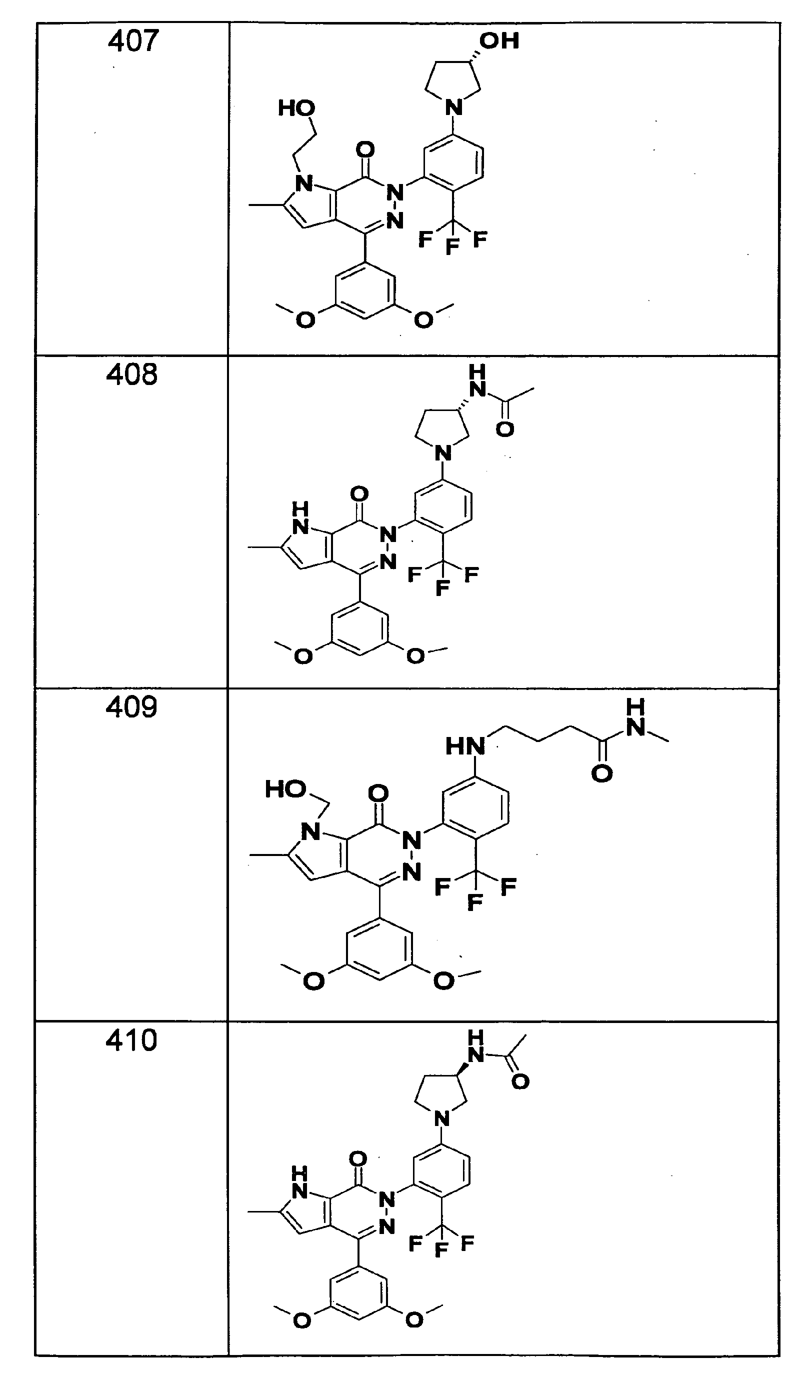

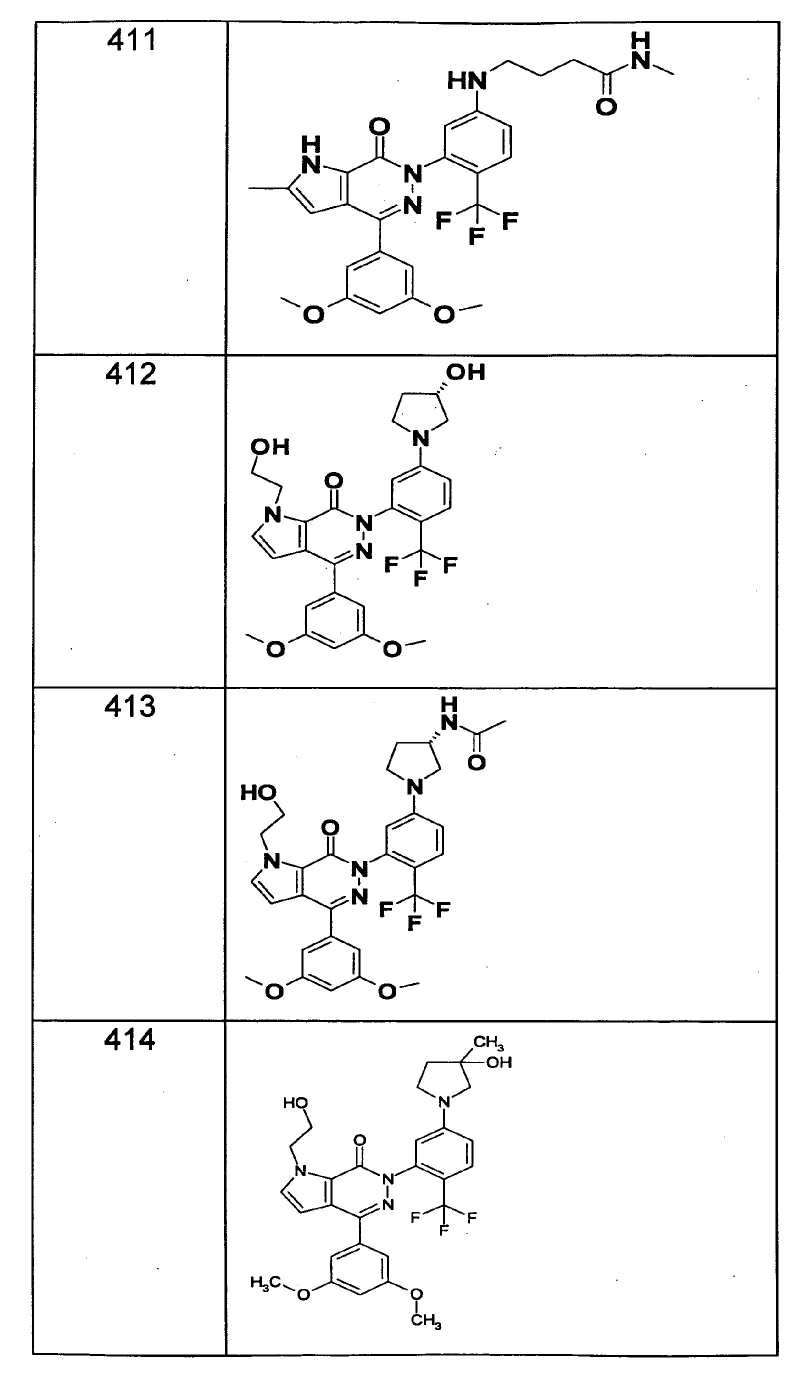

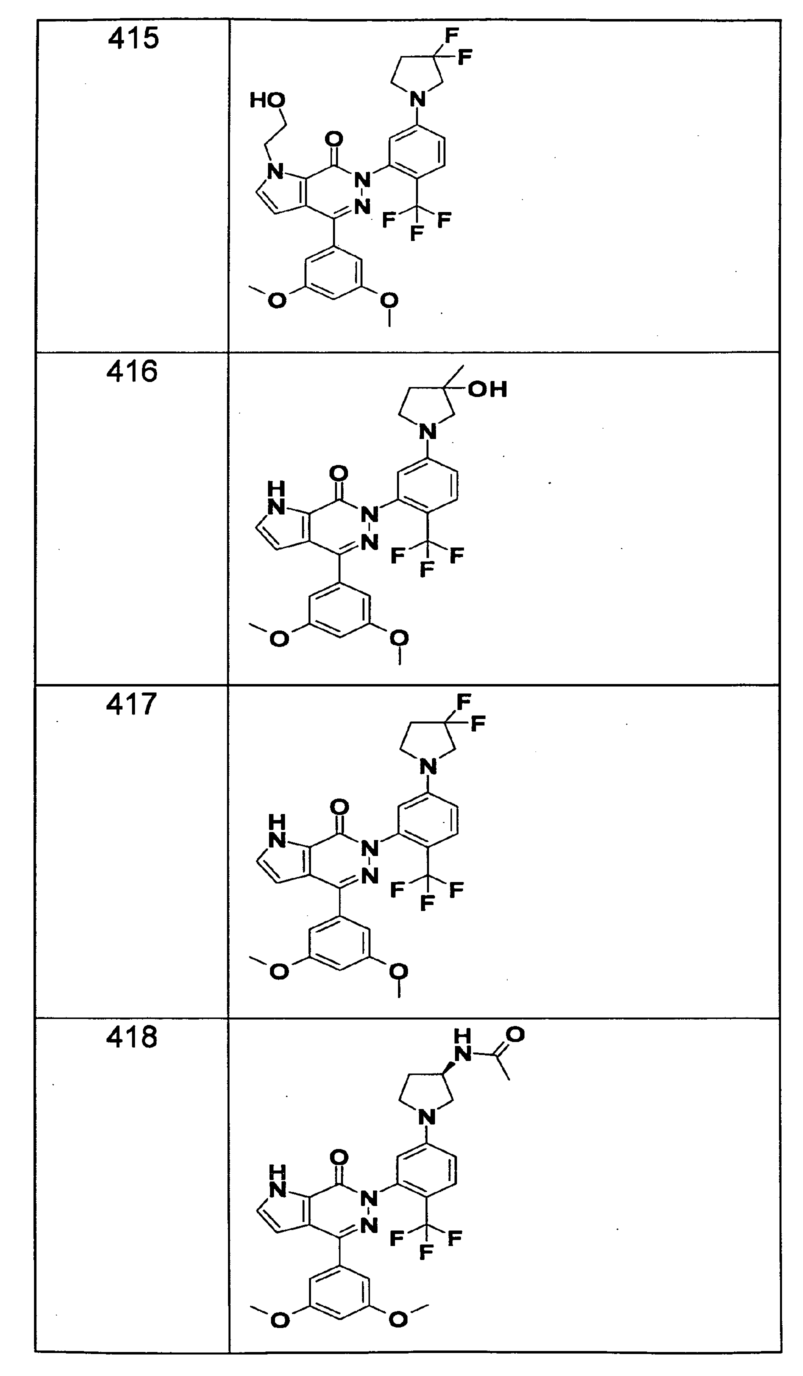

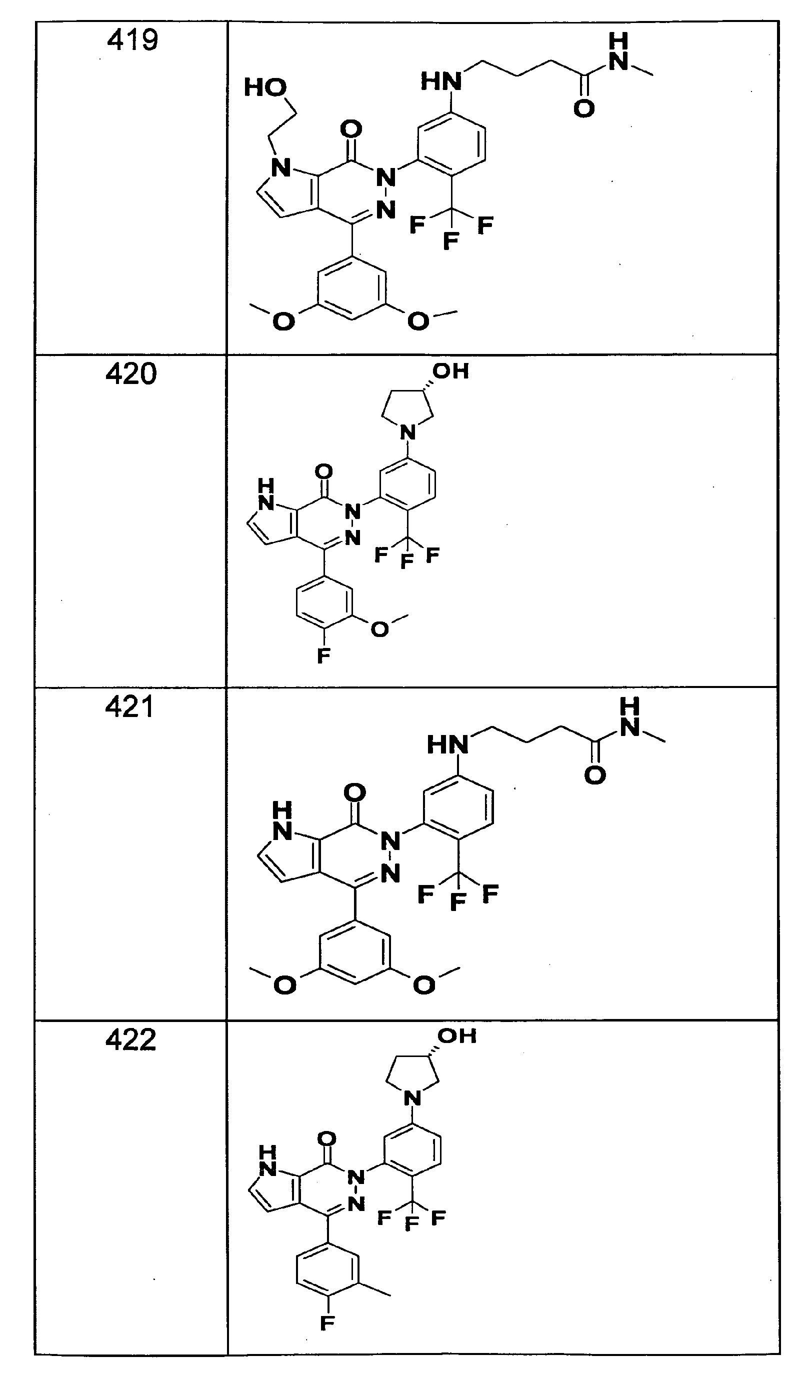

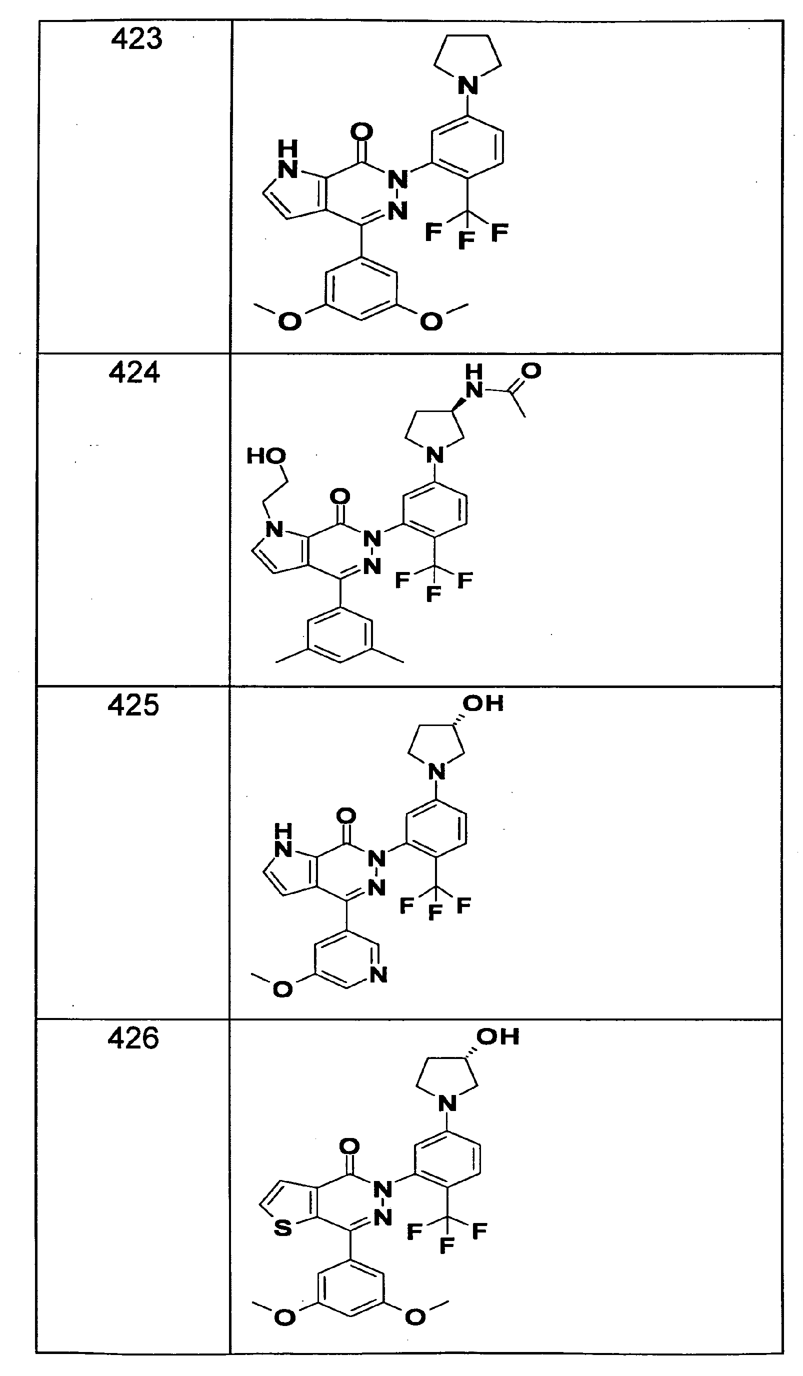

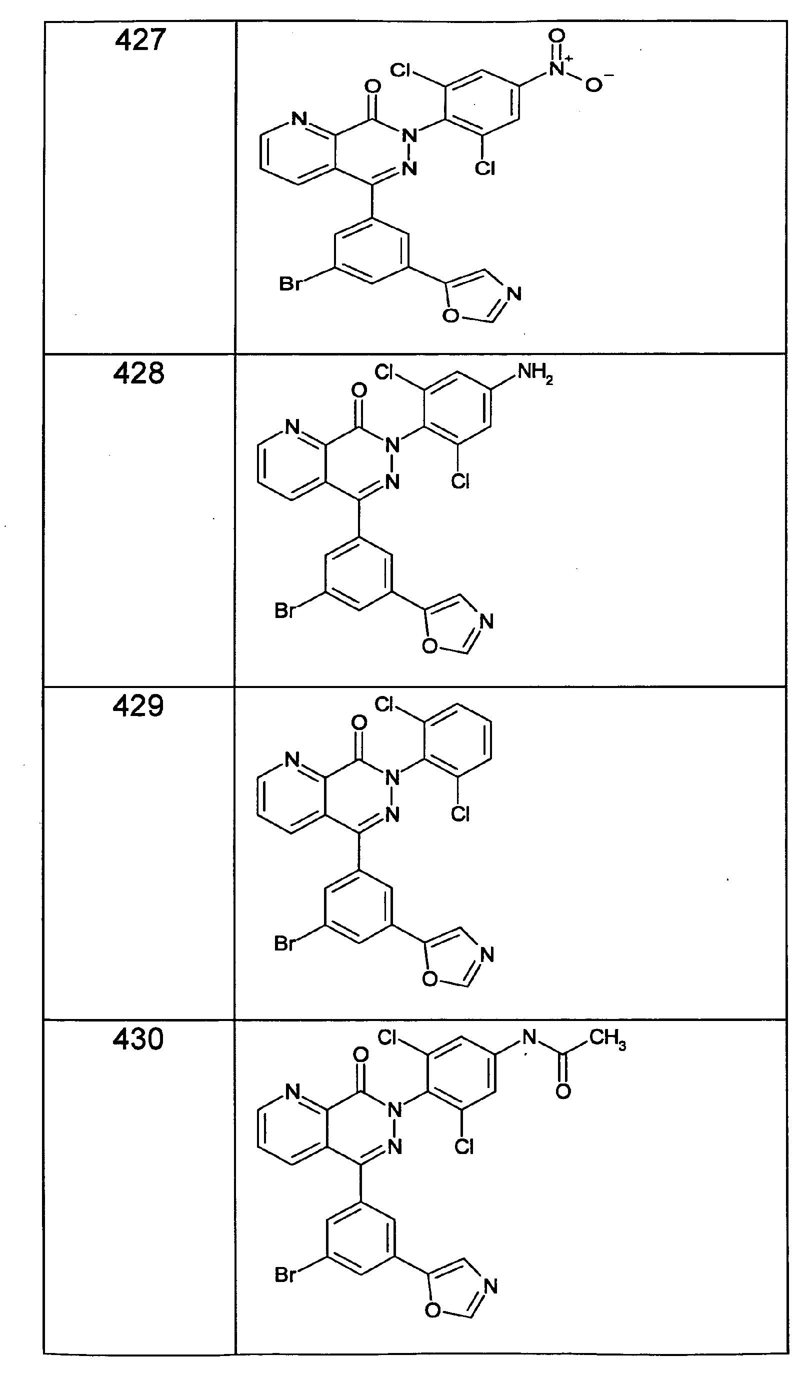

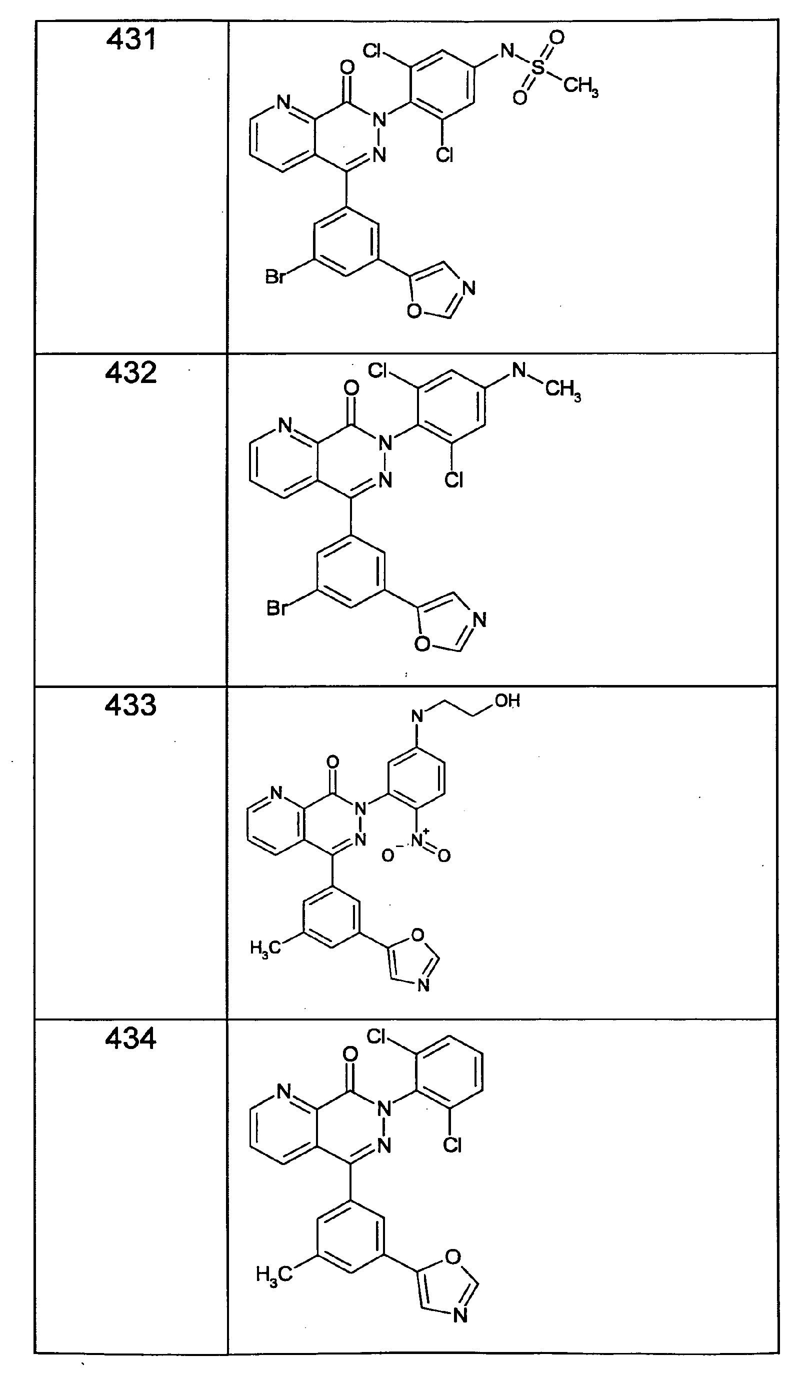

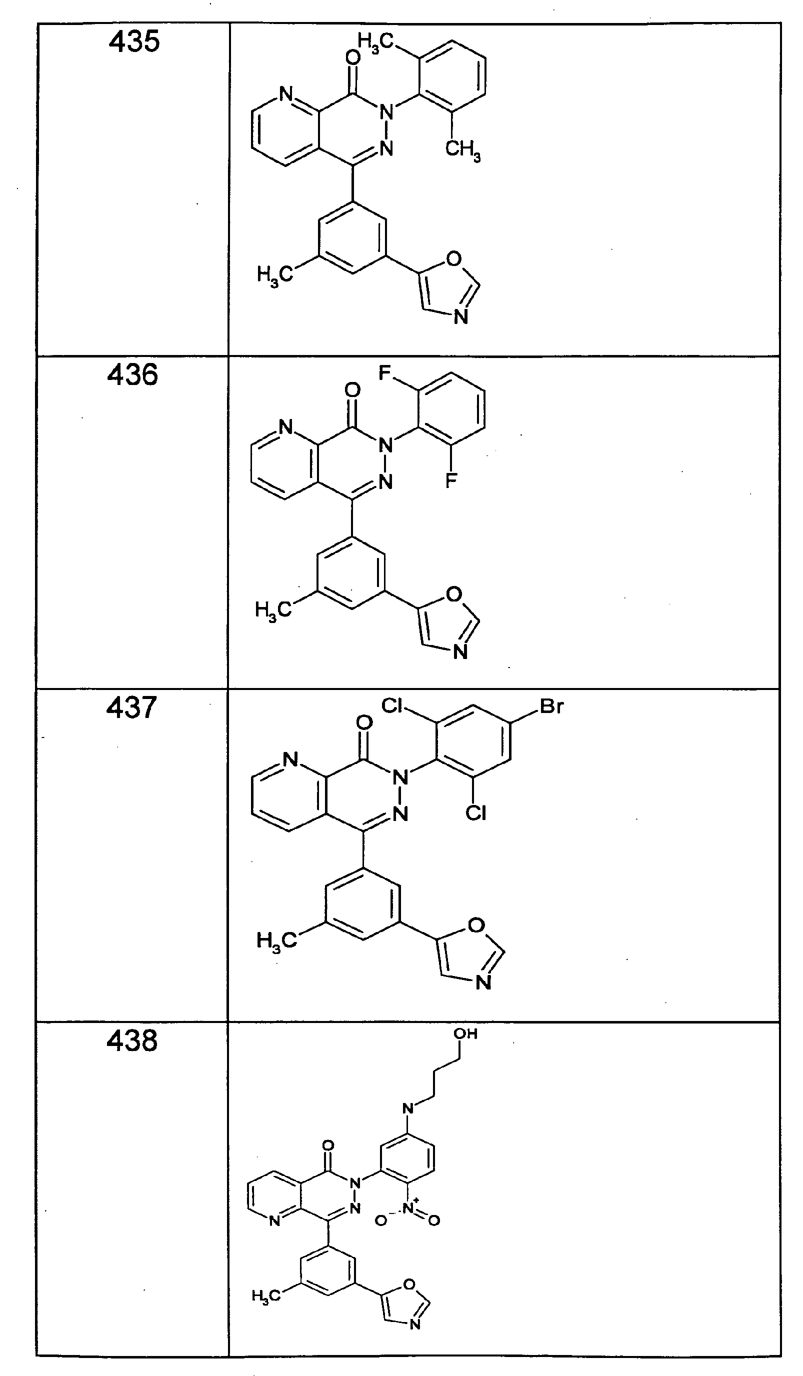

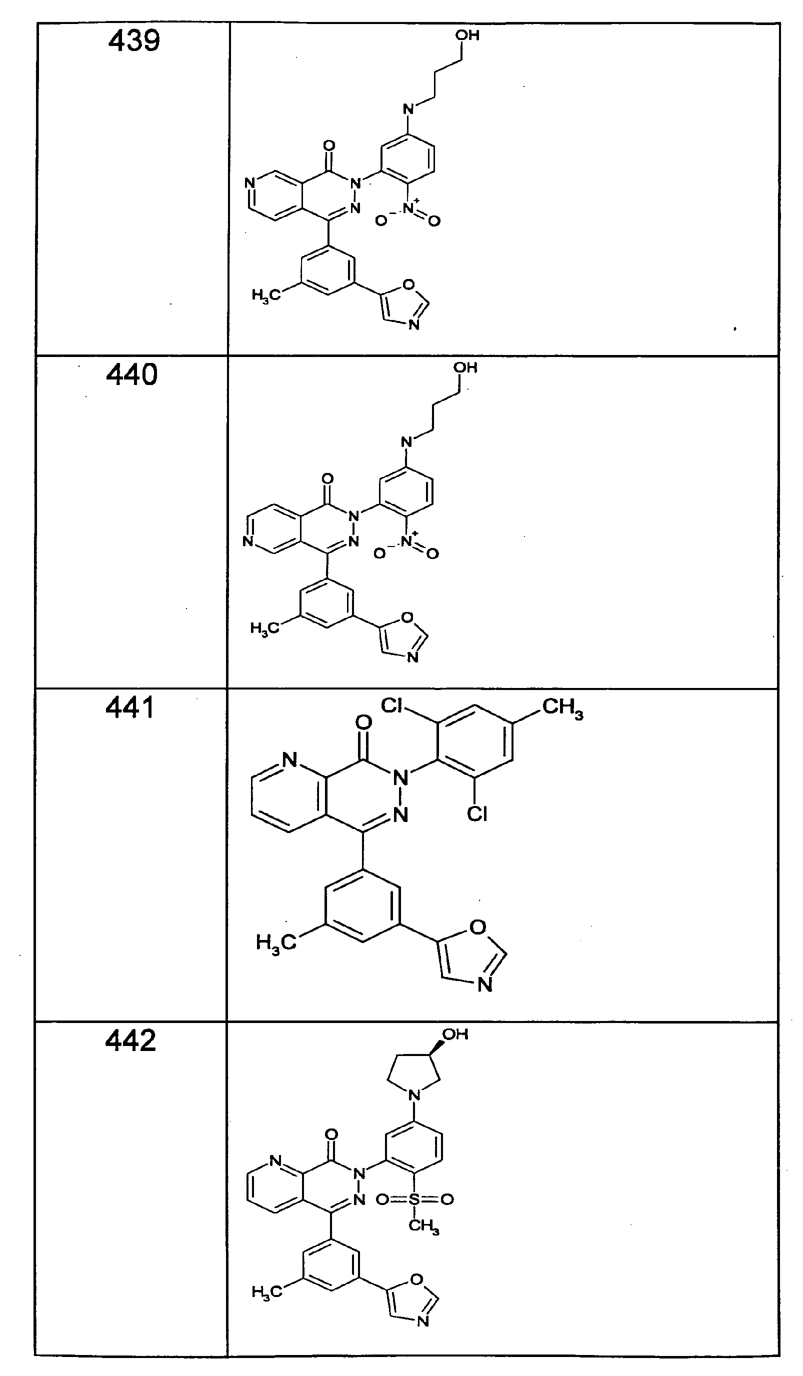

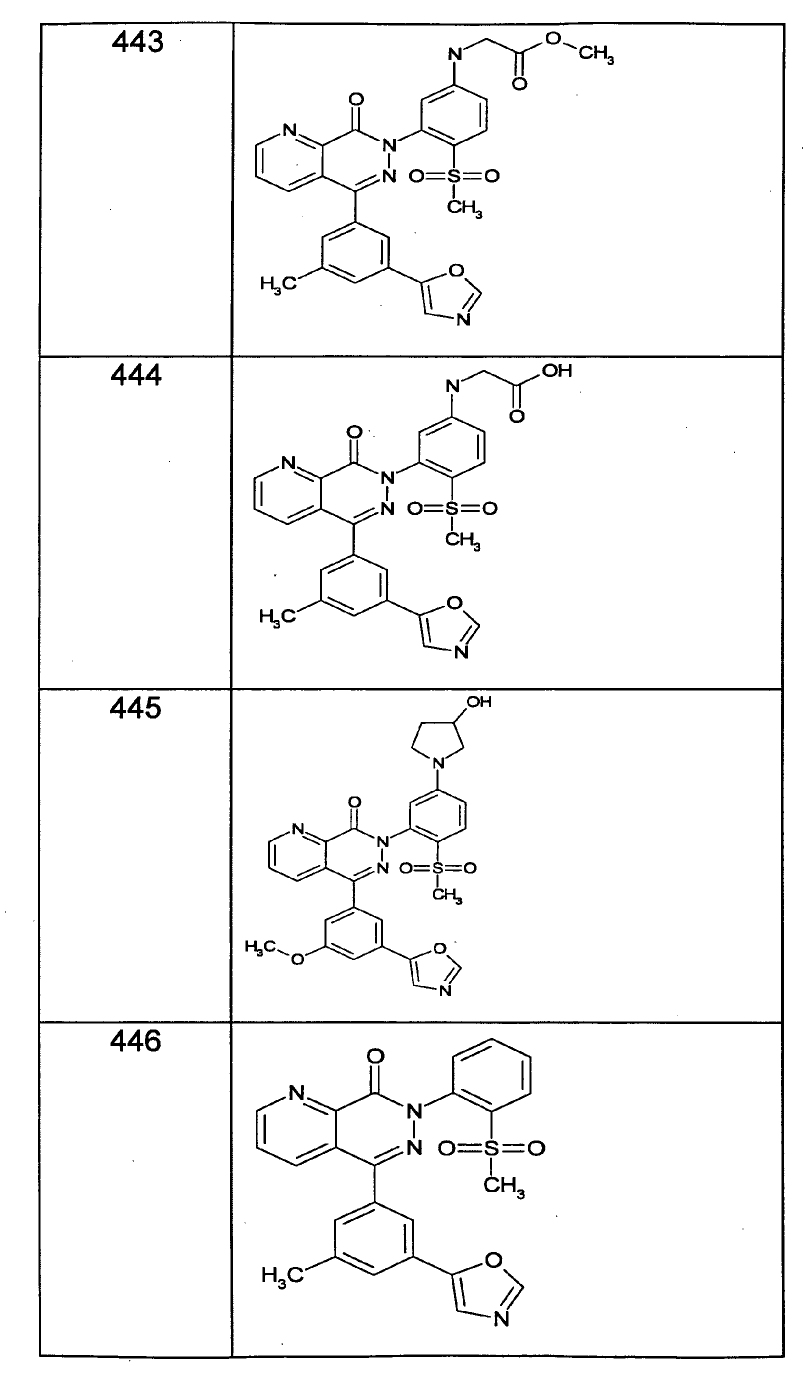

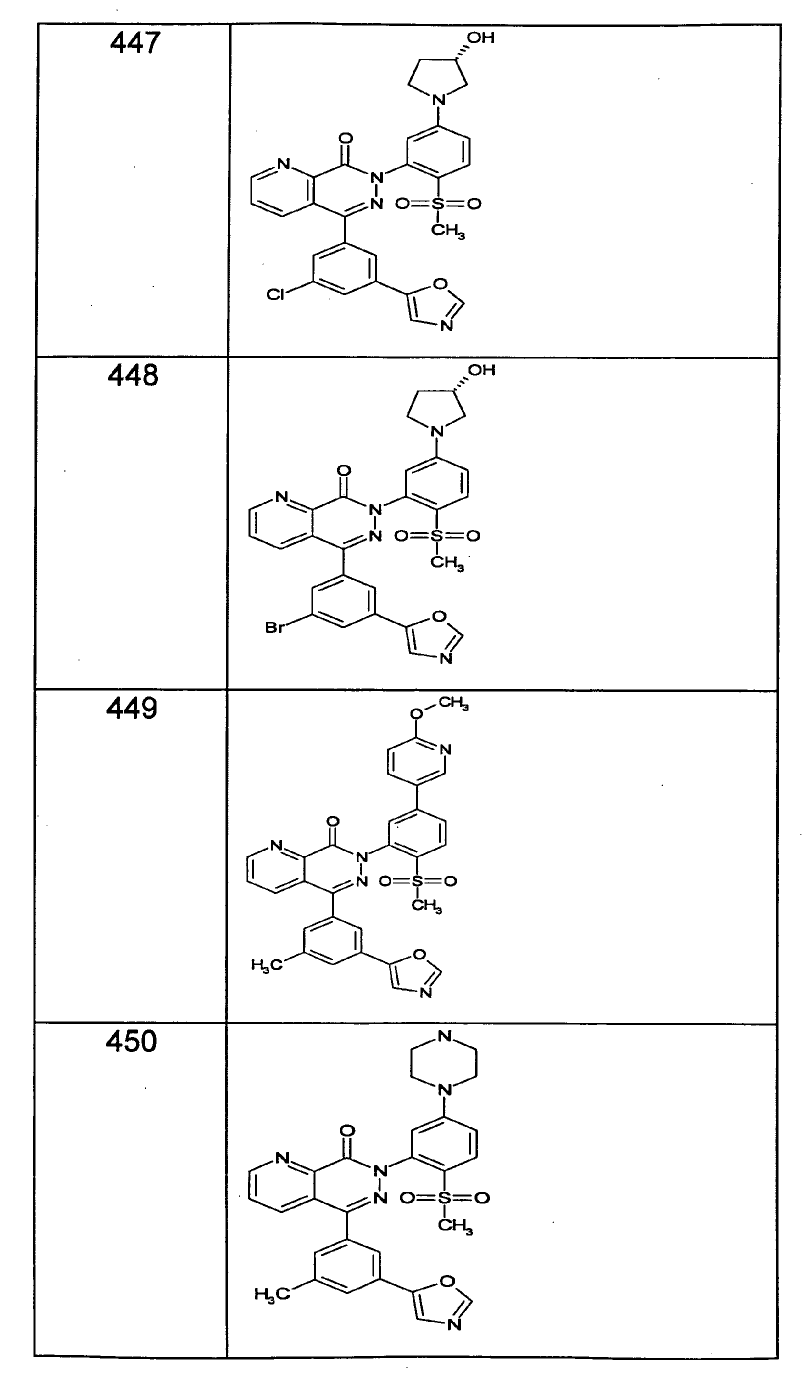

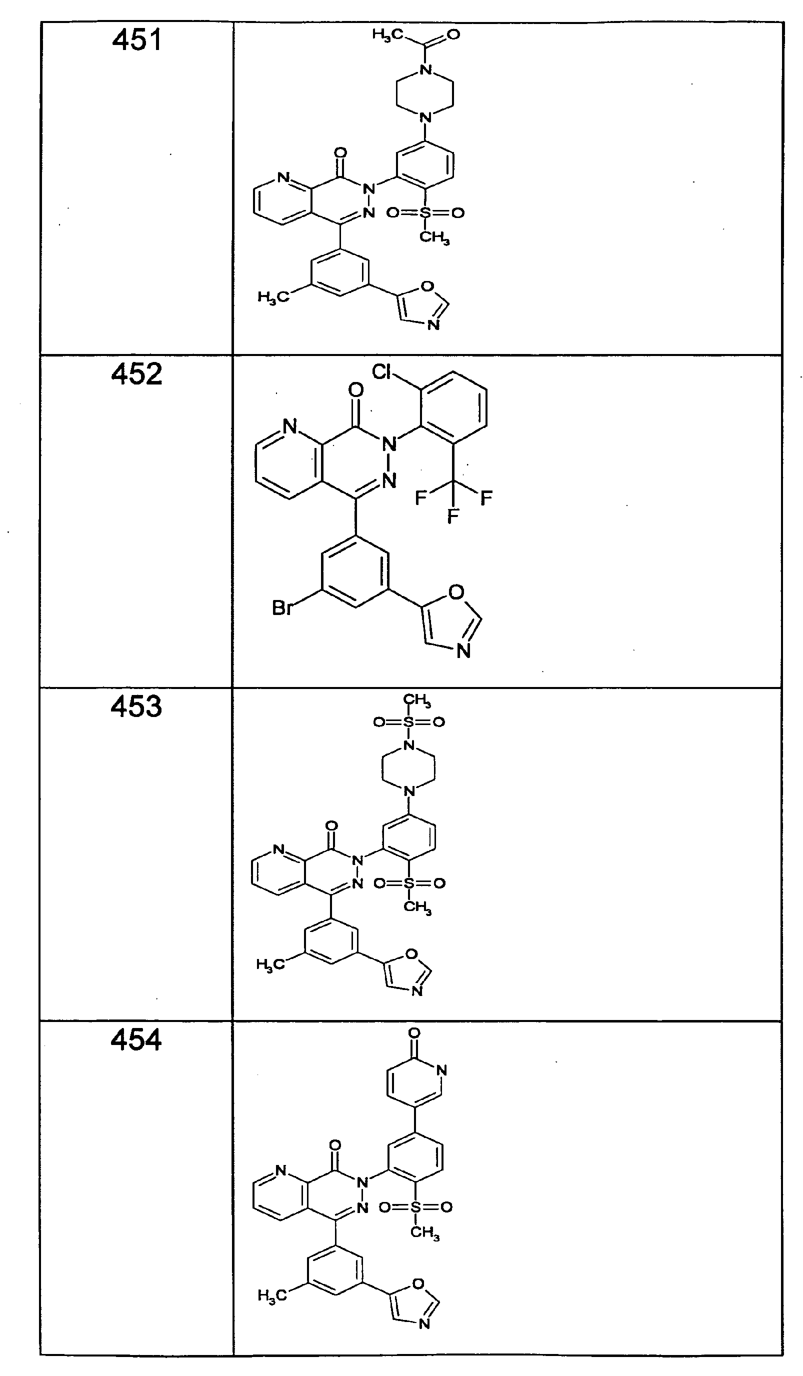

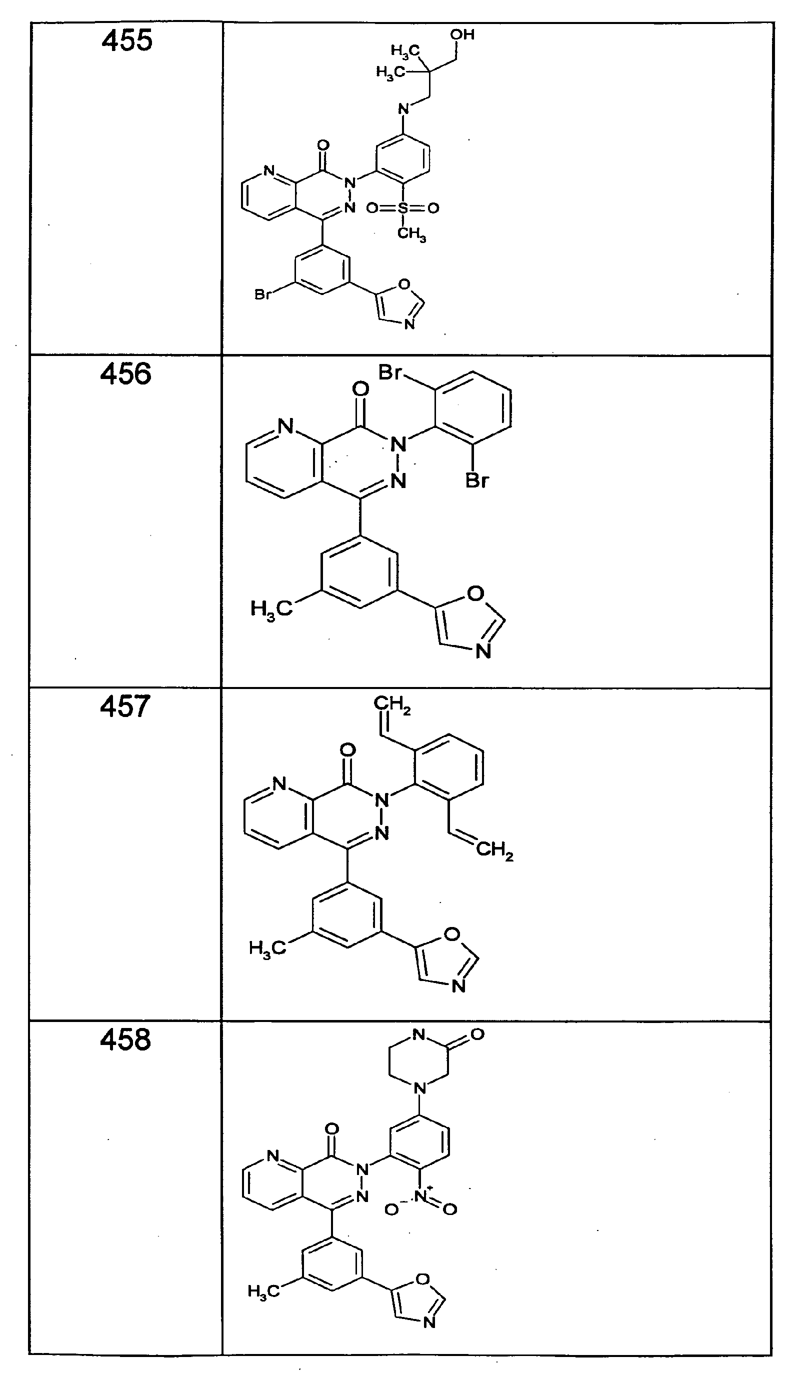

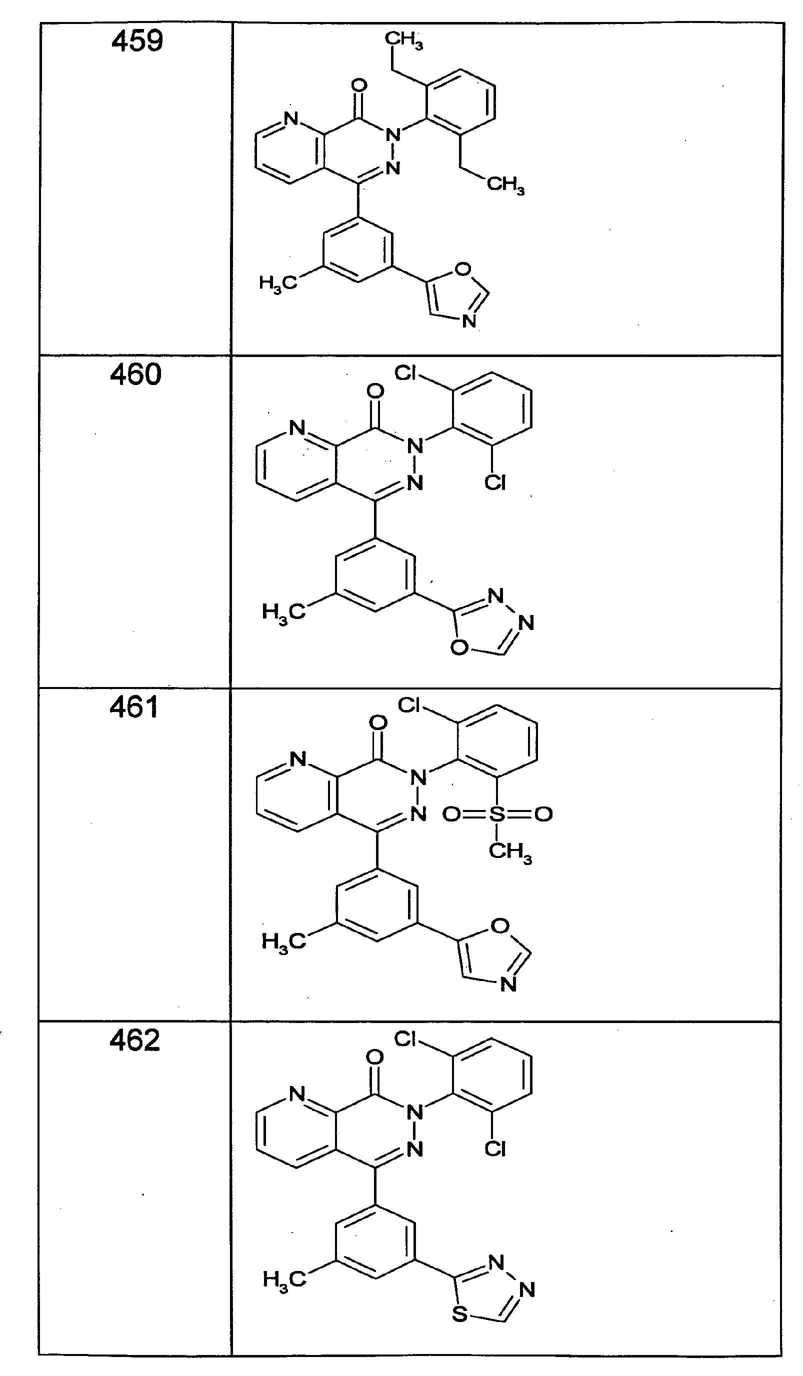

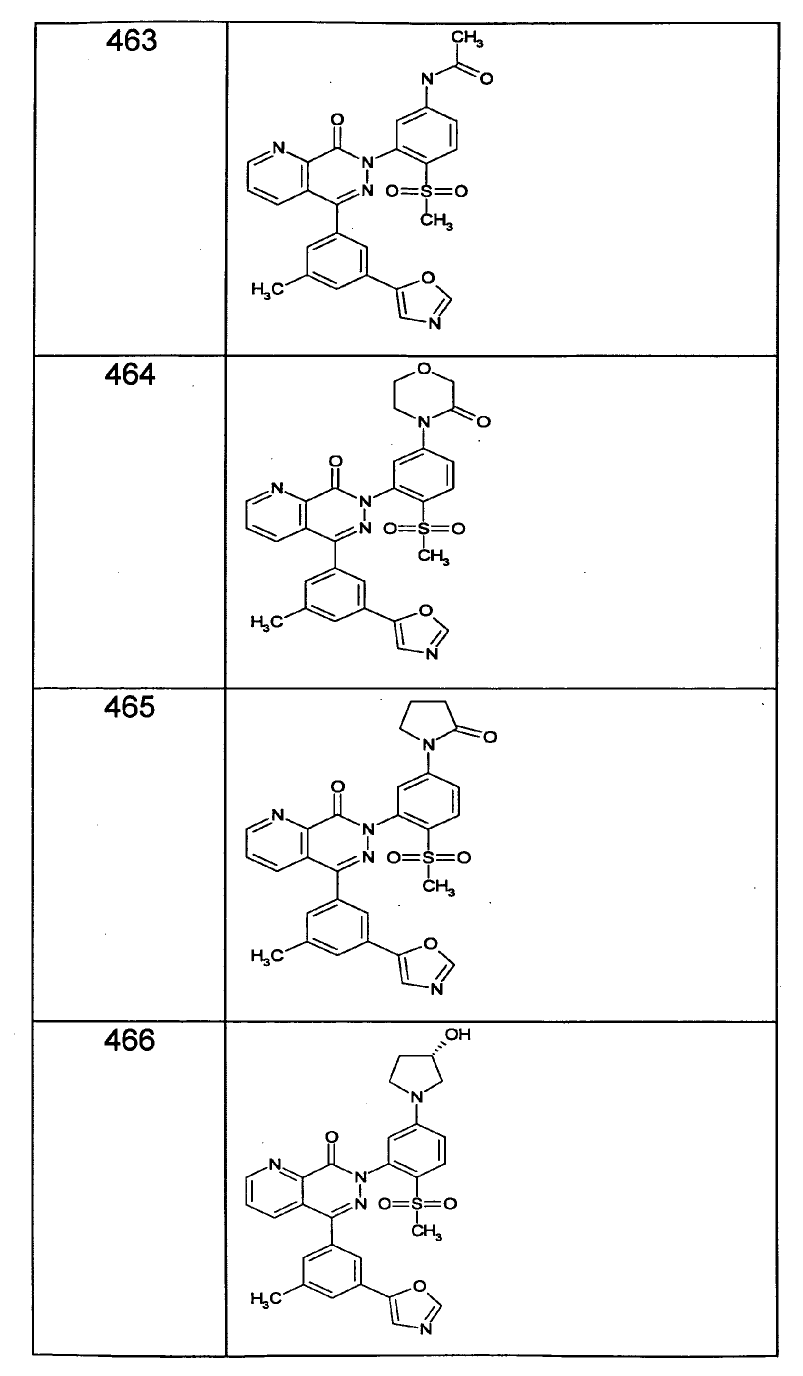

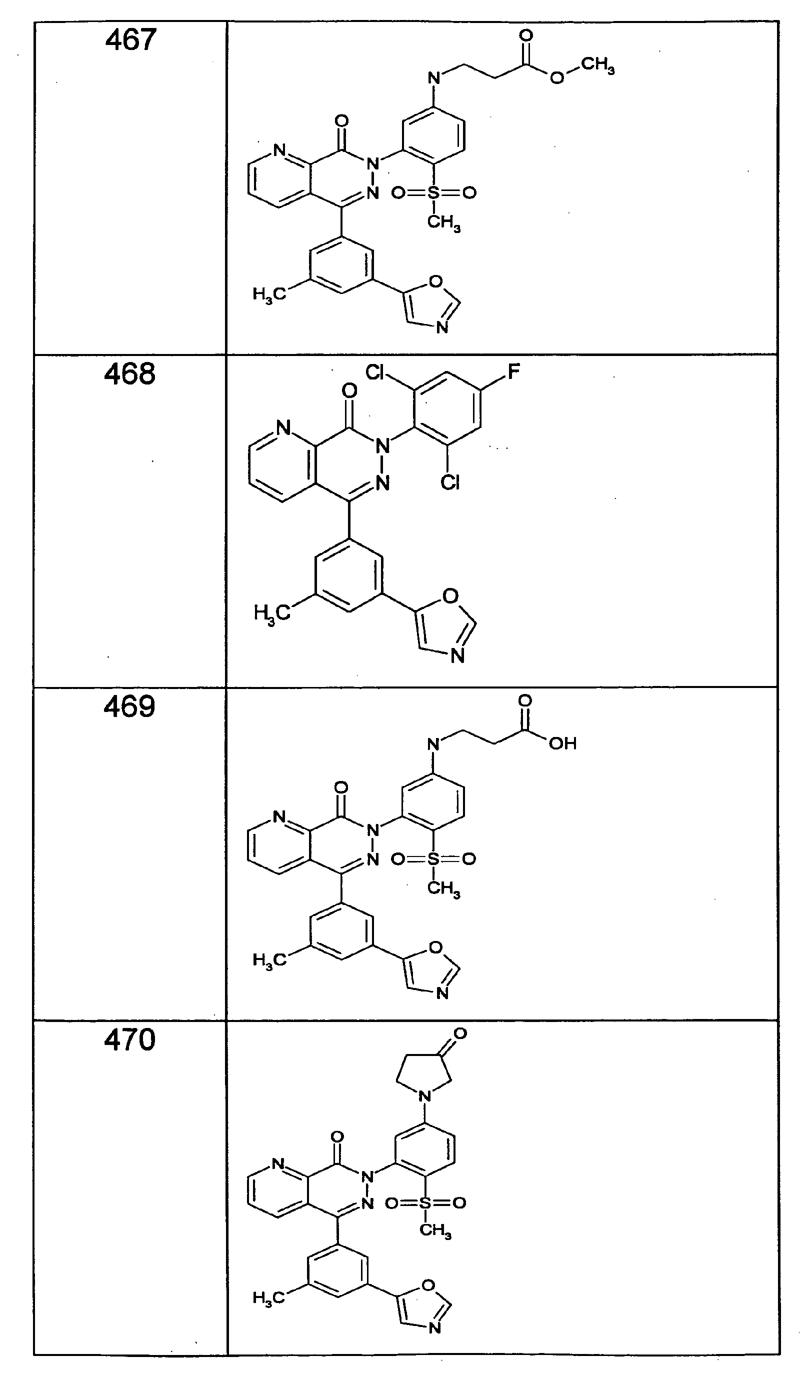

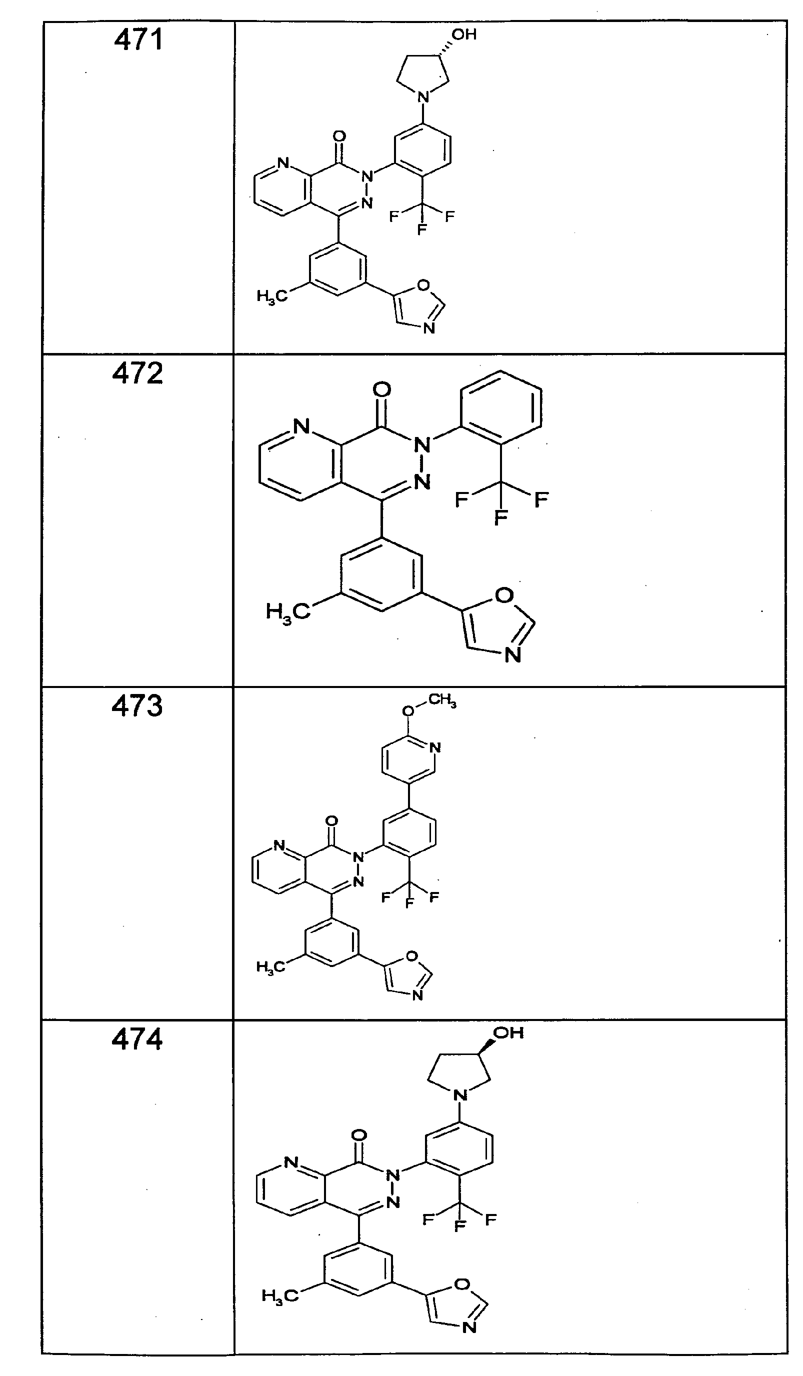

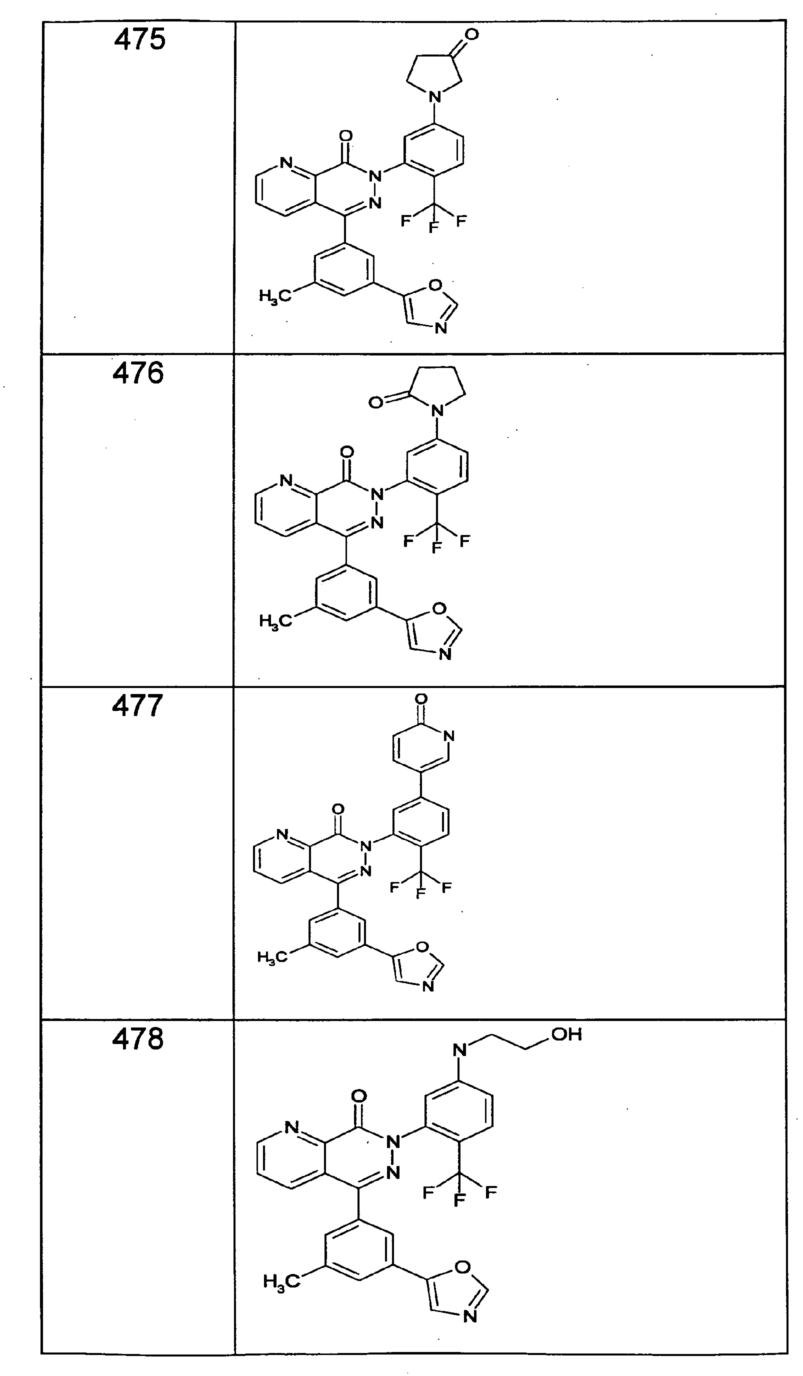

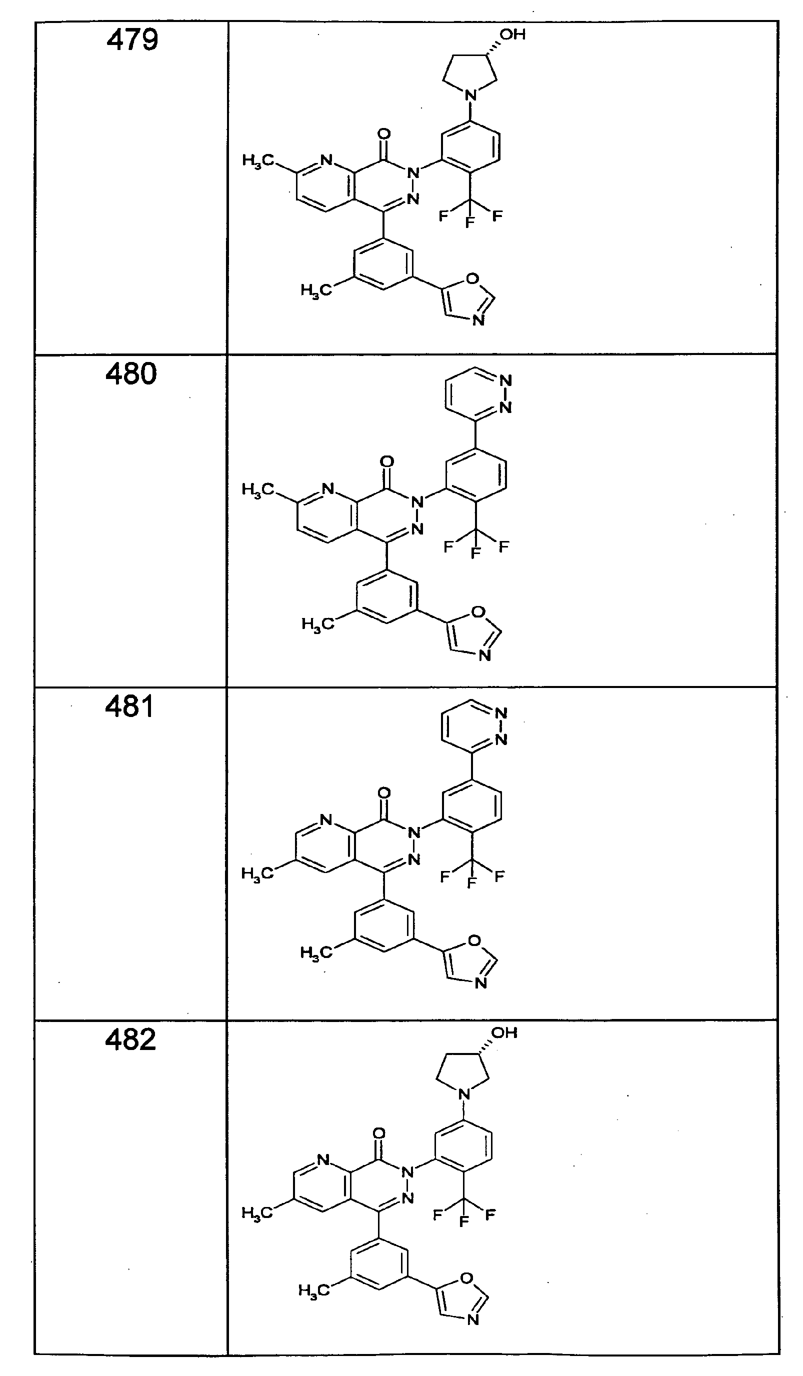

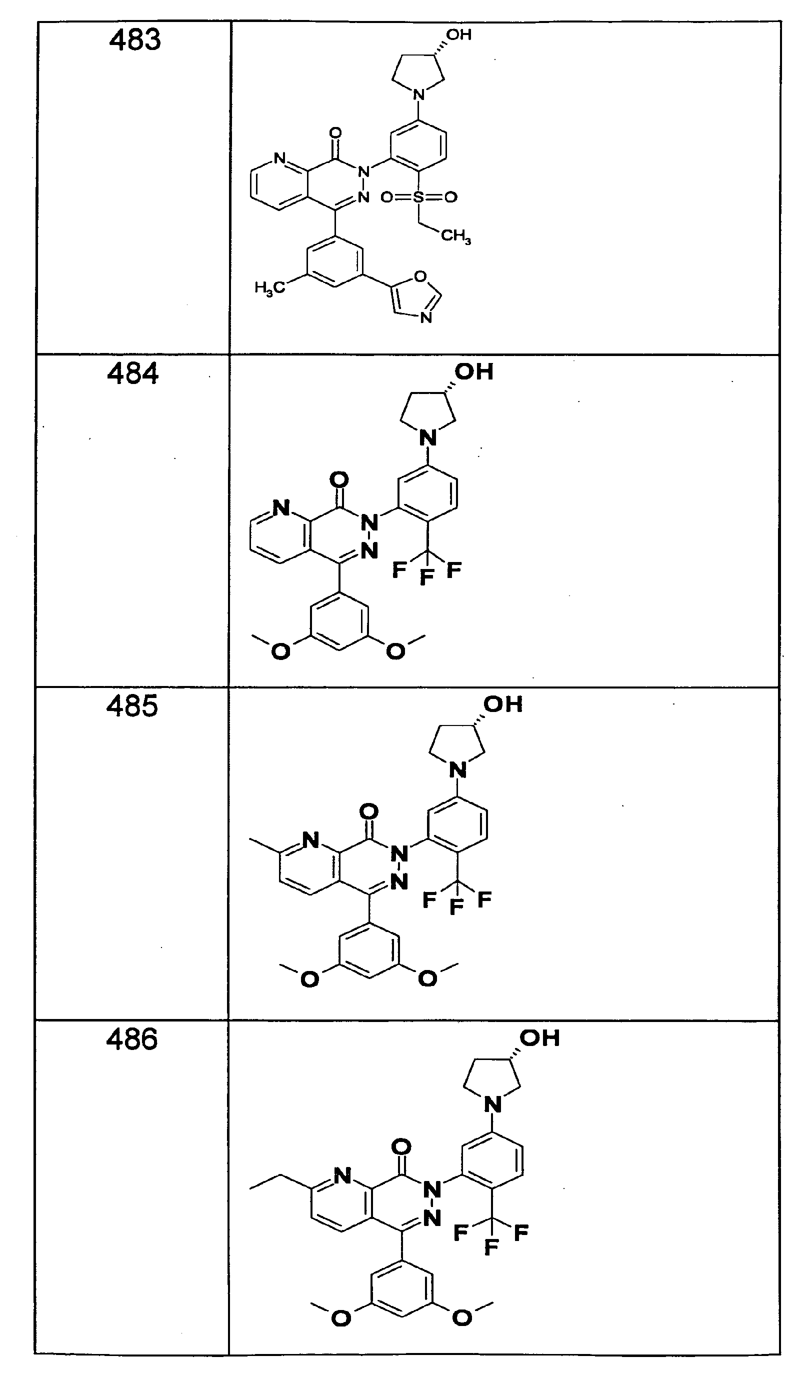

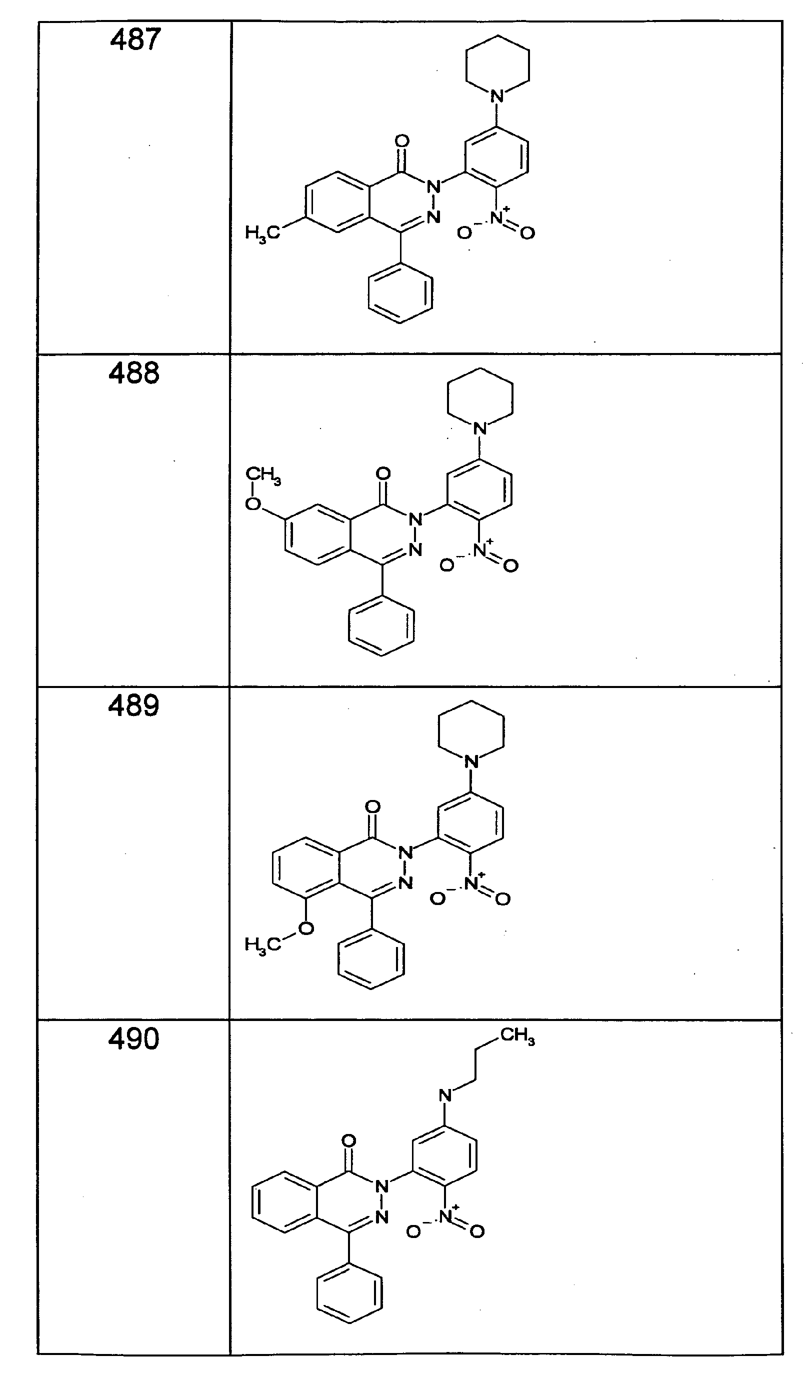

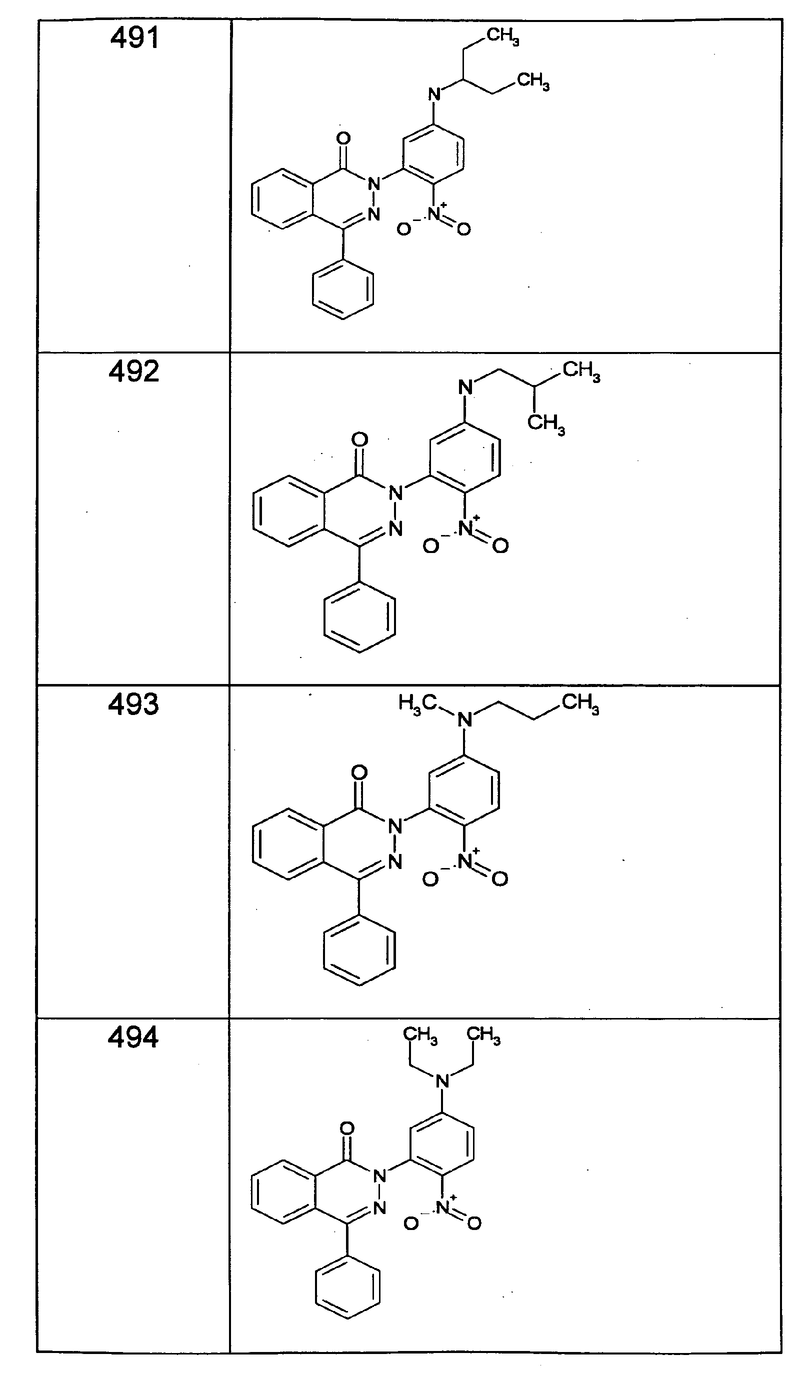

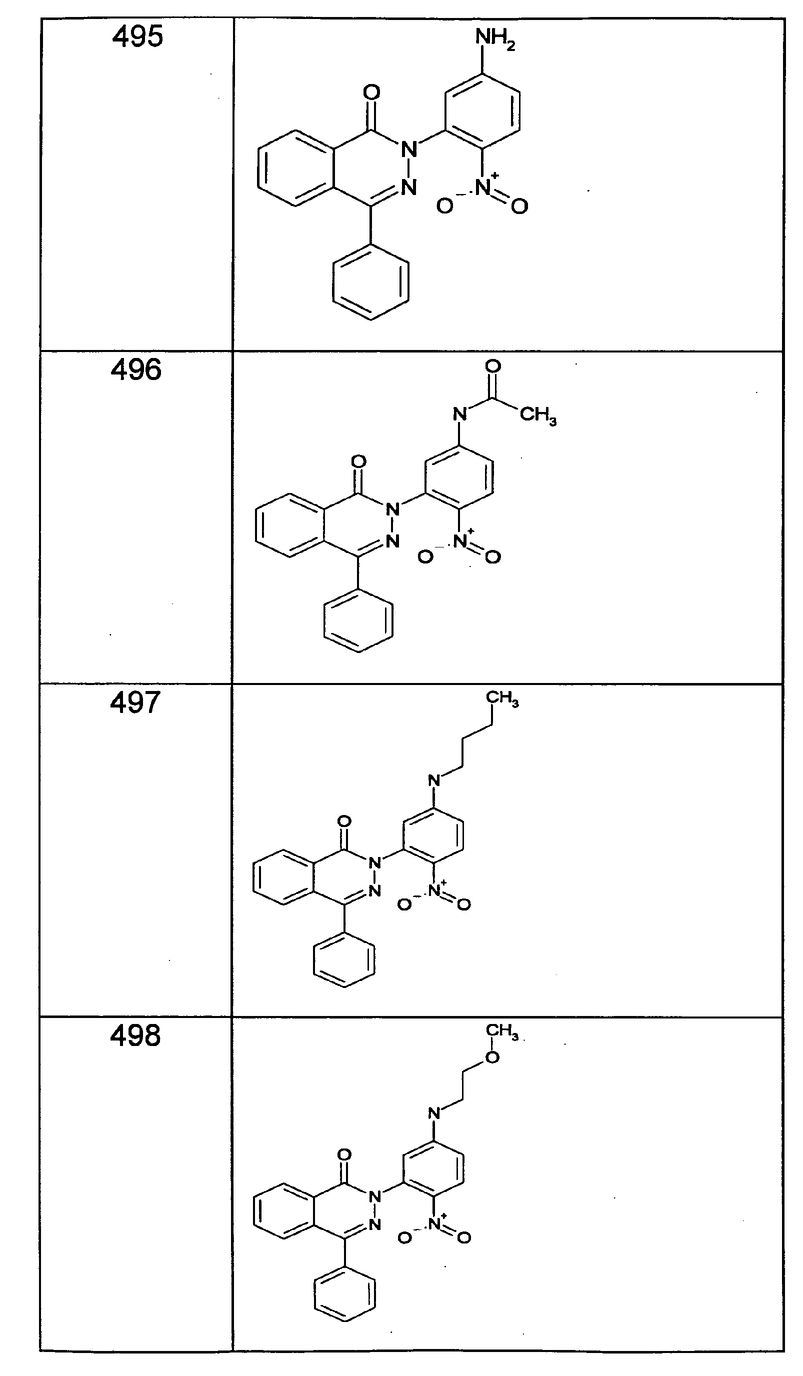

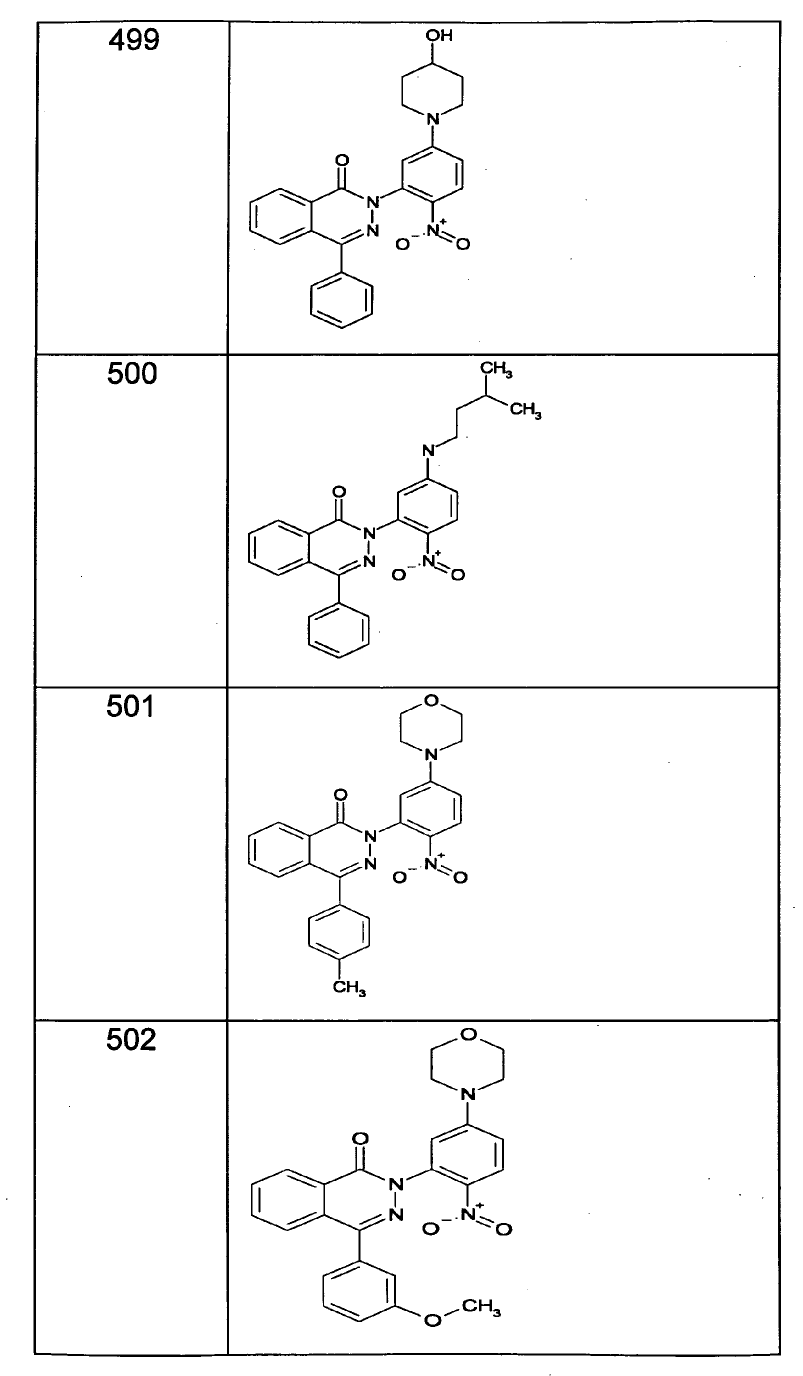

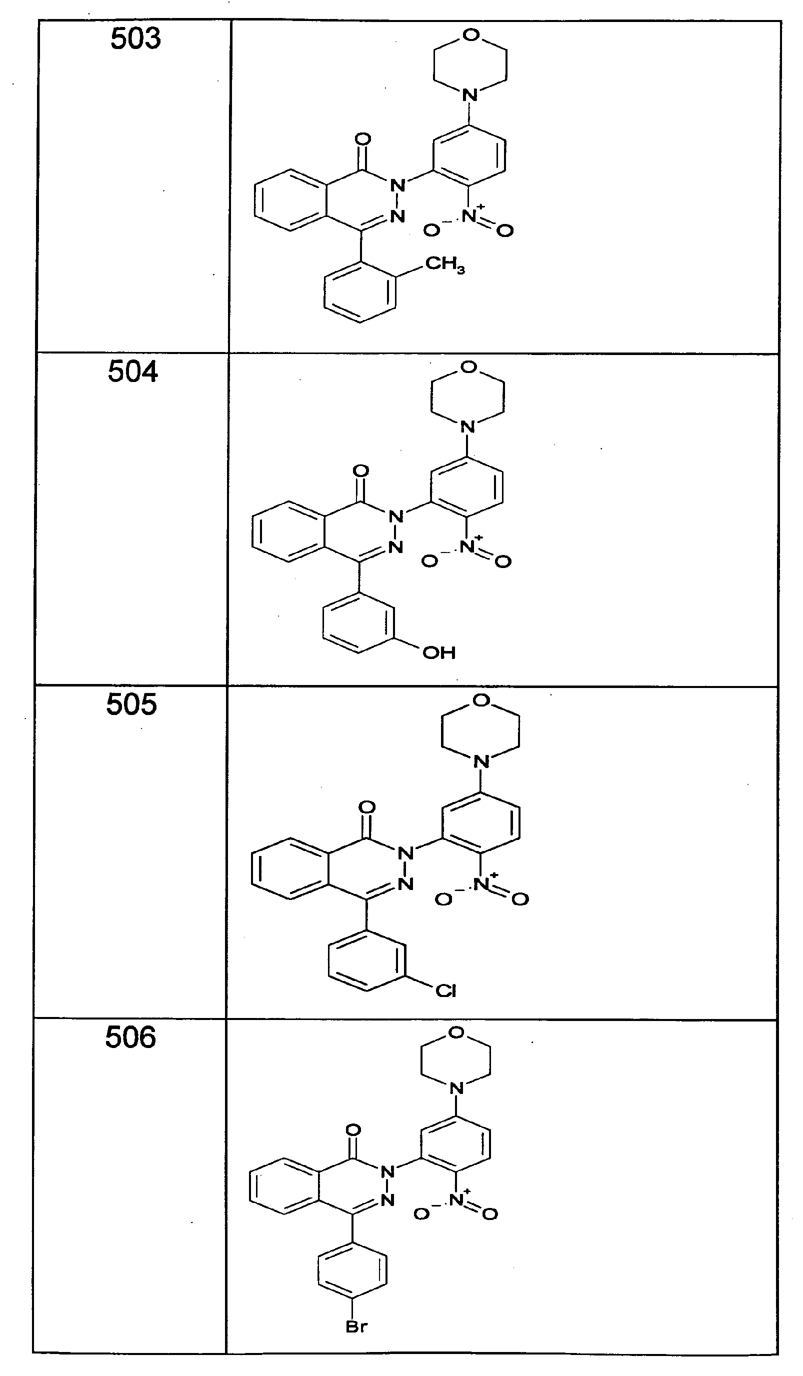

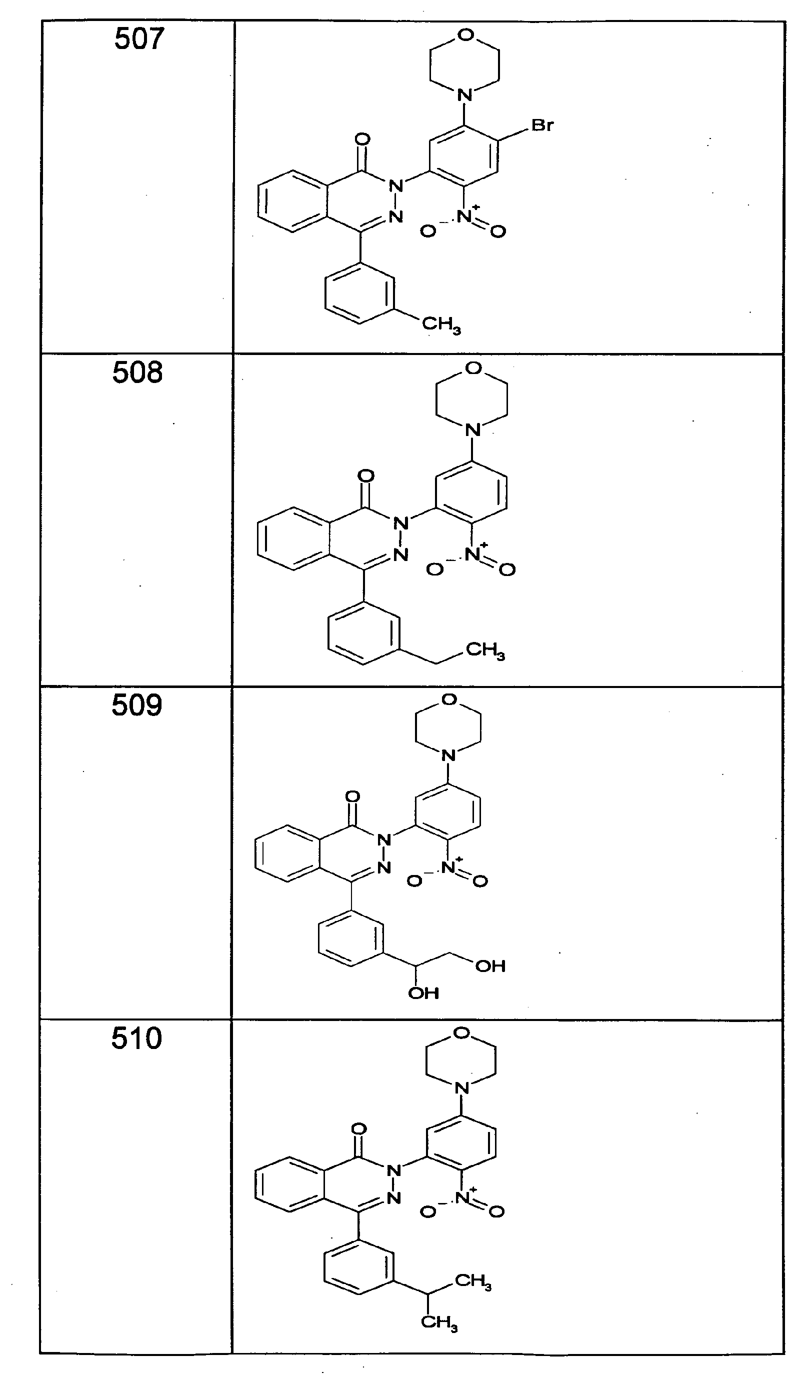

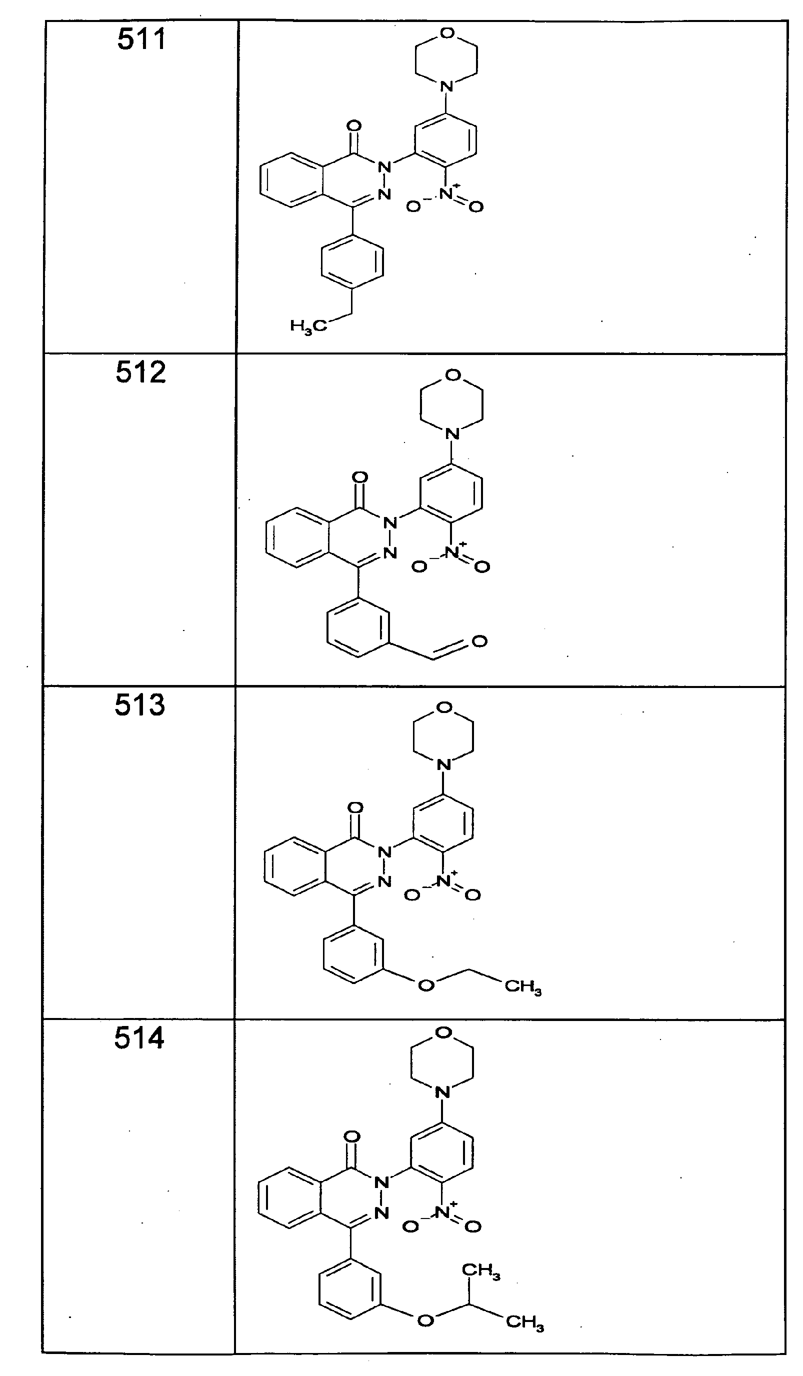

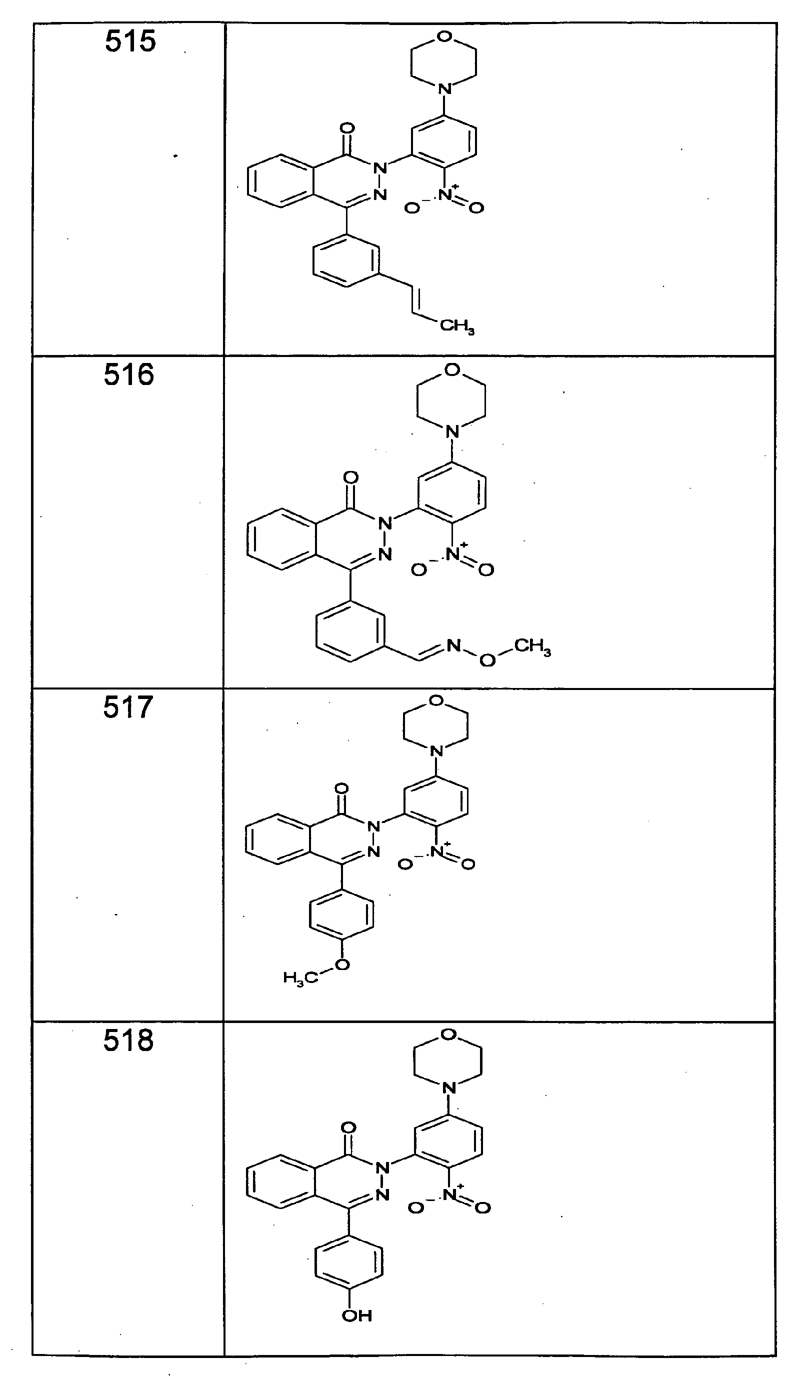

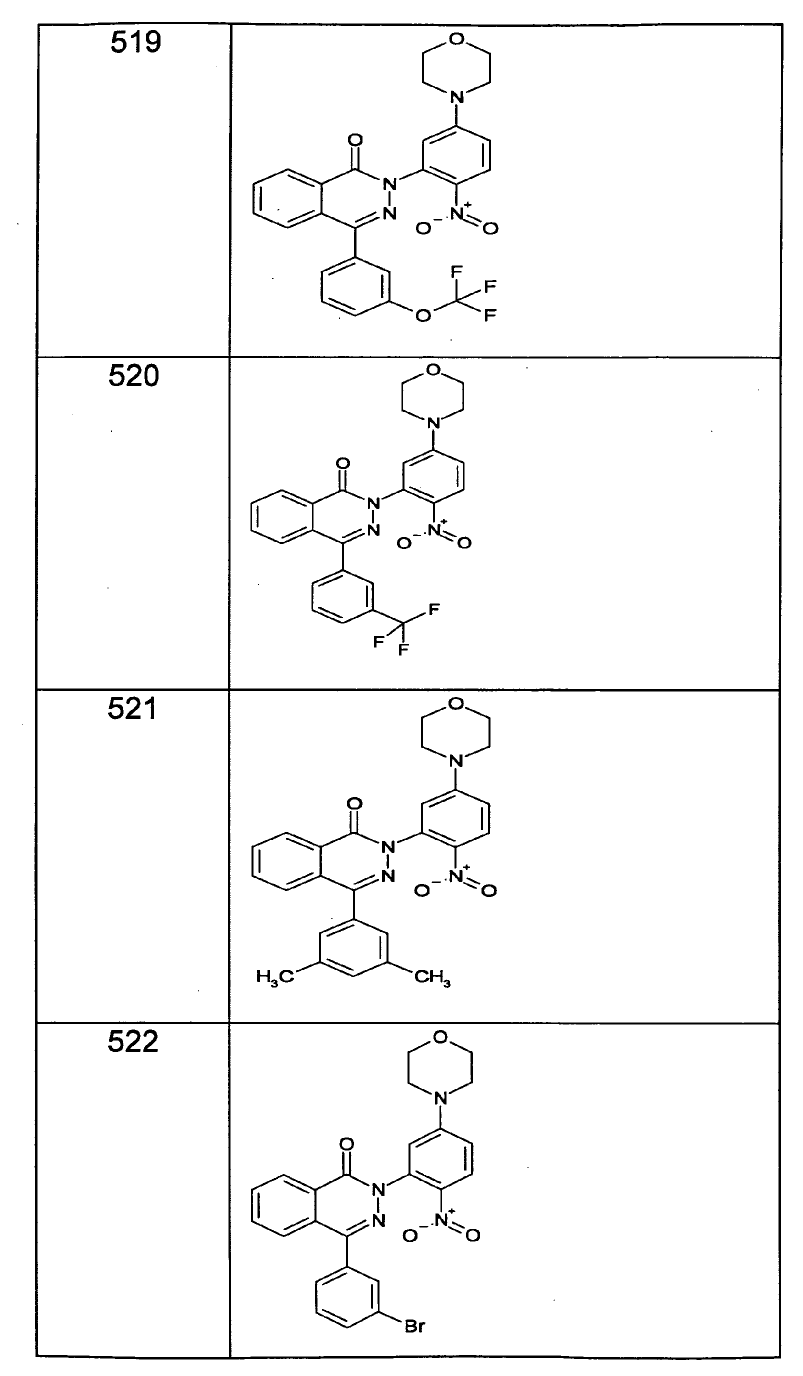

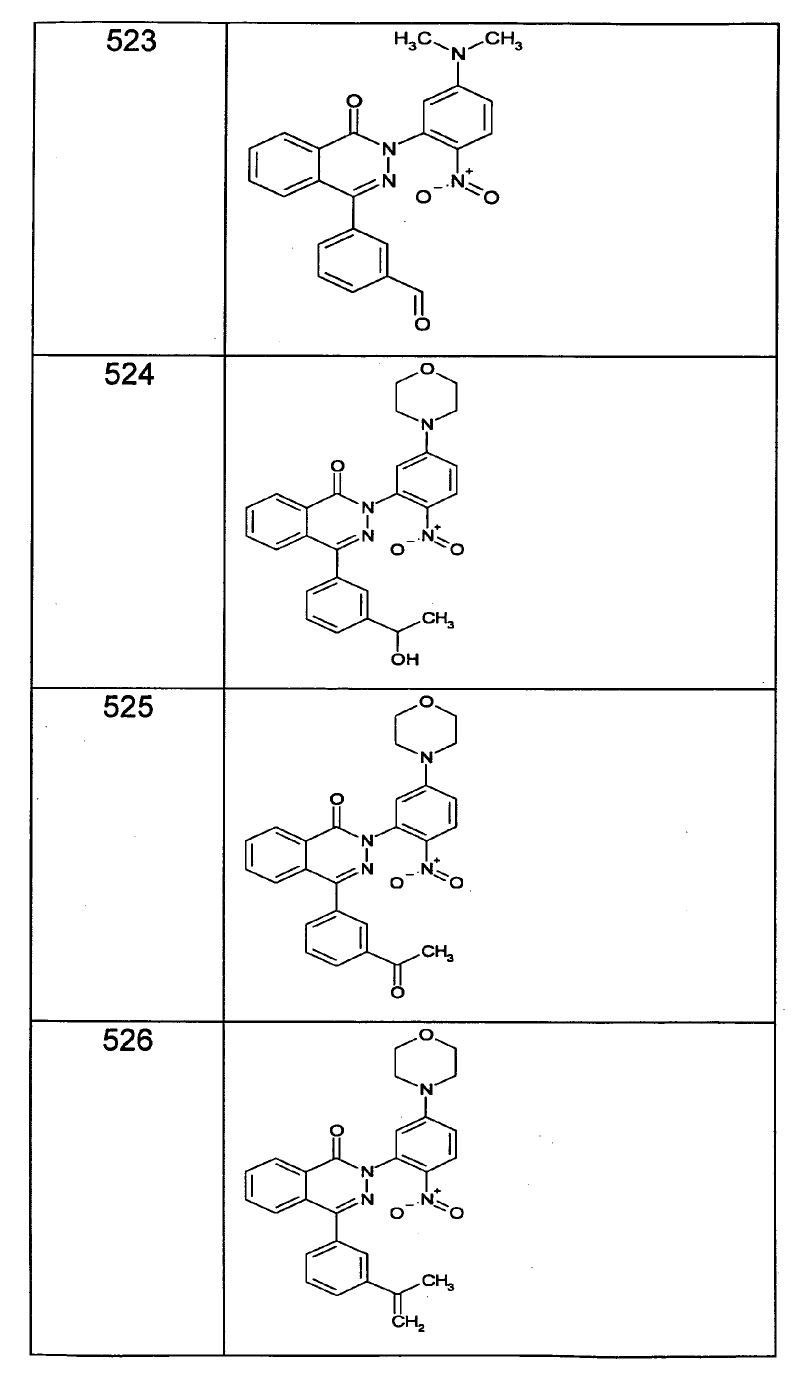

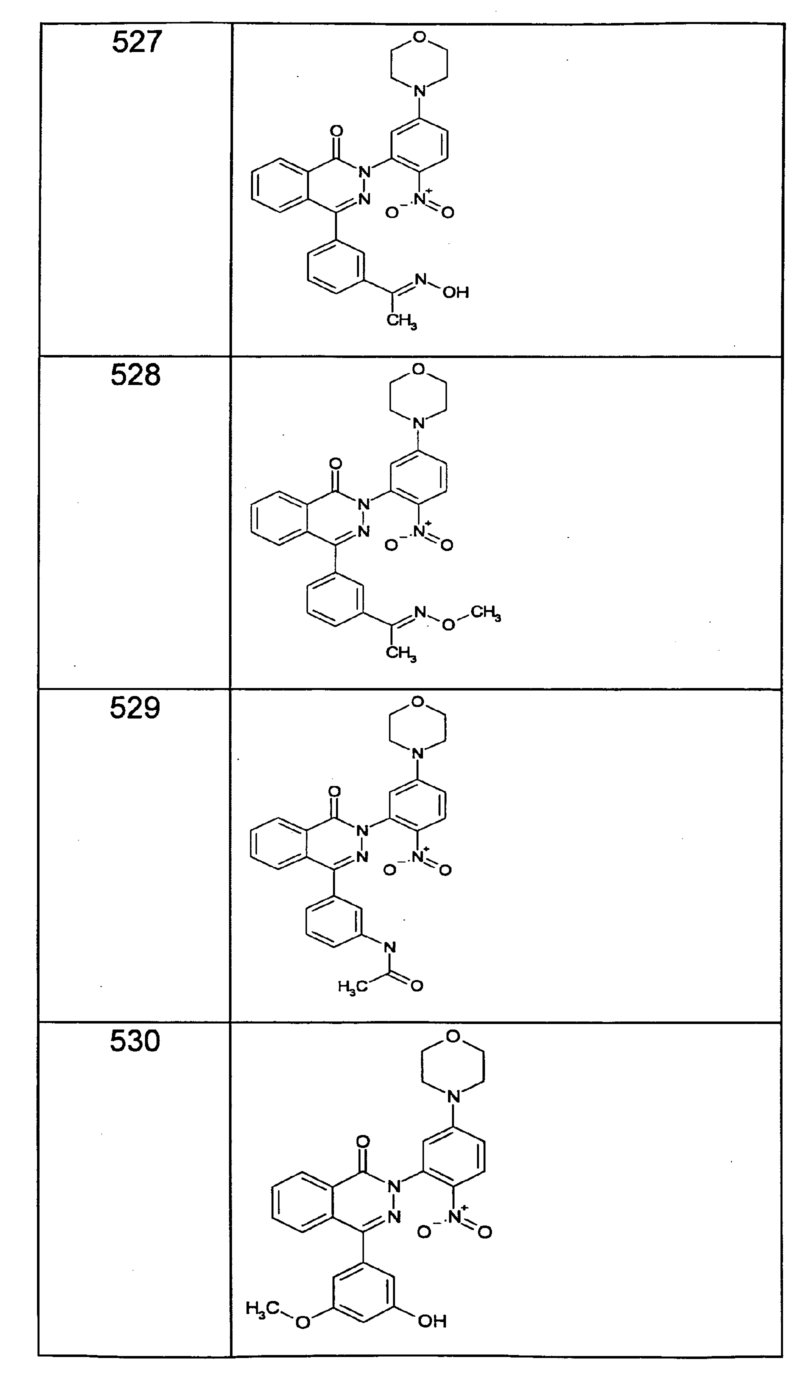

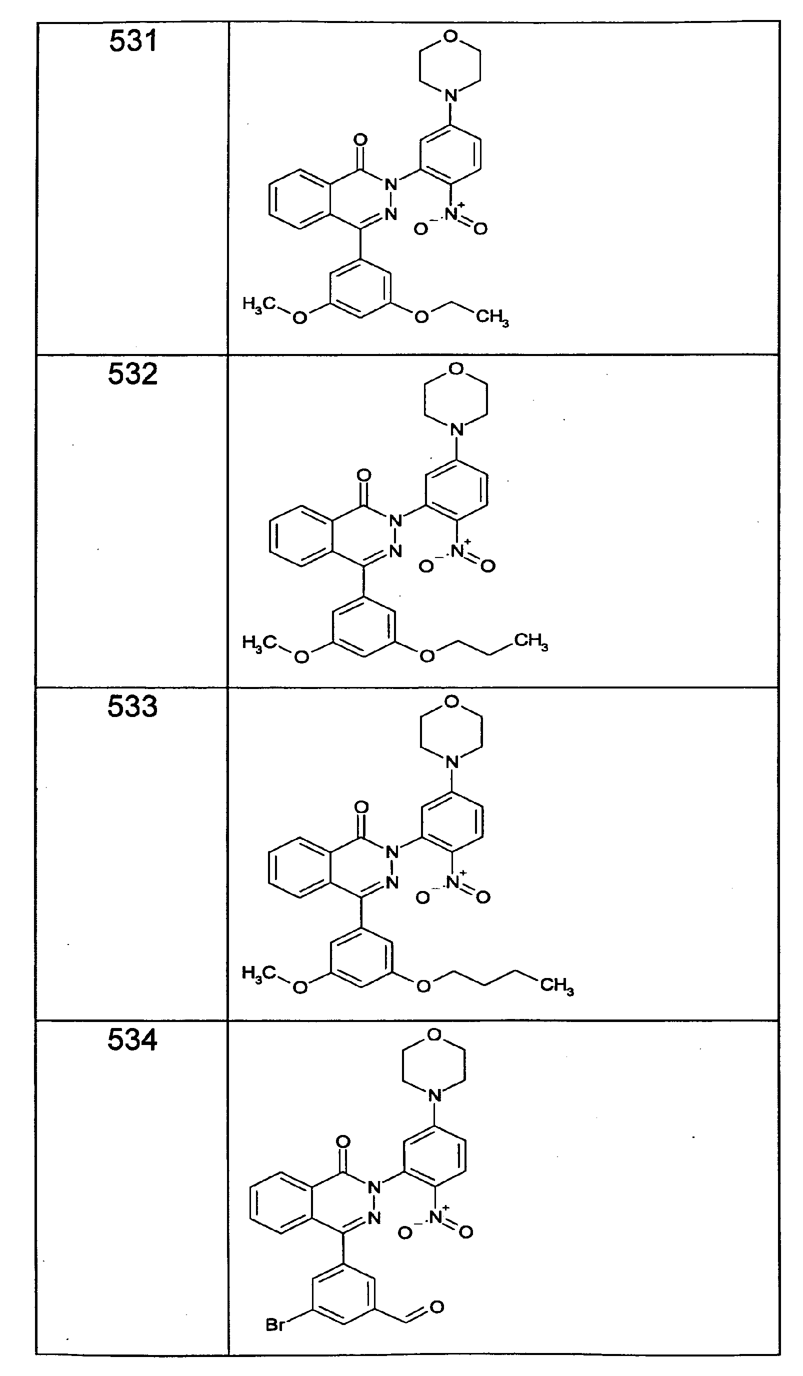

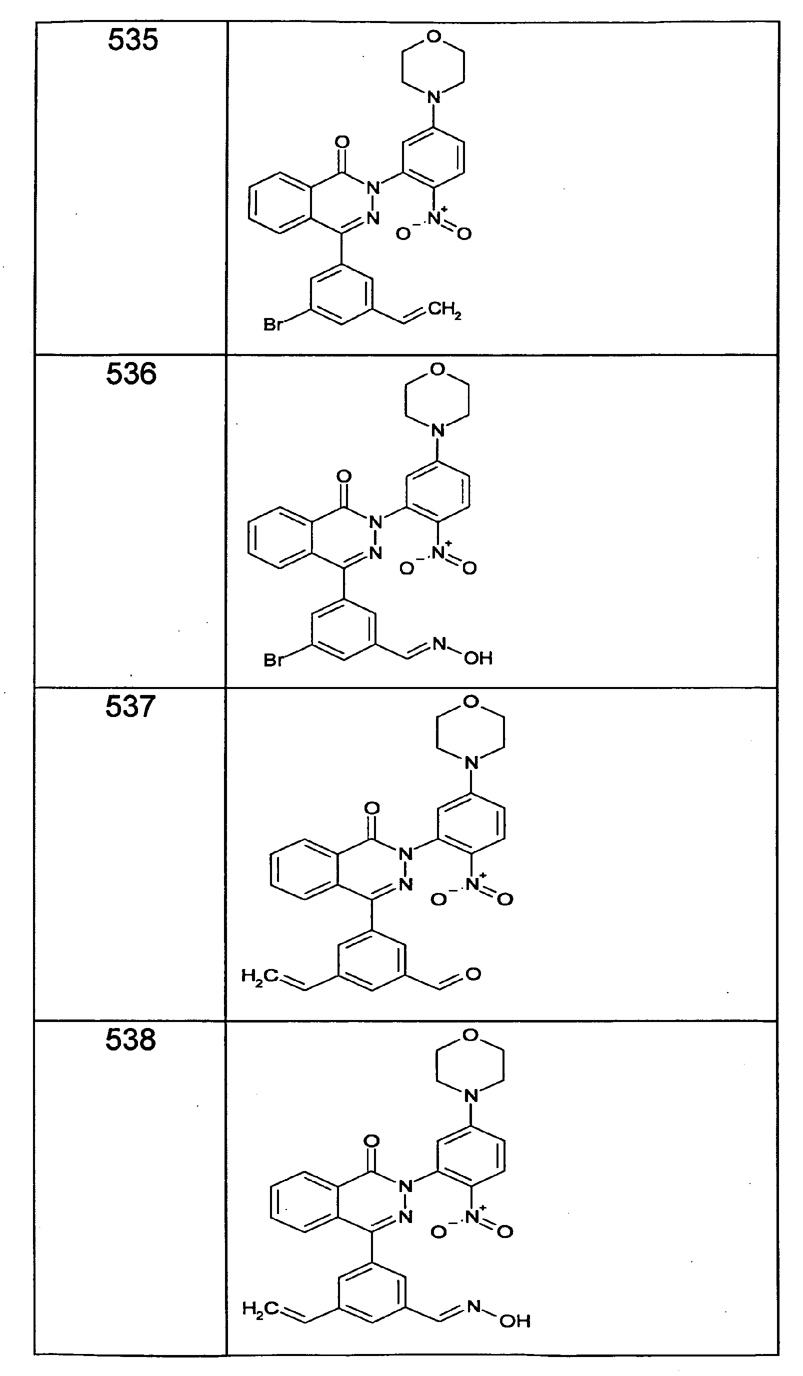

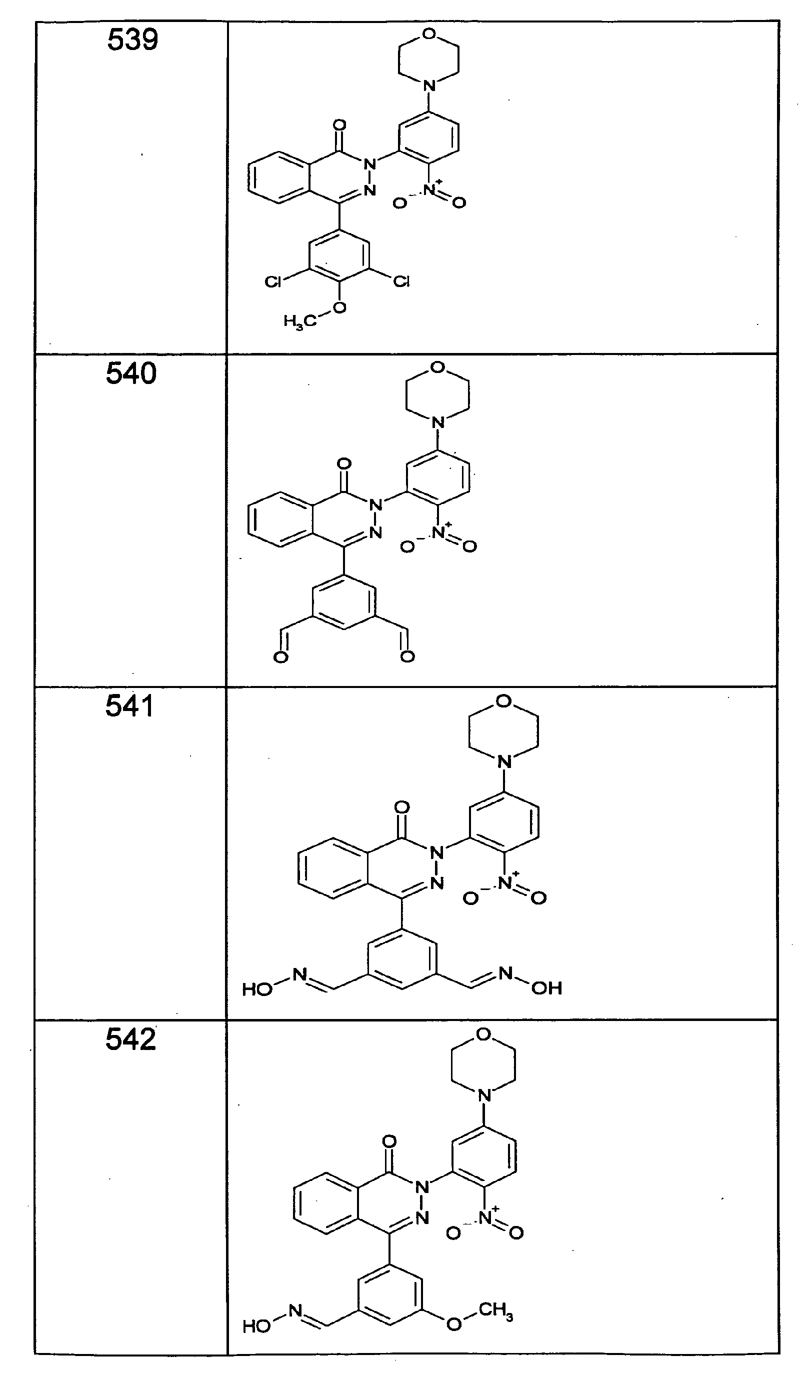

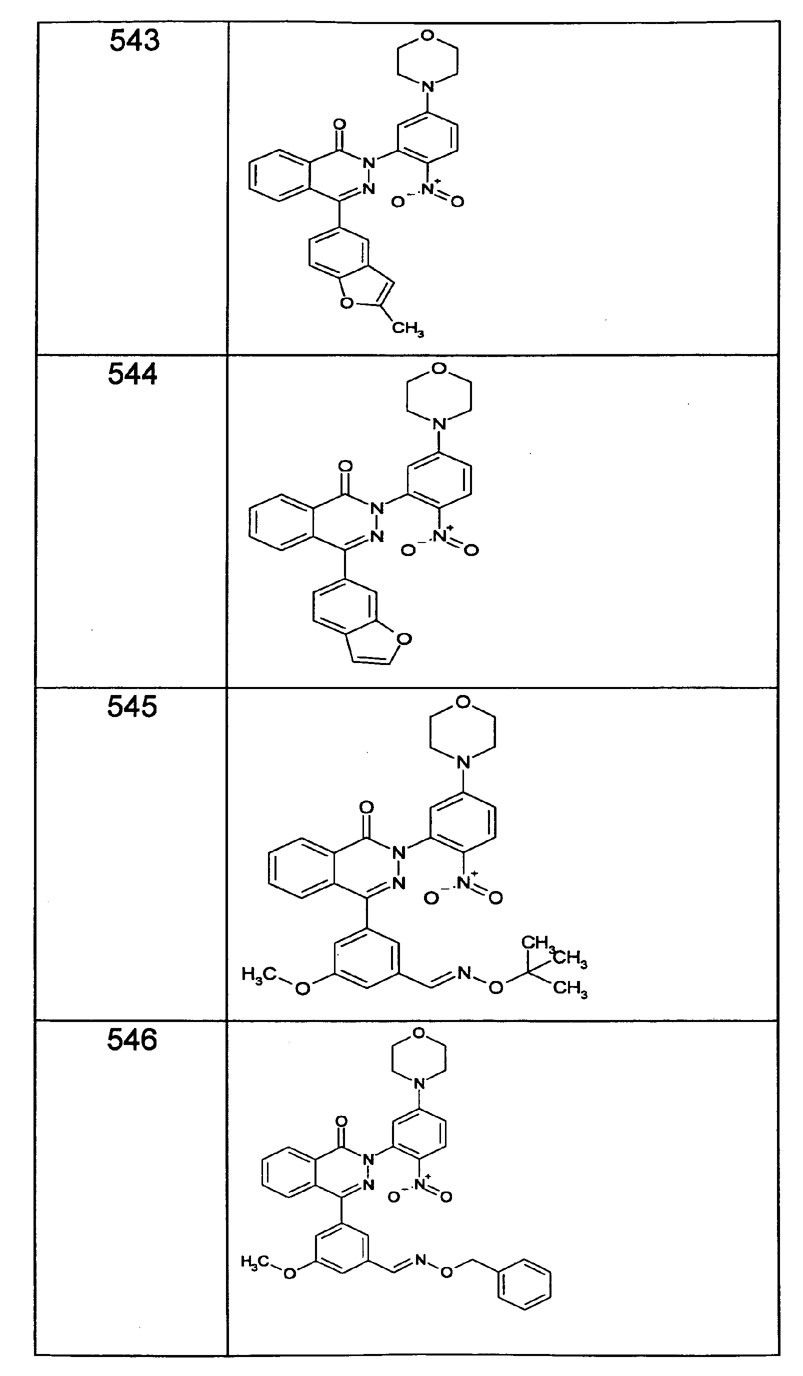

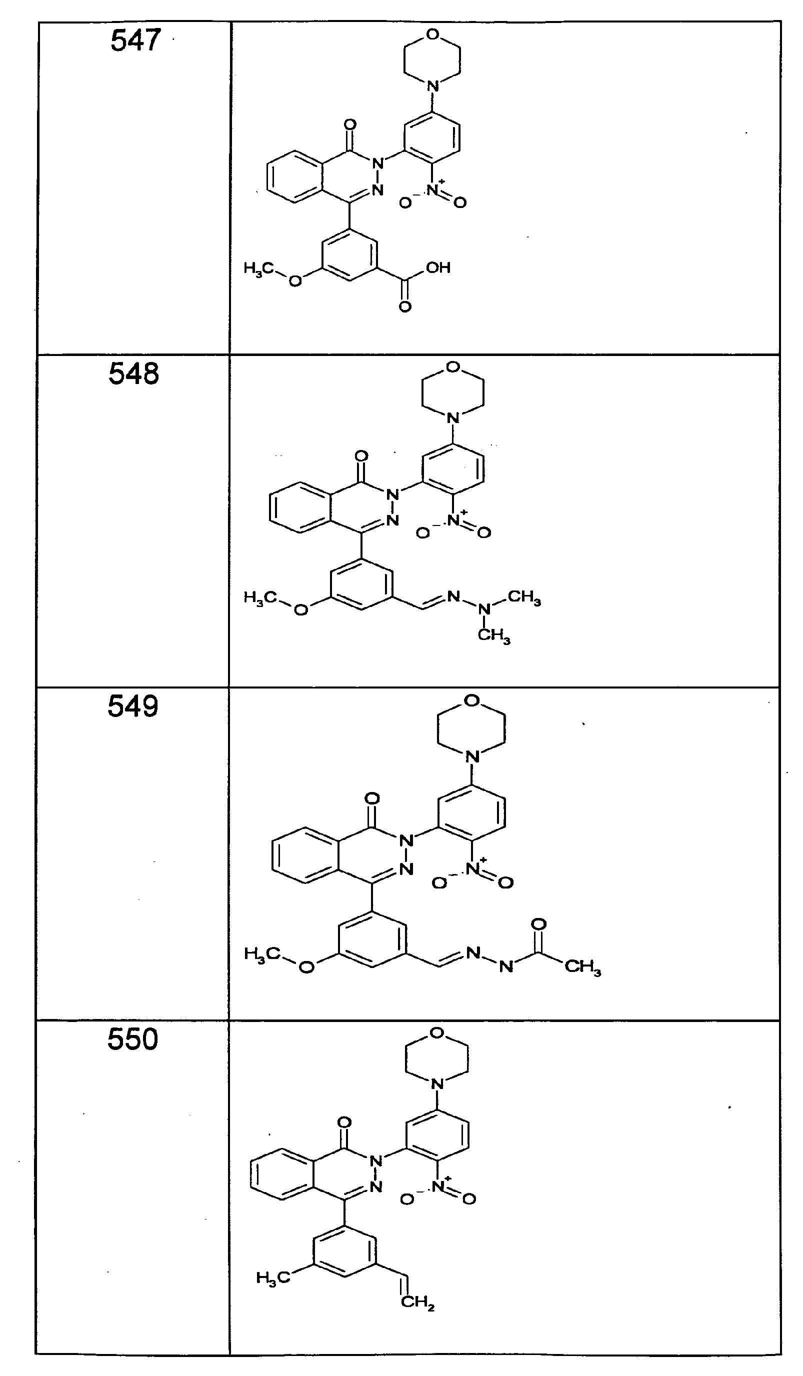

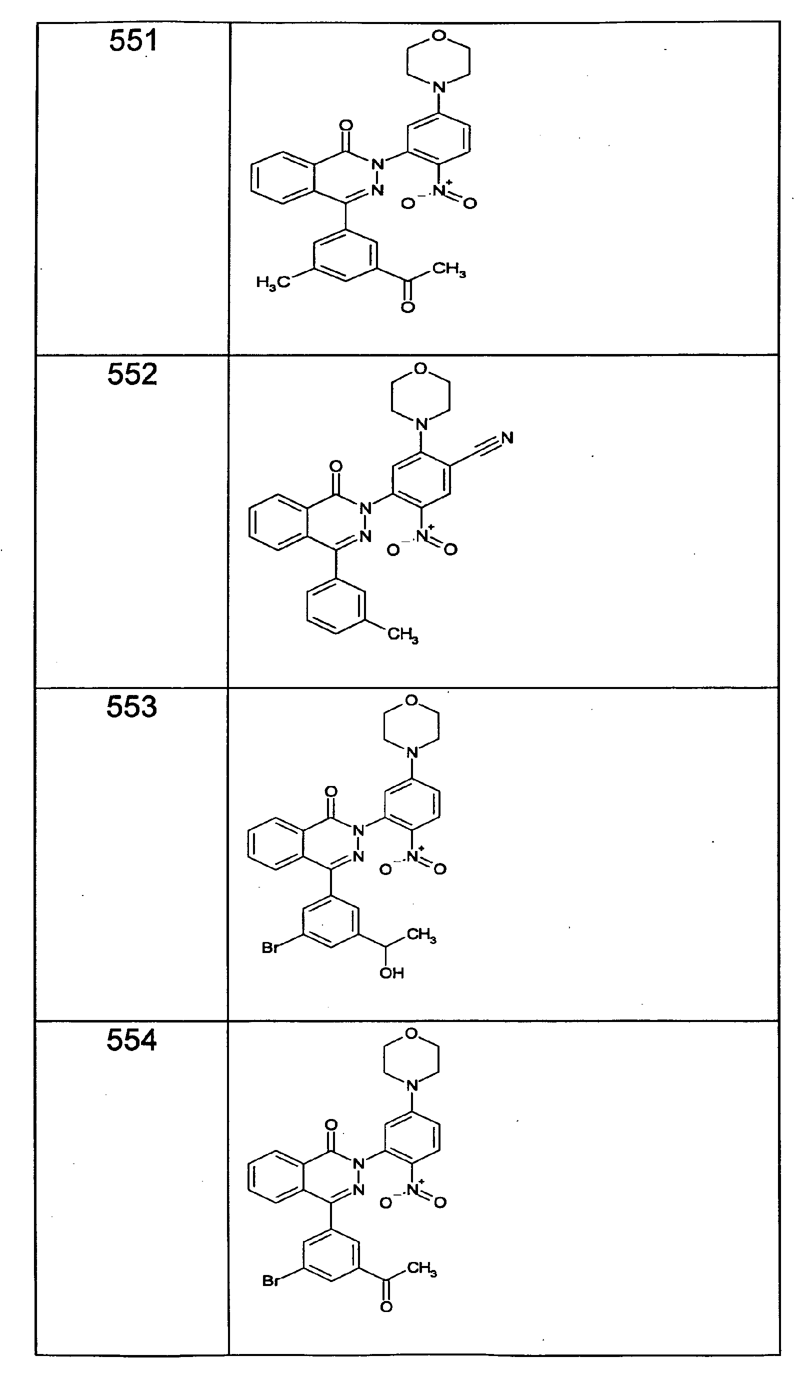

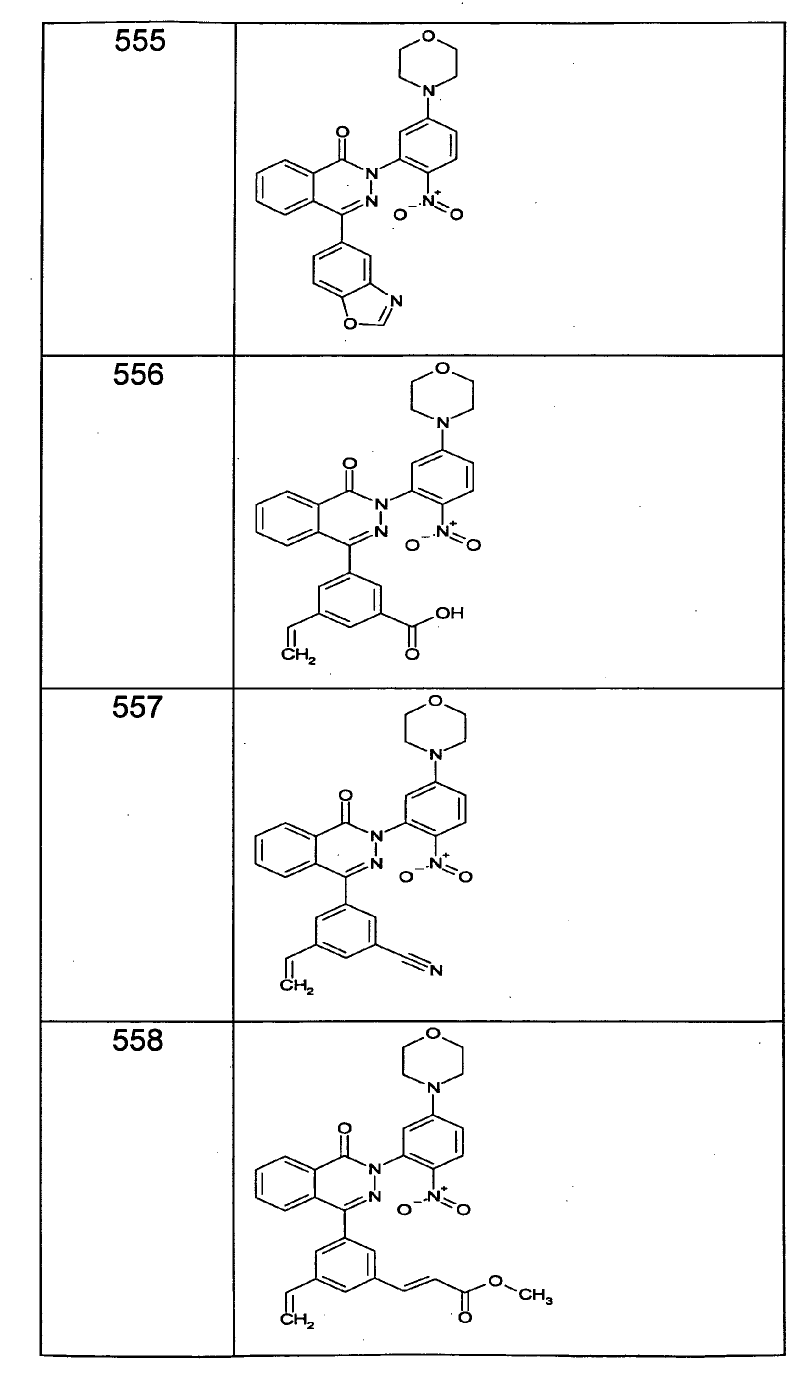

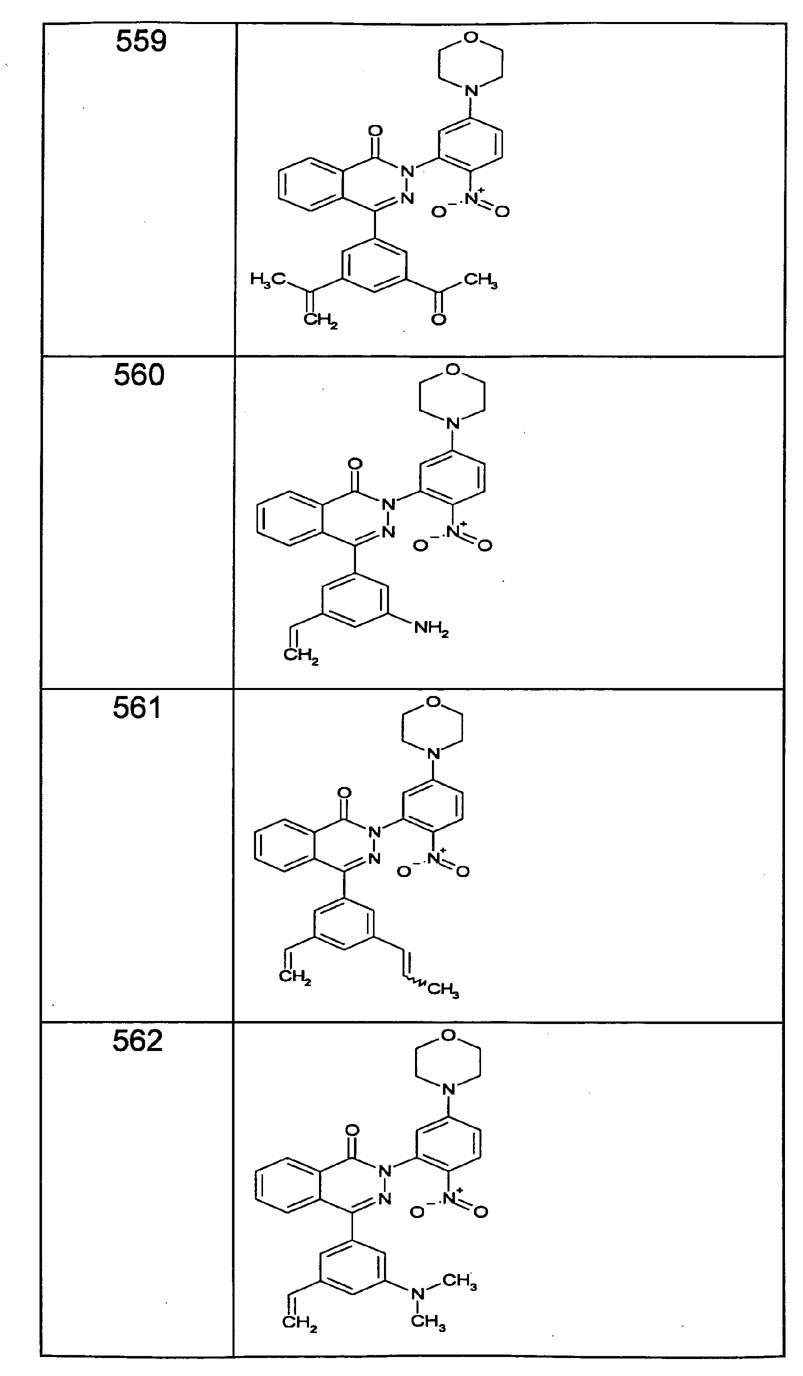

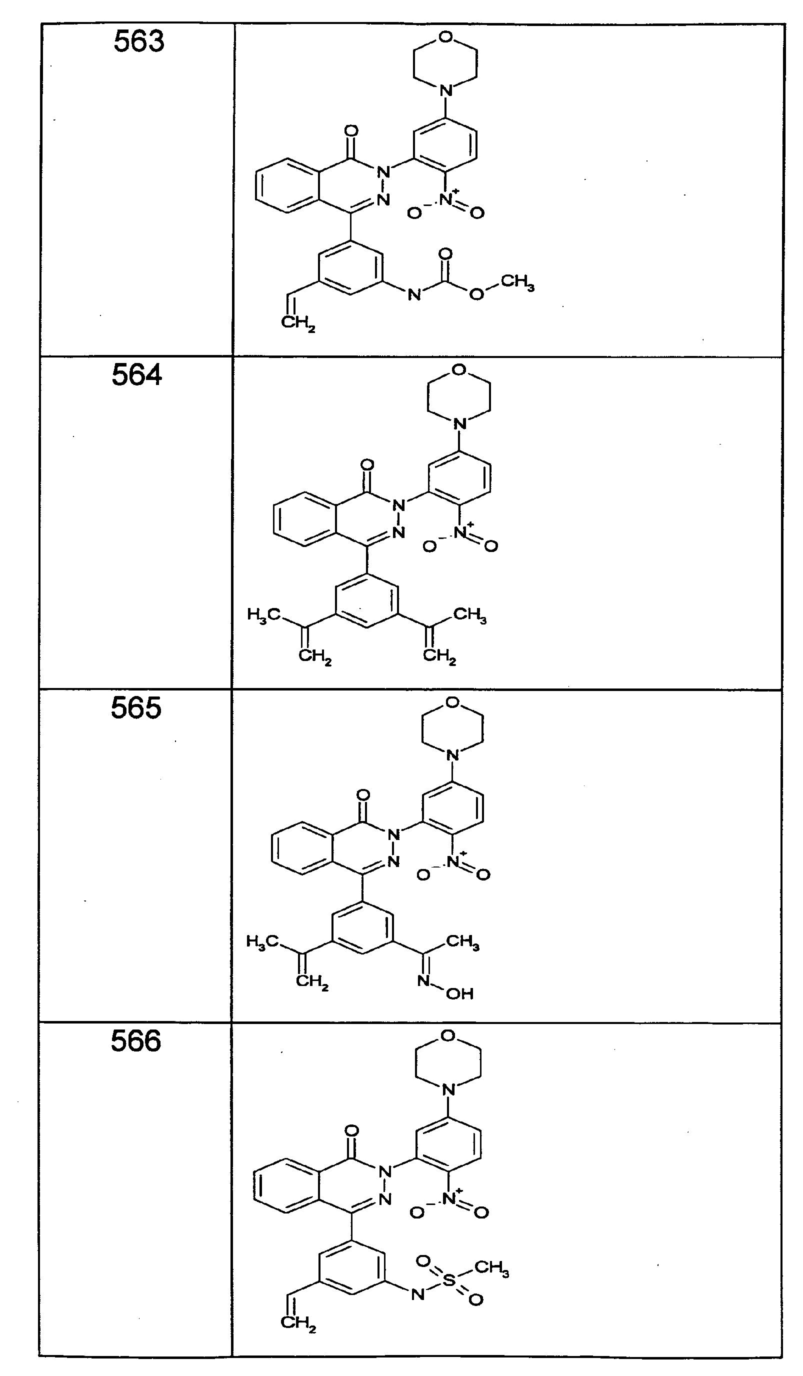

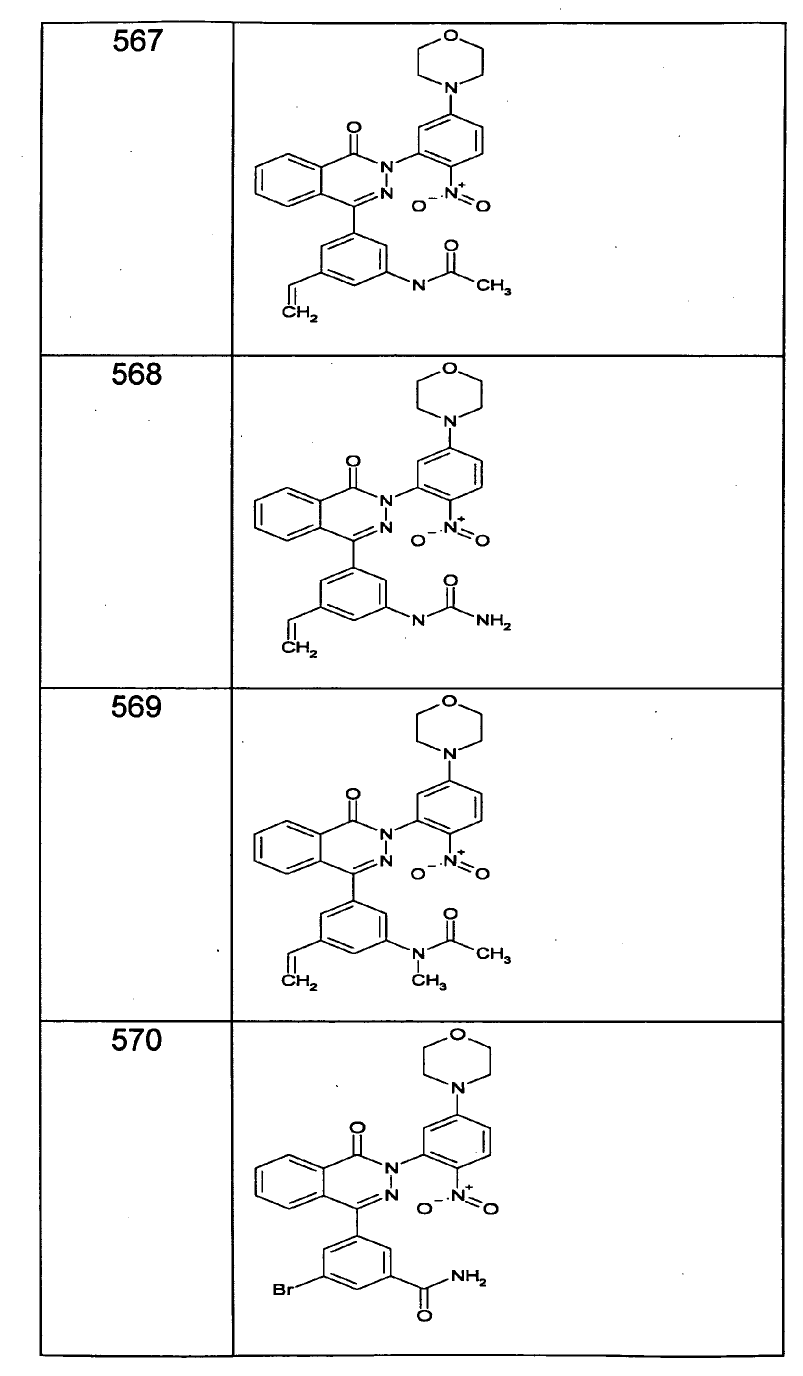

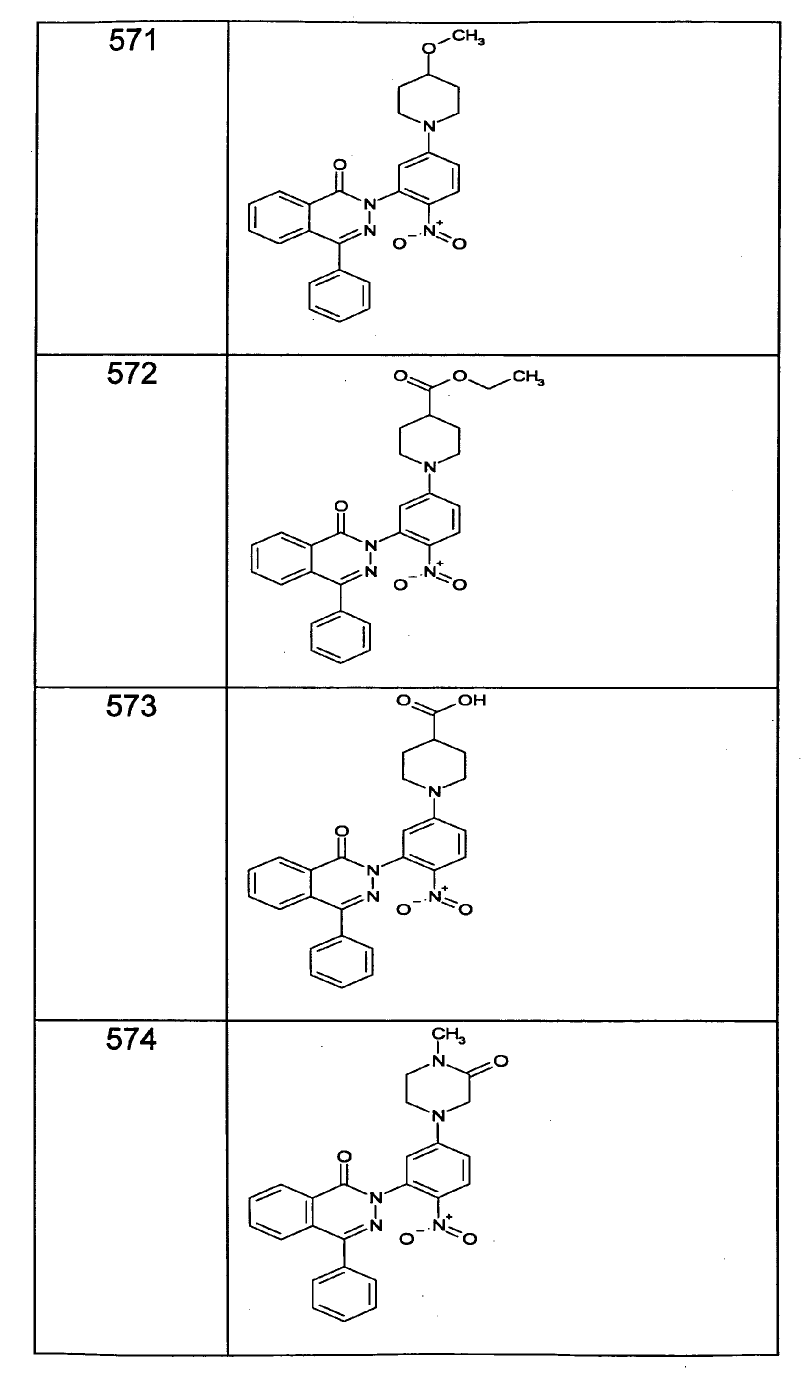

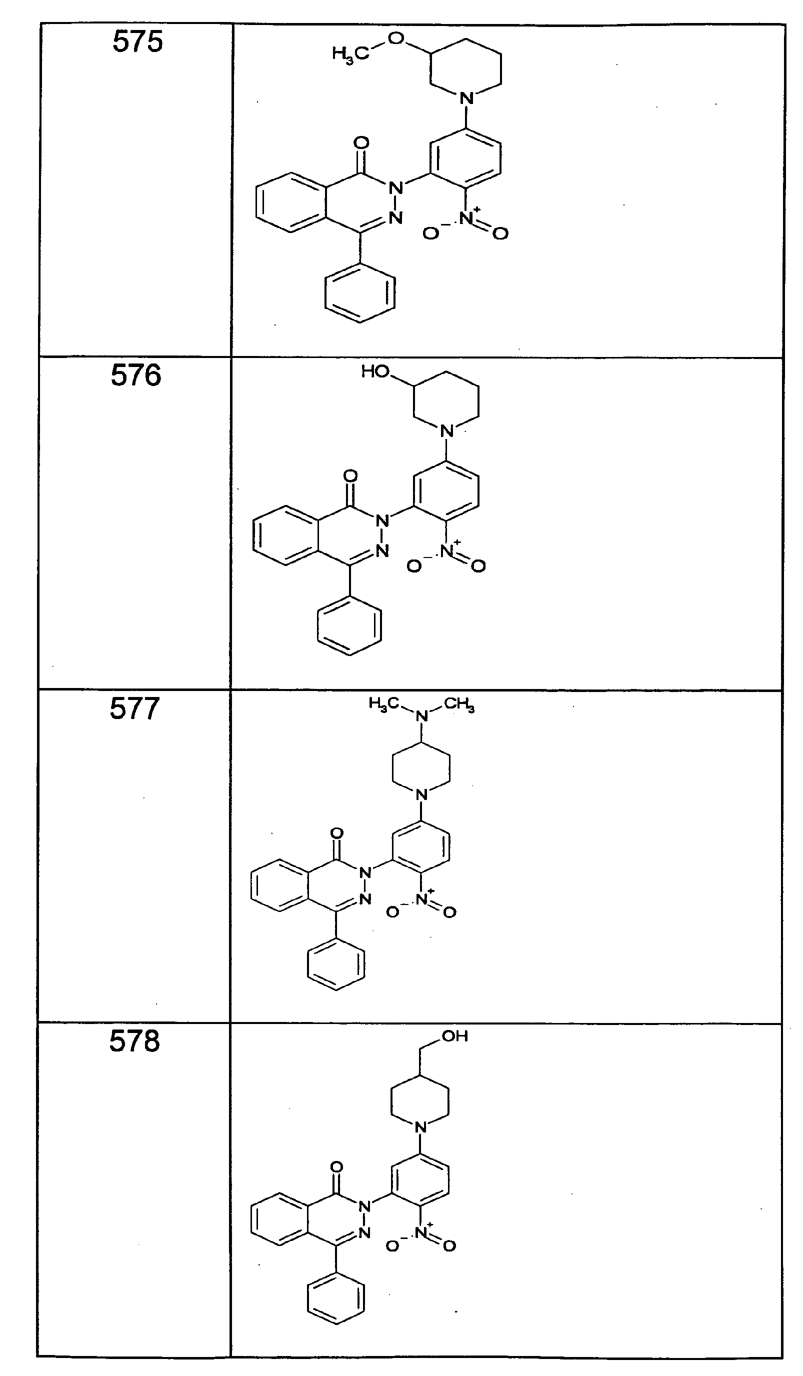

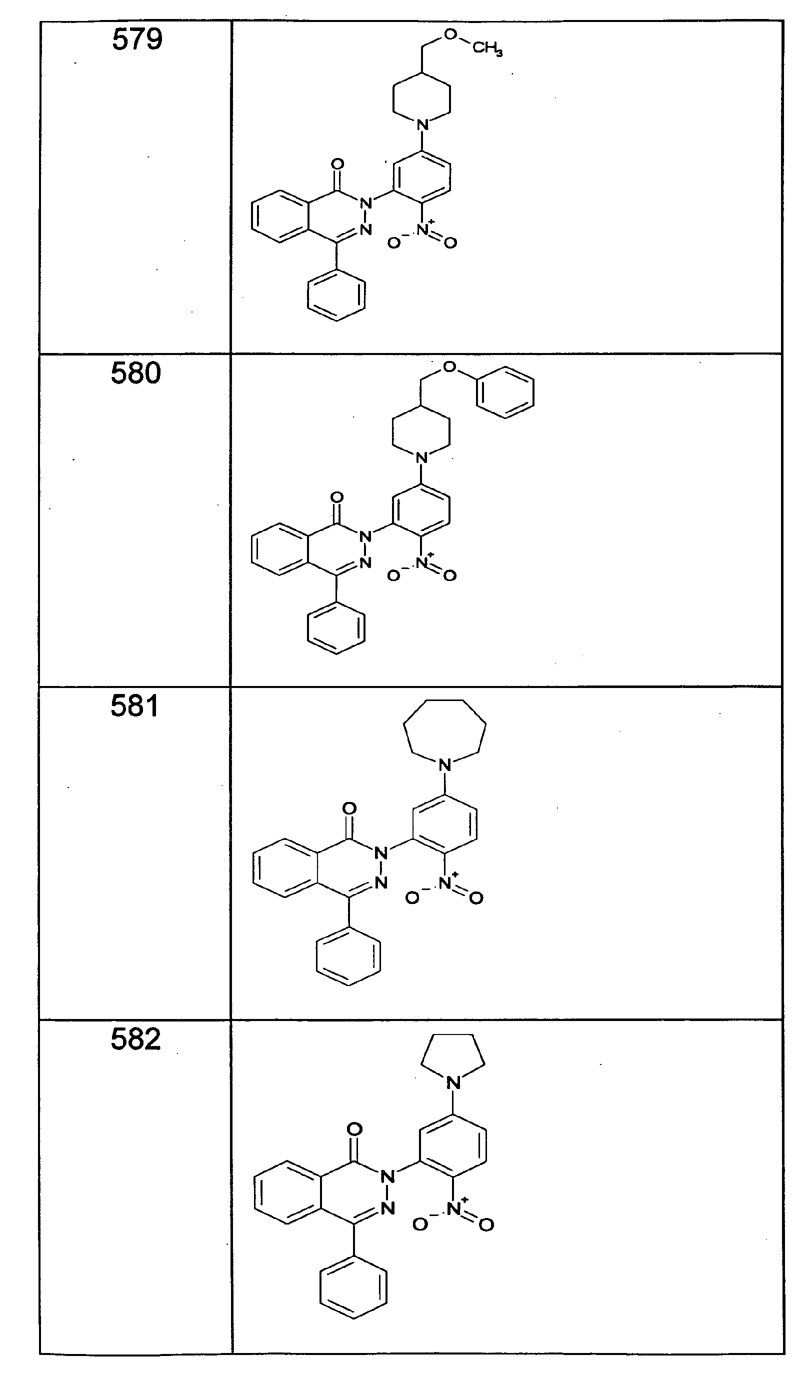

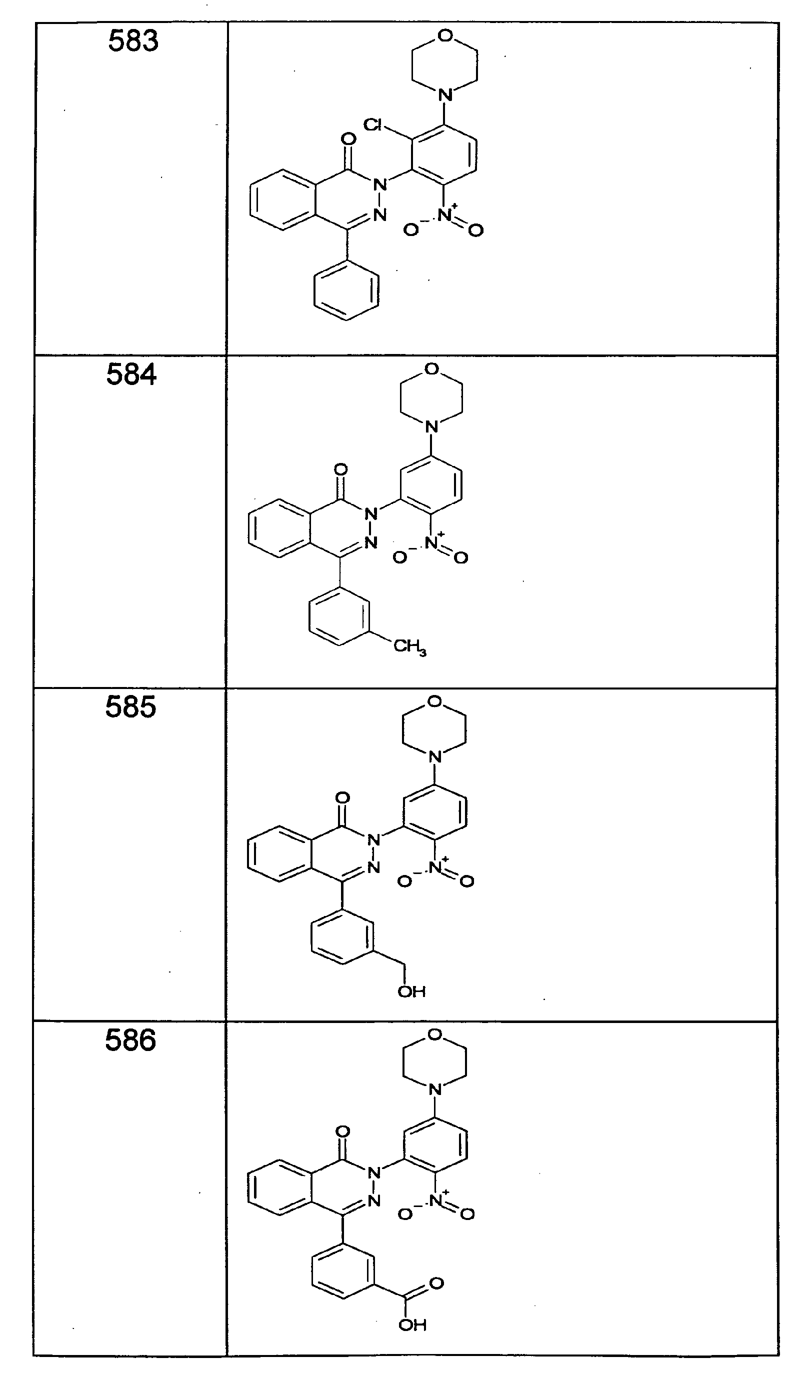

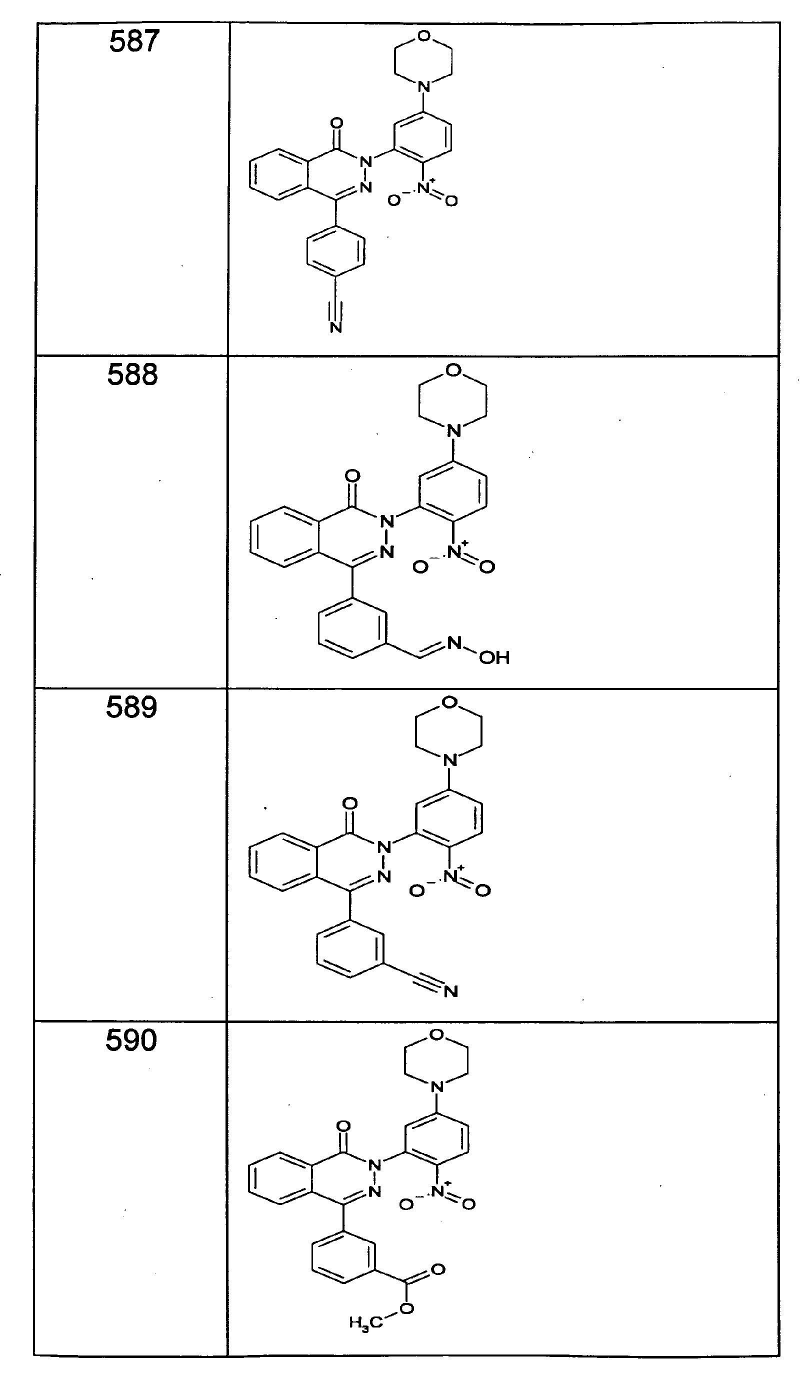

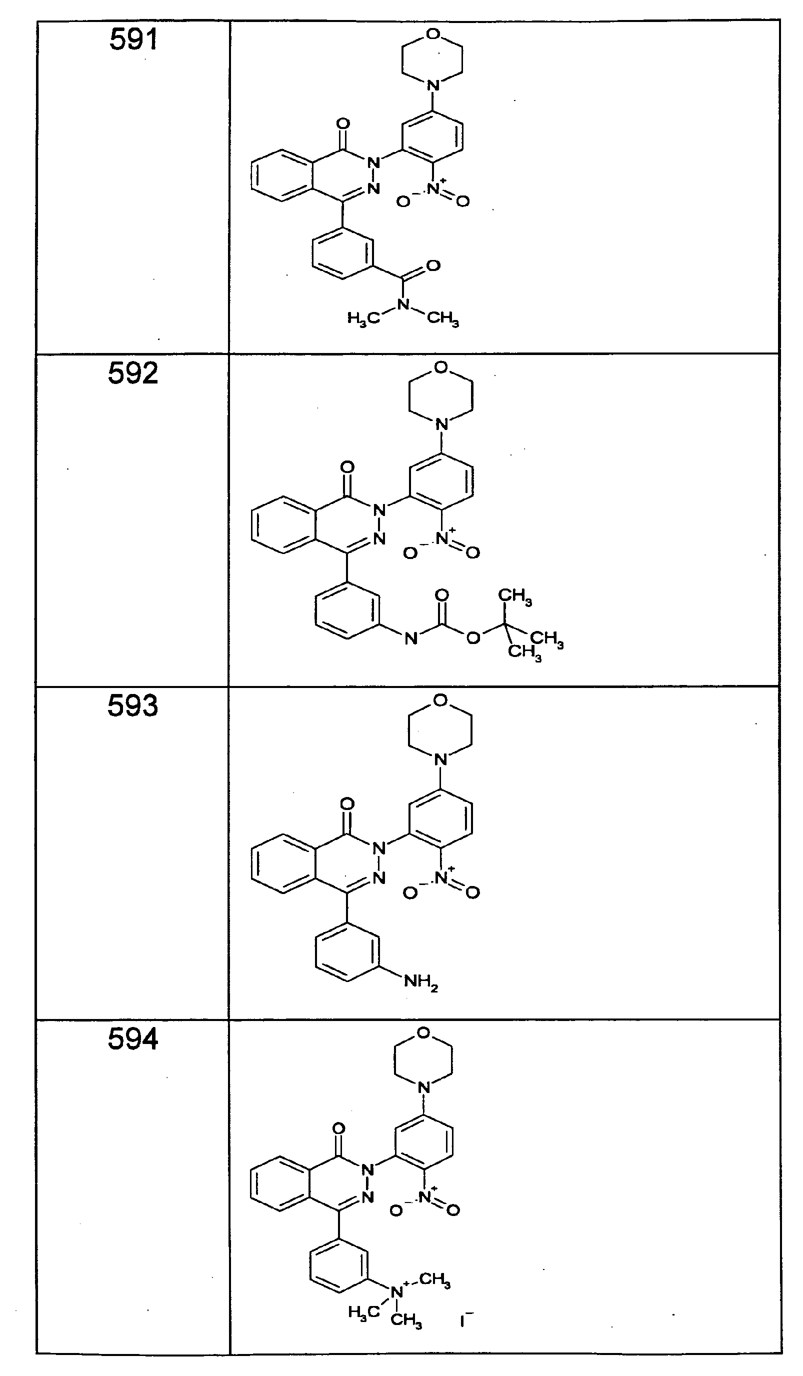

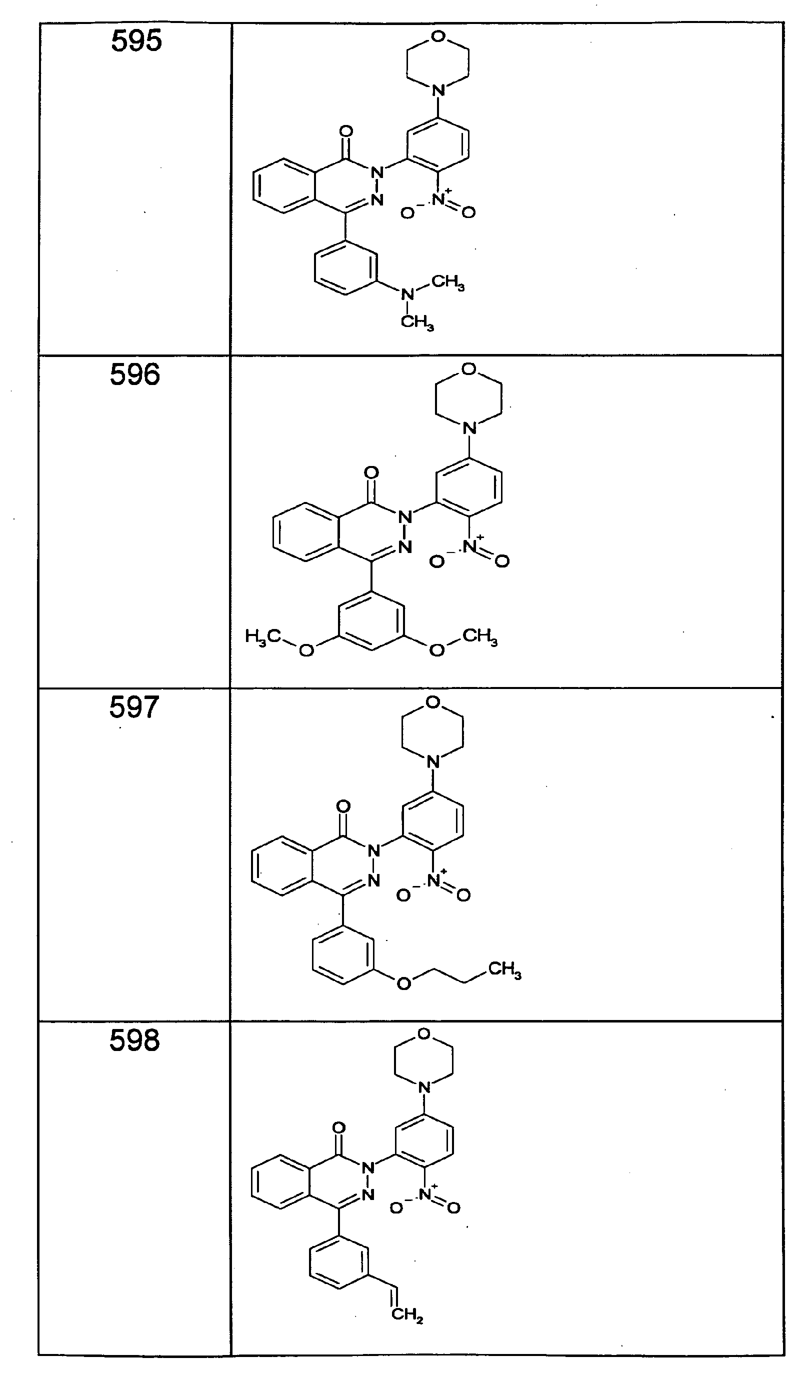

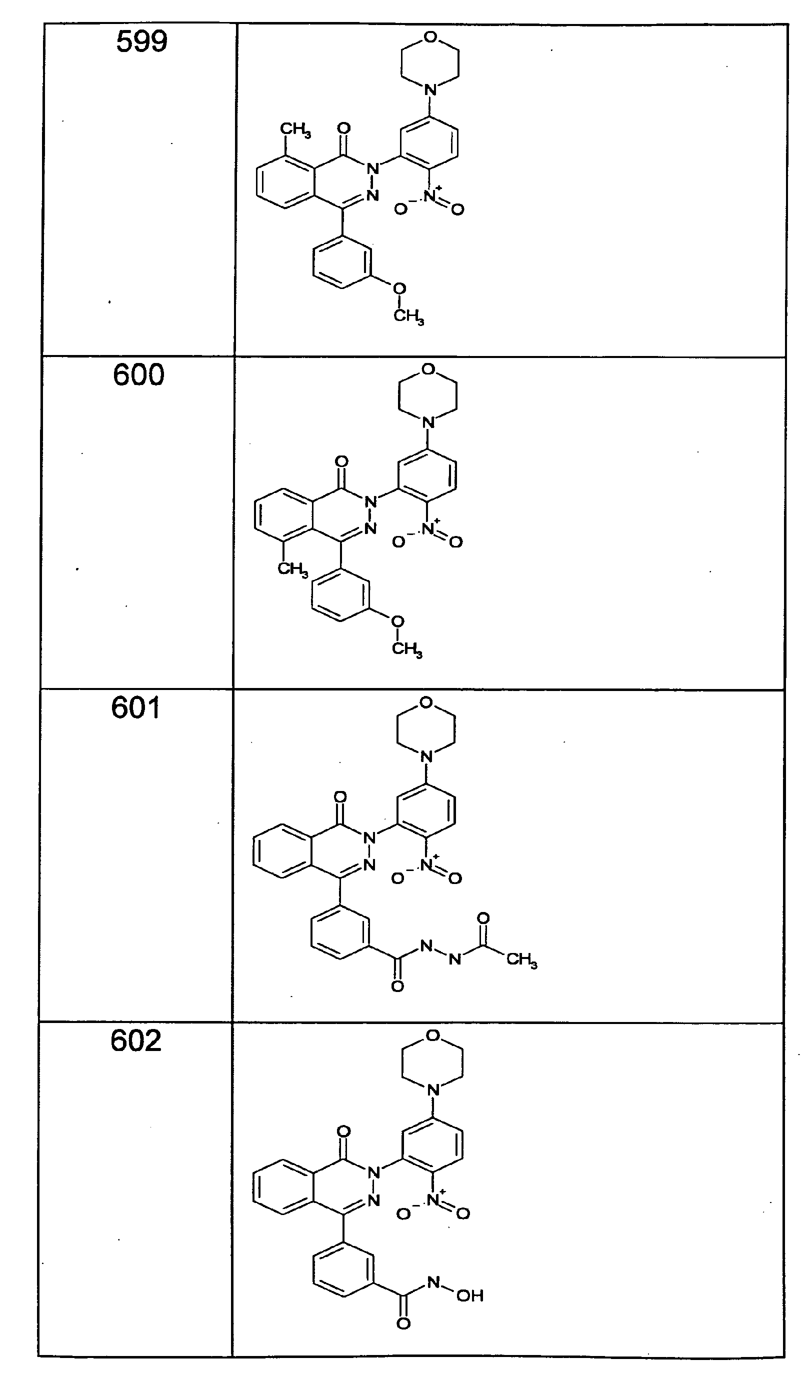

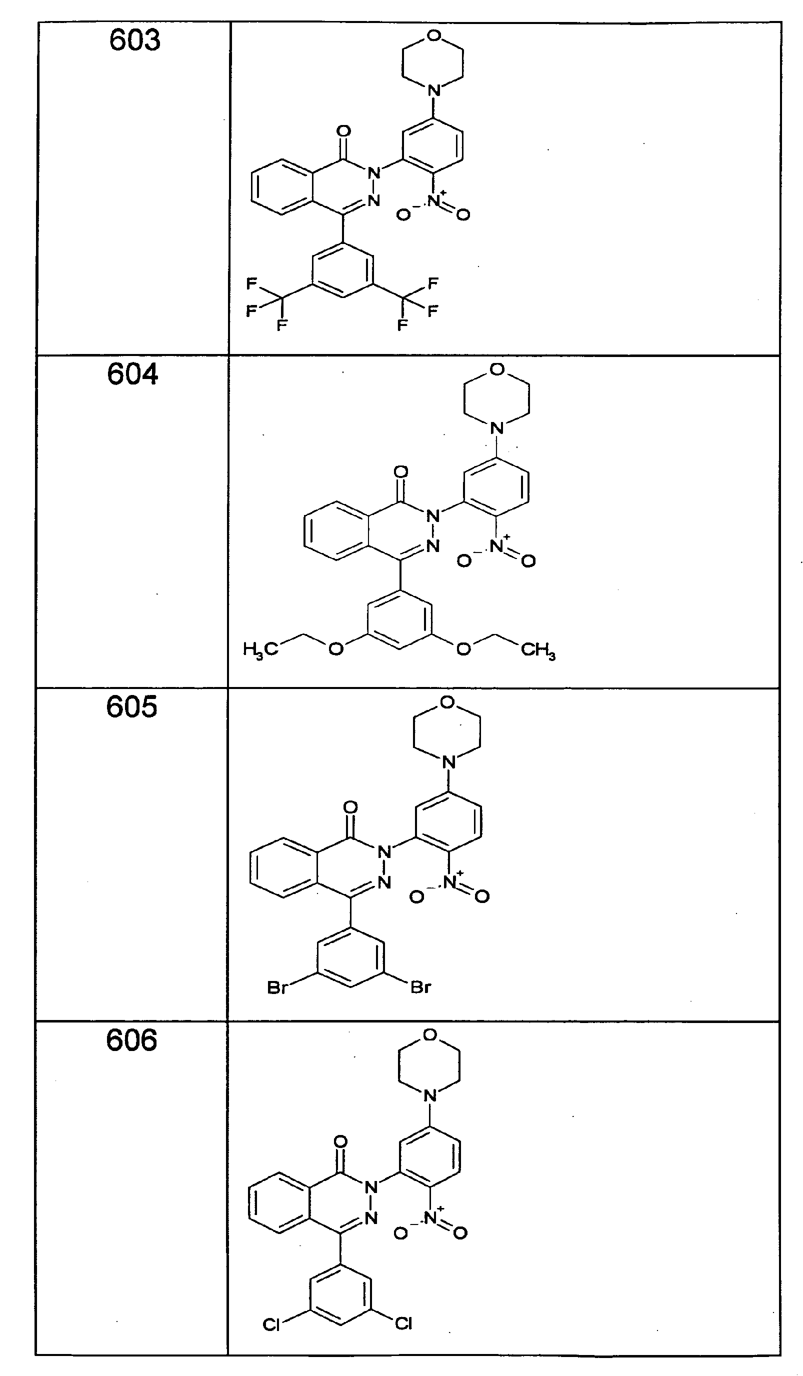

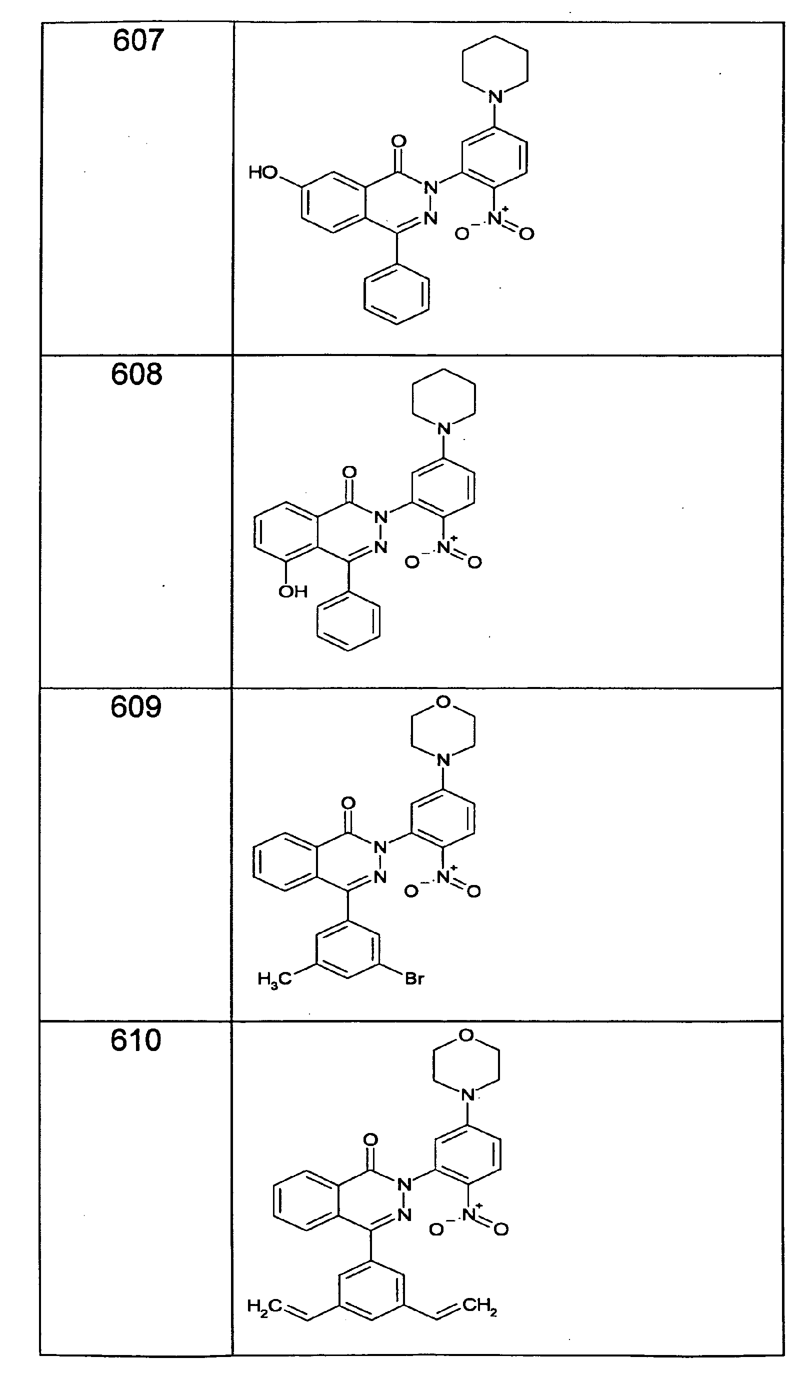

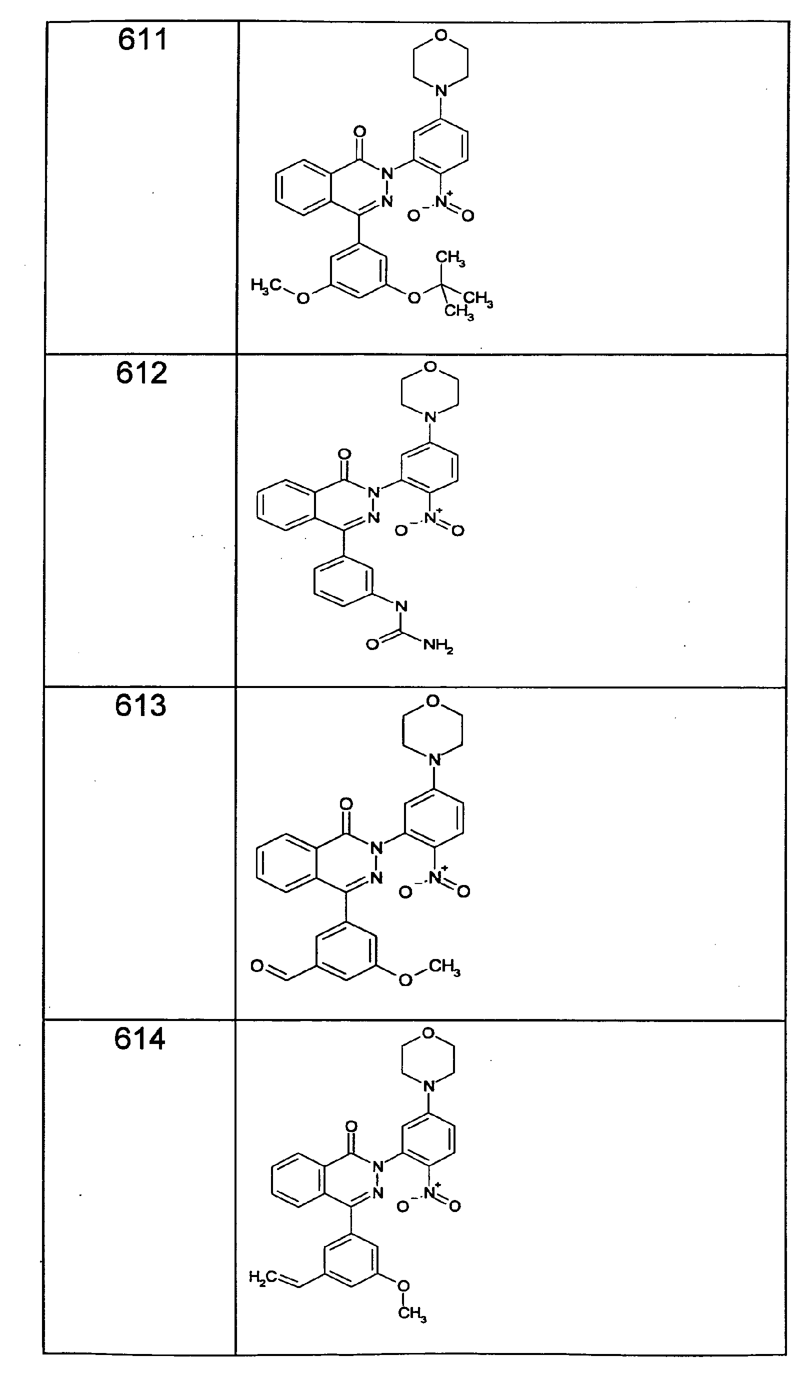

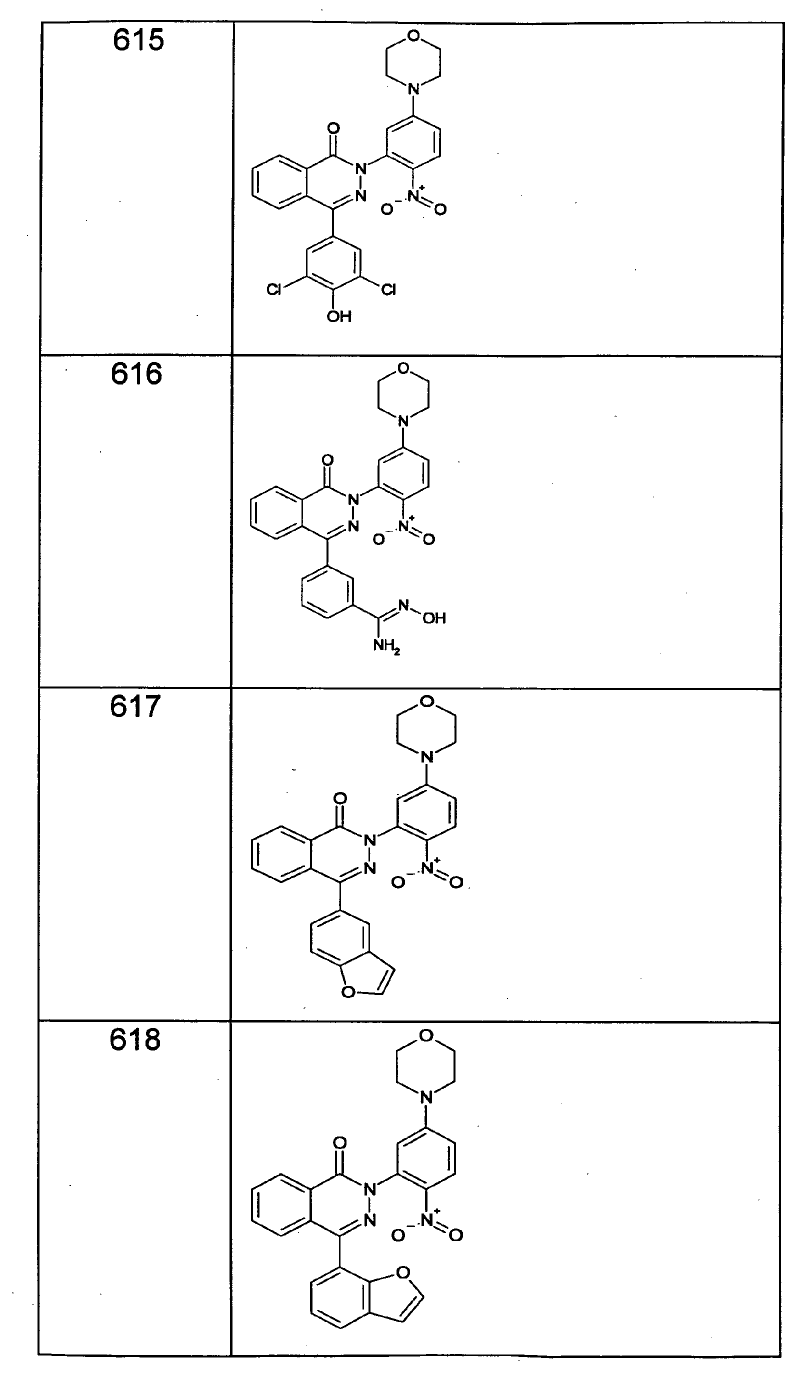

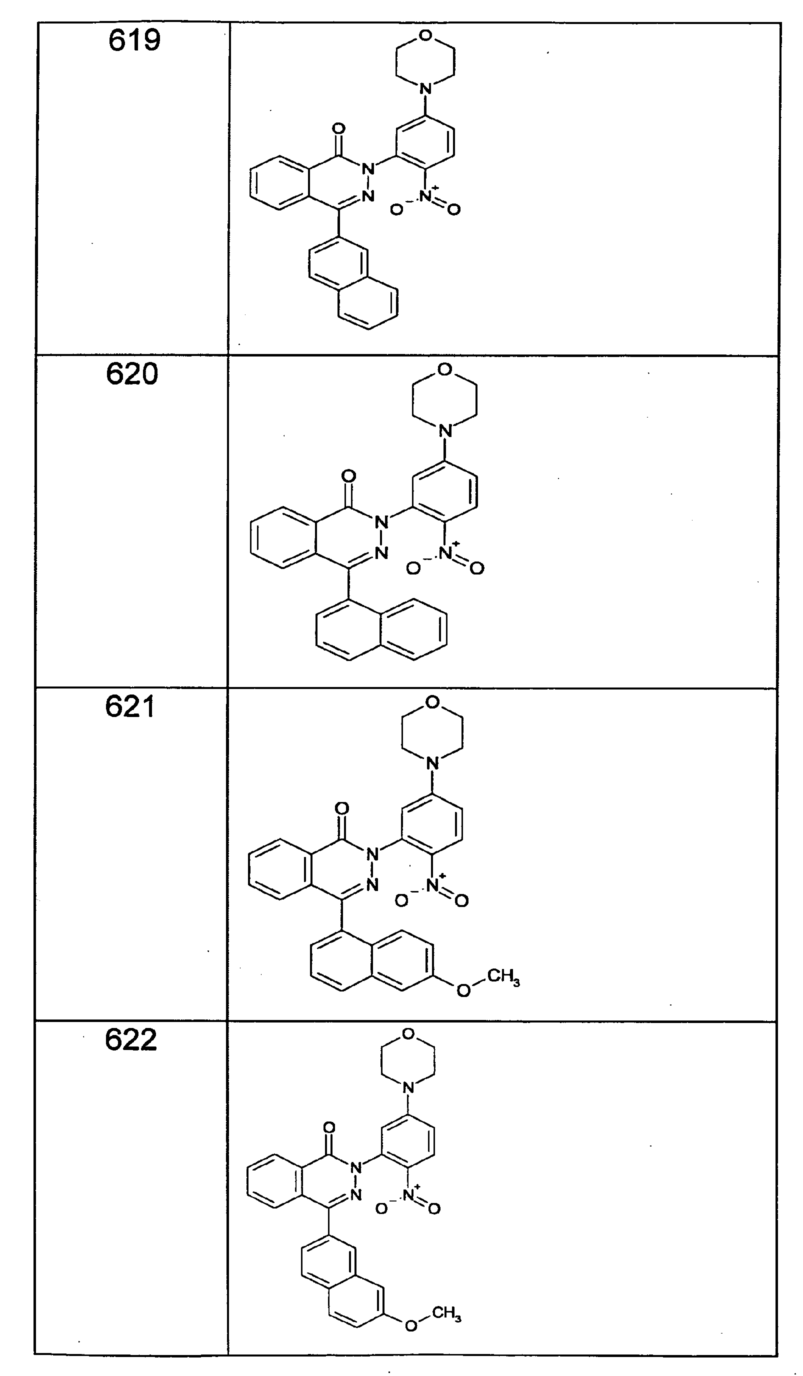

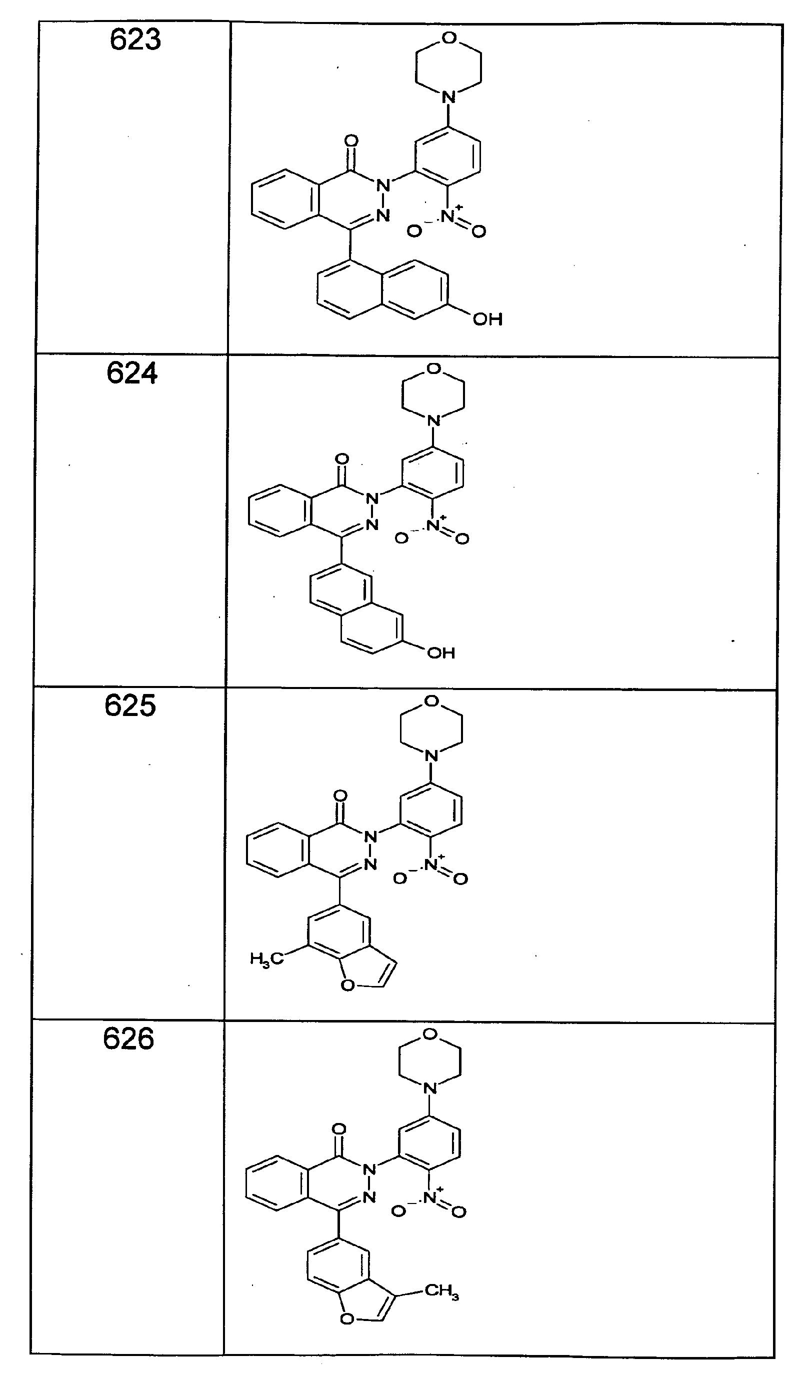

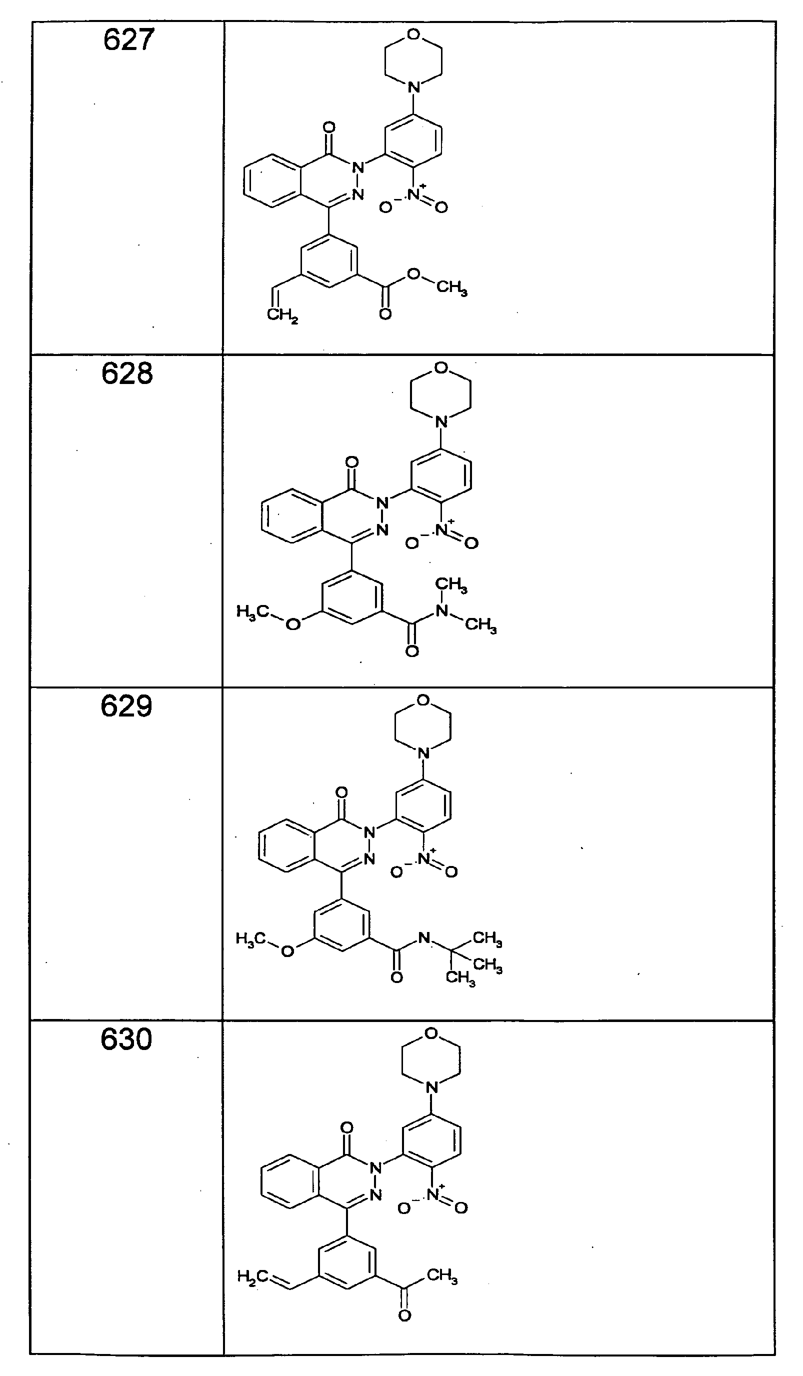

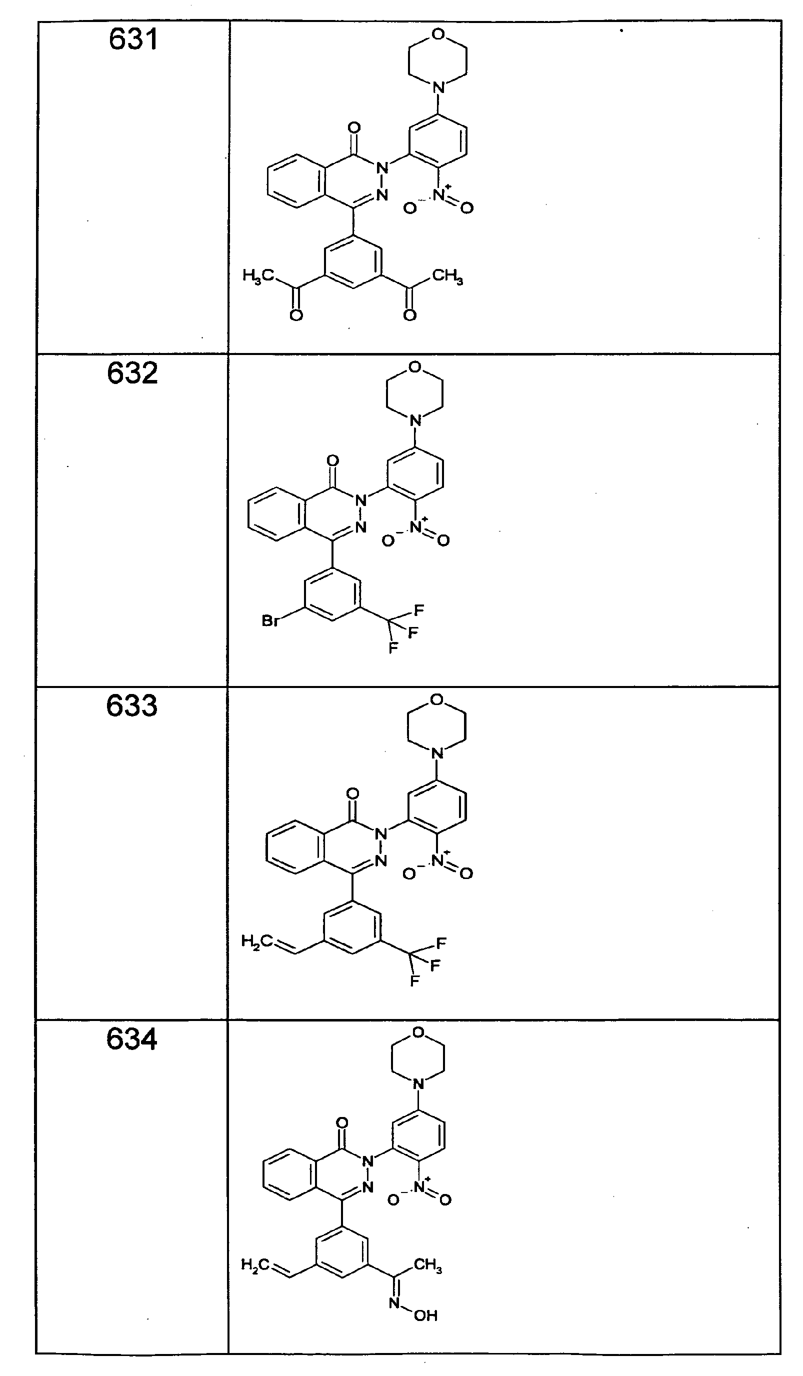

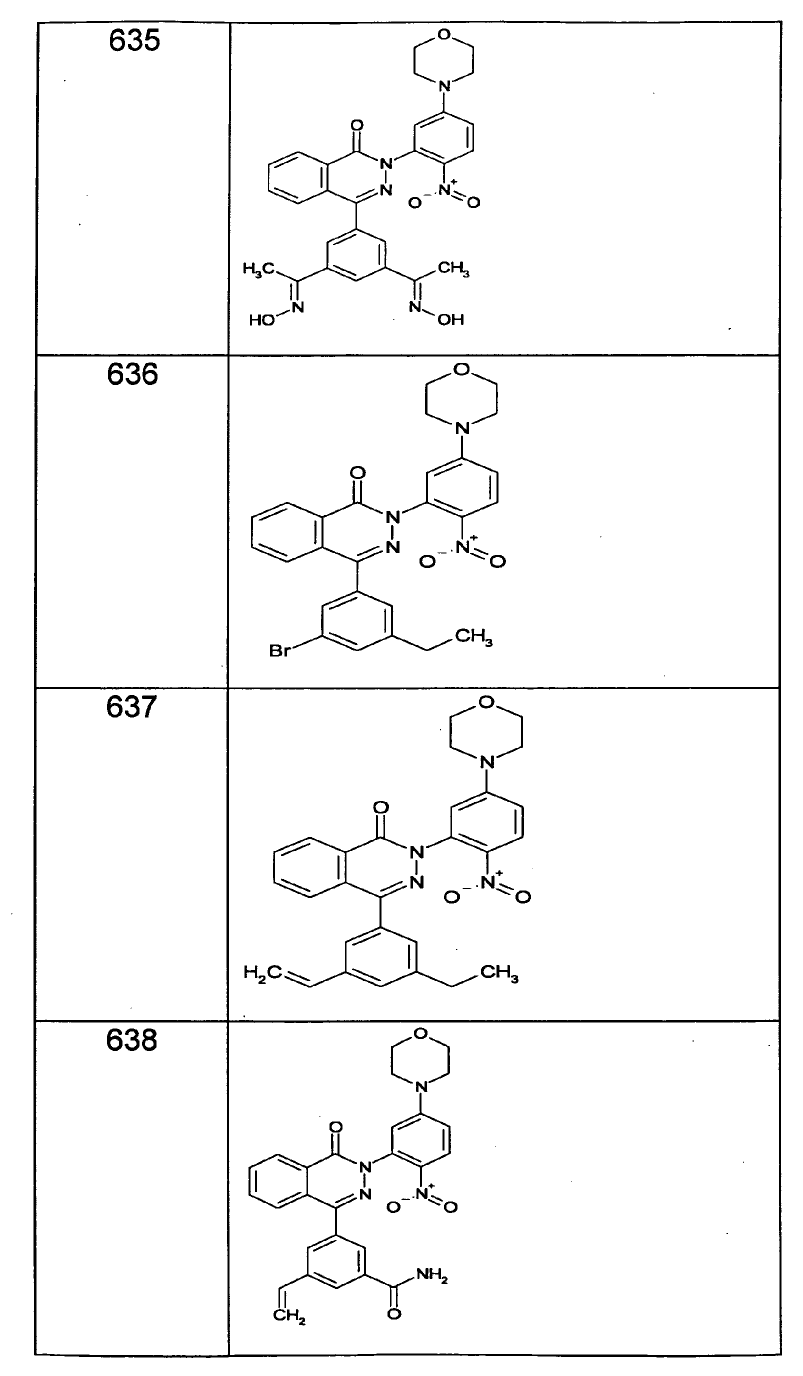

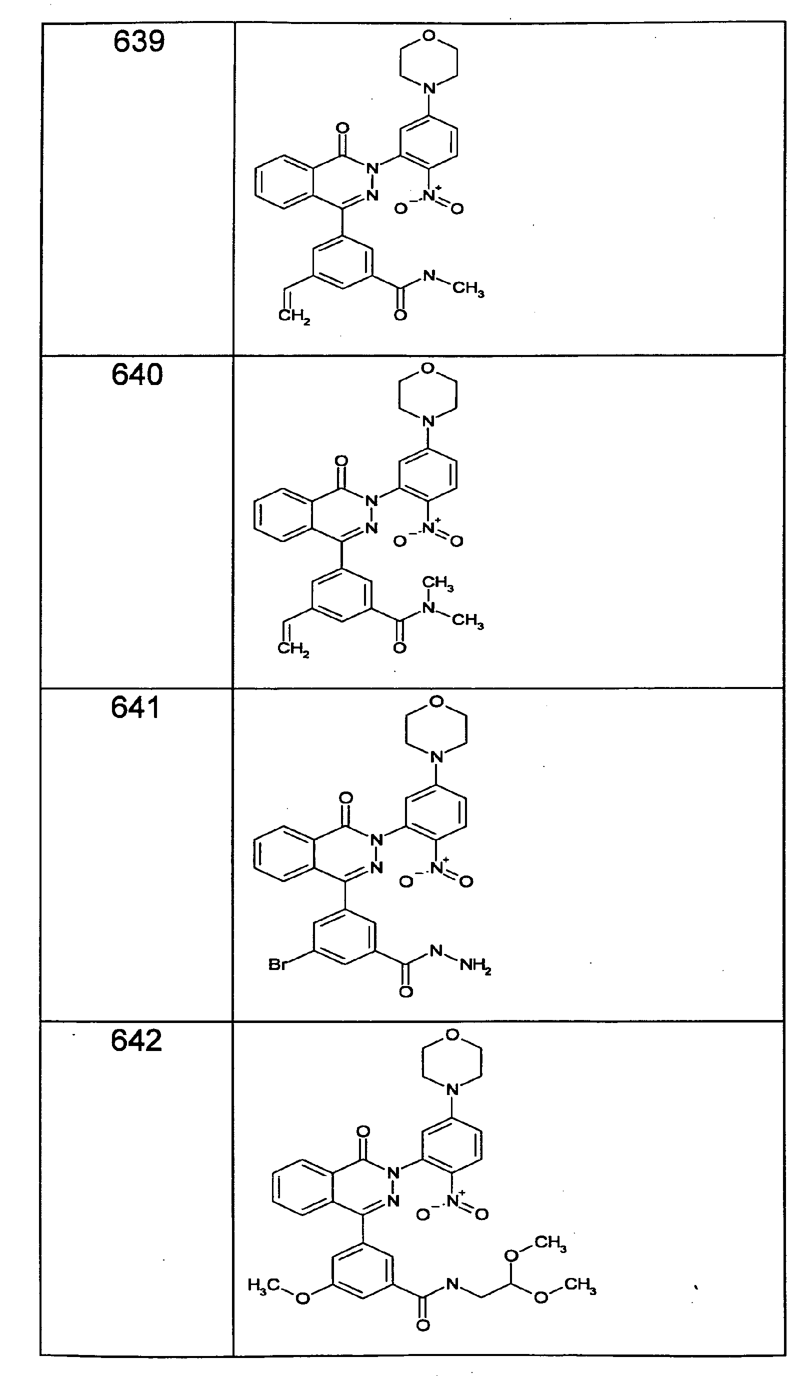

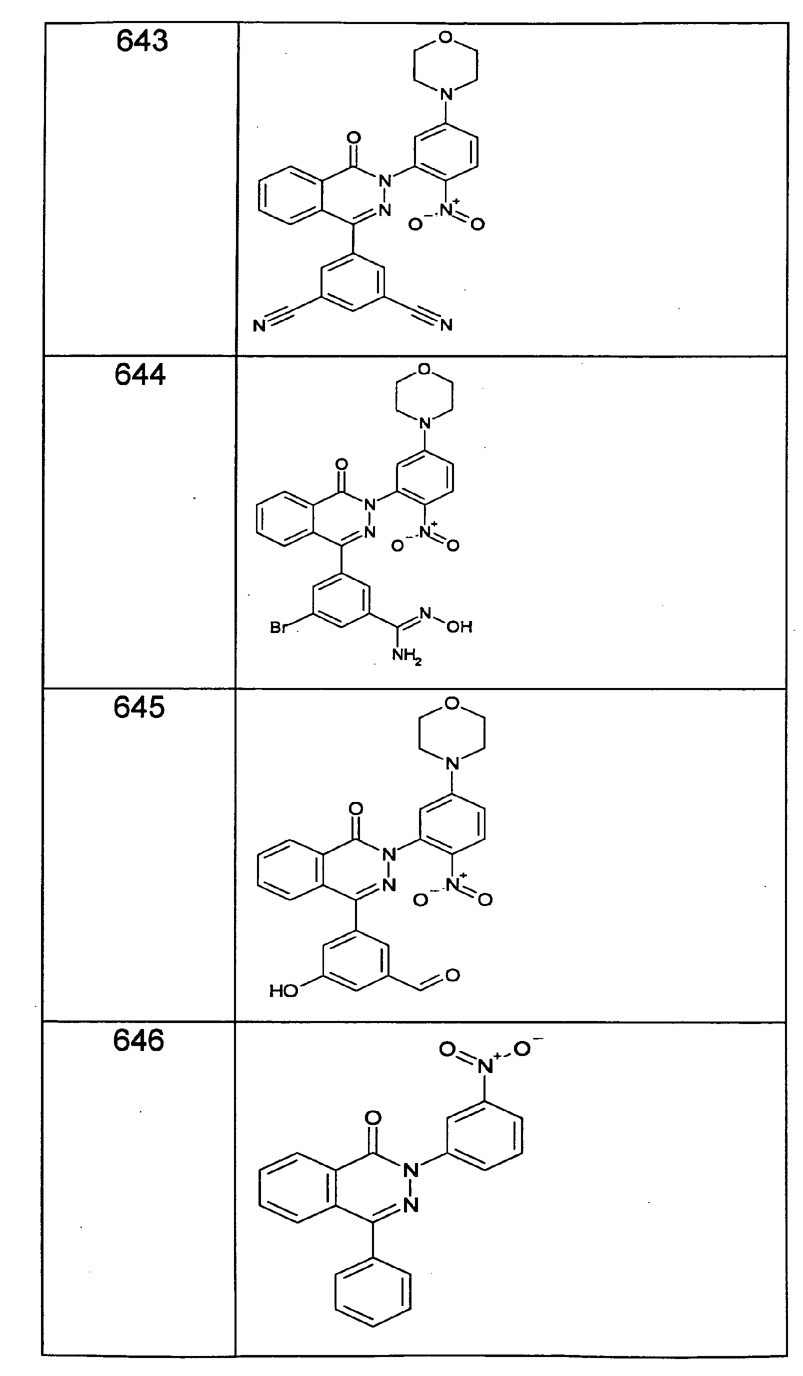

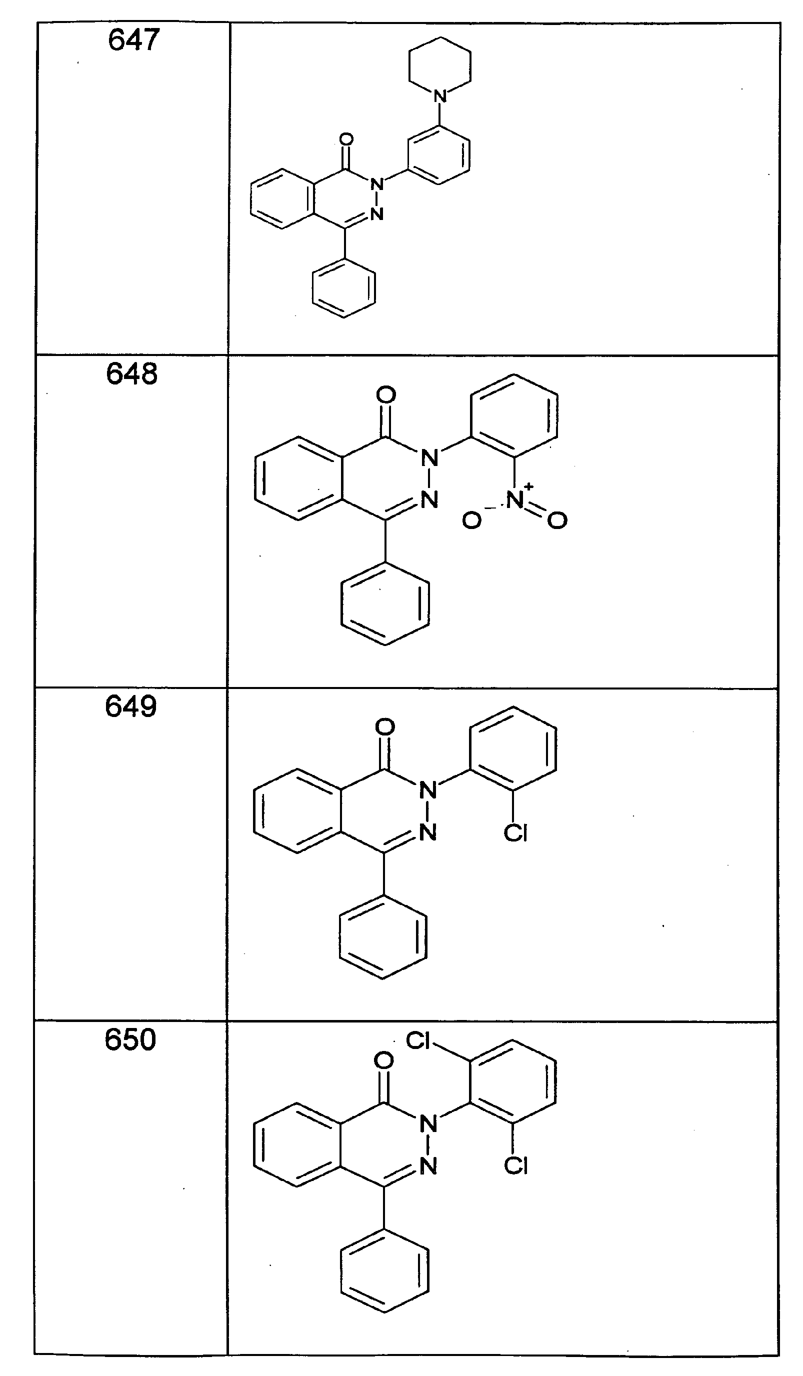

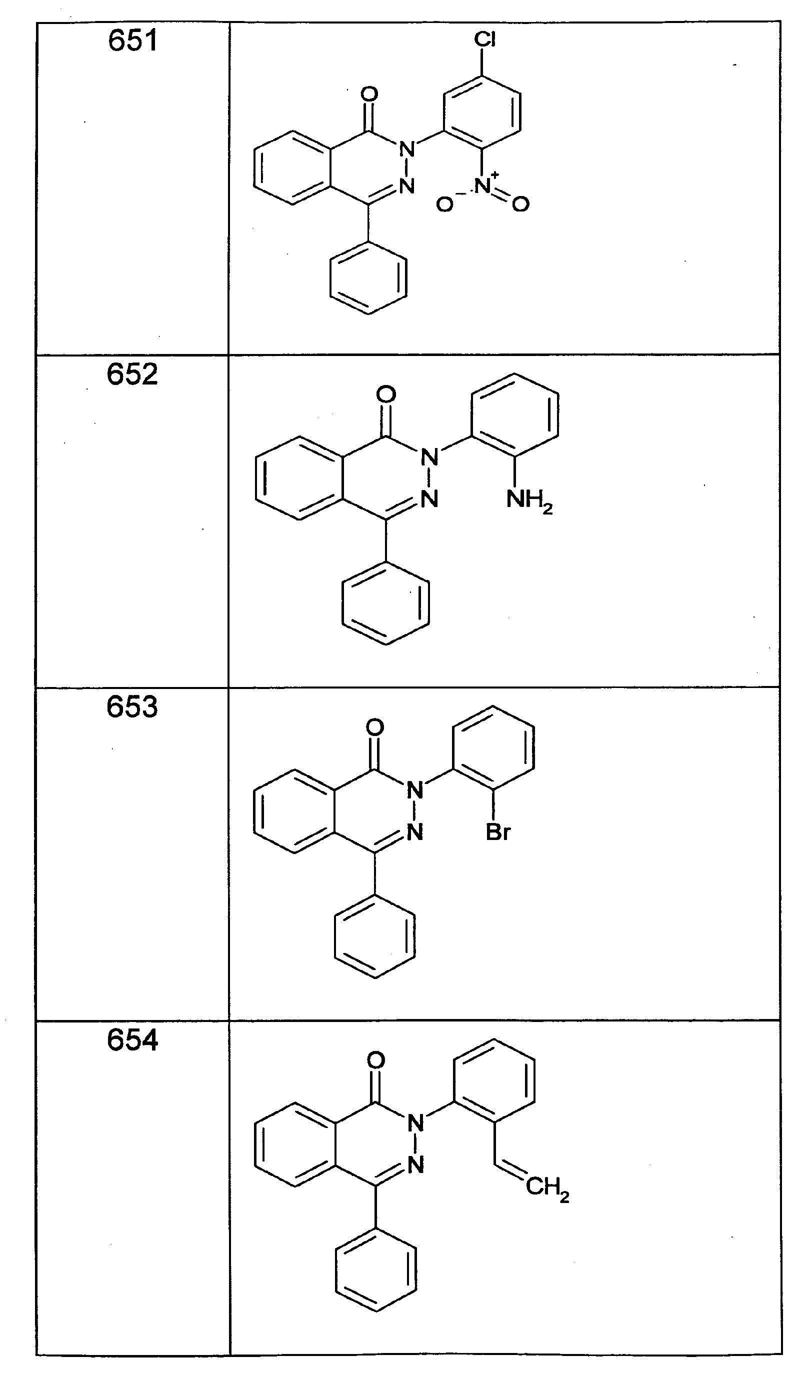

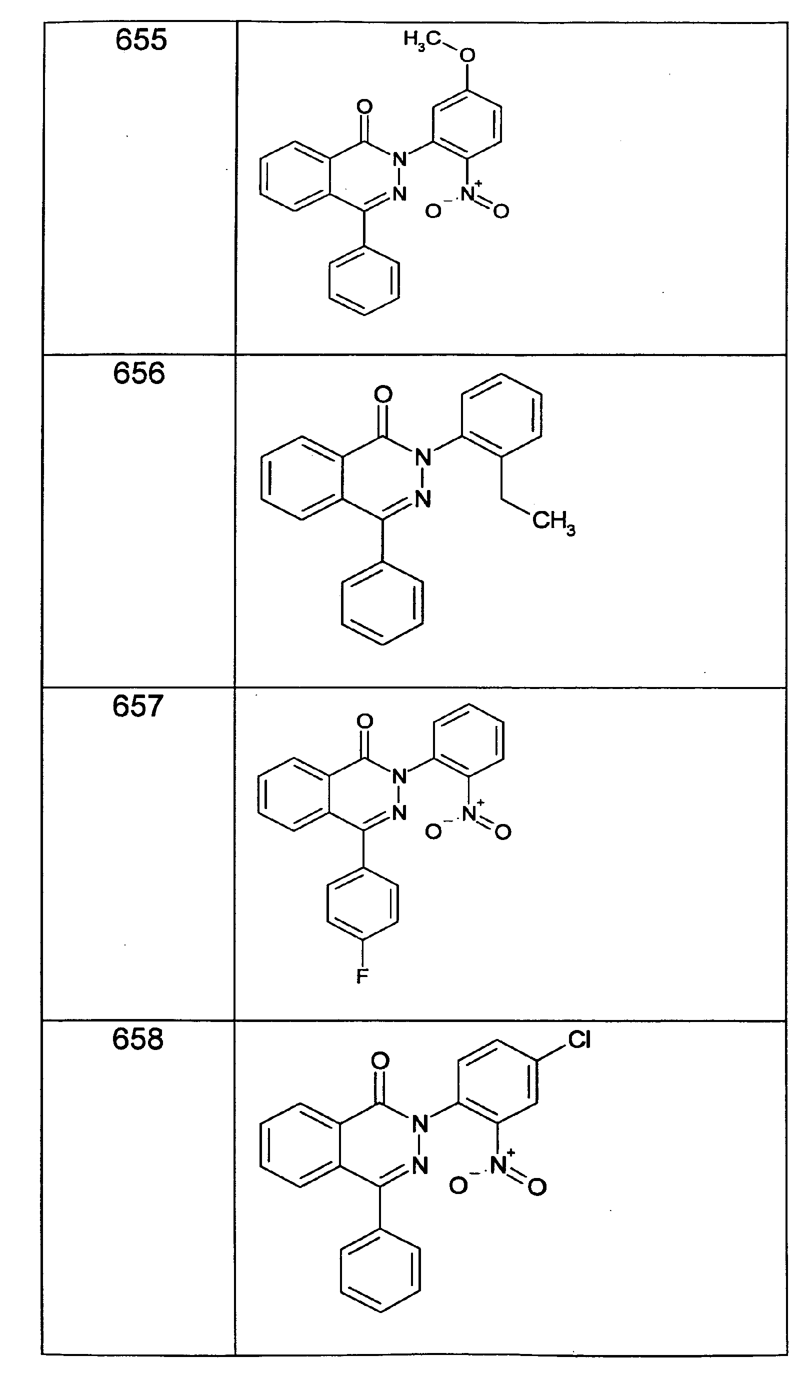

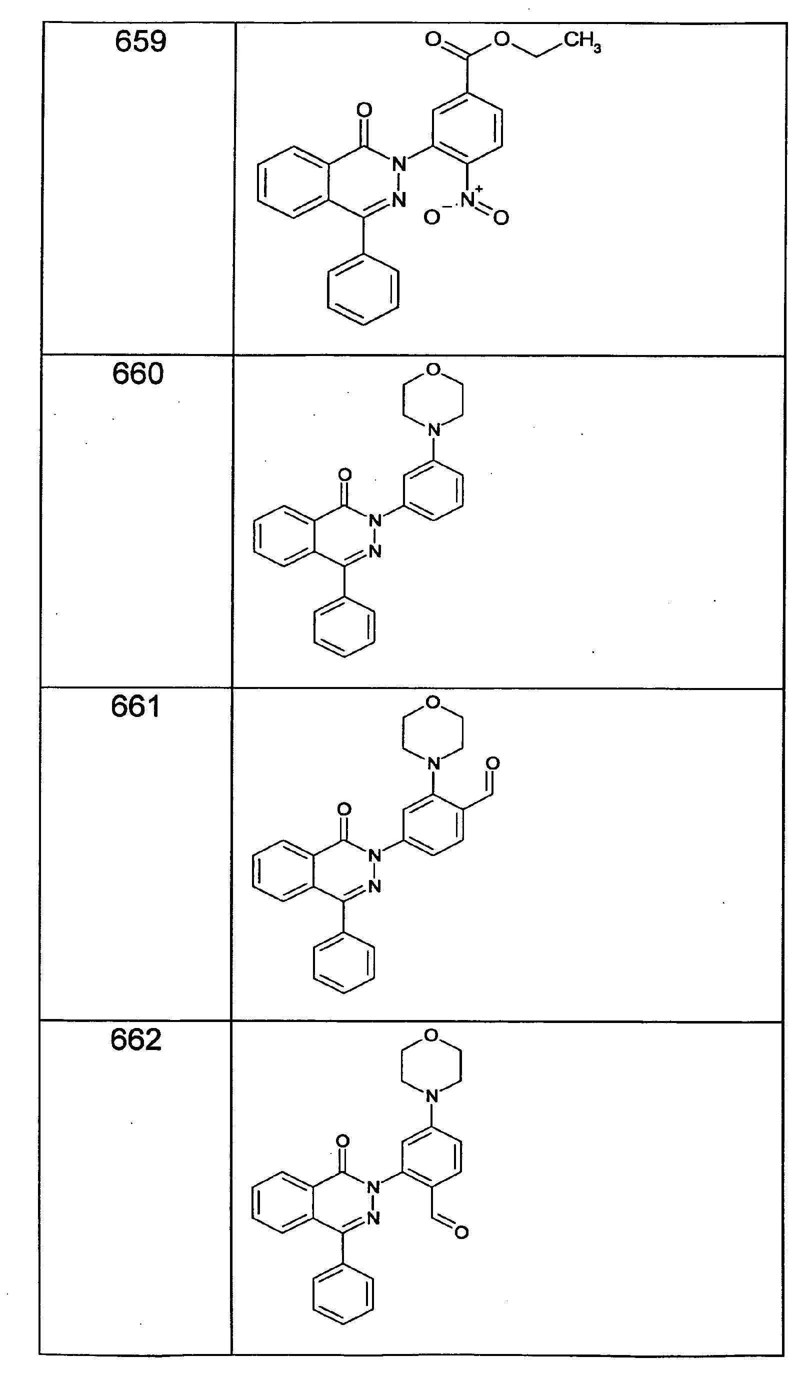

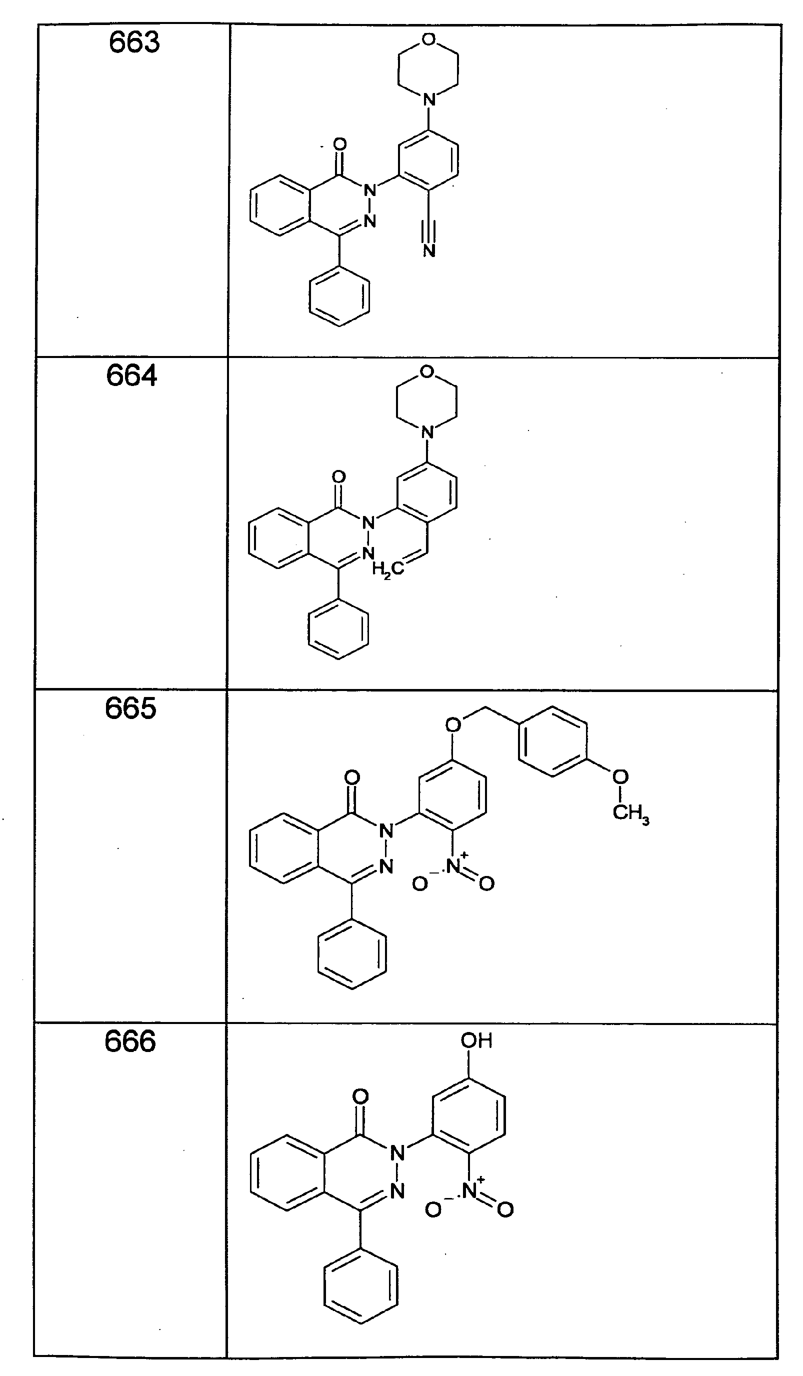

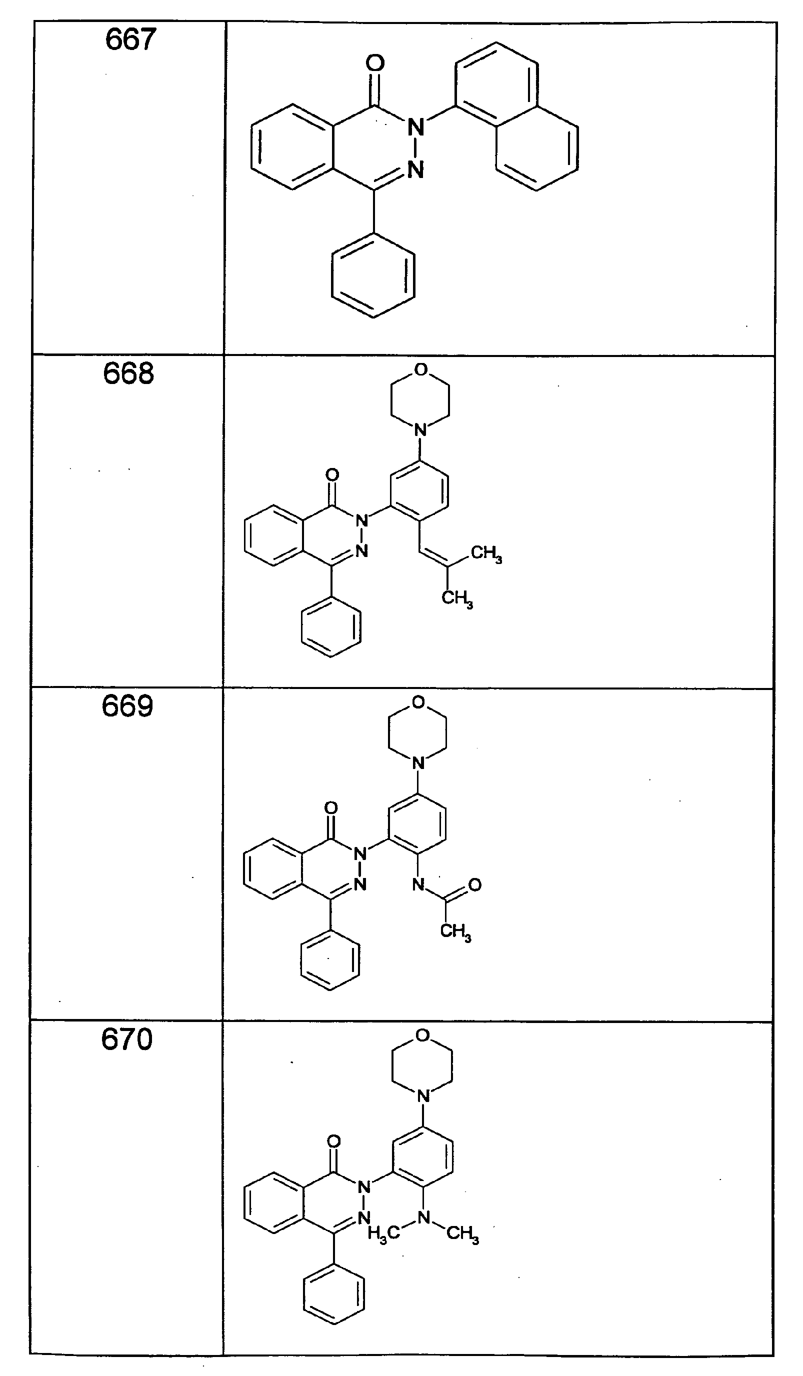

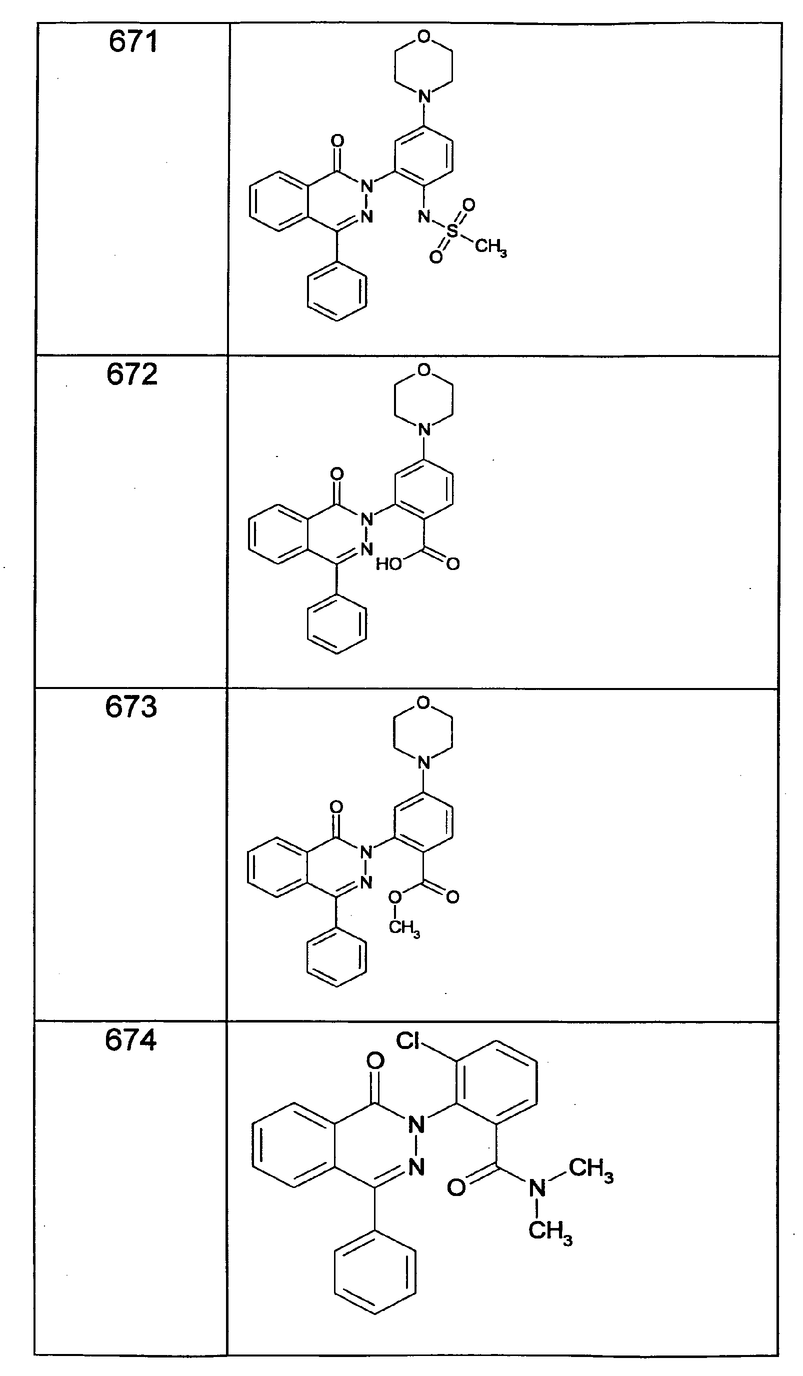

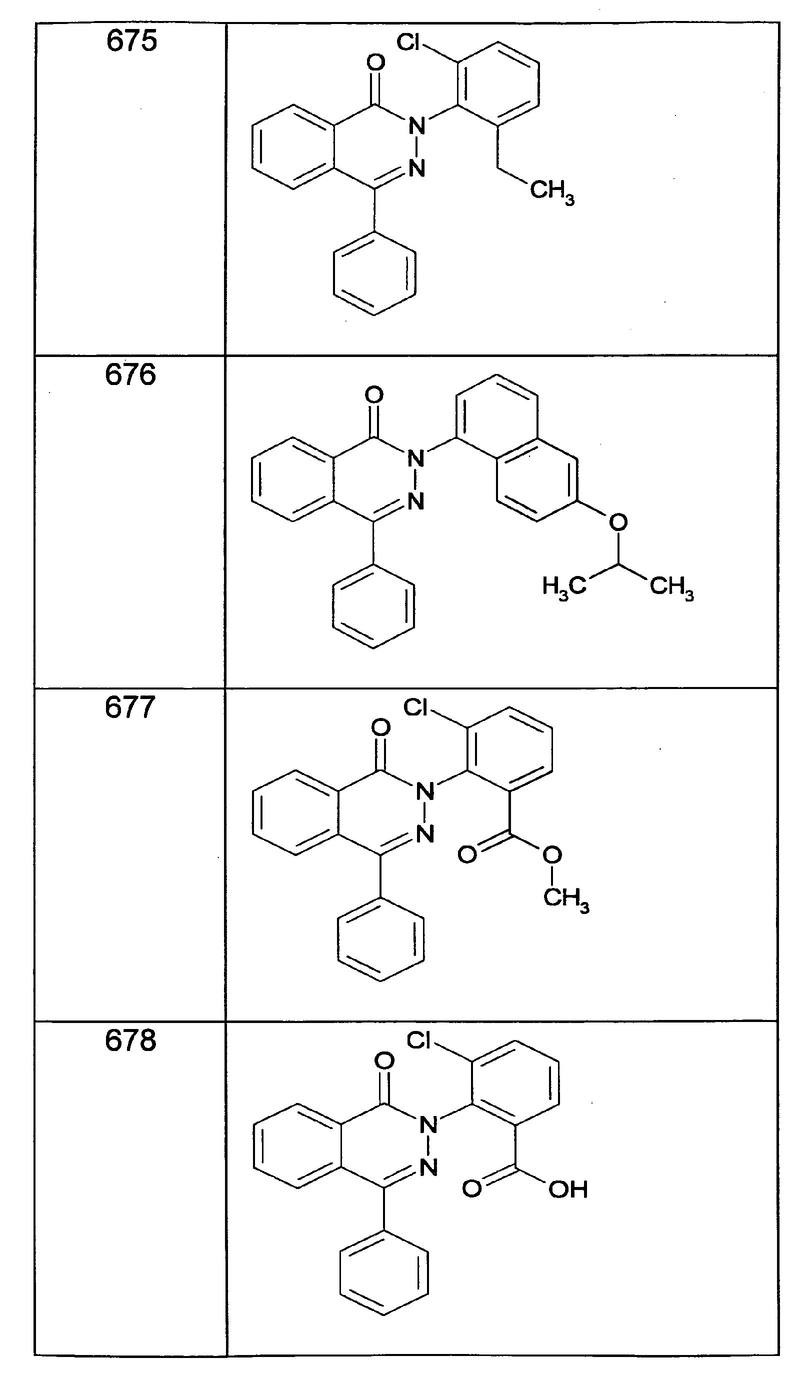

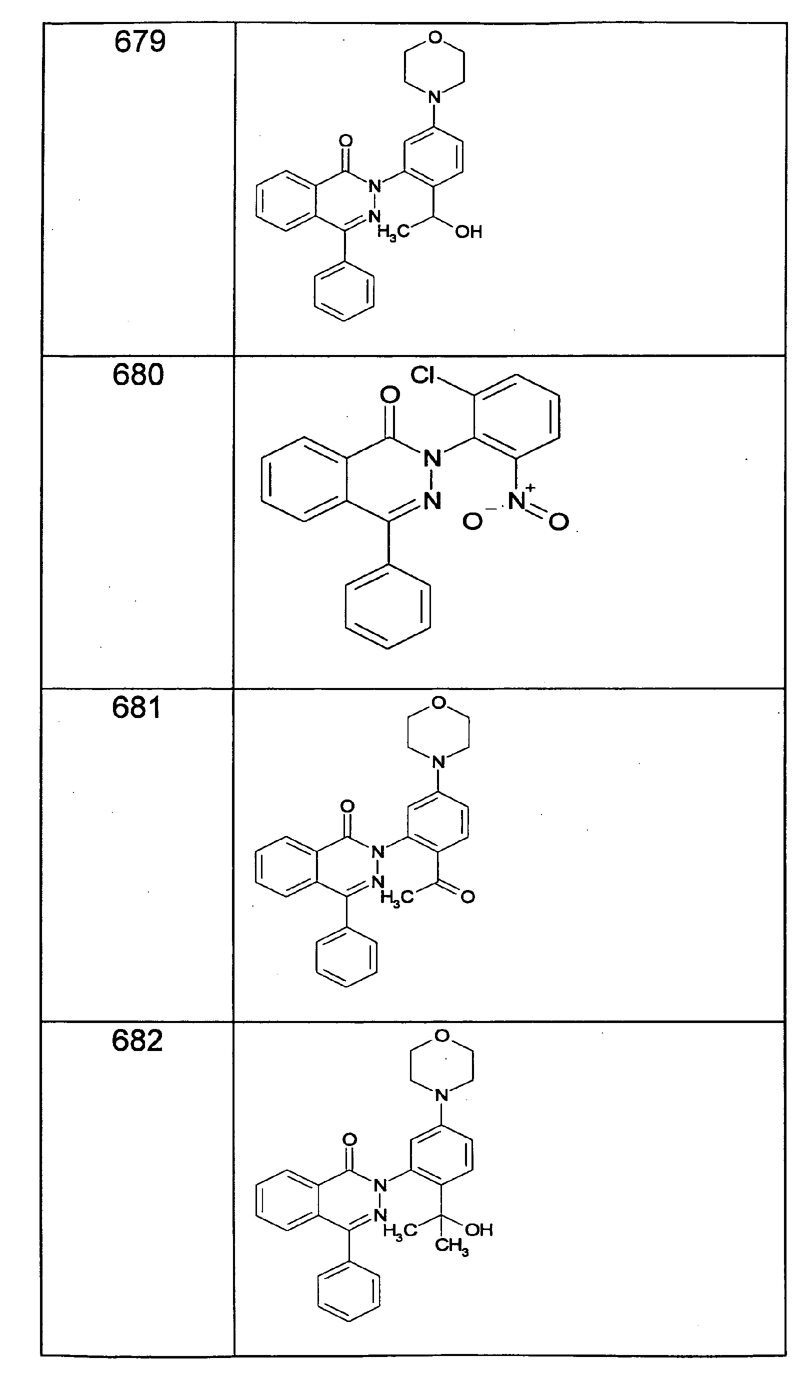

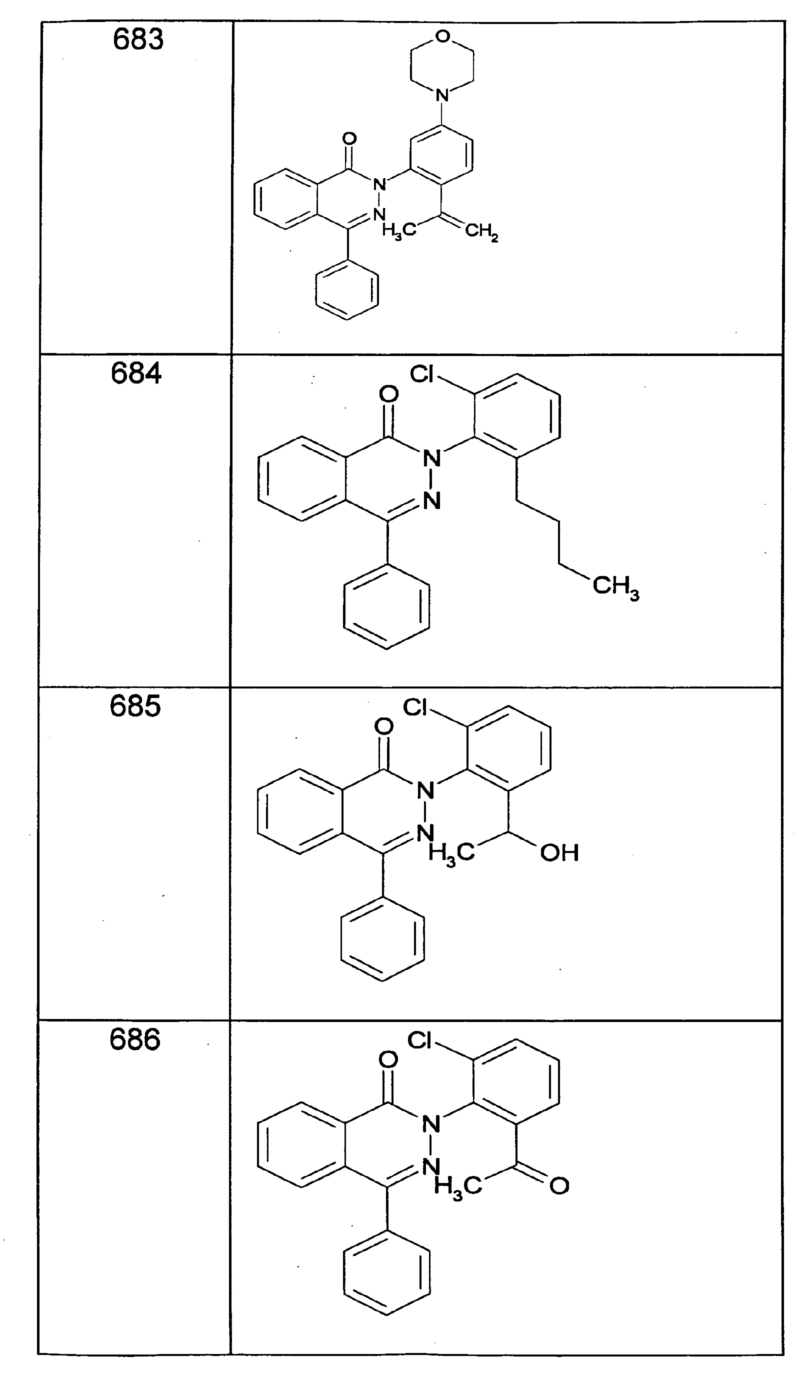

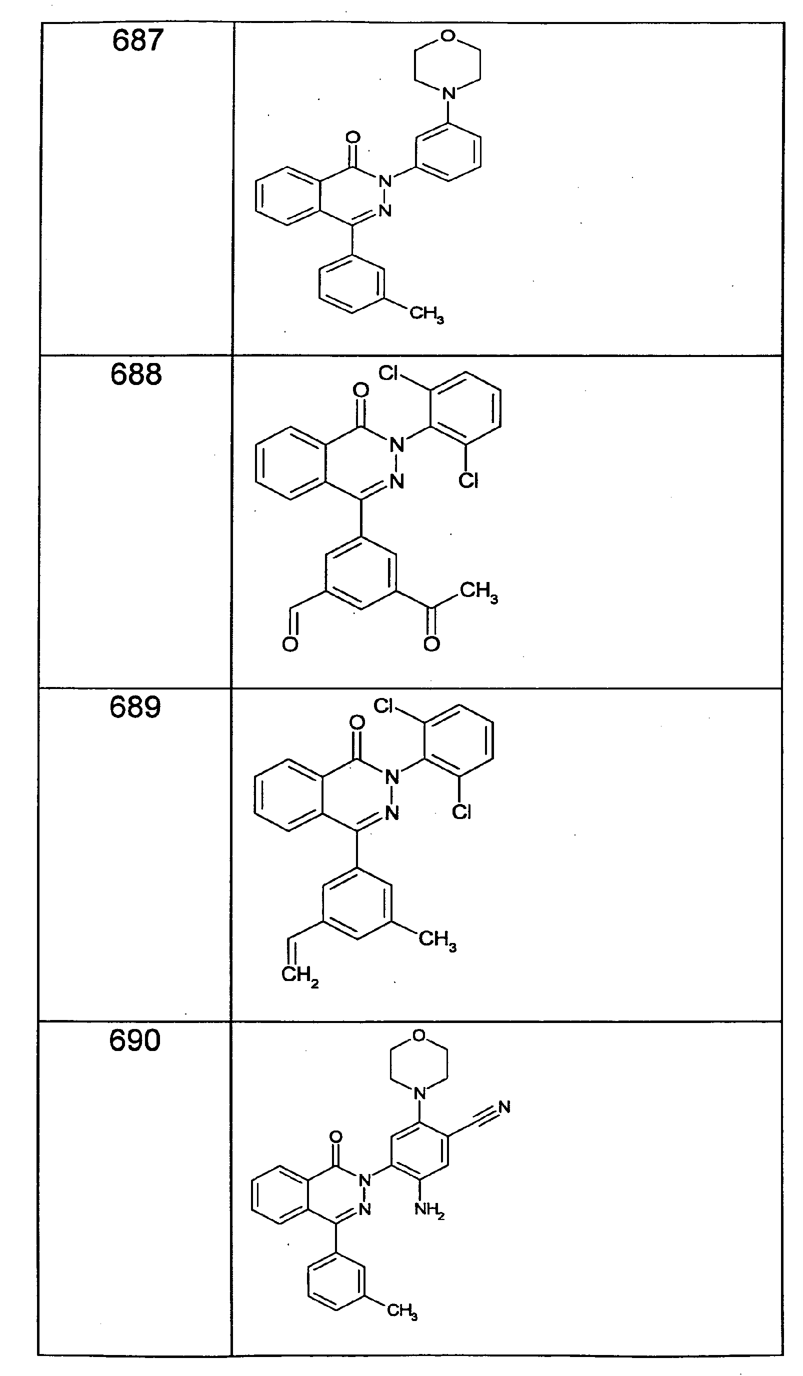

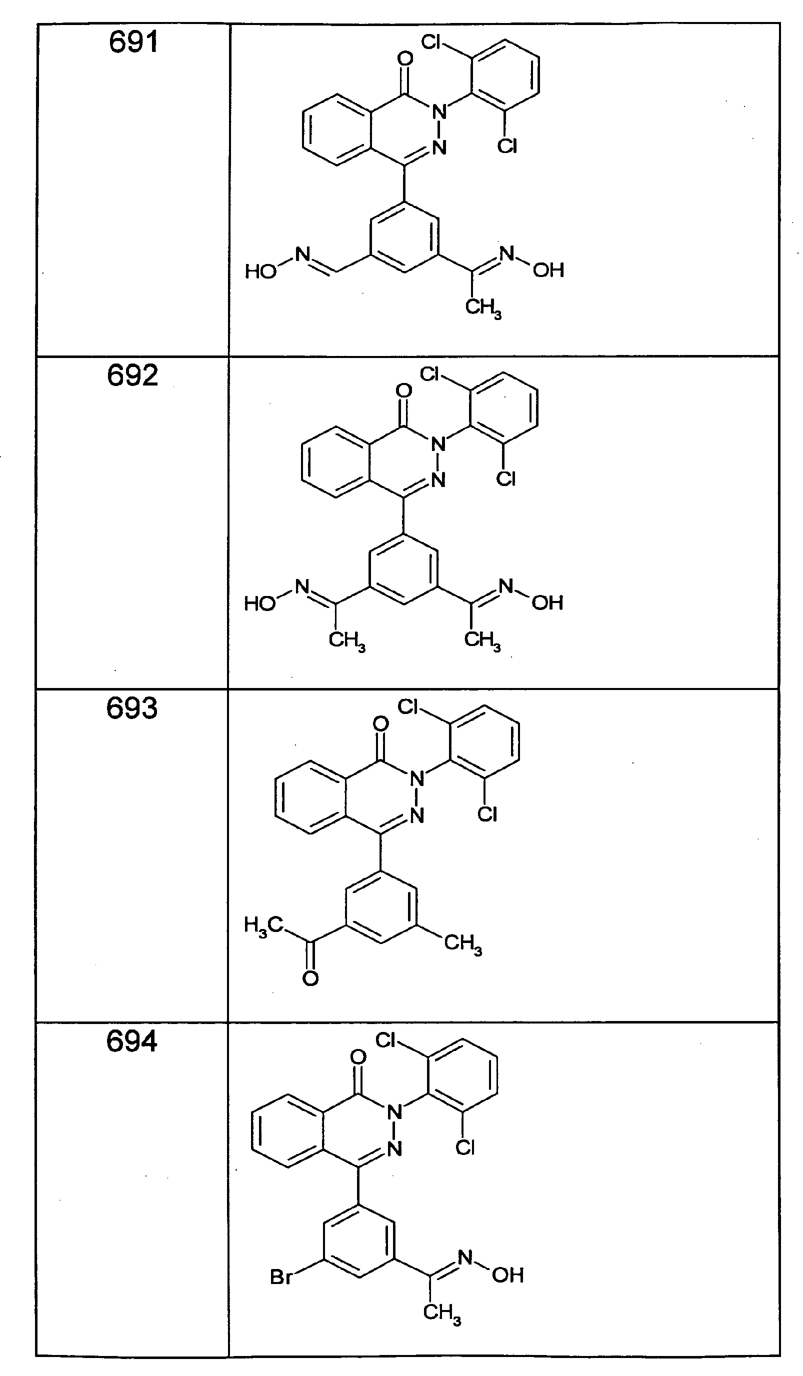

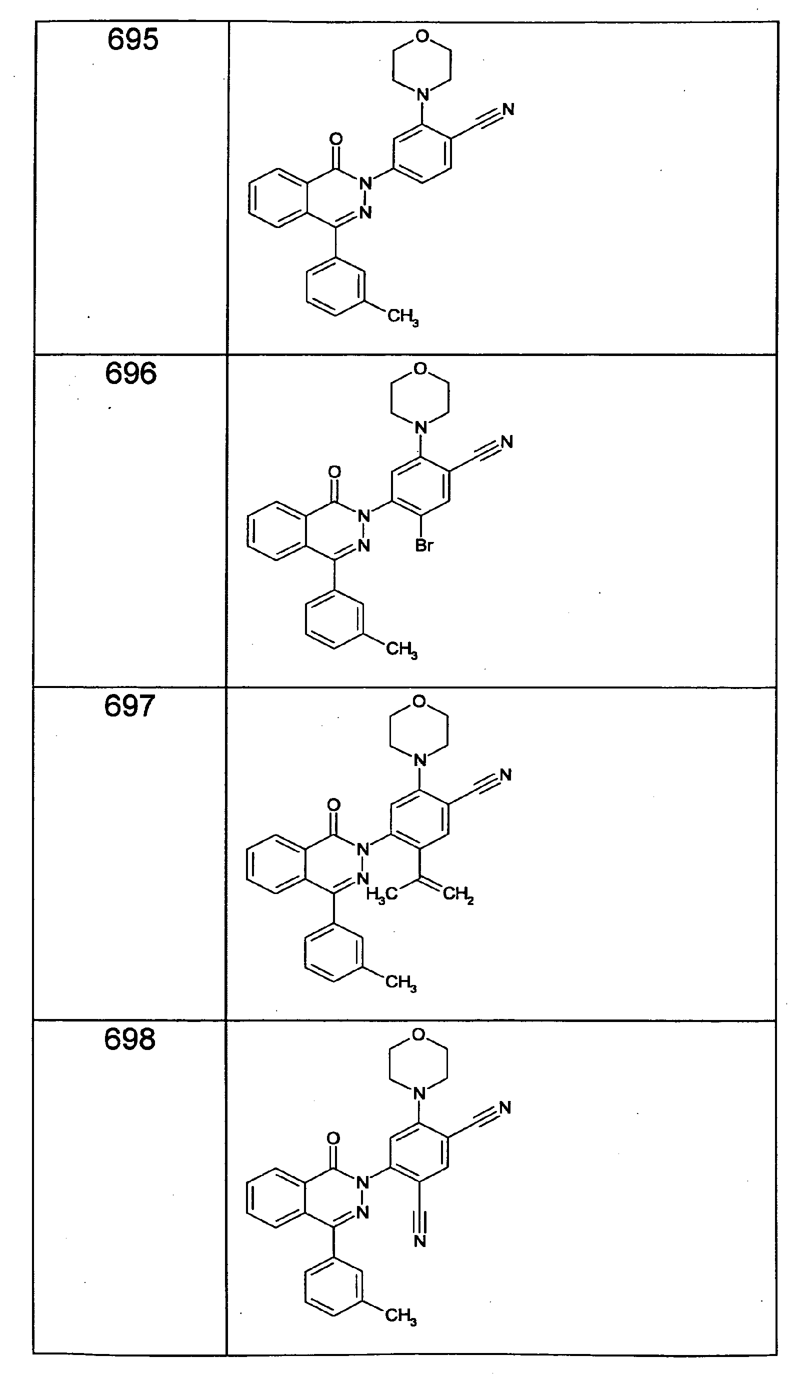

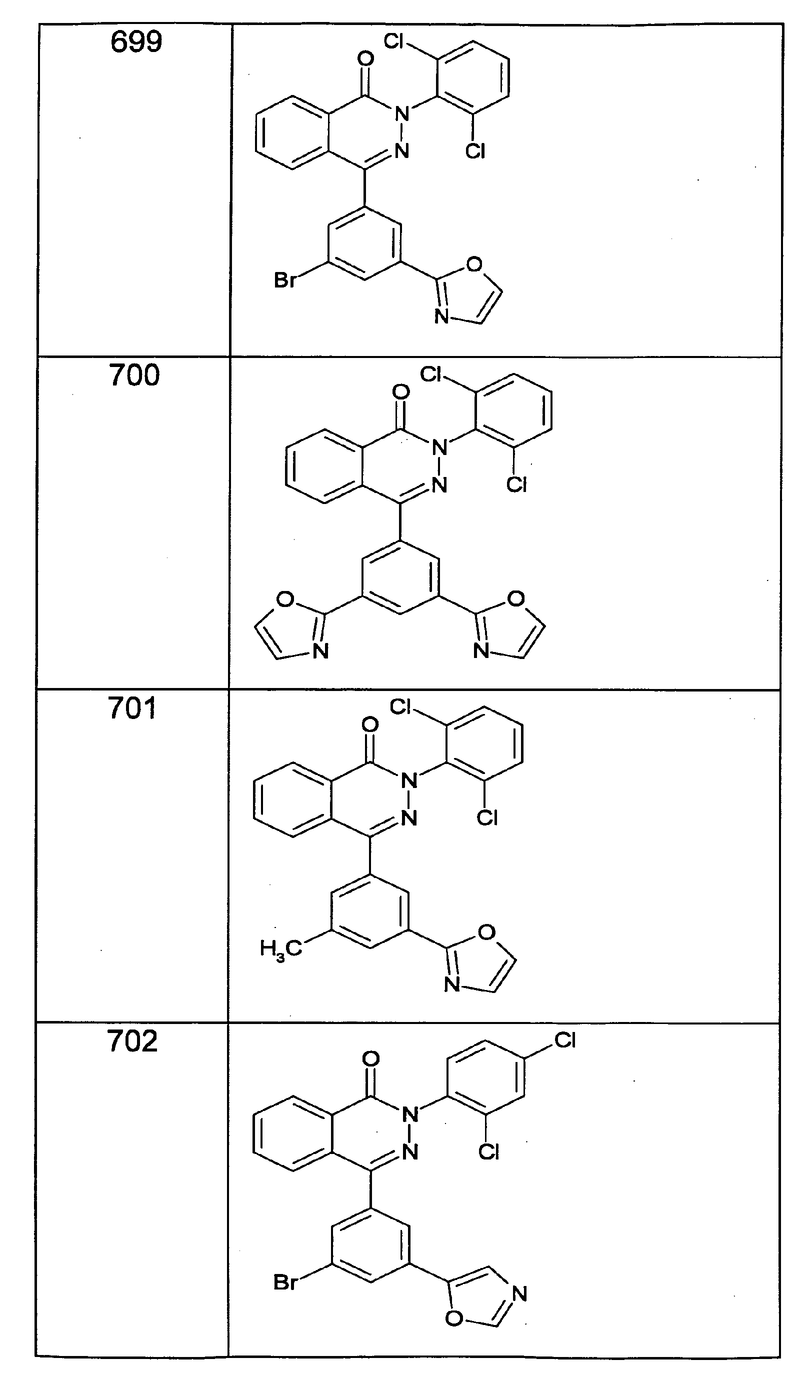

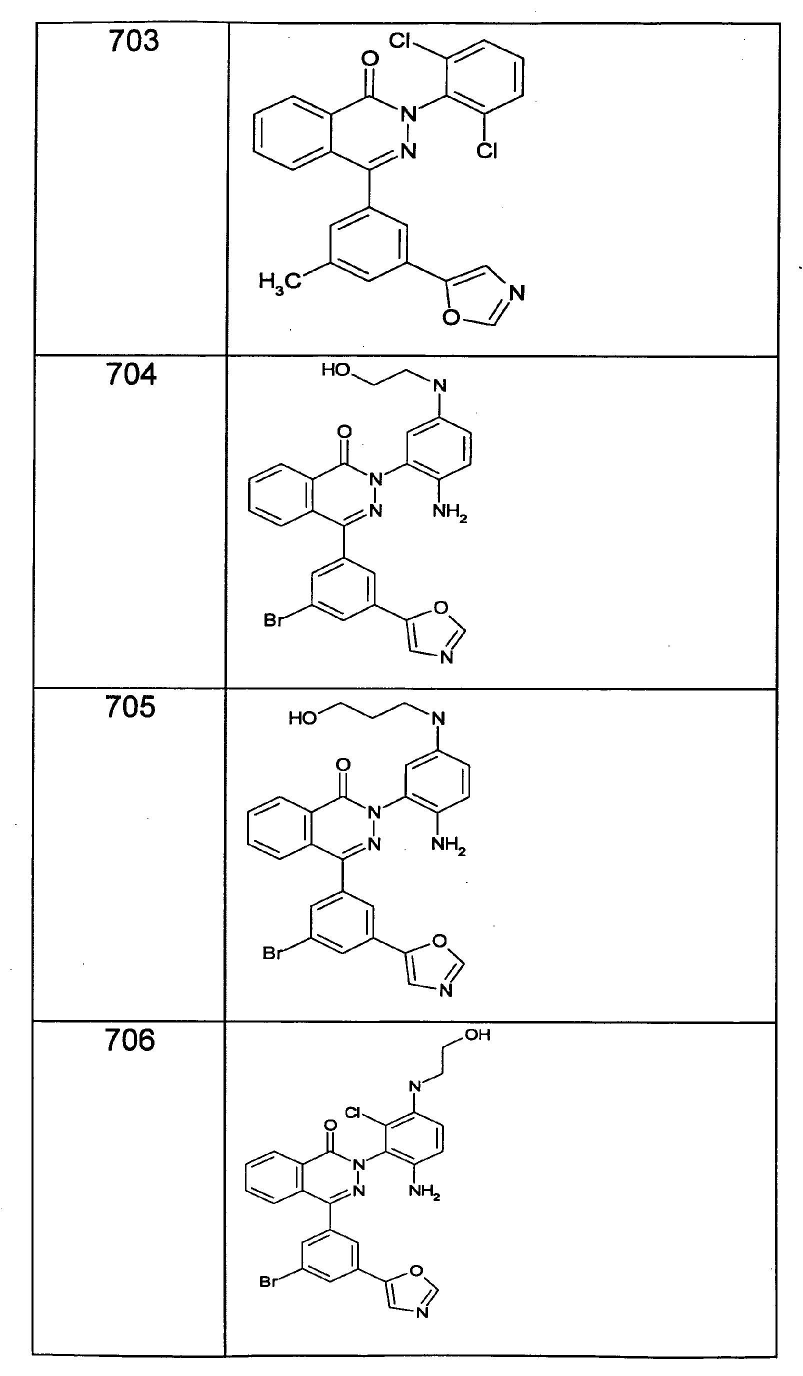

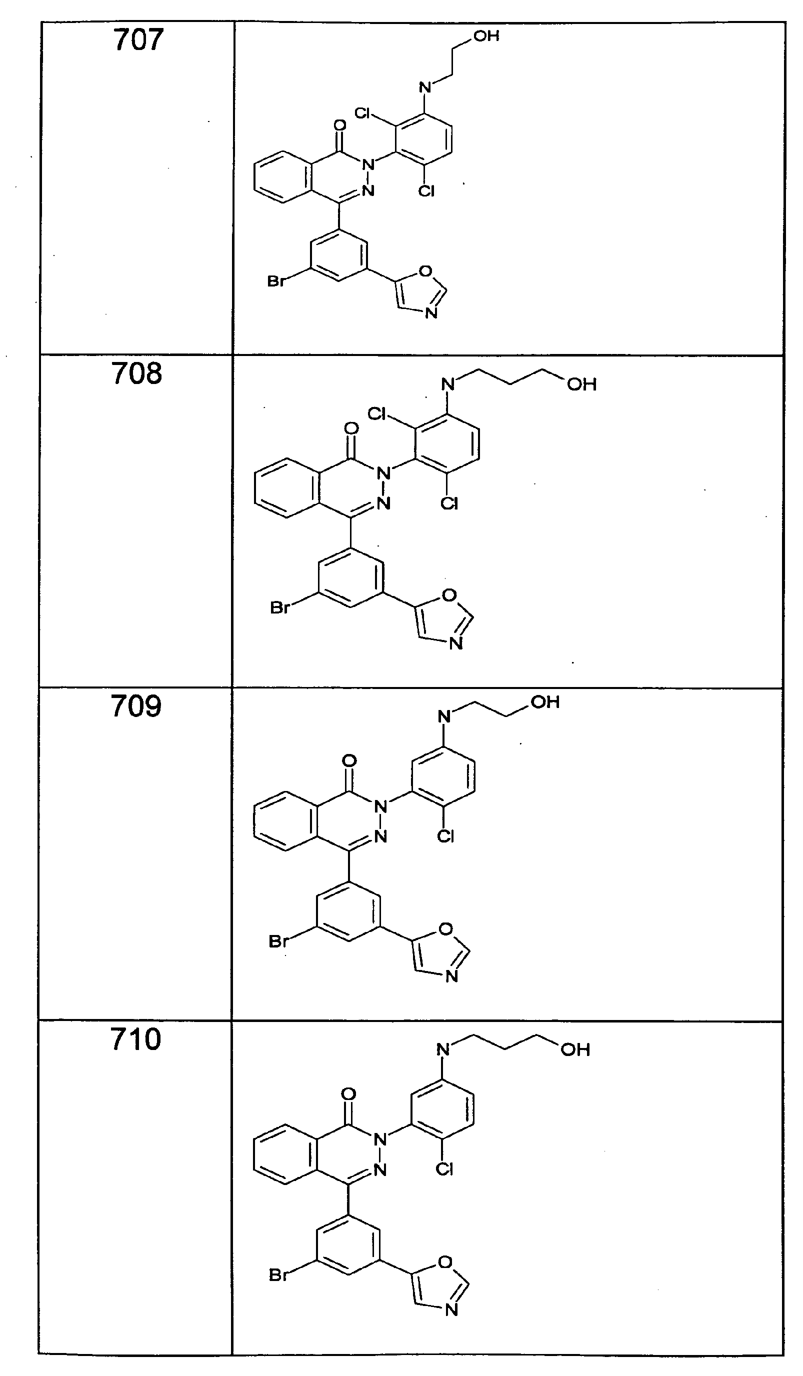

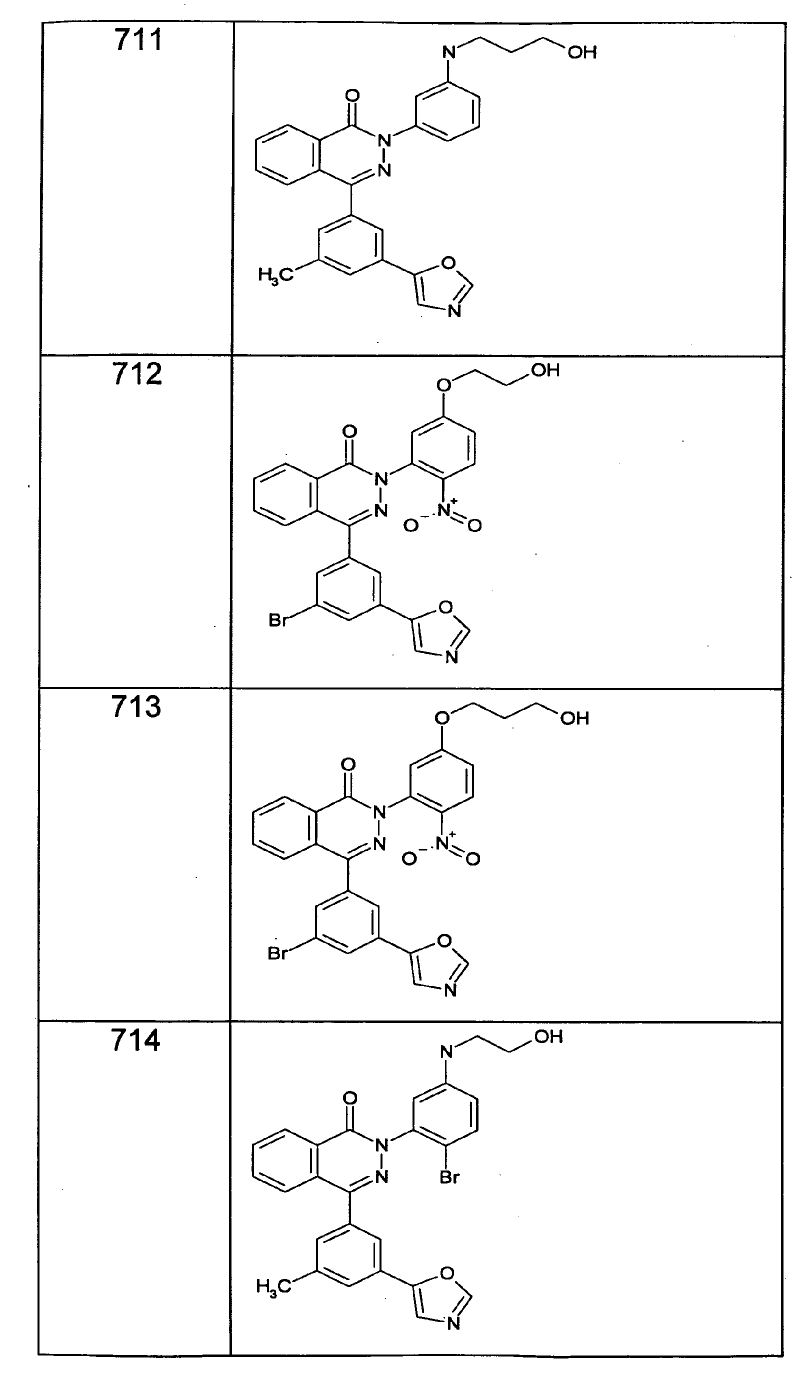

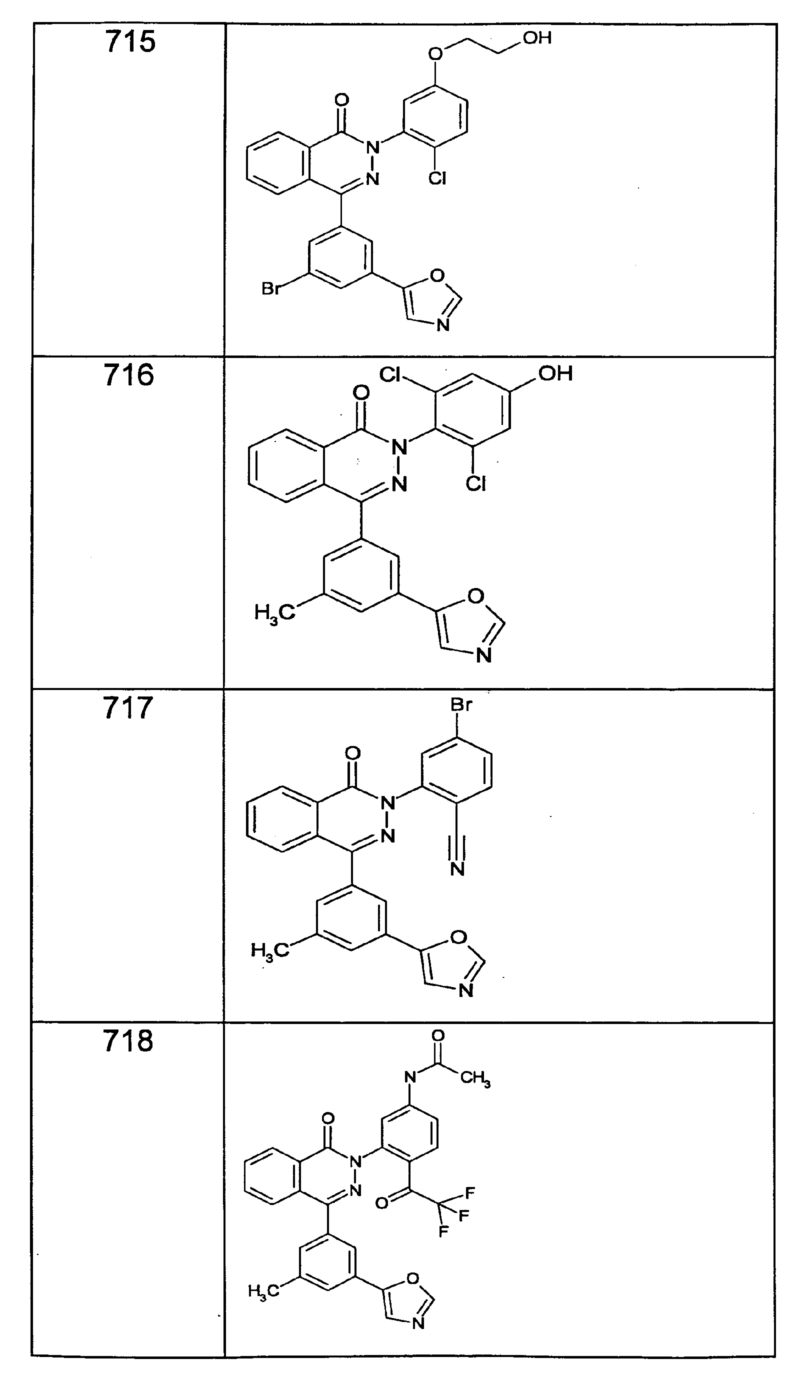

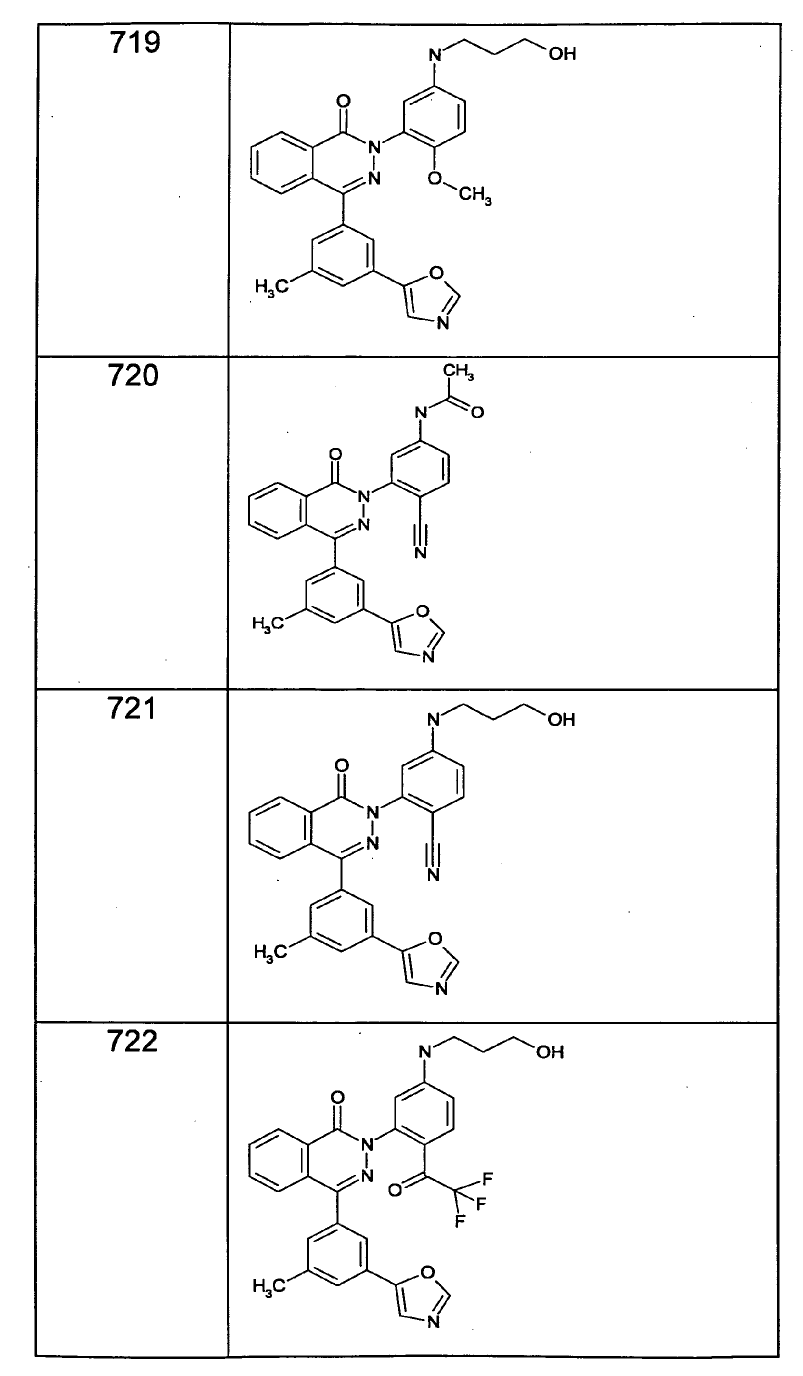

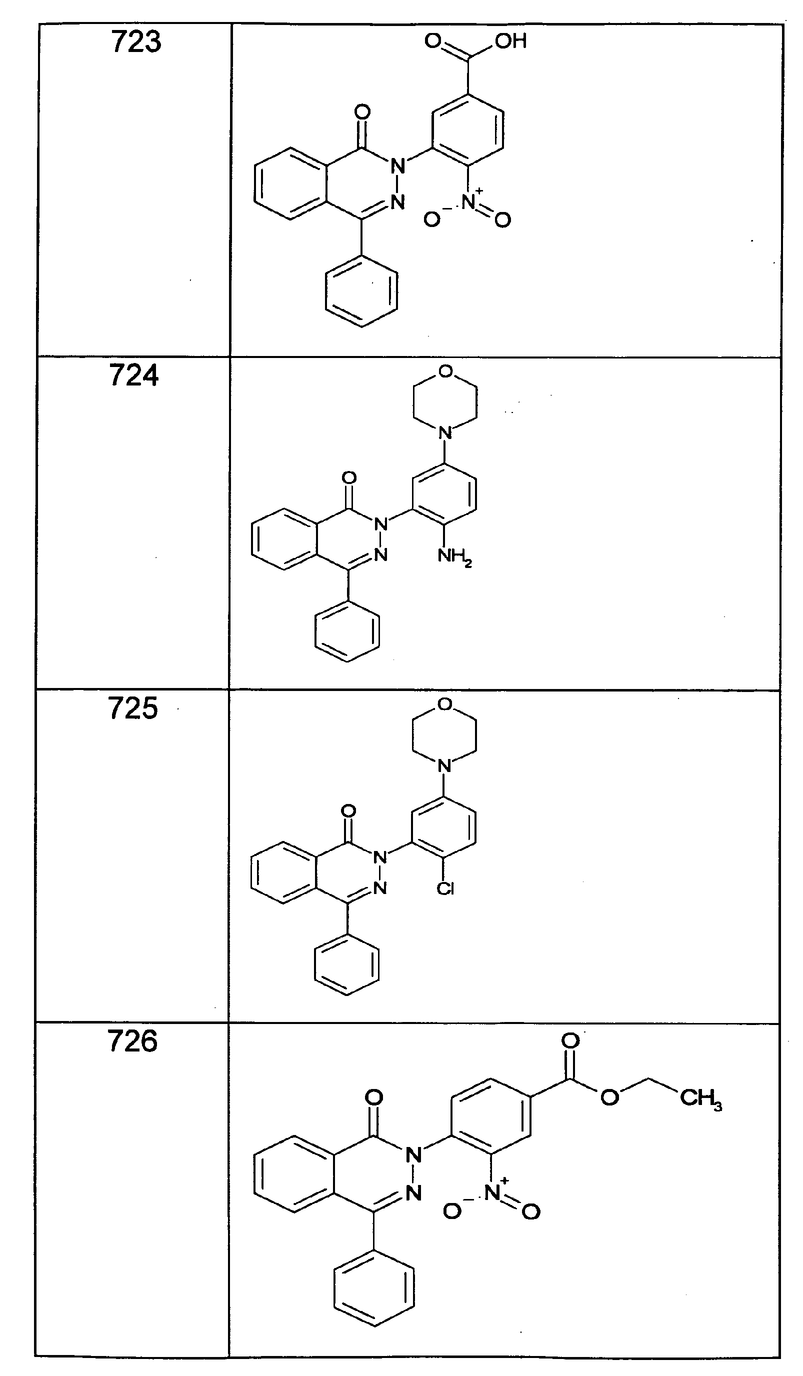

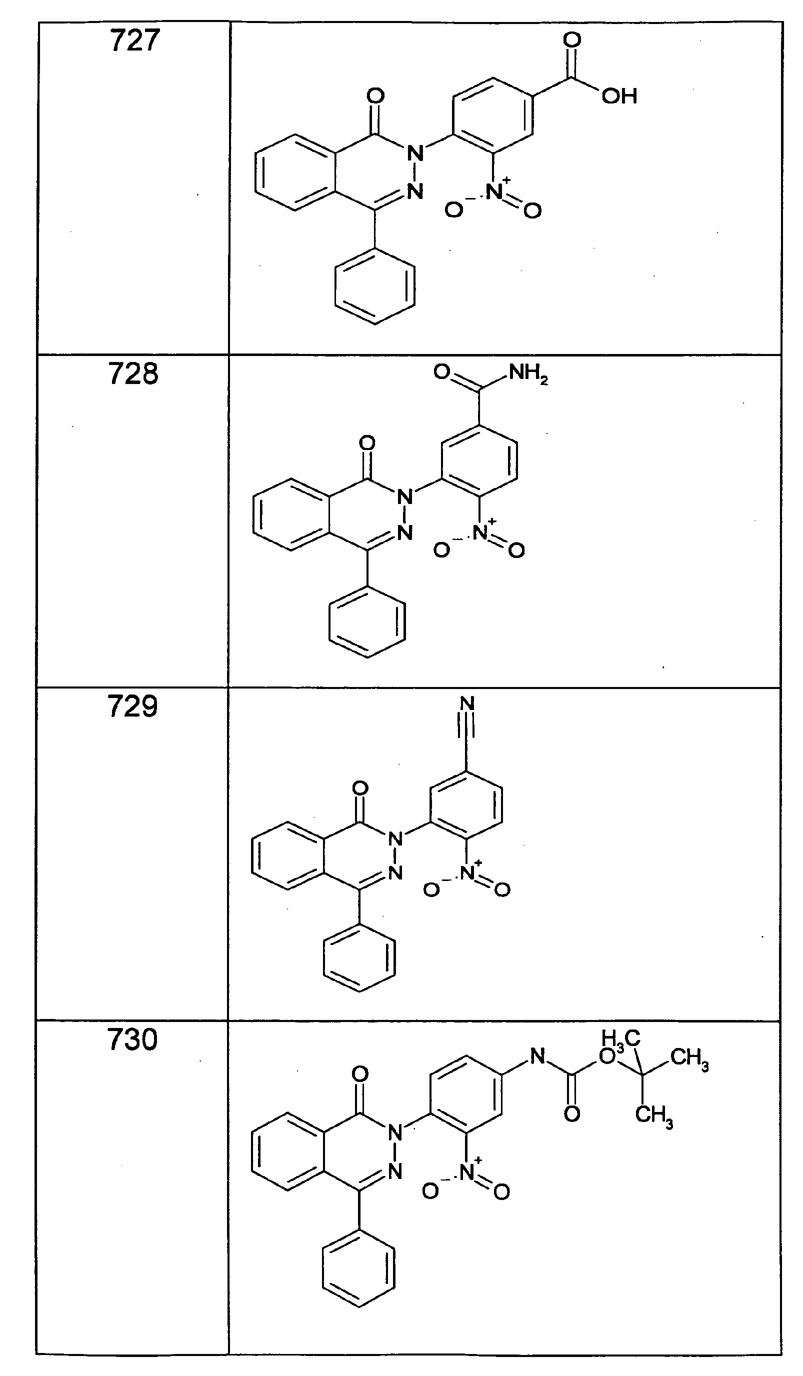

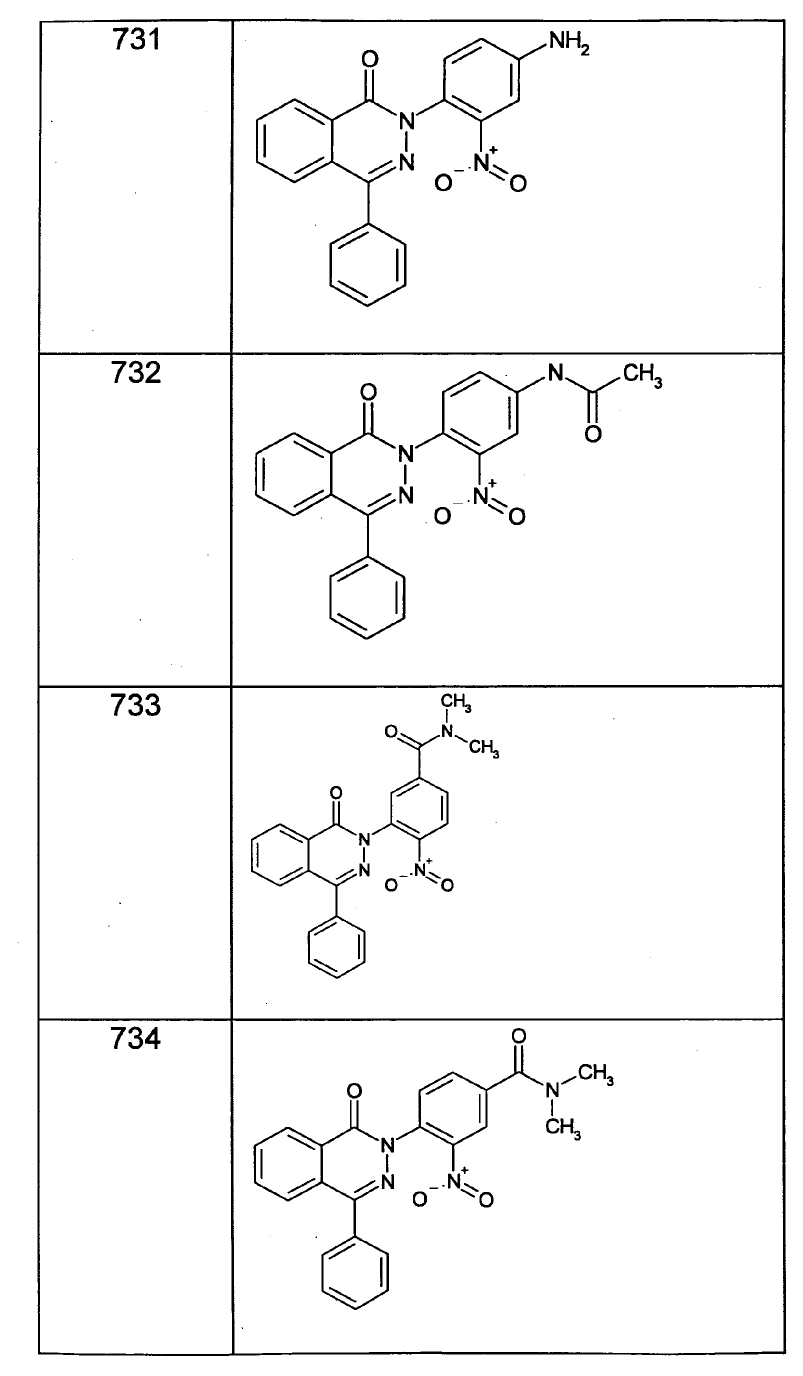

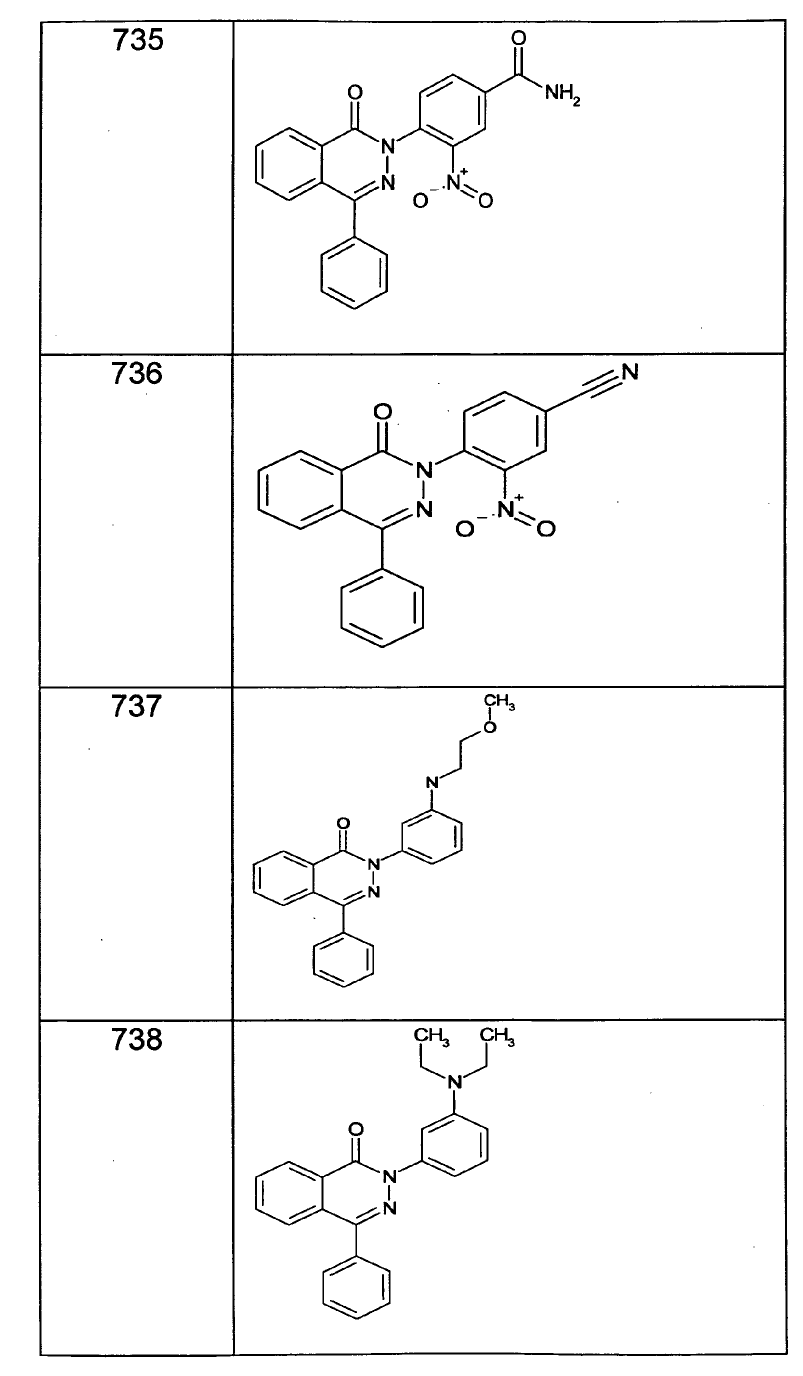

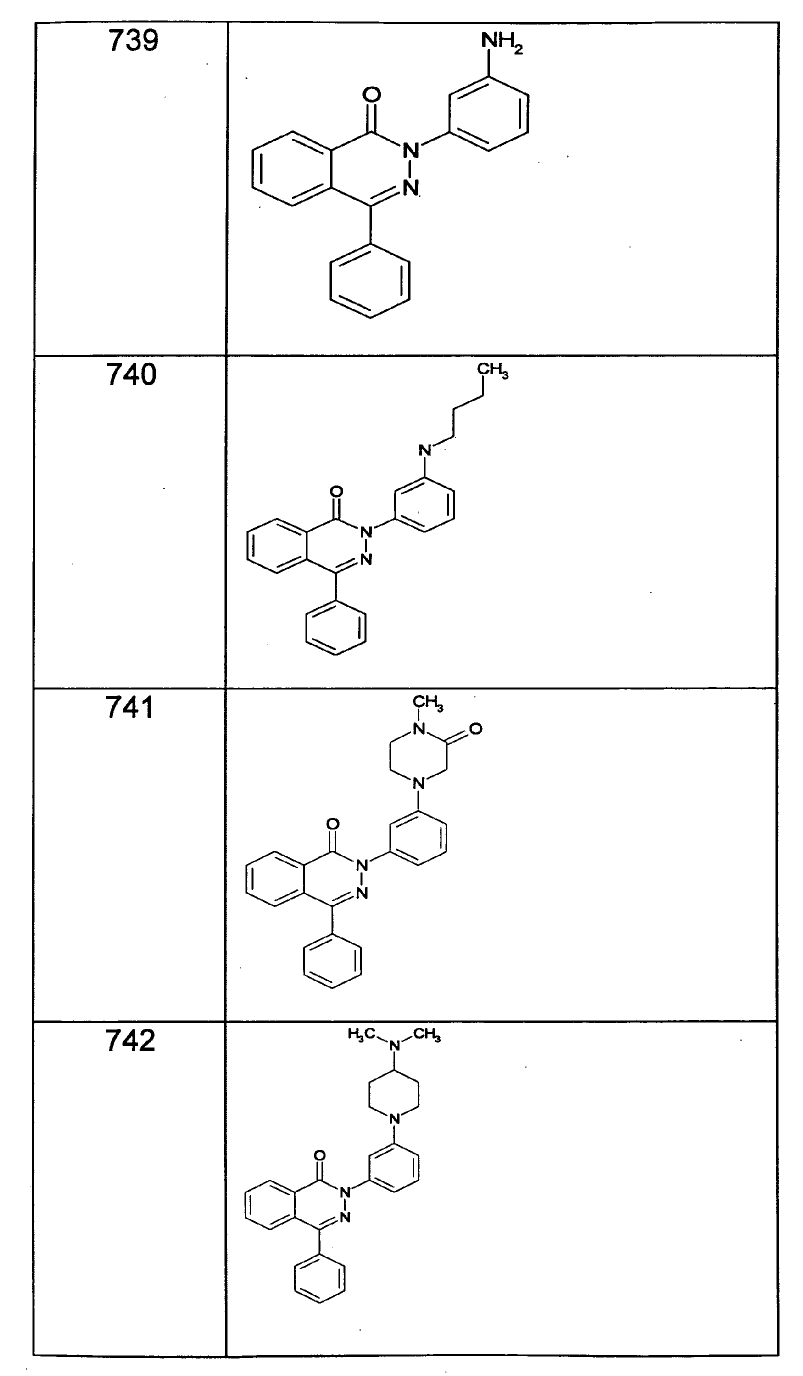

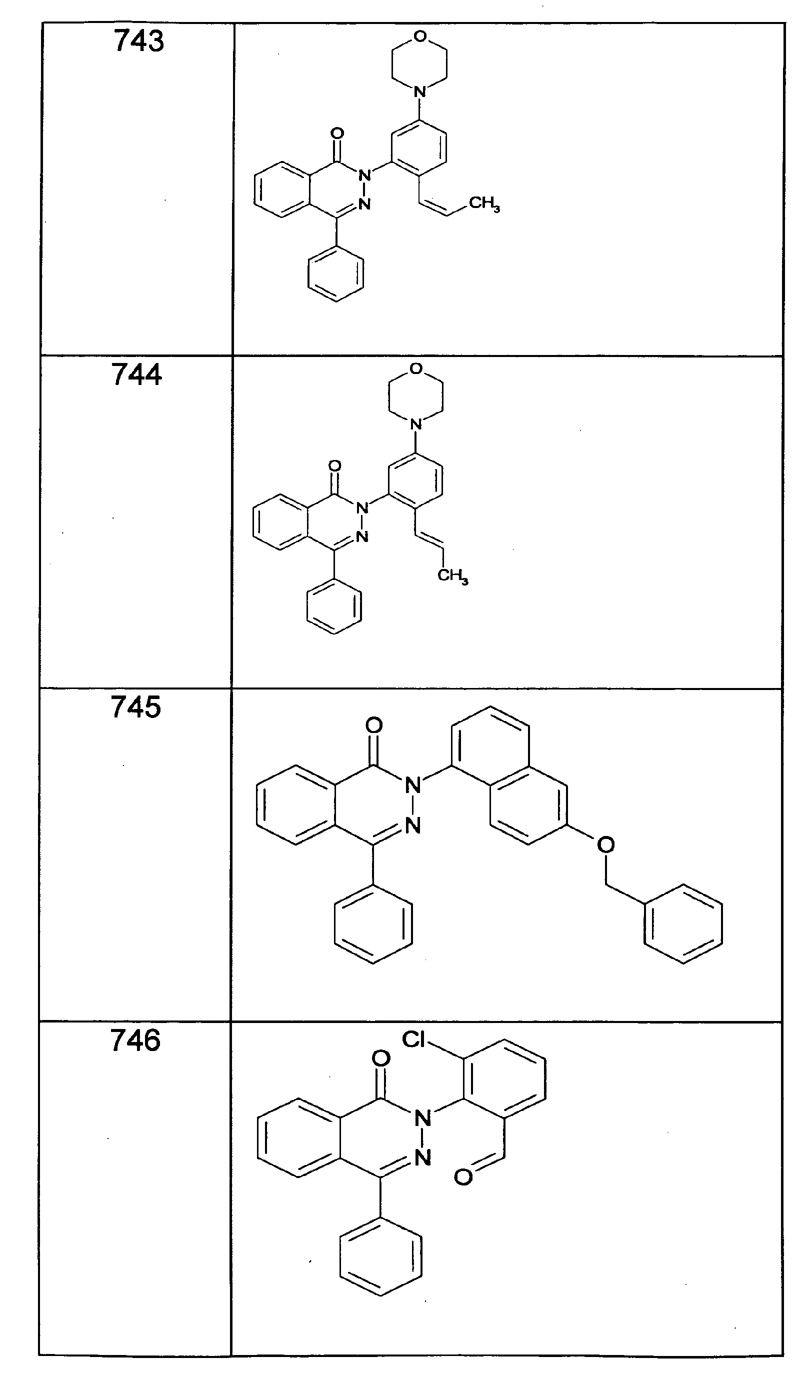

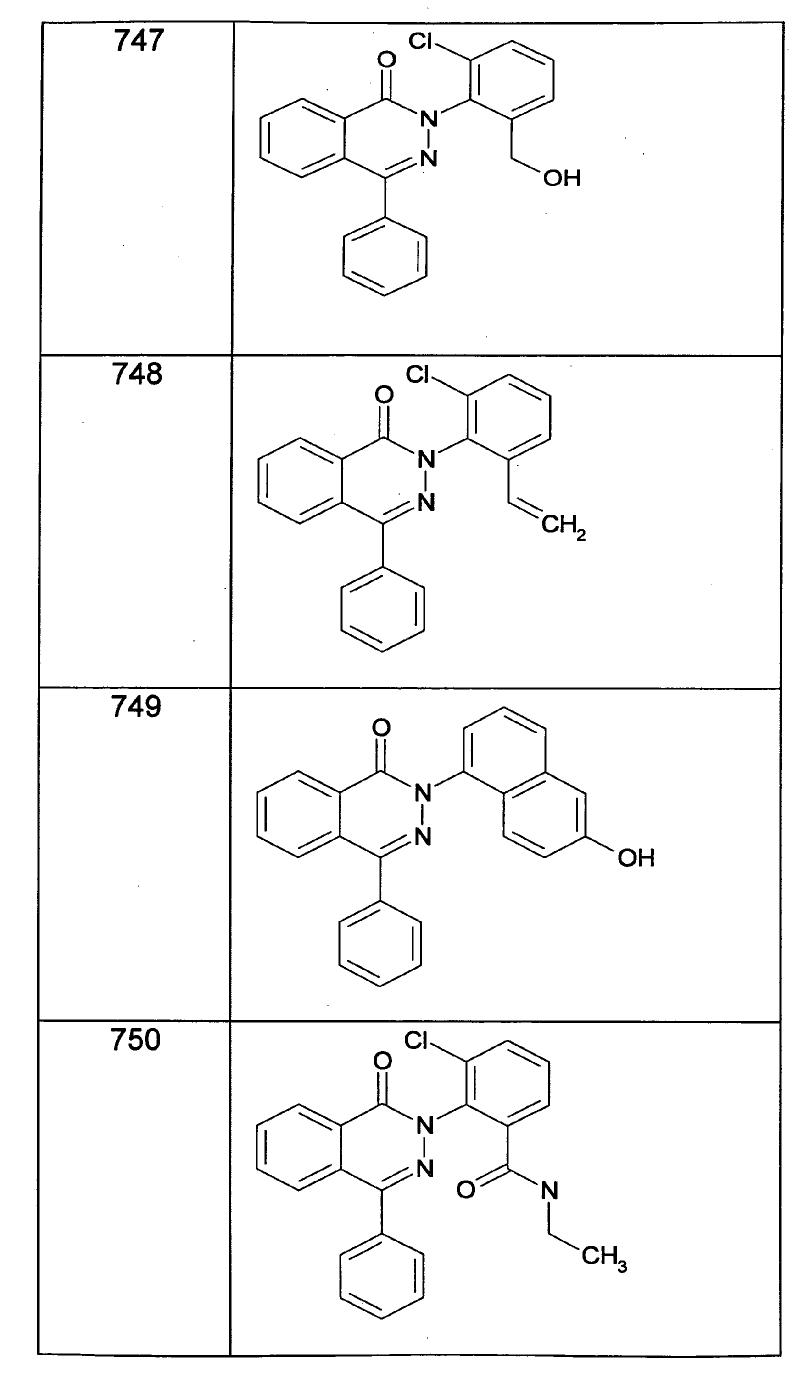

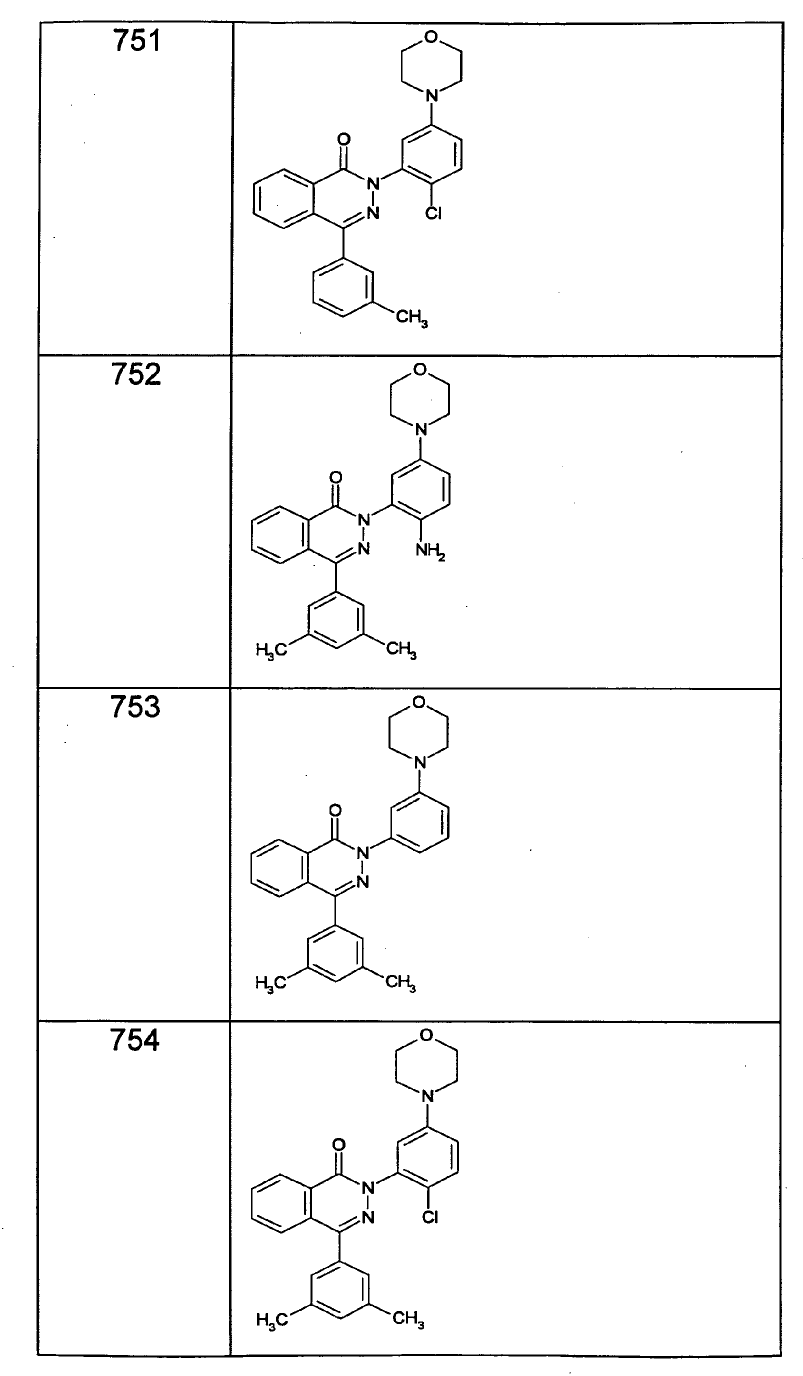

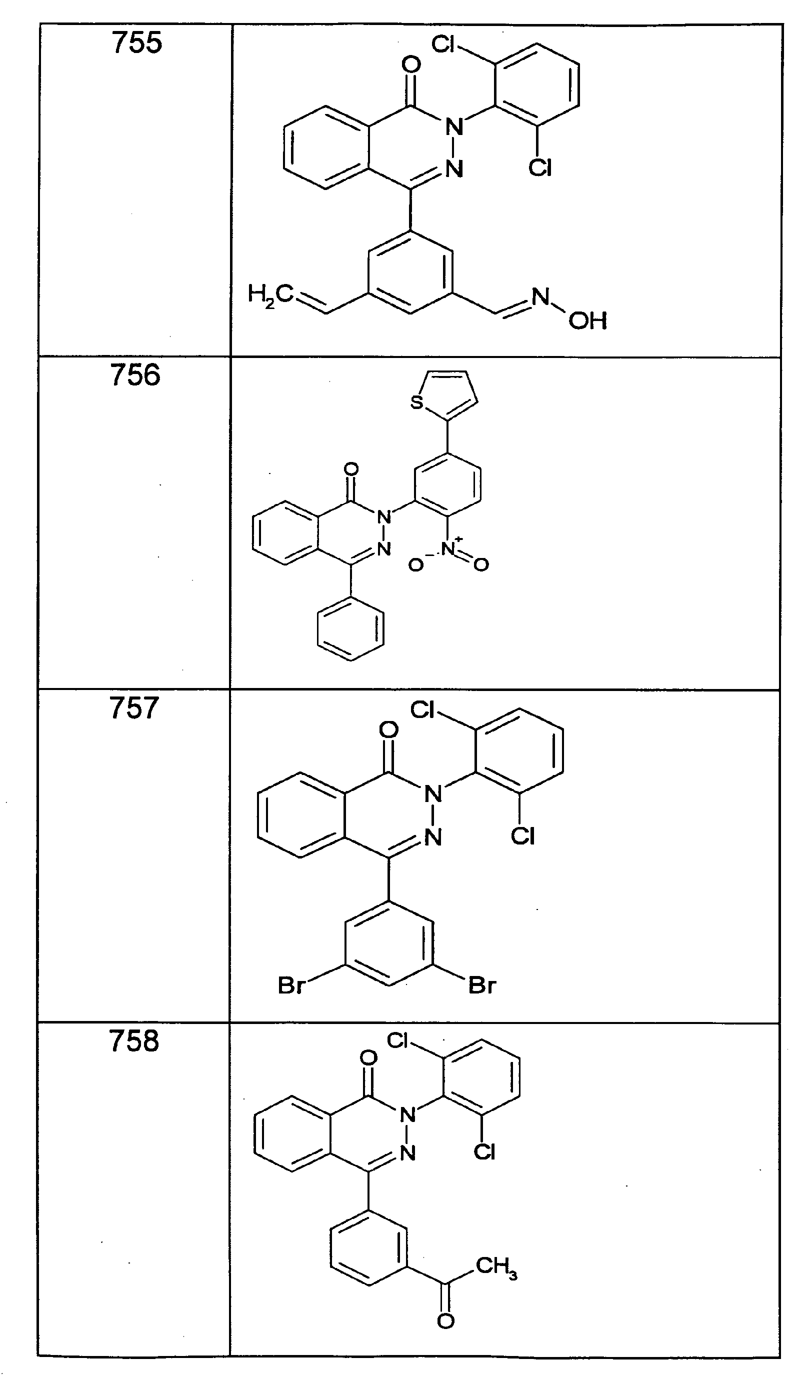

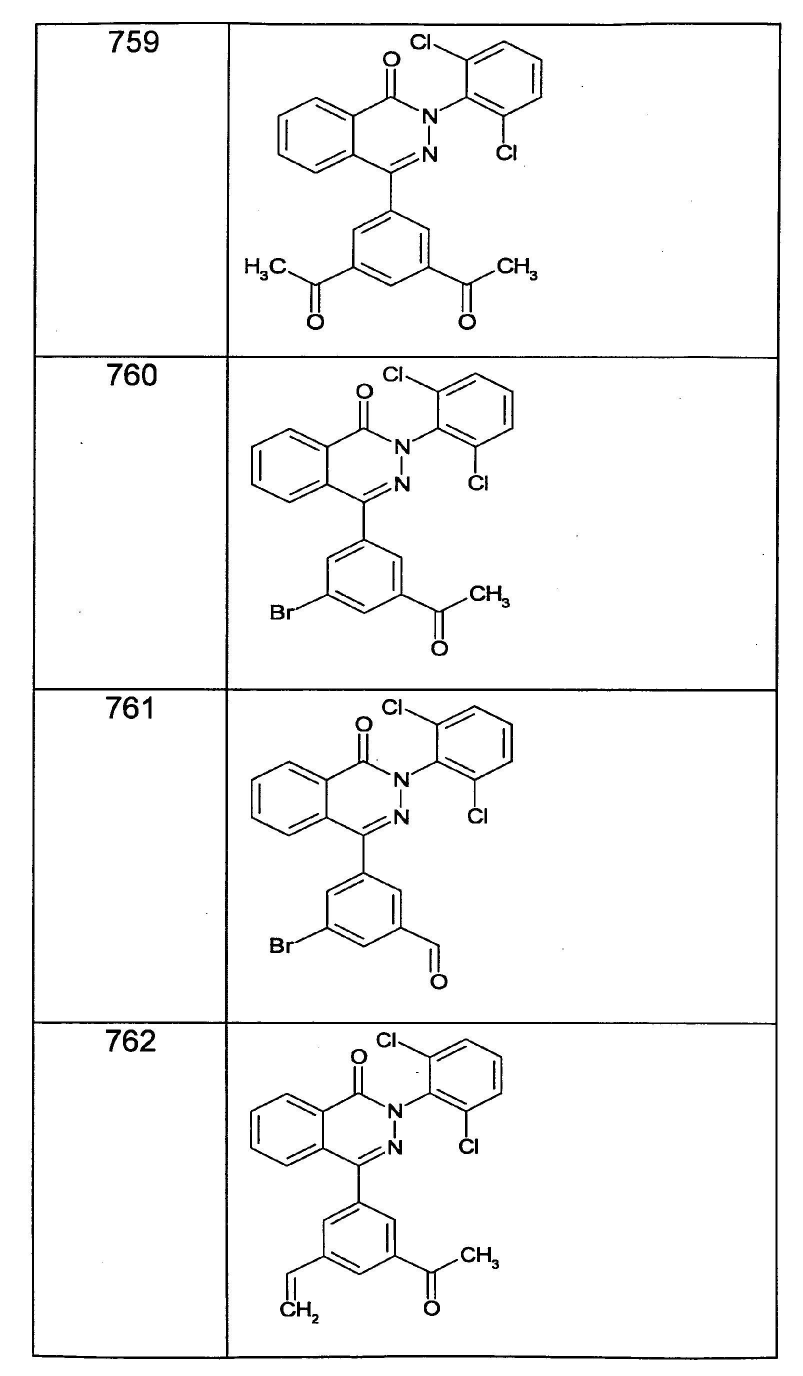

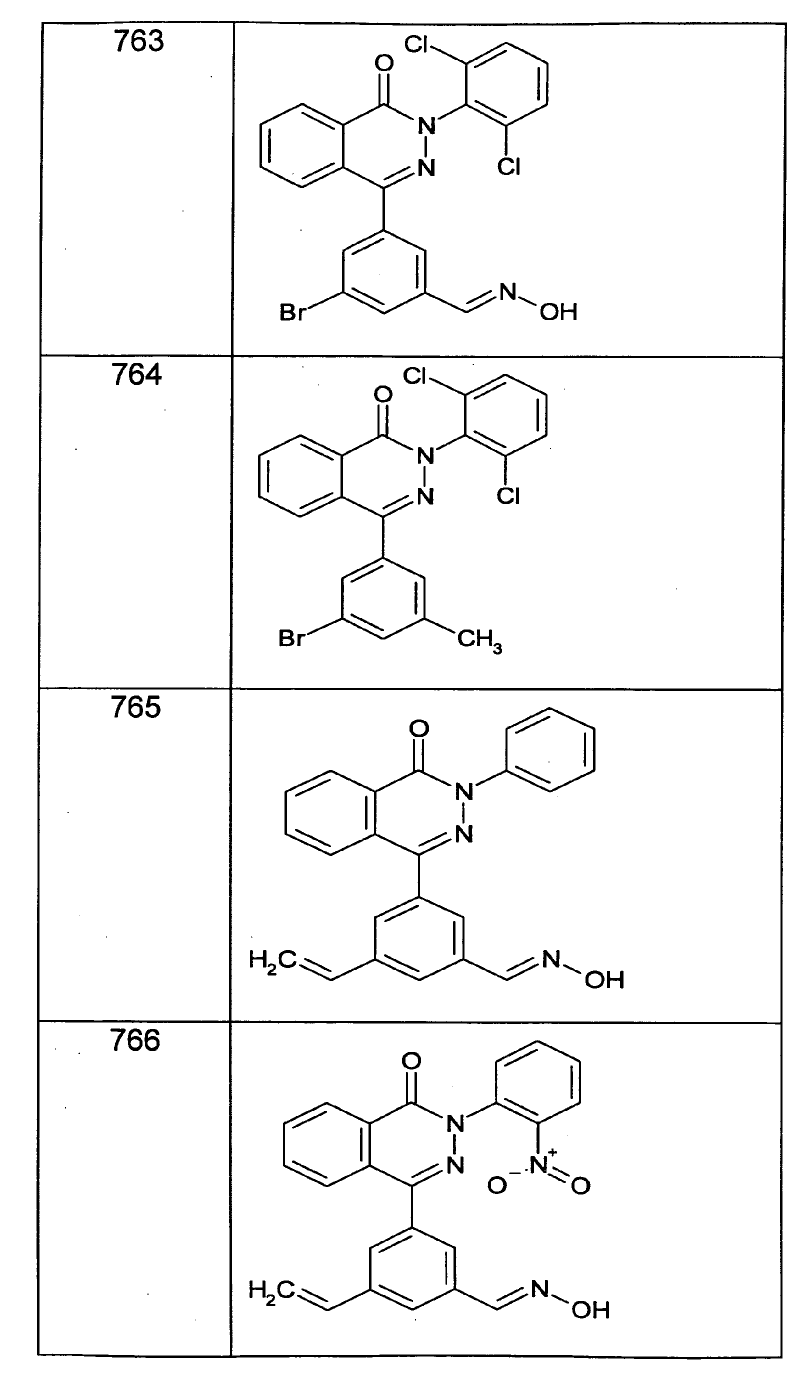

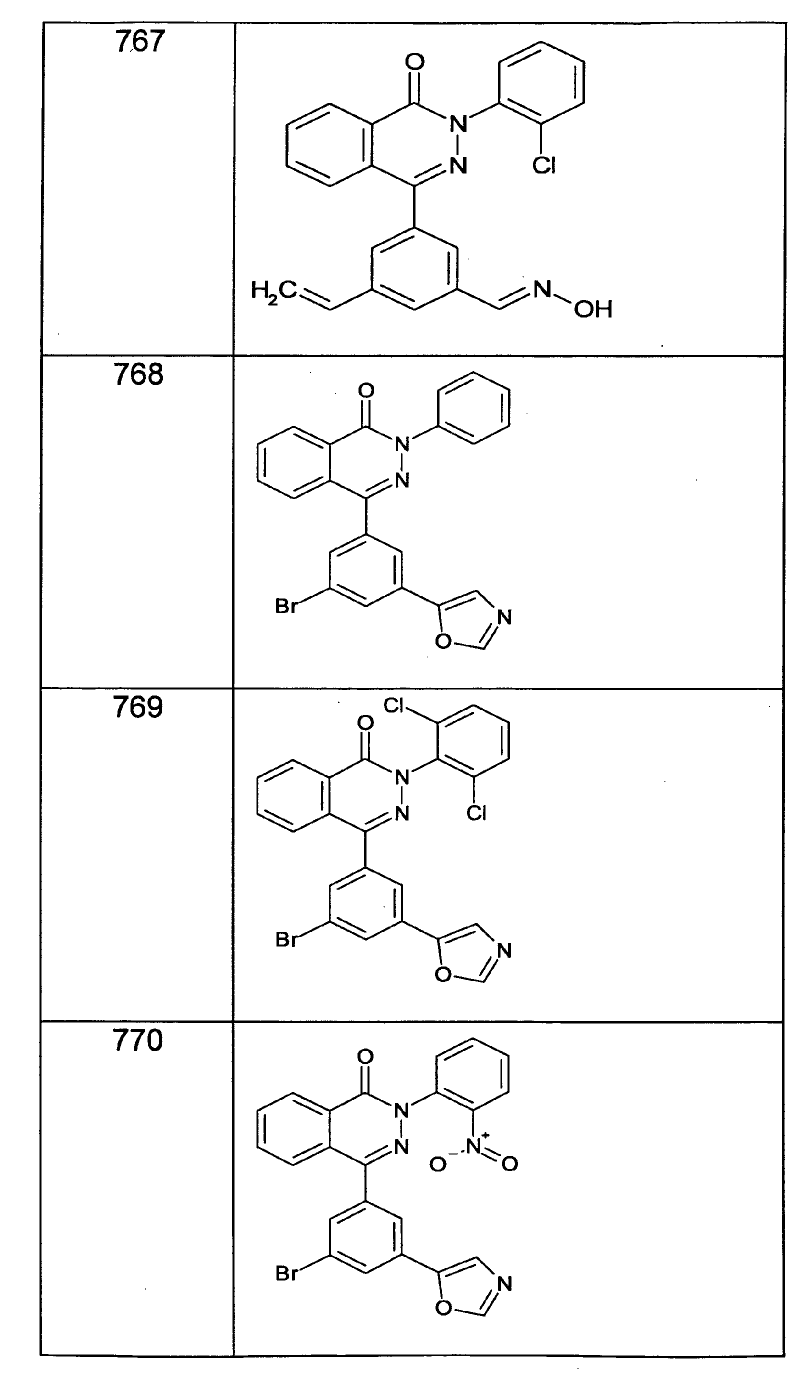

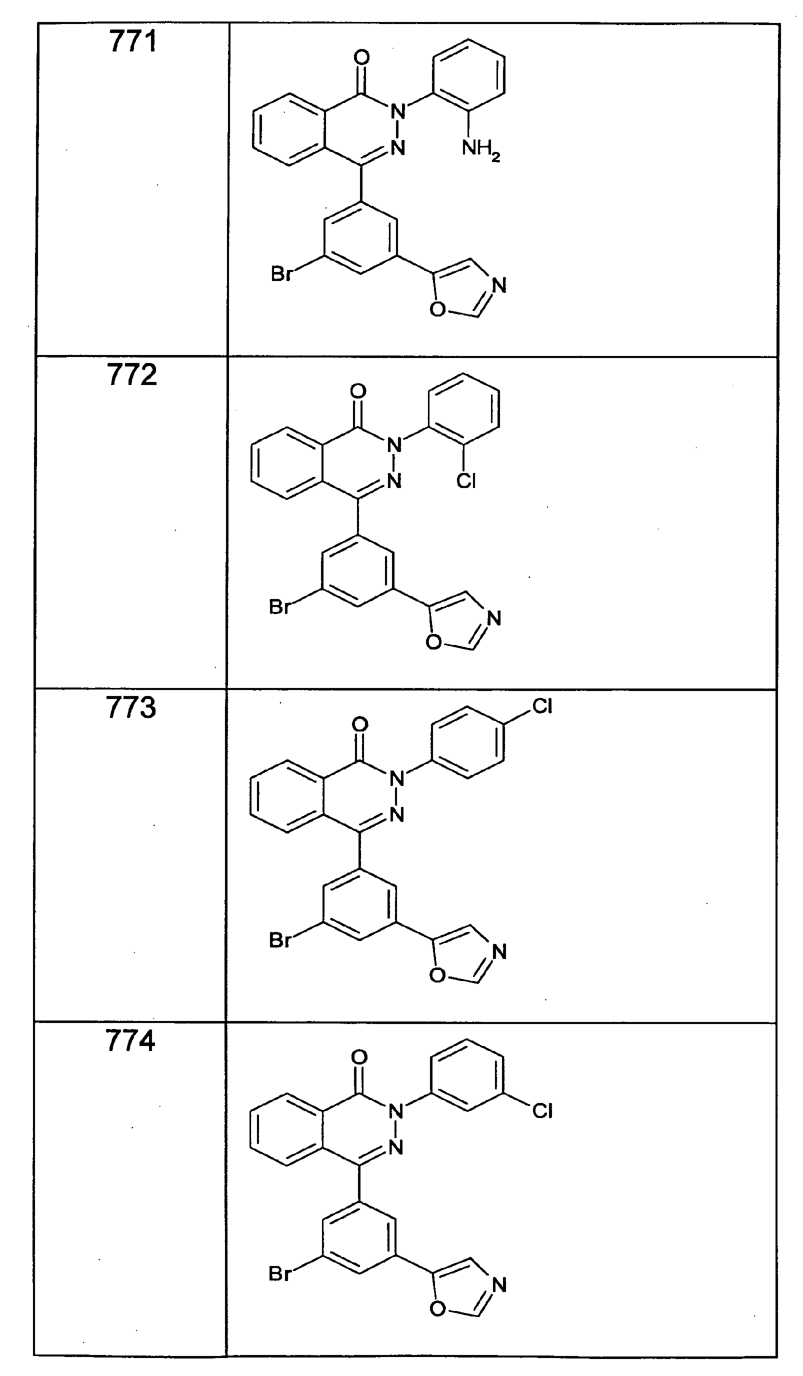

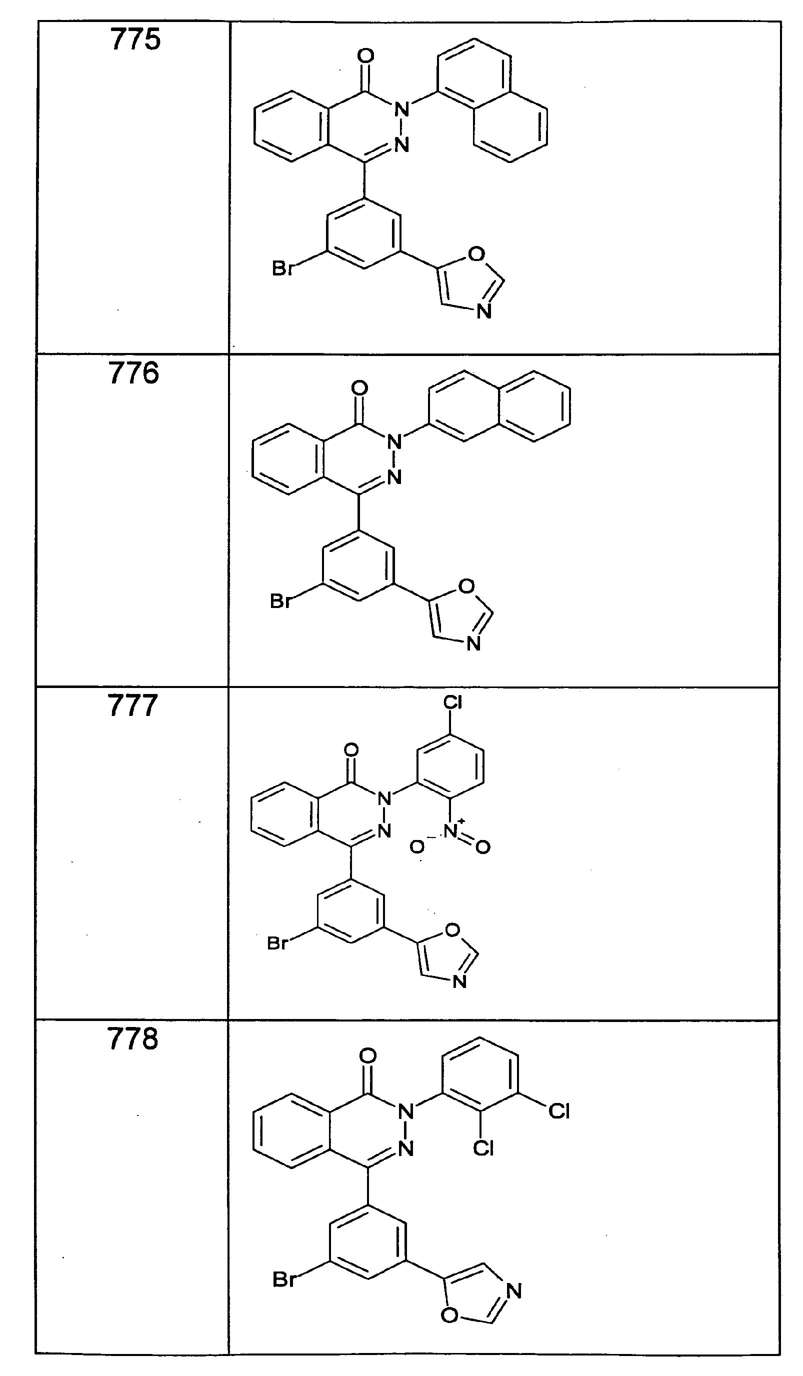

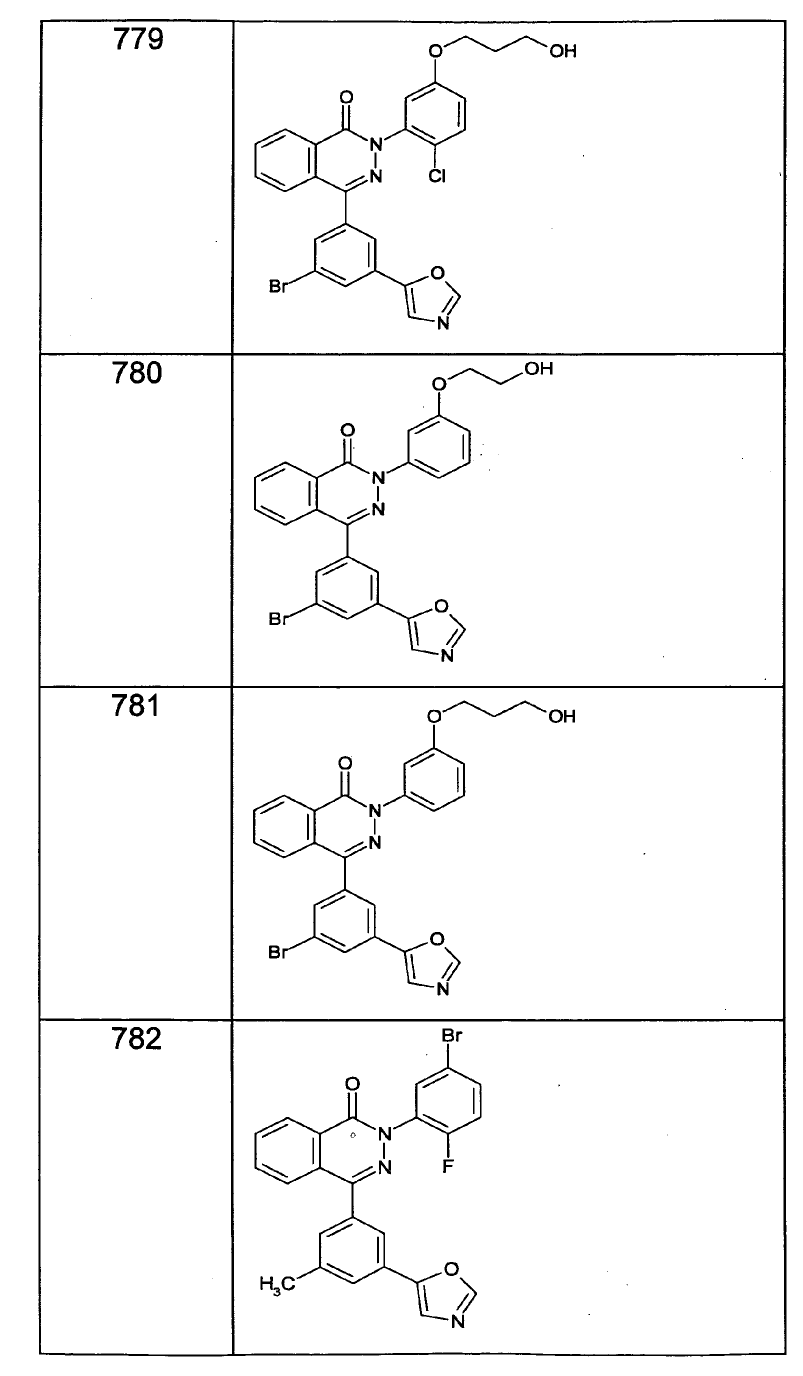

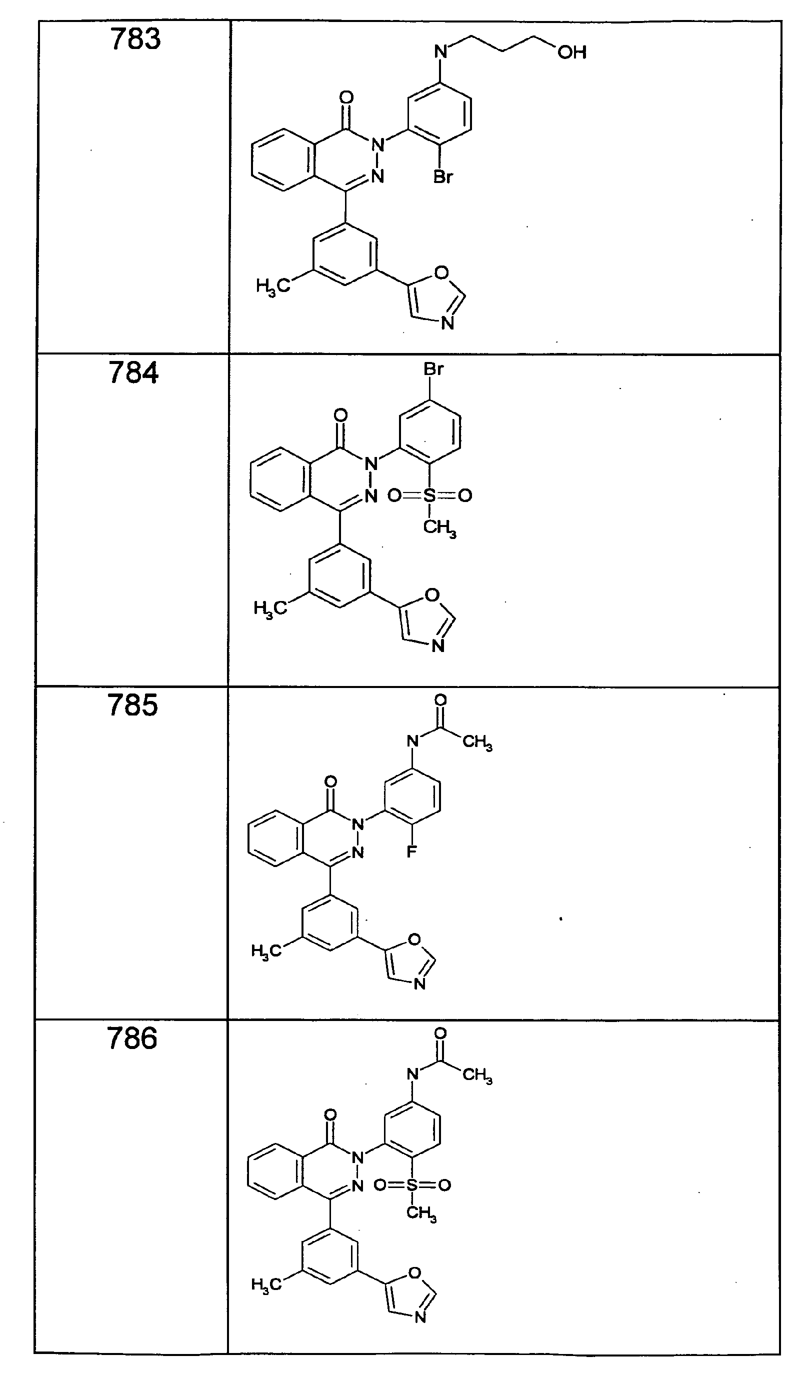

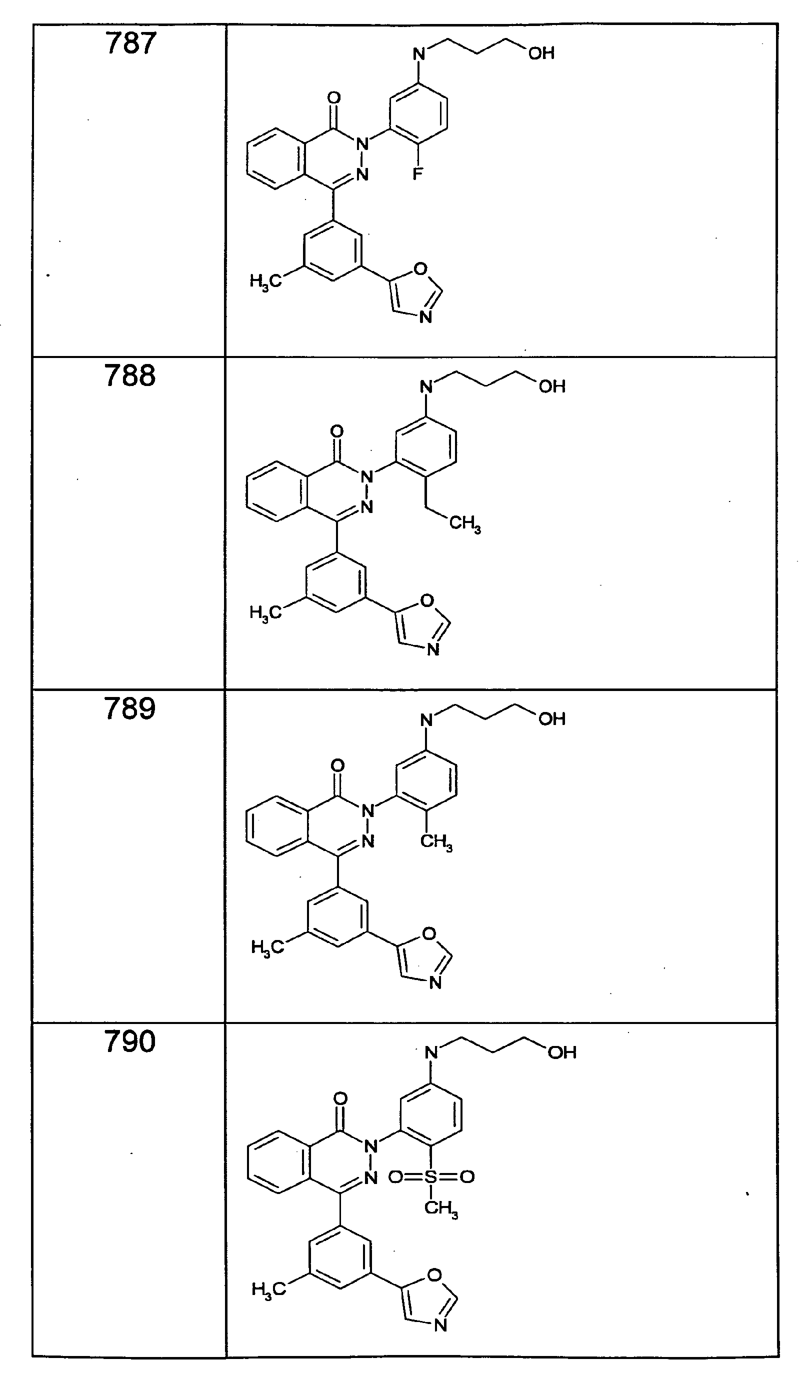

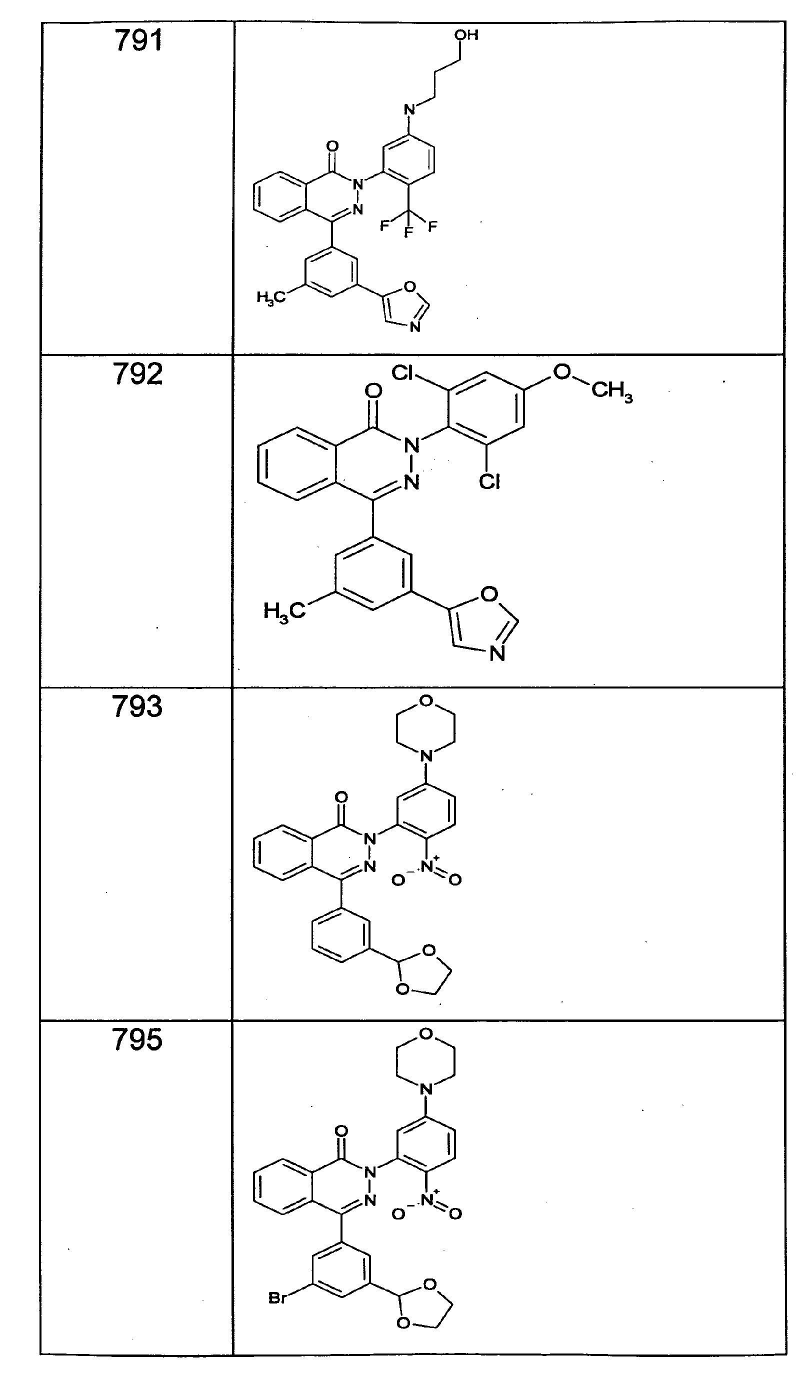

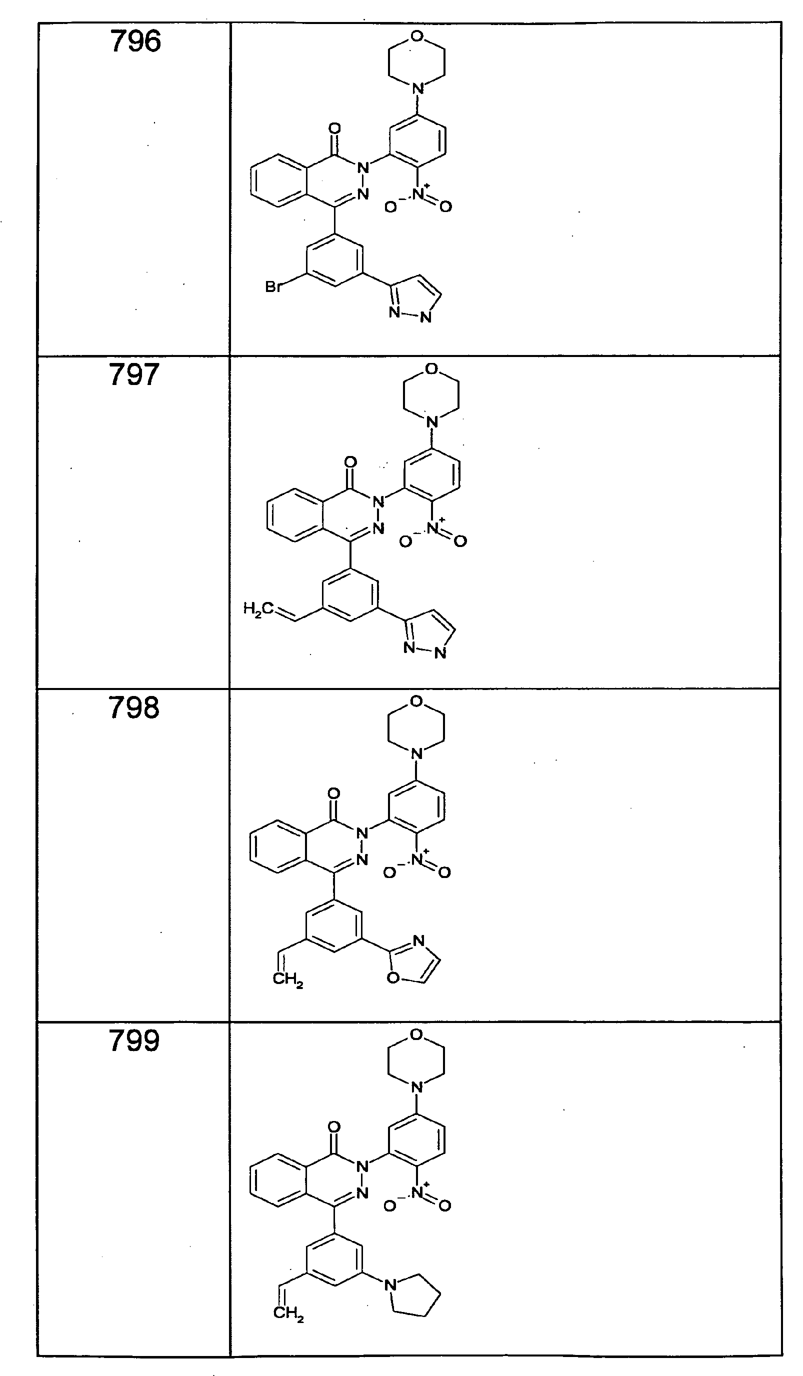

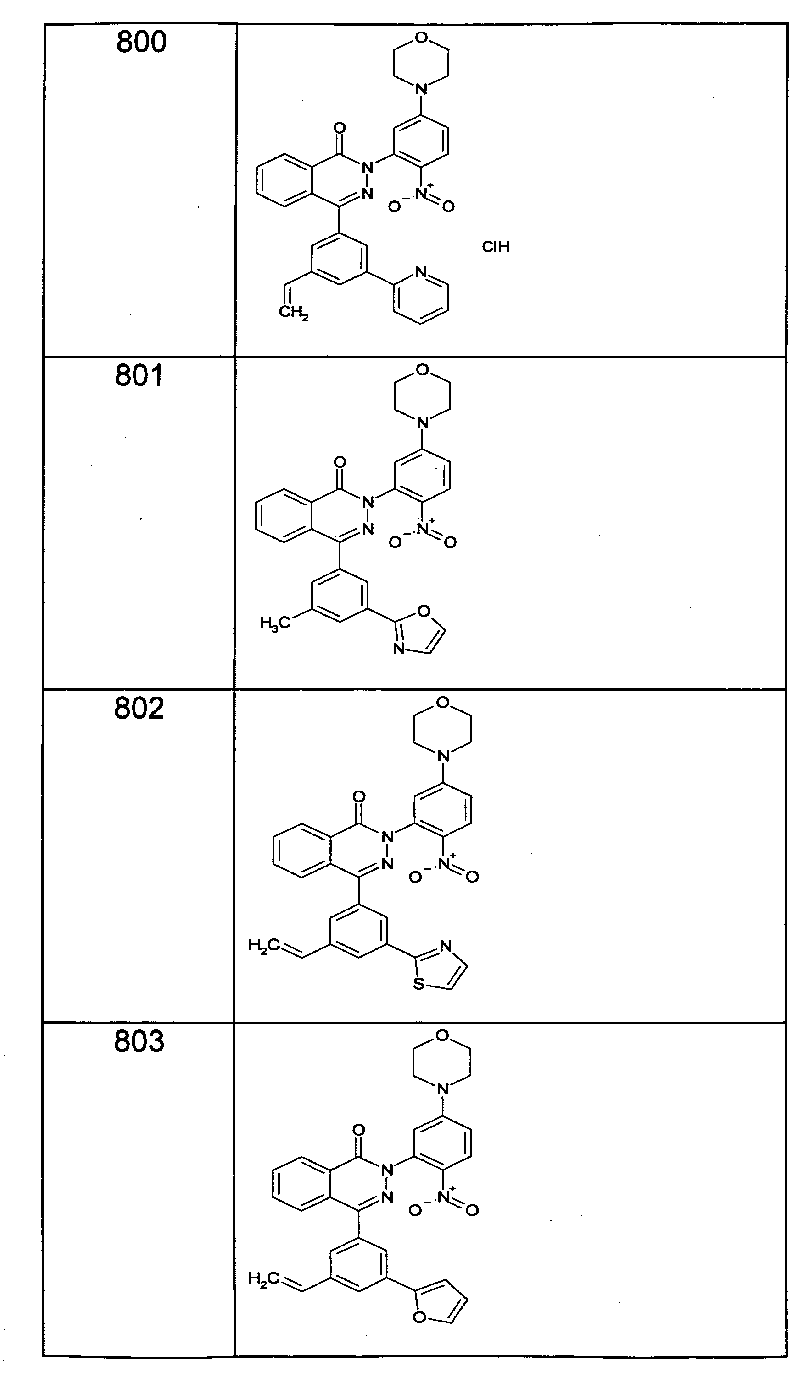

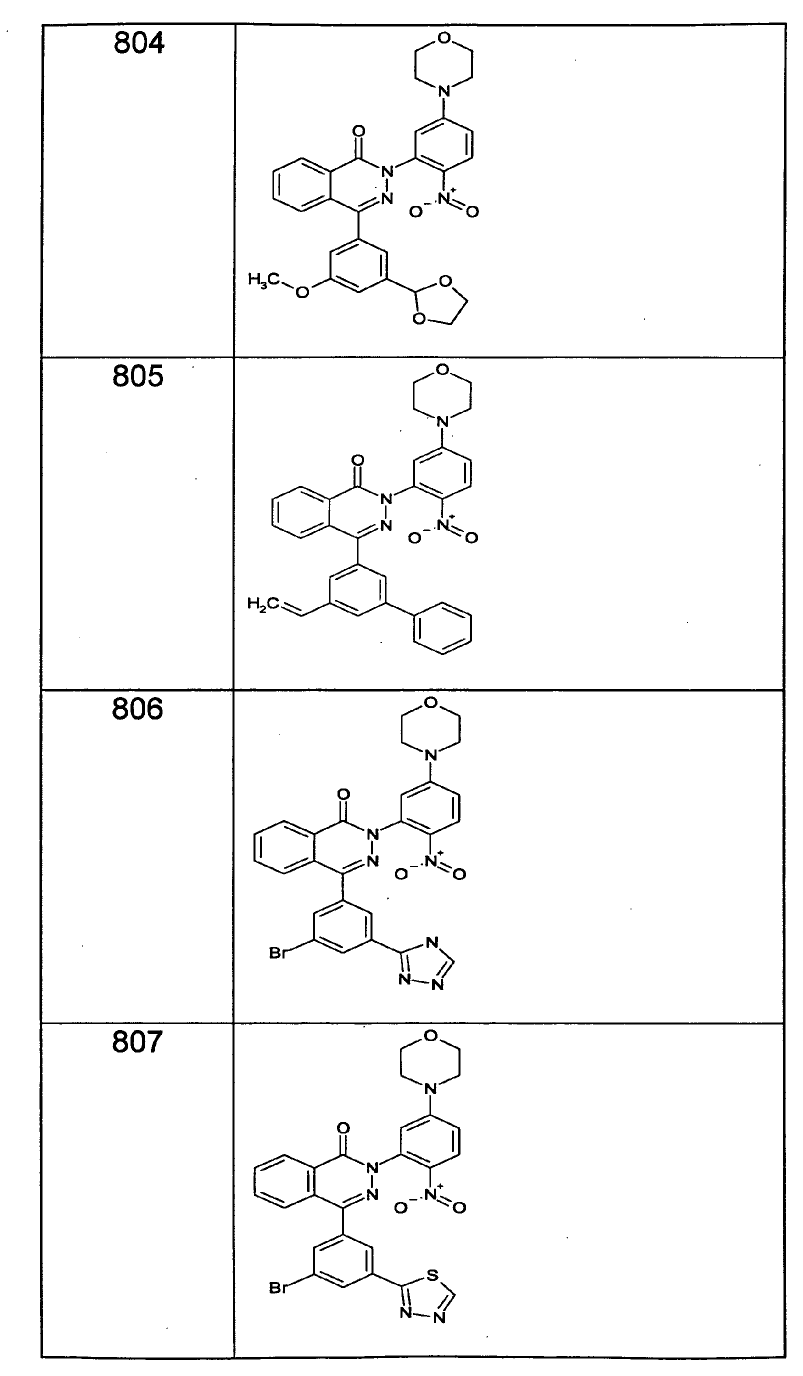

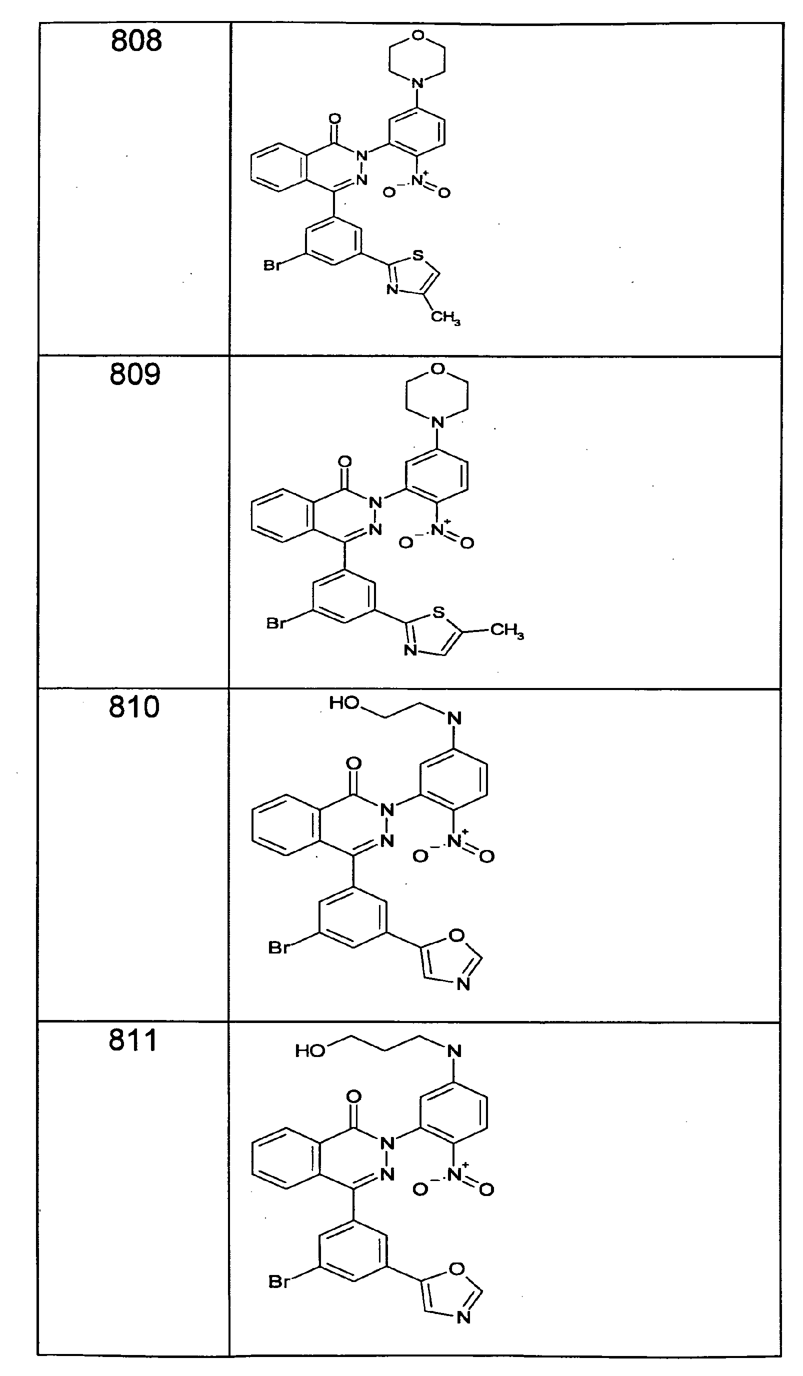

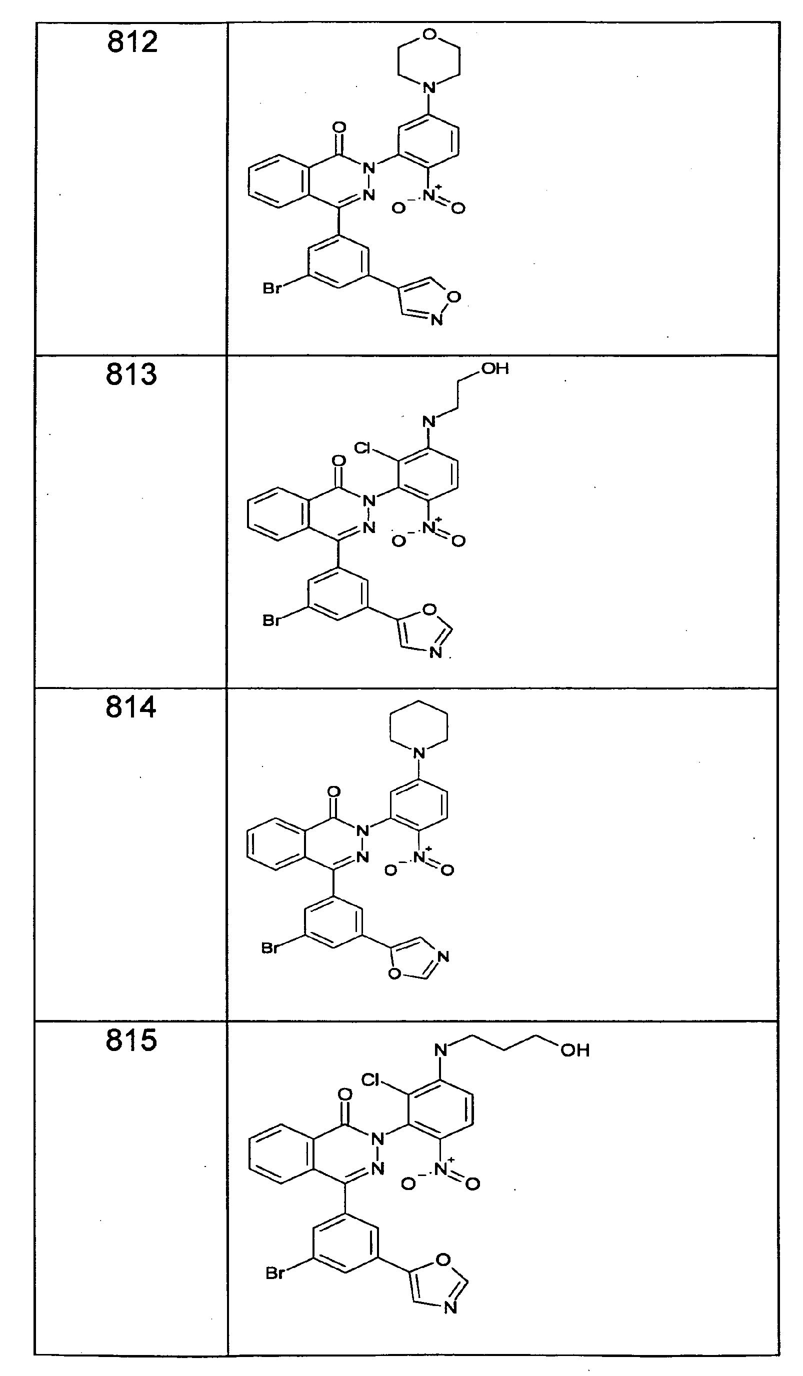

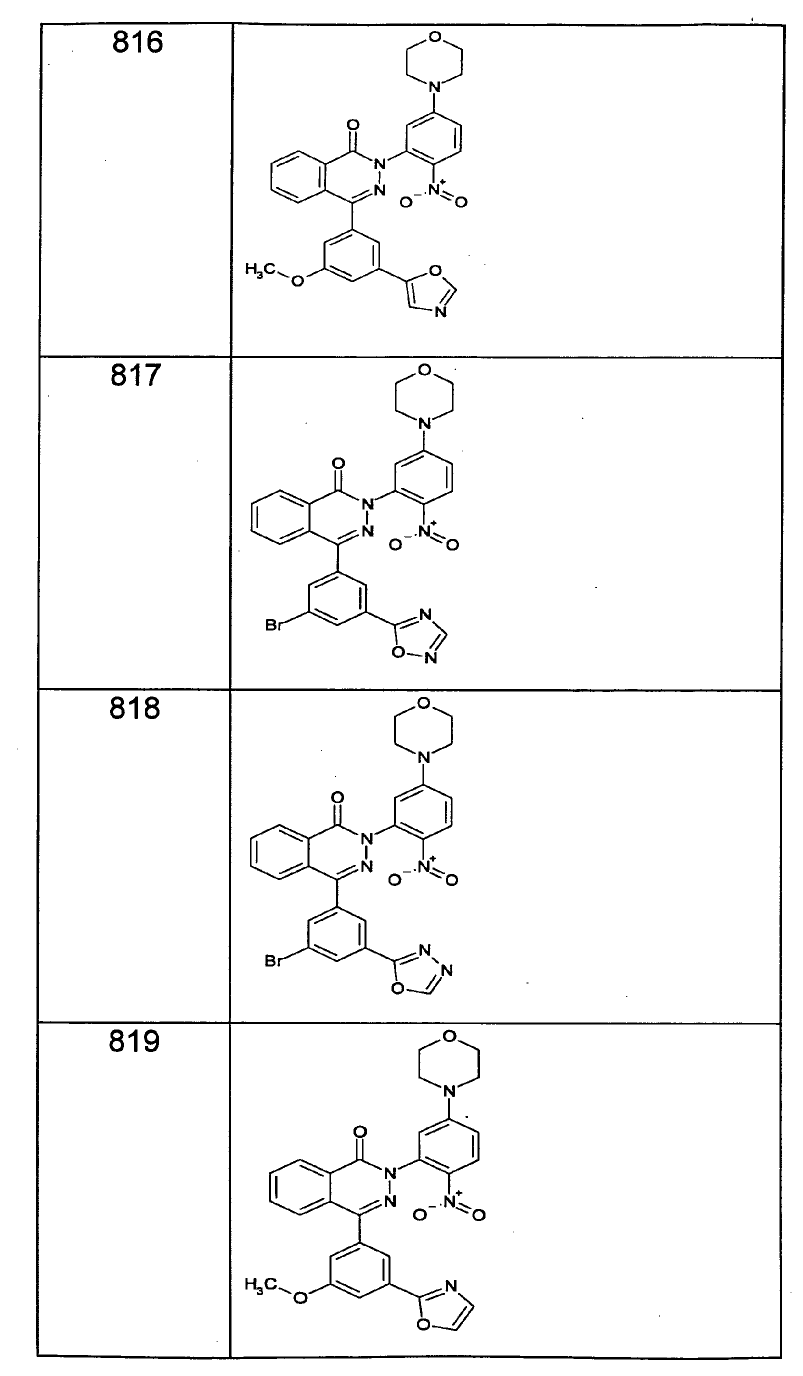

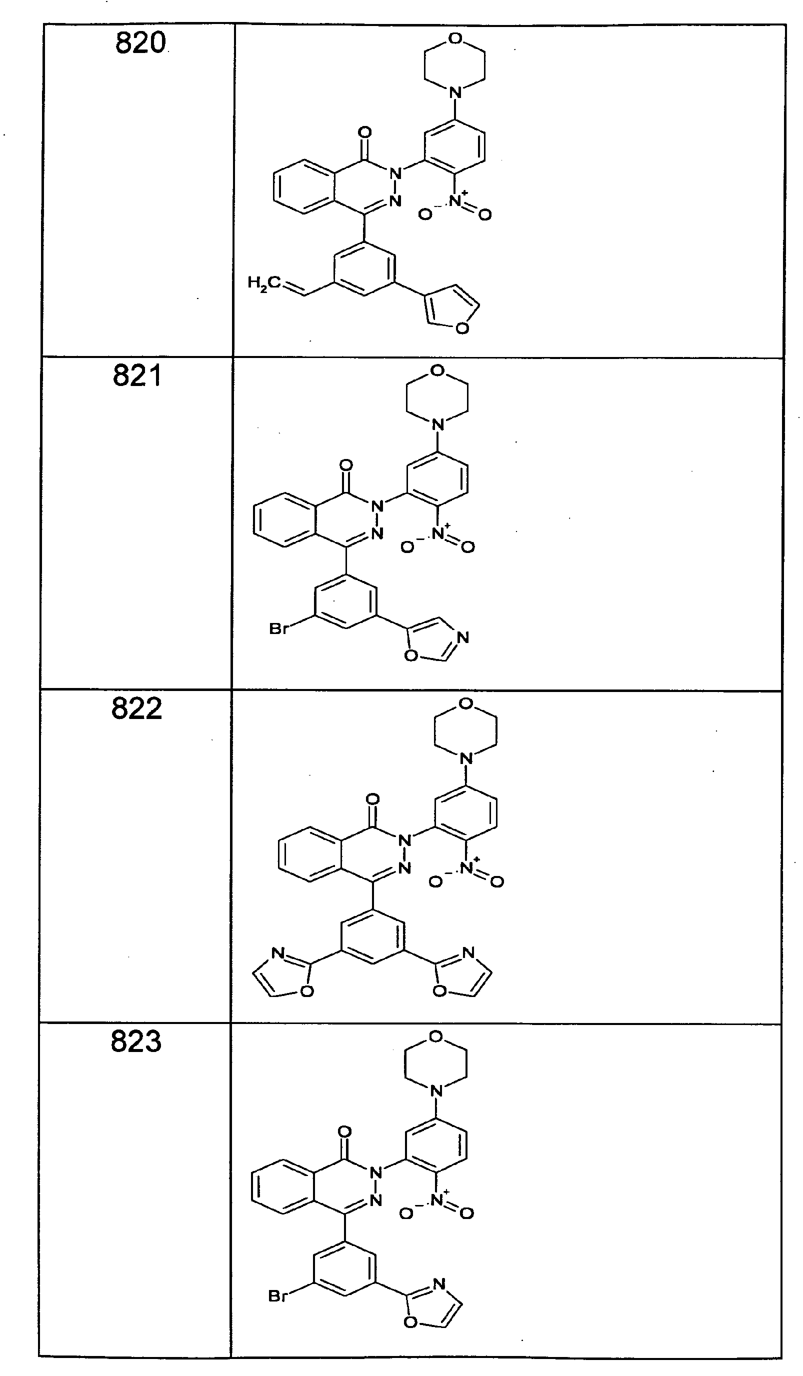

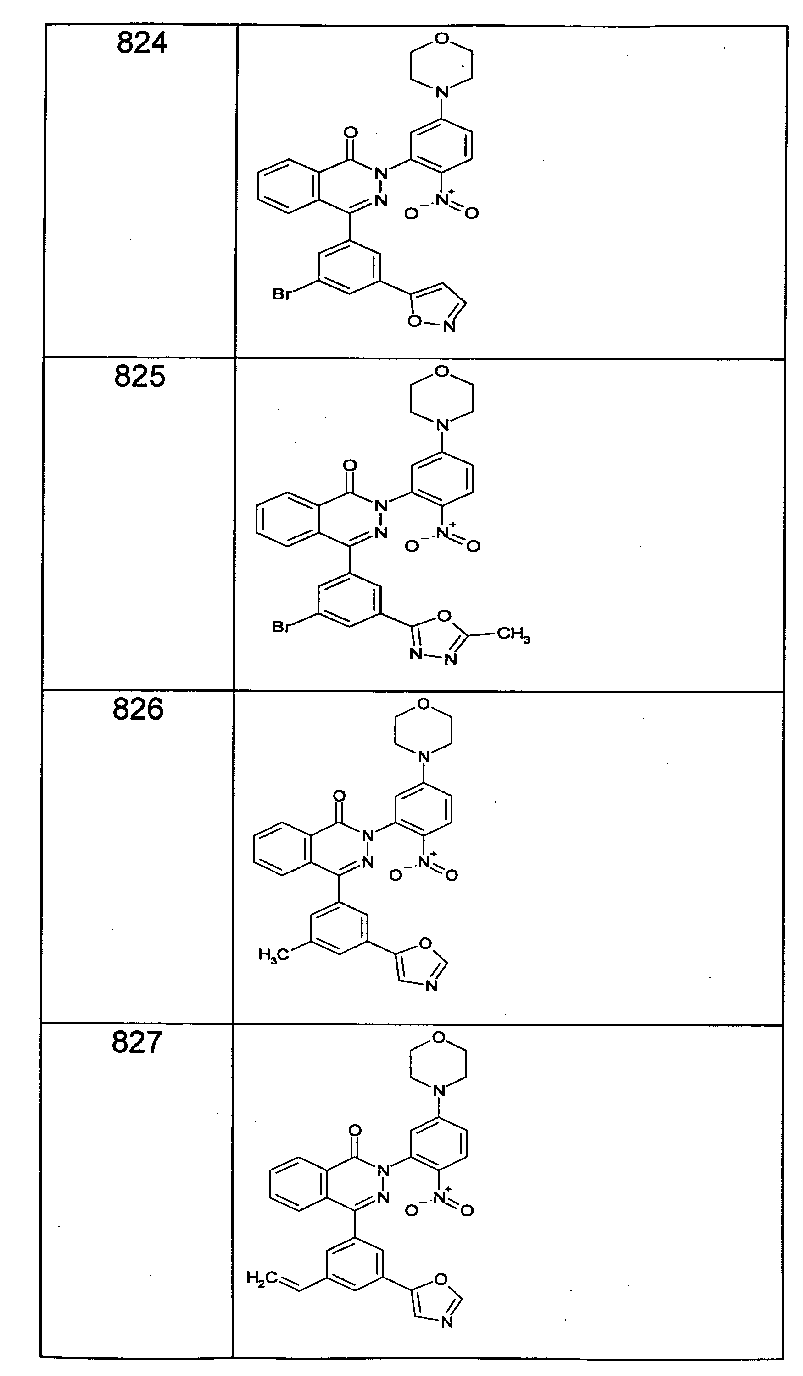

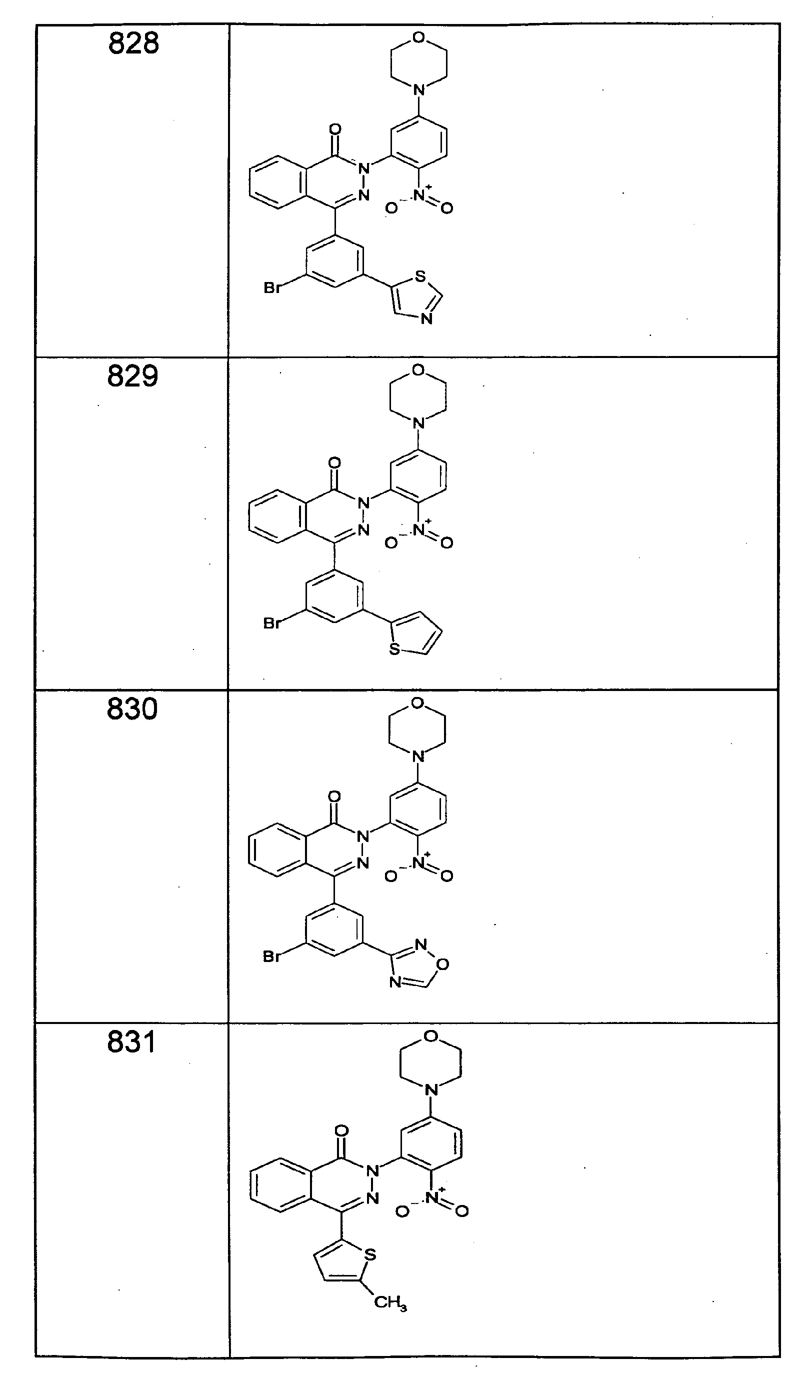

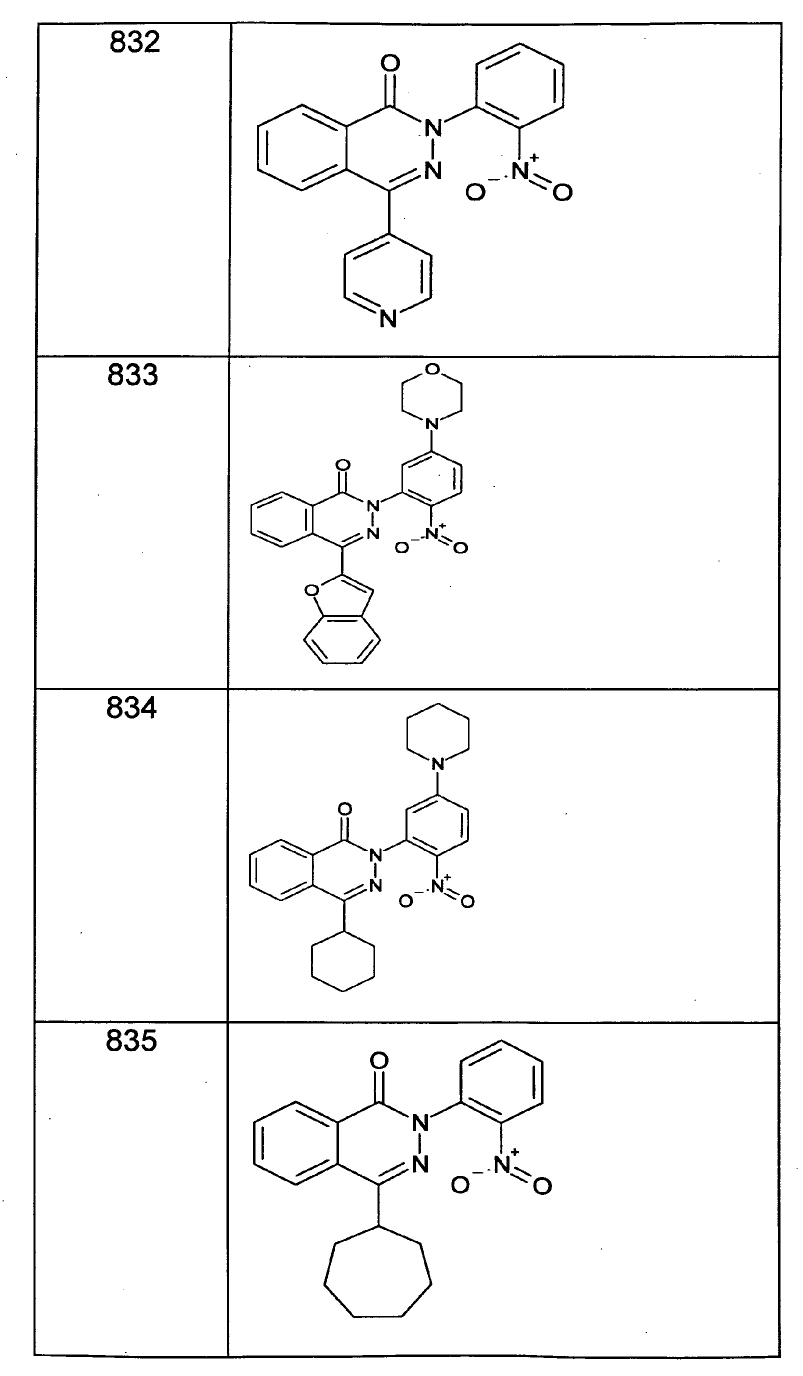

### Example 836-1

### 4-isopropyl-6-(3-methoxy-phenyl)-2-(5-morpholin-4-yl-2-nitro-phenyl)-2H-pyridazin-3-one

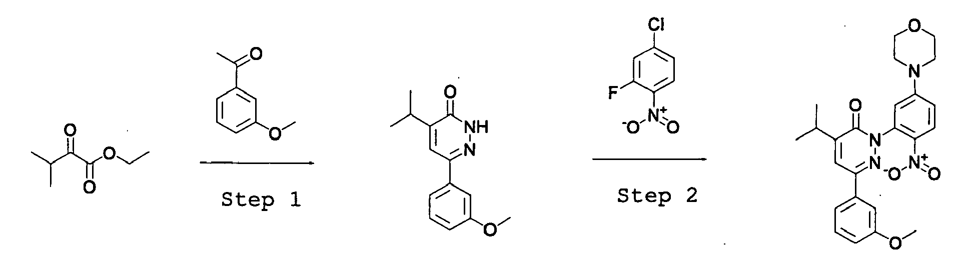

### Step 1

To a solution of ethyl 3-methyl-2-oxo-butyrate (4.93 g) in ethanol (20 mL) was added a solution of potassium hydroxide (1.92 g) in water (10 mL) under ice-cooling. After stirring at room temperature for 2 hr, the mixture was ice-cooled again, and 3-methoxyacetophenone (4.7 mL) and potassium hydroxide (1.92 g) in water (10 mL) were added. After stirring at 4°C for 44 hr, concentrated hydrochloric acid (5.5 mL) was added, and the mixture was extracted with ethyl acetate. The organic layer was washed with water, dried and concentrated. Ethanol (25 mL) and hydrazine monohydrate (8.3 mL) were added. After stirring under refluxing for 2 hr, the mixture was allowed to return to room temperature, and water (50 mL) was added. The precipitated crystals were collected by filtration to give 4-isopropyl-6-(3-methoxy-phenyl)-2H-pyridazin-3-one (3.2 g).
¹H-NMR(CDCl₃)δ: 11.25(brs,1H), 7.50(d,1H,J=1.2Hz), 7.39-7.32(m,3H), 6.97(ddd,1H,J=1.2,2.8,7.7Hz), 3.88(s,3H), 3.28(d-sep,1H,J=1.2,6.7Hz), 1.29(d,6H,J=6.7Hz)

### Step 2

To a solution of 4-isopropyl-6-(3-methoxy-phenyl)-2H-pyridazin-3-one (100 mg) obtained in step 1 in 1,3-dimethyl-2-imidazolidone (1 mL) were added potassium carbonate (113 mg) and 4-chloro-2-fluoro-1-nitrobenzene (74 mg). After stirring at 100°C for 1 hr, the mixture was allowed to return to room temperature, and morpholine (0.18 mL) was added. After stirring at 100°C for 1.5 hr, water was added, and the mixture was extracted with ethyl acetate. The organic layer was washed successively with a 1N aqueous sodium hydroxide solution, water, 1N hydrochloric acid and water, dried, concentrated, and purified by silica gel thin layer chromatography (ethyl acetate:hexane=1:1) to give 4-isopropyl-6-(3-methoxy-phenyl)-2-(5-morpholin-4-yl-2-nitro-phenyl)-2H-pyridazin-3-one (154 mg).
¹H-NMR(CDCl₃)δ: 8.17(ddd,1H,J=1.2,1.4,9.5Hz), 7.53(d,1H,J=0.9Hz), 7.38-7.29(m,3H), 6.96(ddd,1H,J=1.4,2.8,7.7Hz), 6.89(s,1H), 6.88(dd,1H,J=2.7,9.7Hz), 3.85(s,3H), 3.84(m,4H), 3.39(m,4H), 3.29(d-sep,1H,J=0.9,6.7Hz), 1.30(d,6H,J=6.7Hz)

### Example 836-2

### 2-(5-chloro-2-nitro-phenyl)-6-(3-methoxy-phenyl)-4-propoxy-2H-pyridazin-3-one

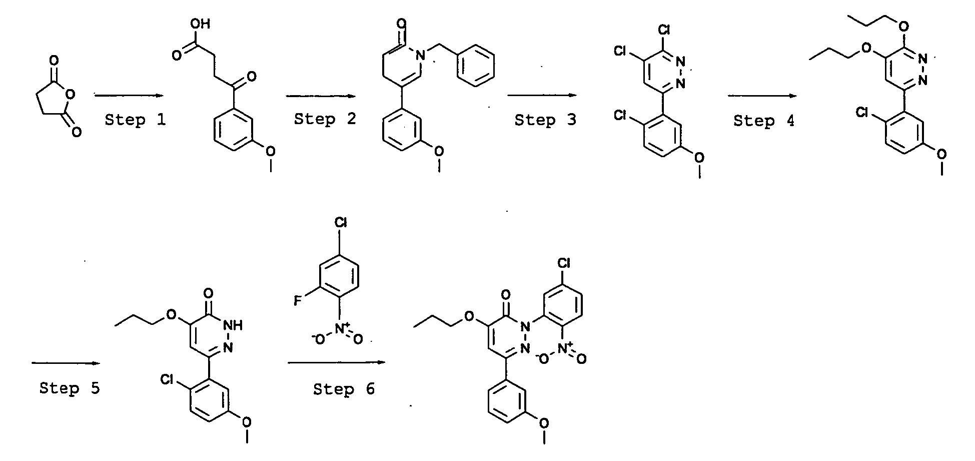

### Step 1

To a solution of succinic anhydride (4.55 g) in tetrahydrofuran (200 mL) was added dropwise 3-methoxyphenylmagnesium bromide (1.0 M tetrahydrofuran solution; 50 mL) under argon at -78°C. After the dropwise addition, the mixture was allowed to return to room temperature, and stirred for 2 hr. The mixture was acidified with 2N hydrochloric acid, and extracted with ethyl acetate. The organic layer was concentrated, basified with a 1N aqueous sodium hydroxide solution, and washed with diethyl ether. The aqueous layer was acidified with 2N hydrochloric acid, and the mixture was extracted with ethyl acetate. The organic layer was washed with water, dried and concentrated to give 4-(3-methoxy-phenyl)-4-oxo-butyric acid (8.3 g).
¹H-NMR(DMSO-d₆)δ: 12.1(brs,1H), 7.58(d,1H,J=7.9Hz), 7.47-7.42(m,2H), 7.21(dd,J=1.9,7.9Hz), 3.82(s,3H), 3.24(t,2H,J=6.0Hz), 2.57(t,2H,J=6.0Hz)

### Step 2

To a suspension of sodium ethoxide (5.99 g) in ethanol (50 mL) was added benzylhydrazine dihydrochloride (8.6 g). After stirring at 100°C for 10 min, 4-(3-methoxy-phenyl)-4-oxo-butyric acid (8.3 g) obtained in step 1, sodium acetate (7.22 g) and ethanol (35 mL)-water (10 mL) were added. After stirring under refluxing for 2 hr, the mixture was allowed to return to room temperature and concentrated. Chloroform-water was added to the residue, the insoluble material was filtered off, and the mixture was separated into two layers. The organic layer was washed with water, dried, concentrated and purified by silica gel column chromatography (ethyl acetate:hexane=1:2) to give 2-benzyl-6-(3-methoxy-phenyl)-4,5-dihydro-2H-pyridazin-3-one (9.6 g).
¹H-NMR(CDCl₃)δ: 7.42-7.40(m,2H), 7.34-7.25(m,6H), 6.95-6.91(m,1H), 5.03(s,2H), 3.83(s,3H), 2.94(t,2H,J=7.9Hz), 2.63(t,2H,J=7.9Hz)

### Step 3

To 2-benzyl-6-(3-methoxy-phenyl)-4,5-dihydro-2H-pyridazin-3-one (9.6 g) obtained in step 2 were added phosphorus oxychloride (10 mL) and phosphorous pentachloride (23 g). After stirring under refluxing for 7.5 hr, the mixture was ice-cooled and ice water was added by small portions. The mixture was extracted with ethyl acetate, and the organic layer was washed with water, dried, concentrated, and subjected to silica gel column chromatography (chloroform-ethyl acetate:hexane=1:1), and further recrystallized from ethanol to give 3,4-dichloro-6-(2-chloro-5-methoxyphenyl)-pyridazine (1.15 g).
¹H-NMR(CDCl₃)δ: 8.02(s,1H), 7.40(d,1H,J=8.8Hz), 7.27(d,1H,J=3.2Hz), 7.00(dd,1H,J=3.2,8.8Hz), 3.84(s,3H)

### Step 4

To a solution of 3,4-dichloro-6-(2-chloro-5-methoxy-phenyl)-pyridazine (150 mg) obtained in step 3 in dioxane (2 mL) were added 1-propanol (2 mL) and sodium hydride (60%; 42 mg). After stirring at 100°C for 3 hr, the mixture was allowed to return to room temperature, and the reaction mixture was concentrated. Water was added, and the mixture was extracted with ethyl acetate. The organic layer was washed with water, dried, concentrated and purified by silica gel thin layer chromatography (ethyl acetate:hexane=1:2) to give 6-(2-chloro-5-methoxy-phenyl)-3,4-dipropoxy-pyridazine (164 mg).
¹H-NMR(CDCl₃)δ: 7.35(d,1H,J=8.8Hz), 7.27(d,1H,J=3.0Hz), 7.13(s,1H), 6.92(dd,1H,J=3.0,8.8Hz), 4.56(t,2H,J=6.7Hz), 4.06(t,2H,J=6.7Hz), 3.83(s,3H), 1.92(tq,4H,J=6.7,7.4Hz), 1.07(t,6H,J=7.4Hz)

### Step 5

A solution of 6-(2-chloro-5-methoxy-phenyl)-3,4-dipropoxy-pyridazine (164 mg) obtained in step 4 in acetic acid (2 mL) was stirred at 120°C for 73.5 hr. The mixture was allowed to return to room temperature, water was added, and the mixture was extracted with ethyl acetate. The organic layer was washed with an aqueous sodium hydrogencarbonate solution and brine, dried, concentrated and purified by silica gel thin layer chromatography (ethyl acetate:hexane=1:2) to give 6-(2-chloro-5-methoxy-phenyl)-4-propoxy-2H-pyridazin-3-one (52 mg).
¹H-NMR(CDCl₃)δ: 10.34(brs,1H), 7.35(d,1H,J=8.8Hz), 7.02(d,1H,J=3.0Hz), 6.92(dd,1H,J=3.0,8.8Hz), 6.78(s,1H), 4.02(t,2H,J=6.7Hz), 3.82(s,3H), 1.94(tq,2H,J=6.7,7.4Hz), 1.07(t,3H,J=7.4Hz)

### Step 6

To a solution of 6-(2-chloro-5-methoxy-phenyl)-4-propoxy-2H-pyridazin-3-one (52 mg) obtained in step 5 in tetrahydrofuran (2 mL) were added sodium hydrogencarbonate (30 mg) and 7.5% palladium carbon (150 mg). After stirring under hydrogen (3 atm) for 24 hr, the insoluble material was filtered off, and the mixture was concentrated. To the residue were added 1,3-dimethyl-2-imidazolidone (1 mL), potassium carbonate (35 mg) and 4-chloro-2-fluoro-1-nitrobenzene (22 mg). After stirring at 120°C for 3 hr, water was added, and the precipitated crystals were collected by filtration to give 2-(5-chloro-2-nitro-phenyl)-6-(3-methoxy-phenyl)-4-propoxy-2H-pyridazin-3-one (52 mg).
¹H-NMR(CDCl₃)δ: 8.04 (d,1H,J=8.6Hz), 7.71(d,1H,J=2.3Hz) 7.54(dd,1H,J=2.3,8.6Hz), 7.38-7.26(m,3H), 6.98(dd,1H,J=2.0,8.1Hz), 6.88(s,1H), 4.09(t,2H,J=6.7Hz), 3.86(s,3H), 1.98(tq,2H,J=6.7,7.4Hz), 1.10(t,3H,J=7.4Hz)

### Example 836-3

### 6-(3-methoxy-phenyl)-2-[5-(morpholin-4-yl)-2-nitrophenyl]-4-propoxy-2H-pyridazin-3-one

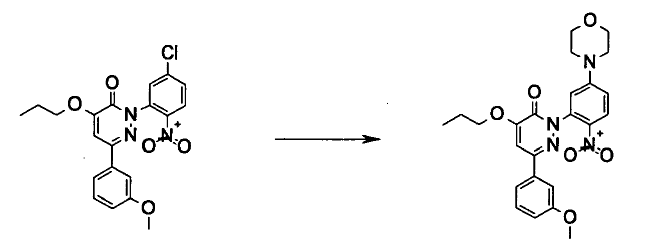

2-(5-Chloro-2-nitro-phenyl)-6-(3-methoxy-phenyl)-4-propoxy-2H-pyridazin-3-one (50 mg) obtained in Example 836-2 was reacted in the same manner as in Example 836-1, step 2, and purified by silica gel thin layer chromatography (ethyl acetate:hexane=3:1) to give 6-(3-methoxy-phenyl)-2-[5-(morpholin-4-yl)-2-nitrophenyl]-4-propoxy-2H-pyridazin-3-one (43 mg).
¹H-NMR(CDCl₃)δ: 8.17(d,1H,J=8.8Hz), 7.38-7.26(m,3H), 6.96(ddd,1H,J=1.1,2.5,8.0Hz), 6.92-6.87(m,3H), 4.09(t,2H,J=6.6Hz), 3.84(s,3H), 3.83(m,4H), 3.38(m,4H), 1.98(tq,2H,J=6.6,7.3Hz), 1.10(t,3H,J=7.3Hz)

### Example 836-4

### 6-(2-chloro-5-methoxy-phenyl)-4-ethylsulfanyl-2-[5-(morpholin-4-yl)-2-nitro-phenyl]-2H-pyridazin-3-one

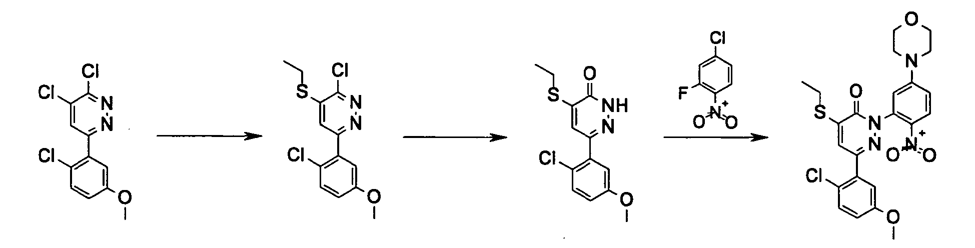

### Step 1

To a suspension of 3,4-dichloro-6-(2-chloro-5-methoxy-phenyl)-pyridazine (200 mg) obtained in Example 836-2, step 3, in ethanol (2 mL) was added sodium ethanethiolate (80%; 80 mg) and the mixture was stirred at 90°C. After 5.5 hr, sodium ethanethiolate (80%; 40 mg) was added again, and the mixture was stirred at 100°C for 4 hr. The reaction mixture was concentrated and purified by silica gel thin layer chromatography (ethyl acetate:hexane=1:2) to give 3-chloro-6-(2-chloro-5-methoxy-phenyl)-4-ethylsulfanyl-pyridazine (157 mg).
¹H-NMR(CDCl₃)δ: 7.55(s,1H), 7.39(d,1H,J=8.8Hz),7.27(d,1H,J=3.0Hz),6.97(dd,1H,J=3.0, 8.8Hz),3.84(s,3H),3.02(q,2H,J=7.4Hz),1.47(t,3H,J=7.4Hz)

### Step 2

3-Chloro-6-(2-chloro-5-methoxy-phenyl)-4-ethylsulfanyl-pyridazine (157 mg) obtained in step 1 was reacted in the same manner as in Example 836-2, step 5, to give 6-(2-chloro-5-methoxy-phenyl)-4-ethylsulfanyl-2H-pyridazin-3-one (140 mg).
¹H-NMR(CDCl₃)δ: 10.75(brs,1H), 7.37(d,1H,J=8.7Hz), 7.21(s,1H), 7.05(d,1H,J=3.0Hz), 6.94(dd,1H,J=3.0,8.7Hz), 3.83(s,3H), 2.92(q,2H,J=7.5Hz), 1.44(t,3H,J=7.5Hz)

### Step 3

6-(2-Chloro-5-methoxy-phenyl)-4-ethylsulfanyl-2H-pyridazin-3-one (60 mg) obtained in step 2 and 4-chloro-2-fluoro-1-nitrobenzene (35.5 mg) were reacted in the same manner as in Example 836-1, step 2, and crystallized from a mixed solvent of ethyl acetate-hexane to give 6-(2-chloro-5-methoxy-phenyl)-4-ethylsulfanyl-2-[5-(morpholin-4-yl)-2-nitro-phenyl]-2H-pyridazin-3-one (89 mg).
¹H-NMR(CDCl₃)δ: 8.18(d,1H,J=9.2Hz), 7.35(d,1H,J=9.0Hz), 7.23(s,1H), 7.00(m,1H), 6.93-6.86(m,3H), 3.83(m,4H), 3.80(s,3H), 3.38(m,4H), 2.95(q,2H,J=7.4Hz), 1.46(t,3H,J=7.4Hz)

### Example 836-5

### ethyl 6-(3-hydroxymethyl-5-methyl-phenyl)-2-(5-nitro-2-trifluoromethyl-phenyl)-3-oxo-2,3-dihydro-pyridazine-4-carboxylate

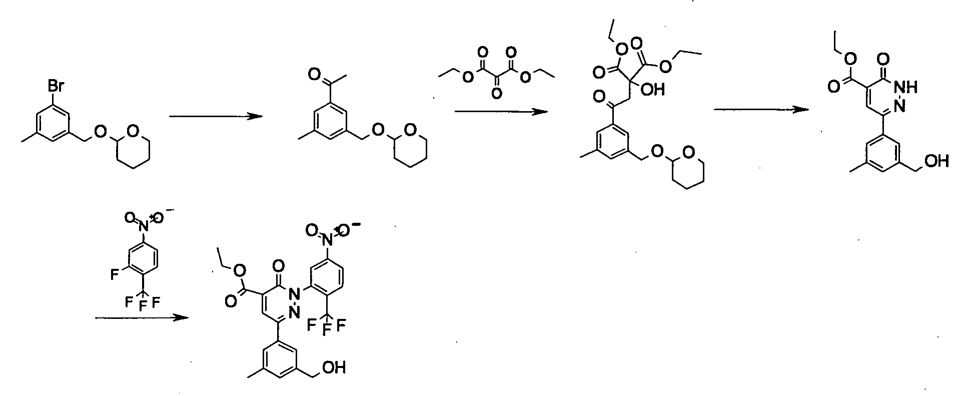

### Step 1

To a solution of 2-(3-bromo-5-methyl-benzyloxy)-tetrahydropyran (28 g) obtained in Example 471, step 1, in tetrahydrofuran (200 mL) was added dropwise n-butyllithium (1.59 M hexane solution; 68 mL) over 10 min under argon at -78°C. After stirring at the same temperature for 40 min, N-methoxy-N-methyl-acetamide (14 mL) was added. The cooling bath was removed and, after stirring for 50 min, a saturated aqueous ammonium chloride solution was added, and the mixture was extracted with ethyl acetate. The organic layer was washed with water, dried, concentrated, and purified by silica gel column chromatography (ethyl acetate:hexane=1:1) to give 1-[3-methyl-5-(tetrahydropyran-2-yloxymethyl)-phenyl]-ethanone (17.5 g).
¹H-NMR(CDCl₃)δ: 7.74(s,1H), 7.69(s,1H), 7.39(s,1H), 4.82-4.49(AB,2H), 4.72(t,1H,J=3.7Hz), 3.96-3.89(m,1H), 3.60-3.53(m,1H), 2.59(s,3H), 2.41(s,3H), 1.93-1.52(m,6H).

### Step 2

To a solution of 1-[3-methyl-5-(tetrahydro-pyran-2-yloxymethyl)-phenyl]-ethanone (17.5 g) obtained in step 1 in tetrahydrofuran (200 mL) was added dropwise lithium diisopropylamide (2.0 M heptane-tetrahydrofuran-ethylbenzene solution; 38.8 mL) over 10 min under argon at -78°C. After stirring at the same temperature for 1 hr, a solution of diethyl ketomalonate (12.9 mL) in tetrahydrofuran (100 mL) was added dropwise over 10 min. The cooling bath was removed, and after stirring for 2 hr, a saturated aqueous ammonium chloride solution was added, and the mixture was extracted with ethyl acetate. The organic layer was washed with water, dried, concentrated and purified by silica gel column chromatography (ethyl acetate:hexane=1:1) to give diethyl 2-hydroxy-2-{2-[3-methyl-5-(tetrahydro-pyran-2-yloxymethyl)-phenyl]-2-oxo-ethyl}-malonate (28.3 g).
¹H-NMR(CDCl₃)δ: 7.75(s,1H), 7.68(s,1H), 7.41(s,1H), 4.82-4.48(AB,2H), 4.71(t,1H,J=3.7Hz), 4.30(q,4H,J=7.2Hz), 3.95-3.88(m,1H), 3.82(s,2H), 3.60-3.52(m,1H), 2.41(s,3H), 1.90-1.53(m,6H), 1.30(t,6H,J=7.2Hz).

### Step 3

To a solution of diethyl 2-hydroxy-2-{2-[3-methyl-5-(tetrahydro-pyran-2-yloxymethyl)-phenyl]-2-oxoethyl}-malonate (8.02 g) obtained in step 2 in ethanol (370 mL) was added hydrazine dihydrochloride (2.59 g). After stirring under refluxing for 18 hr, ethanol was evaporated. Water was added to the obtained residue, and the mixture was extracted with ethyl acetate. The organic layer was washed with water, dried and concentrated. Ethyl acetate-hexane (1:2) was added, and the insoluble material was filtered off. The filtrate was purified by silica gel column chromatography (ethyl acetate:hexane=1:2-ethyl acetate) to give ethyl 6-(3-hydroxymethyl-5-methyl-phenyl)-3-oxo-2,3-dihydro-pyridazine-4-carboxylate (3.95 g). ¹H-NMR(CDCl₃)δ: 11.66(brs,1H), 8.29(s,1H), 7.61(s,1H), 7.52(s,1H),7.27(s,1H), 4.75(s,2H), 4.46(q,2H,J=7.0Hz), 2.43(s,3H), 1.43(t,3H,J=7.0Hz).

### Step 4

Ethyl 6-(3-hydroxymethyl-5-methyl-phenyl)-3-oxo-2,3-dihydro-pyridazine-4-carboxylate (3.77 g) obtained in step 3 and 2-fluoro-4-nitro-1-trifluoromethylbenzene (3.25 g) produced by the method of Example 1-1, supplementary step 2, were reacted in the same manner as in Example 1-1, step 4, and purified by silica gel column chromatography (ethyl acetate:hexane=2:3-2:1) to give ethyl 6-(3-hydroxymethyl-5-methyl-phenyl)-2-(5-nitro-2-trifluoromethyl-phenyl)-3-oxo-2,3-dihydro-pyridazine-4-carboxylate (2.82 g).
¹H-NMR(CDCl₃)δ: 8.48(1H,d,J=8.8Hz), 8.41(1H,d,J=5.7Hz), 8.40(1H,s), 8.07(1H,d,J=8.8Hz), 7.59(1H,s), 7.51(1H,s), 7.29(1H,s), 4.73(2H,d,J=5.7Hz), 4.47(3H,q,J=7.1Hz), 2.42(3H,s), 1.43(4H,t,J=7.1Hz).

### Example 836-6

### methyl 2-(5-bromo-2-trifluoromethyl-phenyl)-6-(3-hydroxymethyl-5-methyl-phenyl)-3-oxo-2,3-dihydro-pyridazine-4-carboxylate

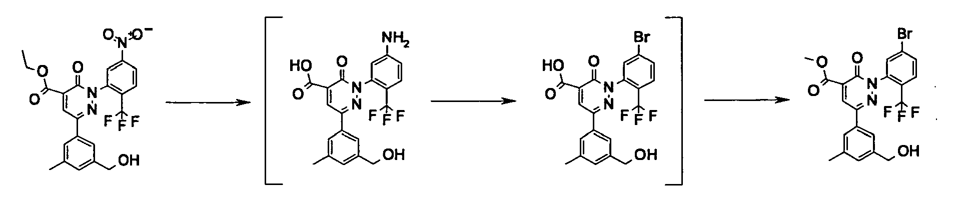

Ethyl 6-(3-hydroxymethyl-5-methyl-phenyl)-2-(5-nitro-2-trifluoromethyl-phenyl)-3-oxo-2,3-dihydro-pyridazine-4-carboxylate (2.8 g) obtained in Example 836-5 was reacted in the same manner as in Example 1-2 to give a crude product (2.46 g). The resultant product was dissolved in tetrahydrofuran (5 mL)-ethanol (10 mL), and a 1N aqueous sodium hydroxide solution (5 mL) was added under ice-cooling. After stirring at room temperature for 30 min, the solvent was evaporated, a 5% aqueous potassium hydrogen sulfate solution was added, and the mixture was extracted with ethyl acetate. The organic layer was washed with water, dried and concentrated. The obtained residue were reacted in the same manner as in Example 1-3 and the obtained crude product was dissolved in N,N-dimethylformamide (17 mL). Potassium carbonate (1.02 g) and methyl iodide (0.445 mL) were added, and the mixture was stirred at 45°C for 1 hr. Ice water was added to the reaction mixture, and the mixture was extracted with ethyl acetate. The organic layer was washed with water, dried, concentrated and purified by silica gel column chromatography (ethyl acetate:hexane=1:1-3:2-2:1) to give methyl 2-(5-bromo-2-trifluoromethyl-phenyl)-6-(3-hydroxymethyl-5-methyl-phenyl)-3-oxo-2,3-dihydro-pyridazine-4-carboxylate (1.28 g).
¹H-NMR(CDCl₃)δ: 8.41(1H,s), 7.82-7.66(3H,m), 7.58(1H,s), 7.52(1H,s), 7.28(1H,s), 4.73(2H,s), 3.99(3H,s), 2.41(3H,s), 1.49(1H,brs).

### Example 836-7

### tert-butyl {2-(5-bromo-2-trifluoromethyl-phenyl)-6-[3-methyl-5-(oxazol-5-yl)-phenyl]-3-oxo-2,3-dihydro-pyridazin-4-yl}-carbamate

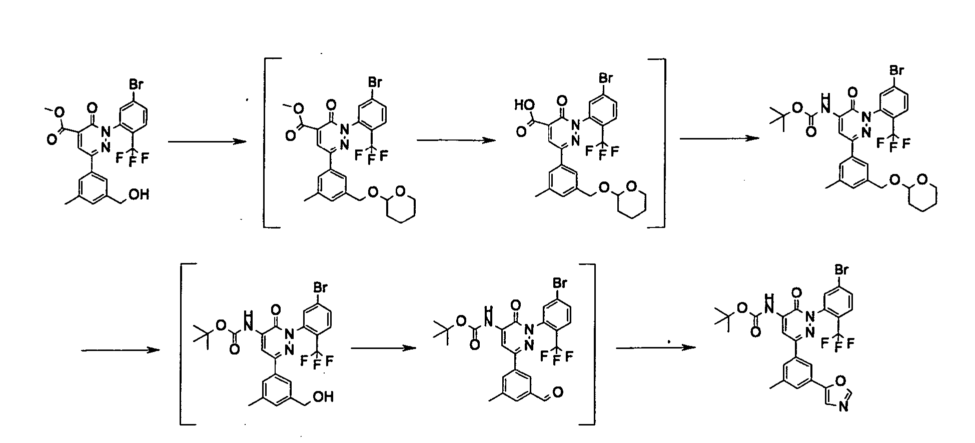

### Step 1

To a solution of methyl 2-(5-bromo-2-trifluoromethyl-phenyl)-6-(3-hydroxymethyl-5-methylphenyl)-3-oxo-2,3-dihydro-pyridazine-4-carboxylate (1.28 g) obtained in Example 836-6 in chloroform (15 mL) were added dihydropyran(0.26 mL) and camphorsulfonic acid (60 mg). After stirring at room temperature for 2 hr, the mixture was poured into a saturated aqueous sodium hydrogencarbonate solution, and extracted with chloroform. The organic layer was washed with water, dried and concentrated to give a crude product (1.3 g). The resultant product was dissolved in tetrahydrofuran (5 mL)-methanol (10 mL), and a 1N aqueous sodium hydroxide solution (2.5 mL) was added under ice-cooling. After stirring at room temperature for 10 min, the solvent was evaporated, 1N hydrochloric acid (2.5 mL) was added, and the mixture was extracted with diethyl ether. The organic layer was washed with water, dried and concentrated. The obtained residue was dissolved in dioxane (15 mL)-tert-butanol (7 mL), and triethylamine (0.75 mL) and diphenylphosphorylazide (0.58 mL) were added. After stirring at room temperature for 20 min, the temperature was raised to 100°C over 20 min, and the mixture was stirred for 1 hr. Further, diphenylphosphorylazide (0.15 mL) was added, and the mixture was stirred at the same temperature for 1 hr. The mixture was allowed to return to room temperature, and the reaction mixture was concentrated. Ice water was added, and the mixture was extracted with ethyl acetate. The organic layer was washed with water, dried, concentrated and purified by silica gel column chromatography (ethyl acetate:hexane=1:10-1:5) to give tert-butyl {2-(5-bromo-2-trifluoromethyl-phenyl)-6-[3-methyl-5-(tetrahydro-pyran-2-yloxymethyl)-phenyl]-3-oxo-2,3-dihydro-pyridazin-4-yl}-carbamate (700 mg).
¹H-NMR(CDCl₃)δ: 8.32(1H,s), 7.92(1H,s), 7.81-7.63(3H,m), 7.57(1H,s), 7.51(1H,s), 7.25(1H,s), 4.78(1H,d,J=12.1Hz), 4.71(1H,t,J=3.5Hz), 4.51(1H,d,J=12.1Hz), 3.99-3.87(1H,m), 3.61-3.49(1H,m), 2.39(3H,s), 1.94-1.52(6H,m), 1.56(9H,s).

### Step 2

tert-Butyl {2-(5-bromo-2-trifluoromethyl-phenyl)-6-[3-methyl-5-(tetrahydro-pyran-2-yloxymethyl)-phenyl]-3-oxo-2,3-dihydro-pyridazin-4-yl}-carbamate (450 mg) obtained in step 1 was reacted in the same manner as in Example 1-23, step 2, Example 471, step 7 and Example 471, step 8, and purified by silica gel column chromatography (ethyl acetate:hexane=1:4-1:1-3:1) to give tert-butyl {2-(5-bromo-2-trifluoromethyl-phenyl)-6-[3-methyl-5-(oxazol-5-yl)-phenyl]-3-oxo-2,3-dihydro-pyridazin-4-yl}-carbamate (290 mg).
¹H-NMR(CDCl₃)δ: 8.35(s,1H), 7.97(brs,1H), 7.93(s,1H), 7.88(br,1H), 7.79(d,1H,J=8.3Hz), 7.73(d,1H,J=8.3Hz), 7.69(d,1H,J=1.9Hz), 7.57(s,1H), 7.54(s,1H), 7.40(s,1H), 1.58(s,9H).

### Example 836-8

### tert-butyl {2-(5-bromo-2-trifluoromethyl-phenyl)-6-[3-methyl-5-(oxazol-5-yl)-phenyl]-3-oxo-2,3-dihydro-pyridazin-4-yl}-ethyl-carbamate

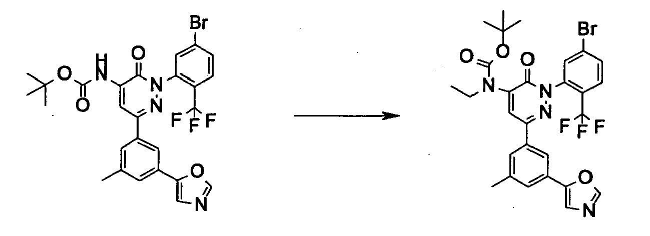

To a solution of tert-butyl {2-(5-bromo-2-trifluoromethyl-phenyl)-6-[3-methyl-5-(oxazol-5-yl)-phenyl]-3-oxo-2,3-dihydro-pyridazin-4-yl}-carbamate (160 mg) obtained in Example 836-7 in N,N-dimethylformamide (2 mL) were added sodium hydride (60%; 16 mg) and ethyl iodide (0.043 mL) under ice-cooling. After stirring at room temperature for 1 hr and at 50°C for 2 hr, ice water was added, and the mixture was extracted with ethyl acetate. The organic layer was washed with water, dried, concentrated and purified by silica gel column chromatography (ethyl acetate:hexane=1:3) to give tert-butyl {2-(5-bromo-2-trifluoromethyl-phenyl)-6-[3-methyl-5-(oxazol-5-yl)-phenyl]-3-oxo-2,3-dihydro-pyridazin-4-yl}-ethylcarbamate (140 mg).
¹H-NMR(CDCl₃)δ: 7.94(s,1H), 7.83(s,1H), 7.78(d,1H,J=9.3Hz), 7.74-7.69(m,3H), 7.55(s,1H), 7.53(s,1H), 7.41(s,1H), 3.83-3.70(m,2H), 1.51(s,9H), 1.23(t,3H,J=7.0Hz).

### Example 836-9

### 4-ethylamino-2-[5-((S)-3-hydroxy-pyrrolidin-1-yl)-2-trifluoromethyl-phenyl]-6-[3-methyl-5-(oxazol-5-yl)-phenyl]-2H-pyridazin-3-one

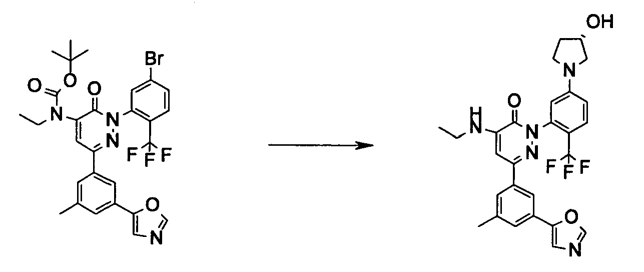

tert-Butyl {2-(5-bromo-2-trifluoromethyl-phenyl)-6-[3-methyl-5-(oxazol-5-yl)-phenyl]-3-oxo-2,3-dihydro-pyridazin-4-yl}-ethyl-carbamate (140 mg) obtained in Example 836-8 and (S)-3-(tert-butyl-diphenyl-silanyloxy)-pyrrolidine (110 mg) were reacted in the same manner as in Example 1-32 and trifluoroacetic acid (0.5 mL) was added to the obtained crude product. After stirring at room temperature for 15 min, the solvent was evaporated. An aqueous sodium hydrogencarbonate solution was added to the residue, and the mixture was extracted with ethyl acetate. The organic layer was washed with water, dried, concentrated and purified by silica gel column chromatography (ethyl acetate:hexane=2:1-ethyl acetate) to give 4-ethylamino-2-[5-((S)-3-hydroxy-pyrrolidin-1-yl)-2-trifluoromethyl-phenyl]-6-[3-methyl-5-(oxazol-5-yl)-phenyl]-2H-pyridazin-3-one (72 mg).
¹H-NMR(CDCl₃)δ: 7.91(s,1H), 7.83(s,1H), 7.62(d,1H,J=8.8Hz), 7.55(s,1H), 7.49(s,1H), 7.38(s,1H), 6.63(dd,1H,J=8.8,2.6Hz), 6.56(d,1H,J=2.6Hz), 6.42(s,1H), 5.80(t,1H,J=5.3Hz), 4.65-4.58(m,1H), 3.58-3.29(m,6H), 2.43(s,3H), 2.16-2.10(m,2H), 1.38(t,3H,J=7.2Hz).

### Example 836-10

### ethyl 2-(2-chloro-6-trifluoromethyl-phenyl)-6-(3-hydroxymethyl-5-methyl-phenyl)-3-oxo-2,3-dihydro-pyridazine-4-carboxylate

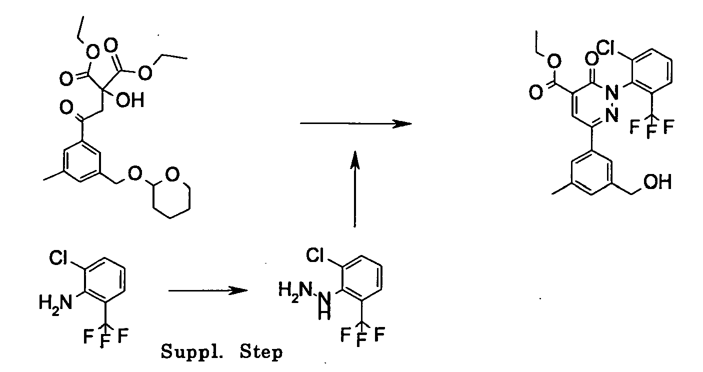

To a solution of diethyl 2-hydroxy-2-{2-[3-methyl-5-(tetrahydro-pyran-2-yloxymethyl)-phenyl]-2-oxo-ethyl}-malonate (3 g) produced by the method of Example 836-5, step 2, in ethanol (20 mL) were added (2-chloro-6-trifluoromethyl-phenyl)-hydrazine (1.8 g) obtained in supplementary step 1 and a 4N hydrogen chloride-dioxane solution (2.13 mL). After stirring at room temperature for 20 min, and under refluxing for 16.5 hr, the mixture was allowed to return to room temperature, and water-ethyl acetate was added to separate the mixture into two layers. The organic layer was washed with water, dried, concentrated, subjected to silica gel column chromatography (ethyl acetate:hexane=1:2-1:1), and crystallized (ethyl acetate-hexane) to give ethyl 2-(2-chloro-6-trifluoromethyl-phenyl)-6-(3-hydroxymethyl-5-methyl-phenyl)-3-oxo-2,3-dihydro-pyridazine-4-carboxylate (1.17 g).
¹H-NMR (CDCl₃)δ: 8.40(s,1H), 7.82-7.75(m,2H), 7.62-7.56(m,2H), 7.52(s,1H), 7.29(s,1H), 4.73(s,2H), 4.46(q,2H,J=7.2Hz), 2.42(s,3H), 1.43(t,3H,J=7.2Hz).

### Supplementary step 1

To a solution of 2-amino-3-chloro-benzotrifluoride (7.0 mL) in trifluoroacetic acid (85 mL) and concentrated hydrochloric acid (42 mL) was added dropwise a solution of sodium nitrite (4.2 g) in water (42 mL) under ice-cooling. After stirring at the same temperature for 1 hr, tin chloride(II) (19.3 g) was added, and the mixture was stirred under ice-cooling for 2 hr, and at room temperature for 14 hrs. The insoluble material was filtered off, and the filtrate was neutralized with an aqueous sodium hydroxide solution. The mixture was extracted with diethyl ether, and the organic layer was washed with water, dried, concentrated and purified by silica gel column chromatography (chloroform:methanol=80:1-40:1) to give (2-chloro-6-trifluoromethyl-phenyl)-hydrazine (4.35 g).
¹H-NMR(CDCl₃)δ: 7.53(d,1H,J=8.3Hz), 7.51(d,1H,J=8.3Hz), 7.04(t,1H,J=8.3Hz), 5.52(brs,1H), 3.94(brs,2H).

### Example 836-11

### tert-butyl {2-(2-chloro-6-trifluoromethyl-phenyl)-6-[3-methyl-5-(oxazol-5-yl)-phenyl]-3-oxo-2,3-dihydro-pyridazin-4-yl}-carbamate

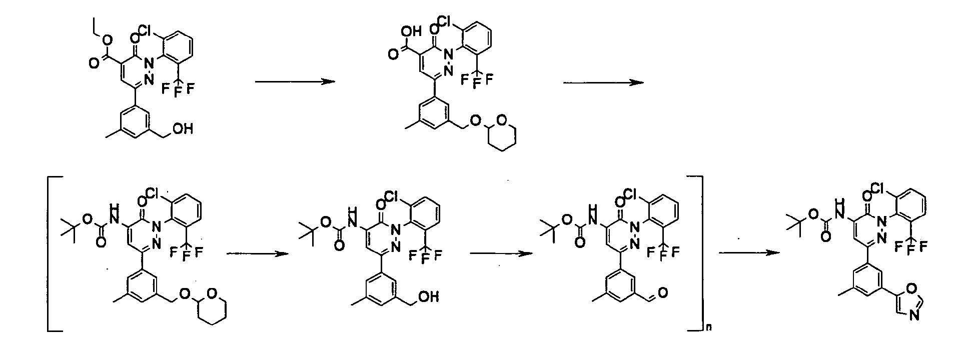

### Step 1

Ethyl 2-(2-chloro-6-trifluoromethyl-phenyl)-6-(3-hydroxymethyl-5-methyl-phenyl)-3-oxo-2,3-dihydro-pyridazine-4-carboxylate (1.17 g) obtained in Example 836-10 was reacted in the same manner as in Example 836-7, step 1, former half, to give 2-(2-chloro-6-trifluoromethyl-phenyl)-6-[3-methyl-5-(tetrahydropyran-2-yloxymethyl)-phenyl]-3-oxo-2,3-dihydro-pyridazine-4-carboxylic acid (1.11 g).
¹H-NMR(CDCl₃)δ: 8.83(s,1H), 7.88-7.81(m,2H), 7.69(t,1H,J=8.1Hz), 7.63(s,1H), 7.57(s,1H), 7.34(s,1H), 4.81(d,1H,J=12.1Hz), 4.74-4.71(m,1H), 4.52(d,1H,J=12.1Hz), 3.96-3.88(m,1H), 3.60-3.53(m,1H), 2.43(s,3H), 1.90-1.54(m,6H).

### Step 2

2-(2-Chloro-6-trifluoromethyl-phenyl)-6-[3-methyl-5-(tetrahydro-pyran-2-yloxymethyl)-phenyl]-3-oxo-2,3-dihydro-pyridazine-4-carboxylic acid (1.11 g) obtained in step 2 and diphenylphosphorylazide (0.55 mL) were reacted in the same manner as in Example 836-7, step 1, latter half, and successively reacted in the same manner as in Example 1-23, step 2, Example 471, step 7 and Example 471, step 8, and purified by silica gel column chromatography (ethyl acetate:hexane=1:2) to give tert-butyl {2-(2-chloro-6-trifluoromethyl-phenyl)-6-[3-methyl-5-(oxazol-5-yl)-phenyl]-3-oxo-2,3-dihydro-pyridazin-4-yl}-carbamate (236 mg).
¹H-NMR(CDCl₃)δ: 8.36(br,1H), 7.93(s,1H), 7.87(s,1H), 7.80(t,2H,J=7.4Hz), 7.63-7.53(m,4H), 7.39(s,1H), 2.46(s,3H), 1.57(s,9H).

### Example 836-12

### 2-(2-chloro-6-trifluoromethyl-phenyl)-4-ethylamino-6-[3-methyl-5-(oxazol-5-yl)-phenyl]-2H-pyridazin-3-one

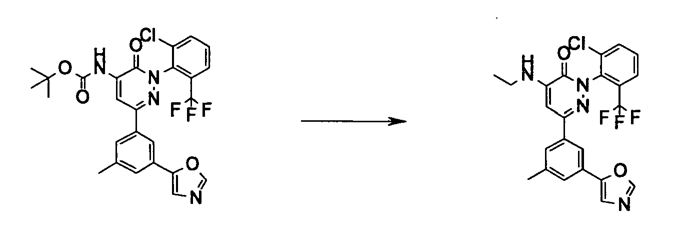

tert-Butyl {2-(2-chloro-6-trifluoromethyl-phenyl)-6-[3-methyl-5-(oxazol-5-yl)-phenyl]-3-oxo-2,3-dihydro-pyridazin-4-yl}-carbamate (118 mg) obtained in Example 836-11 was reacted in the same manner as in Example 836-8 and the obtained crude product was dissolved in a 4N hydrogen chloride-dioxane solution. After stirring at room temperature for 4.5 hr, an aqueous sodium hydrogencarbonate solution was added, and the mixture was extracted with ethyl acetate. The organic layer was washed with water, dried, concentrated and purified by silica gel thin layer chromatography (ethyl acetate:hexane=1:2) to give 2-(2-chloro-6-trifluoromethyl-phenyl)-4-ethylamino-6-[3-methyl-5-(oxazol-5-yl)-phenyl]-2H-pyridazin-3-one (81 mg).
¹H-NMR(CDCl₃)δ: 7.92(s,1H), 7.81-7.77(m,3H), 7.58-7.51(m,3H), 7.39(s,1H), 6.42(s,1H), 5.80(t,1H,J=4.9Hz), 3.35(dq,2H,J=4.9,7.4Hz), 2.45(s,3H), 1.40(t,4H,J=7.4Hz).

### Example 836-13

### ethyl 6-(3-methoxy-phenyl)-2-[4-(morpholin-4-yl)-pyridin-2-yl]-3-oxo-2,3-dihydro-pyridazine-4-carboxylate

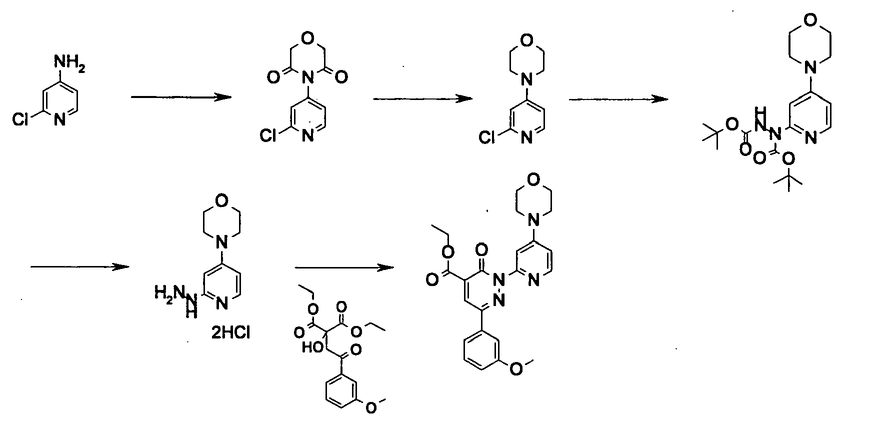

### Step 1

To a solution of 4-amino-2-chloro-pyridine (2.5 g) in dioxane (30 mL) was added diglycolic anhydride (2.26 g). After stirring at 100°C for 12 hr, the precipitated crystals were collected by filtration and suspended in acetic anhydride (40 mL). After stirring at 130°C for 3 hr, the reaction mixture was concentrated. Ethanol was added to the residue, and the precipitated crystals were collected by filtration to give 4-(2-chloro-pyridin-4-yl)-morpholine-3,5-dione (4.48 g).
¹H-NMR(CDCl₃)δ: 8.54(d,1H,J=5.1Hz), 7.25(d,1H,J=1.4Hz), 7.12(dd,1H,J=5.1,1.4Hz), 4.54(s,4H).

### Step 2

To a solution of 4-(2-chloro-pyridin-4-yl)-morpholine-3,5-dione (4.48 g) obtained in step 1 in tetrahydrofuran (60 mL) was added dropwise boranetetrahydrofuran complex (1.13 M tetrahydrofuran solution; 140 mL) under ice-cooling. The cooling bath was removed, and after stirring for 2.5 hr, the reaction mixture was concentrated. 6N hydrochloric acid (40 mL) was added to the residue. After stirring at 100°C for 30 min, a 4N aqueous sodium hydroxide solution (60 mL) was added under ice-cooling. The precipitated crystals were collected by filtration to give 4-(2-chloro-pyridin-4-yl)-morpholine (2.57 g).
¹H-NMR(CDCl₃)δ: 8.06(d,1H,J=6.0Hz), 6.67(d,1H,J=2.3Hz), 6.59(dd,1H,J=6.0,2.3Hz), 3.84(t,4H,J=5.1Hz), 3.31(t,4H,J=5.1Hz).

### Step 3

To a solution of 4-(2-chloro-pyridin-4-yl)-morpholine (520 mg) obtained in step 2 in toluene (15 mL) were added di-tert-butyl azodicarboxylate (1.46 g), tris(dibenzylideneacetone)dipalladium(0) (240 mg) and 1,1'-bis(diphenylphosphino)ferrocene (290 mg) under argon. After stirring at 100°C for 24 hr, the mixture was allowed to return to room temperature, and water-ethyl acetate was added. The mixture was separated into two layers, and the organic layer was washed with water, dried, concentrated and purified by silica gel column chromatography (ethyl acetate:hexane=1:1) to give di-tert-butyl N-[4-(morpholin-4-yl)-pyridin-2-yl]-hydrazodicarboxylate (140 mg).
¹H-NMR(CDCl₃)δ:8.10(d,1H,J=6.0Hz),7.18-7.05(m,1H),6.51(dd,1H,J=6.0,2.3Hz),3.83(t,4H,J=4.6Hz),3 .32(t,4H,J=4.6Hz),1.59(s,9H),1.49(s,9H).

### Step 4

Di-tert-butyl N-[4-(morpholin-4-yl)-pyridin-2-yl]-hydrazodicarboxylate (140 mg) obtained in step 3 was added a 4N hydrogen chloride-ethyl acetate solution (4 mL) under ice-cooling. The cooling bath was removed, and after stirring for 2.5 hr, the reaction mixture was concentrated to give [4-(morpholin-4-yl)-pyridin-2-yl]-hydrazine dihydrochloride (105 mg).
¹H-NMR(DMSO-d₆)δ: 7.58(d,1H,J=7.4Hz), 6.58(dd,1H,J=7.4,2.8Hz), 6.03(br,1H), 3.70(t,4H,J=4.9Hz), 3.41(t,4H,J=4.9Hz).

### Step 5

To a solution of [4-(morpholin-4-yl)-pyridin-2-yl]-hydrazine dihydrochloride (105 mg) obtained in step 4 and diethyl 2-hydroxy-2-[2-(3-methoxy-phenyl)-2-oxoethyl]-malonate (138 mg) produced by a method similar to the method of Example 836-5, step 2, in ethanol (4 mL) was added methanesulfonic acid (0.046 mL). After stirring at room temperature for 14 hr, methanesulfonic acid (0.069 mL) was added. After stirring at 100°C for 19 hr, the reaction mixture was concentrated, and purified by silica gel thin layer chromatography (chloroform:methanol=10:1) to give ethyl 6-(3-methoxyphenyl)-2-[4-(morpholin-4-yl)-pyridin-2-yl]-3-oxo-2,3-dihydro-pyridazine-4-carboxylate (32 mg).
¹H-NMR(CDCl₃)δ: 8.34(d,1H,J=6.0Hz), 8.28(s,1H), 7.39-7.36(m,3H), 7.00-6.94(m,2H), 6.76(dd,1H,J=6.0,2.8Hz), 4.45(q,2H,J=7.4Hz), 3.86-3.83(m,7H), 3.37(t,4H,J=5.1Hz), 1.43(t,3H,J=7.4Hz).

### Examples 836-14 and 836-15

### tert-butyl {6-(3-methoxy-phenyl)-2-[4-(morpholin-4-yl)-pyridin-2-yl]-3-oxo-2,3-dihydro-pyridazin-4-yl}-carbamate (Example 836-14), and 4-amino-6-(3-methoxy-phenyl)-2-[4-(morpholin-4-yl)-pyridin-2-yl]-2H-pyridazin-3-one (Example 836-15)

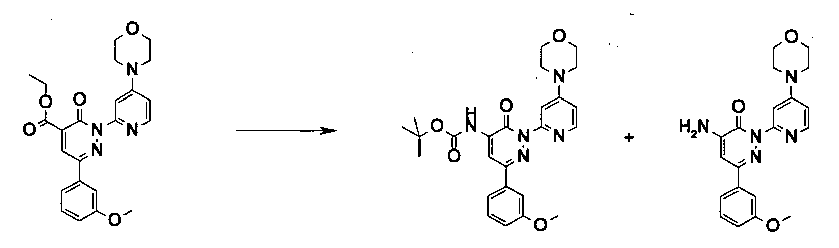

To a solution of ethyl 6-(3-methoxy-phenyl)-2-[4-(morpholin-4-yl)-pyridin-2-yl]-3-oxo-2,3-dihydro-pyridazine-4-carboxylate (32 mg) obtained in Example 836-13 in tetrahydrofuran (1 mL)-ethanol (0.3 mL) was added a 1N aqueous sodium hydroxide solution (0.077 mL). After stirring at room temperature for 1.5 hr, the precipitated crystals were collected by filtration. The obtained crystal (27 mg) and diphenylphosphorylazide (0.016 mL) were reacted in the same manner as in Example 836-7, step 1, latter half, and purified by silica gel thin layer chromatography (chloroform:methanol=10:1) to give tert-butyl {6-(3-methoxy-phenyl)-2-[4-(morpholin-4-yl)-pyridin-2-yl]-3-oxo-2,3-dihydro-pyridazin-4-yl}-carbamate (3 mg) and 4-amino-6-(3-methoxy-phenyl)-2-[4-(morpholin-4-yl)-pyridin-2-yl]-2H-pyridazin-3-one (3 mg).
¹H-NMR(CDCl₃)δ: 8.35(d,1H,J=6.0Hz), 7.45-7.40(m,2H), 7.38-7.31(m,1H), 7.28-7.27(m,1H), 6.98-6.94(m,1H), 6.93-6.90(m,1H), 6.79-6.75(m,1H), 3.88-3.83(m,7H), 3.40-3.35(m,4H), 1.57(s,9H).
¹H-NMR(CDCl₃)δ: 8.35(d,1H,J=6.0Hz), 7.34-7.31(m,3H), 6.94-6.93(m,2H), 6.77-6.73(m,2H), 5.11(brs,2H), 3.89-3.83(m,7H), 3.37(t,4H,J=4.9Hz).

### Example 836-16

### 4-isopropylamino-6-(3-methoxy-phenyl)-2-[4-(morpholin-4-yl)-pyridin-2-yl]-2H-pyridazin-3-one

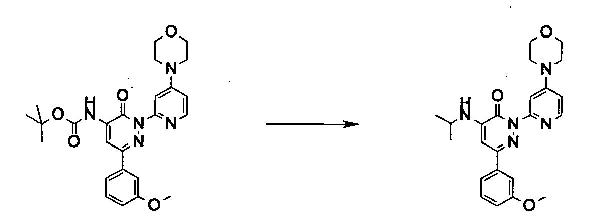

tert-Butyl {6-(3-methoxy-phenyl)-2-[4-(morpholin-4-yl)-pyridin-2-yl]-3-oxo-2,3-dihydro-pyridazin-4-yl}-carbamate (3 mg) obtained in Example 836-14 was added a 4N hydrogen chloride-ethyl acetate solution (0.3 mL), and the mixture was stirred at room temperature for 1 hr. An aqueous sodium hydrogencarbonate solution was added, and the mixture was extracted with ethyl acetate. The organic layer was washed with water, dried and concentrated. The obtained residue and 4-amino-6-(3-methoxy-phenyl)-2-[4-(morpholin-4-yl)-pyridin-2-yl]-2H-pyridazin-3-one (3 mg) obtained in Example 836-15 were reacted in the same manner as in Example 836-8 and purified by silica gel thin layer chromatography (chloroform:methanol=10:1) to give 4-isopropylamino-6-(3-methoxy-phenyl)-2-[4-(morpholin-4-yl)-pyridin-2-yl]-2H-pyridazin-3-one (2.8 mg).
¹H-NMR(CDCl₃)δ: 8.35(d,1H,J=6.0Hz), 7.38-7.32(m,3H), 6.96-6.93(m,2H), 6.74(dd,1H,J=6.0,2.8Hz), 6.40(s,1H), 5.77(d,1H,J=7.9Hz), 3.87-3.82(m,7H), 3.74-3.72(m,1H), 3.36(t,4H,J=4.9Hz), 1.33(d,6H,J=6.0Hz).

### Example 935

### 4-ethylamino-6-(3-methyl-5-oxazol-5-yl-phenyl)-2-(5-pyridazin-4-yl-2-trifluoromethylphenyl)-2H-pyridazin-3-one

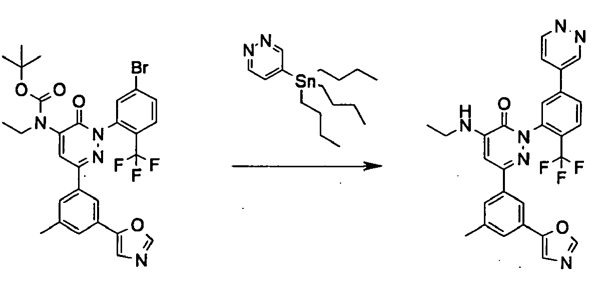

To a solution of *tert*-butyl {2-(5-bromo-2-trifluoromethyl-phenyl)-6-[3-methyl-5-(oxazol-5-yl)-phenyl]-3-oxo-2,3-dihydro-pyridazin-4-yl}-ethylcarbamate (70 mg) obtained in Example 836-8 in toluene (1 mL) were added 4-tributylstannyl-pyridazine (89 mg) produced by the method described in WO01/23383 and tetrakis(triphenylphosphine)palladium(0) (18 mg), and the mixture was stirred under refluxing for 24 hrs. The solvent was evaporated, the tin reagent was removed by silica gel thin layer chromatography (ethyl acetate), and chloroform (2 mL) and trifluoroacetic acid (1 mL) were added. After stirring at room temperature for 1 hr, the mixture was neutralized with an aqueous sodium hydrogencarbonate solution, and the mixture was extracted with ethyl acetate. The organic layer was washed with water, dried, concentrated and crystallized from ethyl acetate-hexane to give 4-ethylamino-6-(3-methyl-5-oxazol-5-yl-phenyl)-2-(5-pyridazin-4-yl-2-trifluoromethylphenyl)-2H-pyridazin-3-one (31 mg).
¹H-NMR(CDCl₃)δ: 9.50-9.49(1H,m), 9.31-9.29(1H,m), 8.04(1H,d,*J*=8.4Hz), 7.92-7.87(2H,m), 7.83(2H,s), 7.69(1H,dd,*J*=5.1,2.6Hz), 7.54-7.51(2H,m), 7.38(1H,s), 6.46(1H,s), 5.85-5.80(1H,m), 3.39-3.34(2H,m), 2.45(3H,s), 1.41(3H,t,*J*=7.3Hz).

### Examples 837 to 1065

The compounds of Examples 837 to 1065 were obtained in the same manner as in a method selected from Examples 836-1 to 836-16. The compounds of Examples 836-1 to 836-16 and the compounds of Examples 837 to 1065 obtained in the same manner are shown in the following Tables. In the tables, the left line shows Example numbers and the right line shows the structural formulas of the corresponding compounds.

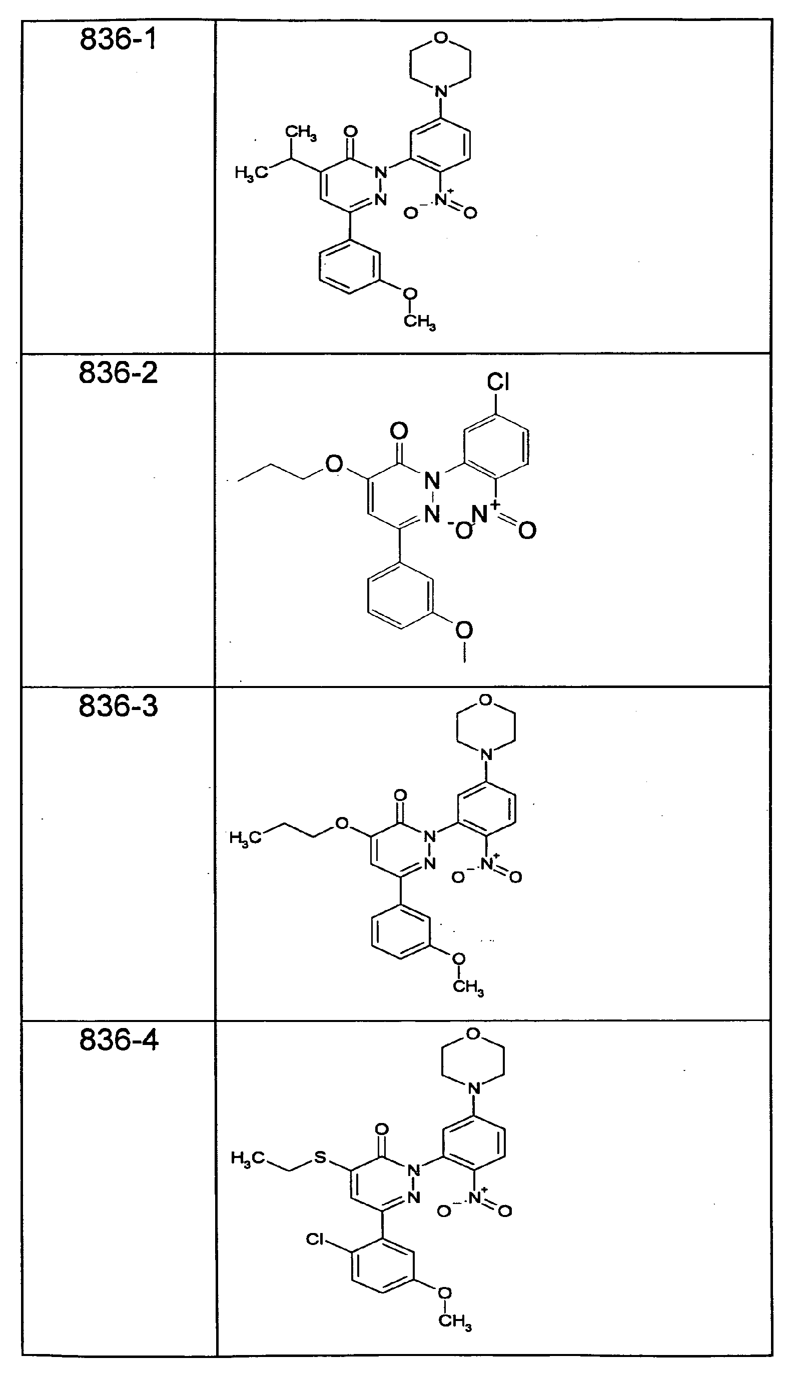

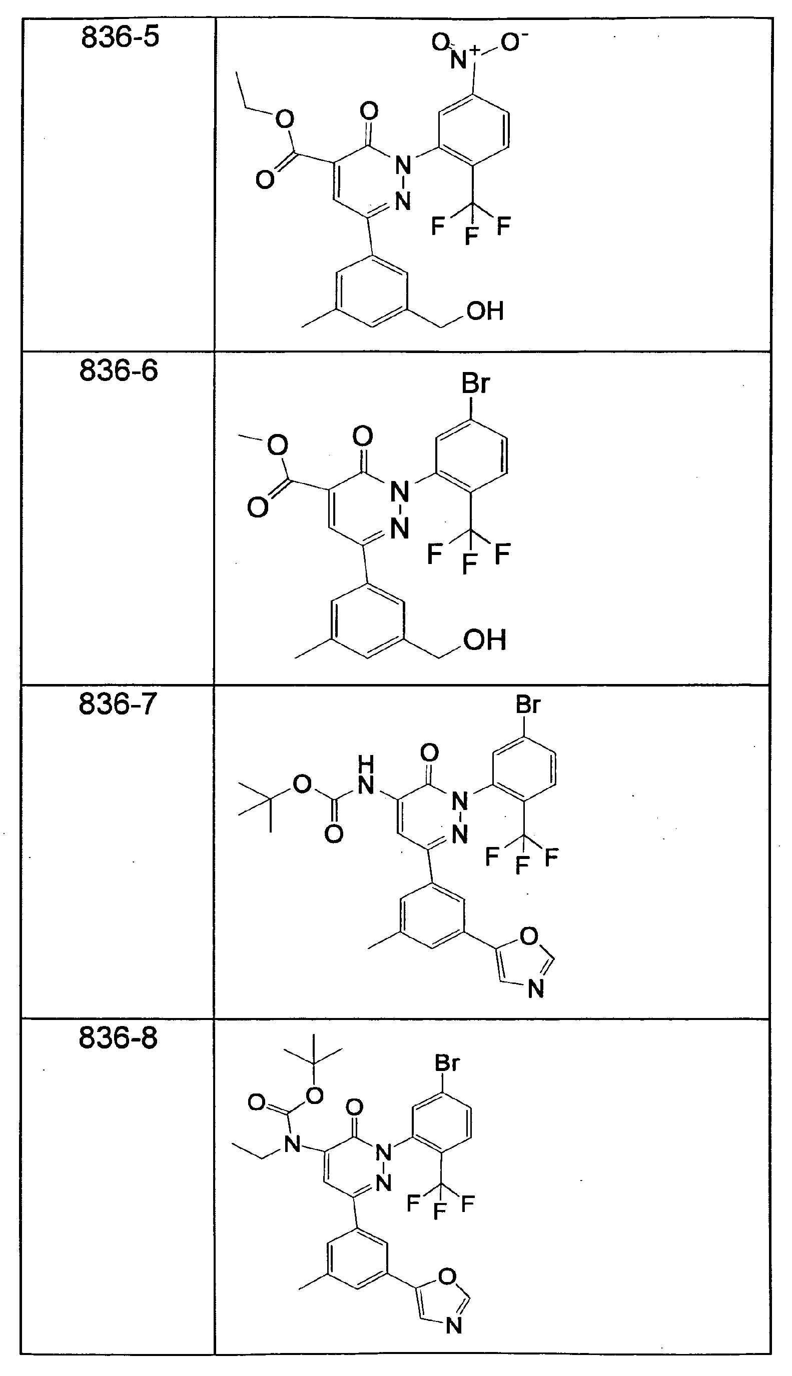

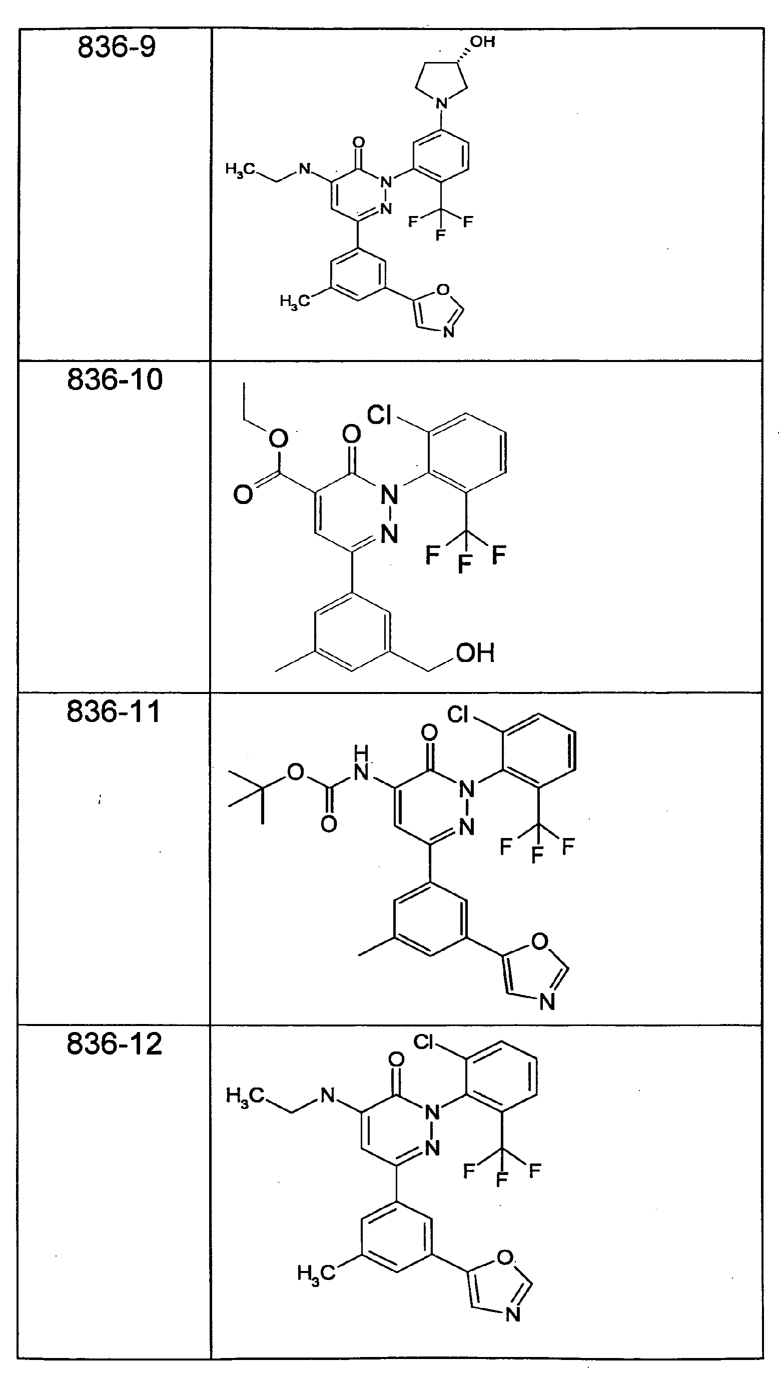

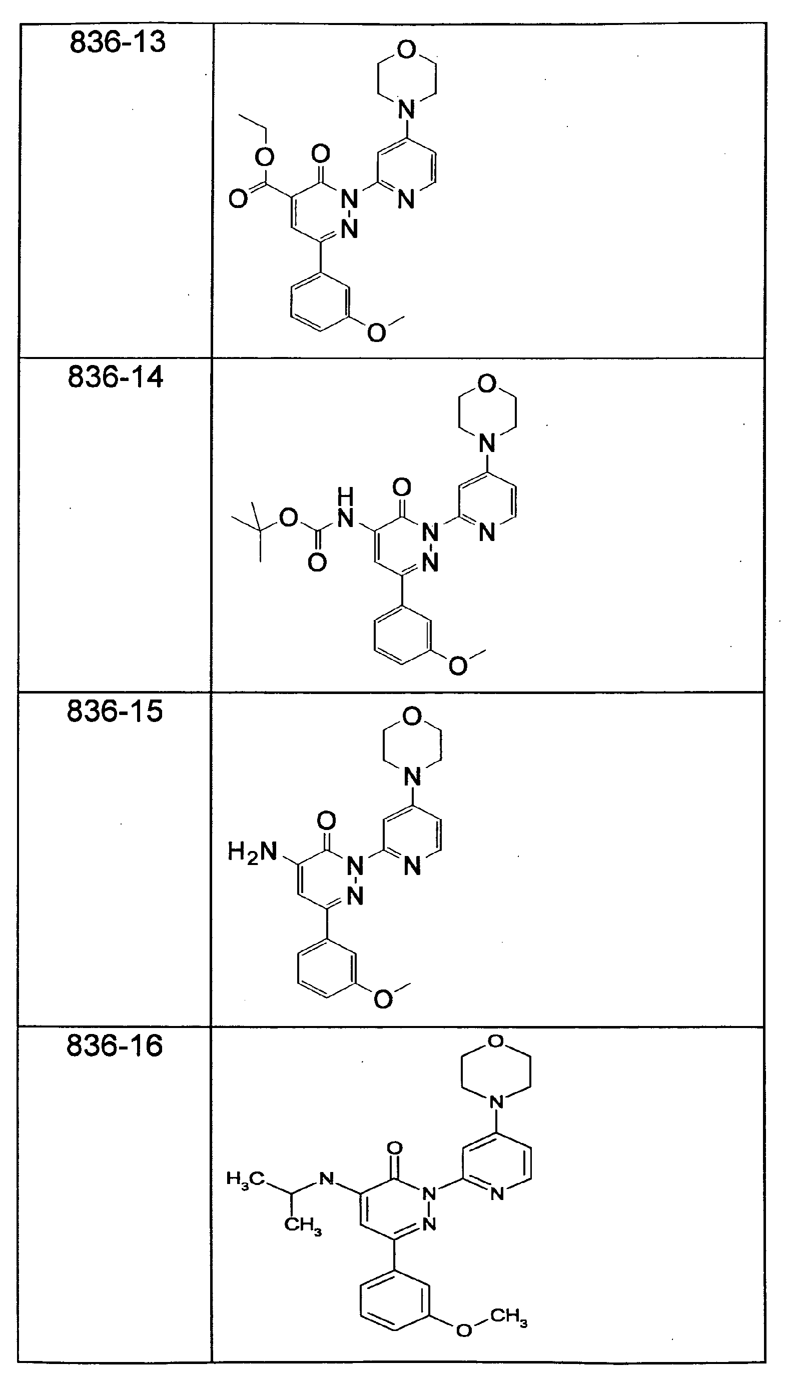

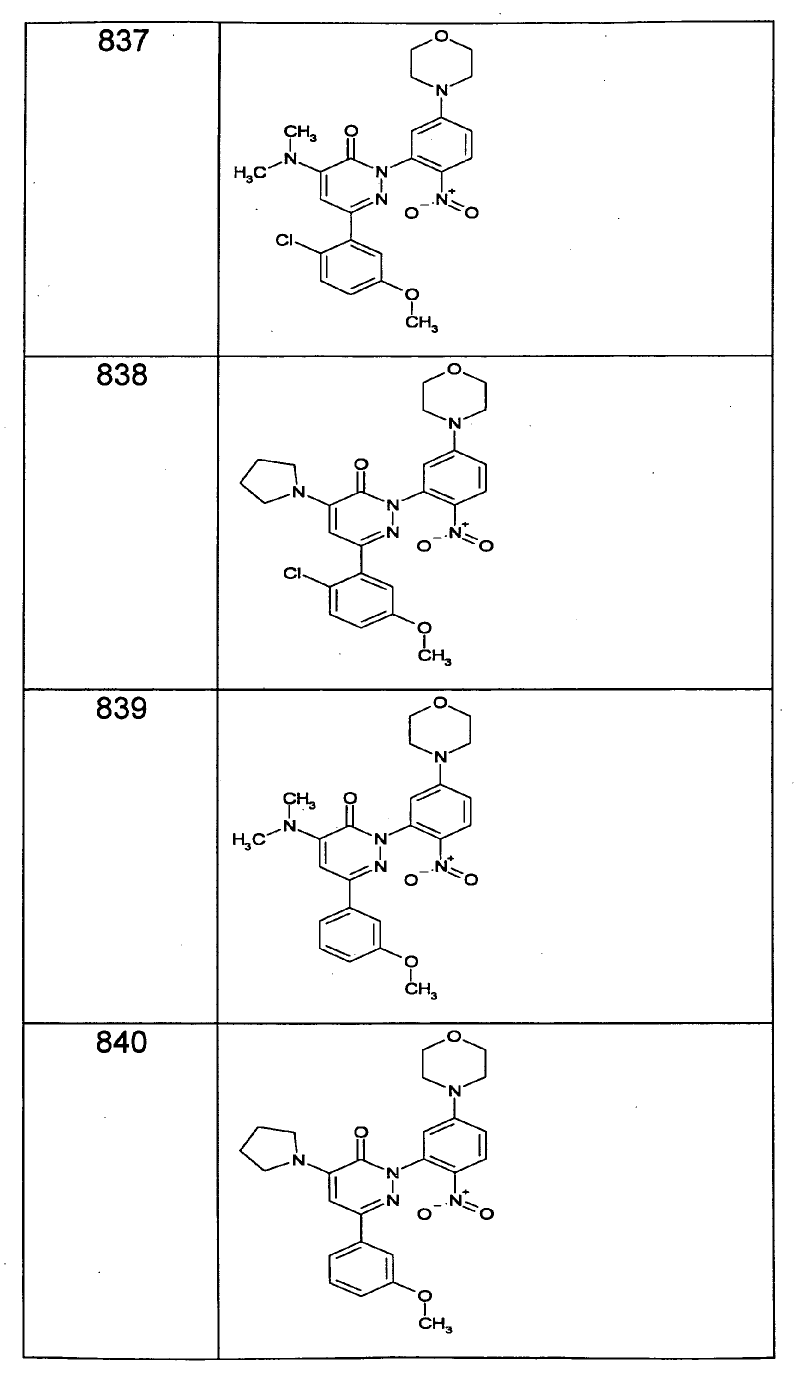

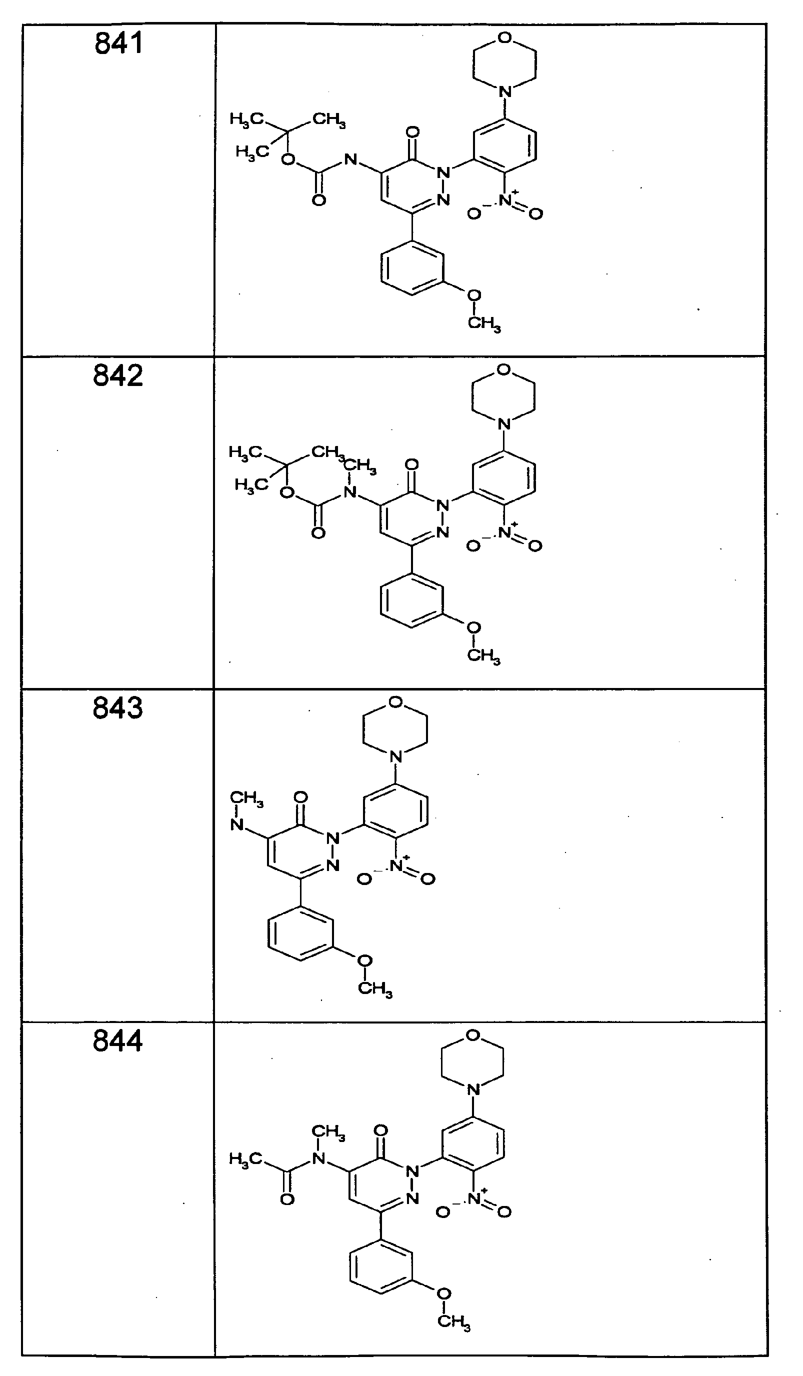

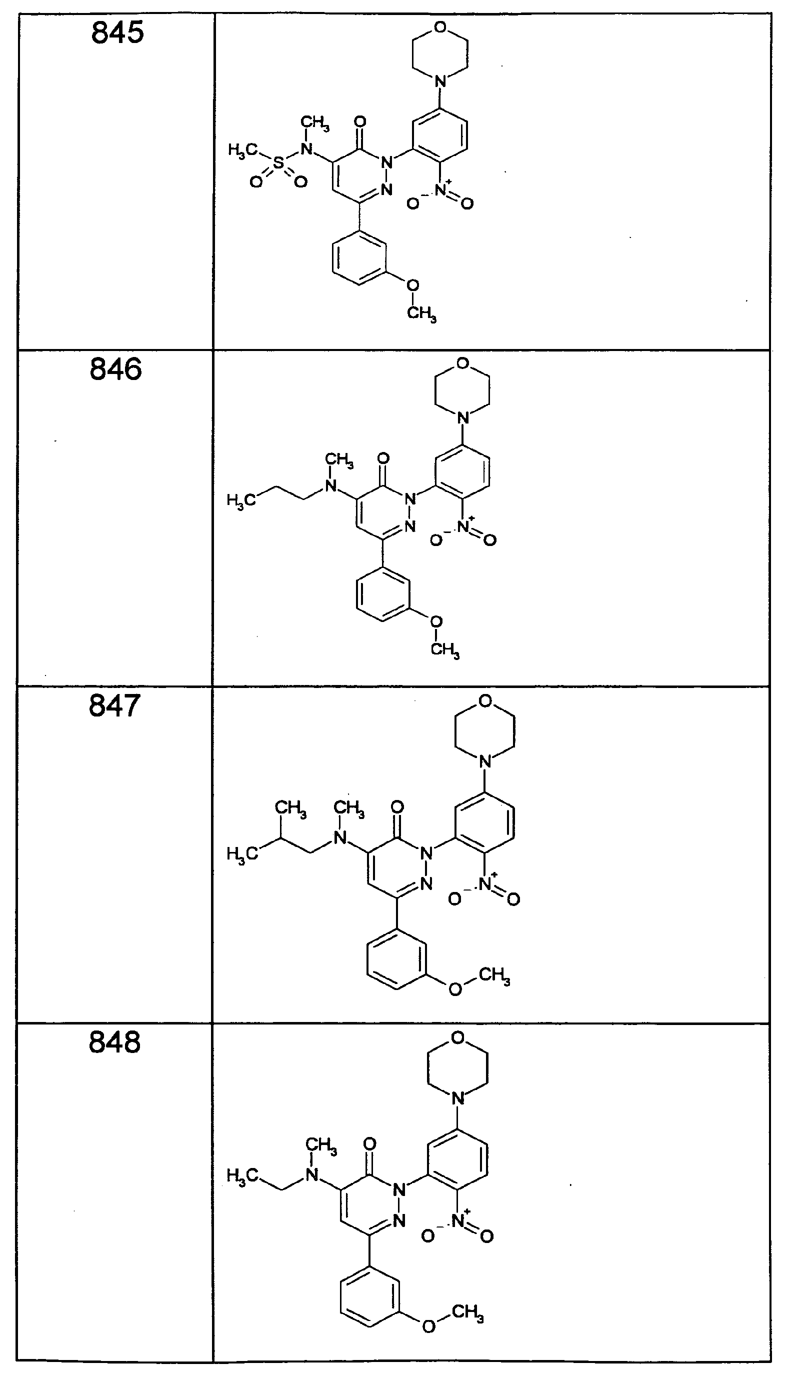

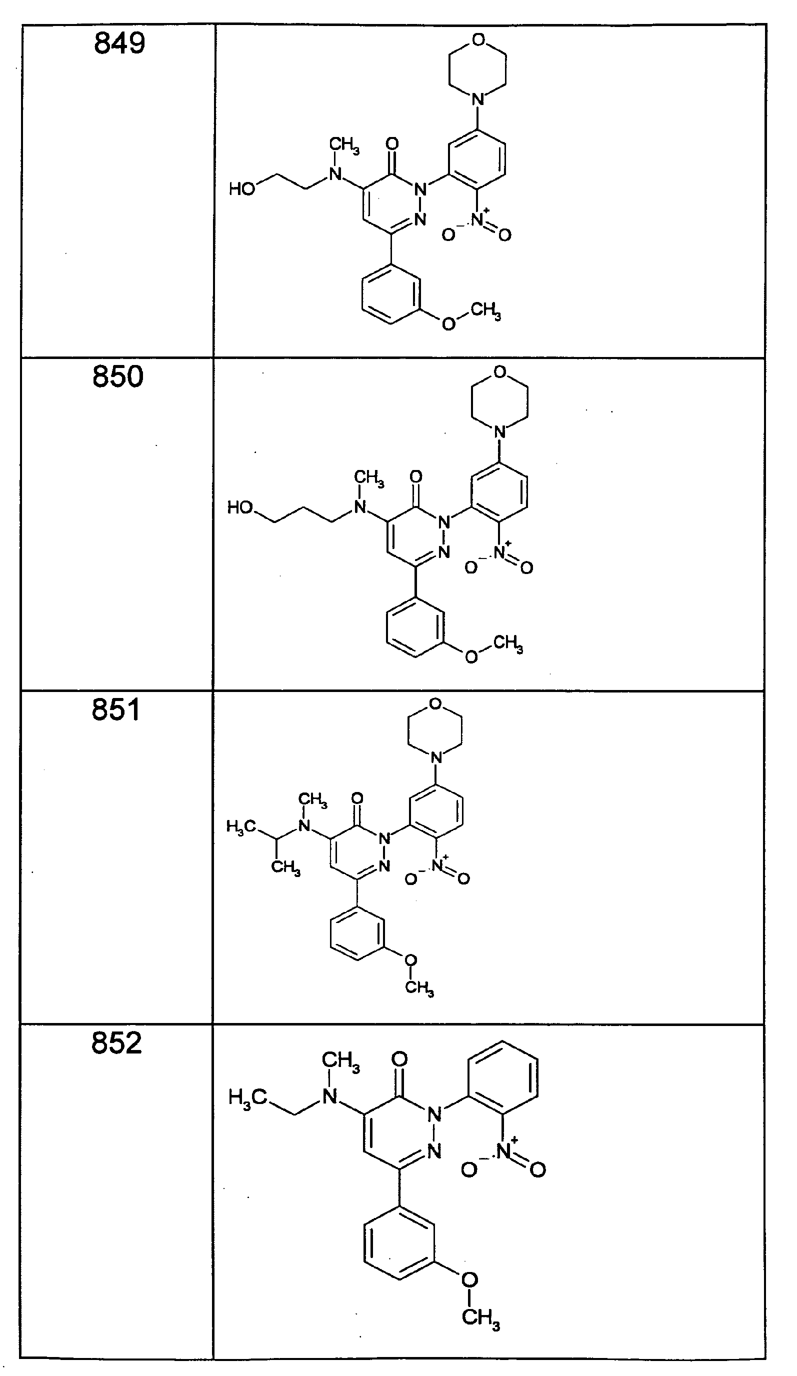

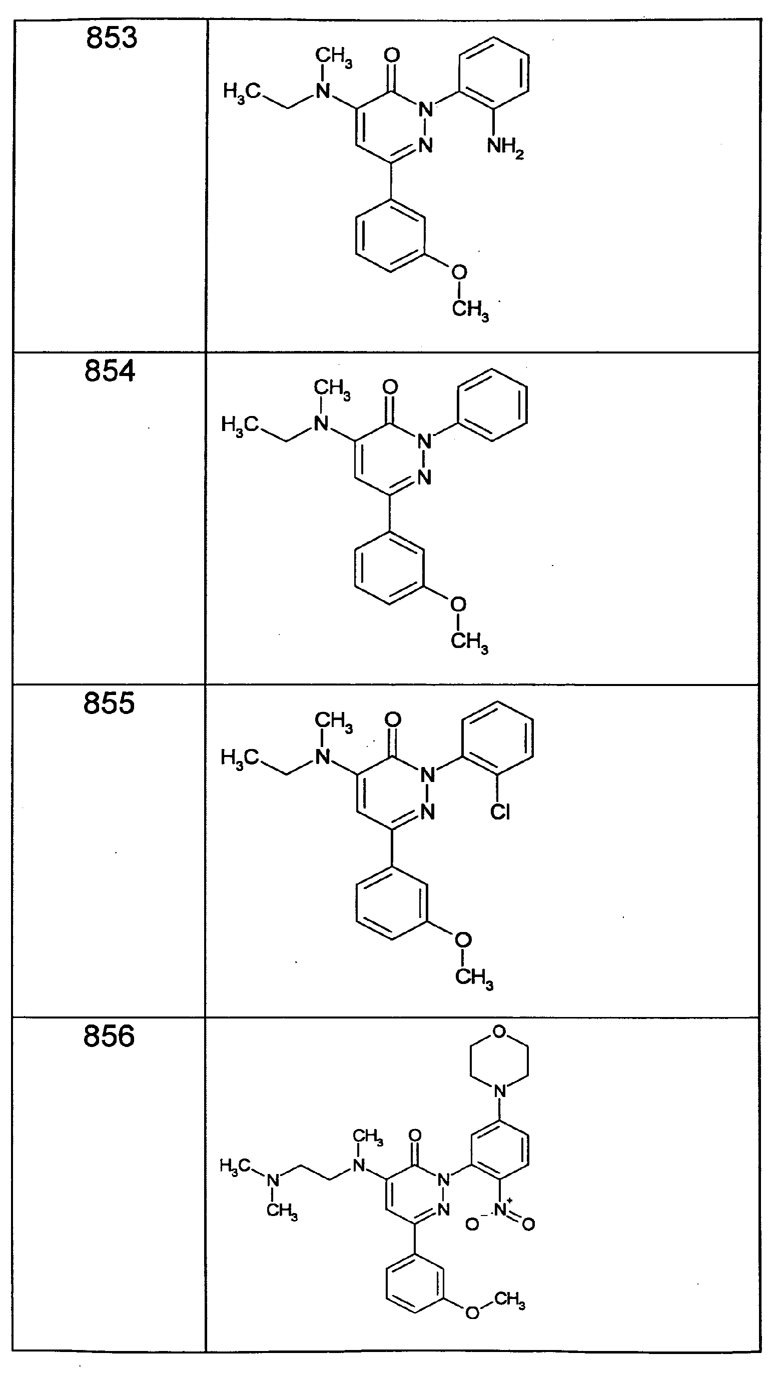

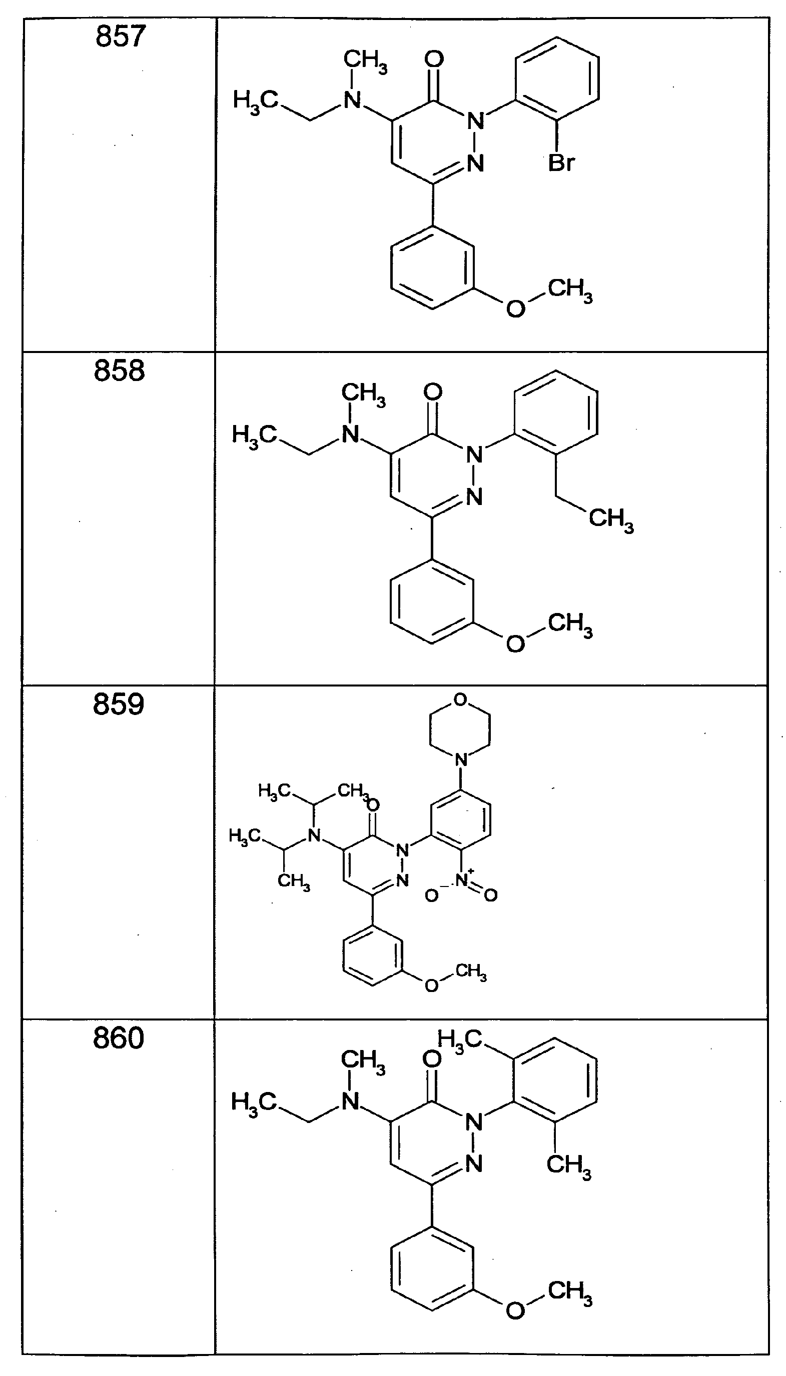

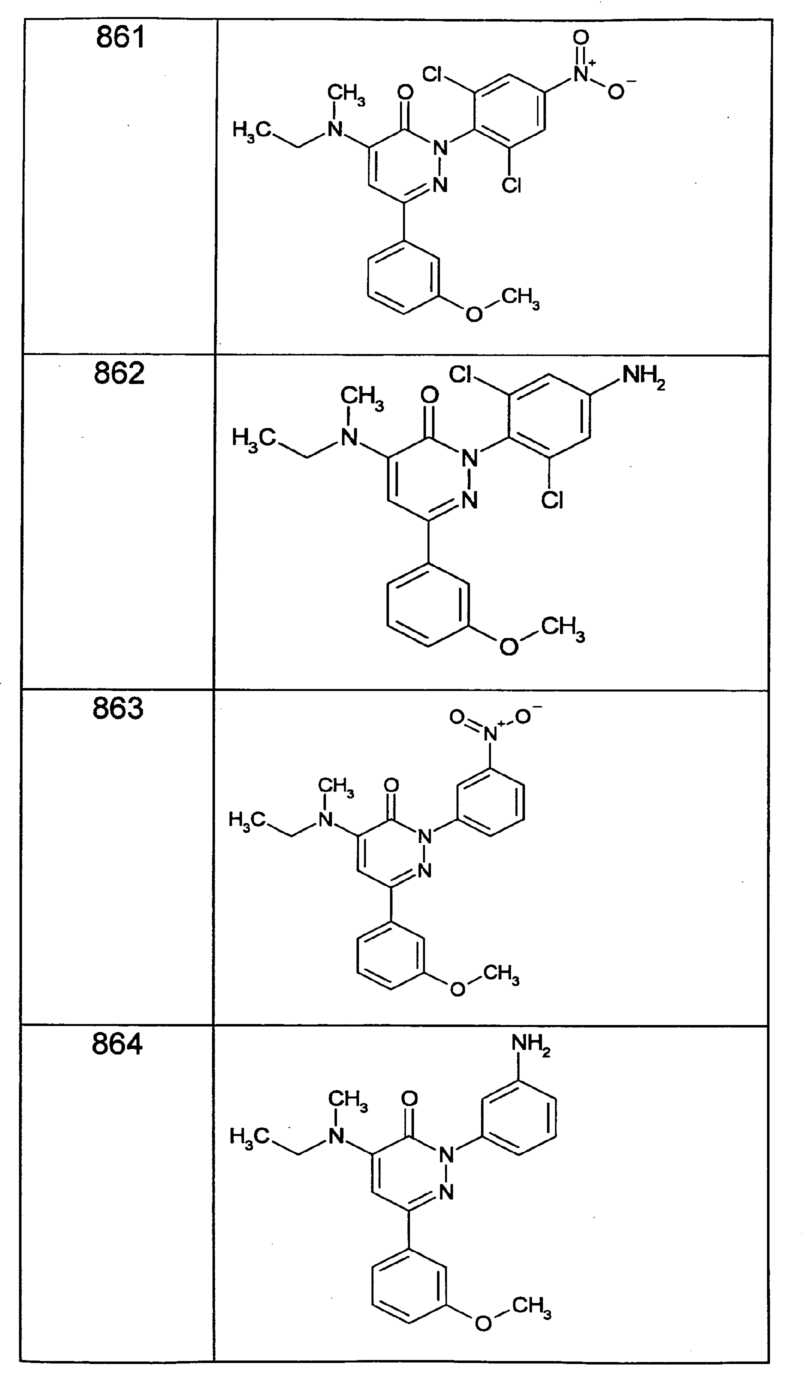

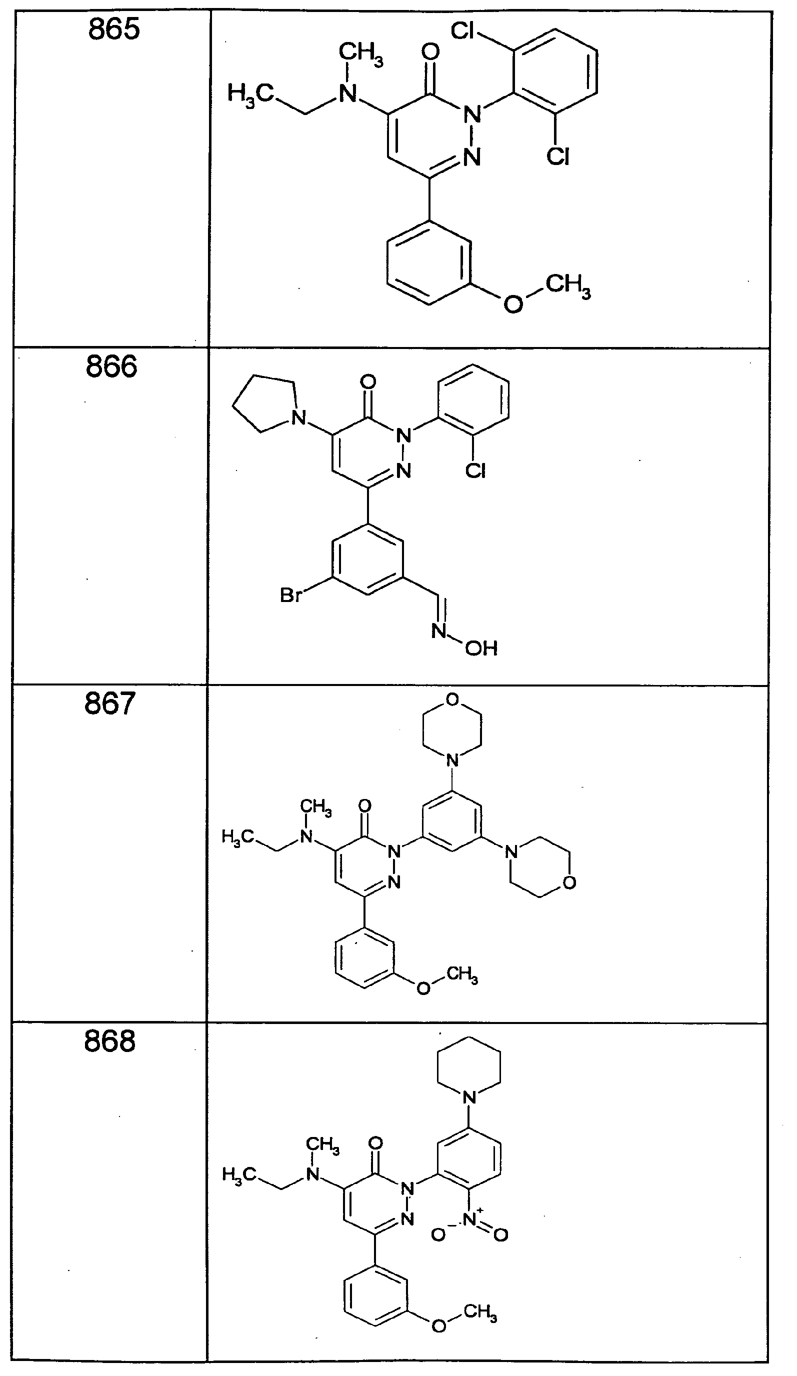

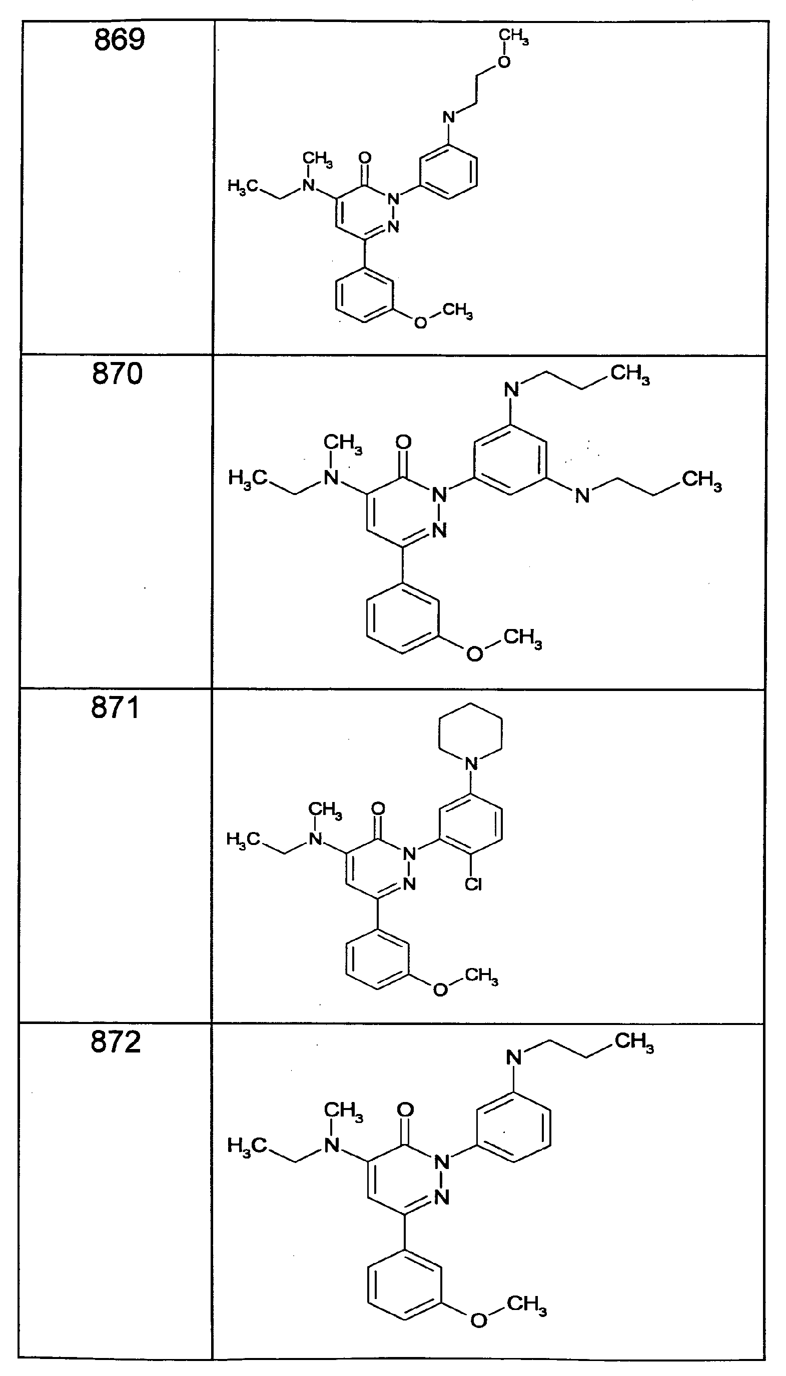

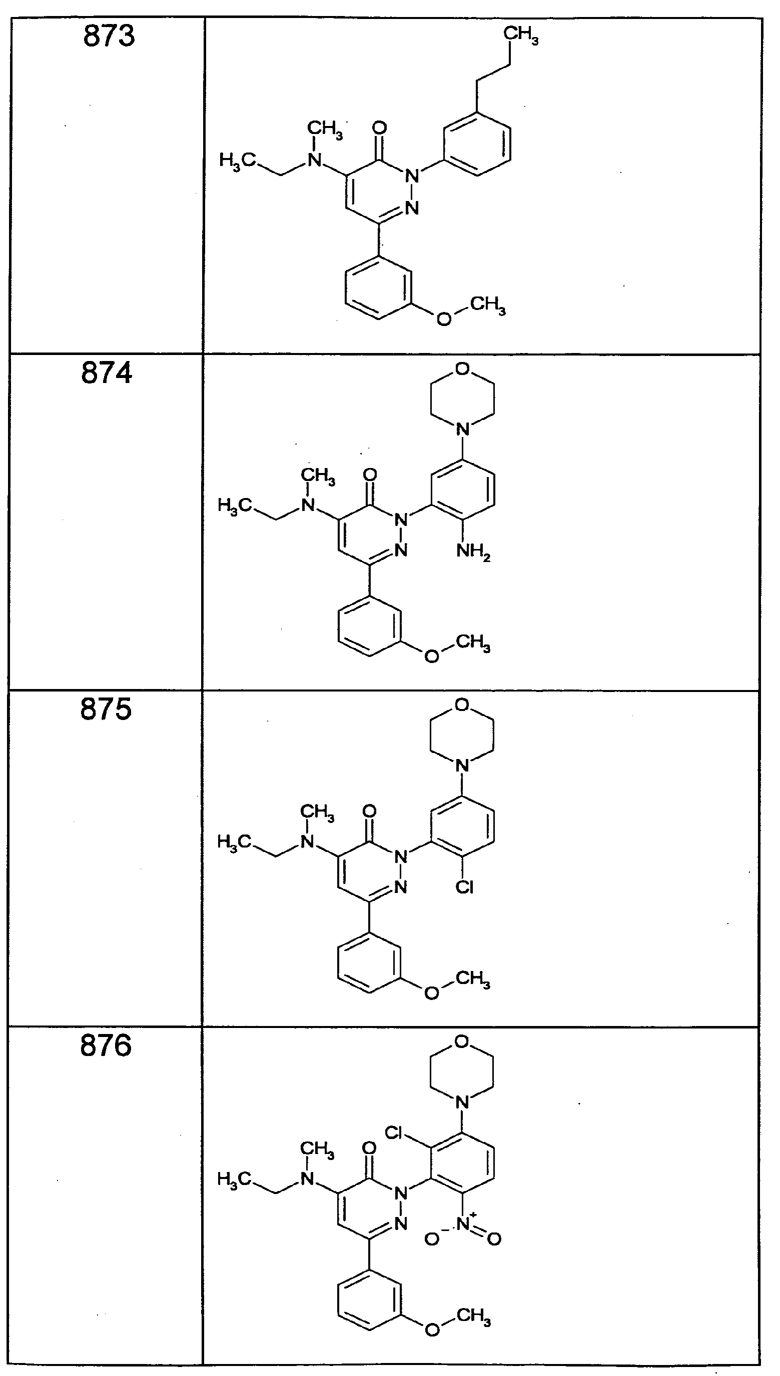

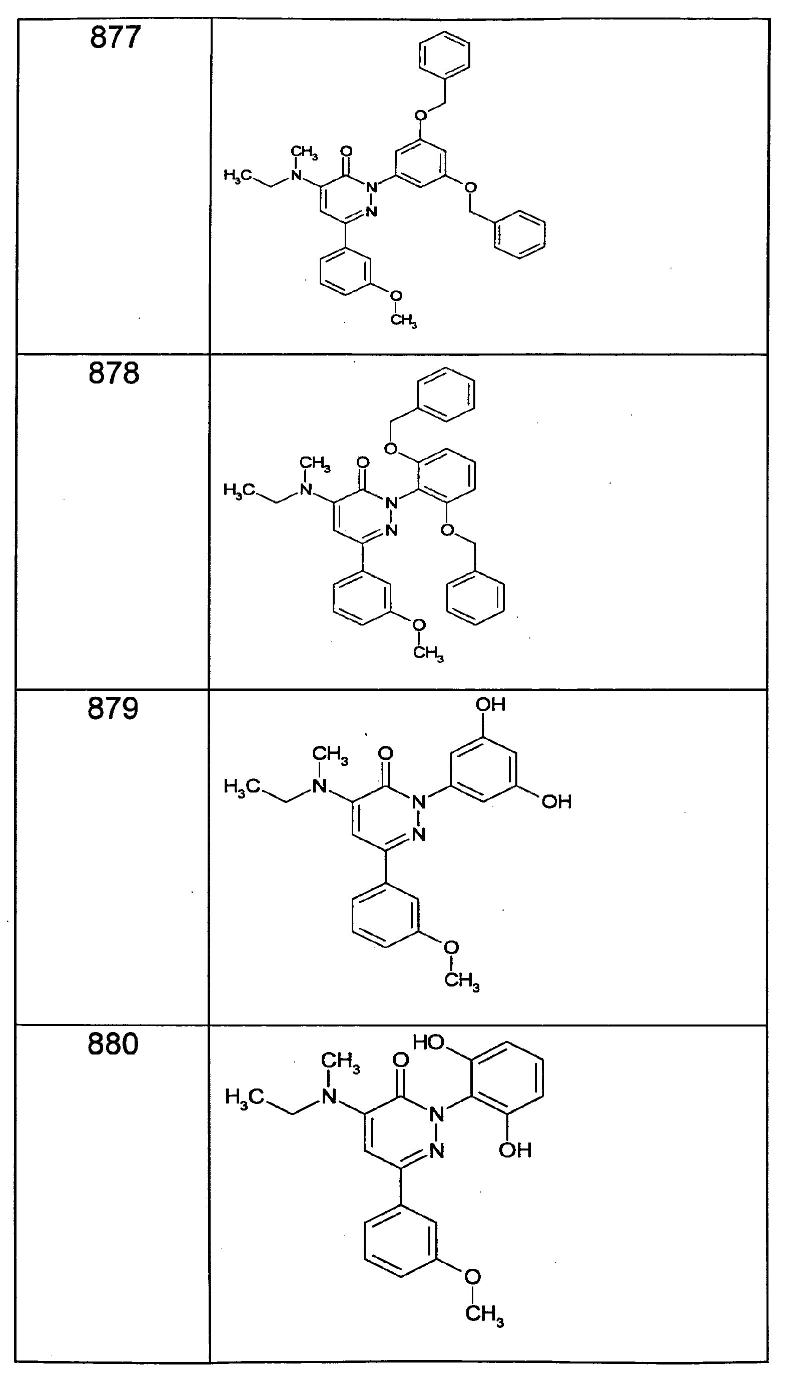

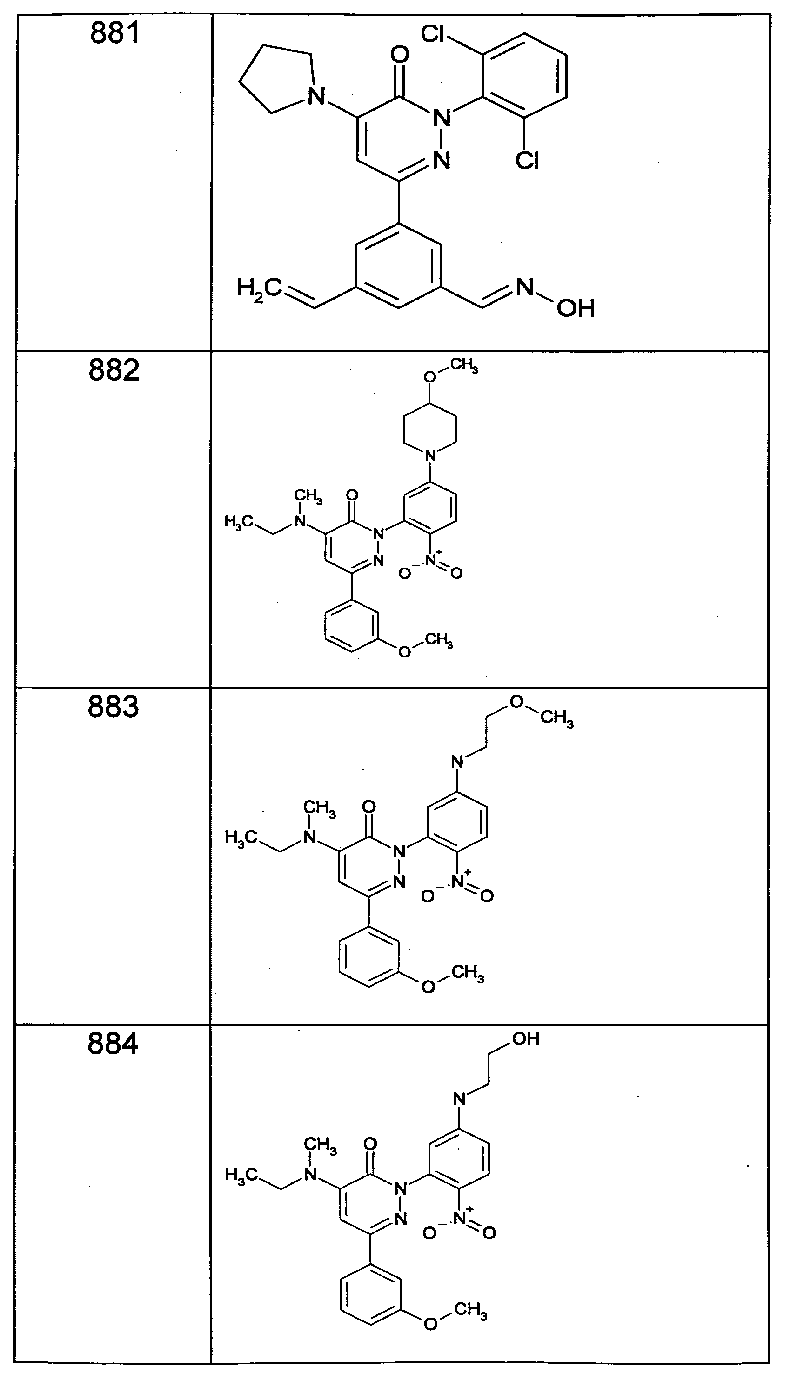

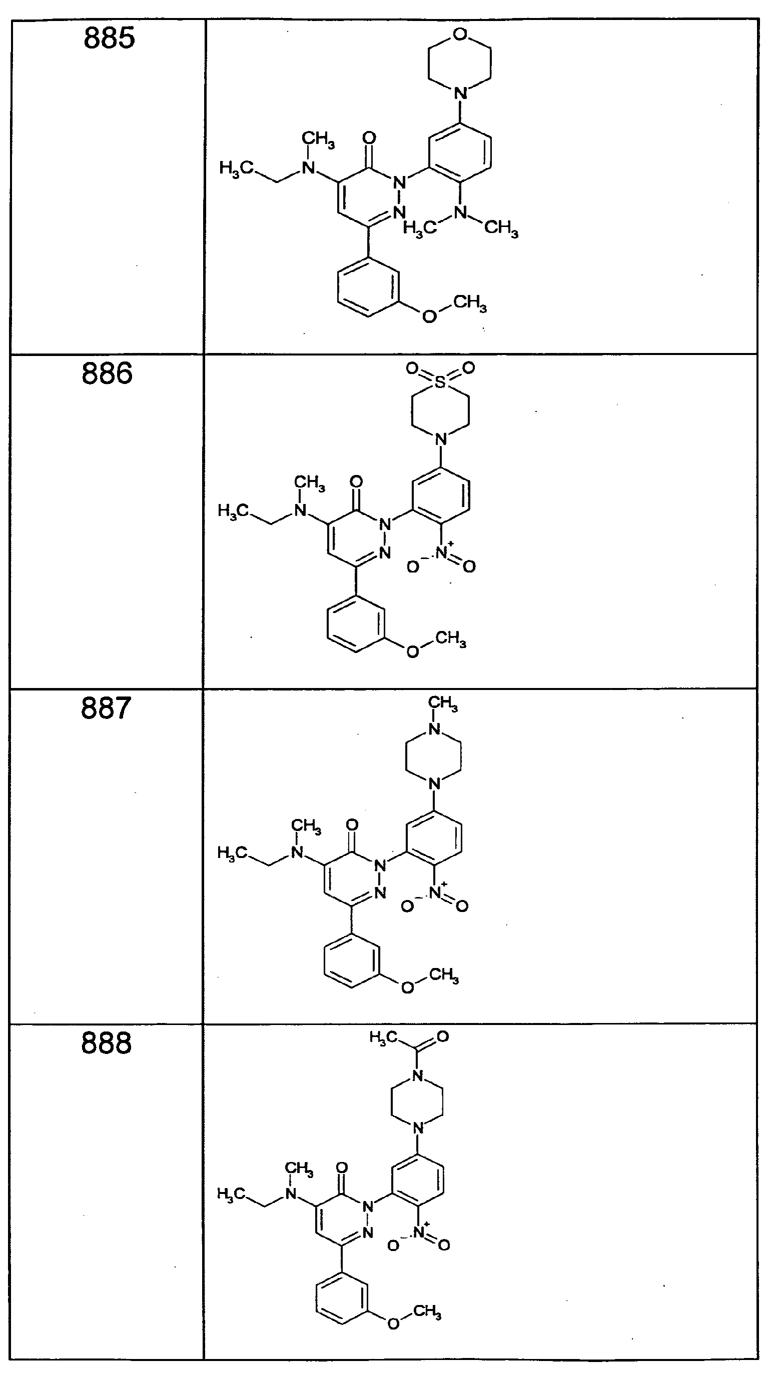

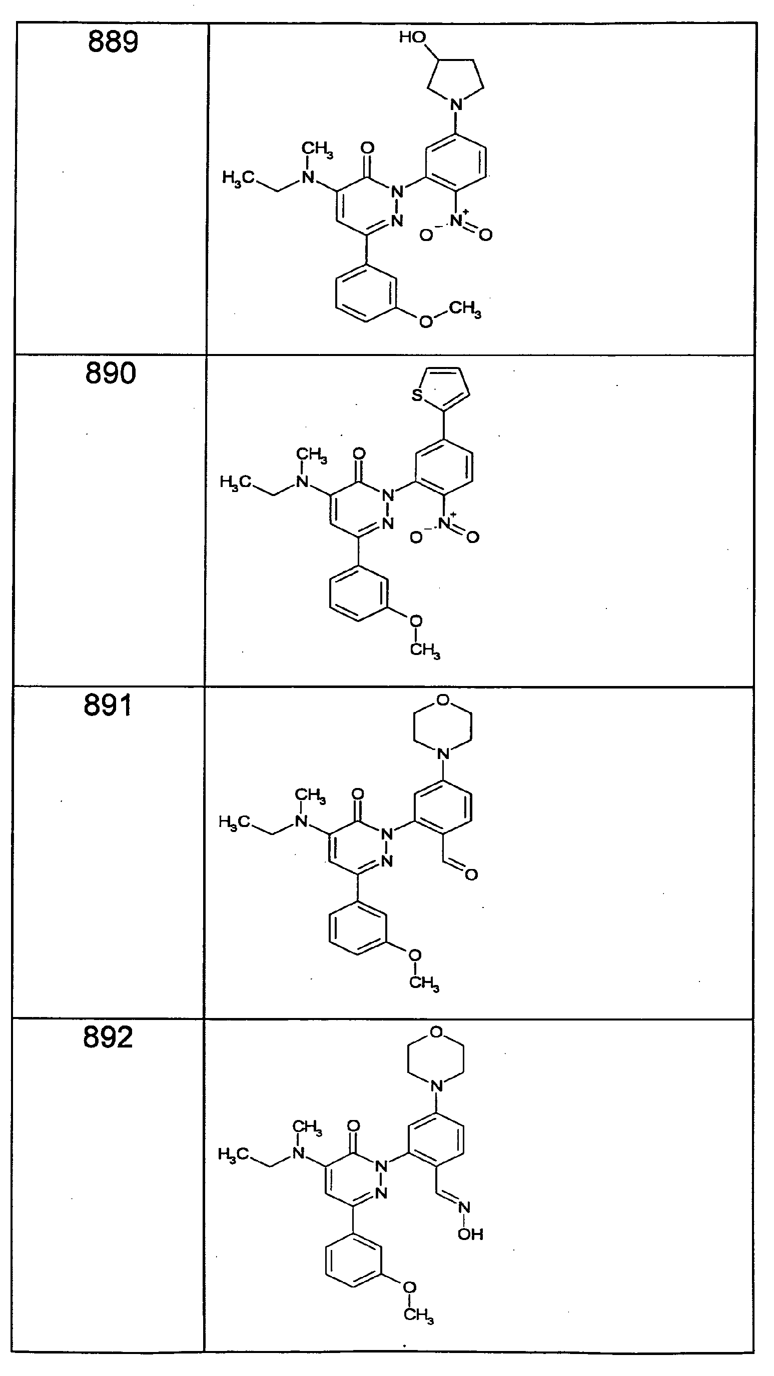

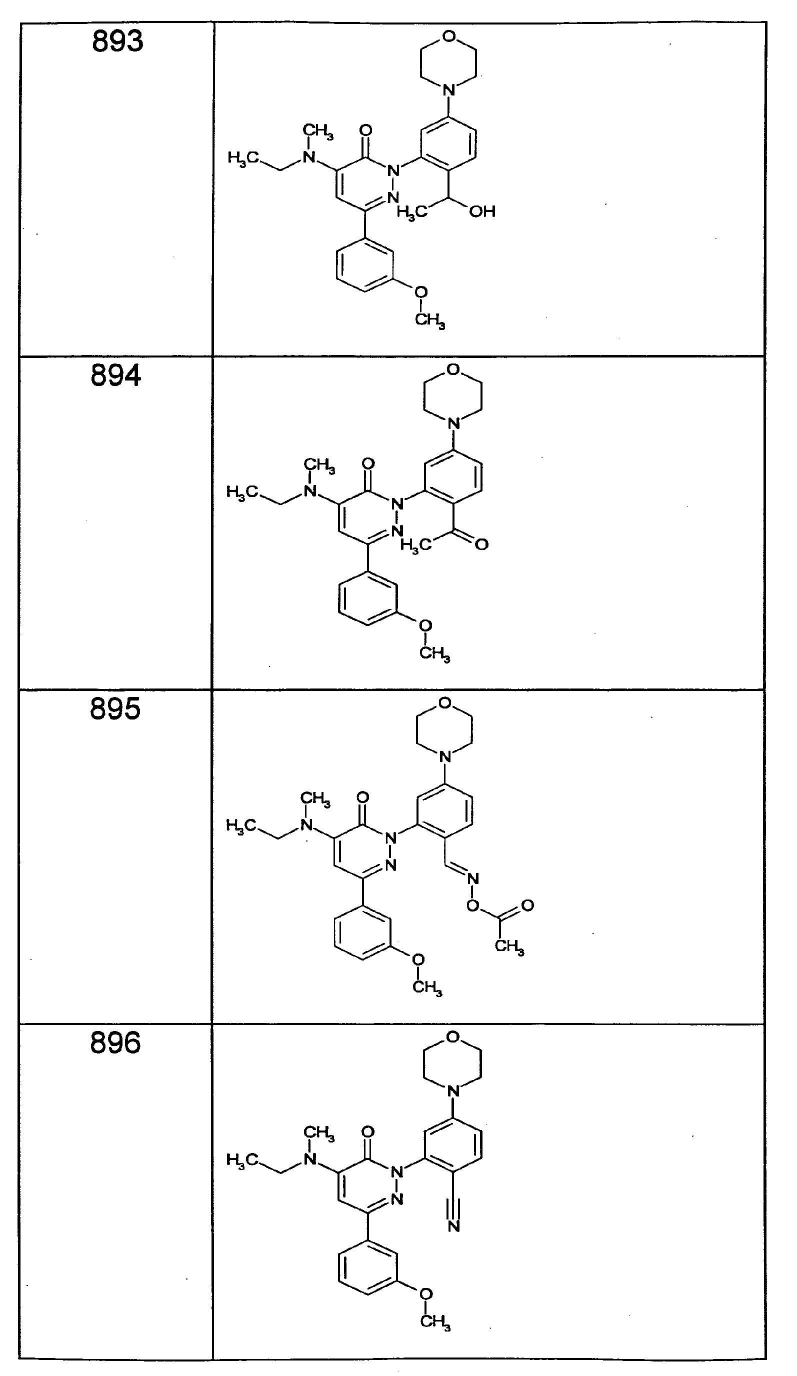

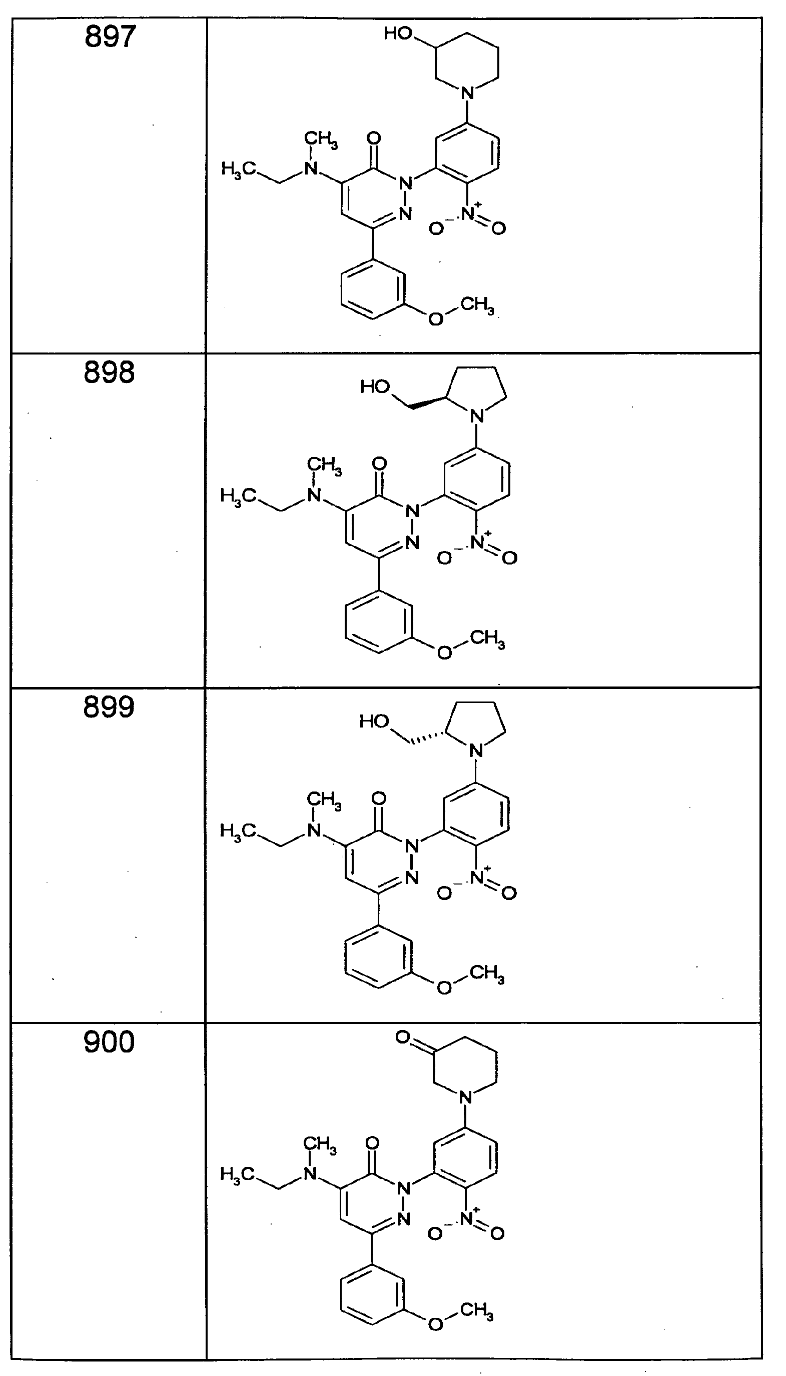

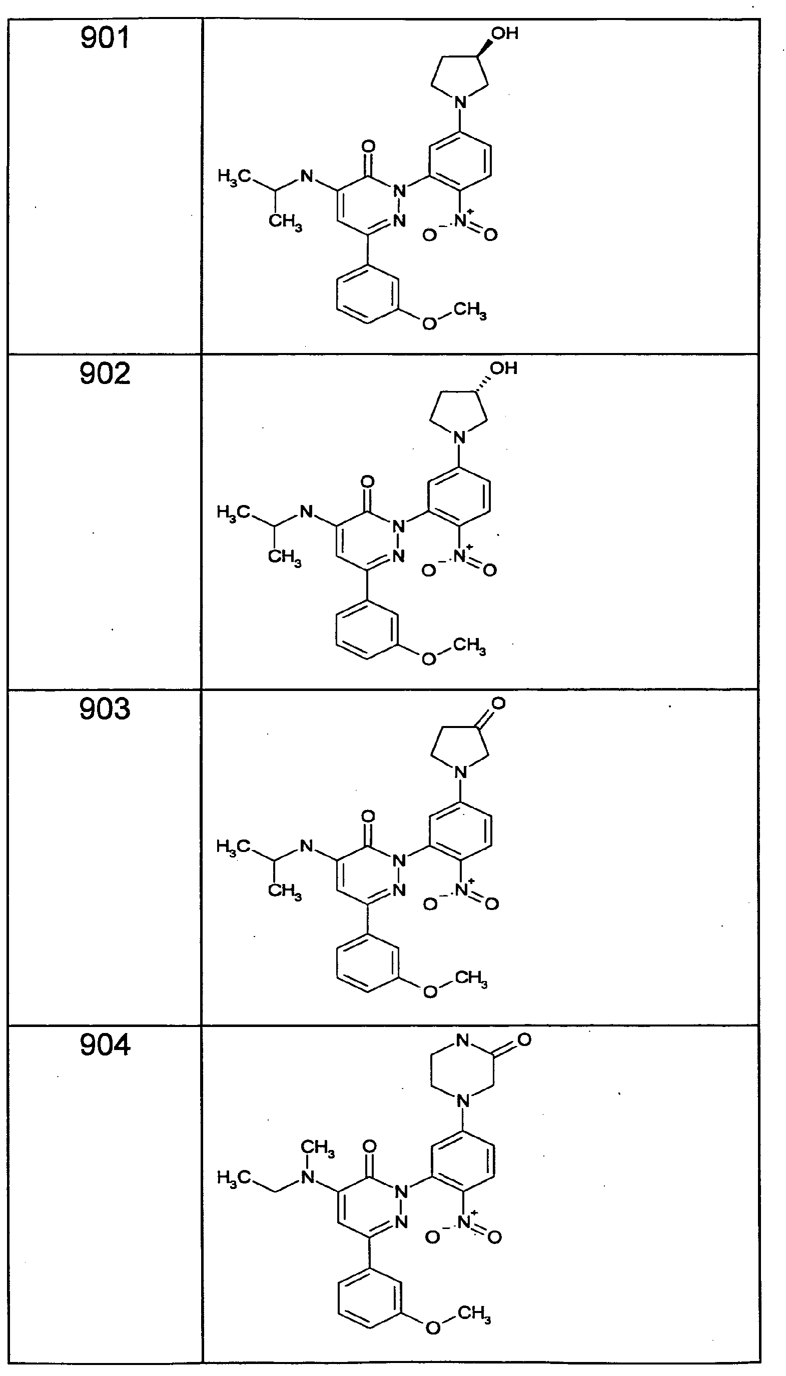

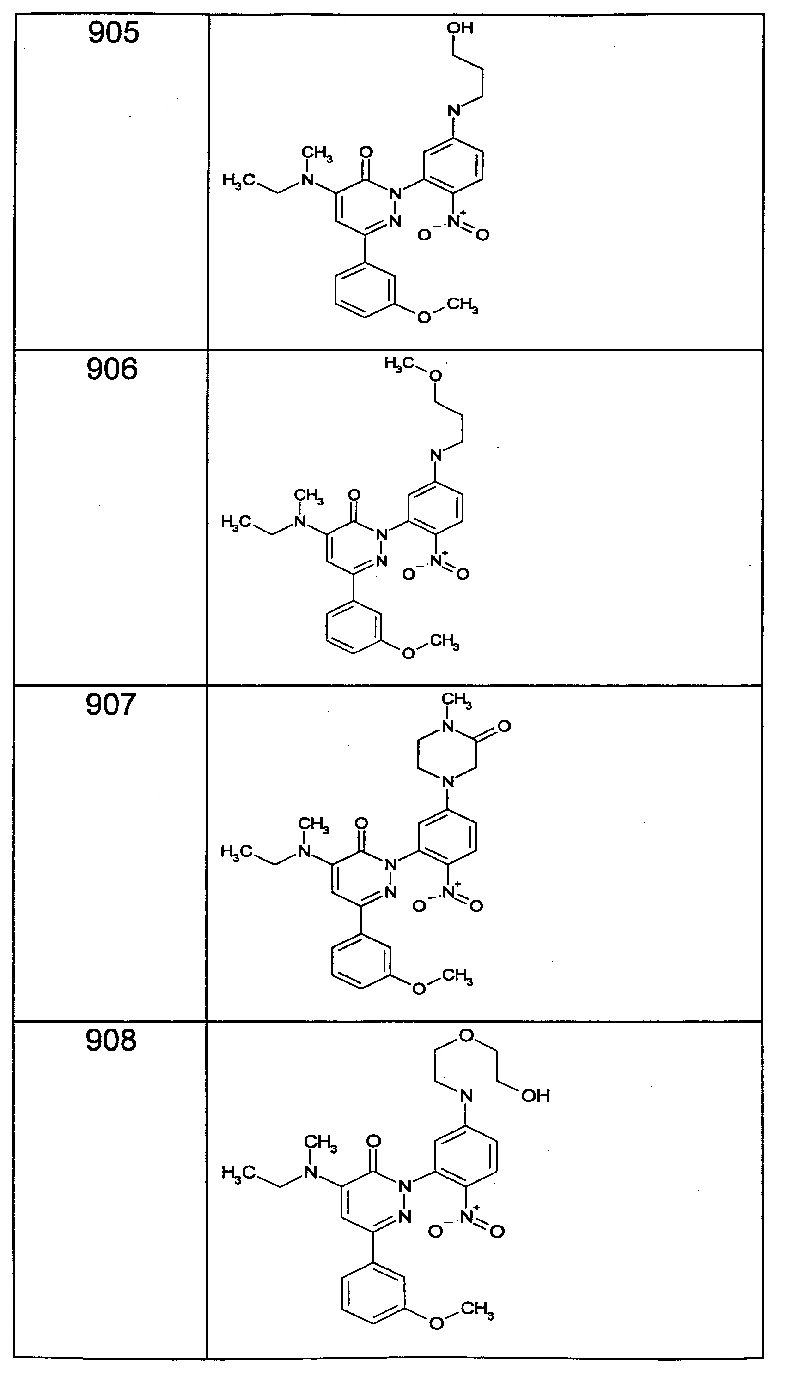

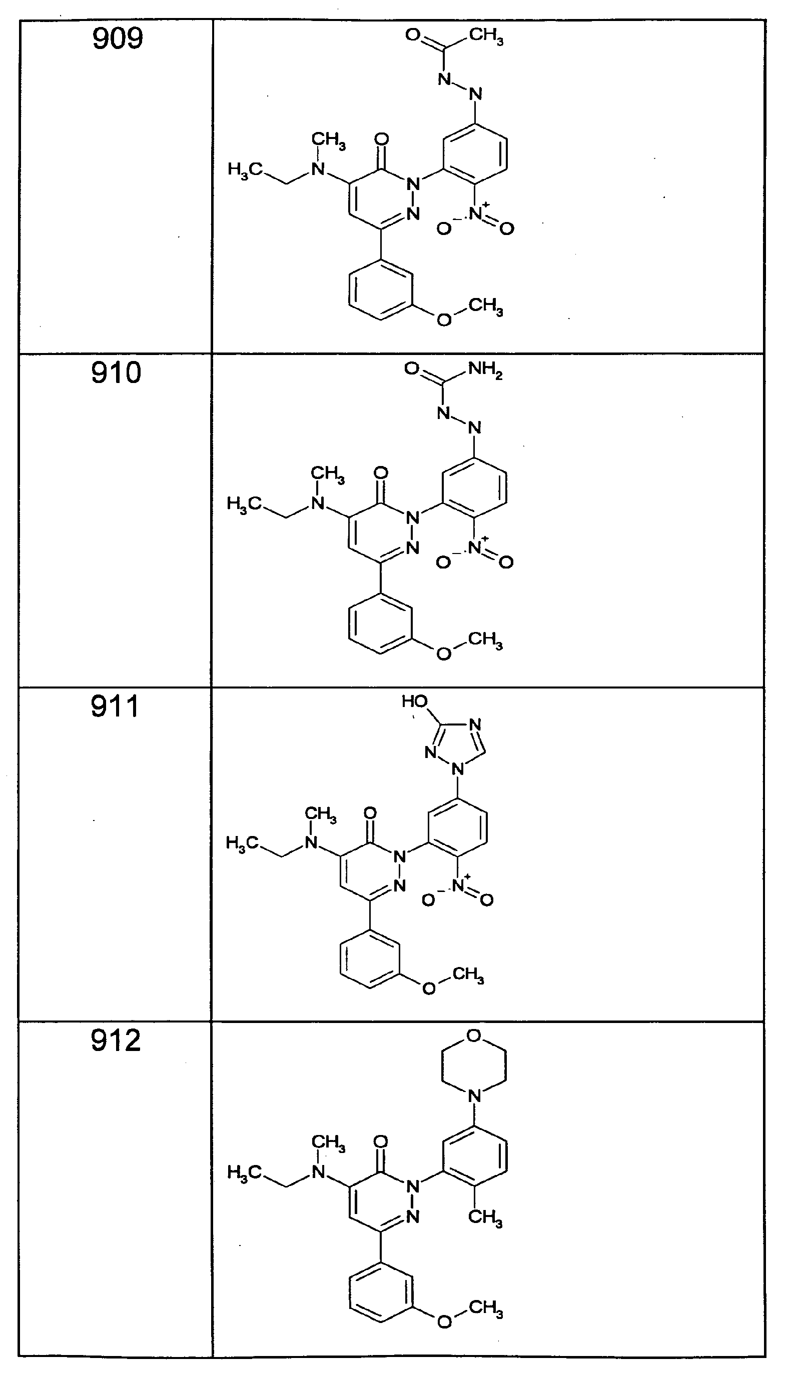

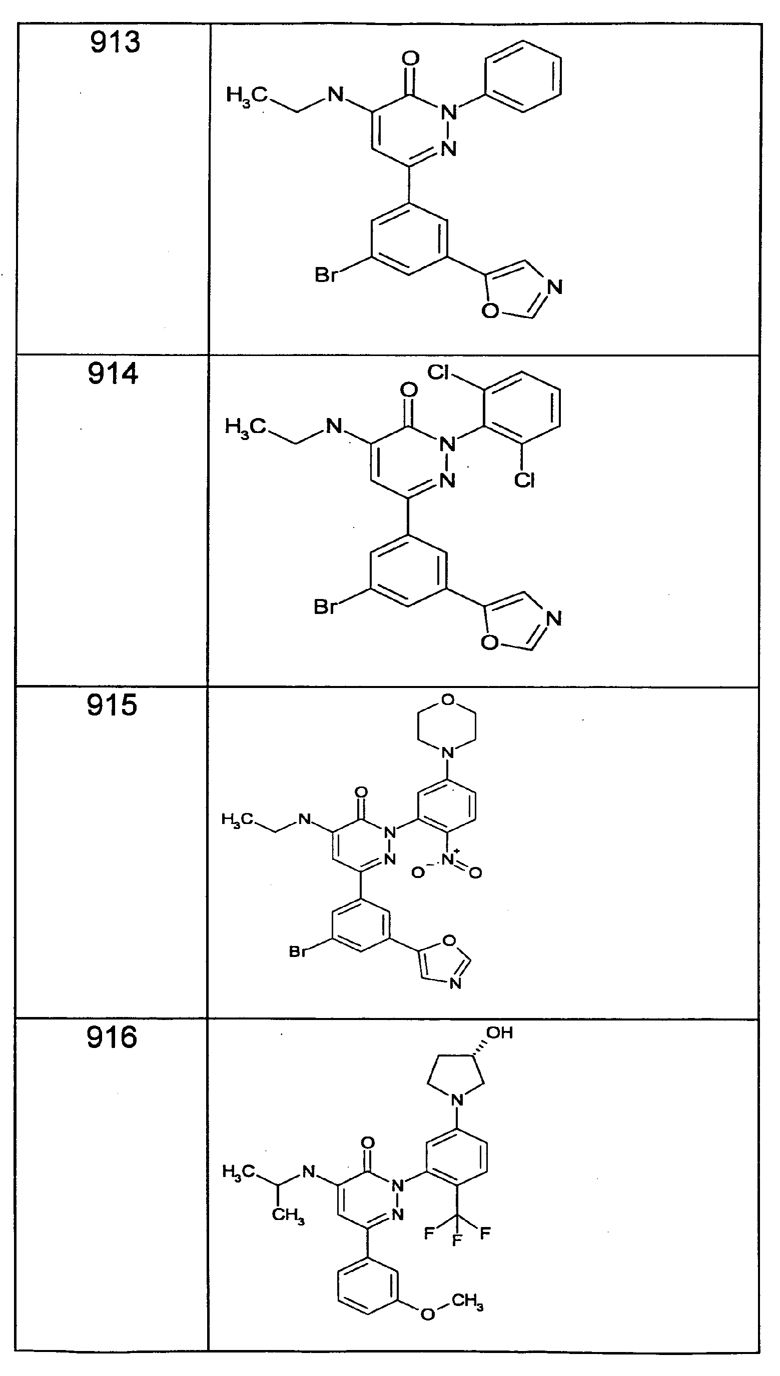

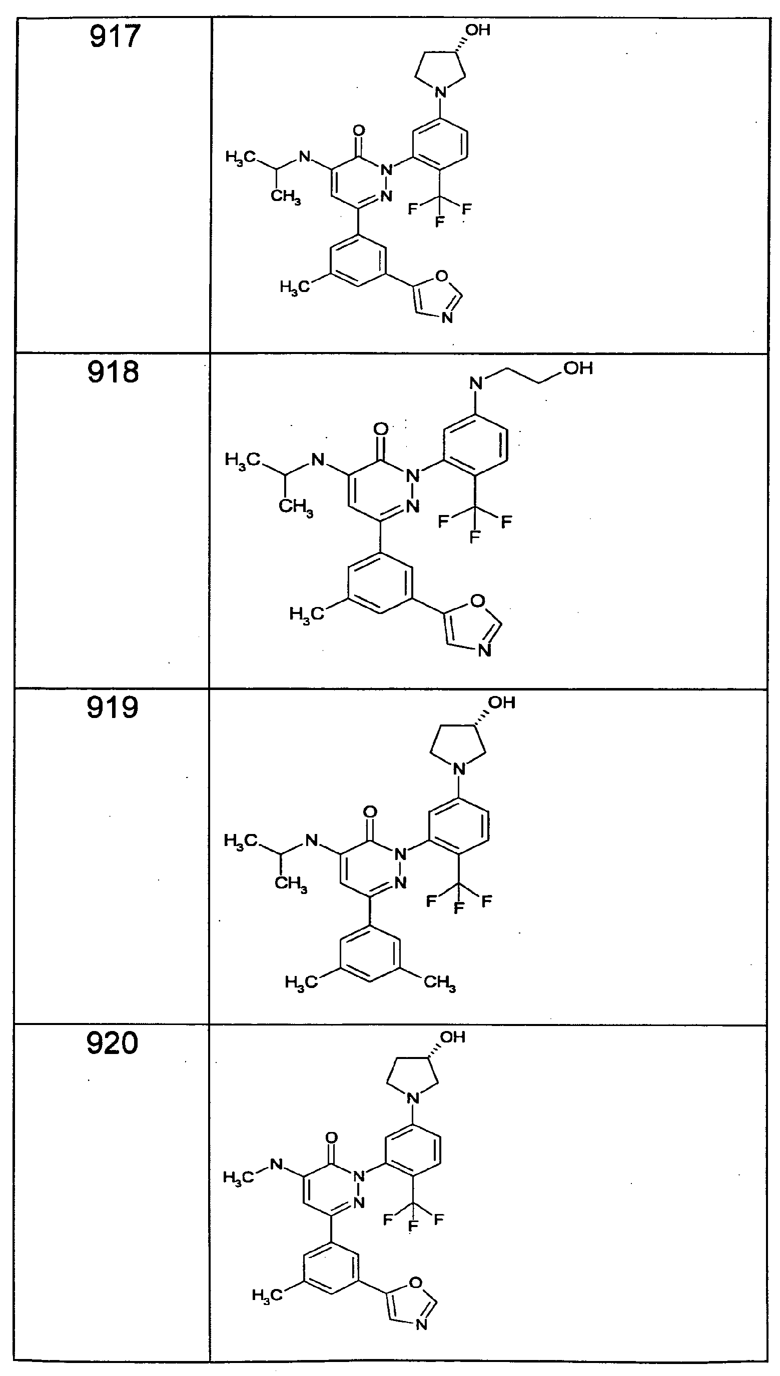

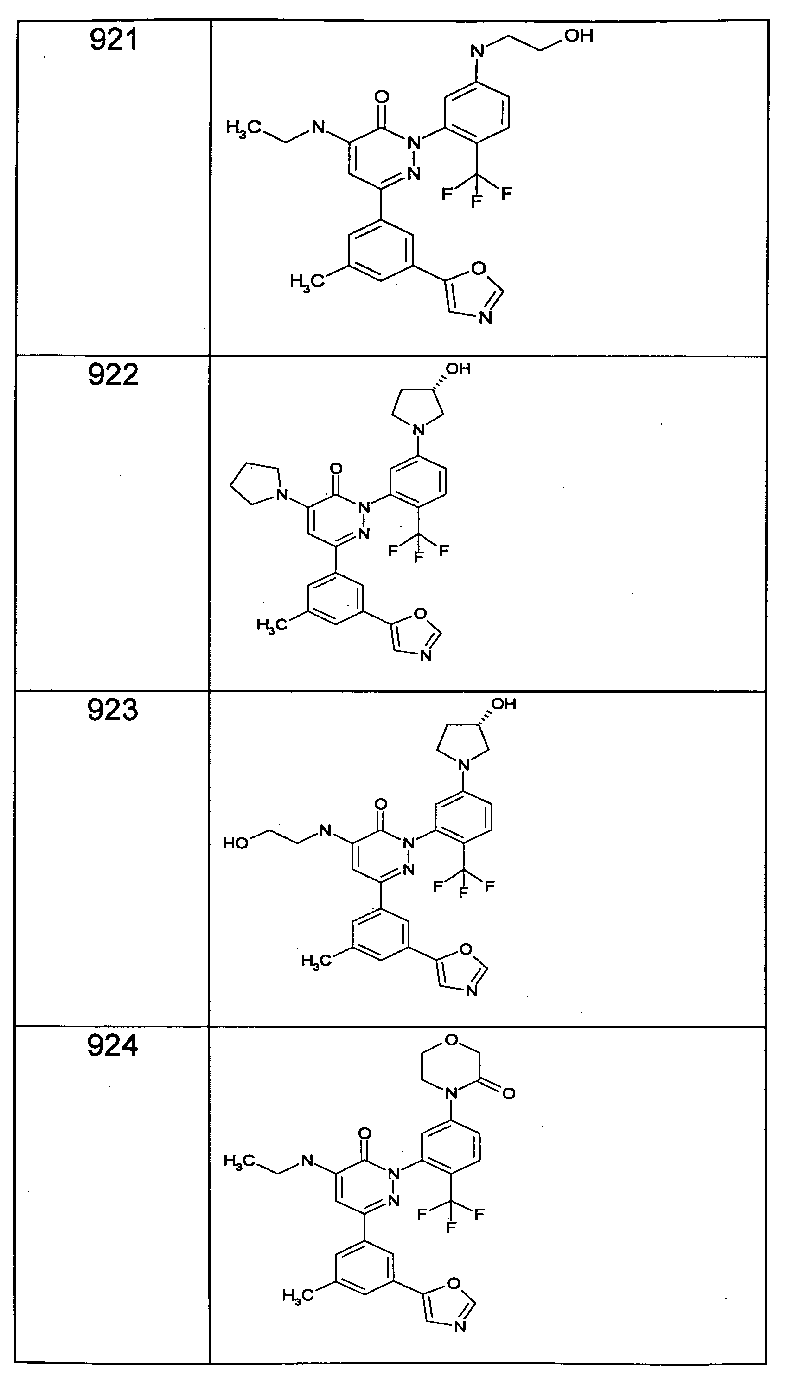

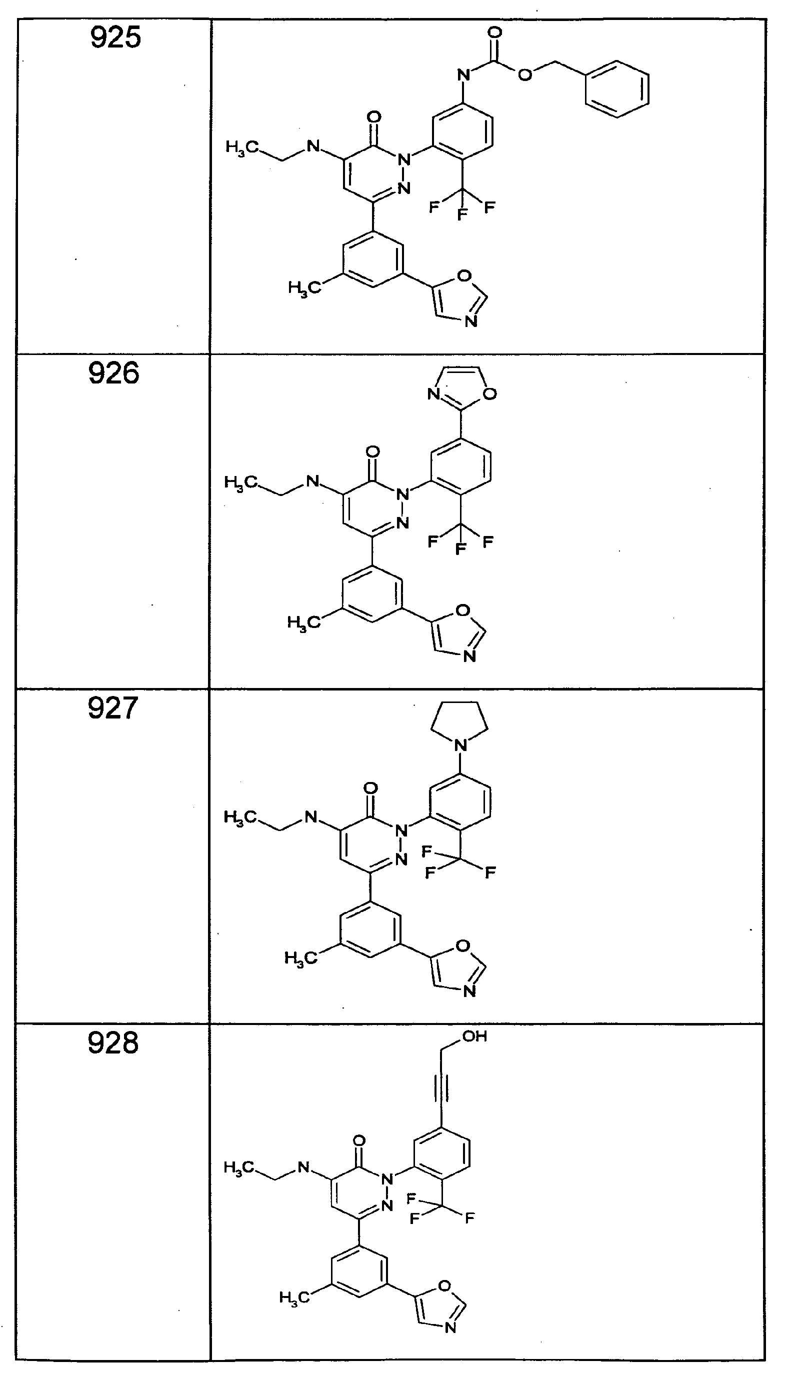

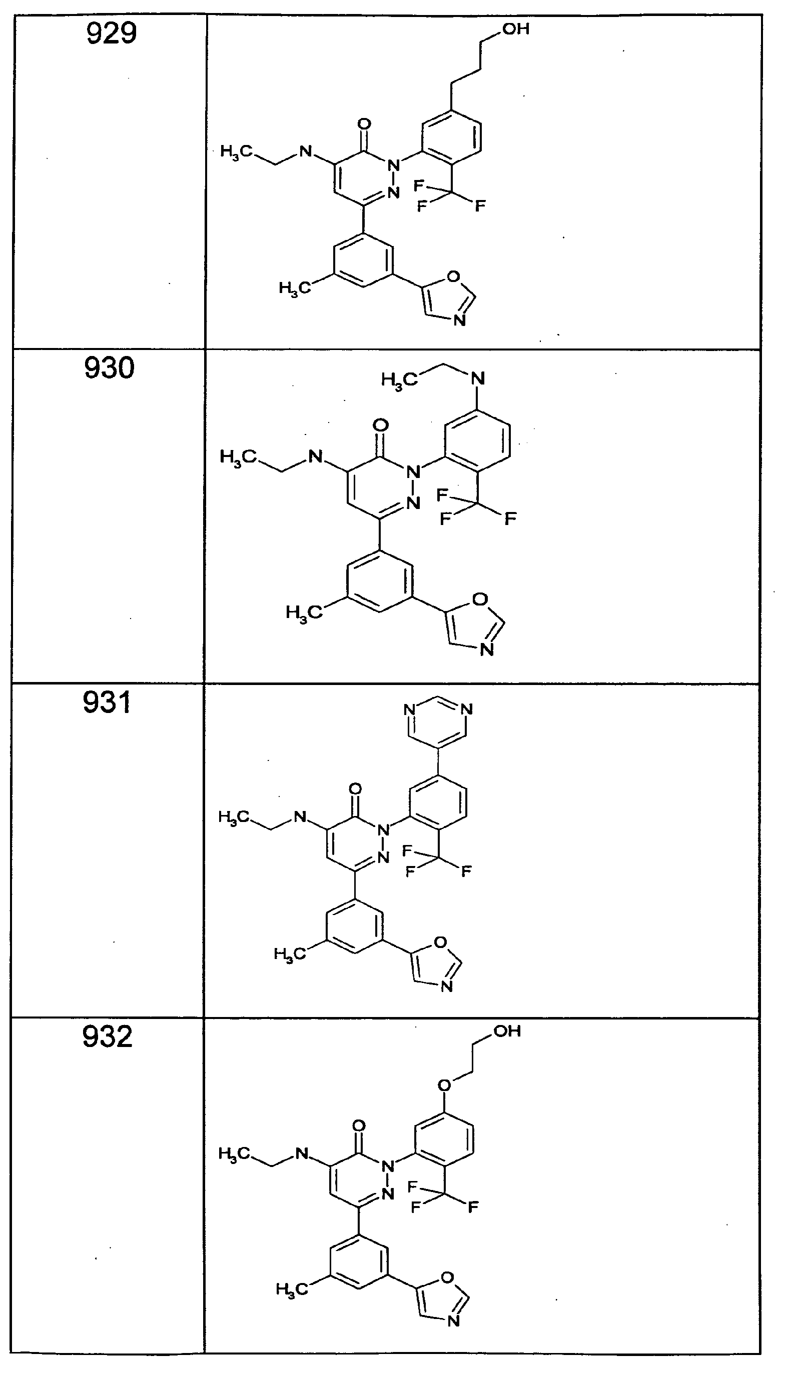

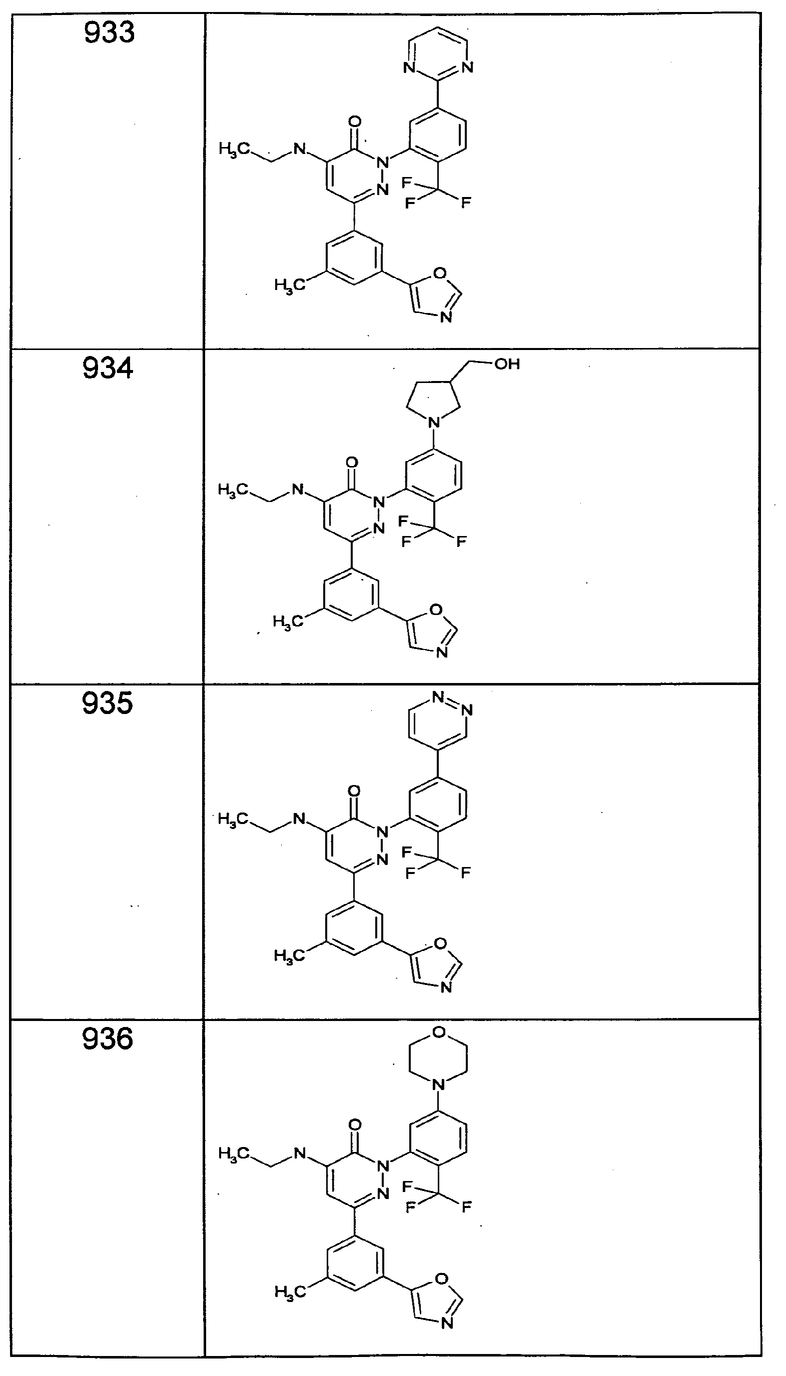

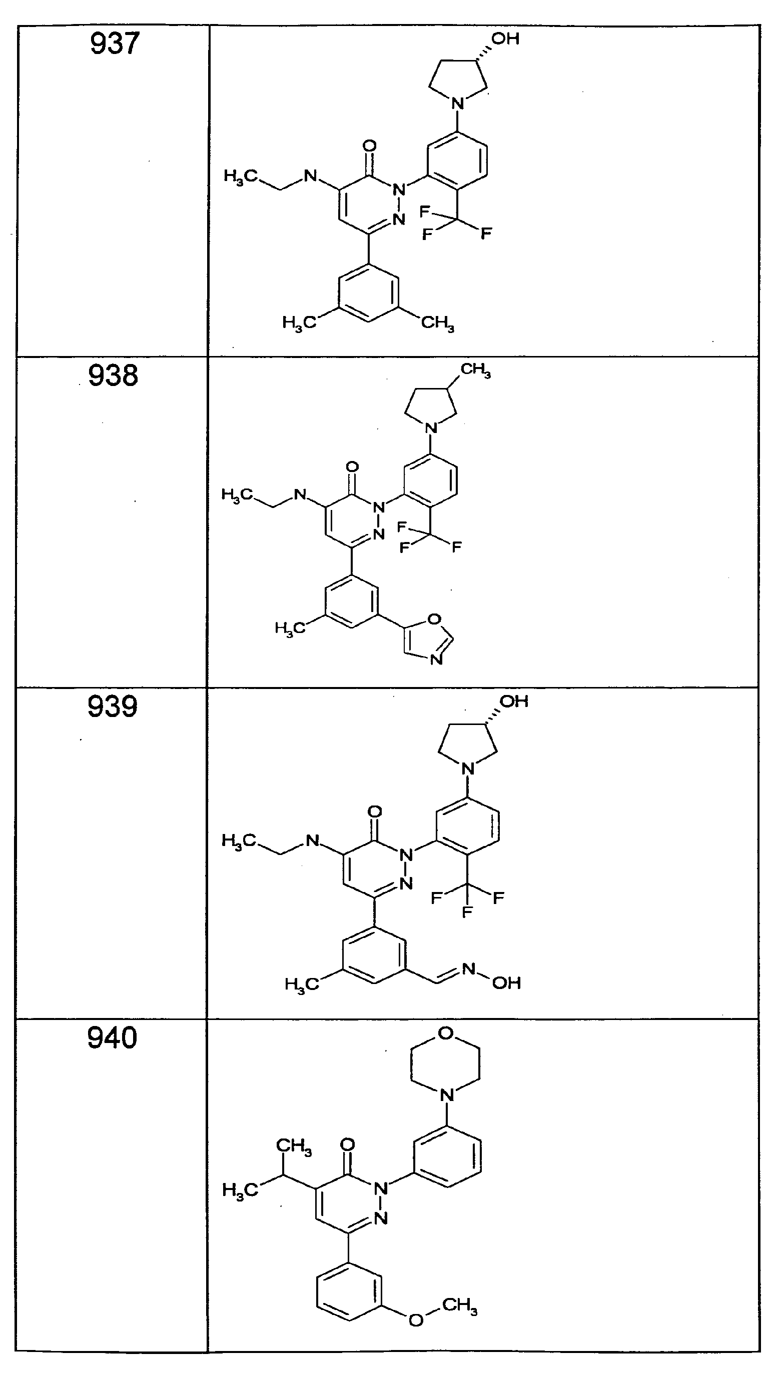

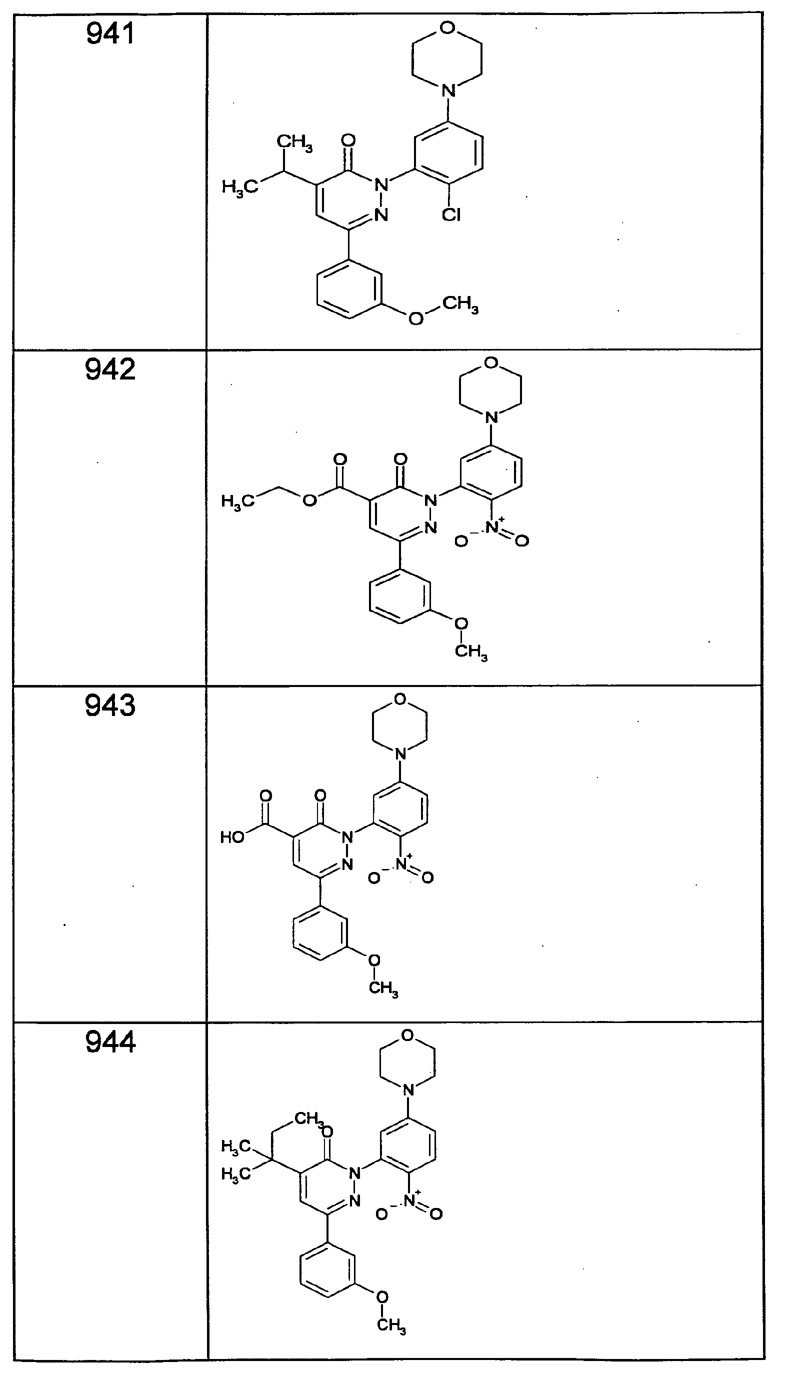

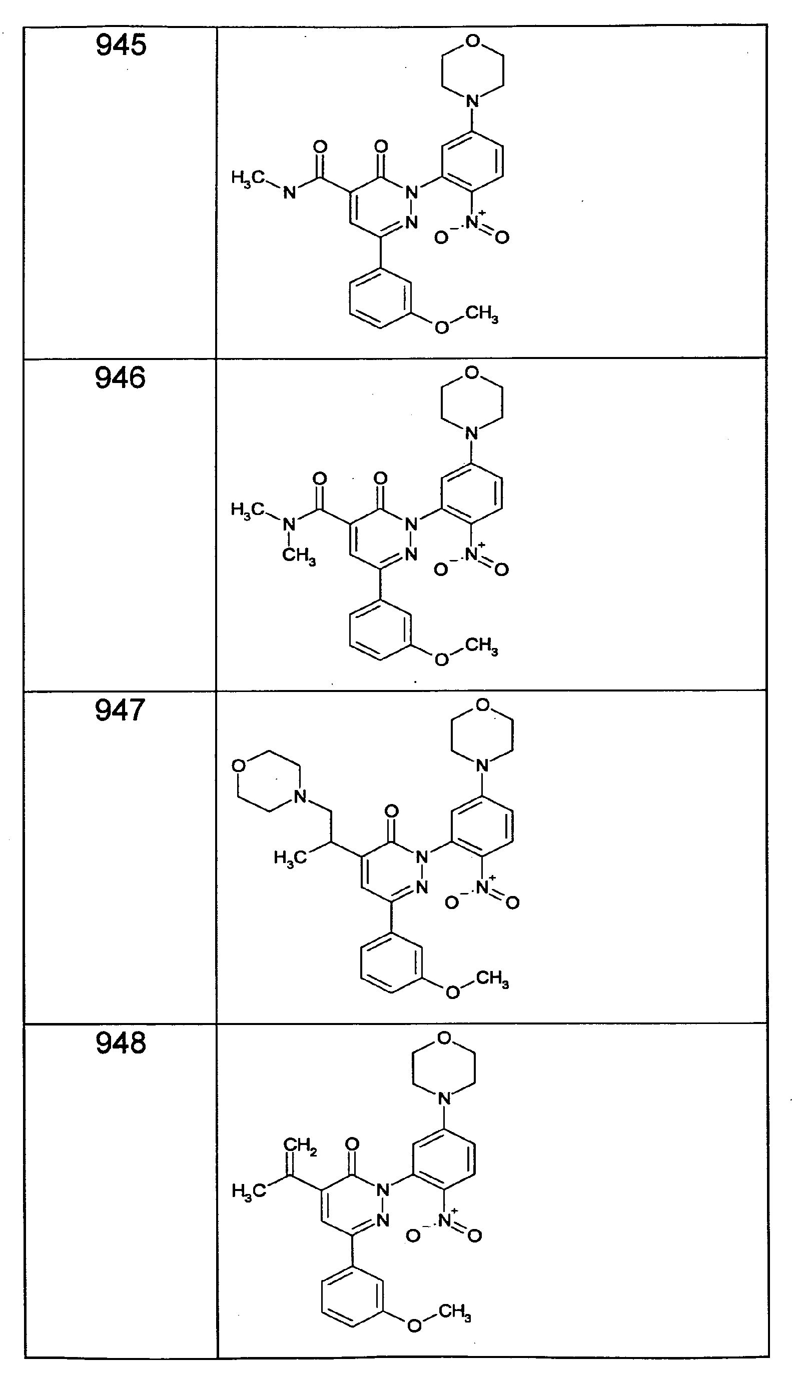

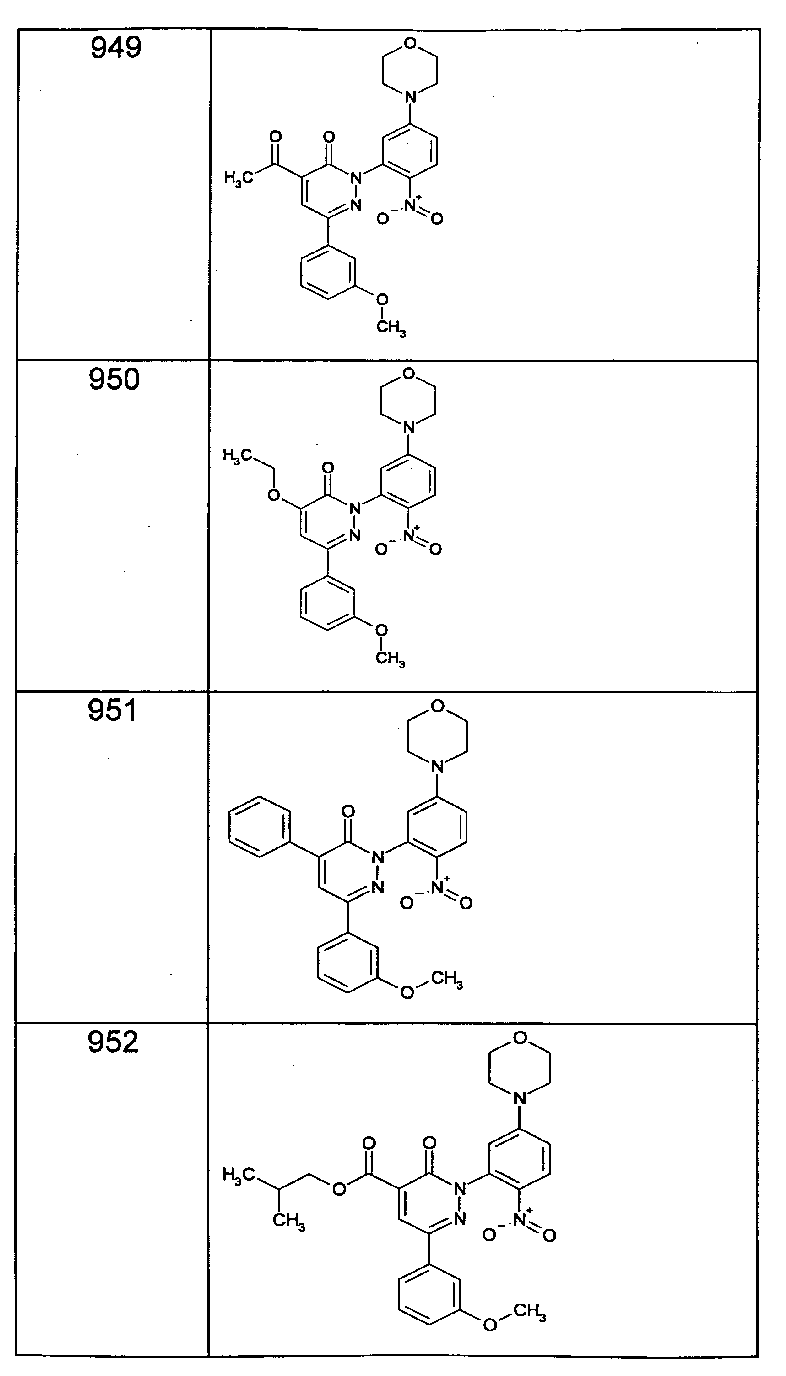

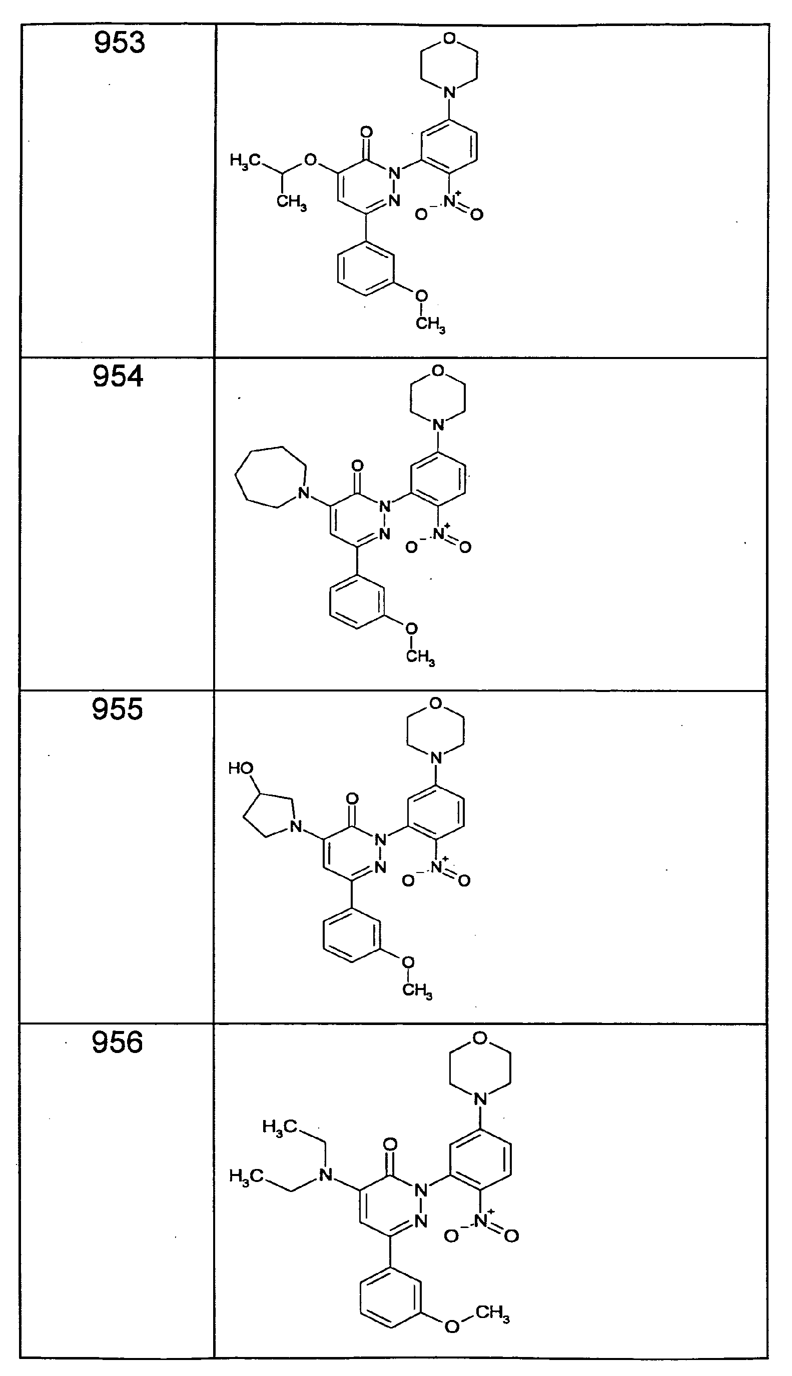

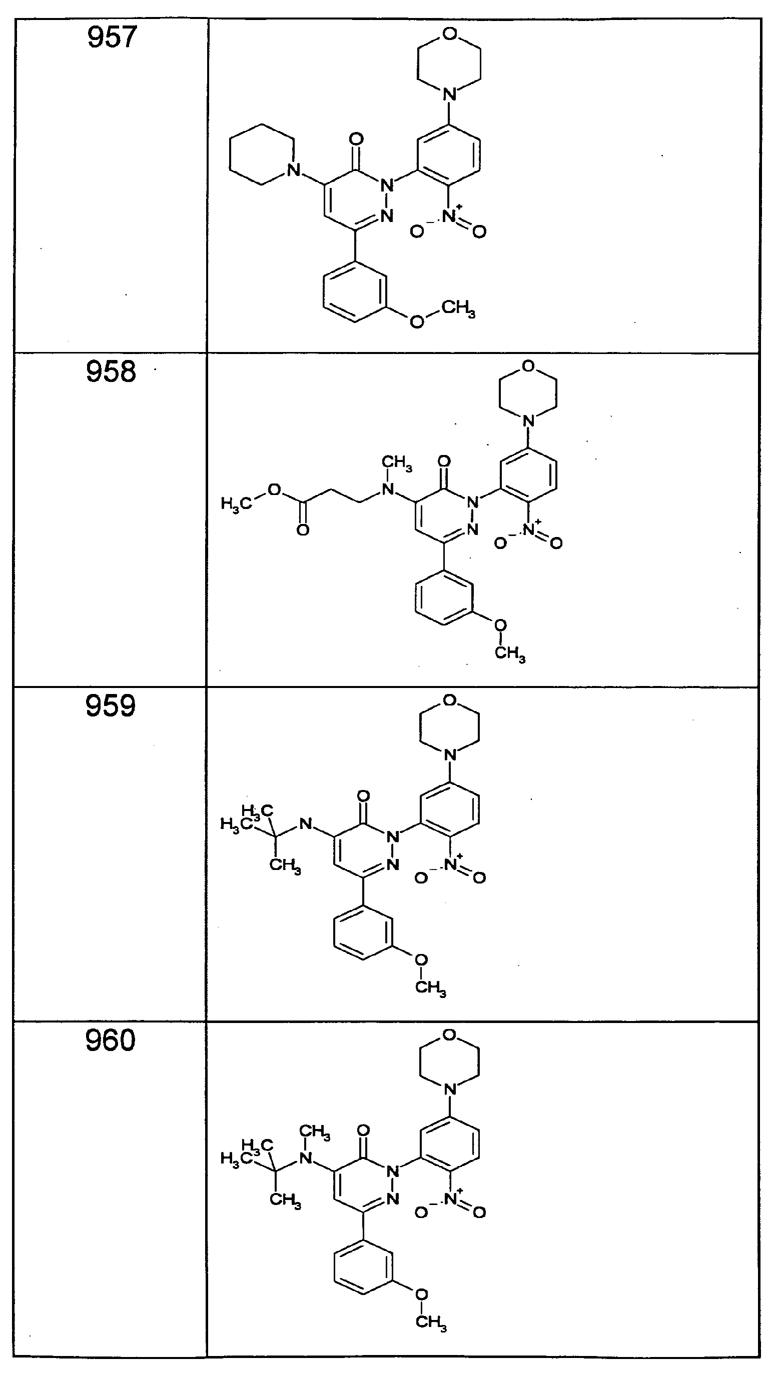

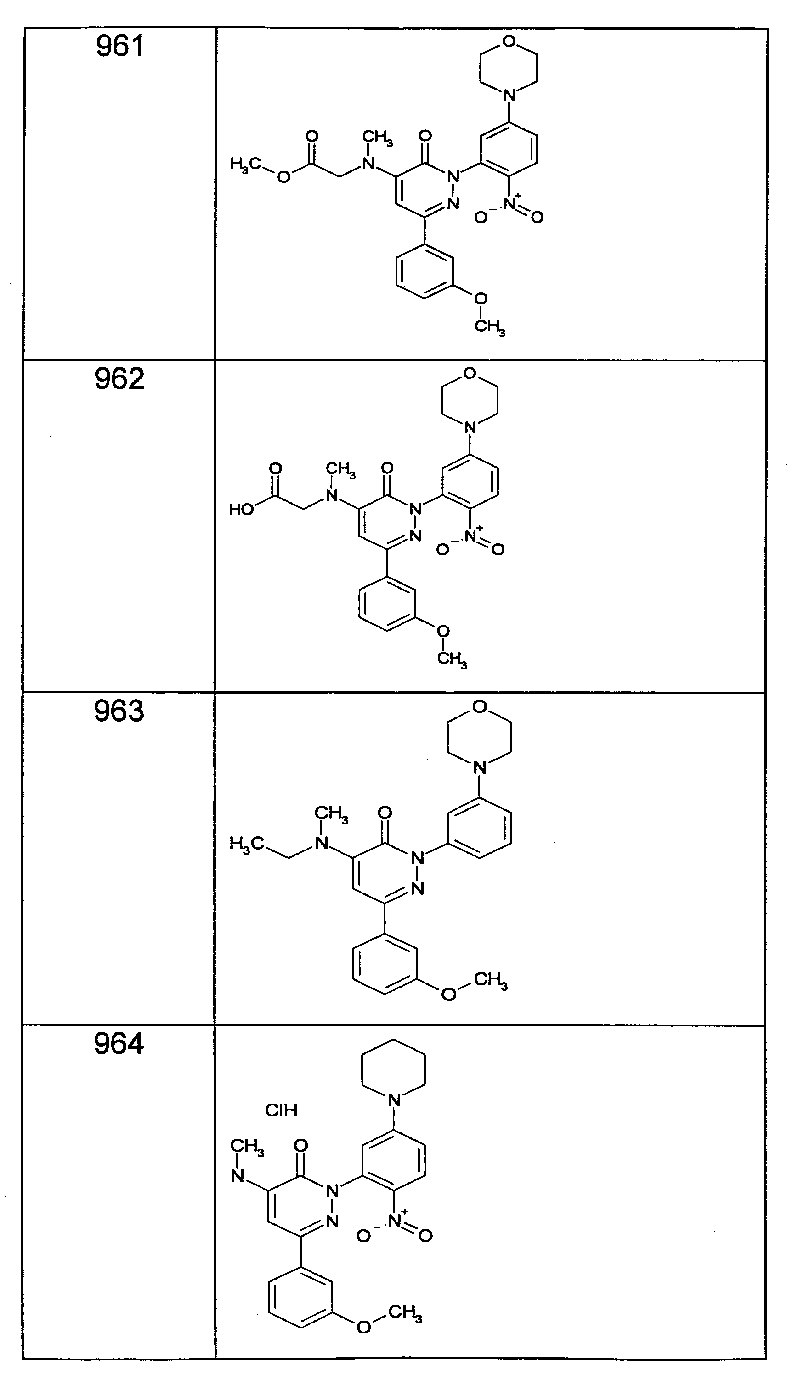

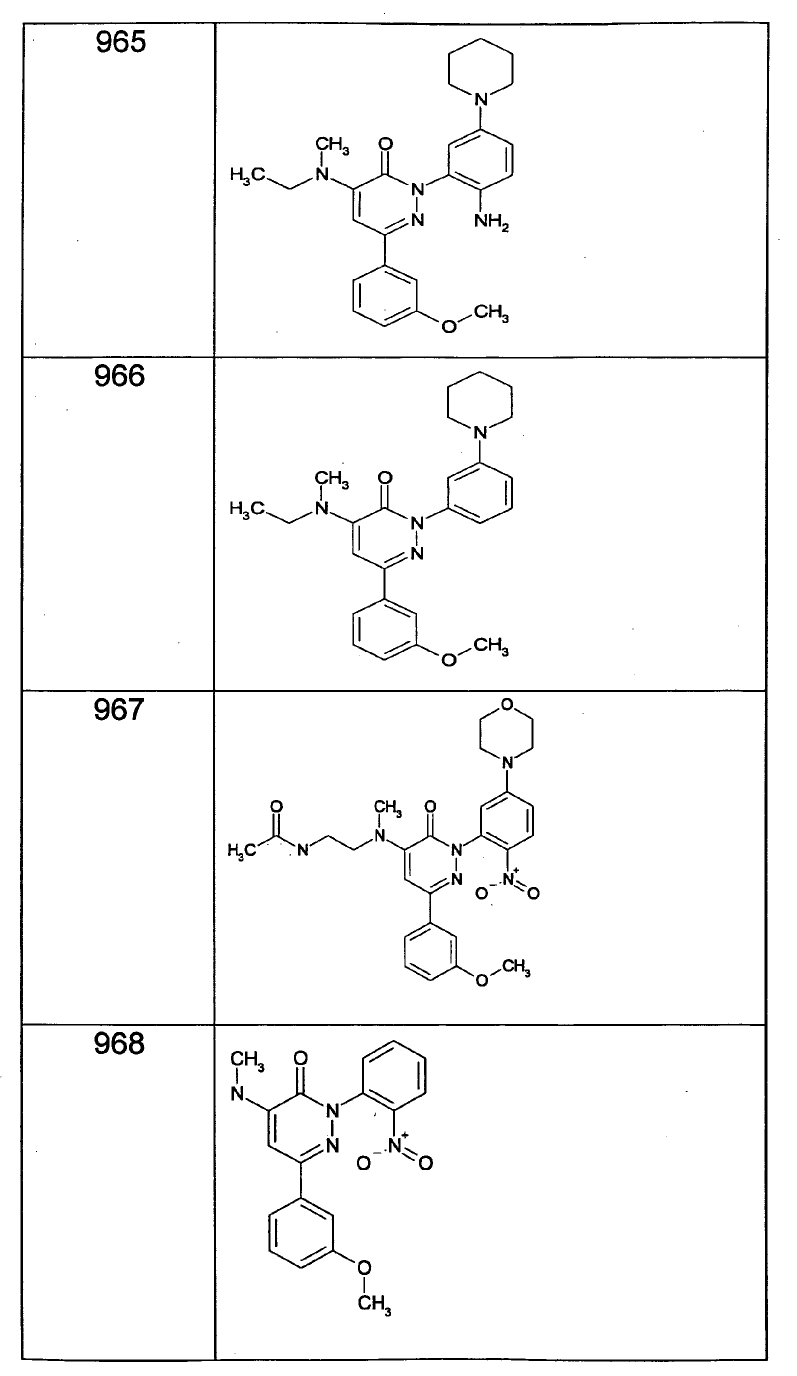

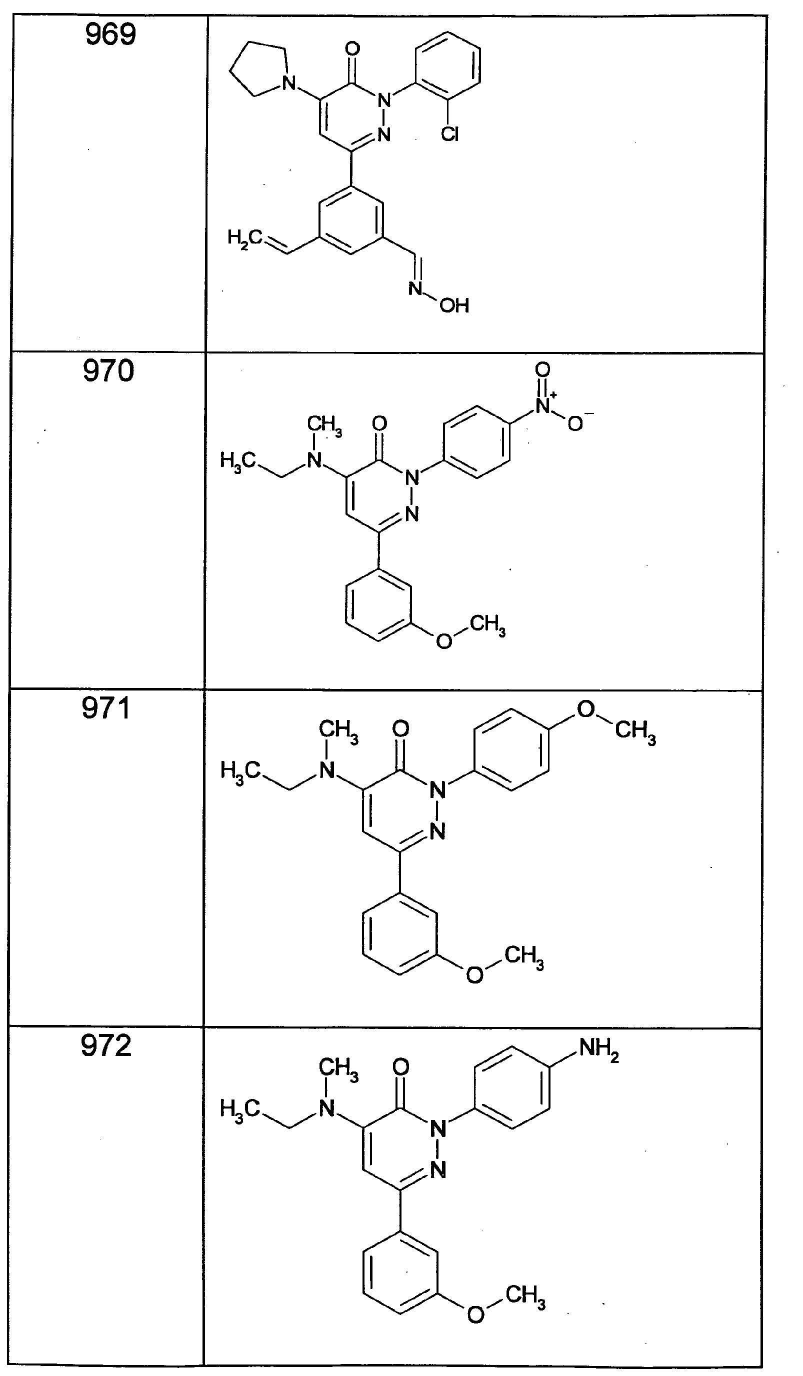

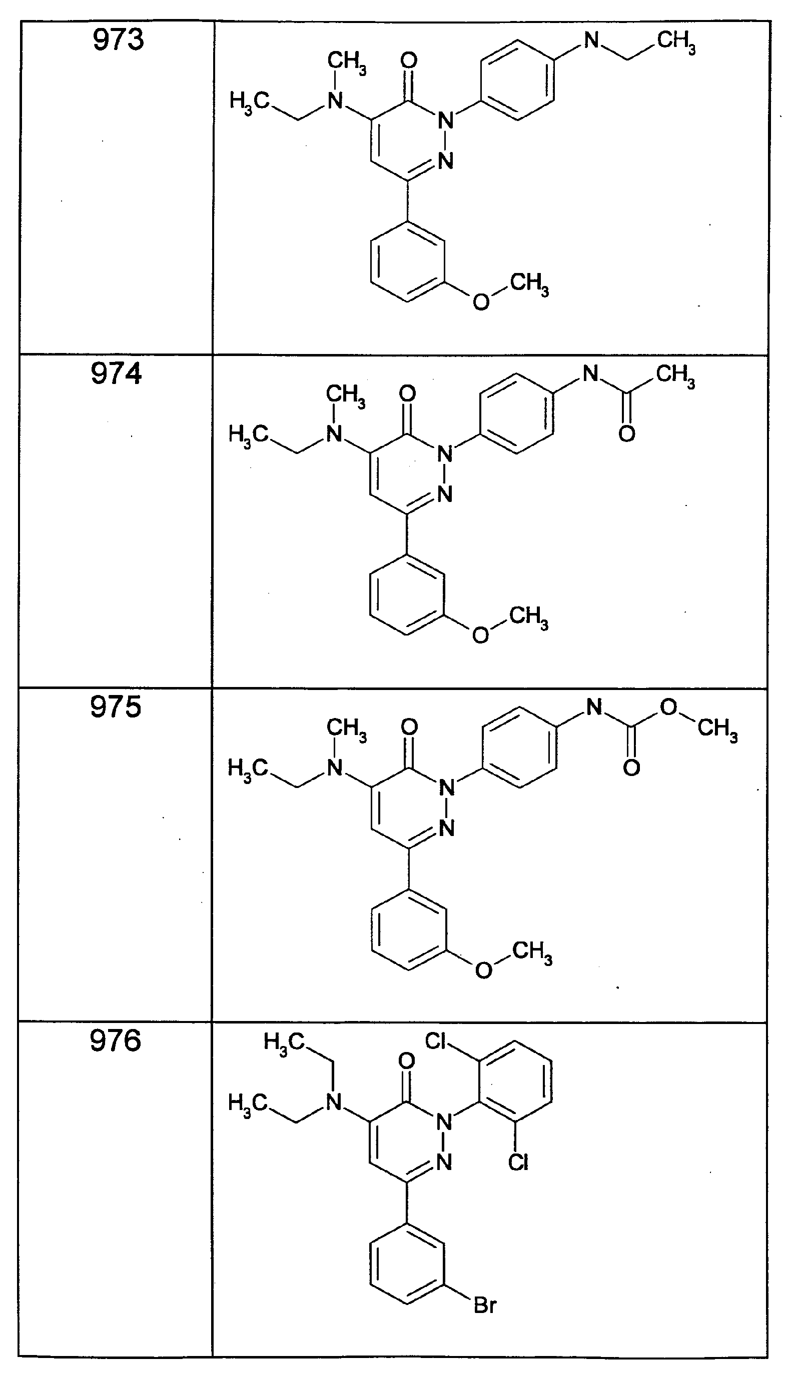

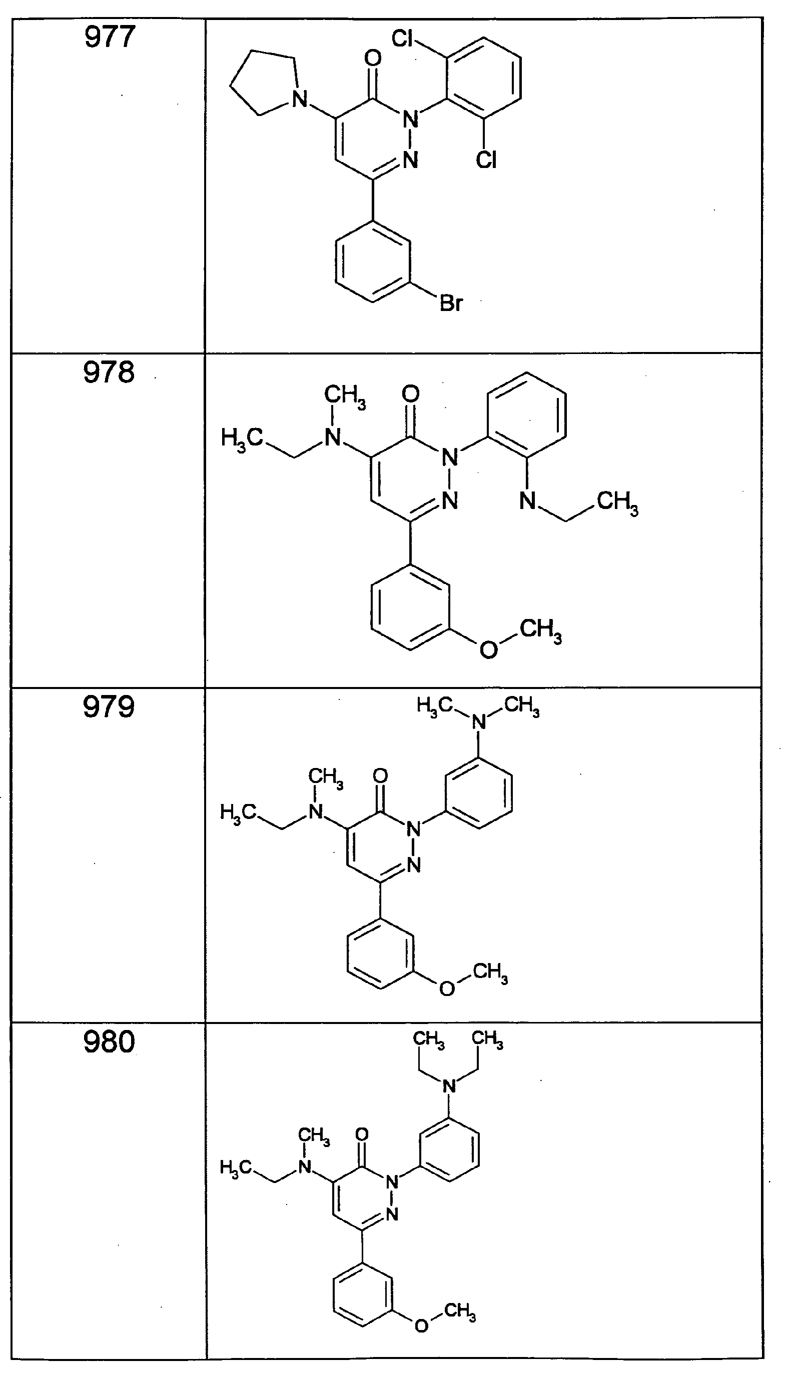

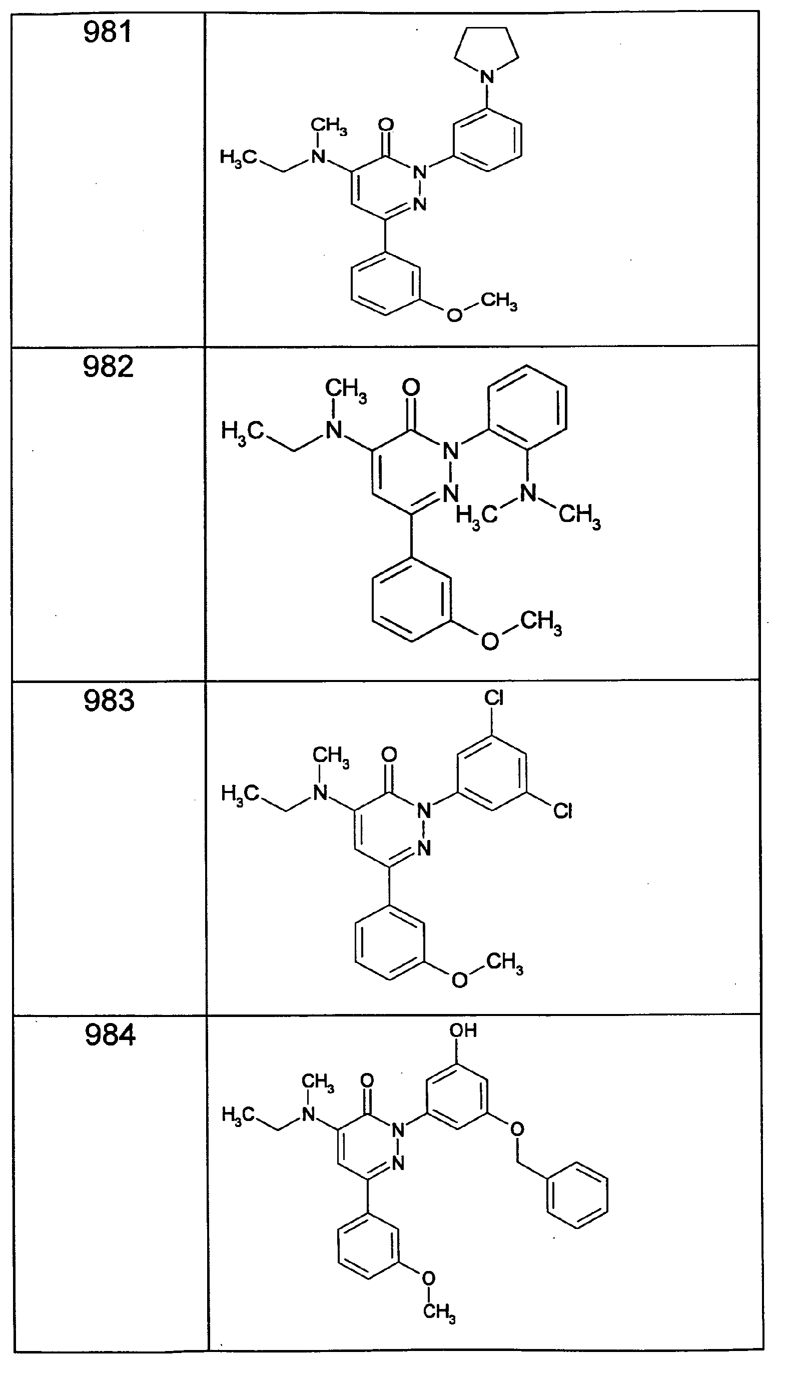

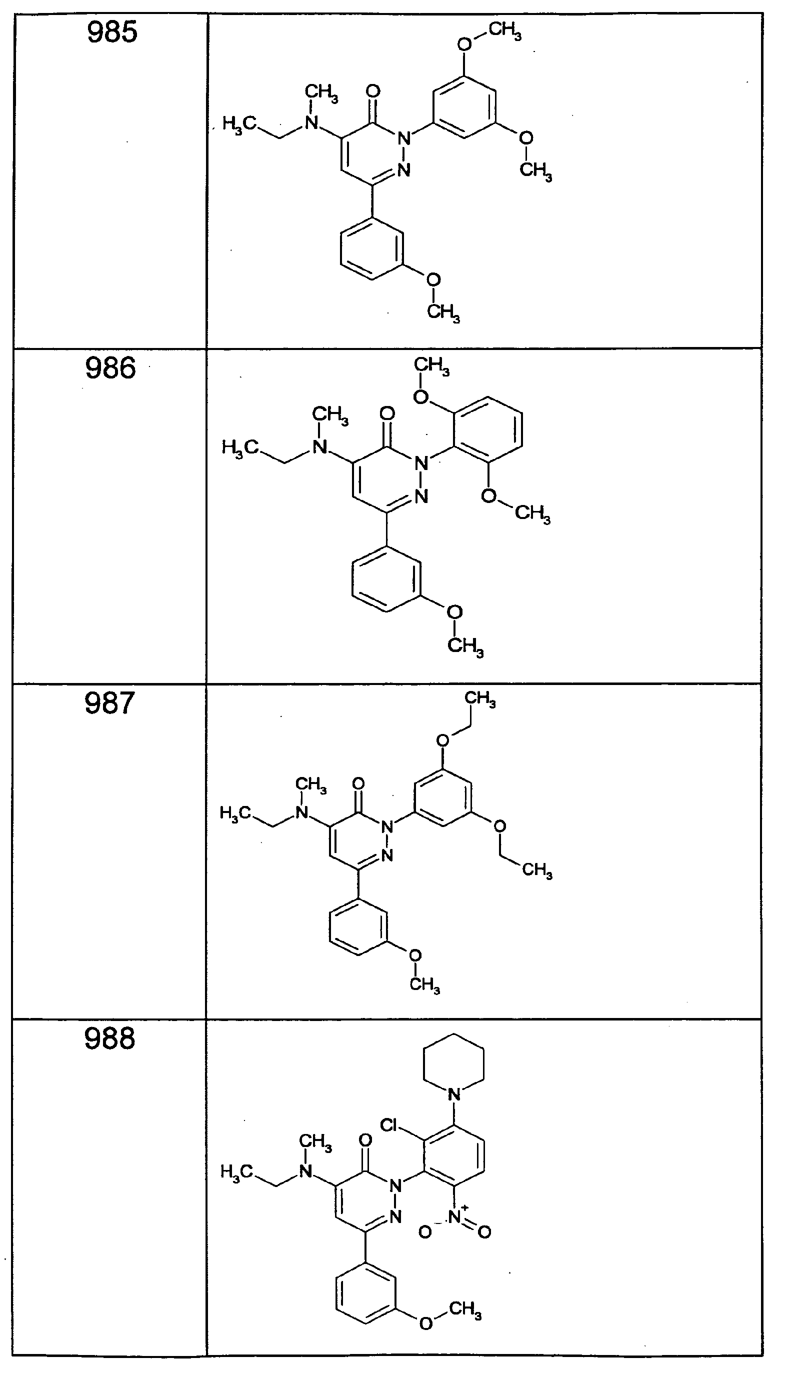

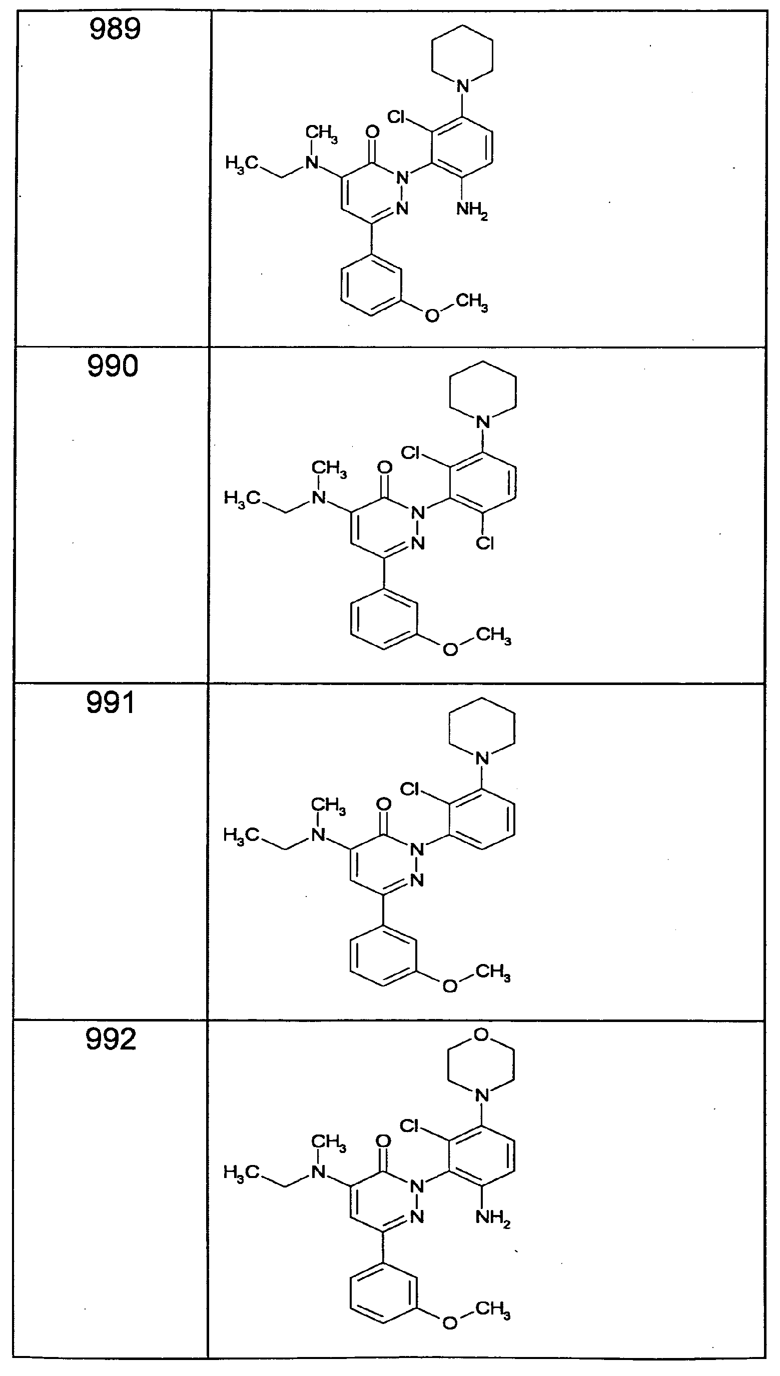

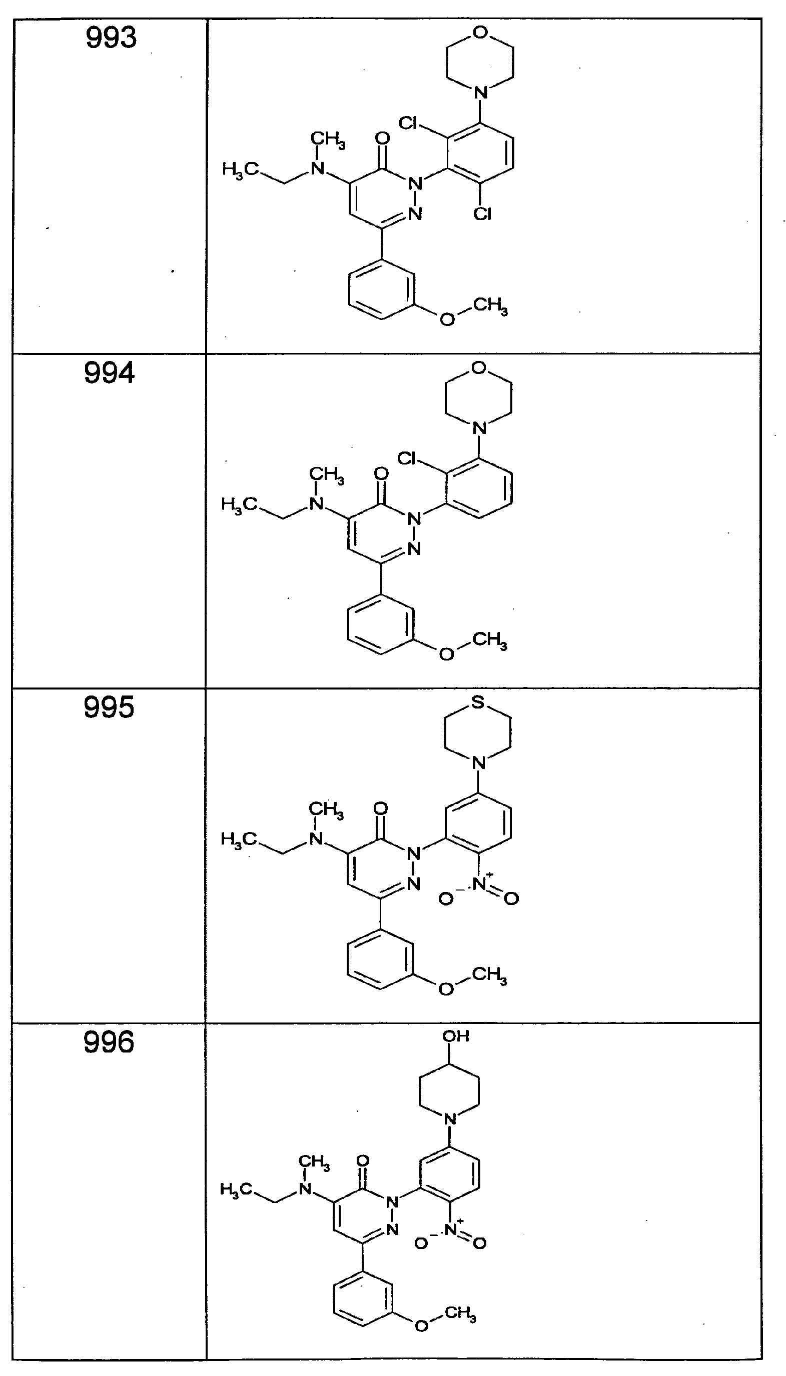

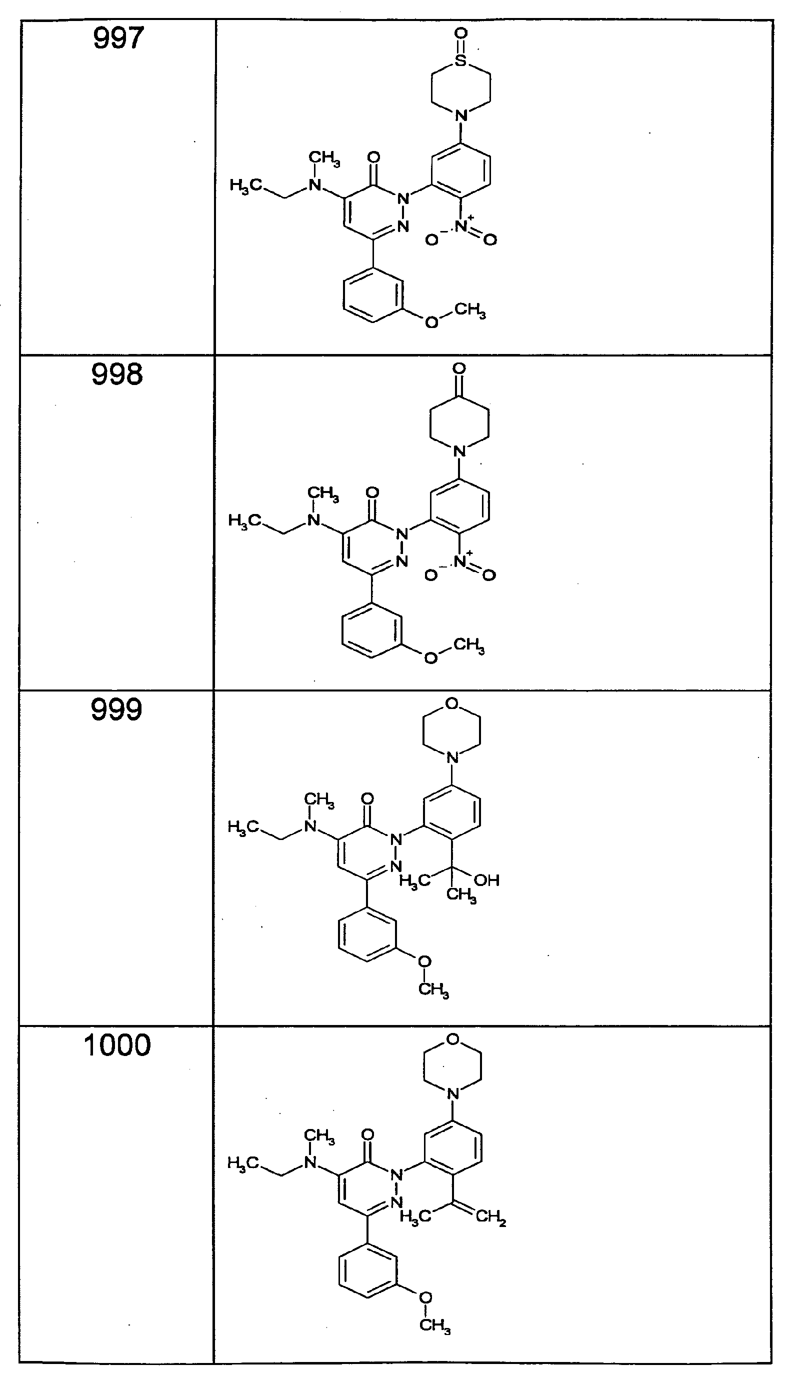

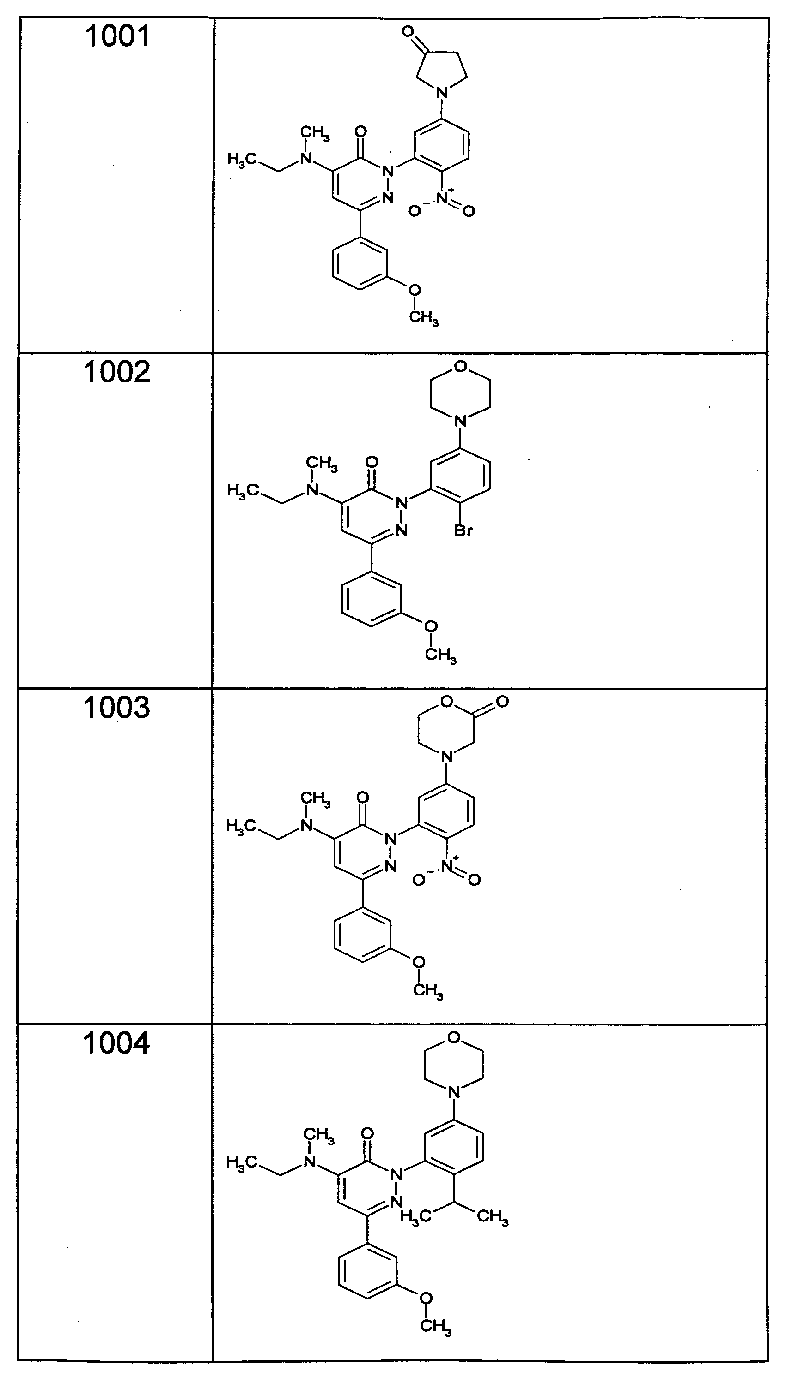

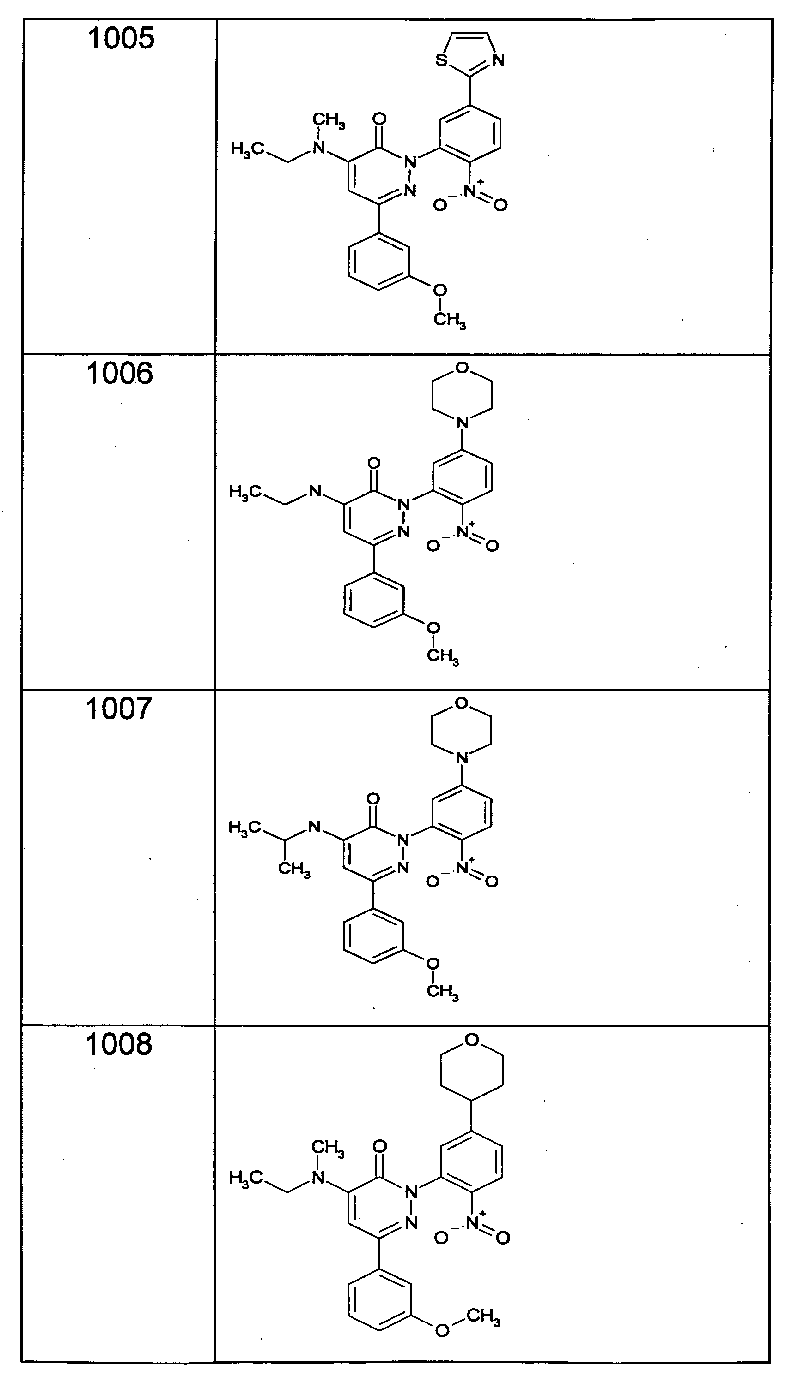

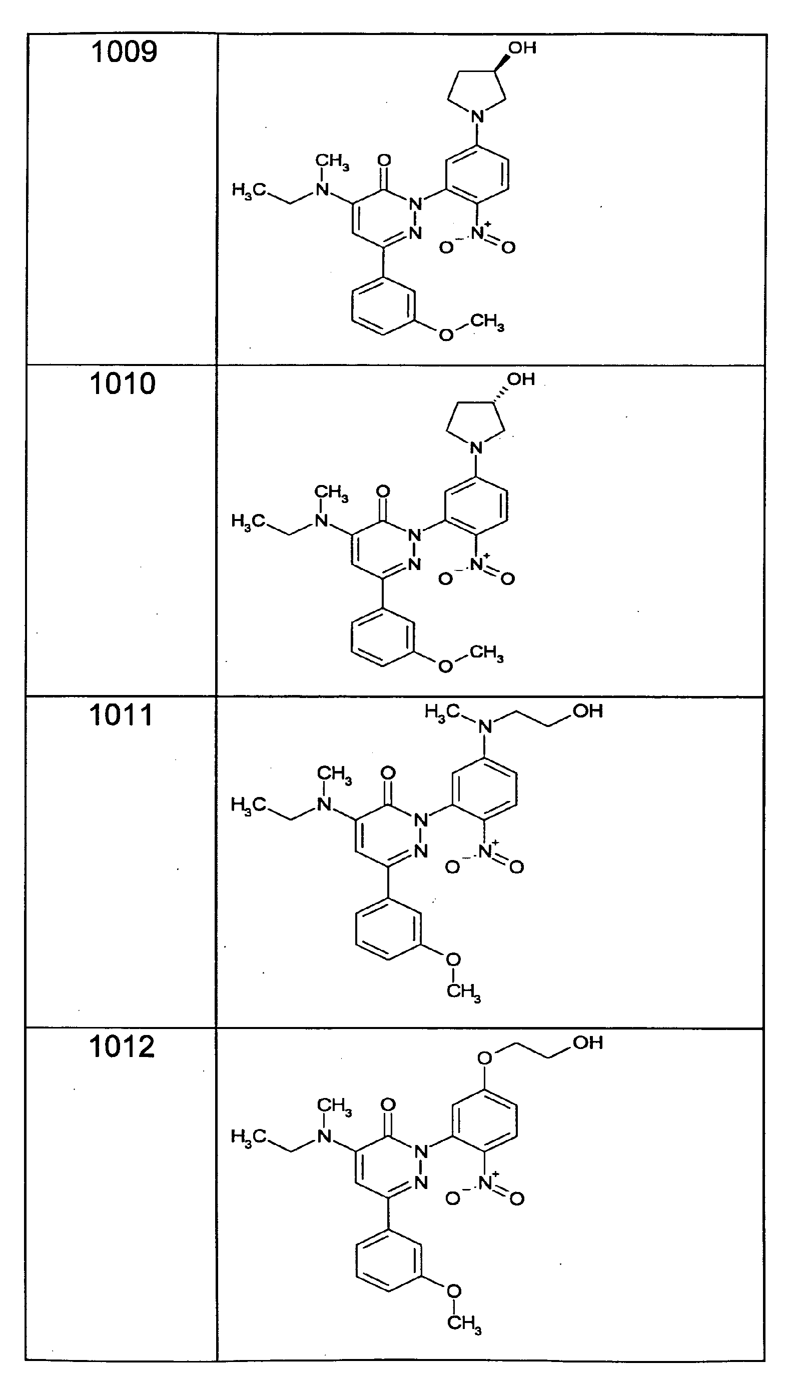

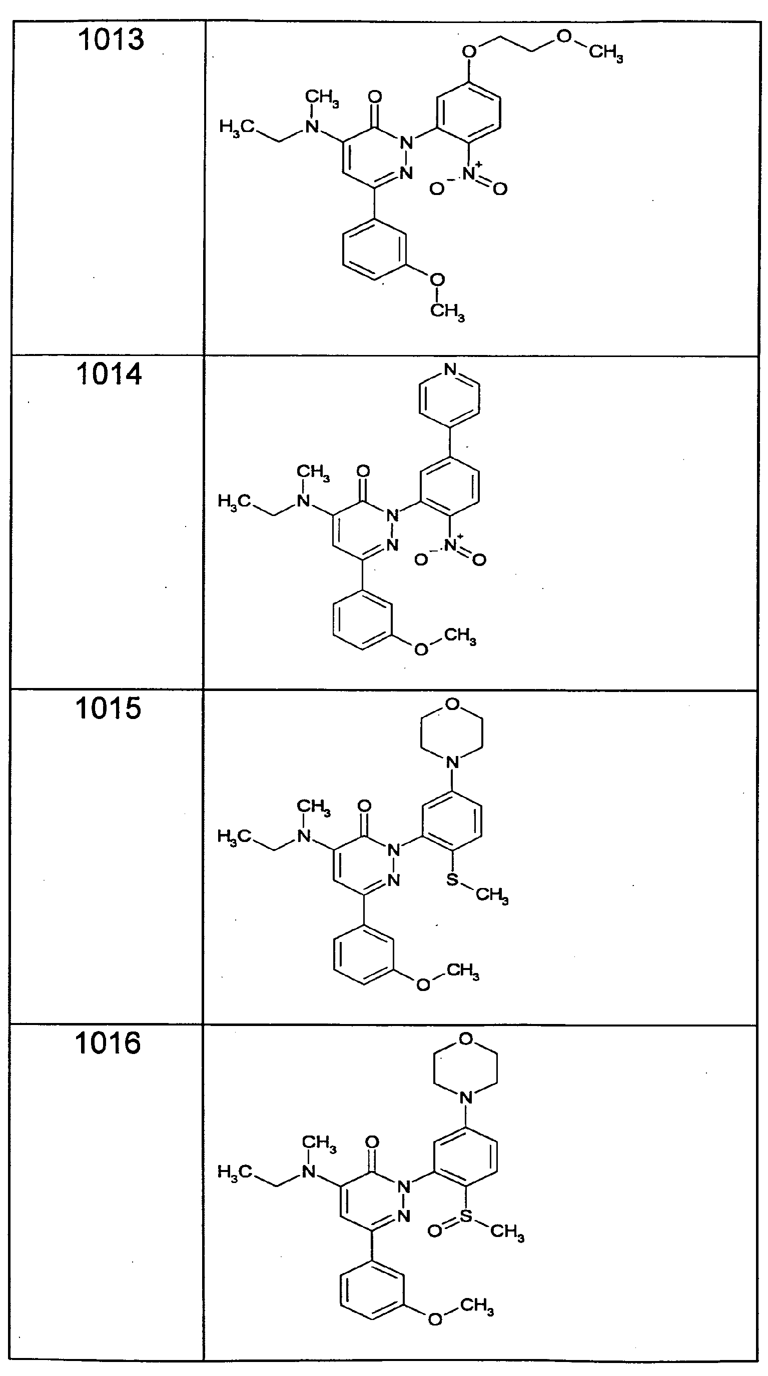

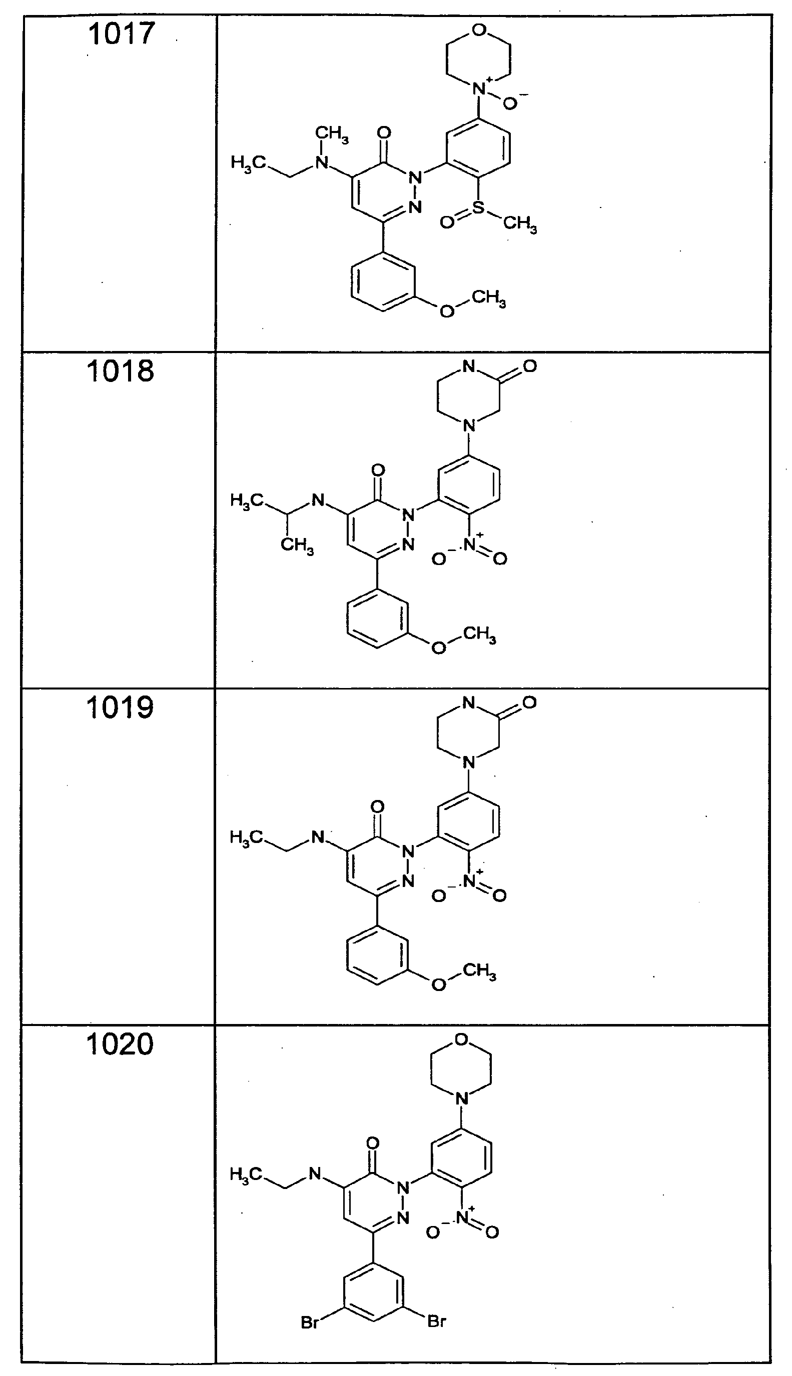

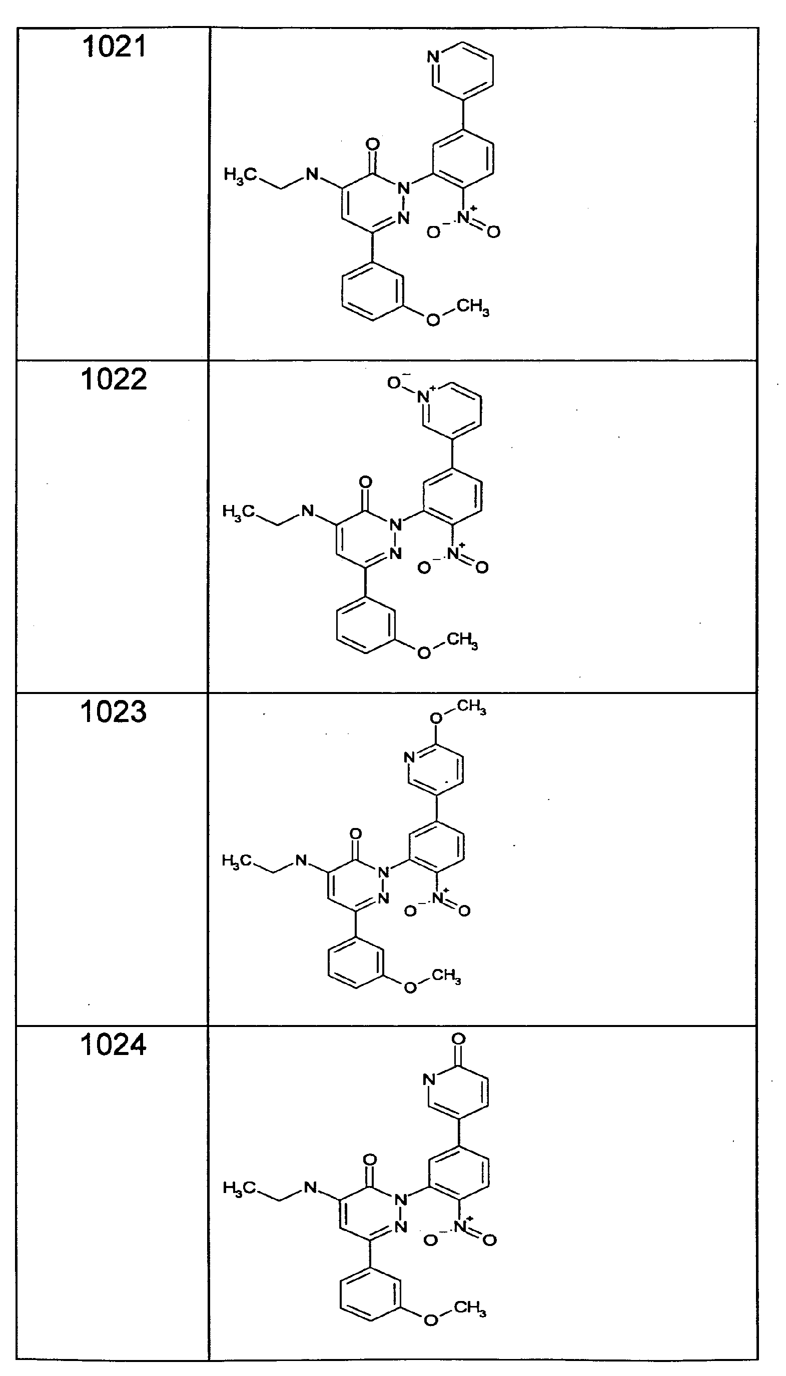

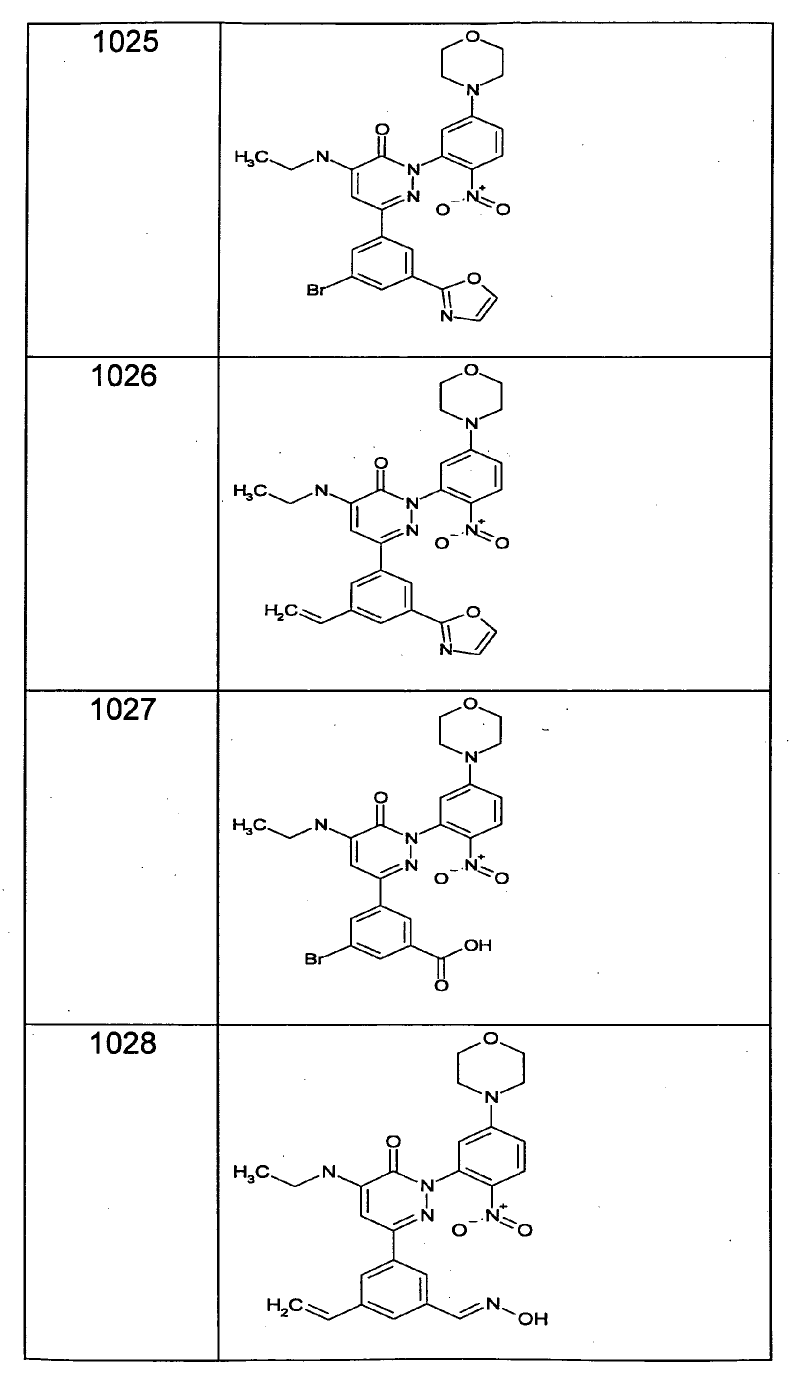

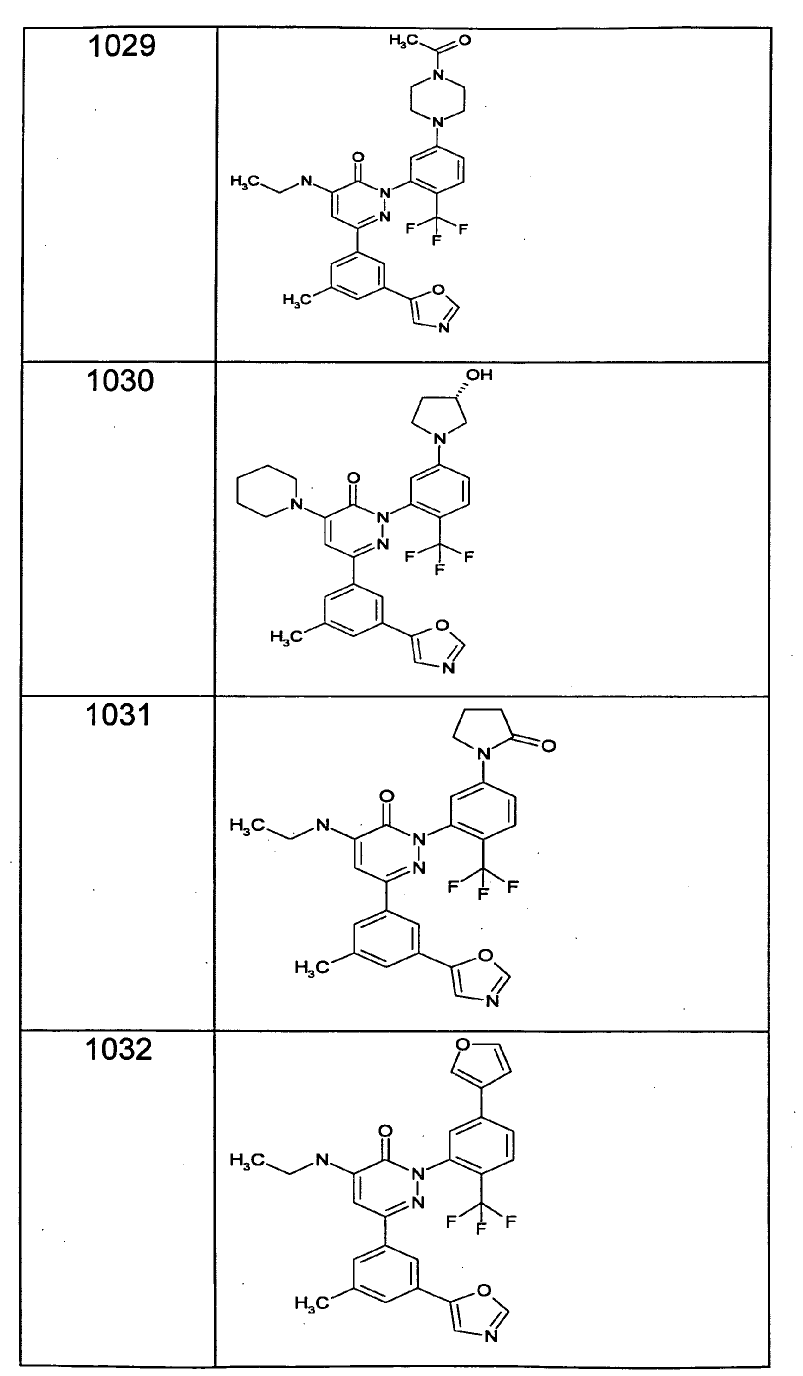

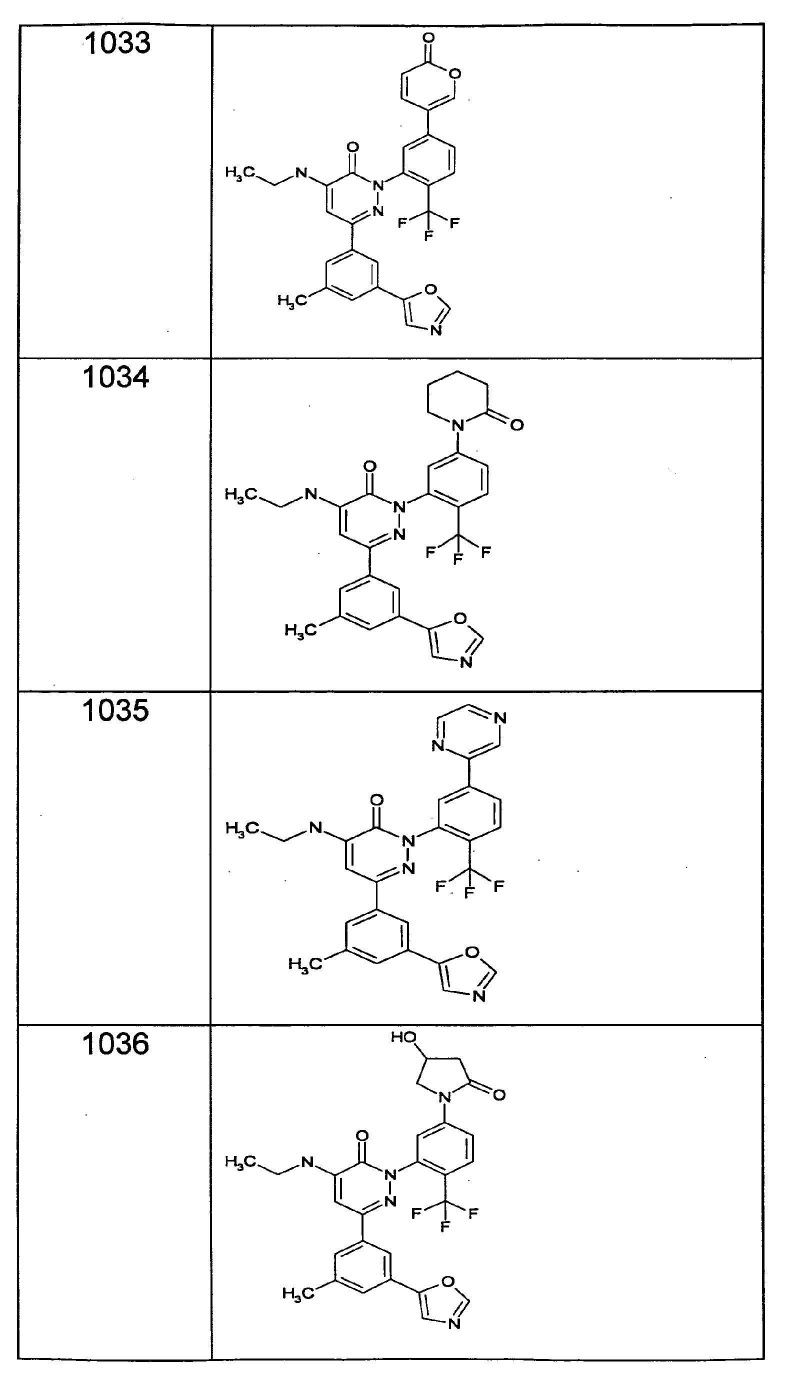

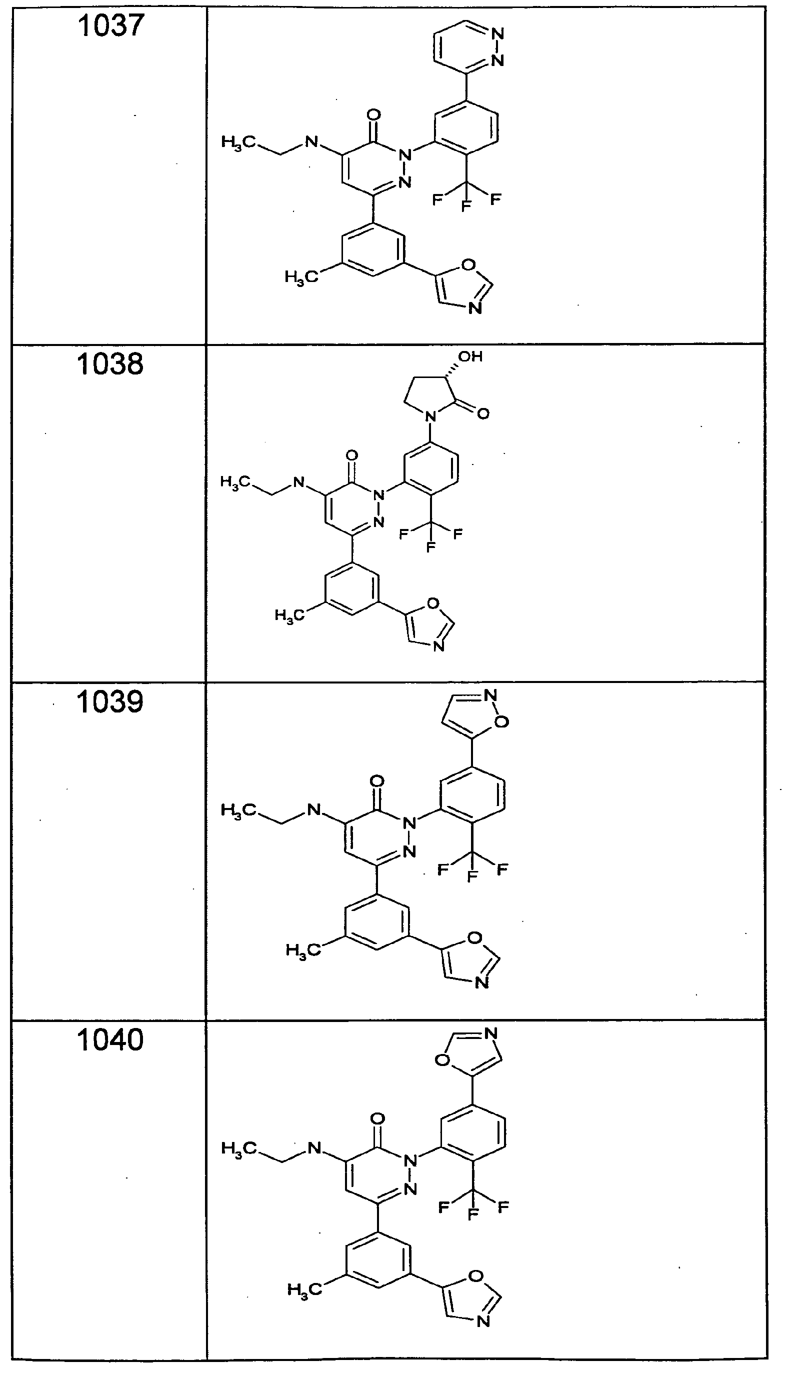

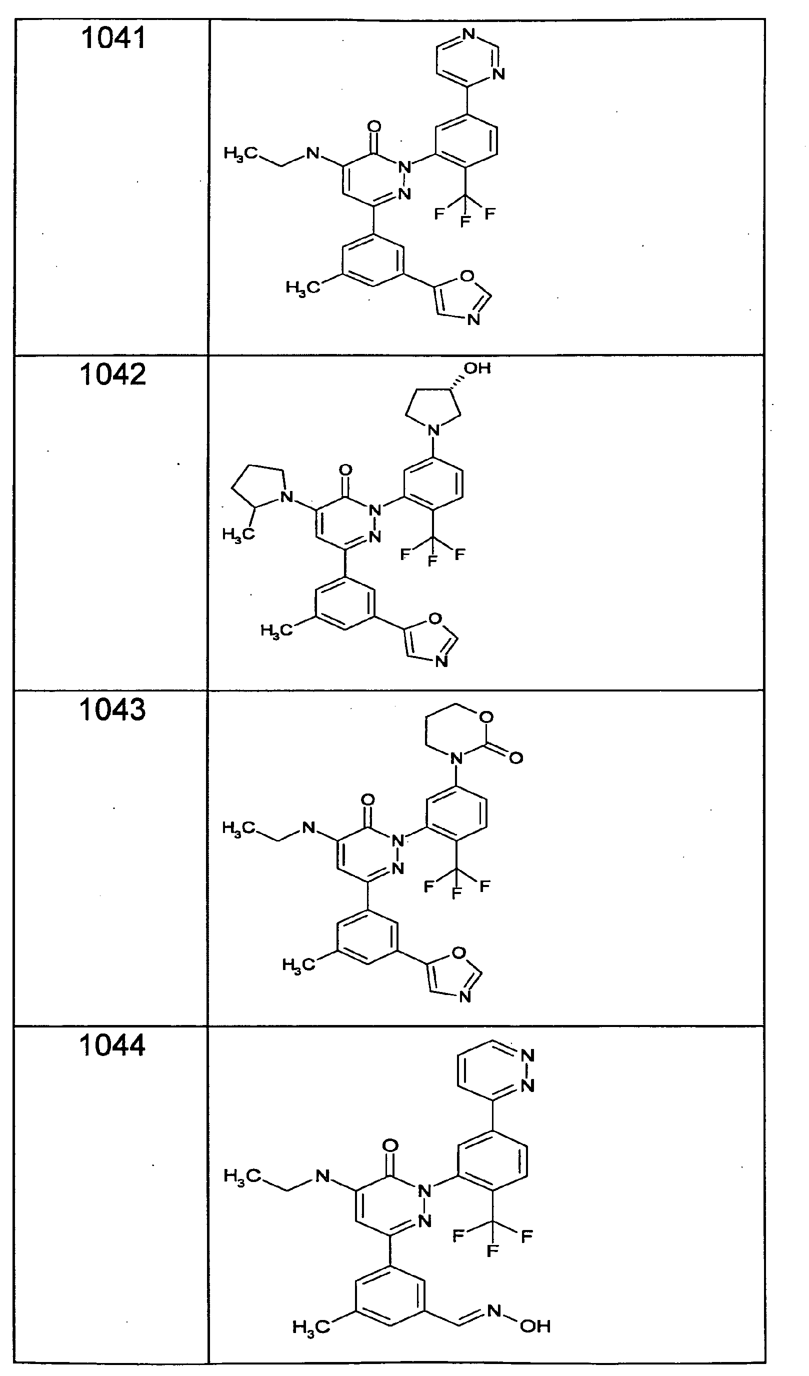

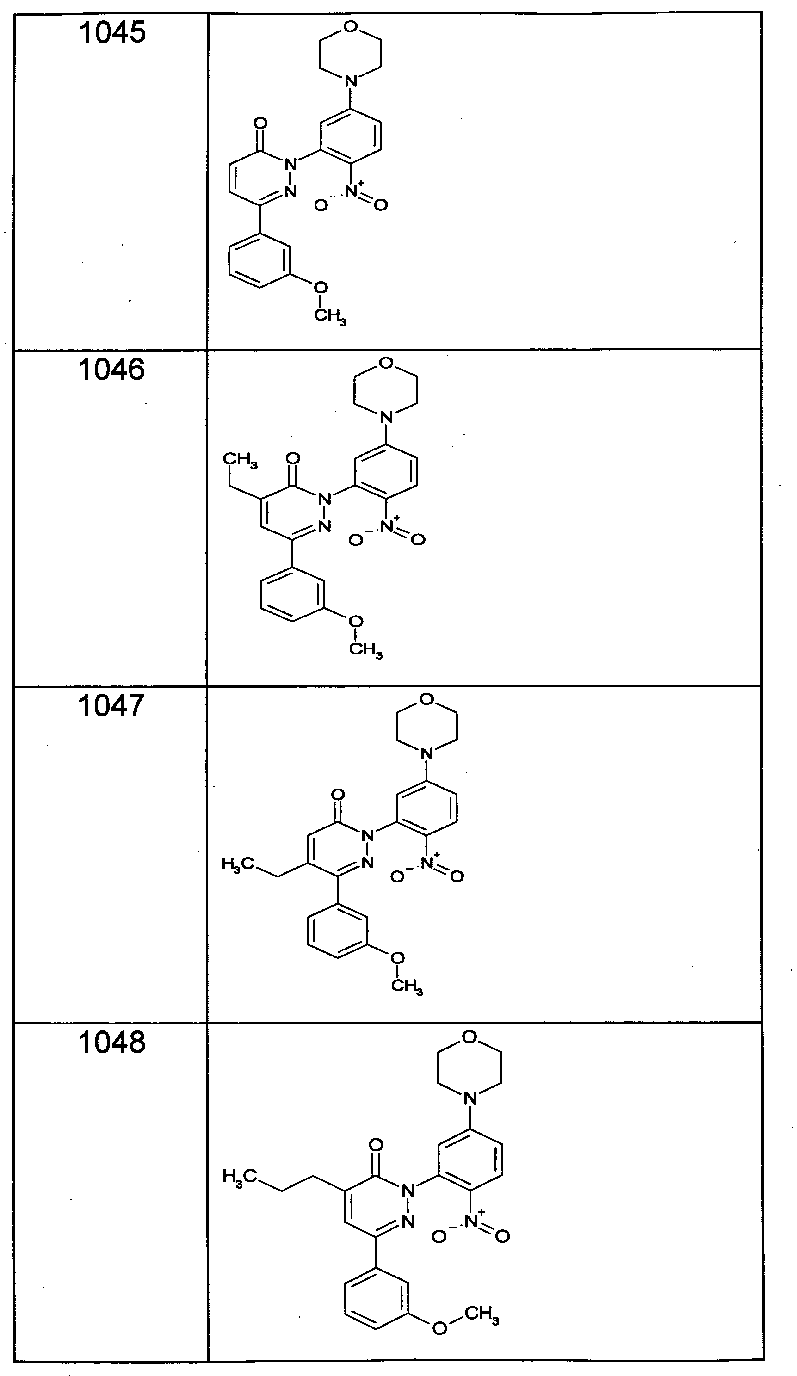

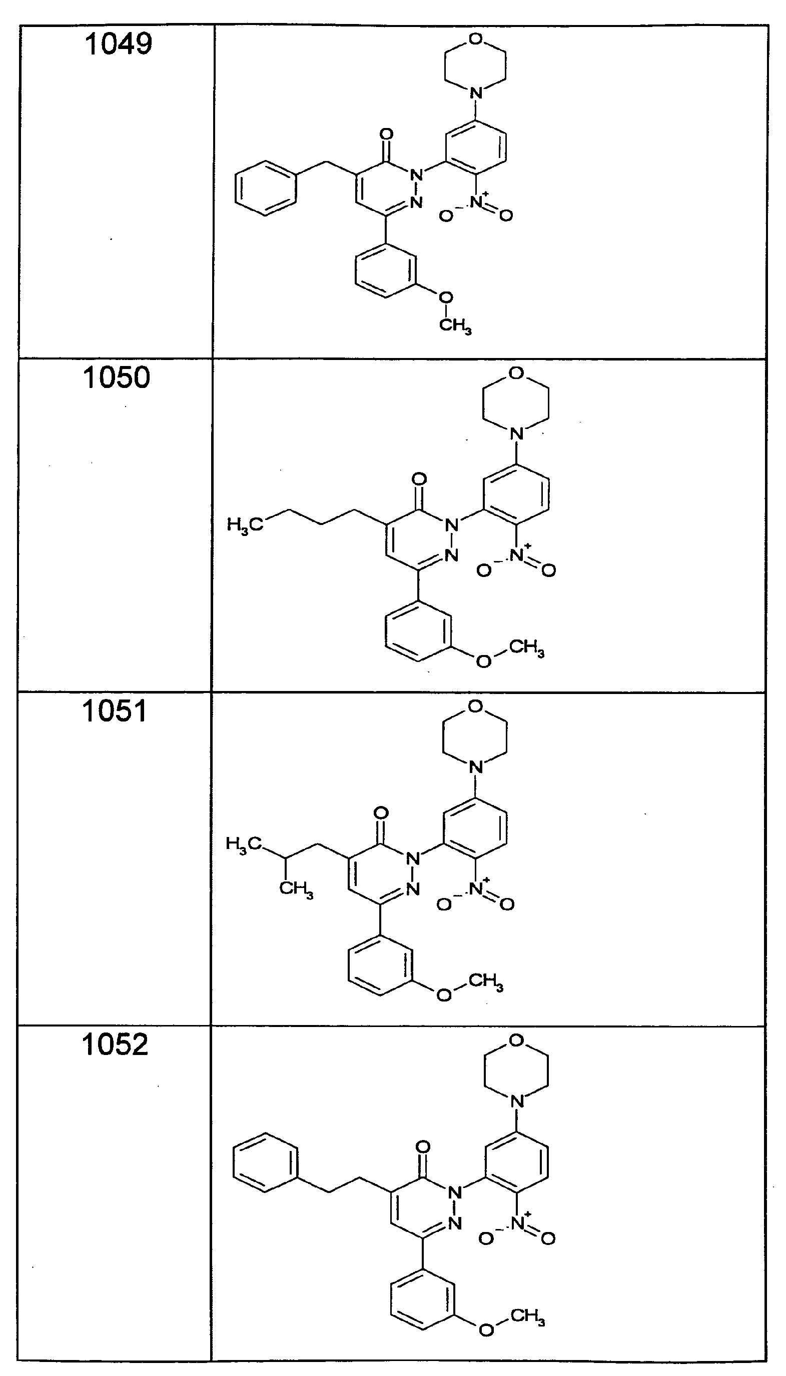

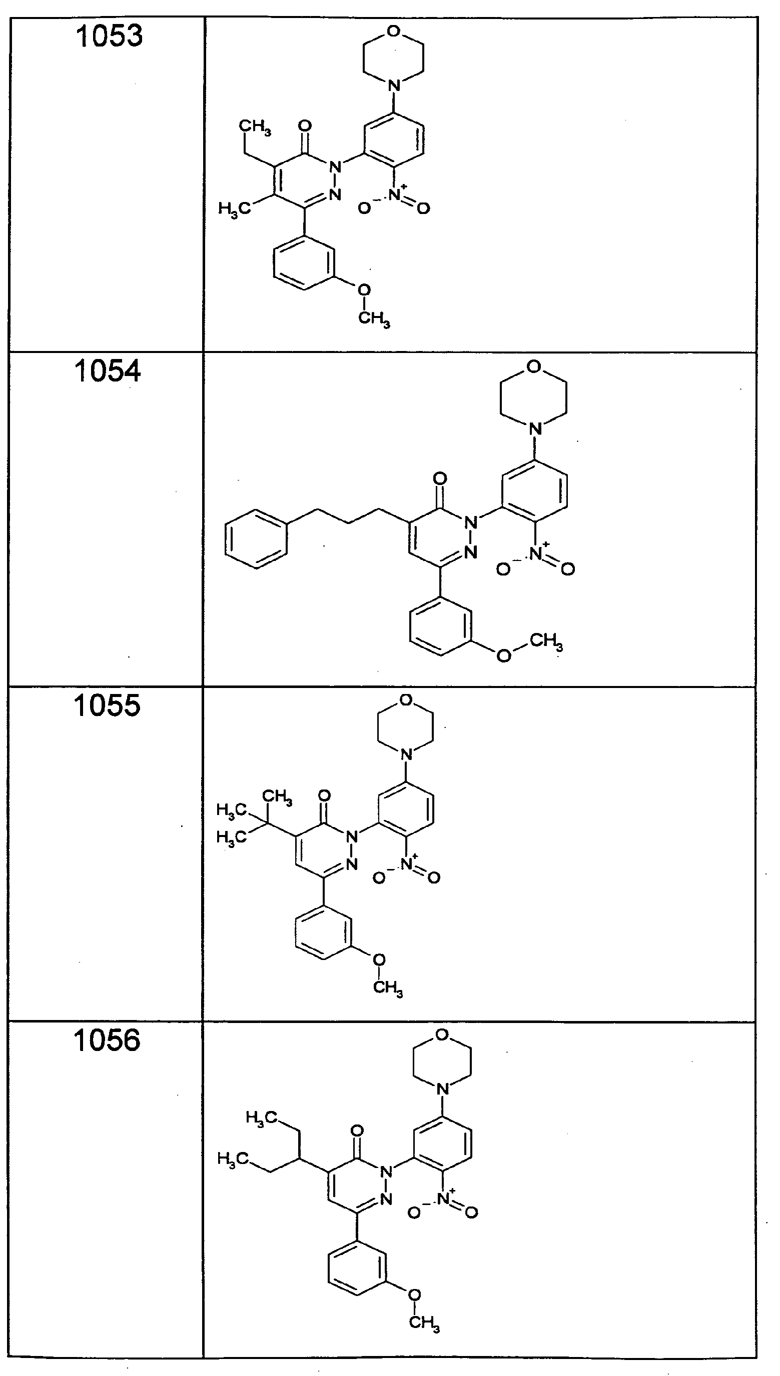

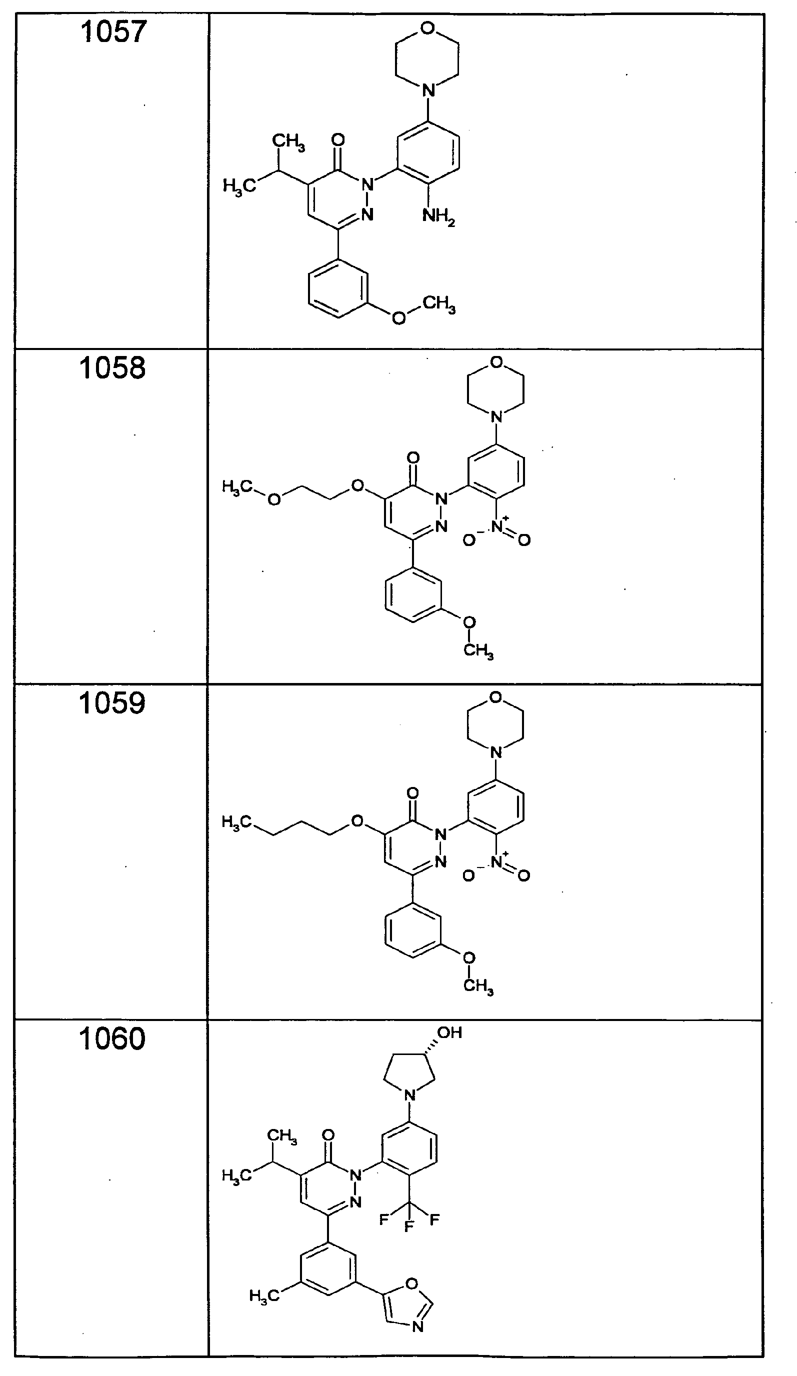

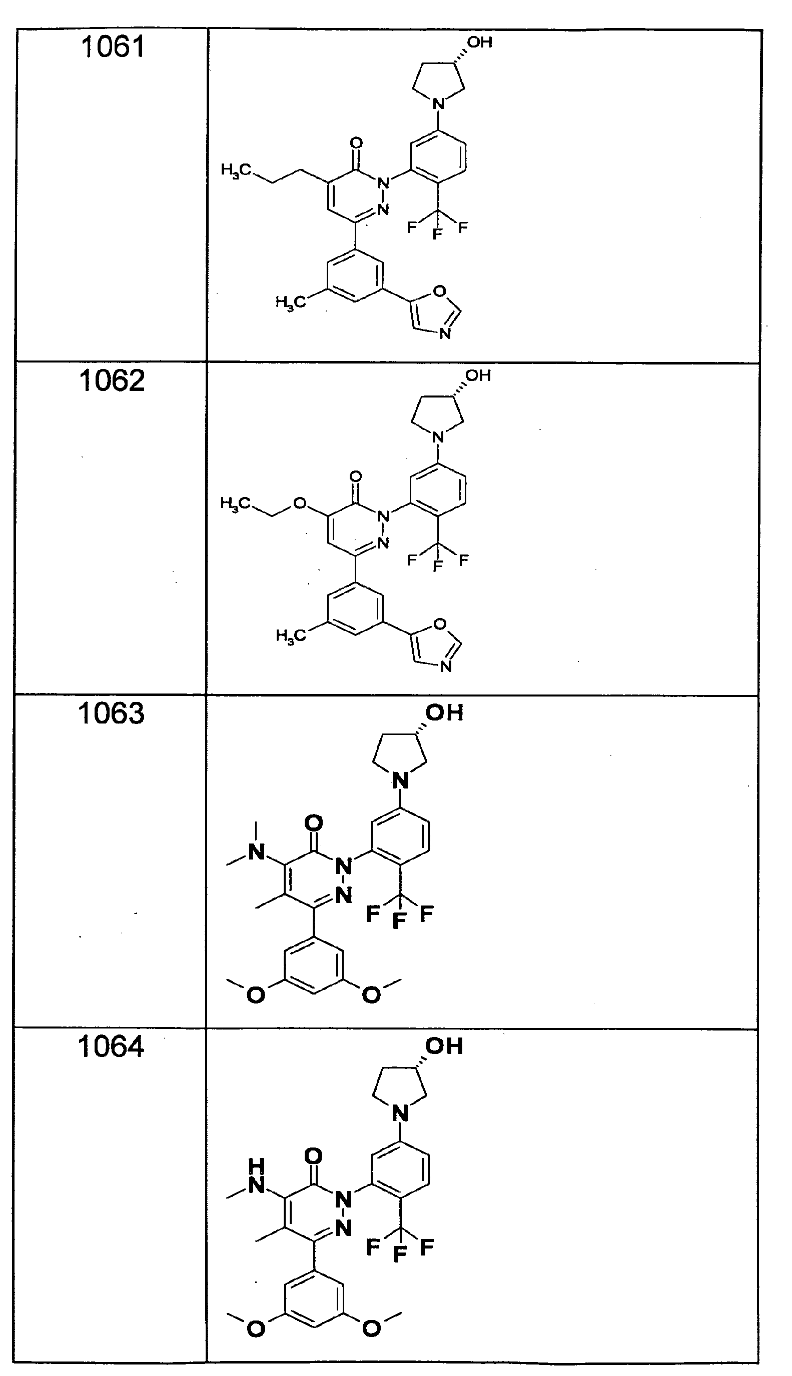

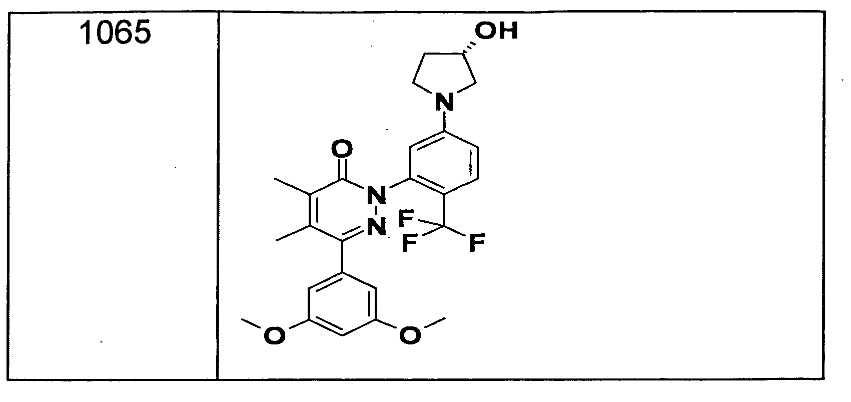

### Example 1066-1

### 4-(3,5-dimethoxy-phenyl)-2-(5-nitro-2-trifluoromethylphenyl)-2,5,6,7-tetrahydro-cyclopenta[c]pyridin-1-one

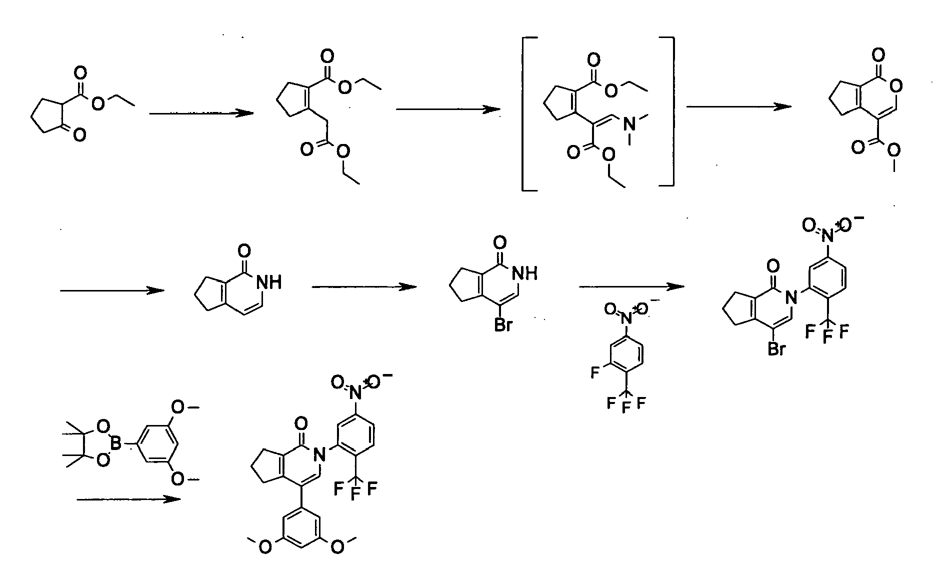

### Step 1

To a solution of ethyl 2-oxo-cyclopentanecarboxylate (7.81 g) in toluene (39 mL) was added ethyl (triphenylphosphoranilidene)-acetate (19.17 g). After stirring at 130°C for 11 hr, the reaction mixture was concentrated, and purified by silica gel column chromatography (ethyl acetate:hexane=1:10) to give ethyl 2-ethoxycarbonylmethyl-cyclopent-1-ene carboxylate (4.67 g).
¹H-NMR(CDCl₃)δ: 4.18(q,2H,J=7.2Hz), 4.15(q,2H,J=7.2Hz), 3.68(s,2H), 2.63-2.71(m,2H), 2.54-2.62(m,2H), 1.82-1.92(m,2H), 1.28(t,3H,J=7.2Hz), 1.26(t,3H,J=7.2Hz).

### Step 2

To a solution of ethyl 2-ethoxycarbonylmethyl-cyclopent-1-ene carboxylate (4.67 g) obtained in step 1 in N,N-dimethylformamide (23 mL) was added N,N-dimethylformamide dimethylacetal (8.25 mL). After stirring at 80°C for 4 hr, N,N-dimethylformamide dimethylacetal (5.5 mL) was added, and the mixture was further stirred for 30 min. Water was added, and the mixture was extracted with ethyl acetate. The organic layer was washed with water, dried and concentrated. To the obtained residue was added 1N hydrochloric acid (52 mL). After stirring at room temperature for 1 hr, the mixture was extracted with ethyl acetate. The organic layer was washed with water, dried and concentrated. The residue was dissolved in ethanol (46 mL), concentrated sulfuric acid (0.46 mL) was added, and the mixture was stirred at 10°C for 1.5 hr. The mixture was allowed to return to room temperature and, after concentration, water was added, and the mixture was extracted with ethyl acetate. The organic layer was washed with water, dried, concentrated and purified by silica gel column chromatography (ethyl acetate:hexane=1:6-1:4) to give methyl 1-oxo-1,5,6,7-tetrahydro-cyclopenta[c]pyran-4-carboxylate (1.89 g).
¹H-NMR(CDCl₃)δ: 8.24(s,1H), 4.31(q,2H,J=7.2Hz), 3.14-3.22(m,2H), 2.76-2.84(m,2H), 2.04-2.15(m,2H), 1.36(t,3H,J=7.2Hz).

### Step 3

To a solution of methyl 1-oxo-1,5,6,7-tetrahydro-cyclopenta[c]pyran-4-carboxylate (290 mg) obtained in step 2 in ethanol (1 mL) was added concentrated aqueous ammonia solution (2 mL). After stirring at 100°C in a sealed tube for 4 hr, the reaction mixture.was concentrated. The obtained residue was purified by silica gel column chromatography (chloroform:methanol=20:1-10:1) to give 2,5,6,7-tetrahydro-cyclopenta[c]pyridin-1-one (61 mg).
¹H-NMR(CDCl₃)δ: 12.16(bs,1H), 7.24(d,1H,J=6.7Hz), 6.26(d,1H,J=6.7Hz), 2.83-2.90(m,4H), 2.04-2.14(m,2H). Step 4

To a solution of 2,5,6,7-tetrahydro-cyclopenta[c]pyridin-1-one (215 mg) obtained in step 3 in acetic acid (2.15 mL) was added bromine (0.098 mL). After stirring at room temperature for 1 hr, water was added, and the precipitated crystals were collected by filtration to give 4-bromo-2,5,6,7-tetrahydro-cyclopenta[c]pyridin-1-one (203 mg).
¹H-NMR(CDCl₃)δ:7.52(s,1H,),2.91-3.01(m,4H),2.08-2.18(m,2H).

### Step 5

To a solution of 4-bromo-2,5,6,7-tetrahydro-cyclopenta[c]pyridin-1-one (197 mg) obtained in step 4 in N,N-dimethylformamide (3 mL) were added 2-fluoro-4-nitro-1-trifluoromethyl-benzene (250 mg) obtained in Example 1-1, supplementary step 2, and potassium carbonate (190 mg). After stirring at 80°C for 18 hr, water was added, and the mixture was extracted with ethyl acetate. The organic layer was washed with water, dried, concentrated and purified by silica gel column chromatography (ethyl acetate:hexane=1:3) to give 4-bromo-2-(5-nitro-2-trifluoromethyl-phenyl)-2,5,6,7-tetrahydro-cyclopenta[c]pyridin-1-one (167 mg).
¹H-NMR(CDCl₃)δ: 8.46(dd,1H,J=8.6,2.4Hz), 8.26(d,1H,J=2.4Hz), 8.05(d,1H,J=8.6Hz), 7.26(s,1H), 2.94-3.04(m,4H), 2.12-2.21(m,2H).

### Step 6

To a solution of 4-bromo-2-(5-nitro-2-trifluoromethyl-phenyl)-2,5,6,7-tetrahydro-cyclopenta[c]pyridin-1-one (167 mg) obtained in step 5 in toluene (2 mL)-ethanol (2 mL) were added 2-(3,5-dimethoxy-phenyl)-4,4,5,5-tetramethyl-[1,3,2]dioxaborolane (120 mg), 1,1'-bis(diphenylphosphino)ferrocene palladium(II) dichloride-dichloromethane complex (34 mg) and 1 M aqueous potassium carbonate solution (2 mL) under argon. After stirring at 100°C for 2 hr, water was added, and the mixture was extracted with ethyl acetate. The organic layer was washed with water, dried, concentrated and purified by silica gel column chromatography (ethyl acetate:hexane=1:2-1:3) to give 4-(3,5-dimethoxy-phenyl)-2-(5-nitro-2-trifluoromethylphenyl)-2,5,6,7-tetrahydro-cyclopenta[c]pyridin-1-one (170 mg).
¹H-NMR(CDCl₃)δ: 8.45(dd,1H,J=8.8,2.3Hz), 8.33(d,1H,J=2.3Hz), 8.06(d,1H,J=8.8Hz), 7.09(s,1H), 6.45(s,3H), 3.81(s,6H), 2.91-3.03(m,4H), 2.09-2.18(m,2H).

### Example 1066-2

### 4-(3,5-dimethoxy-phenyl)-2-(5-iodo-2-trifluoromethylphenyl)-2,5,6,7-tetrahydro-cyclopenta[c]pyridin-1-one

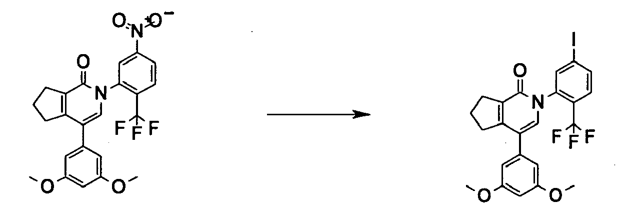

To a solution of 4-(3,5-dimethoxy-phenyl)-2-(5-nitro-2-trifluoromethyl-phenyl)-2,5,6,7-tetrahydro-cyclopenta[c]pyridin-1-one (170 mg) obtained in Example 1066-1 in ethanol (2 mL)-water (1 mL) were added ammonium chloride (99 mg) and reduced iron (103 mg). After stirring under refluxing for 1 hr, the mixture was diluted with ethyl acetate, and the insoluble material was filtered through celite. The filtrate was extracted with ethyl acetate, and the organic layer was washed with water, dried and concentrated. The residue was purified by silica gel column chromatography (ethyl acetate), and to a solution of the obtained resultant product (107 mg) in acetic acid (3 mL)-1N hydrochloric acid (3 mL) was added dropwise a solution of sodium nitrite (19 mg) in water (1 mL) under ice-cooling. After stirring at the same temperature for 1 hr, a solution of sodium iodide (45 mg) in water (1 mL) was added. After stirring at 50°C for 10 min, water was added, and the mixture was extracted with ethyl acetate. The organic layer was washed with an aqueous sodium hydrogencarbonate solution, dried, concentrated and purified by silica gel column chromatography (ethyl acetate:hexane=2:3) to give 4-(3,5-dimethoxy-phenyl)-2-(5-iodo-2-trifluoromethyl-phenyl)-2,5,6,7-tetrahydro-cyclopenta[c]pyridin-1-one (43 mg).
¹H-NMR(CDCl₃)δ: 7.94(dd,1H,J=8.3,0.7Hz), 8.33(d,1H,J=0.7Hz), 8.06(d,1H,J=8.3Hz), 7.08(s,1H), 6.44(s,3H), 3.81(s,6H), 2.89-3.01(m,4H), 2.06-2.16(m,2H).

### Example 1066-3

### 4-(3,5-dimethoxy-phenyl)-2-[5-((S)-3-hydroxy-pyrrolidin-1-yl)-2-trifluoromethyl-phenyl]-2,5,6,7-tetrahydro-cyclopenta[c]pyridin-1-one

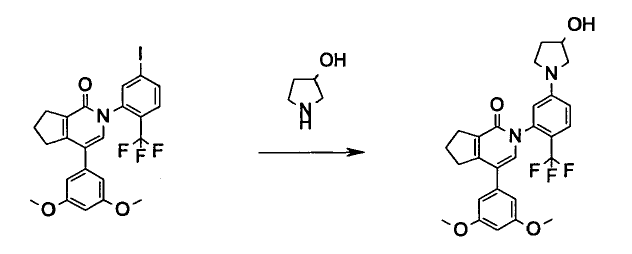

4-(3,5-Dimethoxy-phenyl)-2-(5-iodo-2-trifluoromethyl-phenyl)-2,5,6,7-tetrahydro-cyclopenta[c]pyridin-1-one (43 mg) obtained in Example 1066-2 and (S)-3-hydroxy-pyrrolidine (14 mg) were reacted in the same manner as in Example 1-15 and purified by silica gel thin layer chromatography (chloroform:acetone=2:1) to give 4-(3,5-dimethoxy-phenyl)-2-[5-((S)-3-hydroxy-pyrrolidin-1-yl)-2-trifluoromethyl-phenyl]-2,5,6,7-tetrahydro-cyclopenta[c]pyridin-1-one (21 mg).
¹H-NMR(CDCl₃)δ: 7.58(d,1H,J=8.4Hz), 7.18(s,1H), 6.59(dd,1H,J=8.4,1.6Hz), 6.41-6.49(m,4H), 4.61(bs,1H), 3.80(s,6H), 3.23-3.60(m,4H), 2.85-3.08(m,4H), 2.03-2.21(m,4H), 1.86(m,1H).

### Example 1066-4

### 1-benzoyl-6-(5-bromo-2-trifluoromethyl-phenyl)-4-(3,5-dimethoxy-phenyl)-1,2,3,6-tetrahydro-pyrrolo[2,3-c]pyridin-7-one

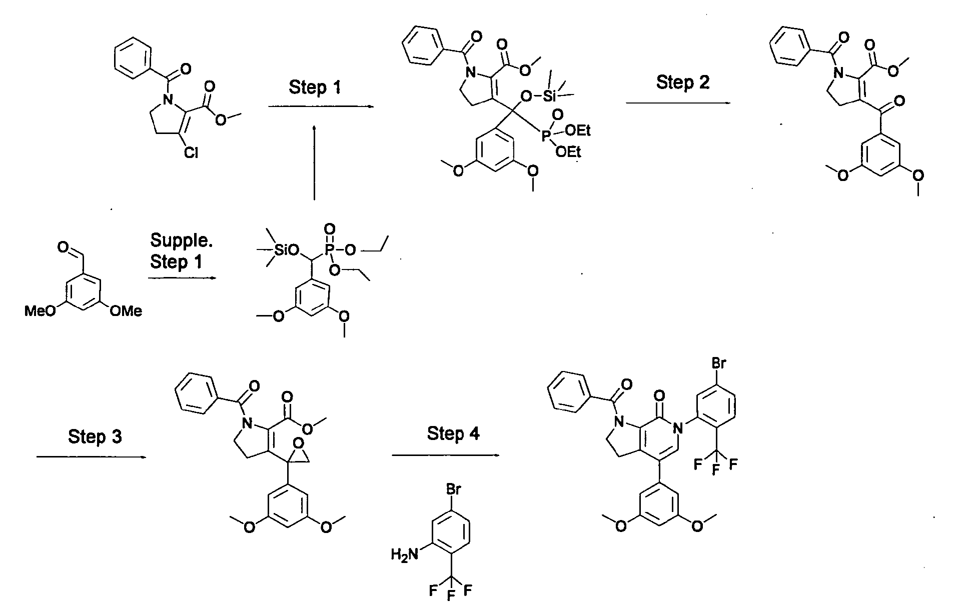

### Step 1

To a solution of [(3,5-dimethoxy-phenyl)-trimethylsiloxy-methyl]-phosphonic acid diethylester (3.0 g) obtained in supplementary step 1 in tetrahydrofuran (15 mL) was added dropwise *n-*butyllithium (2.67 M hexane solution; 3.0 mL) at -70°C under a nitrogen atmosphere. After stirring at -70°C to -40°C for 30 min, a solution of methyl 1-benzoyl-3-chloro-4,5-dihydro-1H-pyrrole-2-carboxylate (1.63 g) obtained in Example 1-24, step 1, in tetrahydrofuran (15 mL) was added. After stirring under ice-cooling for 40 min, an aqueous ammonium chloride solution was added, and the mixture was extracted with ethyl acetate. The organic layer was washed with water, dried, concentrated and purified by silica gel column chromatography (ethyl acetate:hexane=1:1-3:1) to give methyl 1-benzoyl-3-[(diethoxy-phosphoryl)-(3,5-dimethoxy-phenyl)-trimethylsiloxymethyl]-4,5-dihydro-1*H*-pyrrole-2-carboxylate (3.1 g).
¹H-NMR(DMSO-d₆)δ: 7.60-7.54(2H,m), 7.52-7.40(3H,m), 6.77(2H,s), 6.51(1H,s), 4.19-4.08(4H,m), 3.89-3.70(2H,m), 3.74(6H,s), 3.61(3H,brs), 2.90-2.77(1H,m), 2.13-2.02(1H,m), 1.27(6H,t,*J*=7.1Hz), -0.08(9H,s).

### Step 2

To a solution of methyl 1-benzoyl-3-[(diethoxy-phosphoryl)-(3,5-dimethoxy-phenyl)-trimethylsiloxymethyl]-4,5-dihydro-1*H*-pyrrole-2-carboxylate (3.3 g) obtained by a method similar to step 1 in *t*-butanol (6.6 mL) was added dropwise 57% aqueous tetrabutylammonium fluoride solution (2.65 mL). After stirring at room temperature for 30 min, the reaction mixture was ice-cooled, and water was added. The precipitated crystals were collected by filtration, and washed with *t*-butanol-water (1:2) to give methyl 1-benzoyl-3-(3,5-dimethoxy-benzoyl)-4,5-dihydro-1*H-*pyrrole-2-carboxylate (1.23 g).
¹H-NMR(DMSO-d₆)δ: 7.67(2H,d,*J*=7.4Hz), 7.61-7.55(1H,m), 7.53-7.47(2H,m), 6.80-6.78(2H,m), 6.74-6.71(1H,m) , 4.10(2H,t,*J*=8.8Hz), 3.78(6H,s), 3.27(3H,brs), 3.03(2H,t,*J*=8.8Hz).

### Step 3

To a solution of methyl 1-benzoyl-3-(3,5-dimethoxy-benzoyl)-4,5-dihydro-1H-pyrrole-2-carboxylate (12 g) in dimethylsulfoxide (60 mL) produced by a method similar to the method of the above-mentioned steps 1 and 2, or Example 1-24, steps 1 and 2, was added trimethylsulfonium iodide (7.42 g). Under ice-cooling, sodium hydride (60%; 1.45 g) was added by small portions over 5 min, and the mixture was stirred at room temperature for 20 hr. The mixture was ice-cooled again, and water (180 mL) was added, The precipitated crystals were collected by filtration, and washed with ethanol to give methyl 1-benzoyl-3-[2-(3,5-dimethoxy-phenyl)-oxyranyl]-4,5-dihydro-1H-pyrrole-2-carboxylate (7.95 g).
¹H-NMR(DMSO-d₆)δ: 7.70-7.40(m,5H), 6.53(d,2H,J=2.3Hz), 6.47(t,1H,J=2.3Hz), 4.10-3.90(m,2H), 3.75(s,6H), 3.60(s,3H), 3.12(d,1H,J=5.1Hz), 3.09(d,1H,J=5.1Hz), 2.85-2.55(m,2H)

### Step 4

To a solution of methyl 1-benzoyl-3-[2-(3,5-dimethoxy-phenyl)-oxyranyl]-4,5-dihydro-1H-pyrrole-2-carboxylate (4.53 g) obtained in step 3 in ethyl acetate (110 mL) was added a 4N hydrogen chloride - ethyl acetate solution (2.76 mL). After stirring at room temperature for 5 min, the mixture was neutralized with an aqueous sodium hydrogencarbonate solution, and extracted with ethyl acetate. The organic layer was washed with water, dried and concentrated. The obtained crystals were washed with chloroform-ethyl acetate-hexane. The resultant product was dissolved in ethanol (45 mL), and 5-bromo-2-trifluoromethyl-aniline (1.41 g) and concentrated hydrochloric acid (0.49 mL) were added under ice-cooling. After stirring at room temperature for 1 hr, cesium carbonate (3.83 g) was added. After stirring for 12 hr, 1N hydrochloric acid (45 mL) was added under ice-cooling, and the mixture was extracted with chloroform. The organic layer was washed with water, dried, concentrated, purified by silica gel column chromatography (ethyl acetate:hexane=3:2-chloroform :methanol=30:1-15:1) and further recrystallized from ethyl acetate-hexane to give 1-benzoyl-6-(5-bromo-2-trifluoromethyl-phenyl)-4-(3,5-dimethoxy-phenyl)-1,2,3,6-tetrahydro-pyrrolo[2,3-c]pyridin-7-one (1.69 g).
¹H-NMR(CDCl₃)δ: 7.70-7.67 (m,3H), 7.62(d,1H,J=8.6Hz), 7.52(d,1H,J=1.6Hz), 7.45-7.40(m,1H), 7.38-7.34(m,2H), 7.08(d,1H,J=0.9Hz), 6.47(t,1H,J=2.2Hz), 6.45(d,2H,J=2.1Hz), 4.38-4.30(m,1H), 4.23-4.16(m,1H), 3.82(s,6H), 3.24-3.06(m,2H).

### Supplementary step 1

Triethyl phosphite (3.93 g) and trimethylsilyl chloride (3.90 mL) were added to 3,5-dimethoxybenzaldehyde (3.93 g) under nitrogen atmosphere and under ice-cooling, and the mixture was stirred at room temperature for 10 min, and at 60°C for 3 hr. Thereafter, trimethylsilyl chloride (0.90 mL) and triethyl phosphite (0.41 mL) were successively added, and the mixture was stirred at 60°C for 2 hr. The reaction mixture was allowed to return to room temperature, toluene (40 mL) and pyridine (0.57 mL) were added under ice-cooling and the mixture was stirred for 30 min. The mixture was neutralized with 1N hydrochloric acid (7.09 mL) and separated into two layers. The toluene layer was washed with water, an aqueous sodium hydrogencarbonate solution and brine, dried and concentrated to give [(3,5-dimethoxy-phenyl)-trimethylsiloxy-methyl]-phosphonic acid diethyl ester (7.76 g).
¹H-NMR(CDCl₃)δ: 6.64(2H,t,*J*=2.3Hz), 6.39(1H,q,*J*=2.3Hz), 4.91(1H,d,*J*=14.4Hz), 4.10-3.97(4H,m), 3.79(6H,s), 1.28-1.23(6H,m),0.12(9H,s).

### Example 1066-5

### methyl 4-(3,5-dimethoxy-phenyl)-6-[5-(3-hydroxymethyl-azetidin-1-yl)-2-trifluoromethyl-phenyl]-7-oxo-2,3,6,7-tetrahydro-pyrrolo[2,3-c]pyridine-1-carboxylate

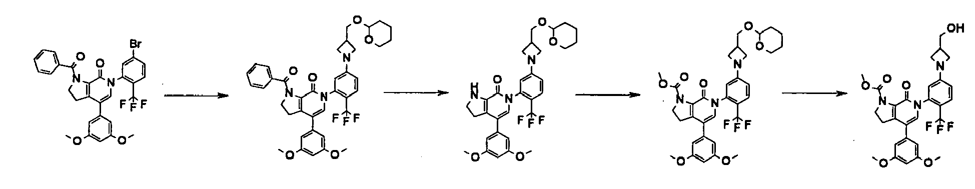

### Step 1

1-Benzoyl-6-(5-bromo-2-trifluoromethyl-phenyl)-4-(3,5-dimethoxy-phenyl)-1,2,3,6-tetrahydro-pyrrolo[2,3-c]pyridin-7-one (660 mg) obtained in Example 1066-4 and 3-(tetrahydro-pyran-2-yloxymethyl)-azetidine (377 mg) obtained in Example 1-23, supplementary step 2, were reacted in the same manner as in Example 1-15 and purified by silica gel column chromatography (ethyl acetate:hexane=3:1-5:1) to give 1-benzoyl-4-(3,5-dimethoxy-phenyl)-6-{5-[3-(tetrahydro-pyran-2-yloxymethyl)-azetidin-1-yl]-2-trifluoromethyl-phenyl}-1,2,3,6-tetrahydro-pyrrolo[2,3-c]pyridin-7-one (389 mg).
¹H-NMR(CDCl₃)δ: 7.69-7.65(m,2H), 7.48(d,1H,J=8.8Hz), 7.42-7.31(m,3H), 7.13(s,1H), 6.47-6.44(m,3H), 6.37(dd,1H,J=8.7,2.2Hz), 6.18(d,1H,J=2.1Hz), 4.61-4.59(m,1H), 4.39-4.30(m,1H), 4.24-4.16(m,1H), 4.02-3.89(m,3H), 3.87-3.75(m,1H), 3.81(s,6H), 3.73-3.63(m,2H), 3.59-3.49(m,2H), 3.23-3.08(m,2H), 3.04-2.96(m,1H), 1.83-1.68(m,2H), 1.61-1.49(m,4H).

### Step 2

To a solution of 1-benzoyl-4-(3,5-dimethoxy-phenyl)-6-{5-[3-(tetrahydro-pyran-2-yloxymethyl)-azetidin-1-yl]-2-trifluoromethyl-phenyl}-1,2,3,6-tetrahydro-pyrrolo[2,3-c]pyridin-7-one (100 mg) obtained in step 1 in tetrahydrofuran (1 mL)-methanol (1 mL) was added 4N aqueous sodium hydroxide solution (0.18 mL). After stirring under refluxing for 30 min, water was added, and the mixture was extracted with ethyl acetate. The organic layer was washed with an aqueous sodium hydrogencarbonate solution, dried, concentrated and purified by silica gel column chromatography (ethyl acetate) to give 4-(3,5-dimethoxy-phenyl)-6-{5-[3-(tetrahydro-pyran-2-yloxymethyl)-azetidin-1-yl]-2-trifluoromethyl-phenyl}-1,2,3,6-tetrahydro-pyrrolo[2,3-c]pyridin-7-one (53 mg).
¹H-NMR(CDCl₃)δ: 7.56(d,1H,J=8.8Hz), 6.82(s,1H), 6.48(d,2H,J=2.1Hz), 6.46-6.44(m,1H), 6.44-6.41(m,2H), 6.30(d,1H,J=2.1Hz), 4.62-4.59(m,1H), 4.07-3.98(m,2H), 3.94(dd,1H,J=9.9,7.3Hz), 3.87-3.82(m,1H), 3.80(s,6H), 3.76-3.49(m,6H), 3.25-3.10(m,2H), 3.05-2.99(m,1H), 1.83-1.68(m,2H), 1.61-1.47(m,4H).

### Step 3

To a solution of 4-(3,5-dimethoxy-phenyl)-6-{5-[3-(tetrahydro-pyran-2-yloxymethyl)-azetidin-1-yl]-2-trifluoromethyl-phenyl}-1,2,3,6-tetrahydro-pyrrolo[2,3-c]pyridin-7-one (187 mg) obtained in step 2 in tetrahydrofuran (3.5 mL) were added pyridine (0.098 mL) and methyl chloroformate (0.047 mL) under ice-cooling. After stirring at room temperature for 30 min, water was added, and the mixture was extracted with ethyl acetate. The organic layer was washed with water, dried, concentrated and purified by silica gel column chromatography (chloroform :acetone=3:1) to give methyl 4-(3,5-dimethoxy-phenyl)-7-oxo-6-{5-[3-(tetrahydropyran-2-yloxymethyl)-azetidin-1-yl]-2-trifluoromethylphenyl}-2,3,6,7-tetrahydro-pyrrolo[2,3-c]pyridine-1-carboxylate (177 mg).
¹H-NMR(CDCl₃)δ: 7.56(d,1H,J=8.6Hz), 7.14(s,1H), 6.46-6.41(m,4H), 6.34(d,1H,J=2.3Hz), 4.64-4.60(m,1H), 4.20-4.00(m,4H), 3.98-3.92(m,1H), 3.88-3.69(m,3H), 3.80(s,6H), 3.79(s,3H), 3.62-3.56(m,1H), 3.56-3.49(m,1H), 3.08-3.02(m,3H), 1.85-1.76(m,1H), 1.76-1.68(m,1H), 1.63-1.50(m,4H).

### Step 4

To a solution of methyl 4-(3,5-dimethoxy-phenyl)-7-oxo-6-{5-[3-(tetrahydro-pyran-2-yloxymethyl)-azetidin-1-yl]-2-trifluoromethyl-phenyl}-2,3,6,7-tetrahydro-pyrrolo[2,3-c]pyridine-1-carboxylate (177 mg) obtained in step 3 in methanol (1.8 mL) were added p-toluenesulfonic acid monohydrate (10 mg). After stirring at room temperature for 2 hr, water was added, and the mixture was extracted with ethyl acetate. The organic layer was washed with an aqueous sodium hydrogencarbonate solution and water, dried, concentrated, purified by silica gel column chromatography (chloroform :acetone=3:4), and further recrystallized from chloroform-diethyl ether-hexane to give methyl 4-(3,5-dimethoxy-phenyl)-6-[5-(3-hydroxymethyl-azetidin-1-yl)-2-trifluoromethyl-phenyl]-7-oxo-2,3,6,7-tetrahydro-pyrrolo[2,3-c]pyridine-1-carboxylate (91 mg).
¹H-NMR(CDCl₃)δ: 7.57(d,1H,J=8.6Hz), 7.14(d,1H,J=0.7Hz), 6.47-6.43(m,2H), 6.42(d,2H,J=2.1 Hz), 6.35(d,1H,J=2.3Hz), 4.20-3.97(m,4H), 3.85(dd,2H,J=6.1, 5.2Hz), 3.81-3.71(m,2H), 3.80(s,6H), 3.79(s,3H), 3.06(td,2H,J=8.2,1.7Hz), 2.99-2.91(m,1H), 1. 68 (t, 1H, J=5.0Hz).

### Example 1066-6

### 6-(5-bromo-2-trifluoromethyl-phenyl)-4-(3,5-dimethoxy-phenyl)-1,2,3,6-tetrahydro-pyrrolo[2,3-c]pyridin-7-one

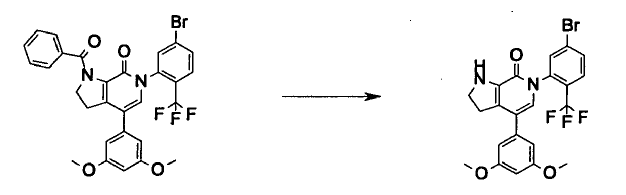

1-Benzoyl-6-(5-bromo-2-trifluoromethyl-phenyl)-4-(3,5-dimethoxy-phenyl)-1,2,3,6-tetrahydro-pyrrolo[2,3-c]pyridin-7-one (1.69 g) obtained in Example 1066-4 was reacted in the same manner as in Example 1066-5, step 2, to give 6-(5-bromo-2-trifluoromethyl-phenyl)-4-(3,5-dimethoxy-phenyl)-1,2,3,6-tetrahydro-pyrrolo[2,3-c]pyridin-7-one (1.43 g).
¹H-NMR(CDCl₃)δ: 7.75-7.72(m,1H), 7.68(d,1H,J=8.6Hz), 7.61(d,1H,J=1.4Hz), 6.75(s,1H), 6.47(d,2H,J=2.3Hz), 6.44(t,1H,J=2.2Hz), 4.13(br s,1H), 3.81(s,6H), 3.68(td,2H,J=9.2,1.7Hz), 3.17(td,2H,J=9.2,4.9Hz).

### Example 1066-7

### 6-(5-bromo-2-trifluoromethyl-phenyl)-4-(3,5-dimethoxy-phenyl)-1,6-dihydro-pyrrolo[2,3-c]pyridin-7-one

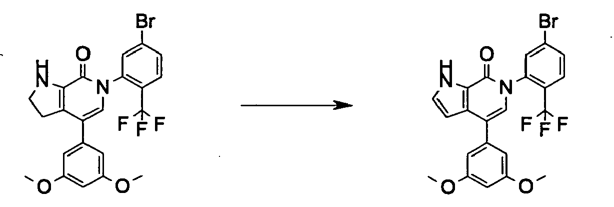

To a solution of 6-(5-bromo-2-trifluoromethylphenyl)-4-(3,5-dimethoxy-phenyl)-1,2,3,6-tetrahydro-pyrrolo[2,3-c]pyridin-7-one (1.43 g) obtained in Example 1066-6 in chloroform (30 mL) was added 2,3-dichloro-5,6-dicyano-p-benzoquinone (1.28 g). After stirring at room temperature for 15 min, the insoluble material was filtered through celite, and the filtrate was concentrated. The obtained residue was purified by silica gel column chromatography (ethyl acetate:hexane=1:1-chloroform:acetone=2:1) to give 6-(5-bromo-2-trifluoromethyl-phenyl)-4-(3,5-dimethoxy-phenyl)-1,6-dihydro-pyrrolo[2,3-c]pyridin-7-one (910 mg) .
¹H-NMR(CDCl₃)δ: 9. 94 (br s,1H), 7.76(d,1H,J=8.6Hz), 7.72(d,1H,J=8.6Hz), 7.67(s,1H), 7.31(t,1H,J=2.7Hz), 6.93(s,1H), 6.71(d,2H,J=1.9Hz), 6.64(t,1H,J=2.3Hz), 6.48(t,1H,J=2.1Hz), 3.83(s,6H).

### Example 1066-8

### 6-(5-bromo-2-trifluoromethyl-phenyl)-4-(3,5-dimethoxy-phenyl)-1-(2-hydroxy-ethyl)-1,6-dihydro-pyrrolo[2,3-c]pyridin-7-one

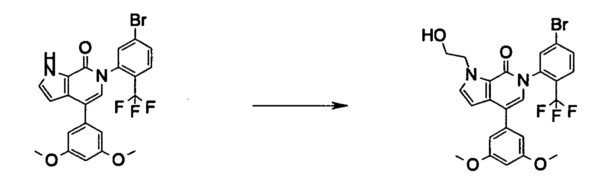

To a solution of 6-(5-bromo-2-trifluoromethylphenyl)-4-(3,5-dimethoxy-phenyl)-1,6-dihydro-pyrrolo[2,3-c]pyridin-7-one (830 mg) obtained in Example 1066-7 in N,N-dimethylacetamide (8.5 mL) were added 2-bromoethanol (0.36 mL) and potassium carbonate (698 mg). After stirring at 120°C for 5 hr, water was added, and the mixture was extracted with ethyl acetate. The organic layer was washed with water, dried, concentrated and purified by silica gel column chromatography (ethyl acetate:hexane=2:1) to give 6-(5-bromo-2-trifluoromethyl-phenyl)-4-(3,5-dimethoxy-phenyl)-1-(2-hydroxy-ethyl)-1,6-dihydro-pyrrolo[2,3-c]pyridin-7-one (913 mg).
¹H-NMR(CDCl₃)δ: 7.75(d,1H,J=8.6Hz), 7.70(d,1H,J=8.6Hz), 7.65(d,1H,J=1.6Hz), 7.22(d,1H,J=2.8Hz), 6.91(d,1H,J=0.7Hz), 6.68(d,2H,J=2.3Hz), 6.56(d,1H,J=2.8Hz), 6.48(t,1H,J=2.3Hz), 4.71(dd,2H,J=9.7,4.4Hz), 3.98(dd,2H,J=10.1,4.3Hz), 3.83(s,6H), 3.25(t,1H,J=5.4Hz).

### Example 1066-9

### 4-(3,5-dimethoxy-phenyl)-1-(2-hydroxy-ethyl)-6-[5-((S)-3-hydroxy-pyrrolidin-1-yl)-2-trifluoromethyl-phenyl]-1,6-dihydro-pyrrolo[2,3-c]pyridin-7-one

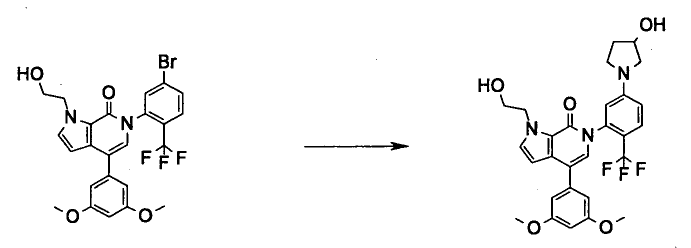

6-(5-Bromo-2-trifluoromethyl-phenyl)-4-(3,5-dimethoxy-phenyl)-1-(2-hydroxy-ethyl)-1,6-dihydro-pyrrolo[2,3-c]pyridin-7-one (100 mg) obtained in Example 1066-8 and (S)-3-hydroxy-pyrrolidine (49 mg) were reacted in the same manner as in Example 1-15, purified by silica gel column chromatography (chloroform :acetone=4:3), and further crystallized from chloroform-hexane to give 4-(3,5-dimethoxy-phenyl)-1-(2-hydroxy-ethyl)-6-[5-((S)-3-hydroxy-pyrrolidin-1-yl)-2-trifluoromethyl-phenyl]-1,6-dihydro-pyrrolo[2,3-c]pyridin-7-one (76 mg).
¹H-NMR(CDCl₃)δ: 7.60(d,1H,J=8.8Hz), 7.18(d,1H,J=2.8Hz), 7.01(s,1H), 6.70(d,2H,J=2.1Hz), 6.61(d,1H,J=8.8Hz), 6.56(d,1H,J=2.8Hz), 6.49(t,1H,J=2.7Hz), 6.47(t,1H,J=2.3Hz), 4.76(ddd,1H,J=14.4,6.6,4.1Hz), 4.68(td,1H,J=9.6,4.8Hz), 4.61(br s,1H), 4.01-3.91(m,2H), 3.82(s,6H), 3.78(q,1H,J=5.2Hz), 3.59-3.36(m,3H), 3.30(dd,1H,J=26.8,10.8Hz), 2.20-2.06(m,2H), 1.80(d,1H,J=4.4Hz).

### Example 1080

### N-(1-{3-[4-(3,5-dimethoxy-phenyl)-1-oxo-1,5,6,7-tetrahydro-cyclopenta[c]pyridin-2-yl]-4-trifluoromethyl-phenyl}-pyrrolidin-3-yl)-acetamide

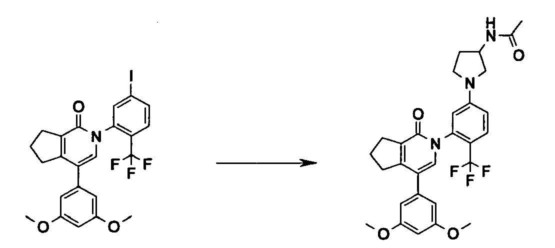

4-(3,5-Dimethoxy-phenyl)-2-(5-iodo-2-trifluoromethyl-phenyl)-2,5,6,7-tetrahydro-cyclopenta[c]pyridin-1-one (120 mg) obtained in Example 1066-2 and (R)-N-(pyrrolidin-3-yl)-acetamide (34 mg) were reacted in the same manner as in Example 1-15. The mixture was purified by silica gel thin layer chromatography, and further crystallized from a mixed solution of ethyl acetate-hexane to give N-(1-{3-[4-(3,5-dimethoxy-phenyl)-1-oxo-1,5,6,7-tetrahydro-cyclopenta[c]pyridin-2-yl]-4-trifluoromethyl-phenyl}-pyrrolidin-3-yl)-acetamide (50 mg).
¹H-NMR(CDCl₃)δ: 7.64-7.59(1H,m), 7.18(1H,s), 6.65-6.58(1H,m), 6.50-6.44(4H,m), 5.85-5.72(1H,m), 4.68-4.57(1H,m), 3.82(6H,s), 3.73-3.18(5H,m), 3.05-2.91(3H,m), 2.38-2.07(4H,m), 2.00(3H,s).

### Examples 1067 to 1084

The compounds of Examples 1067 to 1084 were obtained in the same manner as in a method selected from Examples 1066-1 to 1066-9. The compounds of Examples 1066-1 to 1066-9 and the compounds of Examples 1067 to 1084 obtained in the same manner are shown in the following Tables. In the tables, the left line shows Example numbers and the right line shows the structural formulas of the corresponding compounds.

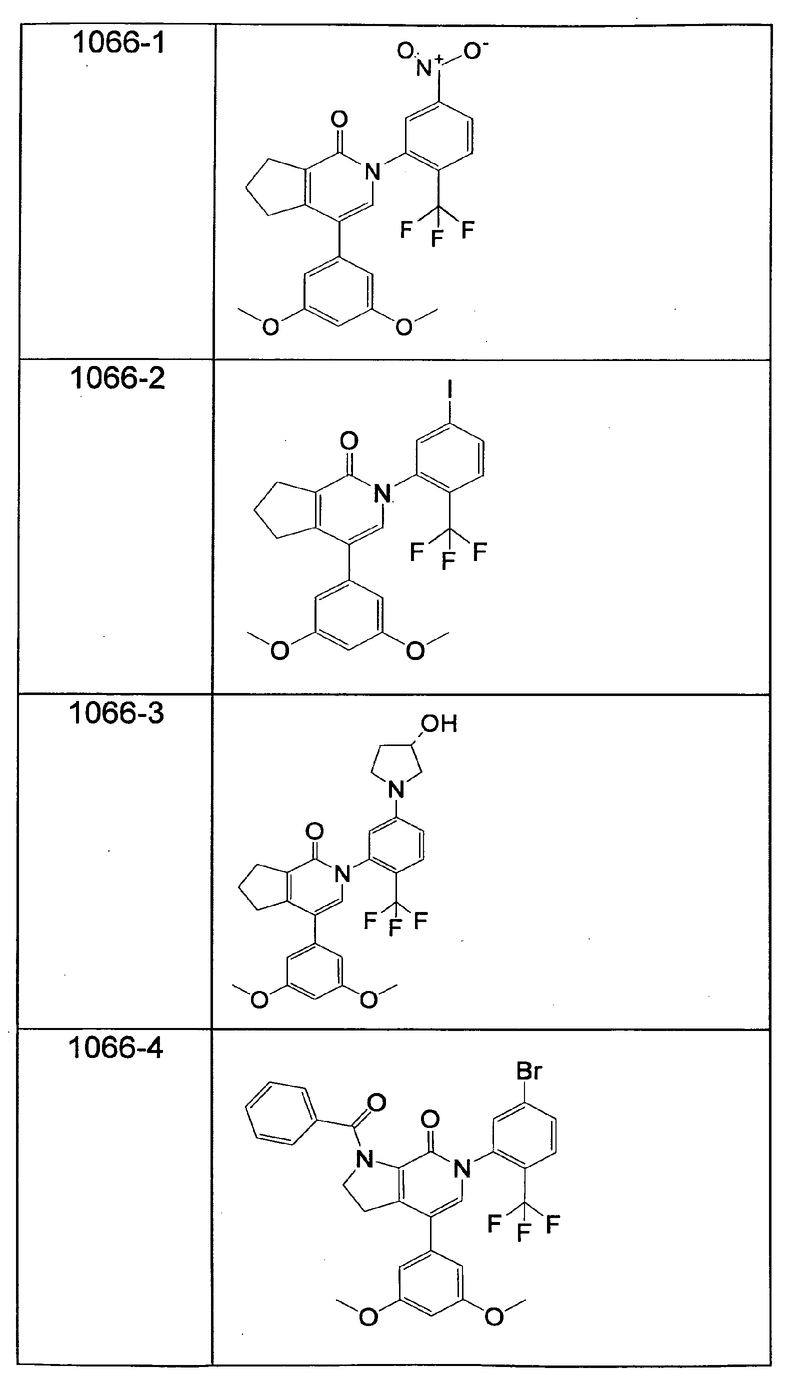

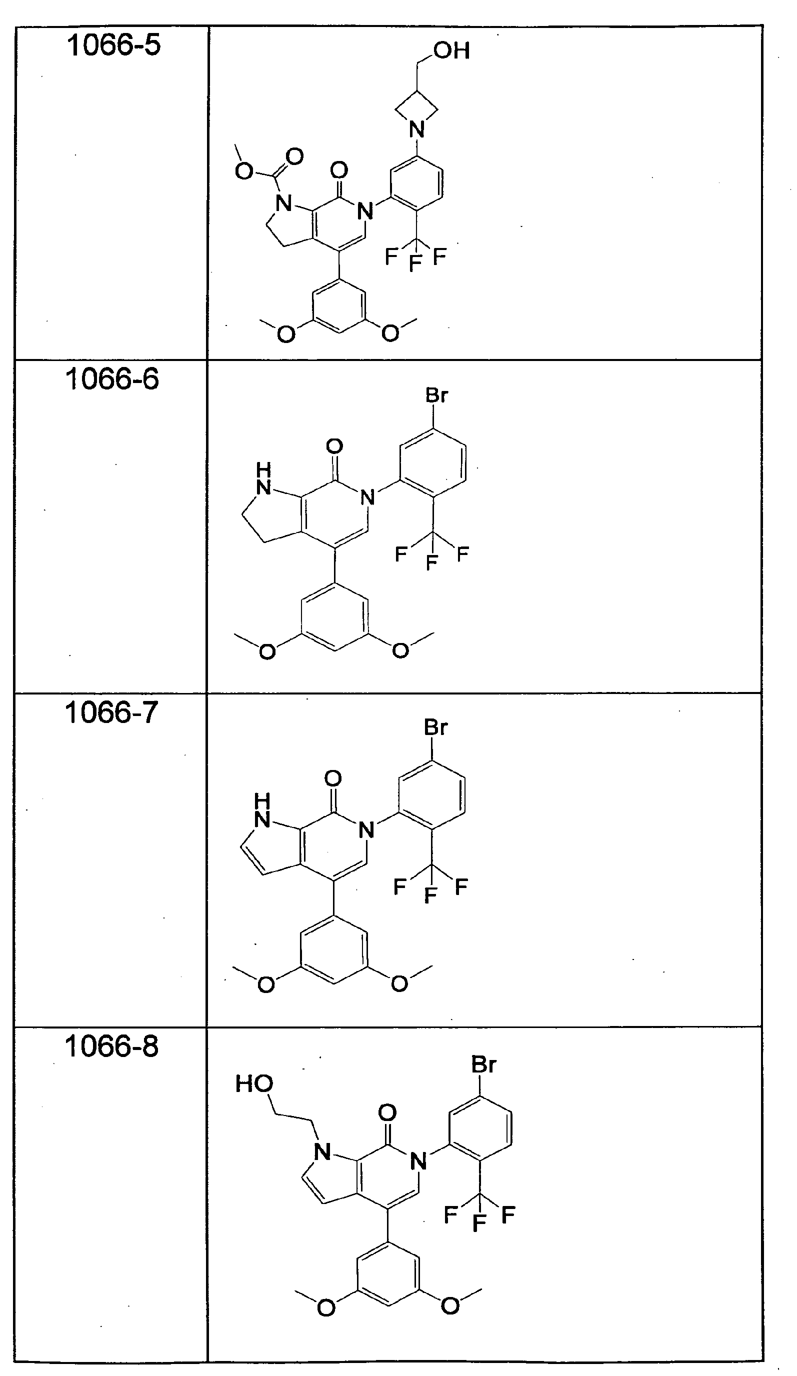

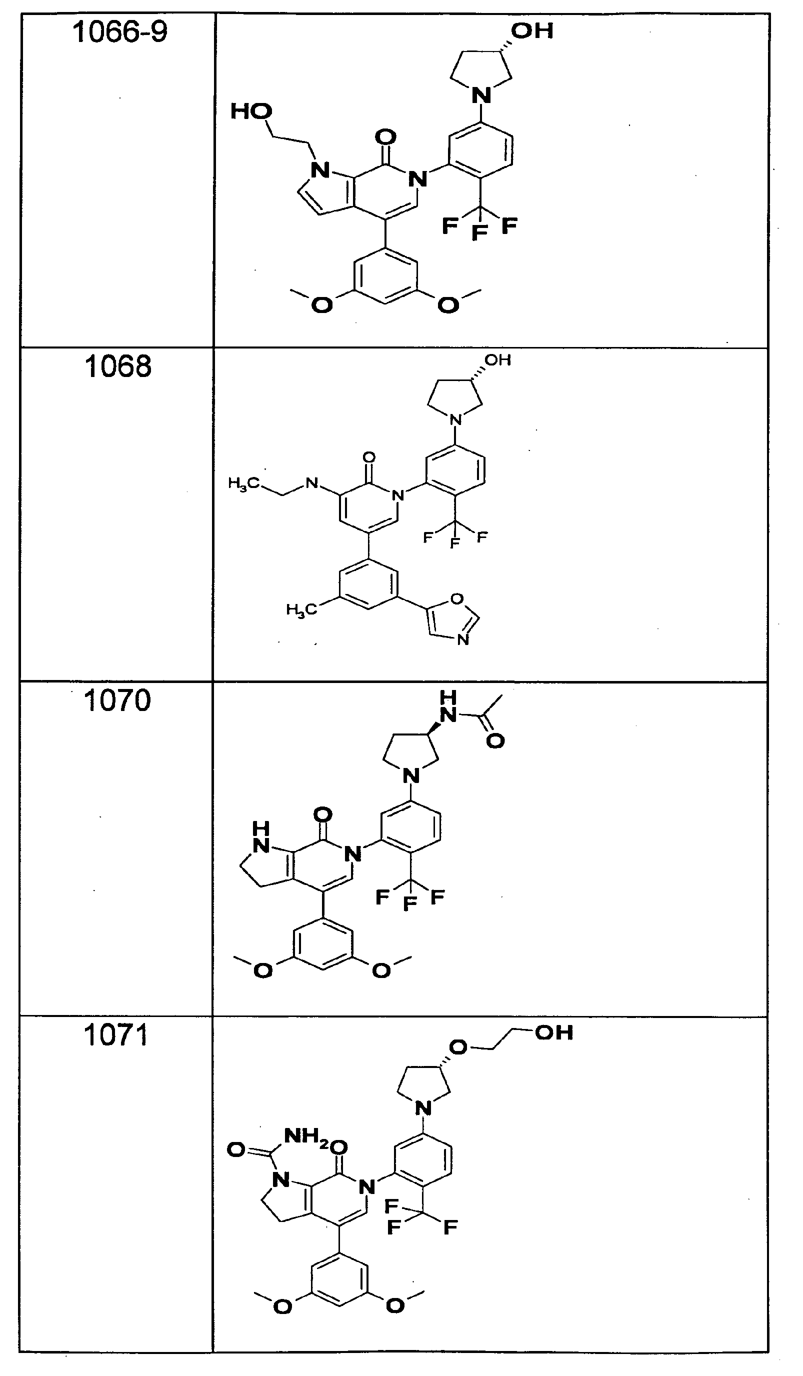

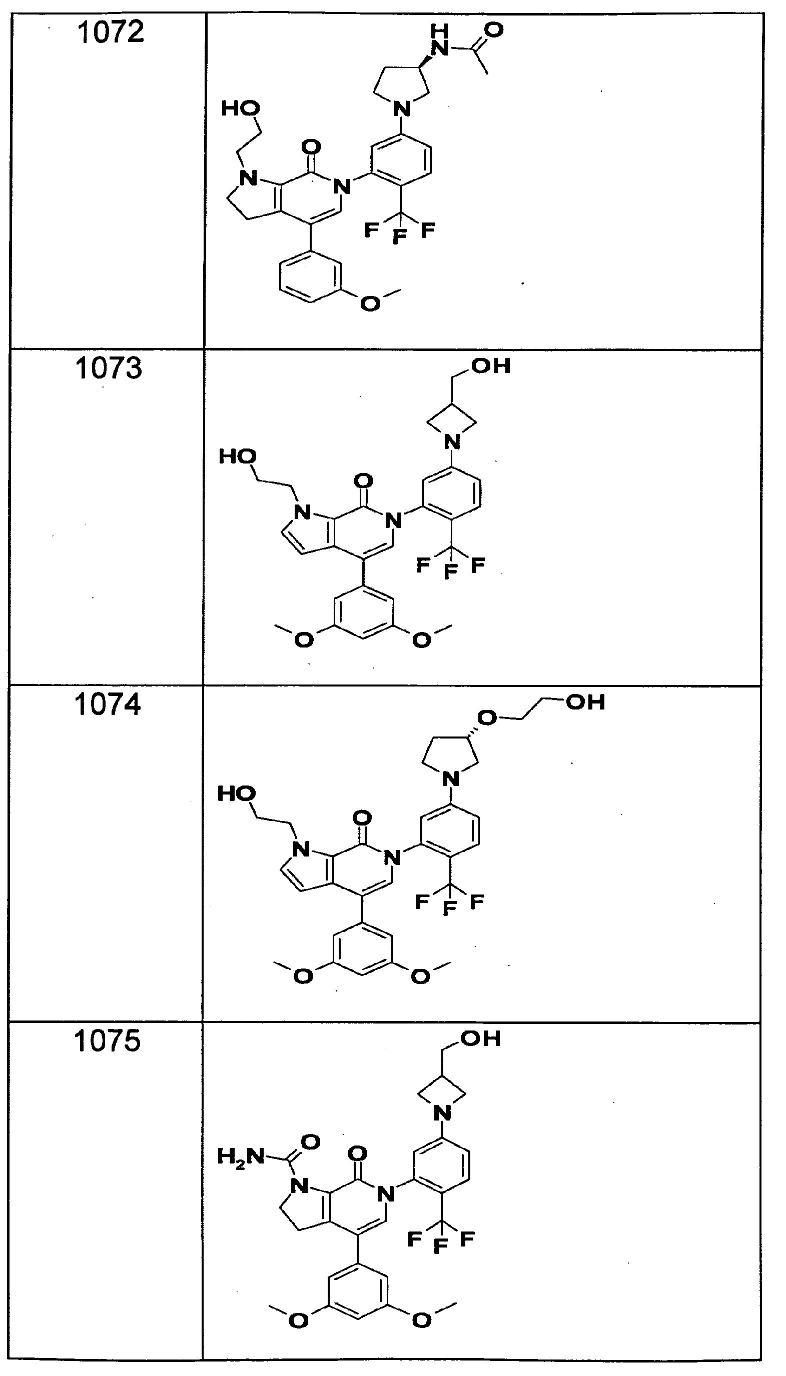

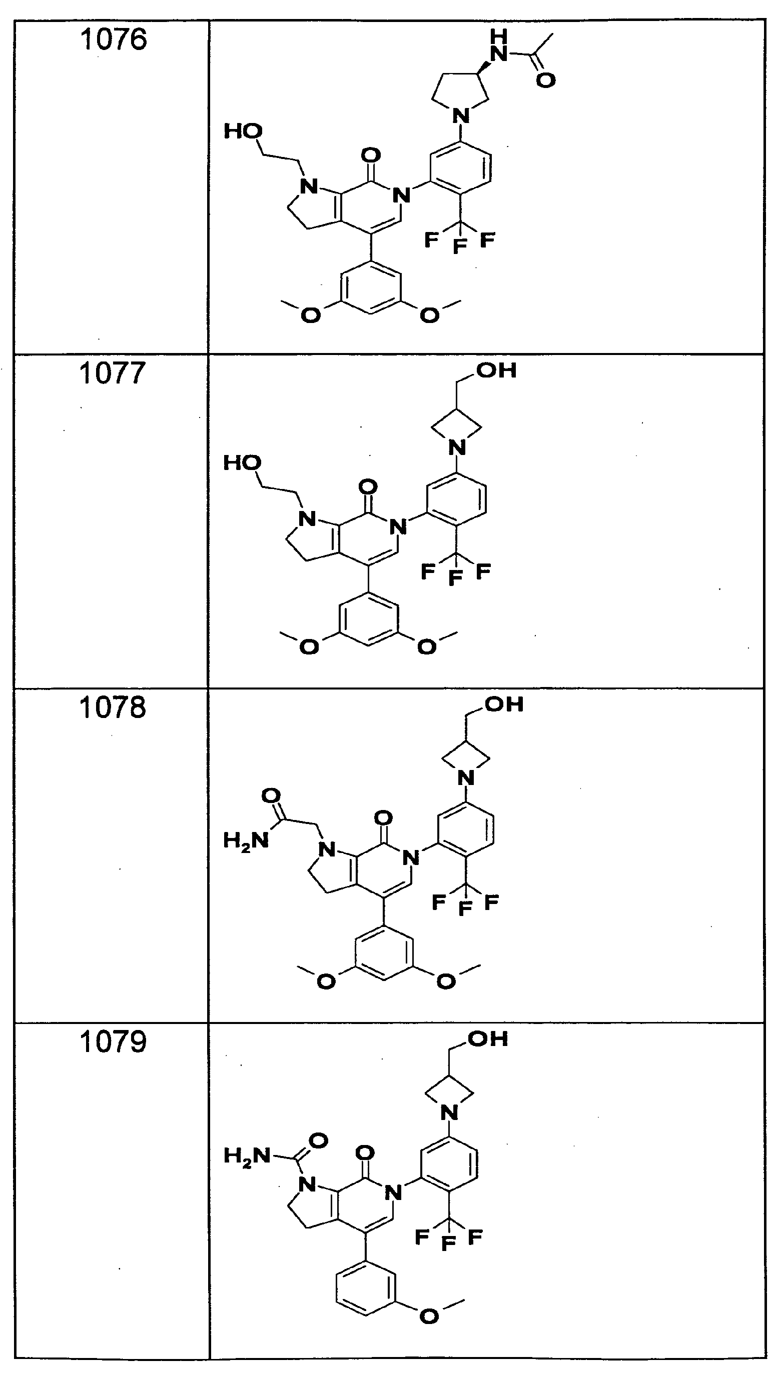

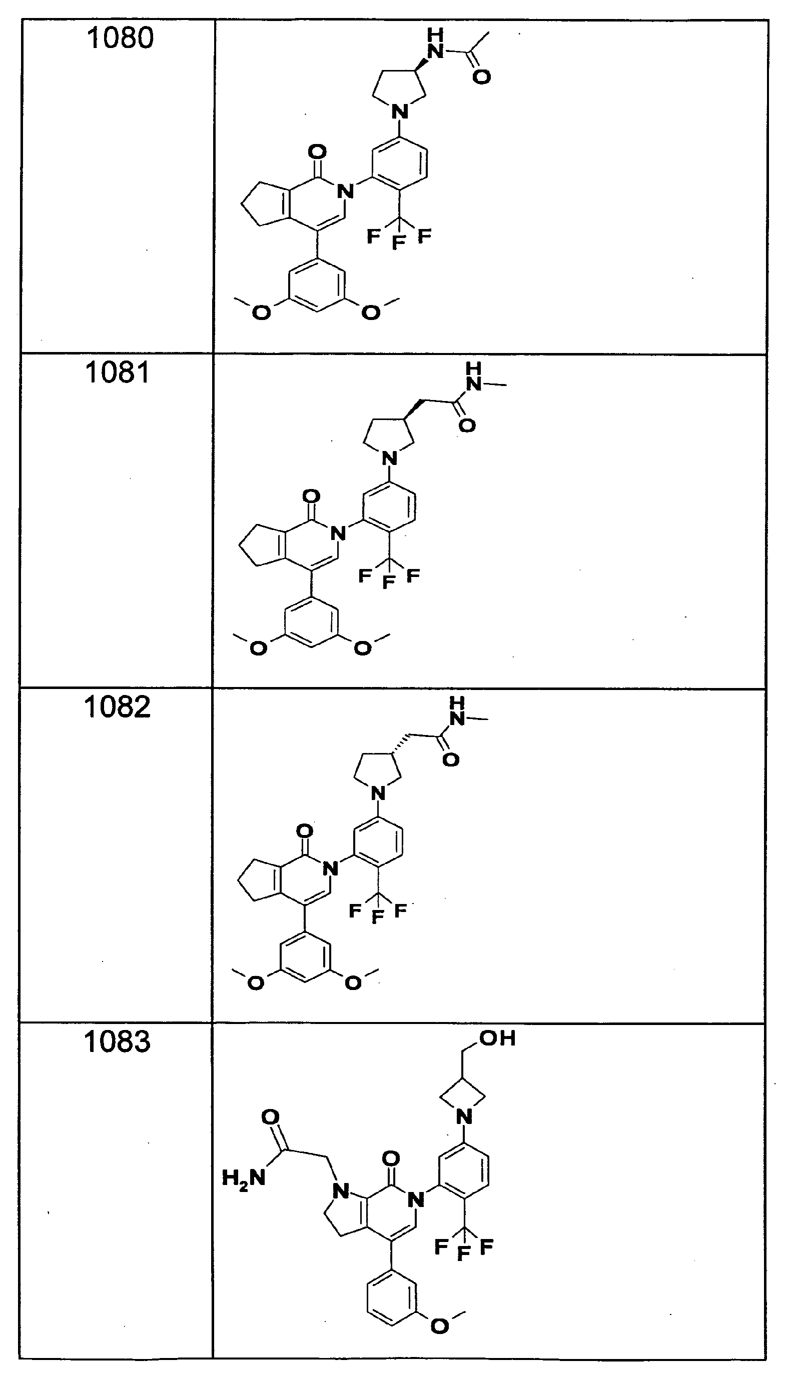

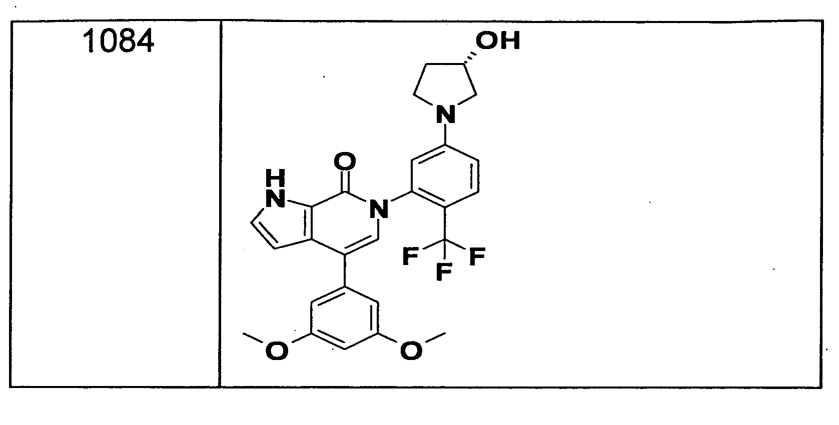

### Example 1085-1

### 8-(3,5-dimethoxy-phenyl)-6-(5-fluoro-2-trifluoromethylphenyl)-2,3-dihydro-1H-indolizin-5-one

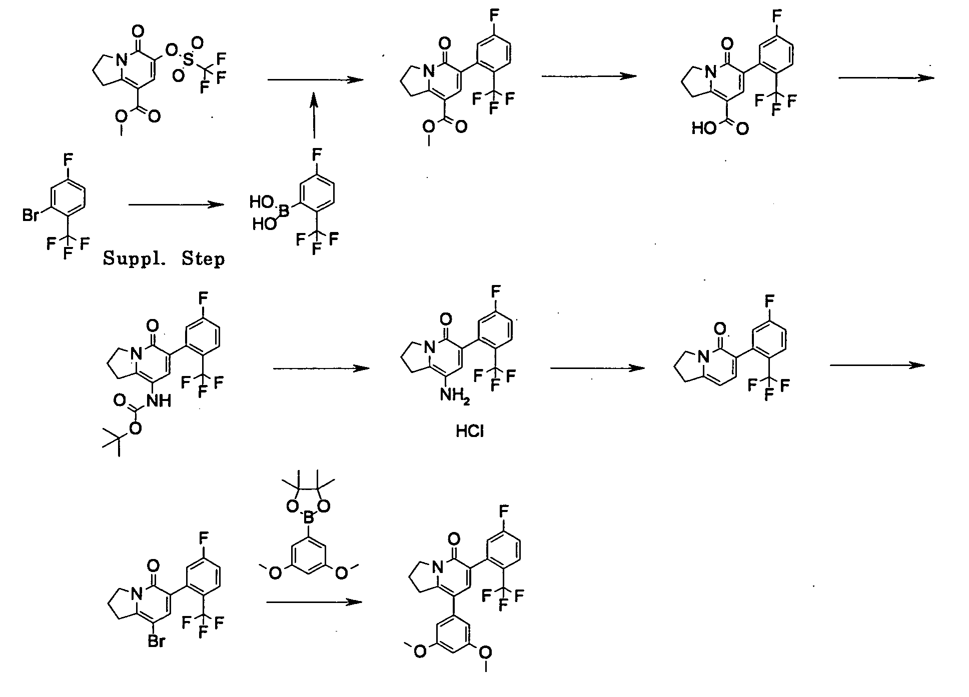

### Step 1

To a solution of methyl 5-oxo-6-trifluoromethanesulfonyloxy-1,2,3,5-tetrahydro-indolizine-8-carboxylate (600 mg) produced according to the method described in J. Org. Chem., 1999, 64, 8648-8659 in toluene (6 mL)-ethanol (6 mL) were added 4-fluoro-2-trifluoromethyl-phenylboronic acid (475 mg) obtained in supplementary step 1, 1,1'-bis(diphenylphosphino)ferrocene palladium(II) dichloride-dichloromethane complex (72 mg) and a 2 M aqueous sodium carbonate solution (6 mL) under argon. After stirring at 100°C for 1.5 hr, 4-fluoro-2-trifluoromethyl-phenylboronic acid (73 mg) and 1,1'-bis(diphenylphosphino)ferrocene palladium(II) dichloride-dichloromethane complex (14 mg) were added. After further stirring at the same temperature for 30 min, water was added, and the mixture was extracted with ethyl acetate. The organic layer was washed with water, dried, concentrated and purified by silica gel column chromatography (ethyl acetate:hexane=1:1-2:1) to give methyl 6-(5-fluoro-2-trifluoromethyl-phenyl)-5-oxo-1,2,3,5-tetrahydro-indolizine-8-carboxylate (471 mg).
¹H-NMR(CDCl₃)δ: 7.89(s,1H), 7.74(dd,1H,J=5.4,8.7Hz), 7.15(t,1H,J=8.1Hz), 7.07(d,1H,J=8.7Hz), 4.24(brt,2H,J=7.9Hz), 3.62(t,2H,J=7.9Hz), 2.29(tt,2H,J=7.9,7.9Hz)

### Step 2

To a solution methyl 6-(5-fluoro-2-trifluoromethyl-phenyl)-5-oxo-1,2,3,5-tetrahydro-indolizine-8-carboxylate (811 mg) produced by the method of step 1 in methanol (10 mL) was added a 4N aqueous sodium hydroxide solution (1.14 mL). After stirring under refluxing for 1.5 hr, the mixture was allowed to return to room temperature, and neutralized with 2N hydrochloric acid (3 mL). Precipitated crystals were collected by filtration to give 6-(5-fluoro-2-trifluoromethyl-phenyl)-5-oxo-1,2,3,5-tetrahydro-indolizine-8-carboxylic acid (740 mg).
¹H-NMR(DMSO-d₆)δ: 12.82(brs,1H), 7.89(dd,1H,J=5.7,8.7Hz), 7.73(s,1H), 7.46(t,1H,J=7.9Hz), 7.29(d,1H,J=9.0Hz), 4.07(t,2H,J=7.5Hz), 3.52(t,2H,J=7.5Hz), 2,17(tt,2H,J=7.5,7.5Hz)

### Step 3

To a solution of 6-(5-fluoro-2-trifluoromethylphenyl)-5-oxo-1,2,3,5-tetrahydro-indolizine-8-carboxylic acid (725 mg) obtained in step 2 in toluene (7 mL)-tert-butanol (7 mL) were added triethylamine (0.591 mL) and diphenylphosphorylazide (0.595 mL). After stirring at 60°C for 2 hr, and at 120°C for 2 hr, the mixture was allowed to return to room temperature, and a saturated aqueous sodium hydrogencarbonate solution was added. The mixture was extracted with ethyl acetate, and the organic layer was washed with an aqueous sodium hydrogencarbonate solution and water, dried, concentrated and purified by silica gel column chromatography (ethyl acetate:hexane=2:1-6:1) to give tert-butyl [6-(5-fluoro-2-trifluoromethyl-phenyl)-5-oxo-1,2,3,5-tetrahydro-indolizine-8-yl]-carbamate (820 mg).
¹H-NMR(CDCl₃)δ: 7.71(dd,1H,J=5.7,8.7Hz), 7.29(m,1H), 7.16-7.09(m,2H), 5.84(brs,1H), 4.23(t,2H,J=7.5Hz), 3.14(t,2H,J=7.5Hz), 2,26(tt,2H,J=7.5,7.5Hz), 1.48(s,9H)

### Step 4

To a solution of tert-butyl [6-(5-fluoro-2-trifluoromethyl-phenyl)-5-oxo-1,2,3,5-tetrahydro-indolizine-8-yl]-carbamate (820 mg) obtained in step 3 in ethyl acetate (8 mL) was added a 4N hydrogen chloride-ethyl acetate solution (8 mL). After stirring at room temperature for 18 hr, the precipitated crystals were collected by filtration to give 8-amino-6-(5-fluoro-2-trifluoromethyl-phenyl)-2,3-dihydro-1H-indolizin-5-one hydrochloride (650 mg).
¹H-NMR(DMSO-d₆)δ: 10.32(brs,3H), 7.90(dd,1H,J=5.6,8.6Hz), 7.50(s,1H), 7.48(td,1H,J=8.6,2.3Hz), 7.29(dd,1H,J=9.4,2.6Hz), 4.08(t,2H,J=7.9Hz), 3.30(t,2H,J=7.9Hz), 2.20(tt,2H,J=7.9,7.9Hz)

### Step 5

To a solution of 8-amino-6-(5-fluoro-2-trifluoromethyl-phenyl)-2,3-dihydro-1H-indolizin-5-one hydrochloride (650 mg) obtained in step 4 in 6N hydrochloric acid (6 mL) was added dropwise a solution of sodium nitrite (135 mg) in water (1 mL) under ice-cooling. After stirring at the same temperature for 20 min, a 50% aqueous phosphorous acid solution (6 mL) was added. The cooling bath was removed, and after stirring for 7 hr, a 8N aqueous sodium hydroxide solution (6 mL) was added, and the mixture was extracted with ethyl acetate. To the aqueous layer was added a saturated aqueous sodium hydrogencarbonate solution, and the mixture was extracted again with ethyl acetate. The combined organic layer was washed with water, dried and concentrated to give 6-(5-fluoro-2-trifluoromethyl-phenyl)-2,3-dihydro-1H-indolizin-5-one (494 mg).
¹H-NMR(CDCl₃)δ: 7.72(dd,1H,J=5.3,8.3Hz), 7.28(m,1H), 7.15-7.08(m,2H), 6.19(d,1H,J=6.8Hz), 4.21(t,2H,J=7.5Hz), 3.15(t,2H,J=7.5Hz), 2.25(tt,2H,J=7.5,7.5Hz)

### Step 6

6-(5-Fluoro-2-trifluoromethyl-phenyl)-2,3-dihydro-1H-indolizin-5-one (494 mg) obtained in step 5 was reacted in the same manner as in Example 1066-1, step 4, and purified by silica gel column chromatography (ethyl acetate:hexane=1:1) to give 8-bromo-6-(5-fluoro-2-trifluoromethyl-phenyl)-2,3-dihydro-1H-indolizin-5-one (463 mg).
¹H-NMR(CDCl₃)δ: 7.73(dd,1H,J=5.7,8.7Hz), 7.36(s,1H), 7.15(dt,1H,J=2.6,8.6Hz), 7.08(dd,1H,J=2.6,9.0Hz), 4.29(t,2H,J=7.9Hz), 3.19(t,2H,J=7.9Hz), 2.29(tt,2H,J=7.9,7.9Hz)

### Step 7

8-Bromo-6-(5-fluoro-2-trifluoromethyl-phenyl)-2,3-dihydro-1H-indolizin-5-one (200 mg) obtained in step 6 and 2-(3,5-dimethoxy-phenyl)-4,4,5,5-tetramethyl-[1,3,2]dioxaborolane (169 mg) were reacted in the same manner as in Example 1066-1, step 6, purified by silica gel thin layer chromatography (ethyl acetate:hexane=2:1) and crystallized (hexane) to give 8-(3,5-dimethoxy-phenyl)-6-(5-fluoro-2-trifluoromethylphenyl)-2,3-dihydro-1H-indolizin-5-one (147 mg).
¹H-NMR(CDCl₃)δ: 7.74(dd,1H,J=5.7,8.7H), 7.43(s,1H), 7.18-7.11(m,2H), 6.45-6.42(m,3H), 4.28(t,2H,J=7.2Hz), 3.81(s,6H), 3.27(t,2H,J=7.5Hz), 2.24(tt,2H,J=7.2,7.5Hz)

### Supplementary step 1

To a suspension of magnesium (330 mg) in tetrahydrofuran (5 mL) was added a small amount of iodine under argon, and a solution of 2-bromo-4-fluoro-1-trifluoromethylbenzene (3 g) in tetrahydrofuran (20 mL) was slowly added dropwise. After completion of the dropwise addition, the mixture was cooled to -70°C, and a solution of trimethylborate (1.15 g) in tetrahydrofuran (5 mL) was added dropwise. After stirring at the same temperature for 15 min, the cooling bath was removed, and the mixture was stirred for 1 hr. The reaction mixture was ice-cooled again, and a 2N hydrochloric acid (20 mL) was added. The mixture was stirred at room temperature for 1 hr and the mixture was separated into two layers. The aqueous layer was extracted two times with diethyl ether. The combined organic layer was washed with water, dried and concentrated. The obtained crystals were washed with hexane to give 4-fluoro-2-trifluoromethyl-phenylboronic acid (1.69 g).
¹H-NMR(DMSO-d₆)δ: 8.46(brs,1H), 7.82-7.62(m,2H), 7.41-7.31(m,2H)

### Example 1085-2

### 8-(3,5-dimethoxy-phenyl)-6-[5-((S)-3-hydroxy-pyrrolidin-1-yl)-2-trifluoromethyl-phenyl]-2,3-dihydro-1H-indolizin-5-one

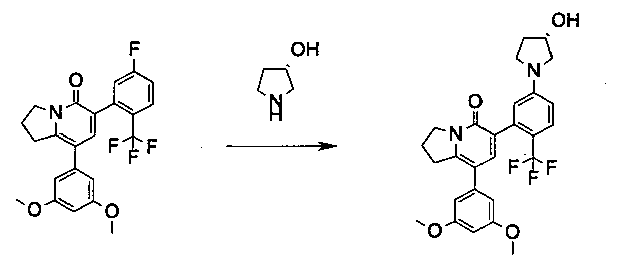

8-(3,5-Dimethoxy-phenyl)-6-(5-fluoro-2-trifluoromethyl-phenyl)-2,3-dihydro-1H-indolizin-5-one (80 mg) obtained in Example 1085-1 and (S)-3-hydroxy-pyrrolidine (0.030 mL) were reacted in the same manner as in Example 1-23, step 1, purified by silica gel thin layer chromatography (chloroform: methanol=9:1) and crystallized (chloroform-hexane) to give 8-(3,5-dimethoxy-phenyl)-6-[5-((S)-3-hydroxy-pyrrolidin-1-yl)-2-trifluoromethyl-phenyl]-2,3-dihydro-1H-indolizin-5-one (52 mg).
¹H-NMR(CDCl₃)δ: 7.56(d,1H,J=9.0Hz), 7.44(s,1H), 6.55-6.40(m,5H), 4.27(m,1H), 4.27(t,2H,J=7.5Hz), 3.81(s,6H), 3.56-3.27(m,4H), 3.26(t,2H,J=7.5Hz), 2.28-2.21(m,4H)

### Examples 1086-1089

The compounds of Examples 1086 to 1089 were obtained in the same manner as in a method selected from Examples 1085-1 and 1085-2. The compounds of Examples 1085-1 and 1085-2 and the compounds of Examples 1086 to 1089 obtained in the same manner are shown in the following Tables. In the tables, the left line shows Example numbers and the right line shows the structural formulas of the corresponding compounds.

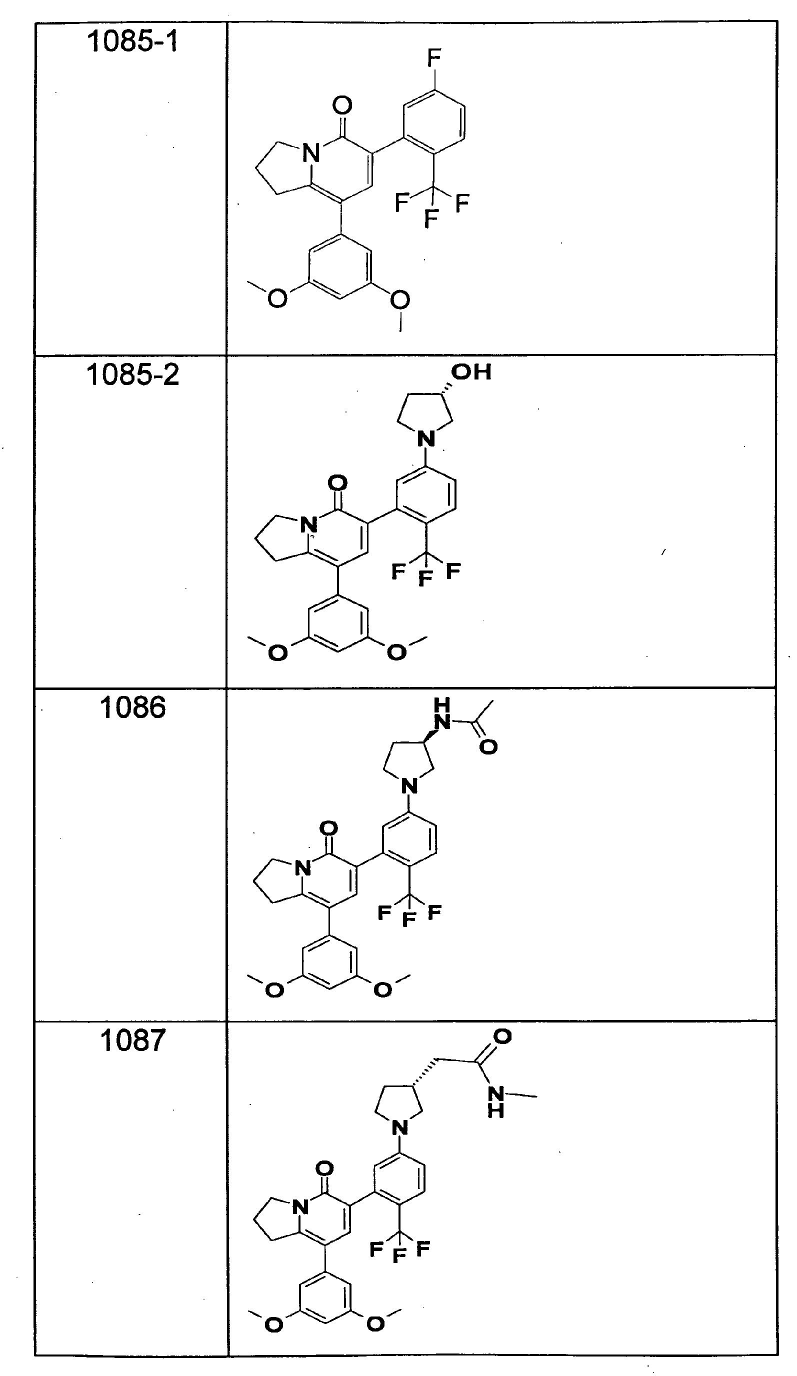

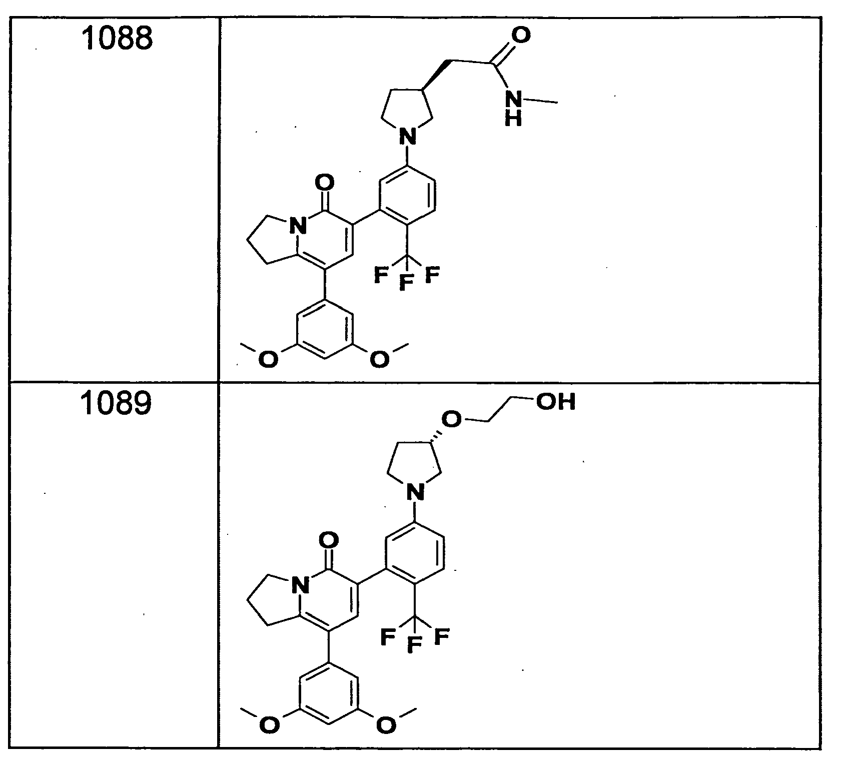

### Example 1090-1

### 1-(3,5-dimethoxy-phenyl)-3-(5-fluoro-2-trifluoromethylphenyl)-1,5,6,7-tetrahydro-cyclopenta[b]pyridin-4-one

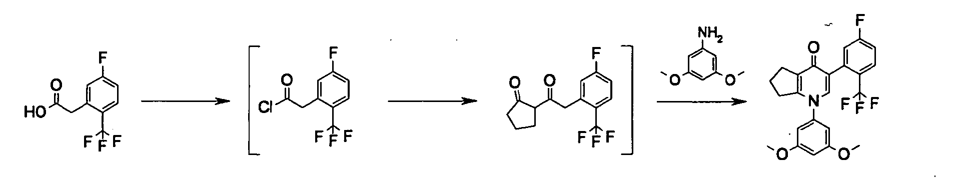

To a solution of (5-fluoro-2-trifluoromethylphenyl)acetic acid (2.0 g) in toluene (20 mL) were added thionyl chloride (1.31 mL) and N,N-dimethylformamide (0.2 mL) under nitrogen. After stirring at room temperature for 2.5 hr, the reaction mixture was concentrated, an azeotropic operation was performed using toluene, and the obtained resultant product was dissolved in chloroform (10 mL). The solution was added to a solution of 1-(morpholino-4-yl)-cyclopentene (4.32 mL) in chloroform (30 mL) under nitrogen and under ice-cooling. The cooling bath was removed, and after stirring for 22 hr, 2N hydrochloric acid (40 mL) was added. The mixture was extracted with chloroform, and the organic layer was washed with water, dried, concentrated, and purified by silica gel column chromatography (ethyl acetate:hexane=1:5). The obtained resultant product (1.89 g) was dissolved in toluene (20 mL). Diethoxymethyldimethylamine (1.69 mL) was added, and the mixture was stirred at 90°C for 5 min, and stirred under refluxing for 1.5 hr. The mixture was allowed to return to room temperature, 3,5-dimethoxyaniline (3.01 g) was added, and the mixture was stirred again under refluxing for 22 hr. The mixture was allowed to return to room temperature, 2N hydrochloric acid was added, and the mixture was extracted with ethyl acetate. The organic layer was washed with water, dried, concentrated and purified by silica gel column chromatography (ethyl acetate:hexane=3:1-ethyl acetate-ethyl acetate:methanol=10:1) and silica gel thin layer chromatography (chloroform:methanol=10:1) to give 1-(3,5-dimethoxy-phenyl)-3-(5-fluoro-2-trifluoromethylphenyl)-1,5,6,7-tetrahydro-cyclopenta[b]pyridin-4-one (354 mg).
¹H-NMR(CDCl₃)δ: 7.72(dd,1H,J=8.7,5.0Hz), 7.39(s,1H), 7.19(dd,1H,J=8.7,3.1Hz), 7.12(td,1H,J=8.1,3.0Hz), 6.52(t,1H,J=2.0Hz), 6.43(d,2H,J=2.3Hz), 3.82(s,6H), 2.96(t,2H,J=7.3Hz), 2.82(t,2H,J=7.5Hz), 2.15-2.03(m,2H).

### Example 1090-2

### 1-(3,5-dimethoxy-phenyl)-3-[5-((S)-3-hydroxy-pyrrolidin-1-yl)-2-trifluoromethyl-phenyl]-1,5,6,7-tetrahydro-cyclopenta[b]pyridin-4-one

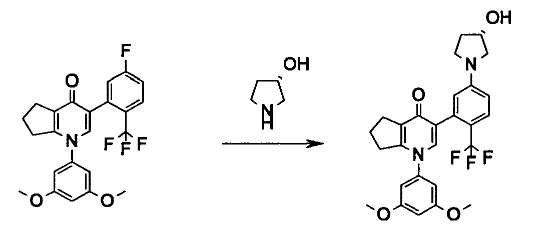

1-(3,5-Dimethoxy-phenyl)-3-(5-fluoro-2-trifluoromethyl-phenyl)-1,5,6,7-tetrahydro-cyclopenta[b]pyridin-4-one (150 mg) obtained in Example 1090-1 and (S)-3-hydroxy-pyrrolidine (121 mg) were reacted in the same manner as in Example 1-23, step 1, and then purified by silica gel thin layer chromatography (chloroform:methanol=10:1) and crystallized (methanol-water) to give 1-(3,5-dimethoxy-phenyl)-3-[5-((S)-3-hydroxy-pyrrolidin-1-yl)-2-trifluoromethyl-phenyl]-1,5,6,7-tetrahydro-cyclopenta[b]pyridin-4-one (52 mg).
¹H-NMR(DMSO-d₆)δ: 7.52-7.43(m,2H), 6.66(d,2H,J=1.9Hz), 6.63-6.53(m,2H), 6.39-6.33(m,1H), 4.98(d,1H,J=3.8Hz), 4.44-4.36(m,1H), 3.79(s,6H), 3.47-3.31(m,3H), 3.17-3.07(m,1H), 2.83(t,2H,J=7.2Hz), 2.70(t,2H,J=7.2Hz), 2.10-1.84(m,4H).

### Example 1091-1

### 4-(3,5-dimethoxy-phenyl)-2-(5-nitro-2-trifluoromethylphenyl)-2,5,6,7-tetrahydro-cyclopenta[c]pyridin-3-one

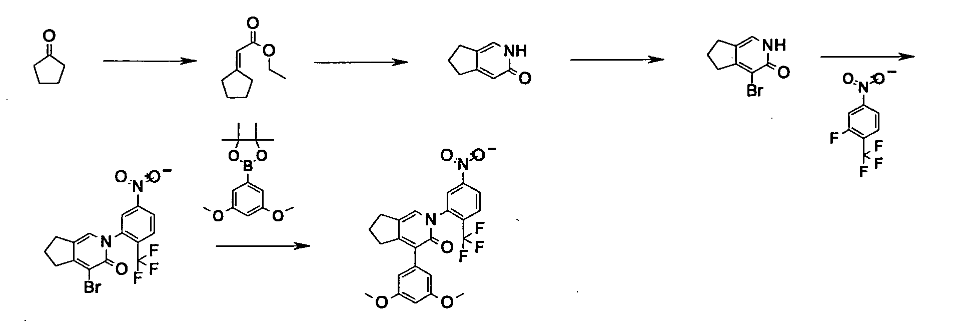

### Step 1

To a solution of cyclopentanone (5.0 mL) in 1,2-dimethoxyethane (50 mL) was added ethyl (diethoxy-phosphoryl)acetate (11.2 mL) under nitrogen and under ice-cooling. Furthermore, at the same temperature, sodium hydride (60%; 2.2 g) was added by small portions. The mixture was stirred under ice-cooling for 15 min, and at room temperature for 15 hr, and the reaction mixture was poured into ice water. The mixture was extracted with ethyl acetate and the organic layer was washed with water, dried, concentrated and purified by silica gel column chromatography (hexane-ethyl acetate:hexane=1:6) to give ethyl cyclopentylidene-acetate (6.66 g).
¹H-NMR(CDCl₃)δ: 5.82-5.78(m,1H), 4.15(q,2H,J=7.2Hz), 2.81-2.74(m,2H), 2.47-2.40(m,2H), 1.80-1.59(m,4H), 1.27(t,3H,J=7.2Hz).

### Step 2

To a solution of ethyl cyclopentylidene-acetate (1.9 g) obtained in step 1 in N,N-dimethylformamide (20 mL) were added sodium hydride (60%; 493 mg) and 1,3,5-triazine (1.0 g) under nitrogen and under ice-cooling. After stirring at 110°C for 25 hr, the mixture was allowed to return to room temperature, and the reaction mixture was concentrated. The residue was suspended in chloroform, and the insoluble material was filtered off, purified by silica gel column chromatography (chloroform:methanol=10:1) and crystallized (ethyl acetate) to give 2,5,6,7-tetrahydro-cyclopenta[c]pyridin-3-one (595 mg).
¹H-NMR(CDCl₃)δ: 12.89(s,1H), 7.16(s,1H), 6.43(s,1H), 2.79(td,2H,J=7.5,1.2Hz), 2.70(td,2H,J=7.3,1.2Hz), 2.08-2.00(m,2H).

### Step 3

2,5,6,7-Tetrahydro-cyclopenta[c]pyridin-3-one (393 mg) obtained in step 2 was reacted in the same manner as in Example 1066-1, step 4, and crystallized from ethyl acetate-hexane to give 4-bromo-2,5,6,7-tetrahydro-cyclopenta[c]pyridin-3-one (511 mg).
¹H-NMR(CDCl₃)δ:12.91(s,1H),7.23(s,1H),2.92-2.82(m,4H),2.16-2.06(m,2H).

### Step 4

4-Bromo-2,5,6,7-tetrahydro-cyclopenta[c]pyridin-3-one (511 mg) obtained in step 3 was reacted in the same manner as in Example 1066-1, step 5, and purified by silica gel column chromatography (ethyl acetate:hexane=1:3) to give 4-bromo-2-(5-nitro-2-trifluoromethyl-phenyl)-2,5,6,7-tetrahydro-cyclopenta[c]pyridin-3-one (329 mg).
¹H-NMR(CDCl₃)δ: 8.46-8.42(m,1H), 8.25(d,1H,J=2.2Hz), 8.04(d,1H,J=8.8Hz), 6.98(s,1H), 2.98-2.85(m,4H), 2.24-2.10(m,2H).

### Step 5

4-Bromo-2-(5-nitro-2-trifluoromethyl-phenyl)-2,5,6,7-tetrahydro-cyclopenta[c]pyridin-3-one (329 mg) obtained in step 4 was reacted in the same manner as in Example 1066-1, step 6, and purified by silica gel column chromatography (ethyl acetate:hexane=1:5) to give 4-(3,5-dimethoxy-phenyl)-2-(5-nitro-2-trifluoromethyl-phenyl)-2,5,6,7-tetrahydro-cyclopenta[c]pyridin-3-one (291 mg).
¹H-NMR(CDCl₃)δ: 8.41(dd,1H,J=8.8,2.1Hz), 8.32(d,1H,J=2.1Hz), 8.03(d,1H,J=8.8Hz), 7.02(s,1H), 6.60(d,2H,J=2.3Hz), 6.43(t,1H,J=2.3Hz), 3.79(s,6H), 2.88(t,2H,J=7.4Hz), 2.83-2.77(m,2H), 2.10-2.02(m,2H).

### Example 1091-2

### 2-(5-amino-2-trifluoromethyl-phenyl)-4-(3,5-dimethoxy-phenyl)-2,5,6,7-tetrahydro-cyclopenta[c]pyridin-3-one

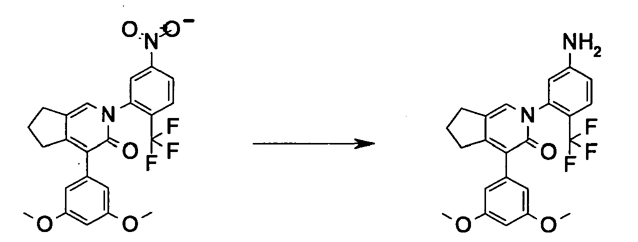

4-(3,5-Dimethoxy-phenyl)-2-(5-nitro-2-trifluoromethyl-phenyl)-2,5,6,7-tetrahydro-cyclopenta[c]pyridin-3-one (291 mg) obtained in Example 1091-1 was reacted in the same manner as in Example 1-2 and purified by silica gel column chromatography (chloroform:methanol=10:1) to give 2-(5-amino-2-trifluoromethyl-phenyl)-4-(3,5-dimethoxy-phenyl)-2,5,6,7-tetrahydro-cyclopenta[c]pyridin-3-one (268 mg).
¹H-NMR(CDCl₃)δ: 7.51(d,1H,J=8.8Hz), 7.03(s,1H), 6.69(dd,1H,J=8.4,2.2Hz), 6.62(d,2H,J=2.2Hz), 6.60(d,1H,J=2.2Hz), 6.41(t,1H,J=2.2Hz), 3.78(s,6H), 2.88-2.81(m,2H), 2.80-2.72(m,2H), 2.08-1.97(m,2H).

### Example 1091-3

### 2-(5-bromo-2-trifluoromethyl-phenyl)-4-(3,5-dimethoxy-phenyl)-2,5,6,7-tetrahydro-cyclopenta[c]pyridin-3-one

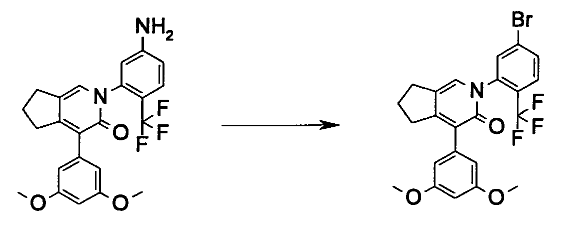

To a solution of 2-(5-amino-2-trifluoromethylphenyl)-4-(3,5-dimethoxy-phenyl)-2,5,6,7-tetrahydro-cyclopenta[c]pyridin-3-one (120 mg) obtained in Example 1091-2 in acetic acid (0.8 mL)-aqueous hydrobromic acid solution (48%; 0.2 mL) was added a solution of sodium nitrite (22 mg) in water (0.2 mL) under ice-cooling. After stirring at the same temperature for 20 min, a mixture of copper(I) bromide (80 mg) and an aqueous hydrobromic acid solution (0.2 mL) was added. After stirring under ice-cooling for 20 min, the mixture was poured into ice water, and the mixture was extracted with ethyl acetate. The organic layer was washed with an aqueous sodium hydrogencarbonate solution, dried, concentrated and purified by silica gel column chromatography (ethyl acetate:hexane=1:3) to give 2-(5-bromo-2-trifluoromethyl-phenyl)-4-(3,5-dimethoxy-phenyl)-2,5,6,7-tetrahydro-cyclopenta[c]pyridin-3-one (45 mg).
¹H-NMR(CDCl₃)δ: 7.72-7.64(m,2H), 7.61-7.59(m,1H), 7.00(s,1H), 6.60(d,2H,J=2.3Hz), 6.42(t,1H,J=2.3Hz), 3.79(s,6H), 2.86(t,2H,J=7.3Hz), 2.81-2.74(m,2H), 2.09-1.99(m,2H).

### Example 1091-4

### 4-(3,5-dimethoxy-phenyl)-2-[5-((S)-3-hydroxy-pyrrolidin-1-yl)-2-trifluoromethyl-phenyl]-2,5,6,7-tetrahydro-cyclopenta[c]pyridin-3-one

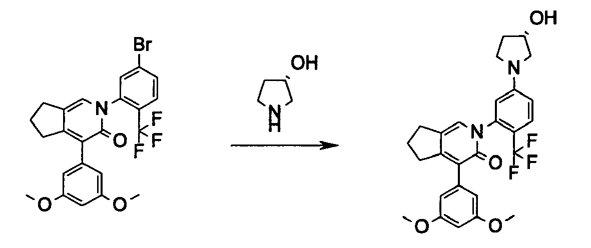

2-(5-Bromo-2-trifluoromethyl-phenyl)-4-(3,5-dimethoxy-phenyl)-2,5,6,7-tetrahydro-cyclopenta[c]pyridin-3-one (53 mg) produced by the method of Example 1091-3 and (S)-3-hydroxy-pyrrolidine (18 mg) were reacted in the same manner as in Example 1-15, purified by silica gel thin layer chromatography (chloroform:methanol=20:1) and crystallized (methanol-water) to give 4-(3,5-dimethoxy-phenyl)-2-[5-((S)-3-hydroxy-pyrrolidin-1-yl)-2-trifluoromethyl-phenyl]-2,5,6,7-tetrahydro-cyclopenta[c]pyridin-3-one (19 mg).
¹H-NMR(CDCl₃)δ: 7.56(d,1H,J=8.8Hz), 7.07(s,1H), 6.64(d,2H,J=2.1Hz), 6.56(d,1H,J=8.8Hz), 6.44(t,1H,J=2.7Hz), 6.41(t,1H,J=2.3Hz), 4.62-4.57(m,1H), 3.78(s,6H), 3.56-3.23(m,4H), 2.88-2.75(m,4H), 2.15-2.00(m,4H).

### Example 1092-1

### 4-(3,5-dimethoxy-phenyl)-2-(5-fluoro-2-trifluoromethylphenyl)-6,7-dihydro-5H-cyclopenta[b]pyridine

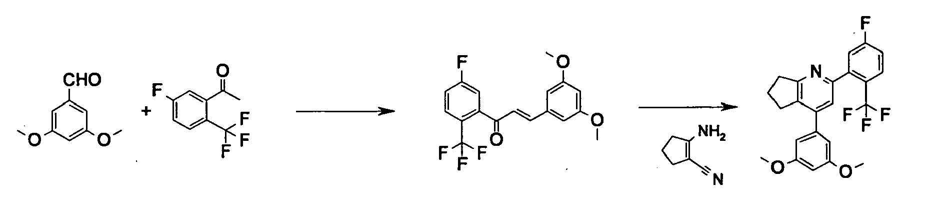

### Step 1

To a solution of 5'-fluoro-2'-trifluoromethyl-actophenone (1.13 g) in ethanol (5 mL) were added a 8N aqueous potassium hydroxide solution (3.13 mL) and 3,5-dimethoxy-benzaldehyde (831 mg) under ice-cooling. After stirring at the same temperature for 1 hr, water was added, and the mixture was extracted with diisopropyl ether. The organic layer was washed with a 10% aqueous citric acid solution, dried, concentrated and purified by silica gel column chromatography (ethyl acetate:hexane=1:6-1:5) to give (E)-3-(3,5-dimethoxy-phenyl)-1-(5-fluoro-2-trifluoromethyl-phenyl)-propenone (985 mg).
¹H-NMR(CDCl₃)δ: 7.76(dd,1H,J=5.1,8.7Hz), 7.27(m,1H), 7.24(d,1H,J=16.2Hz), 7.14(dd,1H,J=2.7,8.7Hz), 6.95(d,1H,J=16.2Hz), 6.64(d,2H,J=2.1Hz), 6.51(t,1H,J=2.1Hz).

### Step 2

To a solution of (E)-3-(3,5-dimethoxy-phenyl)-1-(5-fluoro-2-trifluoromethyl-phenyl)-propenone (177 mg) obtained in step 1 in acetic acid (1.5 mL) were added 1-amino-2-cyano-1-cyclopentene (108 mg) and ammonium acetate (116 mg). After stirring at 130°C for 14 hr, water-ethyl acetate and activated carbon were added, and the mixture was stirred at room temperature. The activated carbon was filtered off, and the filtrate was separated into layers. The organic layer was washed with an aqueous sodium hydrogencarbonate solution, dried, concentrated and purified by silica gel column chromatography (ethyl acetate:hexane=1:4) to give 4-(3,5-dimethoxy-phenyl)-2-(5-fluoro-2-trifluoromethylphenyl)-6,7-dihydro-5H-cyclopenta[b]pyridine (45 mg).
¹H-NMR(CDCl₃)δ: 7.77(dd,1H,J=4.2,6.6Hz), 7.30(m,2H), 7.20(ddd,1H,J=2.1,6.0,6.6Hz), 6.64(d,2H,J=1.8Hz), 6.53(t,1H,J=1.8Hz), 3.85(s,6H), 3.16(t,2H,J=5.4Hz), 3.13(t,2H,J=5.4Hz), 2.19(quintet,2H,J=5.4Hz).

### Example 1092-2

### (S)-1-{3-[4-(3,5-dimethoxy-phenyl)-6,7-dihydro-5H-cyclopenta[b]pyridin-2-yl]-4-trifluoromethyl-phenyl}-pyrrolidin-3-ol

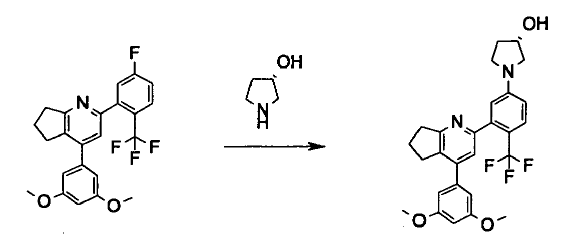

4-(3,5-Dimethoxy-phenyl)-2-(5-fluoro-2-trifluoromethyl-phenyl)-6,7-dihydro-5H-cyclopenta[b]pyridine (43 mg) obtained in Example 1092-1 and (S)-3-hydroxy-pyrrolidine (90 mg) were reacted in the same manner as in Example 1-23, step 1, and purified by silica gel thin layer chromatography (chloroform:methanol=10:1, chloroform:acetone=10:2) to give (S)-1-{3-[4-(3,5-dimethoxy-phenyl)-6,7-dihydro-5H-cyclopenta[b]pyridin-2-yl]-4-trifluoromethyl-phenyl}-pyrrolidin-3-ol (22 mg).
¹H-NMR(CDCl₃)δ: 7.57(d,1H,J=6.6Hz), 7.29(s,1H), 6.67(d,1H,J=1.8Hz), 6.63(d,1H,J=1.8Hz), 6.56(dd,1H,J=1.8,6.6Hz), 6.52(t,1H,J=1.8Hz), 4.62(brs,1H), 3.84(s,6H), 3.57(m,2H), 3.45(dt,1H,J=2.4,6.6Hz), 3.32(d,1H,J=8.1Hz), 3.18(t,2H,J=5.4Hz), 3.12(t,2H,J=5.4Hz), 2.13(m,4H).

### Example 1093-1

### 1-(3,5-dimethoxy-phenyl)-3-(5-fluoro-2-trifluoromethylphenyl)-6,7-dihydro-5H-cyclopenta[c]pyridine

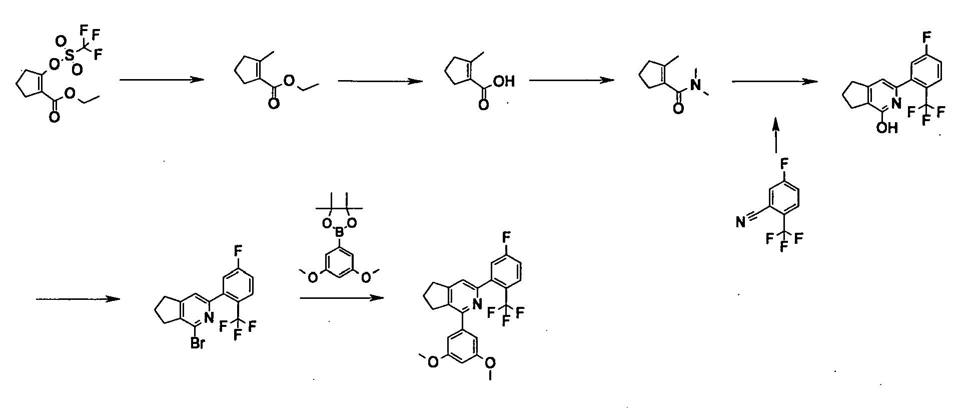

### Step 1

To a suspension of copper(I) iodide (95%; 24.1 g) in diethyl ether (200 mL) was added dropwise methyl lithium (1 M diethyl ether solution; 200 mL) under nitrogen and under ice-cooling. After stirring at the same temperature for 30 min, the mixture was cooled to -78°C, and a solution of ethyl 2-trifluoromethanesulfonyloxy-cyclopent-1-ene carboxylate (16.4 g) produced by a method similar to the method of Example 1-16, step 1, in diethyl ether (100 mL) was added dropwise. After stirring at the same temperature for 2 hr, 3N hydrochloric acid (80 mL) was added dropwise and the mixture was allowed to return to room temperature. The insoluble material was filtered off, and the mixture was separated into two layers. The aqueous layer was extracted with diethyl ether. The organic layer was washed with water, dried and concentrated to give ethyl 2-methyl-cyclopent-1-ene carboxylate (4.85 g).
¹H-NMR(CDCl₃)δ: 4.18(q,2H,J=7.2Hz), 2.64-2.58(m,2H), 2.47(t,2H,J=7.7Hz), 2.09(s,3H), 1.86-1.76(m,2H), 1.29(t,3H,J=7.2Hz).

### Step 2

To a solution of ethyl 2-methyl-cyclopent-1-enecarboxylate (1.0 g) obtained in step 1 in ethanol (10 mL) was added a 4N aqueous sodium hydroxide solution (4.86 mL). After stirring under refluxing for 2 hr, the mixture was allowed to return to room temperature, and ethanol was evaporated. To the residue was added 6N hydrochloric acid (3.24 mL), and the mixture was stirred for 1 hr. The precipitated crystals were collected by filtration to give 2-methyl-cyclopent-1-enecarboxylic acid (625 mg).
¹H-NMR(CDCl₃)δ: 2.67-2.61(m,2H), 2.54-2.50(m,2H), 2.13(s,3H), 1.88-1.80(m,2H).

### Step 3

To a solution of 2-methyl-cyclopent-1-enecarboxylic acid (625 mg) obtained in step 2 in N,N-dimethylformamide (10 mL) were added 1-ethyl-3-(3-dimethylaminopropyl)carbodiimide hydrochloride (1.23 g) and 1-hydroxybenzotriazole hydrate (985 mg). After stirring at room temperature for 2 hr, methylamine hydrochloride (525 mg) and triethylamine (1.79 mL) were added. After further stirring at room temperature for 3 hr, water (30 mL) was added, and the mixture was extracted with ethyl acetate. The organic layer was washed with water, dried and concentrated to give 2,N,N-trimethyl-cyclopent-1-enecarboxamide (777 mg).
¹H-NMR(CDCl₃)δ: 3.00(s,3H), 2.97(s,3H), 2.62-2.55(m,2H), 2.39-2.34(m,2H), 1.95-1.85(m,2H), 1.68(s,3H).

### Step 4

To a solution of 2,N,N-trimethyl-cyclopent-1-enecarboxamide (236 mg) obtained in step 3 in tetrahydrofuran (3.6 mL) was added dropwise lithium diisopropylamide (2.0 M heptane-tetrahydrofuran-ethylbenzene solution; 1.05 mL) under nitrogen at -78°C. After stirring at the same temperature for 1 hr, a solution of 5-fluoro-2-trifluoromethyl-benzonitrile (251 mg) in tetrahydrofuran (2.4 mL) was added dropwise. The cooling bath was removed and, after stirring for 2 hr, 3N hydrochloric acid (5 mL) was added, and the mixture was extracted with ethyl acetate. The organic layer was washed with water, dried, concentrated and purified by silica gel thin layer chromatography (chloroform :methanol=20:1) to give 3-(5-fluoro-2-trifluoromethyl-phenyl)-6,7-dihydro-5H-cyclopenta[c]pyridin-1-ol (106 mg).
¹H-NMR(CDCl₃)δ: 7.79(dd,1H,J=8.8,5.3Hz), 7.29(d,1H,J=8.8Hz), 7.19(dd,1H,J=8.8,2.5Hz), 6.25(s,1H), 2.92-2.84(m,4H), 2.17-2.09(m,2H).

### Step 5

To a solution of 3-(5-fluoro-2-trifluoromethylphenyl)-6,7-dihydro-5H-cyclopenta[c]pyridin-1-ol (106 mg) obtained in step 4 in methylene chloride (1 mL) was added phosphorus oxybromide (204 mg). After stirring at room temperature for 1 hr, and the mixture was stirred at 100°C for 2.5 hr. Phosphorus oxybromide (204 mg) was added, and after stirring at 100°C for 5 hr, the mixture was allowed to return to room temperature, and water was added by small portions. The mixture was extracted with ethyl acetate, and the organic layer was washed with water, dried, concentrated and purified by silica gel thin layer chromatography (ethyl acetate:hexane=1:9) to give 1-bromo-3-(5-fluoro-2-trifluoromethyl-phenyl)-6,7-dihydro-5H-cyclopenta[c]pyridine (80 mg).
¹H-NMR(CDCl₃)δ: 7.74(dd,1H,J=8.6,5.3Hz), 7.25(s,1H), 7.22-7.16(m,2H), 3.10-3.00(m,4H), 2.24-2.16(m,2H).

### Step 6

1-Bromo-3-(5-fluoro-2-trifluoromethyl-phenyl)-6,7-dihydro-5H-cyclopenta[c]pyridine (80 mg) obtained in step 5 was reacted in the same manner as in Example 1066-1, step 6, and purified by silica gel column chromatography (ethyl acetate:hexane=1:9) to give 1-(3,5-dimethoxy-phenyl)-3-(5-fluoro-2-trifluoromethylphenyl)-6,7-dihydro-5H-cyclopenta[c]pyridine (92 mg).
¹H-NMR(CDCl₃)δ: 7.77(dd,1H,J=8.8,5.5Hz), 7.29(s,1H), 7.25(dd,1H,J=9.0,2.6Hz), 7.18(td,1H,J=8.4,2.6Hz), 6.99(d,2H,J=2.3Hz), 6.50(t,1H,J=2.3Hz), 3.84(s,6H), 3.18(t,2H,J=7.4Hz), 3.04(t,2H,J=7.5Hz), 2.20-2.12(m,2H).

### Example 1093-2

### (S)-1-{3-[1-(3,5-dimethoxy-phenyl)-6,7-dihydro-5H-cyclopenta[c]pyridin-3-yl]-4-trifluoromethyl-phenyl}-pyrrolidin-3-ol

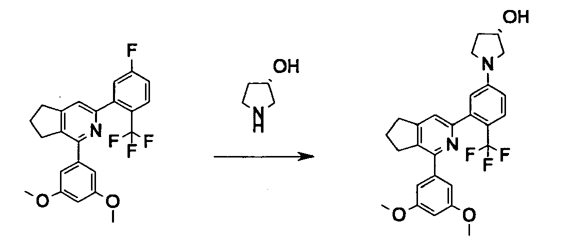

1-(3,5-Dimethoxy-phenyl)-3-(5-fluoro-2-trifluoromethyl-phenyl)-6,7-dihydro-5H-cyclopenta[c]pyridine (92 mg) obtained in Example 1093-1 and (S)-3-hydroxy-pyrrolidine (29 mg) were reacted in the same manner as in Example 1-23, step 1, purified by silica gel thin layer chromatography (hexane:diethyl ether=9:1) and crystallized (ethanol-water) to give (S)-1-{3-[1-(3,5-dimethoxy-phenyl)-6,7-dihydro-5H-cyclopenta[c]pyridin-3-yl]-4-trifluoromethyl-phenyl}-pyrrolidin-3-ol (47 mg).
¹H-NMR(DMSO-d₆)δ: 7.56(d,1H,J=8.7Hz), 7.38(s,1H), 6.99(d,2H,J=2.6Hz), 6.65(dd,1H,J=8.7,2.3Hz), 6.55(t,1H,J=2.6Hz), 6.53(d,1H,J=2.3Hz), 5.00(d,1H,J=3.4Hz), 4.41(brs,1H), 3.78(s,6H), 3.49-3.37(m,3H), 3.18-3.12(m,3H), 2.99(t,2H,J=7.3Hz), 2.12-1.99(m,3H), 1.96-1.87(m,1H).

### Example 1094-1

### (S)-1-{3-[4-(3,5-dimethoxy-phenyl)-6,7-dihydro-5H-cyclopentapyrimidin-2-yl]-4-trifluoromethyl-phenyl}-pyrrolidin-3-yl acetate

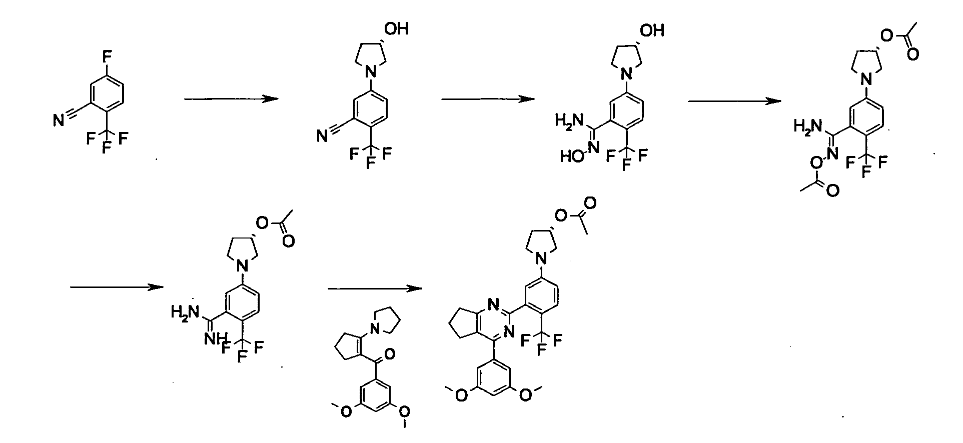

### Step 1

To a solution of 5-fluoro-2-trifluoromethylbenzonitrile (1.92 g) in N,N-dimethylacetamide (5.2 mL) were added (S)-3-hydroxy-pyrrolidine (0.74 mL) and potassium carbonate (2.52 g). After stirring at 110°C for 2 hr, water and 6N hydrochloric acid were added to acidify the mixture, and the mixture was stirred at room temperature for 1 hr. The precipitated crystals were collected by filtration to give 5-((S)-3-hydroxy-pyrrolidin-1-yl)-2-trifluoromethyl-benzonitrile (2.13 g).
¹H-NMR(DMSO-d₆)δ: 7.66(d,1H,J=9.0Hz), 7.15(d,1H,J=2.6Hz), 6.86(dd,1H,J=9.0,2.3Hz), 5.08(d,1H,J=3.7Hz), 4.43(brs,1H), 3.52-3.37(m,3H), 3.20(d,1H,J=10.9Hz), 2.11-2.00(m,2H), 1.98-1.89(m,2H).

### Step 2

To a suspension of 5-((S)-3-hydroxy-pyrrolidin-1-yl)-2-trifluoromethyl-benzonitrile (2.13 g) obtained in step 1 in ethanol (21 mL) were added triethylamine (4.63 mL) and hydroxylamine hydrochloride (1.74 g). After stirring at 100°C for 8 hr, the reaction mixture was concentrated. To the residue was added ethyl acetate (50 mL), and the insoluble material was filtered off. Water was added, and the mixture was separated into two layers. The aqueous layer was extracted with ethyl acetate, and the organic layer was washed with water, dried and concentrated. Ethyl acetate was added again and the insoluble material was filtered off. The filtrate was concentrated to give N-hydroxy-5-((S)-3-hydroxy-pyrrolidin-1-yl)-2-trifluoromethyl-benzamidine (1.59 g).
¹H-NMR(DMSO-d₆)δ: 9.42(s,1H), 7.45(d,1H,J=8.8Hz), 6.60(dd,1H,J=8.8,2.6Hz), 6.51(d,1H,J=2.6Hz), 5.70(brs,2H), 5.01(d,1H,J=3.5Hz), 4.41(brs,1H), 3.46-3.33(m,3H), 3.13(d,1H,J=10.4Hz), 2.09-1.99(m,1H), 1.96-1.88(m,1H).

### Step 3

To a solution of N-hydroxy-5-((S)-3-hydroxy-pyrrolidin-1-yl)-2-trifluoromethyl-benzamidine (1.59 g) obtained in step 2 in acetonitrile (16 mL) were added triethylamine (4.60 mL) and acetyl chloride (1.56 mL) under ice-cooling. After stirring at room temperature for 2.5 days, the insoluble material was filtered off. The filtrate was concentrated, water was added to the residue, and the mixture was extracted with ethyl acetate. The organic layer was washed with water, dried, concentrated and purified by silica gel column chromatography (chloroform-chloroform:methanol=20:1, hexane-ethyl acetate:hexane=1:1) and silica gel thin layer chromatography (ethyl acetate:hexane=2:1) to give N-acetoxy-5-((S)-3-acetoxy-pyrrolidin-1-yl)-2-trifluoromethyl-benzamidine (311 mg).
¹H-NMR(DMSO-d₆)δ: 7.52(d,1H,J=8.8Hz), 6.81(brs,2H), 6.72(dd,1H,J=8.8,2.1Hz), 6.57(d,1H,J=2.1Hz), 5.37(brs,1H), 3.64(dd,1H,J=11.8,4.5Hz), 3.52-3.34(m,3H), 2.34-1.92(m,8H).

### Step 4

To a solution of N-acetoxy-5-((S)-3-acetoxy-pyrrolidin-1-yl)-2-trifluoromethyl-benzamidine (311 mg) obtained in step 3 in methanol (3 mL) was added 10% palladium carbon (31 mg), and the mixture was stirred at ordinary hydrogen pressure at room temperature for 13 hrs. The insoluble material was filtered off, and the filtrate was concentrated to give (S)-1-(3-carbamidoyl-4-trifluoromethyl-phenyl)-pyrrolidin-3-yl acetate (251 mg).
¹H-NMR(DMSO-d₆)δ: 7.58(d,1H,J=8.8Hz), 6.77(dd,1H,J=8.8,2.1Hz), 6.70(d,1H,J=2.1Hz), 5.38(brs,1H), 3.64(dd,1H,J=12.0,4.5Hz), 3.53-3.34(m,3H), 2.32-2.21(m,1H), 2.18-2.08(m,1H), 1.74(s,3H). Step 5

To a solution of (S)-1-(3-carbamidoyl-4-trifluoromethyl-phenyl)-pyrrolidin-3-yl acetate (251 mg) obtained in step 4 in pyridine (2.5 mL) was added the intermediate of Example 1-8, step 1, (3,5-dimethoxy-phenyl)-(2-pyrrolidin-1-yl-cyclopent-1-enyl)-methanone (690 mg). After stirring at 140°C for 48 hr, the reaction mixture was concentrated. The residue was purified by silica gel column chromatography (hexane-ethyl acetate:hexane=1:1) and silica gel thin layer chromatography (ethyl acetate:hexane=1:1) to give (S)-1-{3-[4-(3,5-dimethoxy-phenyl)-6,7-dihydro-5H-cyclopentapyrimidin-2-yl]-4-trifluoromethyl-phenyl}-pyrrolidin-3-yl acetate (65 mg).
¹H-NMR(CDCl₃)δ: 7.62(d,1H,J=8.8Hz), 7.20(d,2H,J=2.3Hz), 6.83(d,1H,J=2.6Hz), 6.60(dd,1H,J=8.8,2.6Hz), 6.57(t,1H,J=2.3Hz), 5.42(s,1H), 3.85(s,6H), 3.69(dd,1H,J=11.4,4.5Hz), 3.53-3.43(m,3H), 3.28(t,2H,J=7.5Hz), 3.15(t,2H,J=7.5Hz), 2.28-2.17(m,4H), 2.04(s,3H).

### Example 1094-2

### (S)-1-{3-[4-(3,5-dimethoxy-phenyl)-6,7-dihydro-5H-cyclopentapyrimidin-2-yl]-4-trifluoromethyl-phenyl}-pyrrolidin-3-ol

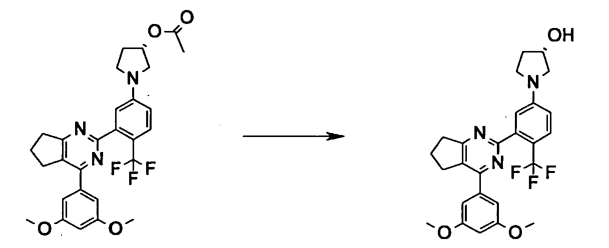

To a solution of (S)-1-{3-[4-(3,5-dimethoxy-phenyl)-6,7-dihydro-5H-cyclopentapyrimidin-2-yl]-4-trifluoromethyl-phenyl}-pyrrolidin-3-yl acetate (65 mg) obtained in Example 1094-1 in methanol (1.4 mL) was added a 2N aqueous sodium hydroxide solution (0.123 mL). After stirring at room temperature for 30 min, the reaction mixture was concentrated, and purified by silica gel thin layer chromatography (ethyl acetate:hexane=4:1) to give (S)-1-{3-[4-(3,5-dimethoxy-phenyl)-6,7-dihydro-5H-cyclopentapyrimidin-2-yl]-4-trifluoromethyl-phenyl}-pyrrolidin-3-ol (45 mg).
¹H-NMR(DMSO-d₆)δ: 7.59(d,1H,J=9.0Hz), 7.15(d,2H,J=2.3Hz), 6.77(d,1H,J=2.6Hz), 6.70(dd,1H,J=9.0,2.6Hz), 6.66(t,1H,J=2.3Hz), 5.00(d,1H,J=3.8Hz), 4.42(s,1H), 3.80(s,6H), 3.51-3.36(m,3H), 3.27(t,2H,J=7.0Hz), 3.17(d,1H,J=10.2Hz), 3.05(t,2H,J=7.0Hz), 2.18-2.00(m,3H), 1.97-1.88(m,1H).

### Example 1095-1

### (S)-1-{3-[7-(3,5-dimethoxy-phenyl)-indan-5-yl]-4-trifluoromethyl-phenyl}-pyrrolidin-3-ol

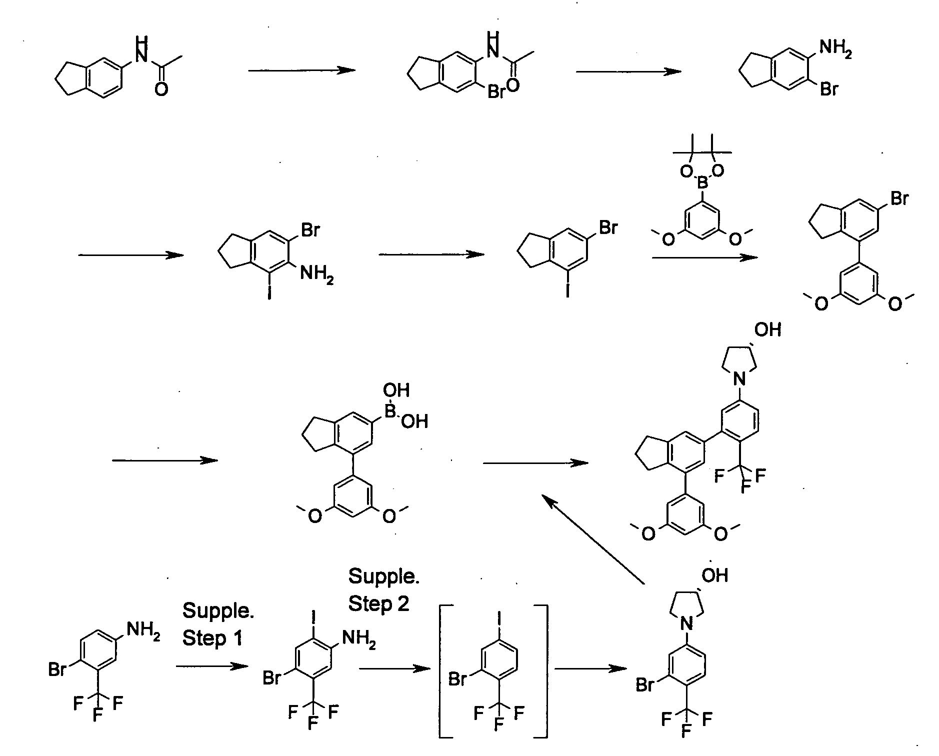

### Step 1

To a solution of N-indan-5-yl-acetamide (12.19 g) in N,N-dimethylformamide (61 mL) was added N-bromosuccinimide (13.64 g) under ice-cooling. The cooling bath was removed, and after stirring for 1.5 hr, water (150 mL) was added, and precipitated crystals were collected by filtration to give N-(6-bromo-indan-5-yl)-acetamide (16.54 g).
¹H-NMR(CDCl₃)δ: 8.13(s,1H), 7.51(brs, 1H), 7.37(s,1H), 2.88(dd,2H,J=7.4,7.4Hz), 2.86(dd,2H,J=7.4,7.4Hz), 2.22(s,3H), 2.07(ddd,2H,J=14.8,7.4,7.4Hz). Step 2

To a solution of N-(6-bromo-indan-5-yl)-acetamide (1.74 g) obtained in step 1 in acetic acid (8.7 mL) was added 6N hydrochloric acid (8.7 mL). After stirring at 100°C for 2 hr, water (10 mL) was added, and the precipitated crystals were collected by filtration. To the obtained crystals was added 1 M aqueous potassium carbonate solution-ethyl acetate, and the mixture was separated into layers. The organic layer was washed with an aqueous sodium hydrogencarbonate solution, dried and concentrated to give 6-bromo-indan-5-ylamine (848 mg).
¹H-NMR(CDCl₃)δ: 7.25(s,1H), 6.67(s,1H), 4.20-3.60(m,1H), 2.79(dd,2H,J=7.4,7.4Hz), 2.78(dd,2H,J=7.4,7.4Hz), 2.03(ddd,2H,J=14.8,7.4,7.4Hz). Step 3

To a solution of 6-bromo-indan-5-ylamine (623 mg) obtained in step 2 in chloroform (6 mL)-methanol (6 mL) were added calcium carbonate (0.44 g) and benzyltrimethylammonium dichloroiodate (1.07 g) under ice-cooling. The cooling bath was removed, and after stirring for 1 hr, the insoluble material was filtered off, and the filtrate was concentrated. Water-ethyl acetate was added to the residue to separate the mixture into layers. The organic layer was washed with a 10% aqueous sodium sulfite solution and brine, dried, concentrated and purified by silica gel column chromatography (ethyl acetate:hexane=1:20) to give 6-bromo-4-iodo-indan-5-ylamine (895 mg).
¹H-NMR(CDCl₃)δ: 7.22(s,1H), 4.47(brs,2H), 2.99(dd,2H,J=7.3,7.3Hz), 2.83(dd,2H,J=7.3,7.3Hz), 2.06(ddd,2H,J=14.7,7.3,7.3Hz). Step 4

To a solution of 6-bromo-4-iodo-indan-5-ylamine (130 mg) obtained in step 3 in acetic acid (1 mL)-1N hydrochloric acid (1 mL) was added dropwise a solution of sodium nitrite (29 mg) in water (0.5 mL) under ice-cooling. After stirring at the same temperature for 45 min, a 50% aqueous phosphorous acid solution (0.5 mL) was added, and the mixture was stirred at room temperature for 40 min and at 50°C for 30 min. Water was added to the reaction mixture, and the mixture was extracted with ethyl acetate. The organic layer was washed with water, dried and concentrated to give 6-bromo-4-iodo-indane (123 mg).
¹H-NMR(CDCl₃)δ: 7.65(s,1H), 7.30(s,1H), 3.05(dd,2H,J=7.3,7.3Hz), 2.84(dd,2H,J=7.3,7.3Hz), 2.07(ddd,2H,J=14.7,7.3,7.3Hz).

### Step 5

6-Bromo-4-iodo-indane (433 mg) produced by the method of step 4 and 2-(3,5-dimethoxyphenyl)-4,4,5,5-tetramethyl-[1,3,2]dioxaborolane (354 mg) were reacted in the same manner as in Example 1066-1, step 6, and purified by silica gel column chromatography (ethyl acetate:hexane=1:10) to give 6-bromo-4-(3,5-dimethoxy-phenyl)-indane (361 mg).
¹H-NMR(CDCl₃)δ: 7.35(s,1H), 7.32(s,1H), 6.53(d,2H,J=2.2Hz), 6.46(t,1H,J=2.2Hz), 3.82(s,6H), 2.95(dd,4H,J=7.3,7.3Hz), 2.90(dd,4H,J=7.3,7.3Hz), 2.04(ddd,2H,J=14.7,7.3,7.3Hz).

### Step 6

To a solution of 6-bromo-4-(3,5-dimethoxy-phenyl)-indane (353 mg) obtained in step 5 in tetrahydrofuran (5 mL) was added dropwise n-butyllithium (1.56 M hexane solution; 0.747 mL) under argon and at -78°C. After stirring at the same temperature for 40 min, triisopropyl borate (0.367 mL) was added dropwise. The cooling bath was removed, and the mixture was stirred for 30 min. 2N hydrochloric acid (20 mL) was added to the reaction mixture and, after stirring at room temperature for 30 min, the reaction mixture was extracted with ethyl acetate. The organic layer was washed with water, dried and concentrated. The obtained residue was crystallized from diethyl ether-hexane to give 7-(3,5-dimethoxy-phenyl)-indane-5-boronic acid (145 mg).
¹H-NMR(DMSO-d₆)δ: 7.72(s,1H), 7.65(s,1H), 6.57(d,2H,J=2.2Hz), 6.48(t,1H,J=2.2Hz), 3.75(s,6H), 2.94(dd,2H,J=6.6,6.6Hz), 2.92(dd,2H,J=6.6,6.6Hz), 1.98(ddd,2H,J=14.3,6.6,6.6Hz).

### Step 7

7-(3,5-Dimethoxy-phenyl)-indane-5-boronic acid (60 mg) obtained in step 6 and (S)-1-(3-bromo-4-trifluoromethyl-phenyl)-pyrrolidin-3-ol (62 mg) obtained in supplementary step 2 were reacted in the same manner as in Example 1085-1, step 1, and purified by silica gel column chromatography (ethyl acetate:hexane=1:5) and silica gel thin layer chromatography (chloroform:acetone=10:1) to give (S)-1-{3-[7-(3,5-dimethoxy-phenyl)-indan-5-yl]-4-trifluoromethyl-phenyl}-pyrrolidin-3-ol (33 mg).
¹H-NMR(CDCl₃)δ: 7.56(d,1H,J=8.8Hz), 7.21(s,2H), 6.63(d,2H,J=2.2Hz), 6.54(dd,1H,J=8.8,2.2Hz), 6.47(d,1H,J=2.2Hz), 6.44(t,1H,J=2.2Hz), 4.63(brs,1H), 3.82(s,6H), 3.59-3.48(m,2H), 3.42(ddd,1H,J=8.8,9.5,2.9Hz), 3.32(d,1H,J=11.0Hz), 3.04(dd,2H,J=8.1,8.1Hz), 3.01(dd,2H,J=8.1,8.1Hz), 2.26-2.04(m,4H), 1.59(brs,1H).

### Supplementary step 1

4-Bromo-3-trifluoromethyl-aniline (2.40 g) was reacted in the same manner as in Example 1095-1, step 3, and purified by silica gel column chromatography (ethyl acetate:hexane=1:4) to give 4-bromo-2-iodo-5-trifluoromethyl-aniline (1.44 g).
¹H-NMR(CDCl₃)δ: 7.93(d,1H,J=0.7Hz), 7.01(s,1H), 4.31(brs,2H).

### Supplementary step 2

4-Bromo-2-iodo-5-trifluoromethyl-aniline (1.44 g) obtained in supplementary step 1 was reacted in the same manner as in Example 1095-1, step 4 and Example 1-15 and purified by silica gel column chromatography (ethyl acetate:hexane=2:1) to give (S)-1-(3-bromo-4-trifluoromethyl-phenyl)-pyrrolidin-3-ol (201 mg).
¹H-NMR(CDCl₃)δ: 7.46(d,1H,J=8.8Hz), 6.81(d,1H,J=2.6Hz), 6.45(dd,1H,J=8.8,2.6Hz), 4.69-4.61(m,1H), 3.54(dd,1H,J=11.0,4.8Hz), 3.51(dd,1H,J=8.4,8.0Hz), 3.40(ddd,1H,J=9.0,8.4,3.5Hz), 3.29(brd,1H,J=11.0Hz), 2.26-2.08(m,2H), 1.61(brs,1H).

### Example 1095-2

### (S)-1-{3-[7-(3,5-dimethoxy-phenyl)-4-hydroxy-indan-5-yl]-4-trifluoromethyl-phenyl}-pyrrolidin-3-ol

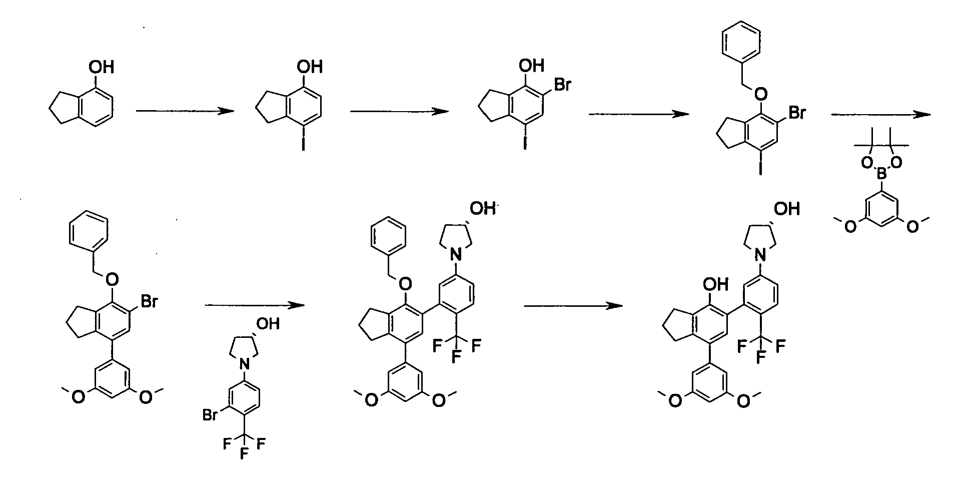

### Step 1

To a solution of indan-4-ol (1.34 g) in chloroform (10 mL)-methanol (10 mL) were added calcium carbonate (1.50 g) and ammonium dichloroiodate (3.48 g) under ice-cooling. After stirring at the same temperature for 4 hr, the insoluble material was filtered off, and the filtrate was concentrated. The residue was purified by silica gel column chromatography (ethyl acetate:hexane=1:6) to give 7-iodo-indan-4-ol (1.66 g).
¹H-NMR(CDCl₃)δ: 6.42(d,1H,J=8.4Hz), 7.37(d,1H,J=8.4Hz), 4.48(s,1H), 2.98(t,2H,J=7.5Hz), 2.89(t,2H,J=7.5Hz), 2.09(quintet,2H,J=7.5Hz).

### Step 2

To a solution of 7-iodo-indan-4-ol (1.66 g) obtained in step 1 in chloroform (15 mL)-methanol (15 mL) were added calcium carbonate (0.958 g) and benzyltrimethylammonium tribromide (2.49 g). After stirring at room temperature for 2 hr, the insoluble material was filtered off, and the filtrate was concentrated. To the obtained residue was added water-ethyl acetate to separate the mixture into layers. The organic layer was washed with a 10% aqueous citric acid solution and brine, dried, concentrated and purified by silica gel column chromatography (ethyl acetate:hexane=1:6) to give 5-bromo-7-iodo-indan-4-ol (1.48 g).
¹H-NMR(CDCl₃)δ: 7.60(s,1H), 5.42(s,1H), 3.07(t,2H,J=7.5Hz), 2.86(t,2H,J=7.2Hz), 2.11(m,2H).

### Step 3

To a solution of 5-bromo-7-iodo-indan-4-ol (1.48 g) obtained in step 2 in N,N-dimethylformamide (15 mL) were added potassium carbonate (1.21 g) and benzyl bromide (0.78 mL). After stirring at room temperature for 1 hr, water was added, and the mixture was extracted with ethyl acetate. The organic layer was washed with water, dried, concentrated and purified by silica gel column chromatography (ethyl acetate:hexane=1:30) to give 4-benzyloxy-5-bromo-7-iodo-indane (1.84 g).
¹H-NMR(CDCl₃)δ: 7.74(s,1H), 7.49(m,2H), 7.38(m,3H), 4.97(s,2H), 3.02(t,2H,J=7.5Hz), 2.84(t,2H,J=7.5Hz), 2.05(m,2H).

### Step 4

4-Benzyloxy-5-bromo-7-iodo-indane (1.84 g) obtained in step 3 and 2-(3,5-dimethoxy-phenyl)-4,4,5,5-tetramethyl-[1,3,2]dioxaborolane (1.13 g) were reacted in the same manner as in Example 1066-1, step 6, and purified by silica gel column chromatography (ethyl acetate:hexane=1:10) to give a mixture (3:1; 1.20 g) of 4-benzyloxy-5-bromo-7-(3,5-dimethoxy-phenyl)-indane and 2-(3,5-dimethoxy-phenyl)-4,4,5,5-tetramethyl-[1,3,2]dioxaborolane.

### Step 5

To a solution of a mixture (3:1; 1.19 g) of 4-benzyloxy-5-bromo-7-(3,5-dimethoxy-phenyl)-indane obtained in step 4 and 2-(3,5-dimethoxy-phenyl)-4,4,5,5-tetramethyl-[1,3,2]dioxaborolane in tetrahydrofuran (12 mL) was added dropwise n-butyllithium (1.56 M hexane solution; 1.39 mL) at -78°C under argon. After stirring at the same temperature for 40 min, trimethyl borate (0.456 mL) was added dropwise. After completion of the dropwise addition, the mixture was stirred for 20 min, water was added under ice-cooling, and the mixture was extracted with ethyl acetate. The organic layer was washed with water, dried, concentrated, and purified by silica gel column chromatography (ethyl acetate:hexane=1:4-1:3) to afford a resultant product (333 mg). The resultant product (220 mg) and (S)-1-(3-bromo-4-trifluoromethyl-phenyl)-pyrrolidin-3-ol (130 mg) obtained in Example 1095-1, supplementary step 2, were reacted in the same manner as in Example 1085-1, step 1, and purified by silica gel column chromatography (ethyl acetate:hexane=1:1) and silica gel thin layer chromatography (chloroform: acetone=10:1) to give (S)-1-{3-[4-benzyloxy-7-(3,5-dimethoxy-phenyl)-indan-5-yl]-4-trifluoromethylphenyl}-pyrrolidin-3-ol (51 mg).
¹H-NMR(CDCl₃)δ: 7.61(dd,1H,J=1.8,6.3Hz), 7.23(m,4H), 7.08(m,2H), 6.62(d,2H,J=1.8Hz), 6.57(t,1H,J=6.3Hz), 6.55(m,1H), 6.44(t,1H,J=1.8Hz), 4.73(d,1H,J=8.4Hz), 4.69(d,1H,J=8.4Hz), 3.83(s,6H), 3.50(m,2H), 3.37(m,1H), 3.26(t,1H,J=8.7Hz), 3.03(m,4H), 2.11(m,4H).

### Step 6

To a solution of (S)-1-{3-[4-benzyloxy-7-(3,5-dimethoxy-phenyl)-indan-5-yl]-4-trifluoromethylphenyl}-pyrrolidin-3-ol (50 mg) obtained in step 5 in ethanol (5 mL) was added 5% palladium carbon (50 mg). After stirring at room temperature under ordinary hydrogen pressure for 5 hr, the insoluble material was filtered off, and the filtrate was concentrated to give (S)-1-{3-[7-(3,5-dimethoxy-phenyl)-4-hydroxy-indan-5-yl]-4-trifluoromethyl-phenyl}-pyrrolidin-3-ol (37 mg).
¹H-NMR(CDCl₃)δ: 7.64(d,1H,J=6.6Hz), 7.08(s,1H), 6.61(m,1H), 6.60(d,2H,J=1.8Hz), 6.51(t,1H,J=6.3Hz), 6.42(t,1H,J=1.8Hz), 4.72(d,1H,J=3.9Hz), 4.66(brs,1H), 3.81(s,6H), 3.54(m,2H), 3.43(m,1H), 3.33(t,1H,J=7.5Hz), 3.08(dt,2H,J=1.8,5.4Hz), 2.99(m,2H), 2.17(m,4H), 1.62(s,1H).

### Example 1096

The Example compound was obtained in the same manner as in a method selected from Examples 1095-1 and 1095-2.

The compounds of Examples 1090-1 to 1095-2 and the compound of Example 1096 obtained in the same manner are shown in the following Tables. In the tables, the left line shows Example numbers and the right line shows the structural formulas of the corresponding compounds.

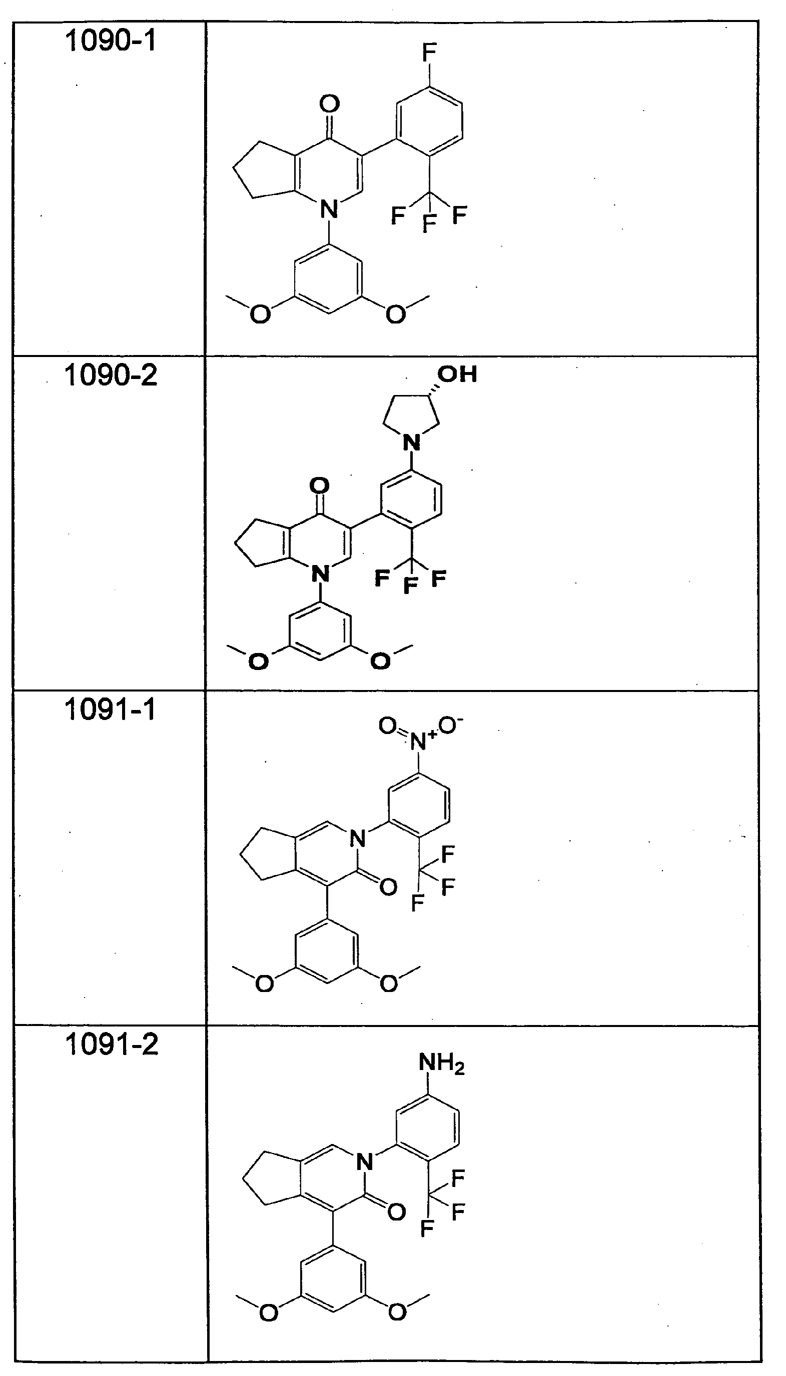

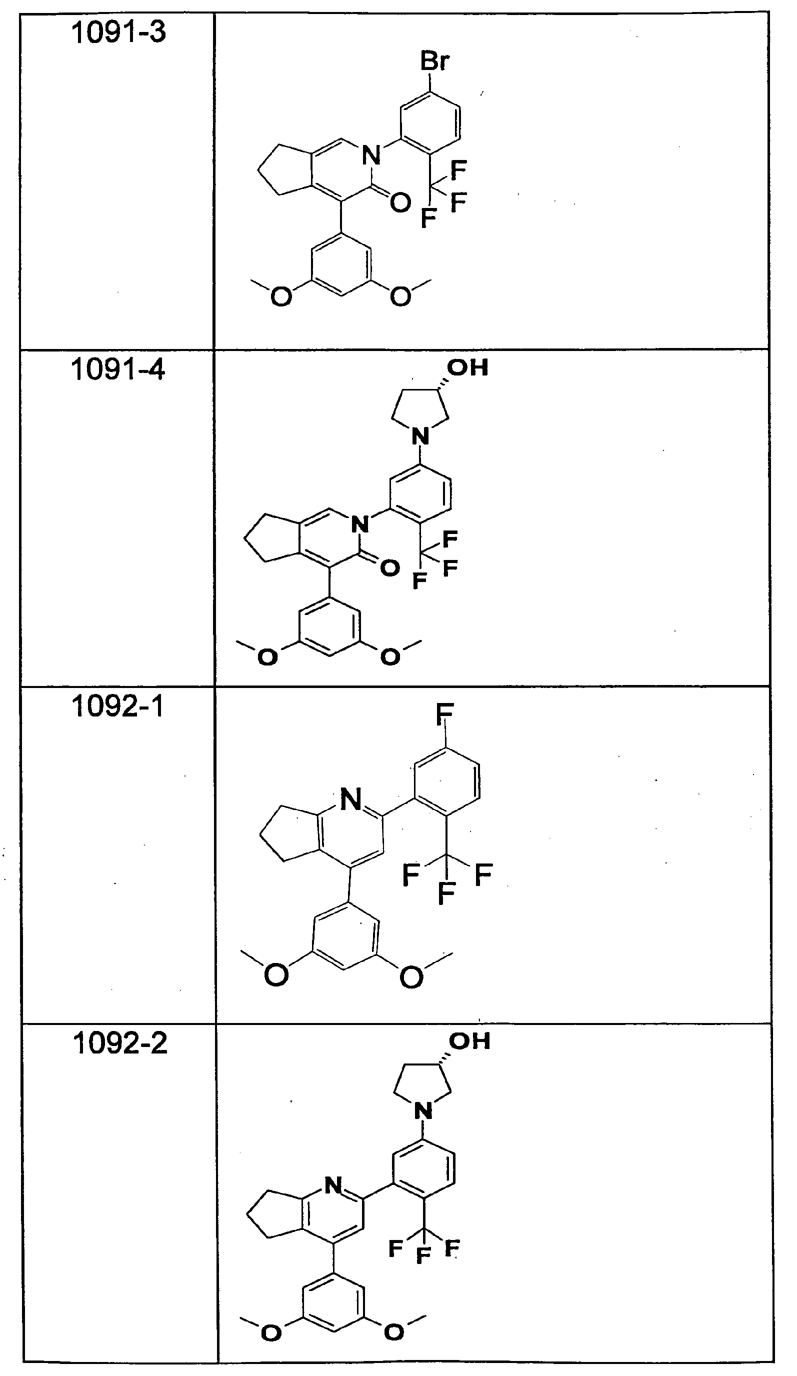

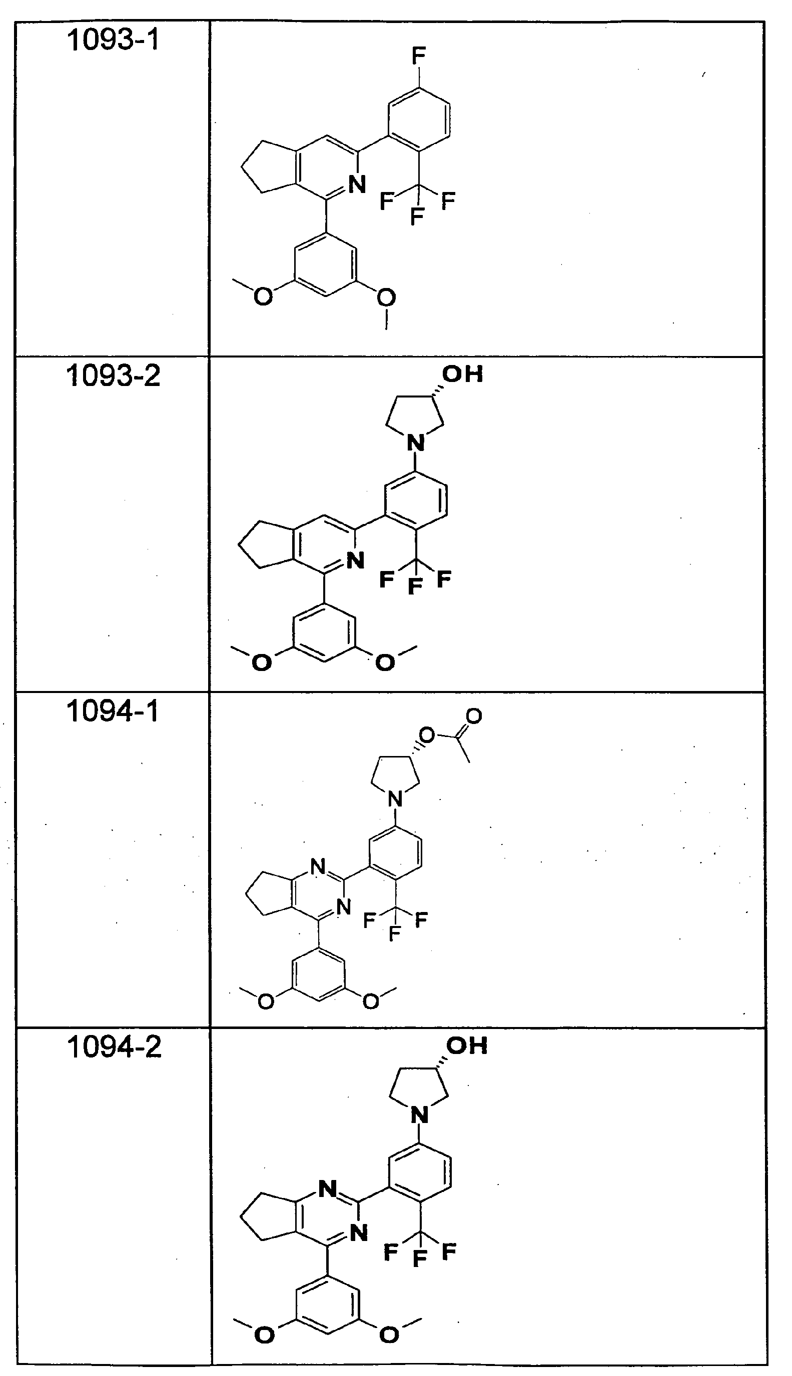

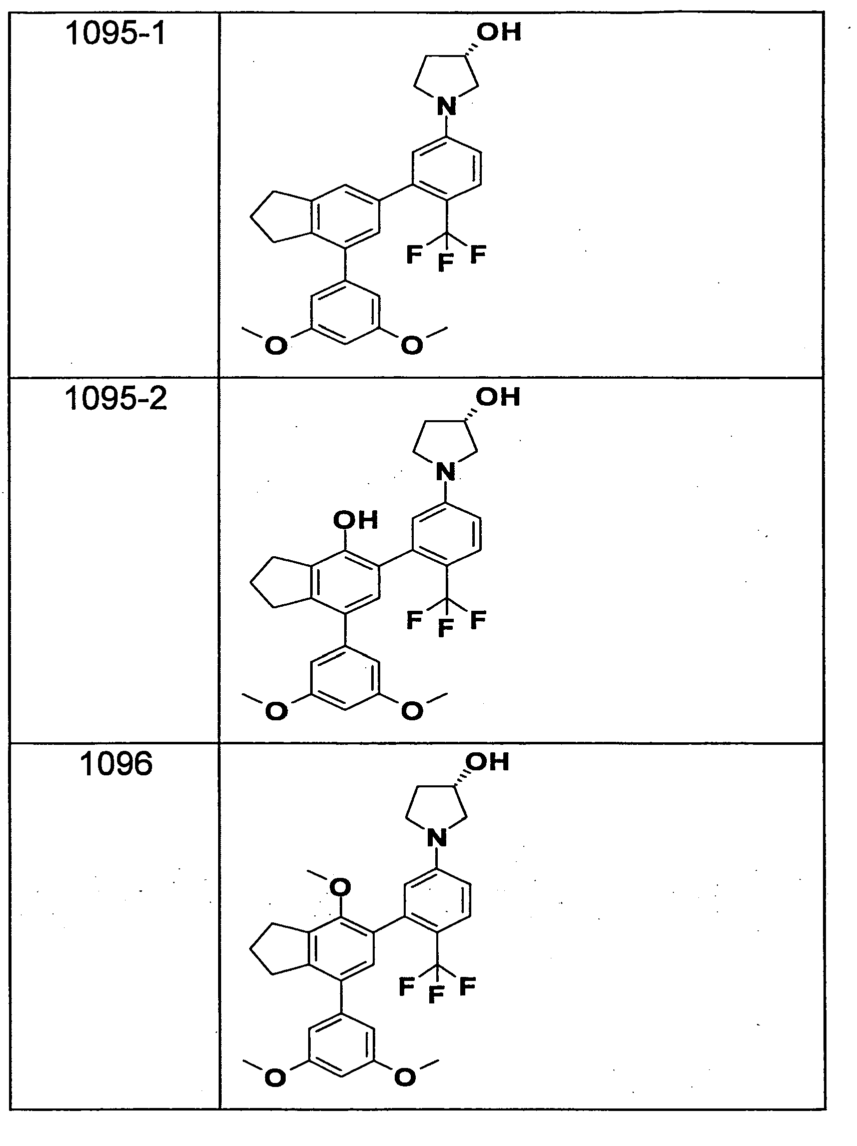

The NMR data of the Example compounds are collectively shown in the following. In the tables, the left line shows Example numbers and the right line shows the structural formulas of the corresponding compounds.

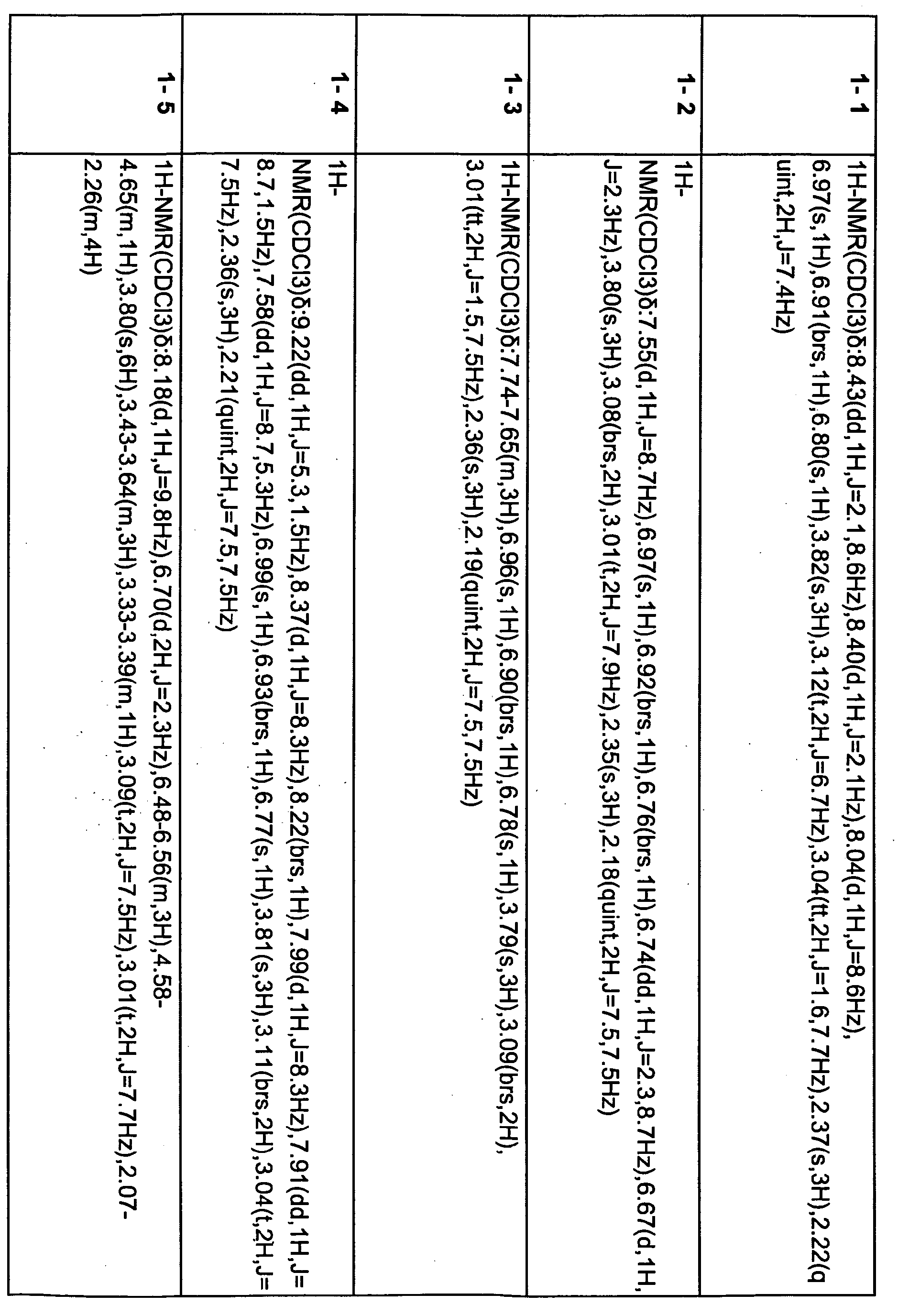

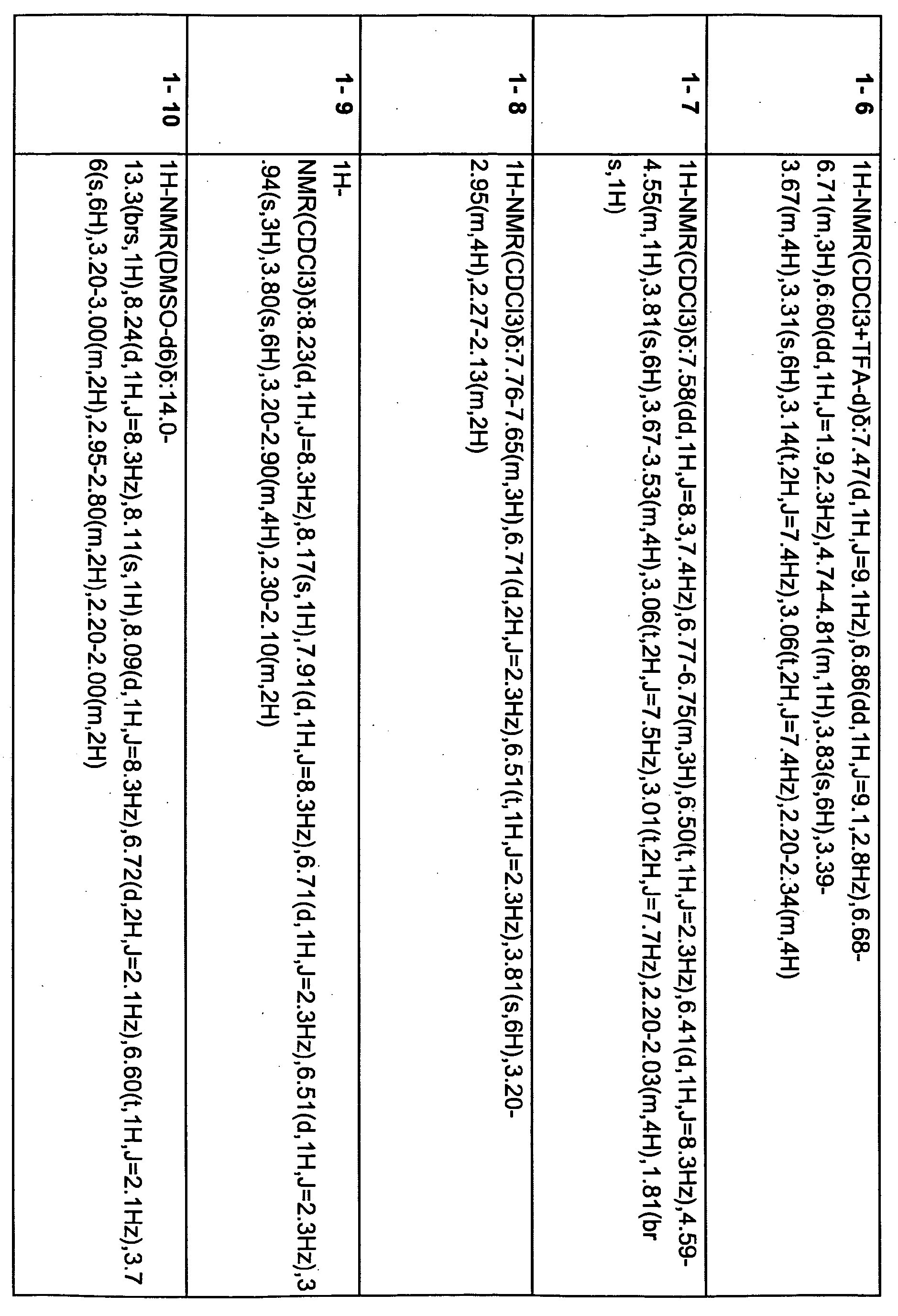

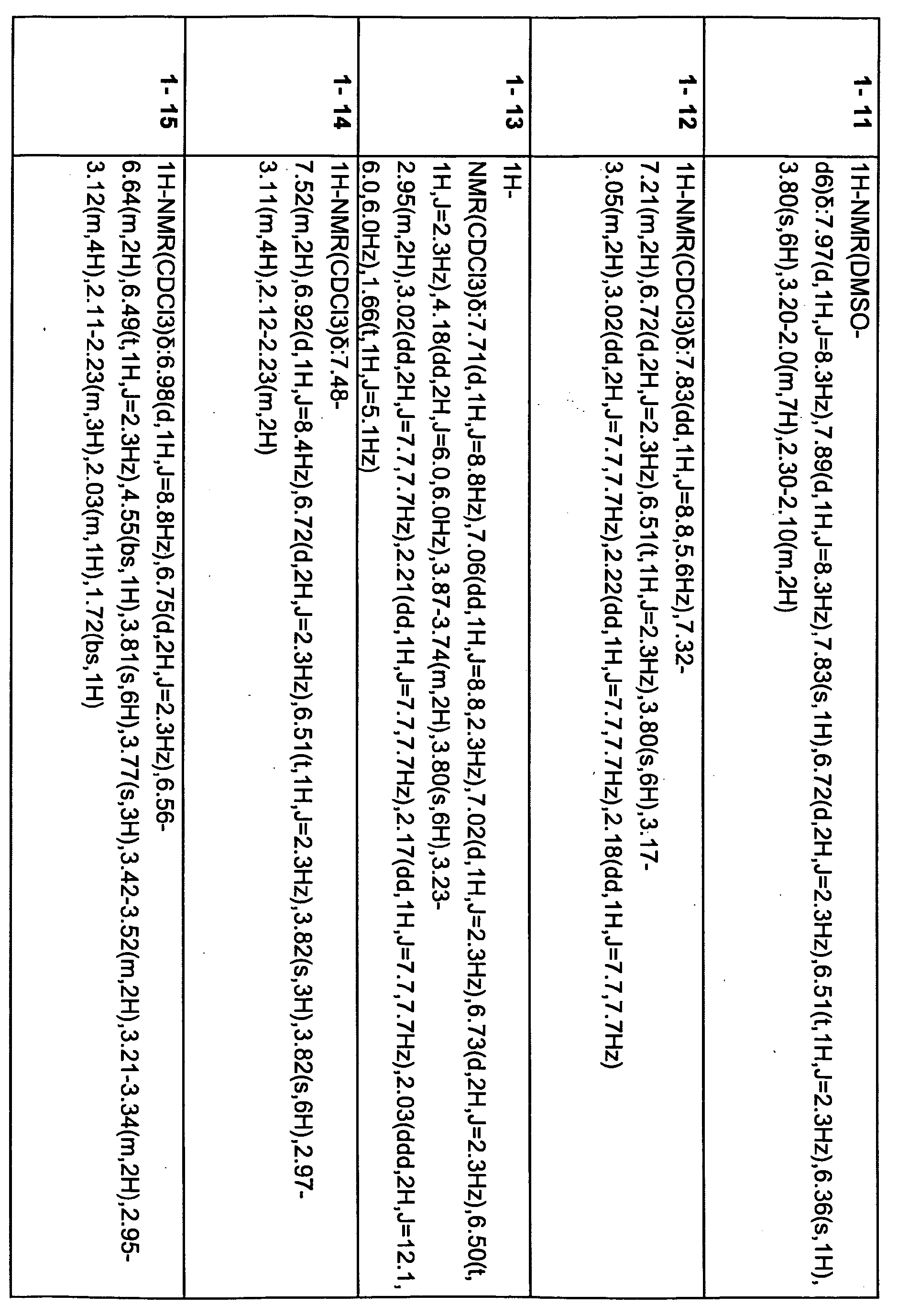

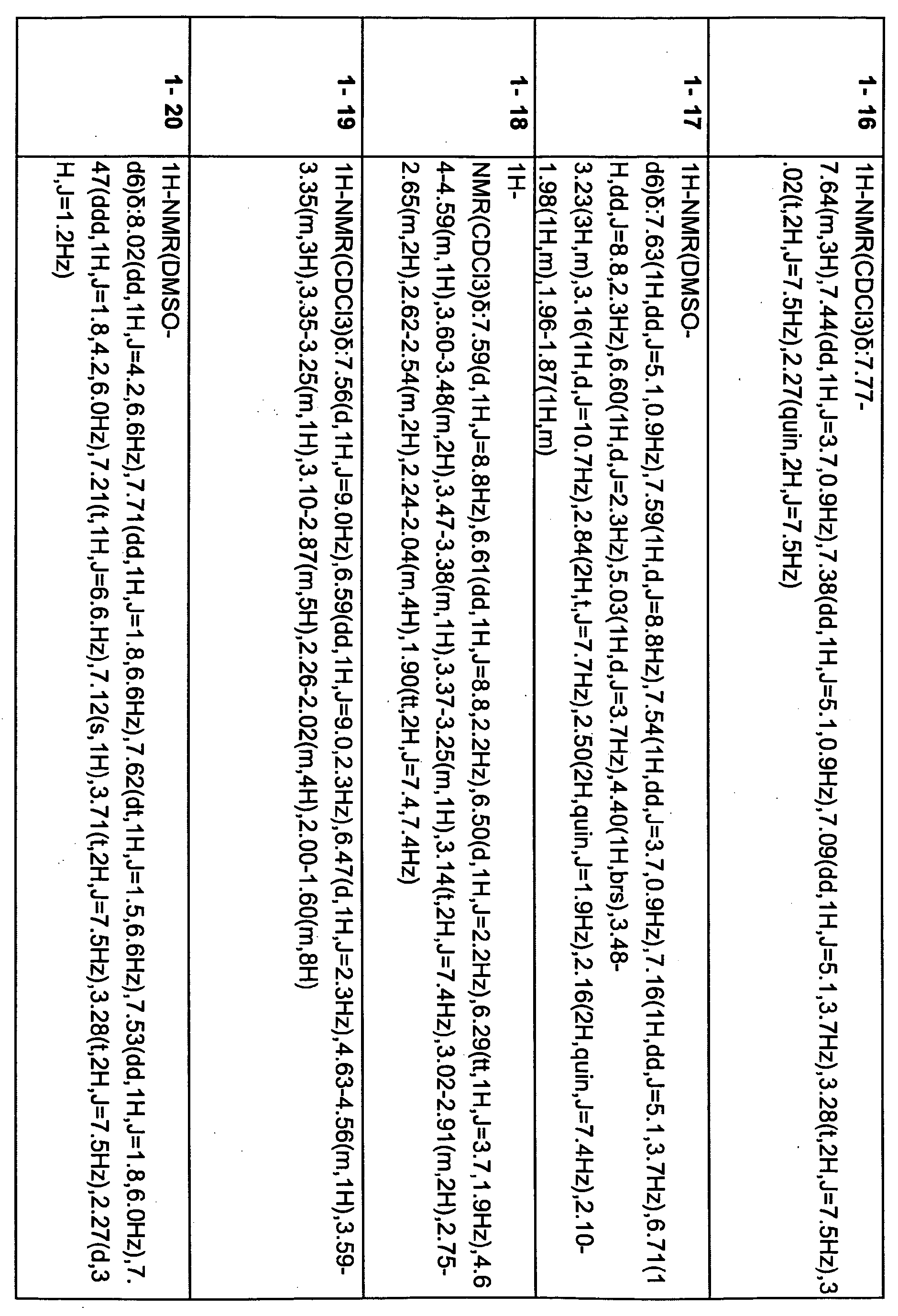

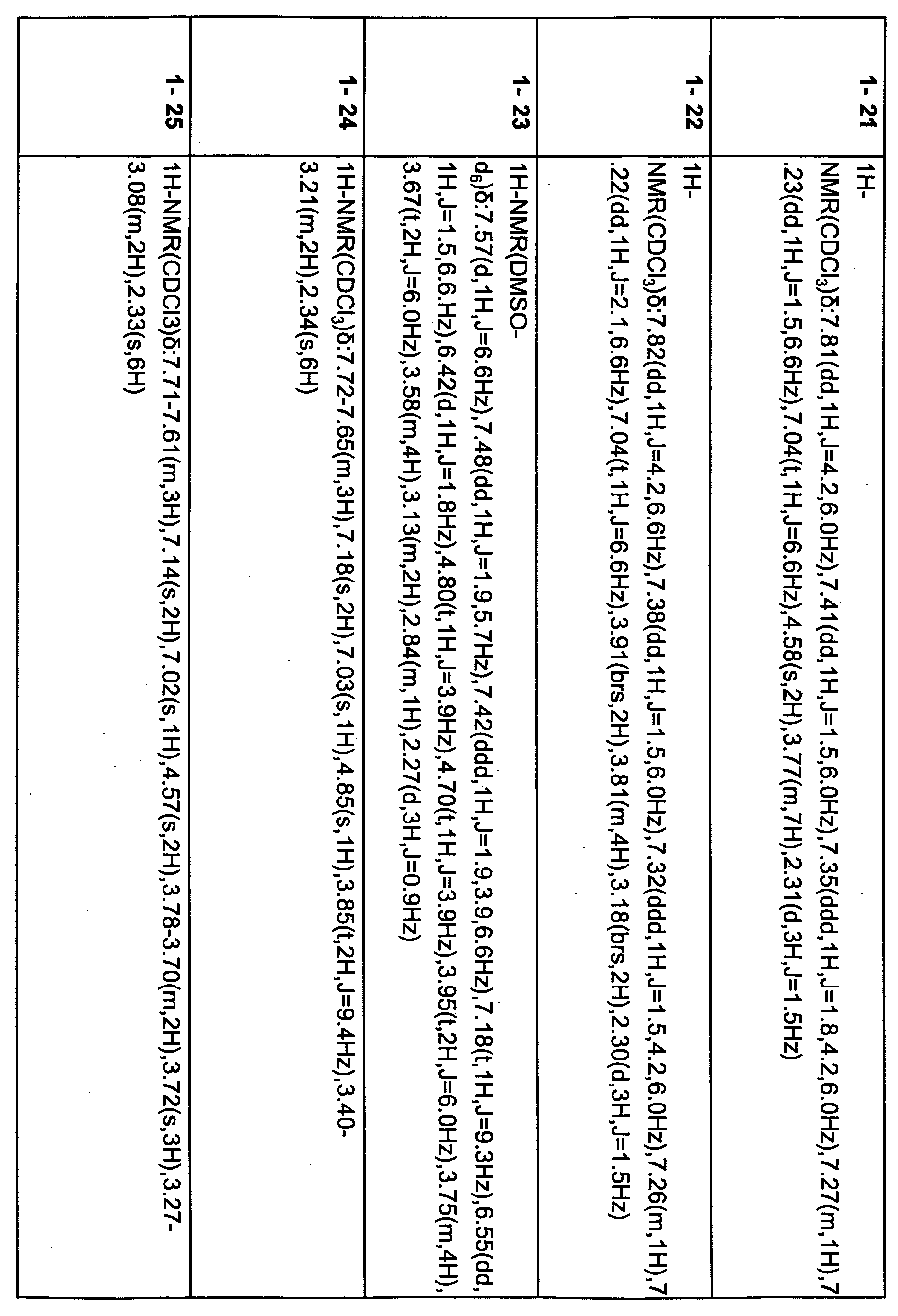

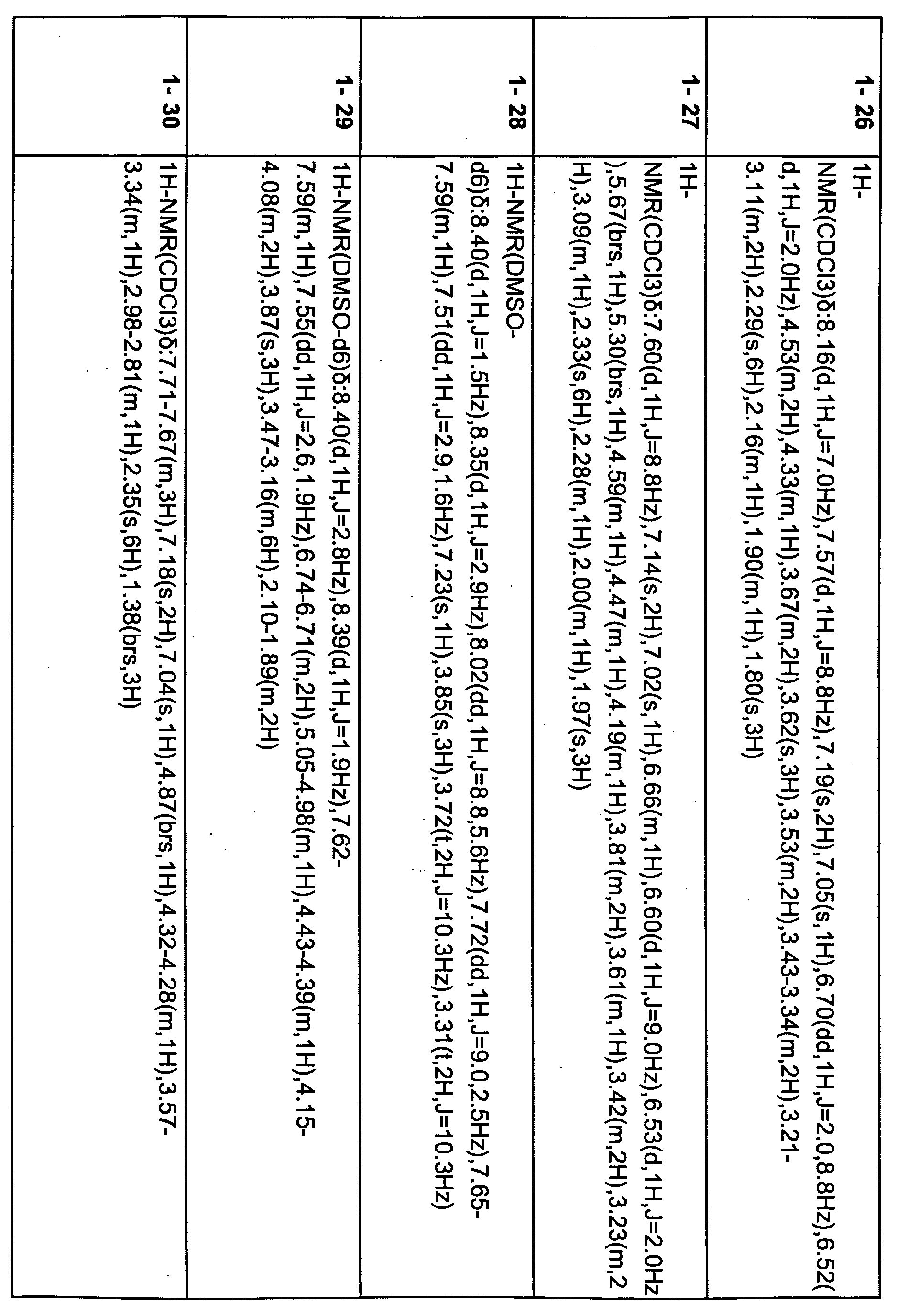

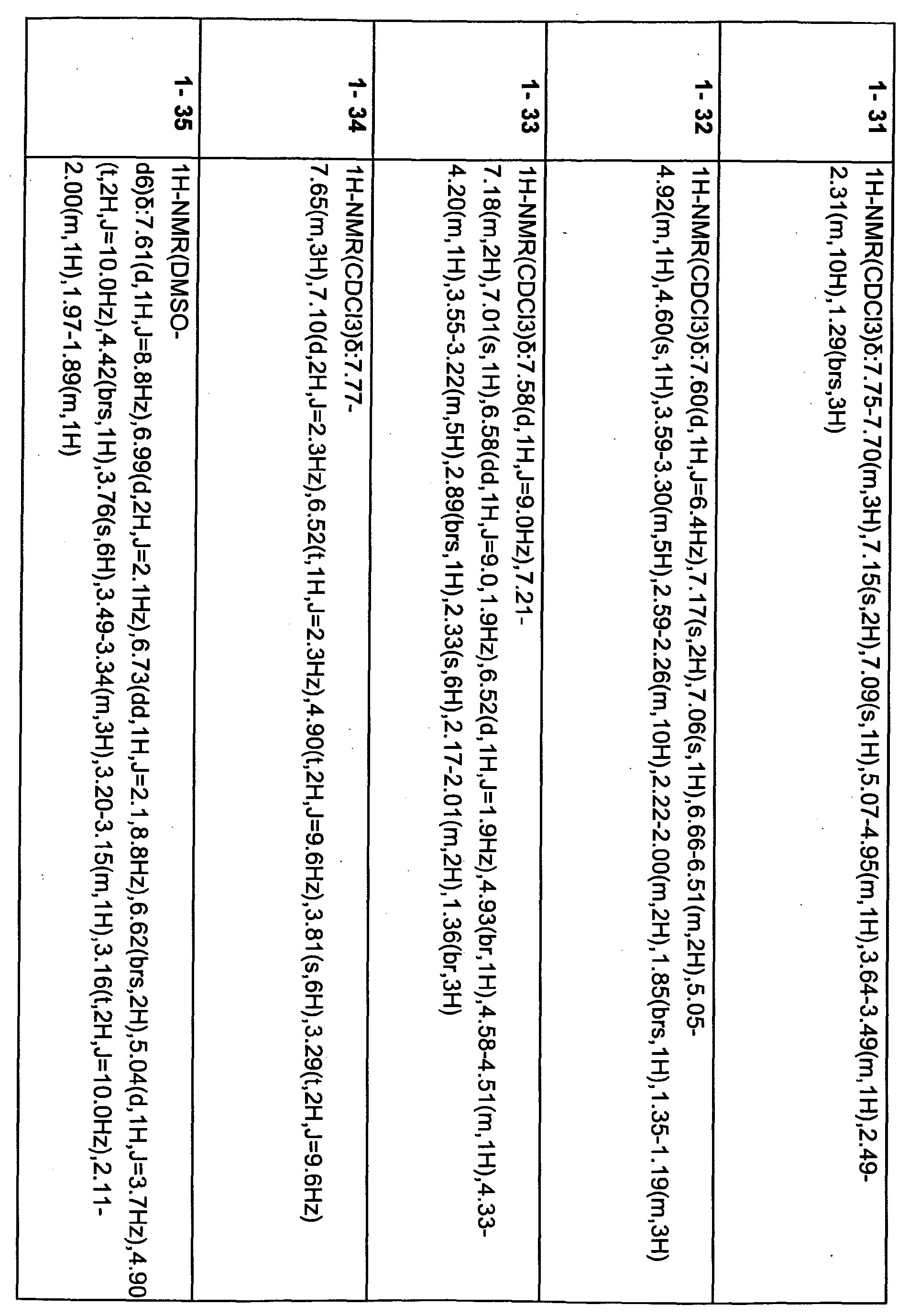

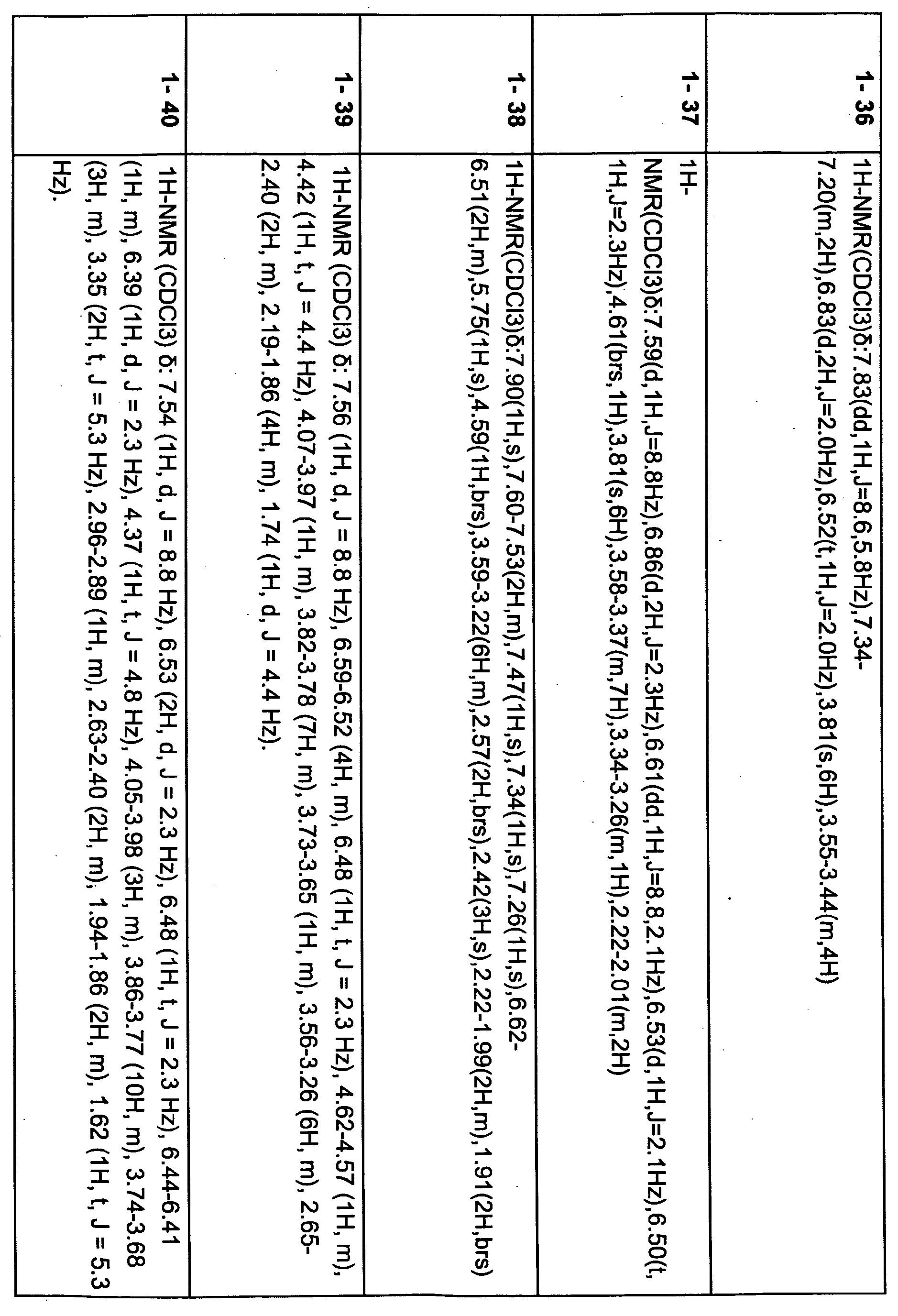

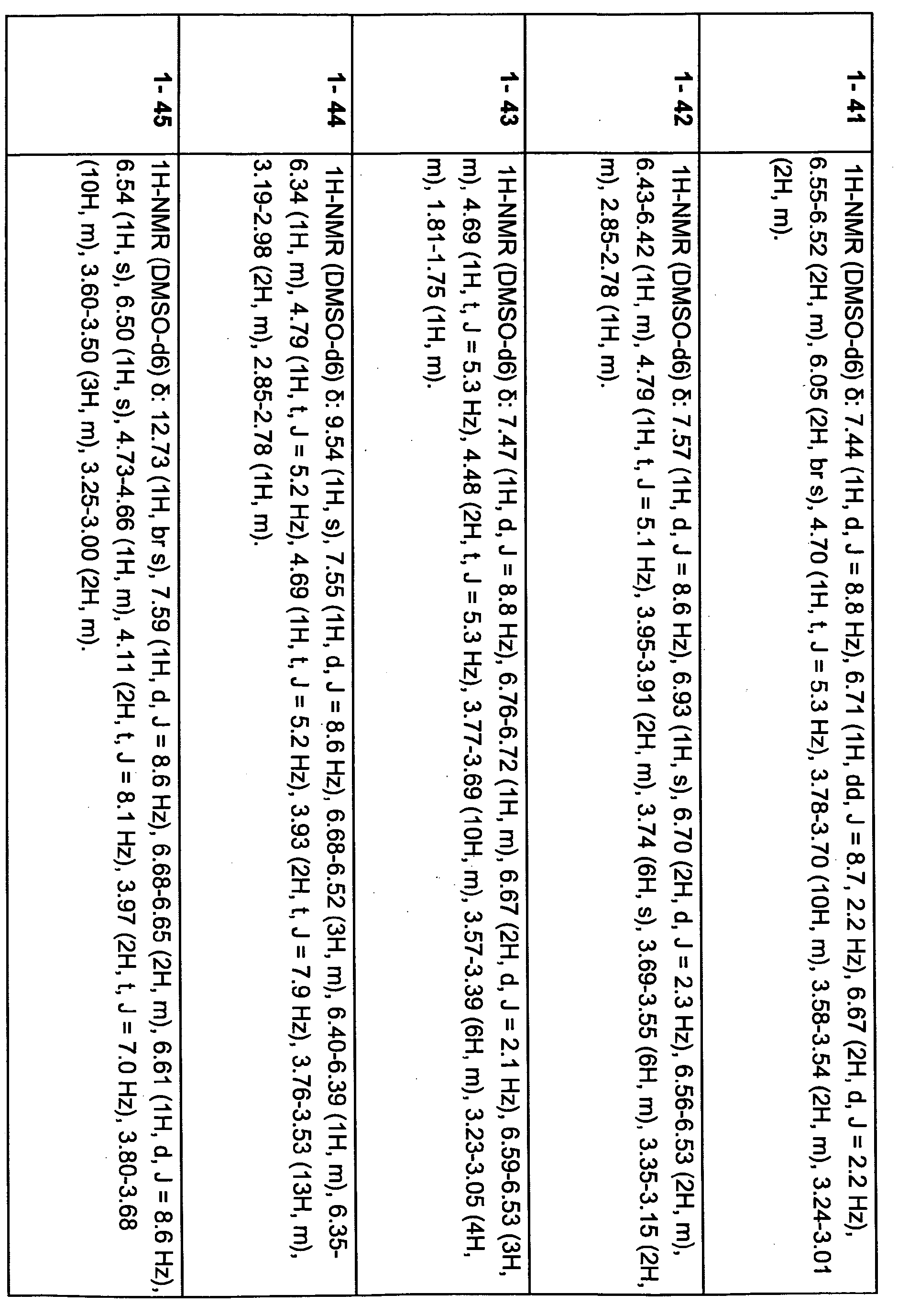

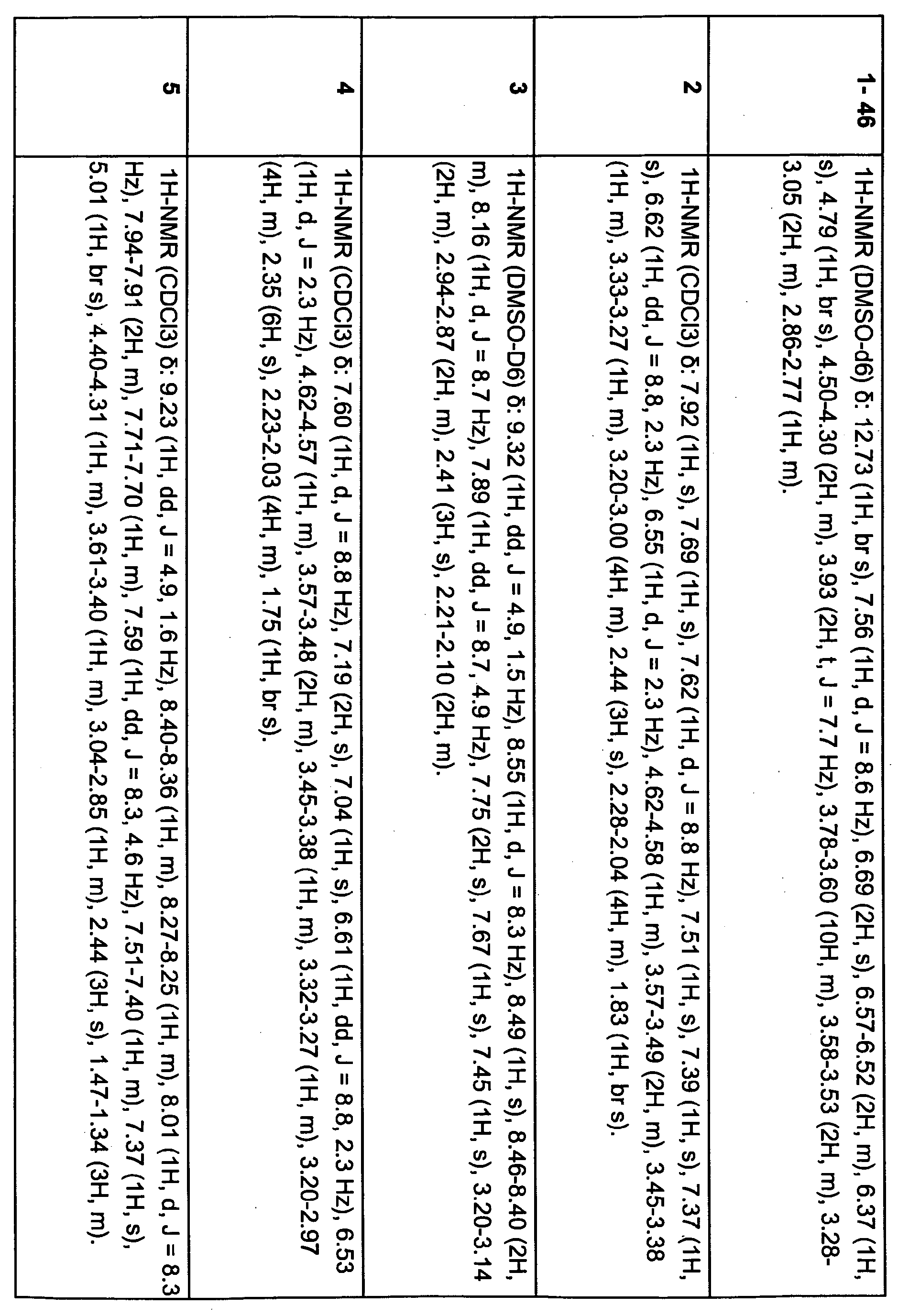

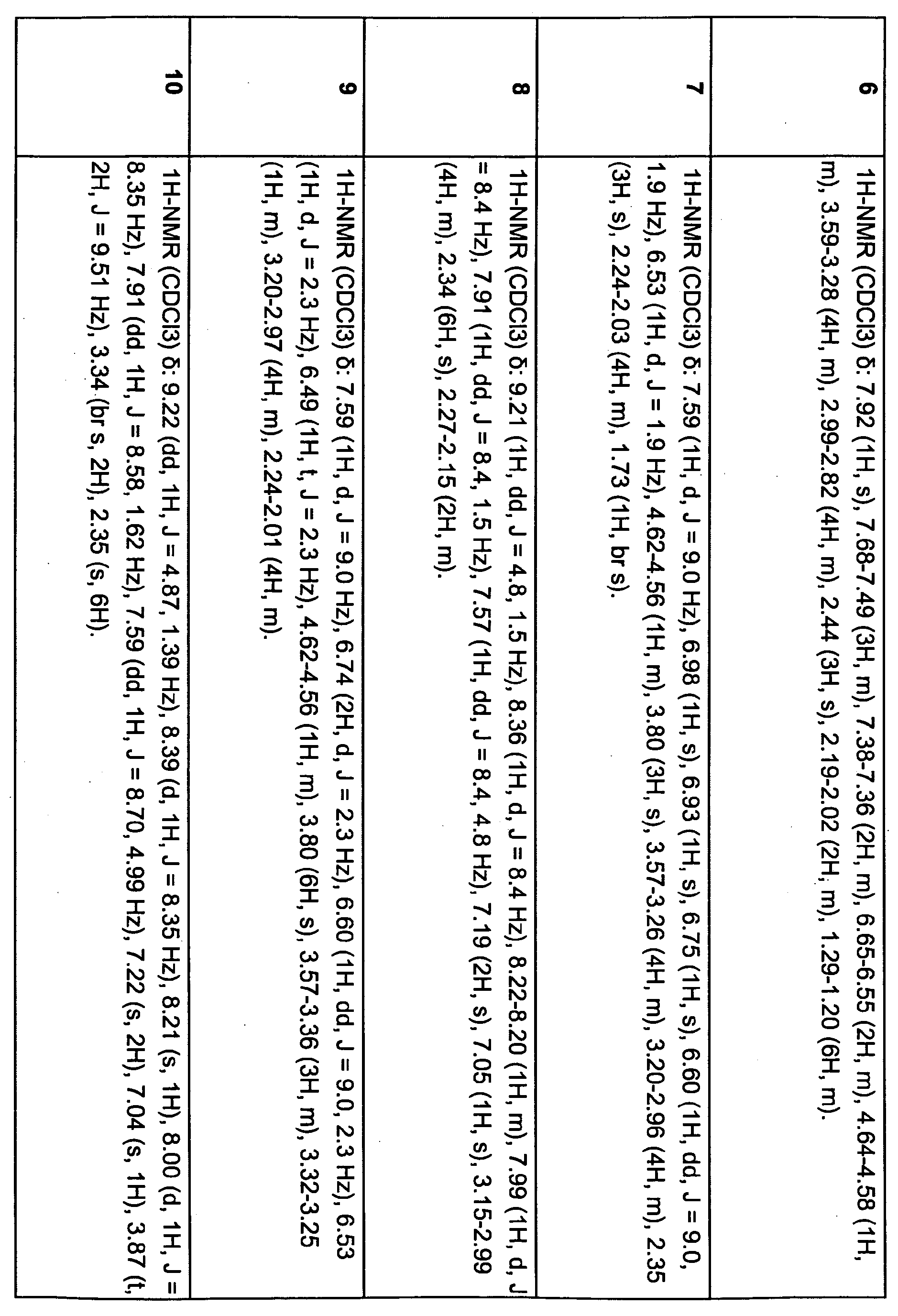

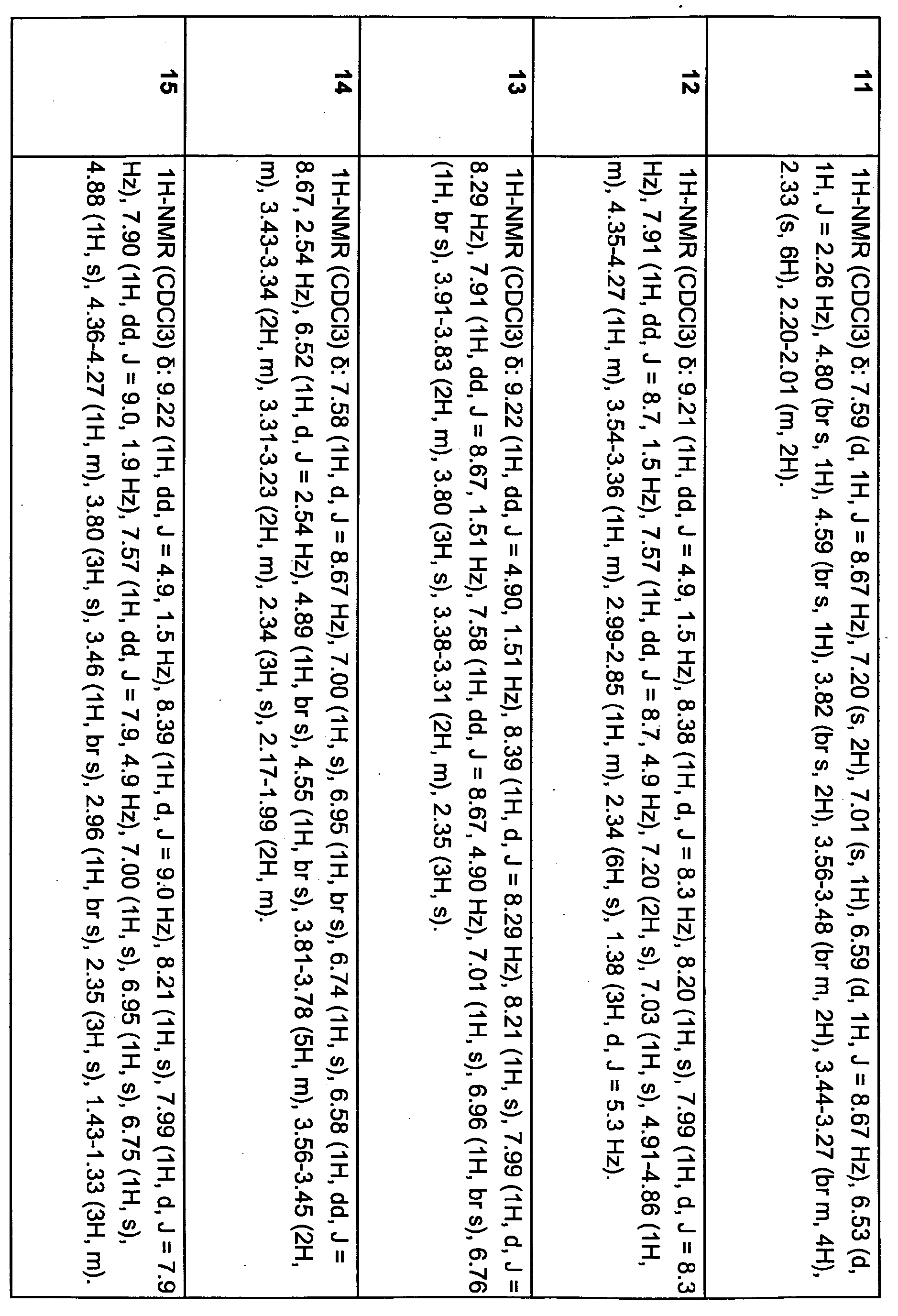

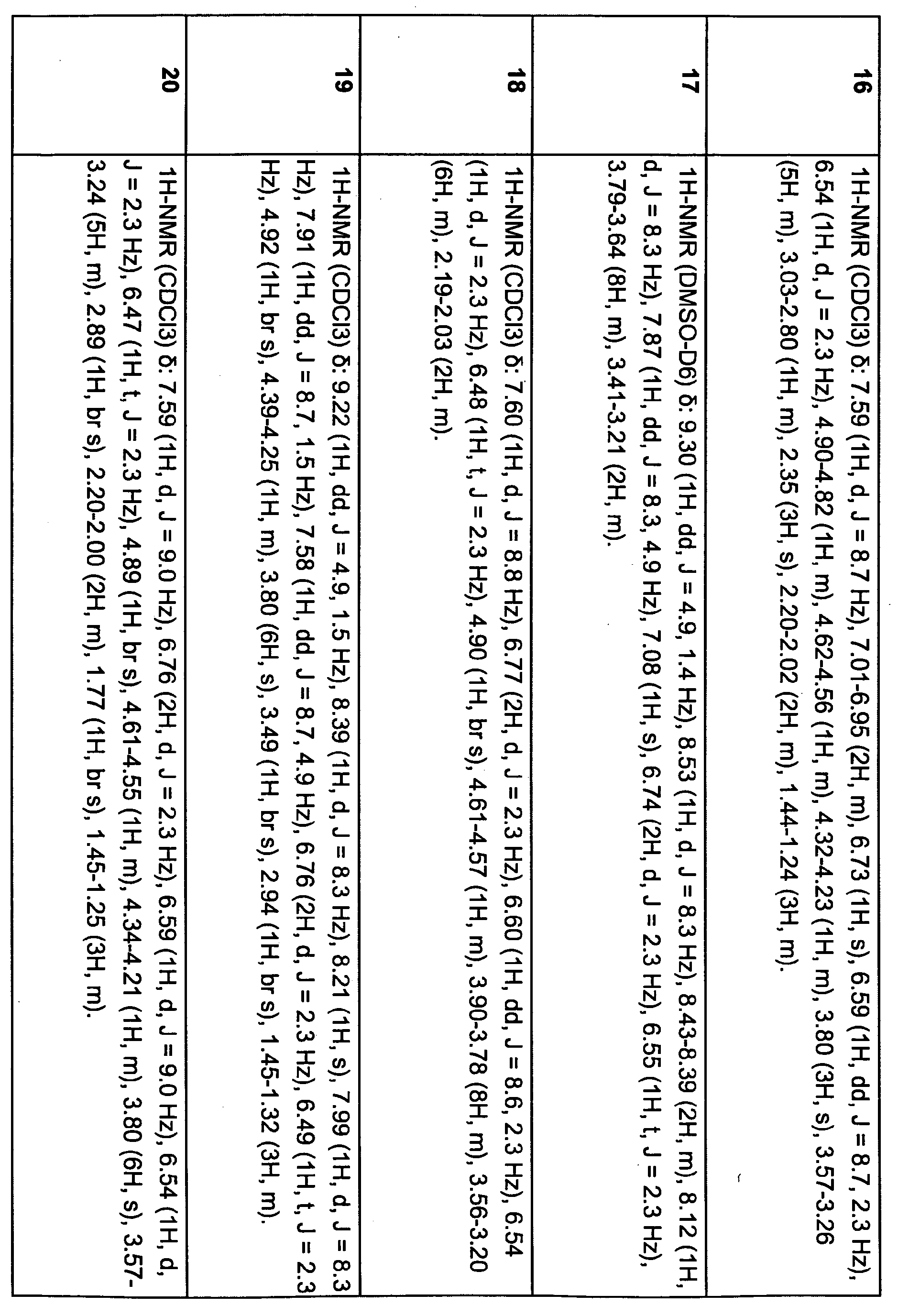

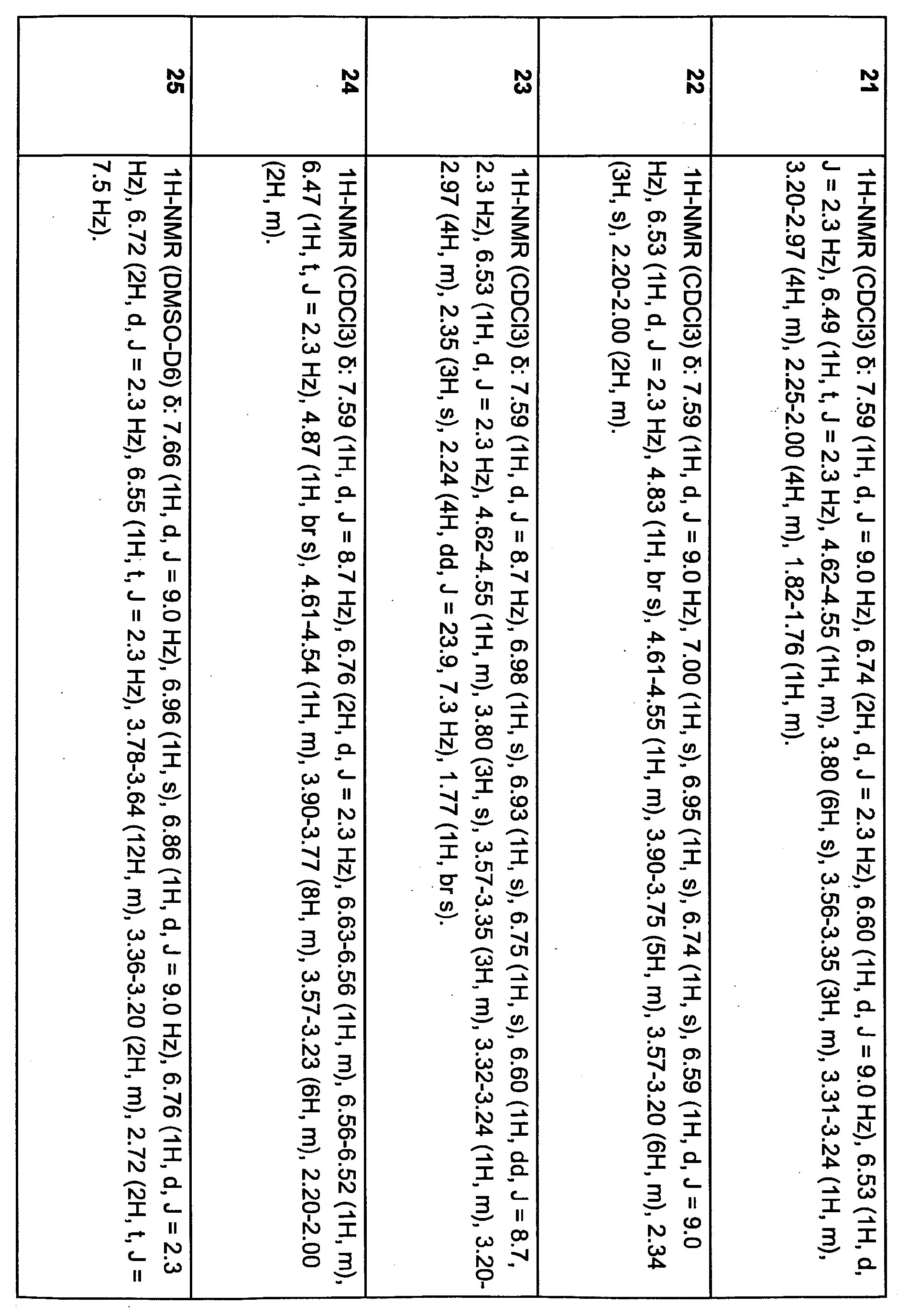

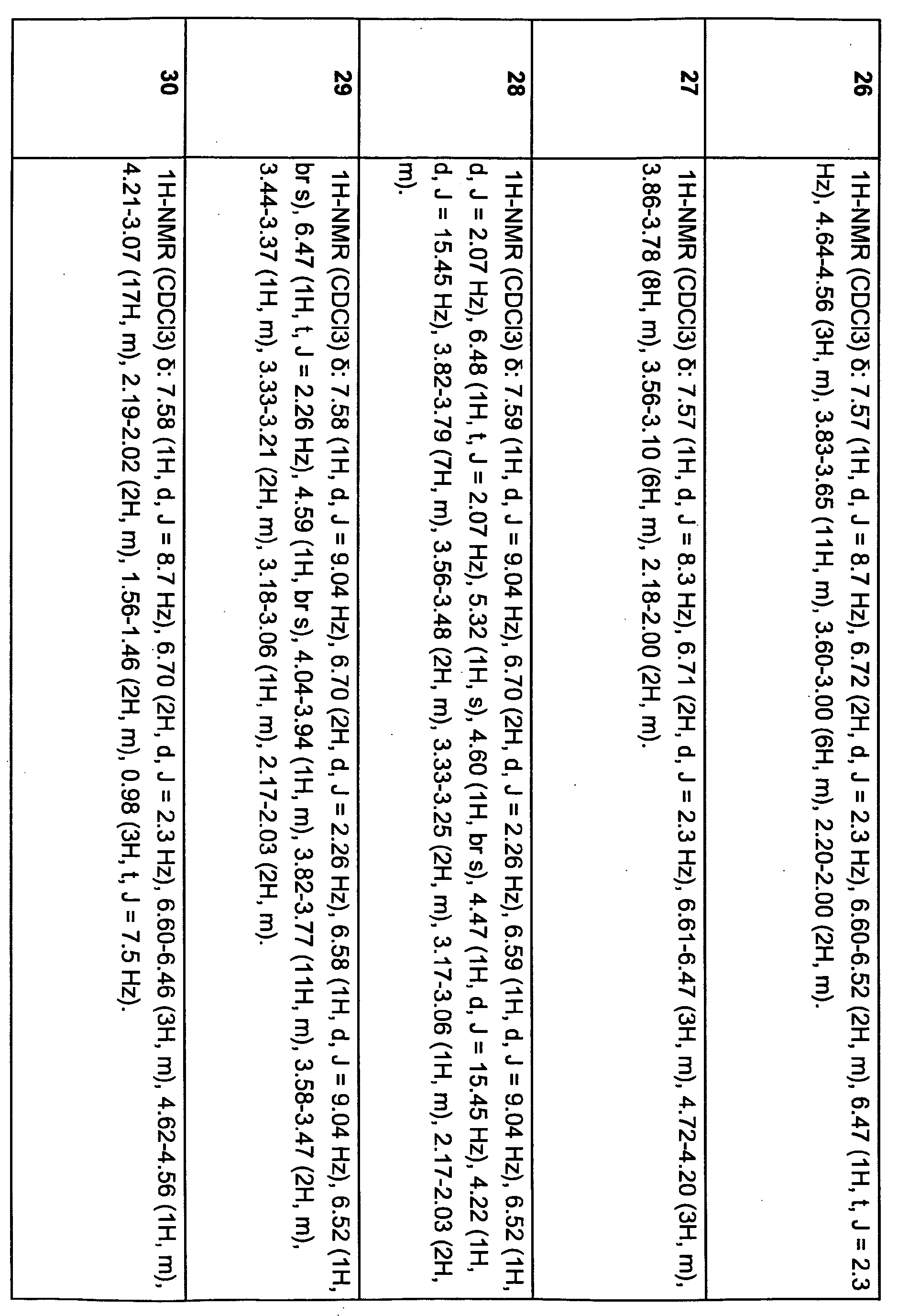

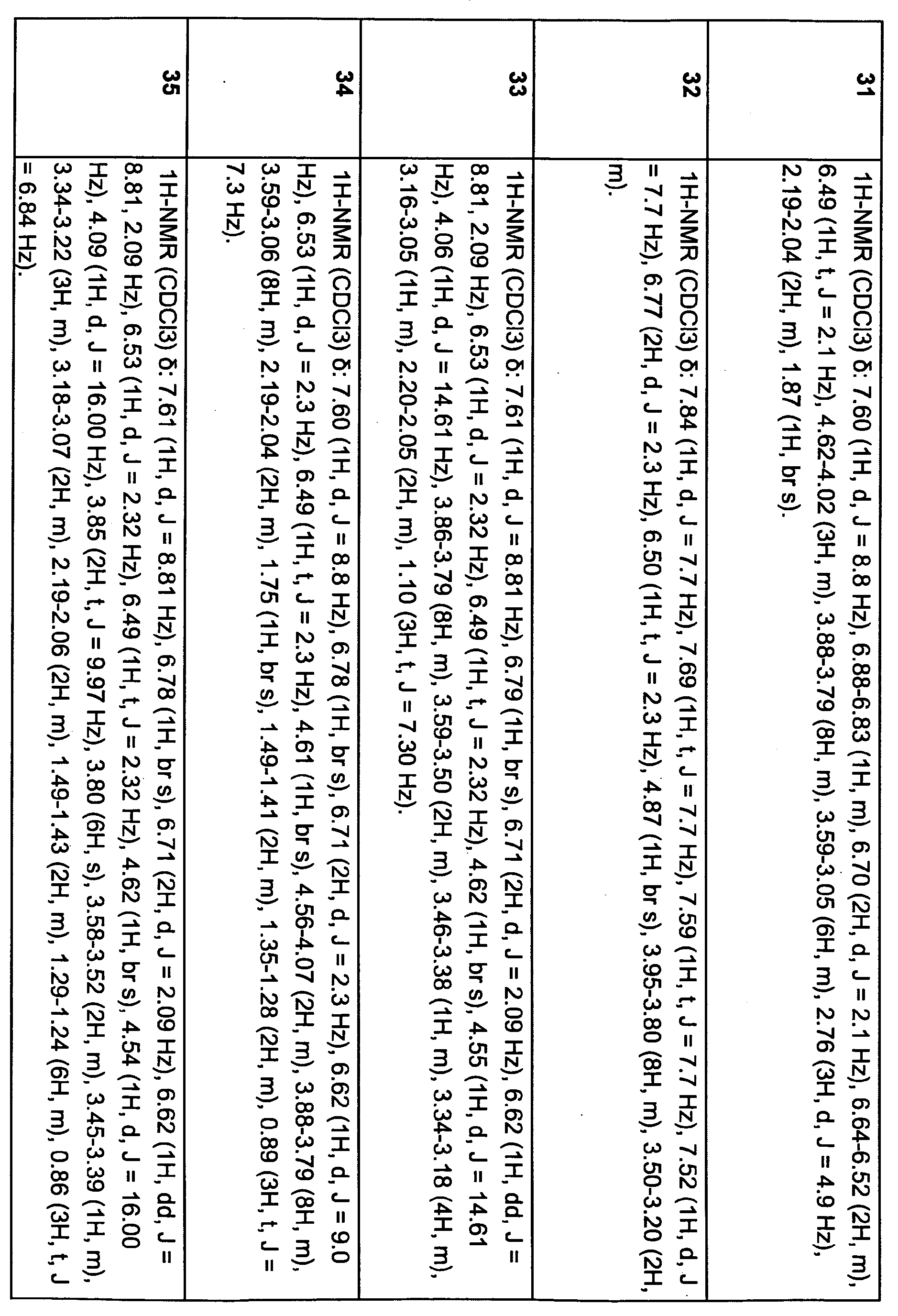

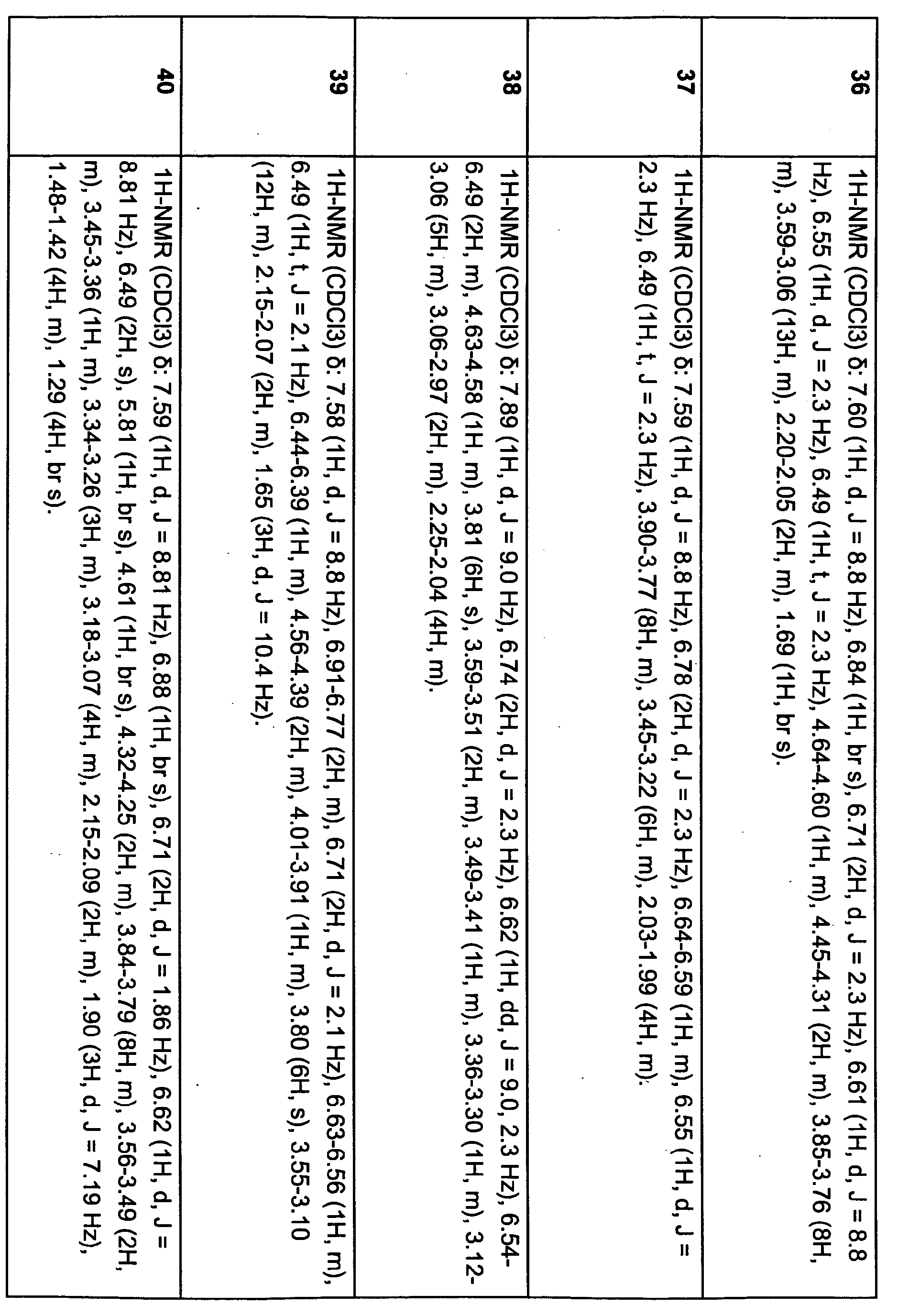

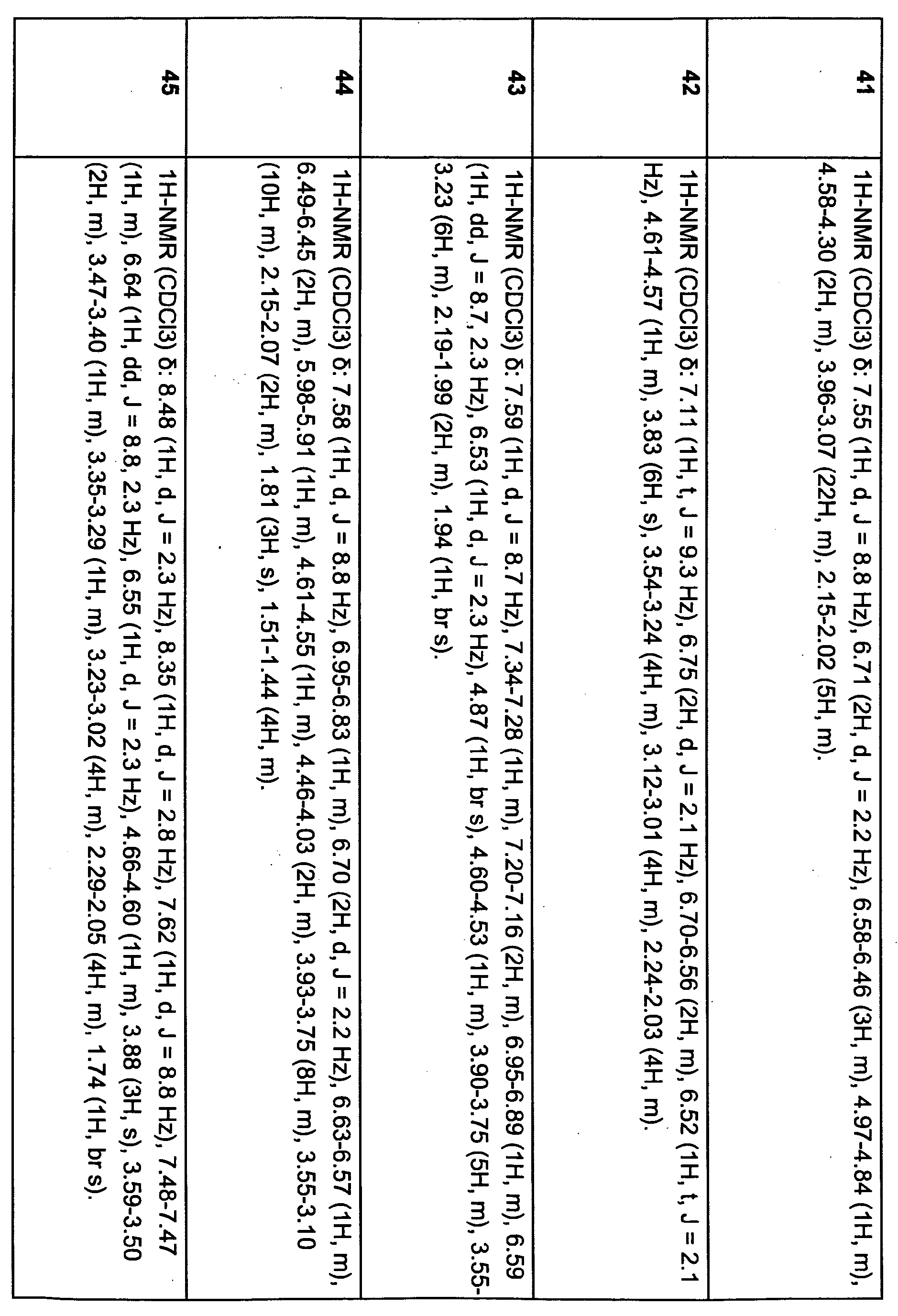

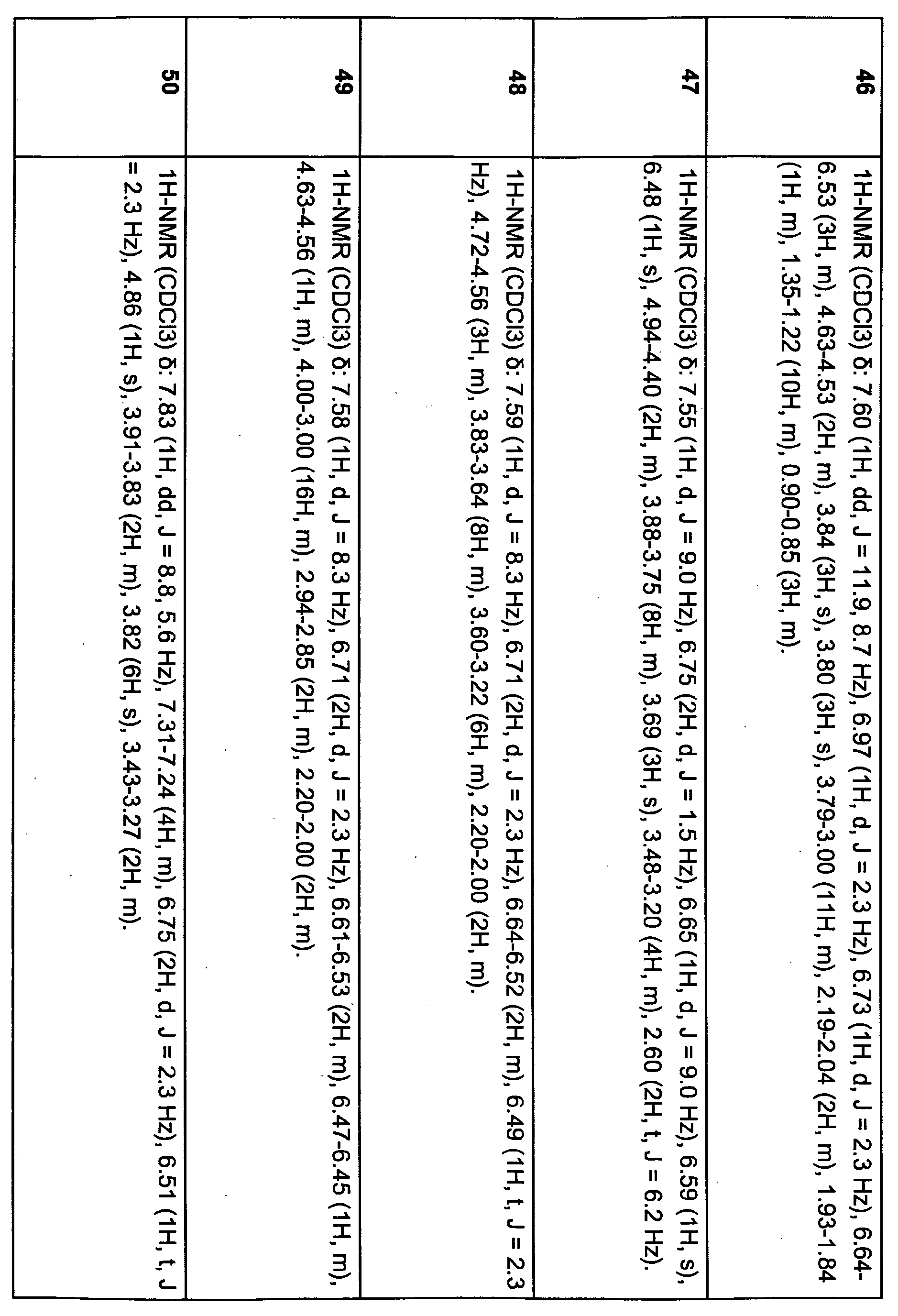

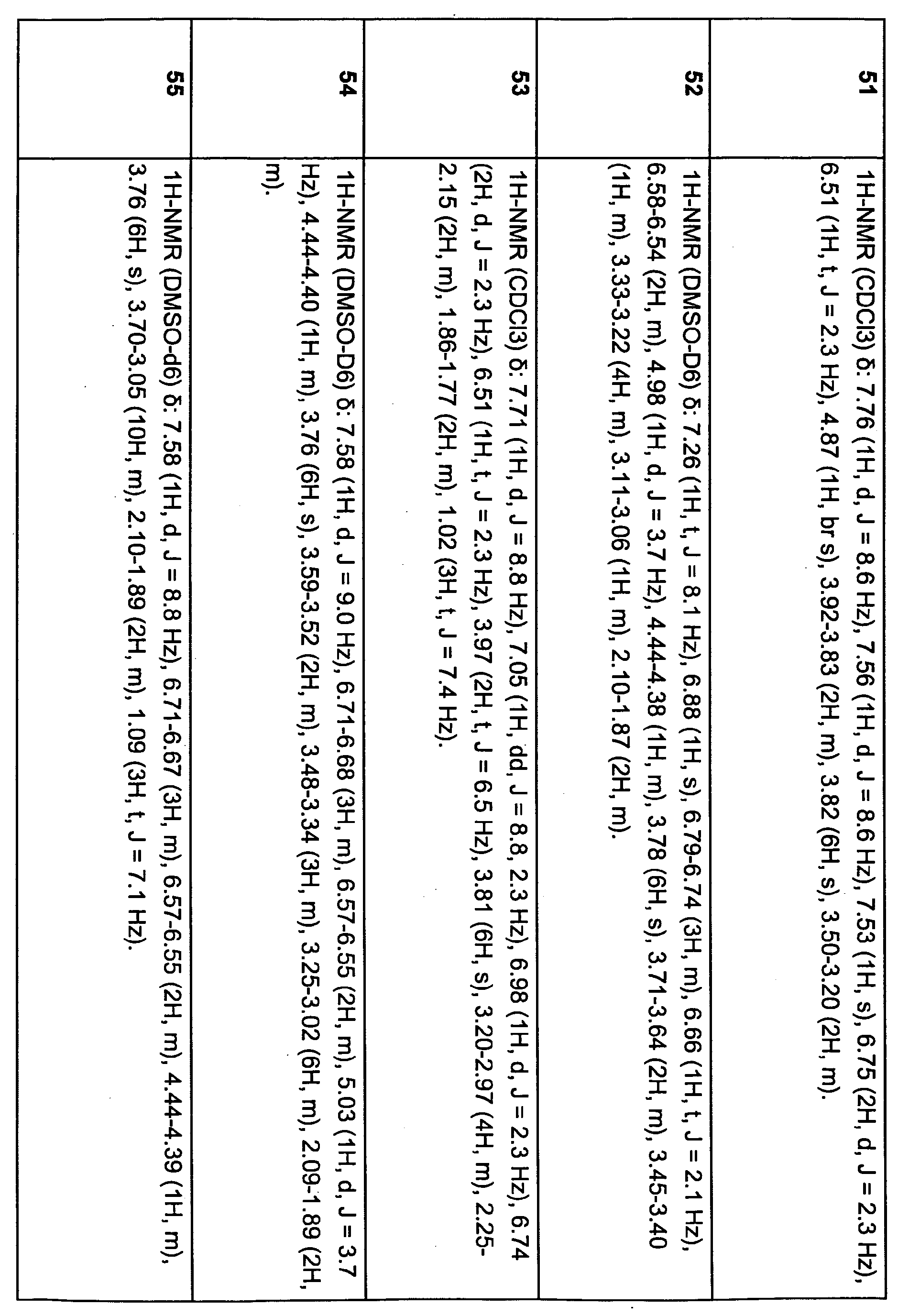

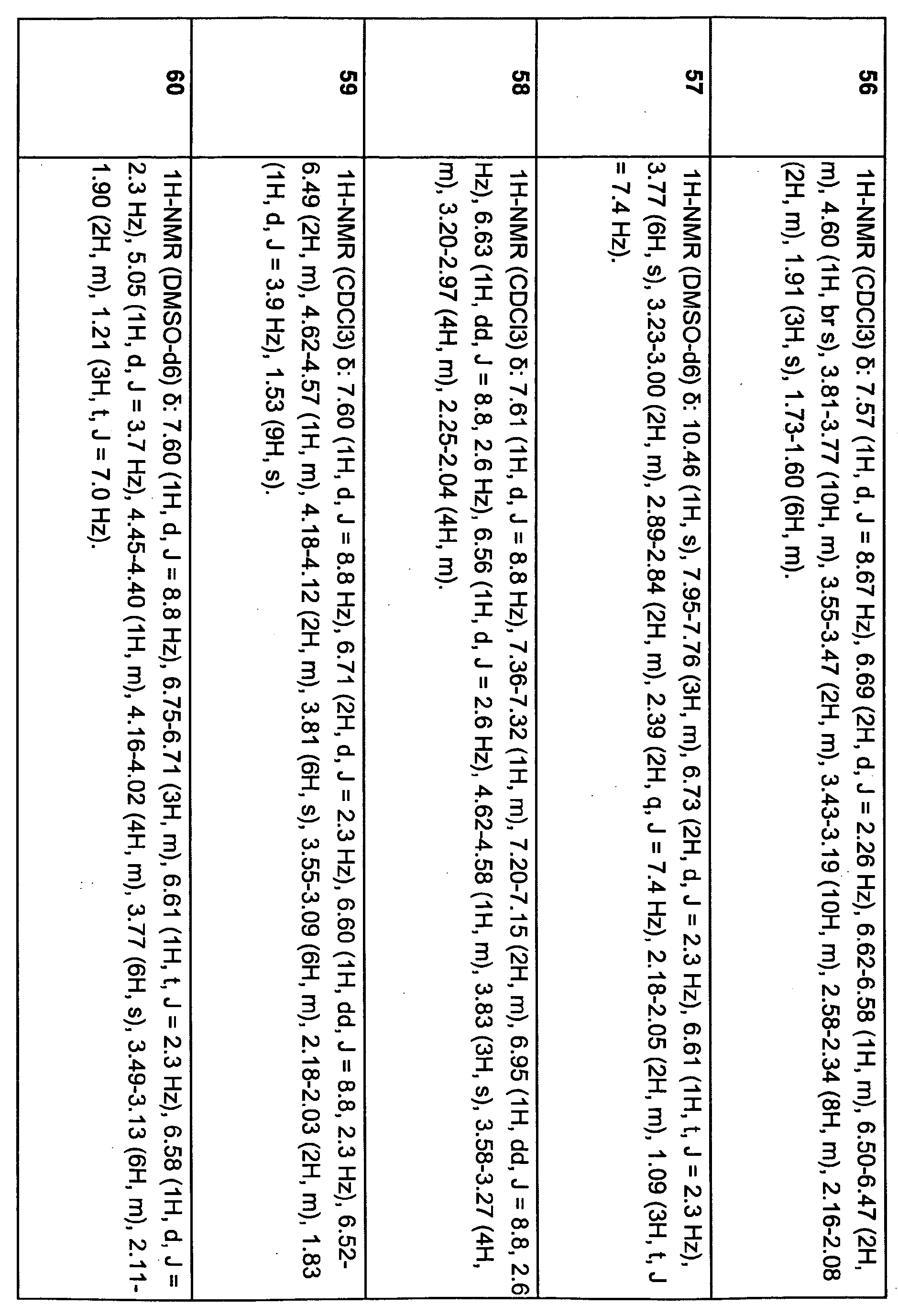

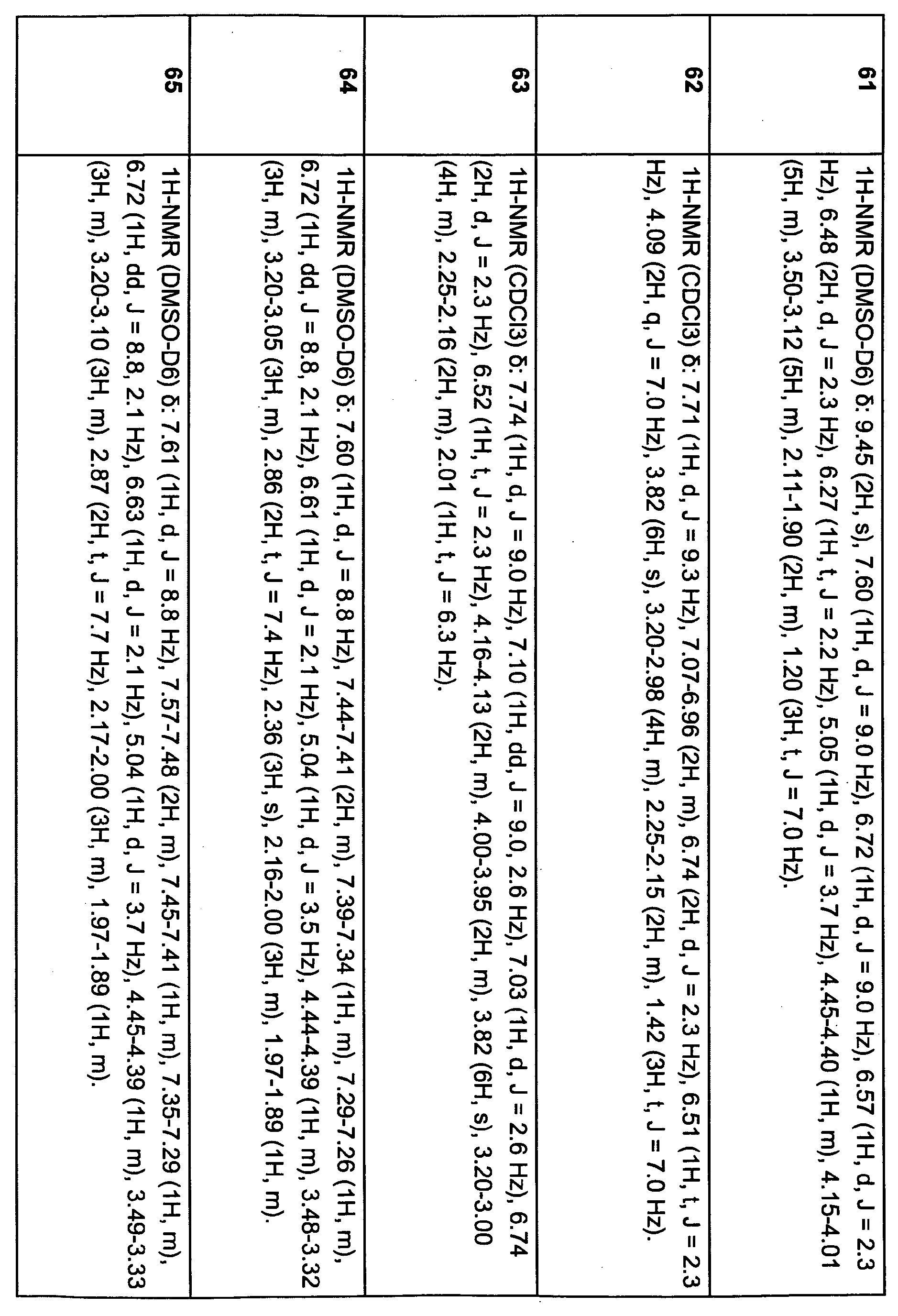

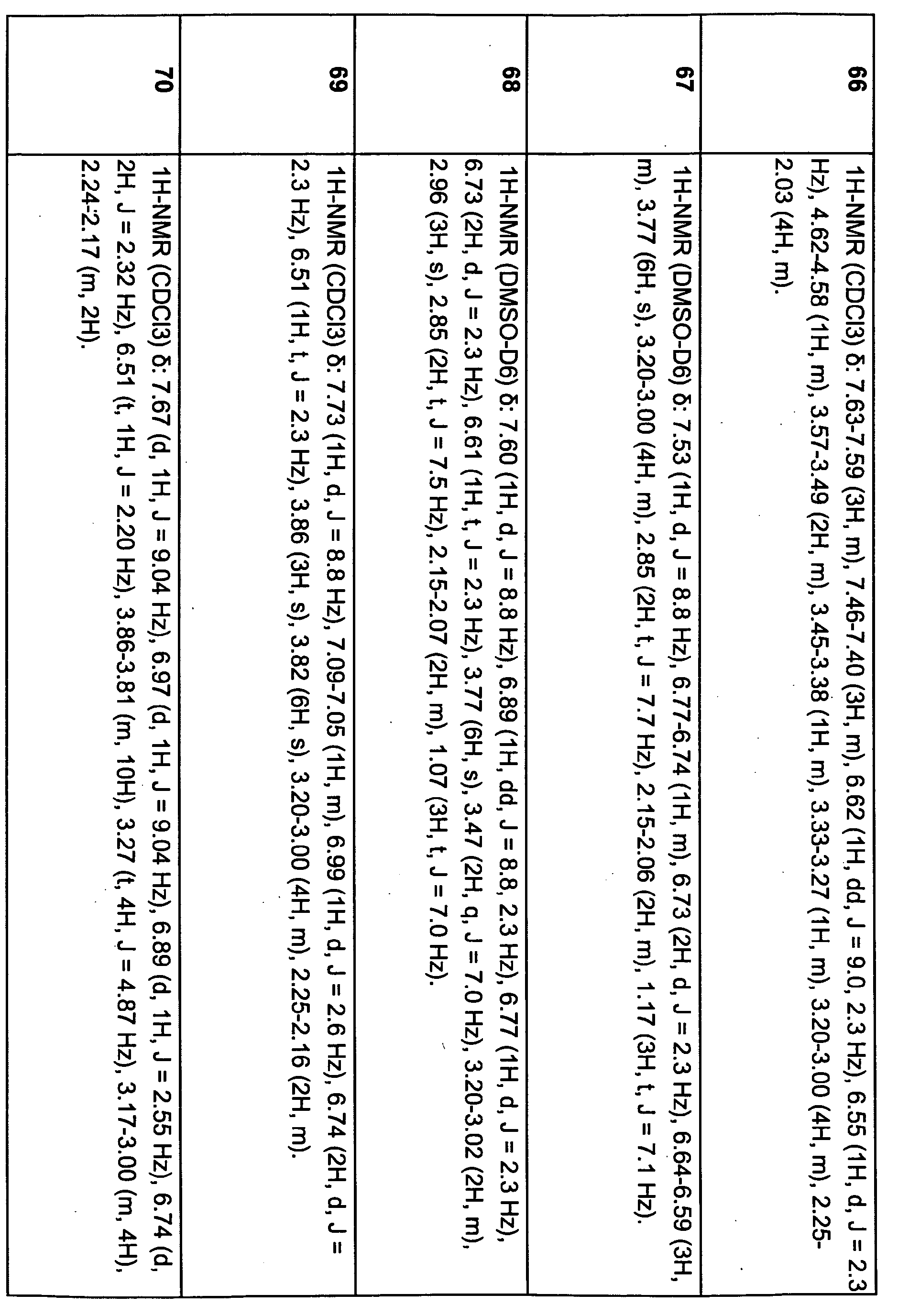

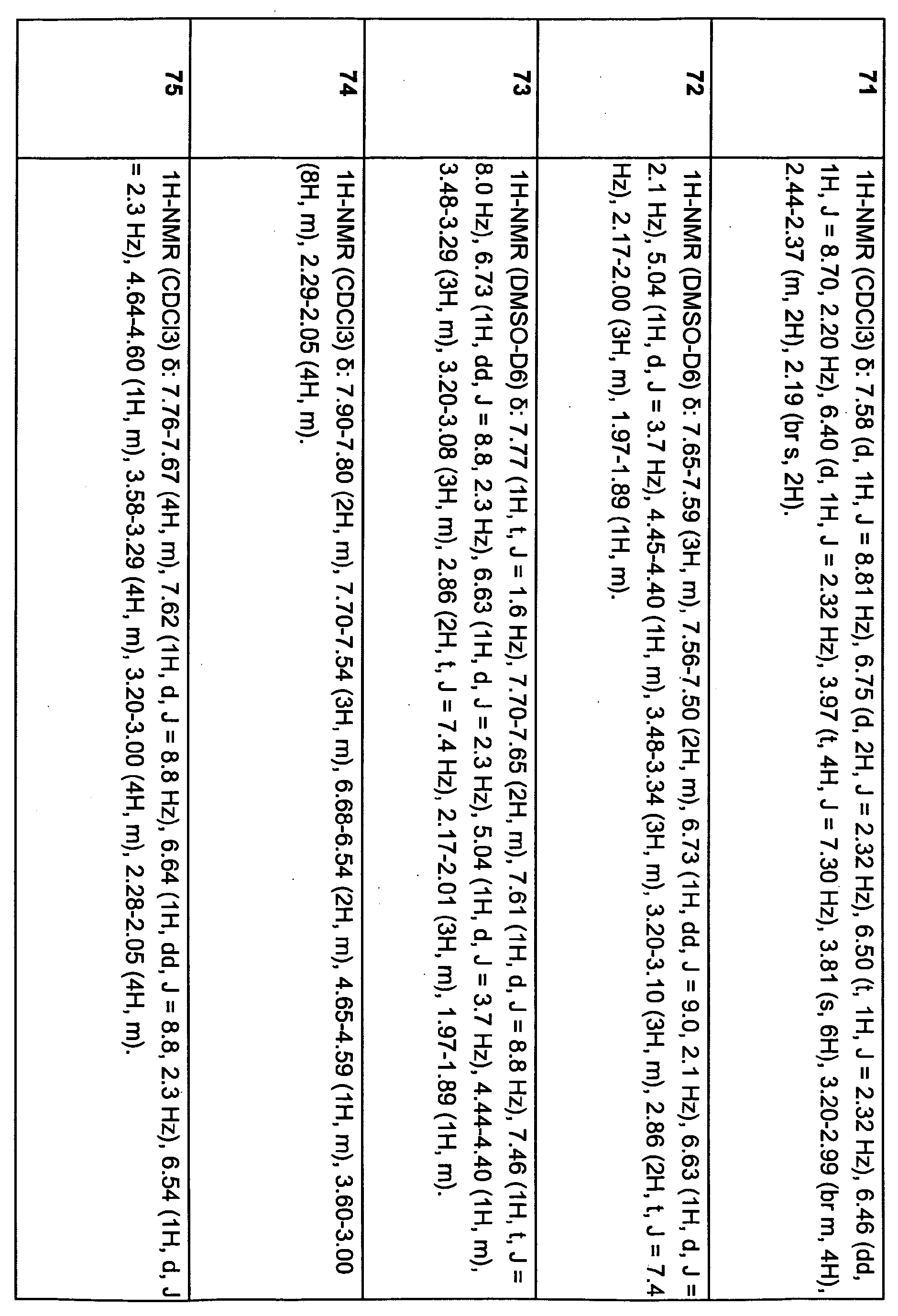

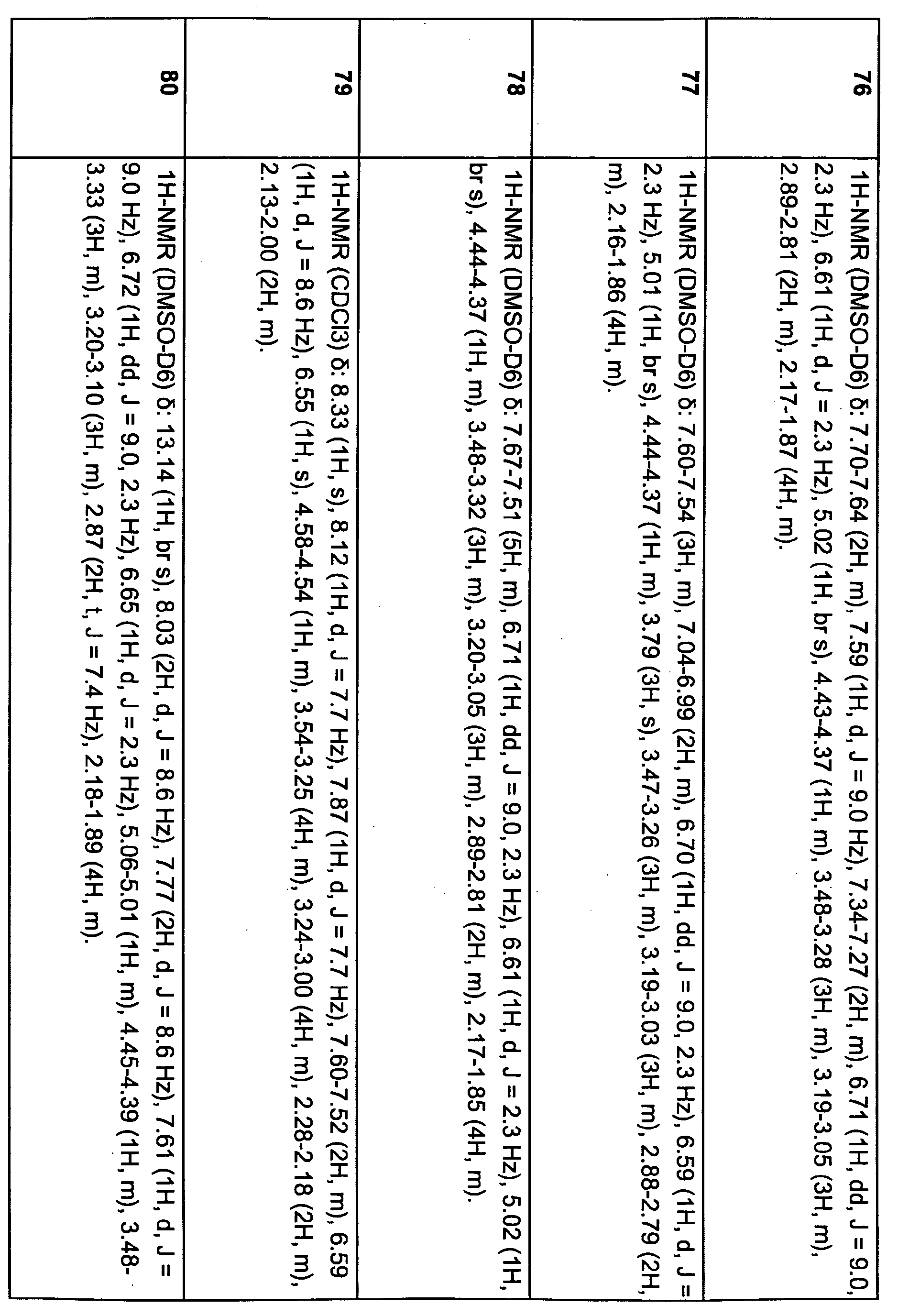

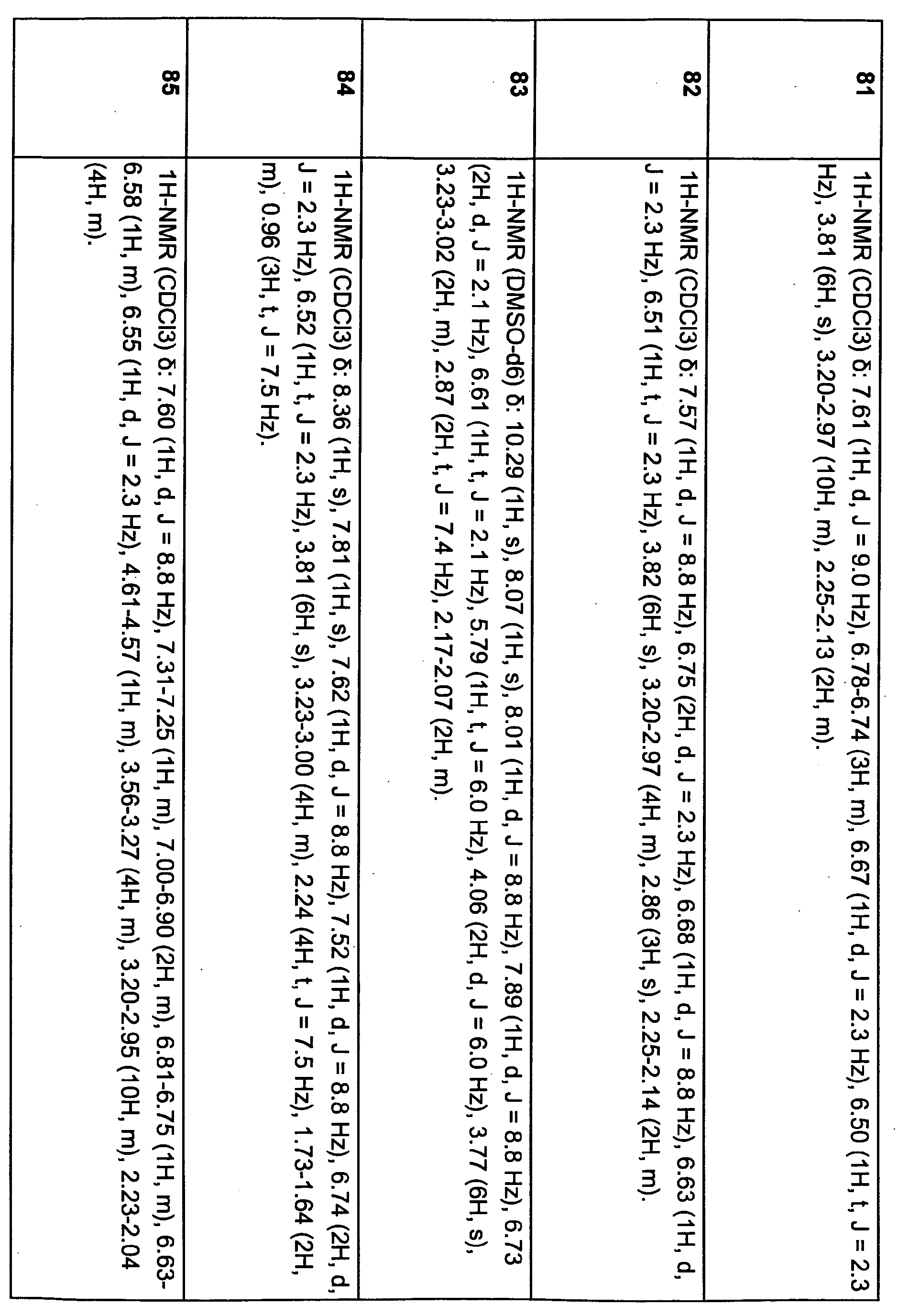

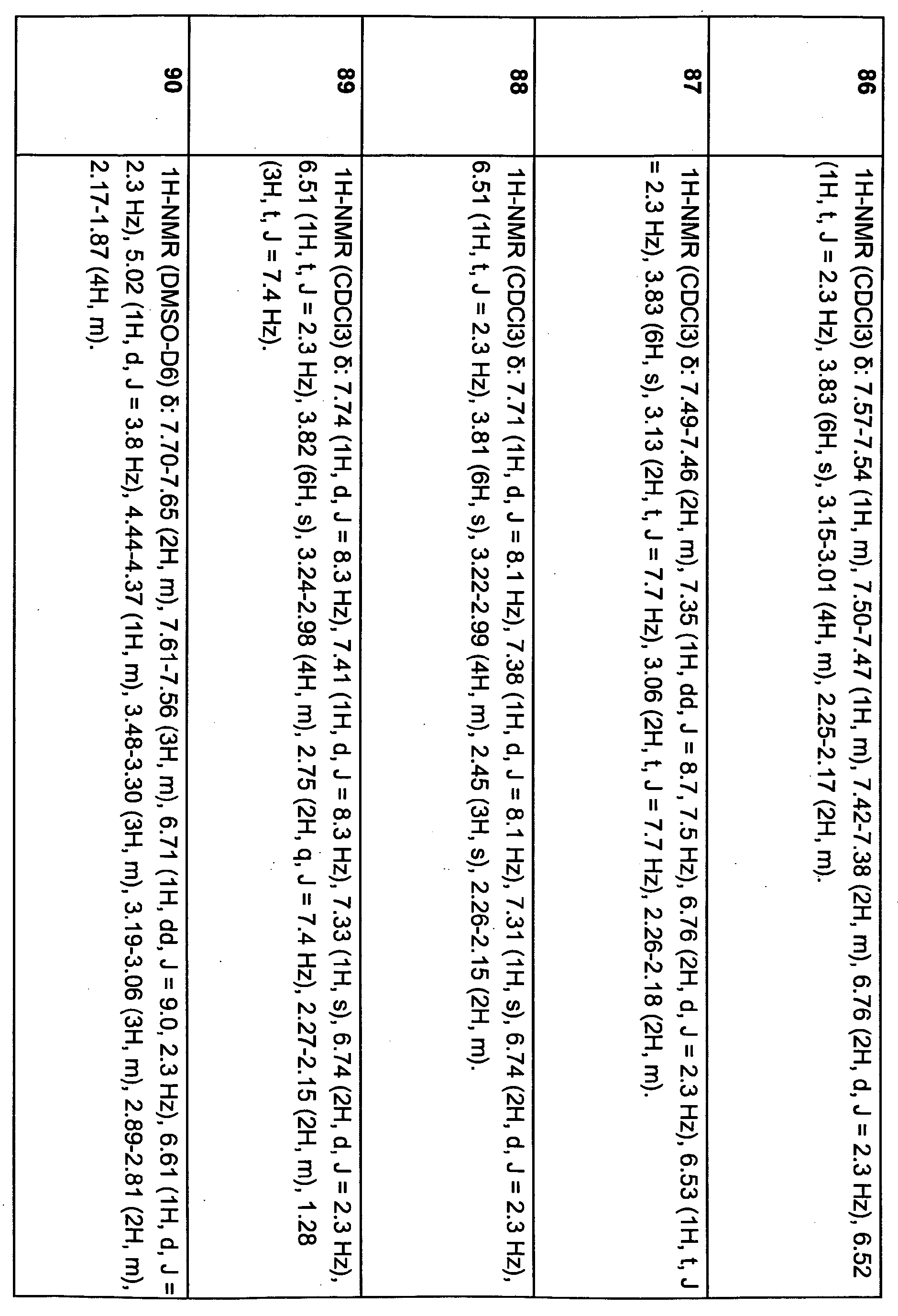

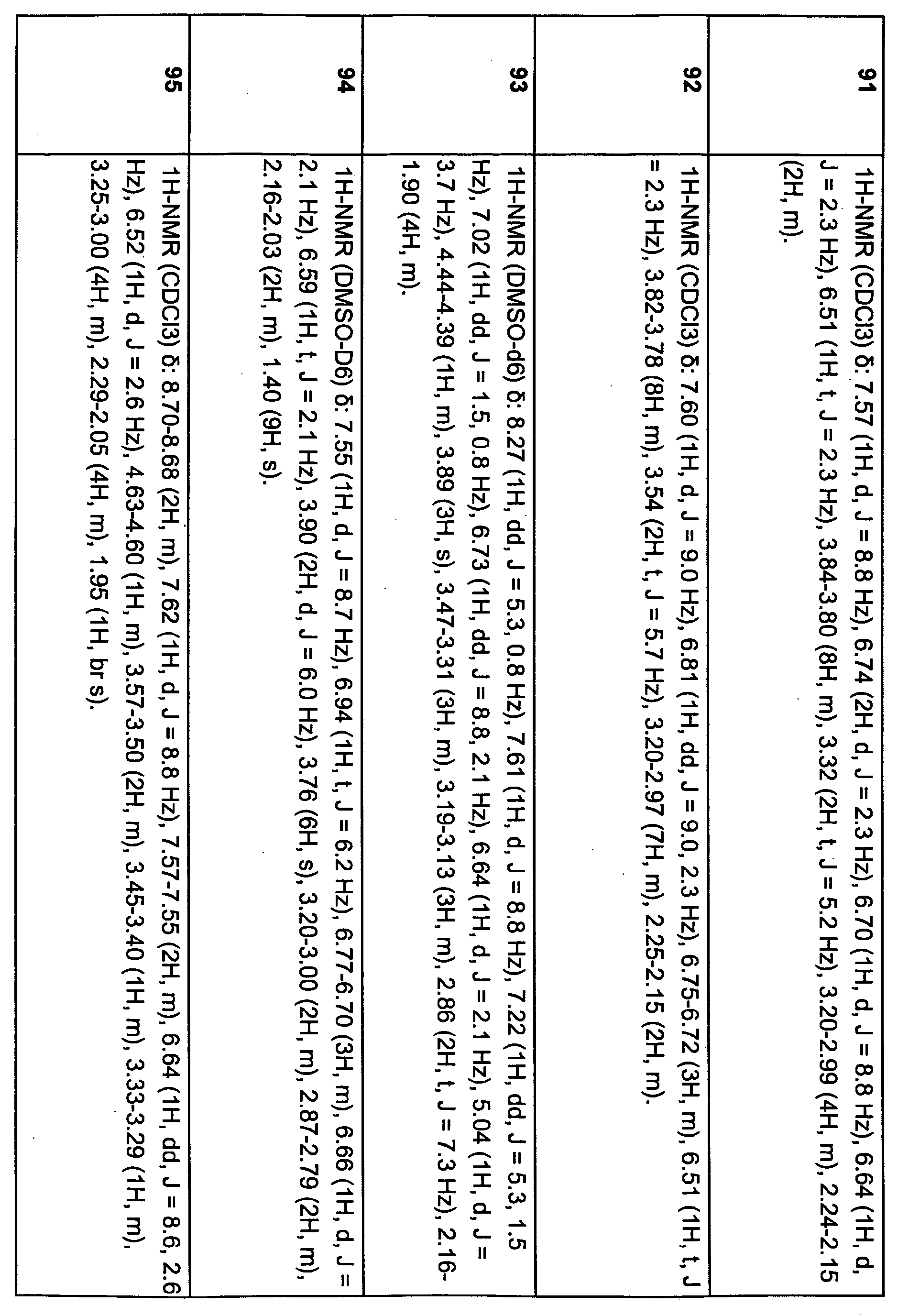

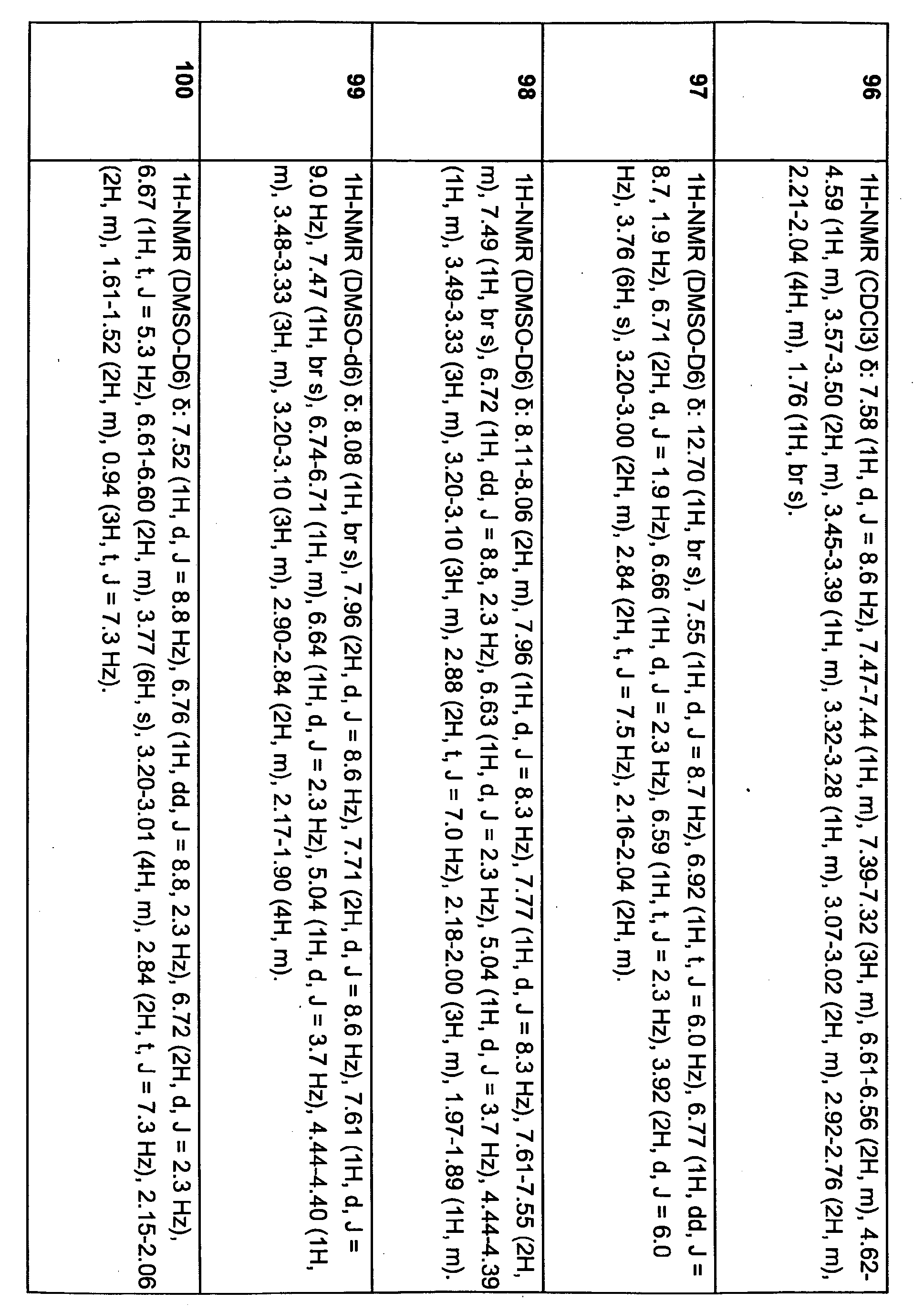

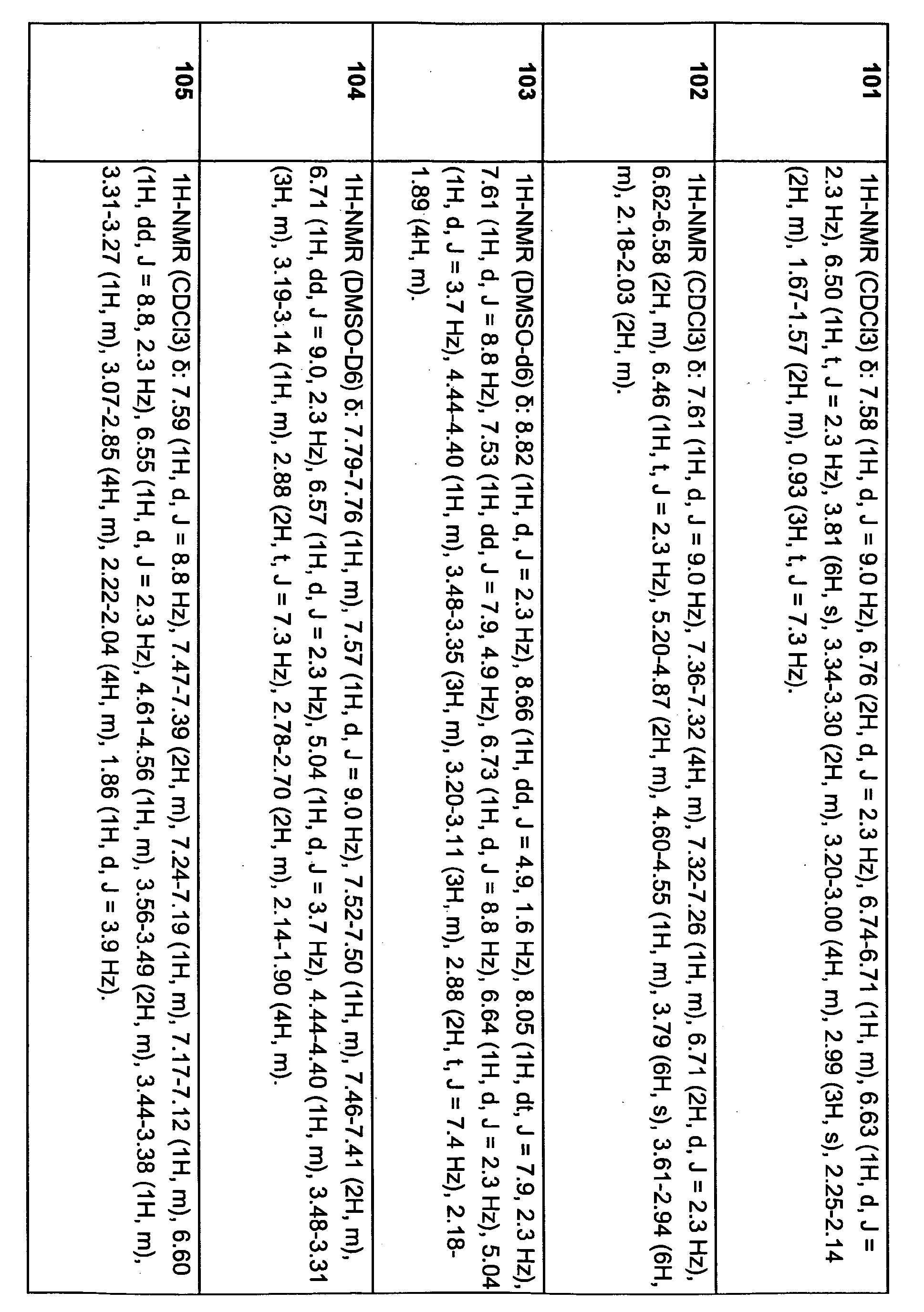

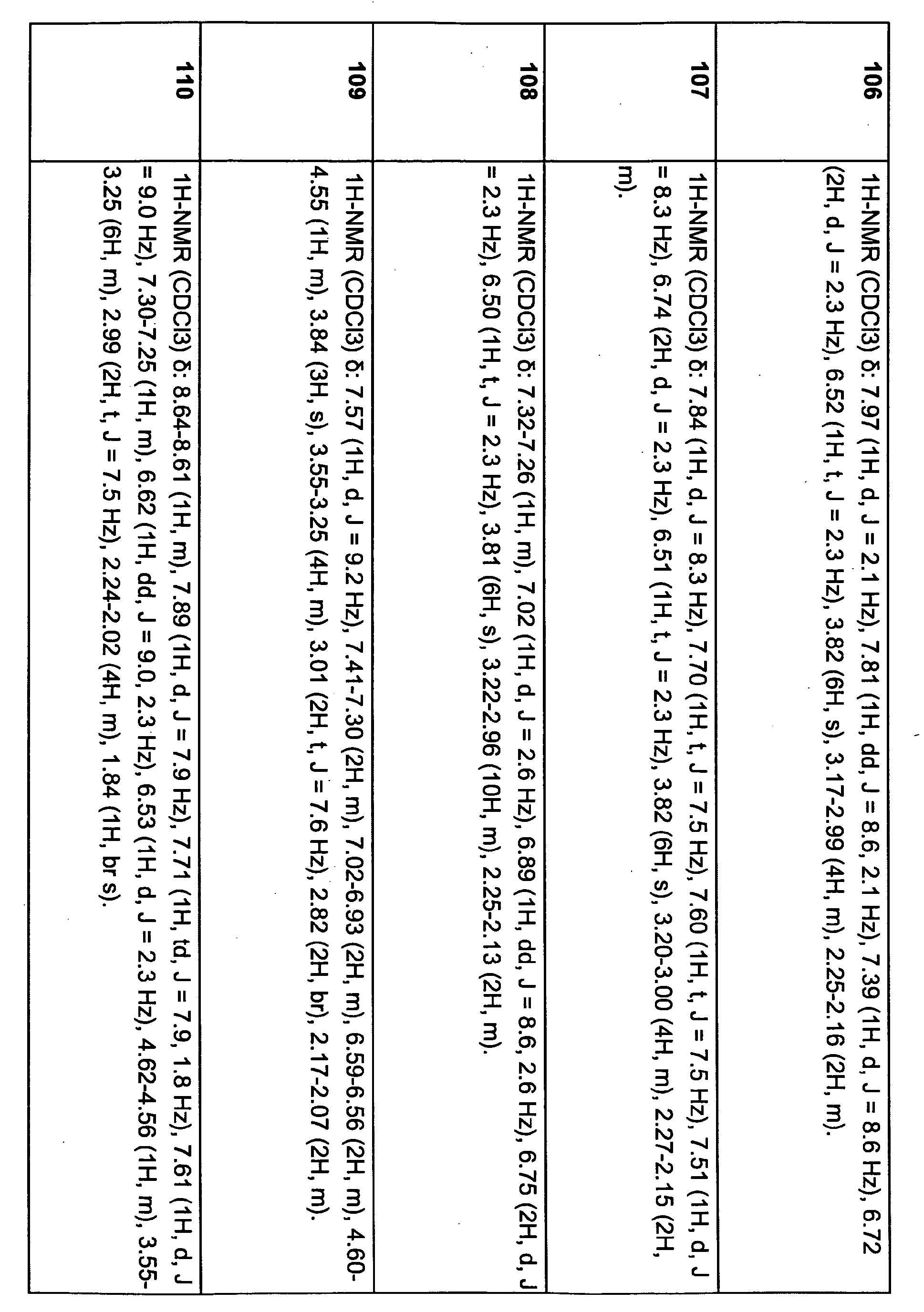

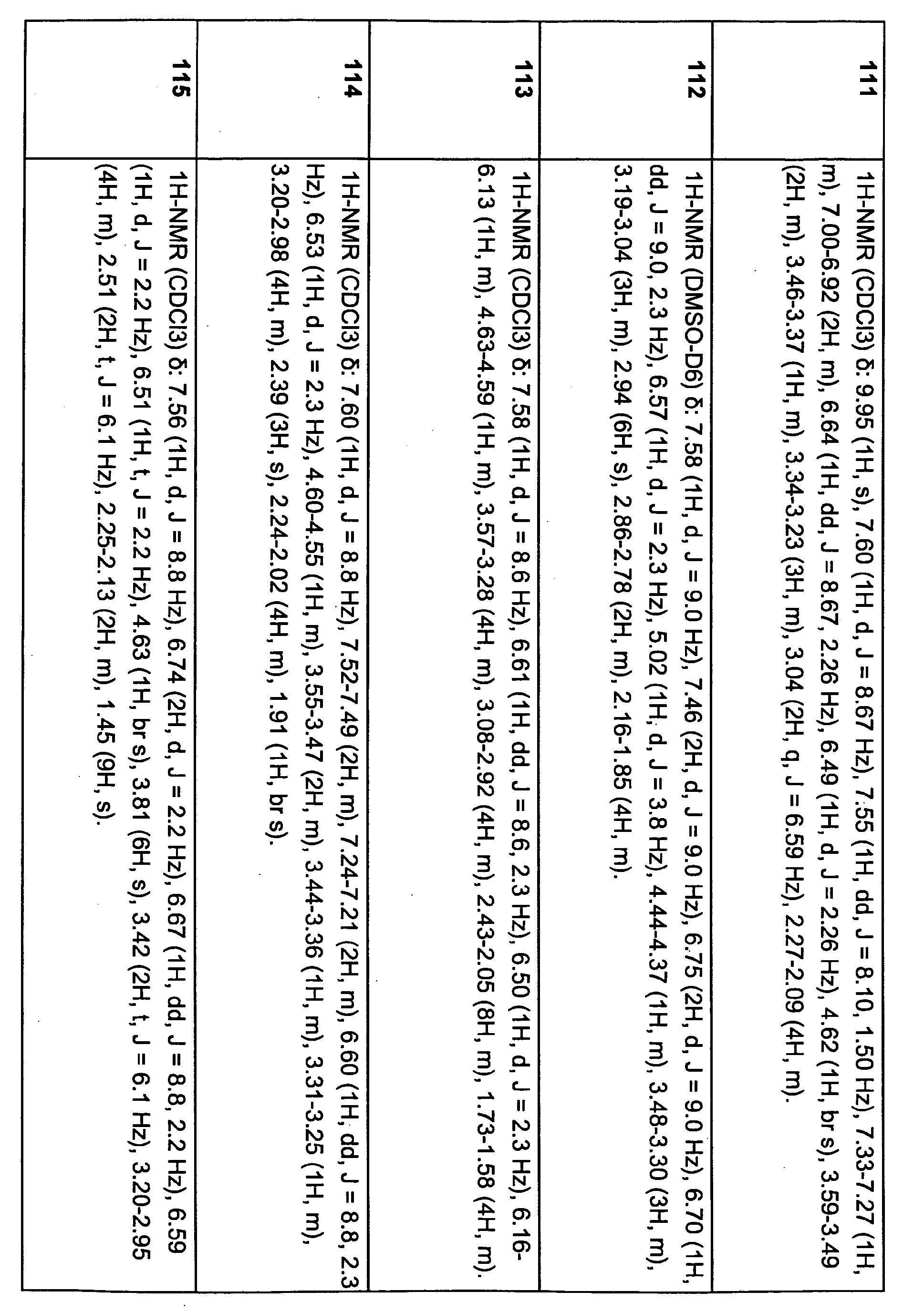

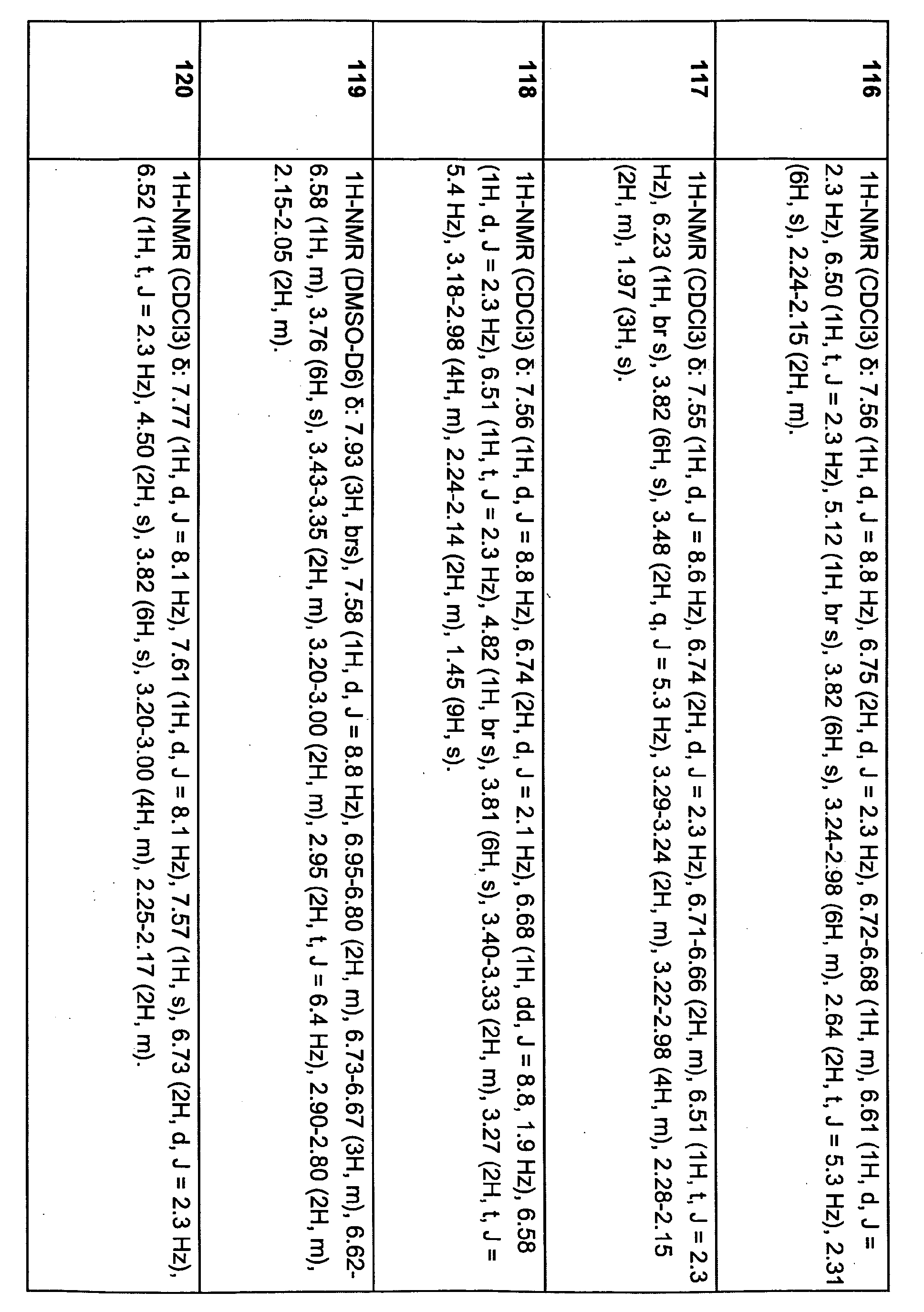

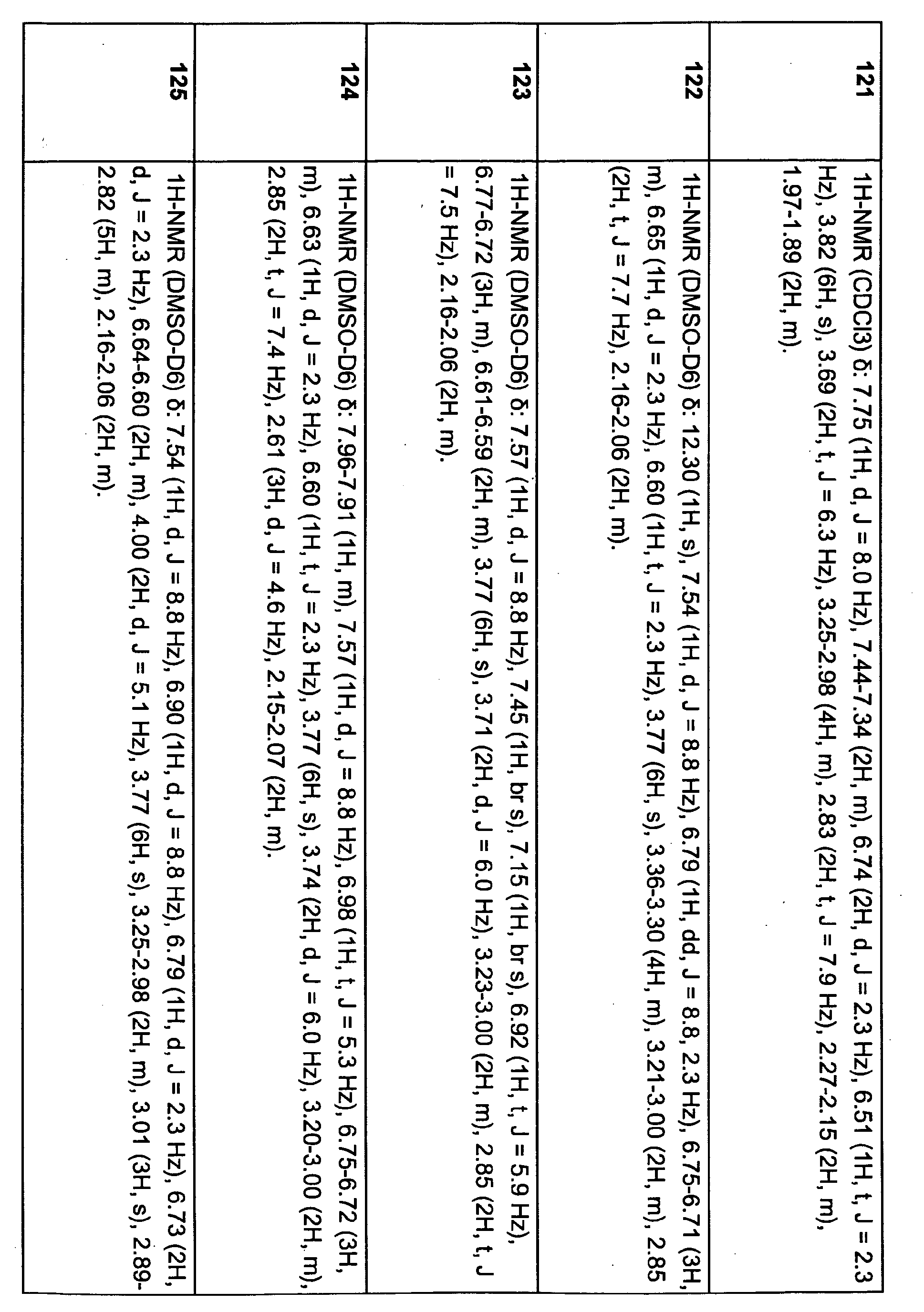

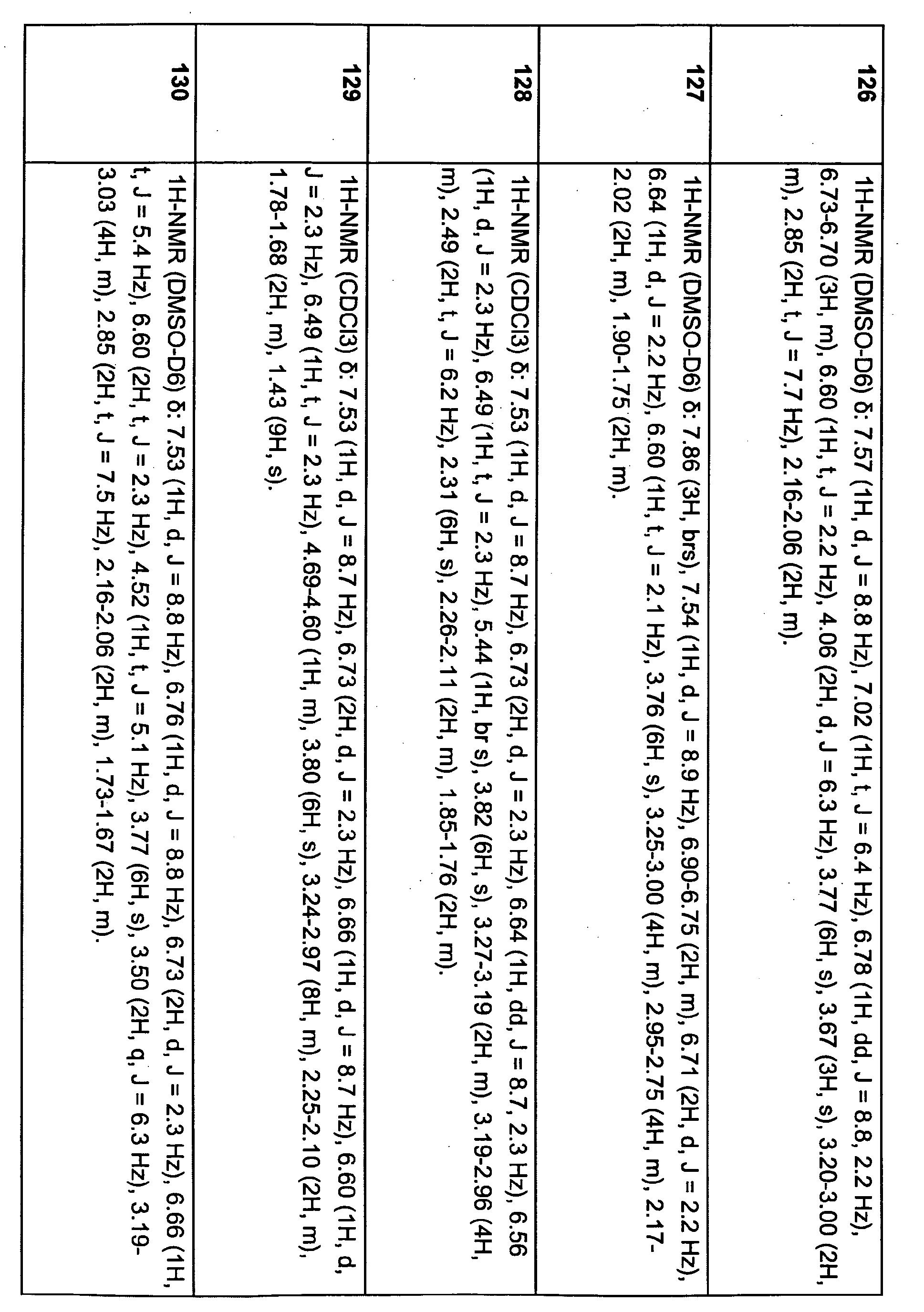

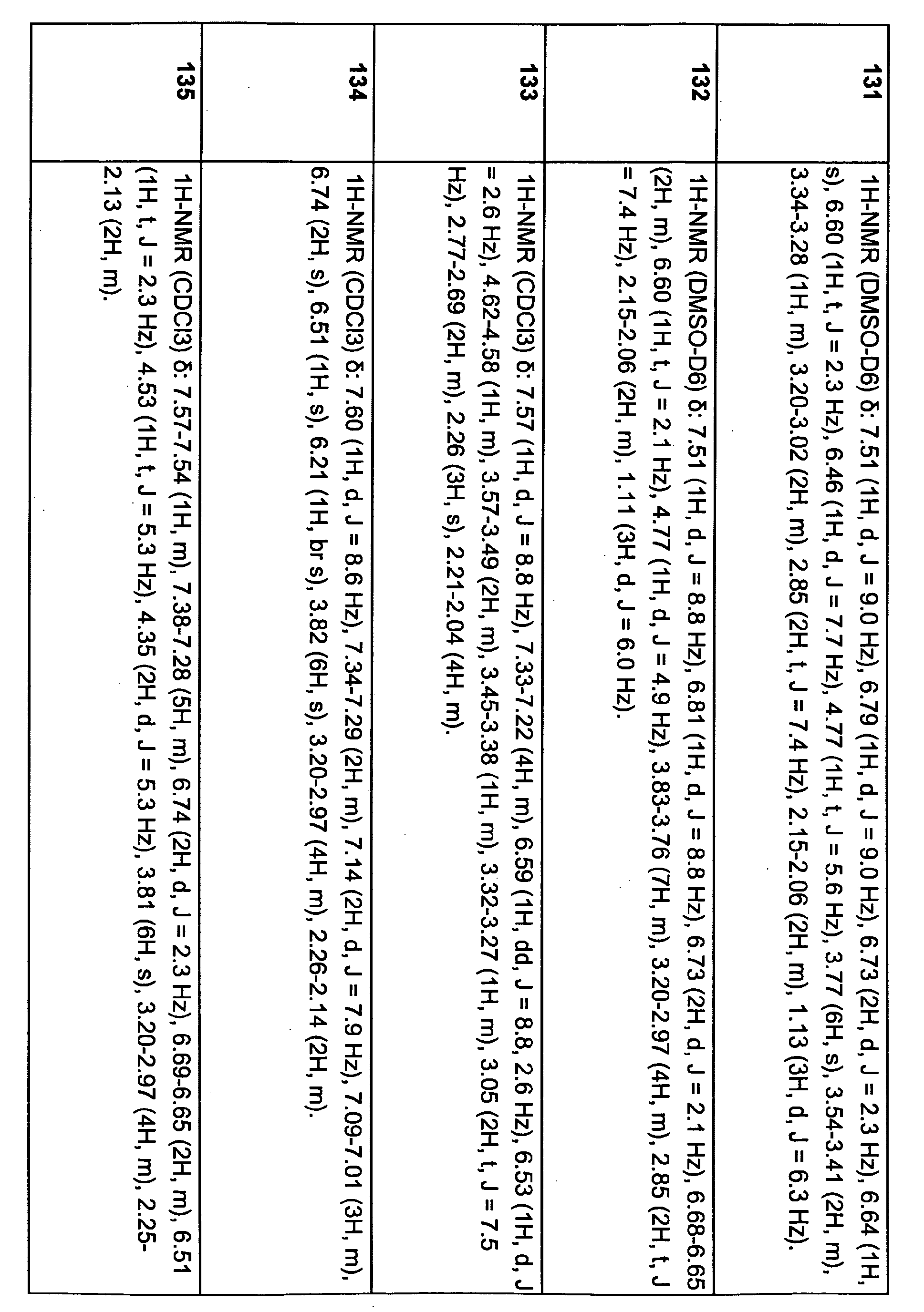

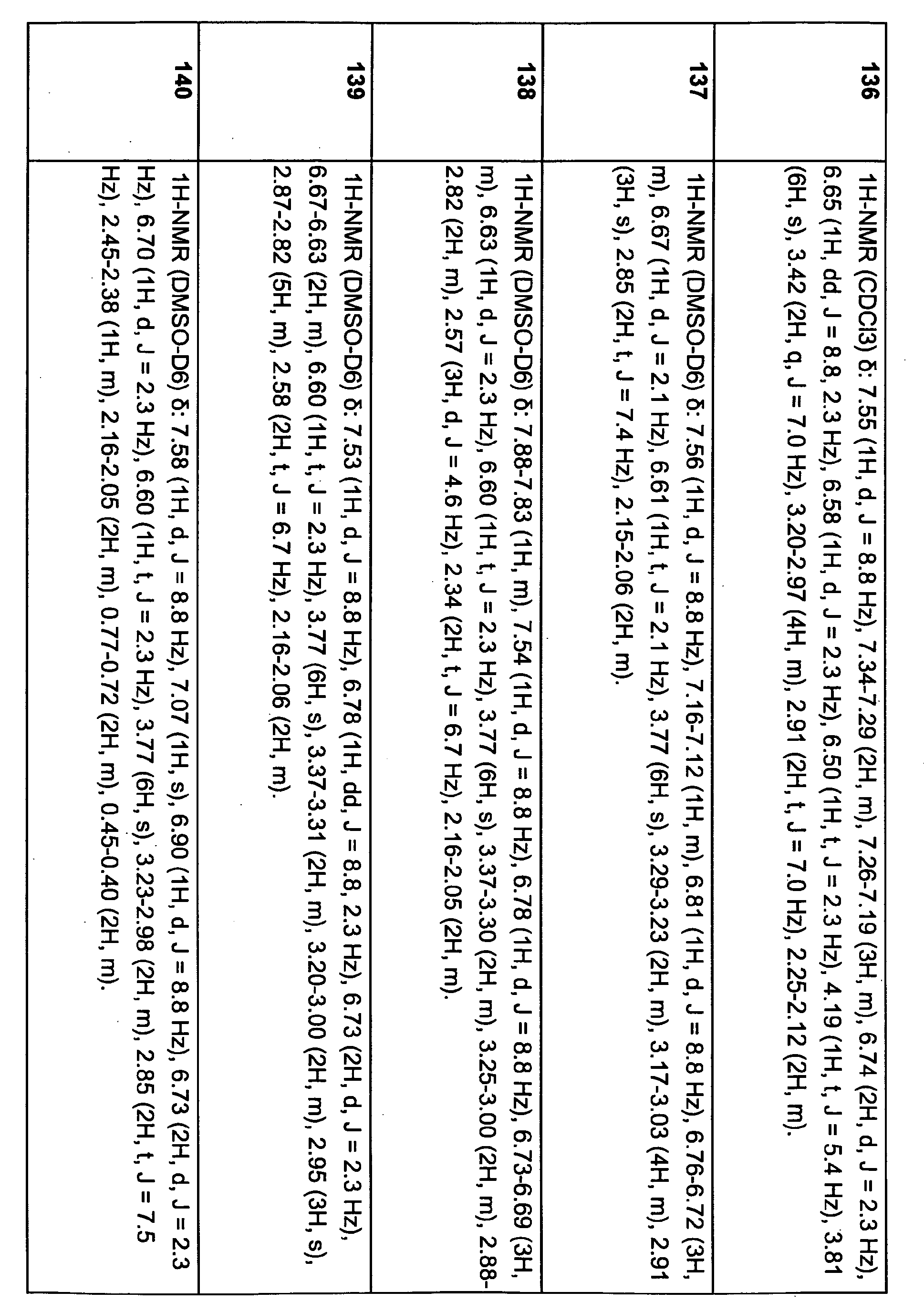

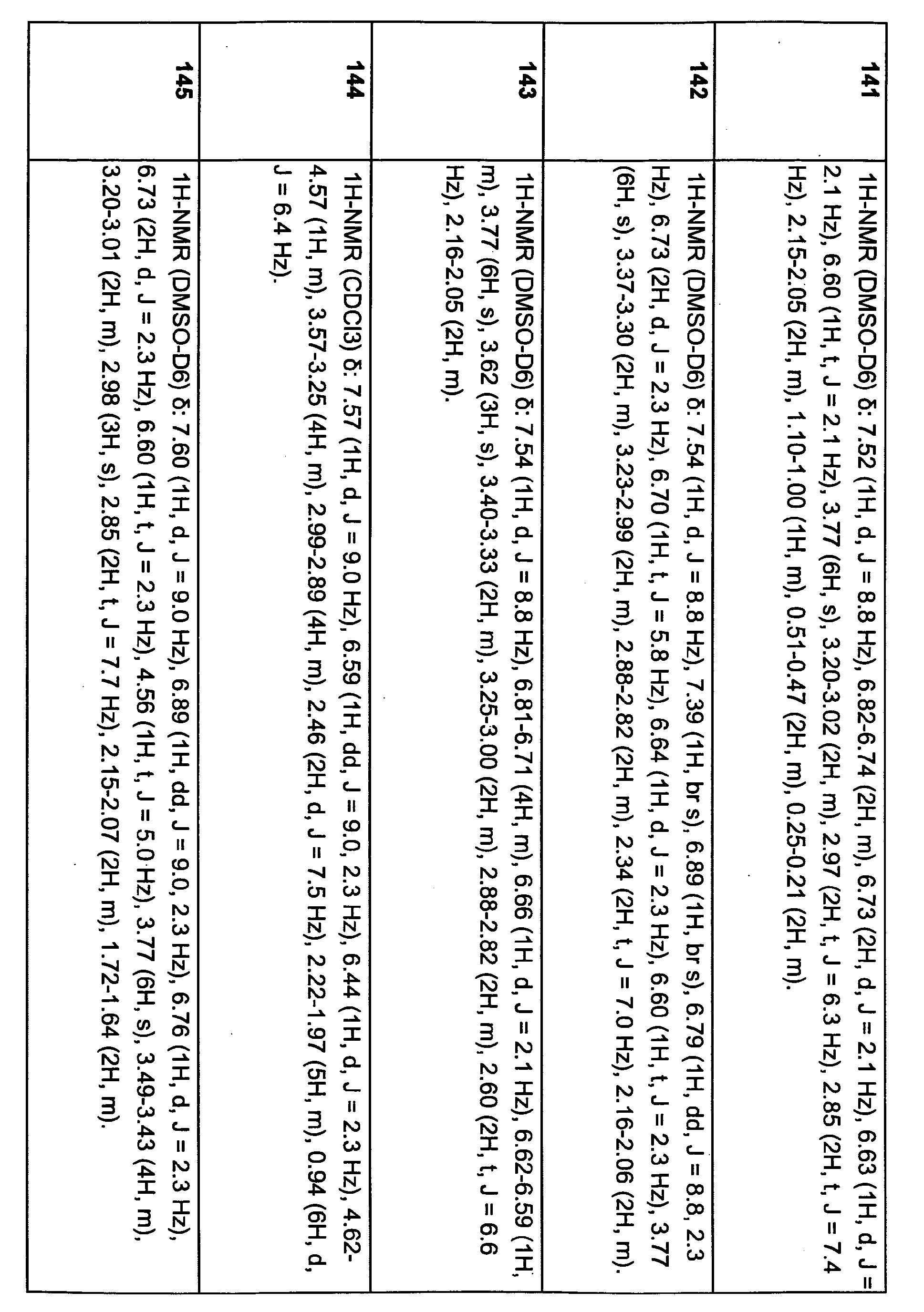

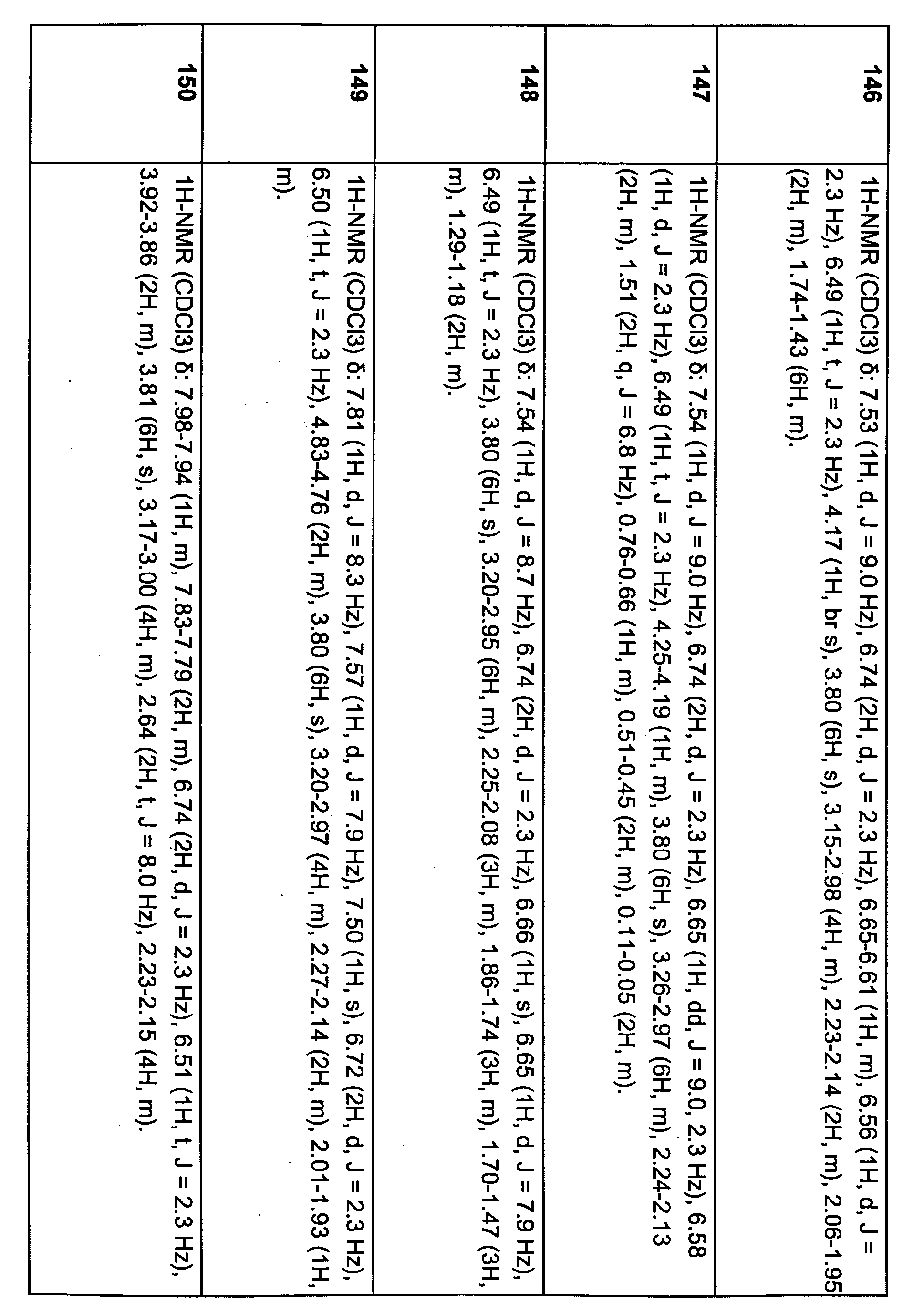

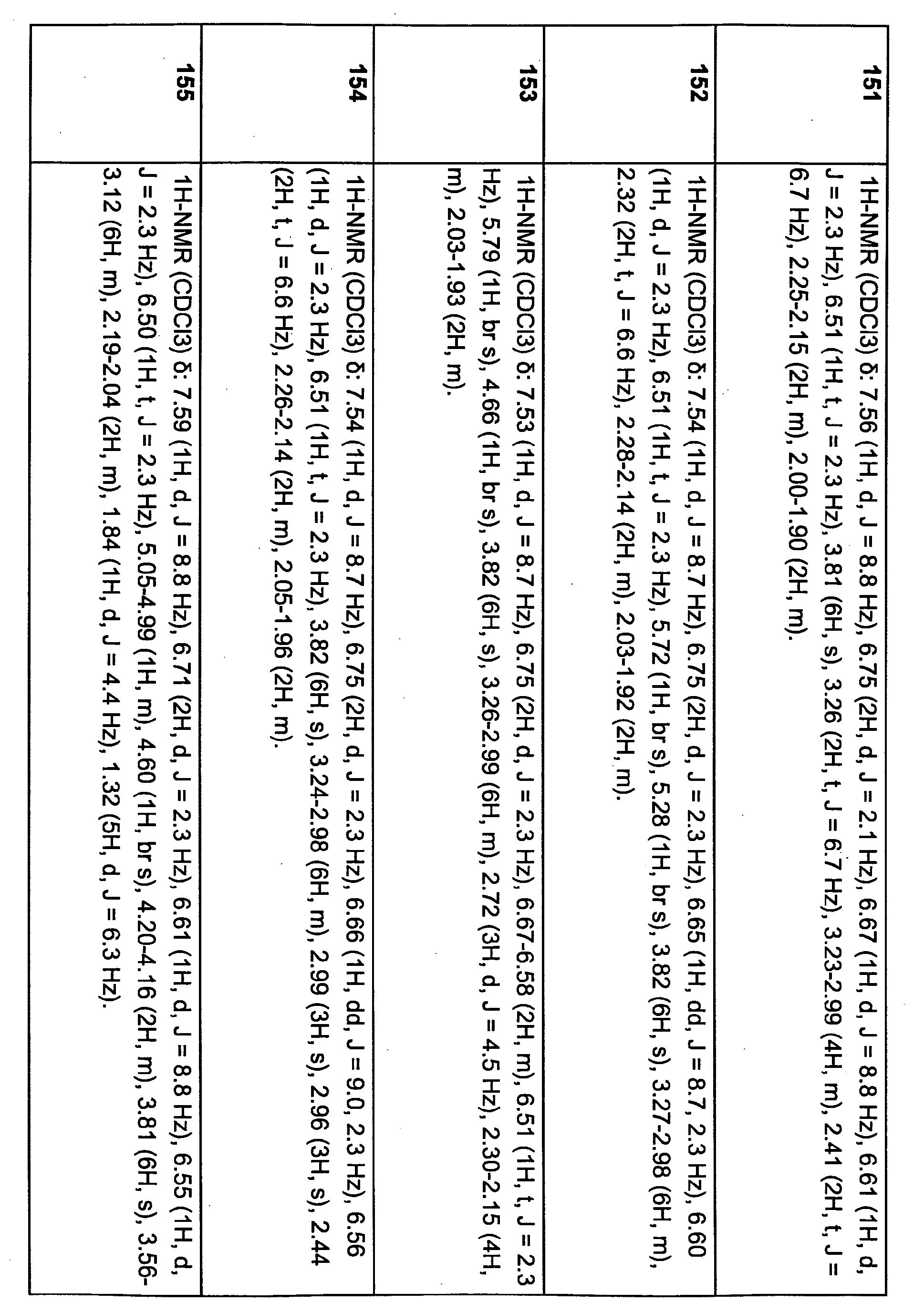

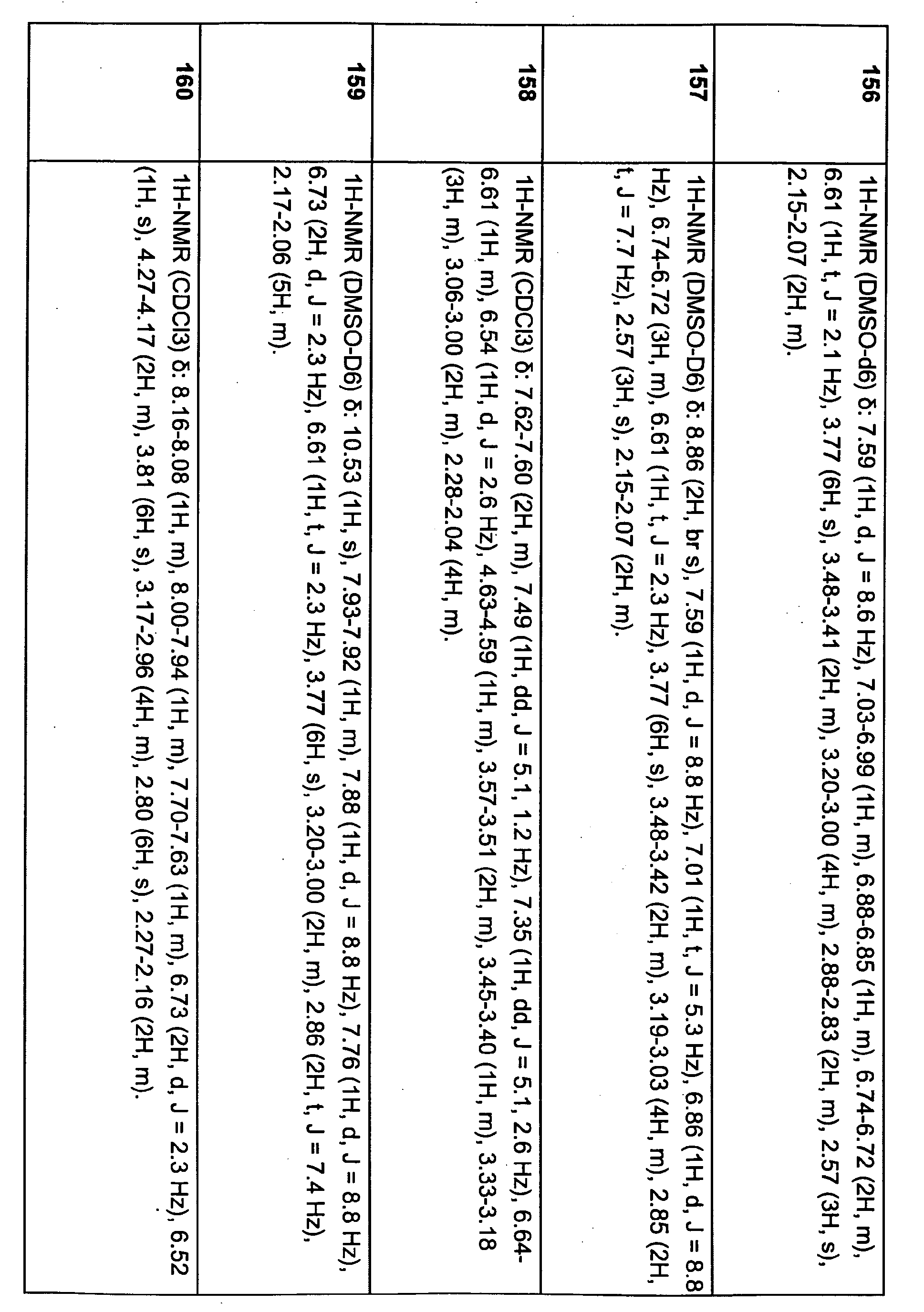

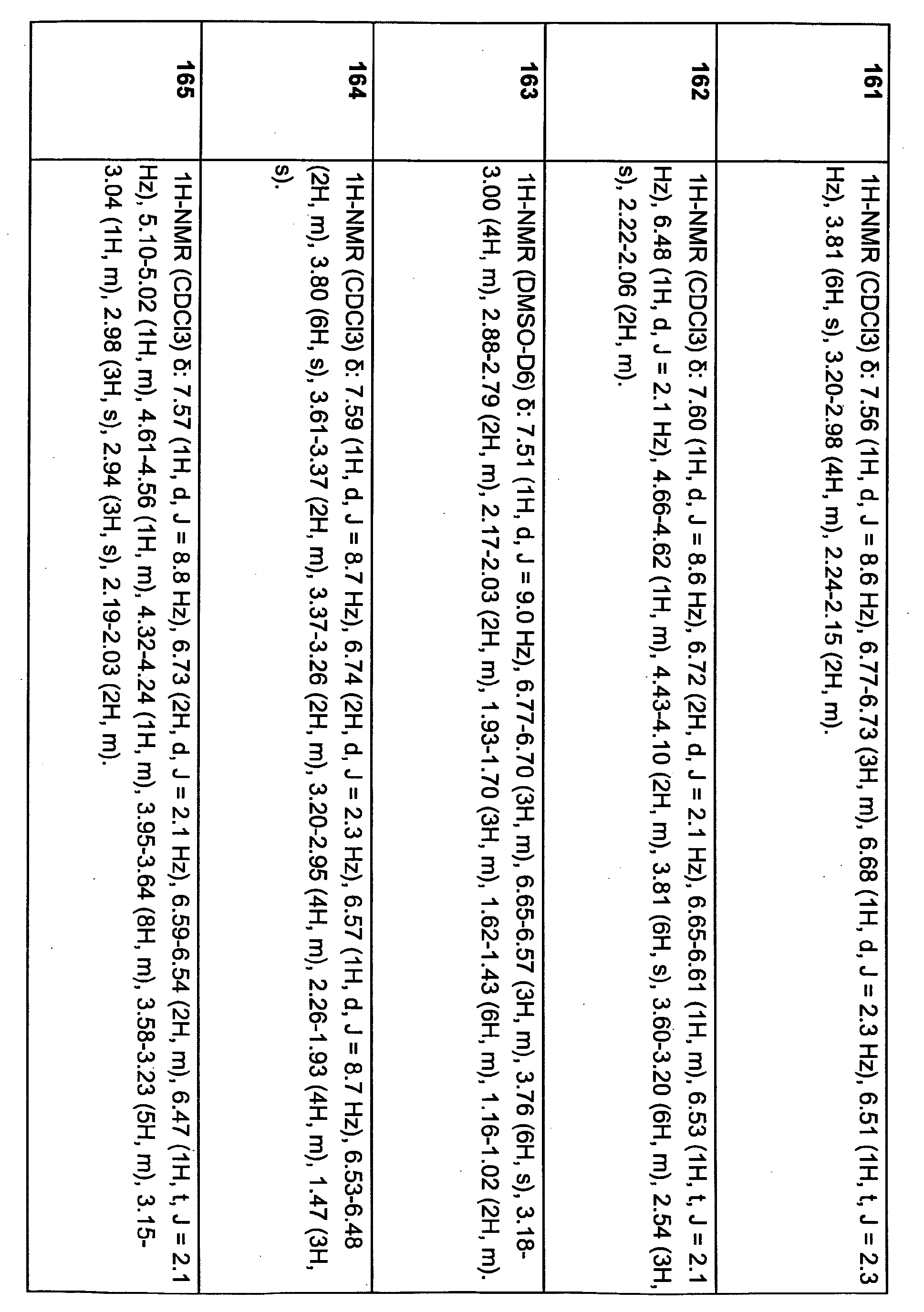

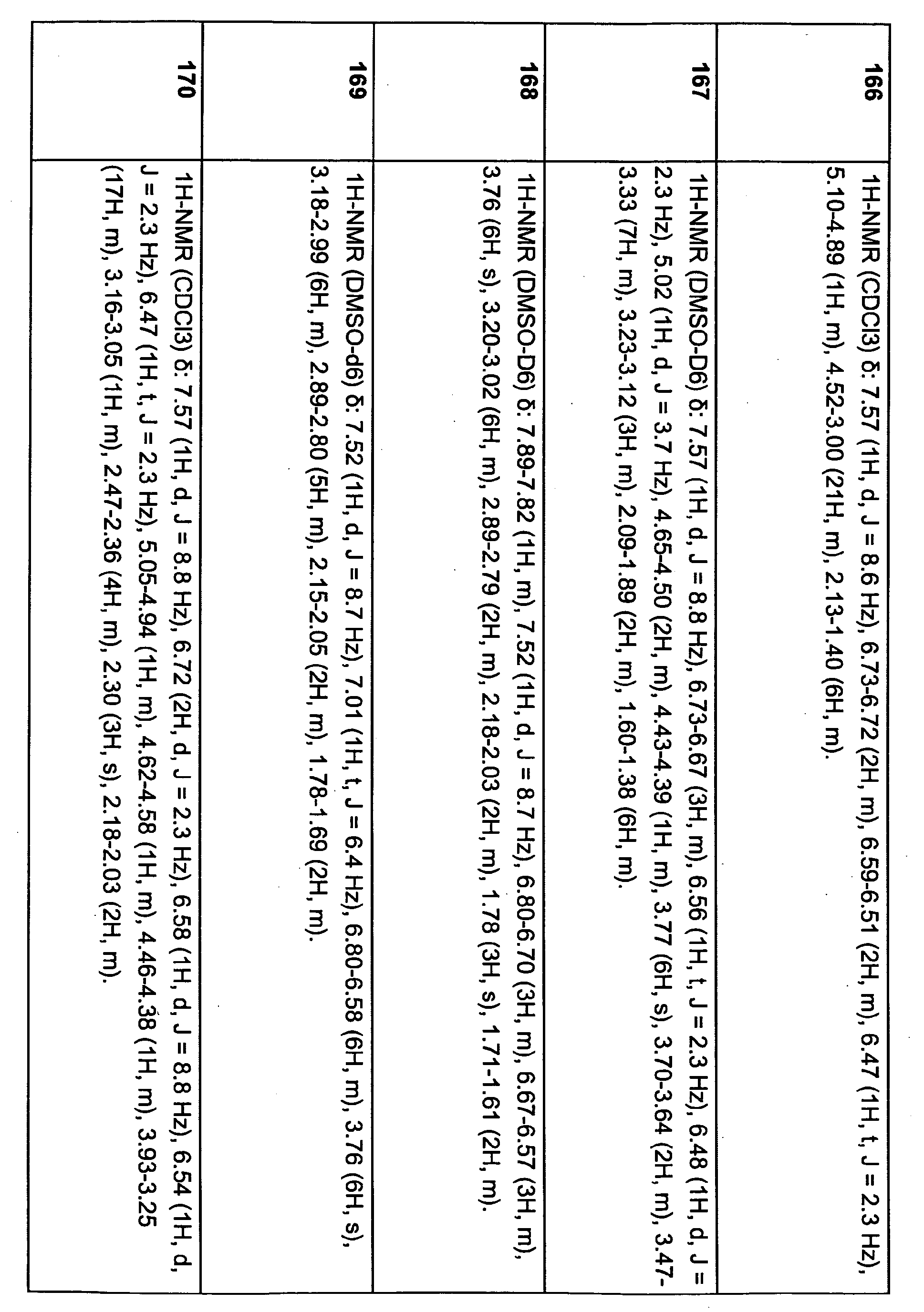

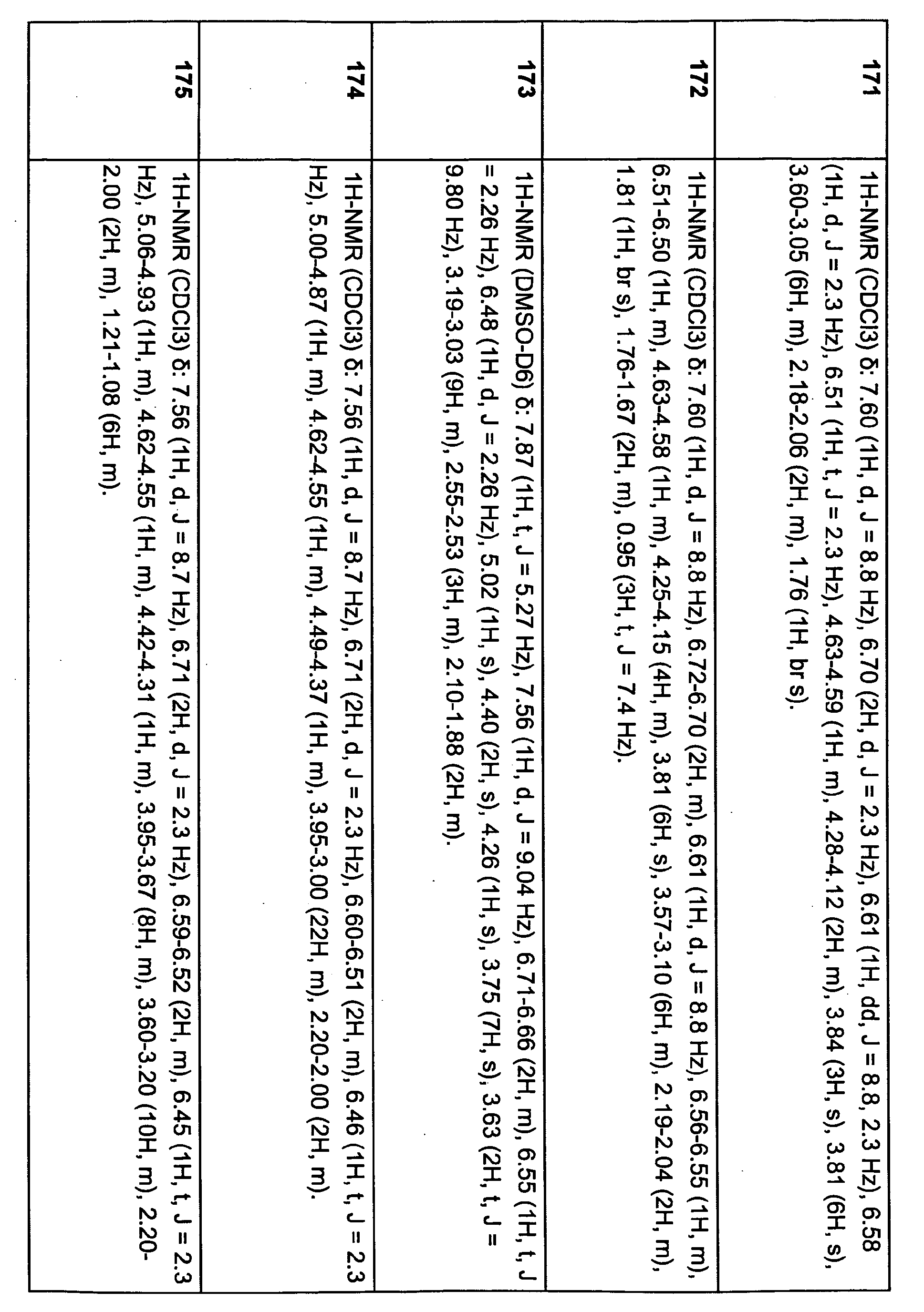

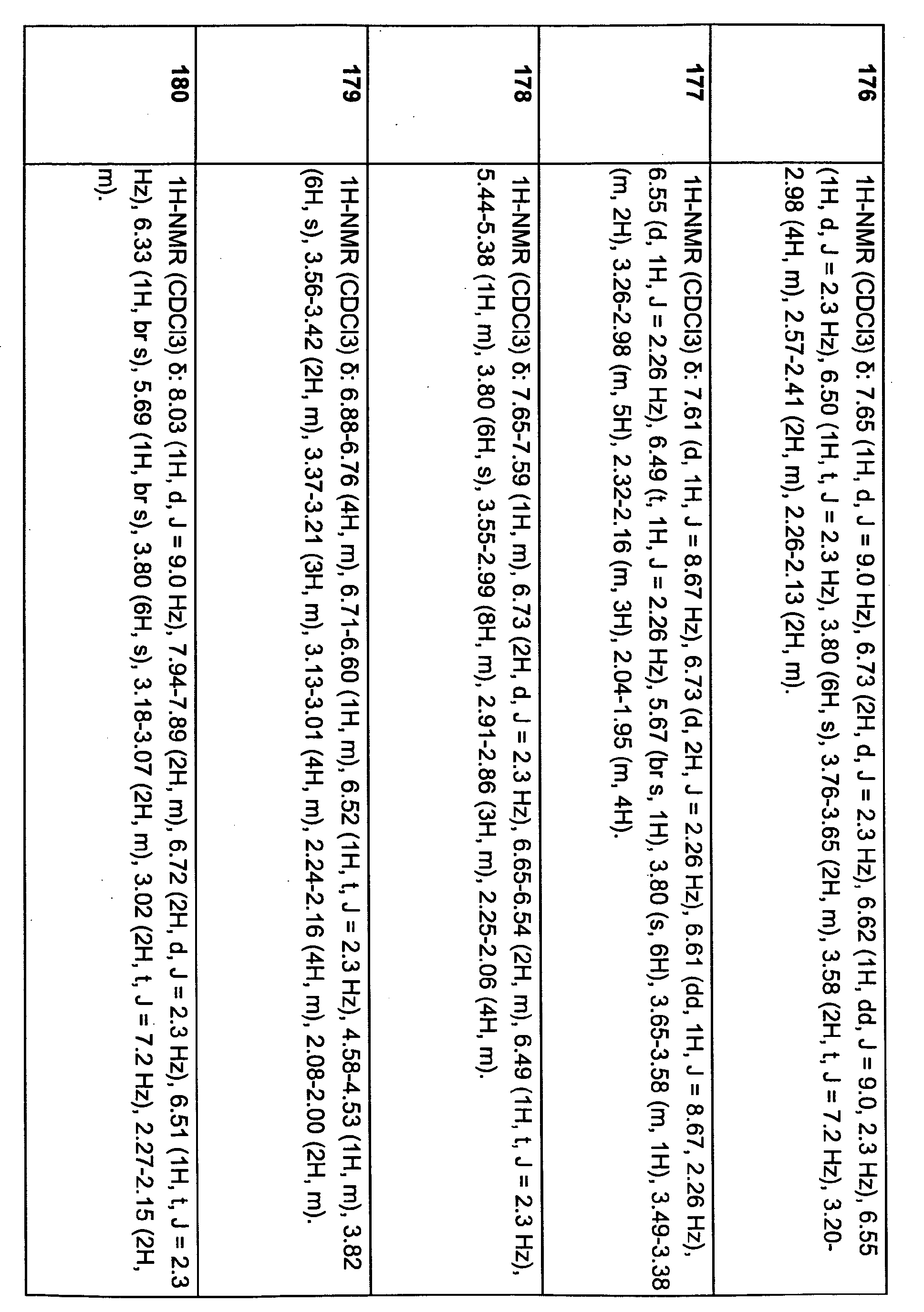

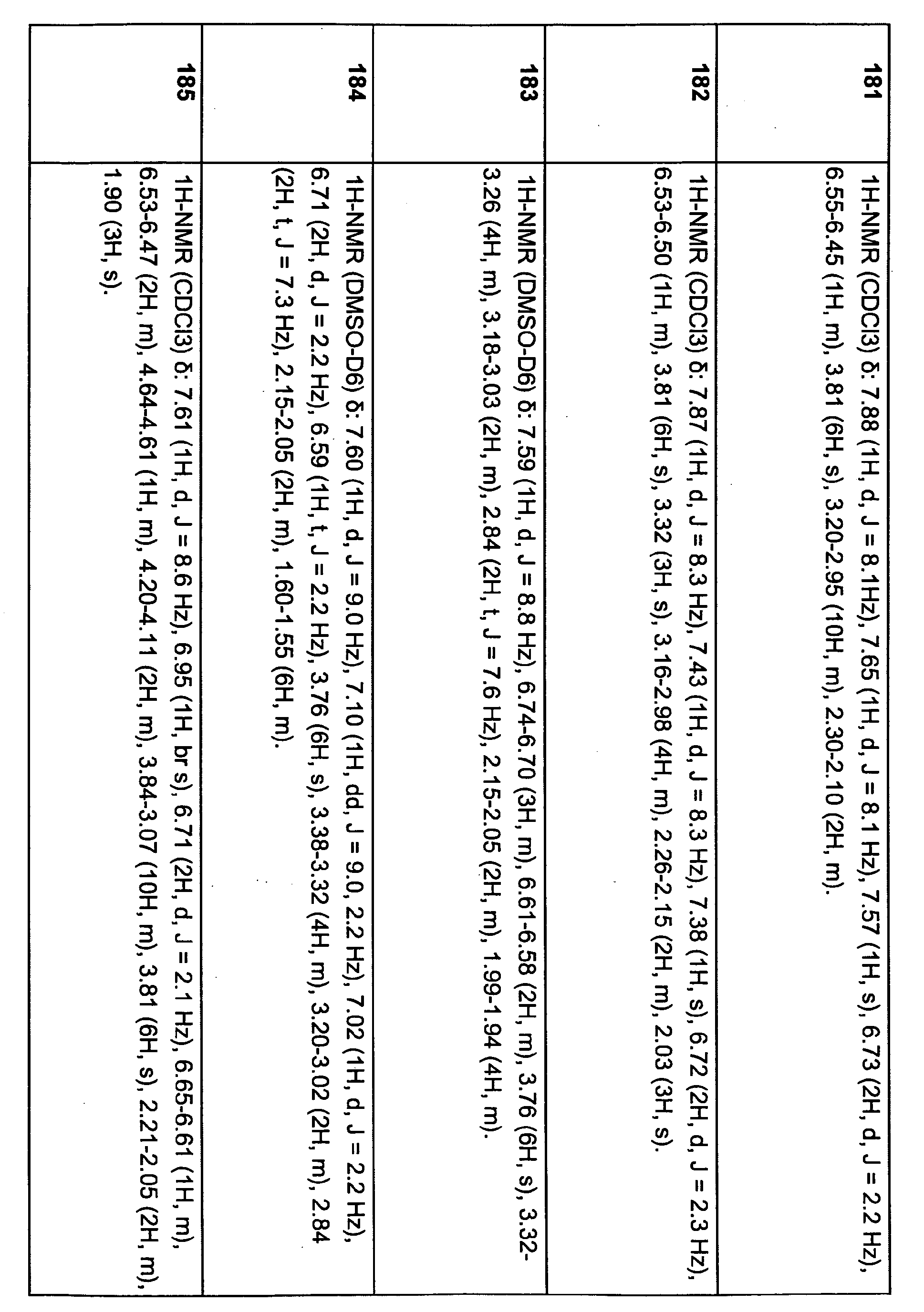

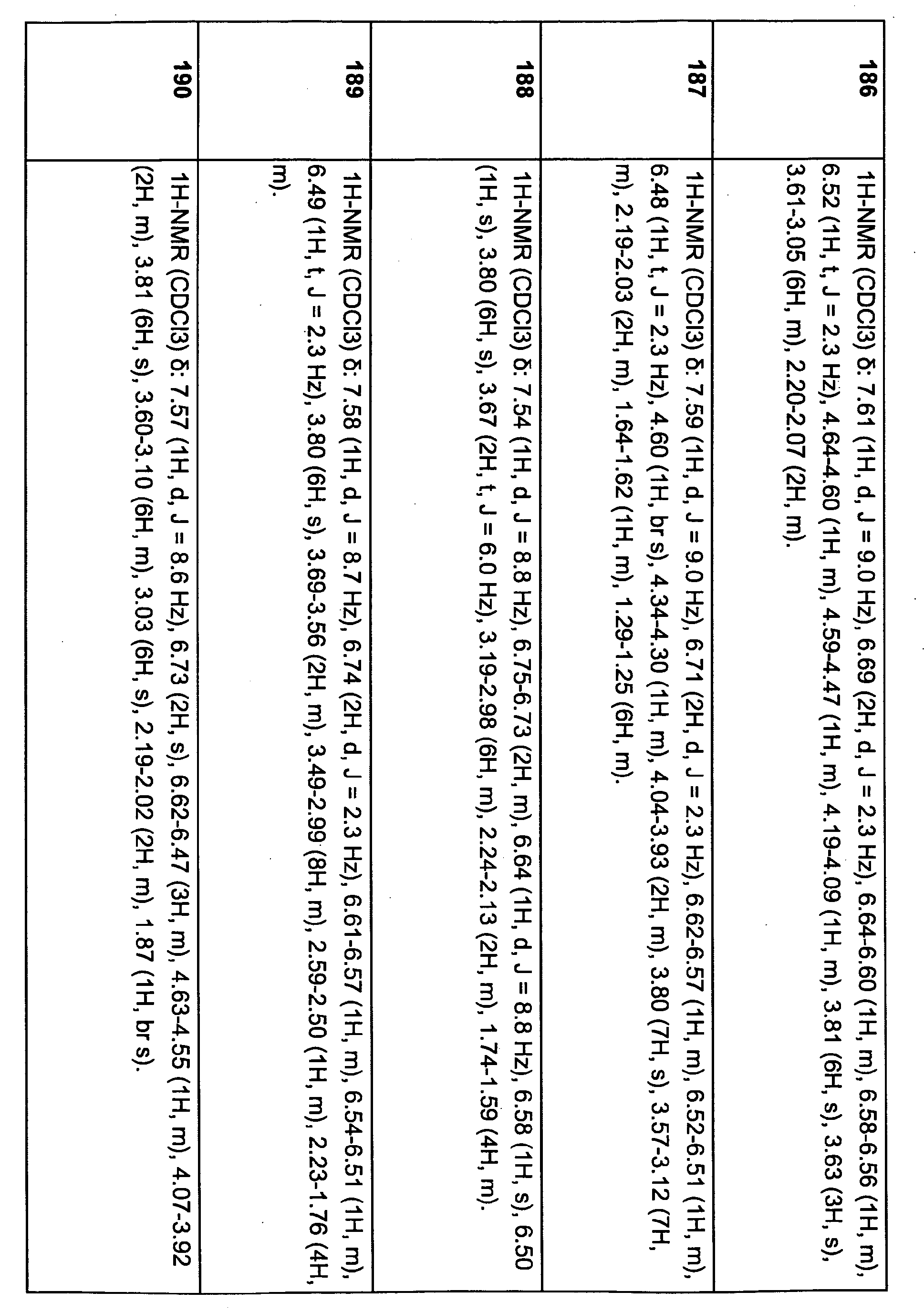

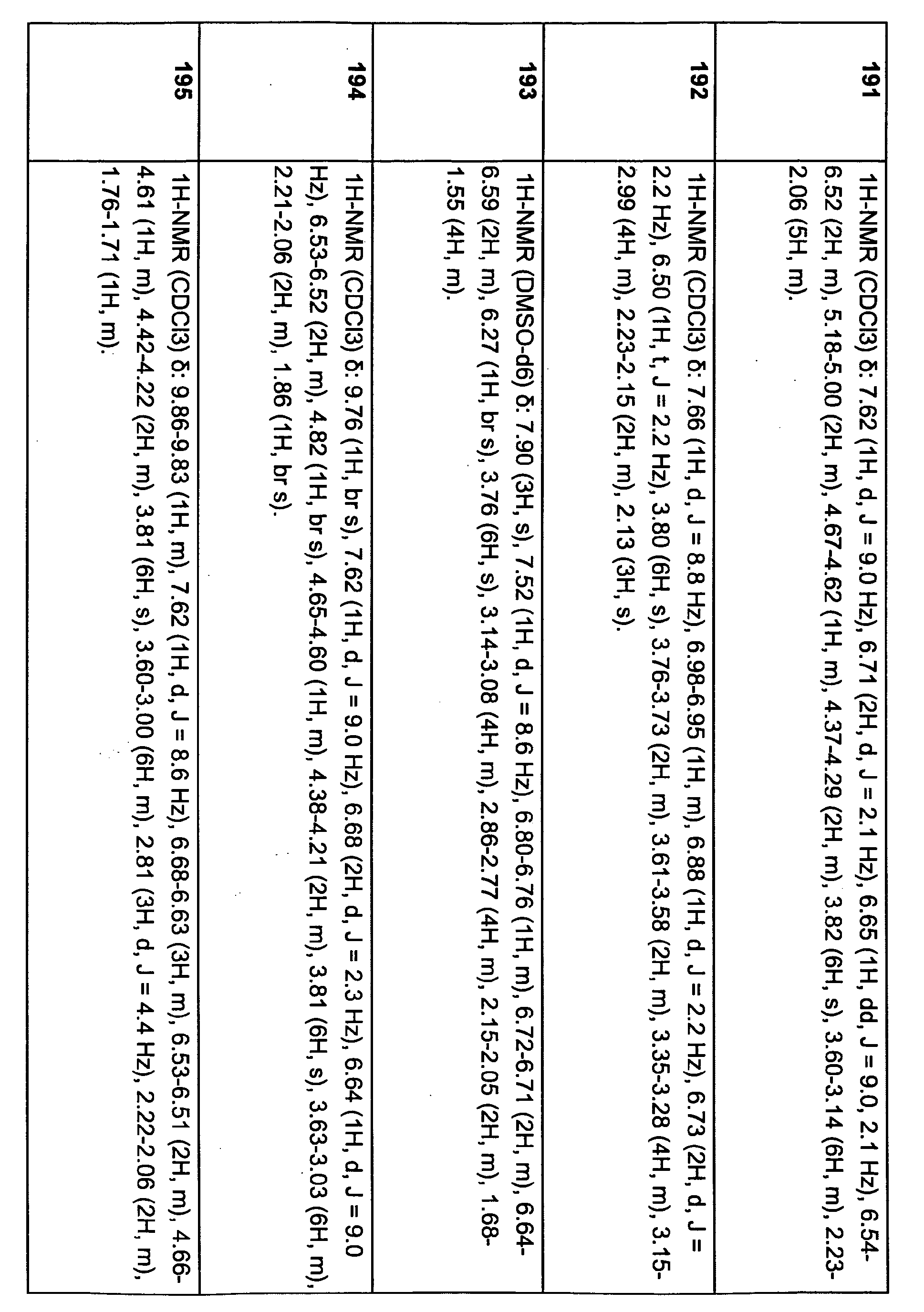

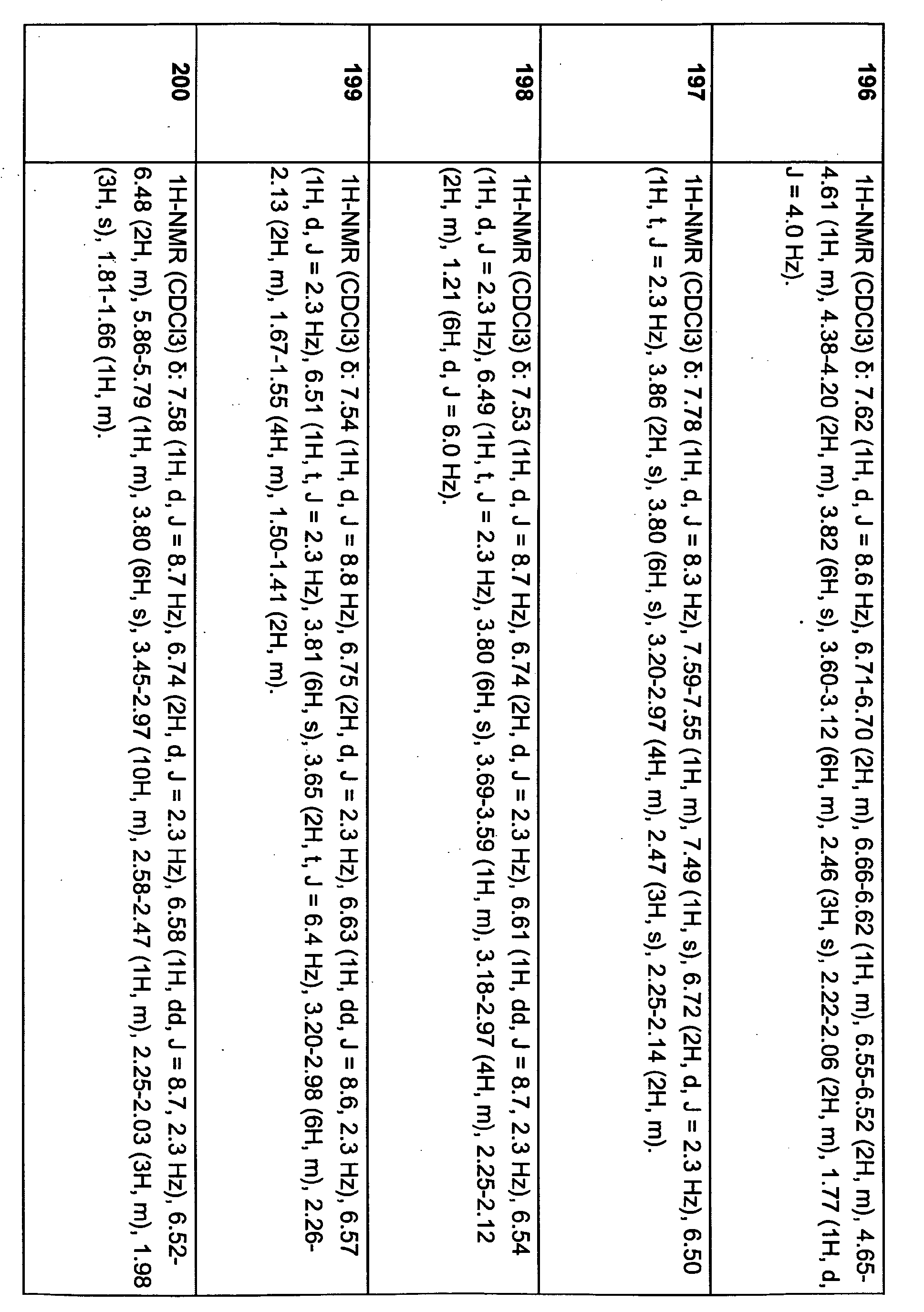

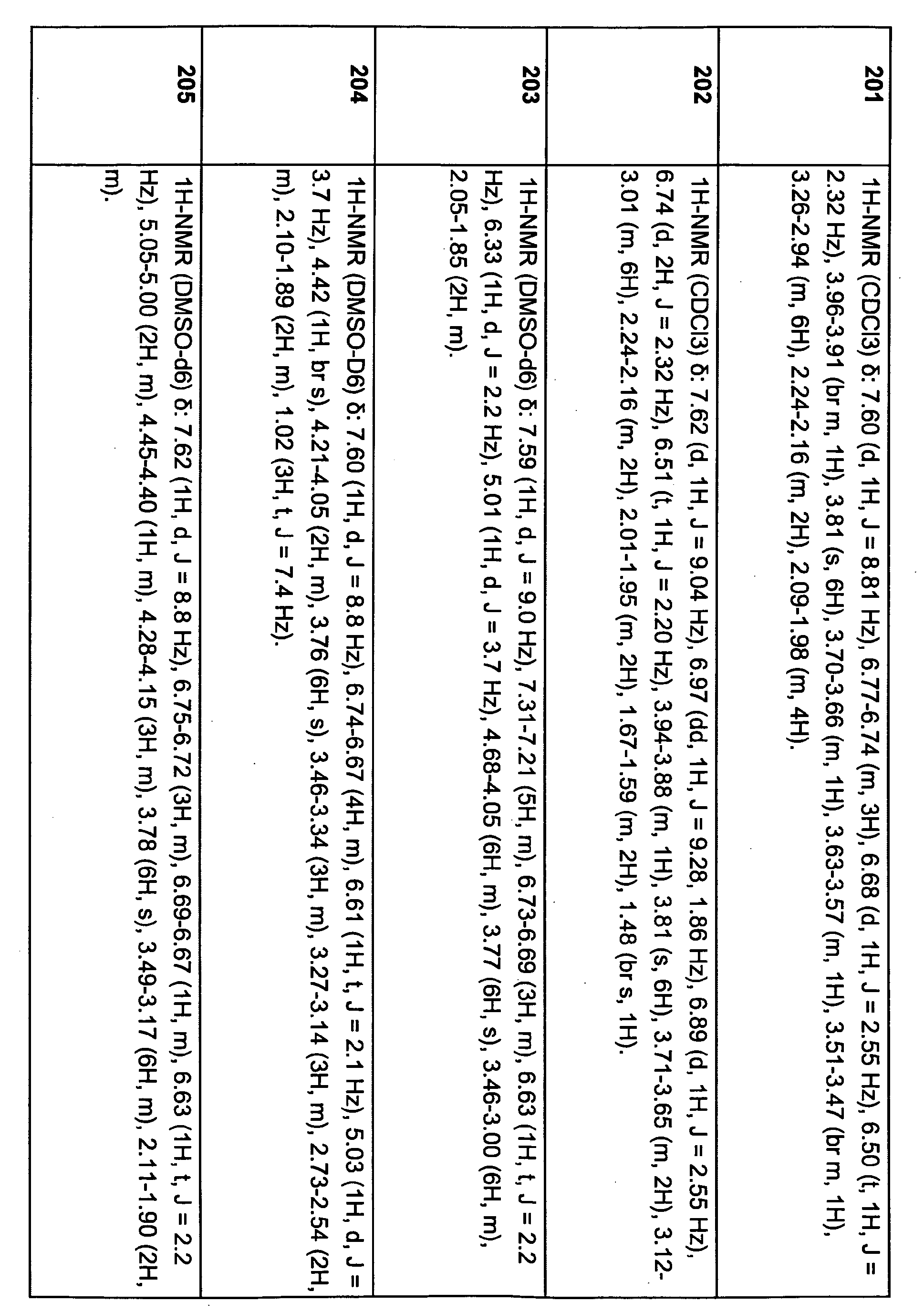

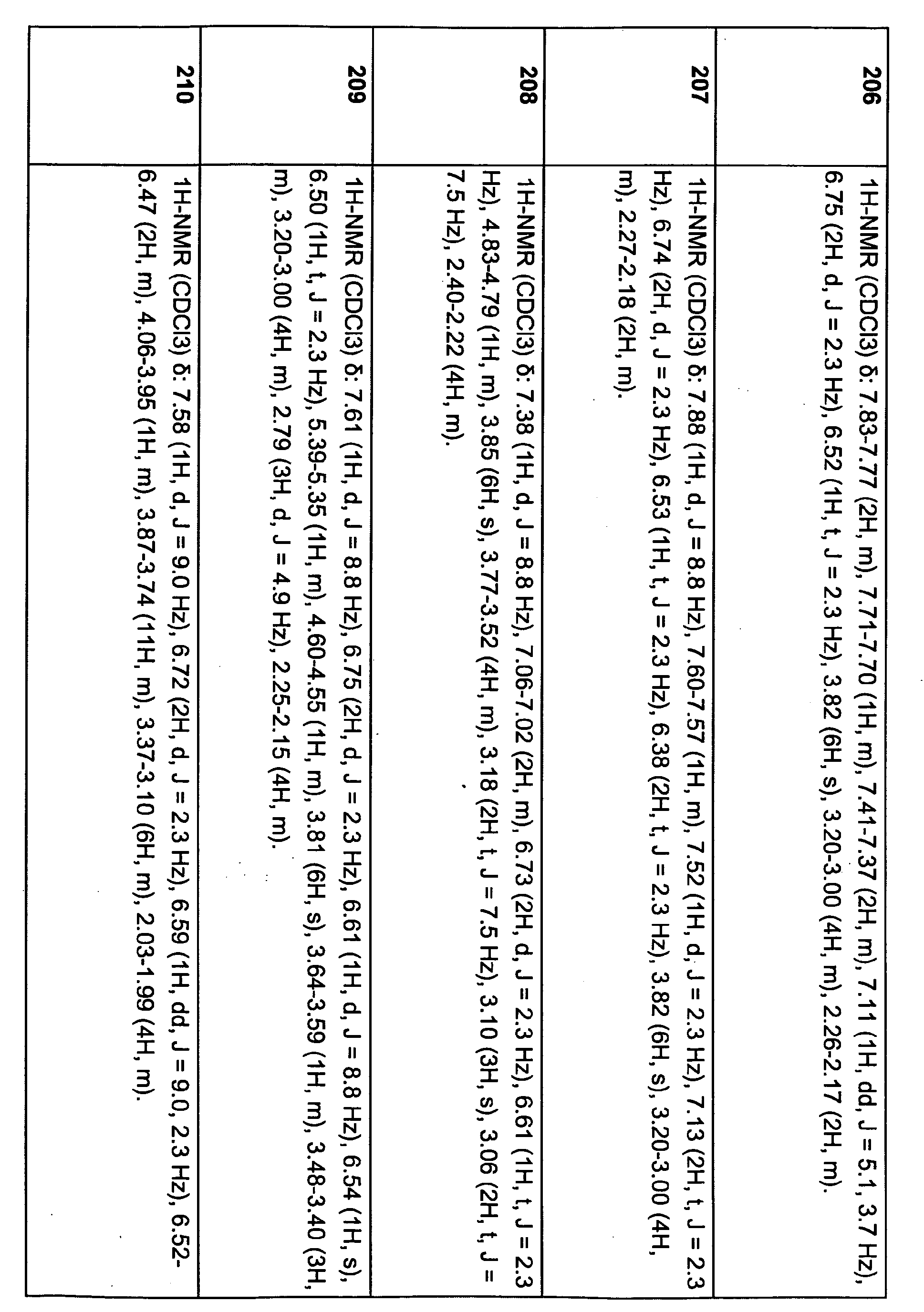

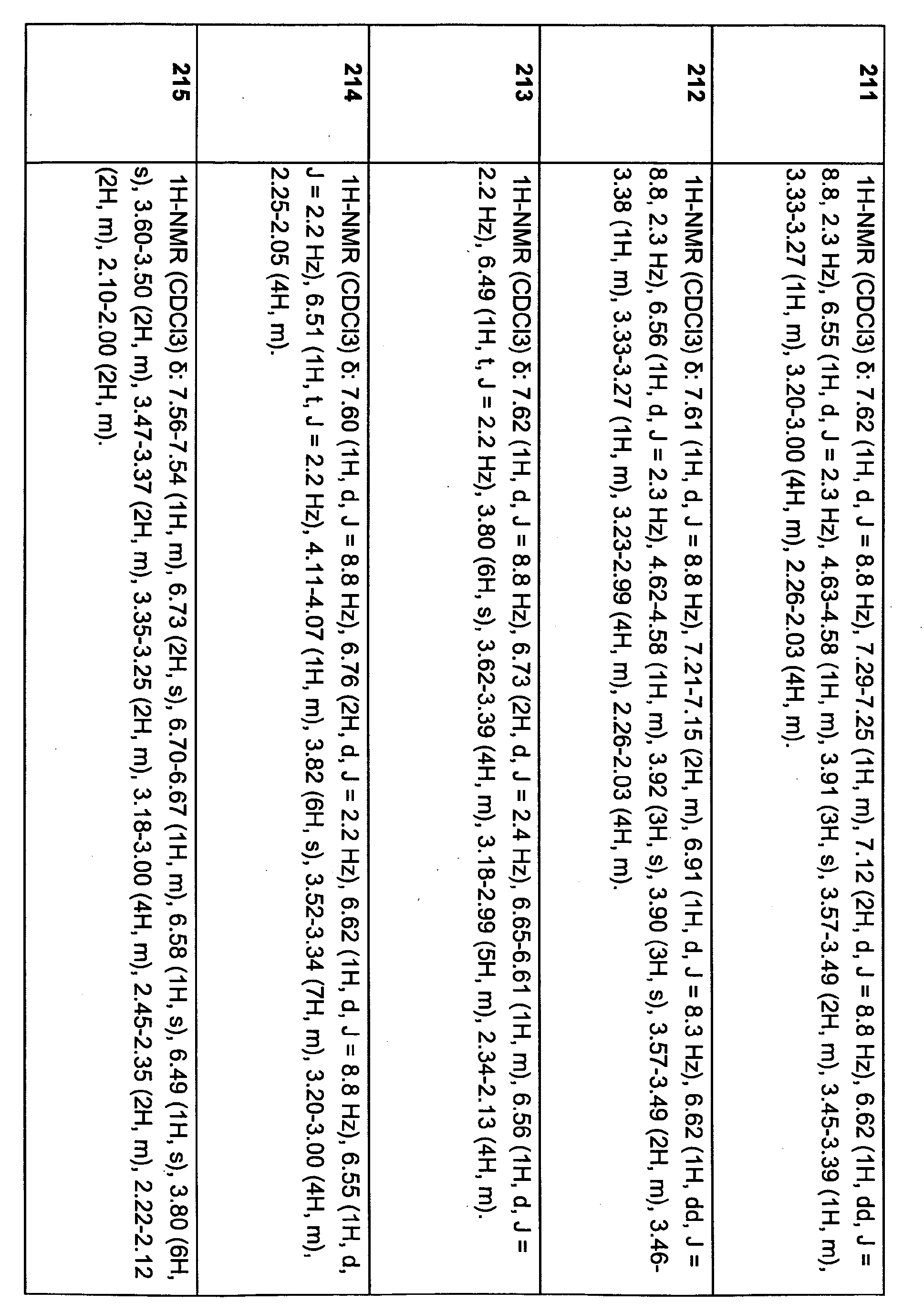

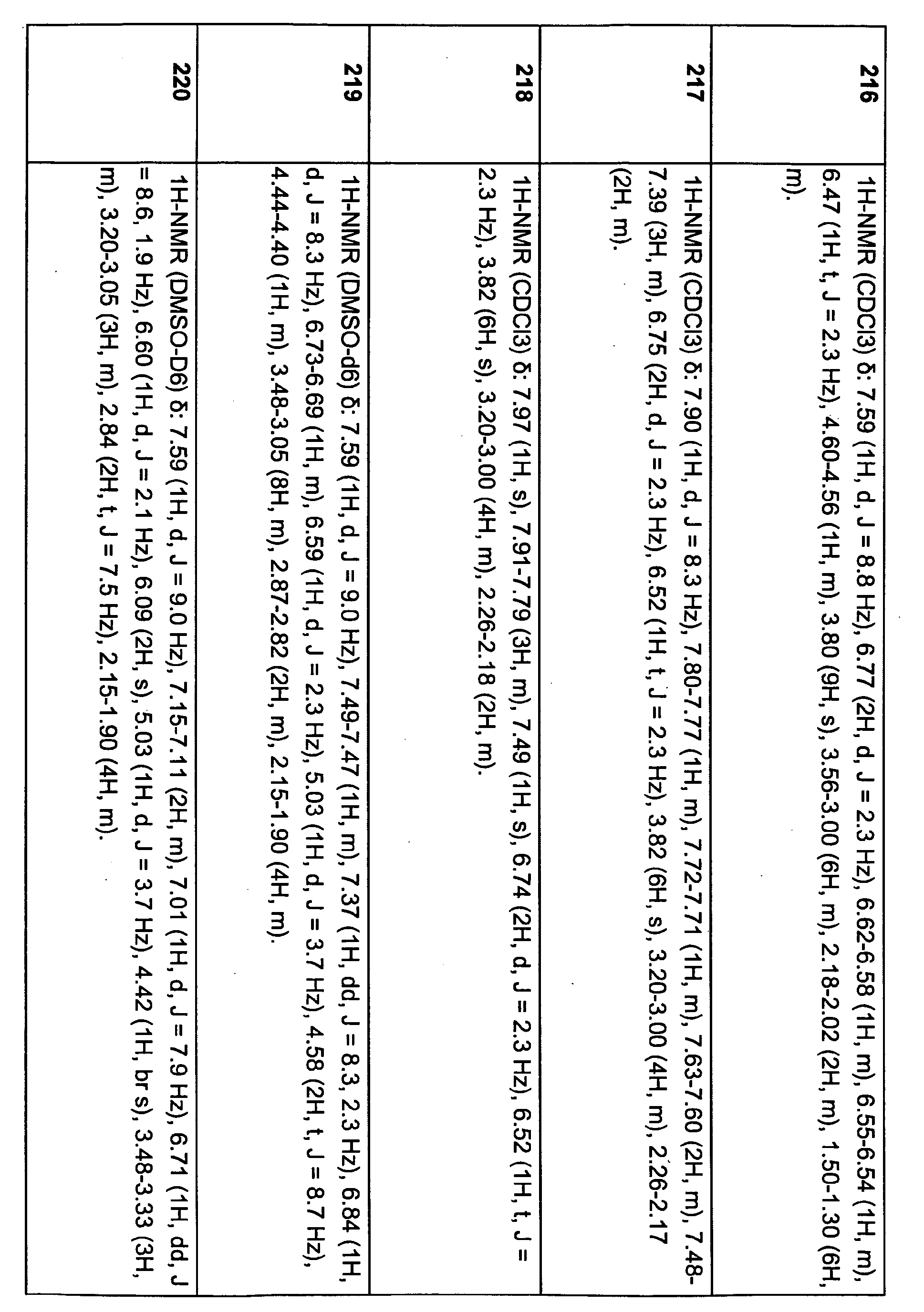

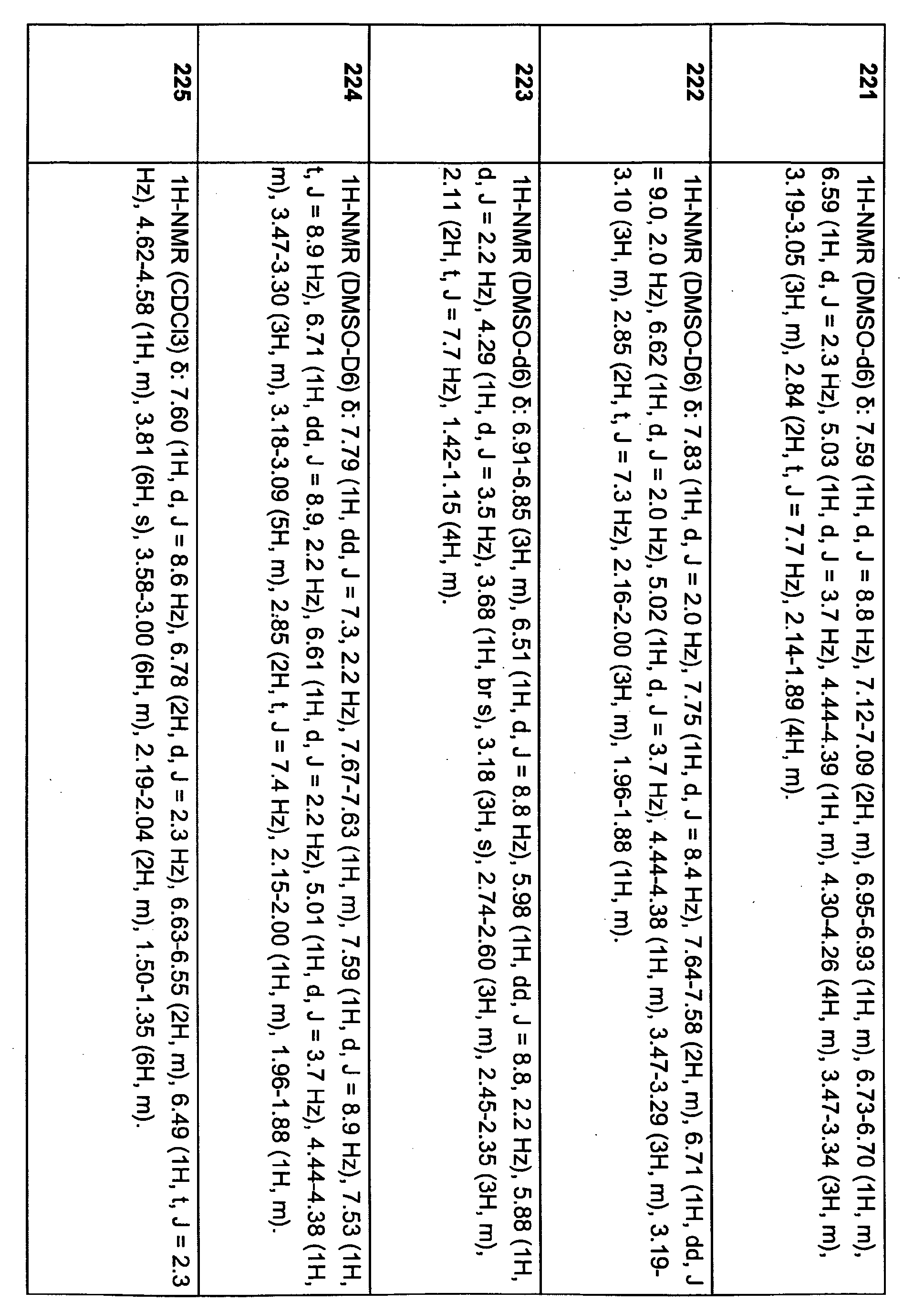

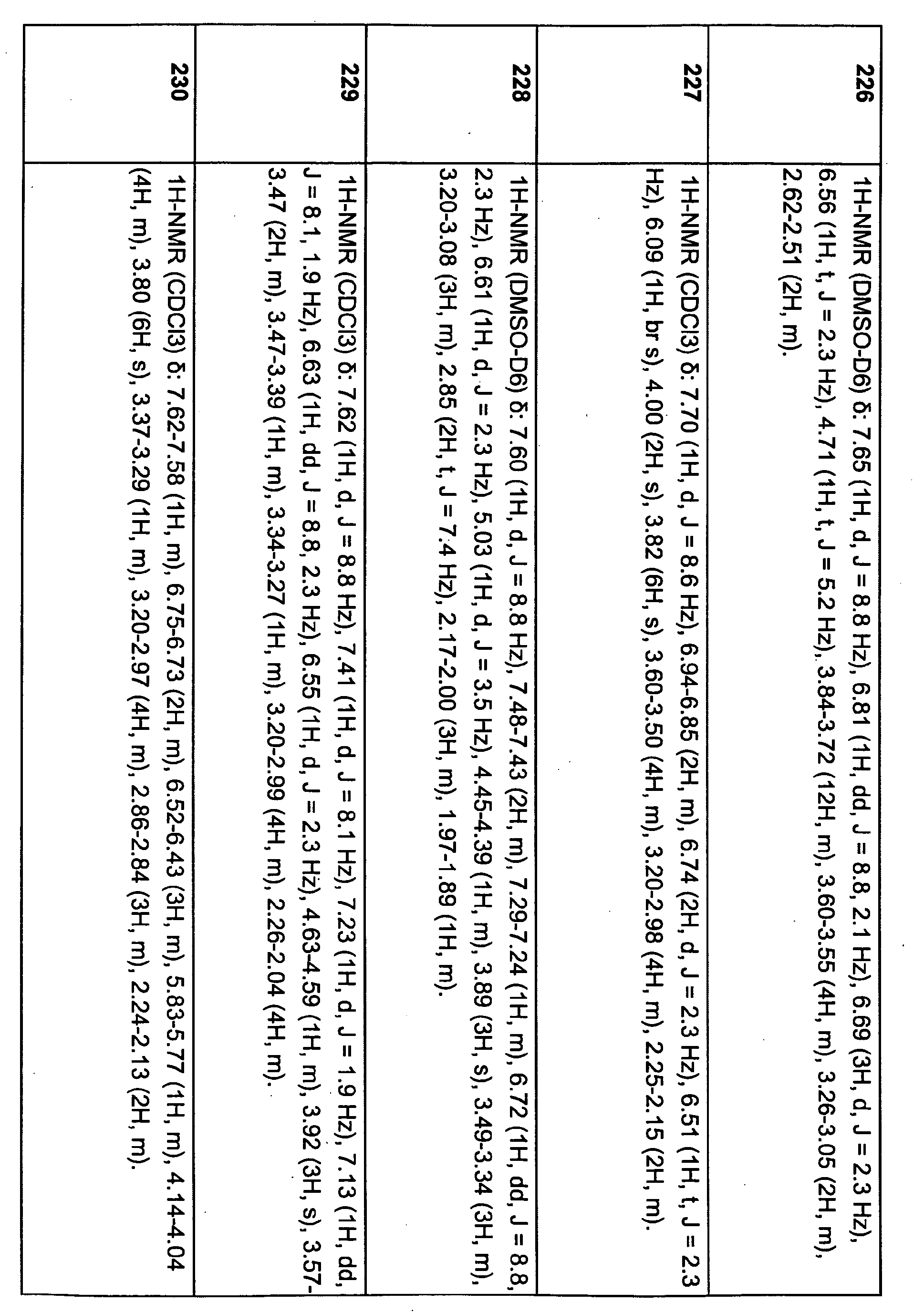

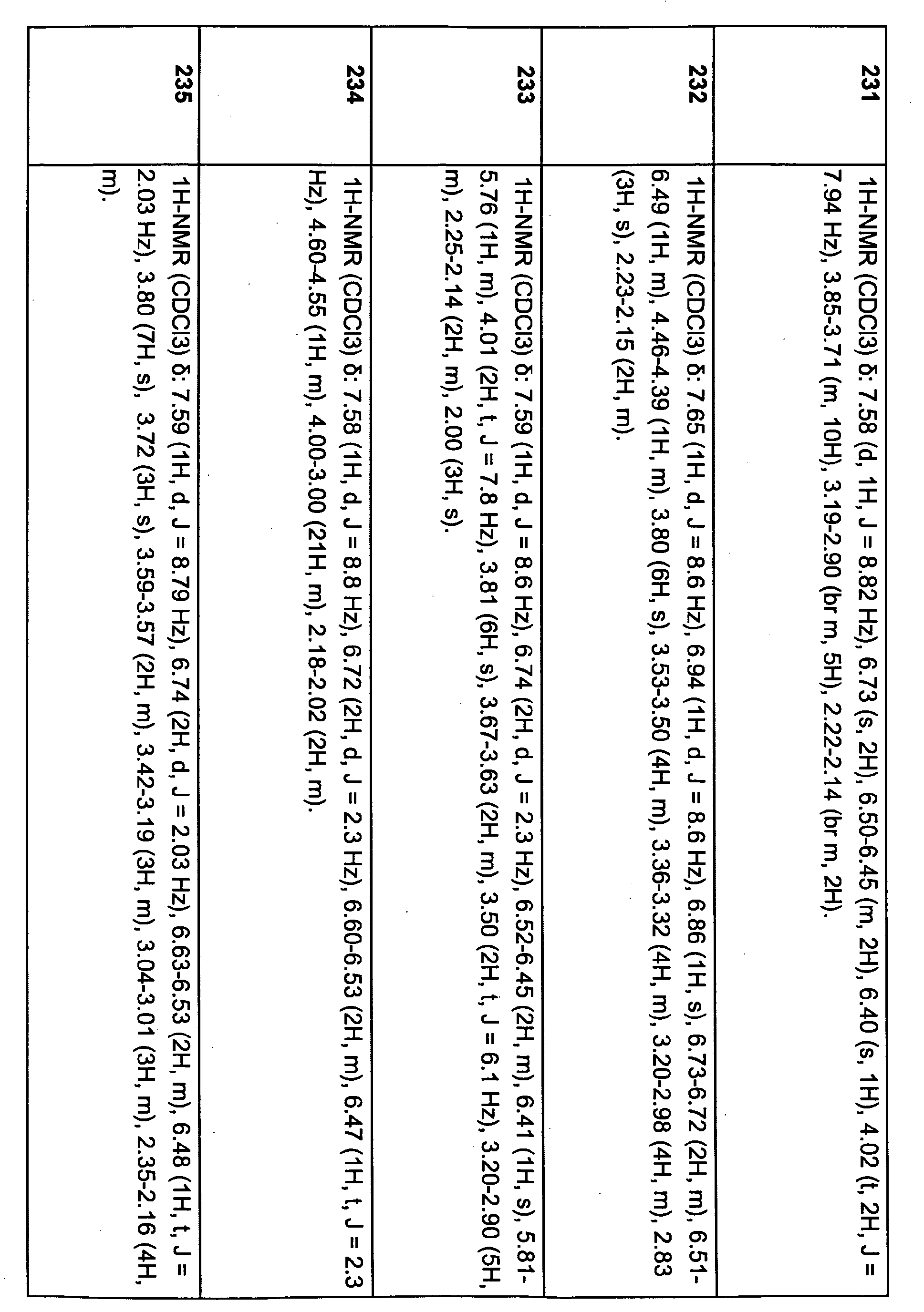

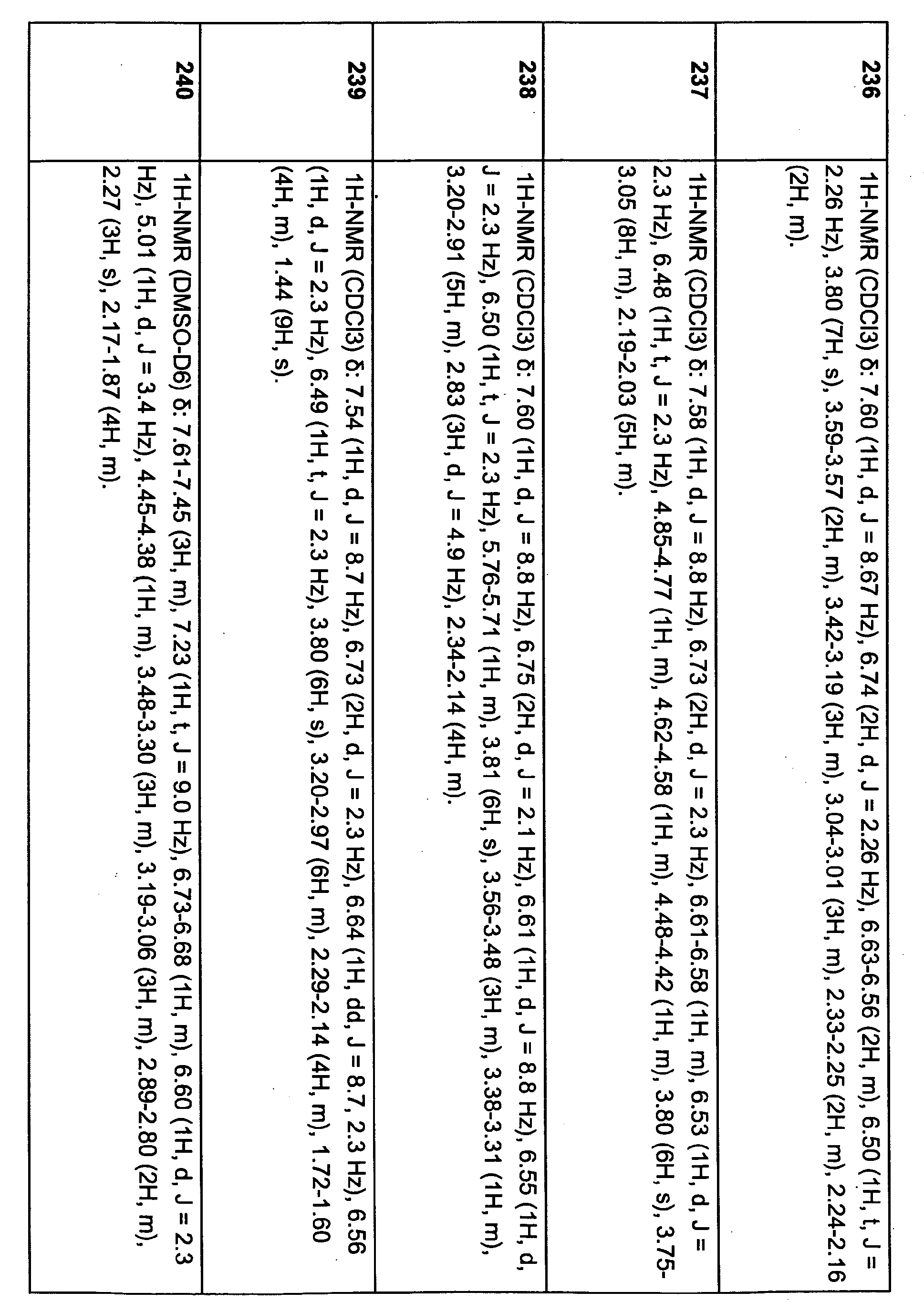

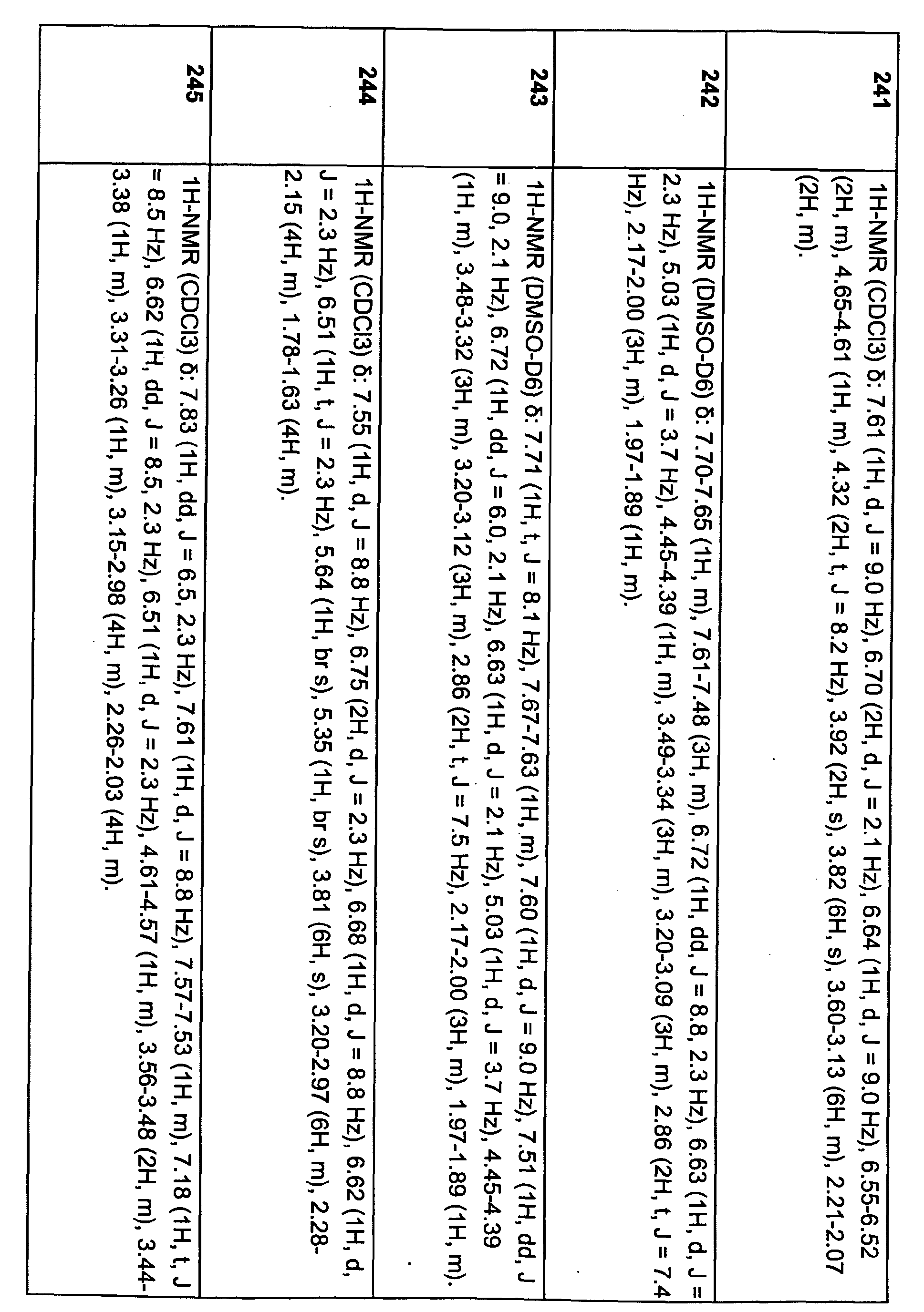

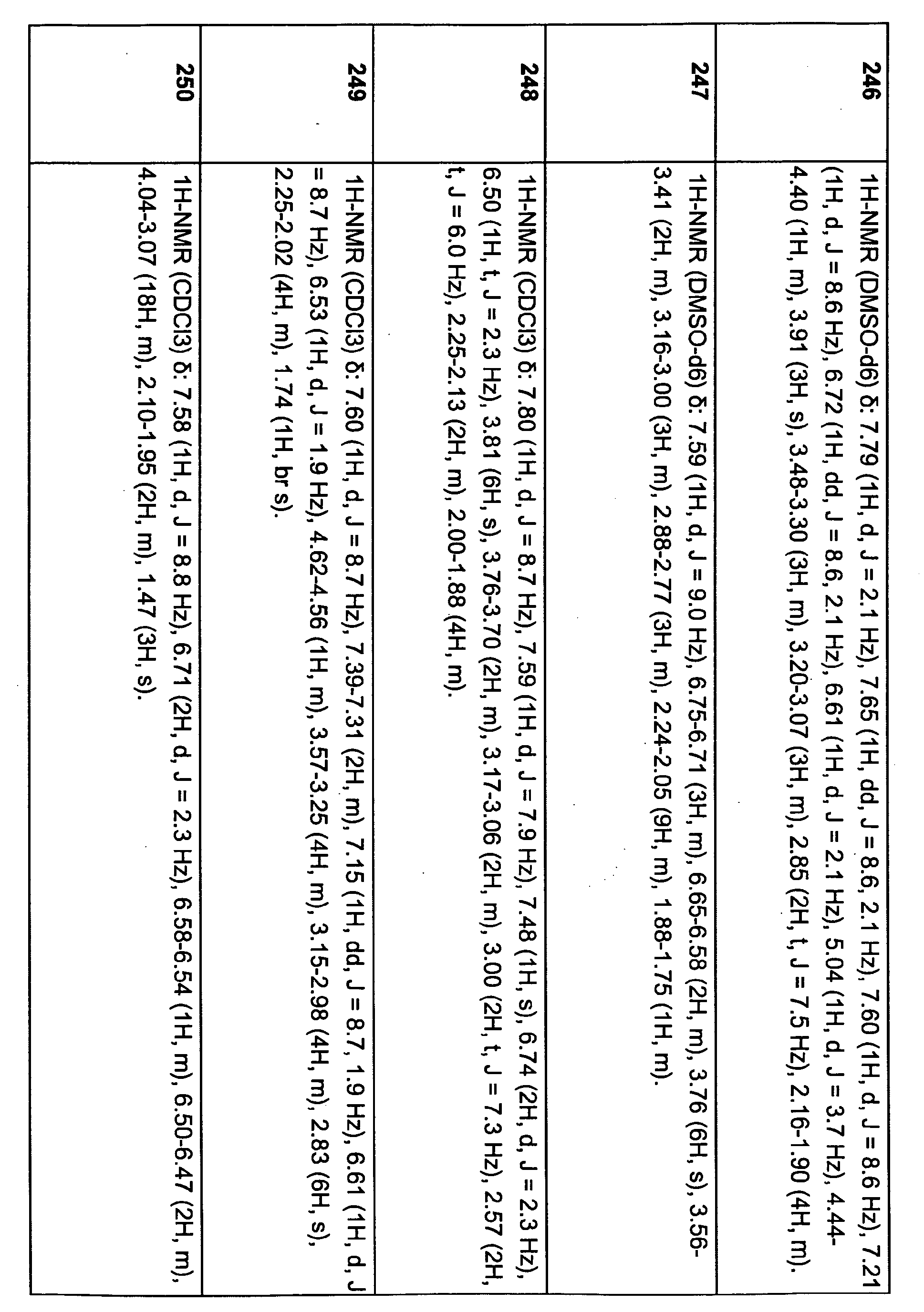

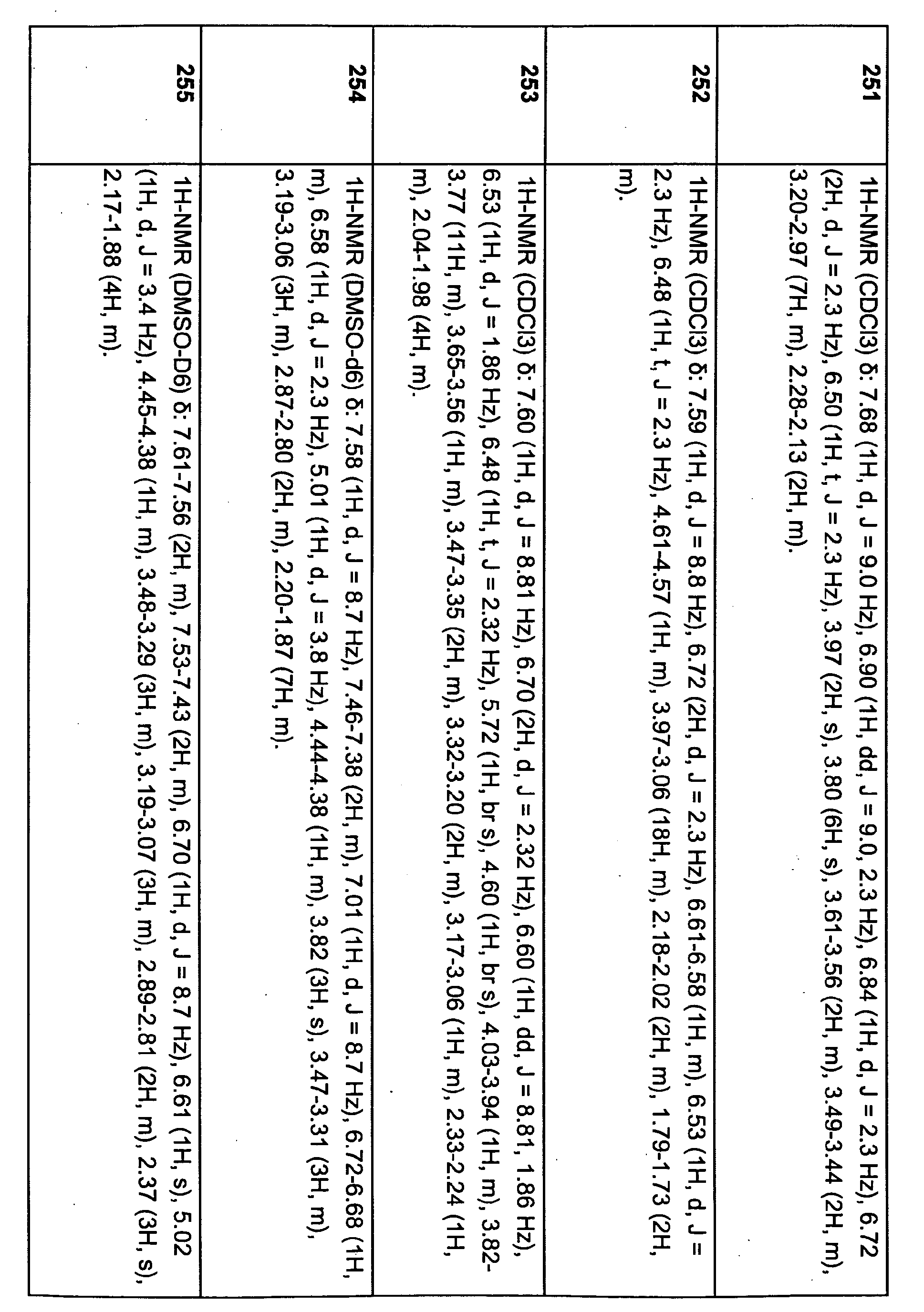

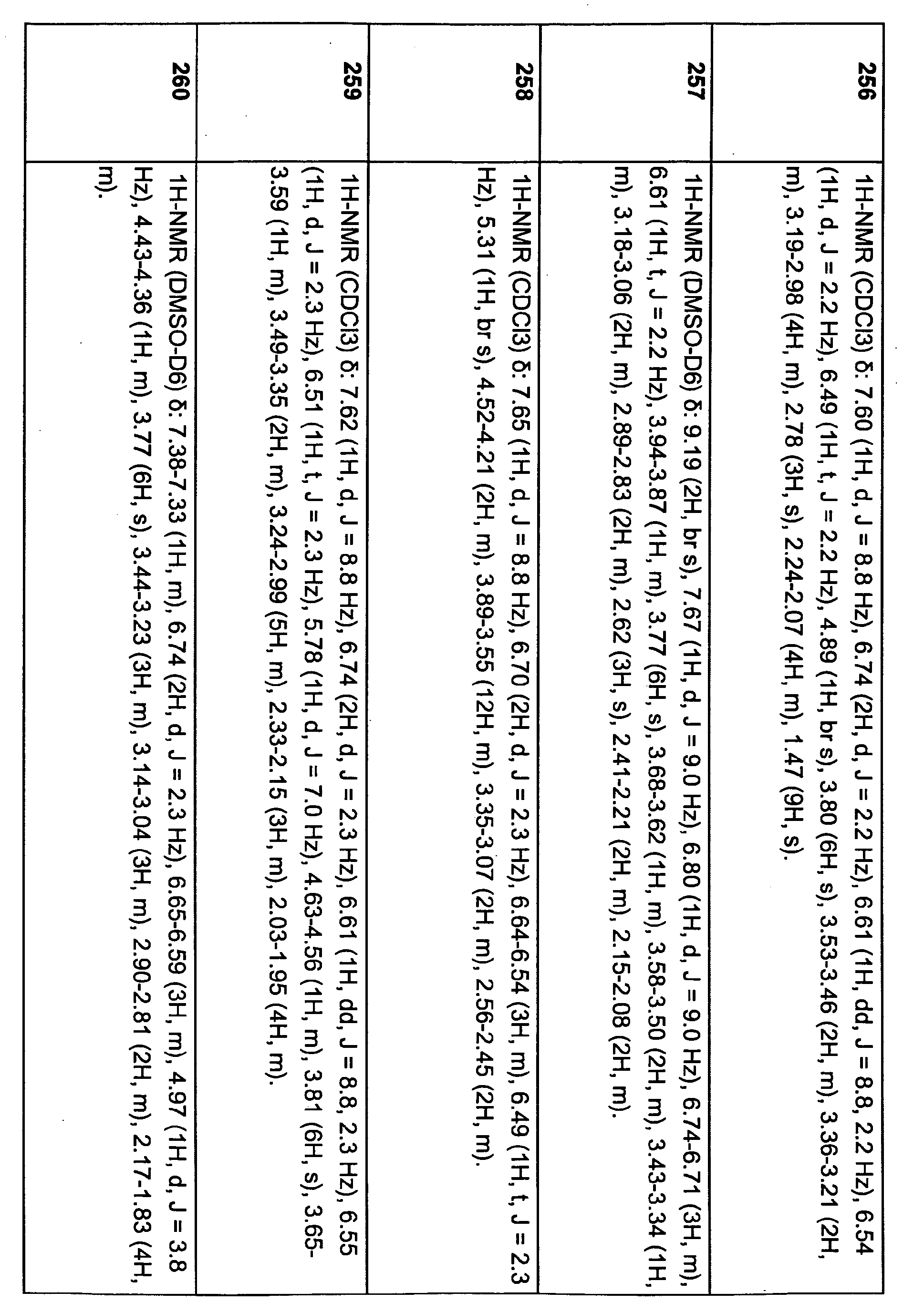

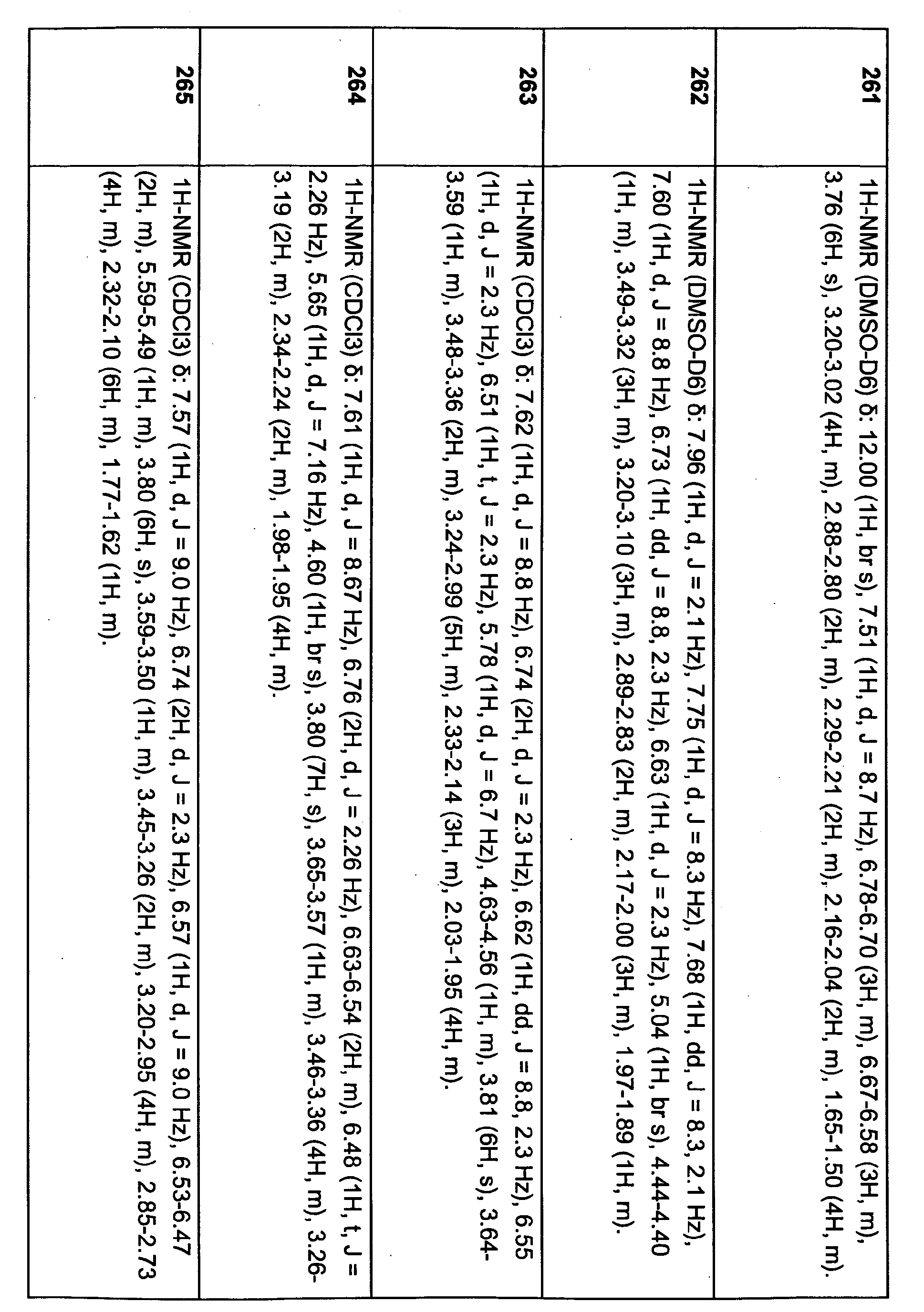

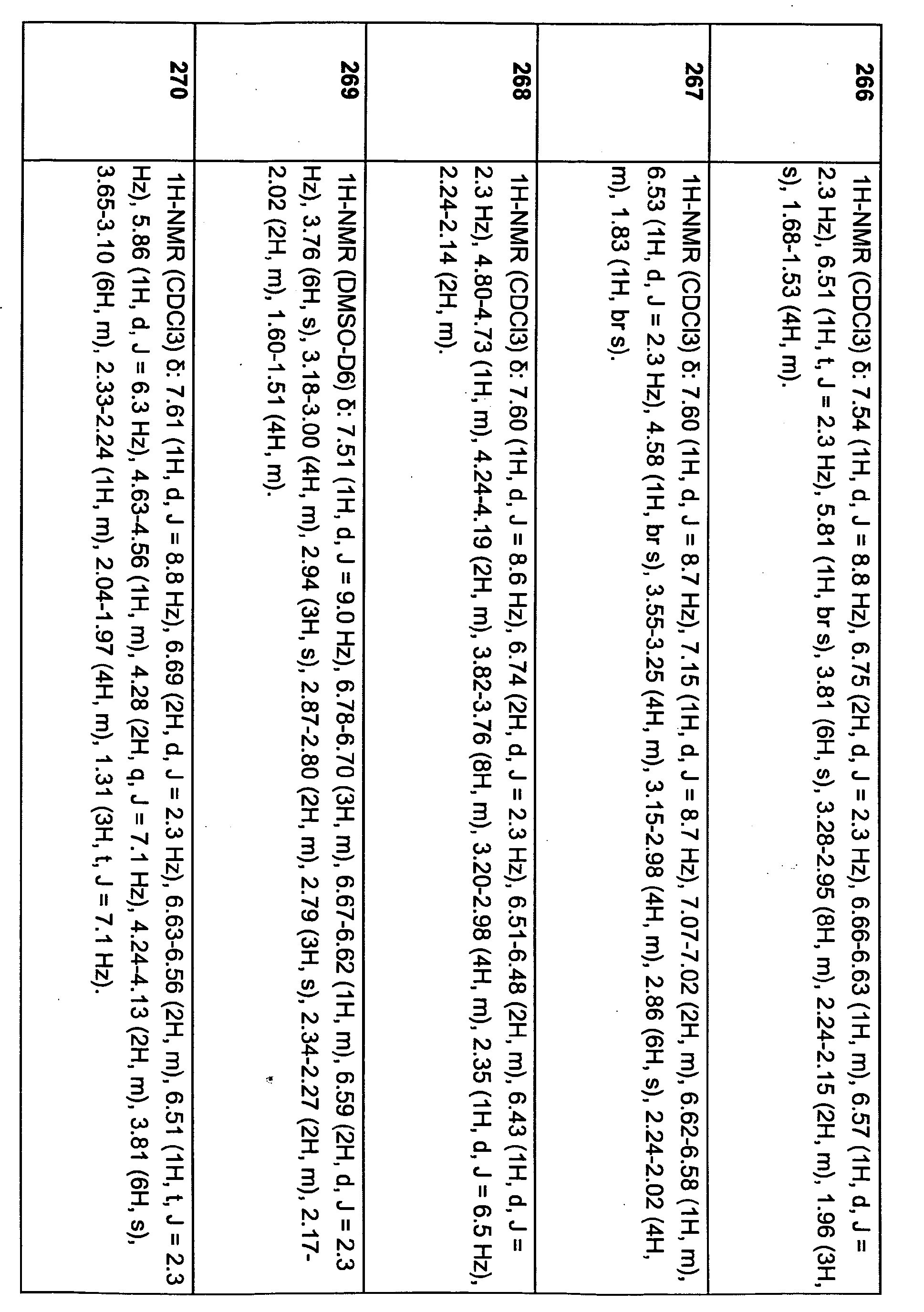

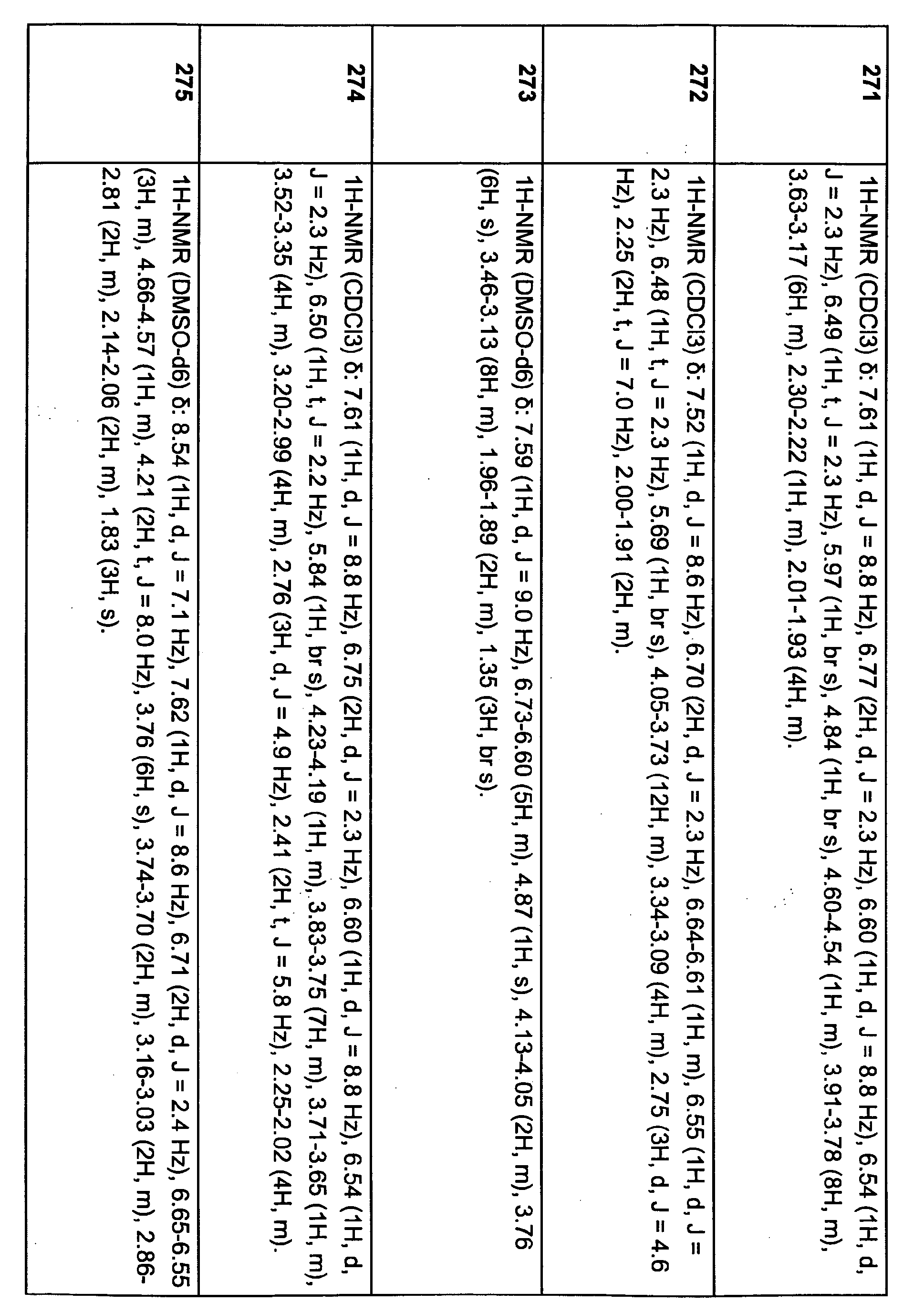

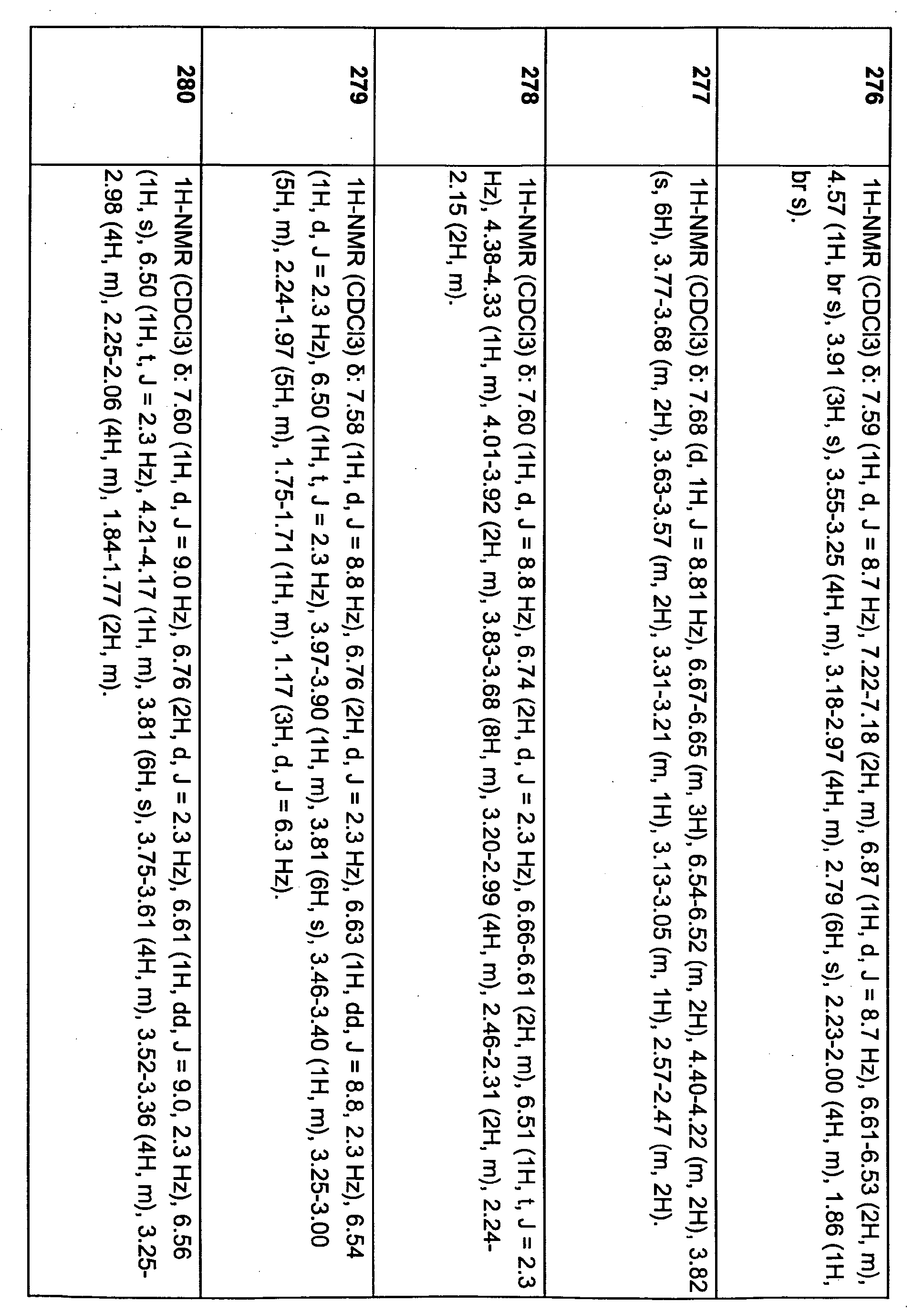

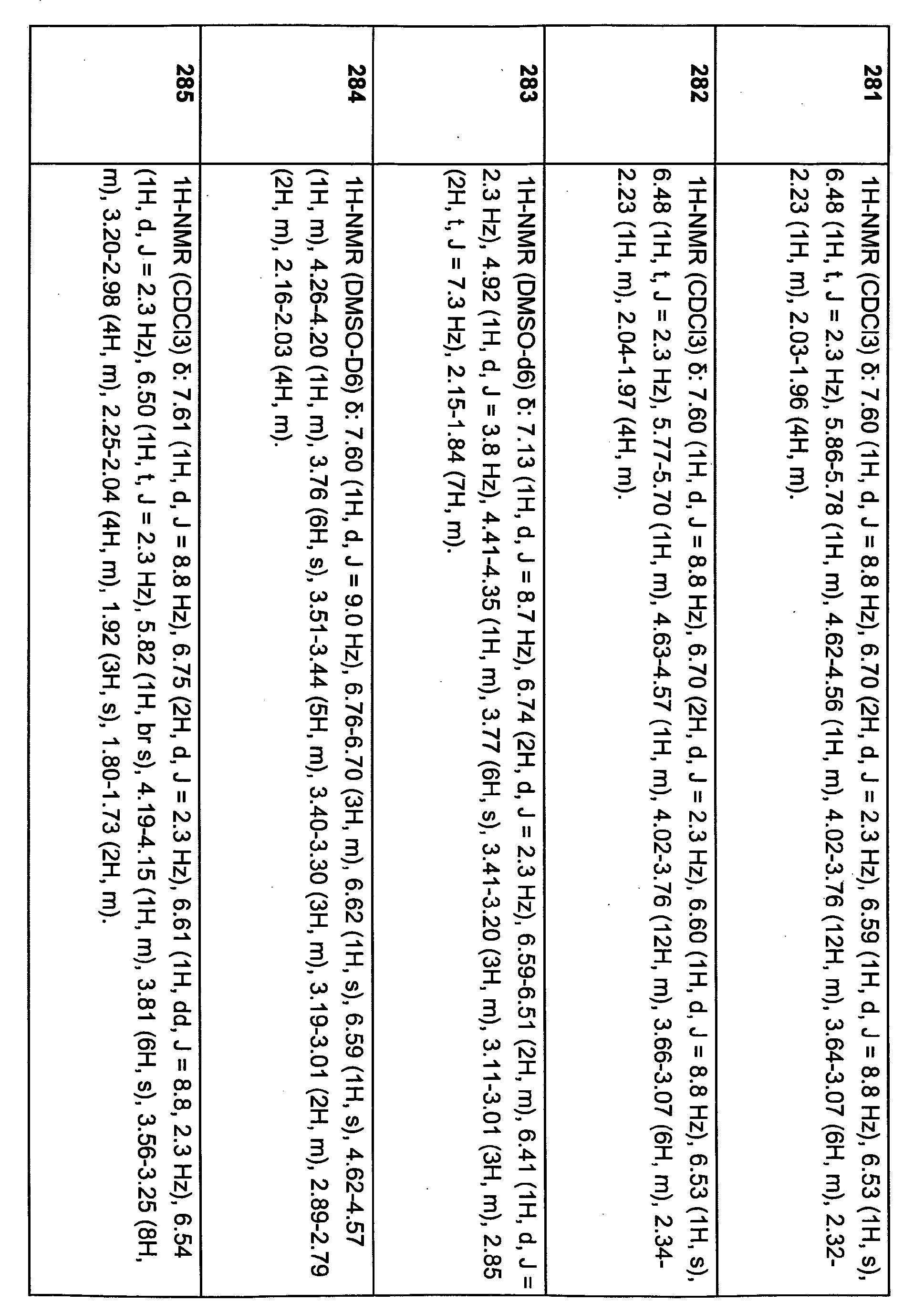

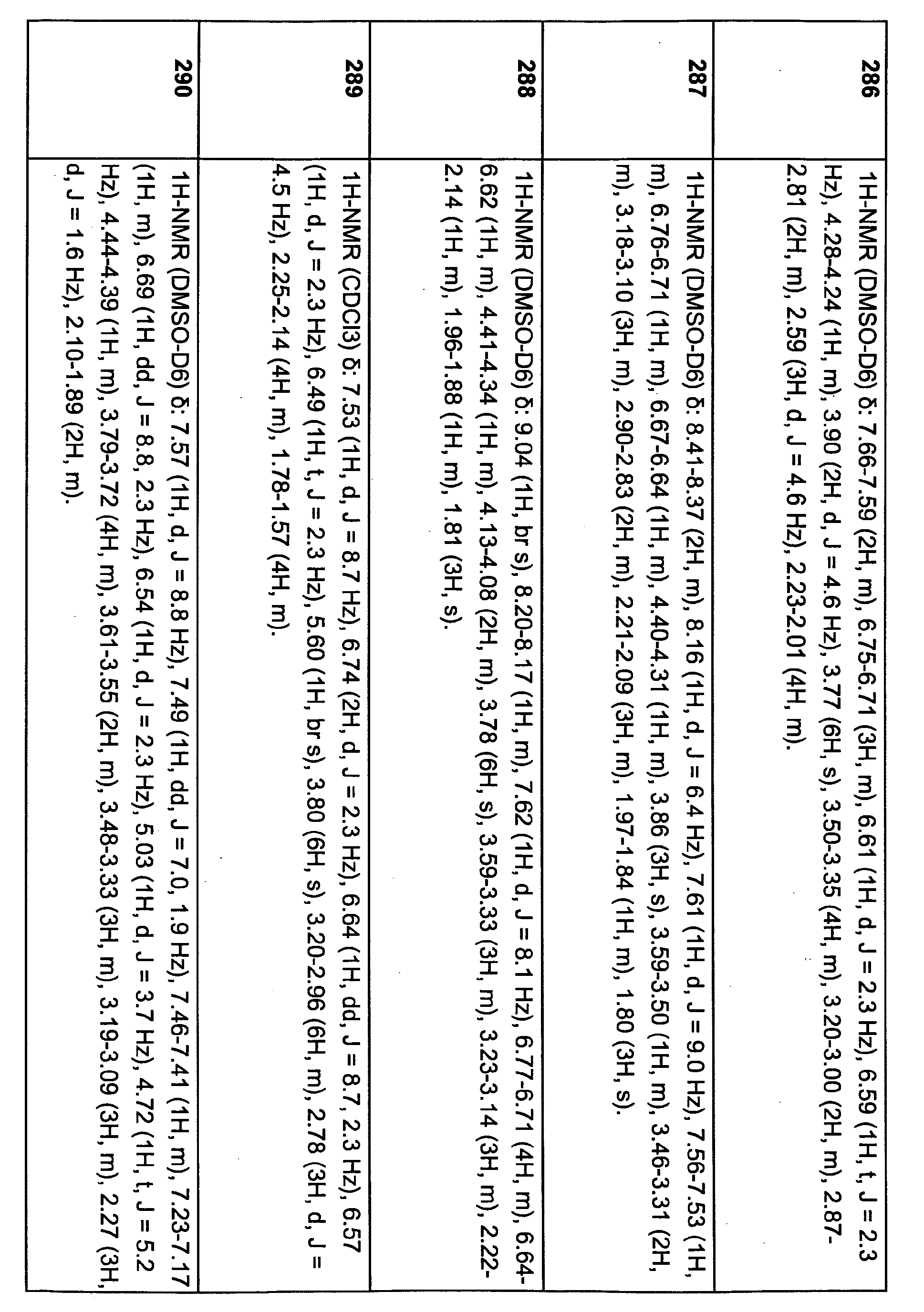

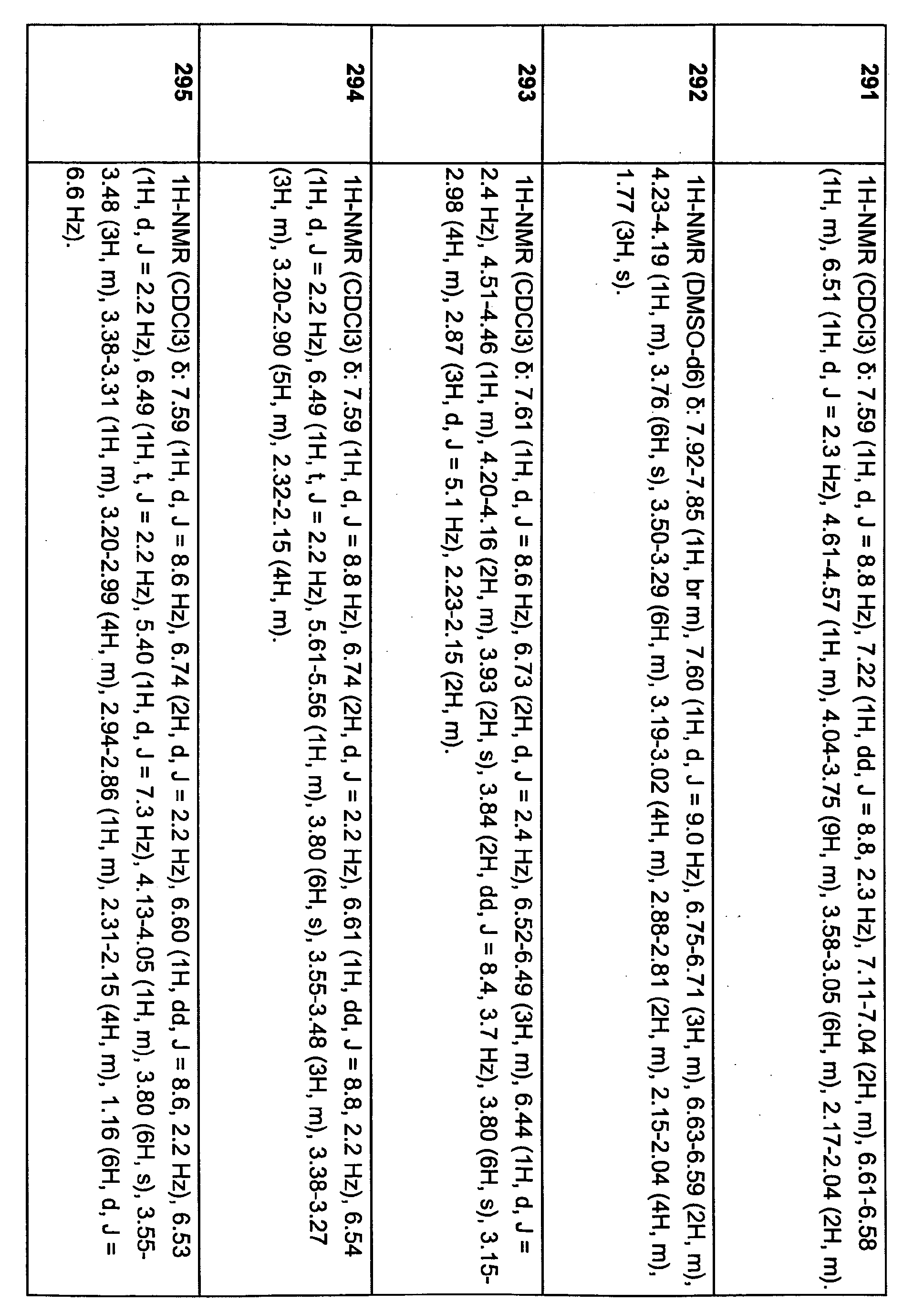

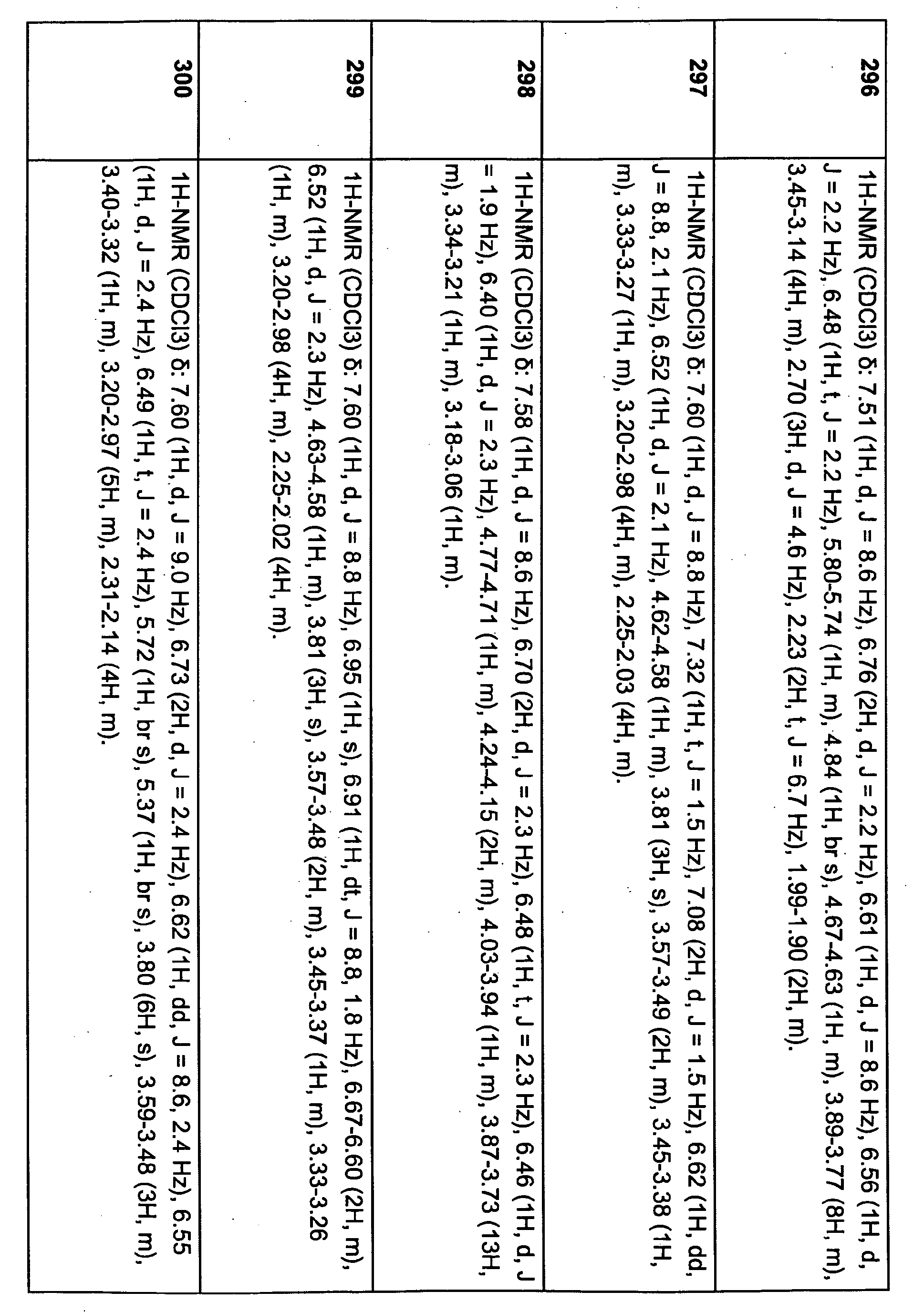

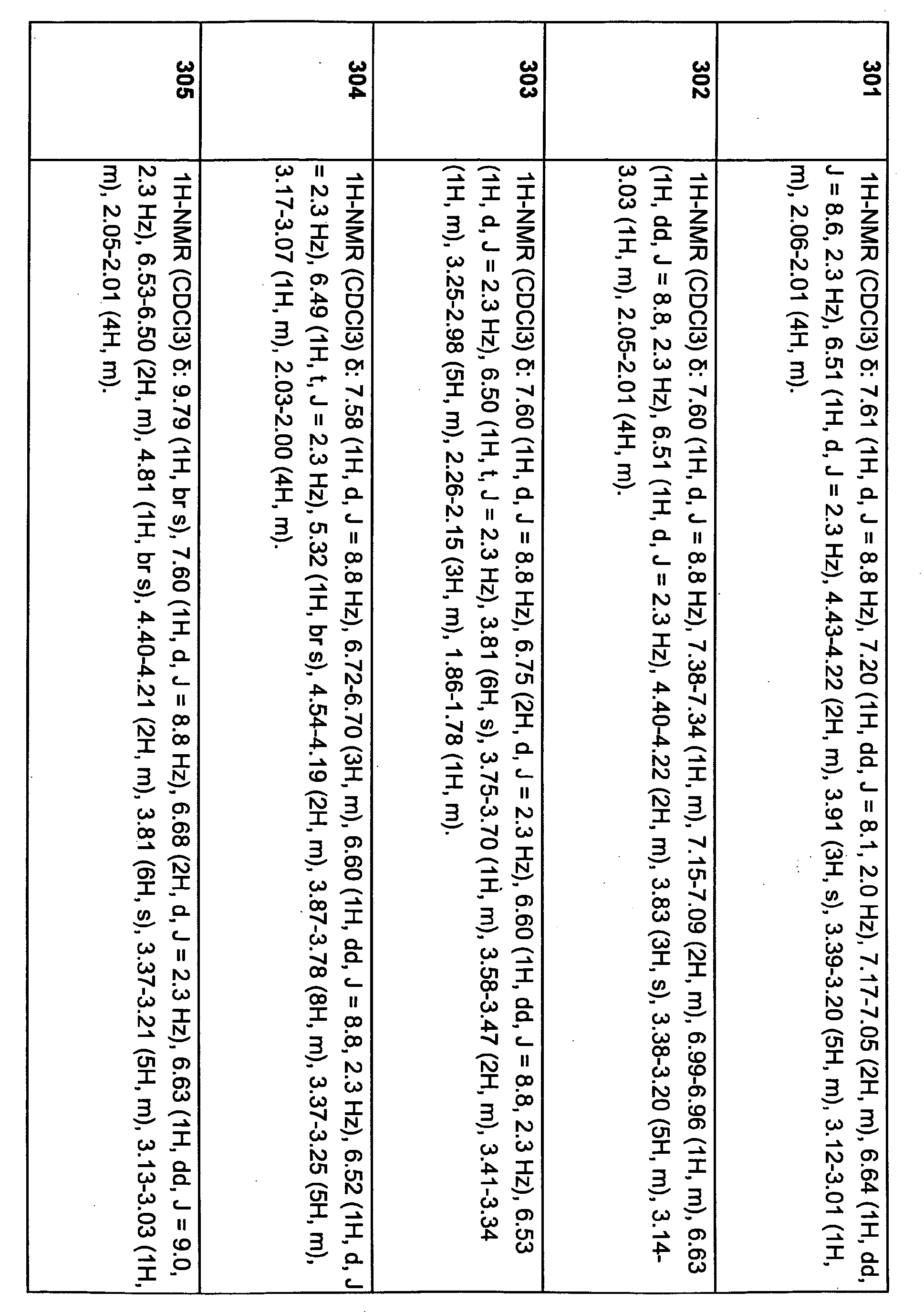

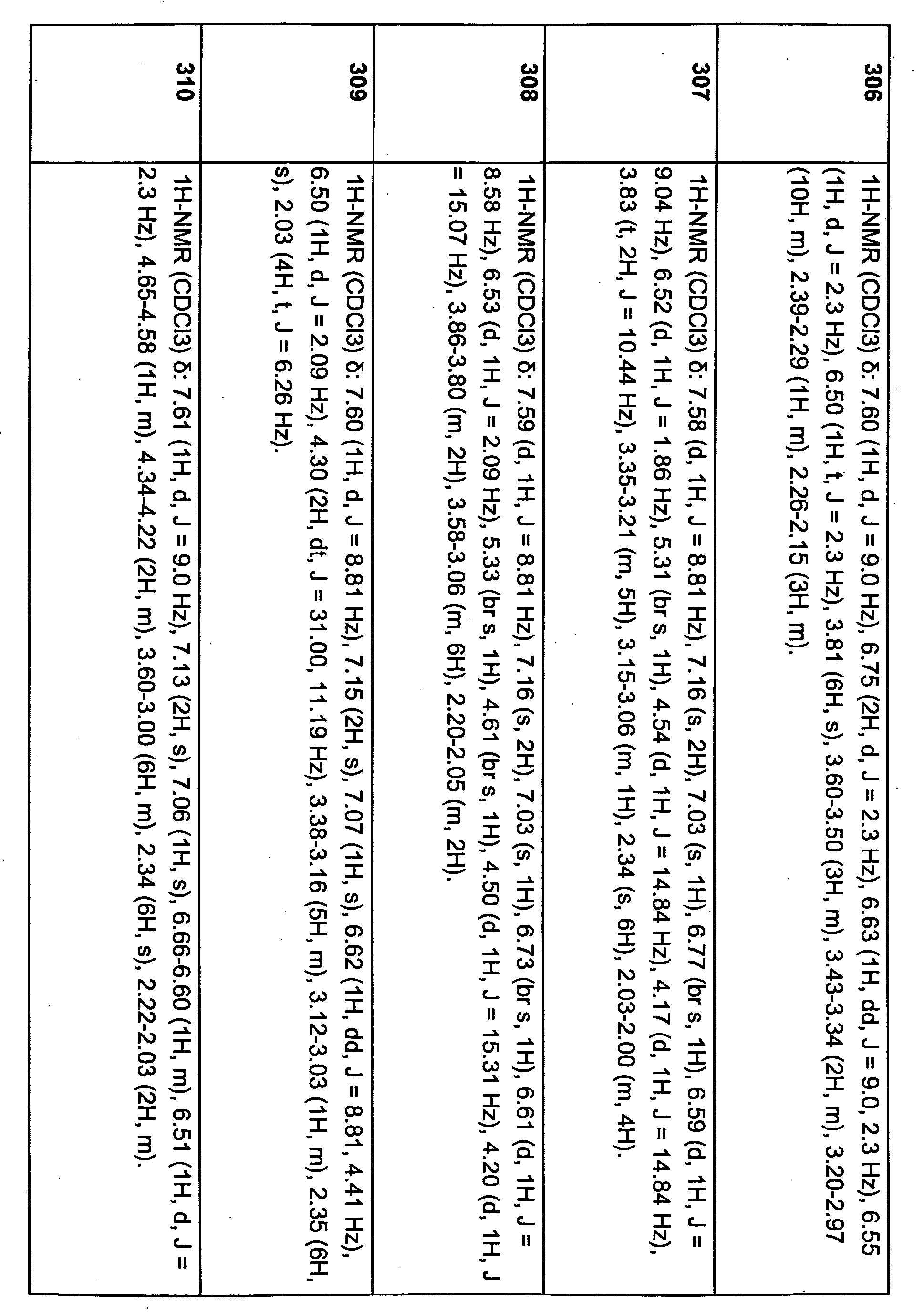

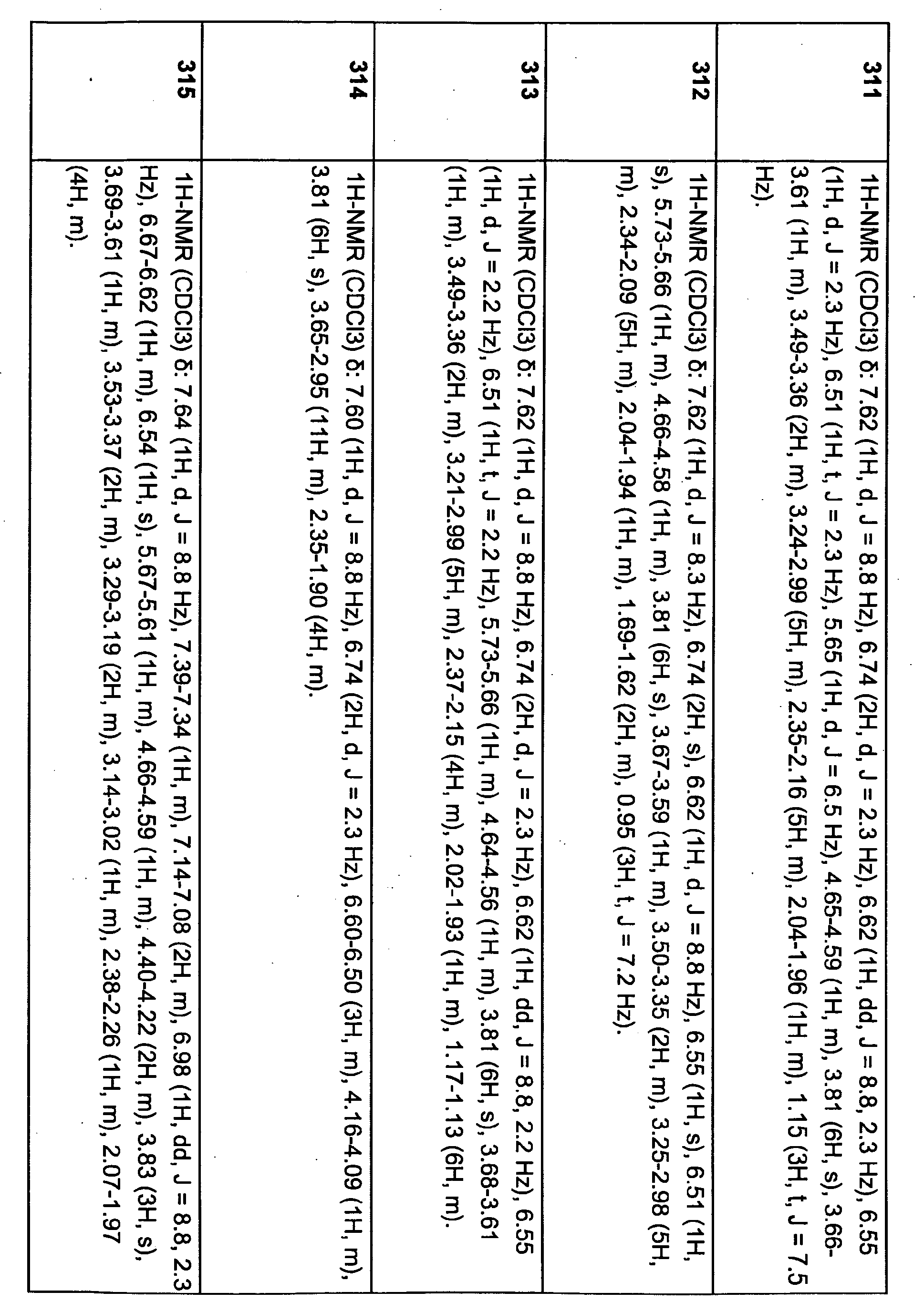

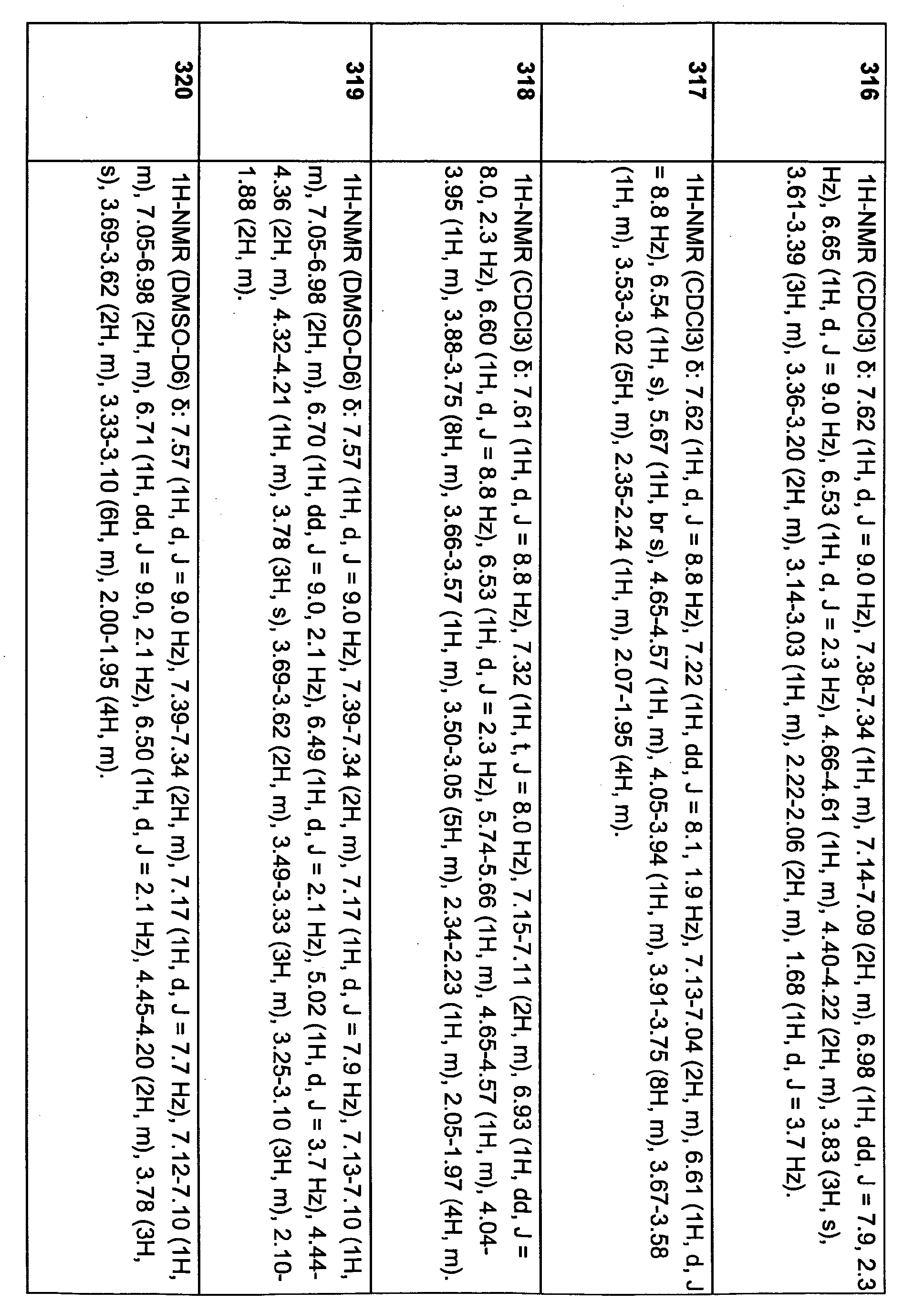

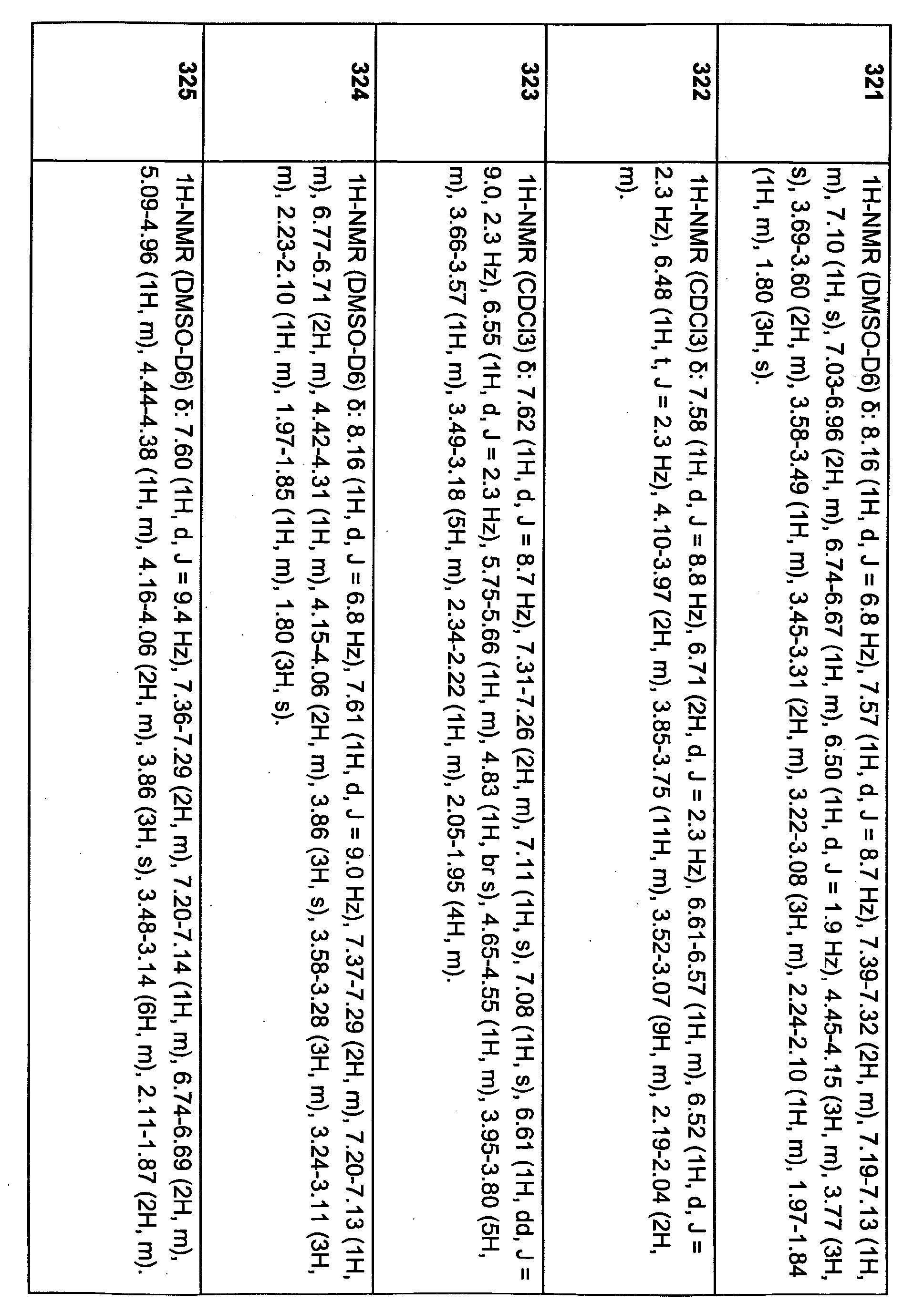

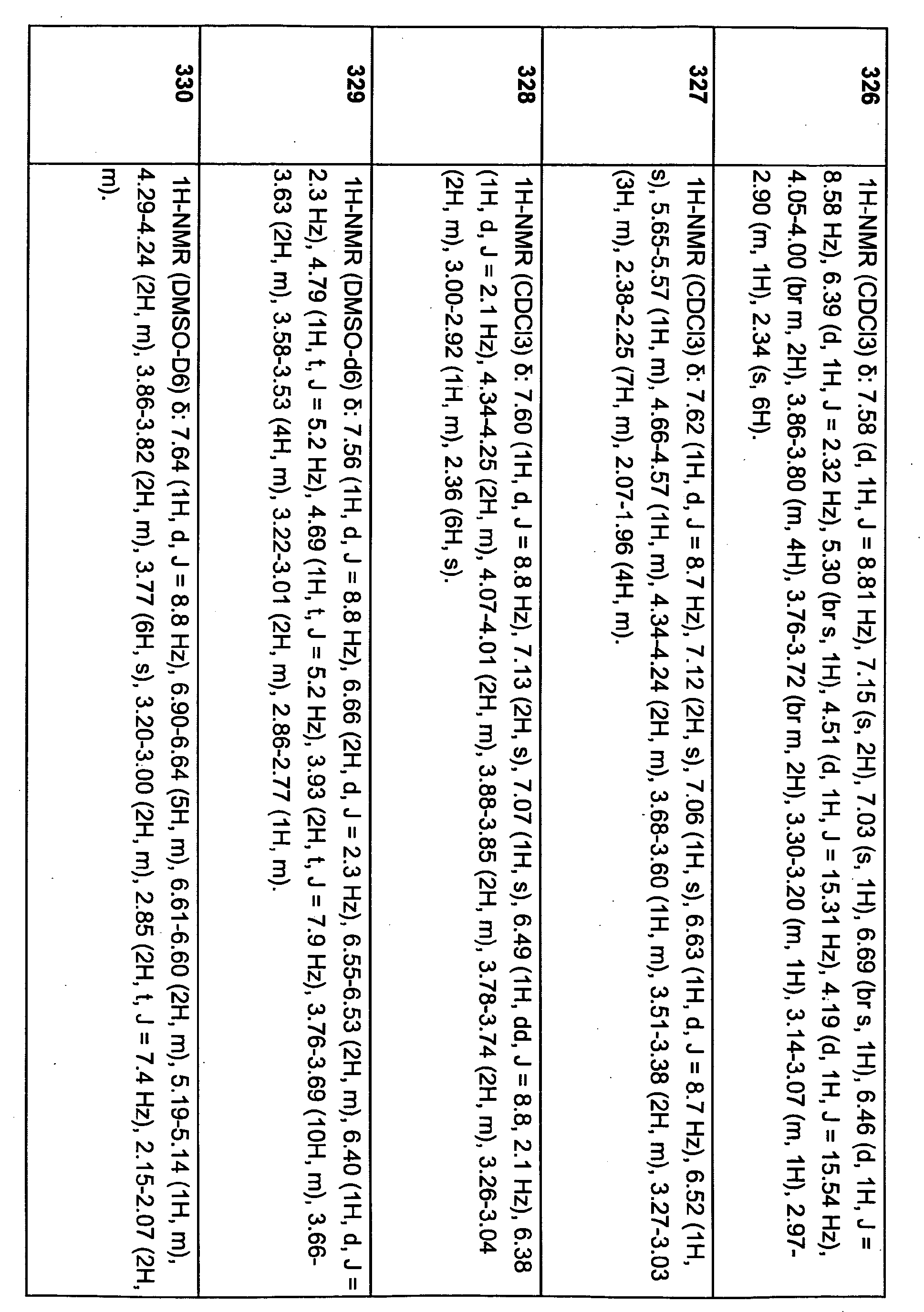

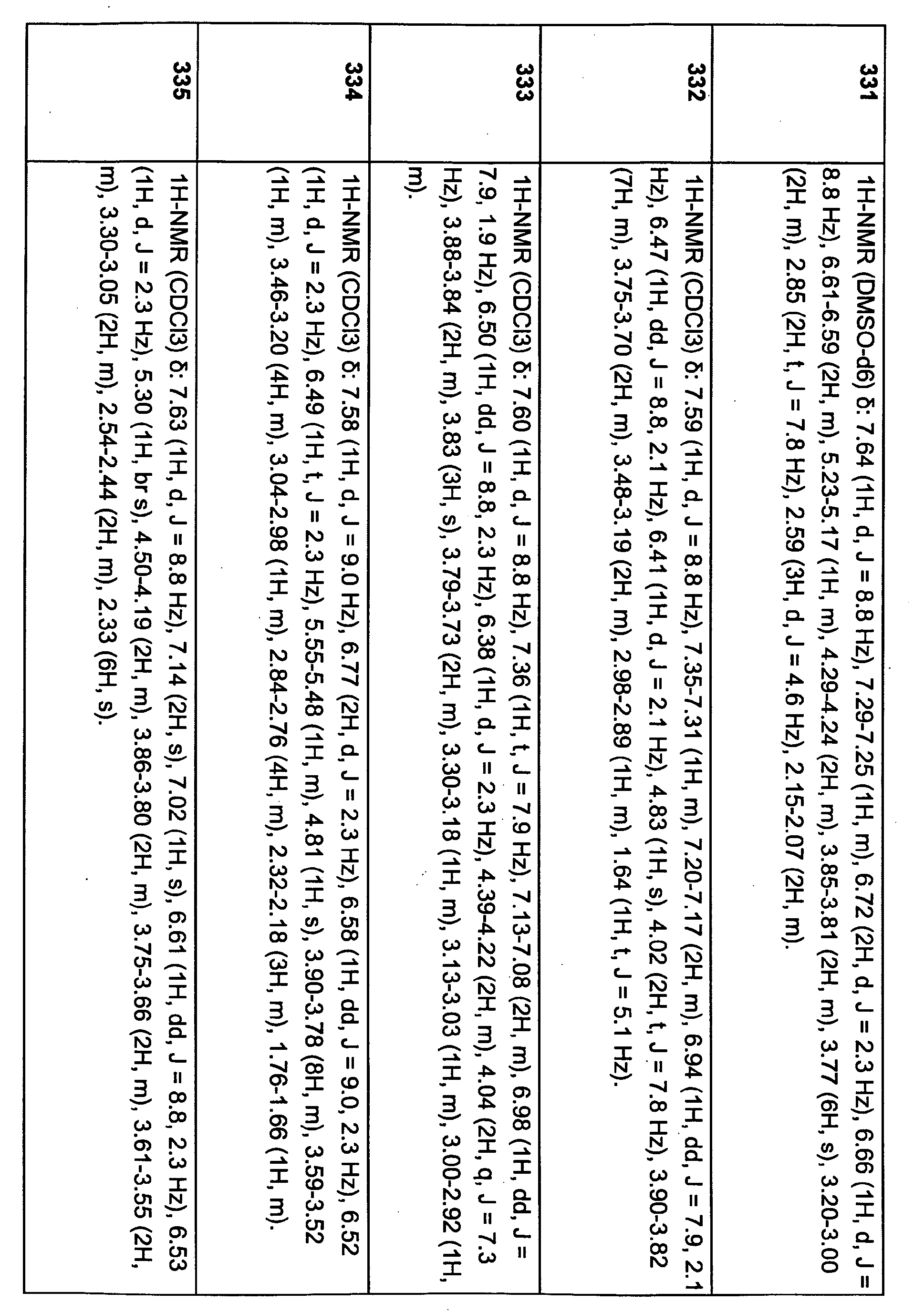

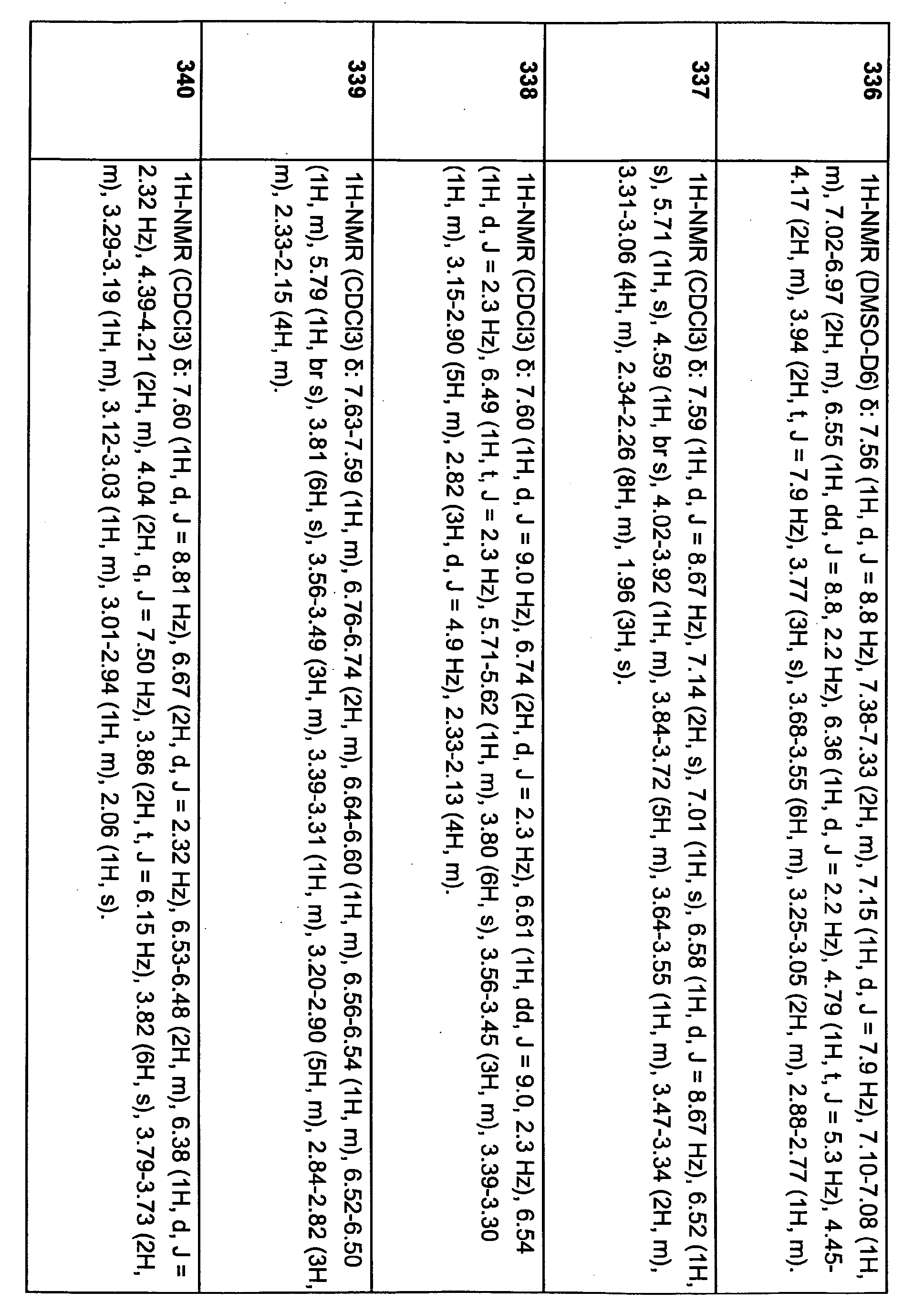

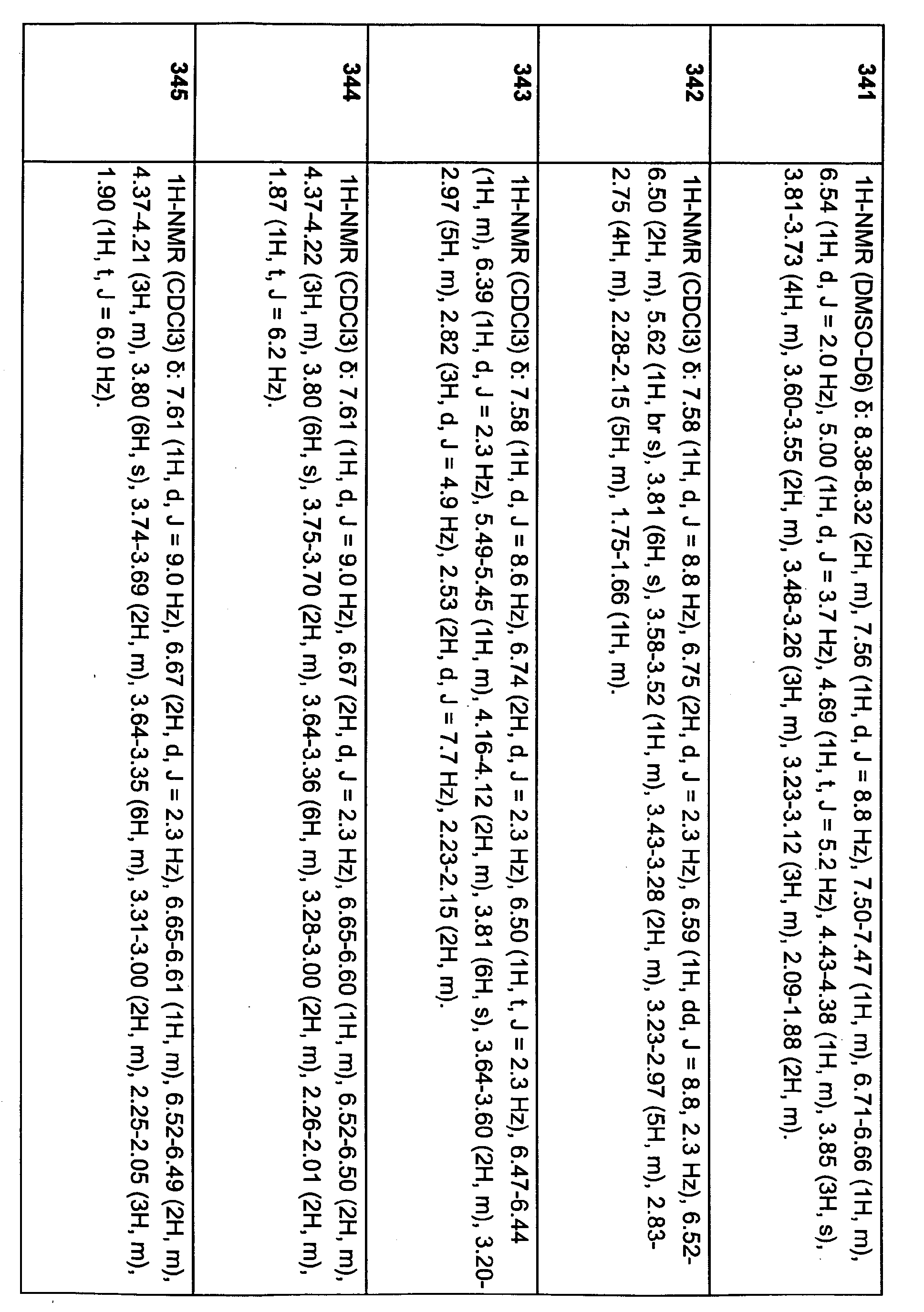

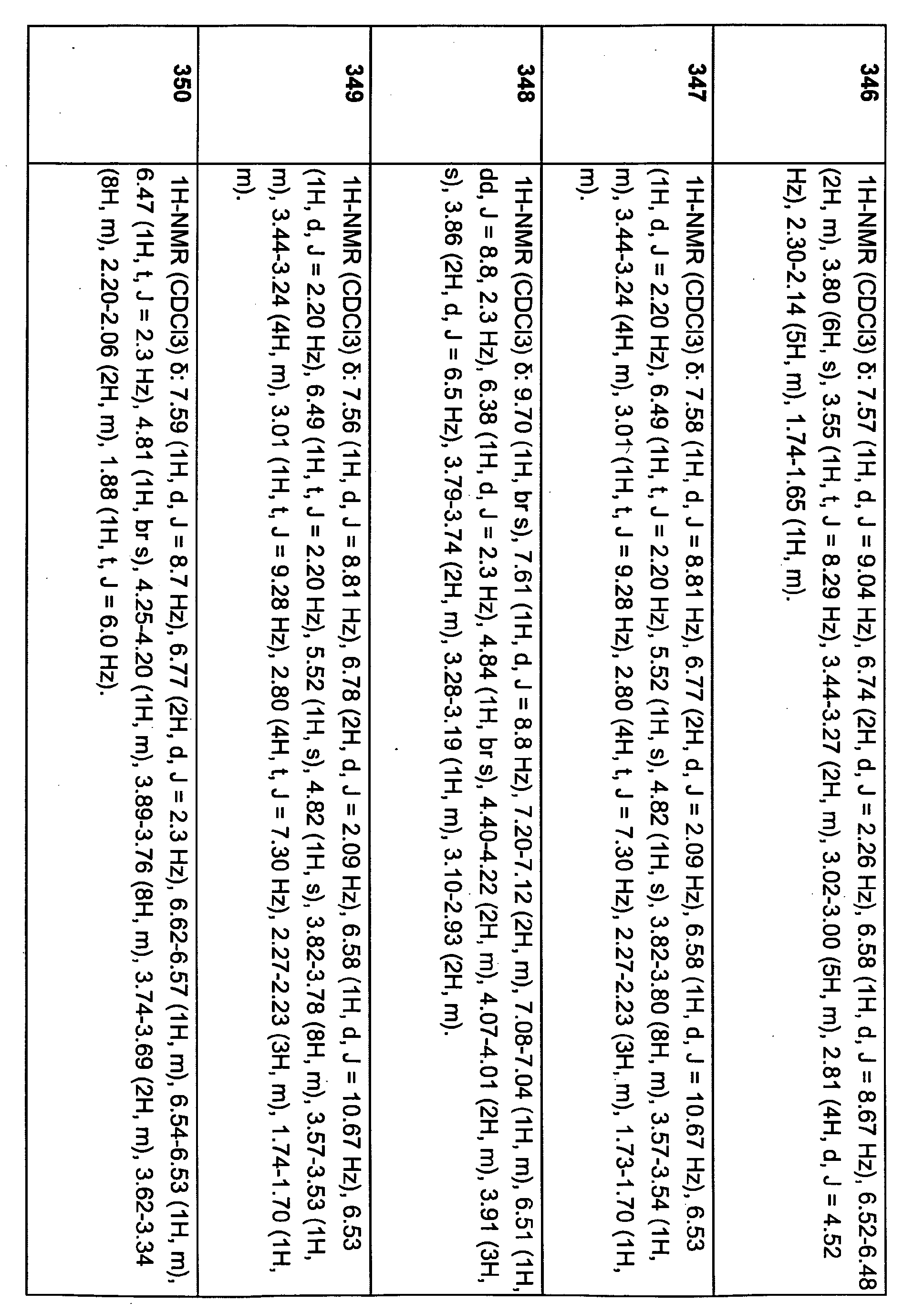

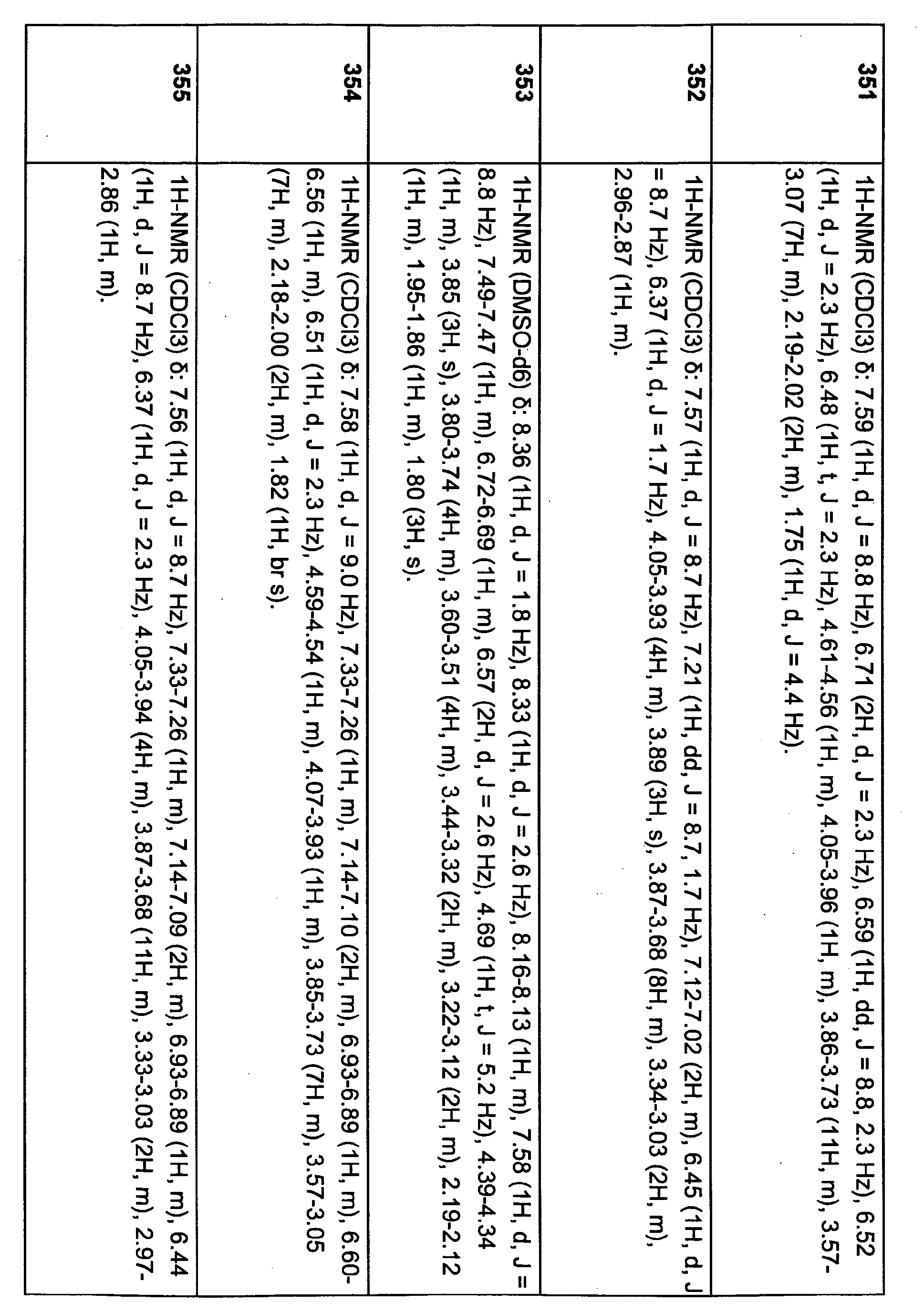

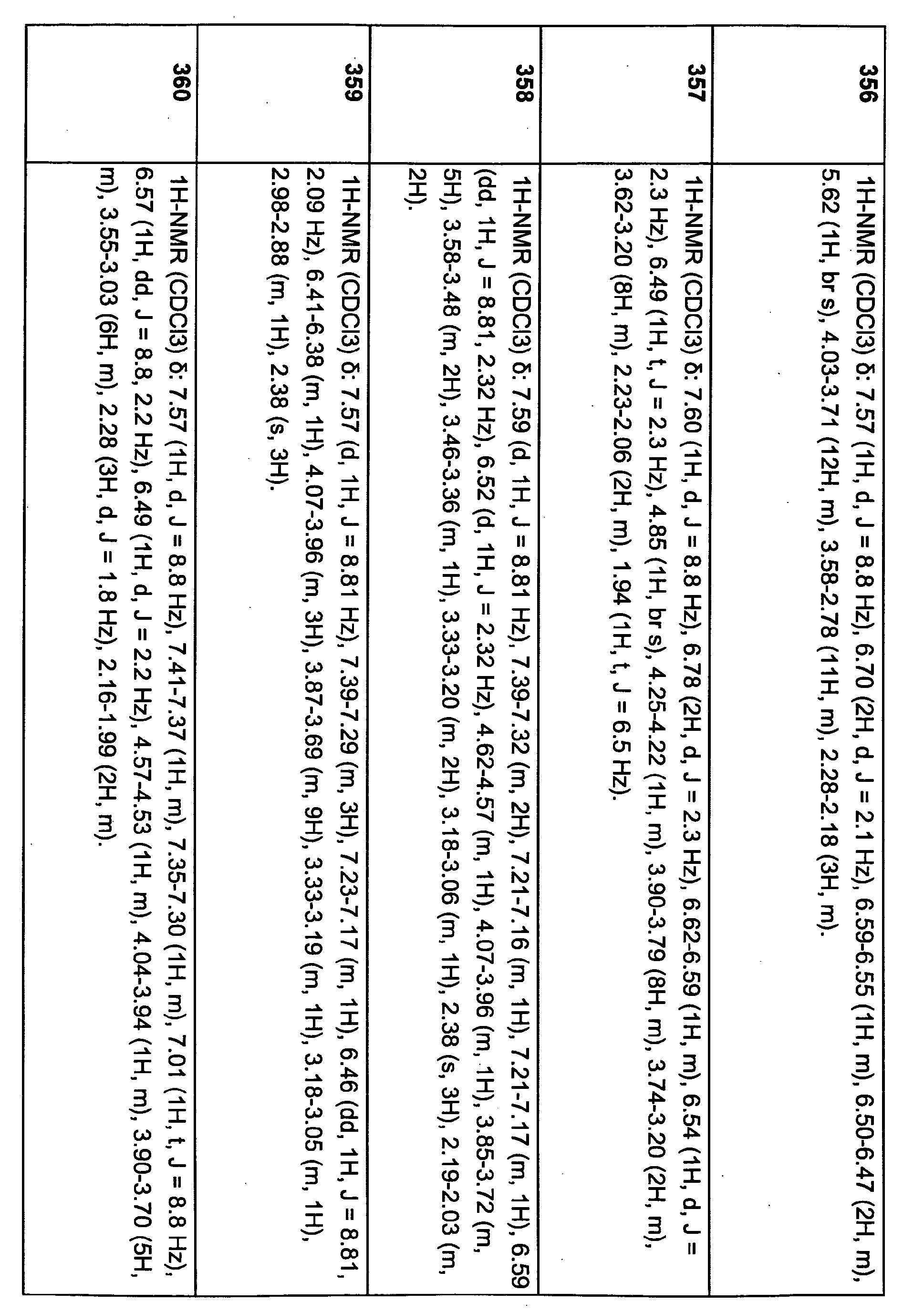

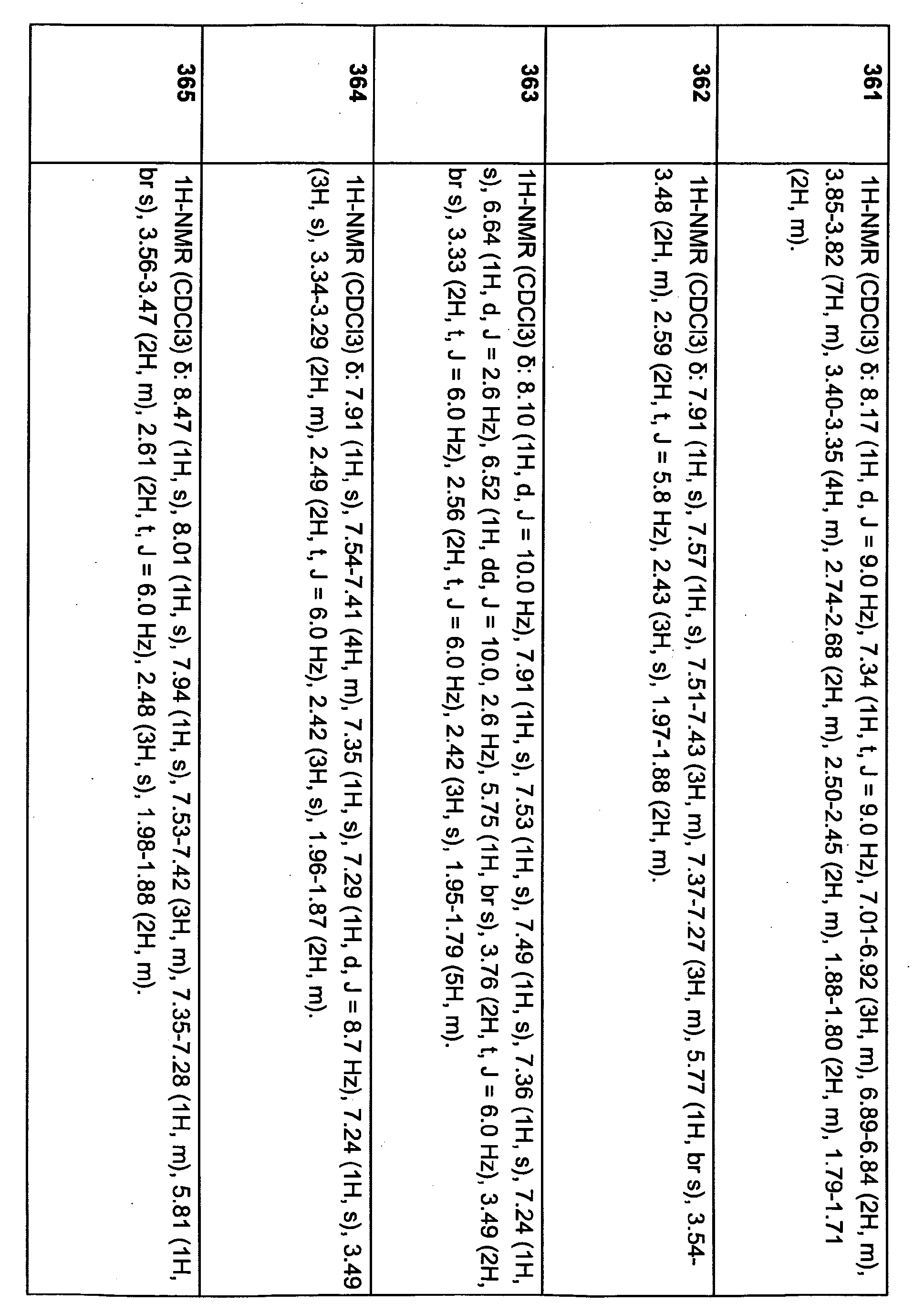

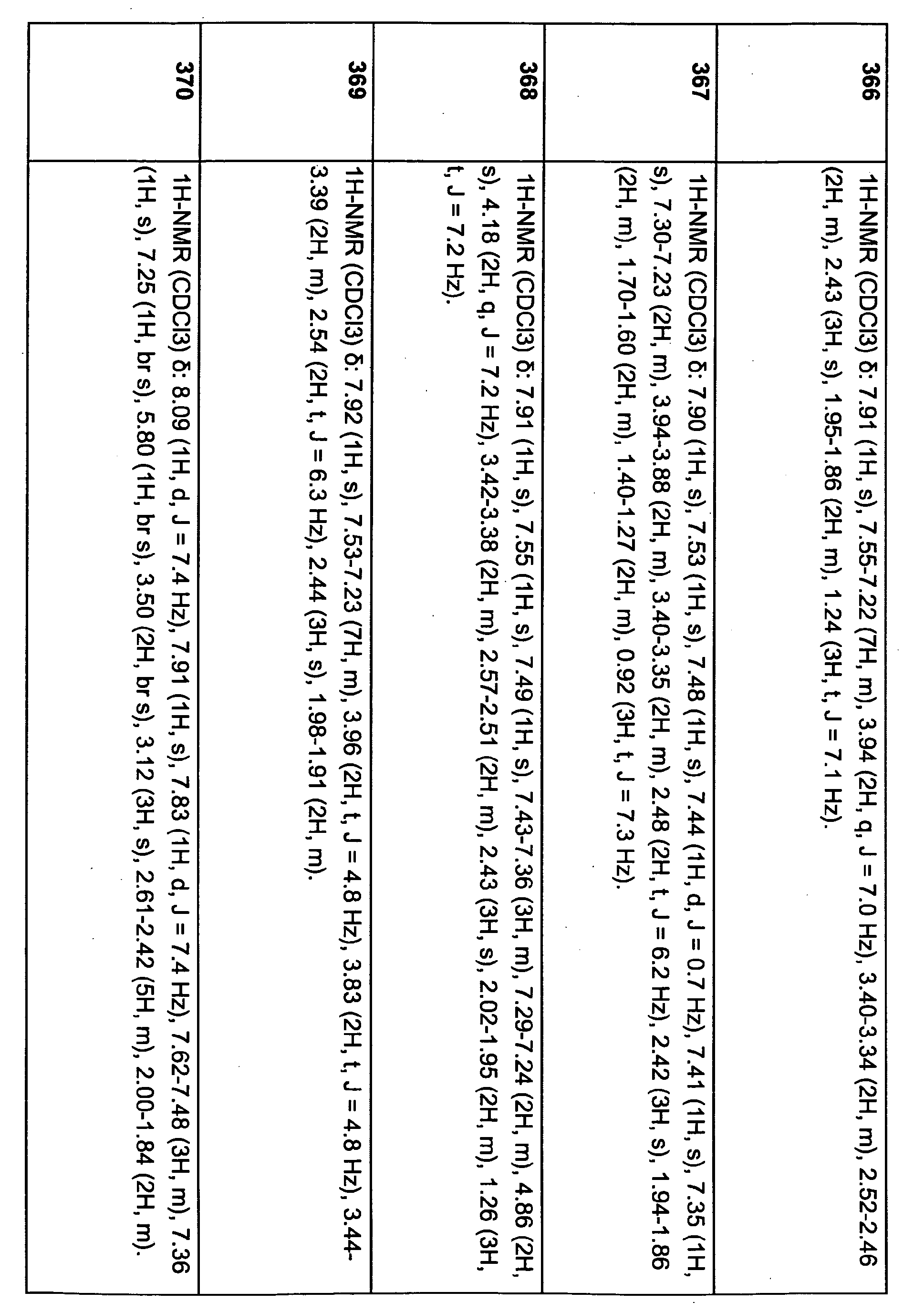

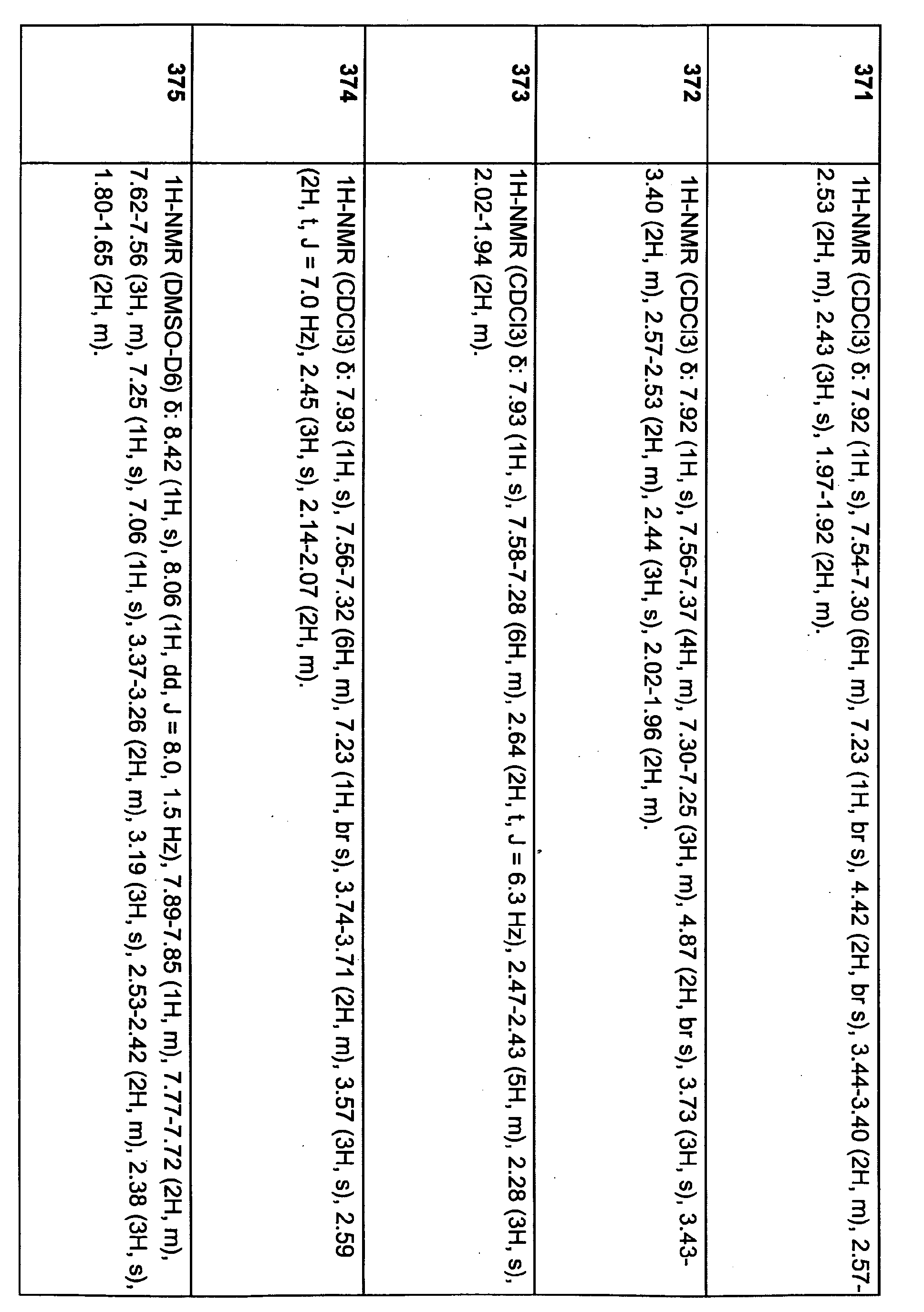

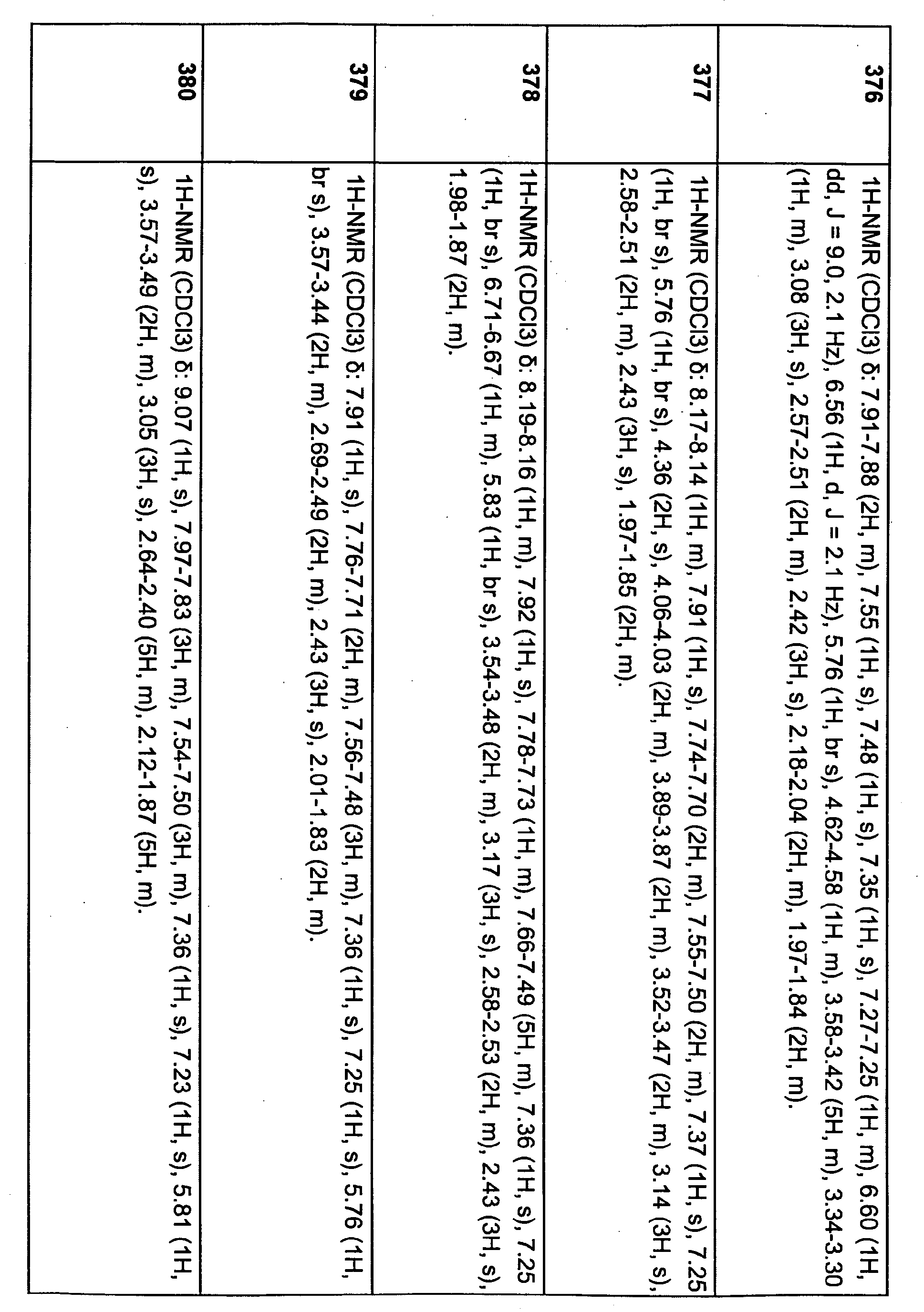

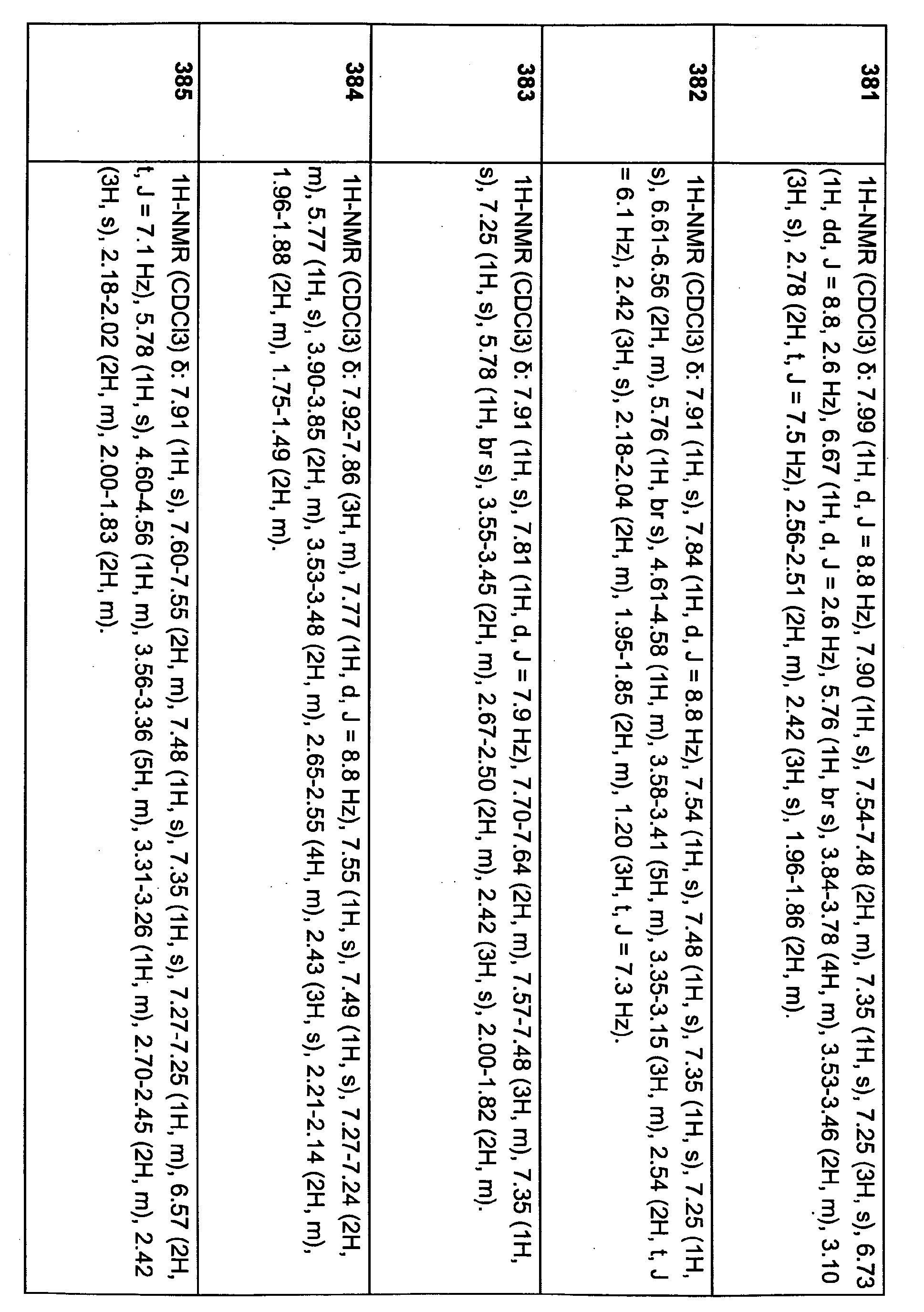

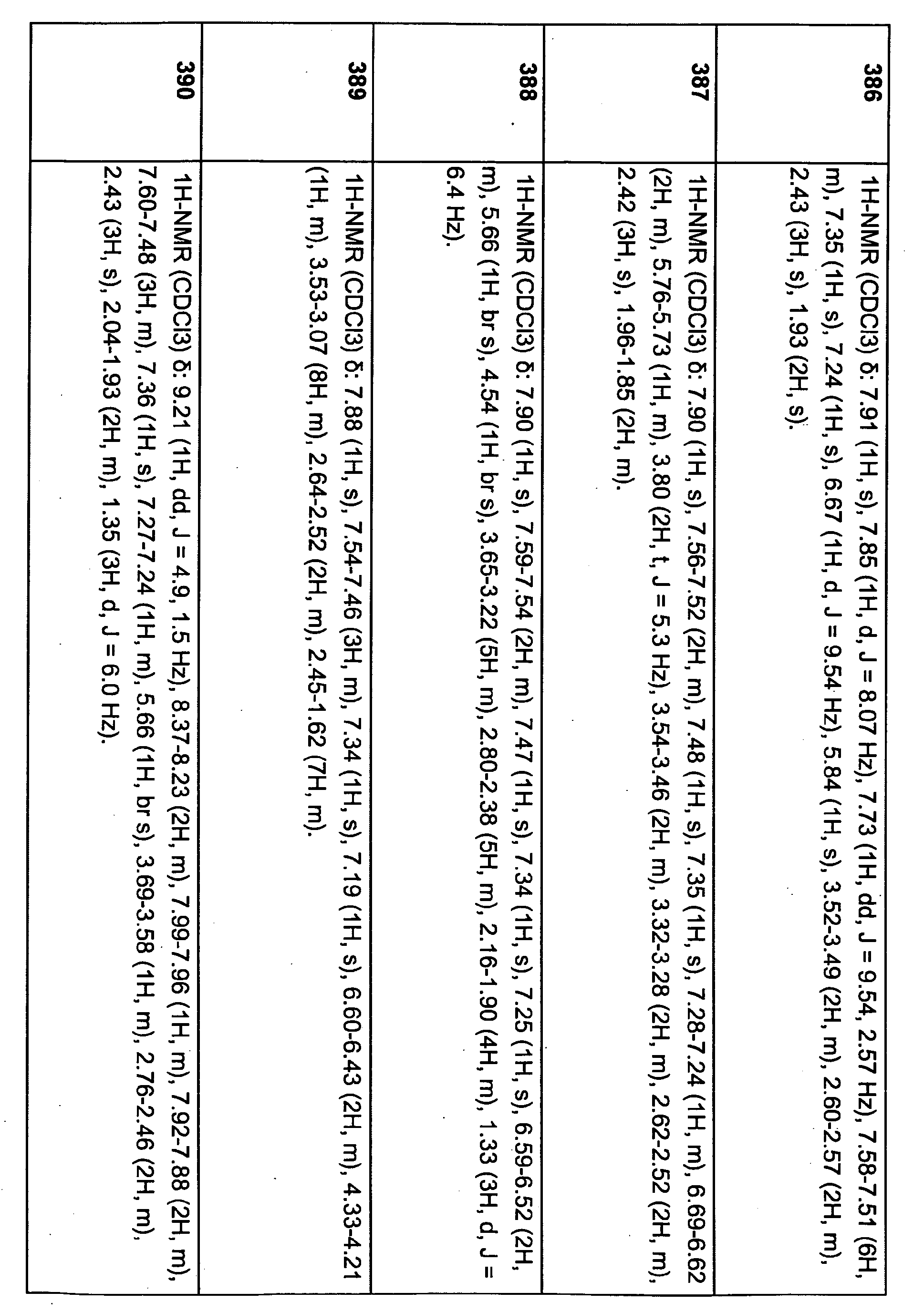

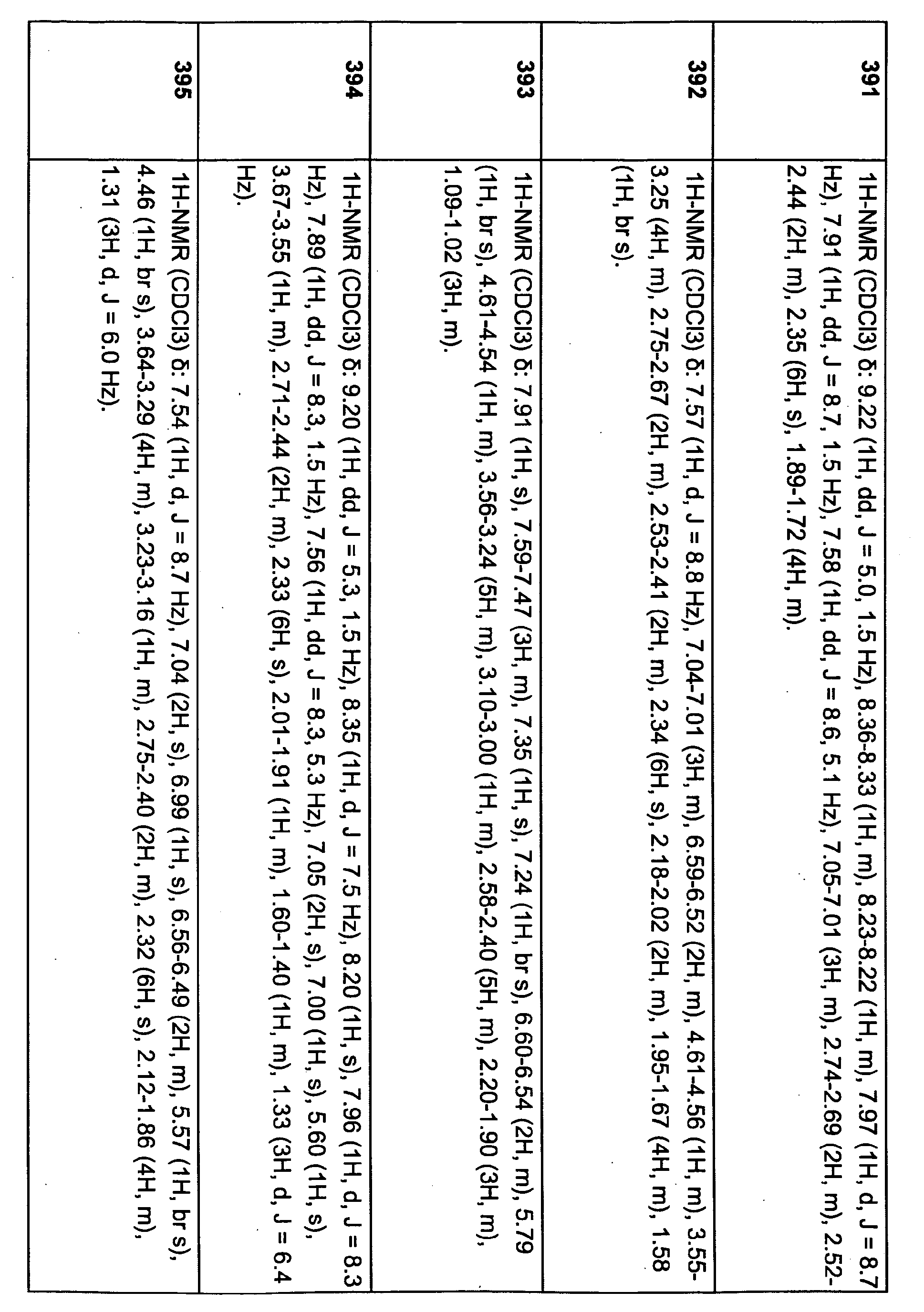

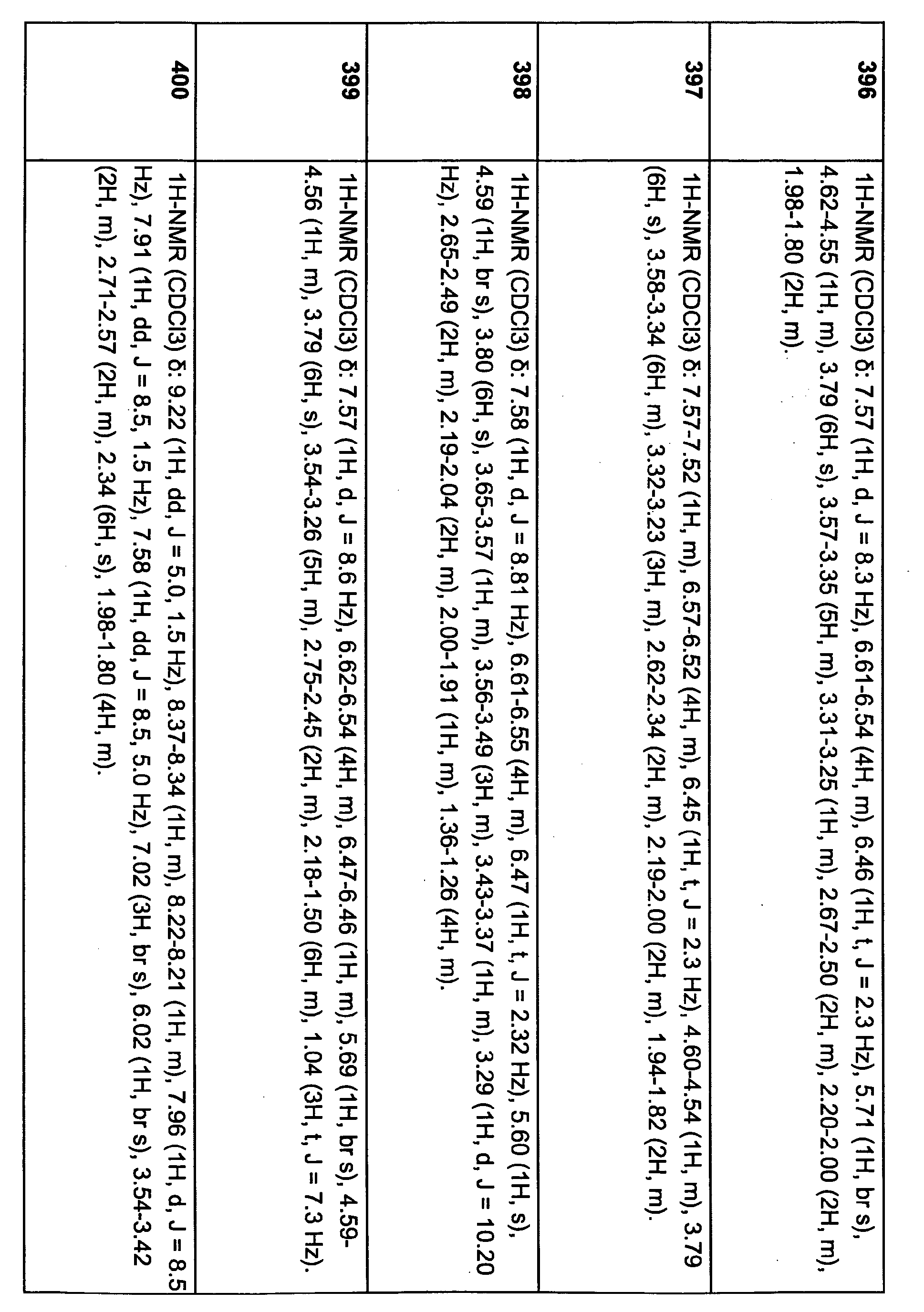

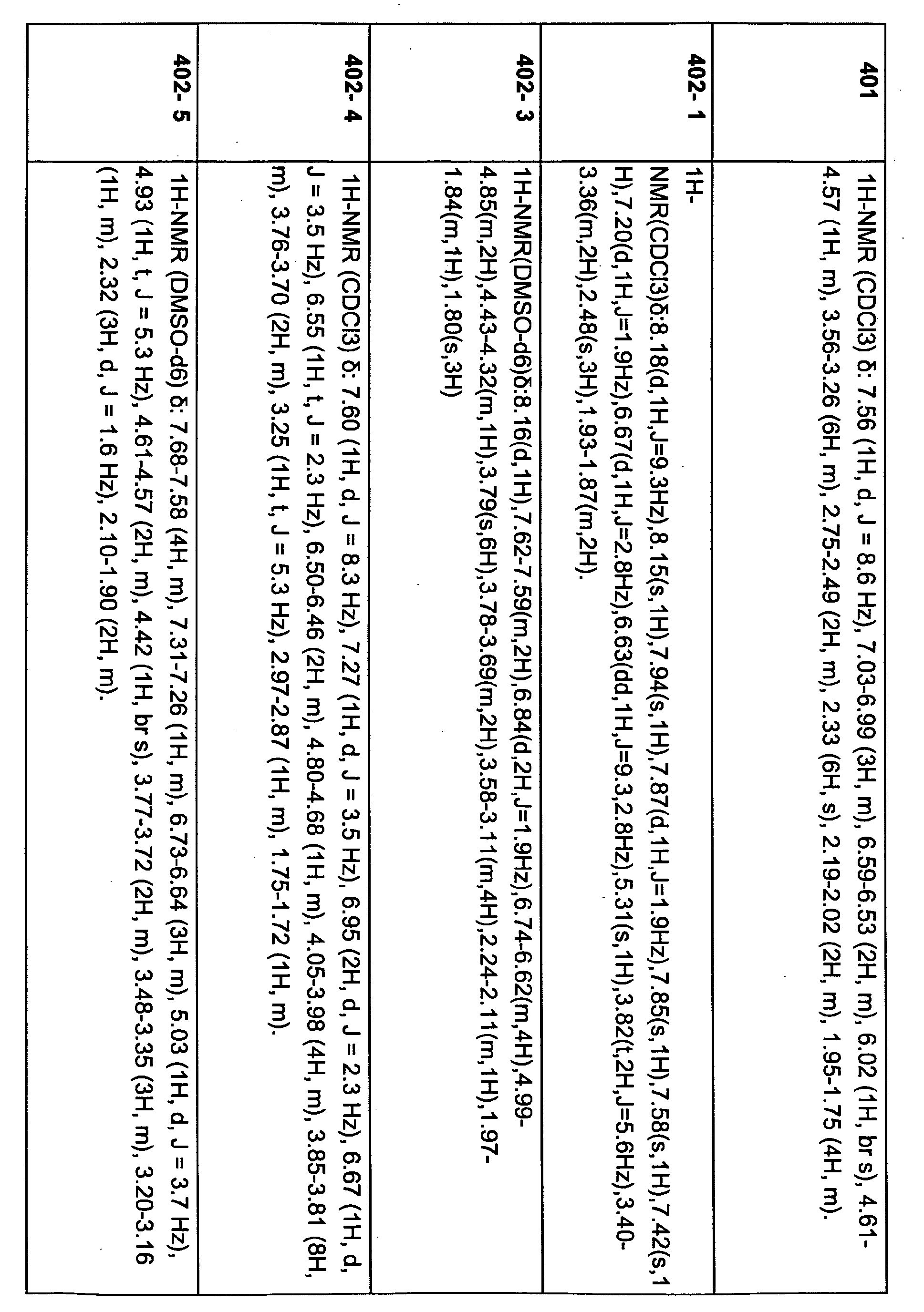

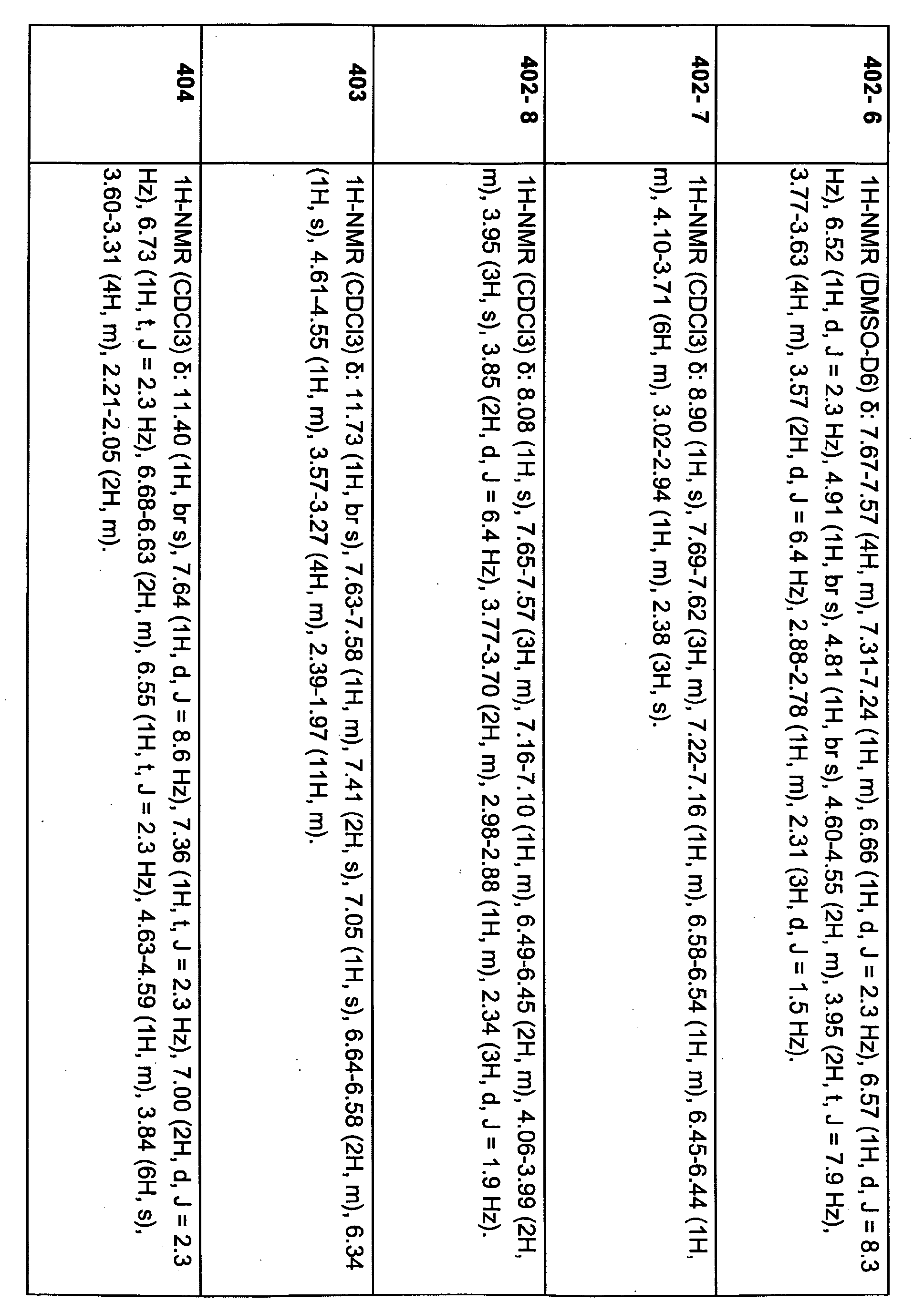

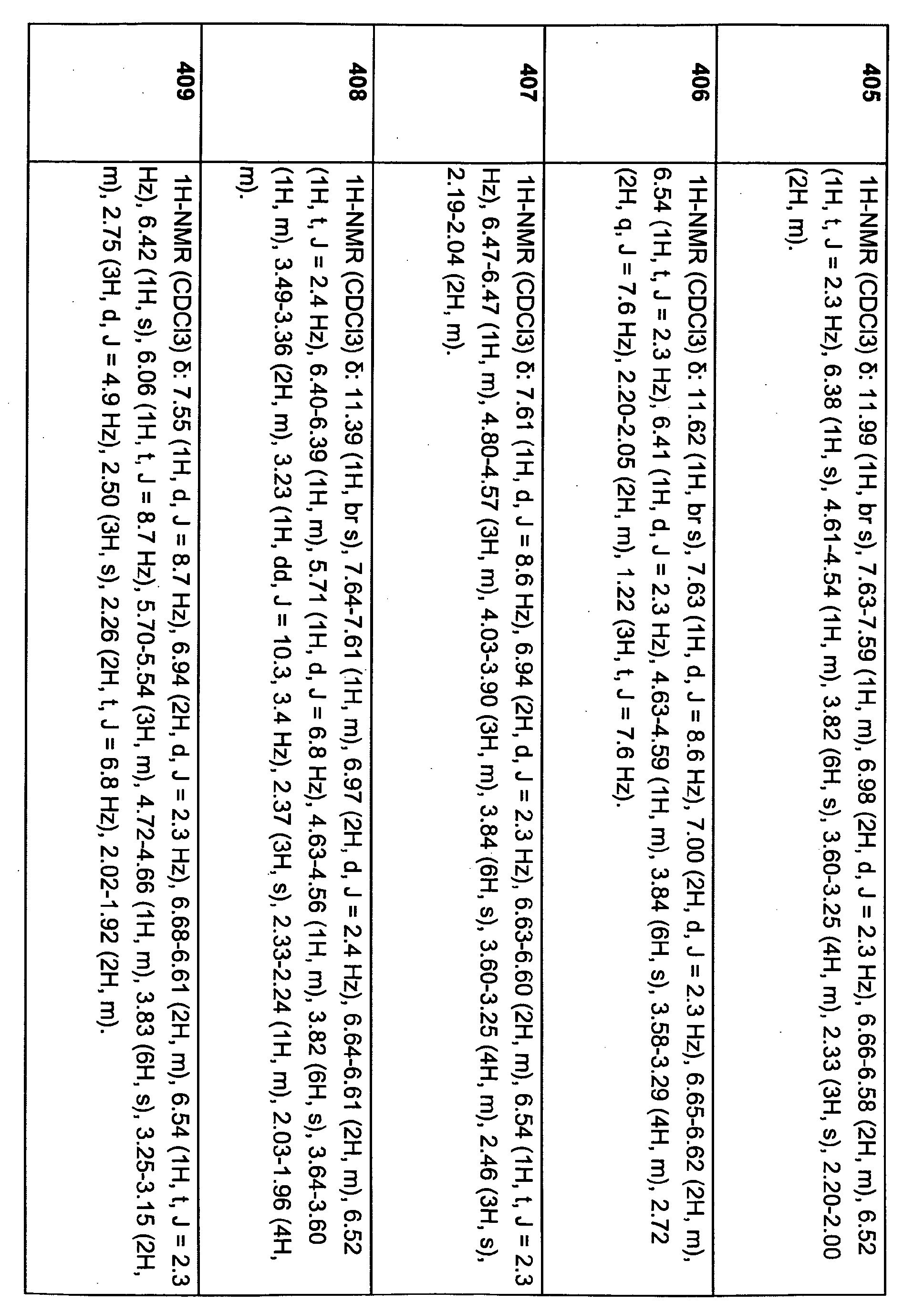

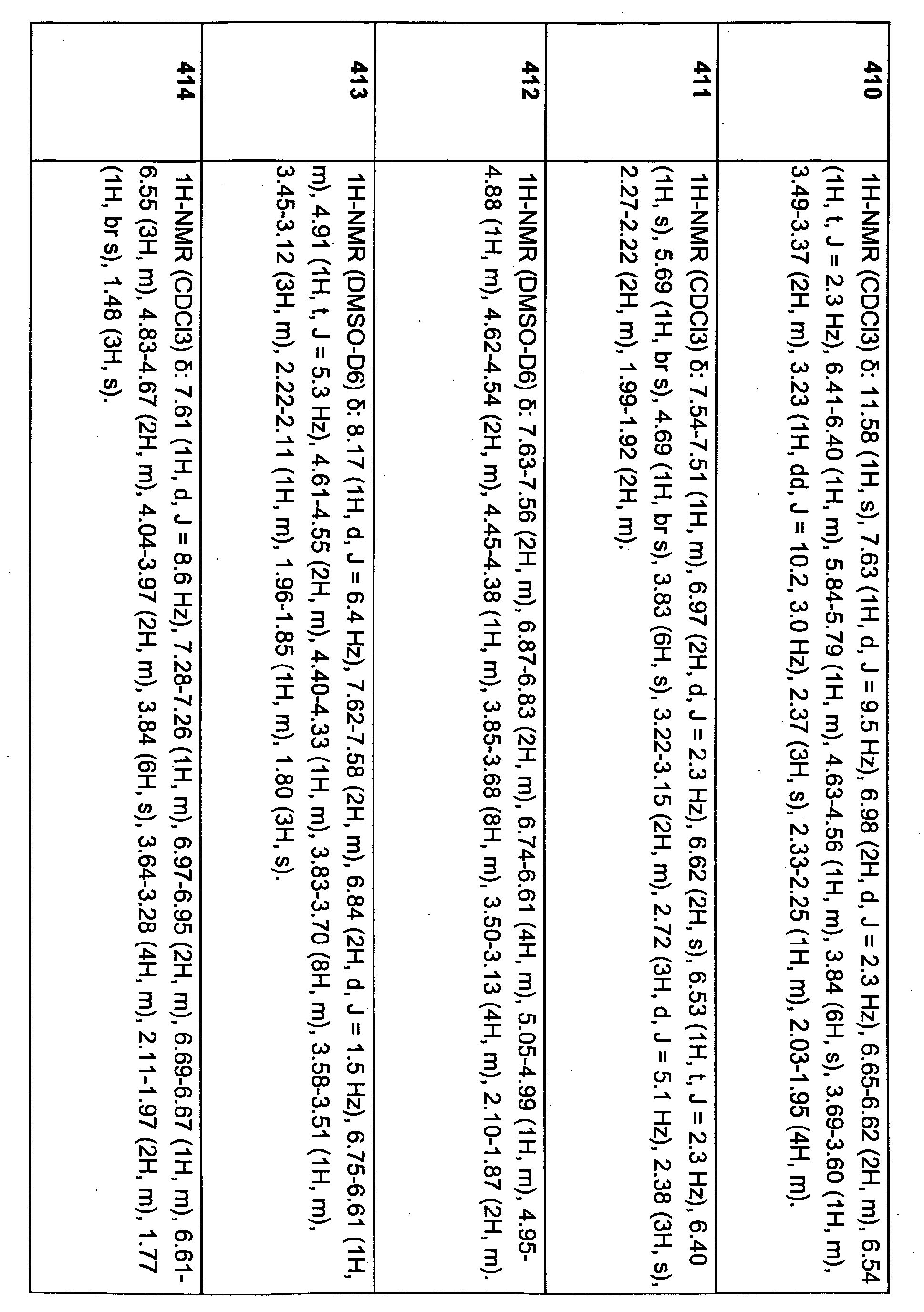

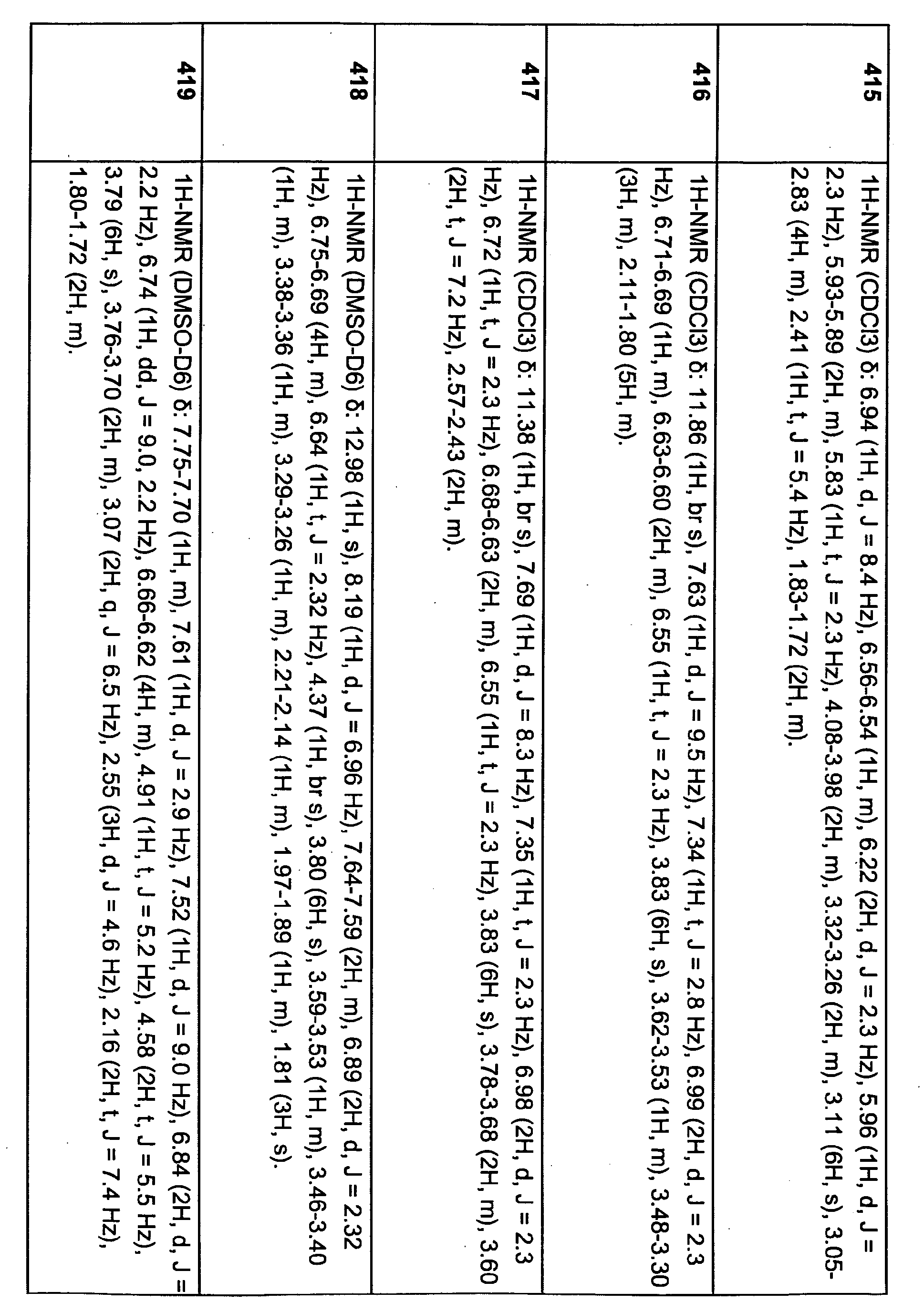

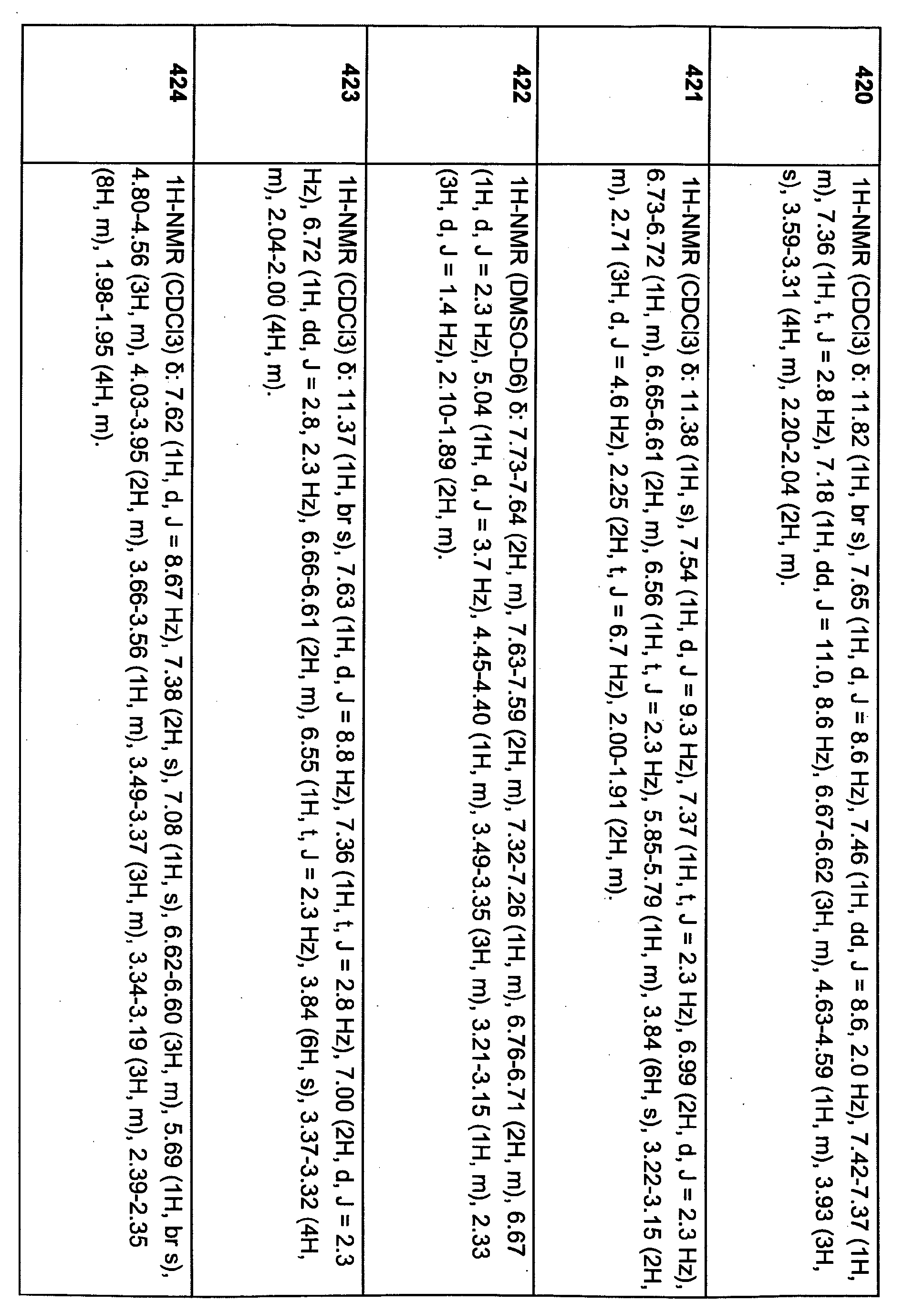

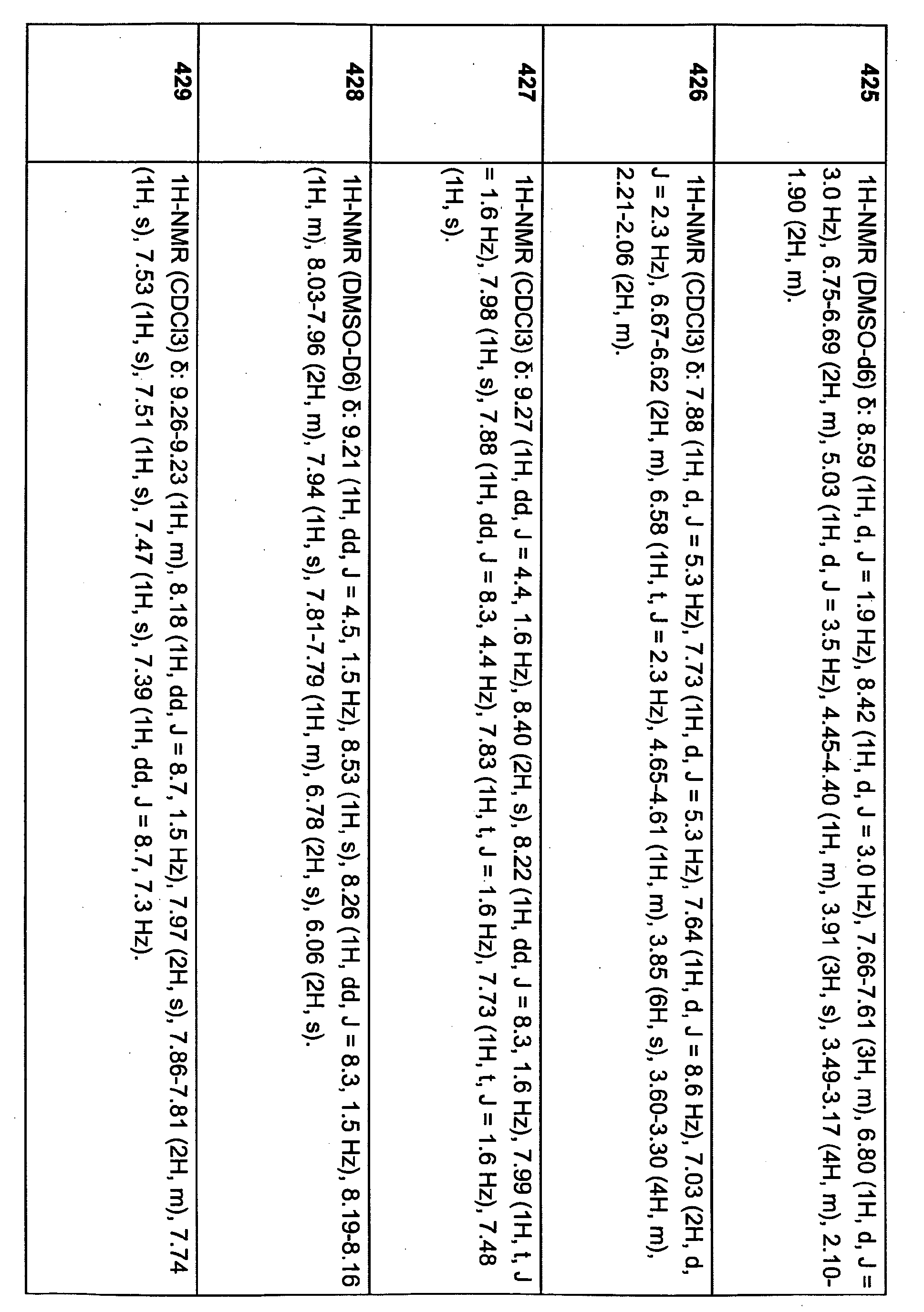

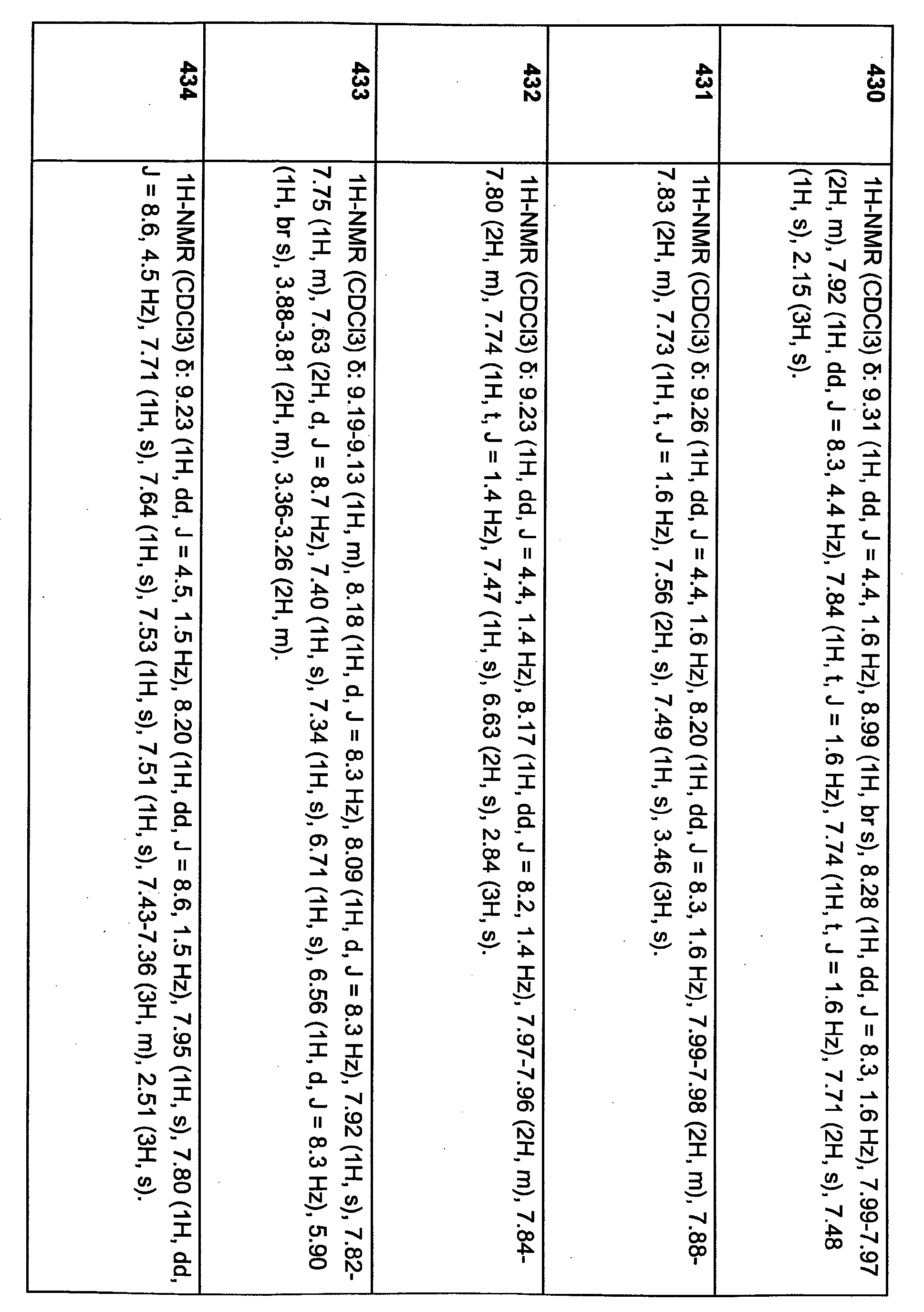

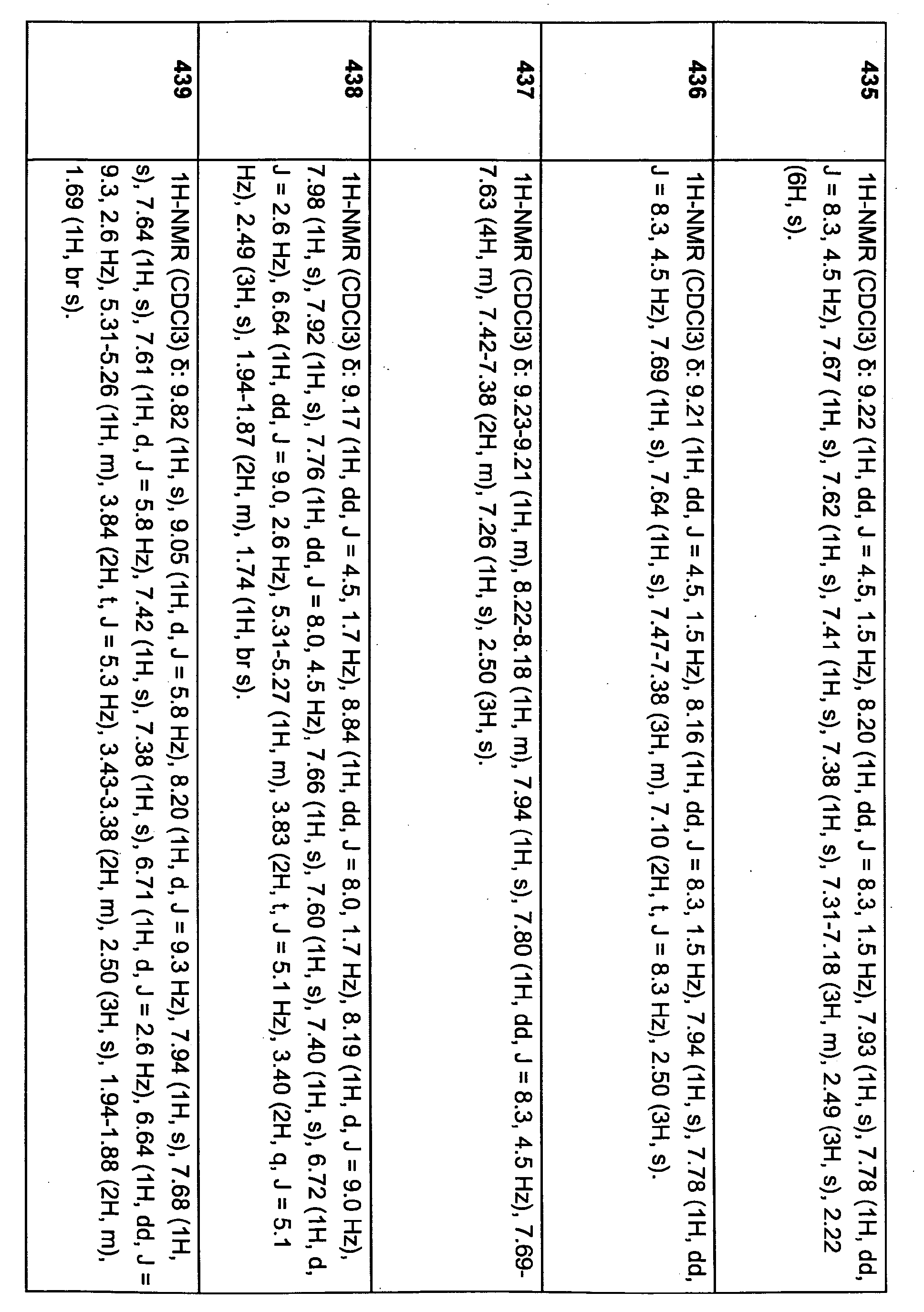

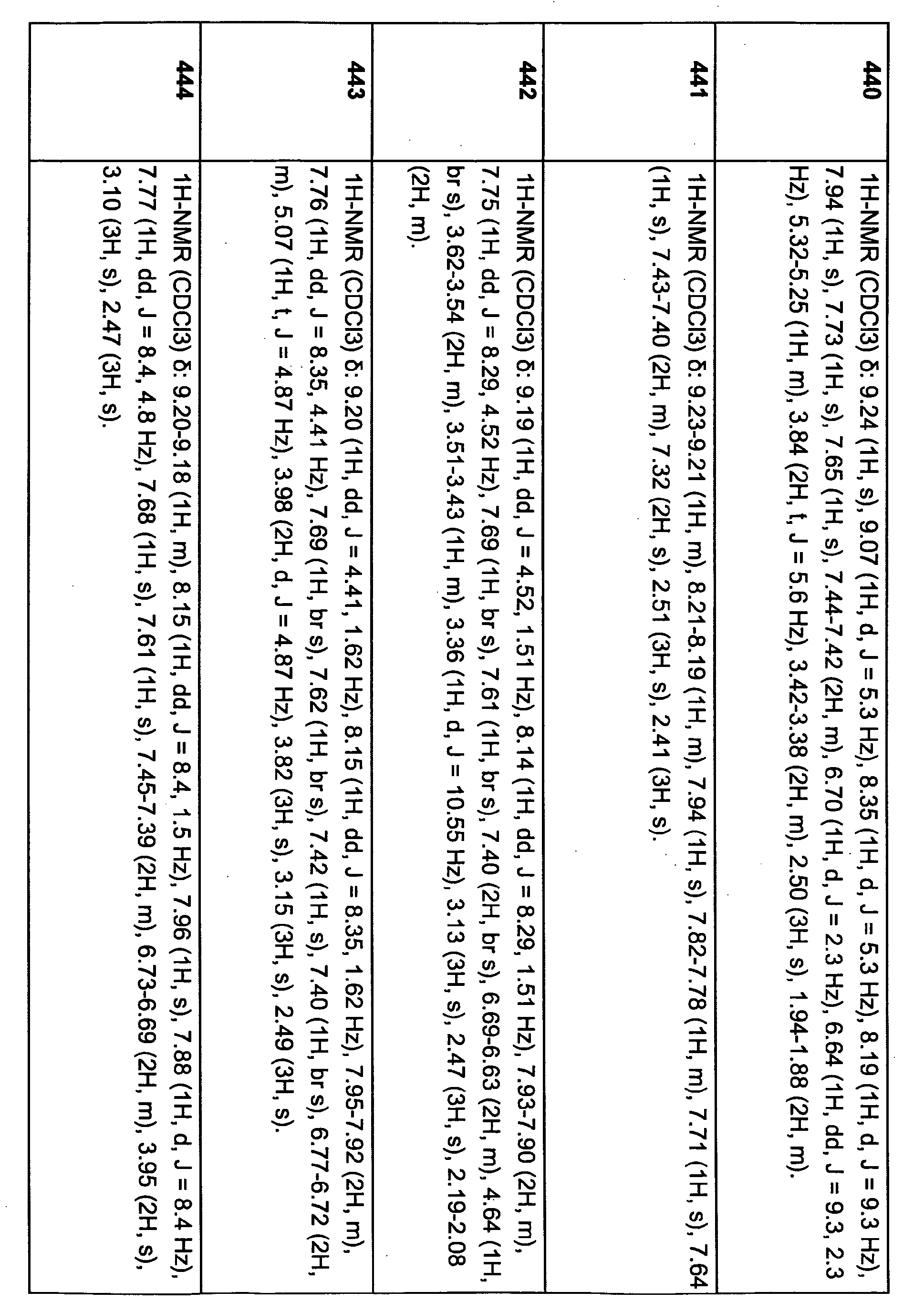

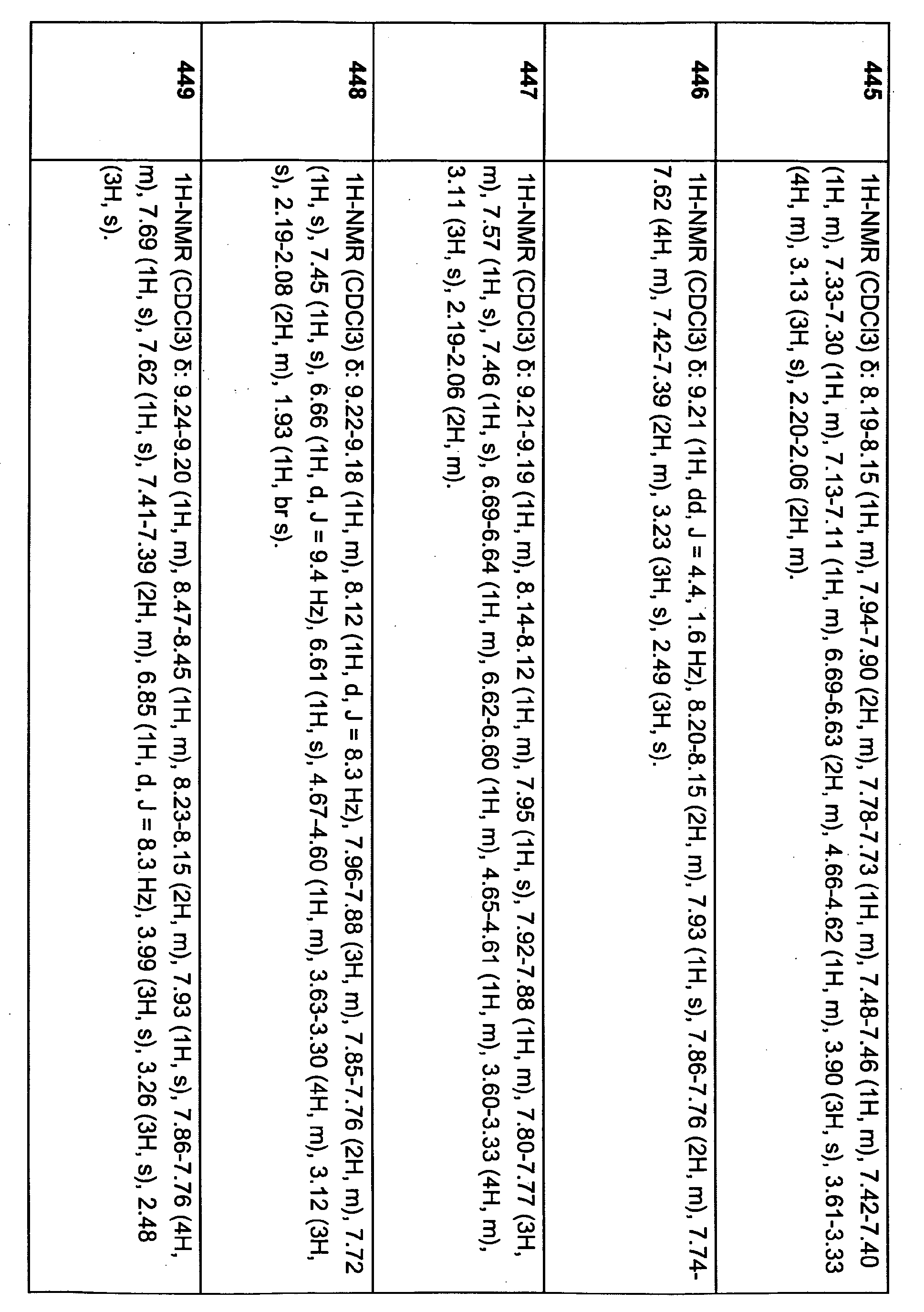

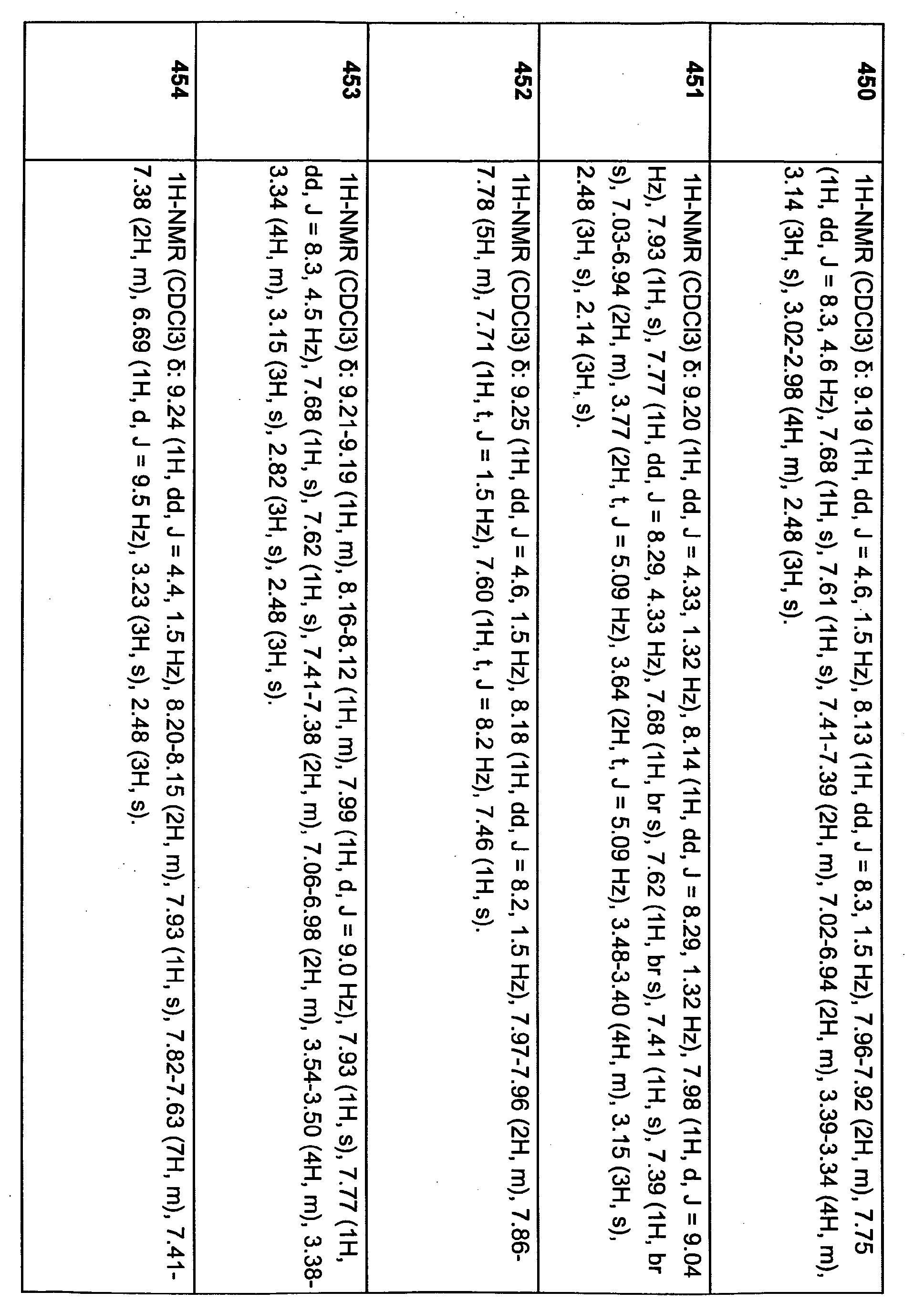

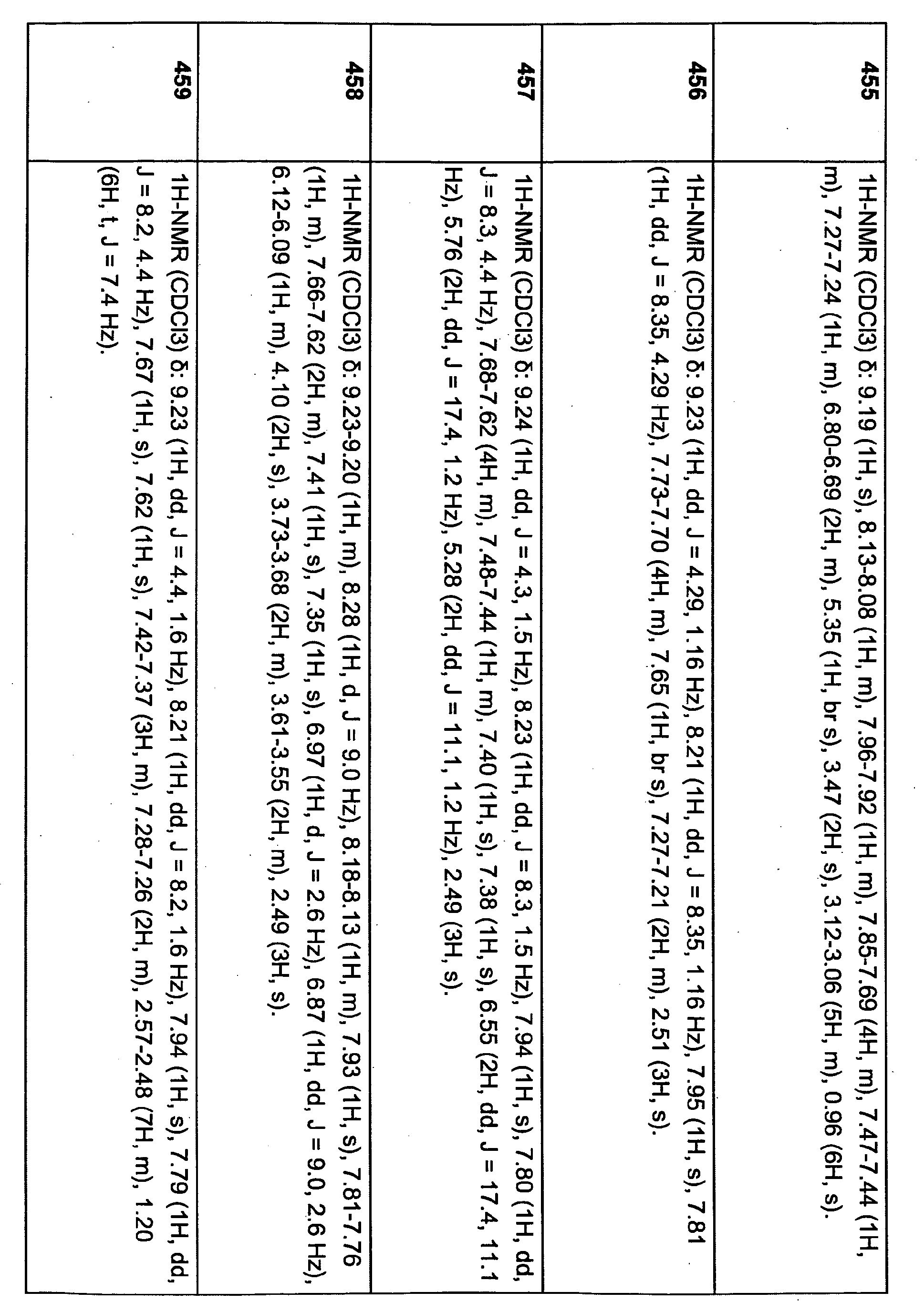

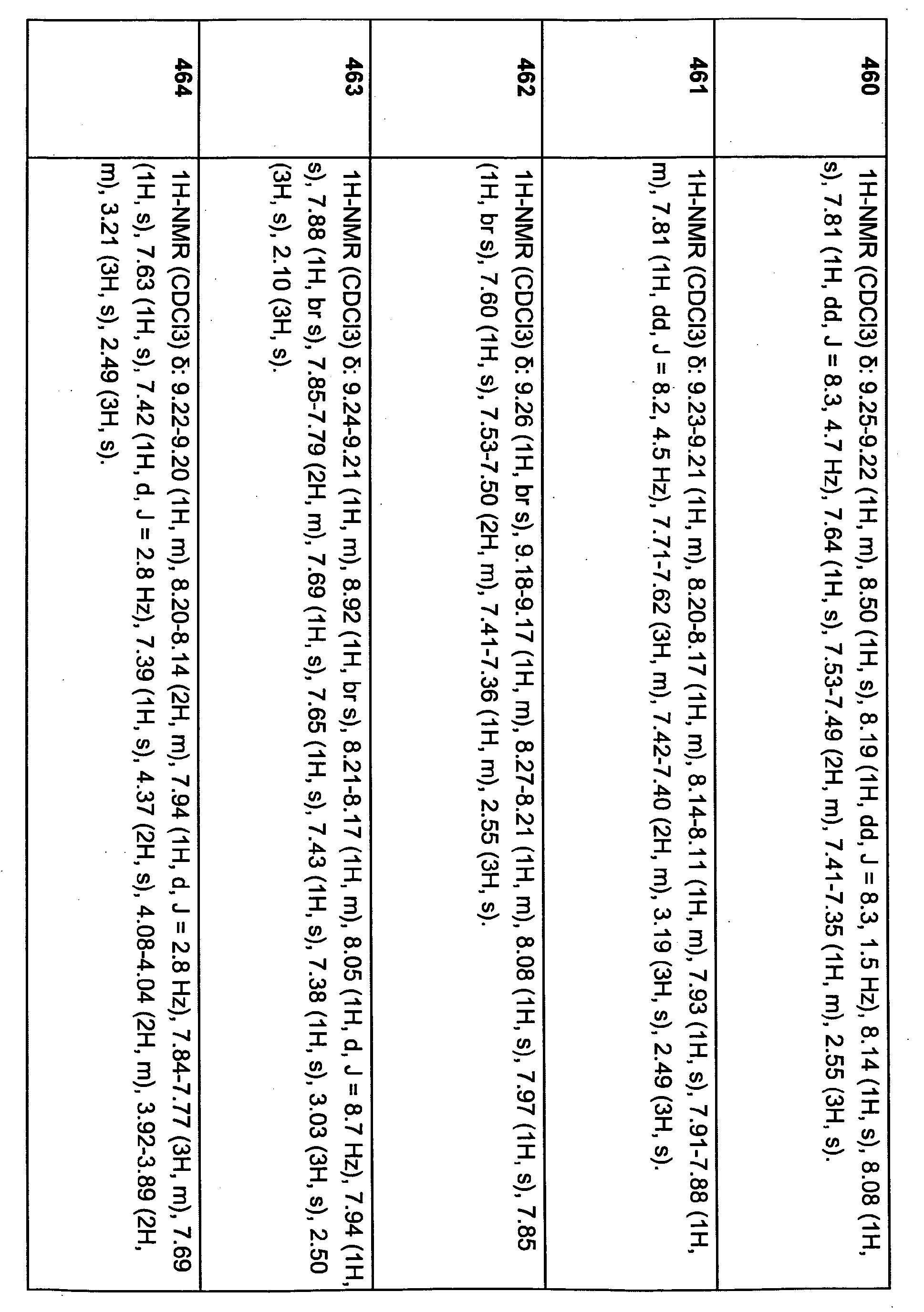

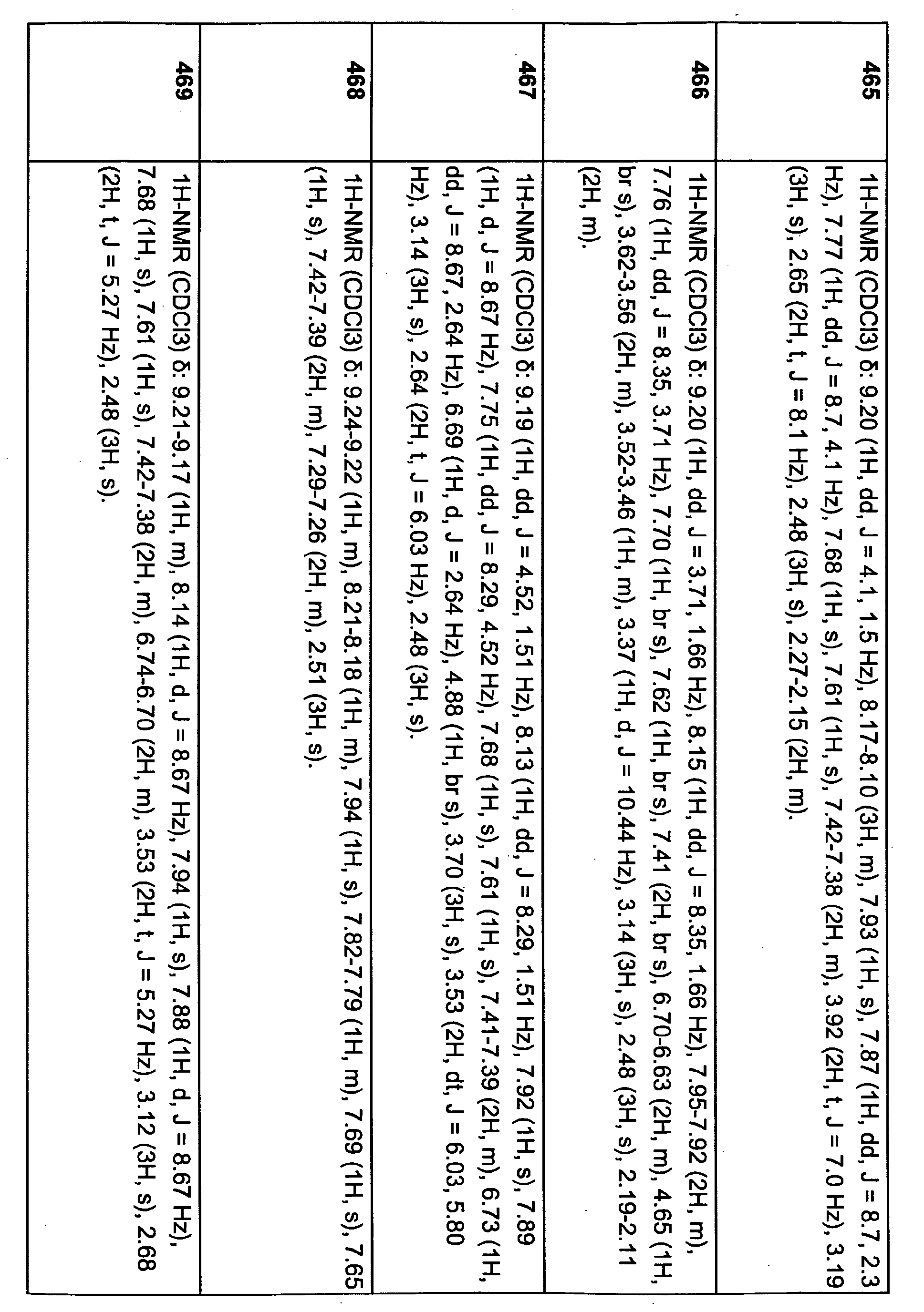

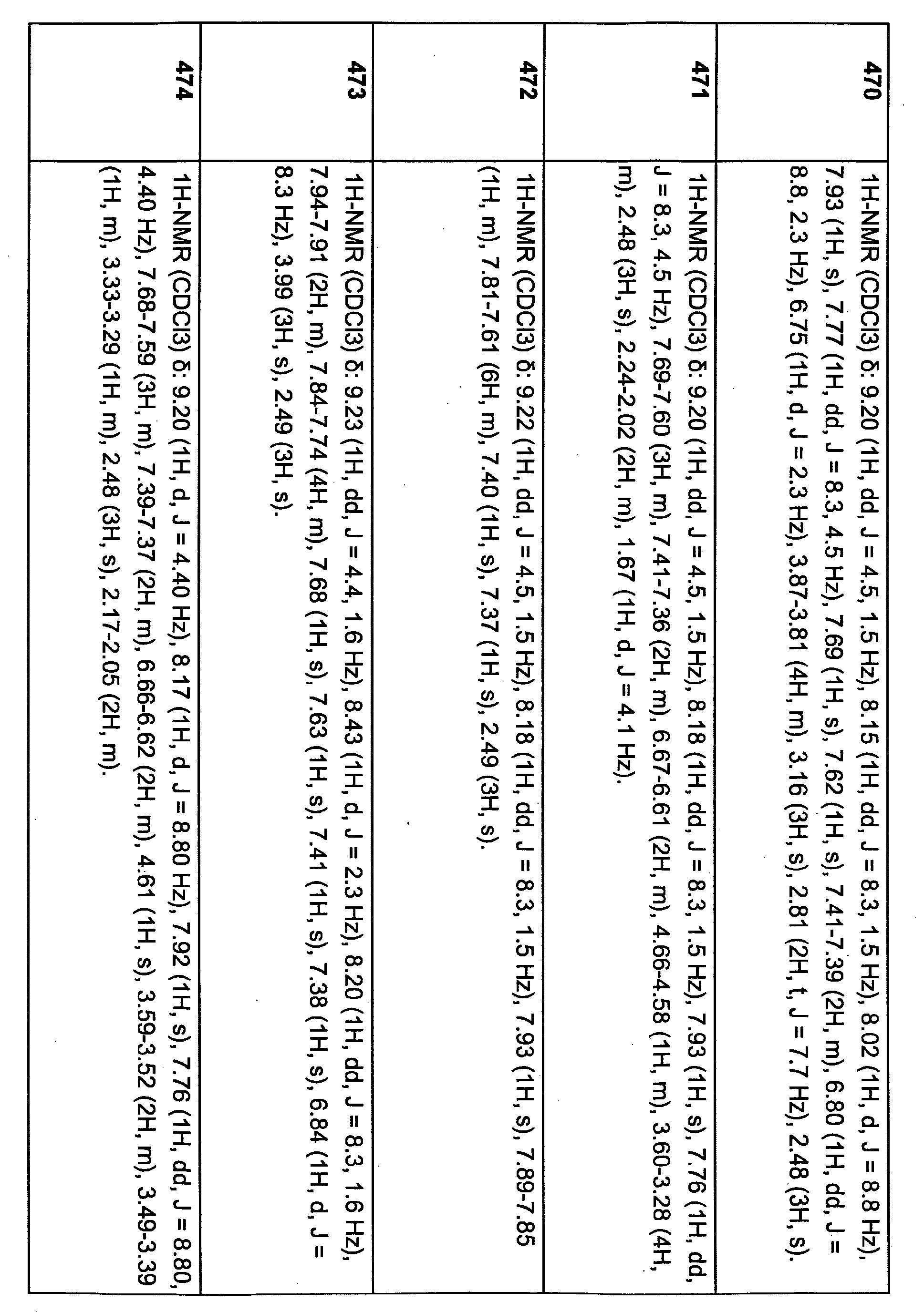

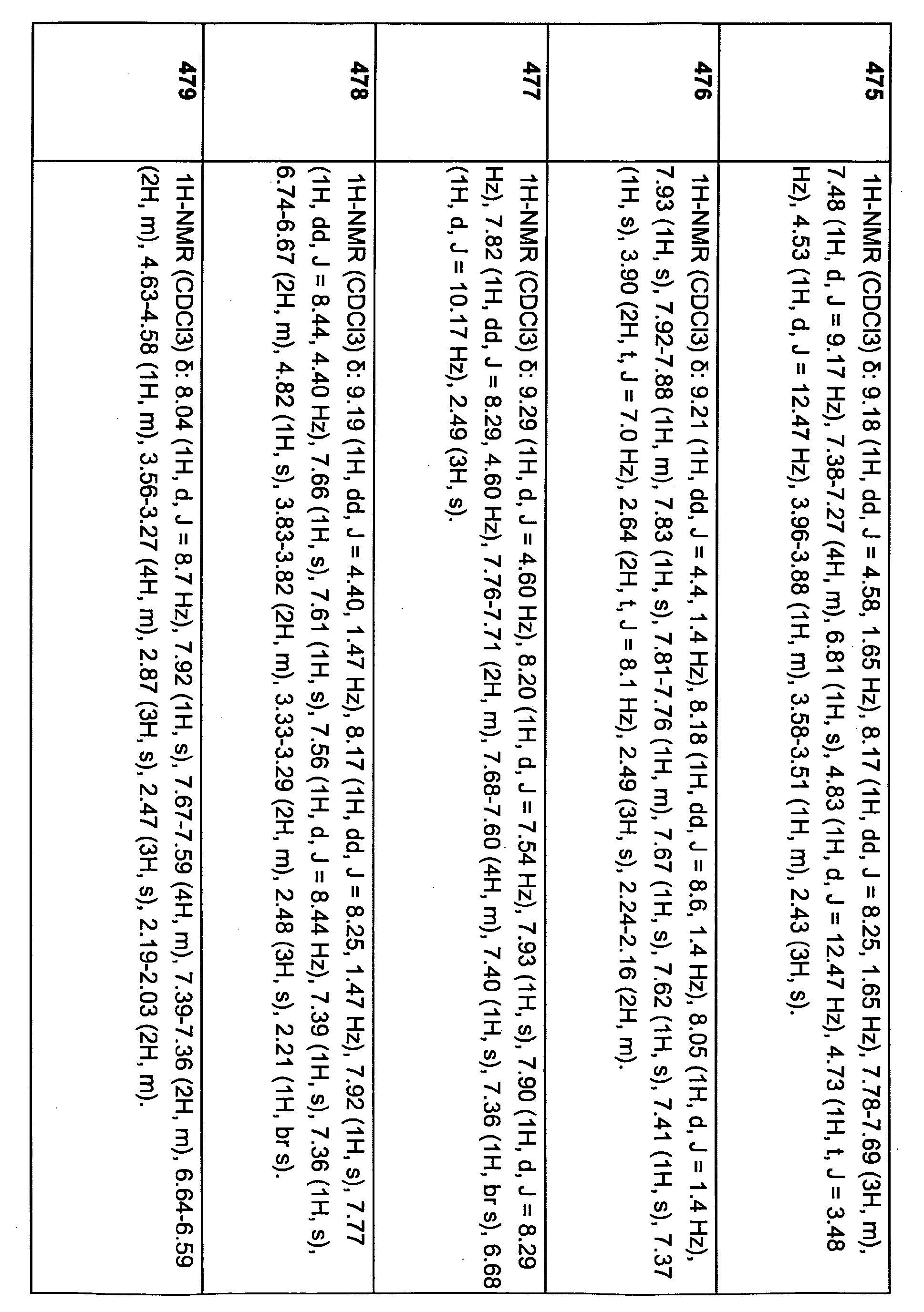

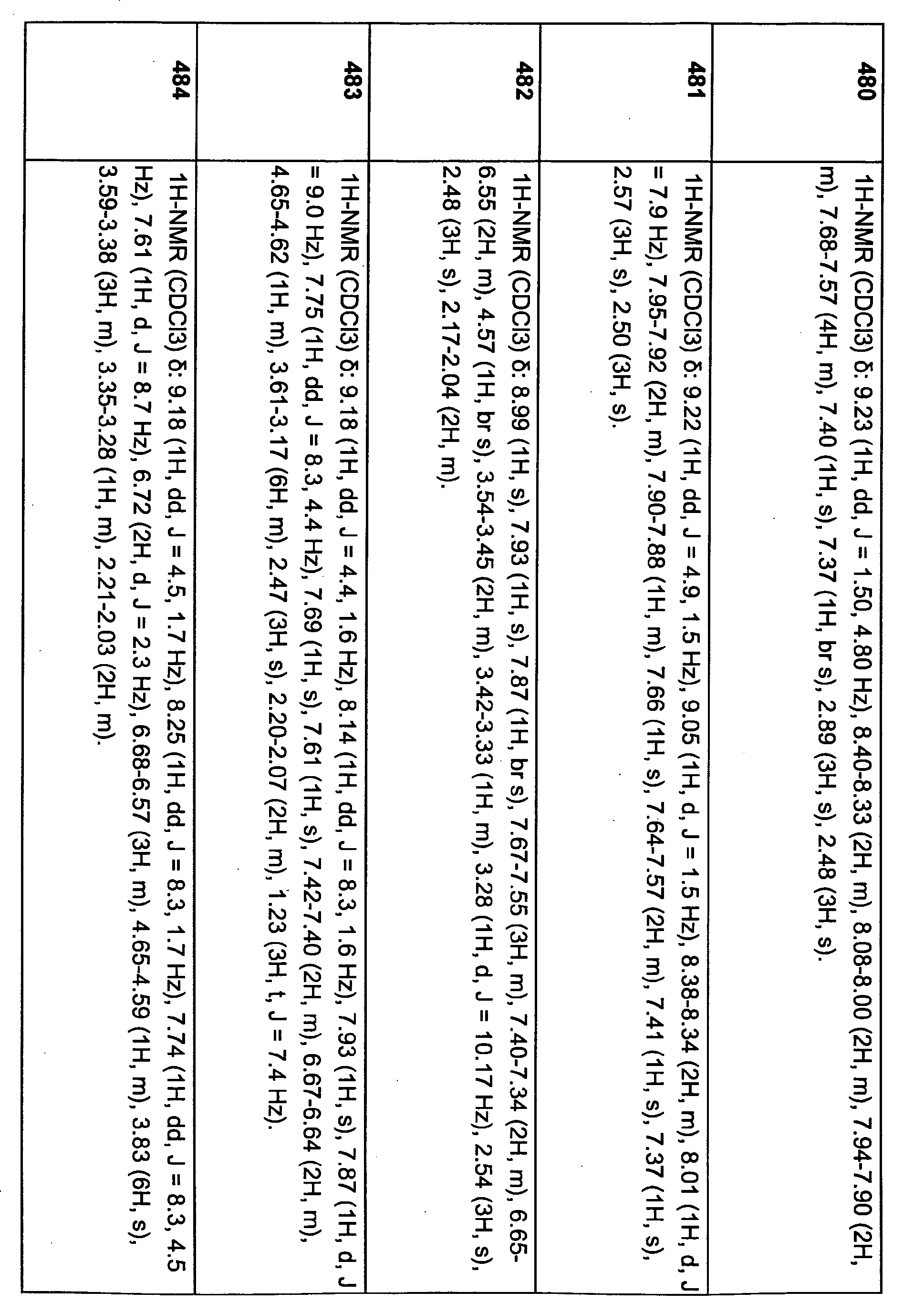

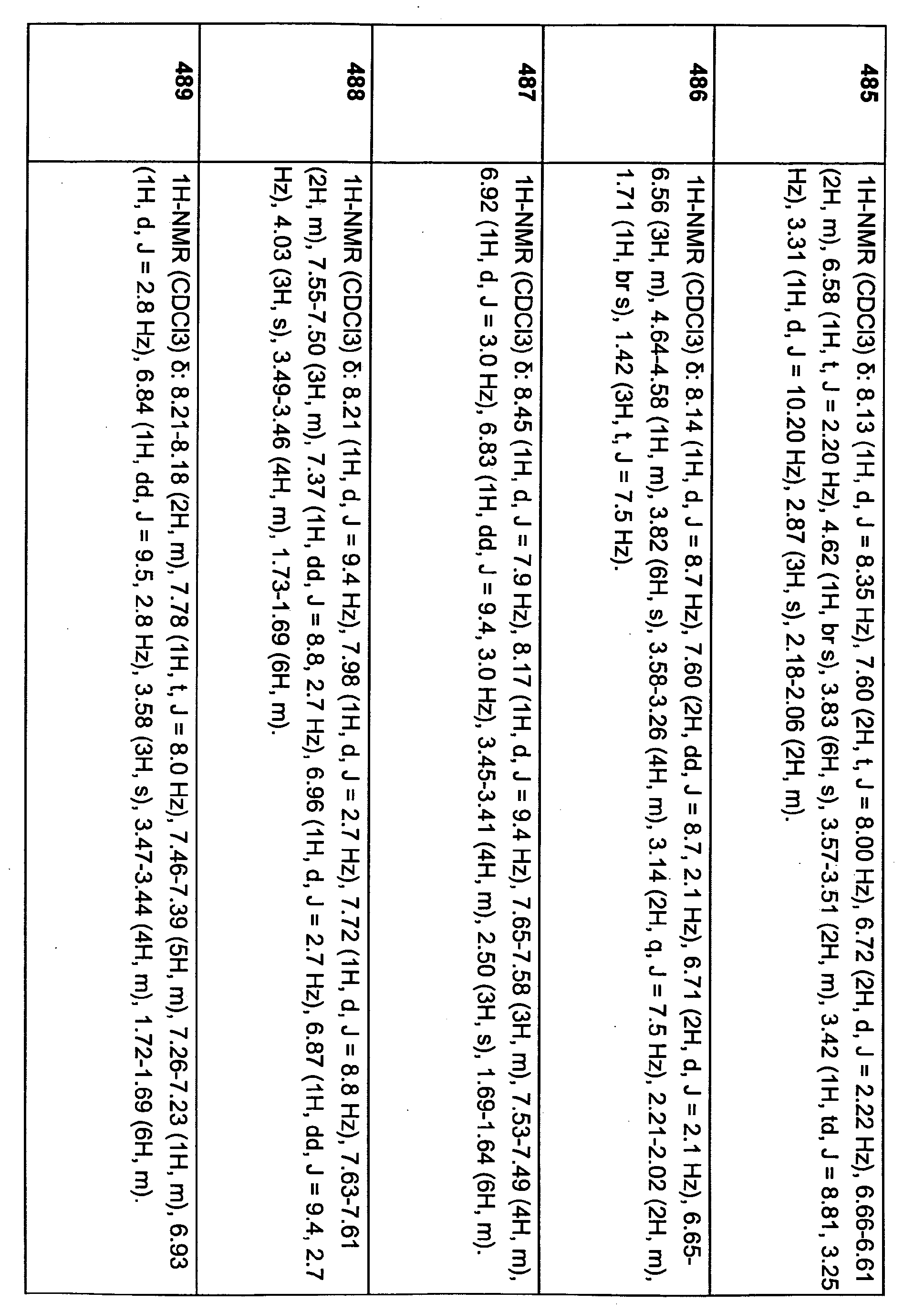

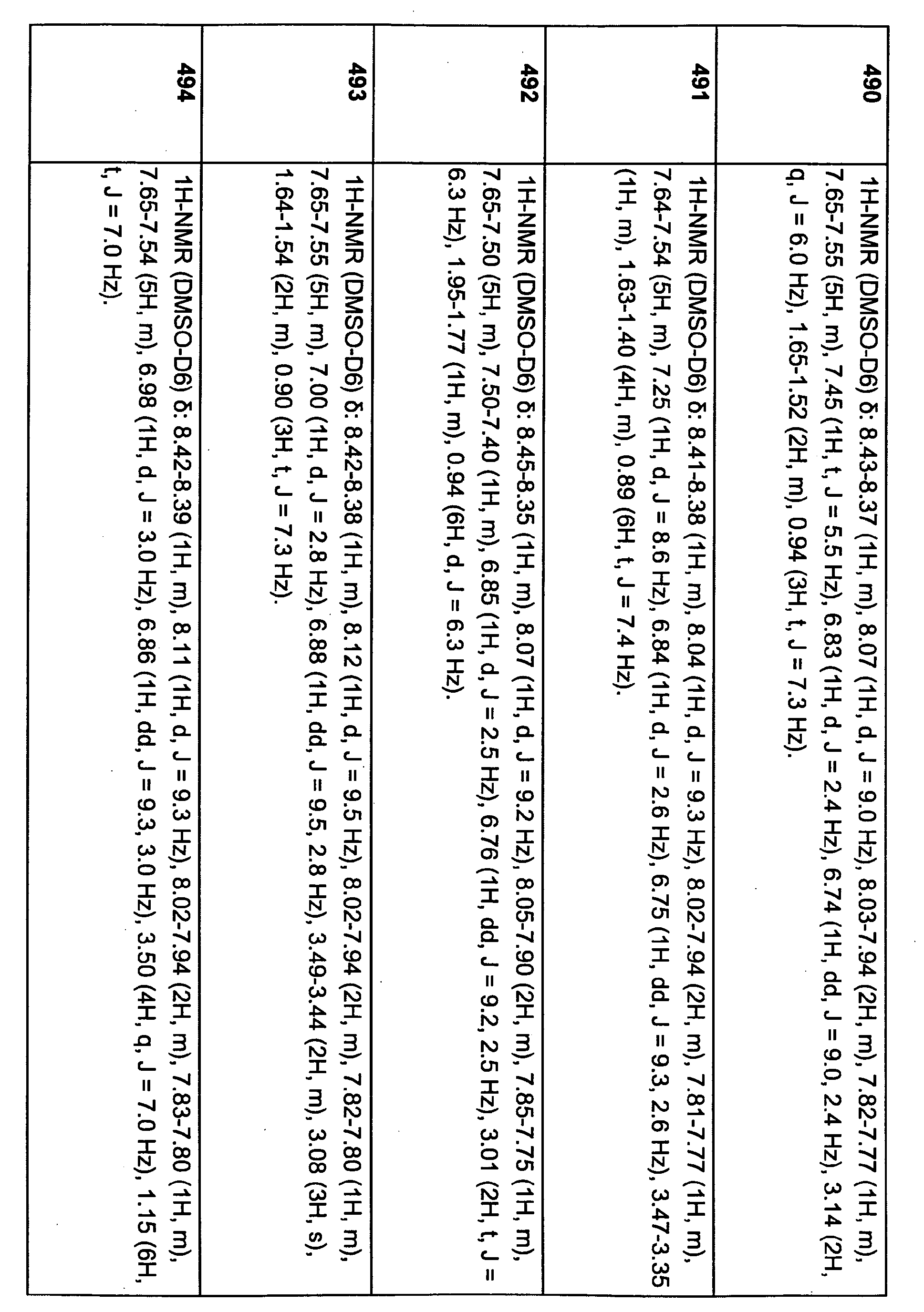

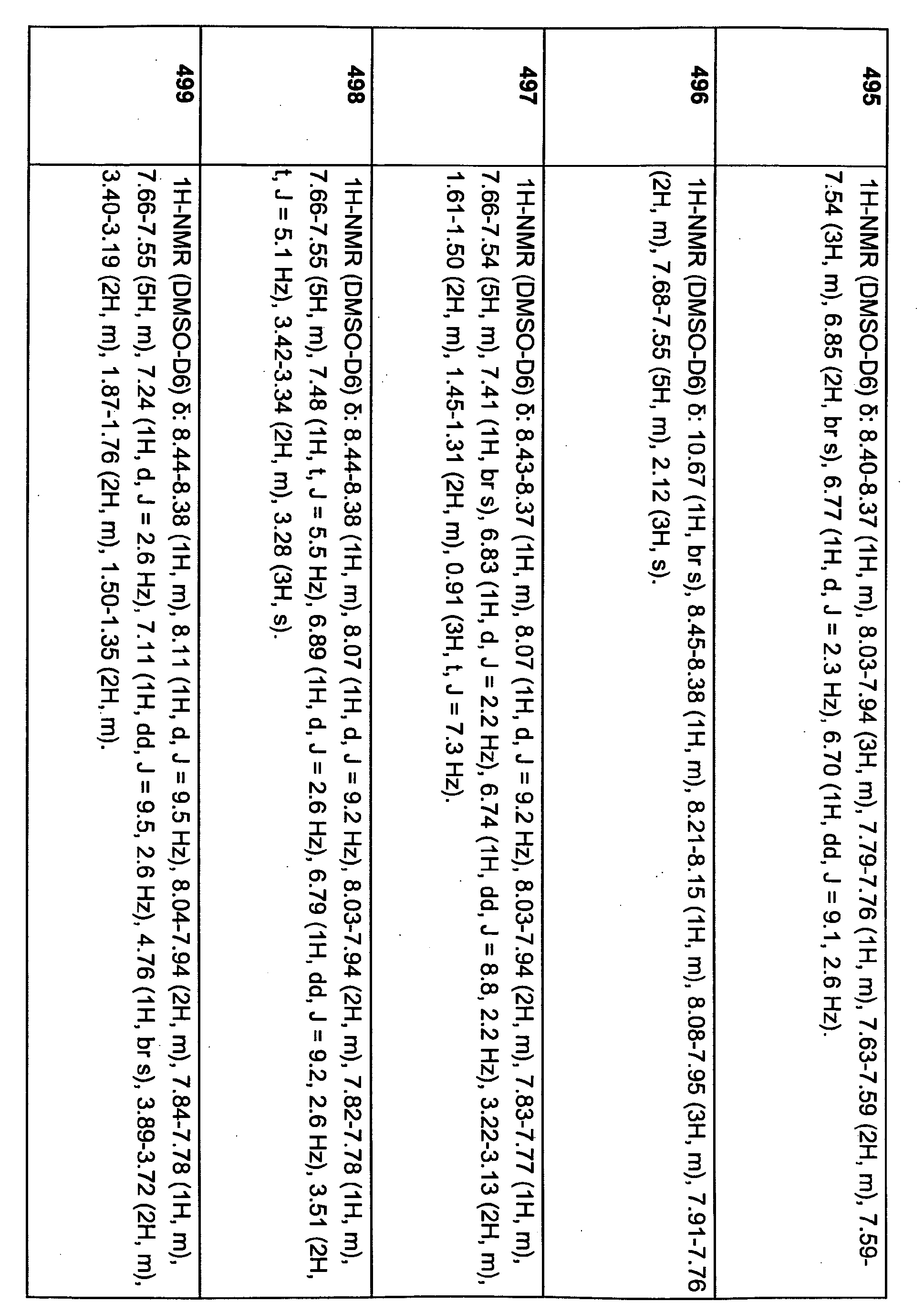

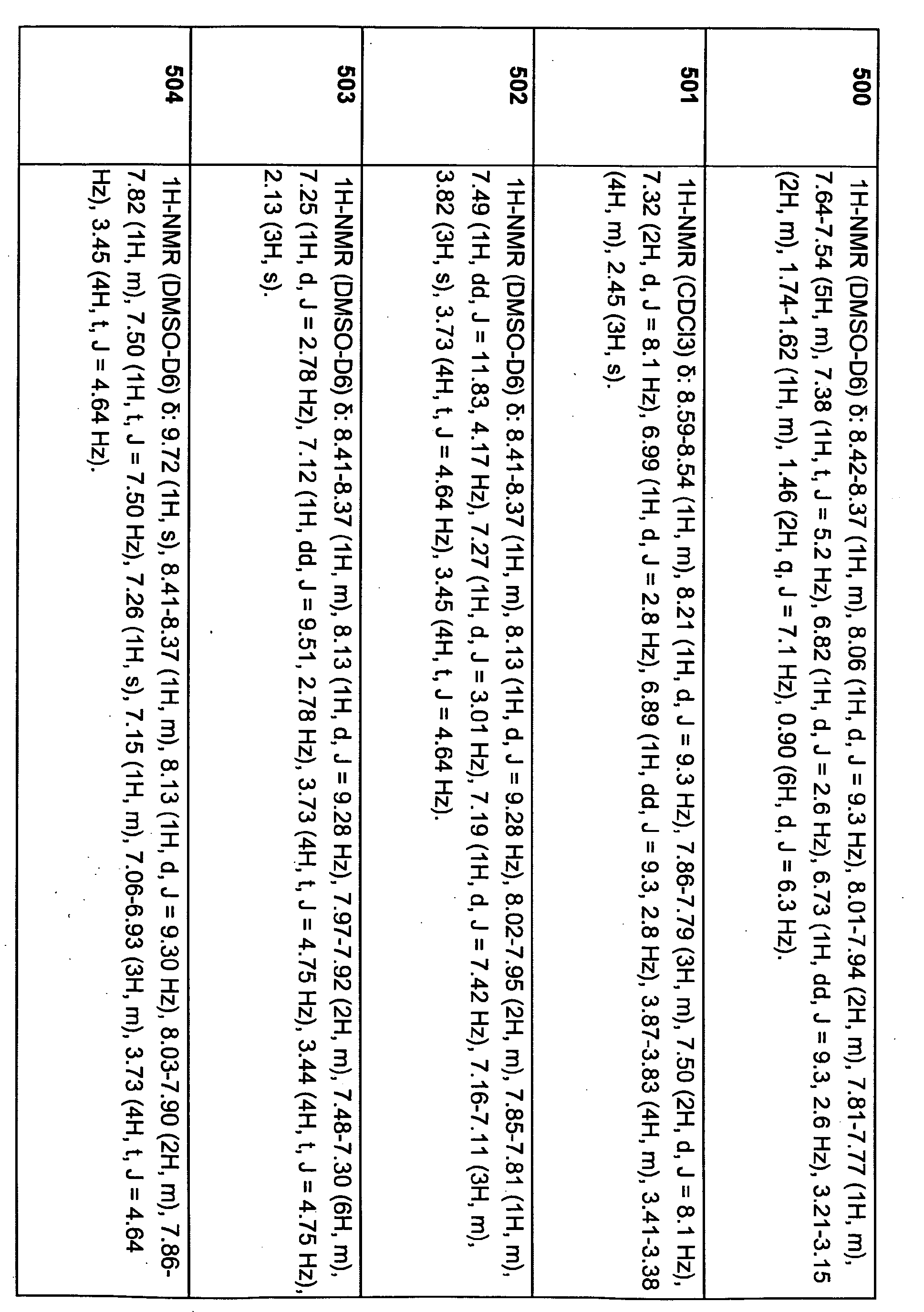

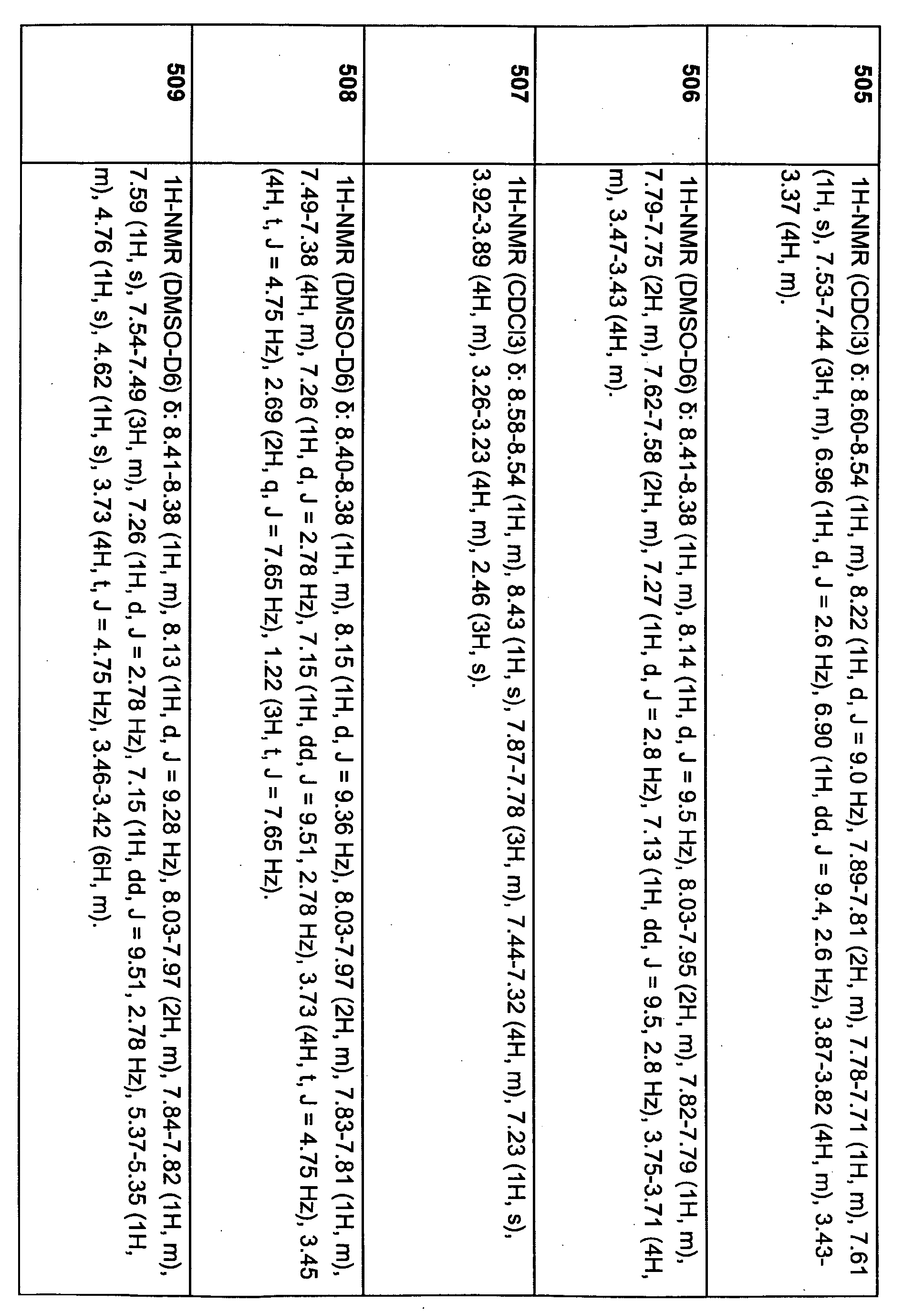

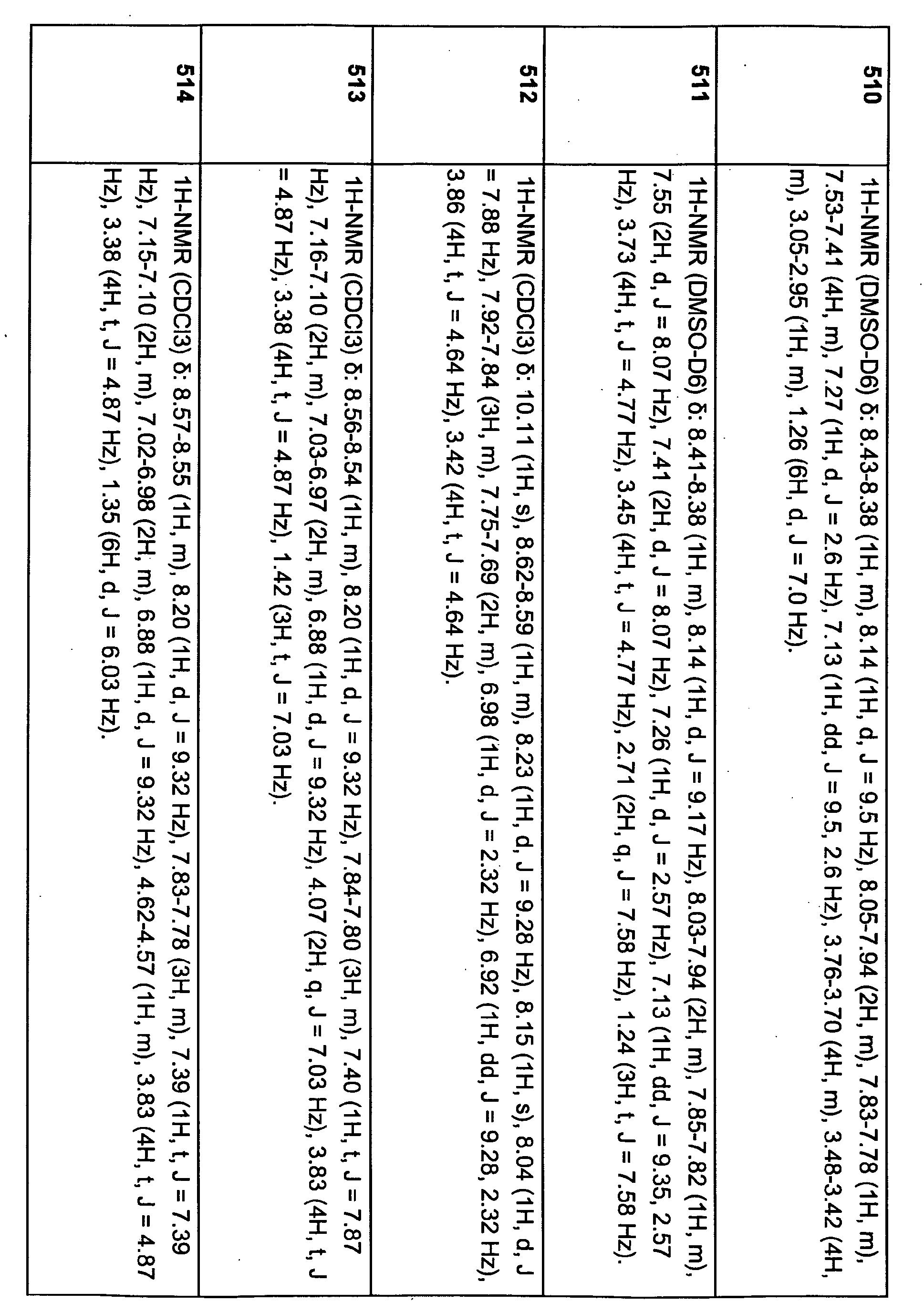

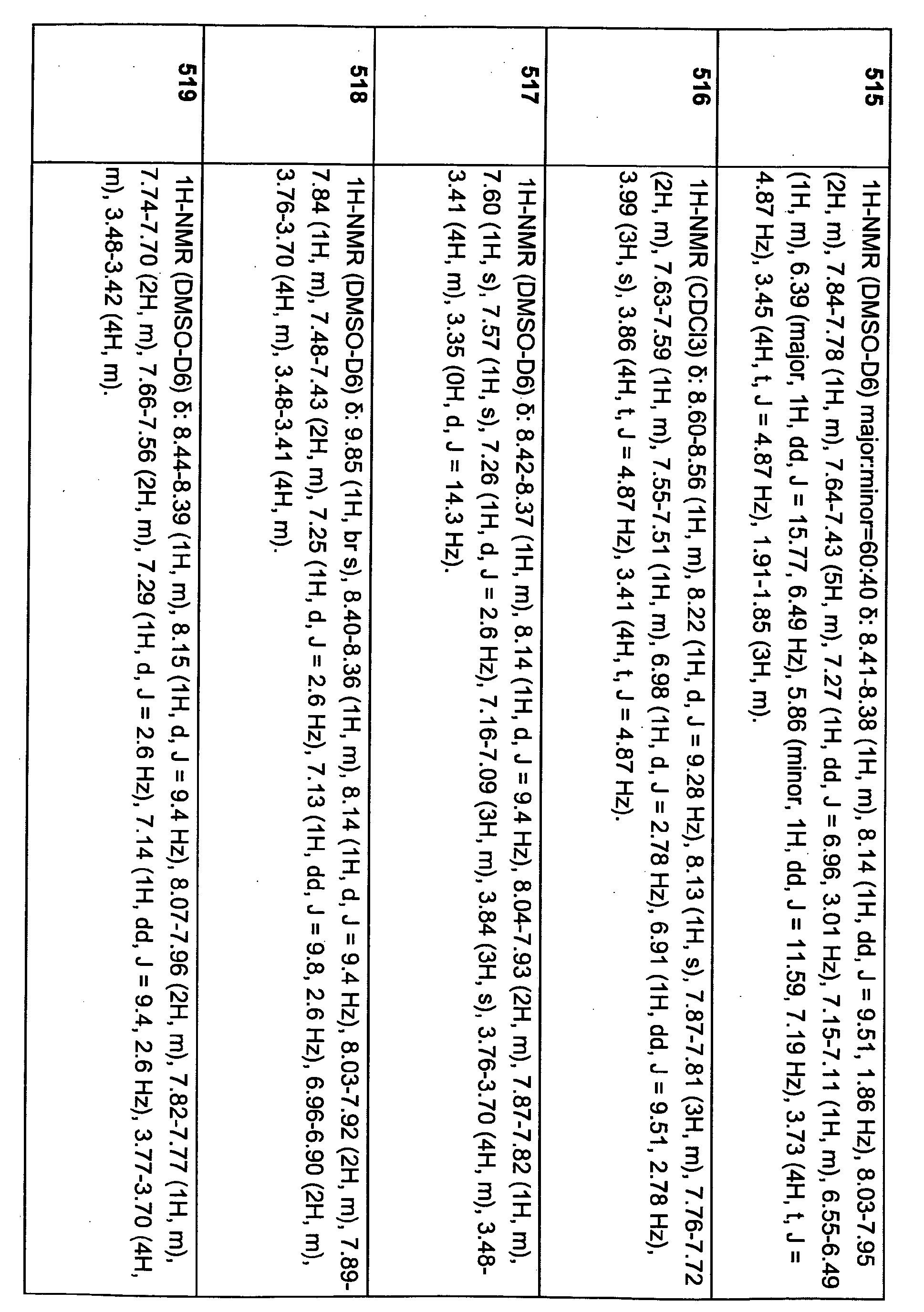

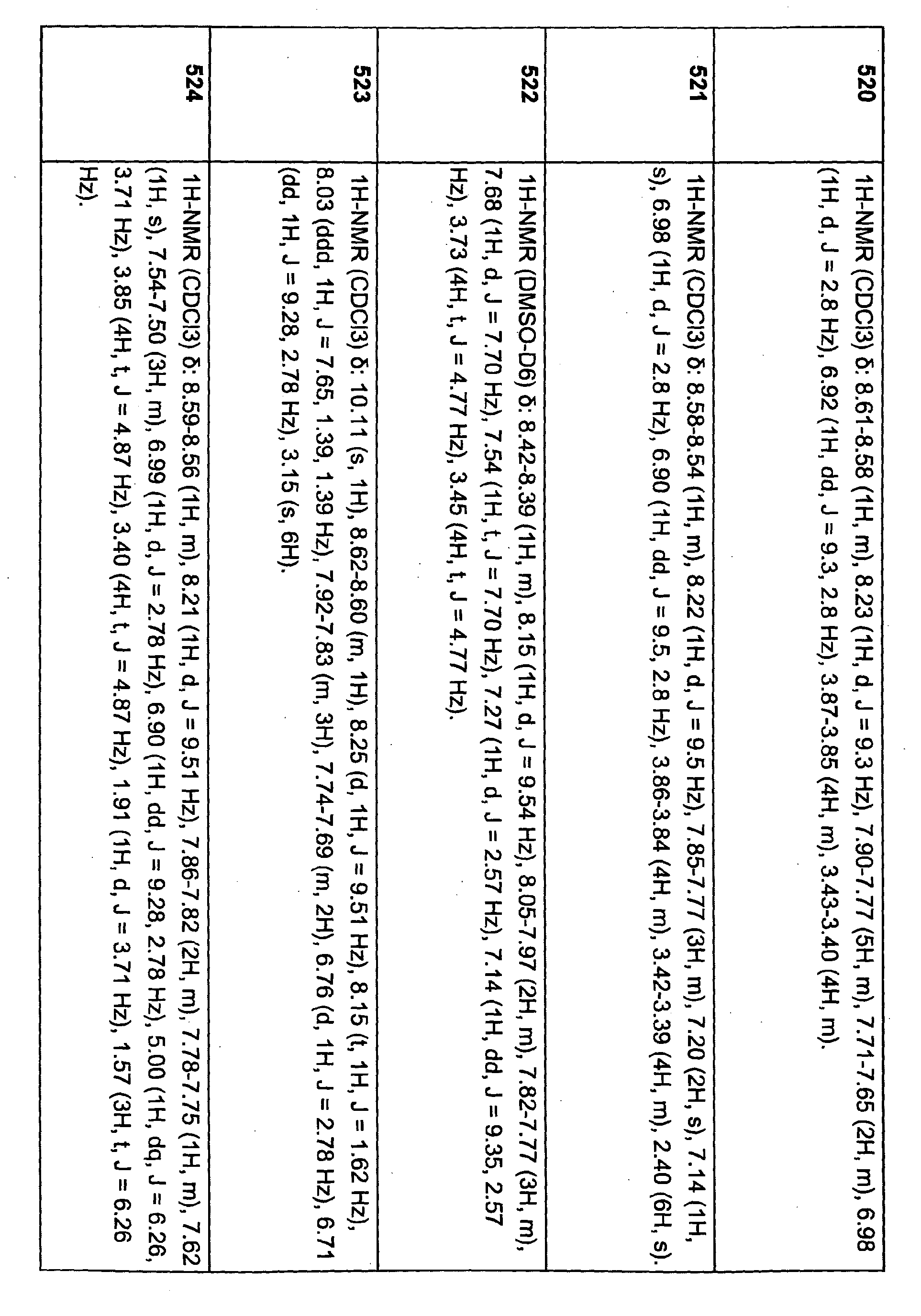

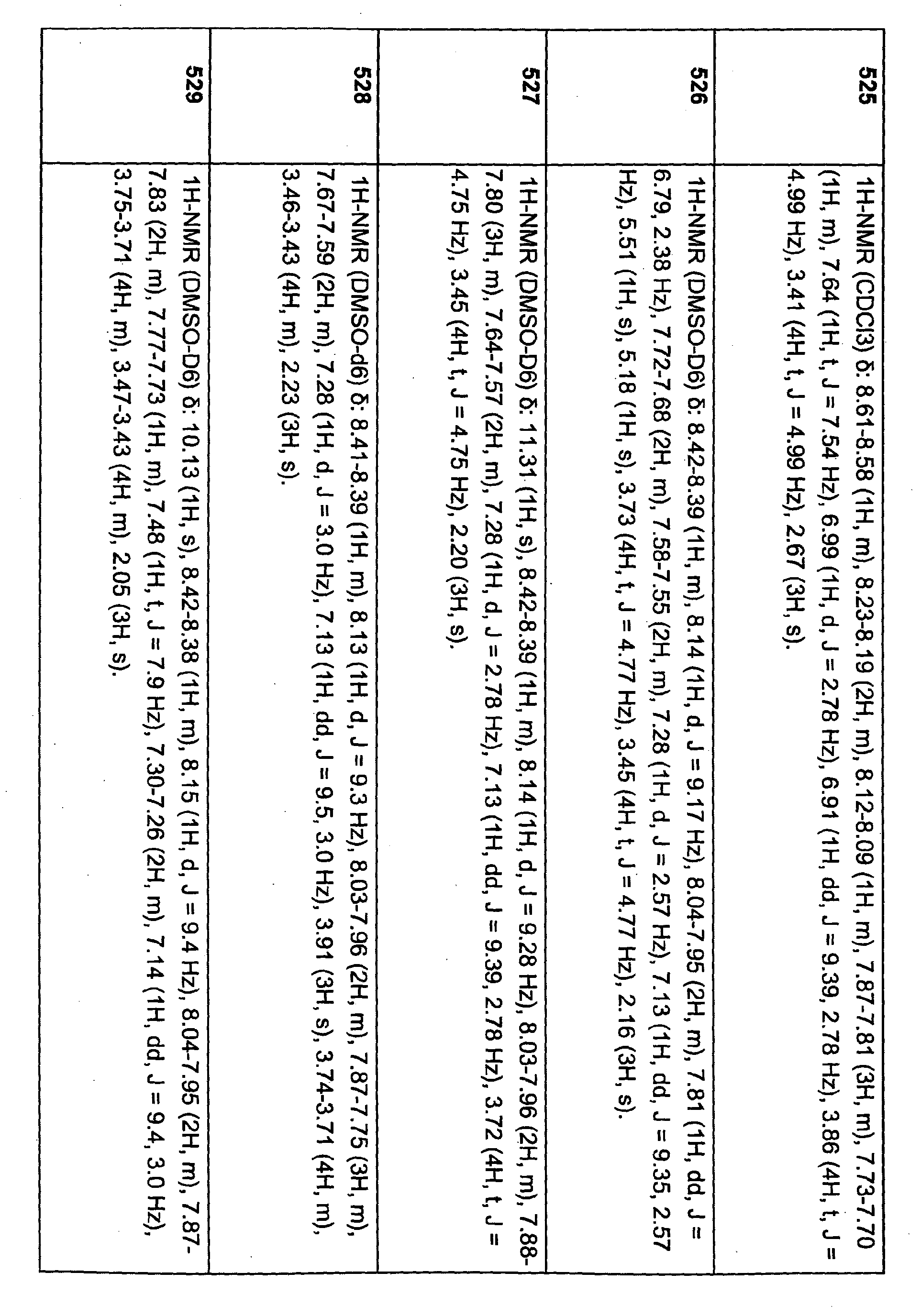

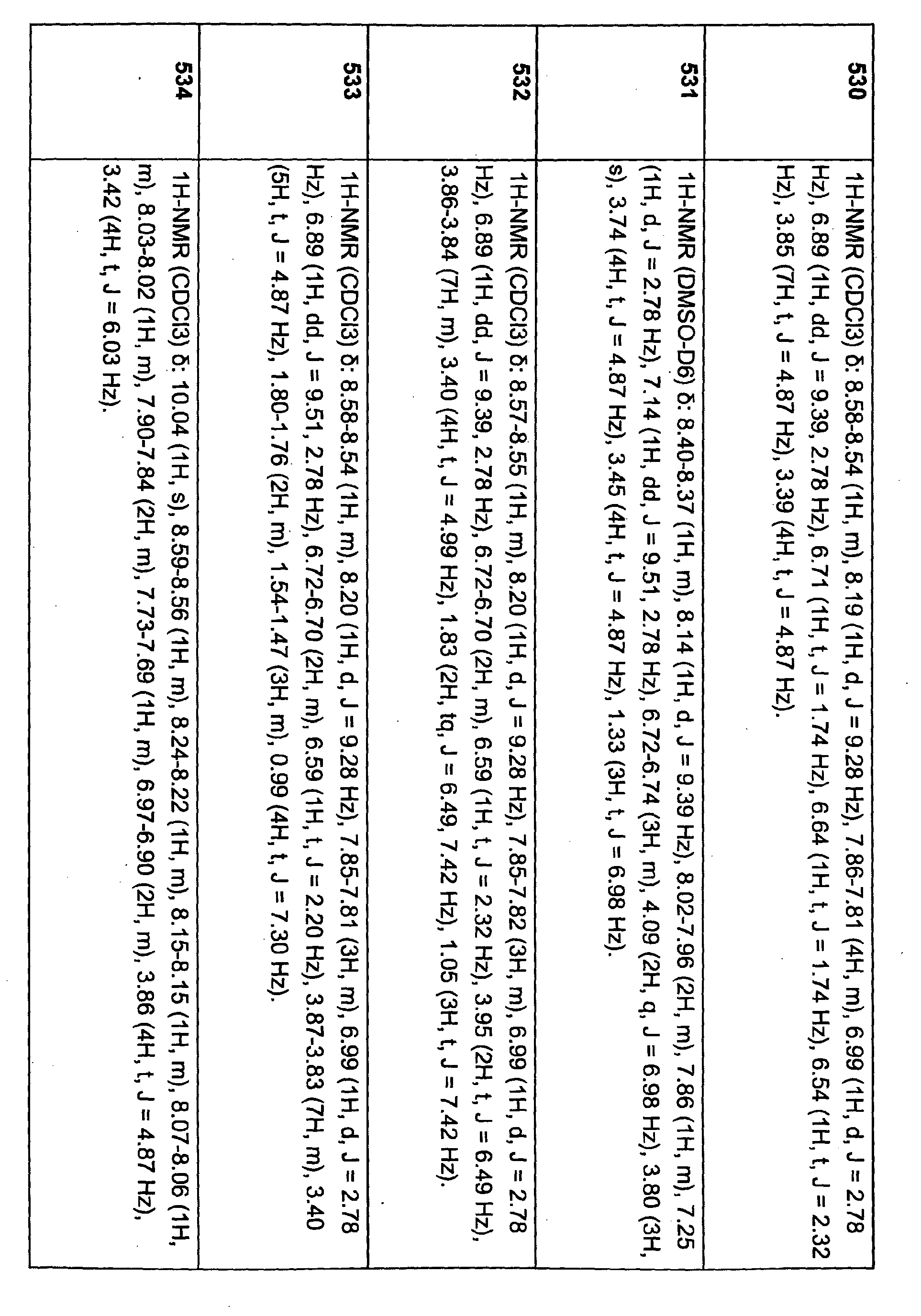

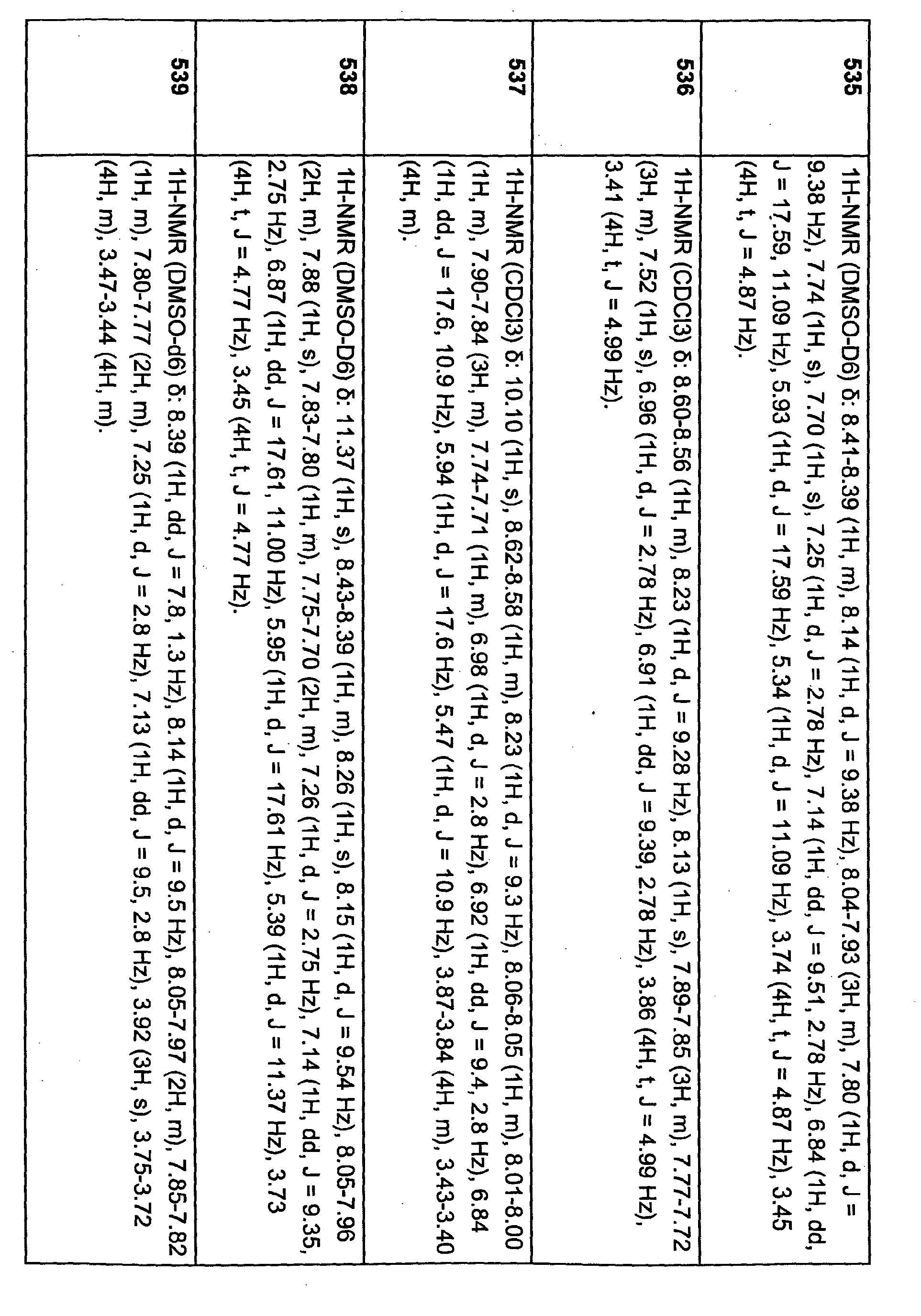

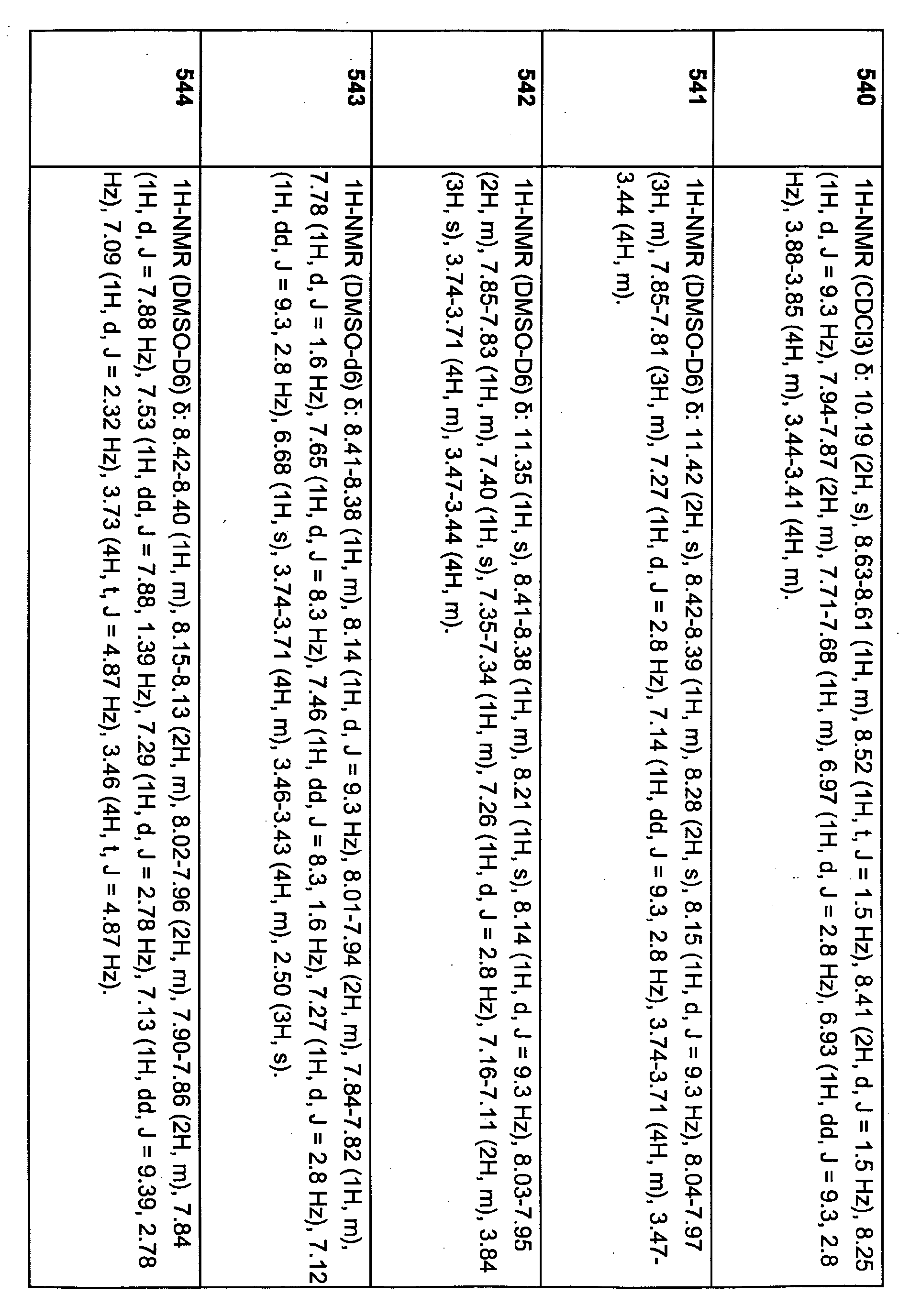

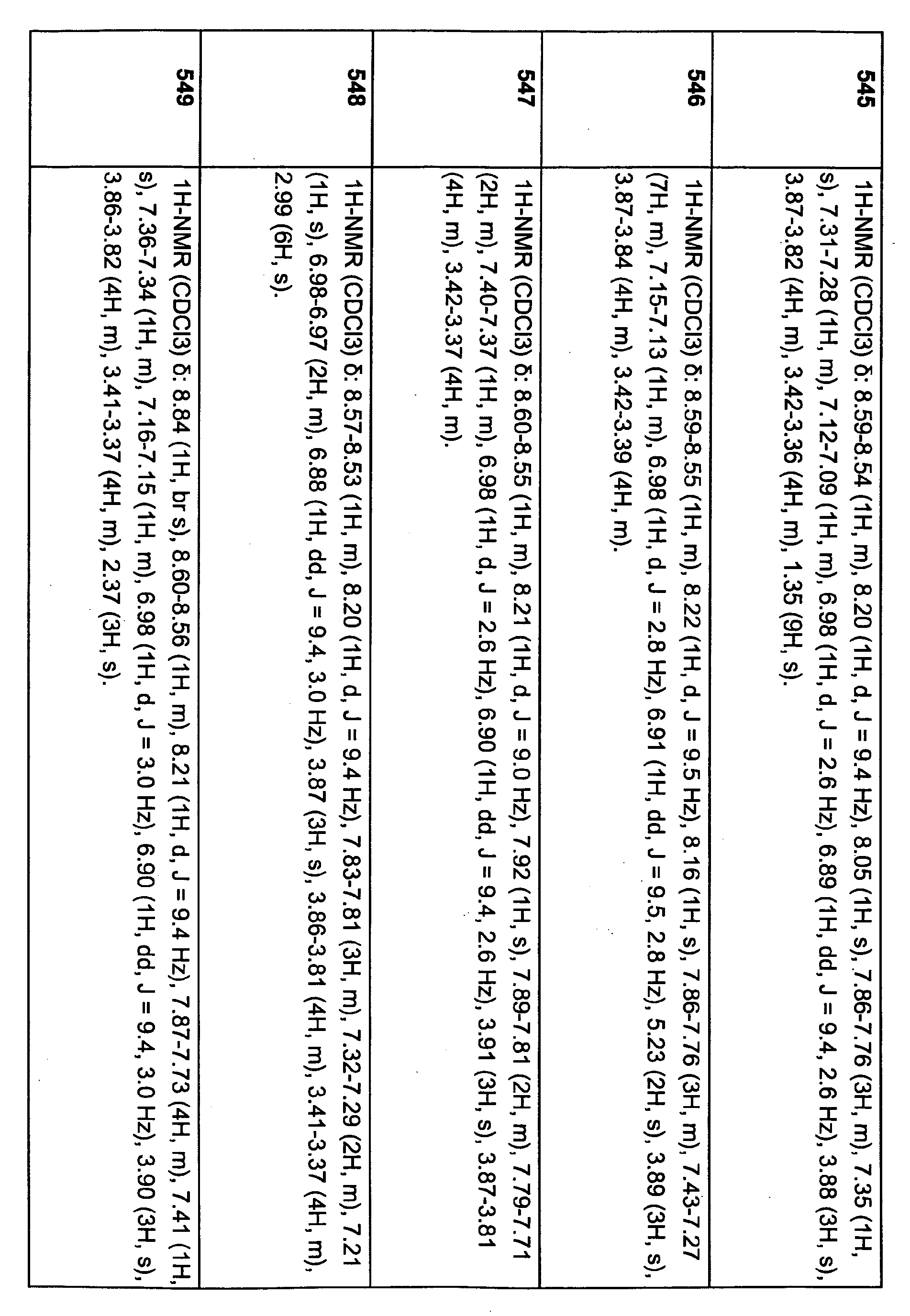

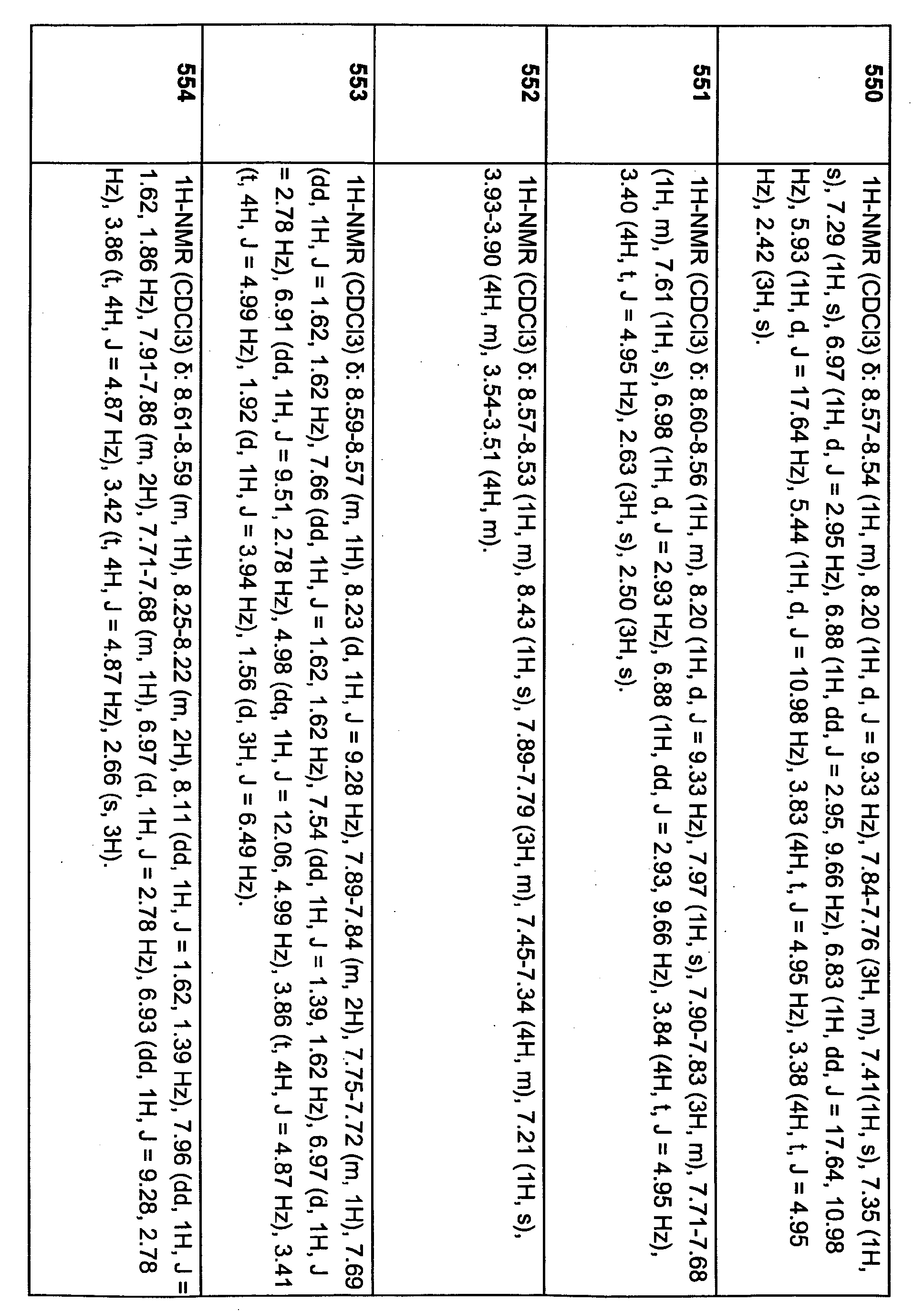

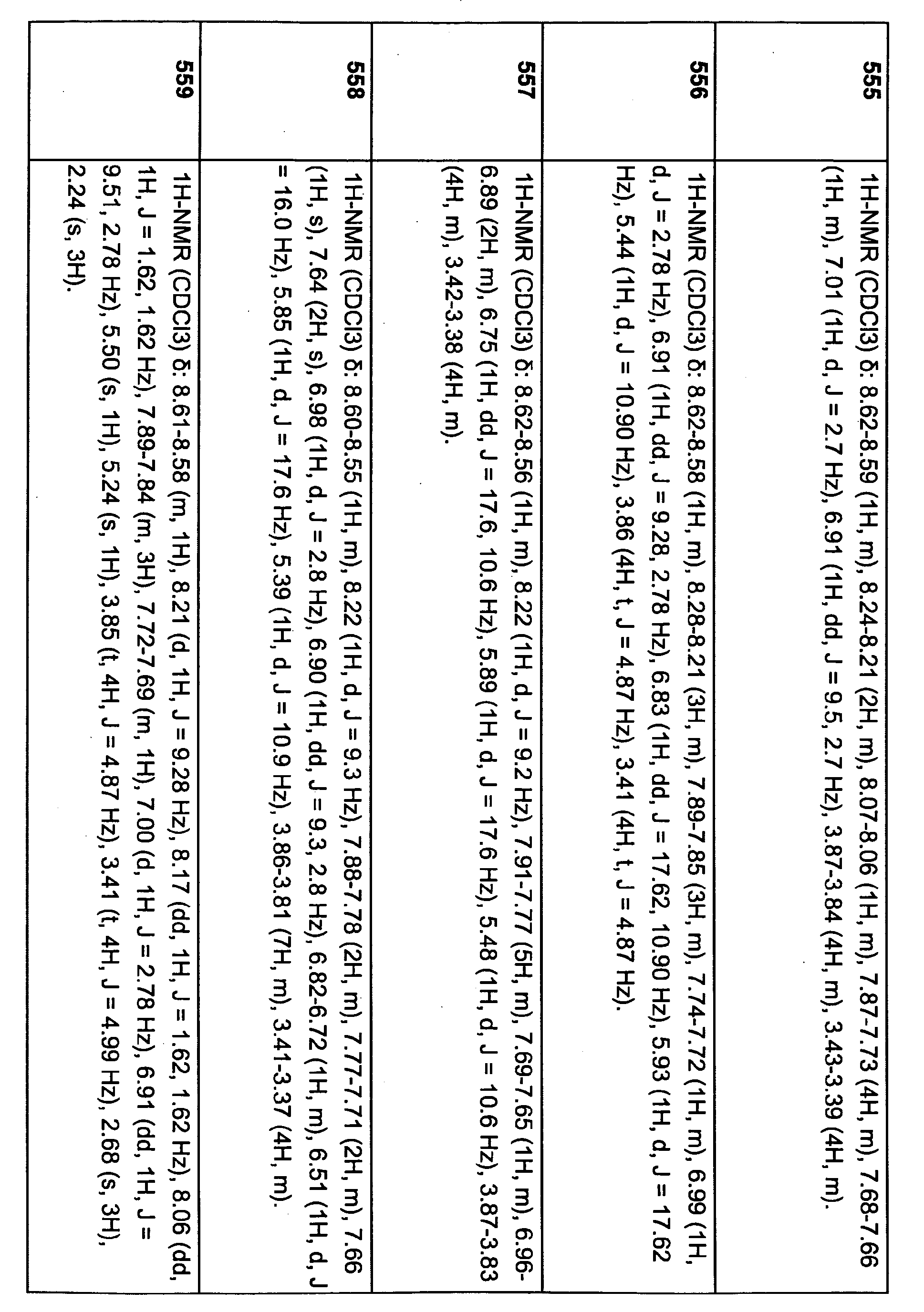

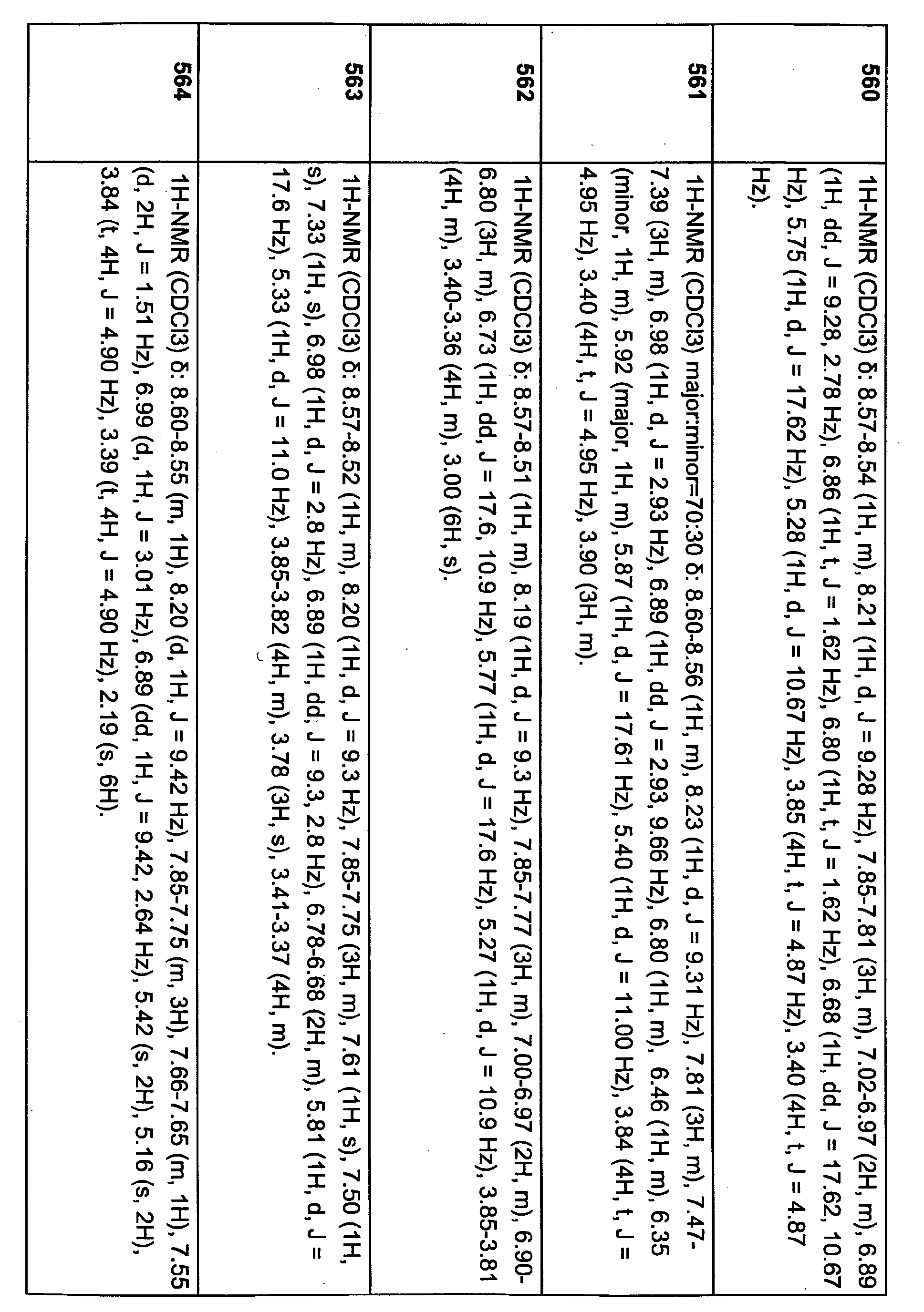

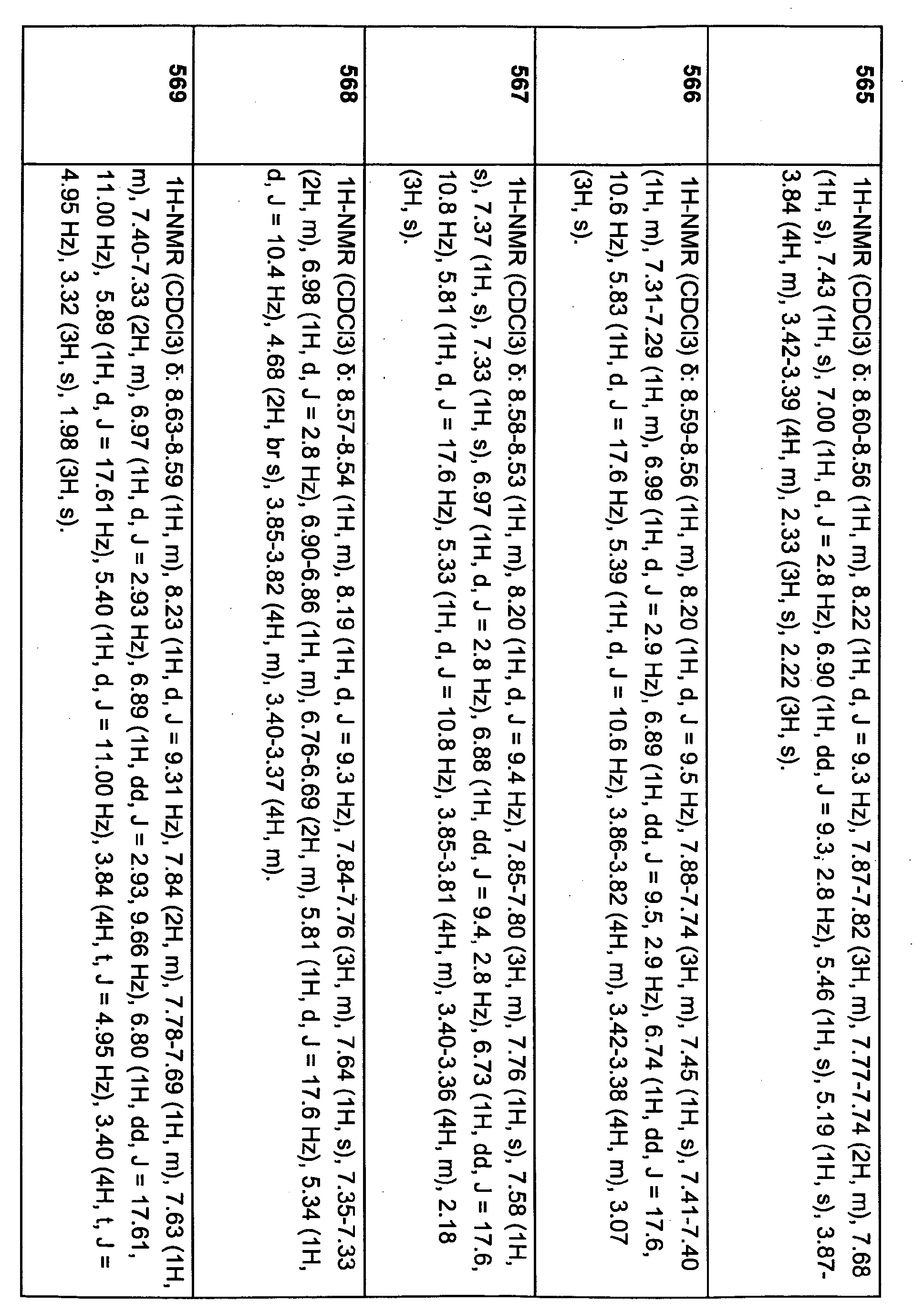

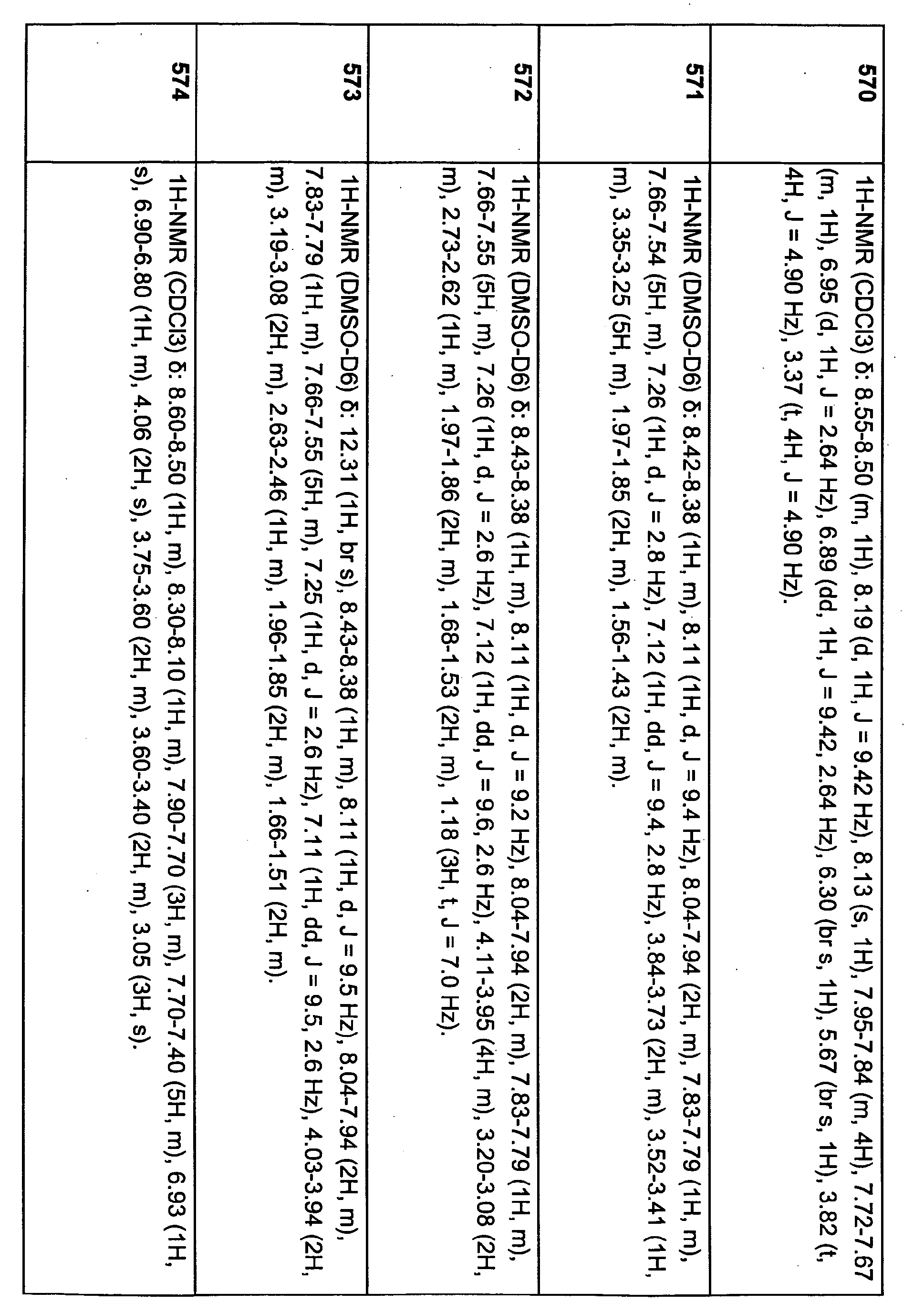

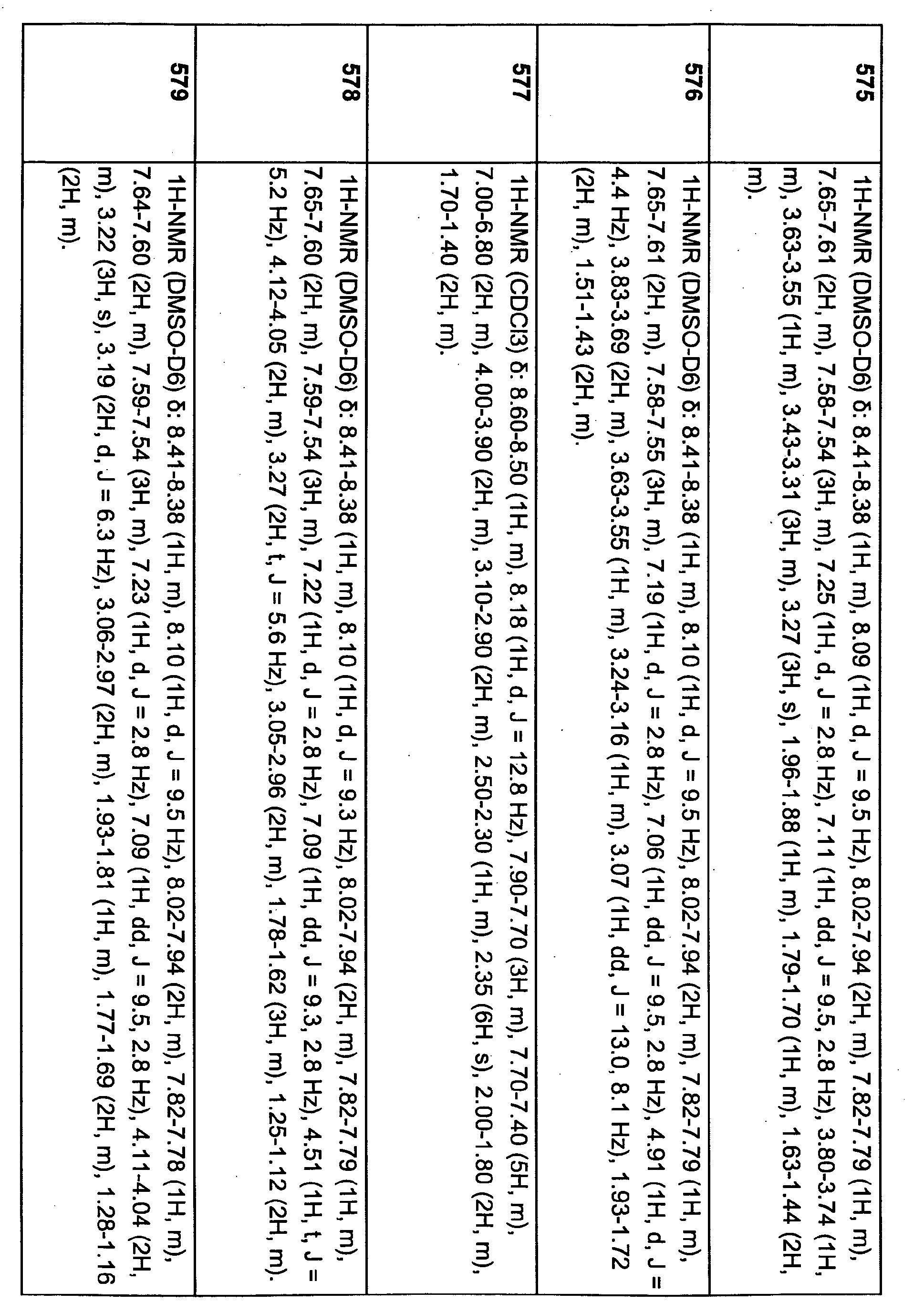

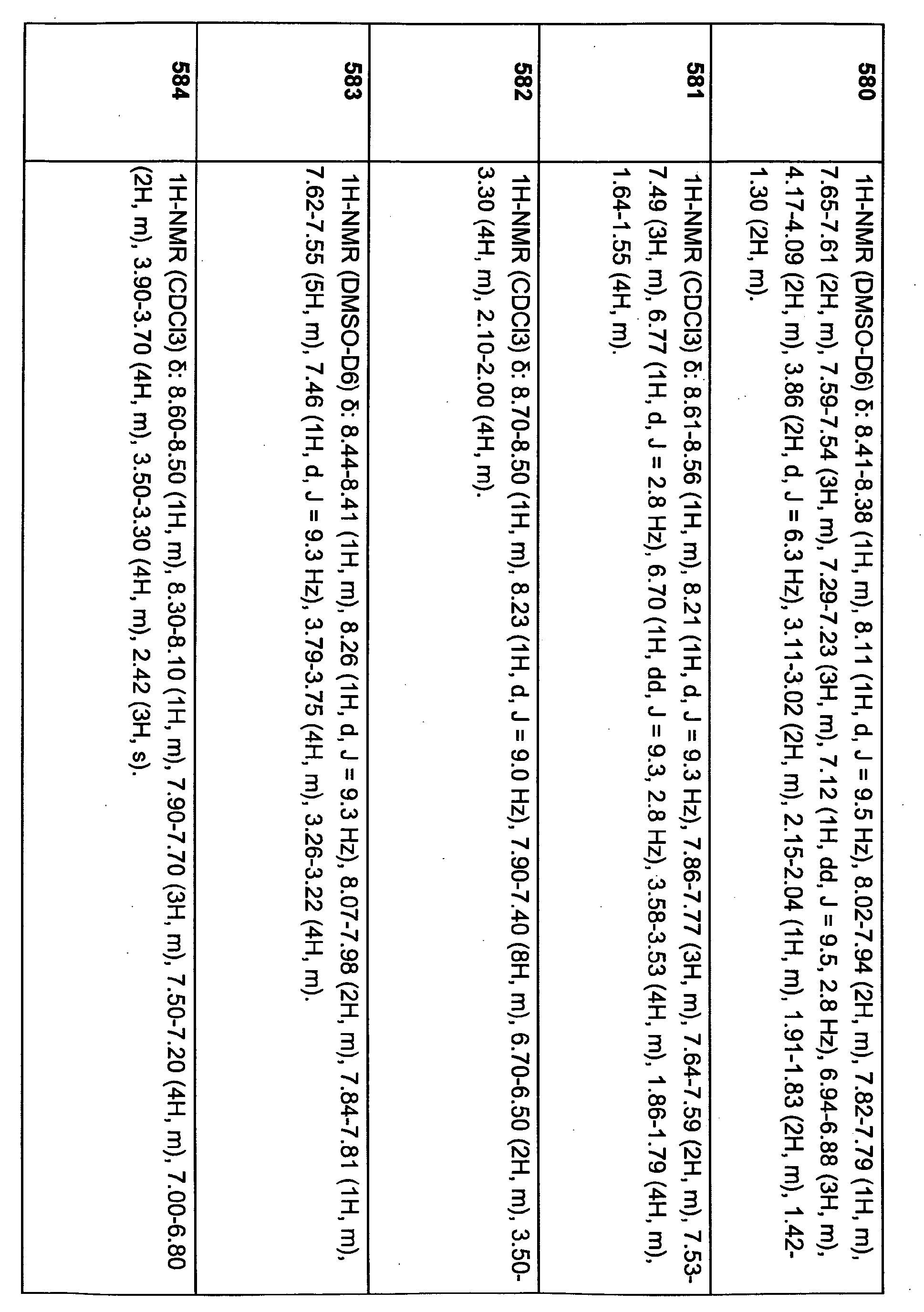

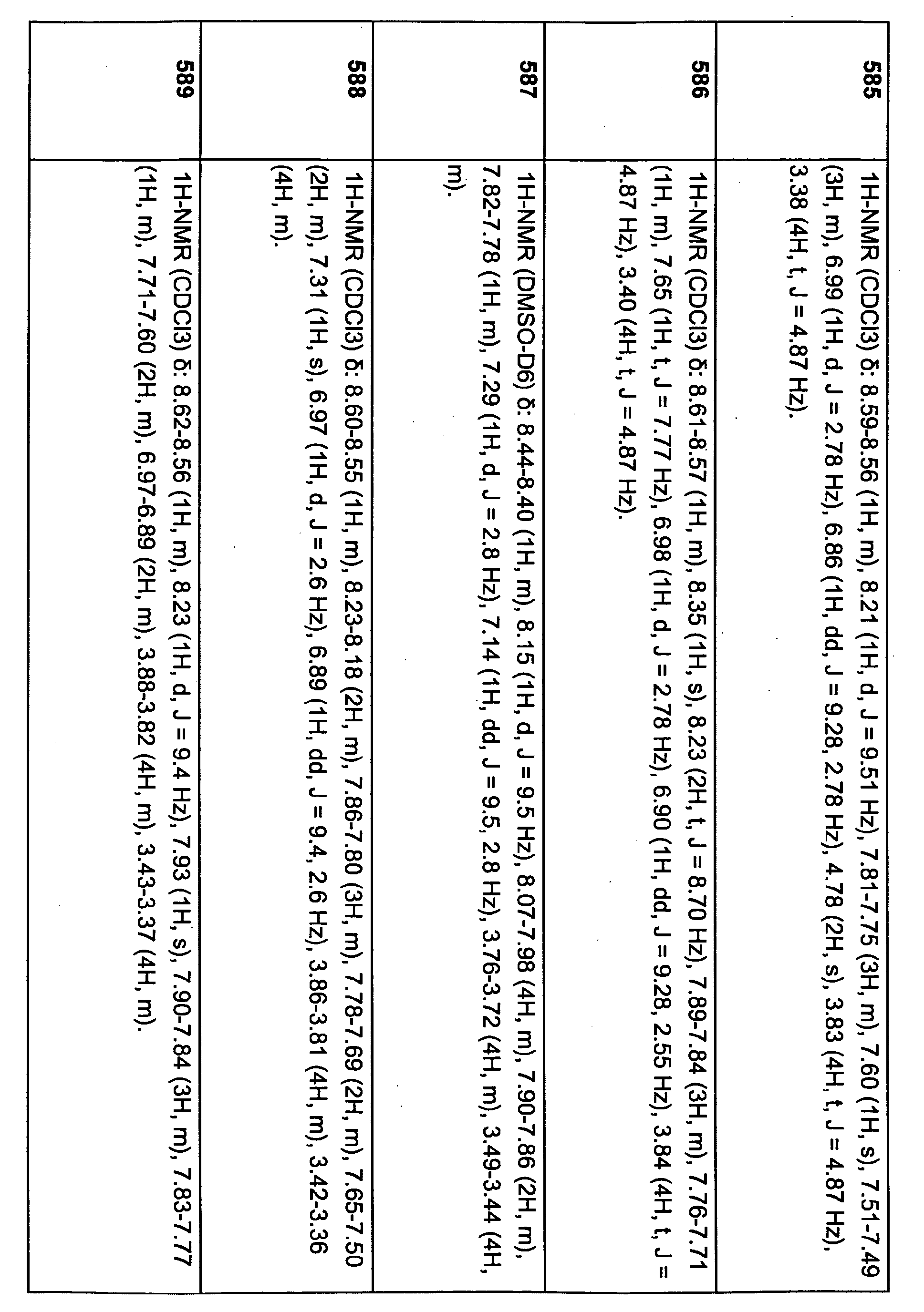

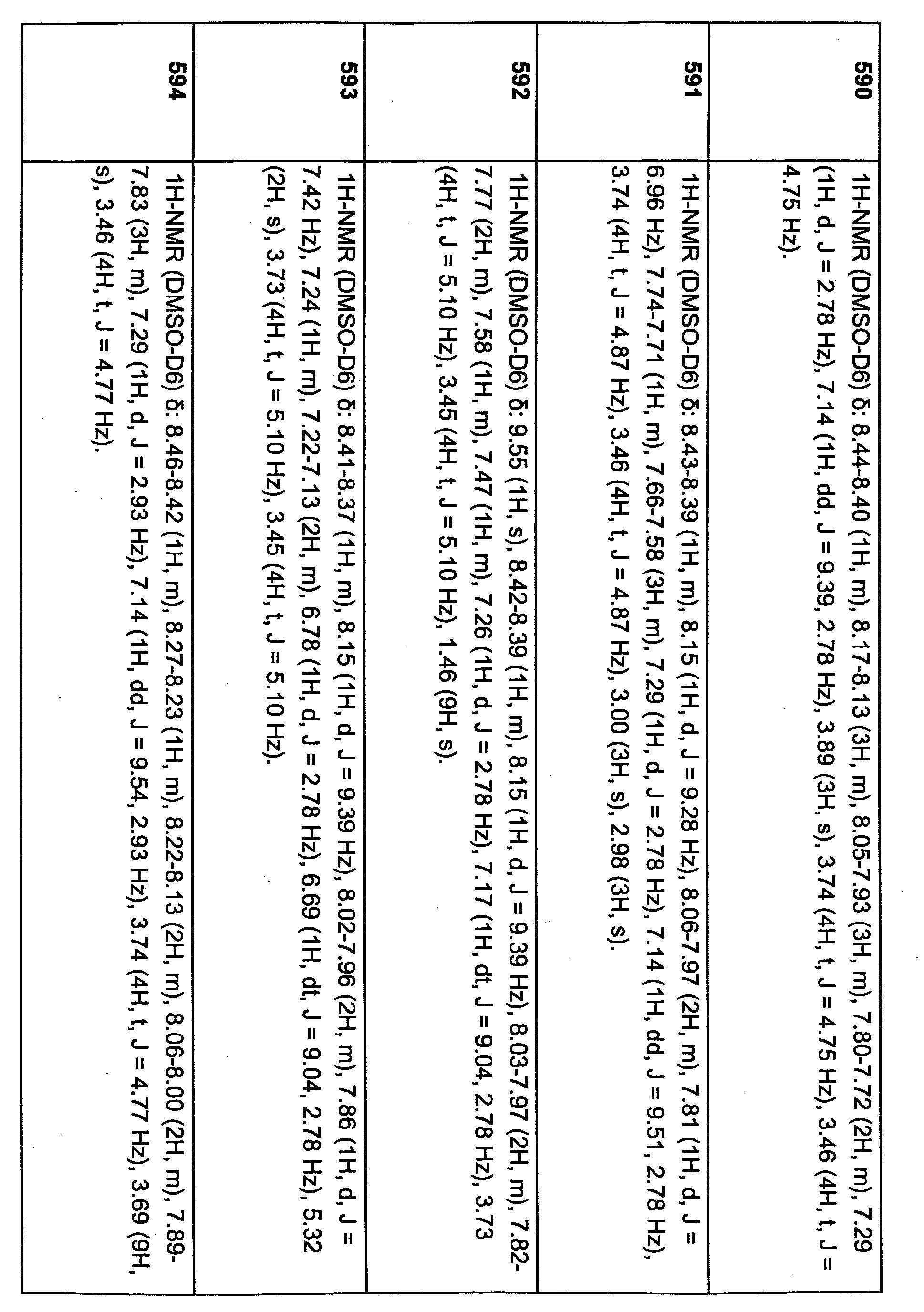

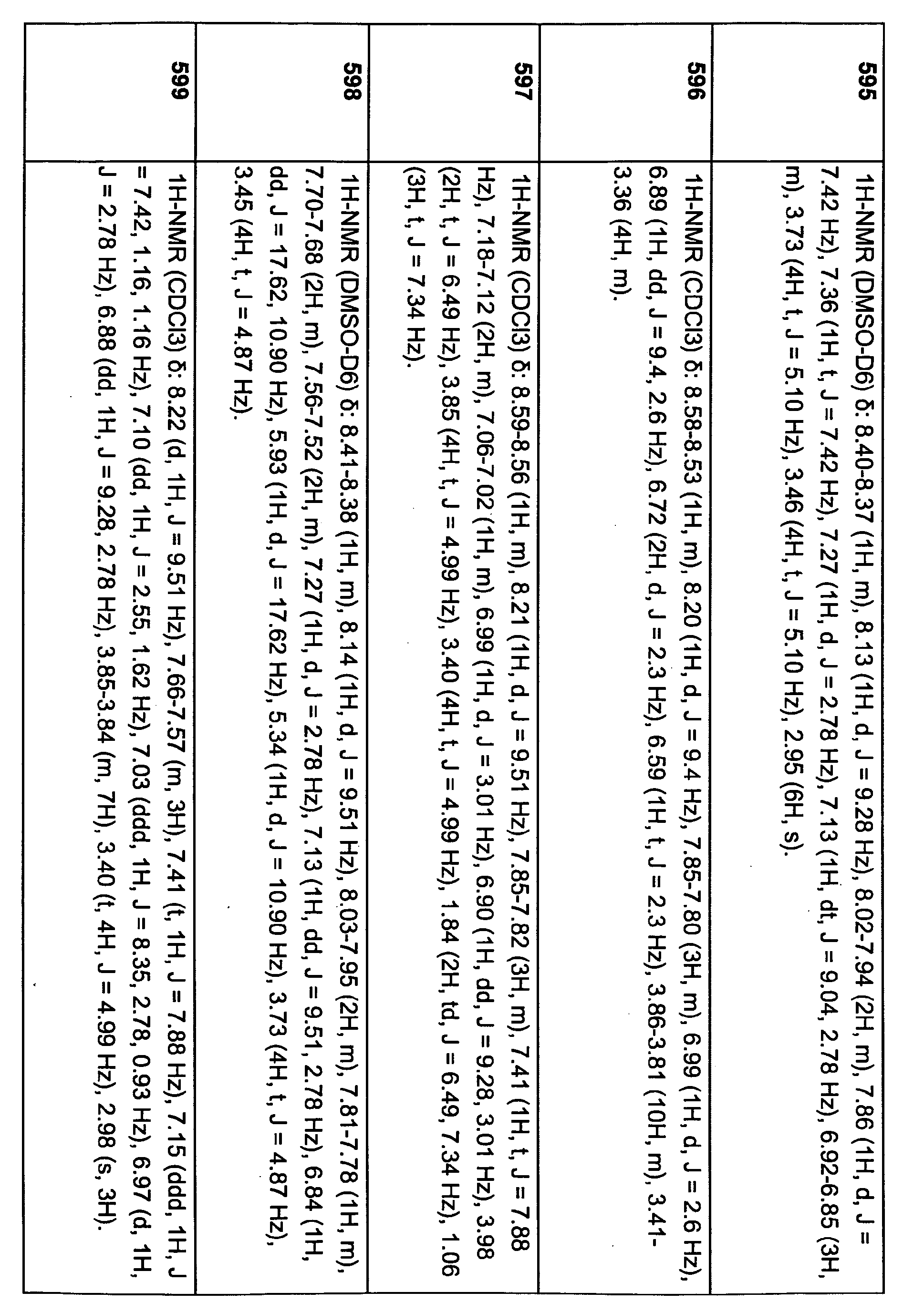

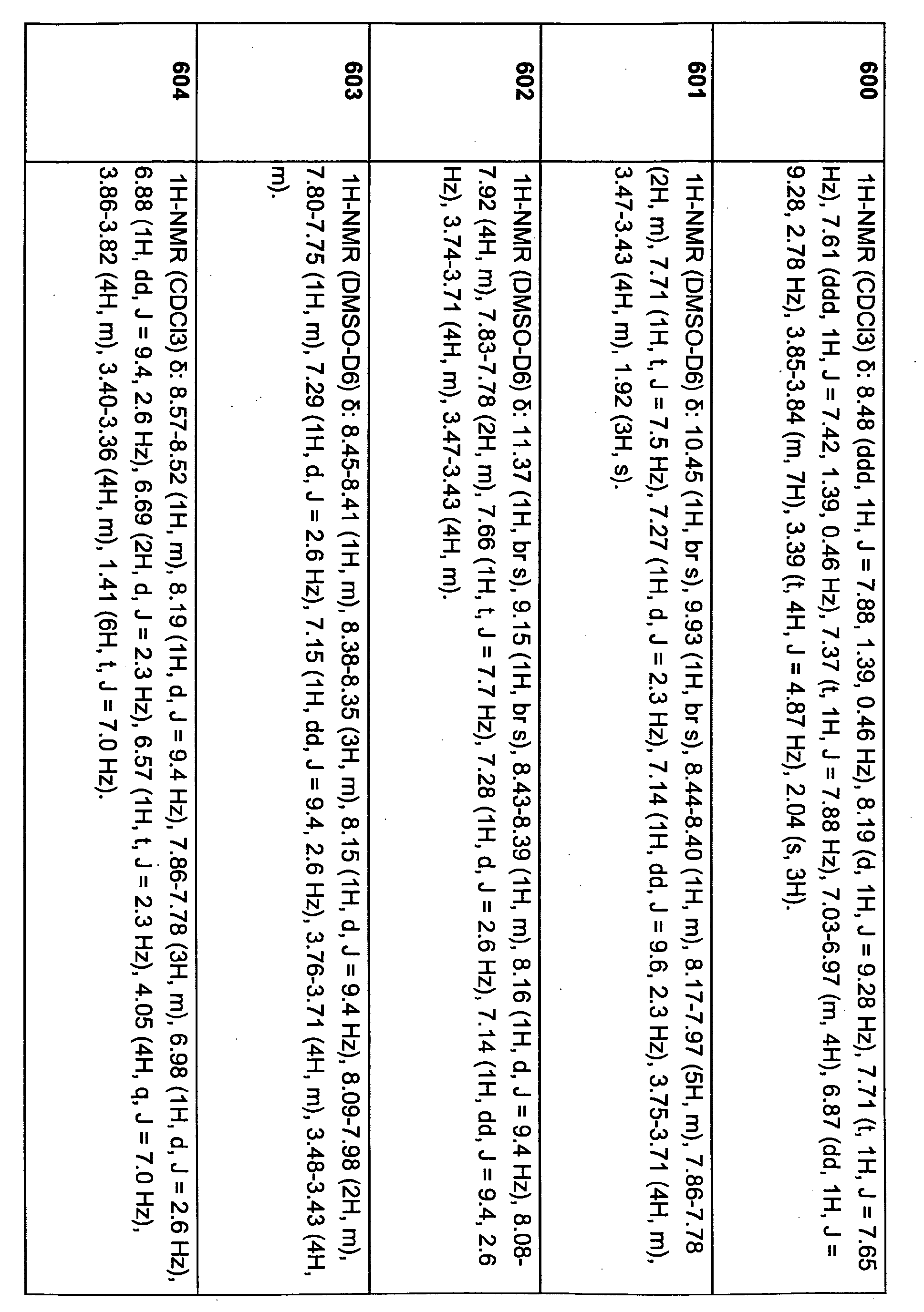

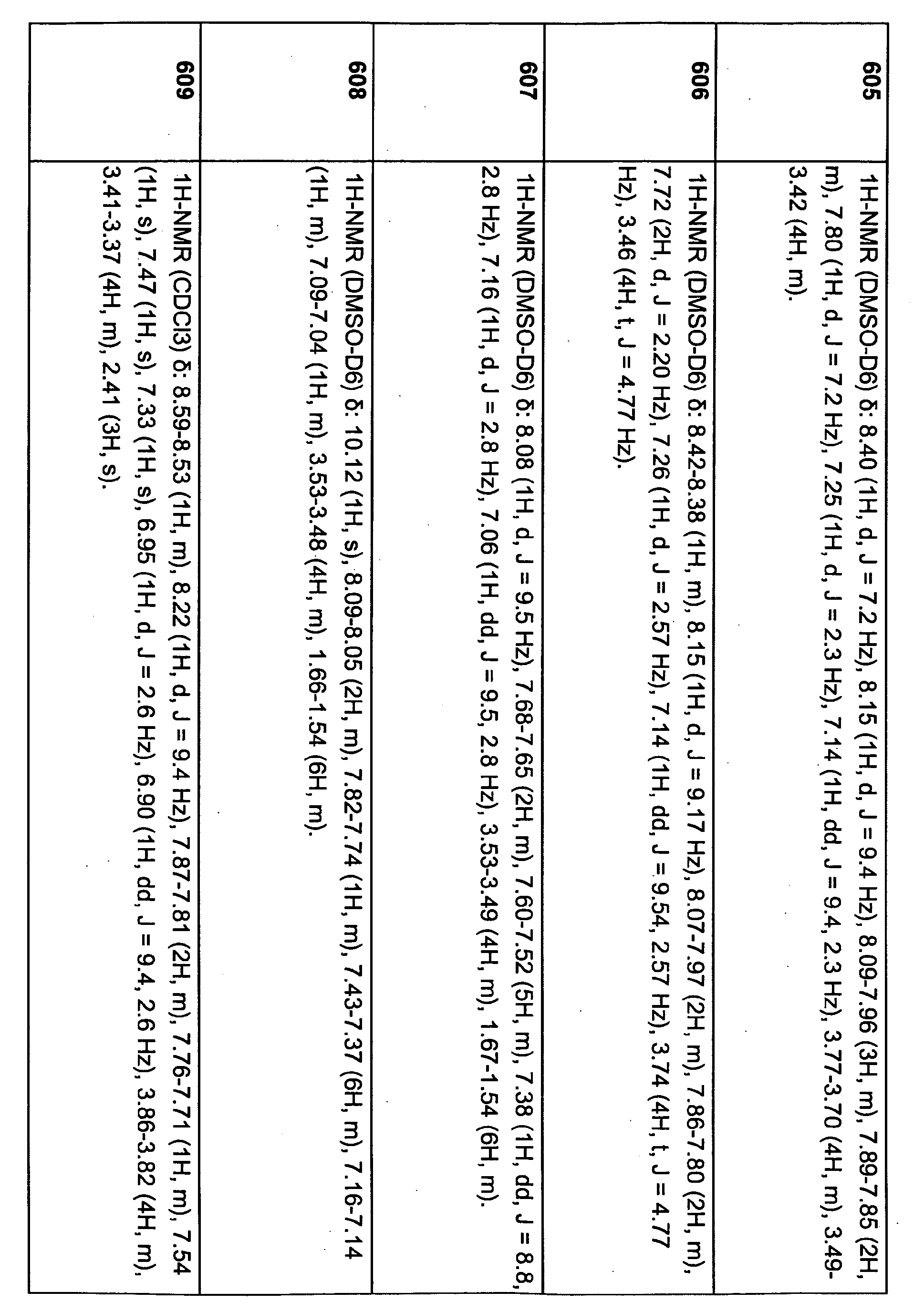

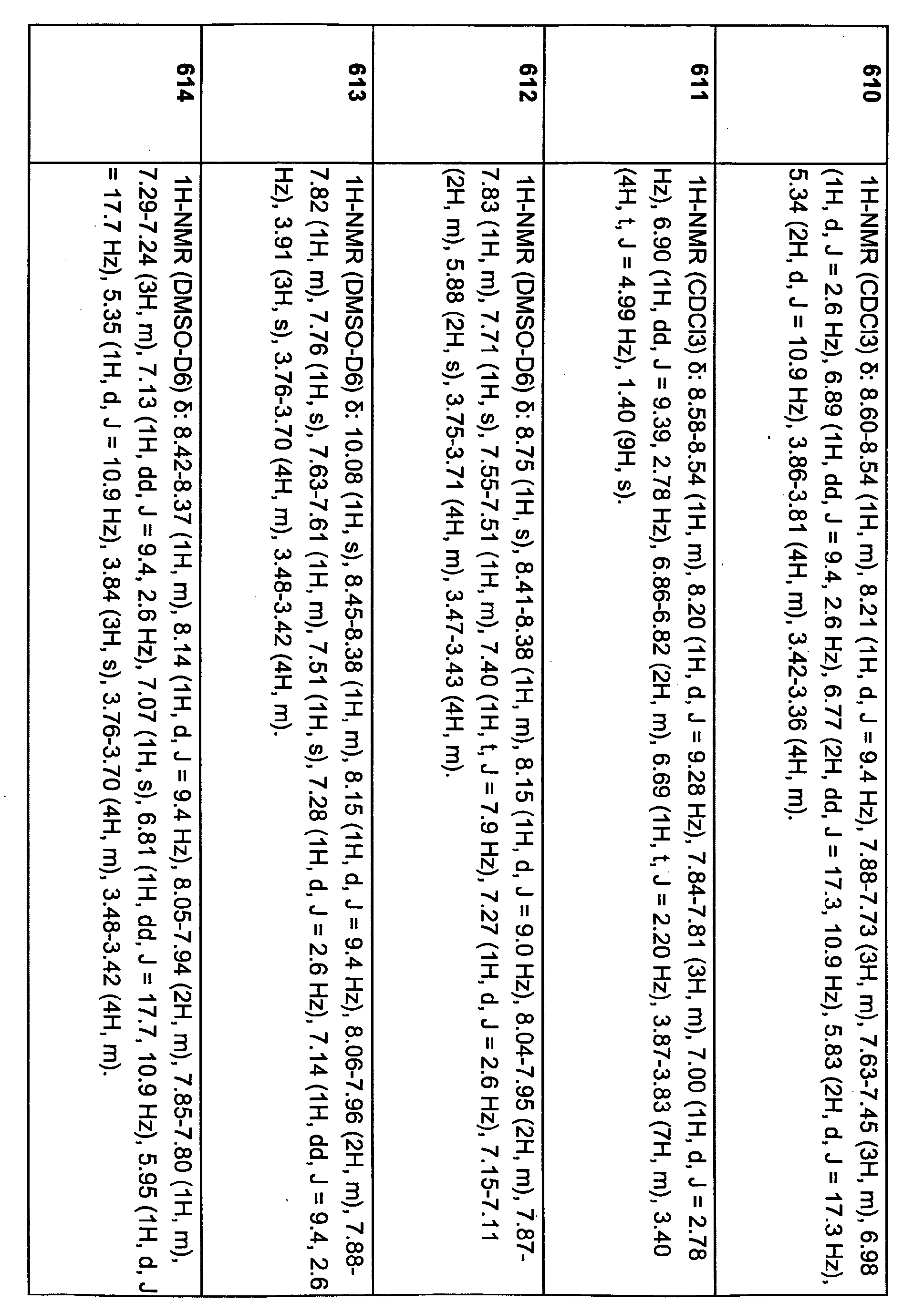

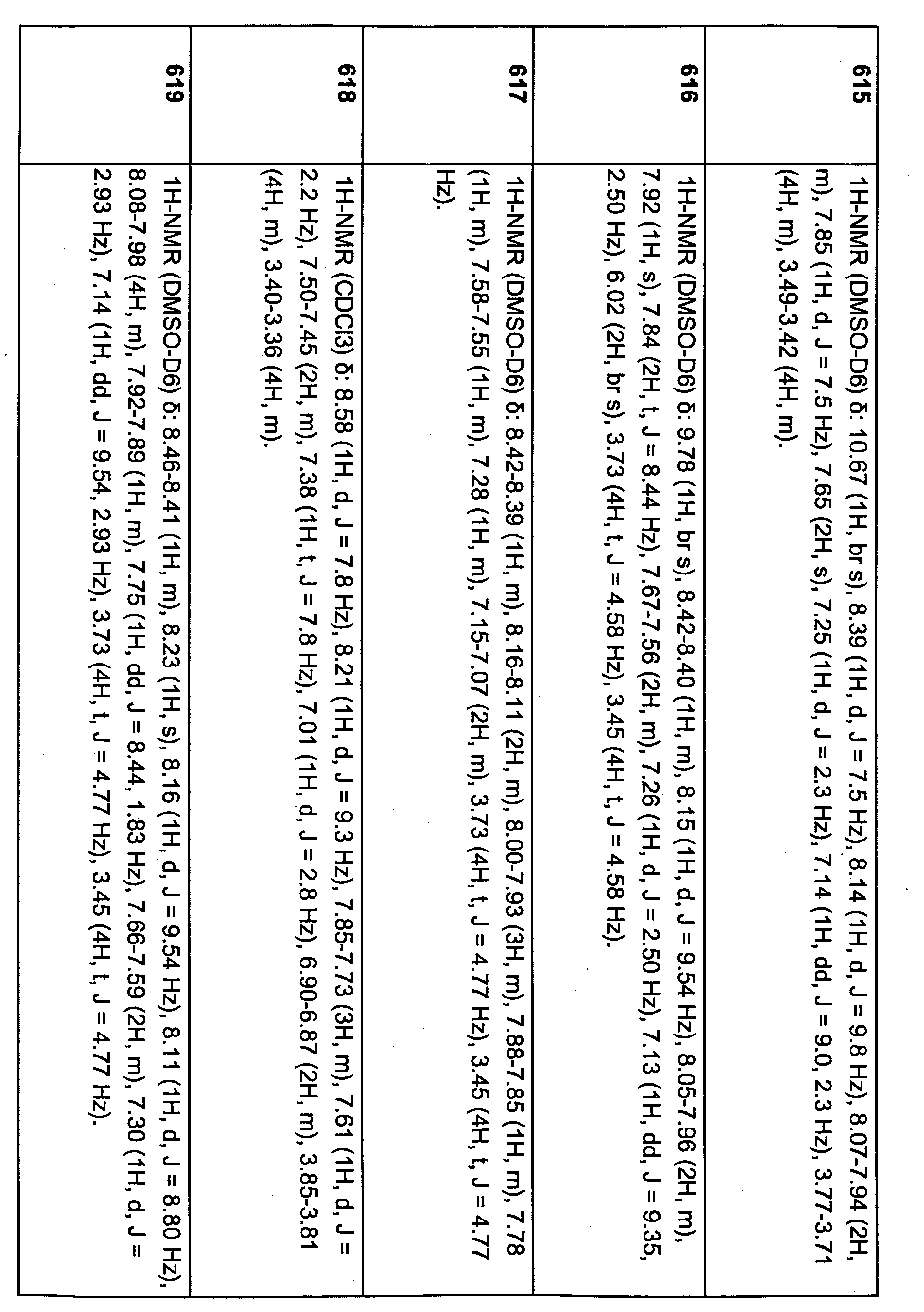

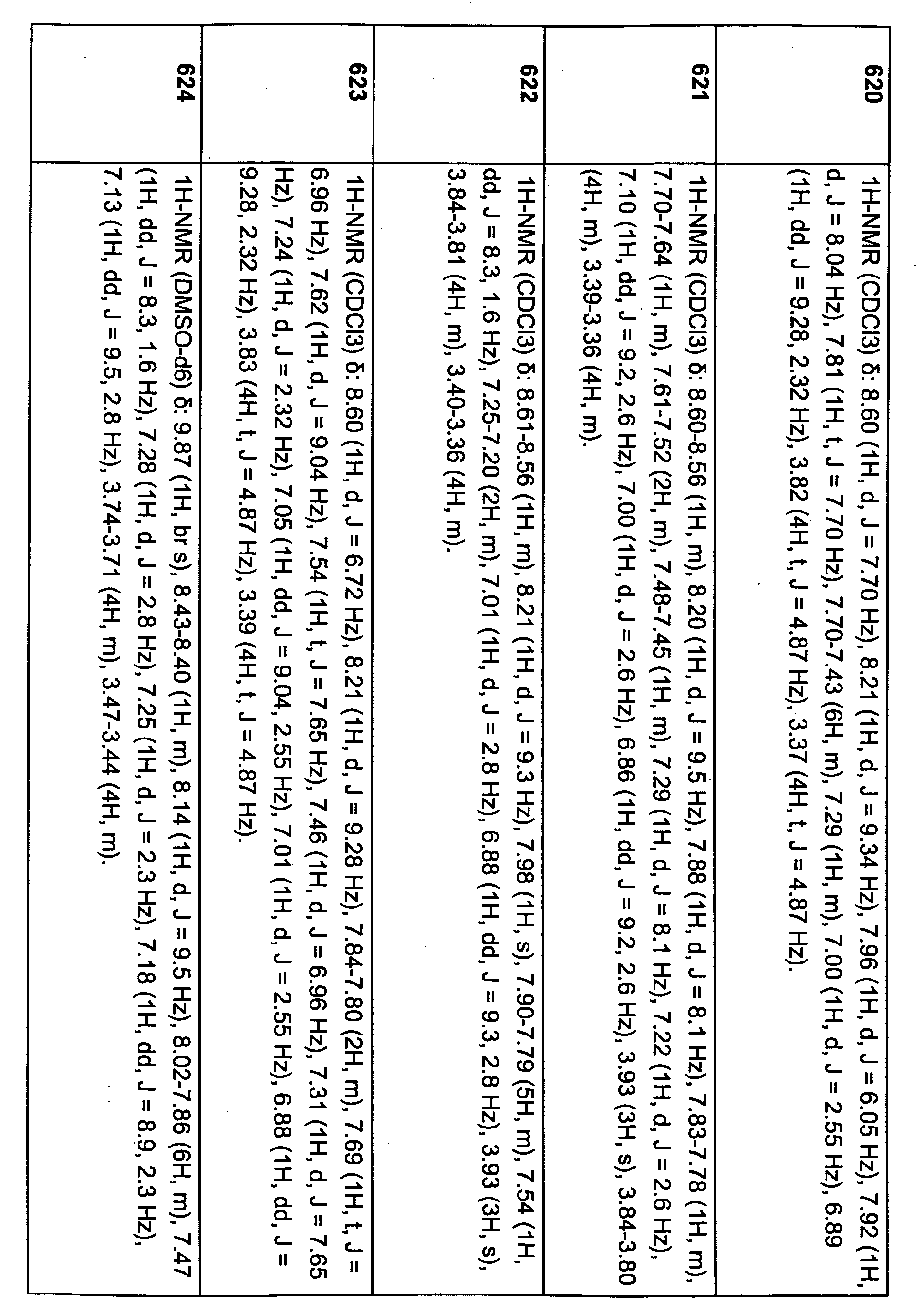

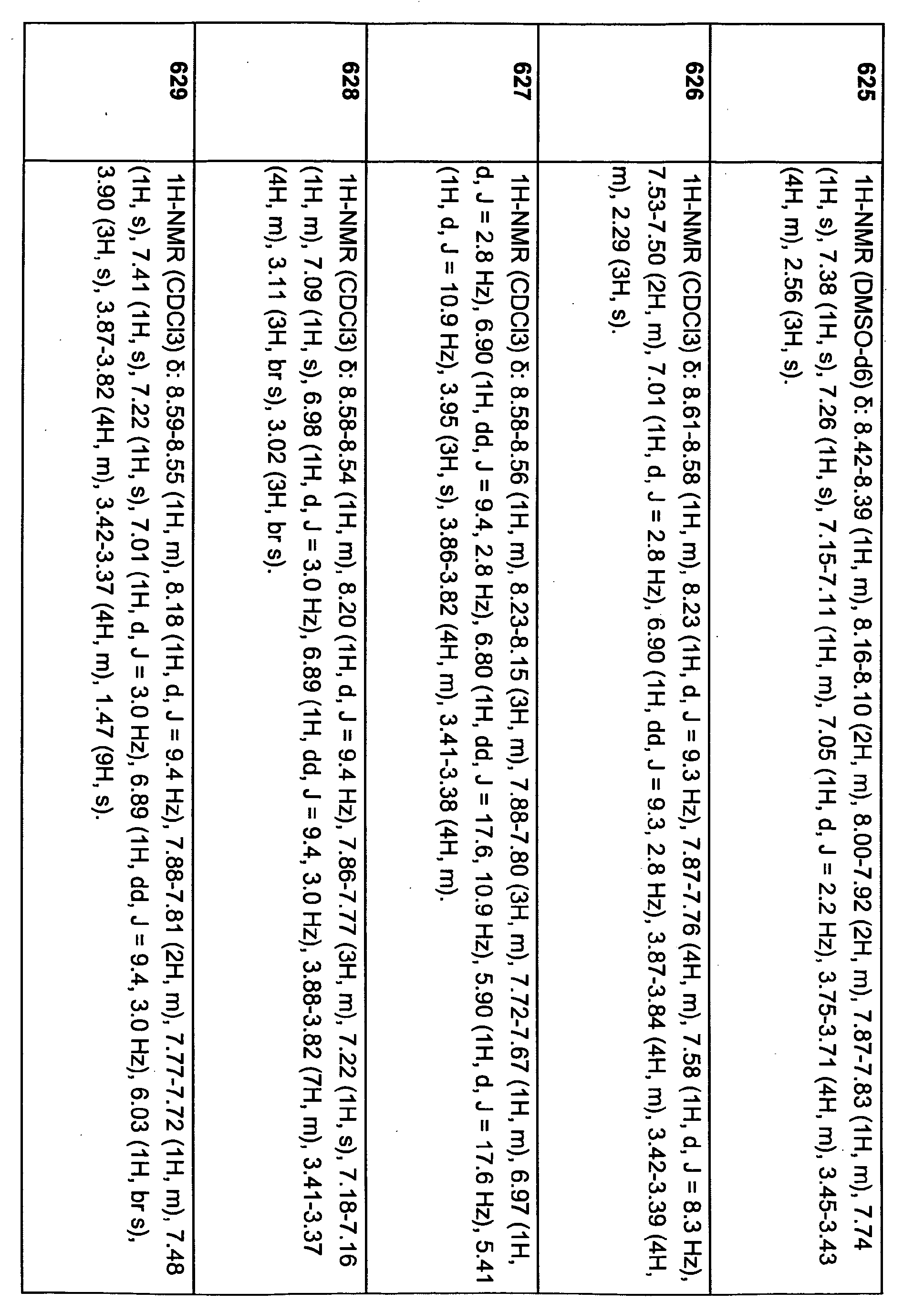

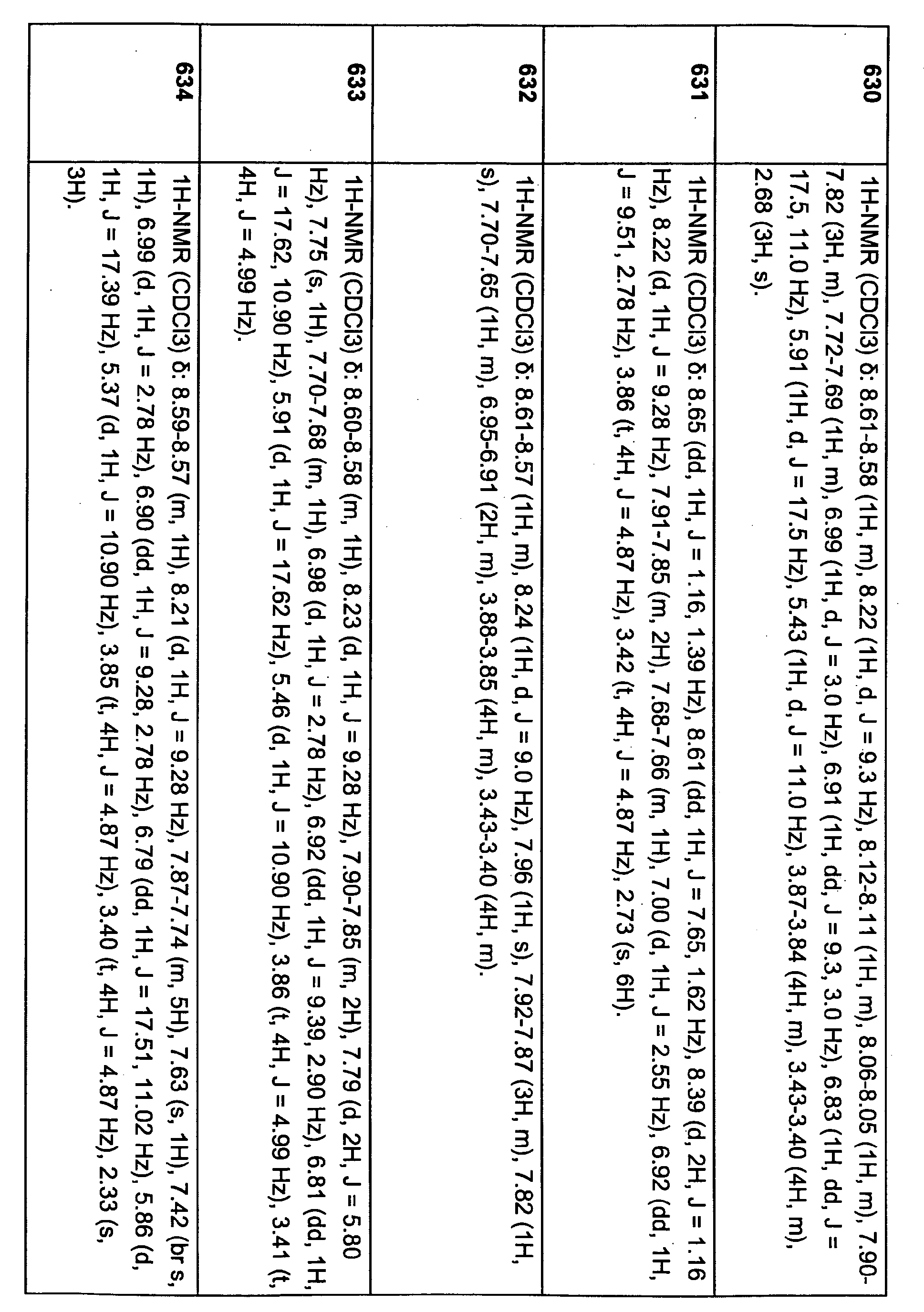

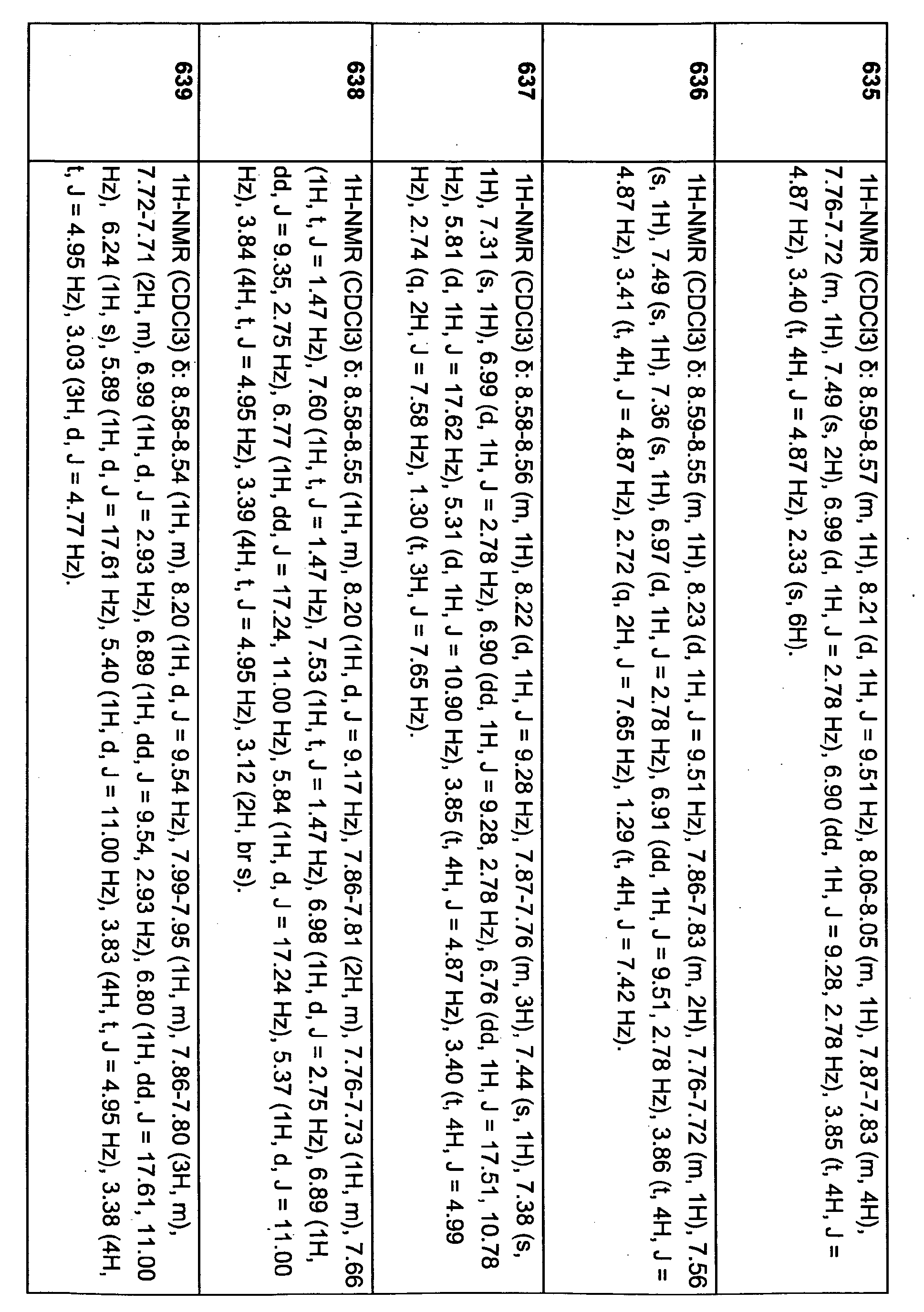

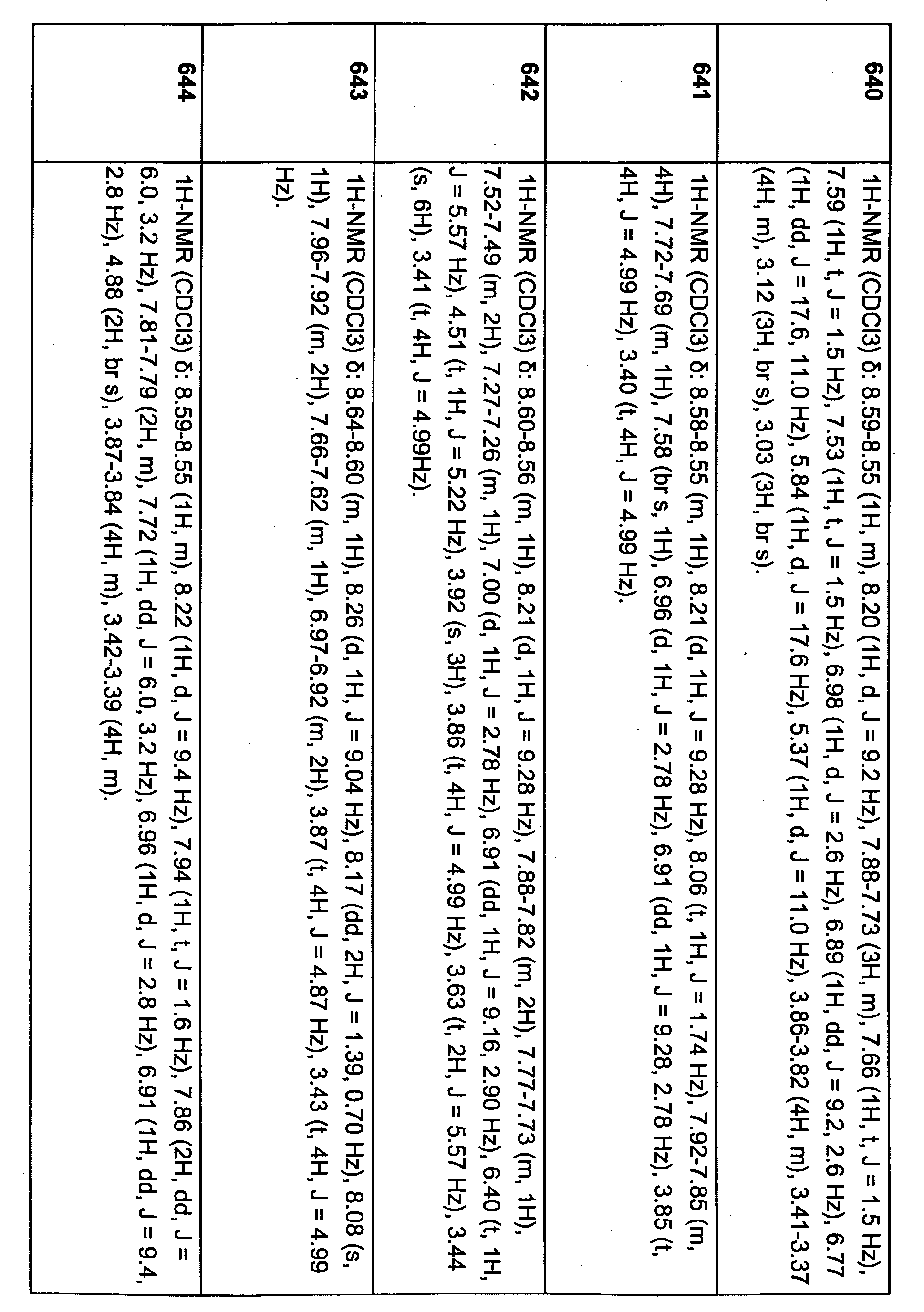

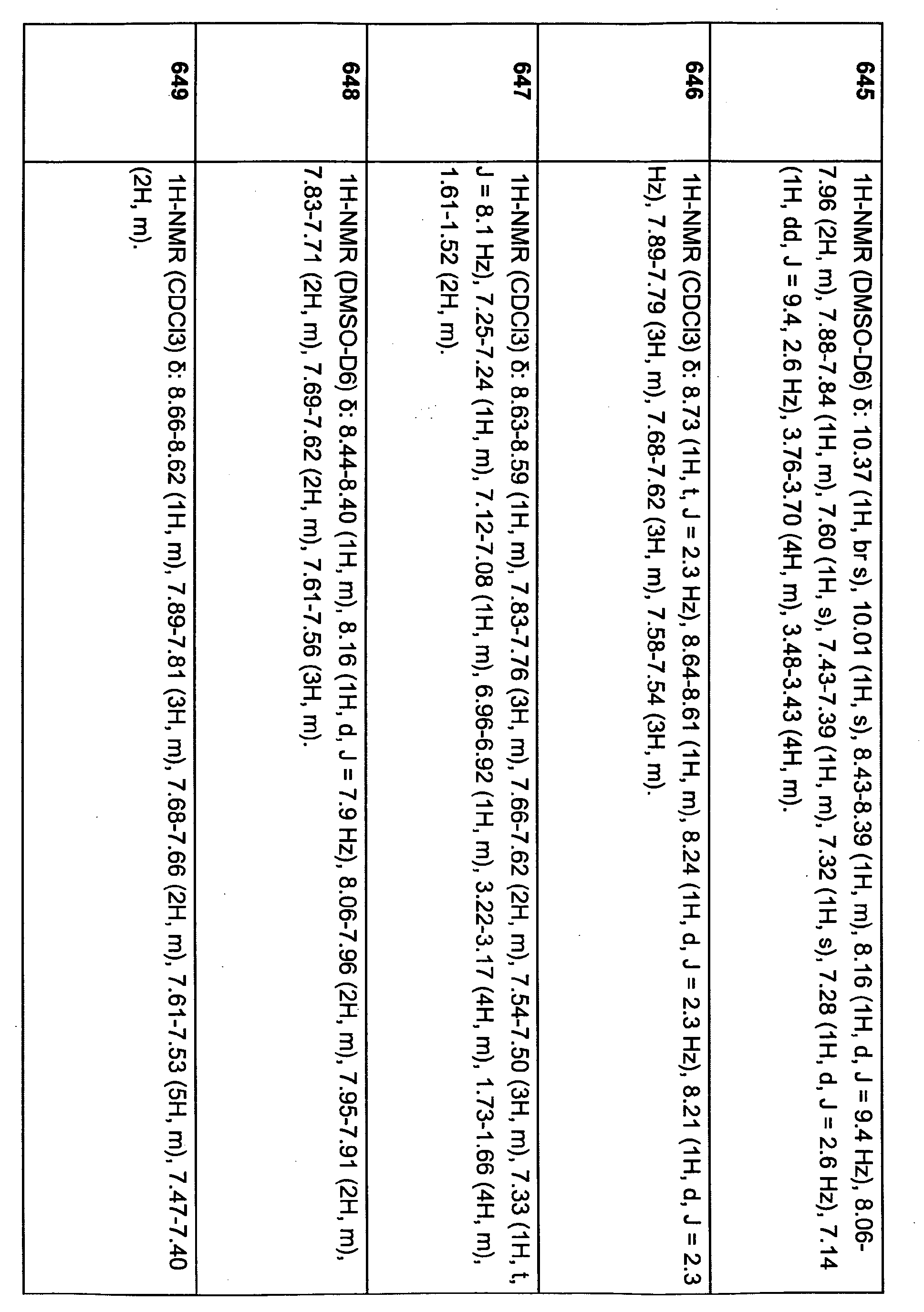

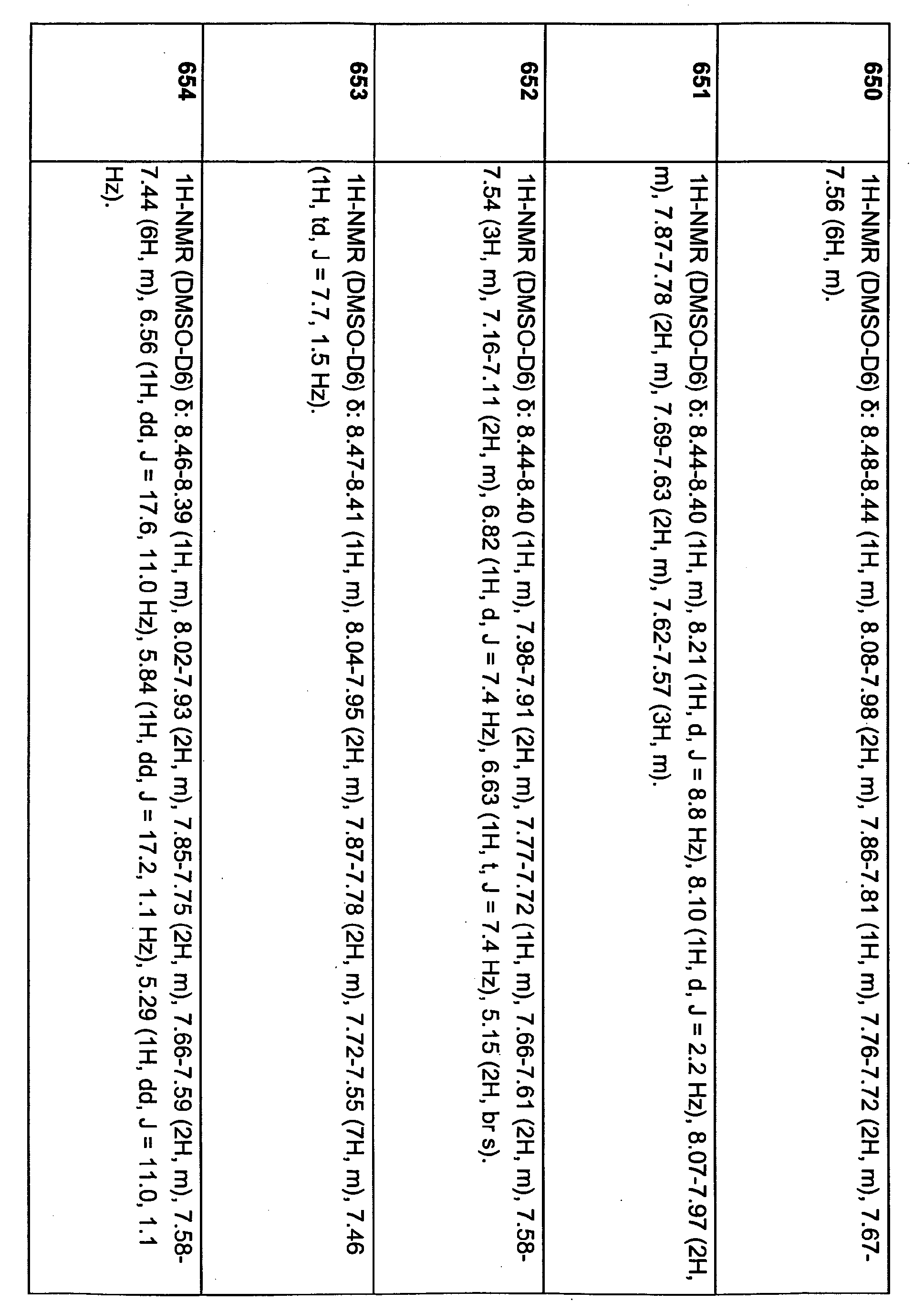

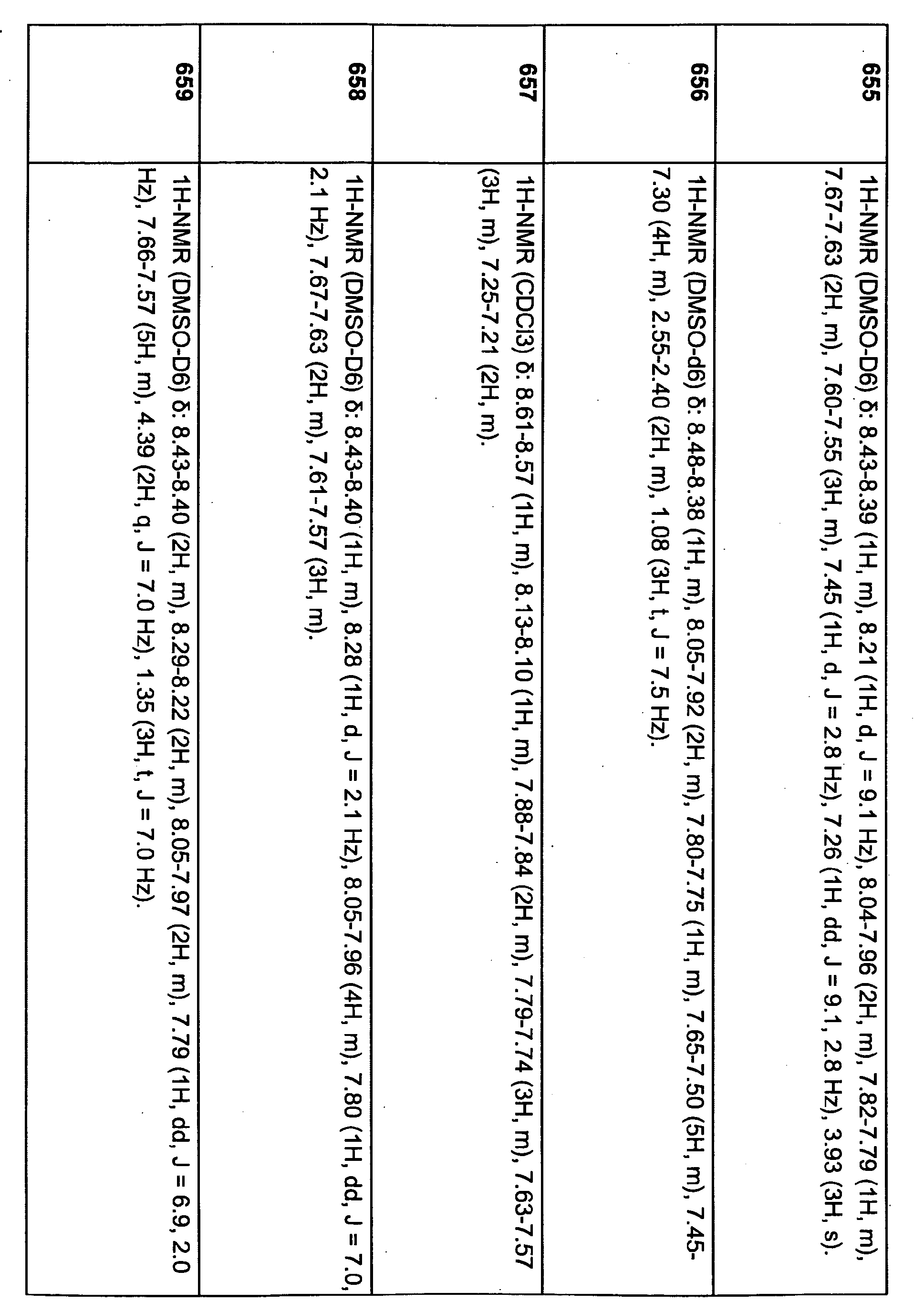

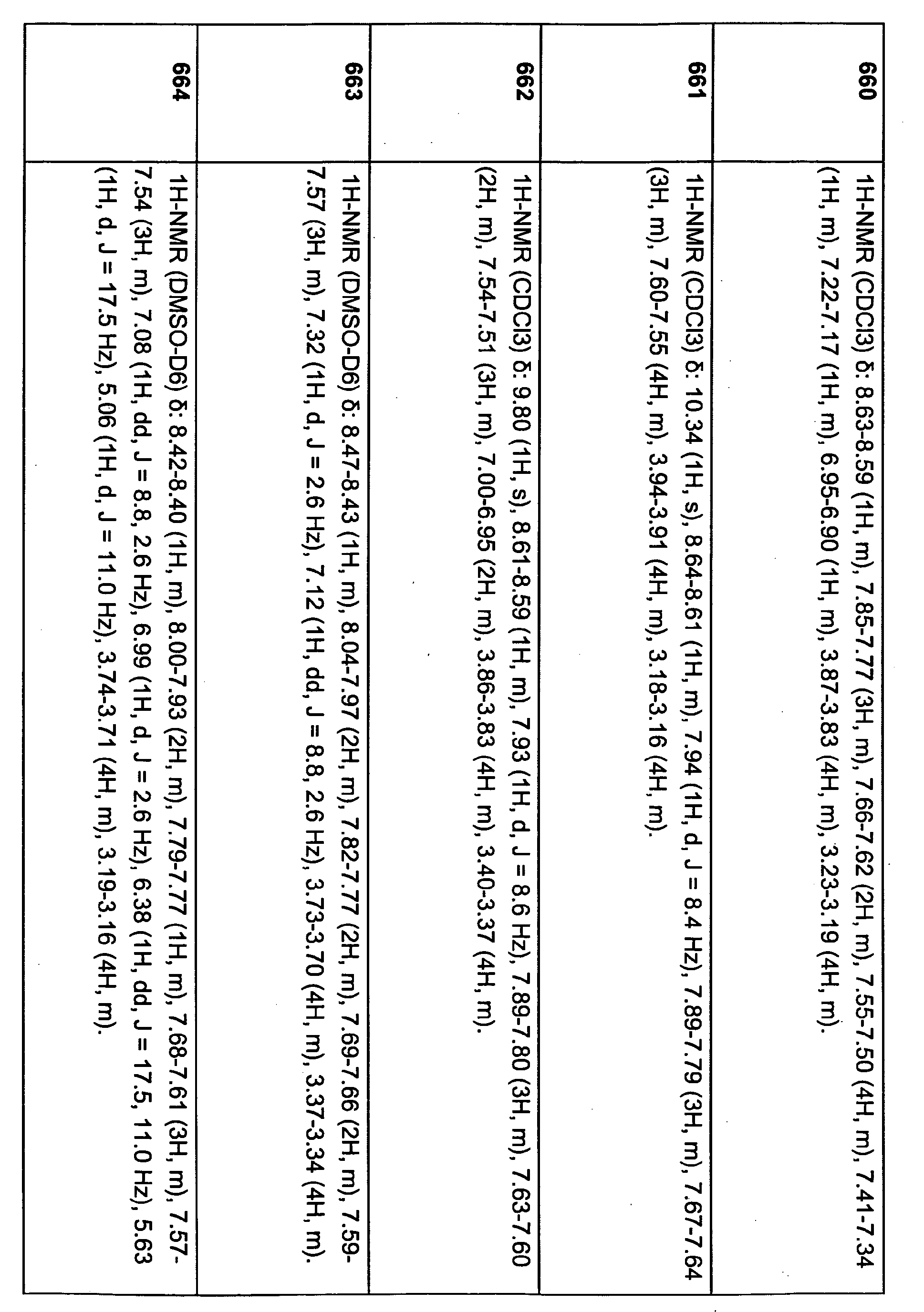

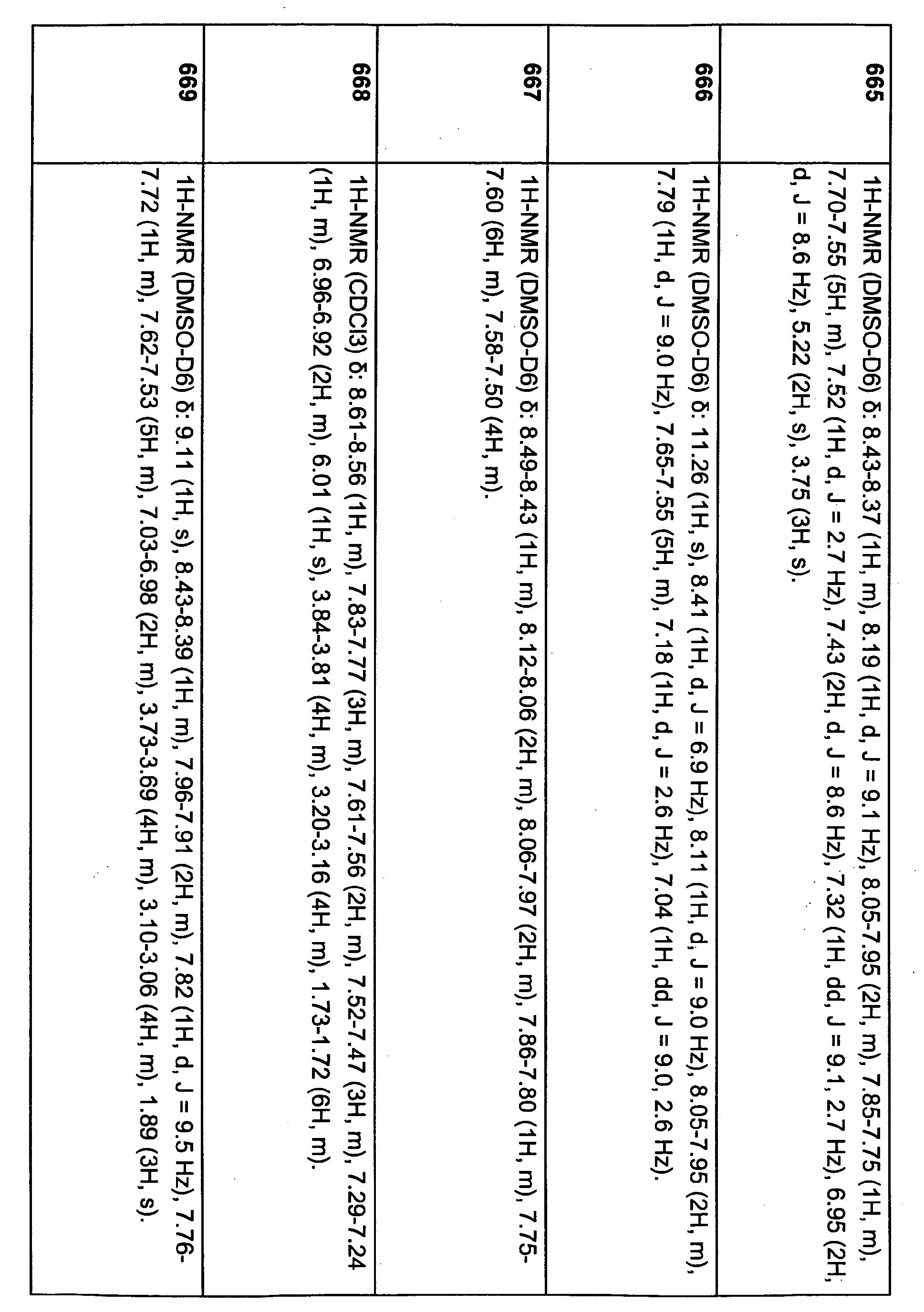

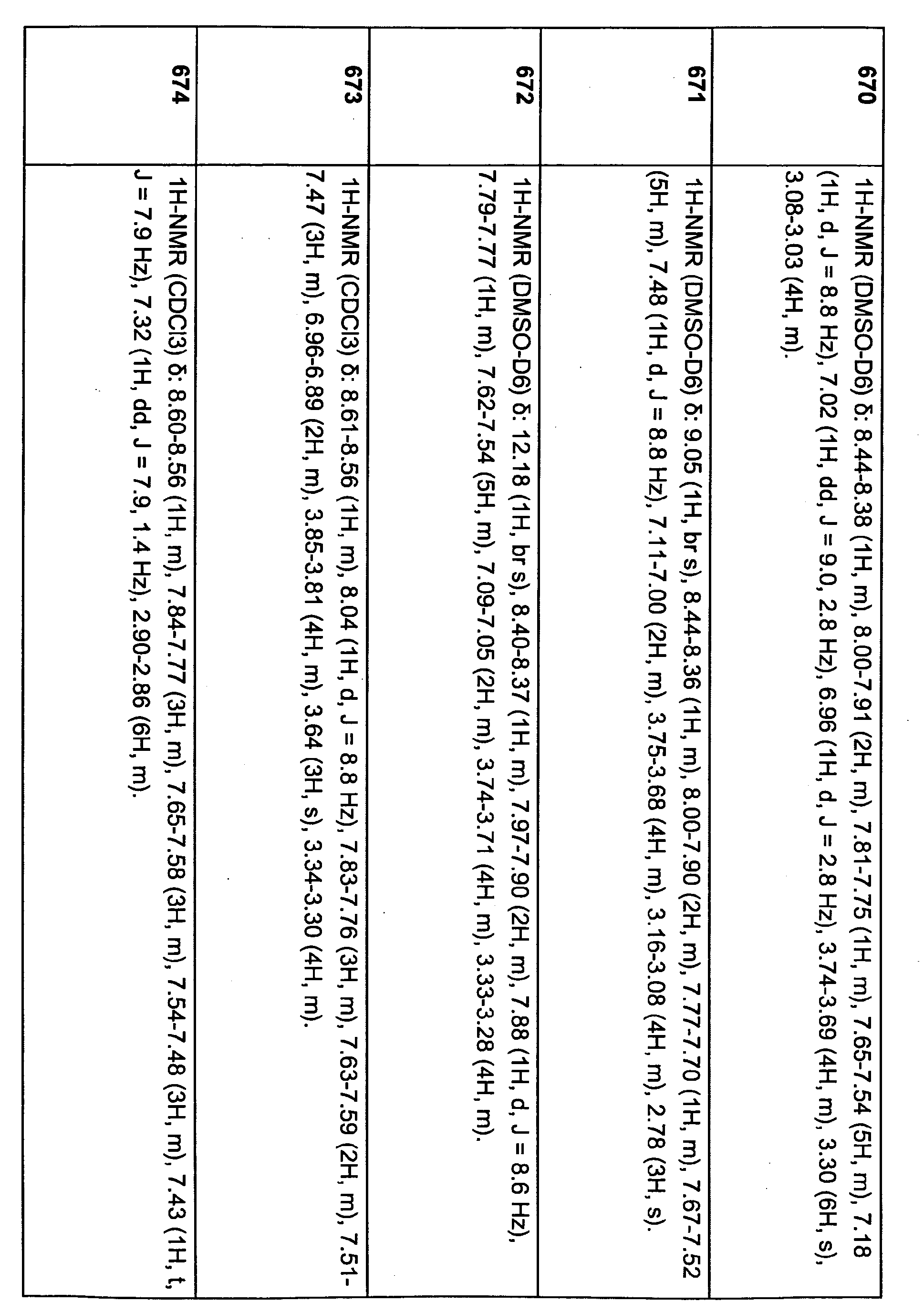

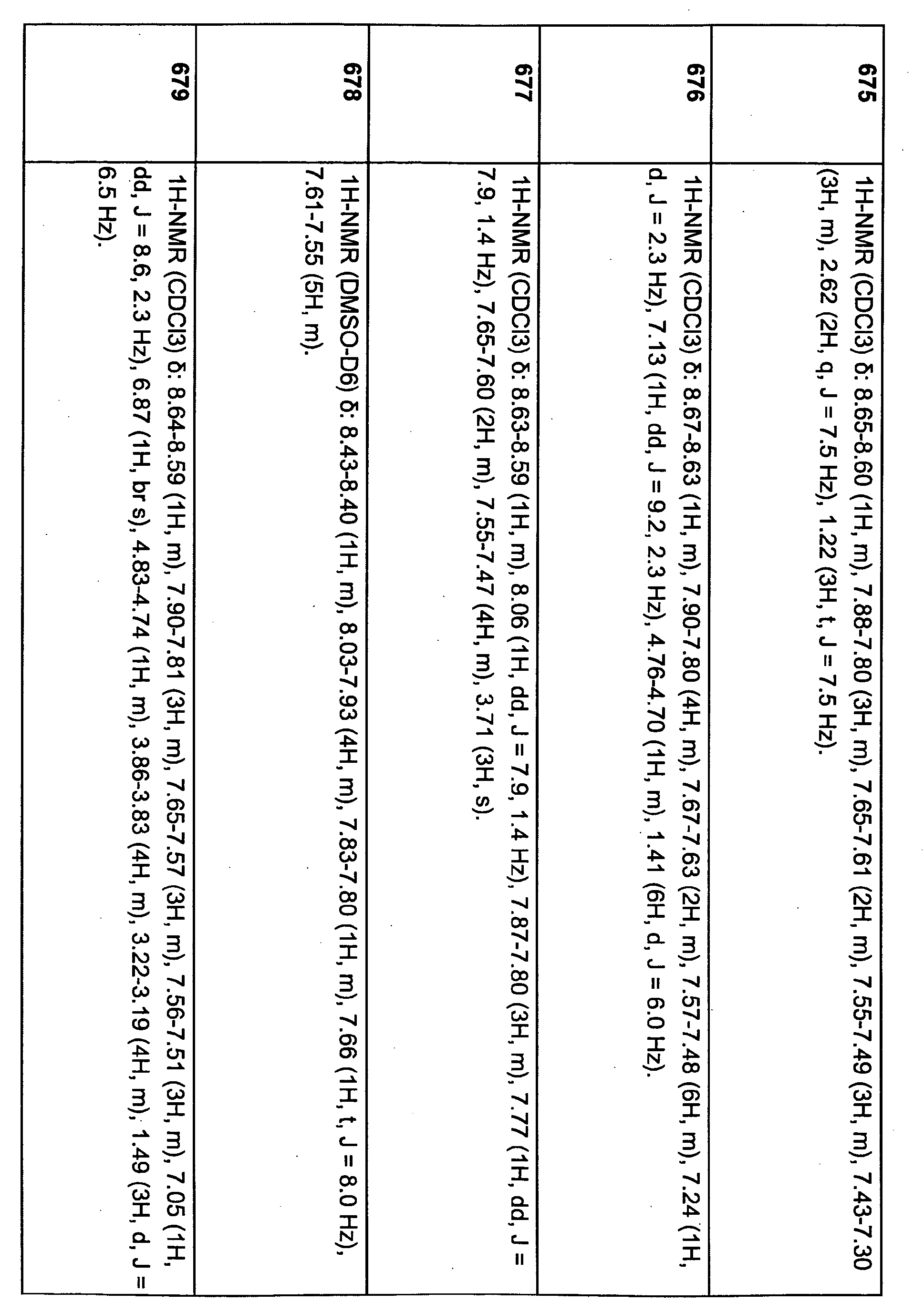

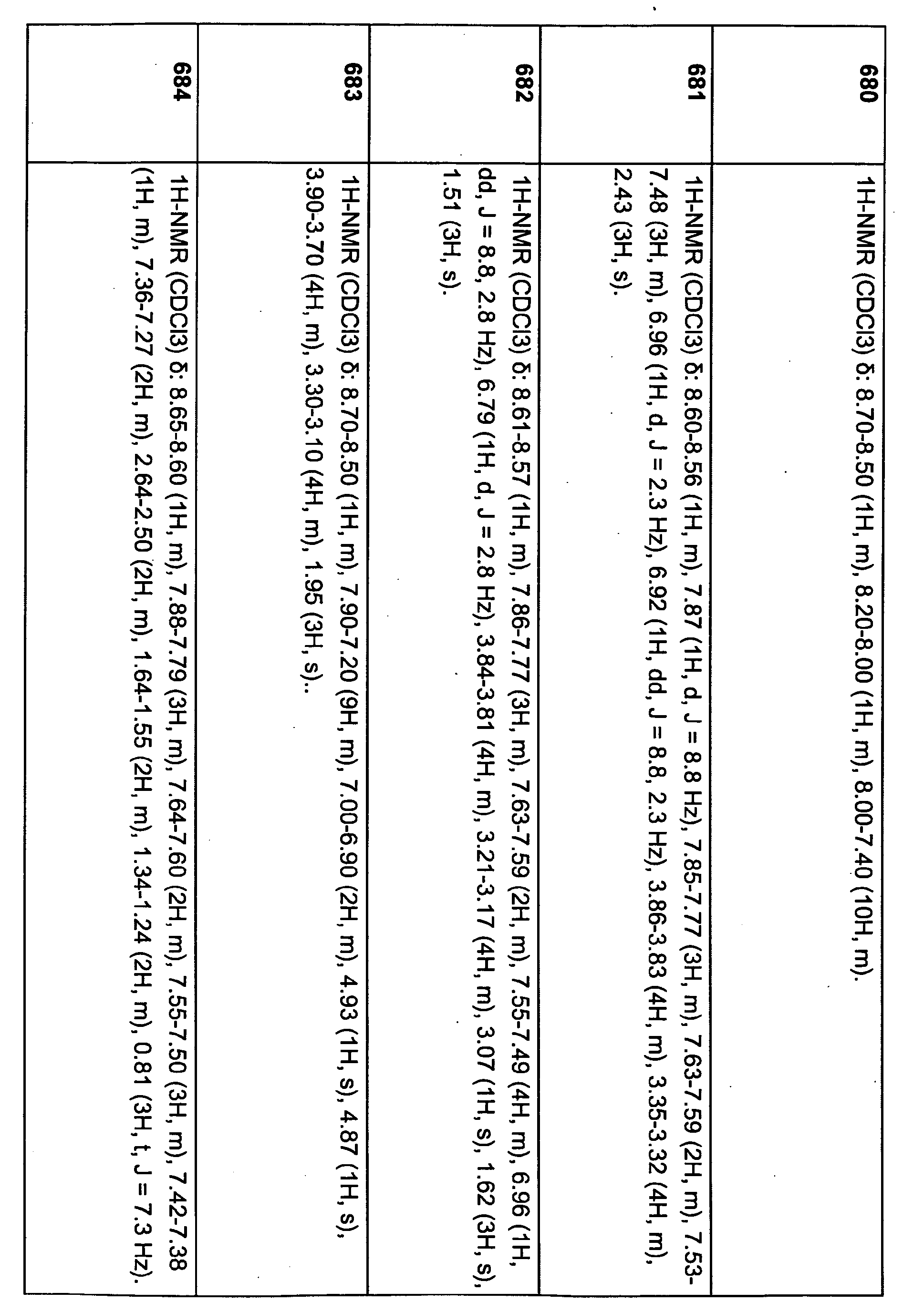

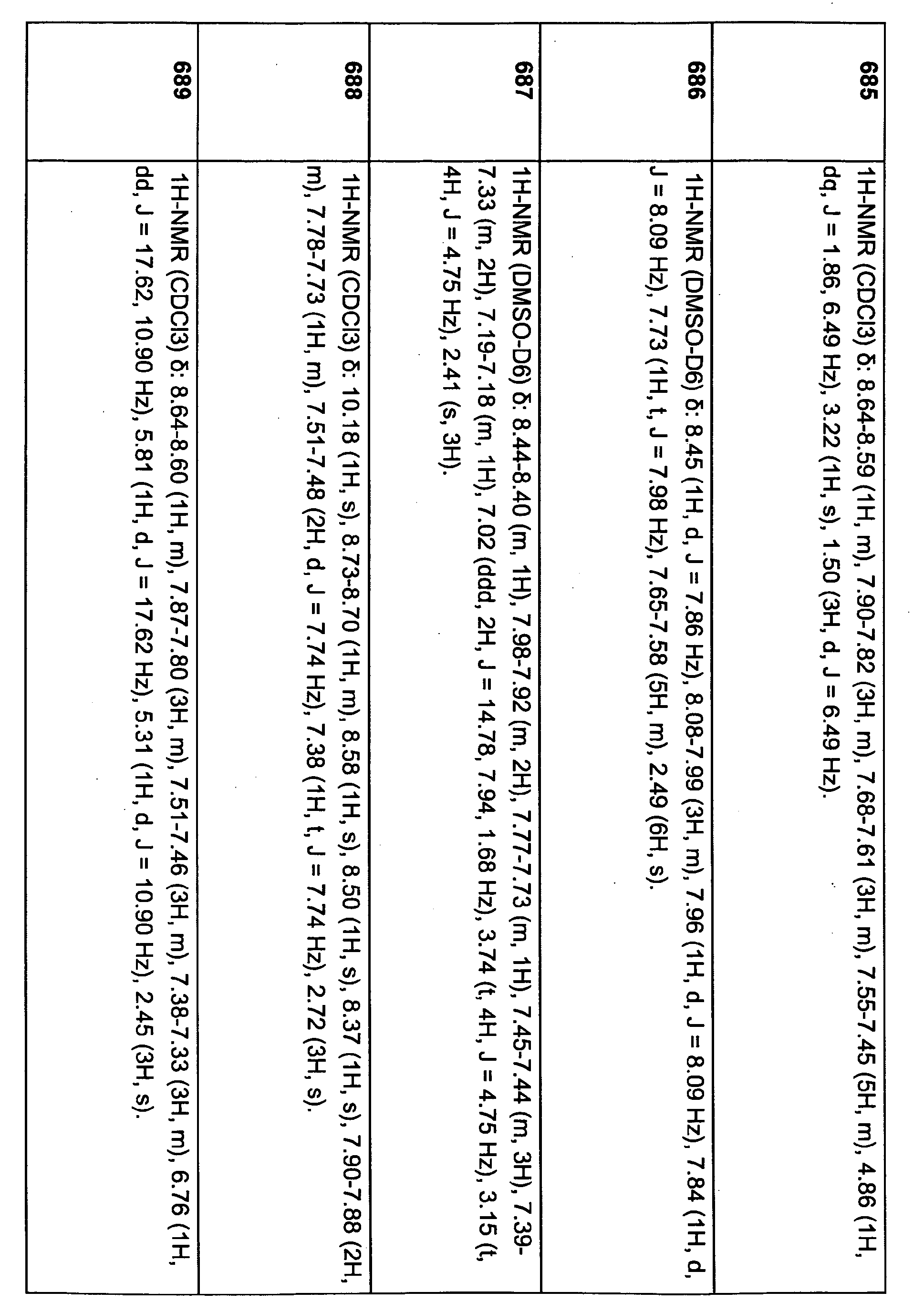

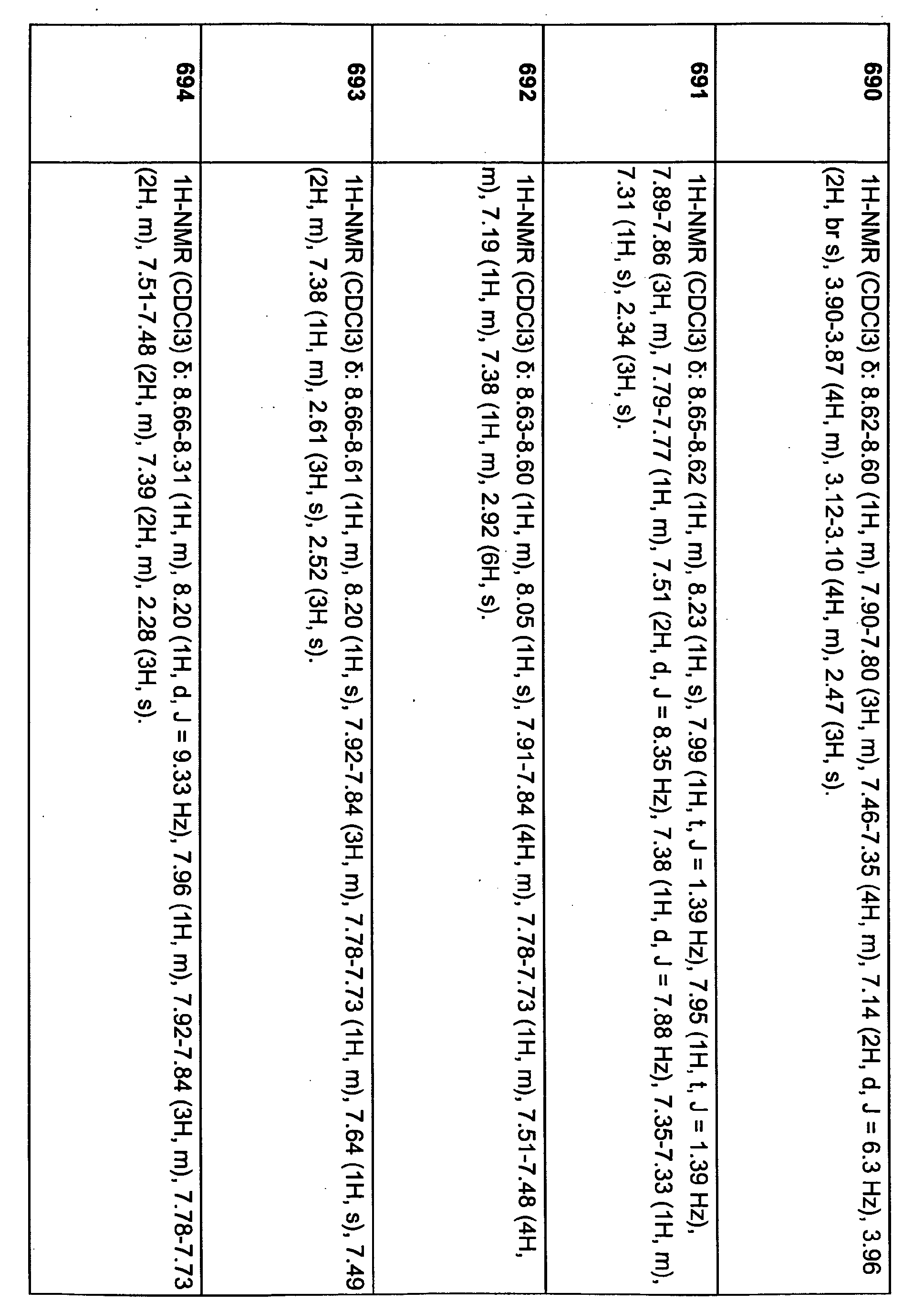

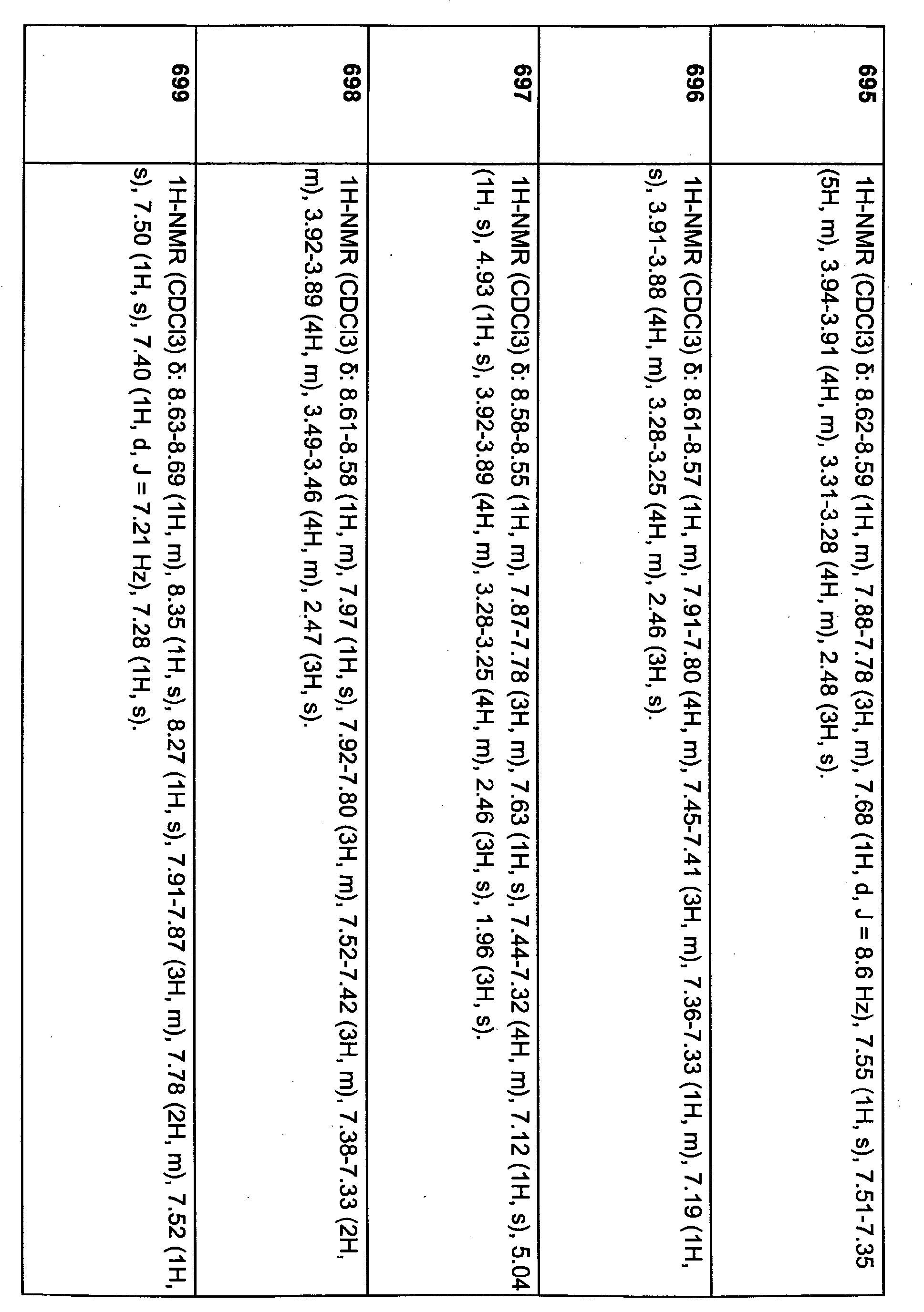

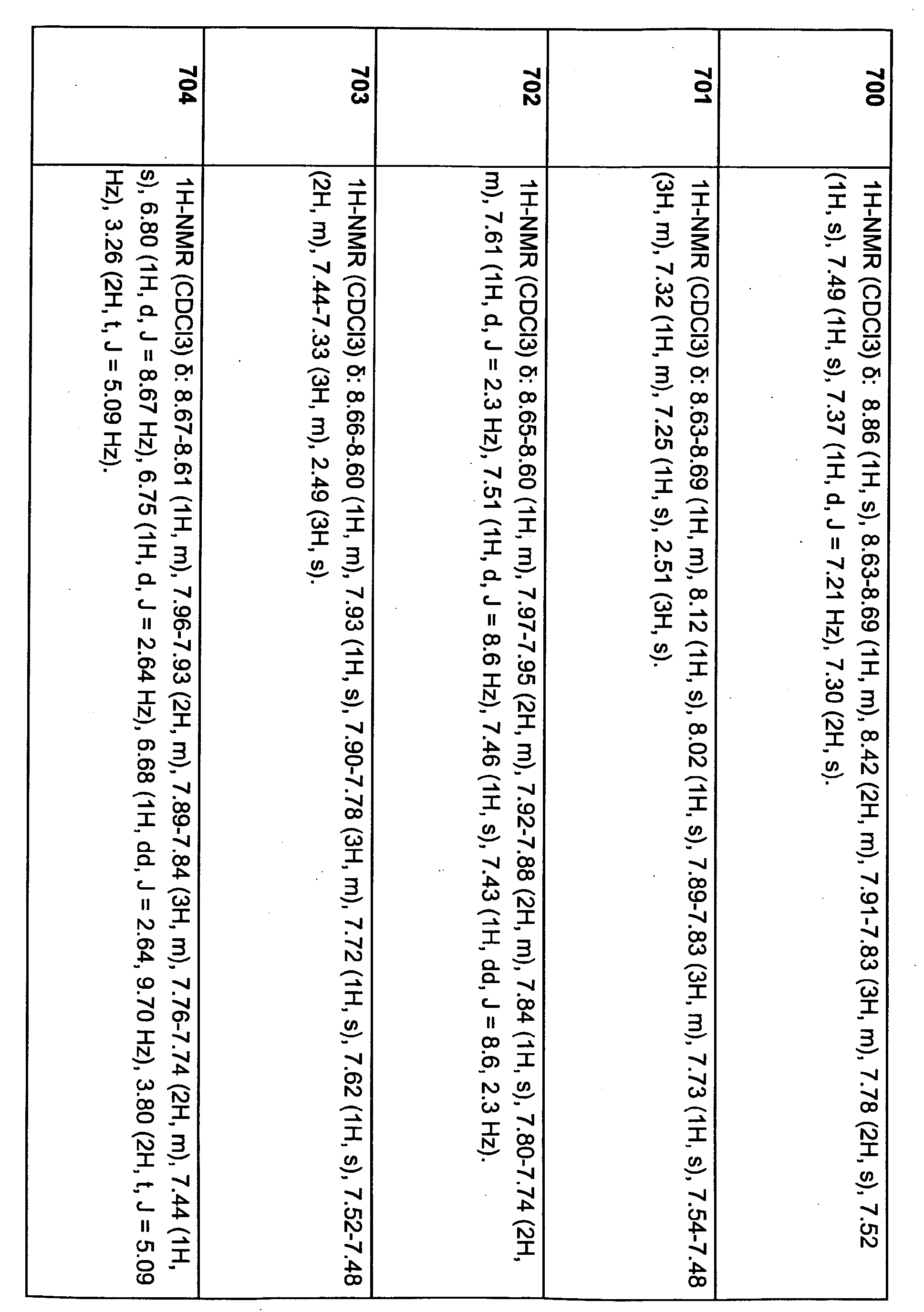

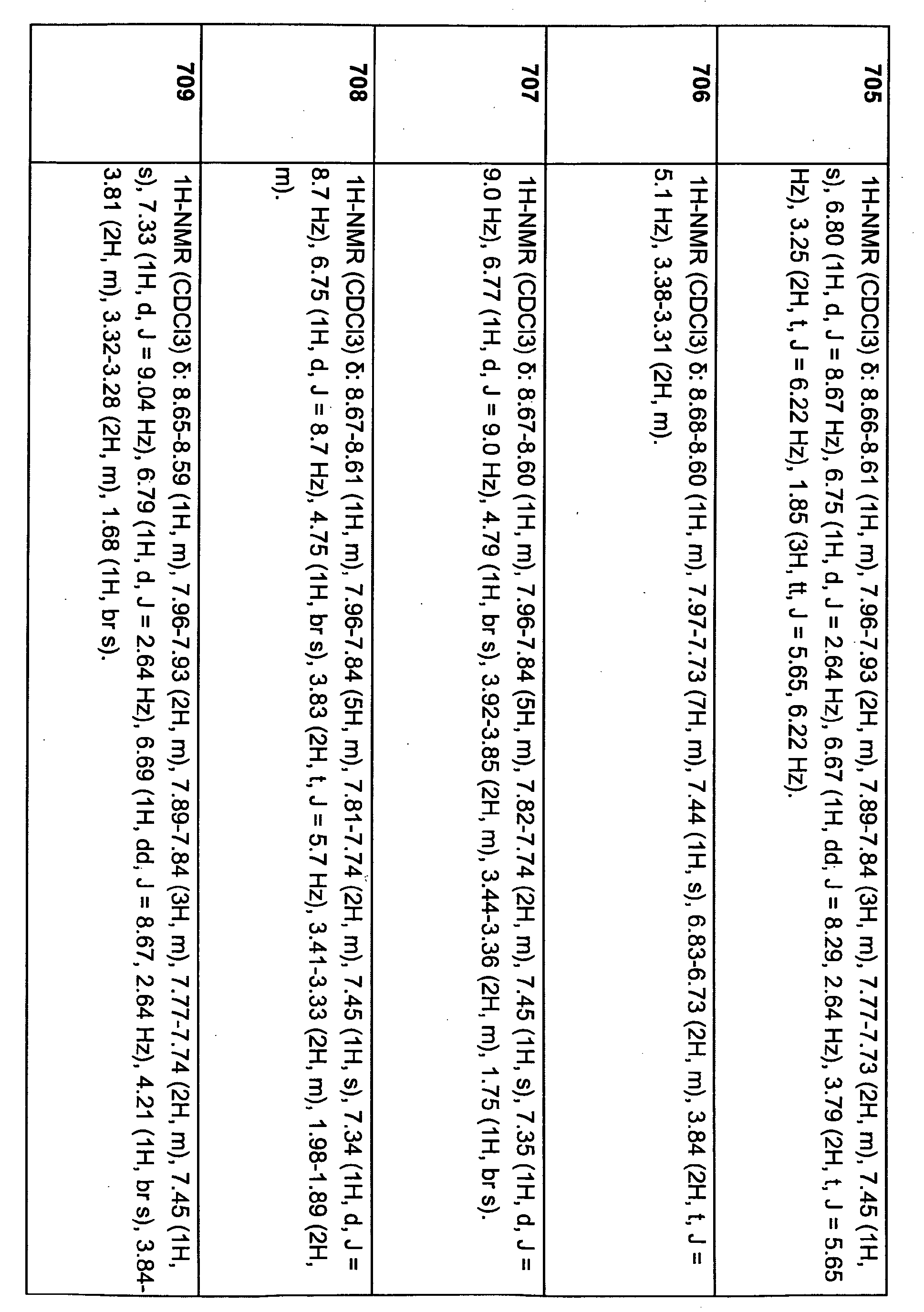

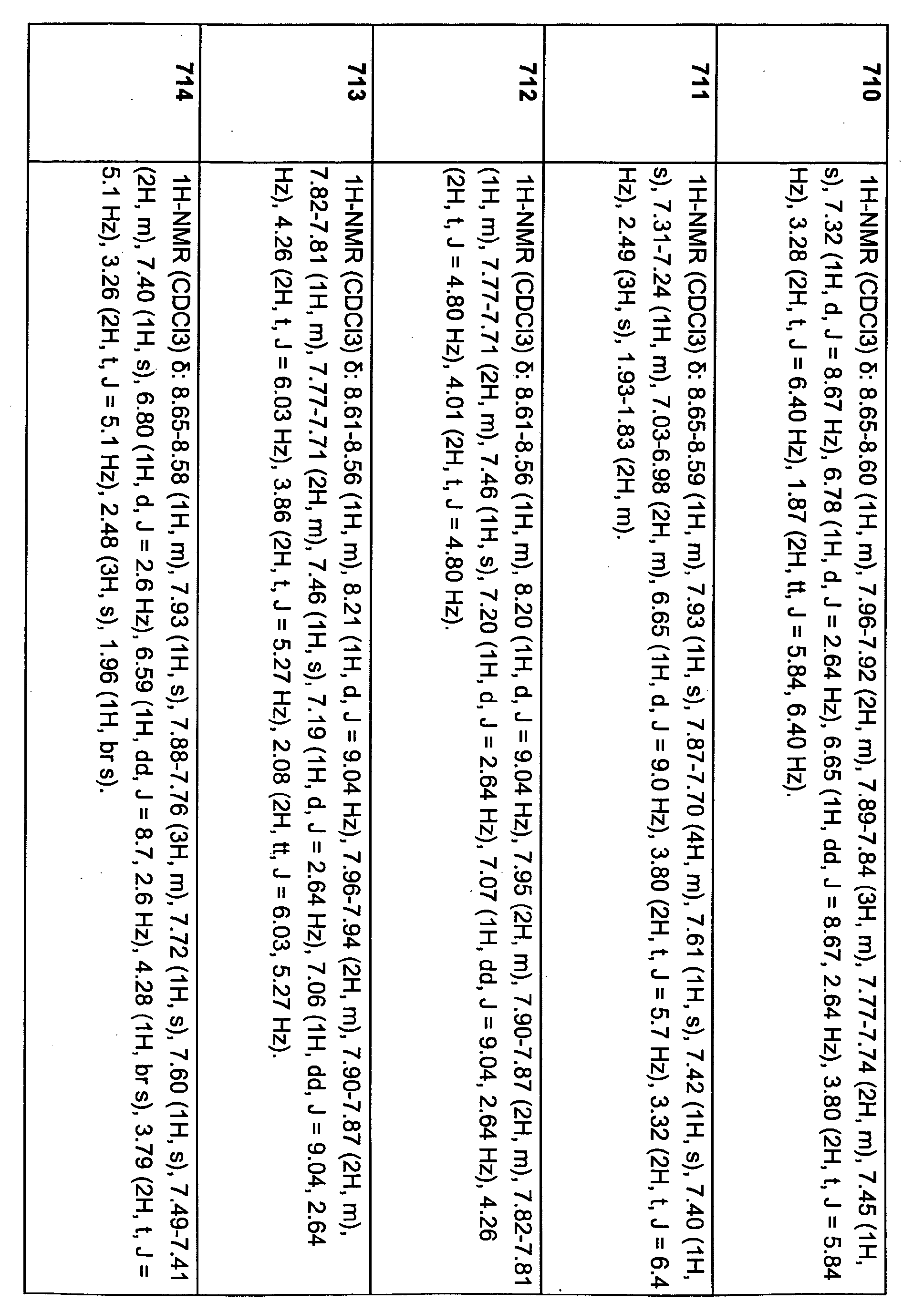

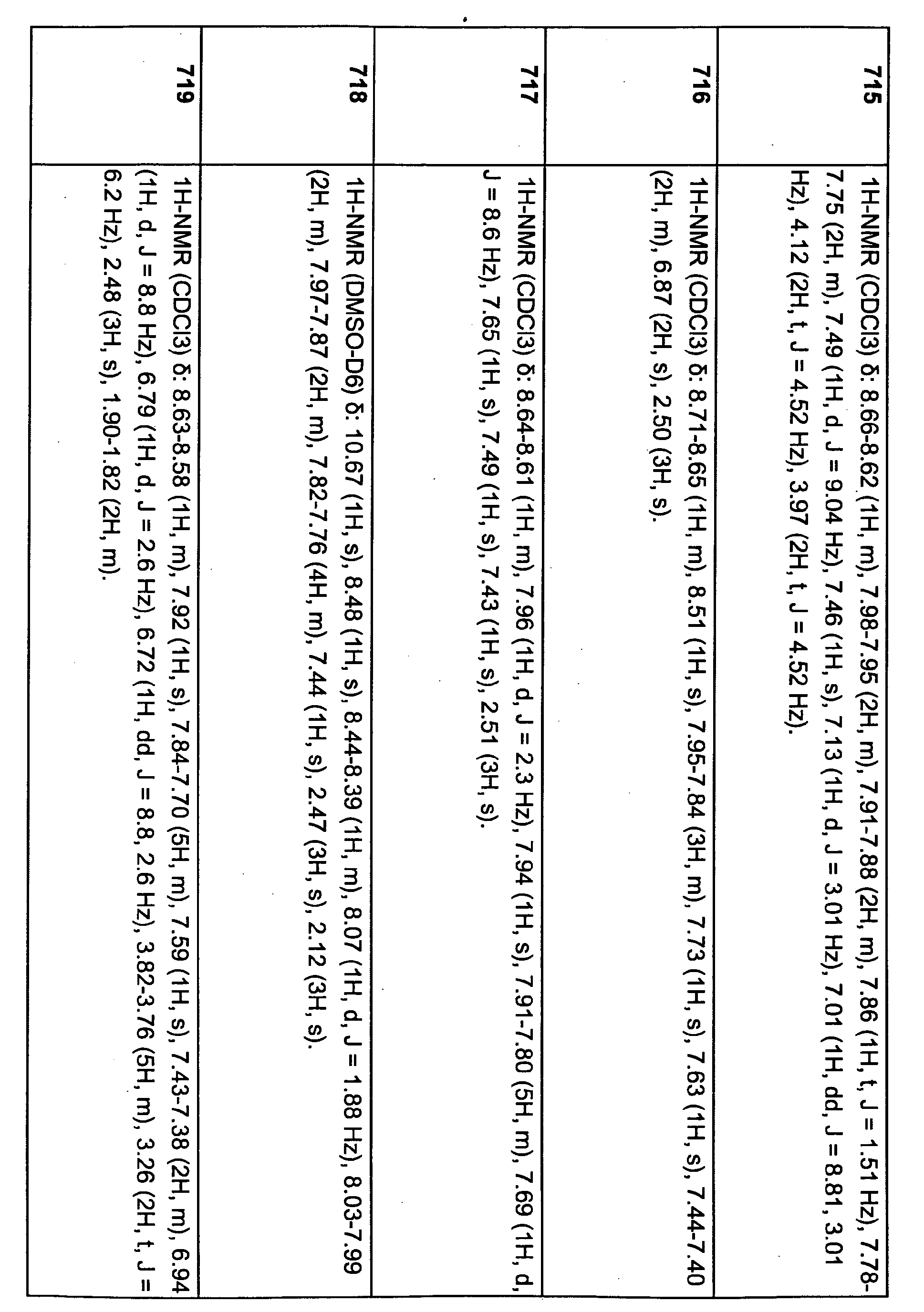

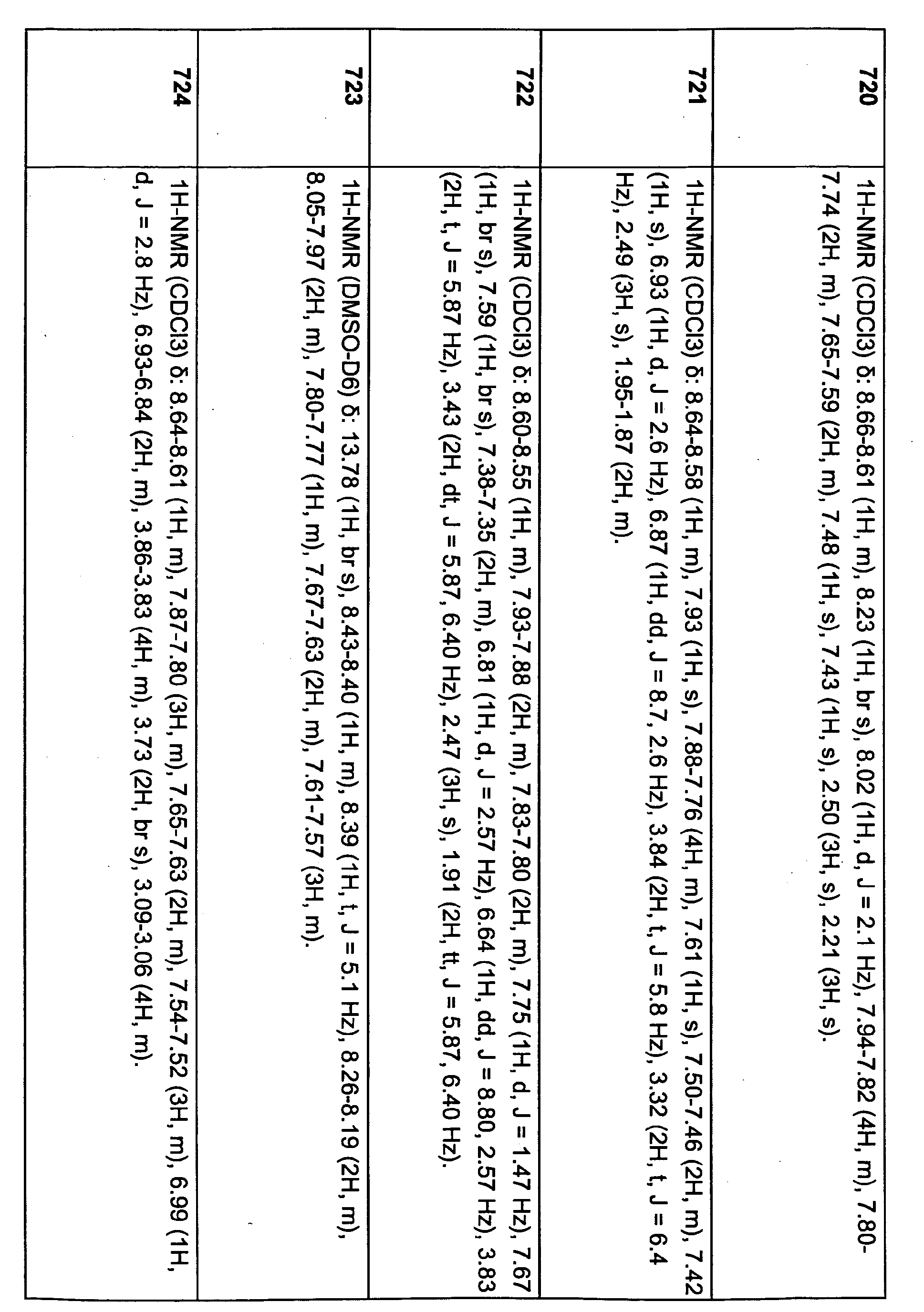

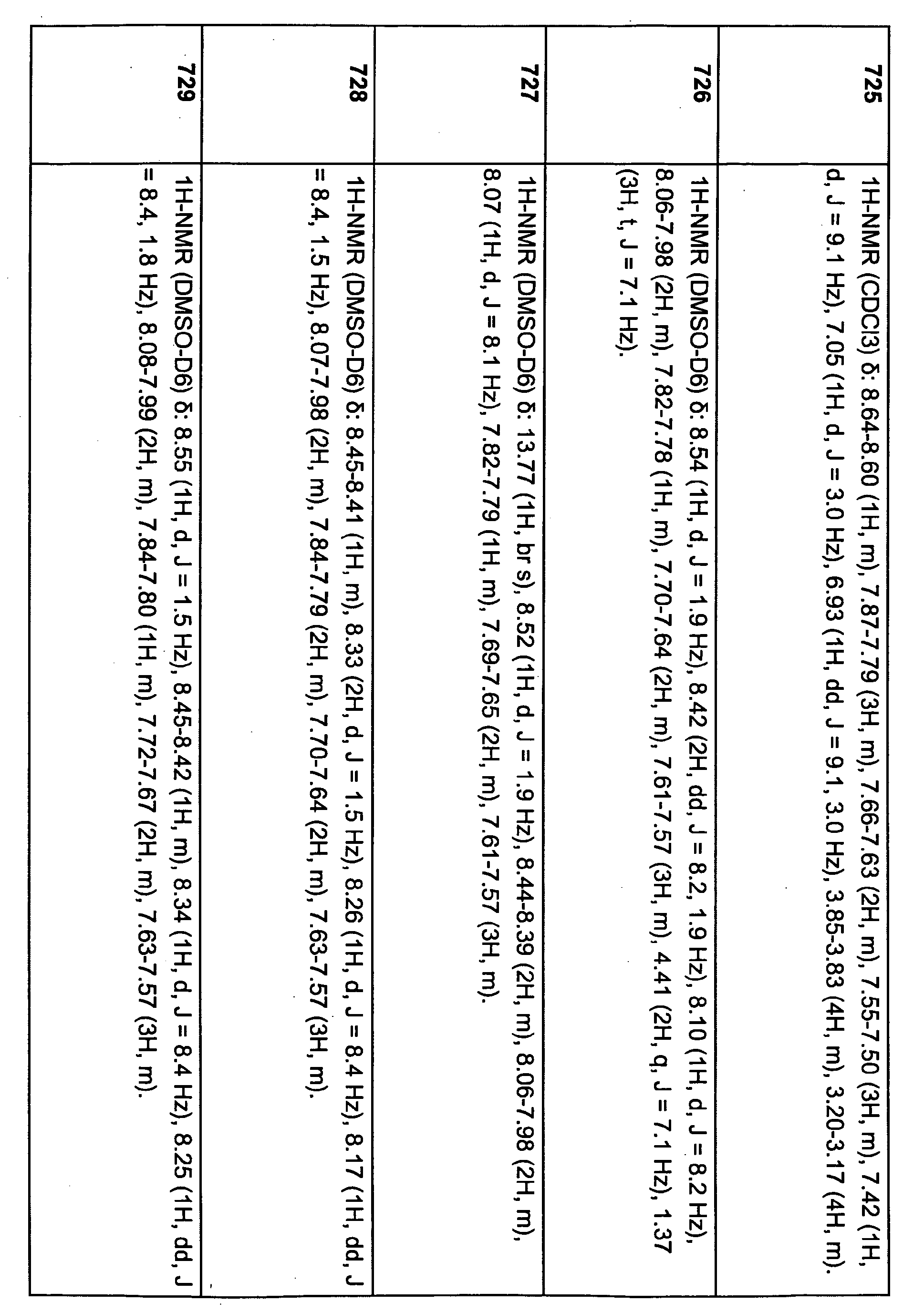

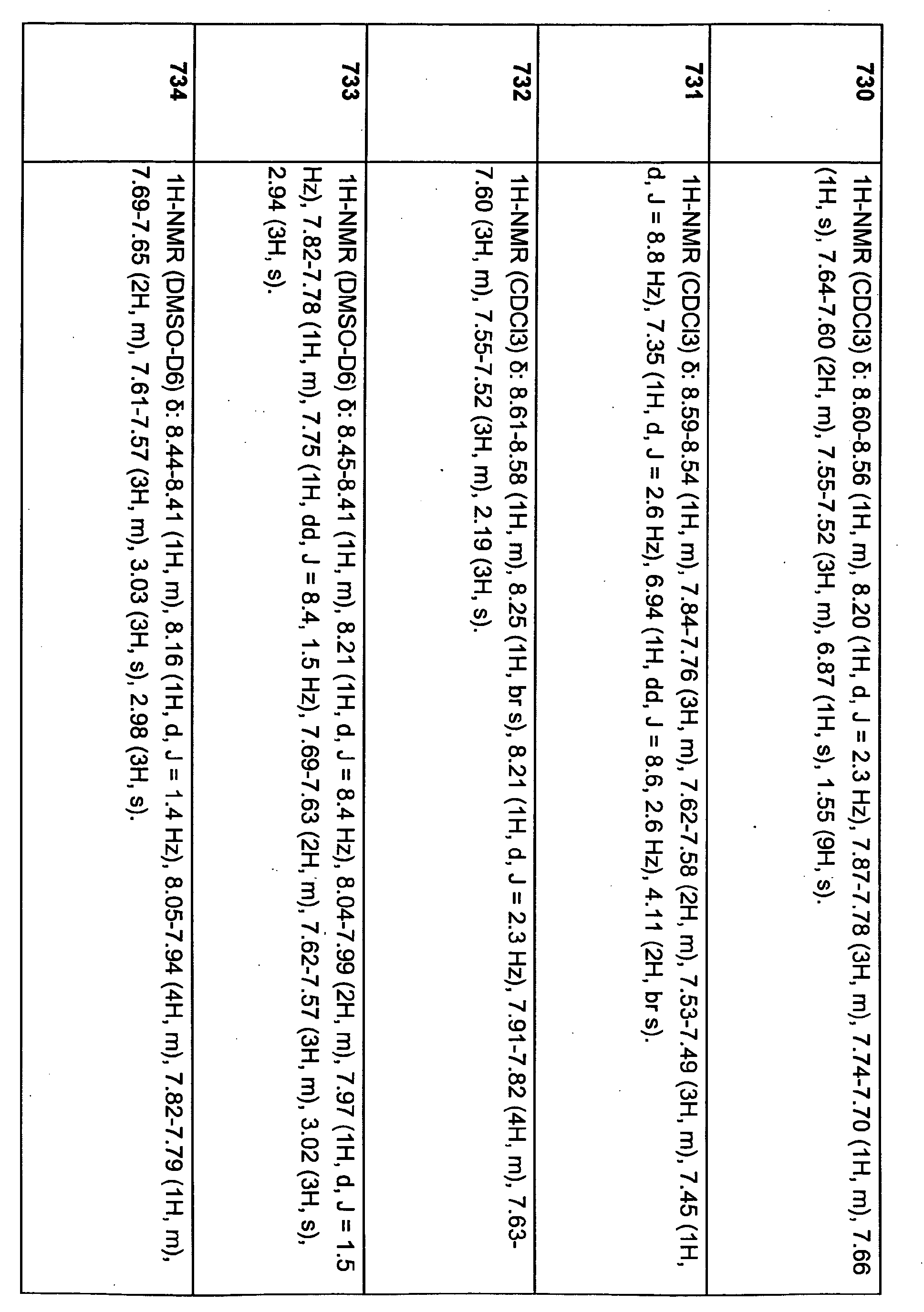

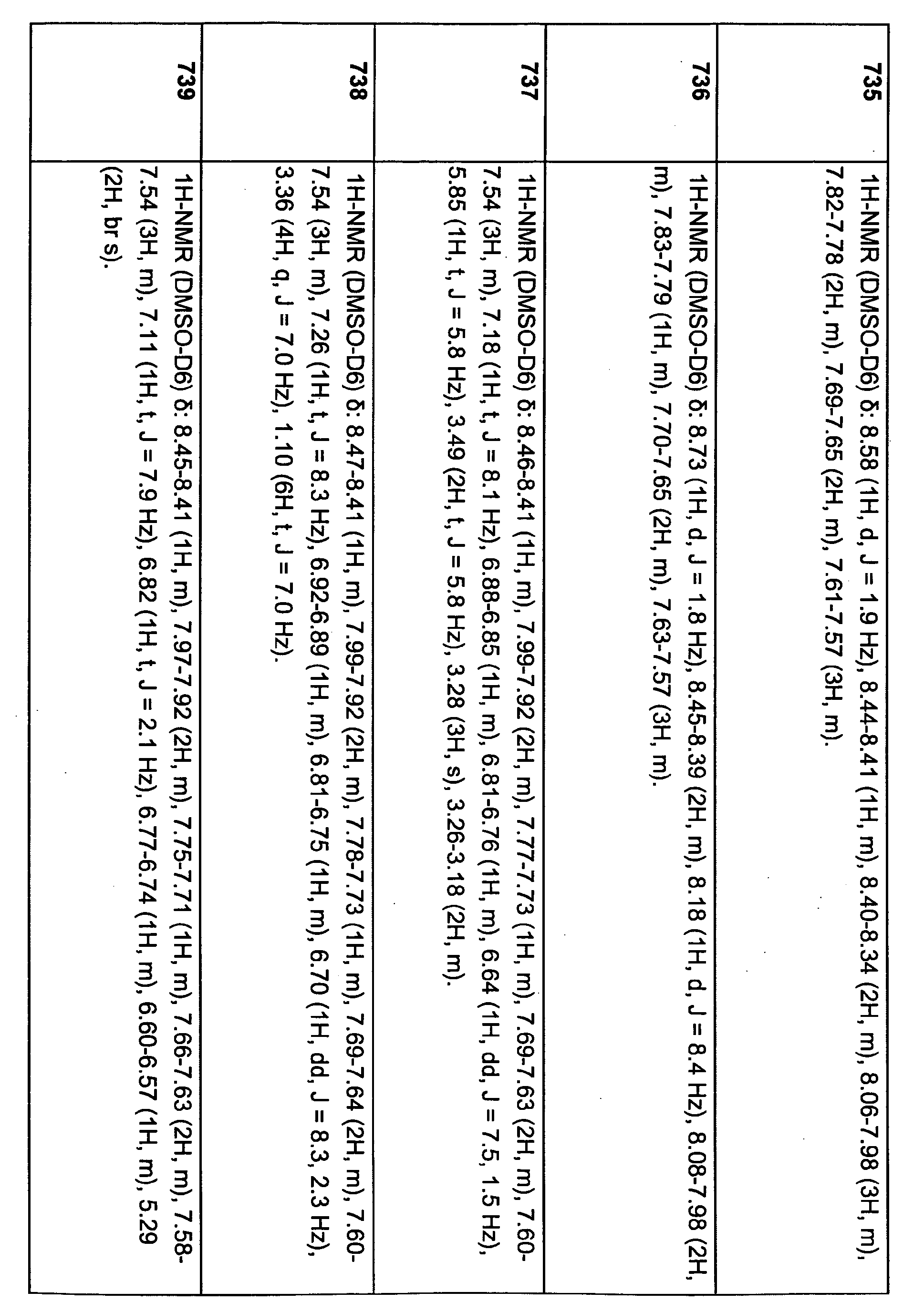

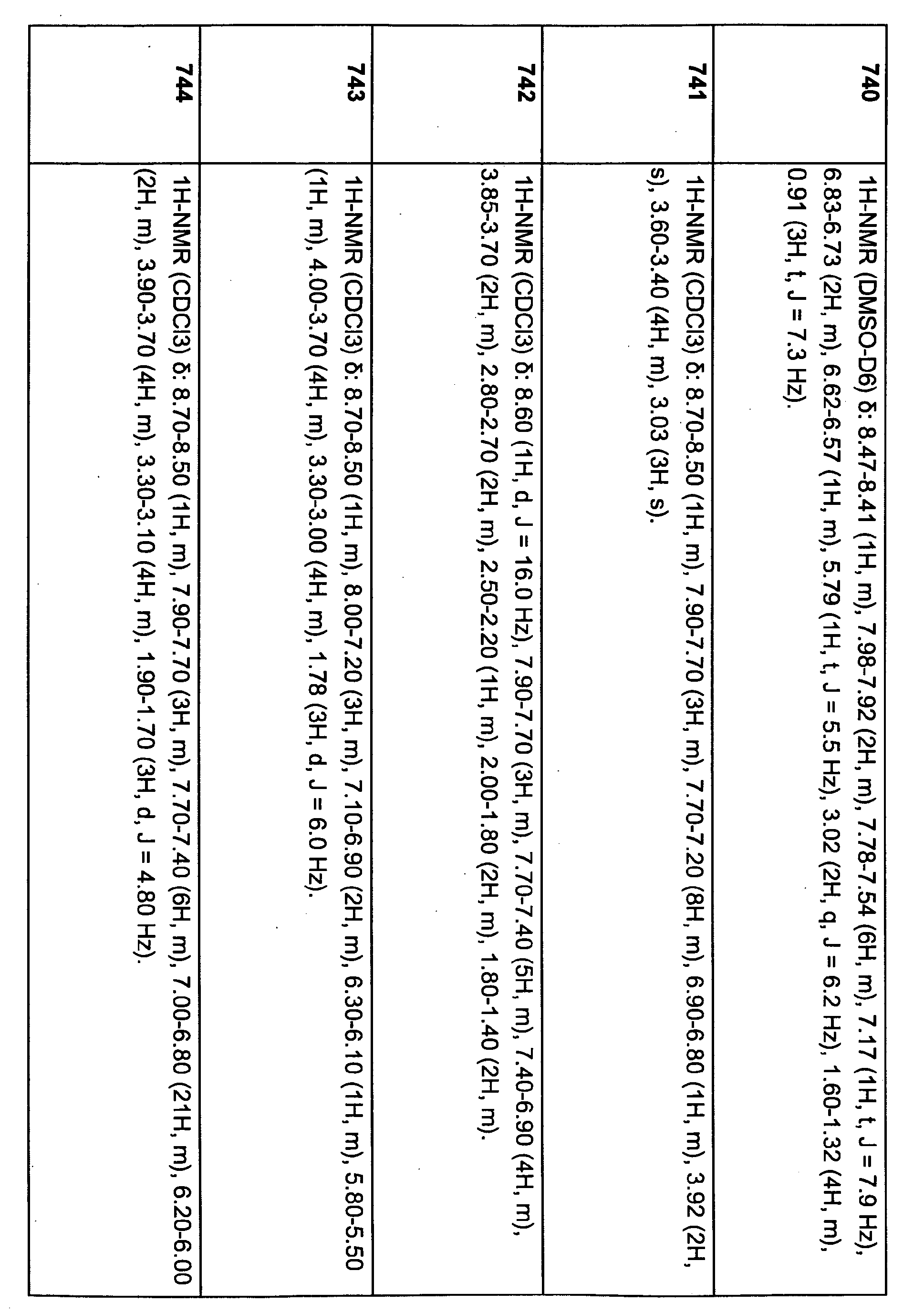

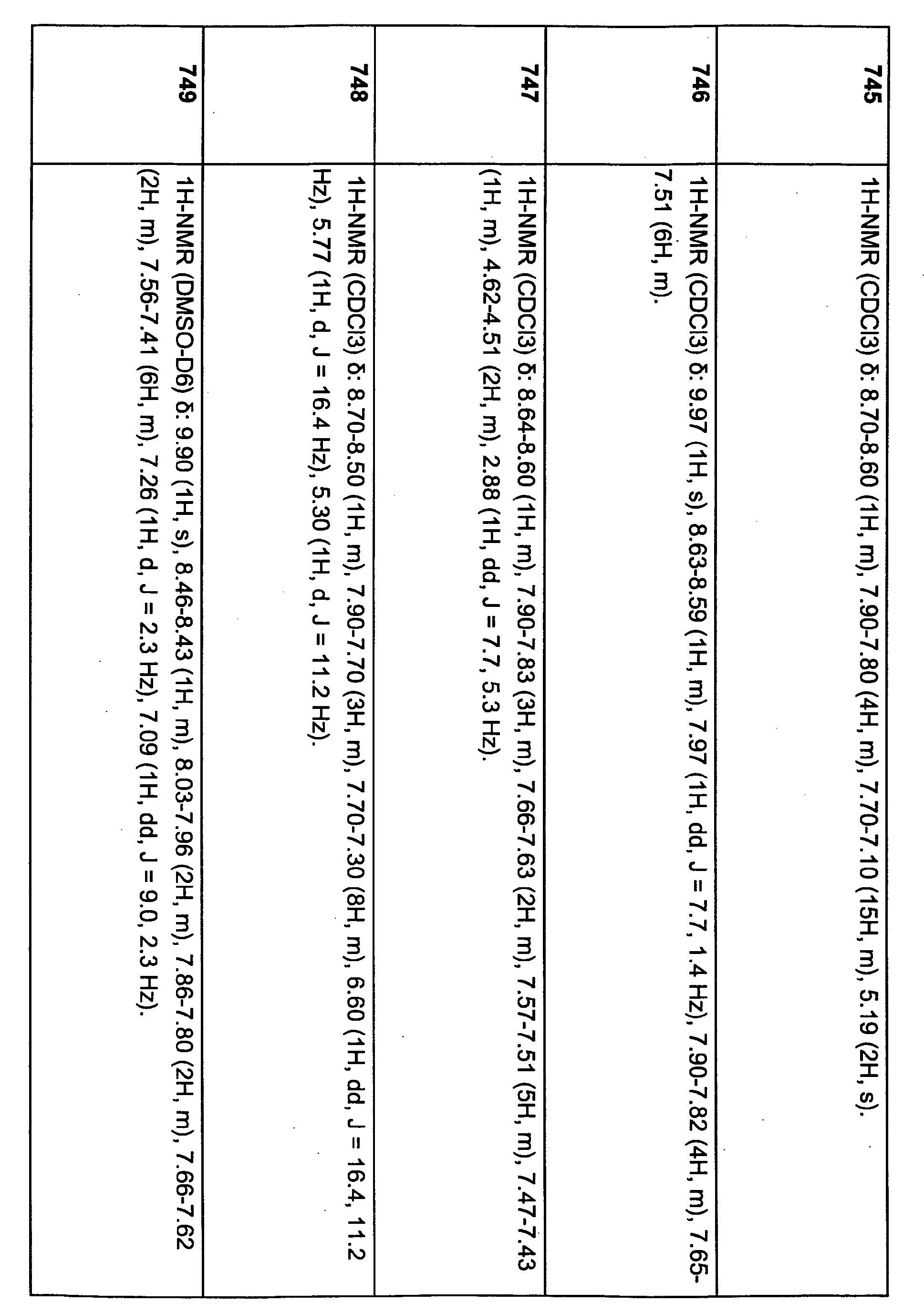

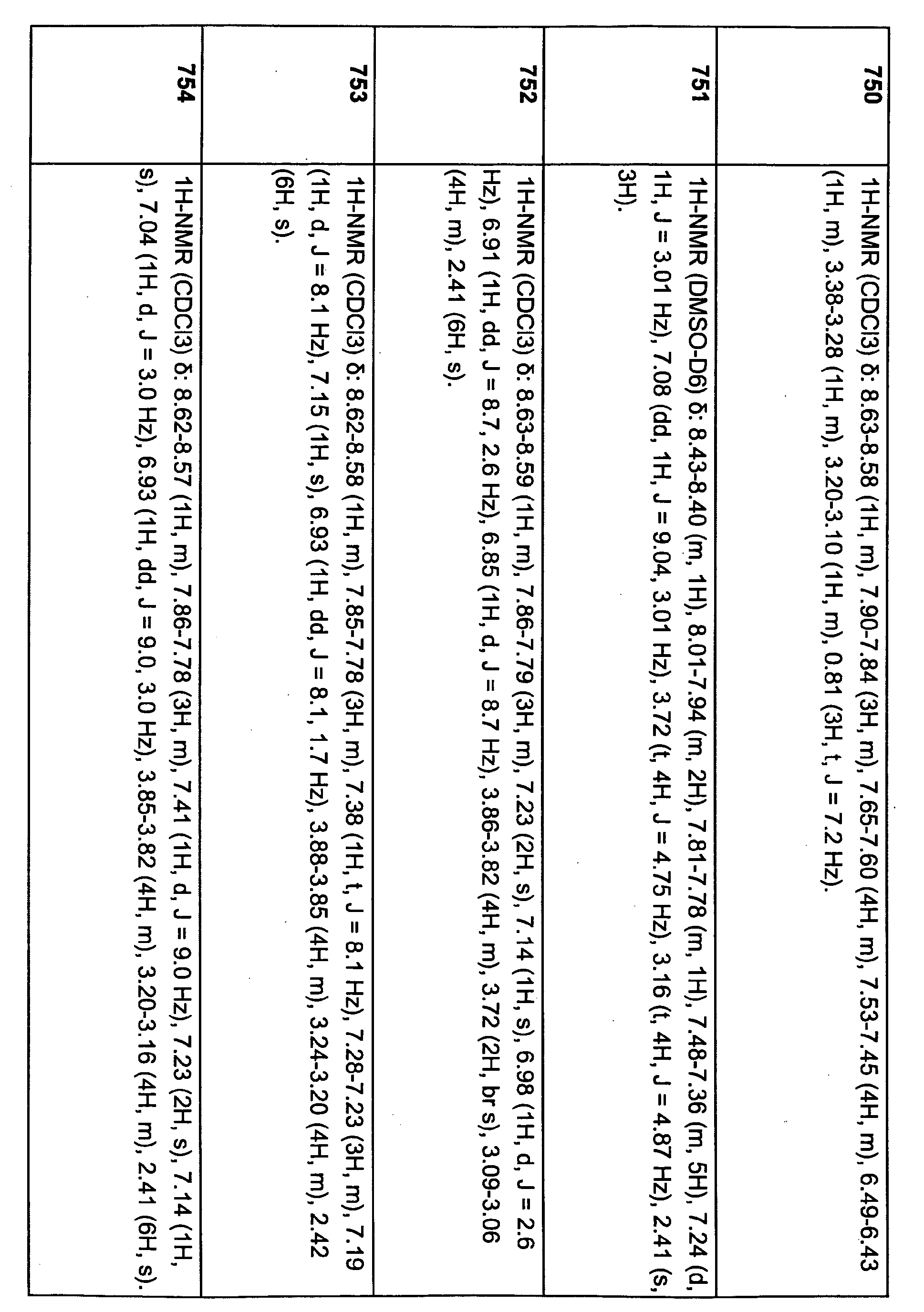

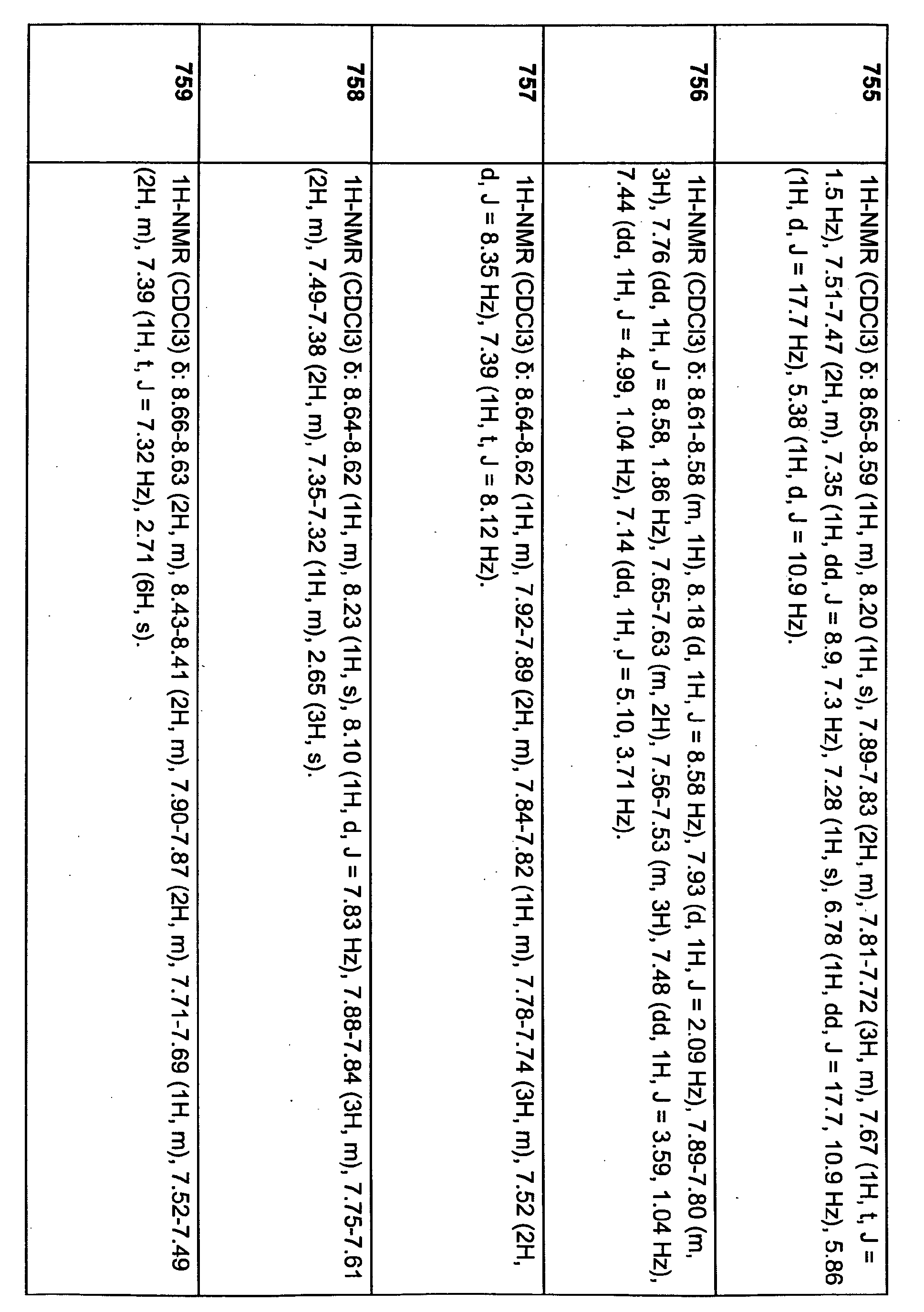

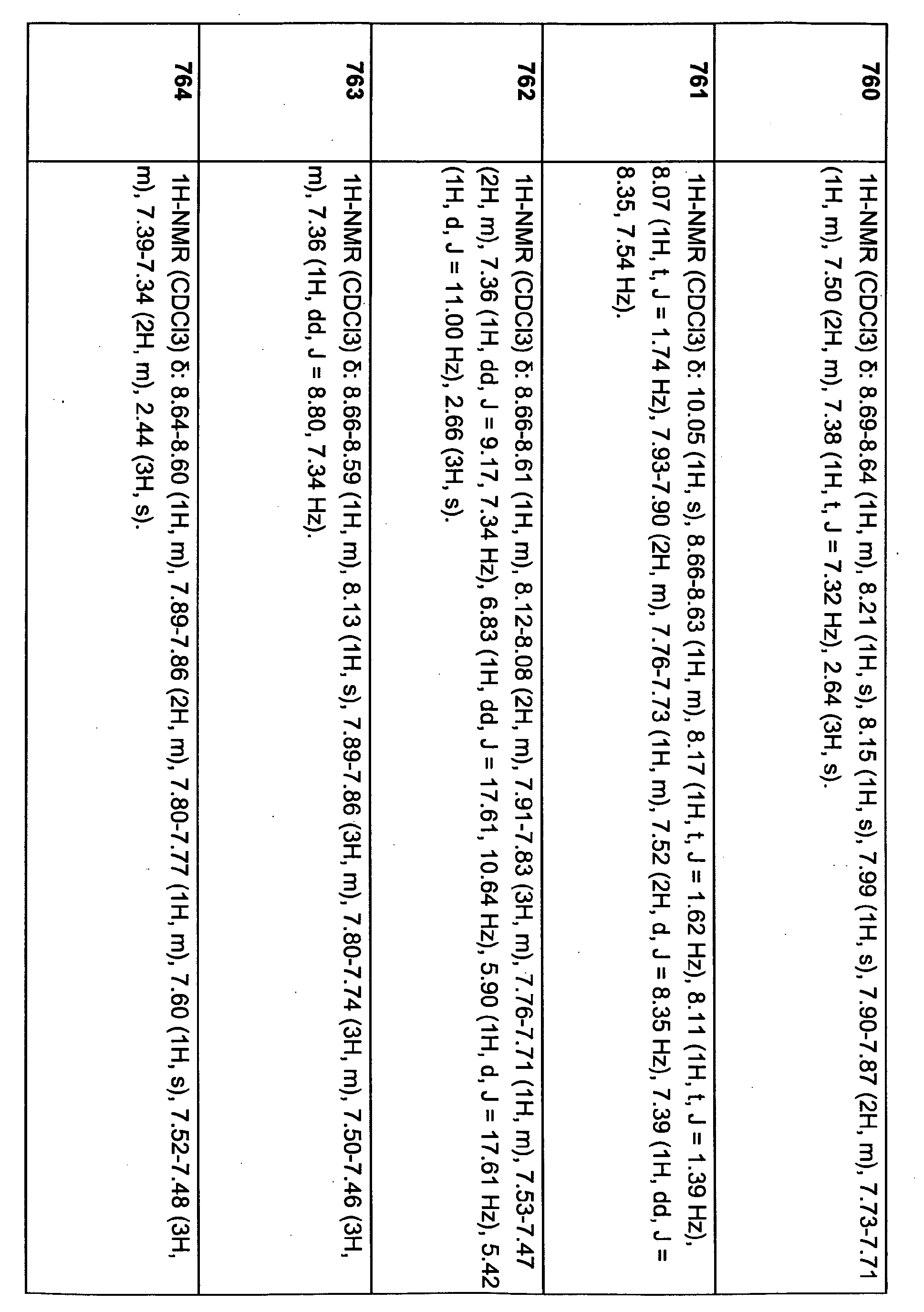

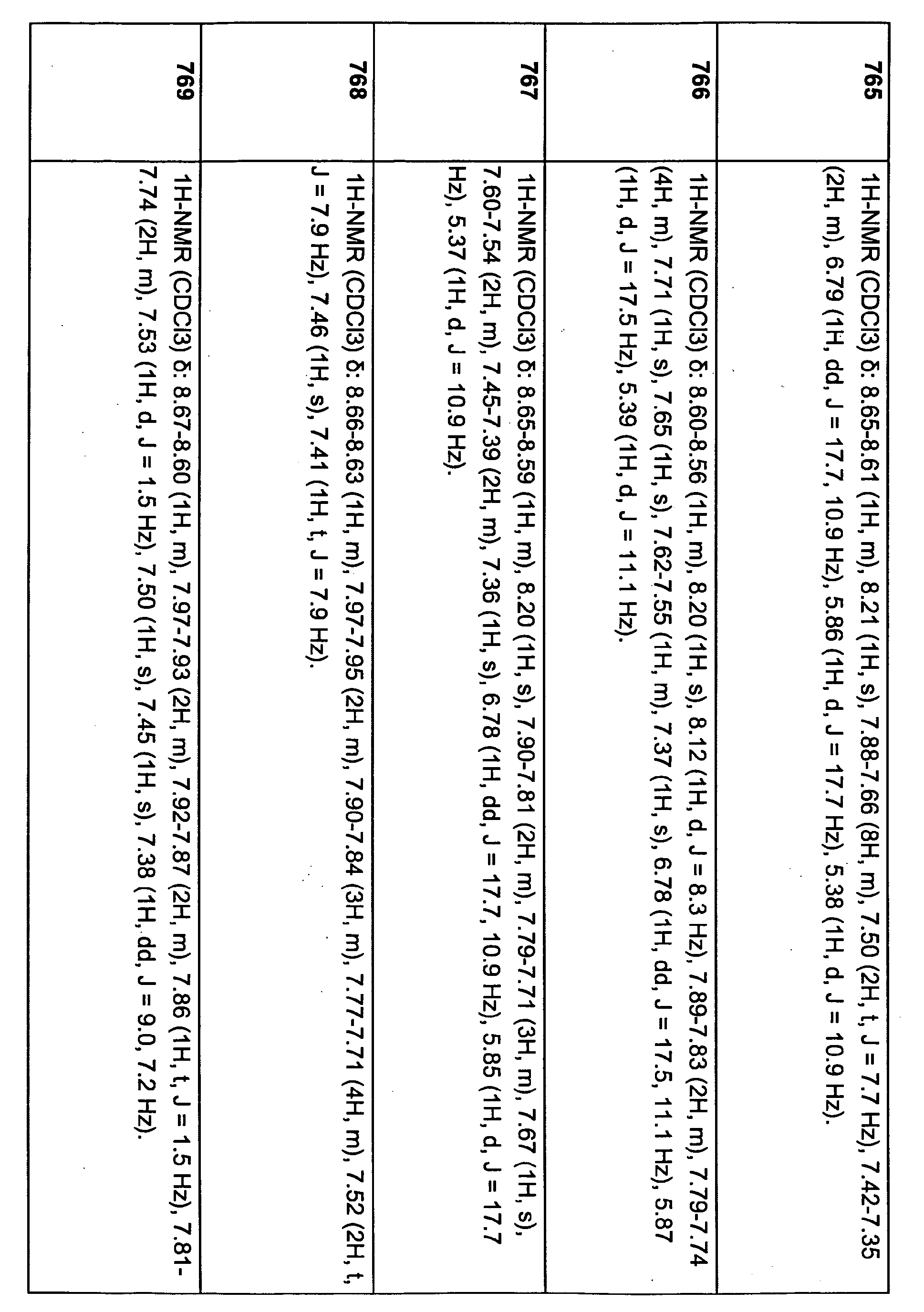

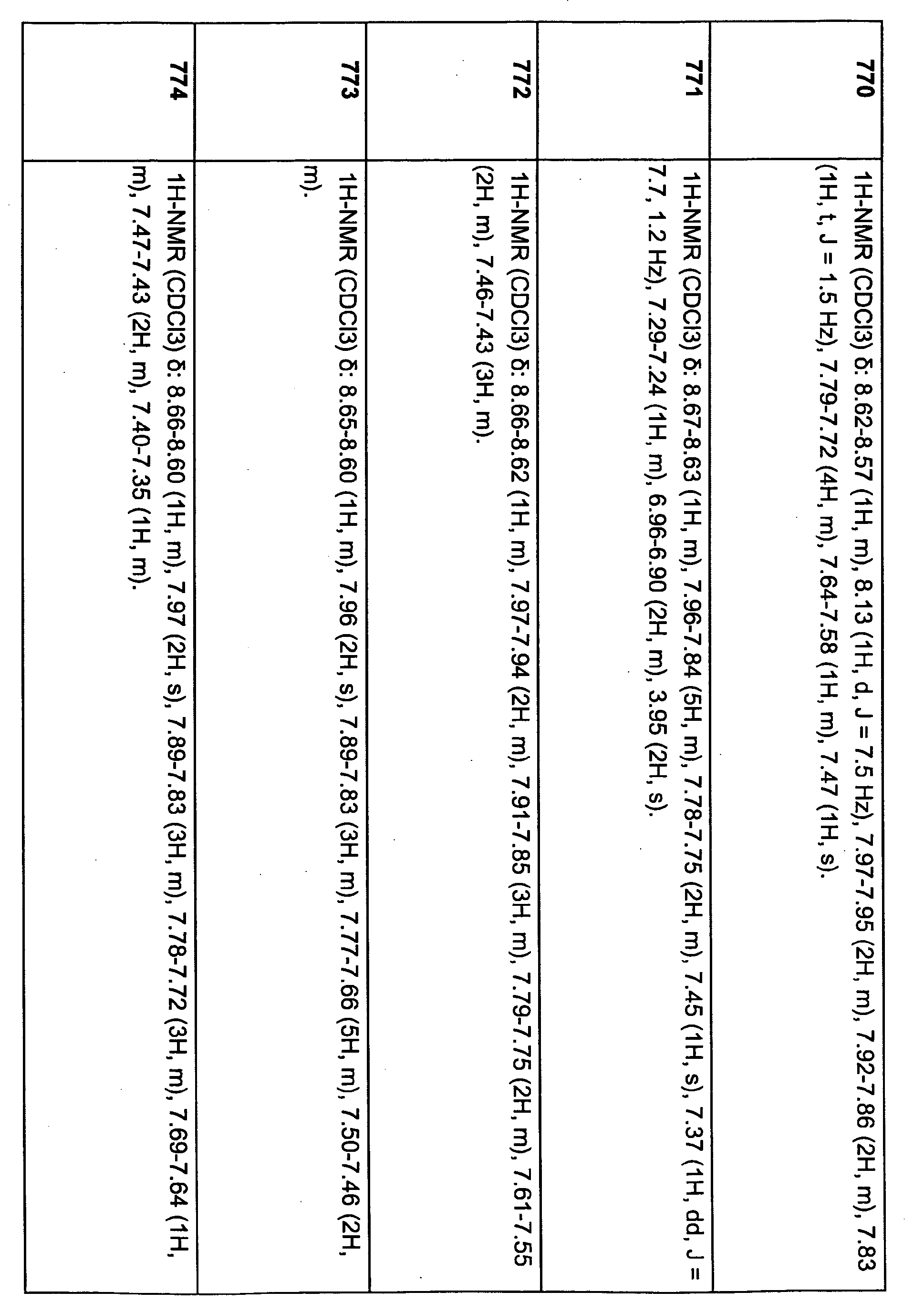

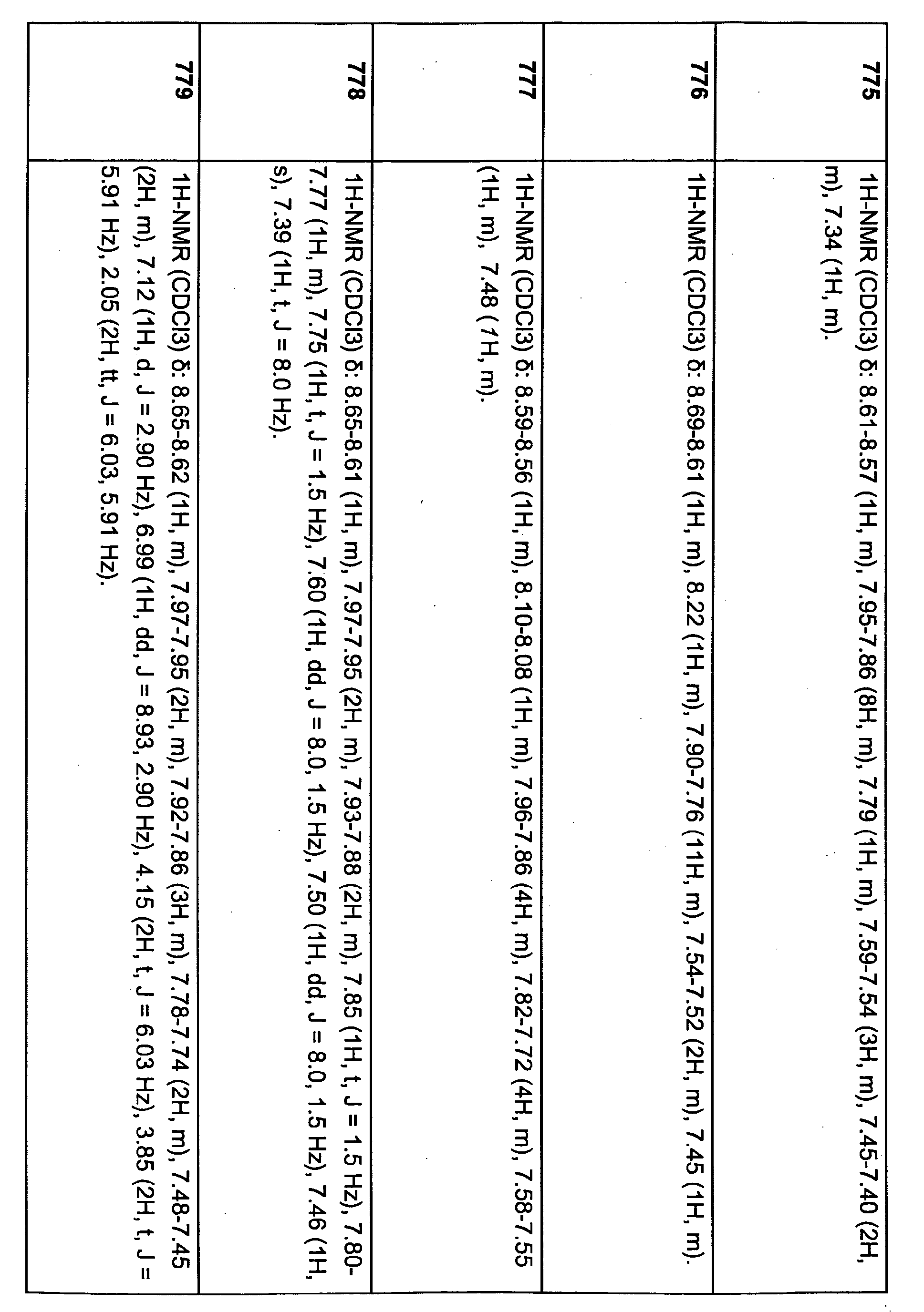

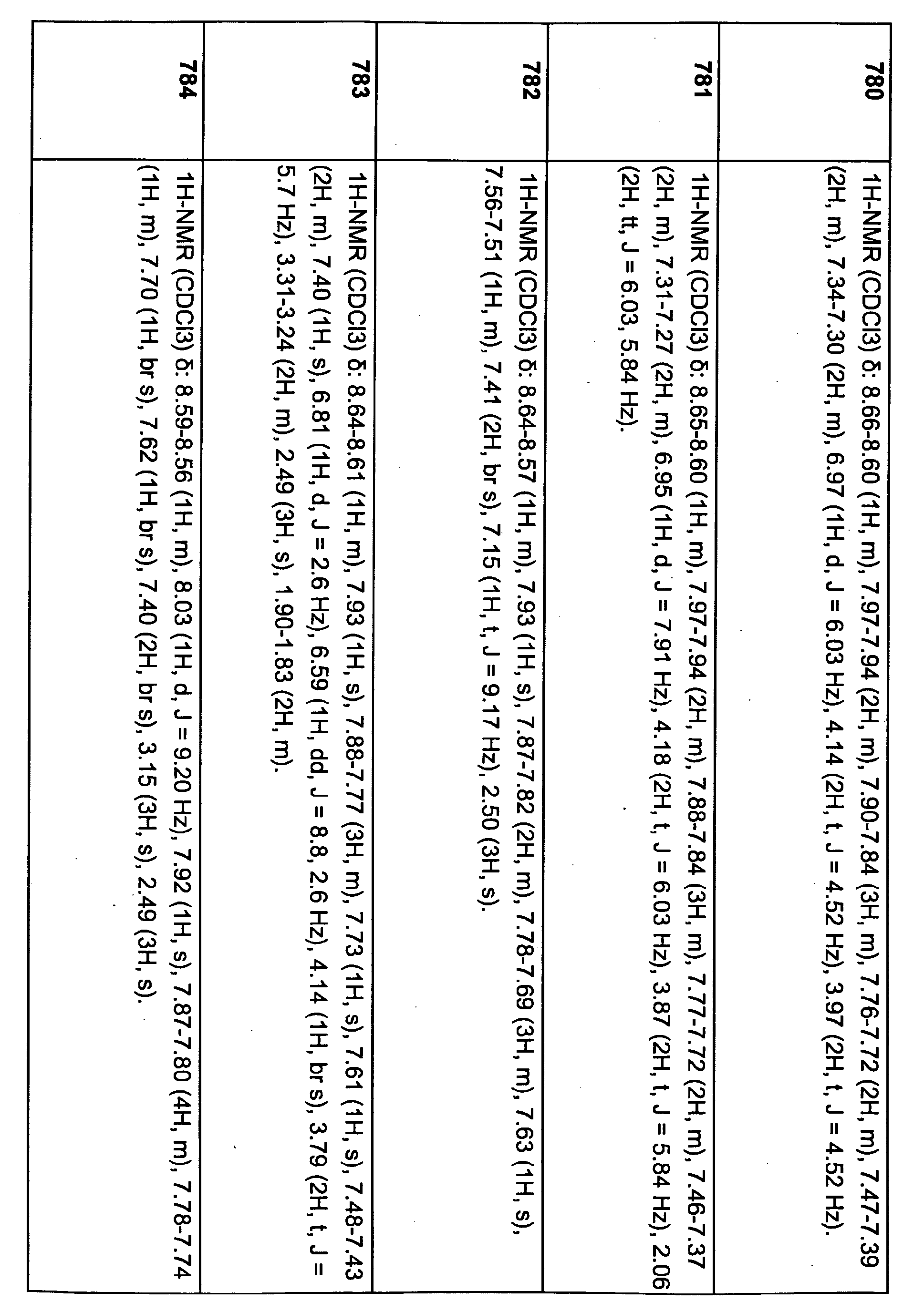

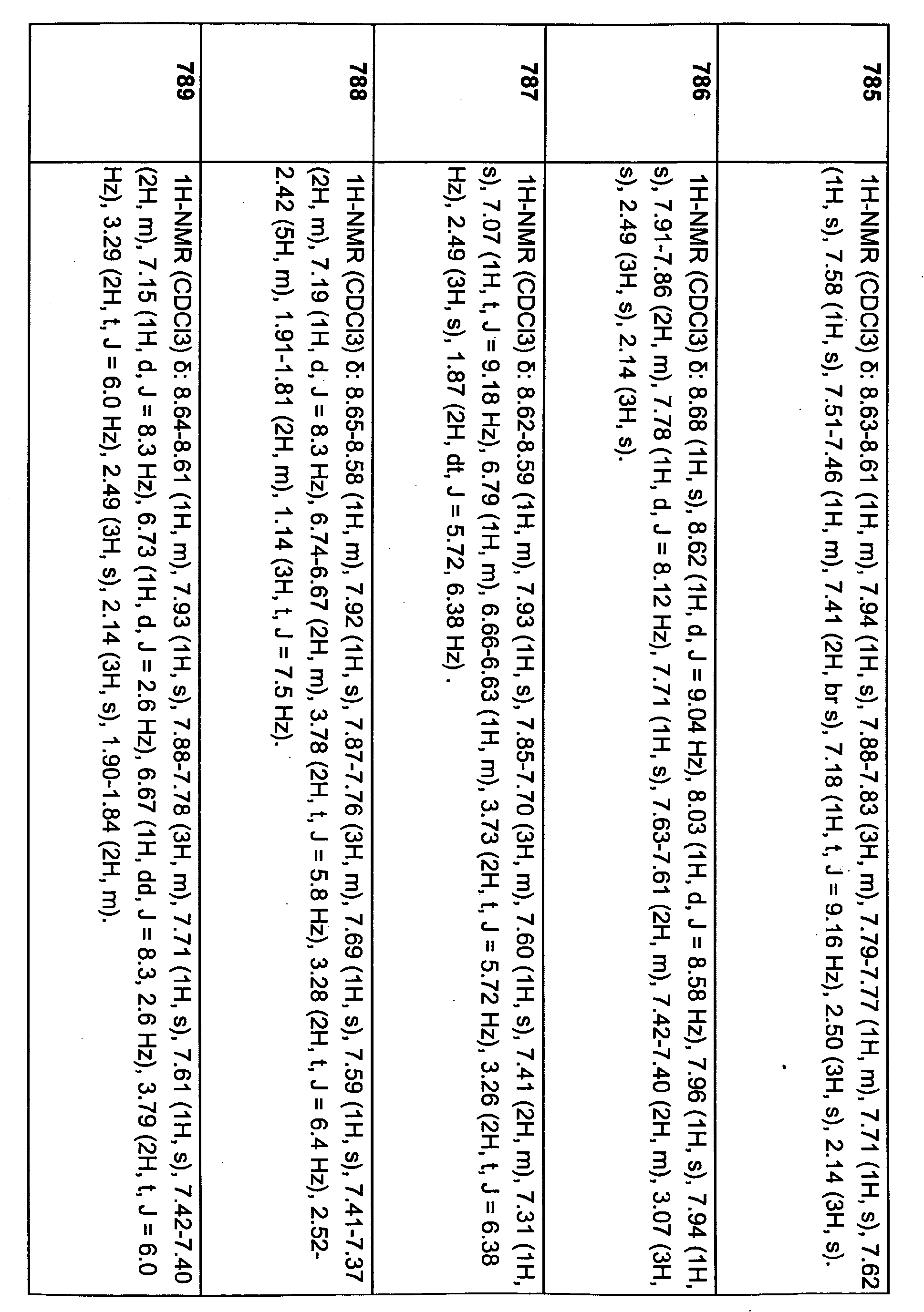

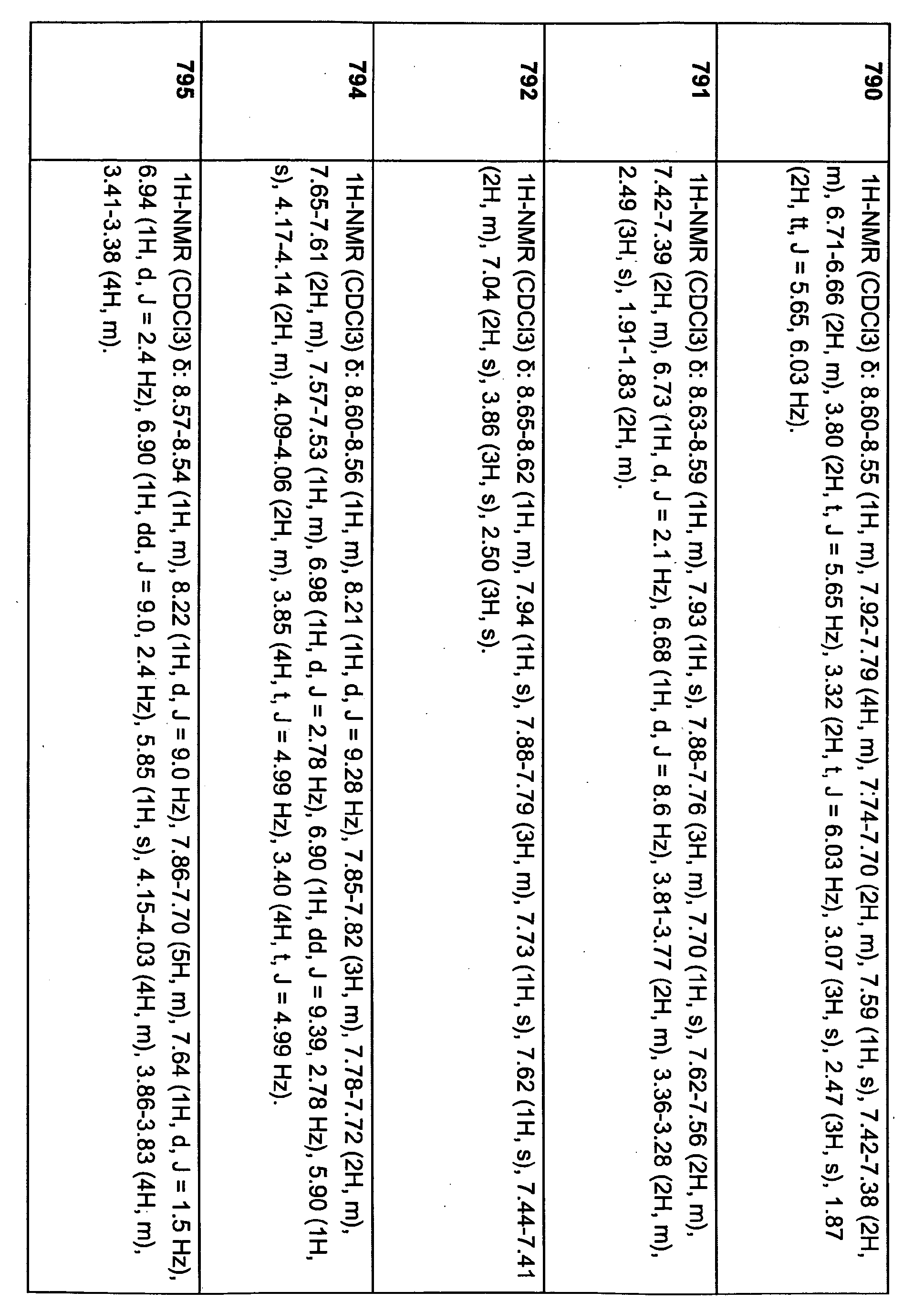

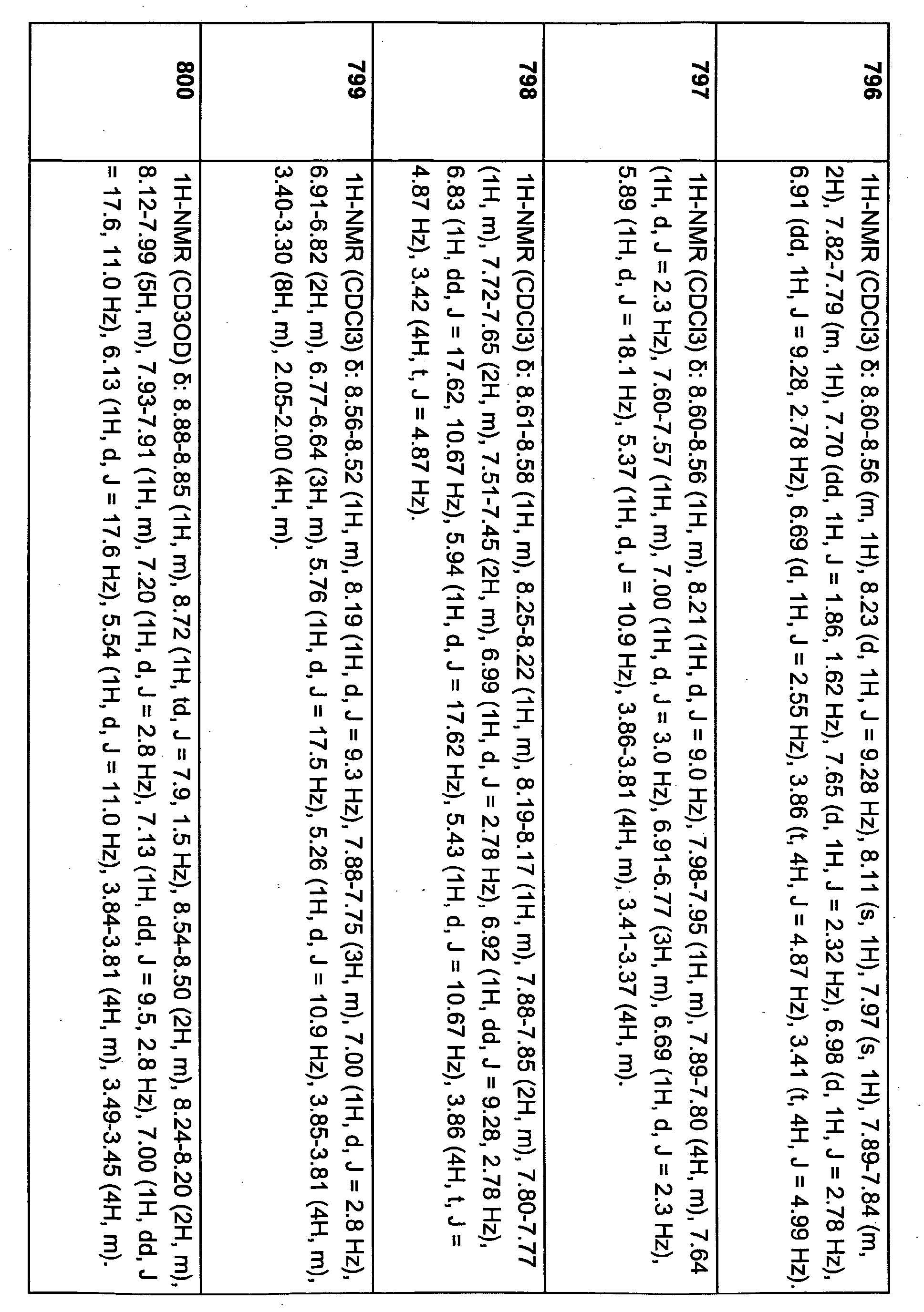

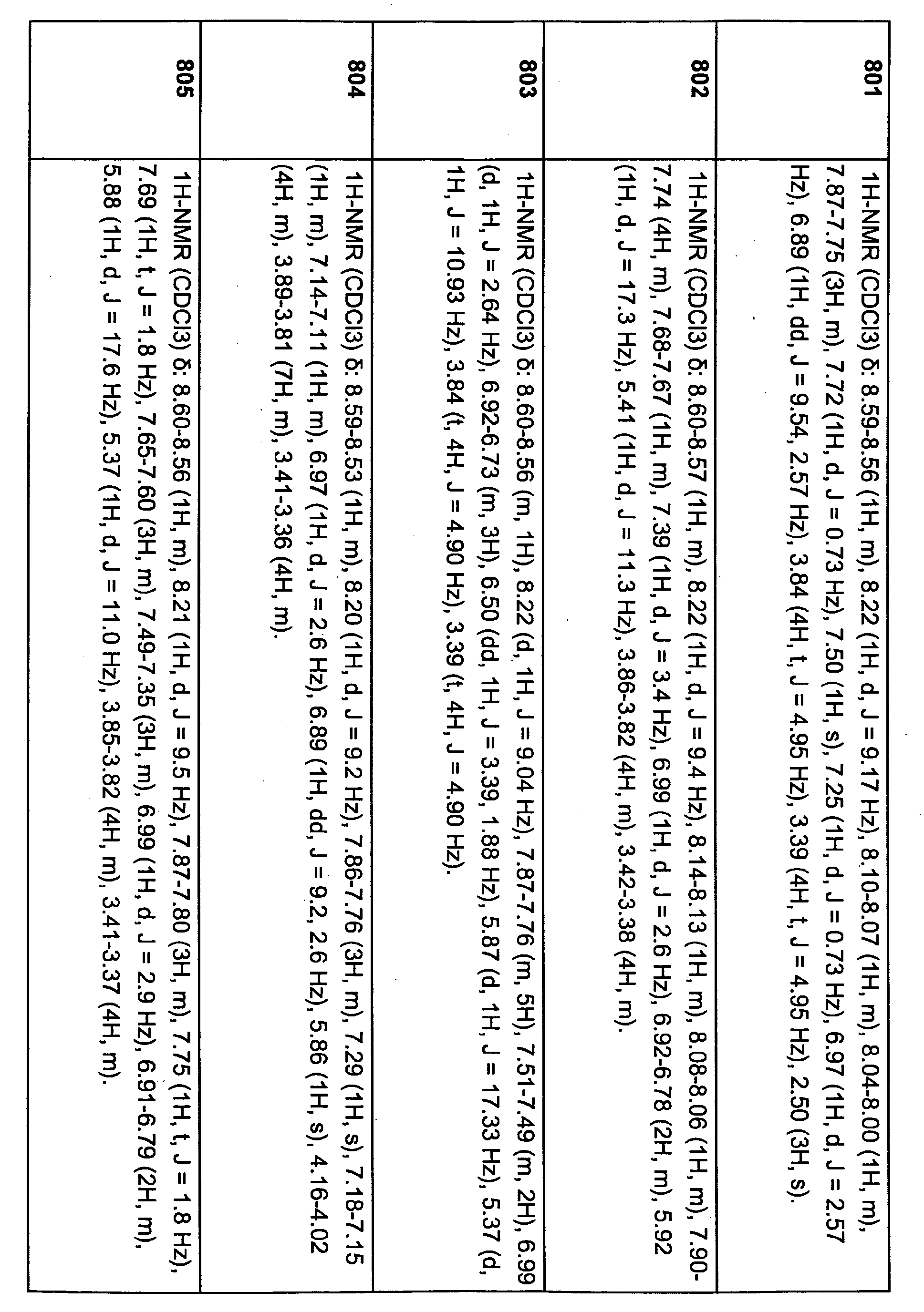

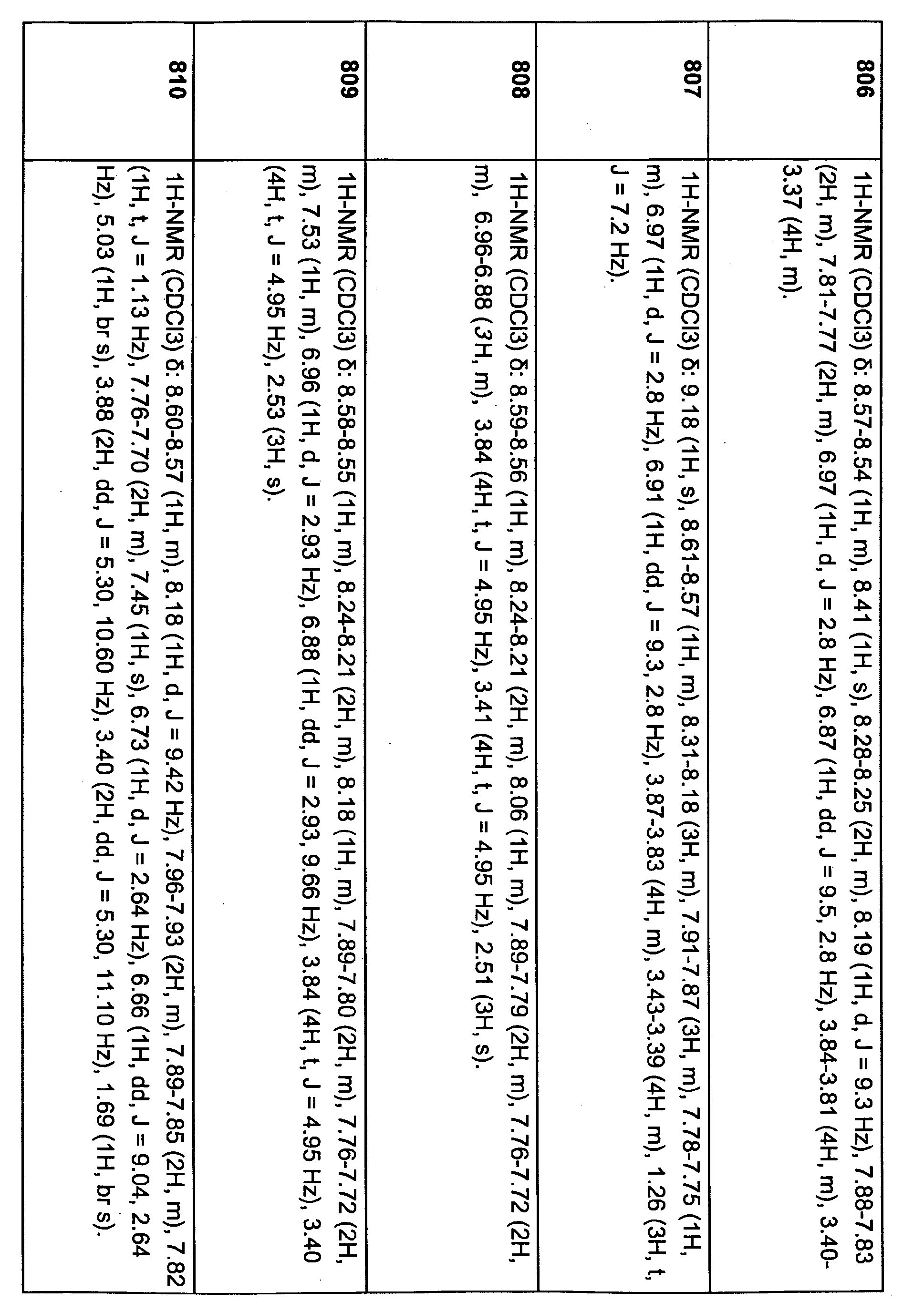

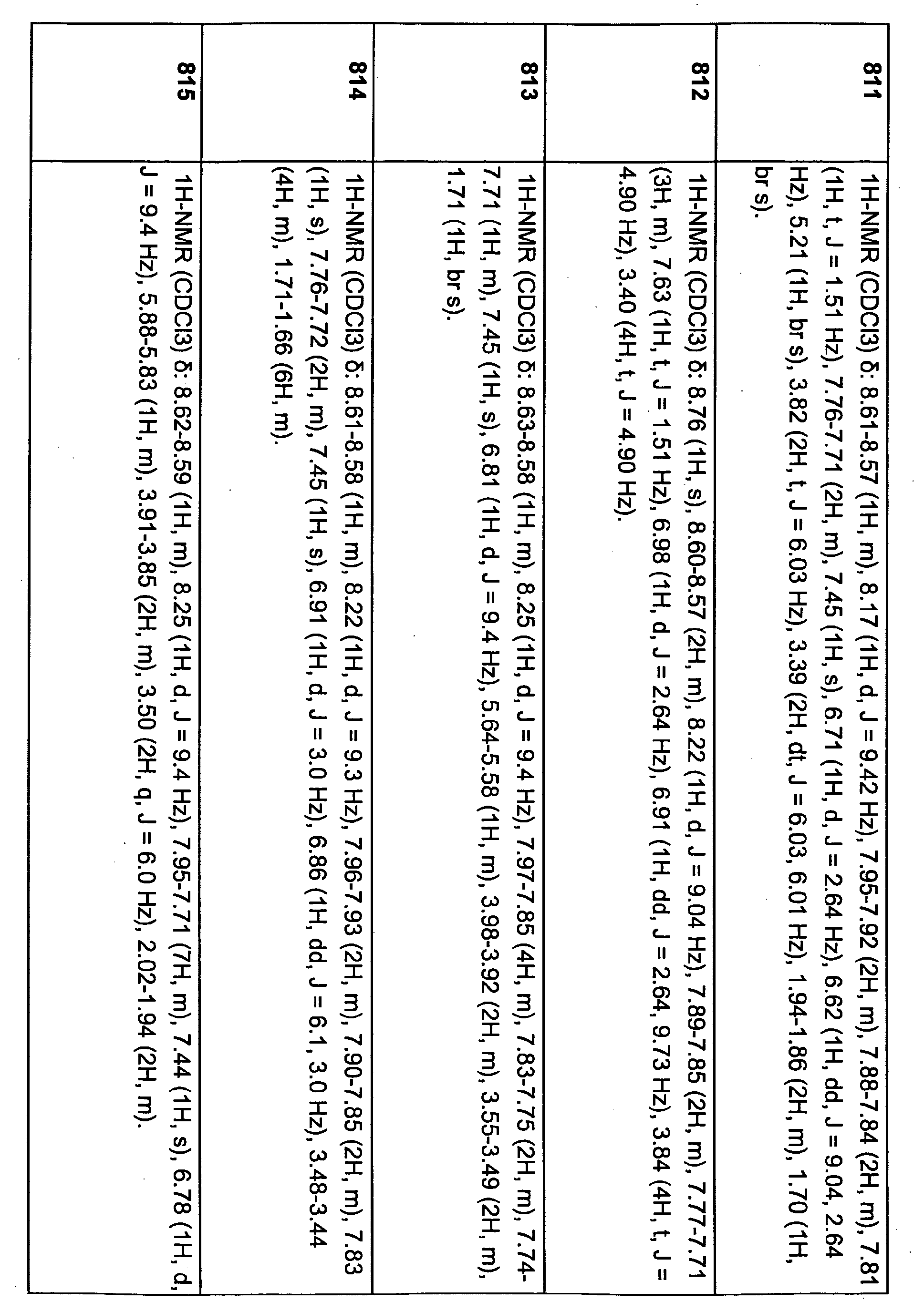

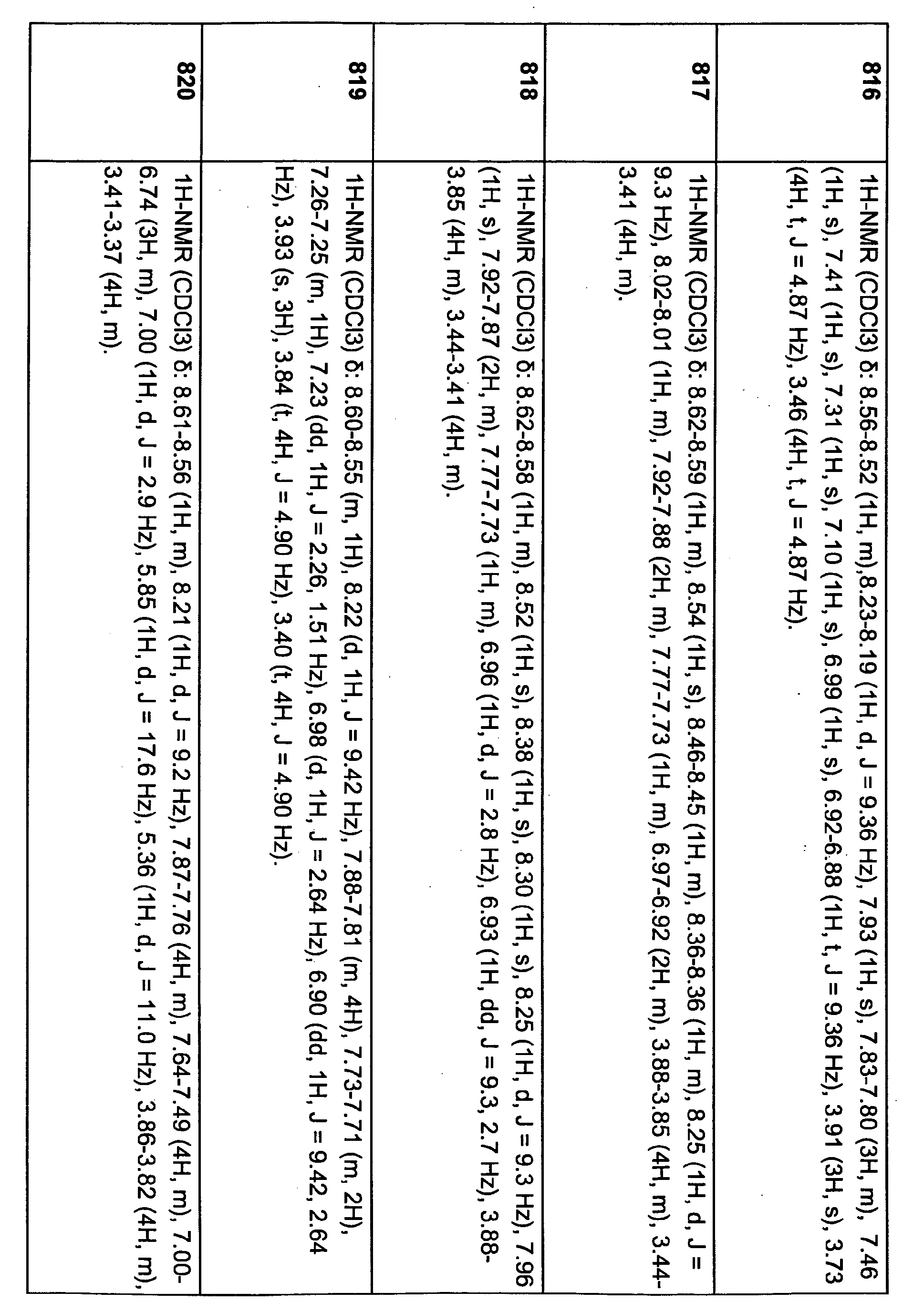

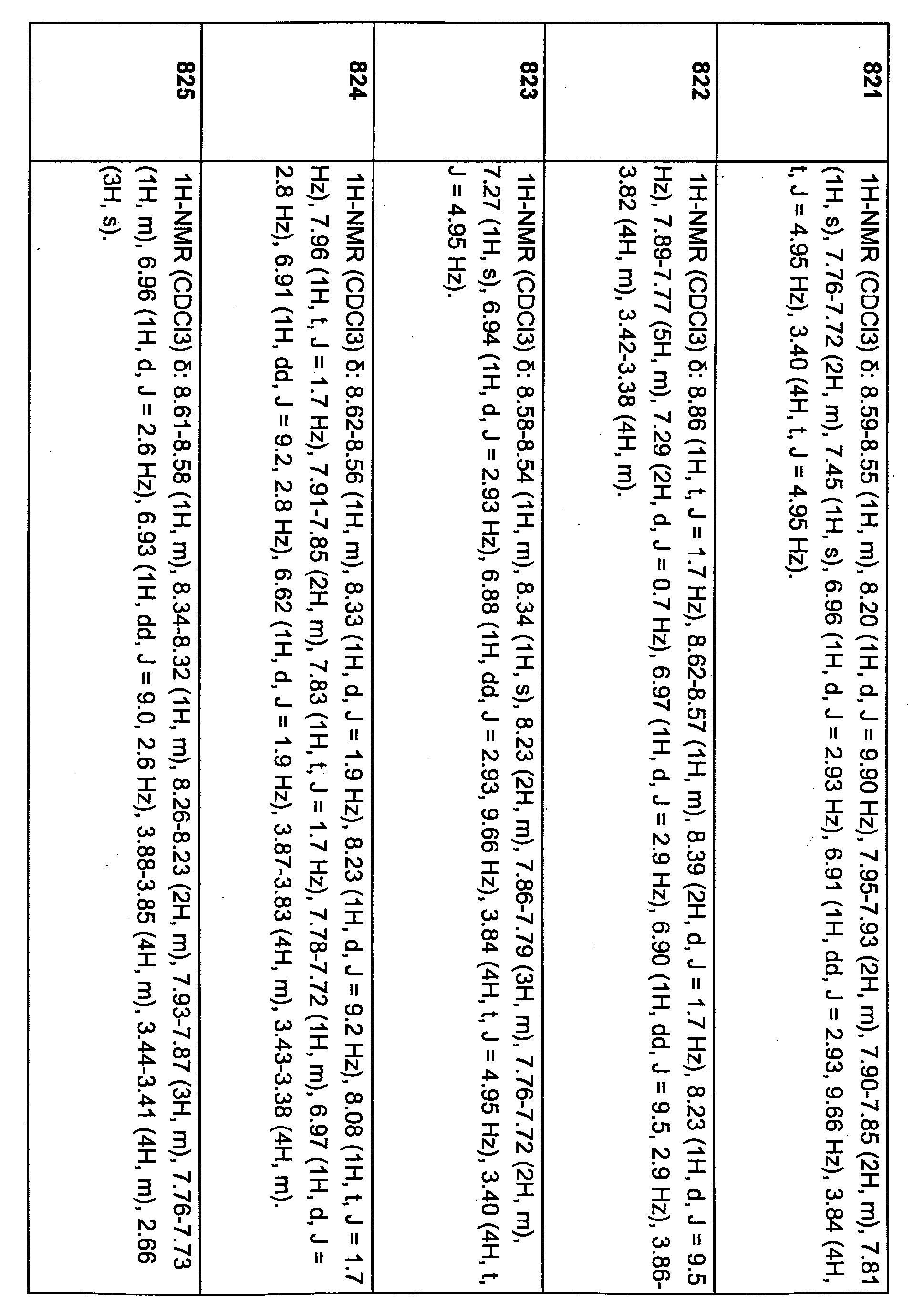

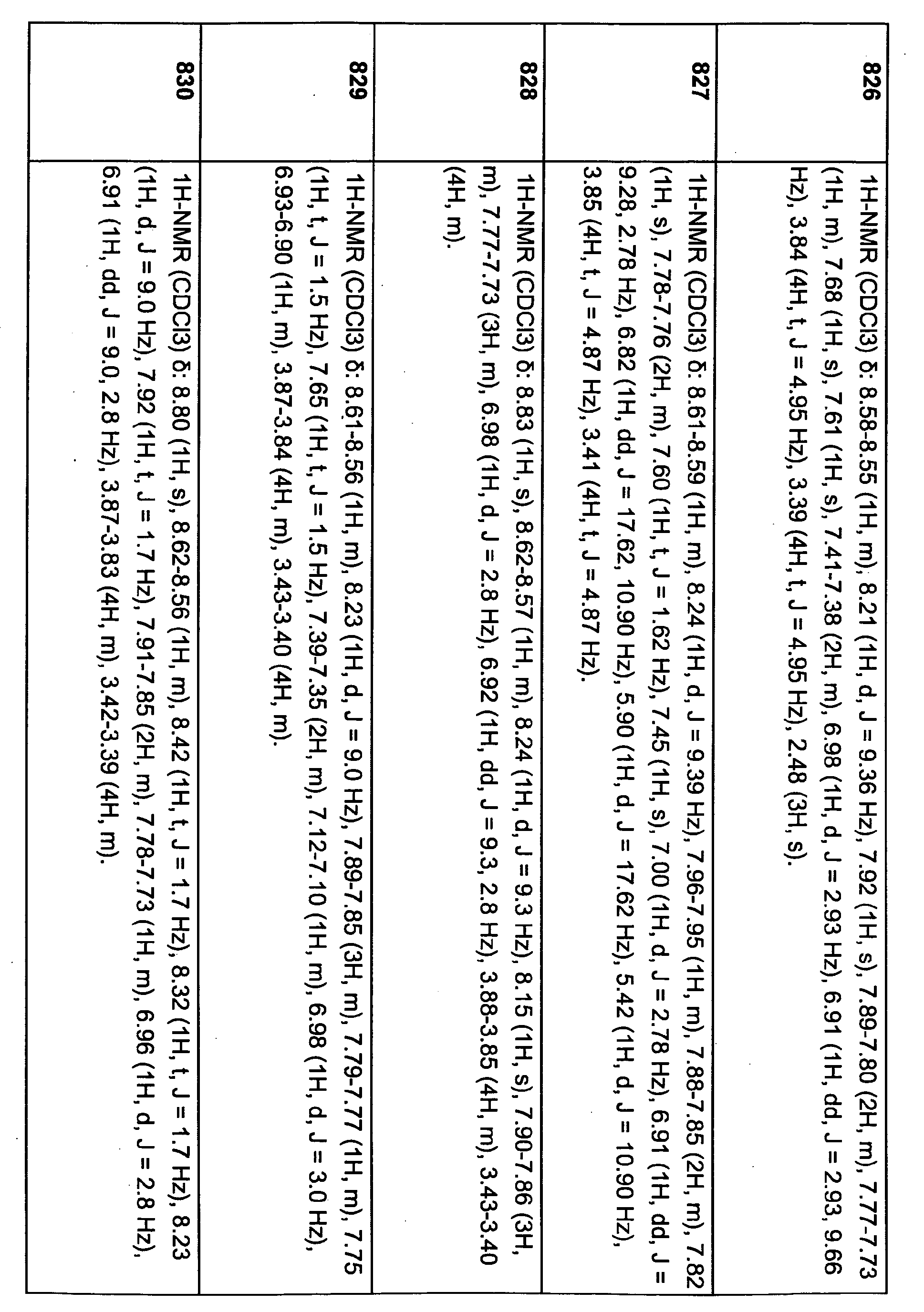

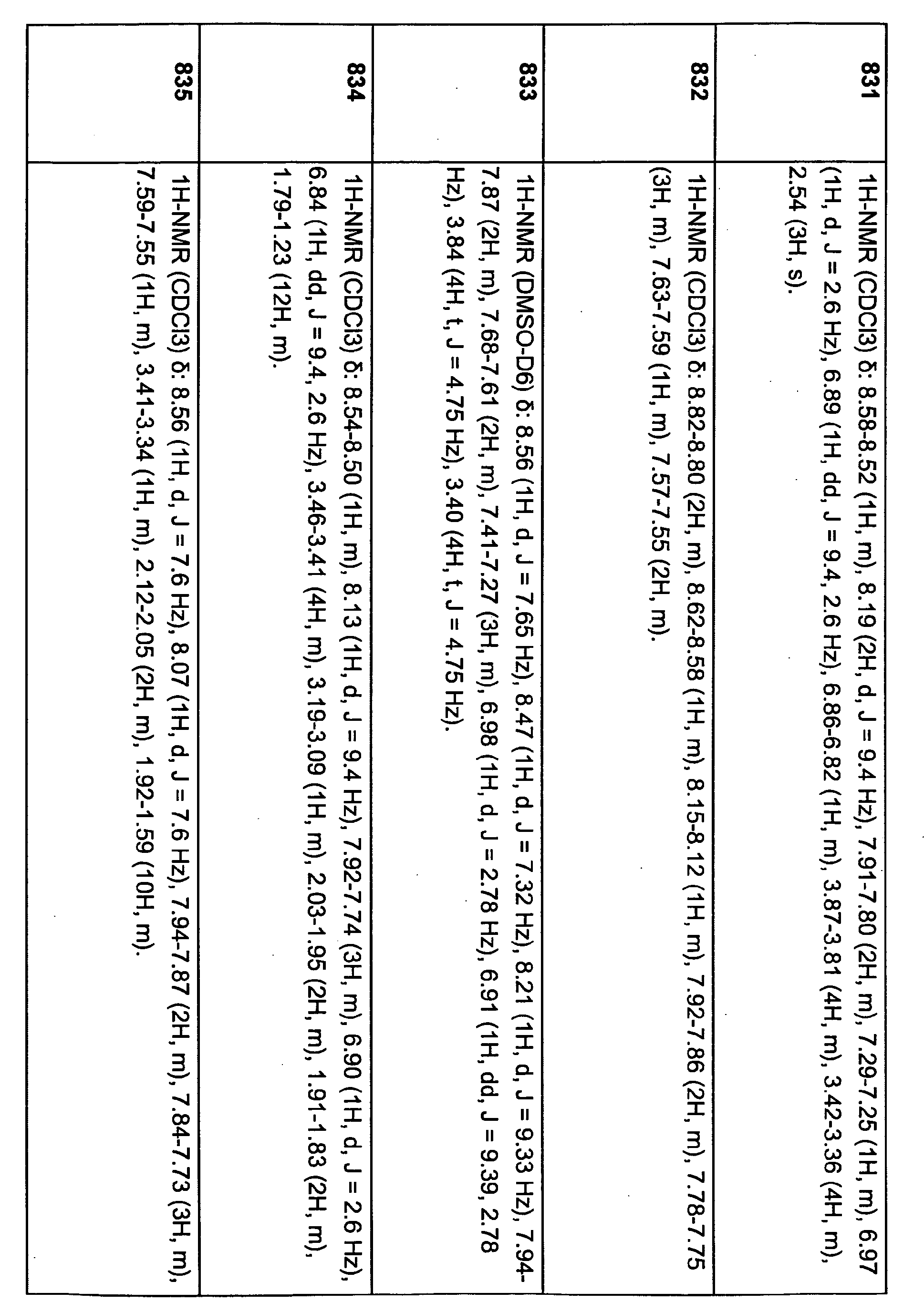

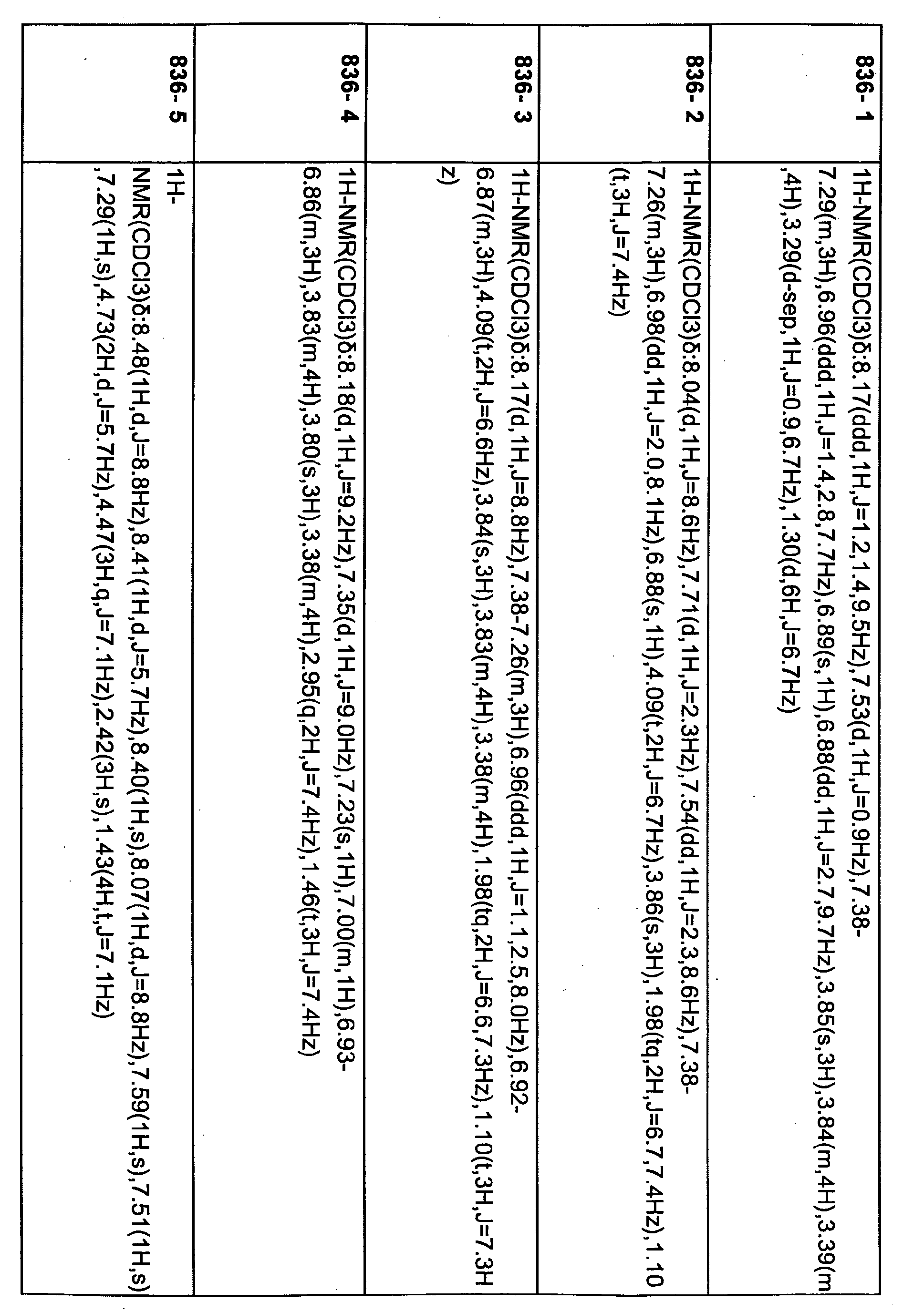

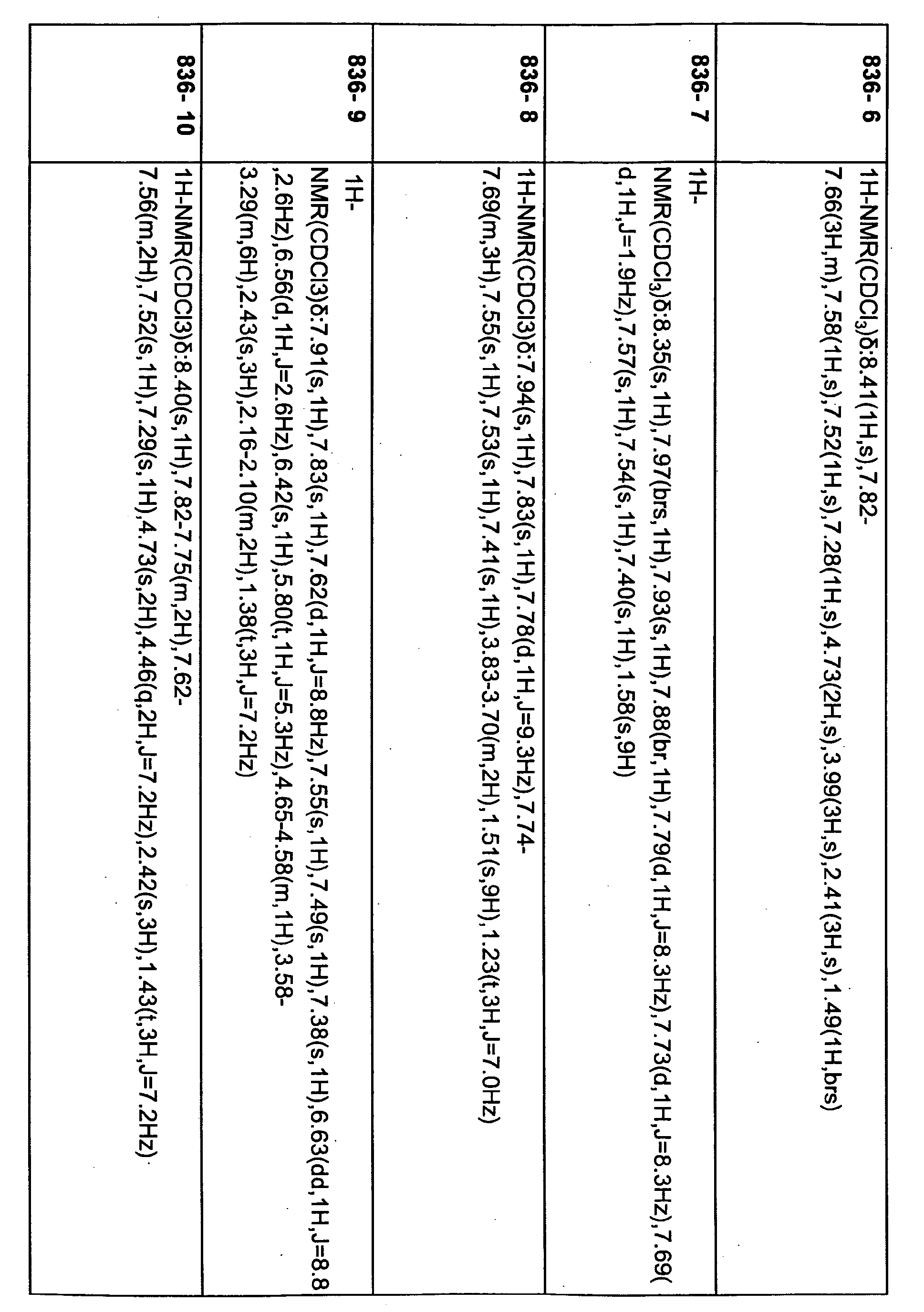

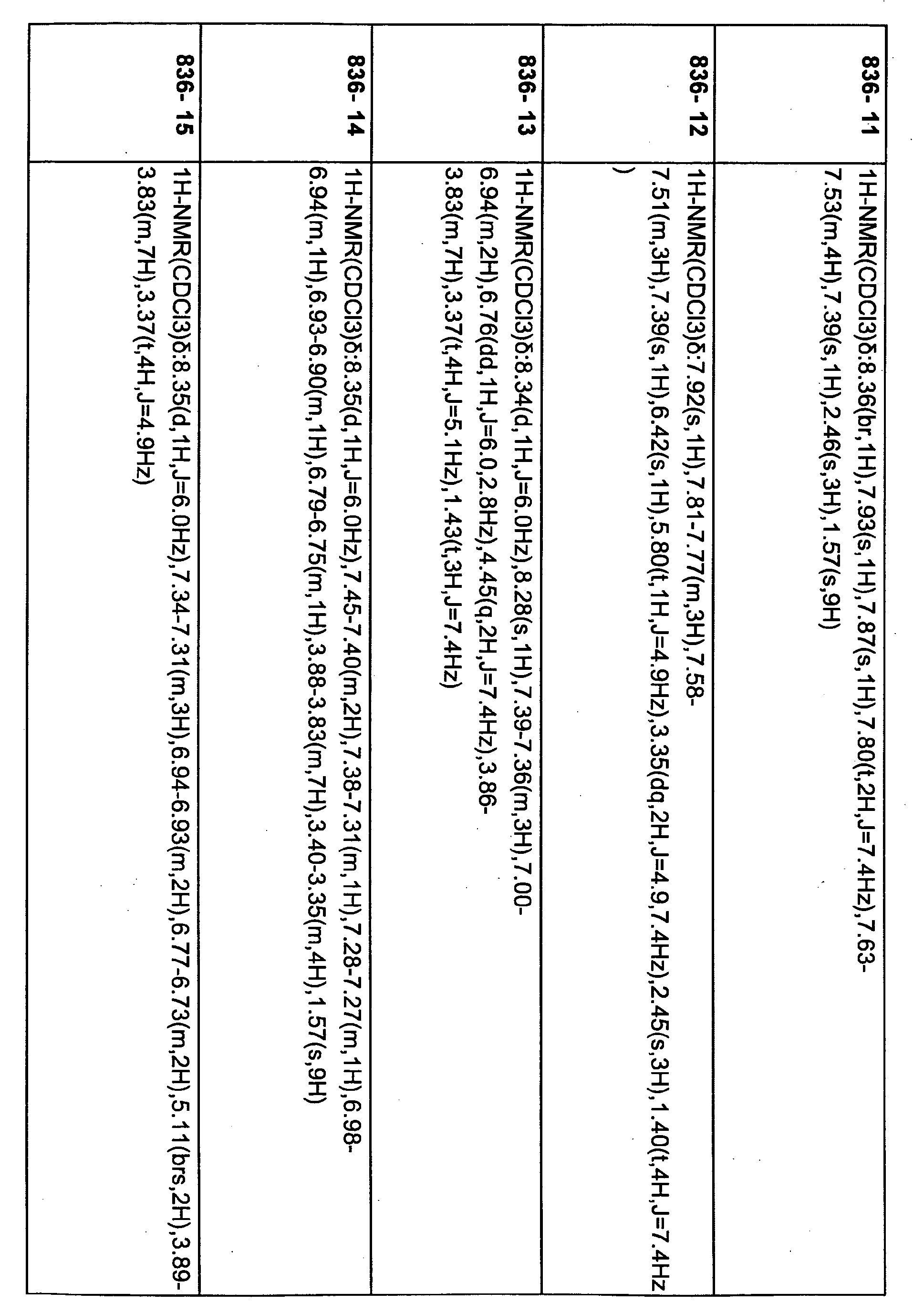

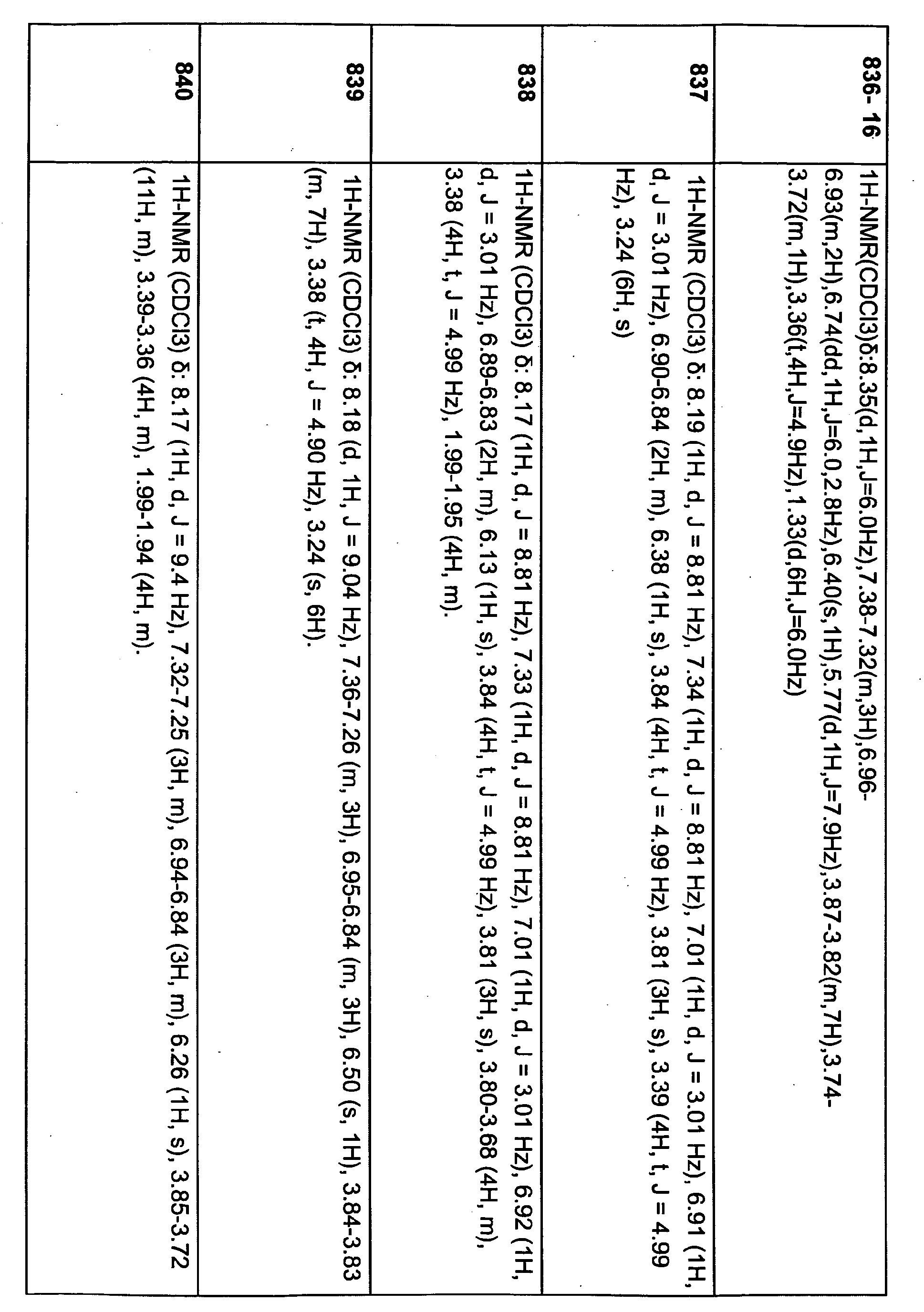

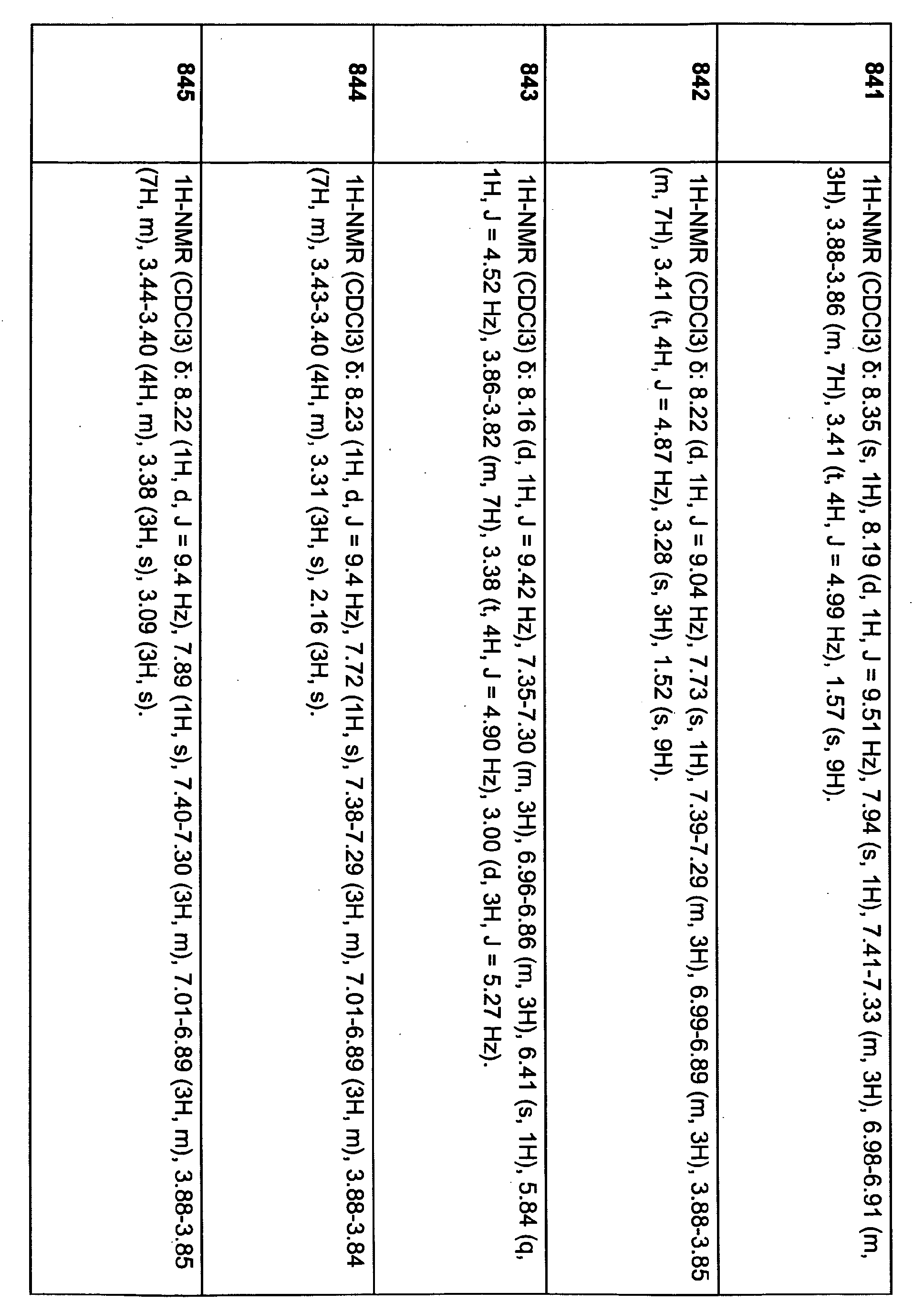

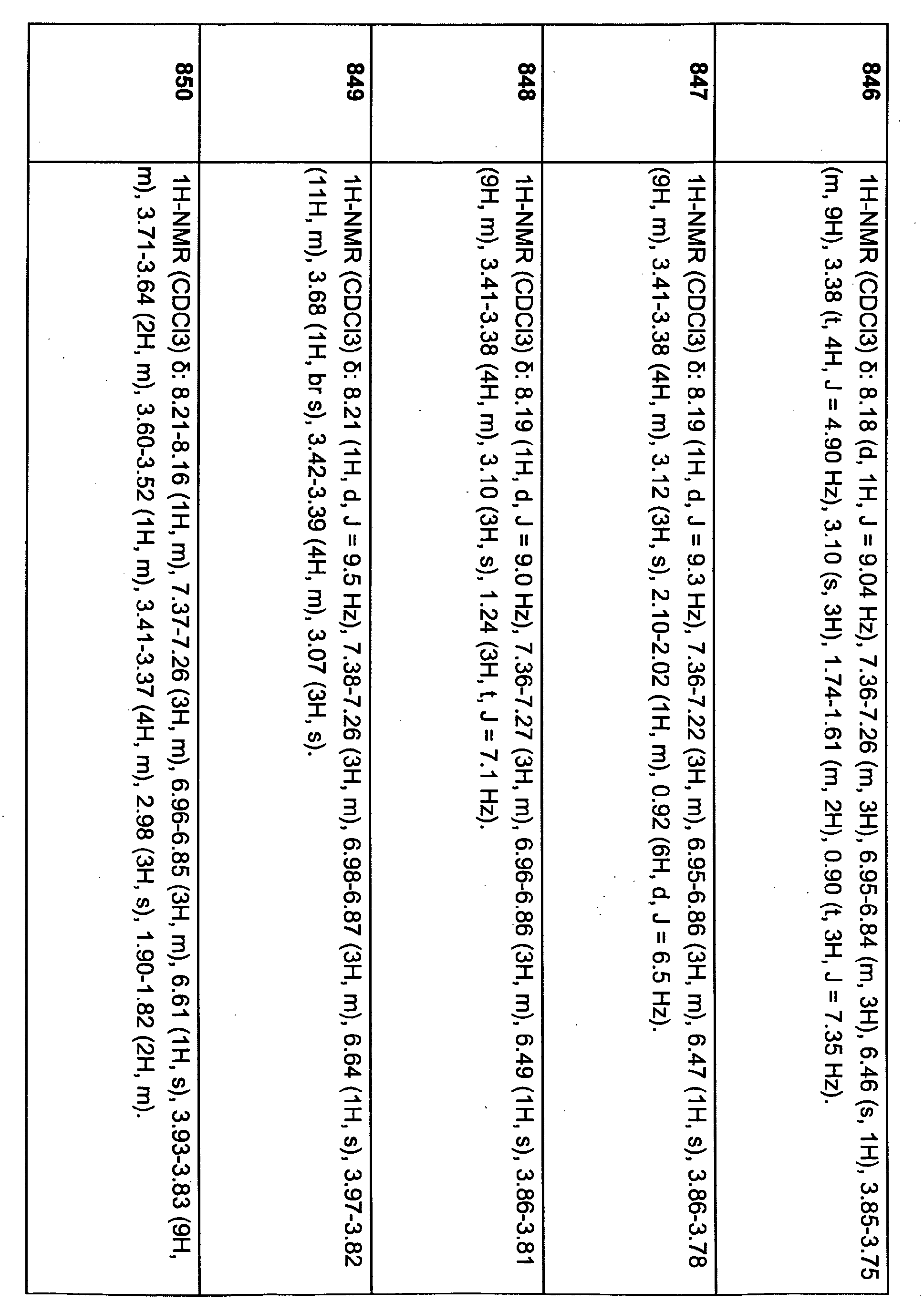

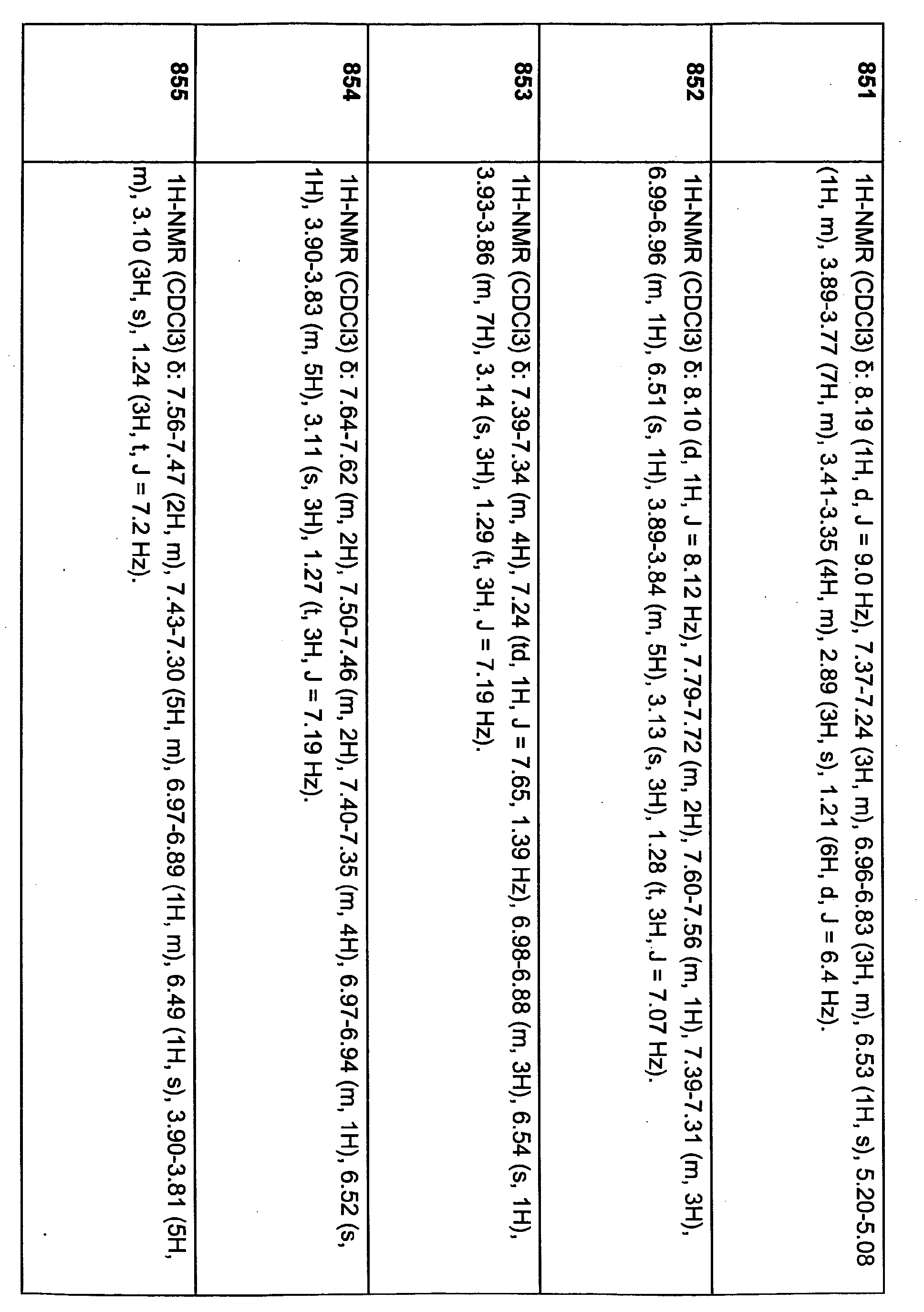

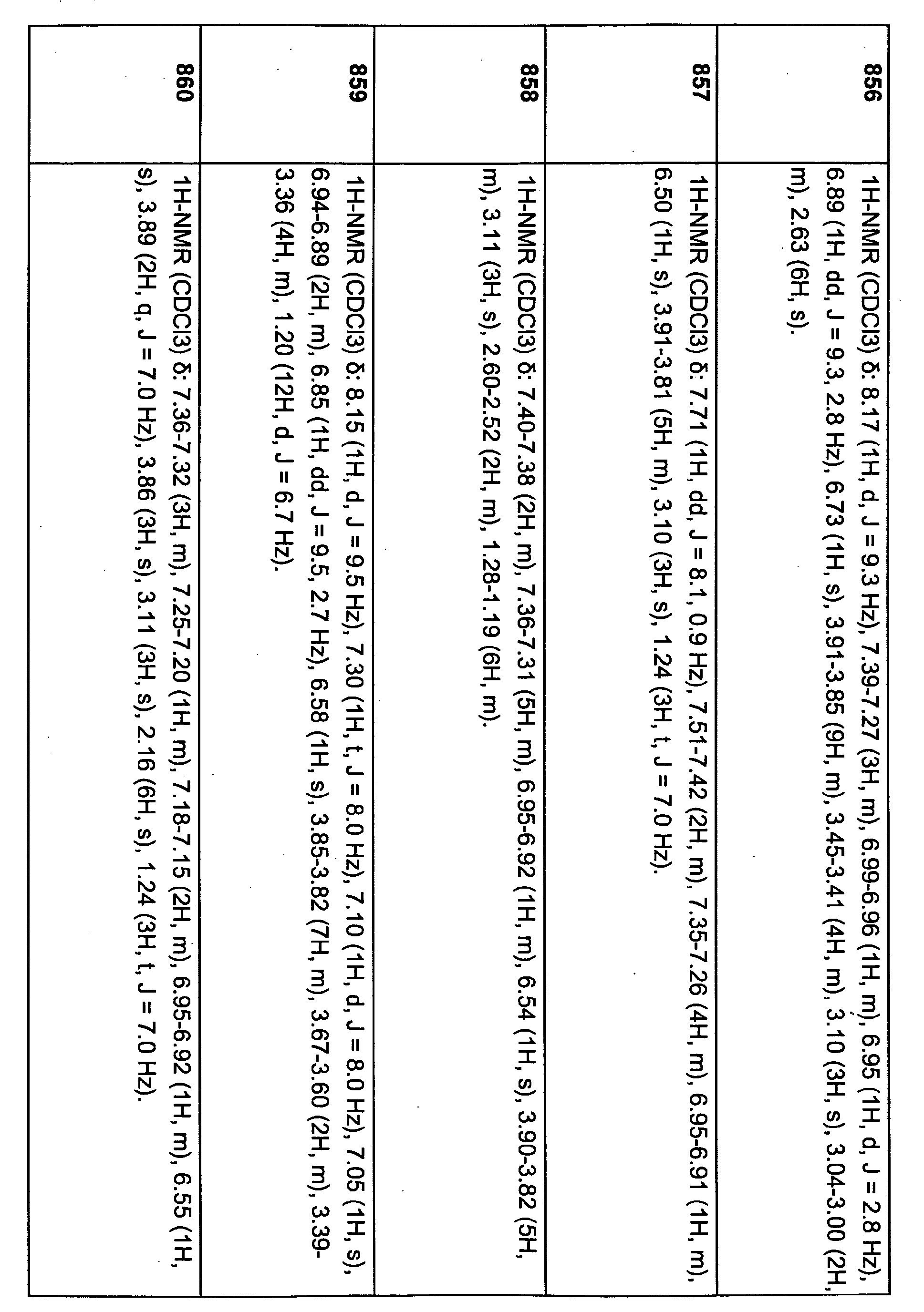

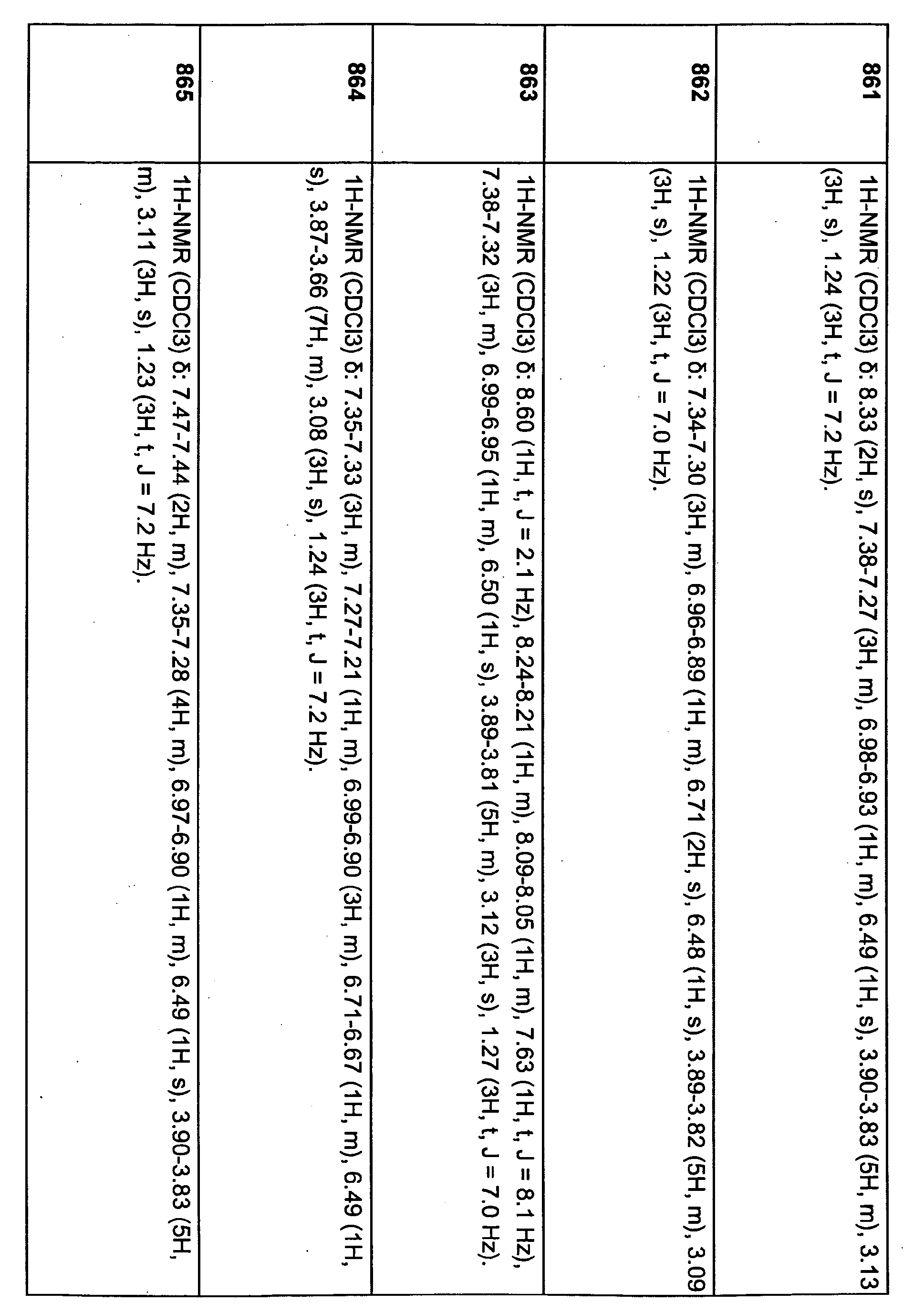

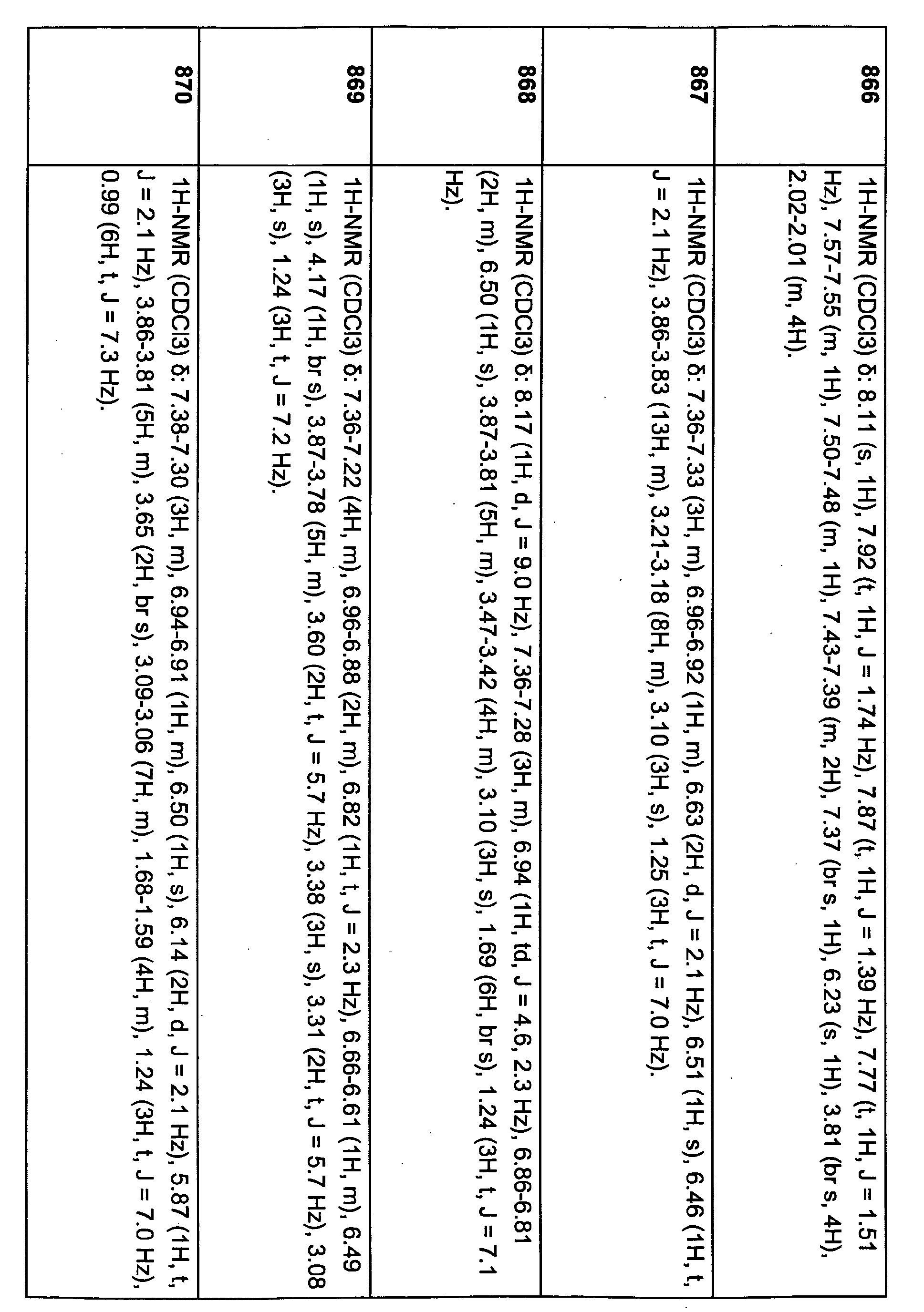

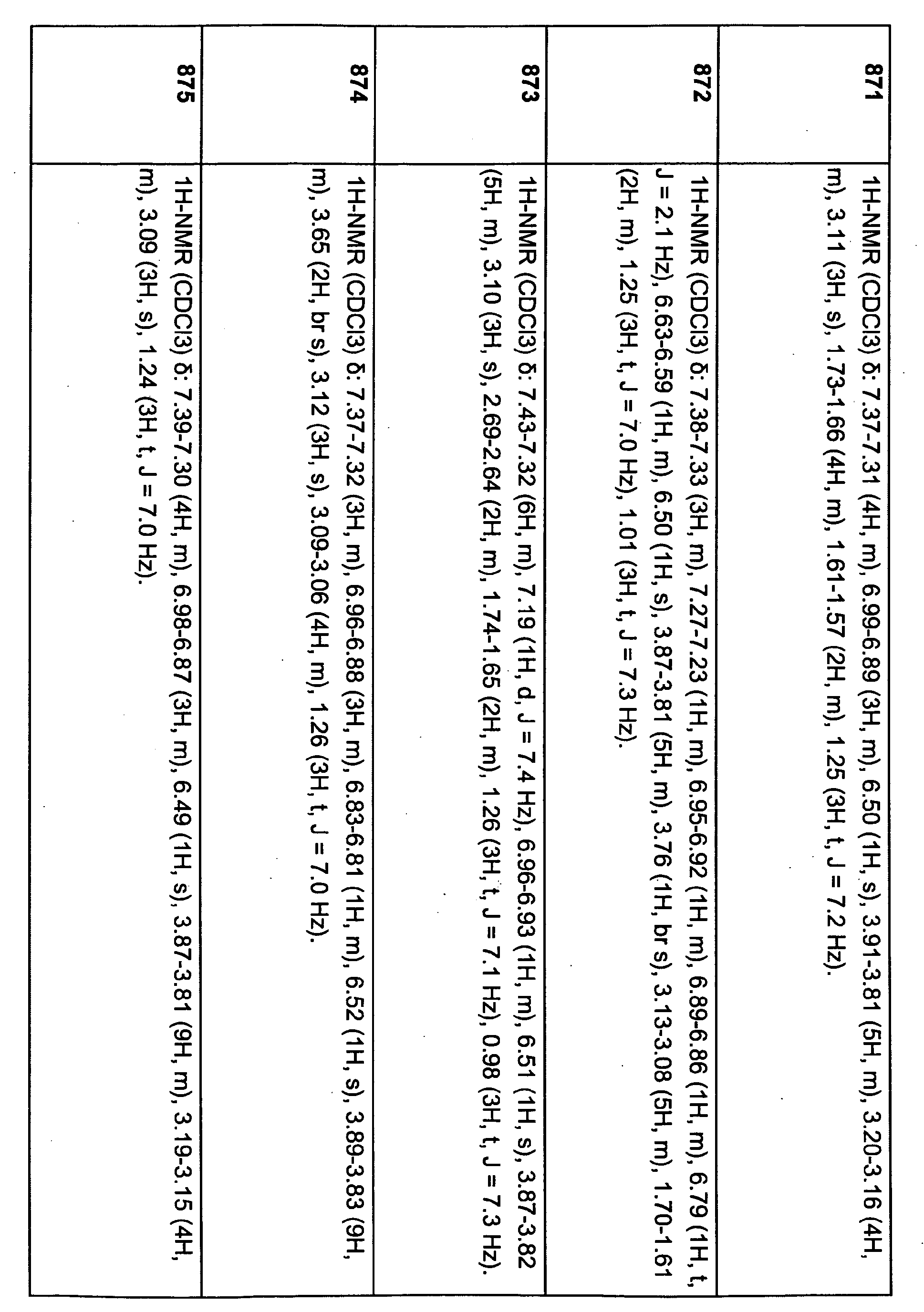

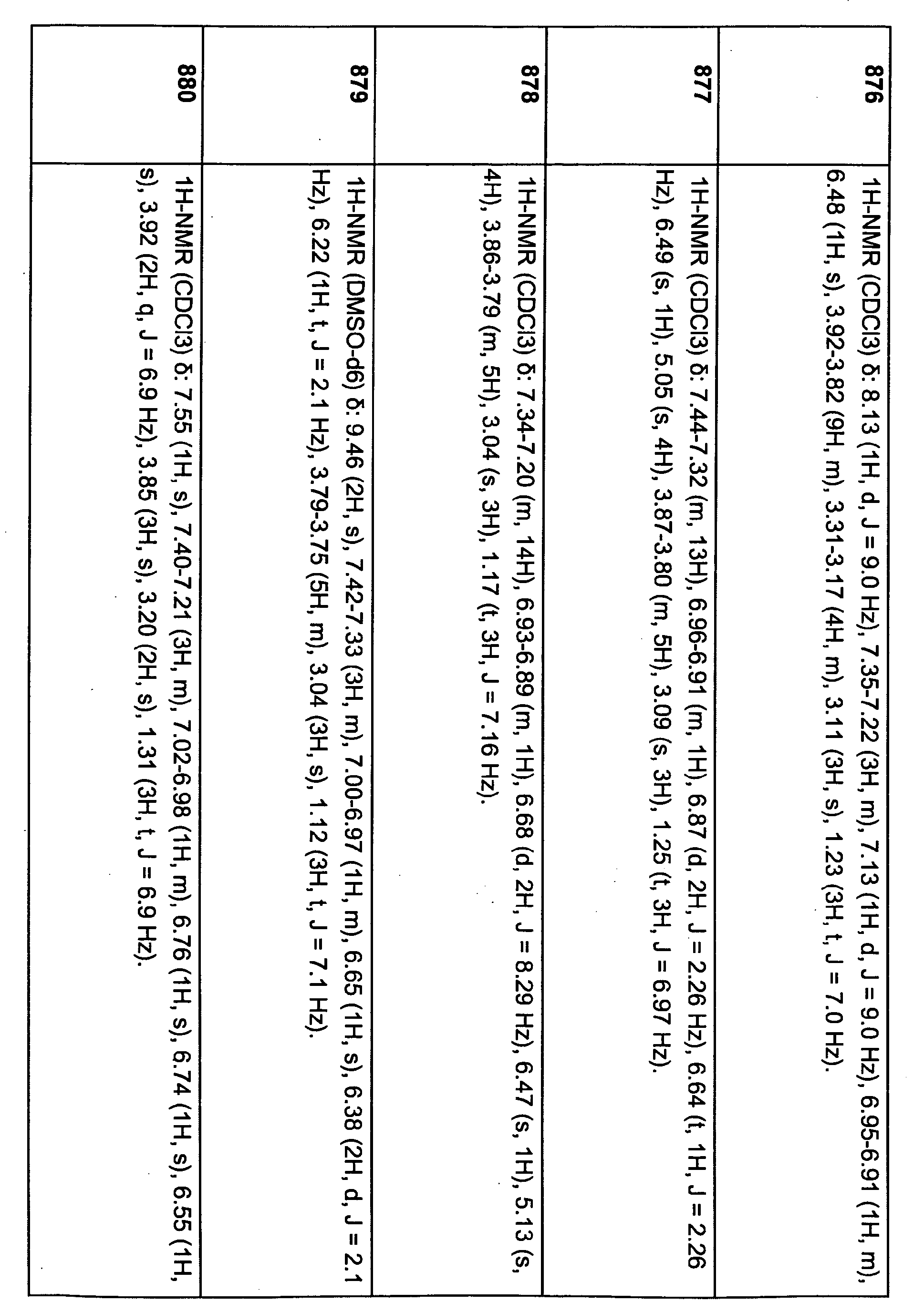

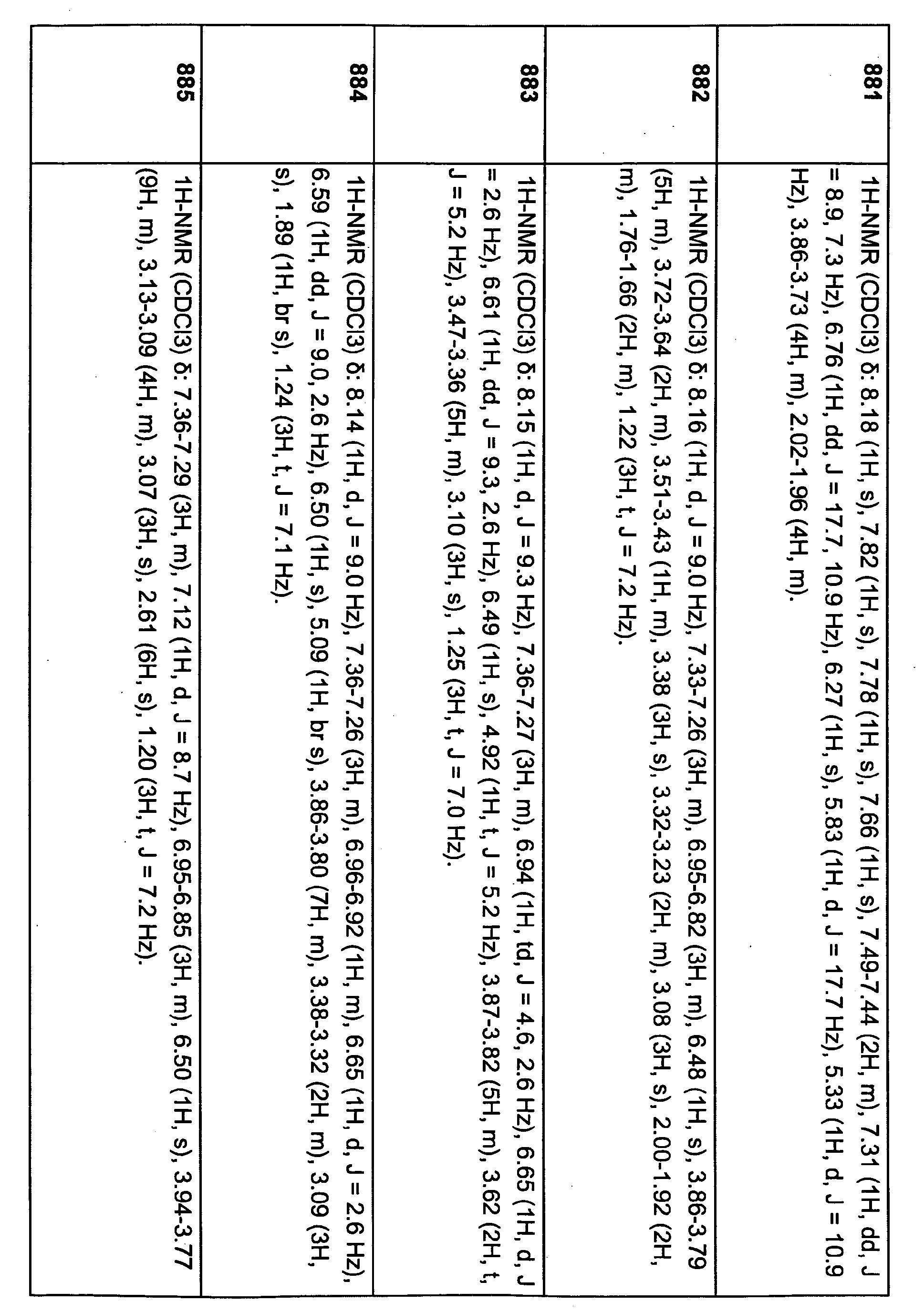

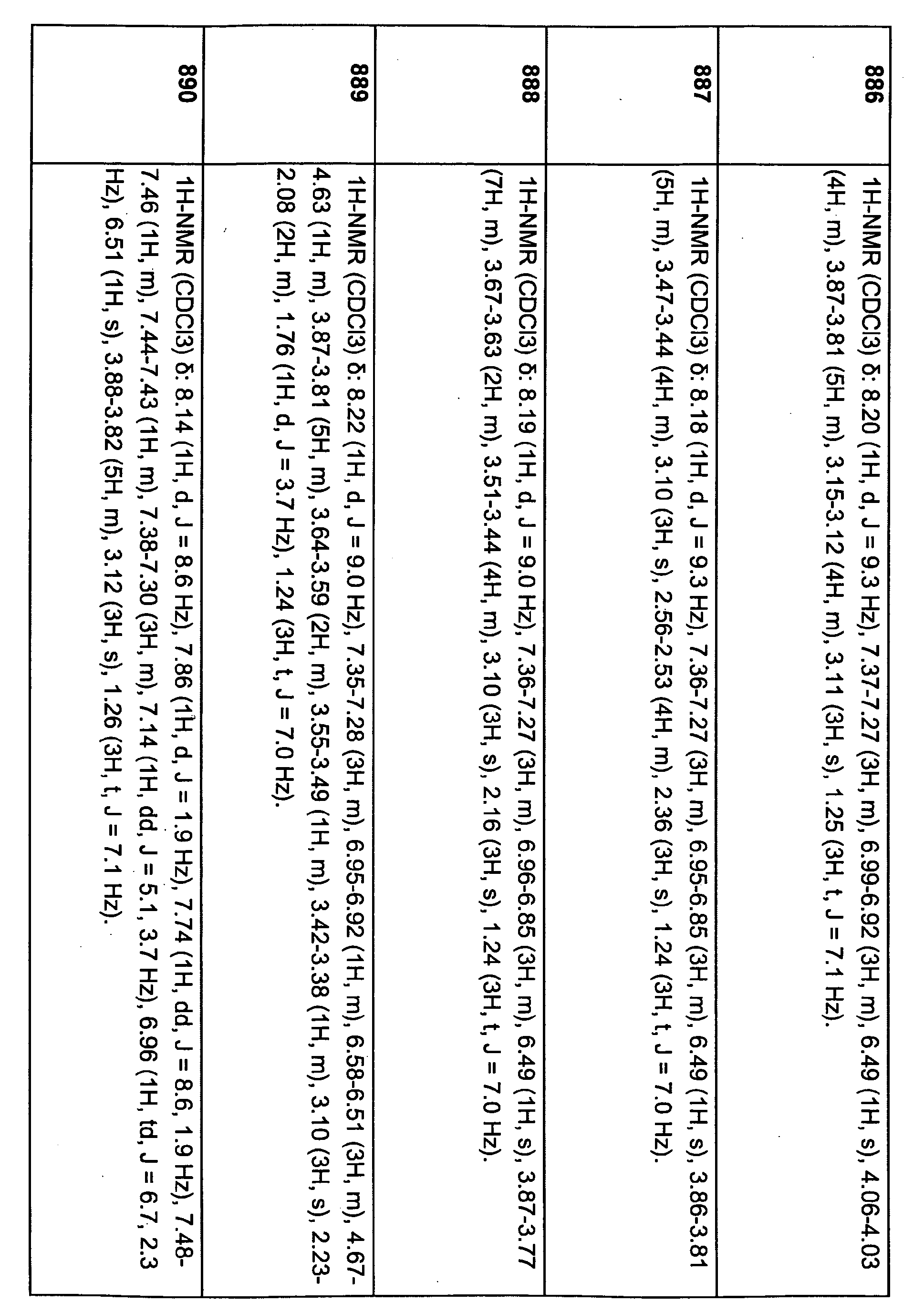

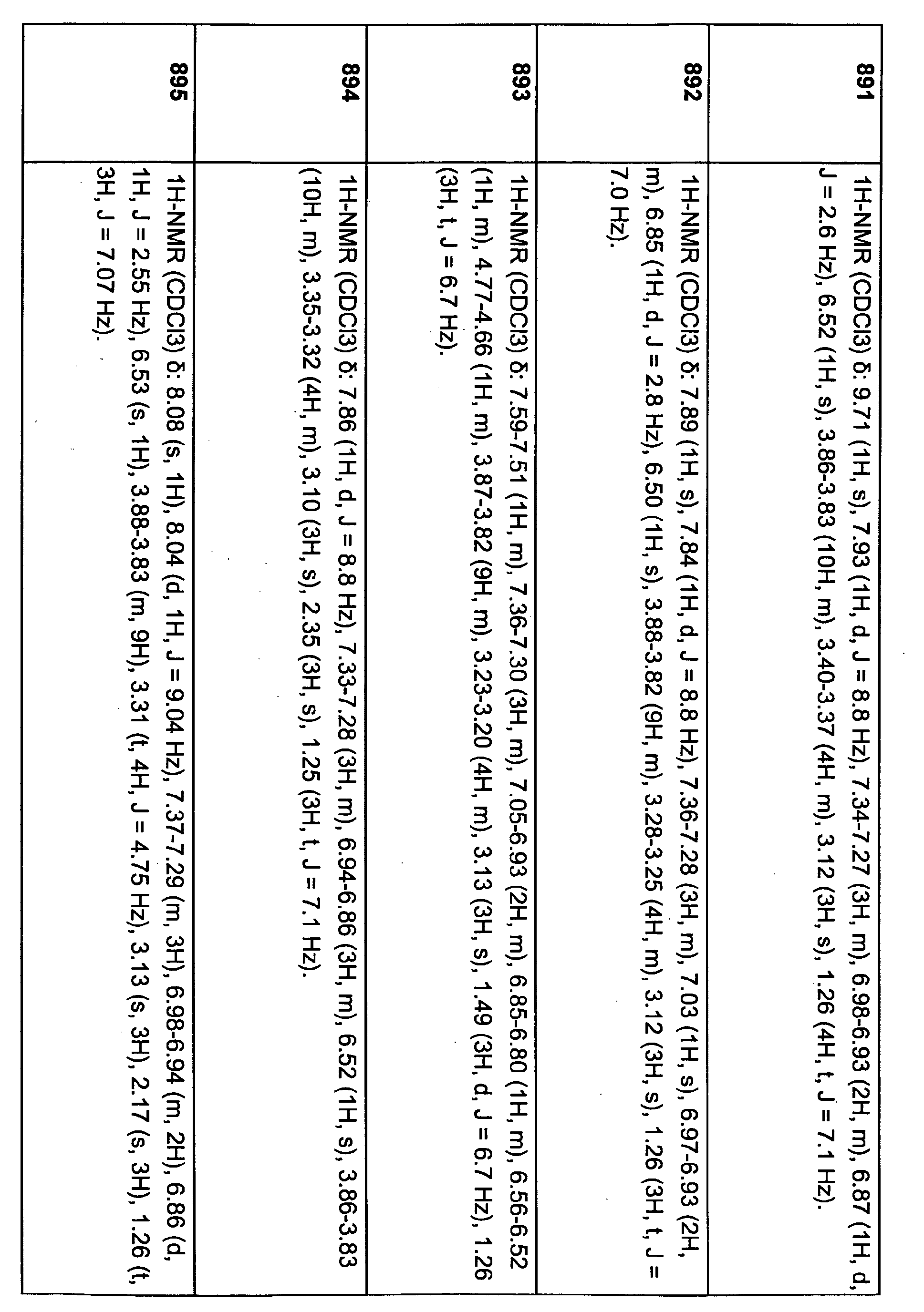

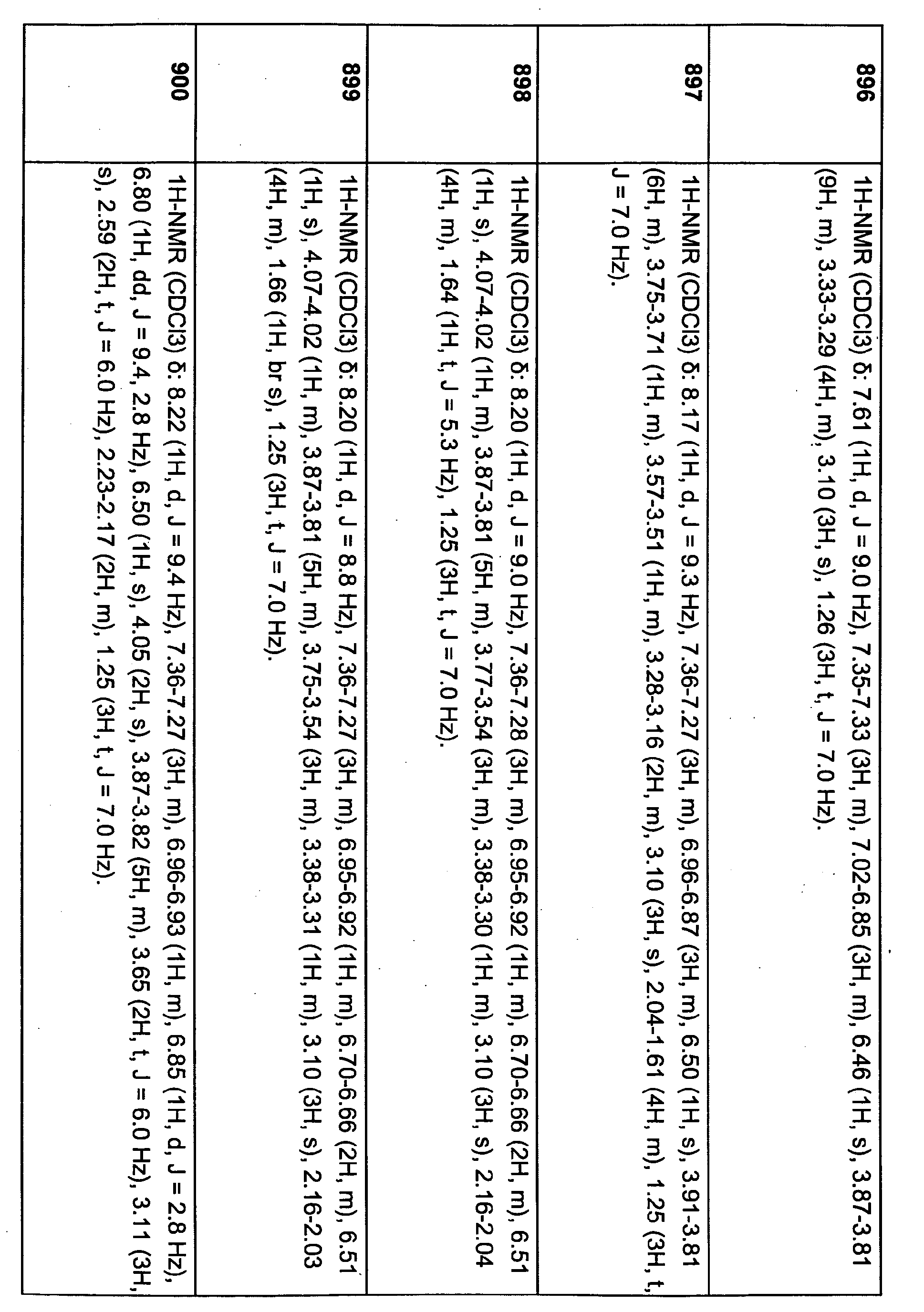

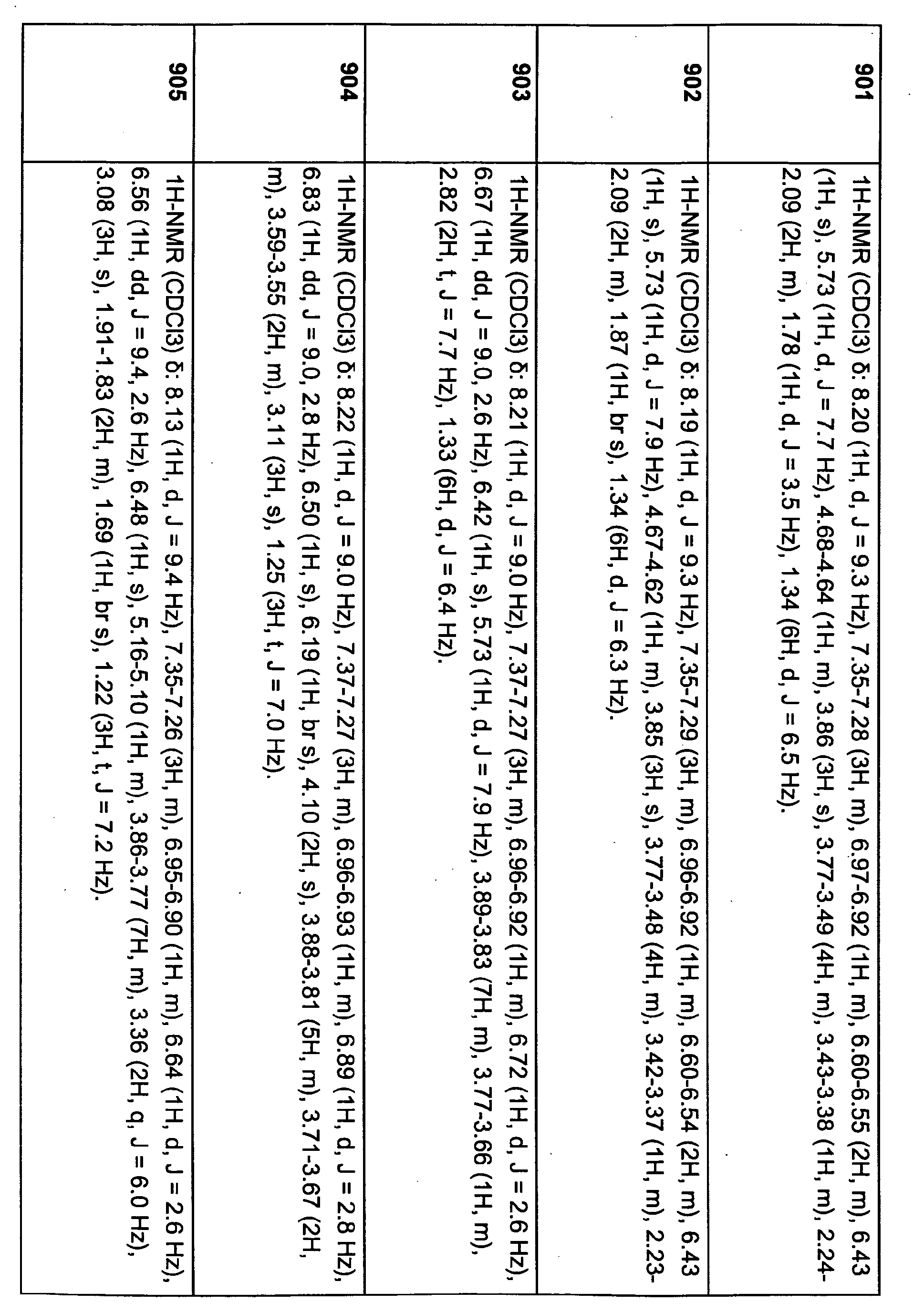

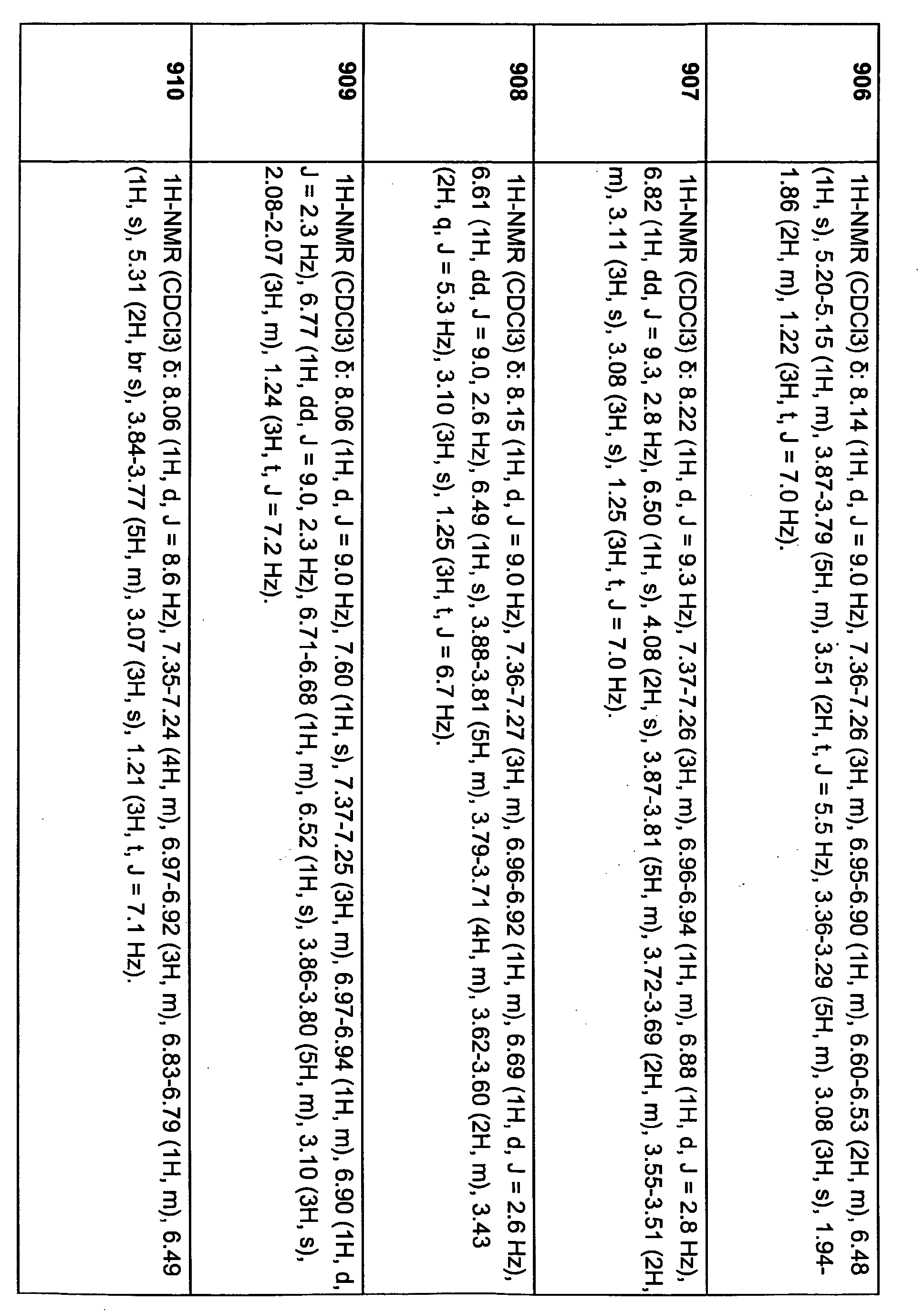

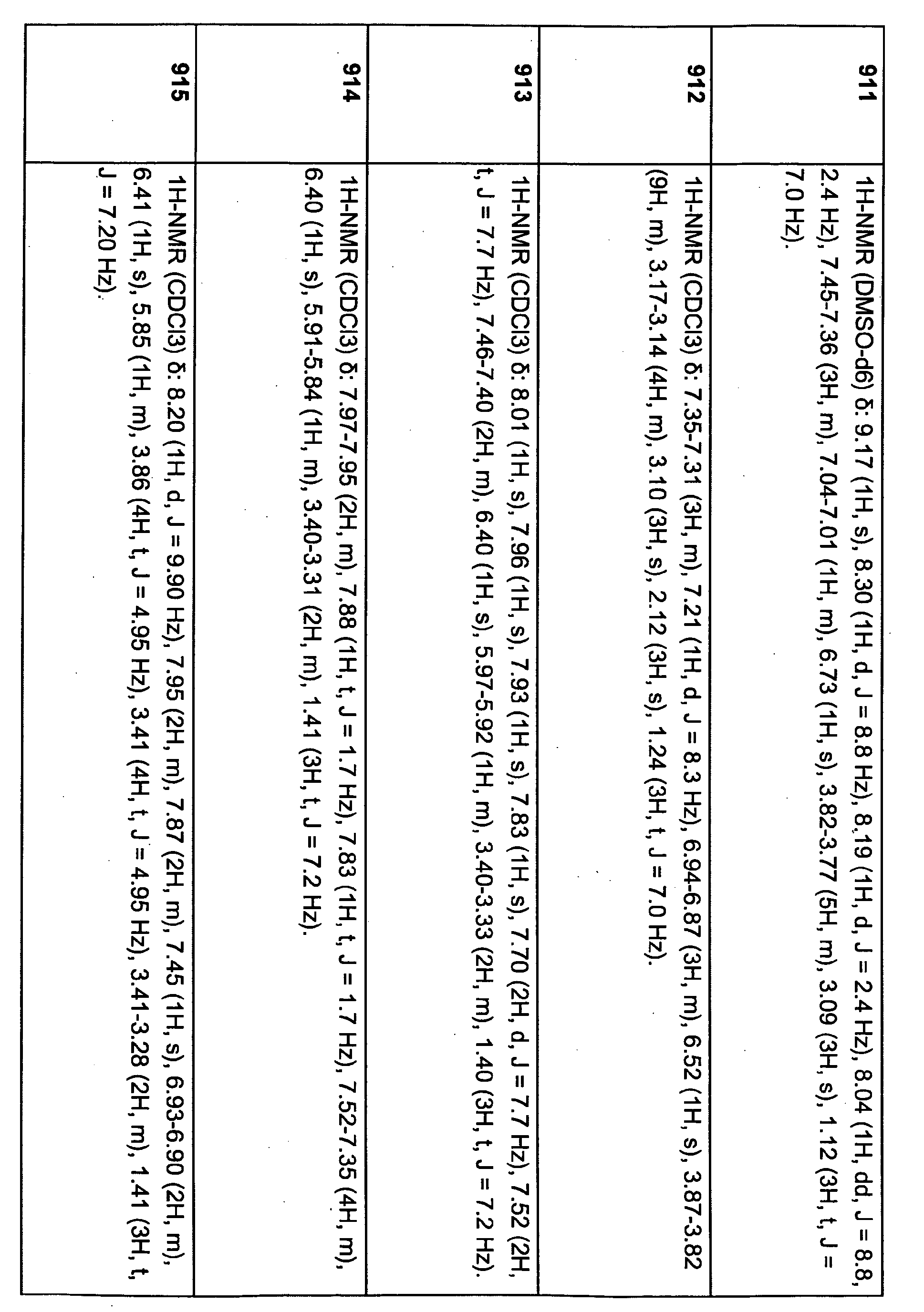

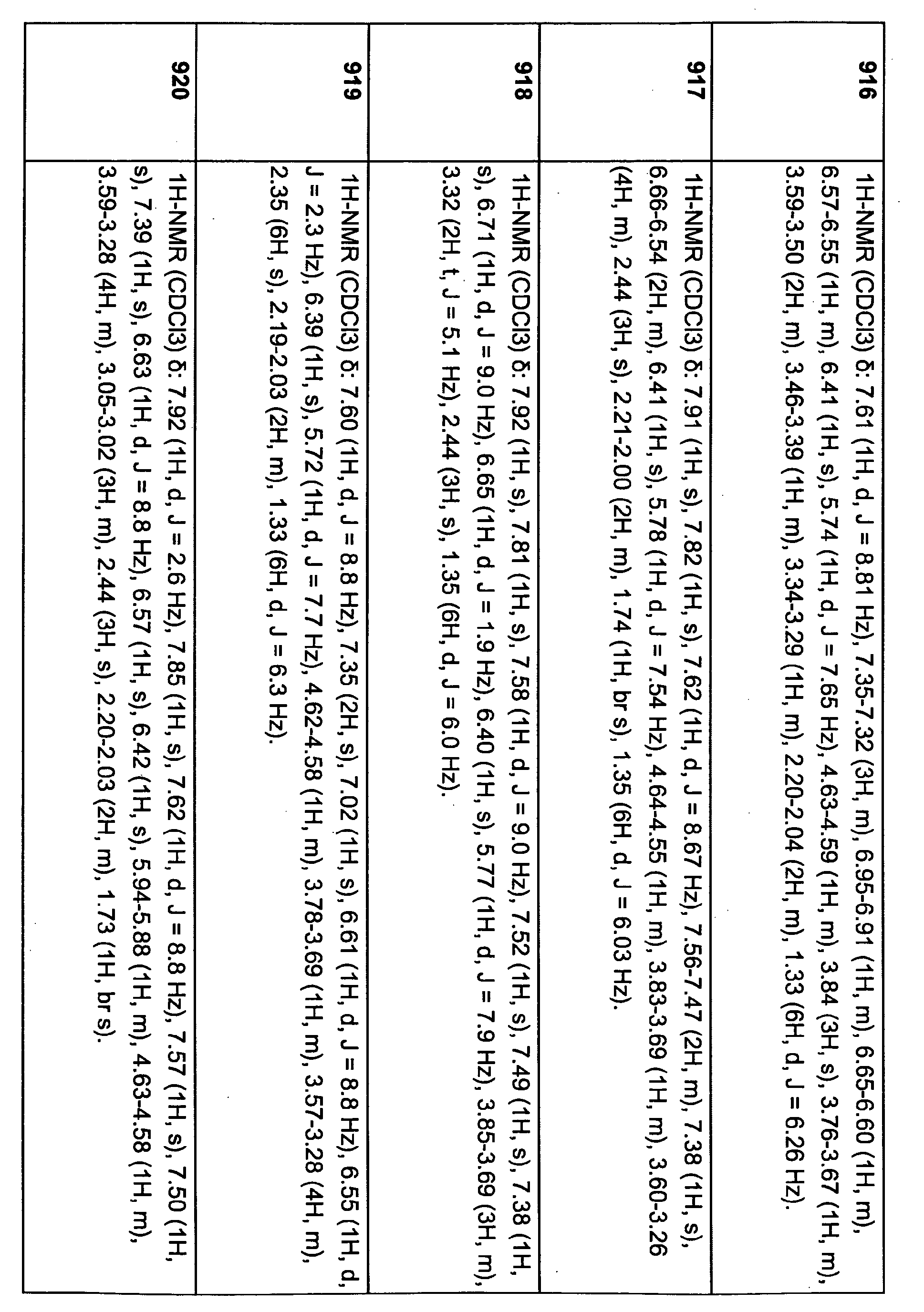

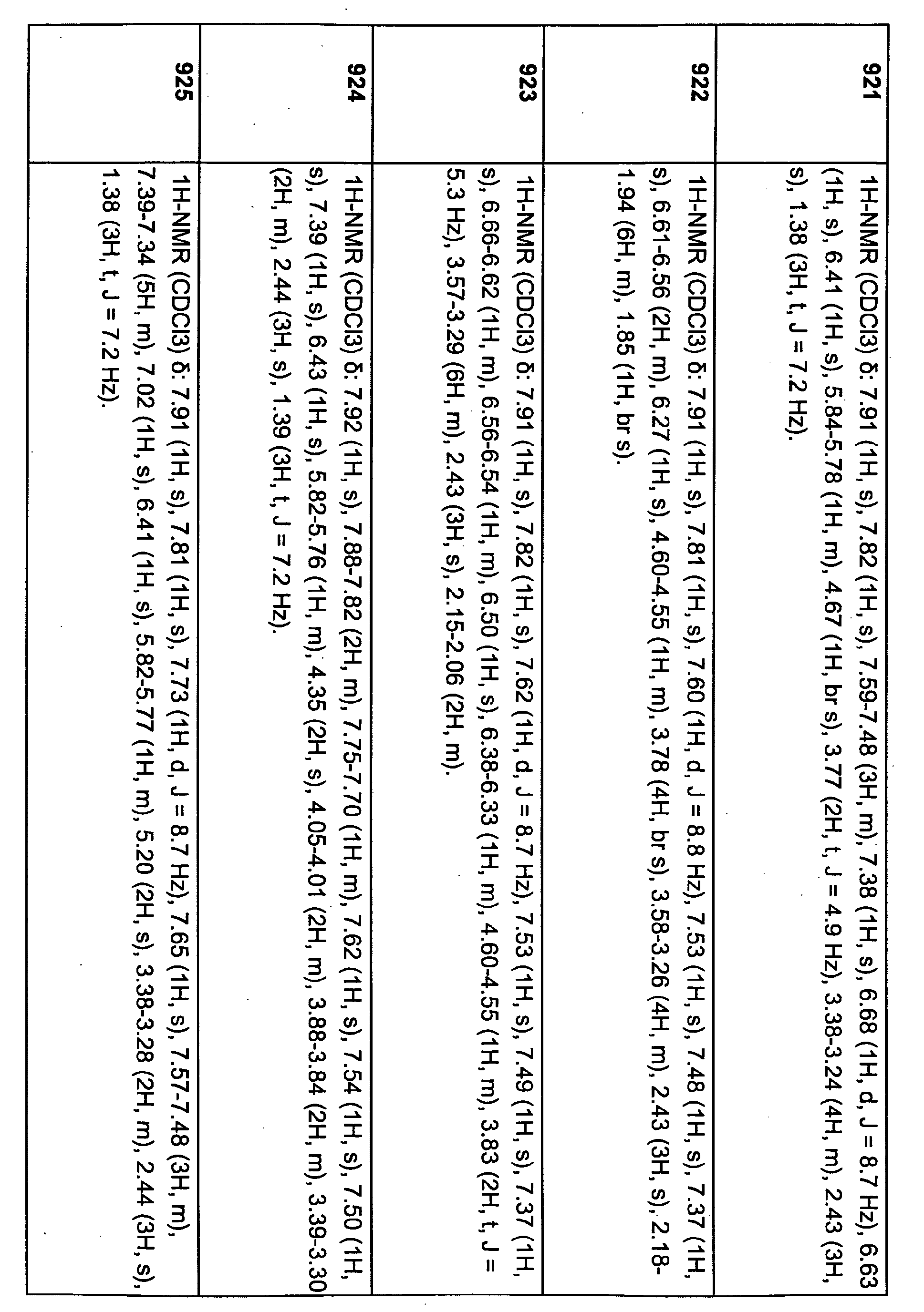

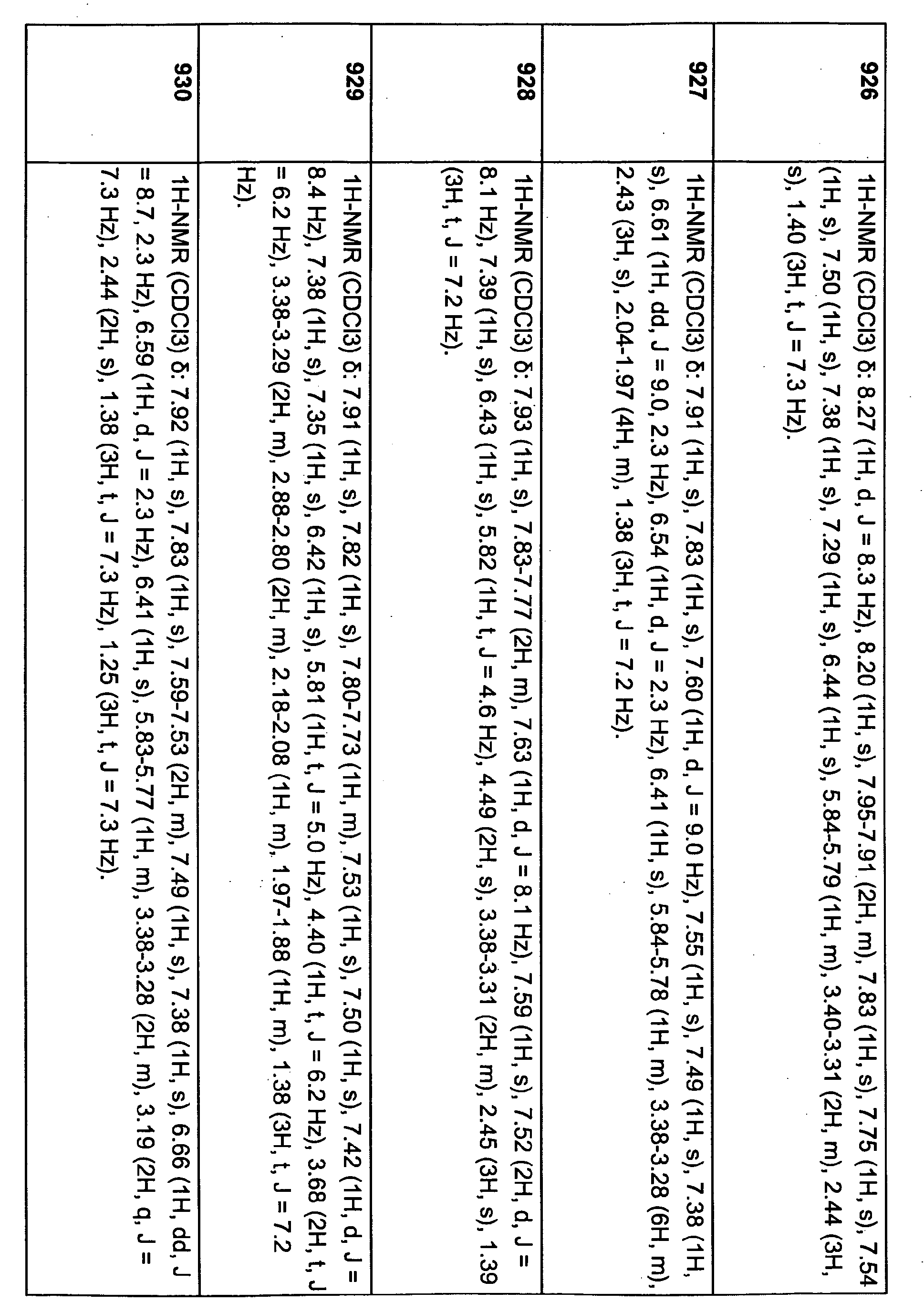

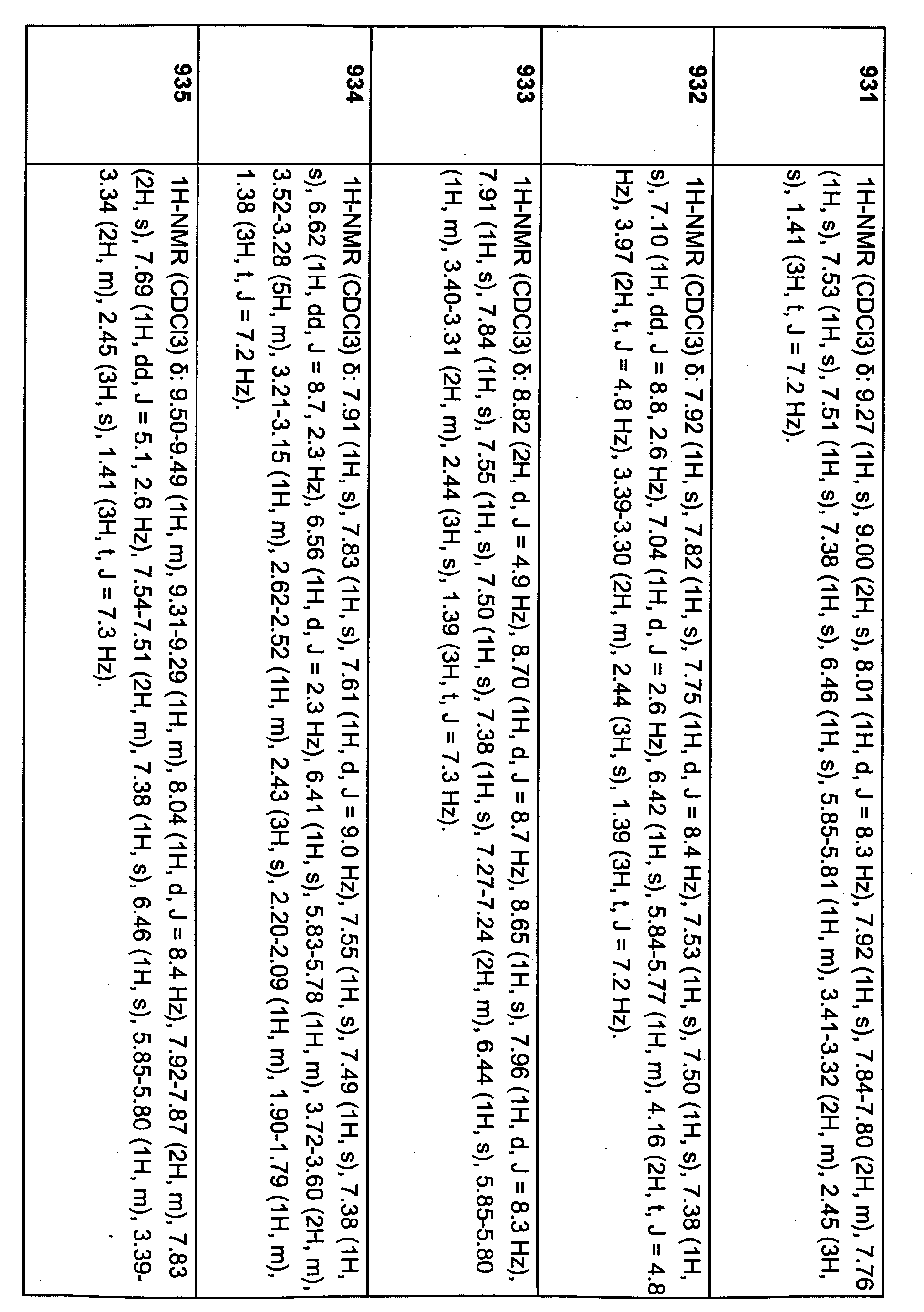

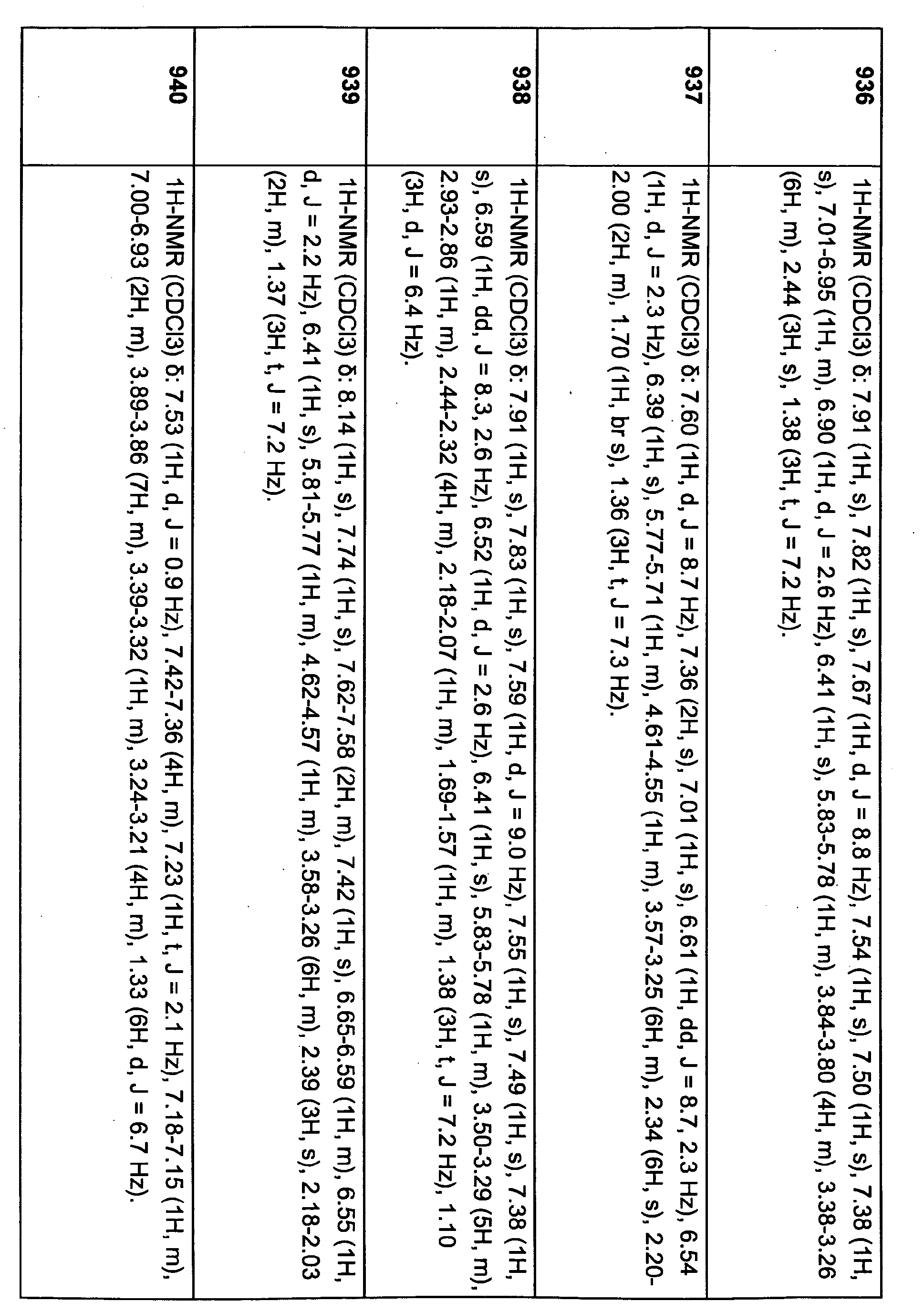

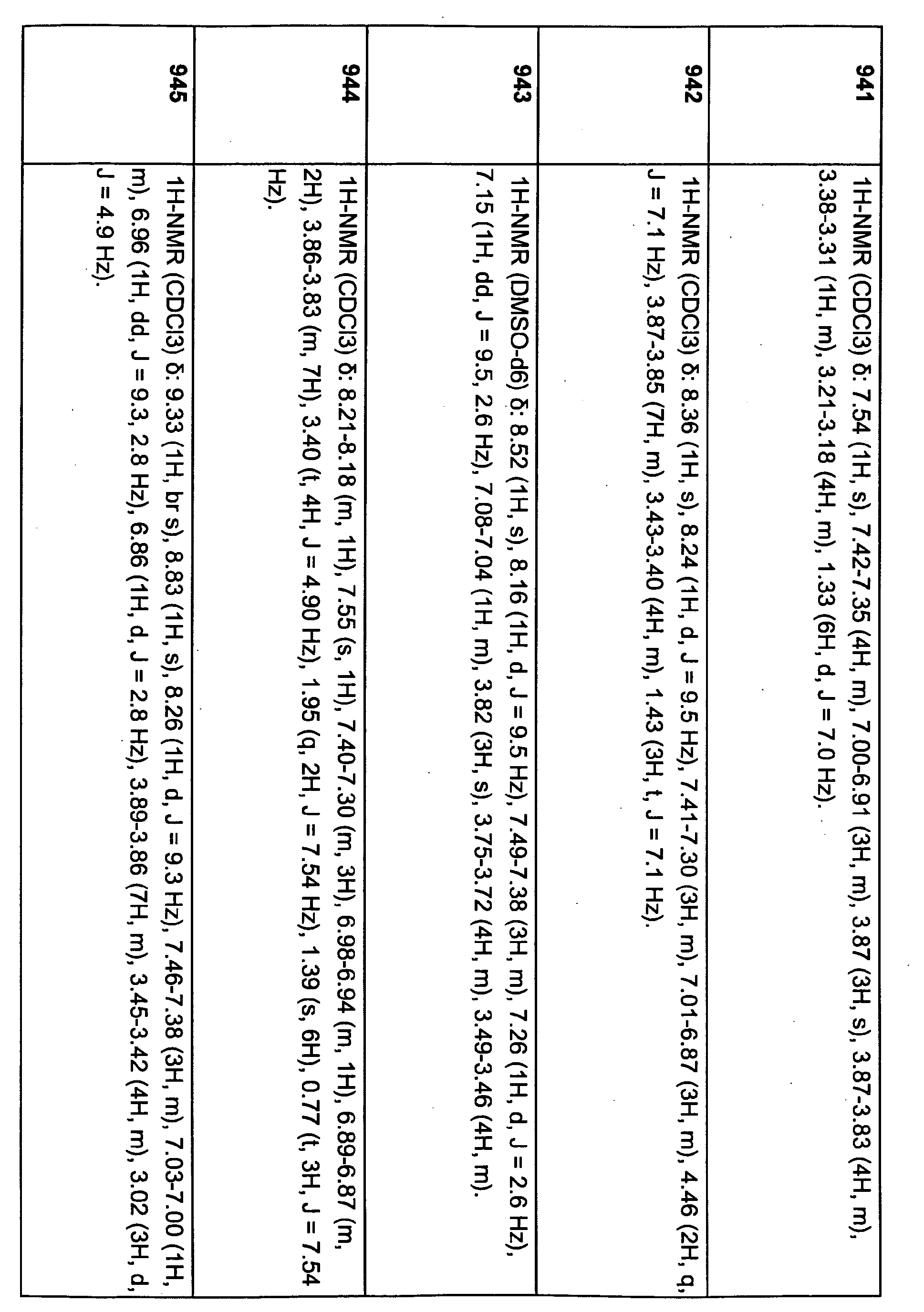

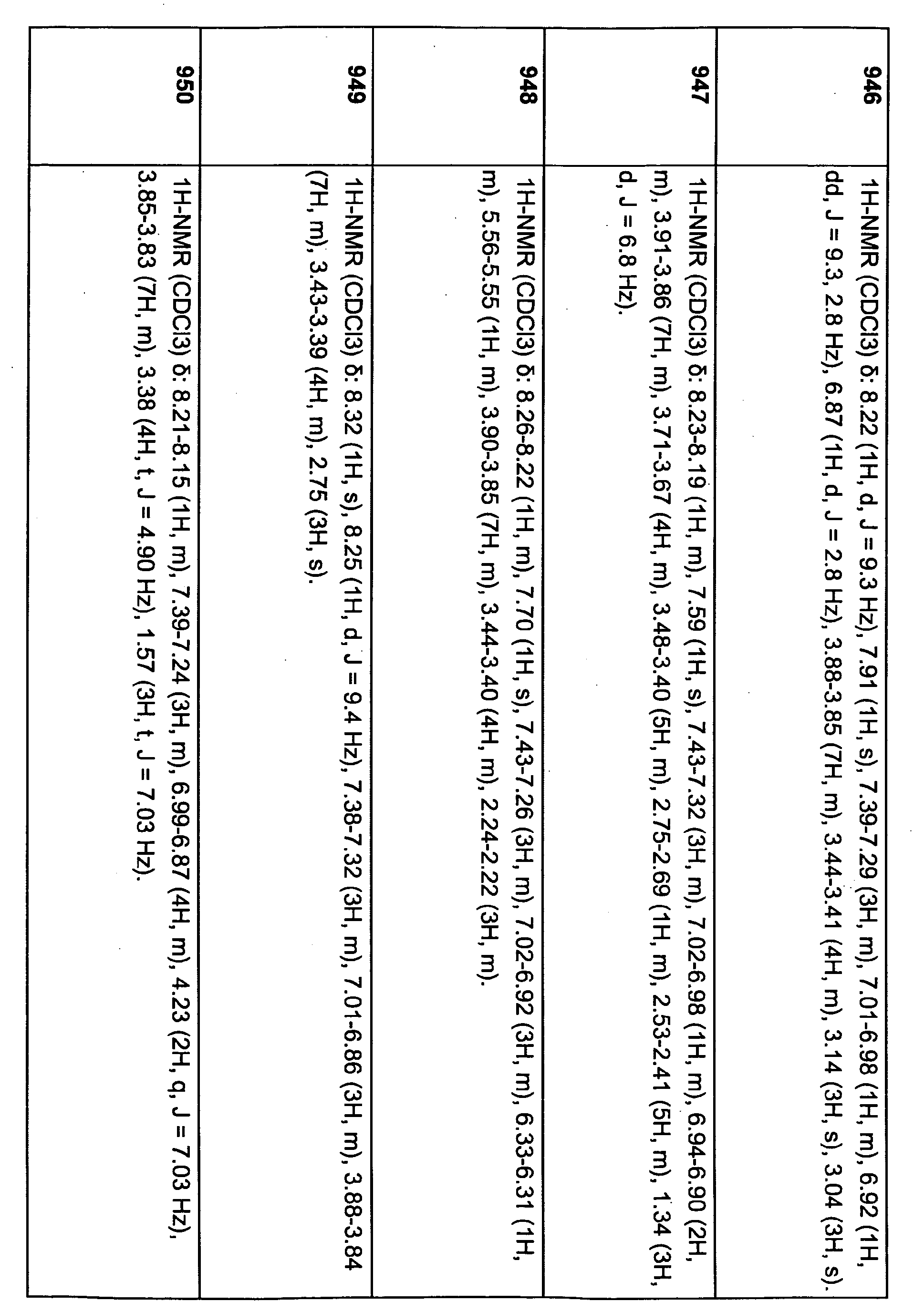

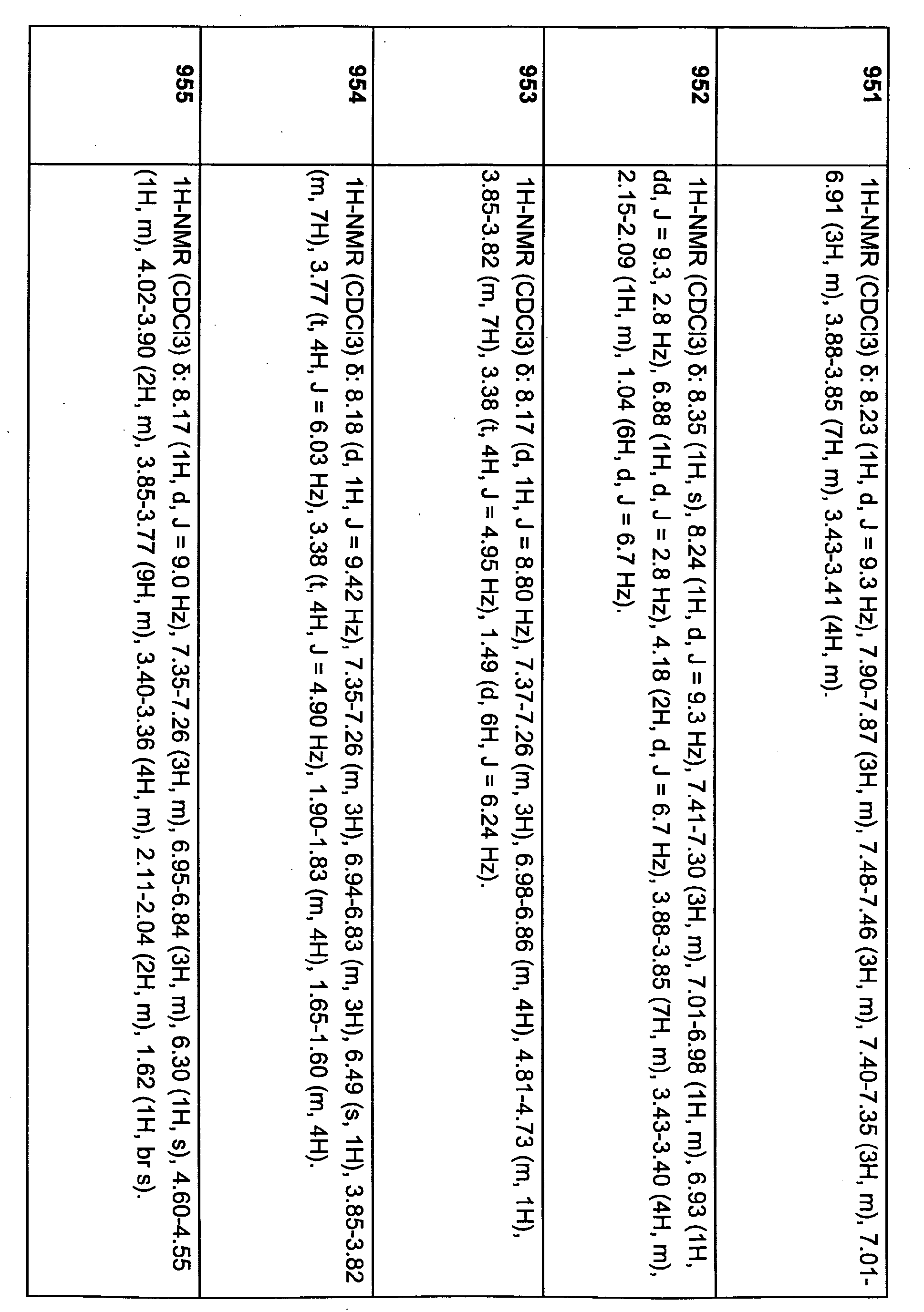

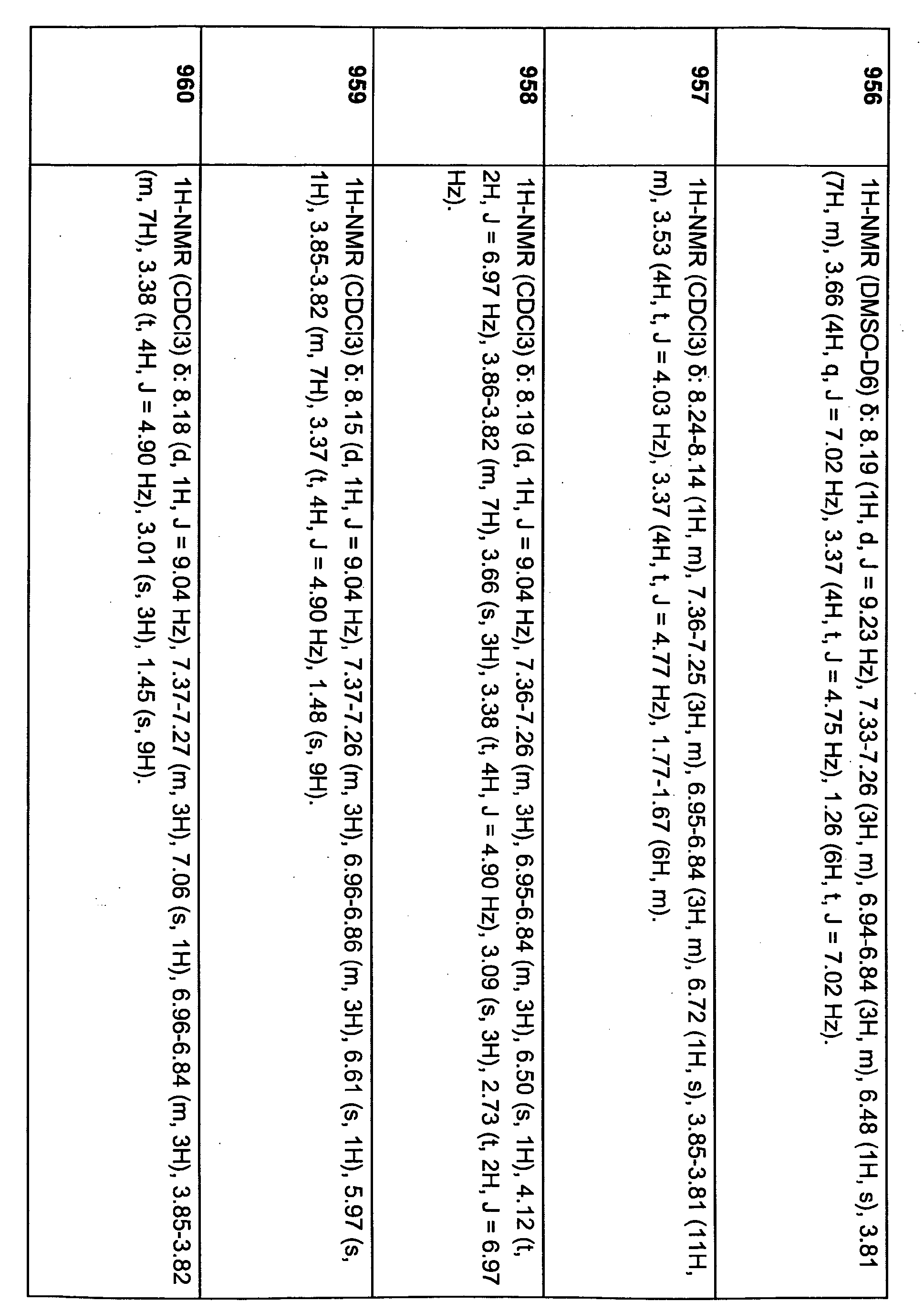

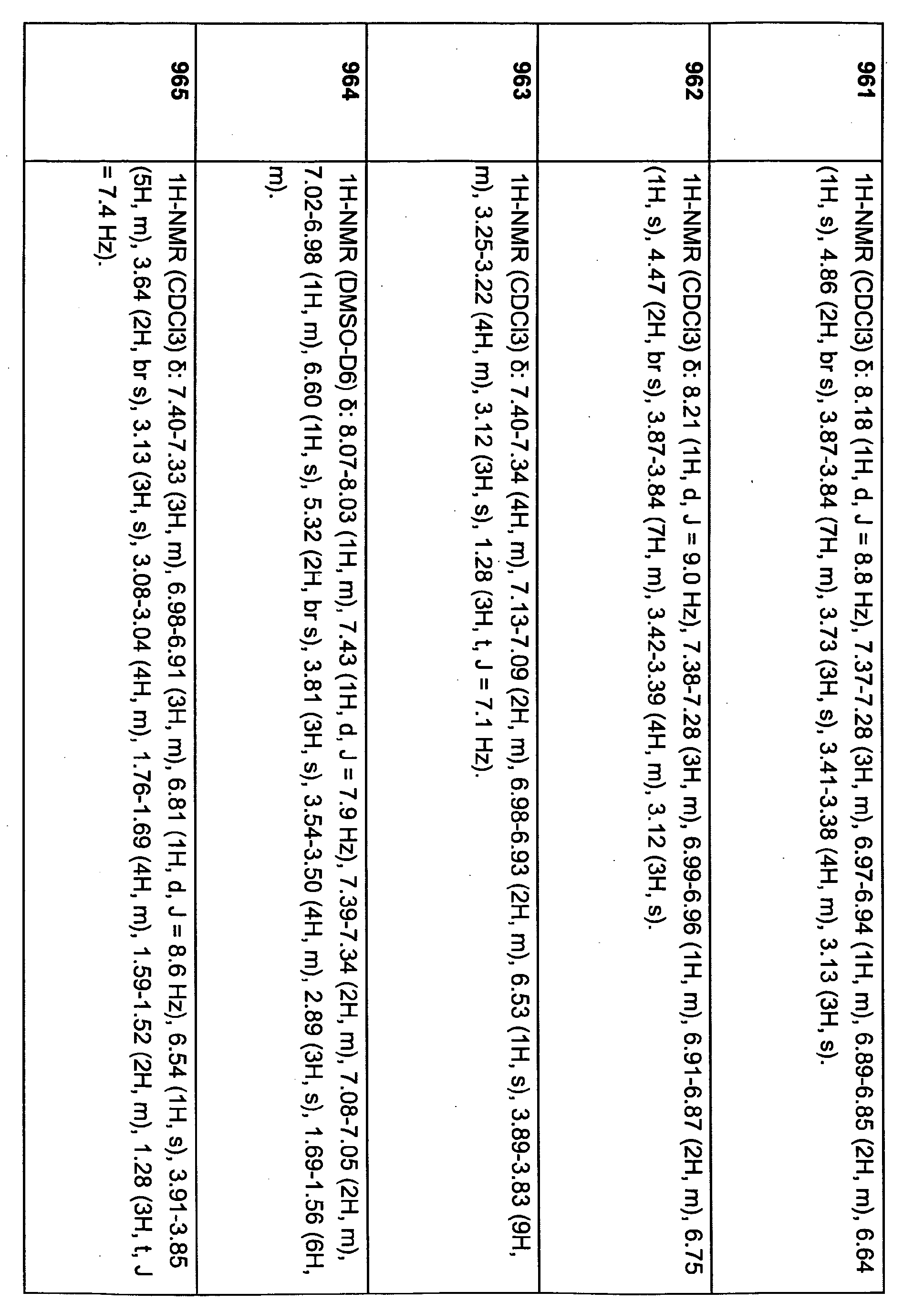

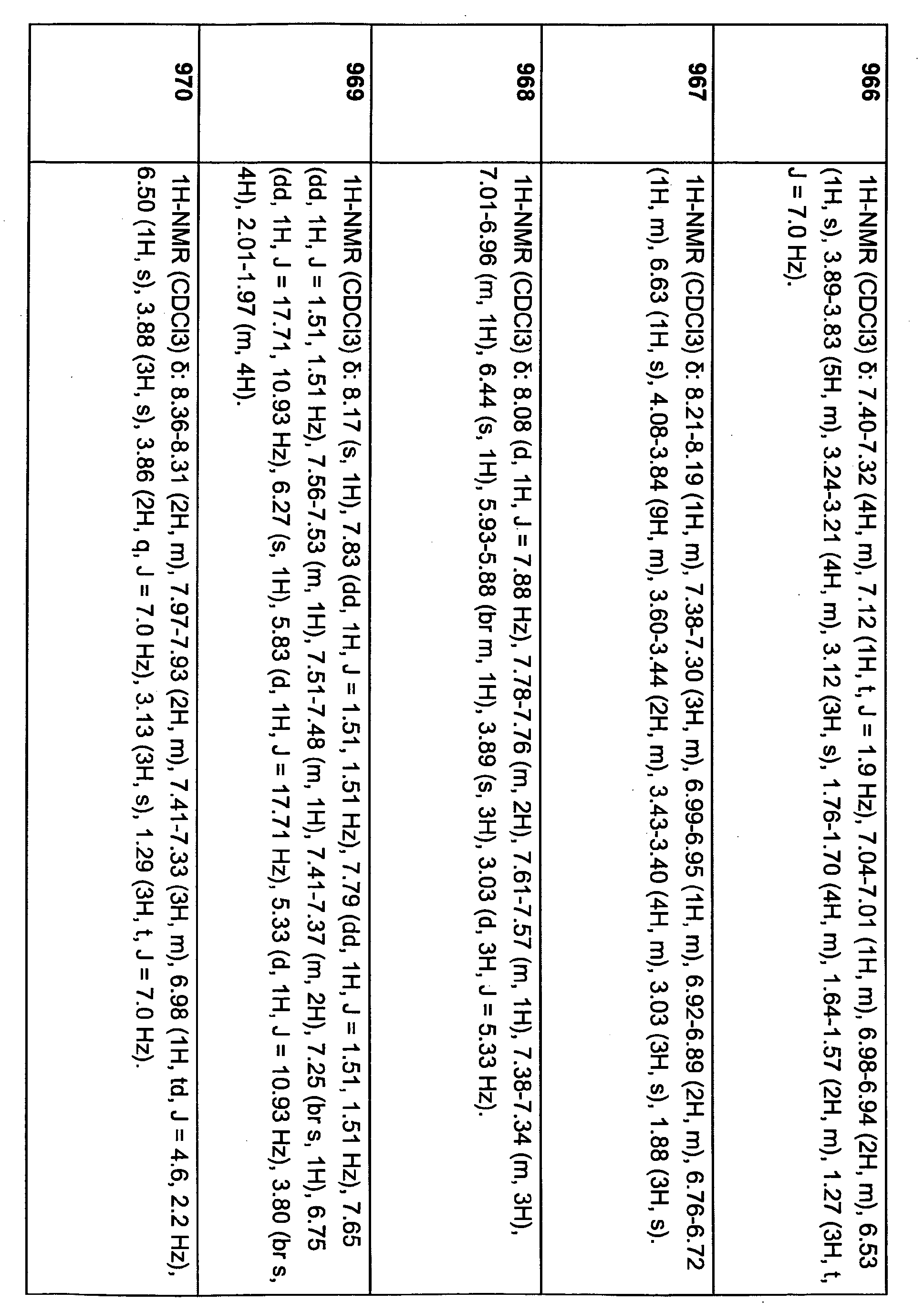

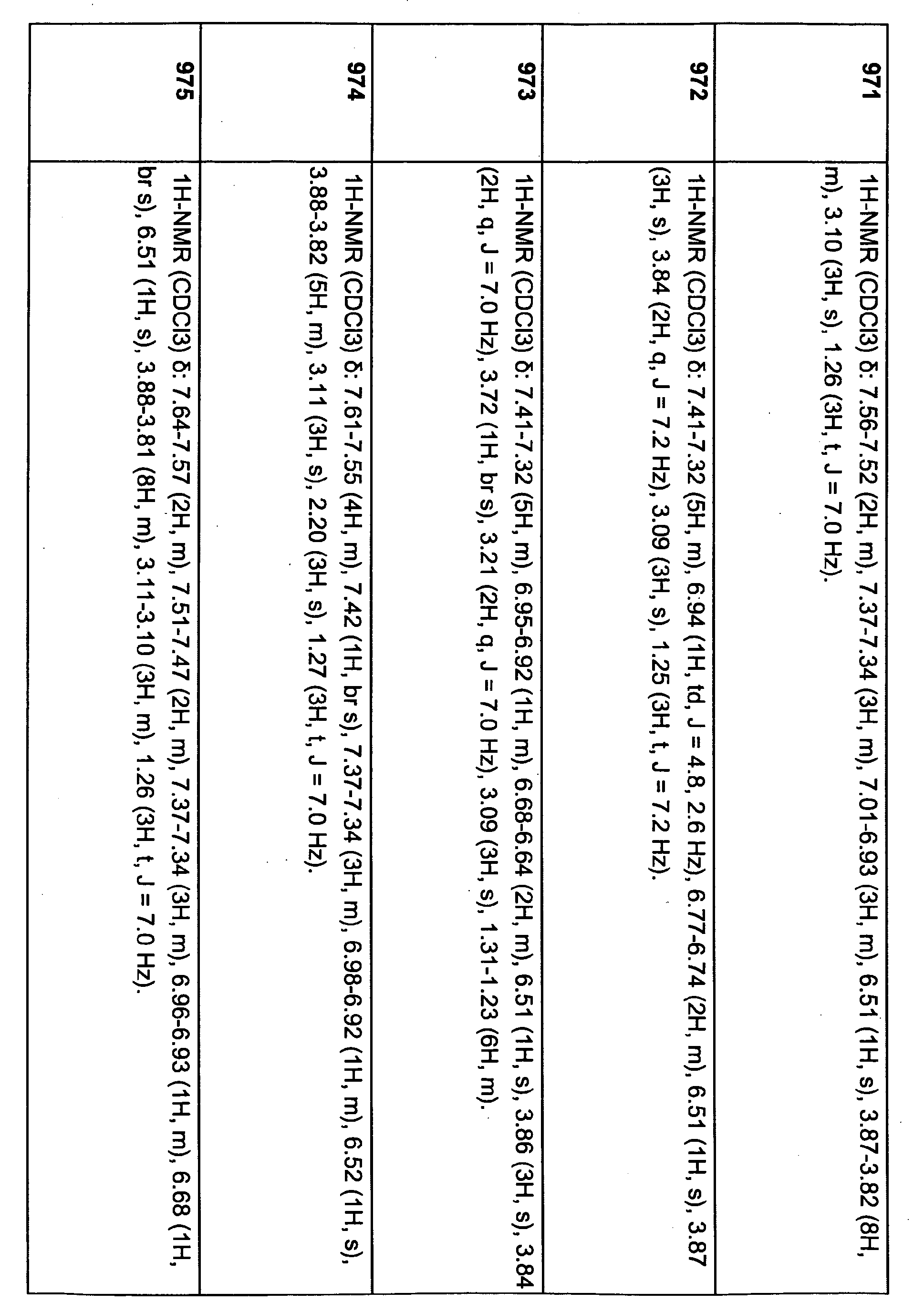

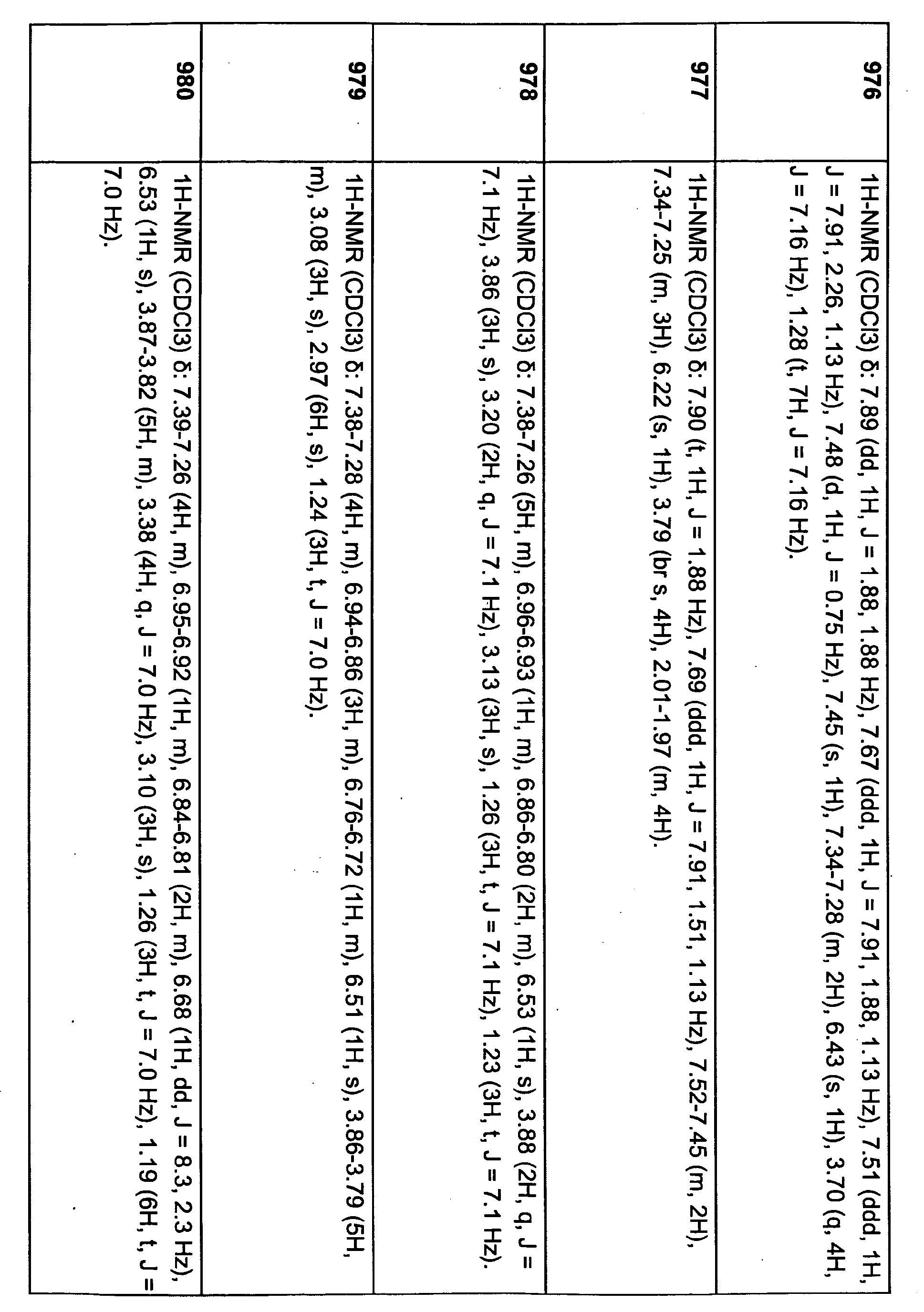

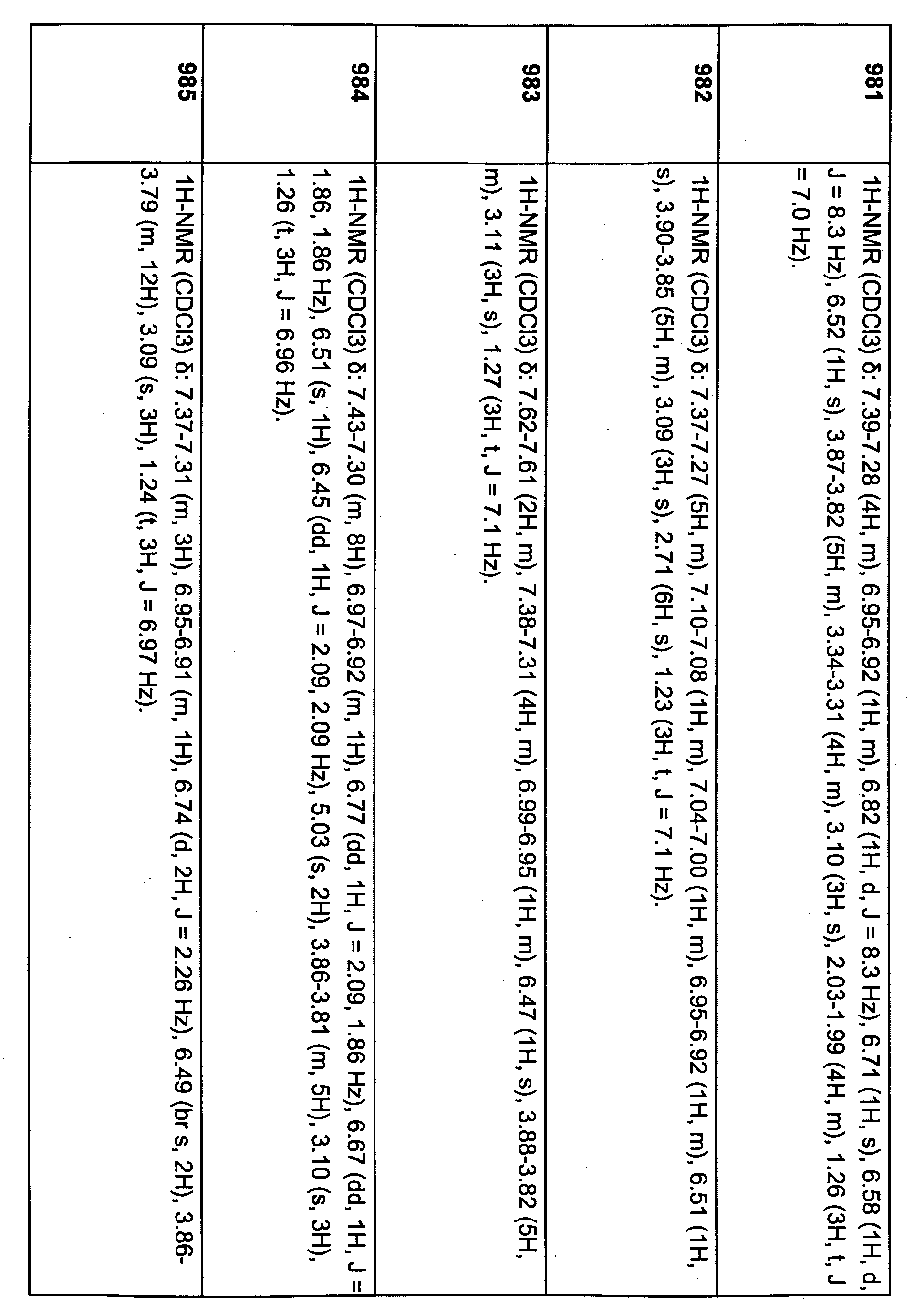

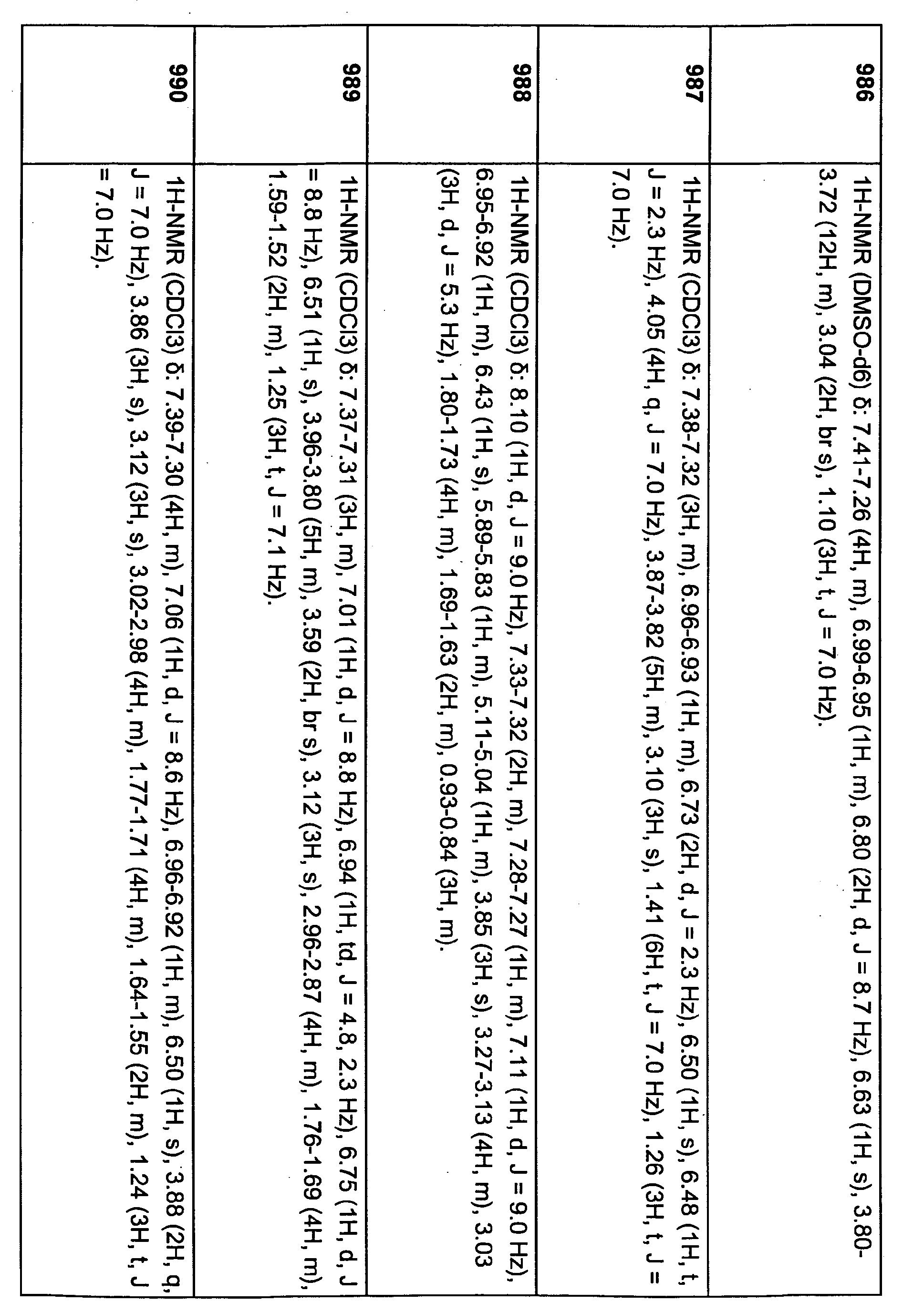

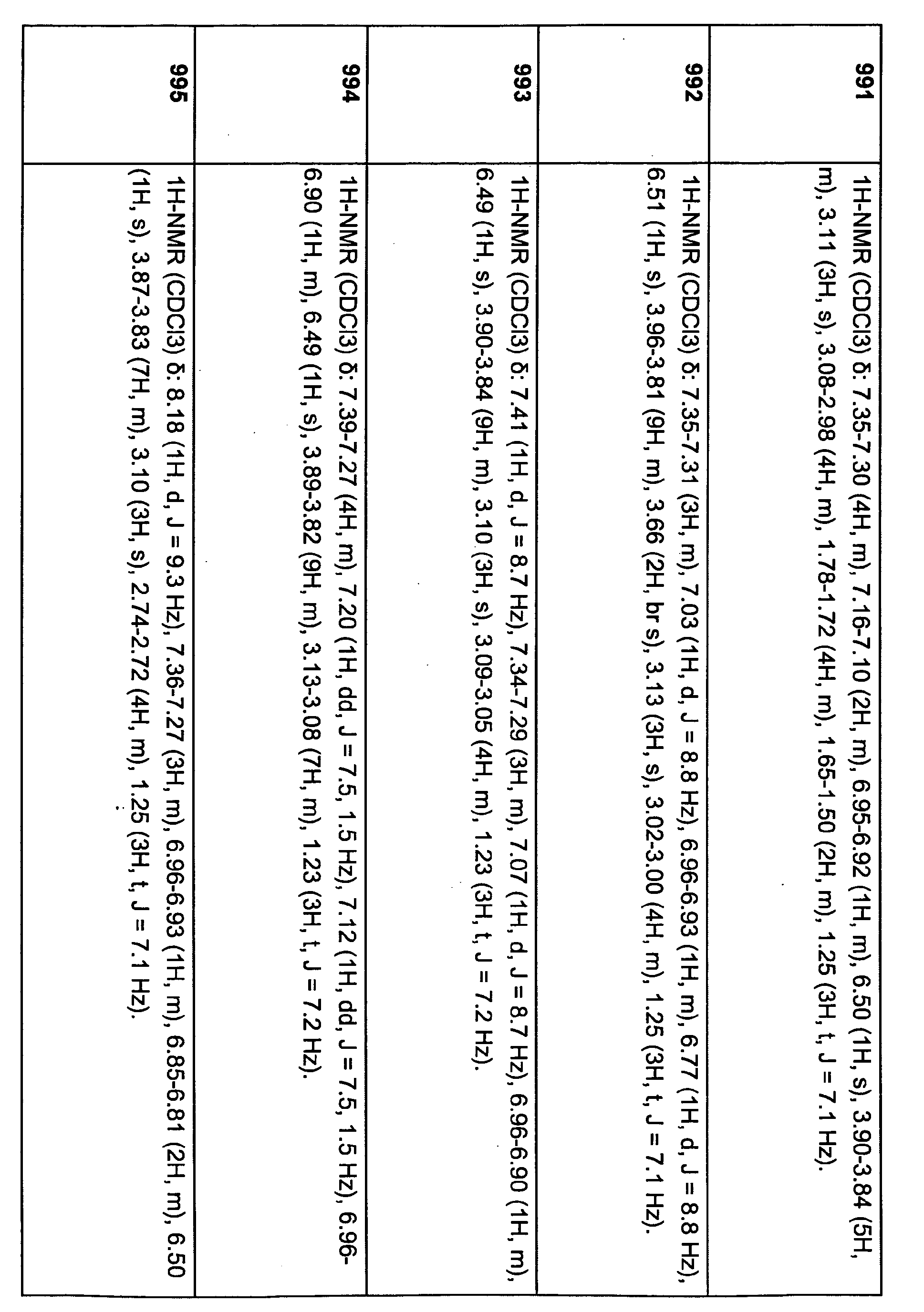

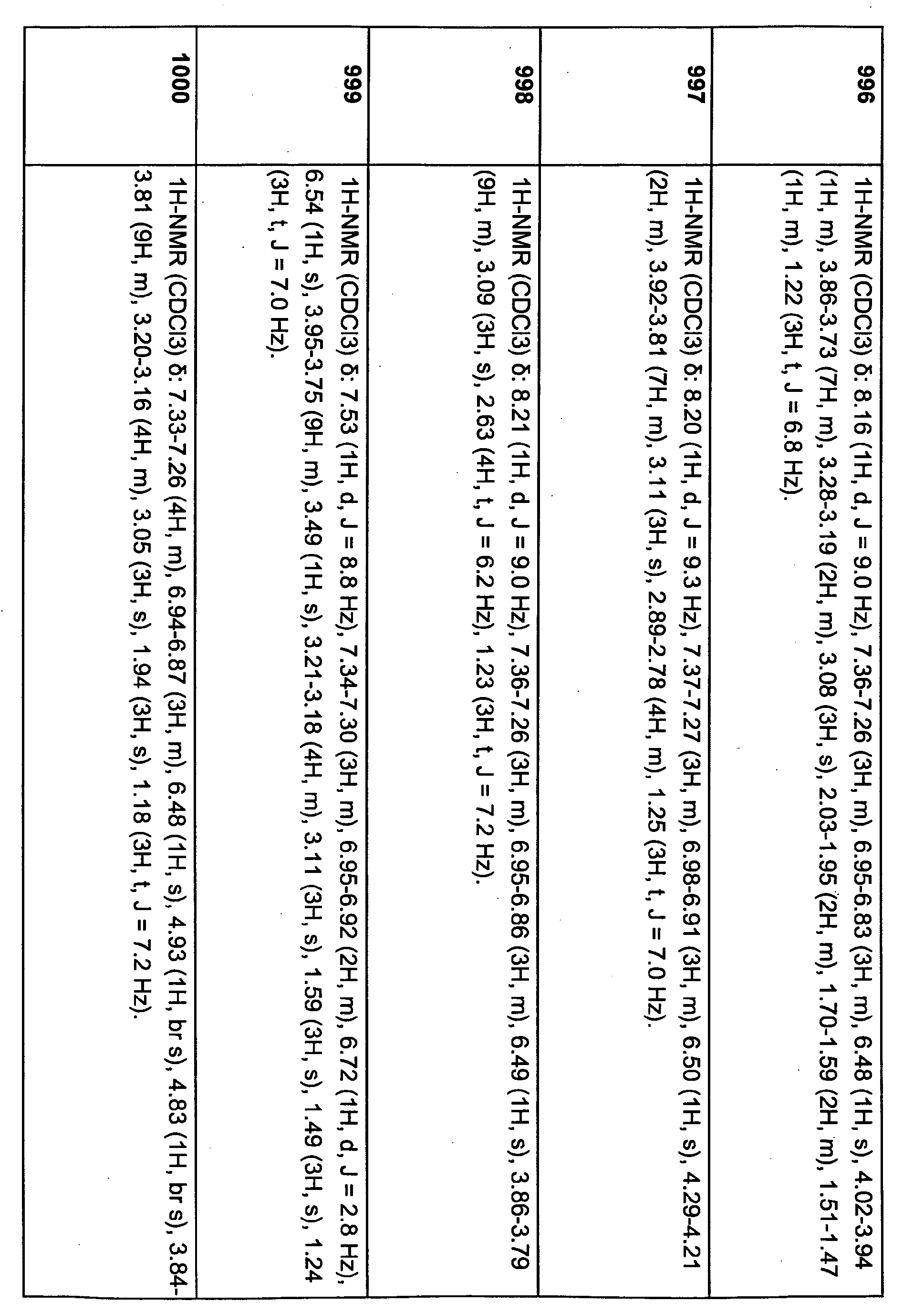

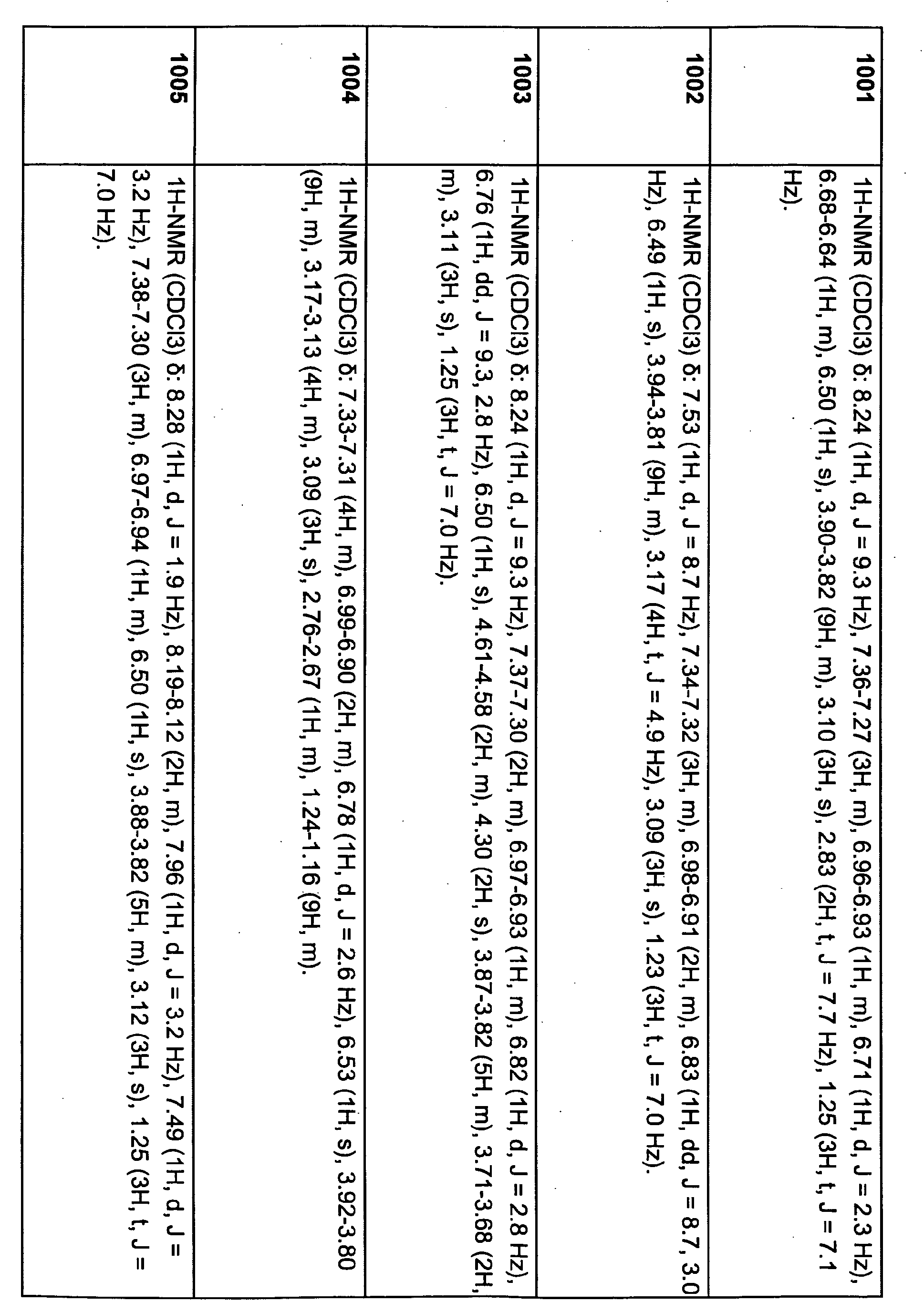

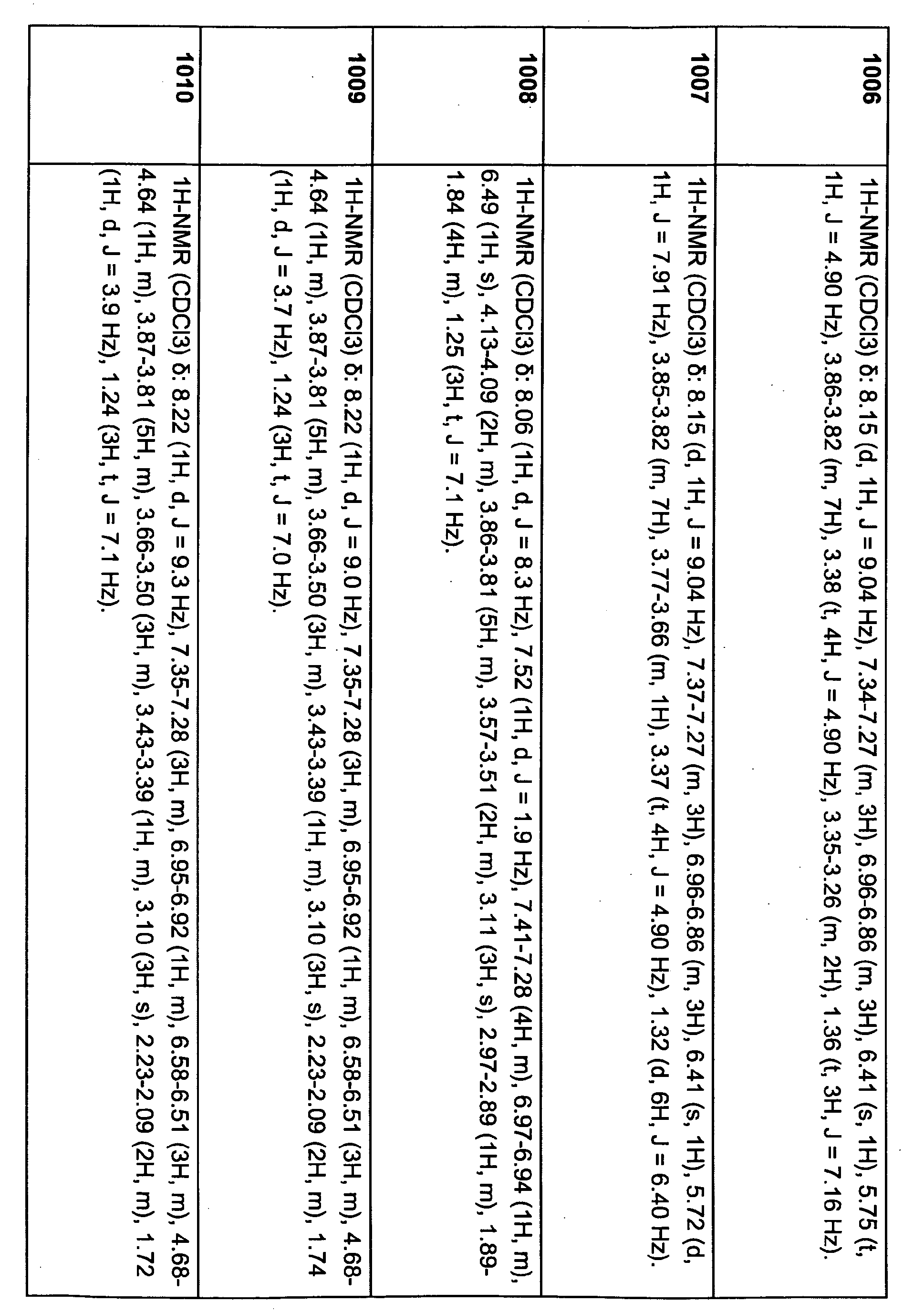

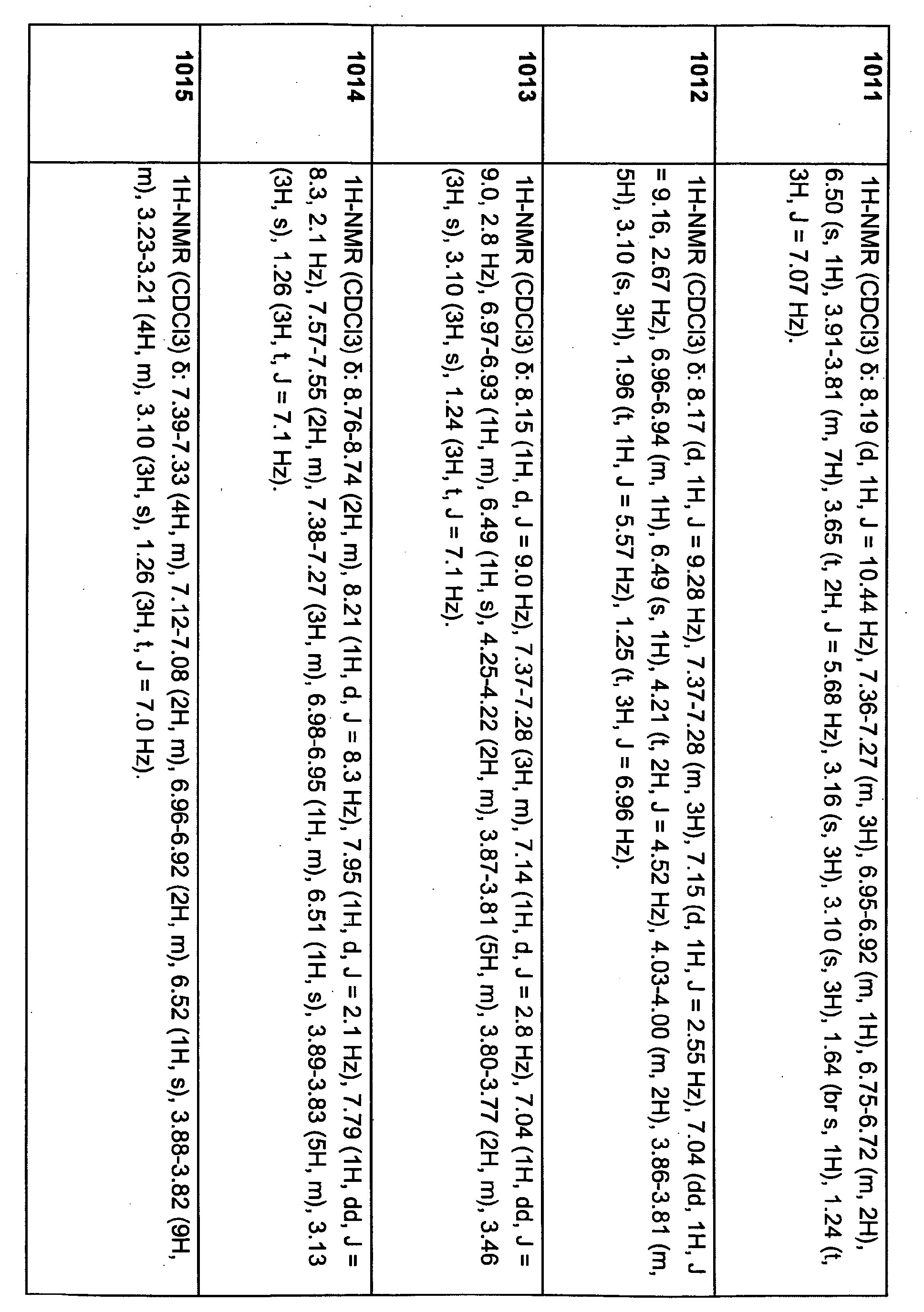

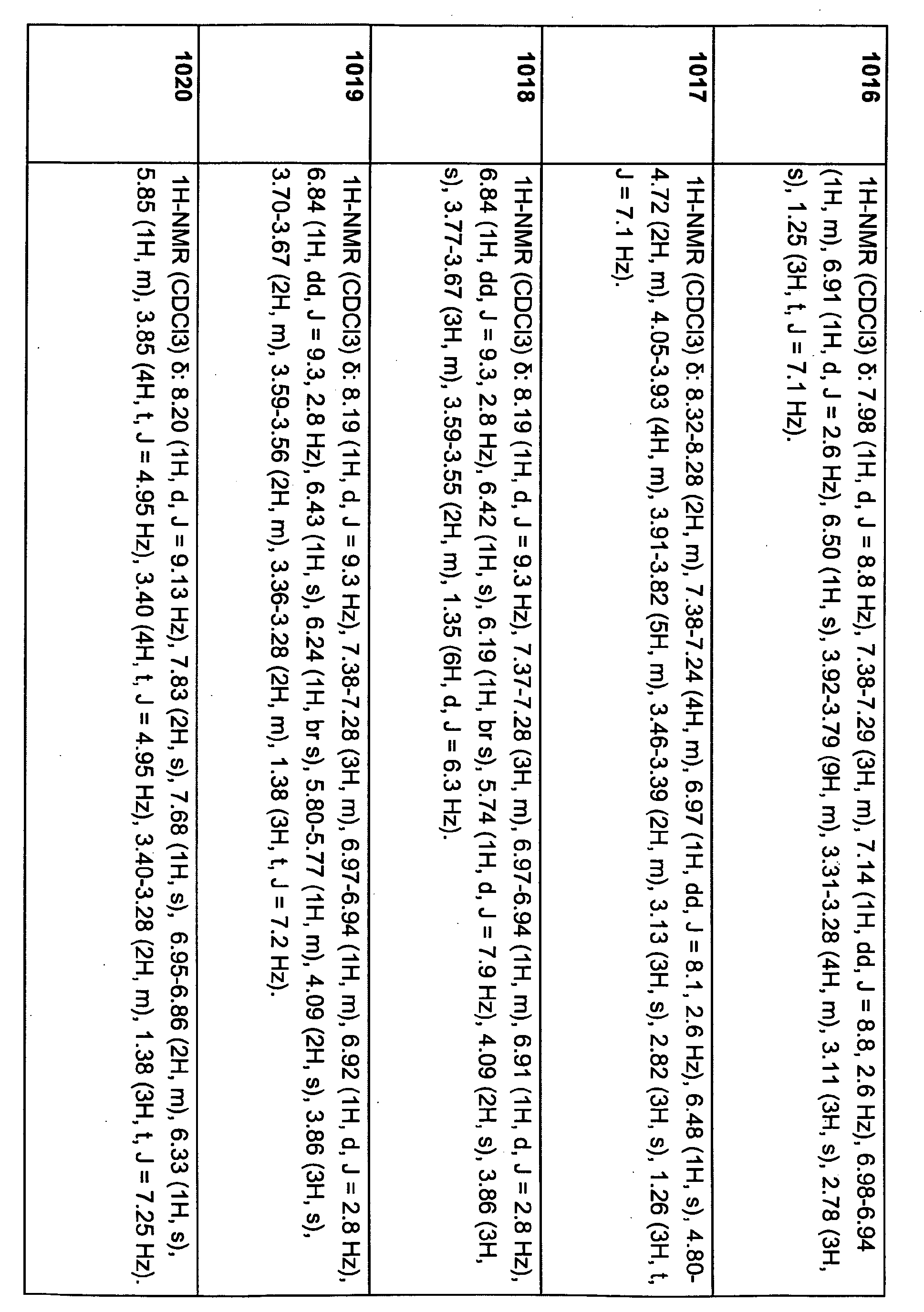

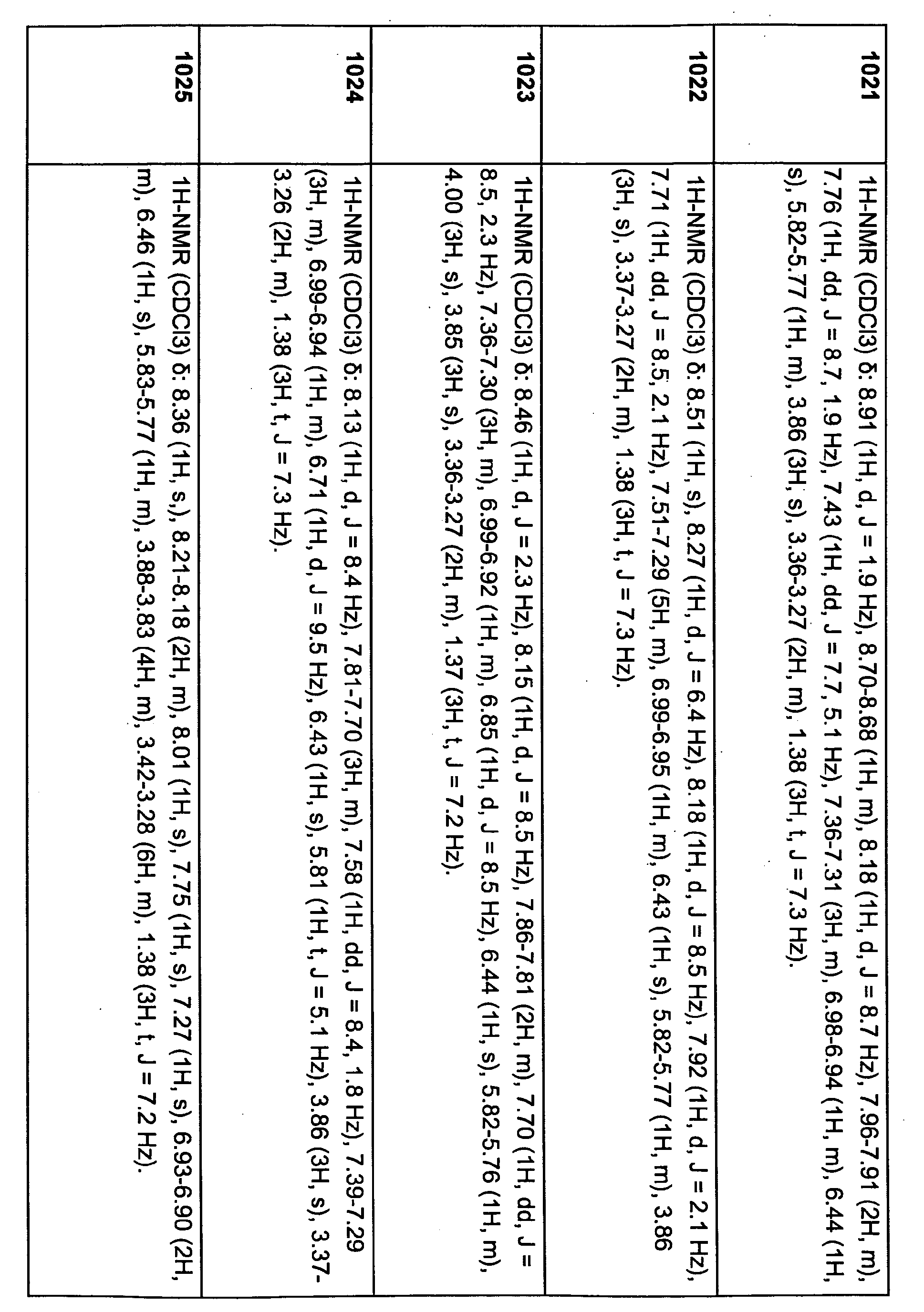

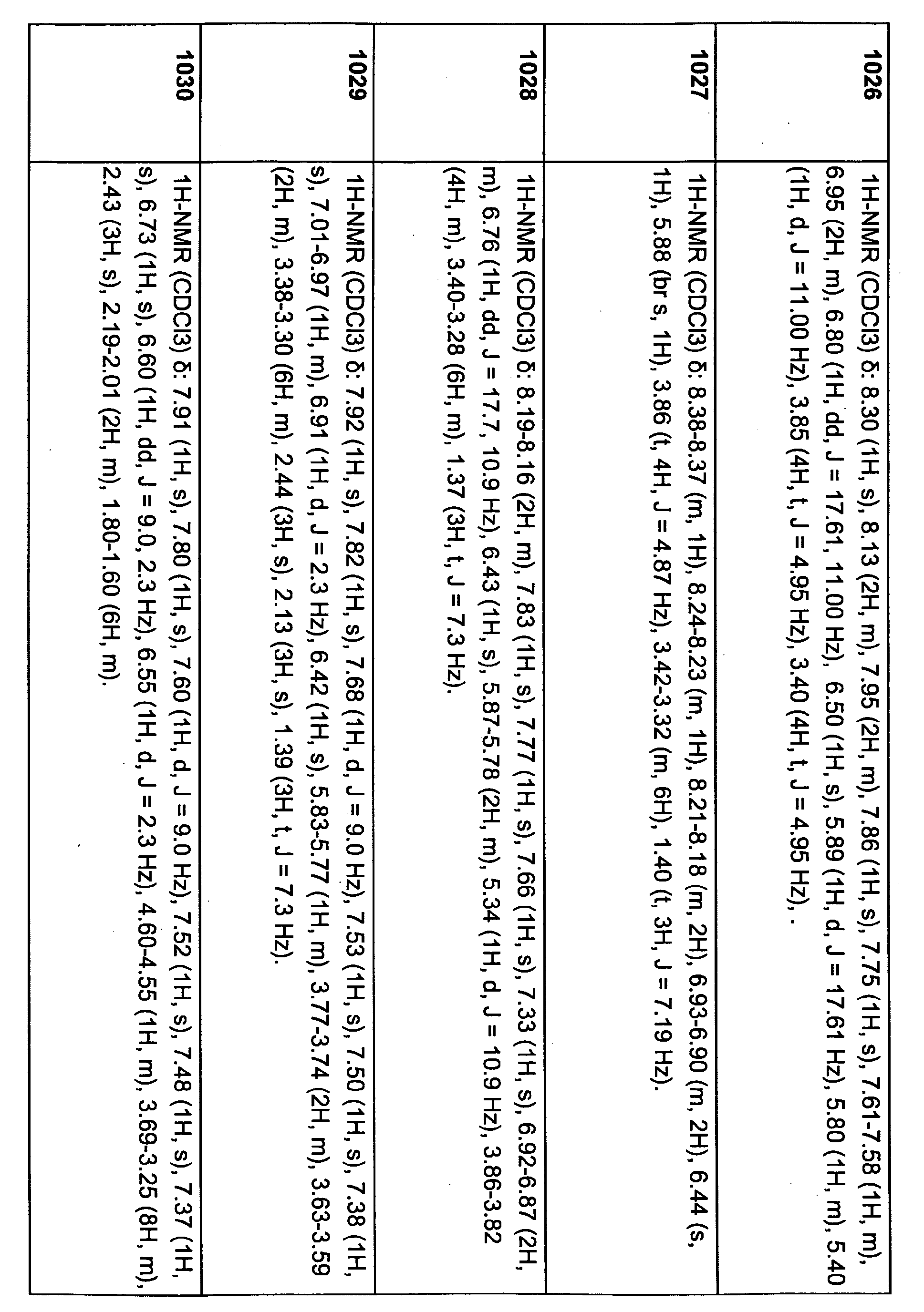

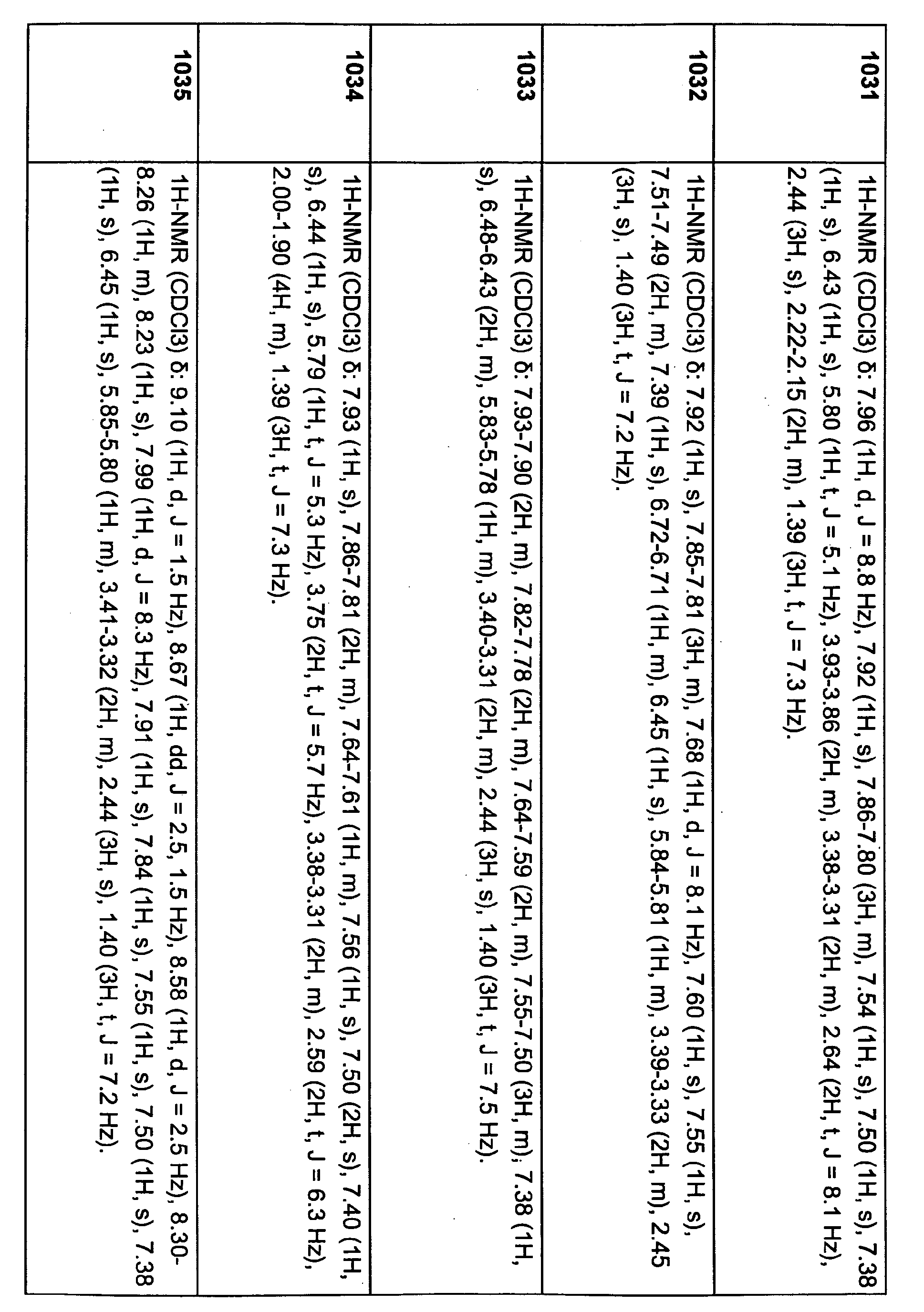

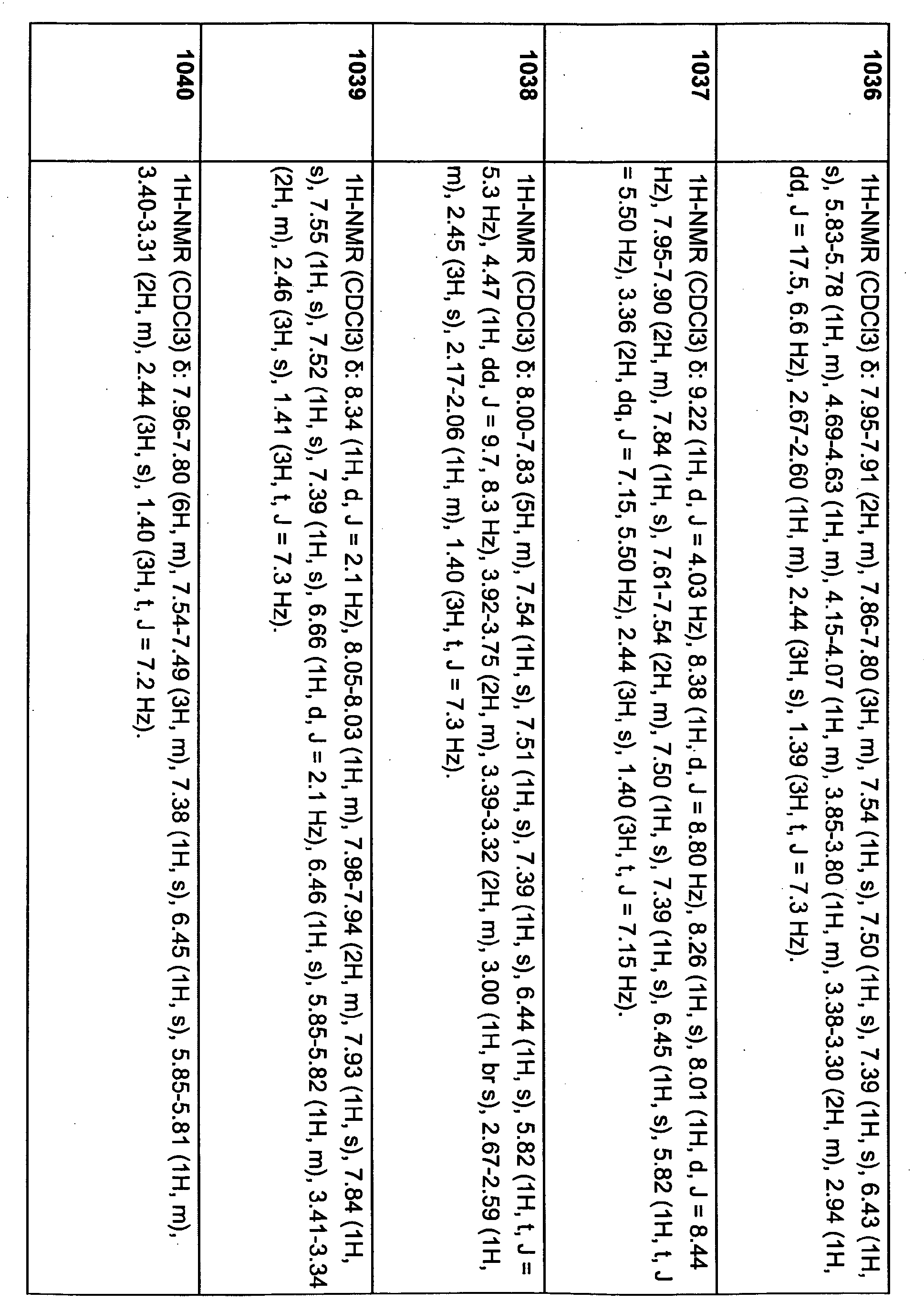

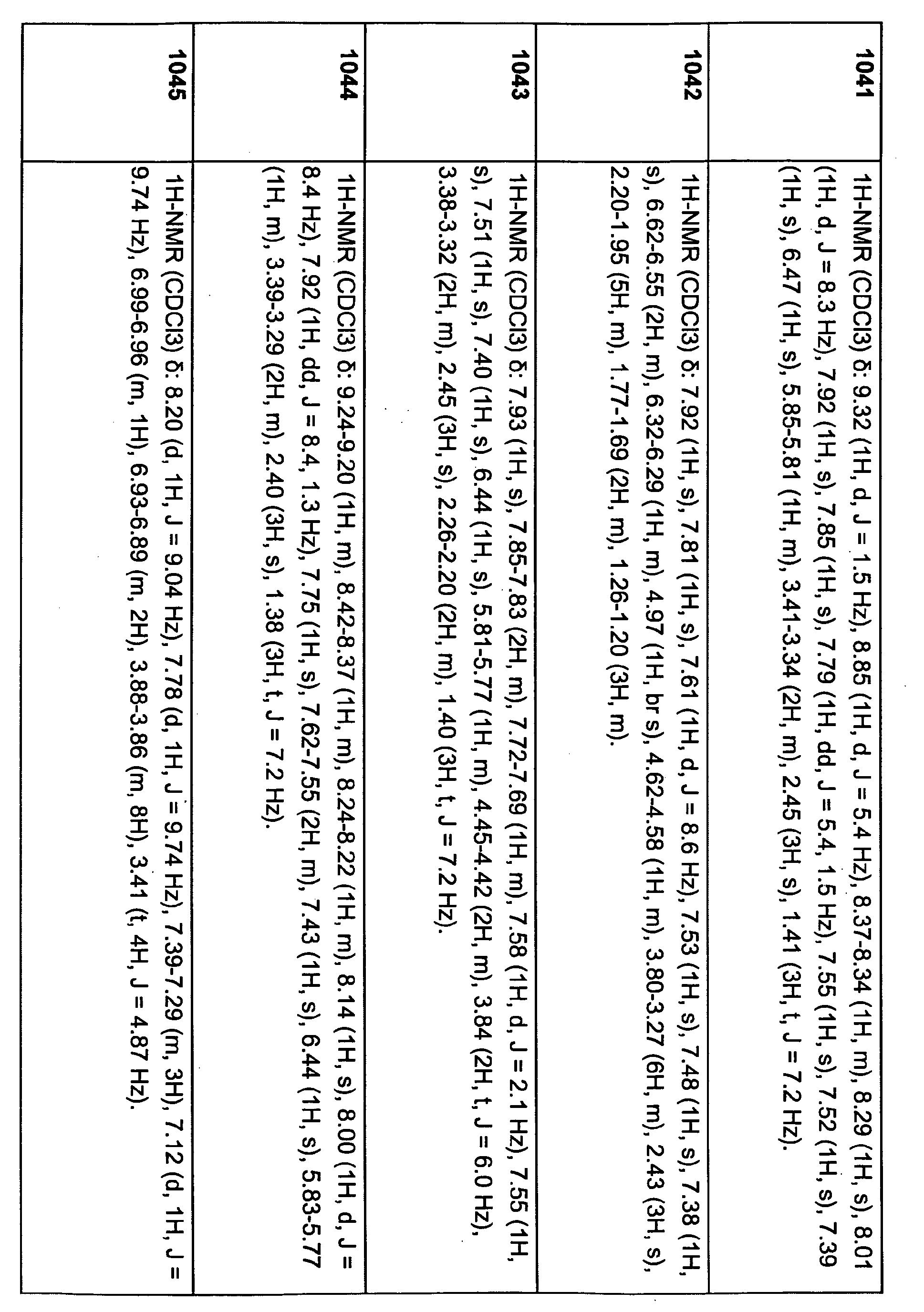

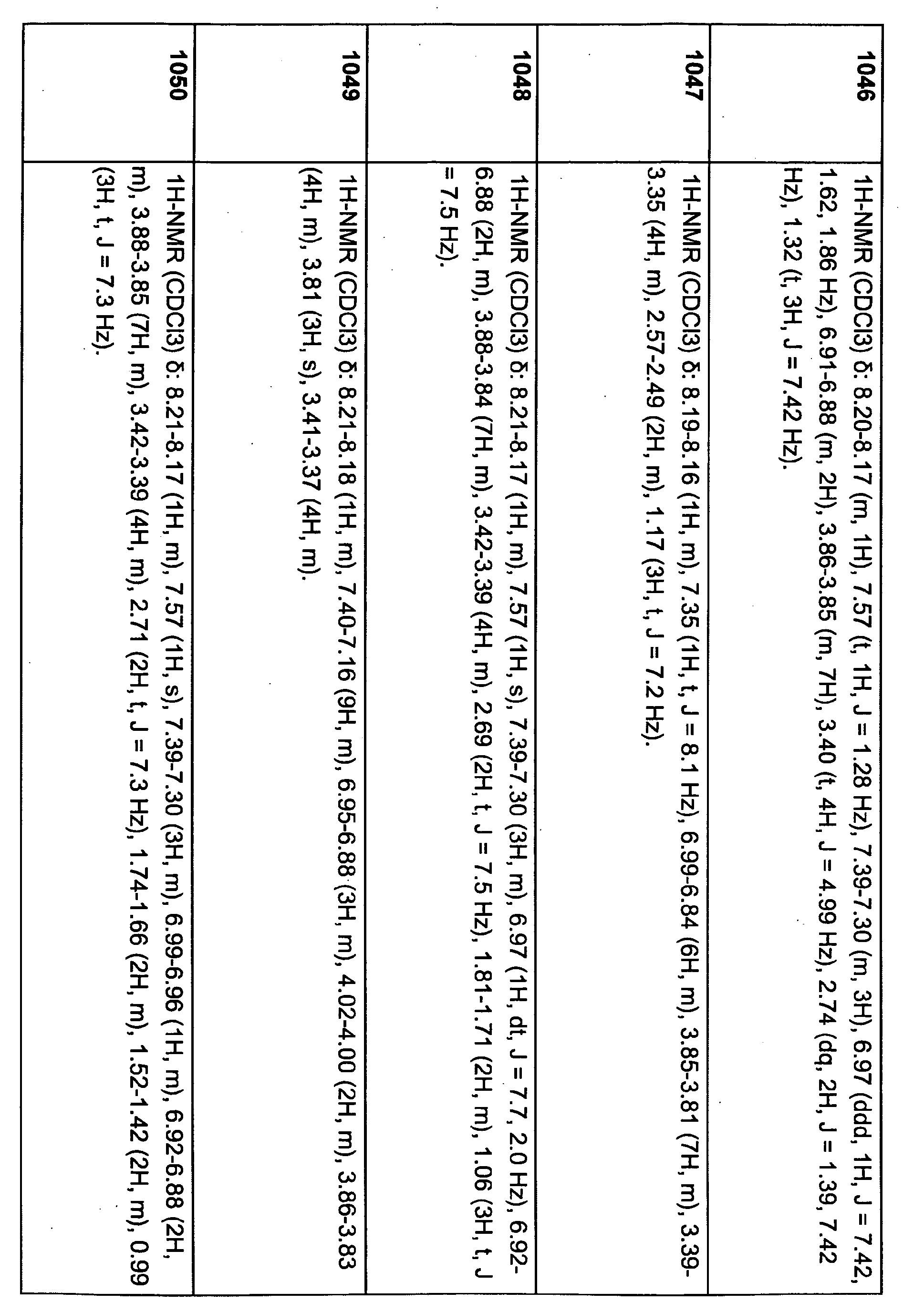

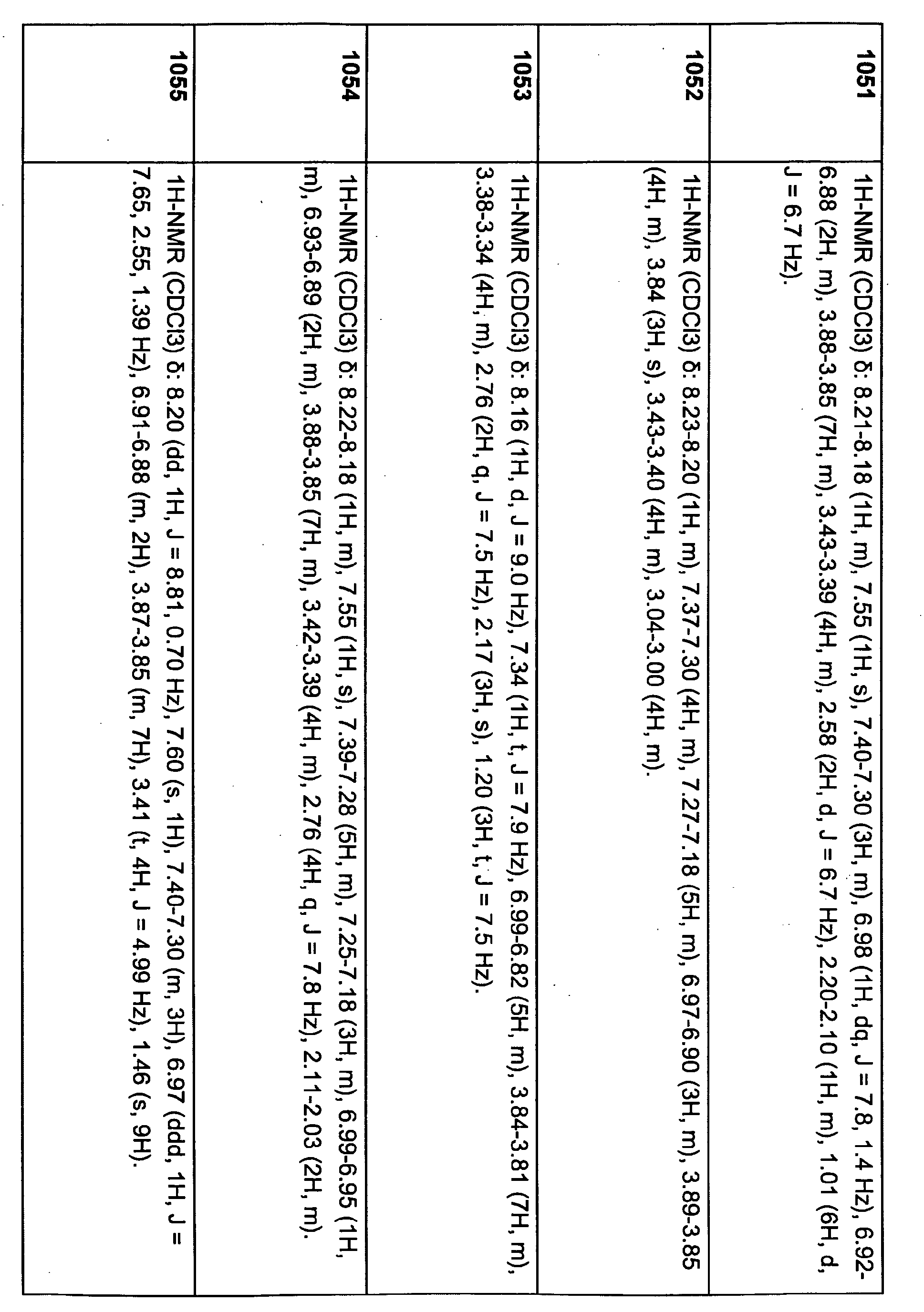

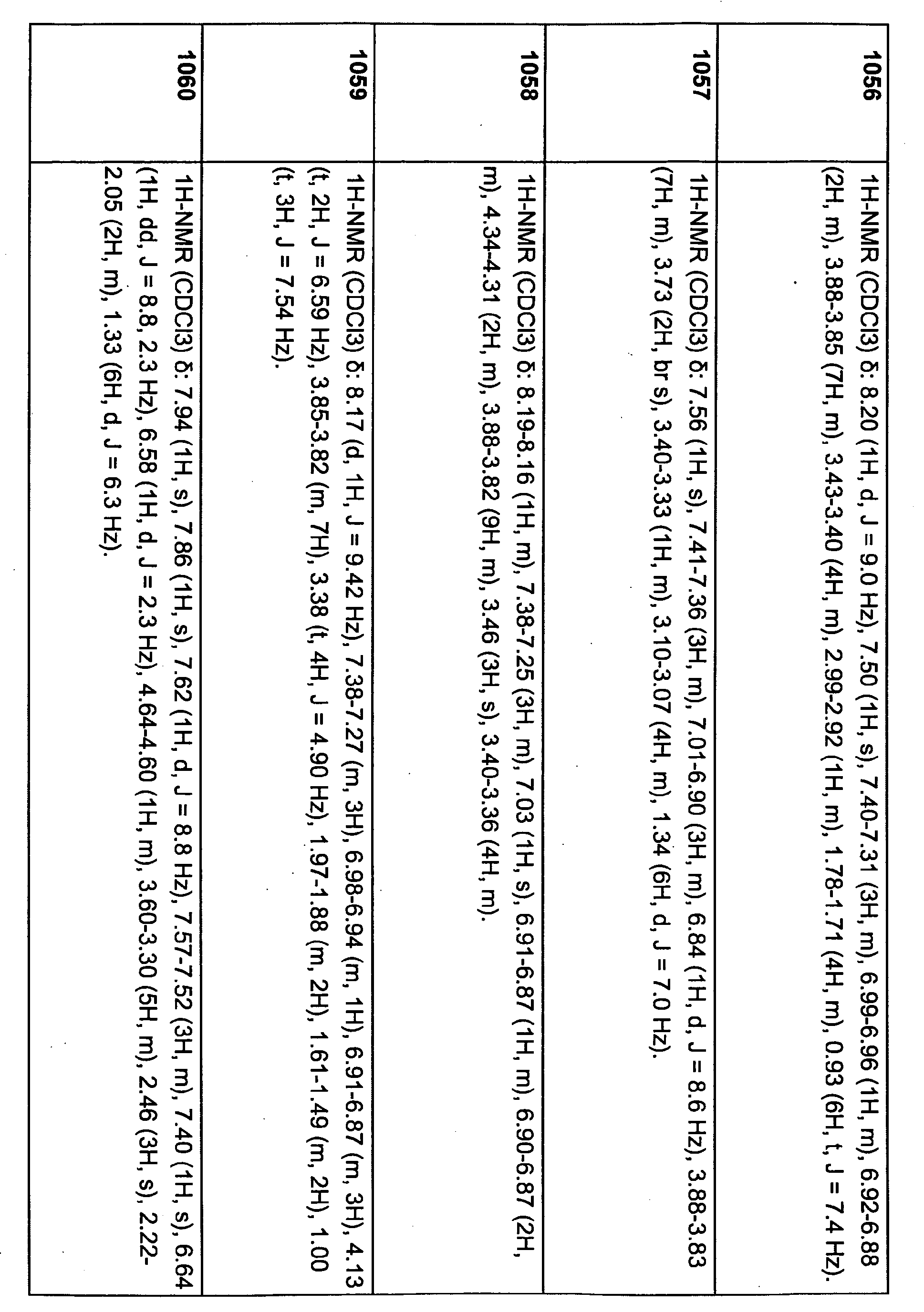

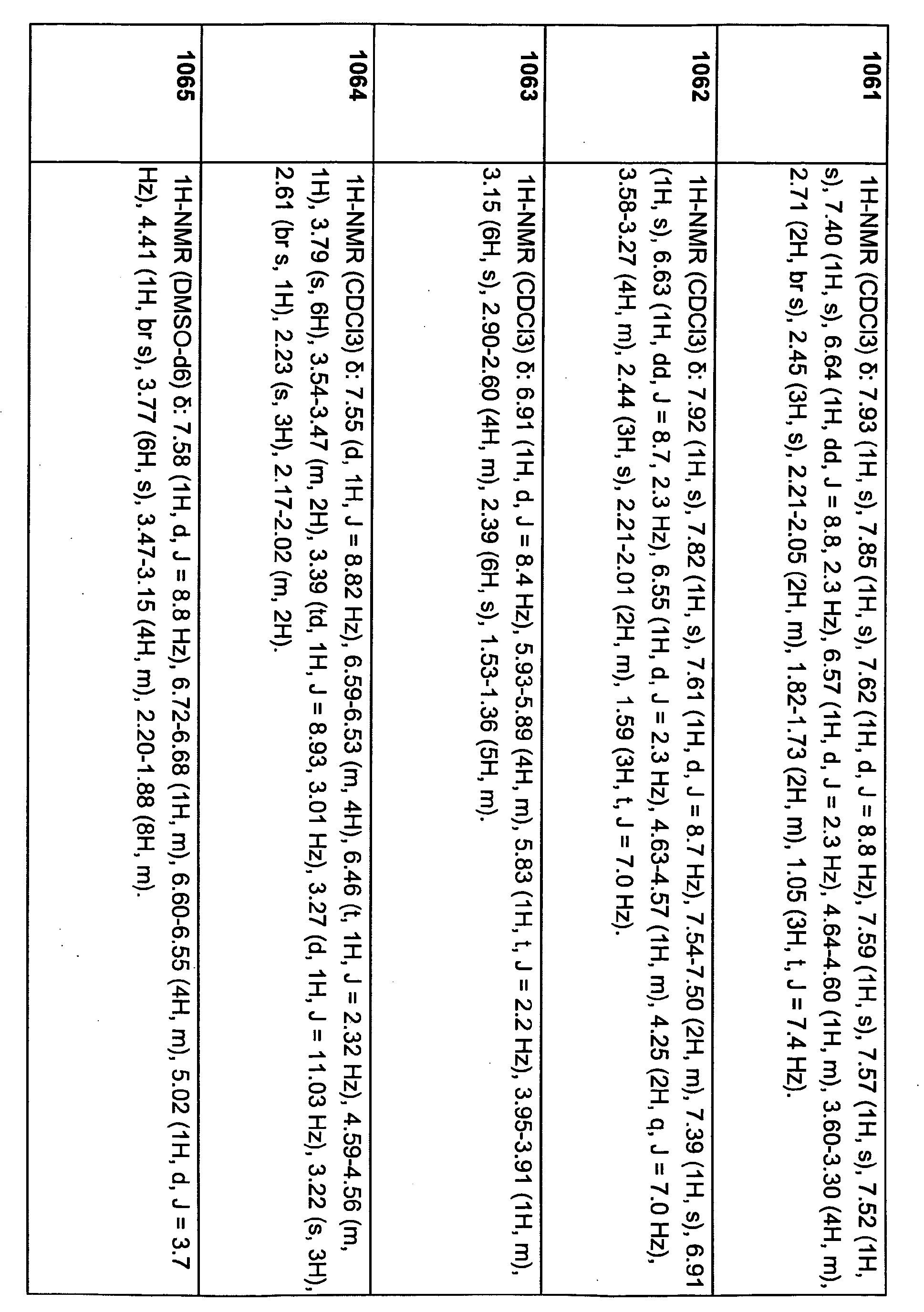

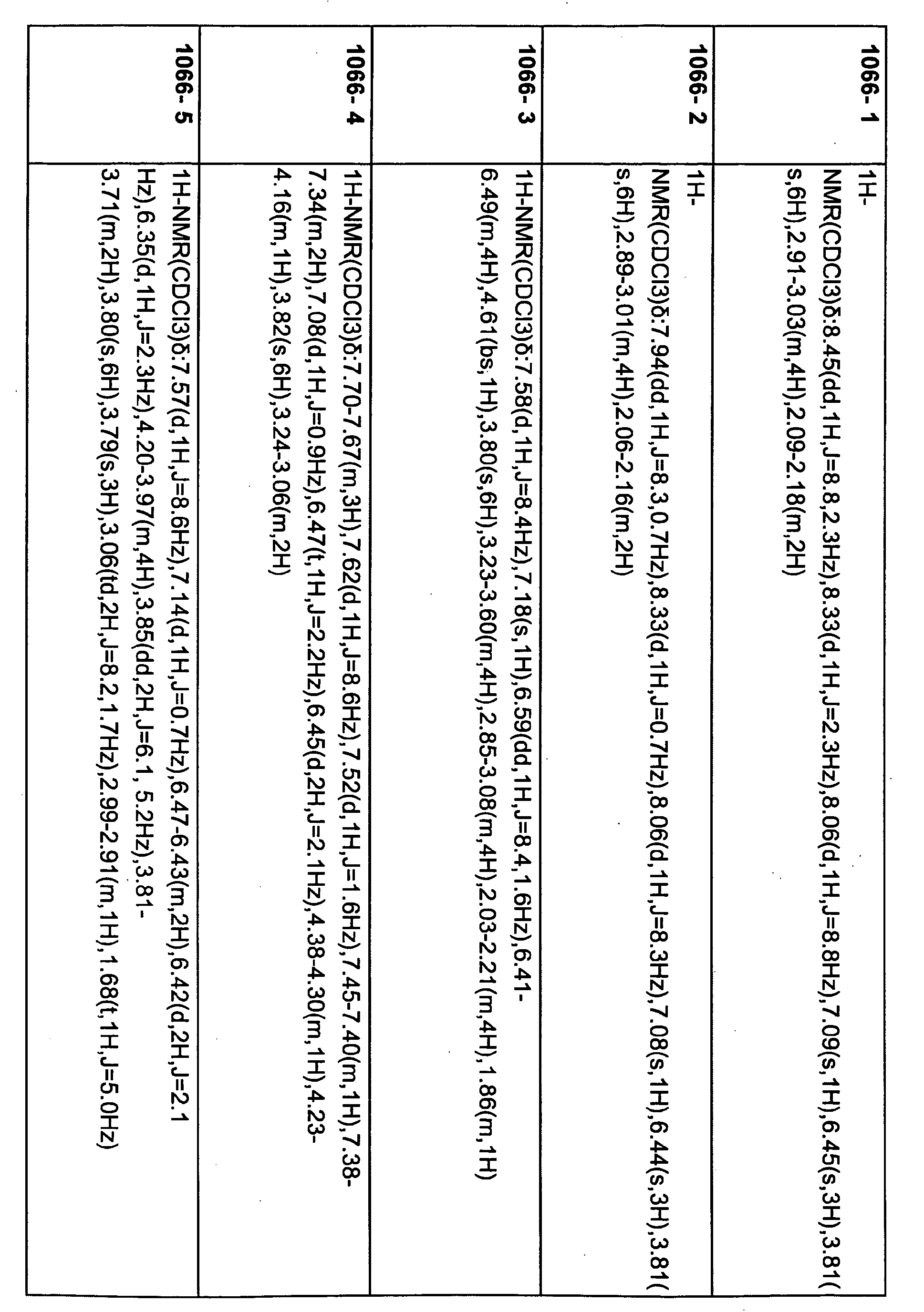

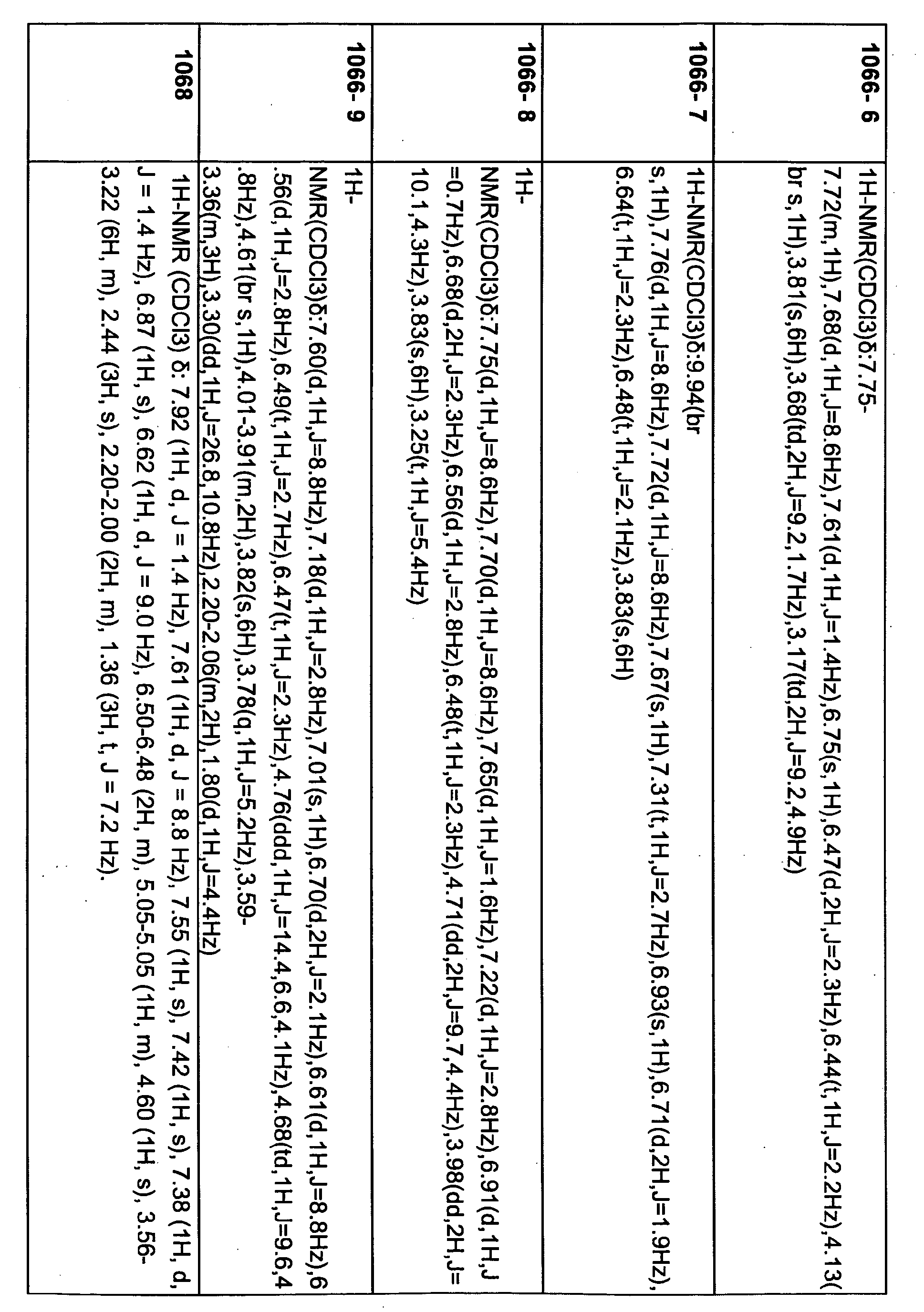

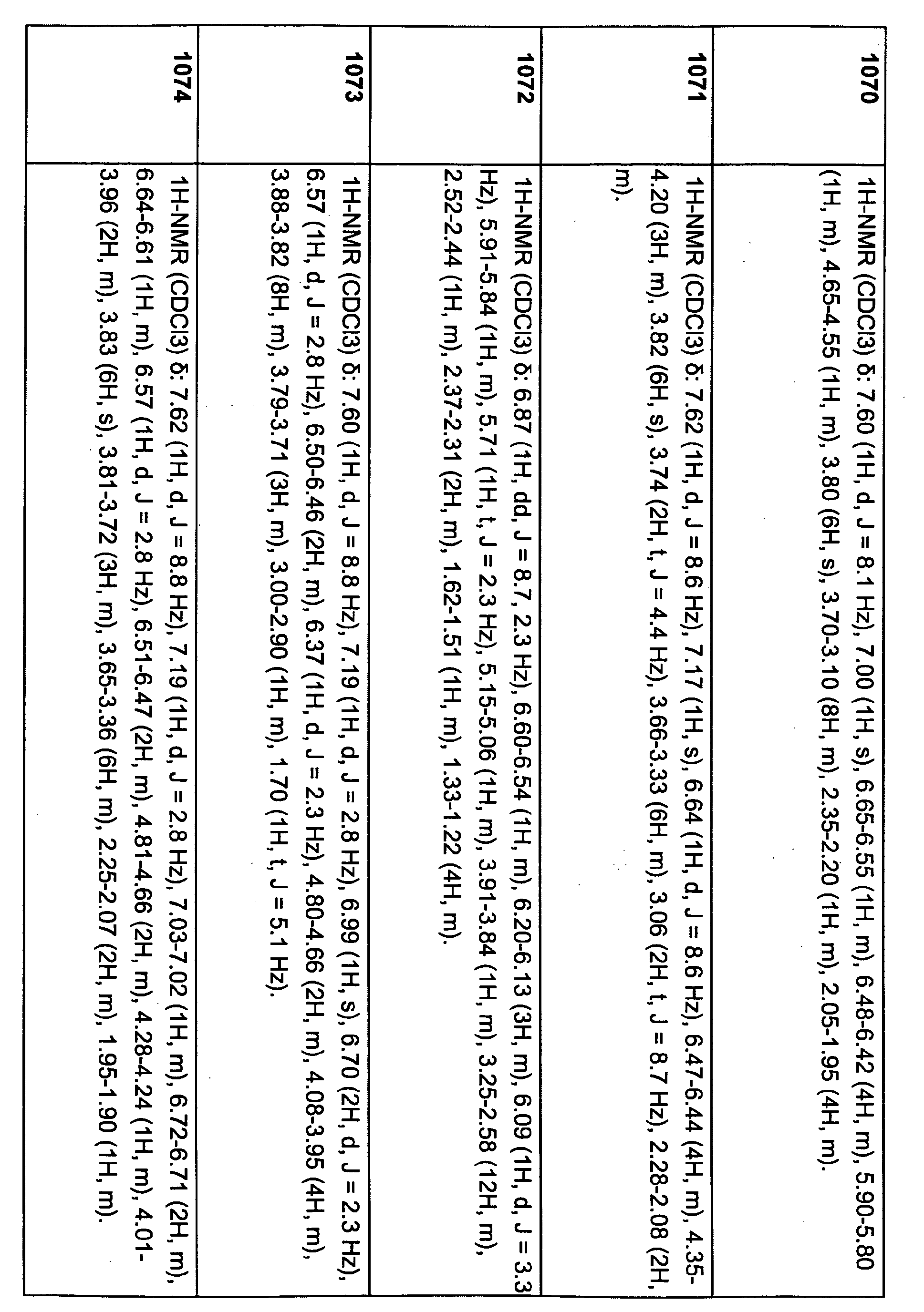

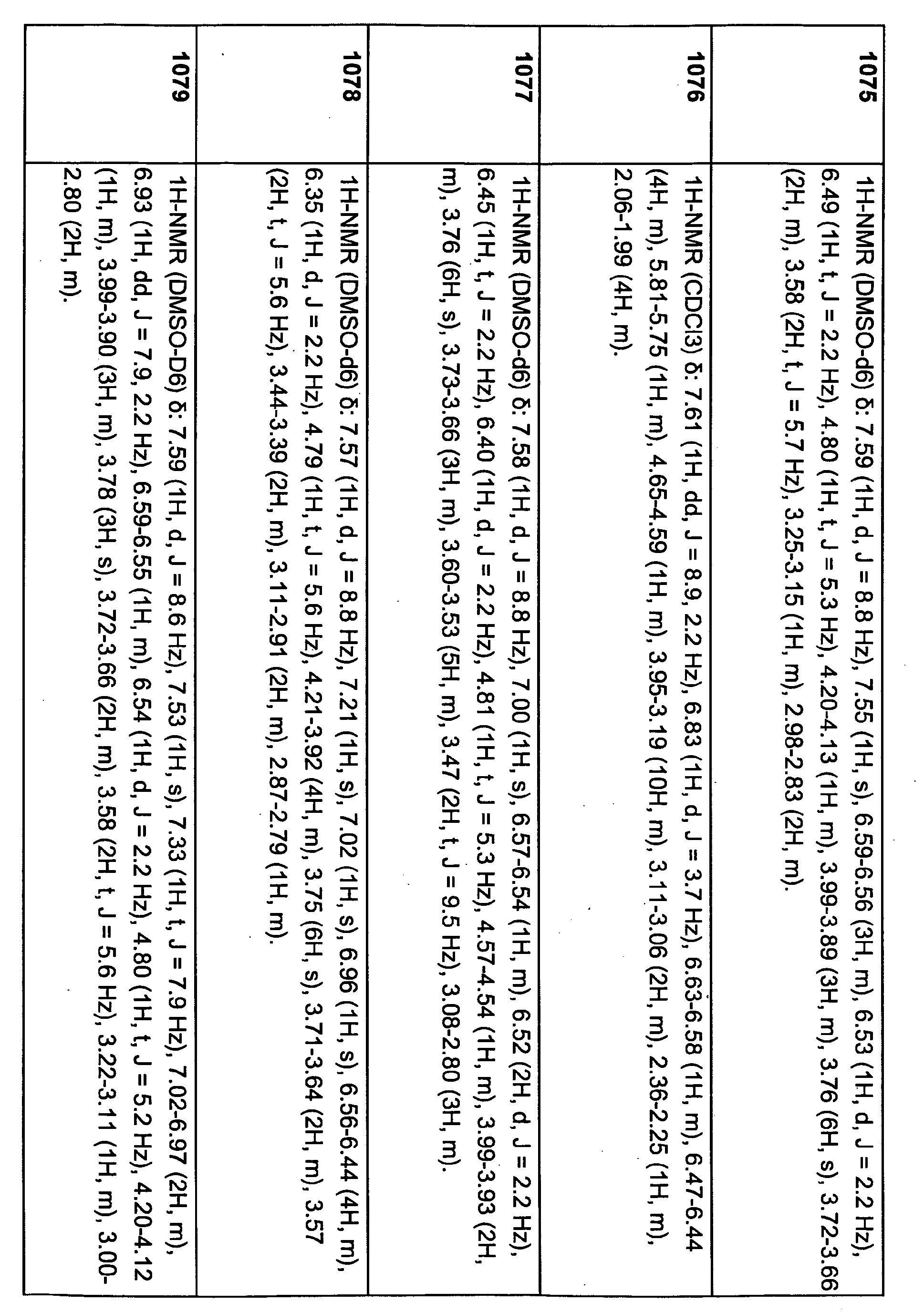

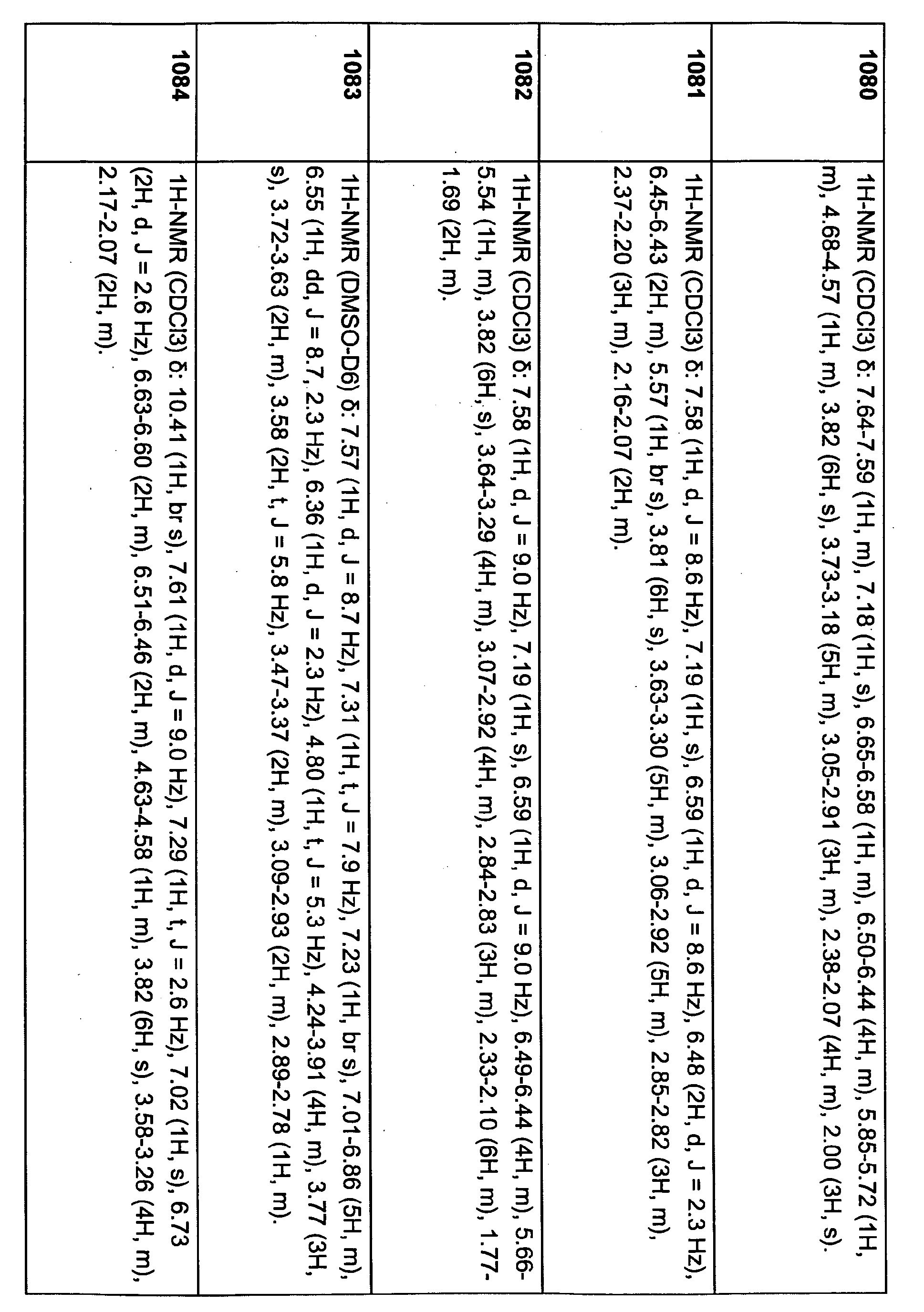

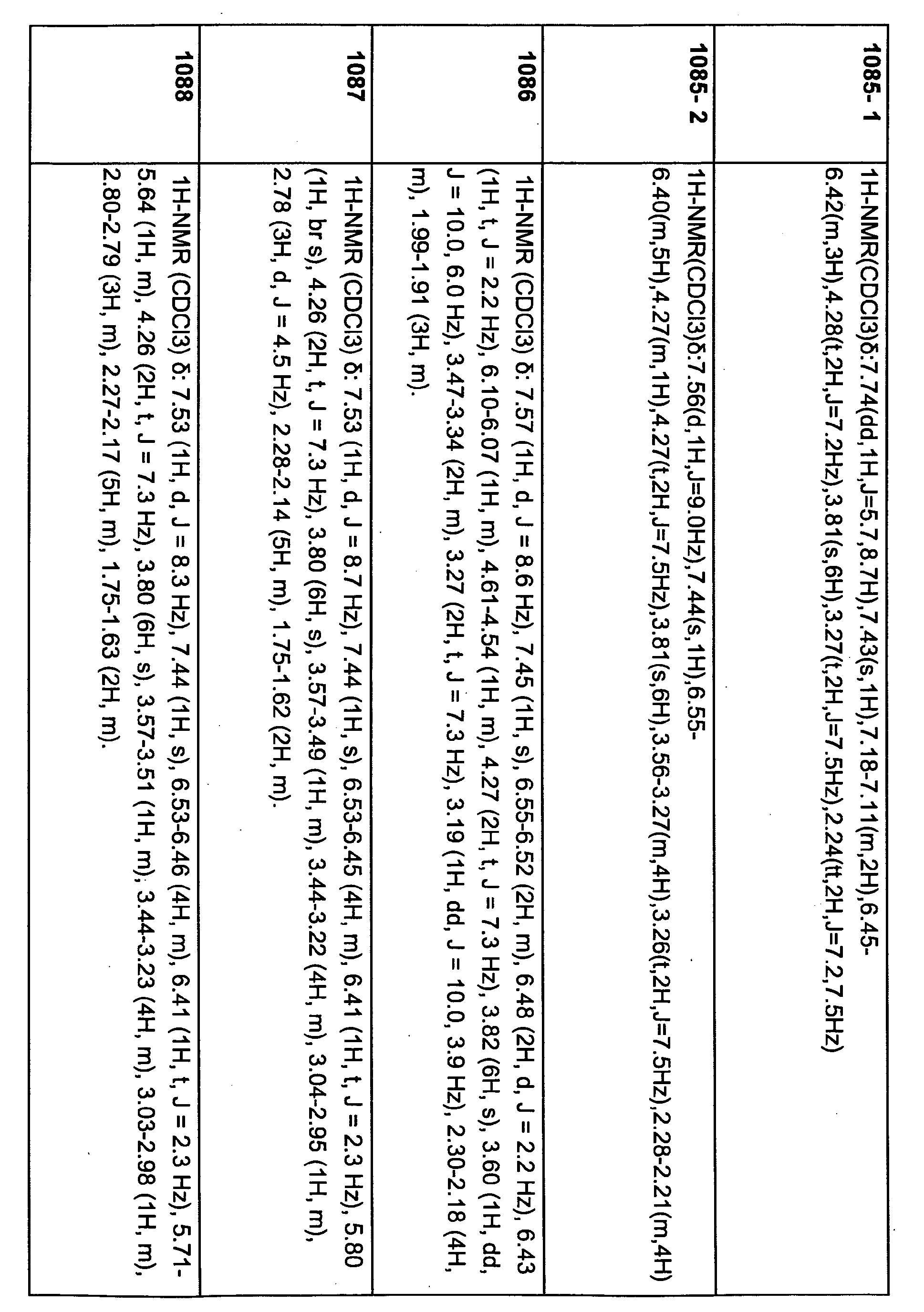

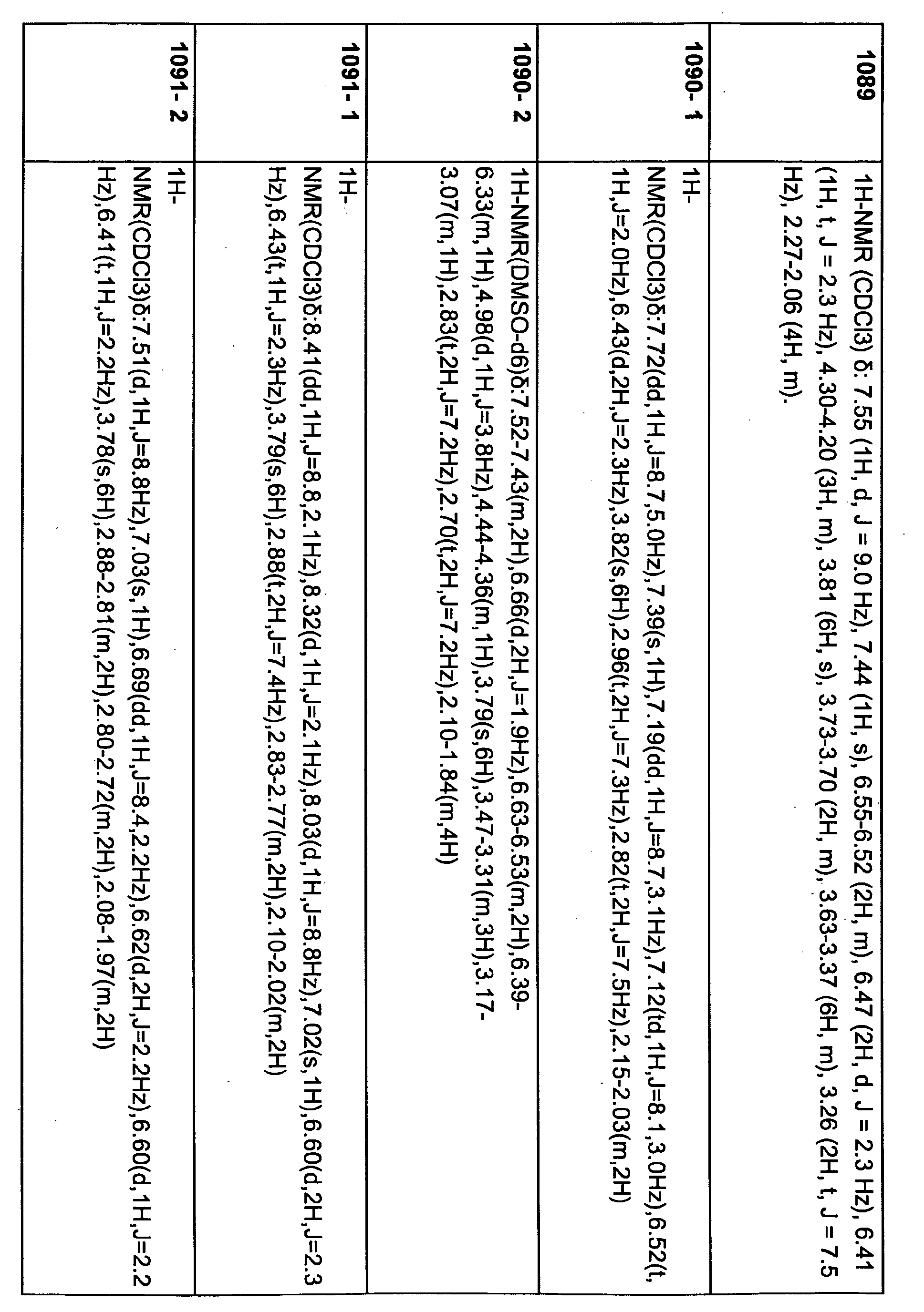

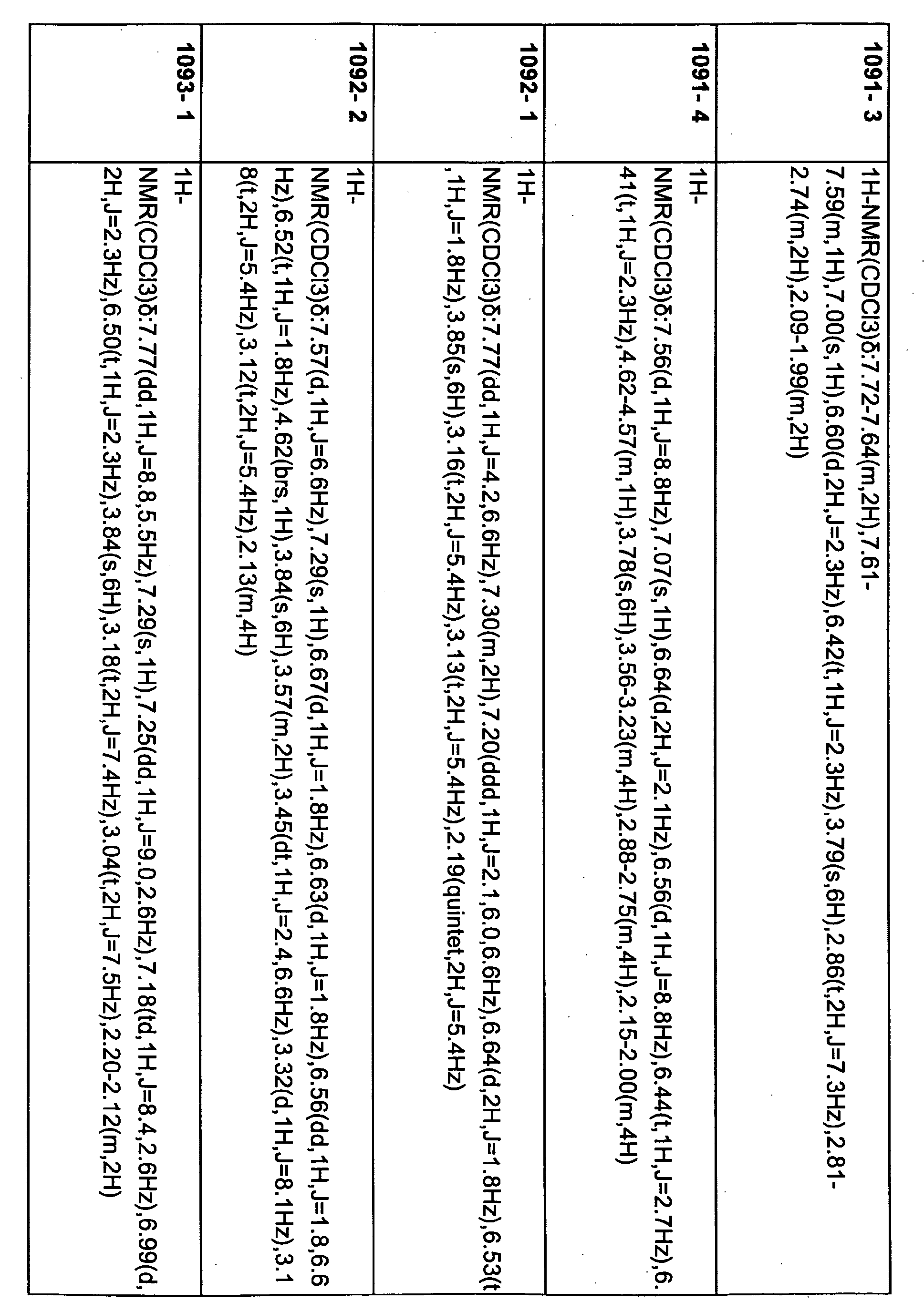

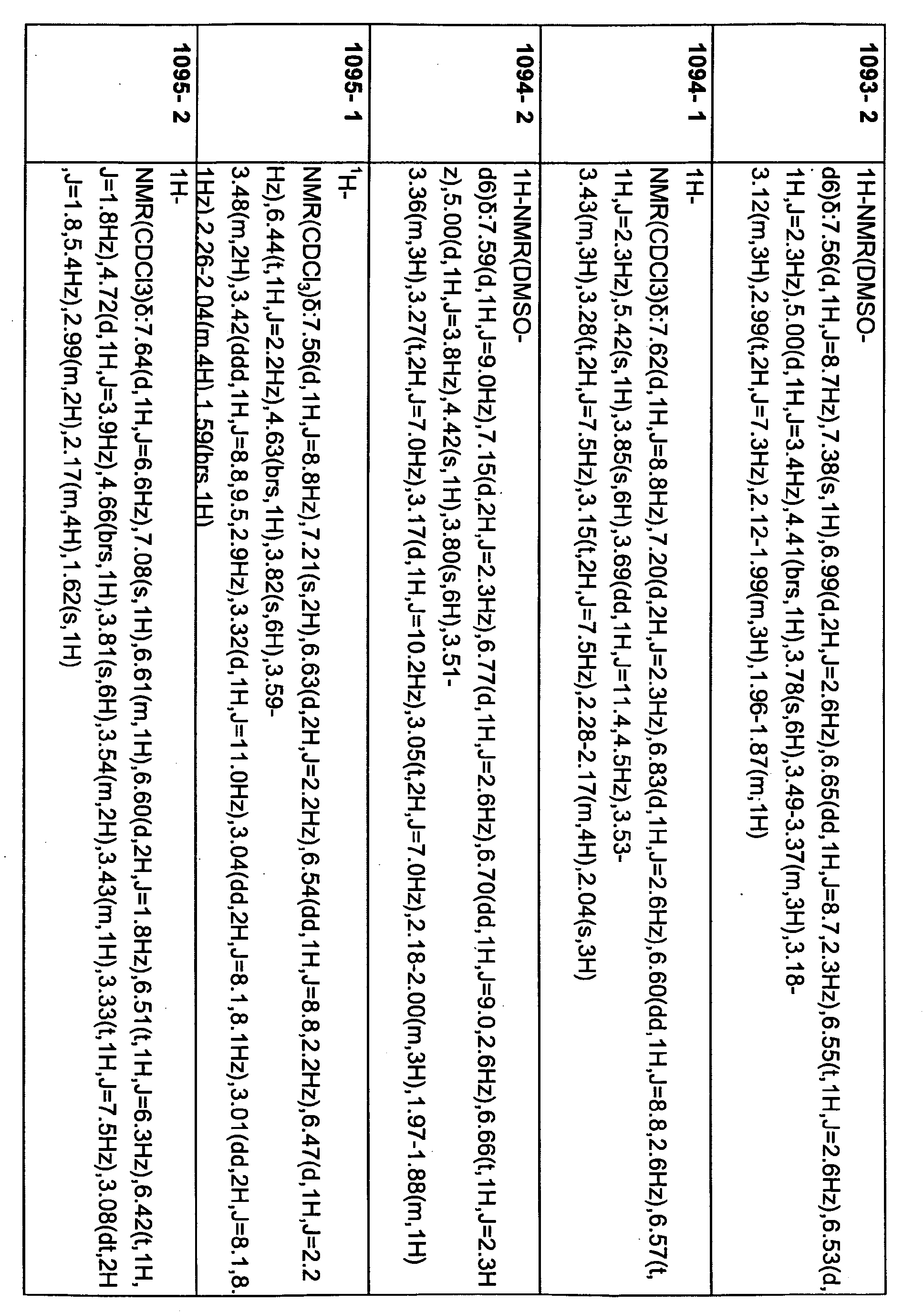

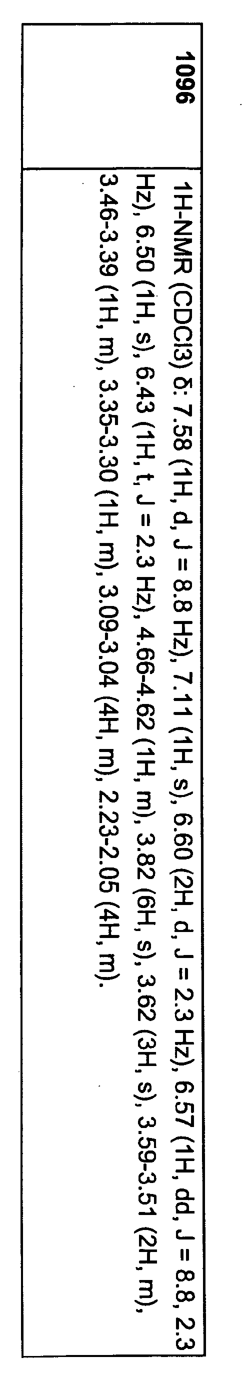

### Preparation Example 1: Preparation of HCV pseudotype virus

An HCV pseudotype virus was prepared according to a method similar to the one described in Virology, Vol. 286, pp. 263-275, 2001. First, based on the methods described in Proc. Natl. Acad. Sci. USA Vol. 94, pp. 14764-14769, 1997 and Proc. Natl. Acad. Sci. USA Vol. 92, pp.4477-4481, 1995, a recombinant virus (hereinafter to be referred to as ΔG/SEAP/VSV G) having a genome wherein the envelope protein (glycoprotein) gene of vesicular stomatitis virus (VSV) had been substituted by a secreted alkaline phosphatase (SEAP) gene, which was covered with the VSV glycoprotein, was prepared. Then, CHO-K1 cells expressing VSV glycoprotein (Above CHO-K1 cells are described as CHO cells transfected with pCAG-VSVG in Virology, Vol. 286, pp. 272, 2001) were infected with ΔG/SEAP/VSV G to prepare a large amount of ΔG/SEAP/VSV G. Further, CHO-K1 cells expressing both chimera protein consisting of ectodomain of protein E1 of HCV with C-terminal region of VSV glycoprotein and chimera protein consisting of ectodomain of protein E2 of HCV with C-terminal region of VSV glycoprotein(Above CHO-K1 cells are described as CHO cells transfected with both pEF-puro E1 and pEF neo E2 in Virology, Vol. 286, pp. 272, 2001)were infected with ΔG/SEAP/VSV G to produce a recombinant virus (hereinafter to be referred to as (G/SEAP/HCV E1E2) in a large amount, which was covered with HCV E1 and E2 proteins and had a virus genome containing the SEAP gene, which was used as the pseudotype virus in the following Example.

### Experimental Example 1: Effect of test substance on cell infection with HCV pseudotype virus

### (Materials)

cell: HepG2
virus: ΔG/SEAP/HCV E1E2
reagent: D-MEM high glucose (Nikken Bio Medical Laboratory), FBS (CTT), Reporter Assay Kit-SEAP-(TOYOBO SAK-101)

### (Method)

HepG2 cells were seeded at 0.8x104 cells/well/80 µl/96 wp, and cultured overnight (O/N) at 37(C, 5% CO2. A test substance, which had been dissolved in DMSO and serially diluted, was further diluted 100-fold with a medium and added thereto by 10 µl.

After incubation at 37°C, 5%CO₂ for 1 hr, ΔG/SEAP/HCV E1E2 was added (1x10¹⁰ genome/10 µl), and the cells were cultured at 37°C, 5% CO₂ for 24 hr. To measure the amount of SEAP secreted from the virus-infected cells into the culture supernatant, the medium (10 µl) was transferred to Corning® 96 Well Black Round Bottom Polystyrene Not Treated Microplate, 25 per Bag, without Lid, Non-Sterile (Cat. #3792) and then Endogenous AP inhibitor liquid (10 µl) in the above-mentioned Reporter Assay Kit was added and allowed to react at 37°C for 30 min.

Then, a chemiluminescence reagent (Lumi-Phos(R) Plus) (80 µl) in the above-mentioned Reporter Assay Kit was added and, after reaction at 37°C for 30 min, the luminescence was measured with a luminometer. The IC₅₀ value of each test substance was determined from the obtained values where the amount of SEAP of the cells cultured with a compound-free medium was 100%. The results are shown in the following Tables.

In the table, "Ex. No." shows Example numbers, "++" shows an IC50 value of less than 100 nM, "+" shows an IC50 value of 100 nM to 10000 nM, and a blank shows no measurement.

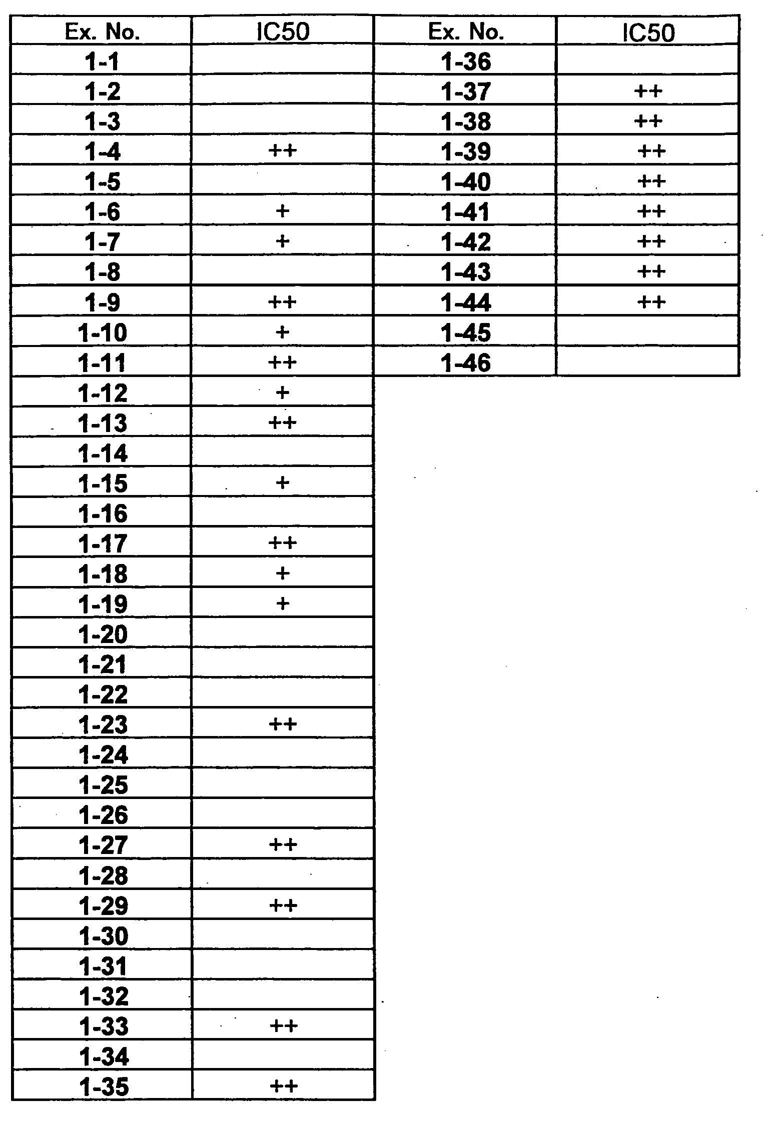

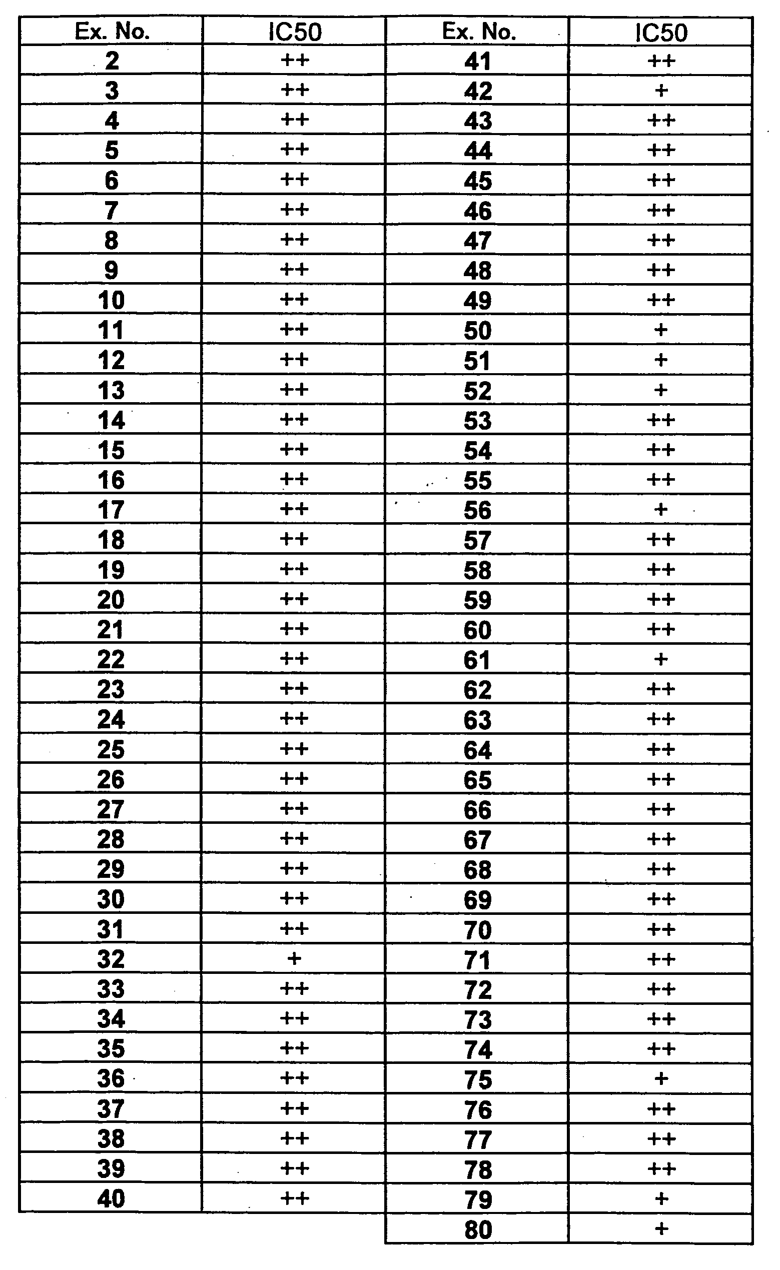

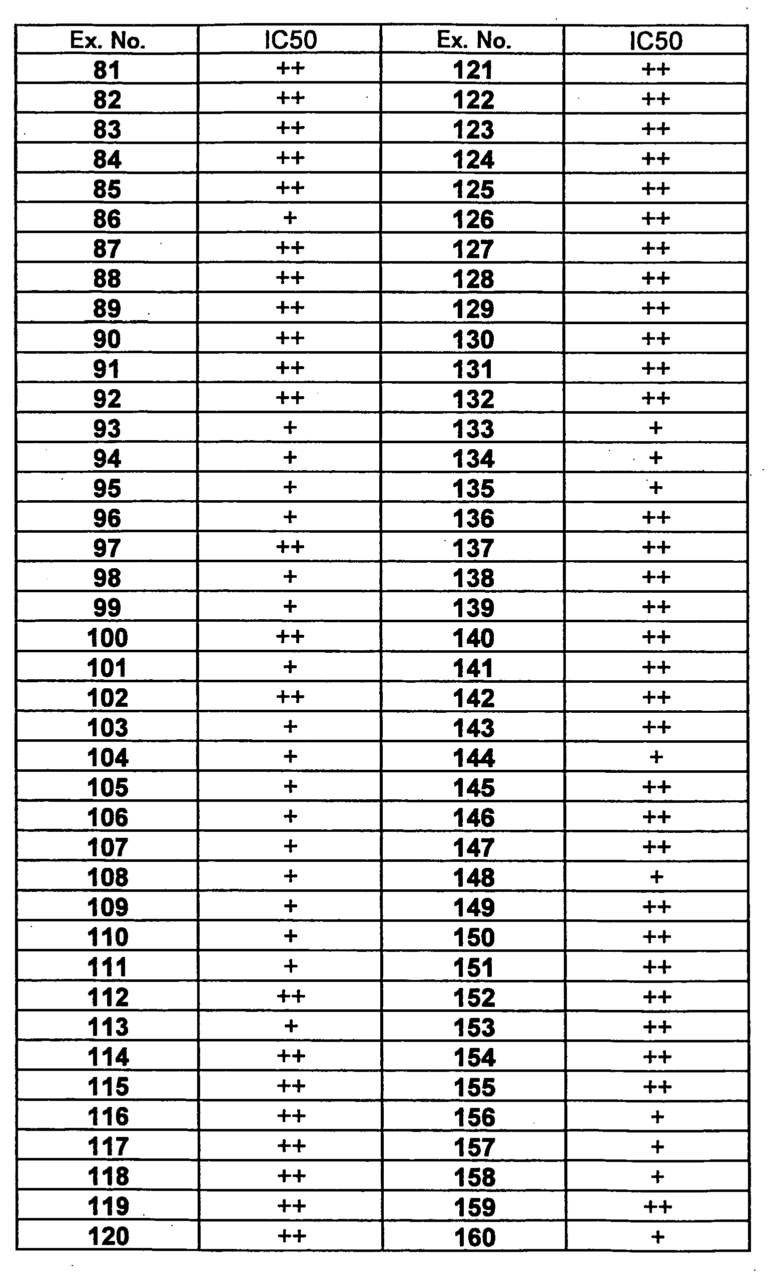

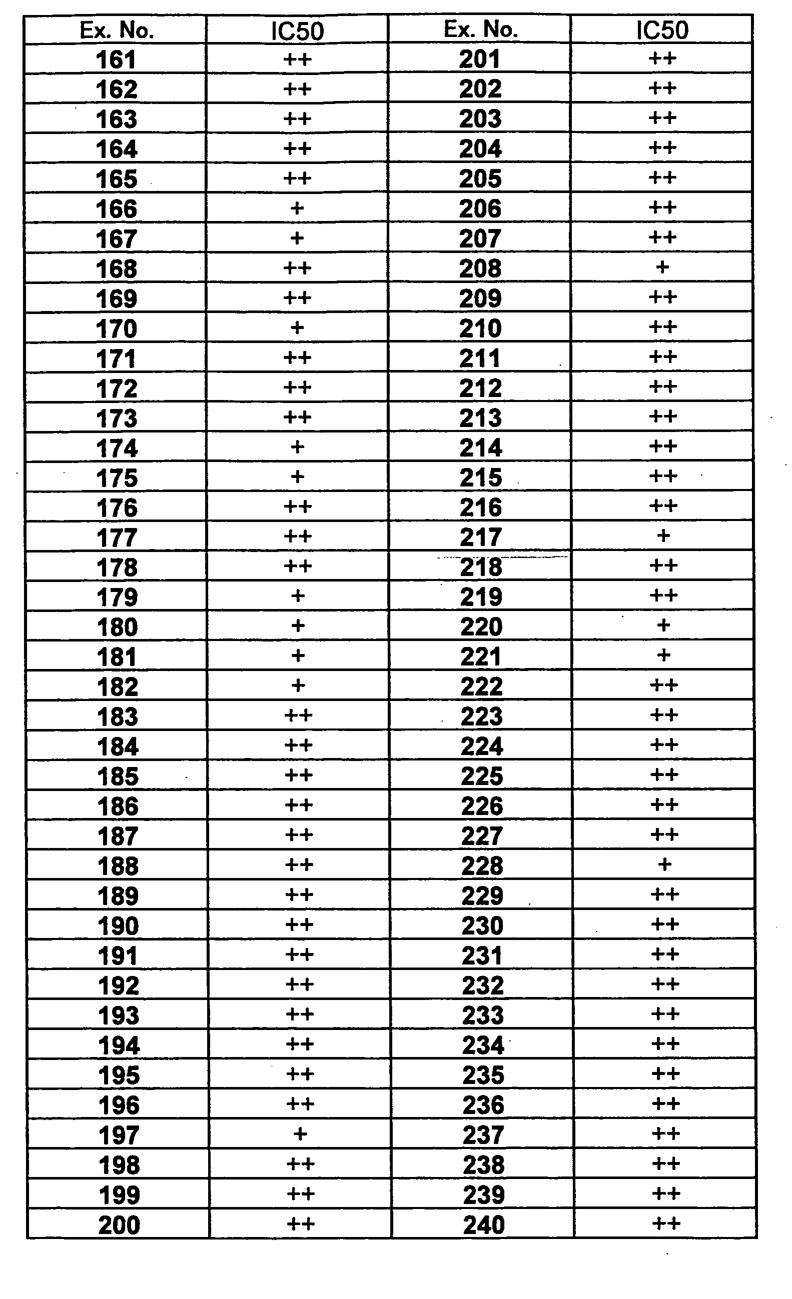

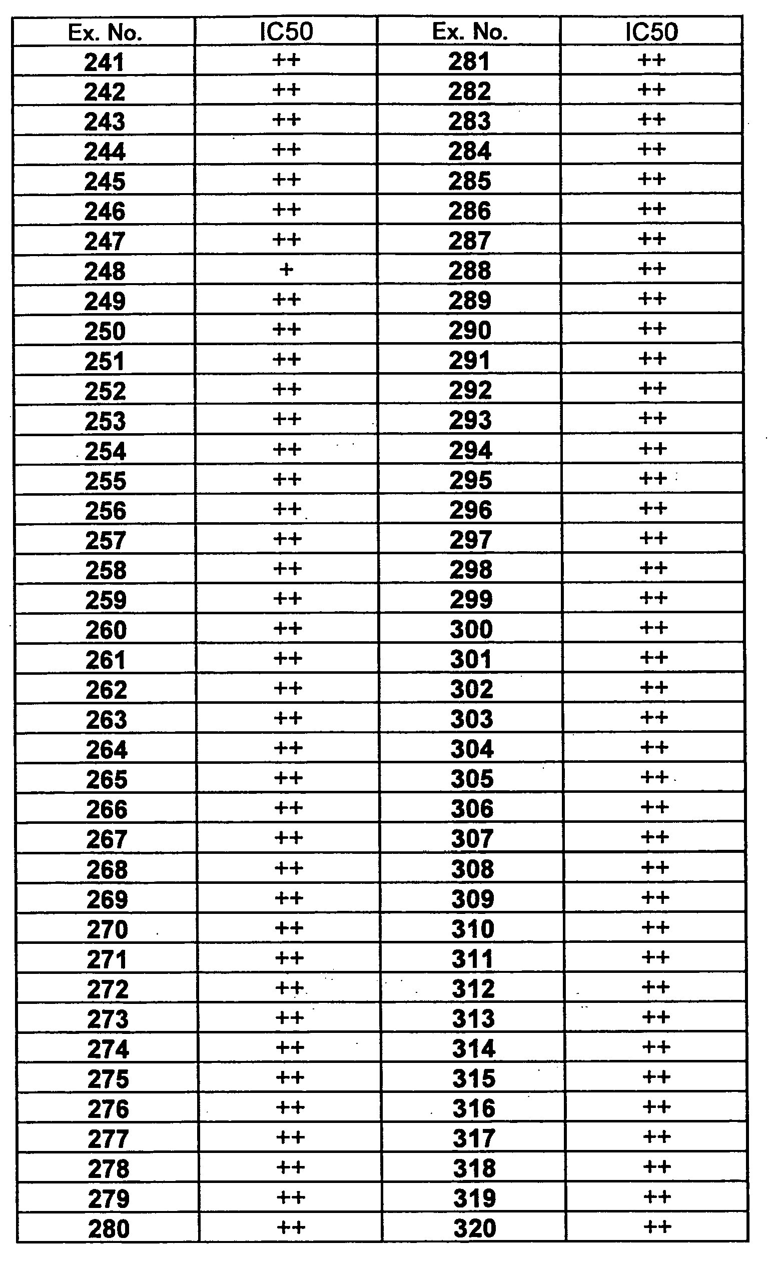

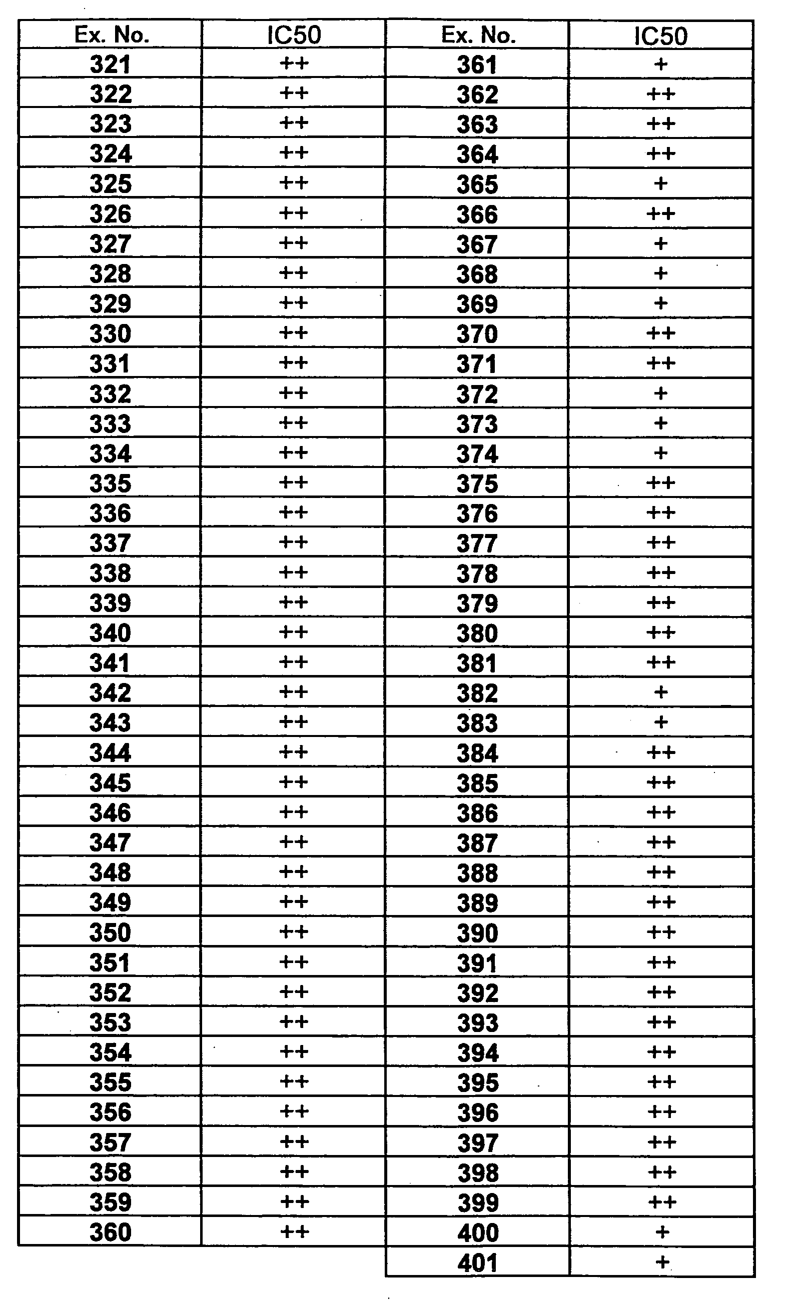

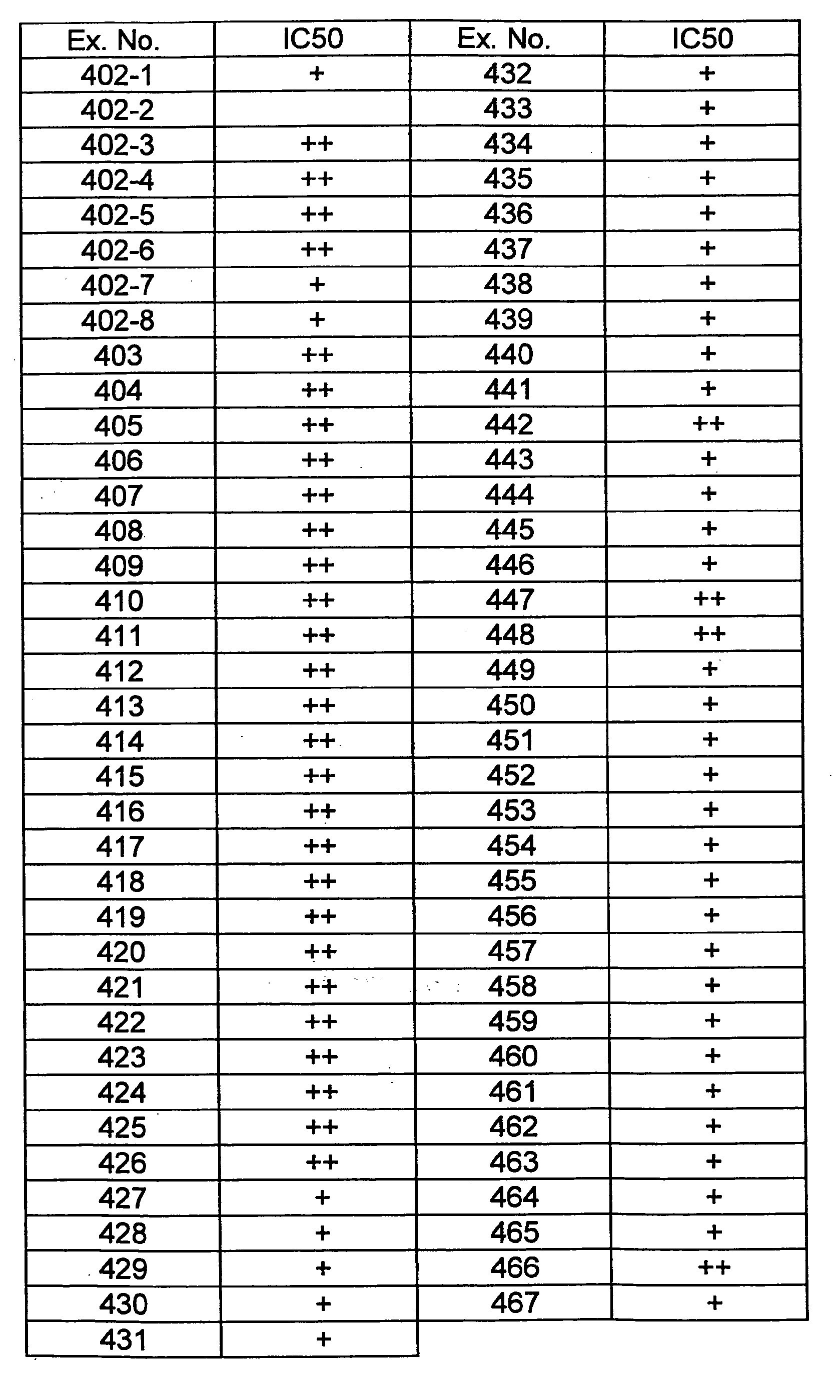

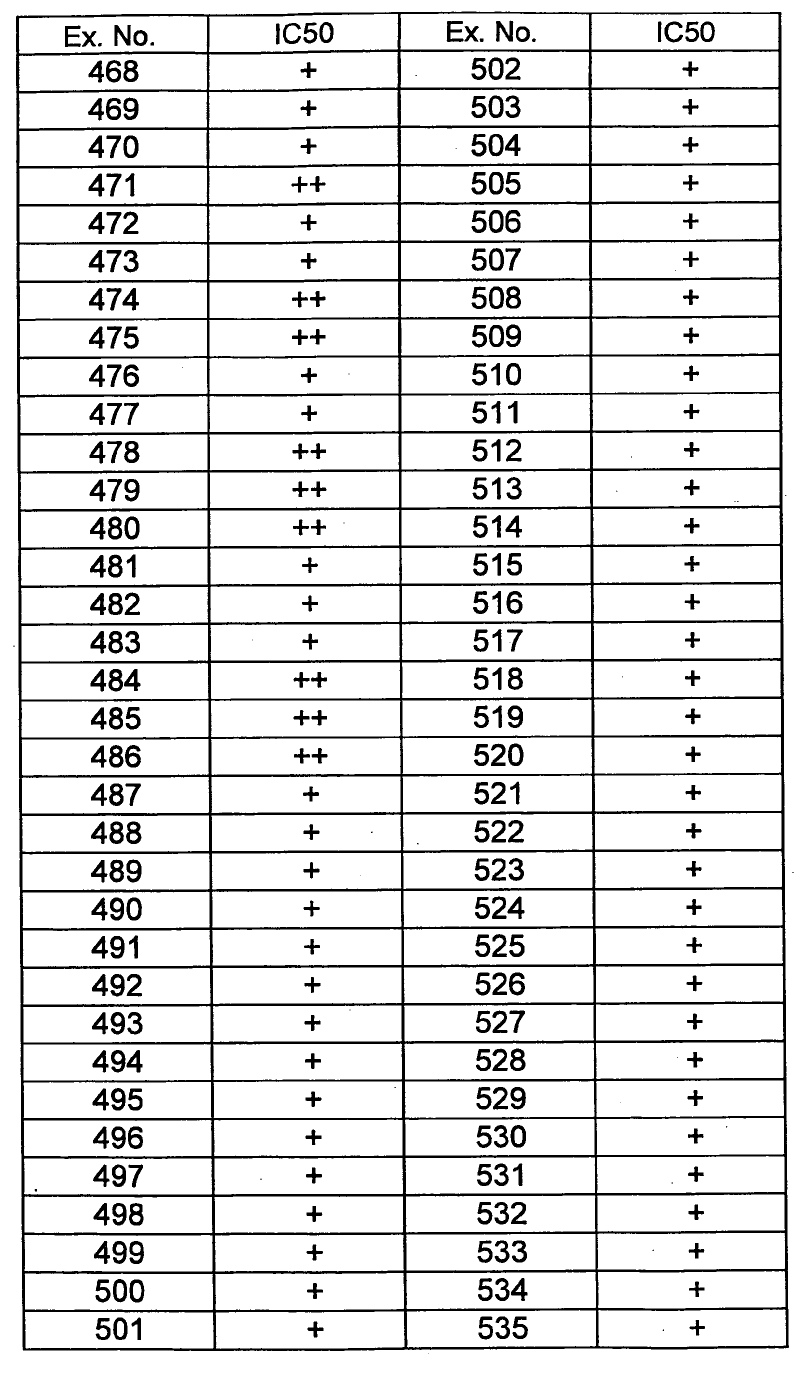

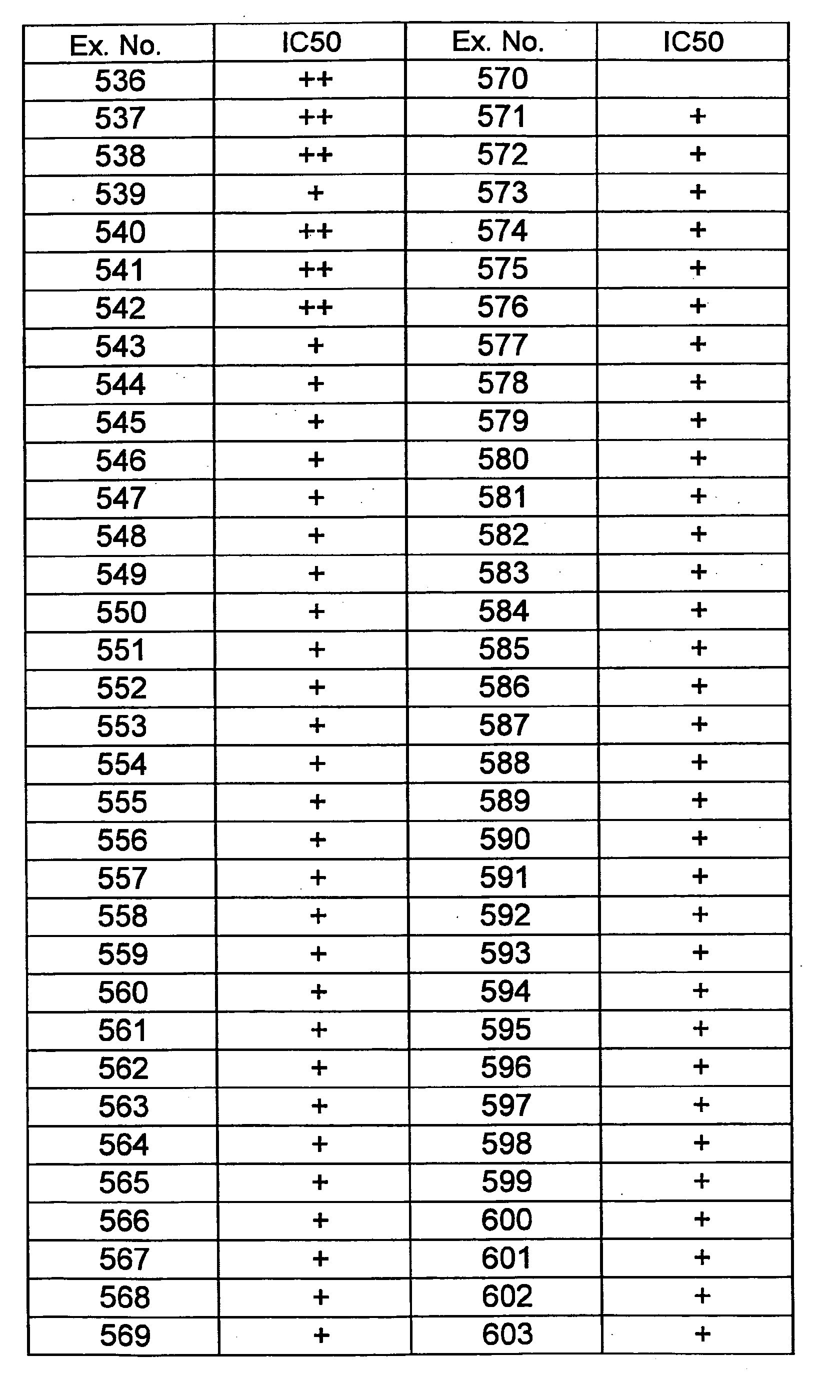

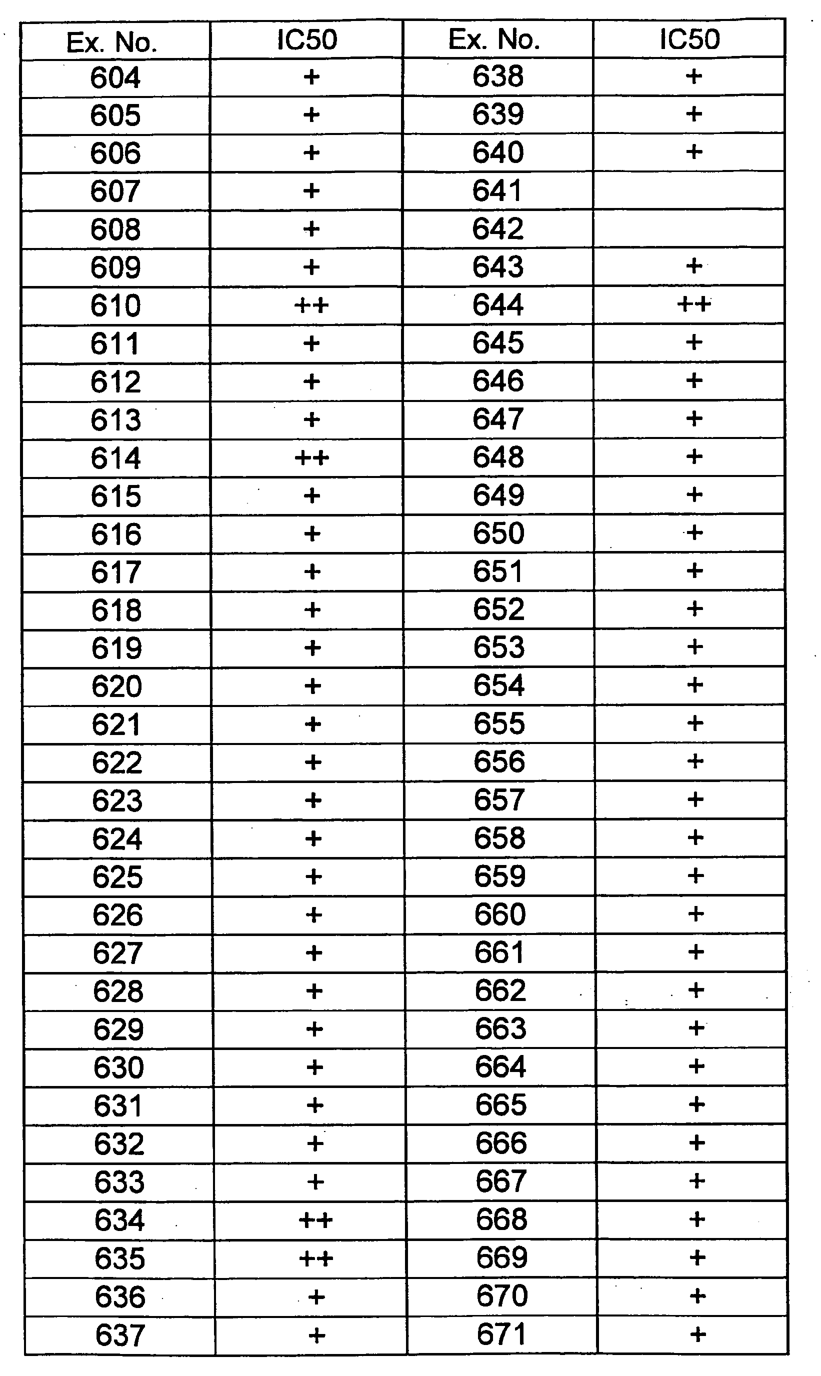

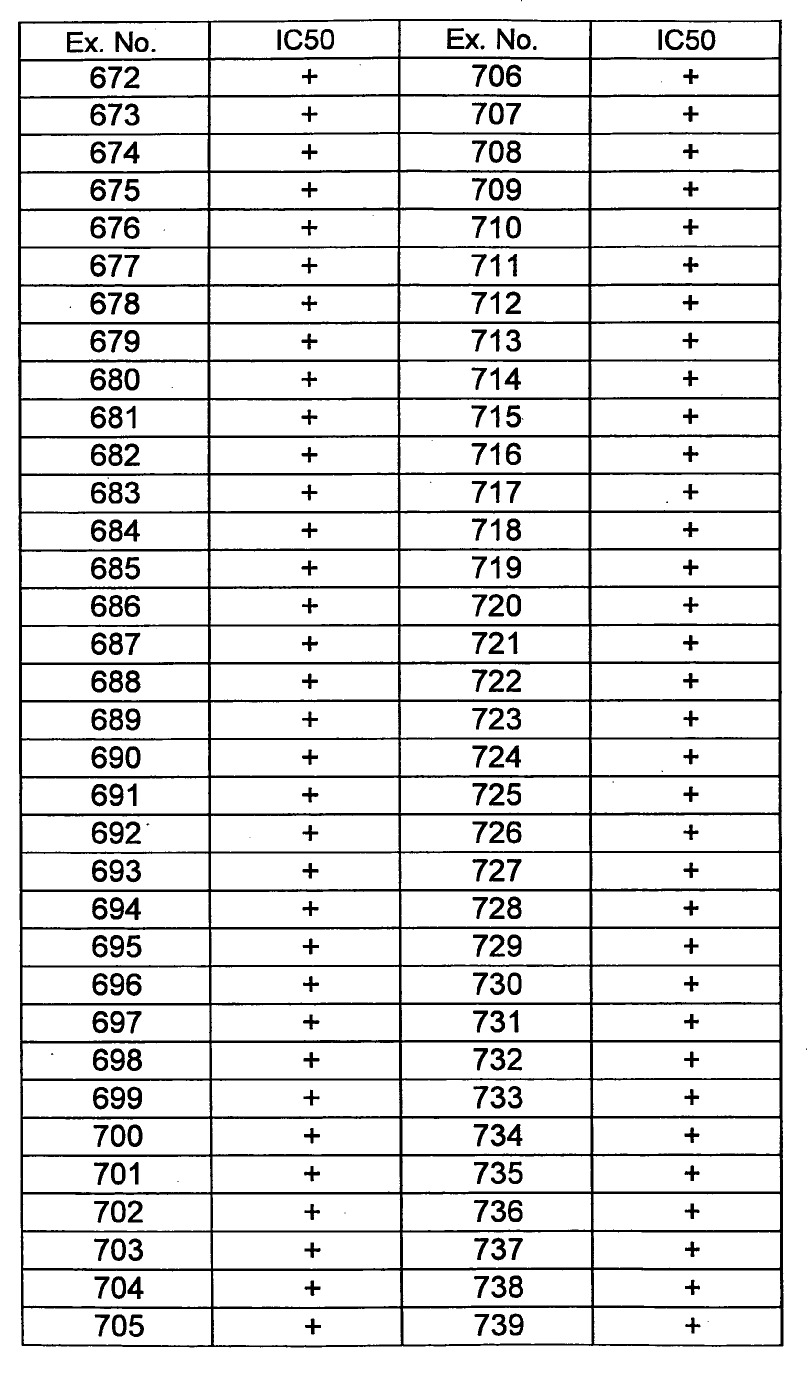

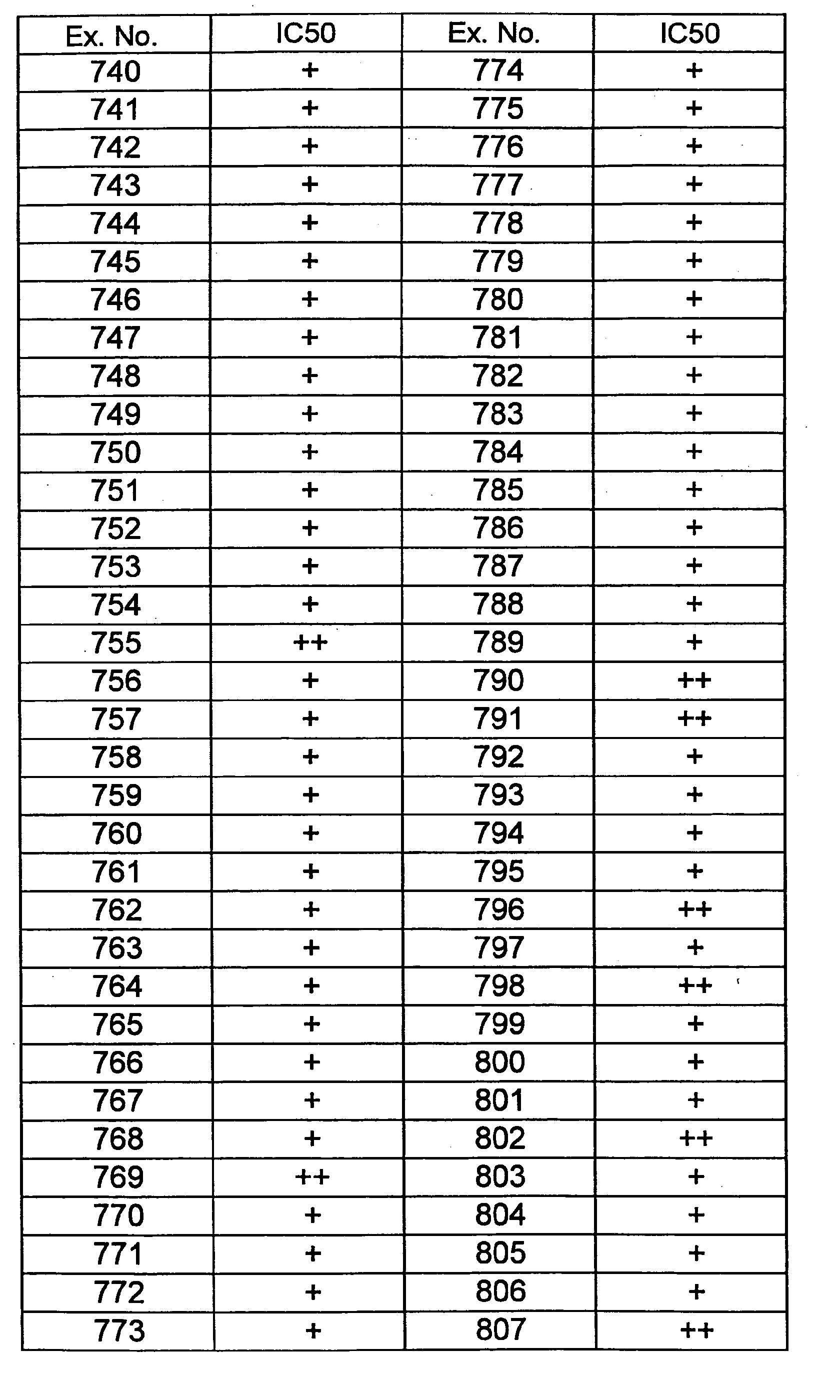

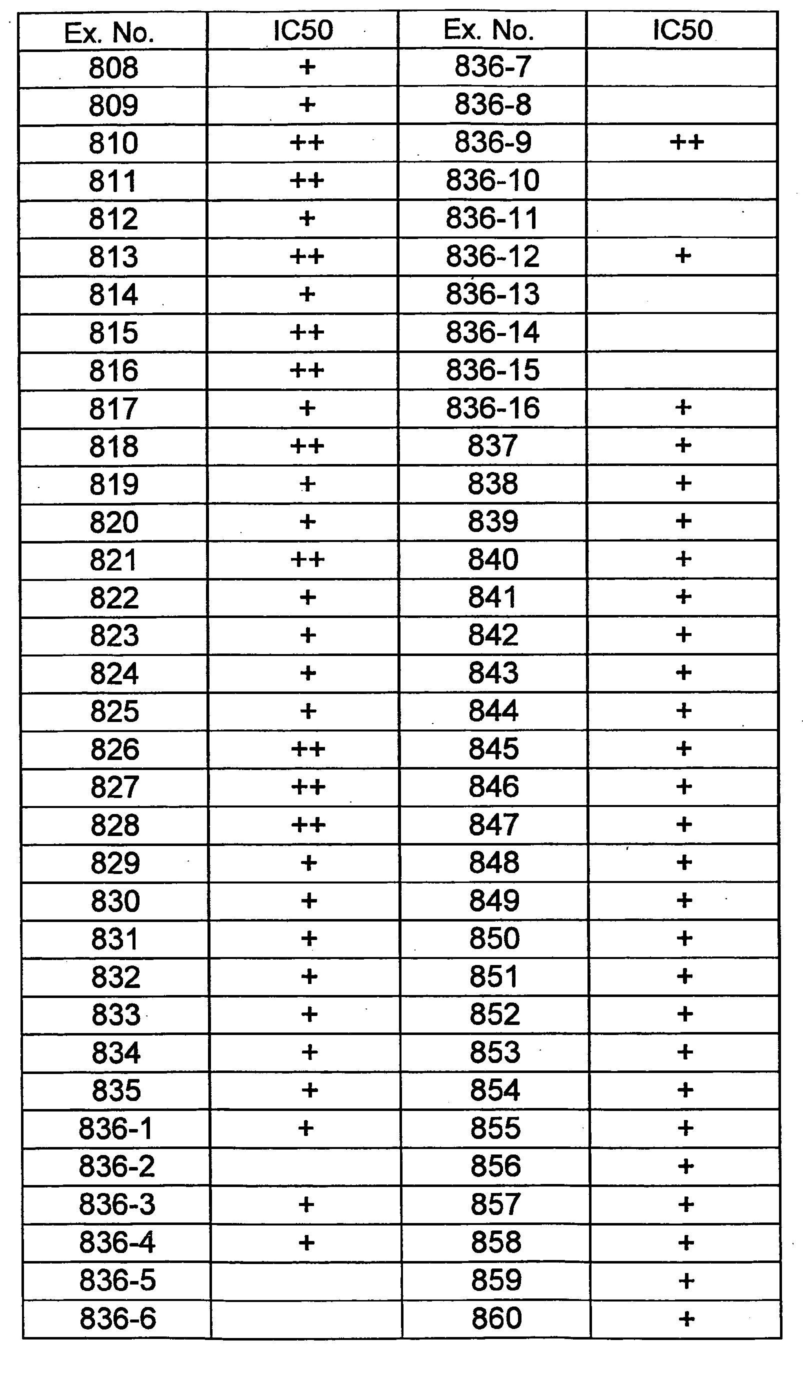

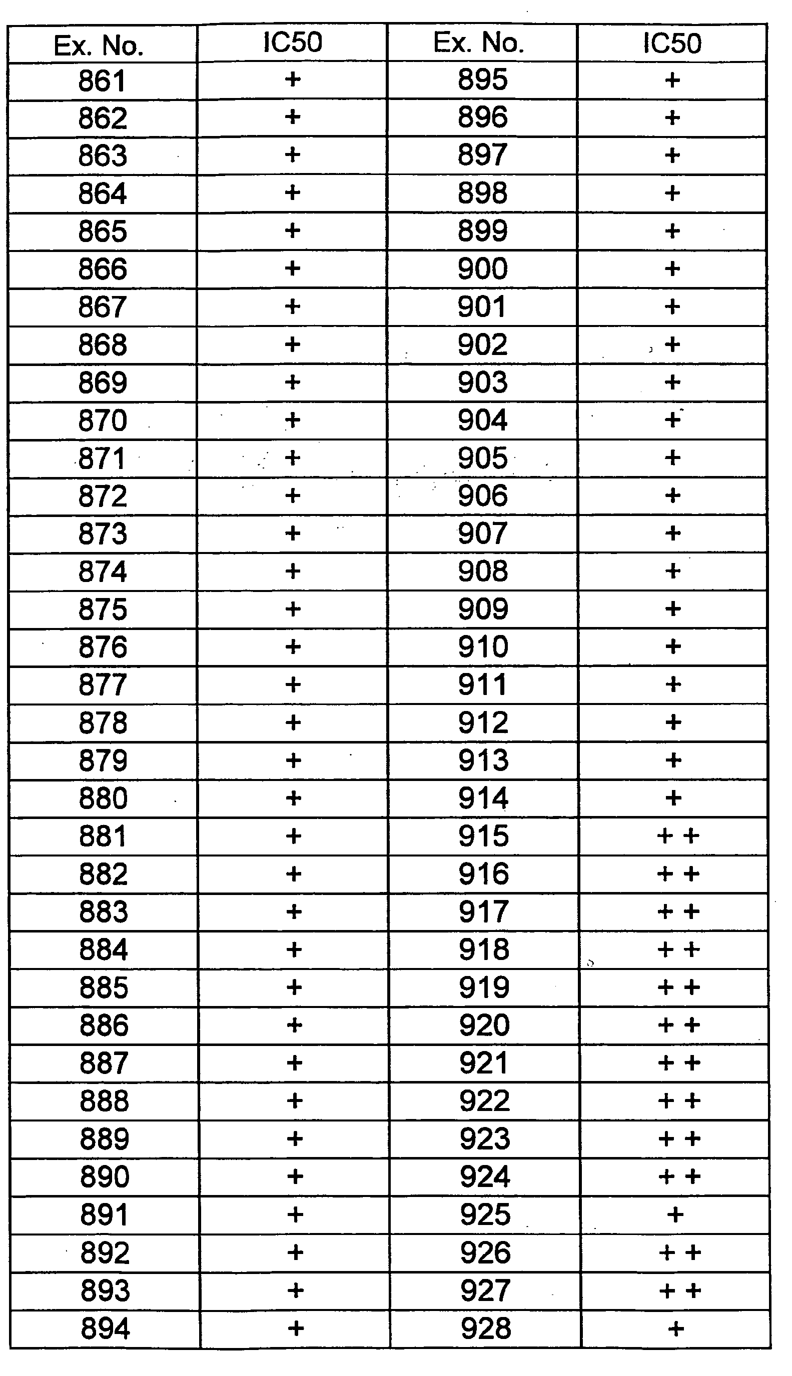

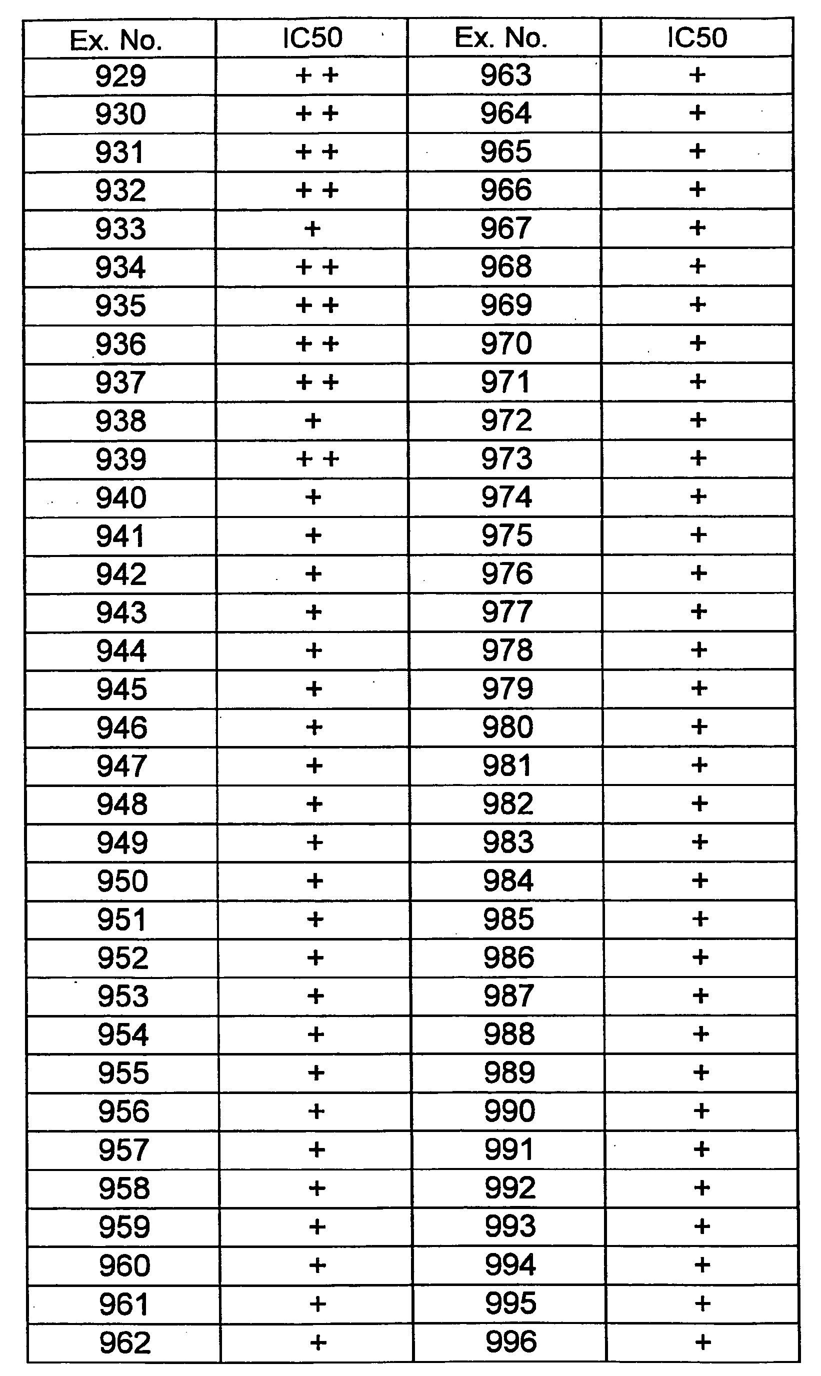

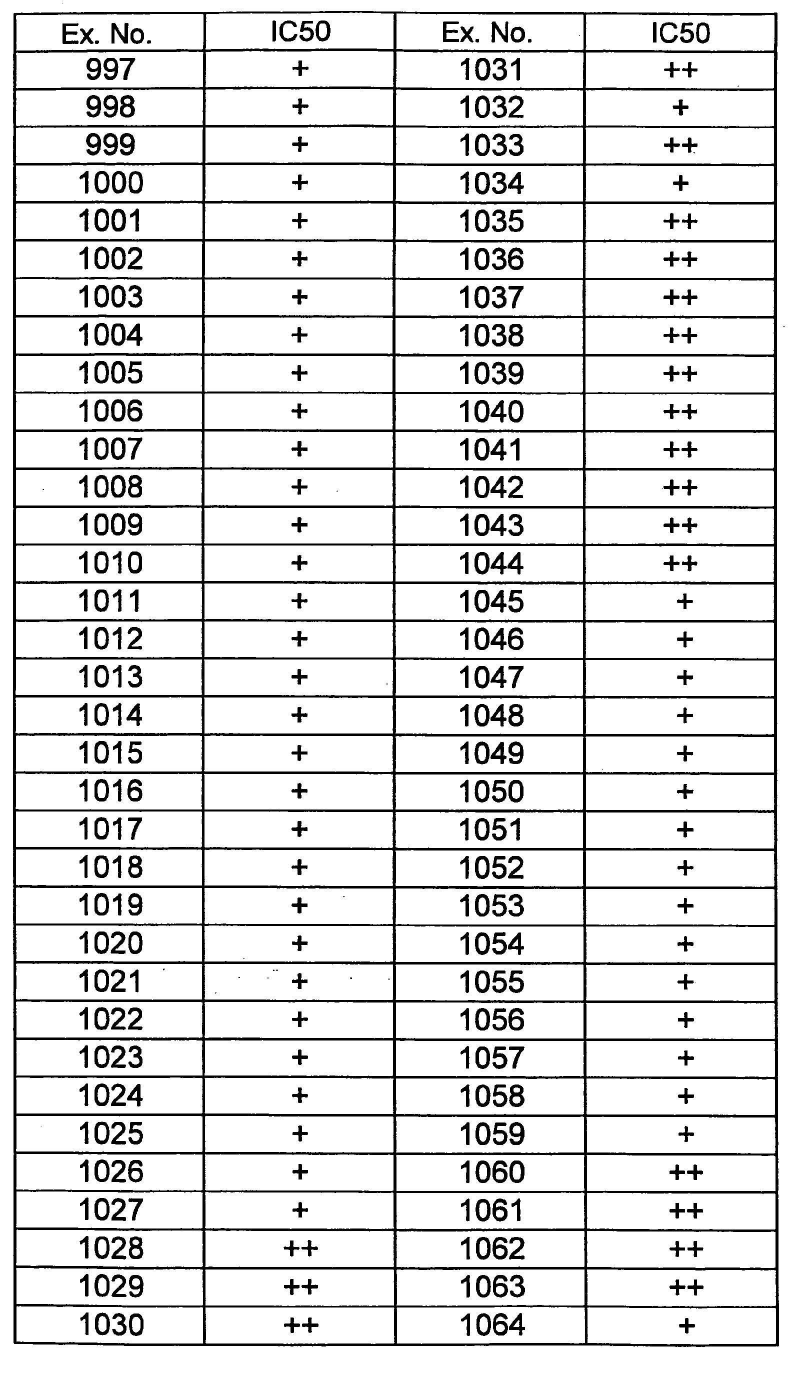

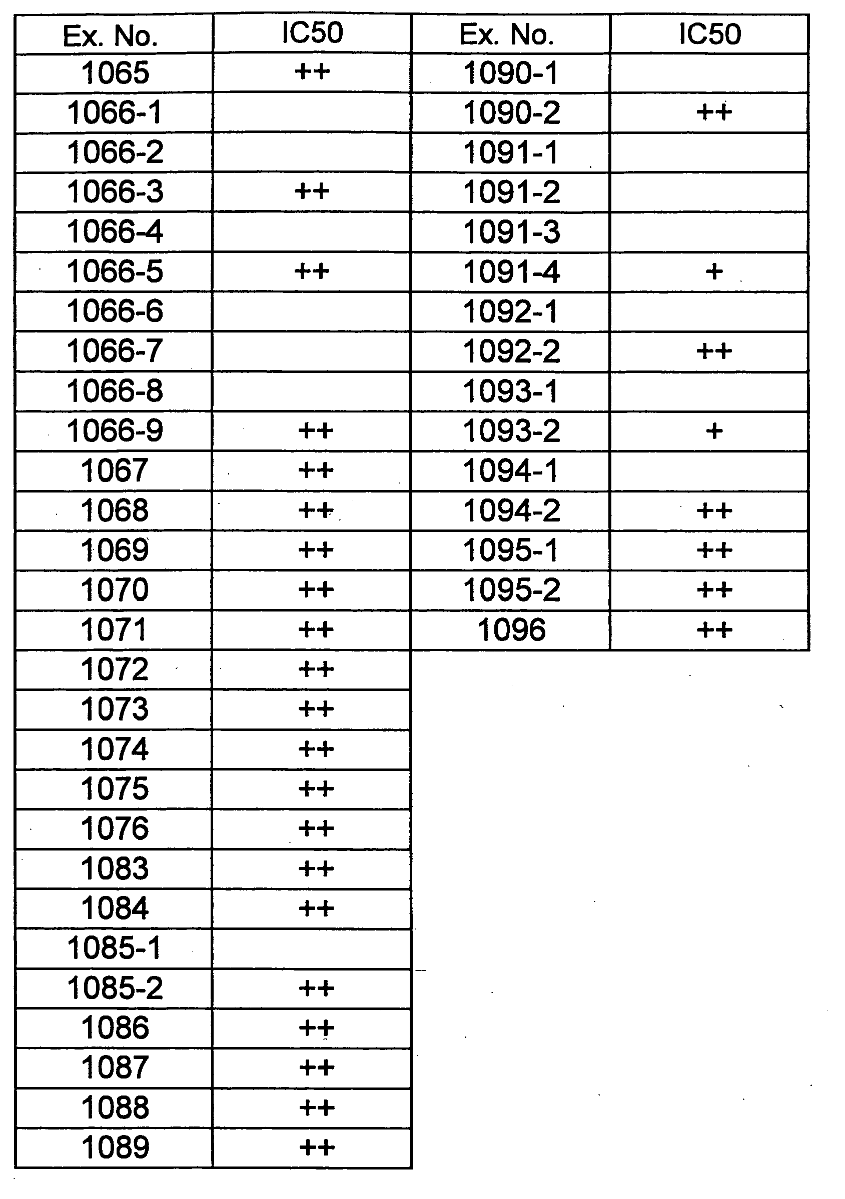

Formulation Example is given in the following. This example is merely for the purpose of exemplification and does not limit the invention.

### Formulation Example

(a) compound of Example 1-9 10 g
(b) lactose 50 g
(c) corn starch 15 g
(d) sodium carboxymethylcellulose 44 g
(e) magnesium stearate 1 g

The entire amounts of (a), (b) and (c) and 30 g of (d) are kneaded with water, dried in vacuo and granulated. The obtained granules are mixed with 14 g of (d) and 1 g of (e) and processed into tablets with a tableting machine to give 1000 tablets each containing 10 mg of (a).

### Industrial Applicability

According to the present invention, a novel heterocyclic compound having HCV entry inhibitory (inhibition of HCV cell infection) activity or a pharmaceutically acceptable salt thereof, and a pharmaceutical composition containing the same, and use thereof are provided. The compound is useful, for example, as an anti-hepatitis virus agent and therapeutic agent for hepatitis C, as well as a concomitant agent combining with at least one pharmaceutical agent selected from other antiviral agent, anti-inflammatory agent and immunity enhancing agent and the like.

This application is based on patent application Nos. 2005-336429 and 2006-092163 filed in Japan, and application Nos. 60/742,308 and 60/790,837 filed in the United States, the contents of which are incorporated in full herein by this reference. The references cited herein, including patents and patent applications, are hereby incorporated in their entireties by reference, to the extent that they have been disclosed herein.

## Claims

1. Use of a heterocyclic compound represented by the following formula [1] or a pharmaceutically acceptable salt thereof for the production of a therapeutic agent for hepatitis C: 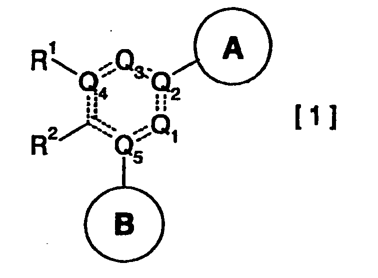 wherein
Q₁ is:
(1) -N=,
(2) -C(R⁵⁰)= wherein R⁵⁰ is a hydrogen atom, a hydroxyl group, a C₁₋₆ alkyl group or a C₁₋₆ alkoxy group, or
(3) -CO-;
Q₂ is:
(1) -N-, or
(2) -C=;
Q₃ is:
(1) -N=,
(2) -C(R⁴⁹)= wherein R⁴⁹ is a hydrogen atom, a hydroxyl group, a C₁₋₆ alkyl group or a C₁₋₆ alkoxy group, or
(3) -CO-;
Q₄ is:
(1) -N-,
(2) -C=, or
(3) -CH-;
Q₅ is:
(1) -N-, or
(2) -C=;
R¹ is:
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₁-R¹⁰¹
wherein
J₁ is
(1) -N(R¹⁰²)- wherein R¹⁰² is a hydrogen atom or a C₁₋₆ alkyl group,
(2) -O-,
(3) -S-,
(4) -CO-,
(5) -CO-O-,
(6) -O-CO-,
(7) -CO-N(R¹⁰²)- wherein R¹⁰² is as defined above, or
(8) -SO₂-N(R¹⁰²)- wherein R¹⁰² is as defined above,
R¹⁰¹ is
(1) a hydrogen atom,
(2) -L₂-J₂-R¹⁰³
wherein
L₂ is
(1) a bond, or
(2) a C₁₋₆ alkylene group,
J₂ is
(1) -N(R¹⁰⁴)- wherein R¹⁰⁴ is a hydrogen atom or a C₁₋₆ alkyl group,
(2) -O-,
(3) -S-,
(4) -CO-,
(5) -SO₂-,
(6) -CO-O-,
(7) -CO-N(R¹⁰⁴)- wherein R¹⁰⁴ is as defined above, or
(8) -SO₂-N(R¹⁰⁴) wherein R¹⁰⁴ is as defined above,
R¹⁰³ is
(1) a hydrogen atom,
(2) a C₁₋₆ alkyl group (said C₁₋₆ alkyl group is optionally substituted by 1 to 3 substituents selected from Group F),
(3) a C₁₋₆ alkyl-carbonyl group (said C₁₋₆ alkyl-carbonyl group is optionally substituted by 1 to 3 substituents selected from Group F), or
(4) a C₁₋₆ alkylsulfonyl group (said C₁₋₆ alkylsulfonyl group is optionally substituted by 1 to 3 substituents selected from Group F),
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹⁰¹ and R¹⁰² may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(7) a C₆₋₁₄ aryl group (said C₆₋₁₄ aryl group is optionally substituted by 1 to 5 substituents selected from Group E), or
(8) a heteroaryl group (said heteroaryl group is optionally substituted by 1 to 5 substituents selected from Group E);
R² is:
(1) a hydrogen atom, or
(2) a C₁₋₆ alkyl group; or
R¹ and R² may form, together with Q₄ and the carbon atom to which they are bonded respectively: 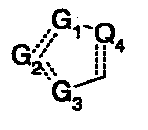 wherein
Q₄ is as defined above,
G₁ is
(1) -N(R³)-
R³ is
(1) a hydrogen atom,
(2) -L₃-J₃-R¹¹³
wherein
L₃ is
(1) a bond, or
(2) a C₁₋₆ alkylene group,
J₃ is
(1) -N(R114)- wherein R114 is a hydrogen atom or a C₁₋₆ alkyl group,
(2) -O-,
(3) -S-,
(4) -CO-,
(5) -SO₂-,
(6) -CO-O-,
(7) -CO-N(R114)- wherein R114 is as defined above, or
(8) -SO₂-N(R¹¹⁴)- wherein R¹¹⁴ is as defined above,
R¹¹³ is
(1) a hydrogen atom,
(2) -L₄-J₄-R¹¹⁵
wherein
L₄ is
(1) a bond, or
(2) a C₁₋₆ alkylene group,
J₄ is
(1) -N(R¹¹⁶)- wherein R¹¹⁶ is a hydrogen atom or a C₁₋₆ alkyl group,
(2) -O-,
(3) -S-,
(4) -CO-,
(5) -SO₂-,
(6) -CO-O-,
(7) -CO-N(R¹¹⁶)- wherein R¹¹⁶ is as defined above, or
(8) -SO₂-N(R¹¹⁶)- wherein R¹¹⁶ is as defined above,
R¹¹⁵ is
(1) a hydrogen atom,
(2) a C₁₋₆ alkyl group (said C₁₋₆ alkyl group is optionally substituted by 1 to 3 substituents selected from Group F),
(3) a C₁₋₆ alkyl-carbonyl group (said C₁₋₆ alkyl-carbonyl group is optionally substituted by 1 to 3 substituents selected from Group F), or
(4) a C₁₋₆ alkylsulfonyl group (said C₁₋₆ alkylsulfonyl group is optionally substituted by 1 to 3 substituents selected from Group F),
or
R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
(3) a C₁₋₆ alkyl group [said C₁₋₆ alkyl group is optionally substituted by 1 to 3 substituents selected from a halogen atom, a cyano group, a nitro group, C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹¹³ and R¹¹⁴ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(2) -C(R³)(R⁴) -
wherein
R³ is as defined above,
R⁴ is
(1) a hydrogen atom,
(2) a hydroxyl group,
(3) a C₁₋₆ alkyl group, or
(4) a C₁₋₆ alkoxy group,
(3) -C(R³)(R⁴)-C(R⁵)(R⁶) -
wherein
R³ and R⁴ are as defined above,
R⁵ and R⁶ are the same or different and each is
(1) a hydrogen atom,
(2) a hydroxyl group,
(3) a C₁₋₆ alkyl group, or
(4) a C₁₋₆ alkoxy group,
(4) -S-,
(5) -O-,
(6) -C(R³)=
wherein R³ is as defined above, or
(7) -N=;
G₂ is
(1) -N(R^{3'})-
wherein
R^{3'} is
(1) a hydrogen atom,
(2) a hydroxyl group,
(3) a C₁₋₆ alkyl group, or
(4) a C₁₋₆ alkoxy group,
(2) -C(R^{3'})(R^{4'})-
wherein
R^{3'} is as defined above,
R^{4'} is
(1) a hydrogen atom,
(2) a hydroxyl group,
(3) a C₁₋₆ alkyl group, or
(4) a C₁₋₆ alkoxy group,
(3) -C(R^{3'})(R^{4'})-C(R^{5'})(R^{6'})-
wherein
R^{3'} and R^{4'} are each as defined above,
R^{5'} and R^{6'} are the same or different and each is
(1) a hydrogen atom,
(2) a hydroxyl group,
(3) a C₁₋₆ alkyl group, or
(4) a C₁₋₆ alkoxy group,
(4) -S-,
(5) -O-,
(6) -C(R^{4'})=
wherein R^{4'} is as defined above,
(7) =C(R^{4'})-C(R^{6'})=
wherein R^{4'} and R^{6'} are each as defined above,
(8) =C(R^{4'})-N=
wherein R^{4'} is as defined above,
(9) =N-C(R^{6'})=
wherein R^{6'} is as defined above, or
(10) =N-N=;
G₃ is
(1) -N(R^{3''})-
wherein
R^{3''} is
(1) a hydrogen atom,
(2) a hydroxyl group,
(3) a C₁₋₆ alkyl group, or
(4) a C₁₋₆ alkoxy group,
(2) -C(R^{3''})(R^{4''})-
wherein
R^{3''} is as defined above,
R^{4''} is
(1) a hydrogen atom,
(2) a hydroxyl group,
(3) a C₁₋₆ alkyl group, or
(4) a C₁₋₆ alkoxy group],
(3) -C(R^{3''})(R^{4''})-C(R^{5''})(R^{6''})-
wherein
R^{3''} and R^{4''} are each as defined above,
R^{5''} and R^{6''} are the same or different and each is
(1) a hydrogen atom,
(2) a hydroxyl group,
(3) a C₁₋₆ alkyl group, or
(4) a C₁₋₆ alkoxy group,
(4) -S-,
(5) -O-,
(6) =C(R^{4''})-
wherein R^{4''} is as defined above, or
(7) =N-; 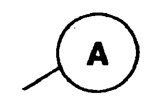 is 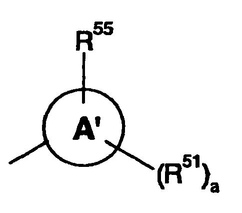
wherein
ring A' is
(1) a monocyclic aryl group, or
(2) a monocyclic heteroaryl group;
R⁵⁵ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₅-R¹²¹
wherein
J₅ is
(1) -N(R¹²²)- wherein R¹²² is a hydrogen atom or a C₁₋₆ alkyl group,
(2) -O-,
(3) -S-,
(4) -CO-,
(5) -CO-O-,
(6) -O-CO-,
(7) -CO-N(R¹²²) - wherein R¹²² is as defined above,
(8) -SO₂-N(R¹²²)- wherein R¹²² is as defined above,
(9) -N(R¹²³)-CO- wherein R¹²³ is a hydrogen atom or a C₁₋₆ alkyl group, or
(10) -N(R¹²³)-SO₂- wherein R¹²³ is as defined above,
R¹²¹ is
(1) a hydrogen atom,
(2) -L₆-J₆-R¹²⁴
wherein
L₆ is
(1) a bond, or
(2) a C₁₋₆ alkylene group,
J₆ is
(1) -N(R¹²⁵)- wherein R¹²⁵ is a hydrogen atom or a C₁₋₆ alkyl group,
(2) -O-,
(3) -S-,
(4) -CO-,
(5) -CO-O-,
(6) -O-CO-,
(7) -CO-N(R¹²⁵)- wherein R¹²⁵ is as defined above,
(8) -SO2-N(R¹²⁵)- wherein R¹²⁵ is as defined above,
(9) -N(R¹²⁶)-CO- wherein R¹²⁶ is a hydrogen atom or a C₁₋₆ alkyl group,
(10) -N(R¹²⁶)-SO₂- wherein R¹²⁶ is as defined above,
(11) -N(R¹²⁶)-CO-O- wherein R¹²⁶ is as defined above, or
(12) -N(R¹²⁶)-CO-N(R¹²⁵)- wherein R¹²⁵ and R¹²⁶ are as defined above,
R¹²⁴ is
(1) a hydrogen atom, or
(2) a C₁₋₆ alkyl group (said C₁₋₆ alkyl group is optionally substituted by 1 to 3 substituents selected from Group G, or
R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹²¹ and R¹²² may form, together with the nitrogen atom to which they are bonded, a nitrogen-containing 4-to 7-membered heterocyclic group, wherein said nitrogen-containing 4- to 7-membered heterocyclic group is optionally substituted by 1 to 5 substituents selected from
(1) Group F,
(2) a C₁₋₆ alkyl group (said C₁₋₆ alkyl group is optionally substituted by 1 to 3 substituents selected from Group F),
(3) a C₁₋₆ alkoxy group (said C₁₋₆ alkoxy group is optionally substituted by 1 to 3 substituents selected from Group F), and
(4) an oxo group,
(7) a monocyclic aryl group (said monocyclic aryl group is optionally substituted by 1 to 5 substituents selected from Group E),
(8) a monocyclic heteroaryl group (said monocyclic heteroaryl group is optionally substituted by 1 to 5 substituents selected from Group E),
(9) a C₃₋₈ cycloalkyl group (said C₃₋₈ cycloalkyl group is optionally substituted by 1 to 5 substituents selected from Group E), or
(10) a heterocycloalkyl group (said heterocycloalkyl group is optionally substituted by 1 to 5 substituents selected from Group E);
a is 1 or 2;
R⁵¹ are the same or different and each is
(1) a hydrogen atom,
(2) a halogen atom,
(3) a cyano group,
(4) a nitro group,
(5) a hydroxyl group,
(6) a mercapto group,
(7) a formyl group,
(8) a carboxyl group,
(9) a C₁₋₆ alkyl group (said C₁₋₆ alkyl group is optionally substituted by 1 to 3 substituents selected from Group G),
(10) a C₂₋₆ alkenyl group (said C₂₋₆ alkenyl group is optionally substituted by 1 to 3 substituents selected from Group G),
(11) a C₂₋₆ alkynyl group (said C₂₋₆ alkynyl group is optionally substituted by 1 to 3 substituents selected from Group G),
(12) a C₁₋₆ alkoxy group (said C₁₋₆ alkoxy group is optionally substituted by 1 to 3 substituents selected from Group G),
(13) a C₁₋₆ alkylthio group (said C₁₋₆ alkylthio group is optionally substituted by 1 to 3 substituents selected from Group G),
(14) a C₁₋₆ alkylsulfinyl group (said C₁₋₆ alkylsulfinyl group is optionally substituted by 1 to 3 substituents selected from Group G),
(15) a C₁₋₆ alkylsulfonyl group (said C₁₋₆ alkylsulfonyl group is optionally substituted by 1 to 3 substituents selected from Group G),
(16) a C₁₋₆ alkyl-carbonyl group (said C₁₋₆ alkyl-carbonyl group is optionally substituted by 1 to 3 substituents selected from Group G),
(17) a C₁₋₆ alkoxy-carbonyl group (said C₁₋₆ alkoxy-carbonyl group is optionally substituted by 1 to 3 substituents selected from Group G),
(18) -N(R⁵⁷)(R⁵⁸)
wherein
R⁵⁷ and R⁵⁸ are the same or different and each is
(1) a hydrogen atom,
(2) a C₁₋₆ alkyl group (said C₁₋₆ alkyl group is optionally substituted by 1 to 3 substituents selected from Group G),
(3) a C₁₋₆ alkylsulfonyl group (said C₁₋₆ alkylsulfonyl group is optionally substituted by 1 to 3 substituents selected from Group G),
(4) a C₁₋₆ alkyl-carbonyl group (said C₁₋₆ alkyl-carbonyl group is optionally substituted by 1 to 3 substituents selected from Group G),
(5) a C₁₋₆ alkoxy-carbonyl group (said C₁₋₆ alkoxy-carbonyl group is optionally substituted by 1 to 3 substituents selected from Group G),
(6) a carbamoyl group,
(7) a C₁₋₆ alkylamino-carbonyl group (said C₁₋₆ alkylamino-carbonyl group is optionally substituted by 1 to 3 substituents selected from Group G),
(8) a di(C₁₋₆ alkyl)amino-carbonyl group (said di(C₁₋₆ alkyl)amino-carbonyl group is optionally substituted by 1 to 3 substituents selected from Group G),
(9) a hydroxyl group,
(10) a C₁₋₆ alkoxy group (said C₁₋₆ alkoxy group is optionally substituted by 1 to 3 substituents selected from Group G),
(11) an amino group,
(12) a C₁₋₆ alkylamino group (said C₁₋₆ alkylamino group is optionally substituted by 1 to 3 substituents selected from Group G),
(13) a di(C₁₋₆ alkyl) amino group (said di(C₁₋₆ alkyl)amino group is optionally substituted by 1 to 3 substituents selected from Group G), or
(14) a C₁₋₆ alkyl-carbonyl-amino group (said C₁₋₆ alkyl-carbonyl-amino group is optionally substituted by 1 to 3 substituents selected from Group G), or
R⁵⁷ and R⁵⁸ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(19) -CO-N(R⁵⁷)(R⁵⁸) wherein R⁵⁷ and R⁵⁸ are as defined above,
(20) -C(R⁵⁹)=N(R⁶⁰)
wherein
R⁵⁹ is
(1) a hydrogen atom,
(2) a C₁₋₆ alkyl group, or
(3) an amino group,
R⁶⁰ is
(1) a hydroxyl group,
(2) a C₁₋₆ alkoxy group (said C₁₋₆ alkoxy group is optionally substituted by 1 to 3 substituents selected from Group G),
(3) a C₁₋₆ alkyl-carbonyl-oxy group (said C₁₋₆ alkyl-carbonyl-oxy group is optionally substituted by 1 to 3 substituents selected from Group G), or
(4) -N(R⁶¹)(R⁶²) wherein R⁶¹ and R⁶² are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
(21) a monocyclic aryl group (said monocyclic aryl group is optionally substituted by 1 to 5 substituents selected from Group E),
(22) a monocyclic heteroaryl group (said monocyclic heteroaryl group is optionally substituted by 1 to 5 substituents selected from Group E), or
(23) a heterocycloalkyl group (said heterocycloalkyl group is optionally substituted by 1 to 5 substituents selected from Group E), or
two adjacent R⁵¹s may form, together with the ring to which they are bonded, a fused ring (said fused ring is optionally further substituted by one substituent selected from Group E); 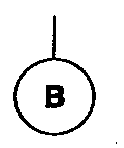 is 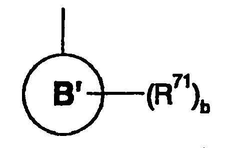 wherein
ring B' is
(1) a monocyclic aryl group,
(2) a monocyclic heteroaryl group,
(3) a C₃₋₈ cycloalkyl group, or
(4) a C₃₋₈ cycloalkenyl group;
b is an integer of 1-3;
R⁷¹ are the same or different and each is
(1) a hydrogen atom,
(2) a halogen atom,
(3) a cyano group,
(4) a nitro group,
(5) a hydroxyl group,
(6) a mercapto group,
(7) a formyl group,
(8) a carboxyl group,
(9) a C₁₋₆ alkyl group (said C₁₋₆ alkyl group is optionally substituted by 1 to 3 substituents selected from Group G),
(10) a C₂₋₆ alkenyl group (said C₂₋₆ alkenyl group is optionally substituted by 1 to 3 substituents selected from Group G),
(11) a C₂₋₆ alkynyl group (said C₂₋₆ alkynyl group is optionally substituted by 1 to 3 substituents selected from Group G),
(12) a C₁₋₆ alkoxy group (said C₁₋₆ alkoxy group is optionally substituted by 1 to 3 substituents selected from Group G),
(13) a C₁₋₆ alkylthio group (said C₁₋₆ alkylthio group is optionally substituted by 1 to 3 substituents selected from Group G),
(14) a C₁₋₆ alkylsulfinyl group (said C₁₋₆ alkylsulfinyl group is optionally substituted by 1 to 3 substituents selected from Group G),
(15) a C₁₋₆ alkylsulfonyl group (said C₁₋₆ alkylsulfonyl group is optionally substituted by 1 to 3 substituents selected from Group G),
(16) a C₁₋₆ alkyl-carbonyl group (said C₁₋₆ alkyl-carbonyl group is optionally substituted by 1 to 3 substituents selected from Group G),
(17) a C₁₋₆ alkoxy-carbonyl group (said C₁₋₆ alkoxy-carbonyl group is optionally substituted by 1 to 3 substituents selected from Group G),
(18) -N(R⁷²)(R⁷³)
wherein
R⁷² and R⁷³ are the same or different and each is
(1) a hydrogen atom,
(2) a C₁₋₆ alkyl group (said C₁₋₆ alkyl group is optionally substituted by 1 to 3 substituents selected from Group G),
(3) a C₁₋₆ alkylsulfonyl group (said C₁₋₆ alkylsulfonyl group is optionally substituted by 1 to 3 substituents selected from Group G),
(4) a C₁₋₆ alkyl-carbonyl group (said C₁₋₆ alkyl-carbonyl group is optionally substituted by 1 to 3 substituents selected from Group G),
(5) a C₁₋₆ alkoxy-carbonyl group (said C₁₋₆ alkoxy-carbonyl group is optionally substituted by 1 to 3 substituents selected from Group G),
(6) a carbamoyl group,
(7) a C₁₋₆ alkylamino-carbonyl group (said C₁₋₆ alkylamino-carbonyl group is optionally substituted by 1 to 3 substituents selected from Group G),
(8) a di(C₁₋₆ alkyl)amino-carbonyl group (said di(C₁₋₆ alkyl)amino-carbonyl group is optionally substituted by 1 to 3 substituents selected from Group G),
(9) a hydroxyl group,
(10) a C₁₋₆ alkoxy group (said C₁₋₆ alkoxy group is optionally substituted by 1 to 3 substituents selected from Group G),
(11) an amino group,
(12) a C₁₋₆ alkylamino group (said C₁₋₆ alkylamino group is optionally substituted by 1 to 3 substituents selected from Group G),
(13) a di(C₁₋₆ alkyl)amino group (said di(C₁₋₆ alkyl)amino group is optionally substituted by 1 to 3 substituents selected from Group G), or
(14) a C₁₋₆ alkyl-carbonyl-amino group (said C₁₋₆ alkyl-carbonyl-amino group is optionally substituted by 1 to 3 substituents selected from Group G), or
R⁷² and R⁷³ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(19) -CO-N(R⁷²)(R⁷³) wherein R⁷² and R⁷³ are as defined above,
(20) -C(R⁷⁴)=N(R⁷⁵)
wherein
R⁷⁴ is
(1) a hydrogen atom,
(2) a C₁₋₆ alkyl group, or
(3) an amino group,
R⁷⁵ is
(1) a hydroxyl group,
(2) a C₁₋₆ alkoxy group (said C₁₋₆ alkoxy group is optionally substituted by 1 to 3 substituents selected from Group G),
(3) a C₁₋₆ alkyl-carbonyl-oxy group (said C₁₋₆ alkyl-carbonyl-oxy group is optionally substituted by 1 to 3 substituents selected from Group G), or
(4) -N(R⁷⁶)(R⁷⁷) wherein R⁷⁶ and R⁷⁷ are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
(21) a monocyclic aryl group (said monocyclic aryl group is optionally substituted by 1 to 5 substituents selected from Group E),
(22) a monocyclic heteroaryl group (said monocyclic heteroaryl group is optionally substituted by 1 to 5 substituents selected from Group E), or
(23) a heterocycloalkyl group (said heterocycloalkyl group is optionally substituted by 1 to 5 substituents selected from Group E), or
two adjacent R⁷¹s may form, together with the ring to which they are bonded, a fused ring (said fused ring is optionally further substituted by one substituent selected from Group E):
wherein
Group E consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkyl group (said C₁₋₆ alkyl group is optionally substituted by 1 to 3 substituents selected from Group G),
(12) a C₂₋₆ alkenyl group (said C₂₋₆ alkenyl group is optionally substituted by 1 to 3 substituents selected from Group G),
(13) a C₂₋₆ alkynyl group (said C₂₋₆ alkynyl group is optionally substituted by 1 to 3 substituents selected from Group G),
(14) a C₁₋₆ alkoxy group (said C₁₋₆ alkoxy group is optionally substituted by 1 to 3 substituents selected from Group G),
(15) a C₁₋₆ alkylthio group,
(16) a C₁₋₆ alkylsulfinyl group,
(17) a C₁₋₆ alkylsulfonyl group,
(18) a C₁₋₆ alkyl-carbonyl group,
(19) a C₁₋₆ alkoxy-carbonyl group,
(20) a C₁₋₆ alkylamino group,
(21) a C₆₋₁₄ aryl group,
(22) a heteroaryl group, and
(23) an oxo group;
Group F consists of
(1) a halogen atom,
(2) a cyano group,
(3) a nitro group,
(4) a hydroxyl group,
(5) a mercapto group,
(6) a formyl group,
(7) a carboxyl group,
(8) a C₁₋₆ alkoxy group,
(9) a C₁₋₆ alkylthio group,
(10) a C₁₋₆ alkylsulfinyl group,
(11) a C₁₋₆ alkylsulfonyl group,
(12) a C₁₋₆ alkyl-carbonyl group,
(13) a C₁₋₆ alkoxy-carbonyl group,
(14) -N(R⁹¹)(R⁹²) wherein R⁹¹ and R⁹² are the same or different and each is a hydrogen atom, a C₁₋₆ alkyl group, a C₁₋₆ alkyl-carbonyl group, a C₁₋₆ alkylsulfonyl group, a C₁₋₆ alkoxy-carbonyl group, a carbamoyl group, a C₁₋₆ alkylamino-carbonyl group, or a di(C₁₋₆ alkyl)amino-carbonyl group, or R⁹¹ and R⁹² may form, together with the nitrogen atom to which they are bonded, a nitrogen-containing 4- to 7-membered heterocyclic group,
(15) -CO-N(R⁹¹)(R⁹²) wherein R⁹¹ and R⁹² are as defined above,
(16) -SO₂-N(R⁹¹)(R⁹²) wherein R⁹¹ and R⁹² are as defined above,
(17) -O-CO-N(R⁹¹)(R⁹²) wherein R⁹¹ and R⁹² are as defined above,
(18) a C₆₋₁₄ aryl group,
(19) a C₆₋₁₄ aryl-oxy group,
(20) a C₆₋₁₄ aryl-carbonyl group, and
(21) a heteroaryl group;
Group G consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkoxy group,
(12) a C₁₋₆ alkylthio group,
(13) a C₁₋₆ alkylsulfinyl group,
(14) a C₁₋₆ alkylsulfonyl group,
(15) a C₁₋₆ alkyl-carbonyl group,
(16) a C₁₋₆ alkoxy-carbonyl group,
(17) a C₁₋₆ alkylamino group,
(18) a C₁₋₆ alkyl-carbonyl-amino group,
(19) a C₆₋₁₄ aryl group, and
(20) a heteroaryl group.

2. Use of a heterocyclic compound represented by the formula [1] of claim 1 or a pharmaceutically acceptable salt thereof for the production of an anti-hepatitis virus agent.

3. Use of a heterocyclic compound represented by the formula [1] of claim 1 or a pharmaceutically acceptable salt thereof for the production of an HCV entry inhibitor.

4. The use of claim 1, which uses a heterocyclic compound as represented by the following formula [1] or a pharmaceutically acceptable salt thereof: 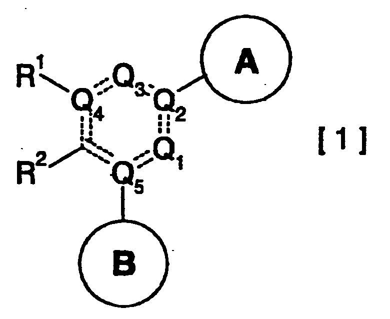 wherein 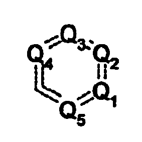 is 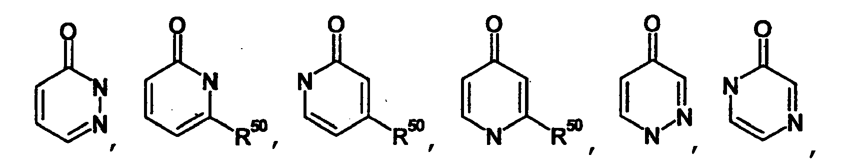 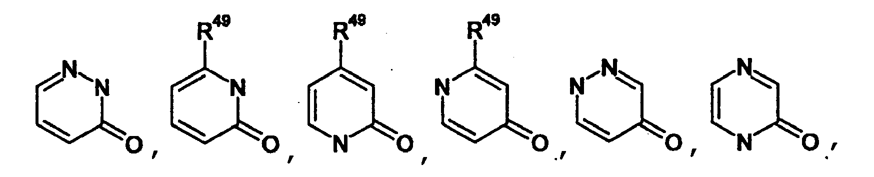 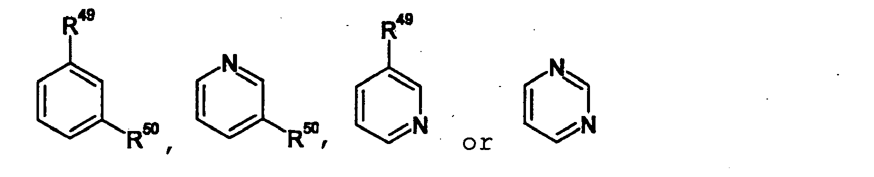 wherein
R⁴⁹ and R⁵⁰ are the same or different and each is
(1) a hydrogen atom,
(2) a hydroxyl group,
(3) a C₁₋₆ alkyl group, or
(4) a C₁₋₆ alkoxy group;
R¹ is:
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₁-R¹⁰¹
wherein
J₁ is
(1) -N(R¹⁰²)- wherein R¹⁰² is a hydrogen atom or a C₁₋₆ alkyl group,
(2) -O-,
(3) -S-,
(4) -CO-,
(5) -CO-O-,
(6) -O-CO-,
(7) -CO-N(R¹⁰²)- wherein R¹⁰² is as defined above, or
(8) -SO₂-N(R¹⁰²)- wherein R¹⁰² is as defined above,
R¹⁰¹ is
(1) a hydrogen atom,
(2) -L₂-J₂-R¹⁰³
wherein
L₂ is
(1) a bond, or
(2) a C₁₋₆ alkylene group,
J₂ is
(1) -N(R¹⁰⁴)- wherein R¹⁰⁴ is a hydrogen atom or a C₁₋₆ alkyl group,
(2) -O-,
(3) -S-,
(4) -CO-,
(5) -SO₂-,
(6) -CO-O-,
(7) -CO-N(R¹⁰⁴)- wherein R¹⁰⁴ is as defined above, or
(8) -SO2-N(R¹⁰⁴)- wherein R¹⁰⁴ is as defined above,
R¹⁰³ is
(1) a hydrogen atom,
(2) a C₁₋₆ alkyl group (said C₁₋₆ alkyl group is optionally substituted by 1 to 3 substituents selected from Group F),
(3) a C₁₋₆ alkyl-carbonyl group (said C₁₋₆ alkyl-carbonyl group is optionally substituted by 1 to 3 substituents selected from Group F), or
(4) a C₁₋₆ alkylsulfonyl group (said C₁₋₆ alkylsulfonyl group is optionally substituted by 1 to 3 substituents selected from Group F),
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹⁰¹ and R¹⁰² may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(7) a C₆₋₁₄ aryl group (said C₆₋₁₄ aryl group is optionally substituted by 1 to 5 substituents selected from Group E), or
(8) a heteroaryl group (said heteroaryl group is optionally substituted by 1 to 5 substituents selected from Group E);
R² is:
(1) a hydrogen atom, or
(2) a C₁₋₆ alkyl group; or
R¹ and R² may form, together with Q₄ and the carbon atom to which they are bonded respectively: 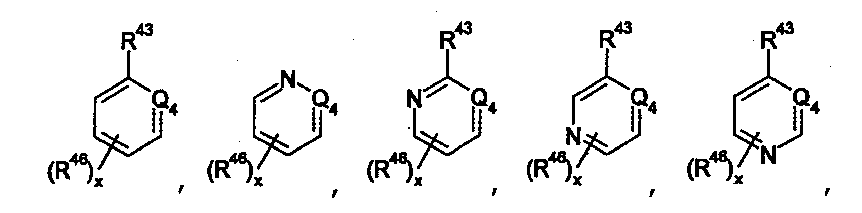 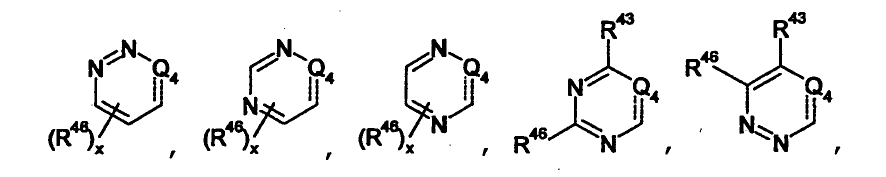 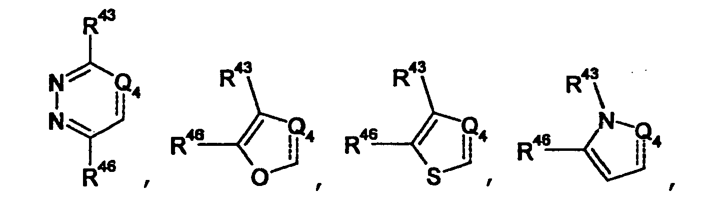 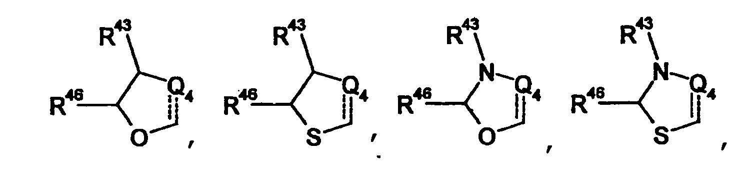 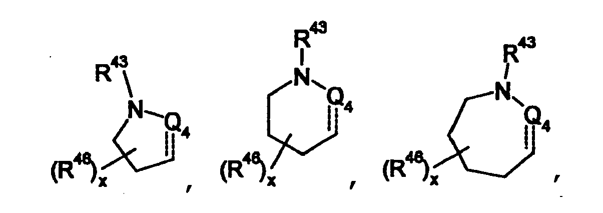 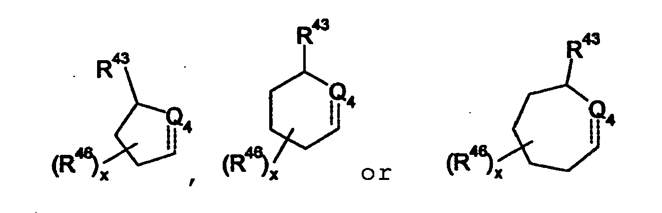 wherein
Q₄ is as defined above,
R⁴³ is
(1) a hydrogen atom,
(2) -L₃-J₃-R¹¹³
wherein
L₃ is
(1) a bond, or
(2) a C₁₋₆ alkylene group,
J₃ is
(1) -N(R¹¹⁴)- wherein R¹¹⁴ is a hydrogen atom or a C₁₋₆ alkyl group,
(2) -O-,
(3) -S-,
(4) -CO-,
(5) -SO₂-,
(6) -CO-O-,
(7) -CO-N(R¹¹⁴)- wherein R¹¹⁴ is as defined above, or
(8) -SO₂-N(R¹¹⁴)- wherein R¹¹⁴ is as defined above,
R¹¹³ is
(1) a hydrogen atom,
(2) -L₄-J₄-R¹¹⁵
wherein
L₄ is
(1) a bond, or
(2) a C₁₋₆ alkylene group,
J₄ is
(1) -N(R¹¹⁶)- wherein R¹¹⁶ is a hydrogen atom or a C₁₋₆ alkyl group,
(2) -O-,
(3) -S-,
(4) -CO-,
(5) -SO₂-,
(6) -CO-O-,
(7) -CO-N(R¹¹⁶)- wherein R¹¹⁶ is as defined above, or
(8) -SO₂-N(R¹¹⁶)- wherein R¹¹⁶ is as defined above,
R¹¹⁵ is
(1) a hydrogen atom,
(2) a C₁₋₆ alkyl group (said C₁₋₆ alkyl group is optionally substituted by 1 to 3 substituents selected from Group G),
(3) a C₁₋₆ alkyl-carbonyl group (said C₁₋₆ alkyl-carbonyl group is optionally substituted by 1 to 3 substituents selected from Group G), or
(4) a C₁₋₆ alkylsulfonyl group (said C₁₋₆ alkylsulfonyl group is optionally substituted by 1 to 3 substituents selected from Group G), or
R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹¹³ and R¹¹⁴ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E);
x is 1 or 2;
R⁴⁶ are the same or different and each is
(1) a hydrogen atom,
(2) a hydroxyl group,
(3) a C₁₋₆ alkyl group, or
(4) a C₁₋₆ alkoxy group; 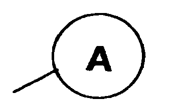 is 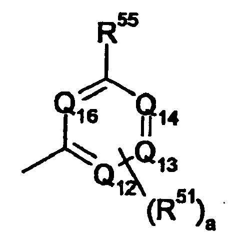
wherein
Q₁₂, Q₁₃, Q₁₄, and Q₁₆ are the same or different and each is
(1) -N=, or
(2) -CH=;
R⁵⁵ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₅-R¹²¹
wherein
J₅ is
(1) -N(R¹²²)- wherein R¹²² is a hydrogen atom or a C₁₋₆ alkyl group,
(2) -O-,
(3) -S-,
(4) -CO-,
(5) -CO-O-,
(6) -O-CO-,
(7) -CO-N(R¹²²)- wherein R¹²² is as defined above, (8) -SO₂-N(R¹²²)- wherein R¹²² is as defined above,
(9) -N(R¹²³)-CO- wherein R¹²³ is a hydrogen atom or a C₁₋₆ alkyl group, or
(10) -N(R¹²³)-SO₂- wherein R¹²³ is as defined above,
R¹²¹ is
(1) a hydrogen atom,
(2) -L₆-J₆-R¹²⁴
wherein
L₆ is
(1) a bond, or
(2) a C₁₋₆ alkylene group,
J₆ is
(1) -N(R¹²⁵)- wherein R¹²⁵ is a hydrogen atom or a C₁₋₆ alkyl group,
(2) -O-,
(3) -S-,
(4) -CO-,
(5) -CO-O-,
(6) -O-CO-,
(7) -CO-N(R¹²⁵)- wherein R¹²⁵ is as defined above,
(8) -SO₂-N(R¹²⁵)- wherein R¹²⁵ is as defined above,
(9) -N(R¹²⁶)-CO- wherein R¹²⁶ is a hydrogen atom or a C₁₋₆ alkyl group,
(10) -N(R¹²⁶)-SO₂- wherein R¹²⁶ is as defined above,
(11) -N(R¹²⁶)-CO-O- wherein R¹²⁶ is as defined above, or
(12) -N(R¹²⁶)-CO-N(R¹²⁵)- wherein R¹²⁵ and R¹²⁶ are as defined above,
R¹²⁴ is
(1) a hydrogen atom, or
(2) a C₁₋₆ alkyl group (said C₁₋₆ alkyl group is optionally substituted by 1 to 3 substituents selected from Group G), or
R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹²¹ and R¹²² may form, together with the nitrogen atom to which they are bonded, a nitrogen-containing 4-to 7-membered heterocyclic group, wherein said nitrogen-containing 4- to 7-membered heterocyclic group is optionally substituted by 1 to 5 substituents selected from
(1) Group F,
(2) a C₁₋₆ alkyl group (said C₁₋₆ alkyl group is optionally substituted by 1 to 3 substituents selected from Group F),
(3) a C₁₋₆ alkoxy group (said C₁₋₆ alkoxy group is optionally substituted by 1 to 3 substituents selected from Group F), and
(4) an oxo group,
(7) a monocyclic aryl group (said monocyclic aryl group is optionally substituted by 1 to 5 substituents selected from Group E),
(8) a monocyclic heteroaryl group (said monocyclic heteroaryl group is optionally substituted by 1 to 5 substituents selected from Group E),
(9) a C₃₋₈ cycloalkyl group (said C₃₋₈ cycloalkyl group is optionally substituted by 1 to 5 substituents selected from Group E), or
(10) a heterocycloalkyl group (said heterocycloalkyl group is optionally substituted by 1 to 5 substituents selected from Group E);
a is 1 or 2;
R⁵¹ are the same or different and each is
(1) a hydrogen atom,
(2) a halogen atom,
(3) a cyano group,
(4) a nitro group,
(5) a hydroxyl group,
(6) a mercapto group,
(7) a formyl group,
(8) a carboxyl group,
(9) a C₁₋₆ alkyl group (said C₁₋₆ alkyl group is optionally substituted by 1 to 3 substituents selected from Group G),
(10) a C₂₋₆ alkenyl group (said C₂₋₆ alkenyl group is optionally substituted by 1 to 3 substituents selected from Group G),
(11) a C₂₋₆ alkynyl group (said C₂₋₆ alkynyl group is optionally substituted by 1 to 3 substituents selected from Group G),
(12) a C₁₋₆ alkoxy group (said C₁₋₆ alkoxy group is optionally substituted by 1 to 3 substituents selected from Group G),
(13) a C₁₋₆ alkylthio group (said C₁₋₆ alkylthio group is optionally substituted by 1 to 3 substituents selected from Group G),
(14) a C₁₋₆ alkylsulfinyl group (said C₁₋₆ alkylsulfinyl group is optionally substituted by 1 to 3 substituents selected from Group G),
(15) a C₁₋₆ alkylsulfonyl group (said C₁₋₆ alkylsulfonyl group is optionally substituted by 1 to 3 substituents selected from Group G),
(16) a C₁₋₆ alkyl-carbonyl group (said C₁₋₆ alkyl-carbonyl group is optionally substituted by 1 to 3 substituents selected from Group G),
(17) a C₁₋₆ alkoxy-carbonyl group (said C₁₋₆ alkoxy-carbonyl group is optionally substituted by 1 to 3 substituents selected from Group G), (18) -N(R⁵⁷)(R⁵⁸)
wherein
R⁵⁷ and R⁵⁸ are the same or different and each is
(1) a hydrogen atom,
(2) a C₁₋₆ alkyl group (said C₁₋₆ alkyl group is optionally substituted by 1 to 3 substituents selected from Group G),
(3) a C₁₋₆ alkylsulfonyl group (said C₁₋₆ alkylsulfonyl group is optionally substituted by 1 to 3 substituents selected from Group G),
(4) a C₁₋₆ alkyl-carbonyl group (said C₁₋₆ alkyl-carbonyl group is optionally substituted by 1 to 3 substituents selected from Group G),
(5) a C₁₋₆ alkoxy-carbonyl group (said C₁₋₆ alkoxy-carbonyl group is optionally substituted by 1 to 3 substituents selected from Group G),
(6) a carbamoyl group,
(7) a C₁₋₆ alkylamino-carbonyl group (said C₁₋₆ alkylamino-carbonyl group is optionally substituted by 1 to 3 substituents selected from Group G),
(8) a di(C₁₋₆ alkyl)amino-carbonyl group (said di(C₁₋₆ alkyl)amino-carbonyl group is optionally substituted by 1 to 3 substituents selected from Group G),
(9) a hydroxyl group,
(10) a C₁₋₆ alkoxy group (said C₁₋₆ alkoxy group is optionally substituted by 1 to 3 substituents selected from Group G),
(11) an amino group,
(12) a C₁₋₆ alkylamino group (said C₁₋₆ alkylamino group is optionally substituted by 1 to 3 substituents selected from Group G),
(13) a di(C₁₋₆ alkyl)amino group (said di(C₁₋₆ alkyl)amino group is optionally substituted by 1 to 3 substituents selected from Group G), or
(14) a C₁₋₆ alkyl-carbonyl-amino group (said C₁₋₆ alkyl-carbonyl-amino group is optionally substituted by 1 to 3 substituents selected from Group G), or
R⁵⁷ and R⁵⁸ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(19) -CO-N(R⁵⁷)(R⁵⁸) wherein R⁵⁷ and R⁵⁸ is as defined above,
(20) -C(R⁵⁹)=N(R⁶⁰)
wherein
R⁵⁹ is
(1) a hydrogen atom,
(2) a C₁₋₆ alkyl group, or
(3) an amino group,
R⁶⁰ is
(1) a hydroxyl group,
(2) a C₁₋₆ alkoxy group (said C₁₋₆ alkoxy group is optionally substituted by 1 to 3 substituents selected from Group G),
(3) a C₁₋₆ alkyl-carbonyl-oxy group (said C₁₋₆ alkyl-carbonyl-oxy group is optionally substituted by 1 to 3 substituents selected from Group G), or
(4) -N(R⁶¹)(R⁶²) wherein R⁶¹ and R⁶² are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
(21) a monocyclic aryl group (said monocyclic aryl group is optionally substituted by 1 to 5 substituents selected from Group E),
(22) a monocyclic heteroaryl group (said monocyclic heteroaryl group is optionally substituted by 1 to 5 substituents selected from Group E), or
(23) a heterocycloalkyl group (said heterocycloalkyl group is optionally substituted by 1 to 5 substituents selected from Group E), or
two adjacent R⁵¹s may form, together with the ring to which they are bonded, a fused ring (said fused ring is optionally further substituted by one substituent selected from Group E); 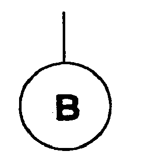 is 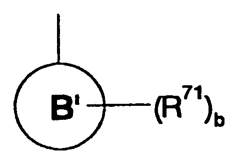 wherein
ring B' is
(1) a monocyclic aryl group, or
(2) a monocyclic heteroaryl group;
b is an integer of 1-3;
R⁷¹ are the same or different and each is
(1) a hydrogen atom,
(2) a halogen atom,
(3) a cyano group,
(4) a nitro group,
(5) a hydroxyl group,
(6) a mercapto group,
(7) a formyl group,
(8) a carboxyl group,
(9) a C₁₋₆ alkyl group (said C₁₋₆ alkyl group is optionally substituted by 1 to 3 substituents selected from Group G),
(10) a C₂₋₆ alkenyl group (said C₂₋₆ alkenyl group is optionally substituted by 1 to 3 substituents selected from Group G),
(11) a C₂₋₆ alkynyl group (said C₂₋₆ alkynyl group is optionally substituted by 1 to 3 substituents selected from Group G),
(12) a C₁₋₆ alkoxy group (said C₁₋₆ alkoxy group is optionally substituted by 1 to 3 substituents selected from Group G),
(13) a C₁₋₆ alkylthio group (said C₁₋₆ alkylthio group is optionally substituted by 1 to 3 substituents selected from Group G),
(14) a C₁₋₆ alkylsulfinyl group (said C₁₋₆ alkylsulfinyl group is optionally substituted by 1 to 3 substituents selected from Group G),
(15) a C₁₋₆ alkylsulfonyl group (said C₁₋₆ alkylsulfonyl group is optionally substituted by 1 to 3 substituents selected from Group G),
(16) a C₁₋₆ alkyl-carbonyl group (said C₁₋₆ alkyl-carbonyl group is optionally substituted by 1 to 3 substituents selected from Group G),
(17) a C₁₋₆ alkoxy-carbonyl group (said C₁₋₆ alkoxy-carbonyl group is optionally substituted by 1 to 3 substituents selected from Group G),
(18) -N(R⁷²)(R⁷³)
wherein
R⁷² and R⁷³ are the same or different and each is
(1) a hydrogen atom,
(2) a C₁₋₆ alkyl group (said C₁₋₆ alkyl group is optionally substituted by 1 to 3 substituents selected from Group G),
(3) a C₁₋₆ alkylsulfonyl group (said C₁₋₆ alkylsulfonyl group is optionally substituted by 1 to 3 substituents selected from Group G),
(4) a C₁₋₆ alkyl-carbonyl group (said C₁₋₆ alkyl-carbonyl group is optionally substituted by 1 to 3 substituents selected from Group G),
(5) a C₁₋₆ alkoxy-carbonyl group (said C₁₋₆ alkoxy-carbonyl group is optionally substituted by 1 to 3 substituents selected from Group G),
(6) a carbamoyl group,
(7) a C₁₋₆ alkylamino-carbonyl group (said C₁₋₆ alkylamino-carbonyl group is optionally substituted by 1 to 3 substituents selected from Group G),
(8) a di(C₁₋₆ alkyl)amino-carbonyl group (said di(C₁₋₆ alkyl)amino-carbonyl group is optionally substituted by 1 to 3 substituents selected from Group G),
(9) a hydroxyl group,
(10) a C₁₋₆ alkoxy group (said C₁₋₆ alkoxy group is optionally substituted by 1 to 3 substituents selected from Group G),
(11) an amino group,
(12) a C₁₋₆ alkylamino group (said C₁₋₆ alkylamino group is optionally substituted by 1 to 3 substituents selected from Group G),
(13) a di(C₁₋₆ alkyl)amino group (said di(C₁₋₆ alkyl)amino group is optionally substituted by 1 to 3 substituents selected from Group G), or
(14) a C₁₋₆ alkyl-carbonyl-amino group (said C₁₋₆ alkyl-carbonyl-amino group is optionally substituted by 1 to 3 substituents selected from Group G), or
R⁷² and R⁷³ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(19) -CO-N(R⁷²)(R⁷³) wherein R⁷² and R⁷³ is as defined above,
(20) -C(R⁷⁴)=N(R⁷⁵)
wherein
R⁷⁴ is
(1) a hydrogen atom,
(2) a C₁₋₆ alkyl group, or
(3) an amino group,
R⁷⁵ is
(1) a hydroxyl group,
(2) a C₁₋₆ alkoxy group (said C₁₋₆ alkoxy group is optionally substituted by 1 to 3 substituents selected from Group G),
(3) a C₁₋₆ alkyl-carbonyl-oxy group (said C₁₋₆ alkyl-carbonyl-oxy group is optionally substituted by 1 to 3 substituents selected from Group G), or
(4) -N(R⁷⁶)(R⁷⁷) wherein R⁷⁶ and R⁷⁷ are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
(21) a monocyclic aryl group (said monocyclic aryl group is optionally substituted by 1 to 5 substituents selected from Group E),
(22) a monocyclic heteroaryl group (said monocyclic heteroaryl group is optionally substituted by 1 to 5 substituents selected from Group E), or
(23) a heterocycloalkyl group (said heterocycloalkyl group is optionally substituted by 1 to 5 substituents selected from Group E), or
two adjacent R⁷¹s may form, together with the ring to which they are bonded, a fused ring (said fused ring is optionally further substituted by one substituent selected from Group E):
wherein
Group E consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkyl group (said C₁₋₆ alkyl group is optionally substituted by 1 to 3 substituents selected from Group G),
(12) a C₂₋₆ alkenyl group (said C₂₋₆ alkenyl group is optionally substituted by 1 to 3 substituents selected from Group G),
(13) a C₂₋₆ alkynyl group (said C₂₋₆ alkynyl group is optionally substituted by 1 to 3 substituents selected from Group G),
(14) a C₁₋₆ alkoxy group (said C₁₋₆ alkoxy group is optionally substituted by 1 to 3 substituents selected from Group G),
(15) a C₁₋₆ alkylthio group,
(16) a C₁₋₆ alkylsulfinyl group,
(17) a C₁₋₆ alkylsulfonyl group,
(18) a C₁₋₆ alkyl-carbonyl group,
(19) a C₁₋₆ alkoxy-carbonyl group,
(20) a C₁₋₆ alkylamino group,
(21) a C₆₋₁₄ aryl group,
(22) a heteroaryl group, and
(23) an oxo group;
Group F consists of
(1) a halogen atom,
(2) a cyano group,
(3) a nitro group,
(4) a hydroxyl group,
(5) a mercapto group,
(6) a formyl group,
(7) a carboxyl group,
(8) a C₁₋₆ alkoxy group,
(9) a C₁₋₆ alkylthio group,
(10) a C₁₋₆ alkylsulfinyl group,
(11) a C₁₋₆ alkylsulfonyl group,
(12) a C₁₋₆ alkyl-carbonyl group,
(13) a C₁₋₆ alkoxy-carbonyl group,
(14) -N(R⁹¹)(R⁹²) wherein R⁹¹ and R⁹² are the same or different and each is a hydrogen atom, a C₁₋₆ alkyl group, a C₁₋₆ alkyl-carbonyl group, a C₁₋₆ alkylsulfonyl group, a C₁₋₆ alkoxycarbonyl group, a carbamoyl group, a C₁₋₆ alkylamino-carbonyl group, or a di(C₁₋₆ alkyl)amino-carbonyl group, or R⁹¹ and R⁹² may form, together with the nitrogen atom to which they are bonded, a nitrogen-containing 4- to 7-membered heterocyclic group,
(15) -CO-N(R⁹¹)(R⁹²) wherein R⁹¹ and R⁹² are as defined above,
(16) -SO₂-N(R⁹¹)(R⁹²) wherein R⁹¹ and R⁹² are as defined above,
(17) -O-CO-N(R⁹¹)(R⁹²) wherein R⁹¹ and R⁹² are as defined above,
(18) a C₆₋₁₄ aryl group,
(19) a C₆₋₁₄ aryl-oxy group,
(20) a C₆₋₁₄ aryl-carbonyl group, and
(21) a heteroaryl group;
Group G consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkoxy group,
(12) a C₁₋₆ alkylthio group,
(13) a C₁₋₆ alkylsulfinyl group,
(14) a C₁₋₆ alkylsulfonyl group,
(15) a C₁₋₆ alkyl-carbonyl group,
(16) a C₁₋₆ alkoxy-carbonyl group,
(17) a C₁₋₆ alkylamino group,
(18) a C₁₋₆ alkyl-carbonyl-amino group,
(19) a C₆₋₁₄ aryl group, and
(20) a heteroaryl group.

5. The use of claim 4, which uses a heterocyclic compound as represented by the following formula [1] or a pharmaceutically acceptable salt thereof: 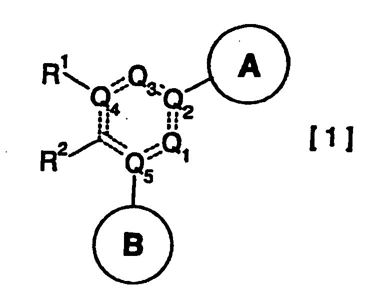 wherein 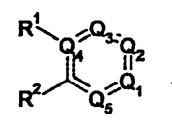 is 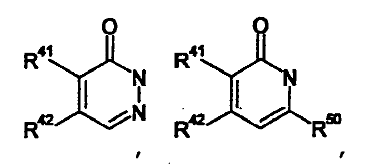 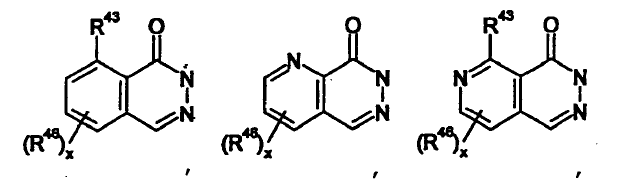 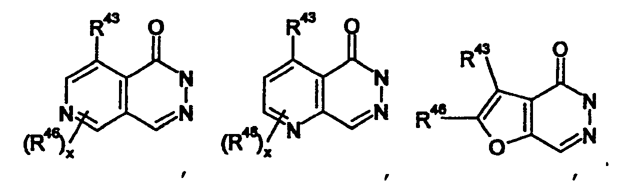 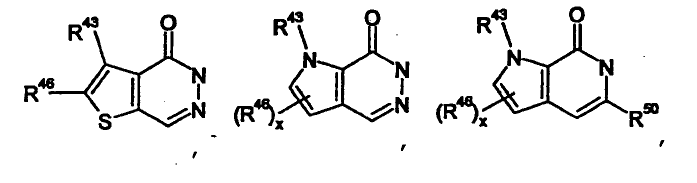 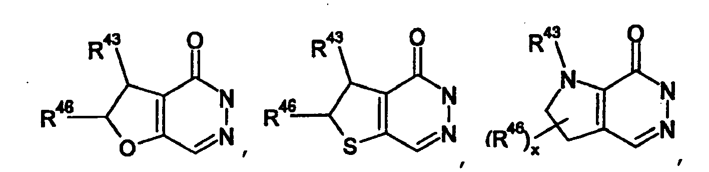 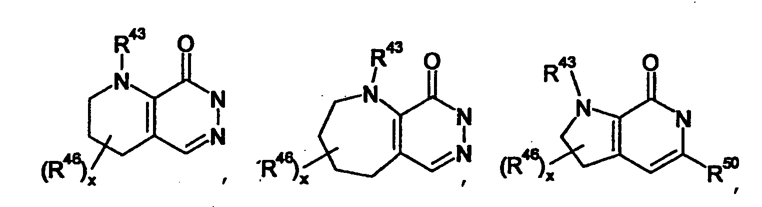 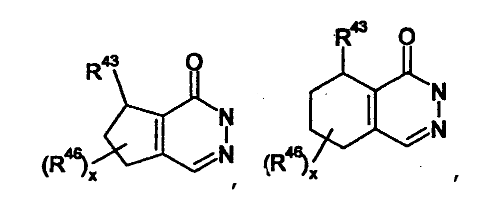 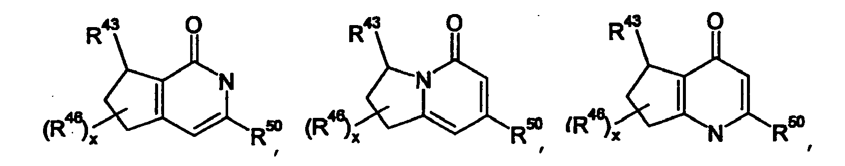 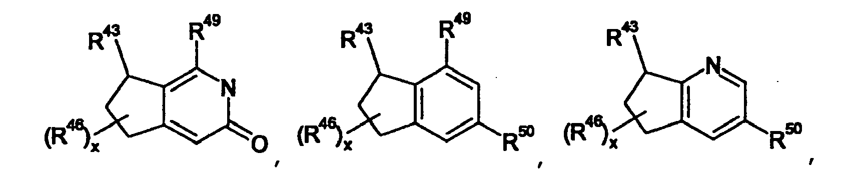 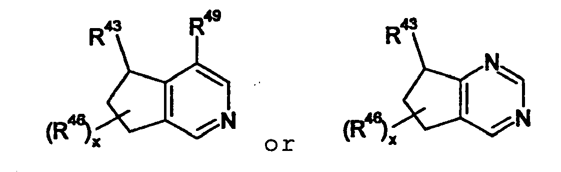 wherein
R⁴¹ is
(1) a hydrogen atom,
(2) a C₁₋₆ alkyl group (said C₁₋₆ alkyl group is optionally substituted by 1 to 3 substituents selected from Group F),
(3) a C₂₋₆ alkenyl group,
(4) -J₁-R¹⁰¹
wherein
J₁ is
(1) -N(R¹⁰²)- wherein R¹⁰² is a hydrogen atom or a C₁₋₆ alkyl group,
(2) -O-,
(3) -S-,
(4) -CO-,
(5) -CO-O-, or
(6) -CO-N(R¹⁰²)- wherein R¹⁰² is as defined above,
R¹⁰¹ is
(1) a hydrogen atom,
(2) -L₂-J₂-R¹⁰³
wherein
L₂ is
(1) a bond, or
(2) a C₁₋₆ alkylene group,
J₂ is
(1) -N(R¹⁰⁴)- wherein R¹⁰⁴ is a hydrogen atom or a C₁₋₆ alkyl group,
(2) -O-,
(3) -CO-,
(4) -SO₂-,
(5) -CO-O-,
(6) -CO-N(R¹⁰⁴)- wherein R¹⁰⁴ is as defined above, or
(7) -SO₂-N(R¹⁰⁴)- wherein R¹⁰⁴ is as defined above,
R¹⁰³ is
(1) a hydrogen atom,
(2) a C₁₋₆ alkyl group, or
(3) a C₁₋₆ alkyl-carbonyl group, or
(3) a C₁₋₆ alkyl group, or
R¹⁰¹ and R¹⁰² may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E), or
(5) a C₆₋₁₄ aryl group;
R⁴² is
(1) a hydrogen atom, or
(2) a C₁₋₆ alkyl group;
R⁴³ is
(1) a hydrogen atom,
(2) -L₃-J₃-R¹¹³
wherein
L₃ is
(1) a bond, or
(2) a C₁₋₆ alkylene group,
J₃ is
(1) -N(R¹¹⁴)- wherein R¹¹⁴ is a hydrogen atom or a C₁₋₆ alkyl group,
(2) -O-,
(3) -CO-,
(4) -SO₂-,
(5) -CO-O-, or
(6) -CO-N(R¹¹⁴)- wherein R¹¹⁴ is as defined above,
R¹¹³ is
(1) a hydrogen atom,
(2) -L₄-J₄-R¹¹⁵
wherein
L₄ is
(1) a bond, or
(2) a C₁₋₆ alkylene group,
J₄ is
(1) -N(R¹¹⁶)- wherein R¹¹⁶ is a hydrogen atom or a C₁₋₆ alkyl group,
(2) -O-, or
(3) -CO-,
R¹¹⁵ is
(1) a hydrogen atom,
(2) a C₁₋₆ alkyl group (said C₁₋₆ alkyl group is optionally substituted by 1 to 3 substituents selected from Group G), or
(3) a C₁₋₆ alkyl-carbonyl group, or
R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
(3) a C₁₋₆ alkyl group, or
R¹¹³ and R¹¹⁴ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈cycloalkyl group is optionally substituted by 1 to 5 substituents selected from Group E);
x is 1 or 2;
R⁴⁶ are the same or different and each is
(1) a hydrogen atom,
(2) a hydroxyl group,
(3) a C₁₋₆ alkyl group, or
(4) a C₁₋₆ alkoxy group;
R⁴⁹ and R⁵⁰ are the same or different and each is
(1) a hydrogen atom,
(2) a hydroxyl group, or
(3) a C₁₋₆ alkoxy group; 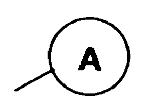 is 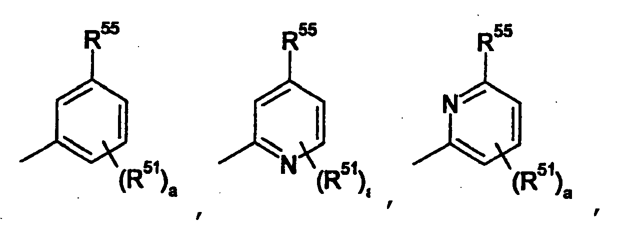 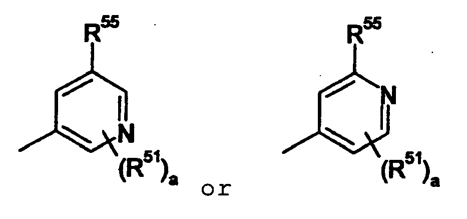
wherein
R⁵⁵ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkynyl group (said C₂₋₆ alkynyl group is optionally substituted by 1 to 3 substituents selected from Group F),
(5) -J₅-R¹²¹
wherein
J₅ is
(1) -N(R¹²²)- wherein R¹²² is a hydrogen atom or a C₁₋₆ alkyl group,
(2) -O-,
(3) -CO-,
(4) -CO-O-,
(5) -CO-N(R¹²²)- wherein R¹²² is as defined above, or
(6) -N(R¹²³)-CO- wherein R¹²³ is a hydrogen atom or a C₁₋₆ alkyl group,
R¹²¹ is
(1) a hydrogen atom,
(2) -L₆-J₆-R¹²⁴
wherein
L₆ is
(1) a bond, or
(2) a C₁₋₆ alkylene group,
J₆ is
(1) -N(R¹²⁵)- wherein R¹²⁵ is a hydrogen atom or a C₁₋₆ alkyl group,
(2) -O-,
(3) -CO-,
(4) -CO-O-,
(5) -CO-N(R¹²⁵)- wherein R¹²⁵ is as defined above,
(6) -N(R¹²⁶)-CO- wherein R¹²⁶ is a hydrogen atom or a C₁₋₆ alkyl group,
(7) -N(R¹²⁶)-SO₂- wherein R¹²⁶ is as defined above,
(8) -N(R¹²⁶)-CO-O- wherein R¹²⁶ is as defined above, or
(9) -N(R¹²⁶)-CO-N(R¹²⁵)- wherein R¹²⁵ and R¹²⁶ are as defined above,
R¹²⁴ is
(1) a hydrogen atom, or
(2) a C₁₋₆ alkyl group (said C₁₋₆ alkyl group is optionally substituted by 1 to 3 substituents selected from Group G), or
R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹²¹ and R¹²² may form, together with the nitrogen atom to which they are bonded, a nitrogen-containing 4-to 7-membered heterocyclic group, wherein said nitrogen-containing 4- to 7-membered heterocyclic group is optionally substituted by 1 to 5 substituents selected from
(1) Group F,
(2) a C₁₋₆ alkyl group (said C₁₋₆ alkyl group is optionally substituted by 1 to 3 substituents selected from Group F),
(3) a C₁₋₆ alkoxy group (said C₁₋₆ alkoxy group is optionally substituted by 1 to 3 substituents selected from Group F), and
(4) an oxo group,
(6) a monocyclic aryl group,
(7) a monocyclic heteroaryl group (said monocyclic heteroaryl group is optionally substituted by 1 to 5 substituents selected from Group E), or
(8) a heterocycloalkyl group;
a is 1 or 2;
R⁵¹ are the same or different and each is
(1) a hydrogen atom,
(2) a halogen atom,
(3) a cyano group,
(4) a nitro group,
(5) a hydroxyl group,
(6) a formyl group,
(7) a carboxyl group,
(8) a C₁₋₆ alkyl group (said C₁₋₆ alkyl group is optionally substituted by 1 to 3 substituents selected from Group G),
(9) a C₂₋₆alkenyl group,
(10) a C₁₋₆ alkoxy group (said C₁₋₆ alkoxy group is optionally substituted by 1 to 3 substituents selected from Group G),
(11) a C₁₋₆ alkylthio group,
(12) a C₁₋₆ alkylsulfinyl group,
(13) a C₁₋₆ alkylsulfonyl group,
(14) a C₁₋₆ alkyl-carbonyl group (said C₁₋₆ alkyl-carbonyl group is optionally substituted by 1 to 3 substituents selected from Group G),
(15) a C₁₋₆ alkoxy-carbonyl group,
(16)-N(R⁵⁷)(R⁵⁸)
wherein
R⁵⁷ and R⁵⁸ are the same or different and each is
(1) a hydrogen atom,
(2) a C₁₋₆ alkyl group,
(3) a C₁₋₆ alkylsulfonyl group,
(4) a C₁₋₆ alkyl-carbonyl group, or
(5) a C₁₋₆ alkoxy-carbonyl group, or
R⁵⁷ and R⁵⁸ may form, together with the nitrogen atom to which they are bonded, a nitrogen-containing 4-to 7-membered heterocyclic group,
(17) -CO-N(R⁵⁷)(R⁵⁸) wherein R⁵⁷ and R⁵⁸ are as defined above, or
(18) -C(R⁵⁹)=N(R⁶⁰)
wherein
R⁵⁹ is
(1) a hydrogen atom,
R⁶⁰ is
(1) a hydroxyl group, or
(2) a C₁₋₆ alkyl-carbonyl-oxy group, or
two adjacent R⁵¹s may form, together with the ring to which they are bonded, a fused ring (said fused ring is optionally further substituted by one substituent selected from Group E); 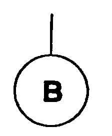 is 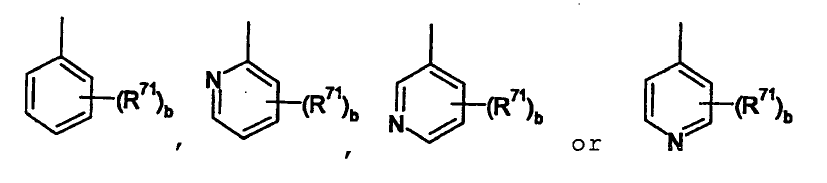
wherein
b is 1 or 2;
R⁷¹ are the same or different and each is
(1) a hydrogen atom,
(2) a halogen atom,
(3) a cyano group,
(4) a hydroxyl group,
(5) a formyl group,
(6) a carboxyl group,
(7) a C₁₋₆ alkyl group (said C₁₋₆ alkyl group is optionally substituted by 1 to 3 substituents selected from Group G),
(8) a C₂₋₆ alkenyl group (said C₂₋₆ alkenyl group is optionally substituted by 1 to 3 substituents selected from Group G),
(9) a C₁₋₆ alkoxy group (said C₁₋₆ alkoxy group is optionally substituted by 1 to 3 substituents selected from Group G),
(10) a C₁₋₆ alkyl-carbonyl group,
(11) a C₁₋₆ alkoxy-carbonyl group,
(12) -N(R⁷²)(R⁷³)
wherein
R⁷² and R⁷³ are the same or different and each is
(1) a hydrogen atom,
(2) a C₁₋₆ alkyl group (said C₁₋₆ alkyl group is optionally substituted by 1 to 3 substituents selected from Group G),
(3) a C₁₋₆ alkylsulfonyl group,
(4) a C₁₋₆ alkyl-carbonyl group,
(5) a C₁₋₆ alkoxy-carbonyl group,
(6) a carbamoyl group,
(7) a hydroxyl group,
(8) an amino group, or
(9) a C₁₋₆ alkyl-carbonyl-amino group, or
R⁷² and R⁷³ may form, together with the nitrogen atom to which they are bonded, a nitrogen-containing 4-to 7-membered heterocyclic group,
(13) -CO-N(R⁷²)(R⁷³) wherein R⁷² and R⁷³ are as defined above,
(14) -C(R⁷⁴)=N(R⁷⁵)
wherein
R⁷⁴ is
(1) a hydrogen atom,
(2) a C₁₋₆ alkyl group, or
(3) an amino group,
R⁷⁵ is
(1) a hydroxyl group,
(2) a C₁₋₆ alkoxy group (said C₁₋₆ alkoxy group is optionally substituted by 1 to 3 substituents selected from Group G), or
(3) -N(R⁷⁶)(R⁷⁷) wherein R⁷⁶ and R⁷⁷ are the same or different and each is a hydrogen atom, or a C₁₋₆ alkyl-carbonyl group,
(15) a monocyclic aryl group,
(16) a monocyclic heteroaryl group (said monocyclic heteroaryl group is optionally substituted by 1 to 5 substituents selected from Group E), or
(17) a heterocycloalkyl group, or
two adjacent R⁷¹s may form, together with the ring to which they are bonded, a fused ring (said fused ring is optionally further substituted by one substituent selected from Group E):
wherein
Group E consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkyl group (said C₁₋₆ alkyl group is optionally substituted by 1 to 3 substituents selected from Group G),
(12) a C₂₋₆ alkenyl group (said C₂₋₆ alkenyl group is optionally substituted by 1 to 3 substituents selected from Group G),
(13) a C₂₋₆ alkynyl group (said C₂₋₆ alkynyl group is optionally substituted by 1 to 3 substituents selected from Group G),
(14) a C₁₋₆ alkoxy group (said C₁₋₆ alkoxy group is optionally substituted by 1 to 3 substituents selected from Group G),
(15) a C₁₋₆ alkylthio group,
(16) a C₁₋₆ alkylsulfinyl group,
(17) a C₁₋₆ alkylsulfonyl group,
(18) a C₁₋₆ alkyl-carbonyl group,
(19) a C₁₋₆ alkoxy-carbonyl group,
(20) a C₁₋₆ alkylamino group,
(21) a C₆₋₁₄ aryl group,
(22) a heteroaryl group, and
(23) an oxo group;
Group F consists of
(1) a halogen atom,
(2) a cyano group,
(3) a nitro group,
(4) a hydroxyl group,
(5) a mercapto group,
(6) a formyl group,
(7) a carboxyl group,
(8) a C₁₋₆ alkoxy group,
(9) a C₁₋₆ alkylthio group,
(10) a C₁₋₆ alkylsulfinyl group,
(11) a C₁₋₆ alkylsulfonyl group,
(12) a C₁₋₆ alkyl-carbonyl group,
(13) a C₁₋₆ alkoxy-carbonyl group,
(14) -N(R⁹¹)(R⁹²) wherein R⁹¹ and R⁹² are the same or different and each is a hydrogen atom, a C₁₋₆ alkyl group, a C₁₋₆ alkyl-carbonyl group, a C₁₋₆ alkylsulfonyl group, a C₁₋₆ alkoxy-carbonyl group, a carbamoyl group, a C₁₋₆ alkylamino-carbonyl group, or a di(C₁₋₆ alkyl)amino-carbonyl group, or R⁹¹ and R⁹² may form, together with the nitrogen atom to which they are bonded, a nitrogen-containing 4- to 7-membered heterocyclic group,
(15) -CO-N(R⁹¹)(R⁹²) wherein R⁹¹ and R⁹² are as defined above,
(16) -SO₂-N(R⁹¹)(R⁹²) wherein R⁹¹ and R⁹² are as defined above,
(17)-O-CO-N(R⁹¹)(R⁹²) wherein R⁹¹ and R⁹² are as defined above,
(18) a C₆₋₁₄ aryl group,
(19) a C₆₋₁₄ aryl-oxy group,
(20) a C₆₋₁₄ aryl-carbonyl group, and
(21) a heteroaryl group;
Group G consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkoxy group,
(12) a C₁₋₆ alkylthio group,
(13) a C₁₋₆ alkylsulfinyl group,
(14) a C₁₋₆ alkylsulfonyl group,
(15) a C₁₋₆ alkyl-carbonyl group,
(16) a C₁₋₆ alkoxy-carbonyl group,
(17) a C₁₋₆ alkylamino group,
(18) a C₁₋₆ alkyl-carbonyl-amino group,
(19) a C₆₋₁₄ aryl group, and
(20) a heteroaryl group.

6. A heterocyclic compound represented by the following formula [1], or a pharmaceutically acceptable salt 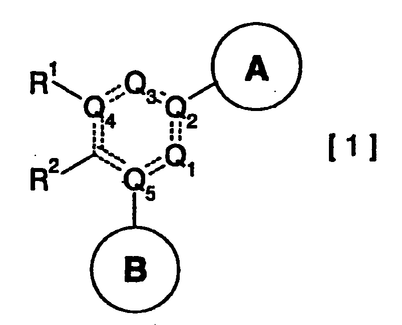 thereof:
wherein 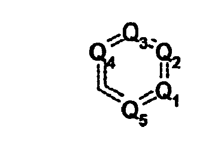 is 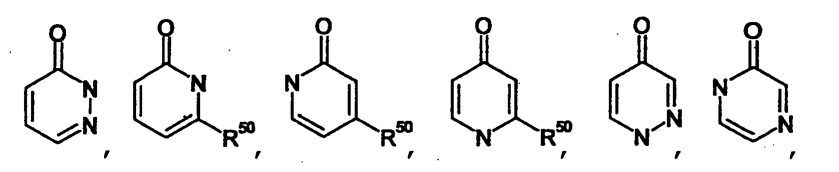 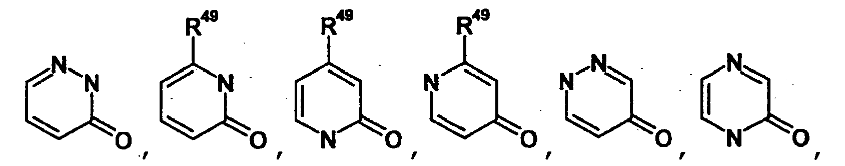 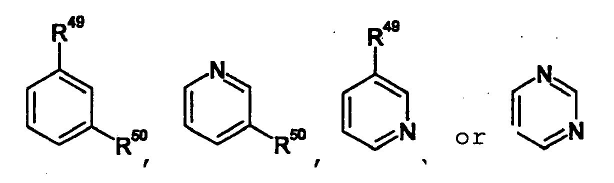 wherein
R⁴⁹ and R⁵⁰ are the same or different and each is
(1) a hydrogen atom,
(2) a hydroxyl group,
(3) a C₁₋₆ alkyl group, or
(4) a C₁₋₆ alkoxy group;
R¹ is:
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₁-R¹⁰¹
wherein
J₁ is
(1) -N(R¹⁰²)- wherein R¹⁰² is a hydrogen atom or a C₁₋₆ alkyl group,
(2) -O-,
(3) -S-,
(4) -CO-,
(5) -CO-O-,
(6) -O-CO-,
(7) -CO-N(R¹⁰²)- wherein R¹⁰² is as defined above, or
(8) -SO₂-N(R¹⁰²)- wherein R¹⁰² is as defined above,
R¹⁰¹ is
(1) a hydrogen atom,
(2) -L₂-J₂-R¹⁰³
wherein
L₂ is
(1) a bond, or
(2) a C₁₋₆ alkylene group,
J₂ is
(1) -N(R¹⁰⁴)- wherein R¹⁰⁴ is a hydrogen atom or a C₁₋₆ alkyl group,
(2) -O-,
(3) -S-,
(4) -CO-,
(5) -SO₂-,
(6) -CO-O-,
(7) -CO-N(R¹⁰⁴)- wherein R¹⁰⁴ is as defined above, or
(8) -SO₂-N(R¹⁰⁴)- wherein R¹⁰⁴ is as defined above,
R¹⁰³ is
(1) a hydrogen atom,
(2) a C₁₋₆ alkyl group (said C₁₋₆ alkyl group is optionally substituted by 1 to 3 substituents selected from Group F),
(3) a C₁₋₆ alkyl-carbonyl group (said C₁₋₆ alkyl-carbonyl group is optionally substituted by 1 to 3 substituents selected from Group F), or
(4) a C₁₋₆ alkylsulfonyl group (said C₁₋₆ alkylsulfonyl group is optionally substituted by 1 to 3 substituents selected from Group F),
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹⁰¹ and R¹⁰² may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(7) a C₆₋₁₄ aryl group (said C₆₋₁₄ aryl group is optionally substituted by 1 to 5 substituents selected from Group E), or
(8) heteroaryl group (said heteroaryl group is optionally substituted by 1 to 5 substituents selected from Group E);
R² is:
(1) a hydrogen atom, or
(2) a C₁₋₆ alkyl group; or
R¹ and R² may form, together with Q₄ and the carbon atom to which they are bonded respectively: 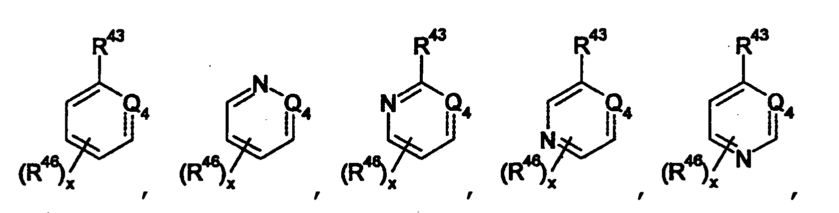 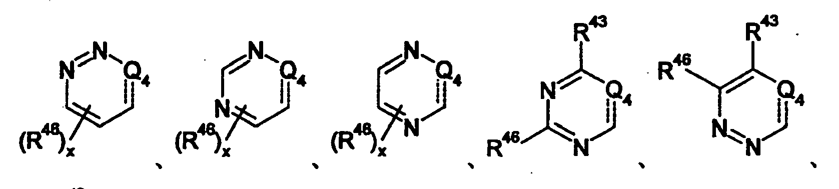 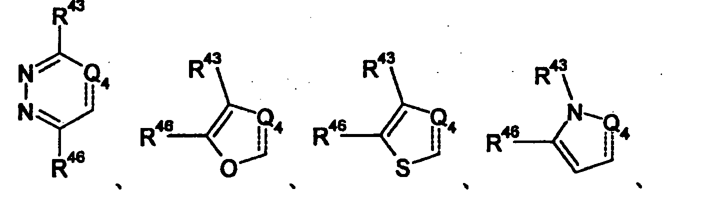 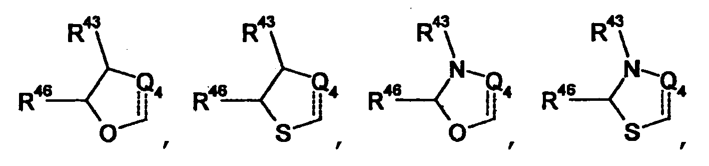 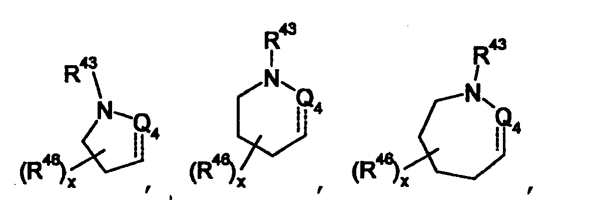 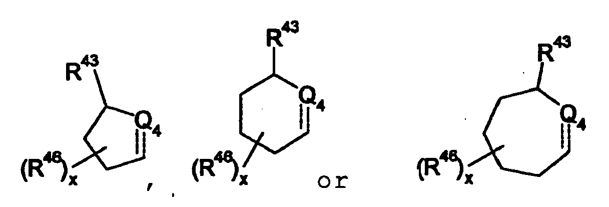 wherein
Q₄ is as defined above,
R⁴³ is
(1) a hydrogen atom,
(2) -L₃-J₃-R¹¹³
wherein
L₃ is
(1) a bond, or
(2) a C₁₋₆ alkylene group,
J₃ is
(1) -N(R¹¹⁴)- wherein R¹¹⁴ is a hydrogen atom or a C₁₋₆ alkyl group,
(2) -O-,
(3) -S-,
(4) -CO-,
(5) -SO₂-,
(6) -CO-O-,
(7) -CO-N(R¹¹⁴)- wherein R¹¹⁴ is as defined above, or
(8) -SO₂-N(R¹¹⁴)- wherein R¹¹⁴ is as defined above,
R¹¹³ is
(1) a hydrogen atom,
(2) -L₄-J₄-R¹¹⁵
wherein
L₄ is
(1) a bond, or
(2) a C₁₋₆ alkylene group,
J₄ is
(1) -N(R¹¹⁶)- wherein R¹¹⁶ is a hydrogen atom or a C₁₋₆ alkyl group,
(2) -O-,
(3) -S-,
(4) -CO-,
(5) -SO₂-,
(6) -CO-O-,
(7) -CO-N(R¹¹⁶)- wherein R¹¹⁶ is as defined above, or
(8) -SO₂-N(R¹¹⁶)- wherein R¹¹⁶ is as defined above,
R¹¹⁵ is
(1) a hydrogen atom,
(2) a C₁₋₆ alkyl group (said C₁₋₆ alkyl group is optionally substituted by 1 to 3 substituents selected from Group G),
(3) a C₁₋₆ alkyl-carbonyl group (said C₁₋₆ alkyl-carbonyl group is optionally substituted by 1 to 3 substituents selected from Group G), or
(4) a C₁₋₆ alkylsulfonyl group (said C₁₋₆ alkylsulfonyl group is optionally substituted by 1 to 3 substituents selected from Group G), or
R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹¹³ and R¹¹⁴ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E);
x is 1 or 2;
R⁴⁶ are the same or different and each is
(1) a hydrogen atom,
(2) a hydroxyl group,
(3) a C₁₋₆ alkyl group, or
(4) a C₁₋₆ alkoxy group; 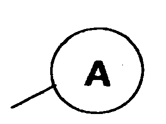 is 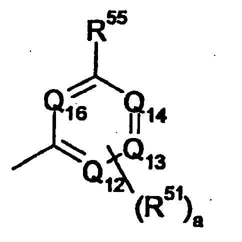
wherein
Q₁₂, Q₁₃, Q₁₄, and Q₁₆ are the same or different and each is
(1) -N=, or
(2) -CH=;
R⁵⁵ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkenyl group (said C₂₋₆ alkenyl group is optionally substituted by 1 to 3 substituents selected from Group F),
(5) a C₂₋₆ alkynyl group (said C₂₋₆ alkynyl group is optionally substituted by 1 to 3 substituents selected from Group F),
(6) -J₅-R¹²¹
wherein
J₅ is
(1) -N(R¹²²)- wherein R¹²² is a hydrogen atom or a C₁₋₆ alkyl group,
(2) -O-,
(3) -S-,
(4) -CO-,
(5) -CO-O-,
(6) -O-CO-,
(7) -CO-N(R¹²²)- wherein R¹²² is as defined above,
(8) -SO₂-N(R¹²²)- wherein R¹²² is as defined above,
(9) -N(R¹²³)-CO- wherein R¹²³ is a hydrogen atom or a C₁₋₆ alkyl group, or
(10) -N(R¹²³)-SO₂- wherein R¹²³ is as defined above,
R¹²¹ is
(1) a hydrogen atom,
(2) -L₆-J₆-R¹²⁴
wherein
L₆ is
(1) a bond, or
(2) a C₁₋₆ alkylene group,
J₆ is
(1) -N(R¹²⁵)- wherein R¹²⁵ is a hydrogen atom or a C₁₋₆ alkyl group,
(2) -O-,
(3) -S-,
(4) -CO-,
(5) -CO-O-,
(6) -O-CO-,
(7) -CO-N(R¹²⁵)- wherein R¹²⁵ is as defined above,
(8) -SO₂-N(R¹²⁵)- wherein R¹²⁵ is as defined above,
(9) -N(R¹²⁶)-CO- wherein R¹²⁶ is a hydrogen atom or a C₁₋₆ alkyl group,
(10) -N(R¹²⁶)-SO₂- wherein R¹²⁶ is as defined above,
(11) -N(R¹²⁶)-CO-O- wherein R¹²⁶ is as defined above, or
(12) -N(R¹²⁶)-CO-N(R¹²⁵)- wherein R¹²⁵ and R¹²⁶ is as defined above,
R¹²⁴ is
(1) a hydrogen atom, or
(2) a C₁₋₆ alkyl group (said C₁₋₆ alkyl group is optionally substituted by 1 to 3 substituents selected from Group G), or
R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹²¹ and R¹²² may form, together with the nitrogen atom to which they are bonded, a nitrogen-containing 4-to 7-membered heterocyclic group, wherein said nitrogen-containing 4- to 7-membered heterocyclic group is optionally substituted by 1 to 5 substituents selected from
(1) Group F,
(2) a C₁₋₆ alkyl group (said C₁₋₆ alkyl group is optionally substituted by 1 to 3 substituents selected from Group F),
(3) a C₁₋₆ alkoxy group (said C₁₋₆ alkoxy group is optionally substituted by 1 to 3 substituents selected from Group F), and
(4) an oxo group,
(7) a monocyclic aryl group (said monocyclic aryl group is optionally substituted by 1 to 5 substituents selected from Group E),
(8) a monocyclic heteroaryl group (said monocyclic heteroaryl group is optionally substituted by 1 to 5 substituents selected from Group E),
(9) a C₃₋₈ cycloalkyl group (said C₃₋₈ cycloalkyl group is optionally substituted by 1 to 5 substituents selected from Group E), or
(10) a heterocycloalkyl group (said heterocycloalkyl group is optionally substituted by 1 to 5 substituents selected from Group E);
a is 1 or 2;
R⁵¹ are the same or different and each is
(1) a hydrogen atom,
(2) a halogen atom,
(3) a cyano group,
(4) a nitro group,
(5) a hydroxyl group,
(6) a mercapto group,
(7) a formyl group,
(8) a carboxyl group,
(9) a C₁₋₆ alkyl group (said C₁₋₆ alkyl group is optionally substituted by 1 to 3 substituents selected from Group G),
(10) a C₂₋₆ alkenyl group (said C₂₋₆ alkenyl group is optionally substituted by 1 to 3 substituents selected from Group G),
(11) a C₂₋₆ alkynyl group (said C₂₋₆ alkynyl group is optionally substituted by 1 to 3 substituents selected from Group G),
(12) a C₁₋₆ alkoxy group (said C₁₋₆ alkoxy group is optionally substituted by 1 to 3 substituents selected from Group G),
(13) a C₁₋₆ alkylthio group (said C₁₋₆ alkylthio group is optionally substituted by 1 to 3 substituents selected from Group G),
(14) a C₁₋₆ alkylsulfinyl group (said C₁₋₆ alkylsulfinyl group is optionally substituted by 1 to 3 substituents selected from Group G),
(15) a C₁₋₆ alkylsulfonyl group (said C₁₋₆ alkylsulfonyl group is optionally substituted by 1 to 3 substituents selected from Group G),
(16) a C₁₋₆ alkyl-carbonyl group (said C₁₋₆ alkyl-carbonyl group is optionally substituted by 1 to 3 substituents selected from Group G),
(17) a C₁₋₆ alkoxy-carbonyl group (said C₁₋₆ alkoxy-carbonyl group is optionally substituted by 1 to 3 substituents selected from Group G),
(18) -N(R⁵⁷)(R⁵⁸)
wherein
R⁵⁷ and R⁵⁸ are the same or different and each is
(1) a hydrogen atom,
(2) a C₁₋₆ alkyl group (said C₁₋₆ alkyl group is optionally substituted by 1 to 3 substituents selected from Group G),
(3) a C₁₋₆ alkylsulfonyl group (said C₁₋₆ alkylsulfonyl group is optionally substituted by 1 to 3 substituents selected from Group G),
(4) a C₁₋₆ alkyl-carbonyl group (said C₁₋₆ alkyl-carbonyl group is optionally substituted by 1 to 3 substituents selected from Group G),
(5) a C₁₋₆ alkoxy-carbonyl group (said C₁₋₆ alkoxy-carbonyl group is optionally substituted by 1 to 3 substituents selected from Group G),
(6) a carbamoyl group,
(7) a C₁₋₆ alkylamino-carbonyl group (said C₁₋₆ alkylamino-carbonyl group is optionally substituted by 1 to 3 substituents selected from Group G),
(8) a di(C₁₋₆ alkyl)amino-carbonyl group (said di(C₁₋₆ alkyl)amino-carbonyl group is optionally substituted by 1 to 3 substituents selected from Group G),
(9) a hydroxyl group,
(10) a C₁₋₆ alkoxy group (said C₁₋₆ alkoxy group is optionally substituted by 1 to 3 substituents selected from Group G),
(11) an amino group,
(12) a C₁₋₆ alkylamino group (said C₁₋₆ alkylamino group is optionally substituted by 1 to 3 substituents selected from Group G),
(13) a di(C₁₋₆ alkyl)amino group (said di(C₁₋₆ alkyl)amino group is optionally substituted by 1 to 3 substituents selected from Group G), or
(14) a C₁₋₆ alkyl-carbonyl-amino group (said C₁₋₆ alkyl-carbonyl-amino group is optionally substituted by 1 to 3 substituents selected from Group G), or
R⁵⁷ and R⁵⁸ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(19) -CO-N(R⁵⁷)(R⁵⁸) wherein R⁵⁷ and R⁵⁸ is as defined above,
(20) -C(R⁵⁹)=N(R⁶⁰)
wherein
R⁵⁹ is
(1) a hydrogen atom,
(2) a C₁₋₆ alkyl group, or
(3) an amino group,
R⁶⁰ is
(1) a hydroxyl group,
(2) a C₁₋₆ alkoxy group (said C₁₋₆ alkoxy group is optionally substituted by 1 to 3 substituents selected from Group G),
(3) a C₁₋₆ alkyl-carbonyl-oxy group (said C₁₋₆ alkyl-carbonyl-oxy group is optionally substituted by 1 to 3 substituents selected from Group G), or
(4) -N(R⁶¹)(R⁶²) wherein R⁶¹ and R⁶² are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
(21) a monocyclic aryl group (said monocyclic aryl group is optionally substituted by 1 to 5 substituents selected from Group E),
(22) a monocyclic heteroaryl group (said monocyclic heteroaryl group is optionally substituted by 1 to 5 substituents selected from Group E), or
(23) a heterocycloalkyl group (said heterocycloalkyl group is optionally substituted by 1 to 5 substituents selected from Group E), or
two adjacent R⁵¹s may form, together with the ring to which they are bonded, a fused ring (said fused ring is optionally further substituted by one substituent selected from Group E); 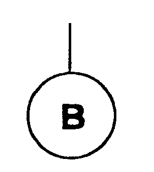 is 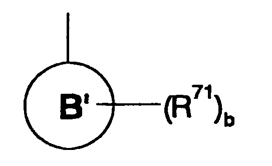 wherein
ring B' is
(1) a monocyclic aryl group, or
(2) a monocyclic heteroaryl group;
b is an integer of 1-3;
R⁷¹ are the same or different and each is
(1) a hydrogen atom,
(2) a halogen atom,
(3) a cyano group,
(4) a nitro group,
(5) a hydroxyl group,
(6) a mercapto group,
(7) a formyl group,
(8) a carboxyl group,
(9) a C₁₋₆ alkyl group (said C₁₋₆ alkyl group is optionally substituted by 1 to 3 substituents selected from Group G),
(10) a C₂₋₆ alkenyl group (said C₂₋₆ alkenyl group is optionally substituted by 1 to 3 substituents selected from Group G),
(11) a C₂₋₆ alkynyl group (said C₂₋₆ alkynyl group is optionally substituted by 1 to 3 substituents selected from Group G),
(12) a C₁₋₆ alkoxy group (said C₁₋₆ alkoxy group is optionally substituted by 1 to 3 substituents selected from Group G),
(13) a C₁₋₆ alkylthio group (said C₁₋₆ alkylthio group is optionally substituted by 1 to 3 substituents selected from Group G),
(14) a C₁₋₆ alkylsulfinyl group (said C₁₋₆ alkylsulfinyl group is optionally substituted by 1 to 3 substituents selected from Group G),
(15) a C₁₋₆ alkylsulfonyl group (said C₁₋₆ alkylsulfonyl group is optionally substituted by 1 to 3 substituents selected from Group G),
(16) a C₁₋₆ alkyl-carbonyl group (said C₁₋₆ alkyl-carbonyl group is optionally substituted by 1 to 3 substituents selected from Group G),
(17) a C₁₋₆ alkoxy-carbonyl group (said C₁₋₆ alkoxy-carbonyl group is optionally substituted by 1 to 3 substituents selected from Group G),
(18) -N(R⁷²)(R⁷³)
wherein
R⁷² and R⁷³ are the same or different and each is
(1) a hydrogen atom,
(2) a C₁₋₆ alkyl group (said C₁₋₆ alkyl group is optionally substituted by 1 to 3 substituents selected from Group G),
(3) a C₁₋₆ alkylsulfonyl group (said C₁₋₆ alkylsulfonyl group is optionally substituted by 1 to 3 substituents selected from Group G),
(4) a C₁₋₆ alkyl-carbonyl group (said C₁₋₆ alkyl-carbonyl group is optionally substituted by 1 to 3 substituents selected from Group G),
(5) a C₁₋₆ alkoxy-carbonyl group (said C₁₋₆ alkoxy-carbonyl group is optionally substituted by 1 to 3 substituents selected from Group G),
(6) a carbamoyl group,
(7) a C₁₋₆ alkylamino-carbonyl group (said C₁₋₆ alkylamino-carbonyl group is optionally substituted by 1 to 3 substituents selected from Group G),
(8) a di(C₁₋₆ alkyl)amino-carbonyl group (said di(C₁₋₆ alkyl)amino-carbonyl group is optionally substituted by 1 to 3 substituents selected from Group G),
(9) a hydroxyl group,
(10) a C₁₋₆ alkoxy group (said C₁₋₆ alkoxy group is optionally substituted by 1 to 3 substituents selected from Group G),
(11) an amino group,
(12) a C₁₋₆ alkylamino group (said C₁₋₆ alkylamino group is optionally substituted by 1 to 3 substituents selected from Group G),
(13) a di(C₁₋₆ alkyl)amino group (said di(C₁₋₆ alkyl)amino group is optionally substituted by 1 to 3 substituents selected from Group G), or
(14) a C₁₋₆ alkyl-carbonyl-amino group (said C₁₋₆ alkyl-carbonyl-amino group is optionally substituted by 1 to 3 substituents selected from Group G), or
R⁷² and R⁷³ may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected from Group E),
(19) -CO-N(R⁷²)(R⁷³) wherein R⁷² and R⁷³ is as defined above,
(20) -C(R⁷⁴)=N(R⁷⁵)
wherein
R⁷⁴ is
(1) a hydrogen atom,
(2) a C₁₋₆ alkyl group, or
(3) an amino group,
R⁷⁵ is
(1) a hydroxyl group,
(2) a C₁₋₆ alkoxy group (said C₁₋₆ alkoxy group is optionally substituted by 1 to 3 substituents selected from Group G),
(3) a C₁₋₆ alkyl-carbonyl-oxy group (said C₁₋₆ alkyl-carbonyl-oxy group is optionally substituted by 1 to 3 substituents selected from Group G), or
(4) -N(R⁷⁶)(R⁷⁷) wherein R⁷⁶ and R⁷⁷ are the same or different and each is a hydrogen atom, a C₁₋₆ alkyl group (said C₁₋₆ alkyl group is optionally substituted by 1 to 3 substituents selected from Group G), or a C₁₋₆ alkyl-carbonyl group (said C₁₋₆ alkyl-carbonyl group is optionally substituted by 1 to 3 substituents selected from Group G),
(21) a monocyclic aryl group (said monocyclic aryl group is optionally substituted by 1 to 5 substituents selected from Group E),
(22) a monocyclic heteroaryl group (said monocyclic heteroaryl group is optionally substituted by 1 to 5 substituents selected from Group E), or
(23) a heterocycloalkyl group (said heterocycloalkyl group is optionally substituted by 1 to 5 substituents selected from Group E), or
two adjacent R⁷¹s may form, together with the ring to which they are bonded, a fused ring (said fused ring is optionally further substituted by one substituent selected from Group E):
wherein
Group E consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkyl group (said C₁₋₆ alkyl group is optionally substituted by 1 to 3 substituents selected from Group G),
(12) a C₂₋₆ alkenyl group (said C₂₋₆ alkenyl group is optionally substituted by 1 to 3 substituents selected from Group G),
(13) a C₂₋₆ alkynyl group (said C₂₋₆ alkynyl group is optionally substituted by 1 to 3 substituents selected from Group G),
(14) a C₁₋₆ alkoxy group (said C₁₋₆ alkoxy group is optionally substituted by 1 to 3 substituents selected from Group G),
(15) a C₁₋₆ alkylthio group,
(16) a C₁₋₆ alkylsulfinyl group,
(17) a C₁₋₆ alkylsulfonyl group,
(18) a C₁₋₆ alkyl-carbonyl group,
(19) a C₁₋₆ alkoxy-carbonyl group,
(20) a C₁₋₆ alkylamino group,
(21) a C₆₋₁₄ aryl group,
(22) a heteroaryl group, and
(23) an oxo group;
Group F consists of
(1) a halogen atom,
(2) a cyano group,
(3) a nitro group,
(4) a hydroxyl group,
(5) a mercapto group,
(6) a formyl group,
(7) a carboxyl group,
(8) a C₁₋₆ alkoxy group,
(9) a C₁₋₆ alkylthio group,
(10) a C₁₋₆ alkylsulfinyl group,
(11) a C₁₋₆ alkylsulfonyl group,
(12) a C₁₋₆ alkyl-carbonyl group,
(13) a C₁₋₆ alkoxy-carbonyl group,
(14) -N(R⁹¹)(R⁹²) wherein R⁹¹ and R⁹² are the same or different and each is a hydrogen atom, a C₁₋₆ alkyl group, a C₁₋₆ alkyl-carbonyl group, a C₁₋₆ alkylsulfonyl group, a C₁₋₆ alkoxycarbonyl group, a carbamoyl group, a C₁₋₆ alkylamino-carbonyl group, or a di(C₁₋₆ alkyl)amino-carbonyl group, or R⁹¹ and R⁹² may form, together with the nitrogen atom to which they are bonded, a nitrogen-containing 4- to 7-membered heterocyclic group,
(15) -CO-N(R⁹¹)(R⁹²) wherein R⁹¹ and R⁹² is as defined above,
(16) -SO₂-N(R⁹¹)(R⁹²) wherein R⁹¹ and R⁹² is as defined above,
(17) -O-CO-N(R⁹¹)(R⁹²) wherein R⁹¹ and R⁹² is as defined above,
(18) a C₆₋₁₄ aryl group,
(19) a C₆₋₁₄ aryl-oxy group,
(20) a C₆₋₁₄ aryl-carbonyl group, and
(21) a heteroaryl group;
Group G consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkoxy group,
(12) a C₁₋₆ alkylthio group,
(13) a C₁₋₆ alkylsulfinyl group,
(14) a C₁₋₆ alkylsulfonyl group,
(15) a C₁₋₆ alkyl-carbonyl group,
(16) a C₁₋₆ alkoxy-carbonyl group,
(17) a C₁₋₆ alkylamino group,
(18) a C₁₋₆ alkyl-carbonyl-amino group,
(19) a C₆₋₁₄ aryl group, and
(20) a heteroaryl group,
provided that the compound of the formula [1] satisfies the following provisions 1 to 12:
provision 1:
when 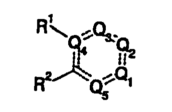 wherein R¹, R², Q₁, Q₂, Q₃, Q₄ and Q₅ are as defined above, is 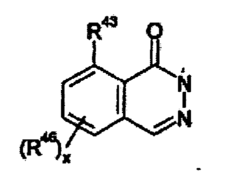 wherein R⁴³, R⁴⁶ and x are as defined above, 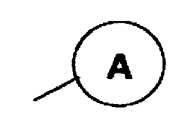 is 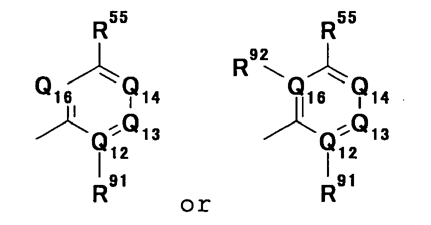 wherein
Q₁₂, Q₁₃, Q₁₄, Q₁₆ and R⁵⁵ are as defined above,
R⁹¹ is as defined for R⁵¹ (provided that a hydrogen atom is excluded), and
R⁹² is as defined for R⁵¹, and
the compounds satisfying the following provisions 1-1 to 1-3 are excluded:
provision 1-1:
ring A is 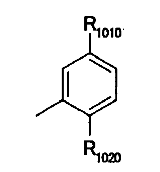 wherein R₁₀₁₀ is a chlorine atom, a piperidinyl group, a pyrrolidinyl group, an azepanylgroup or a morpholinyl group, and R₁₀₂₀ is a nitro group or an amino group, and ring B is 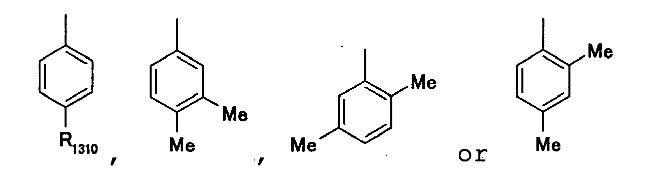 wherein R₁₃₁₀ is a hydrogen atom, a fluorine atom, a chlorine atom, a methyl group or an ethyl group;
provision 1-2:
ring A is 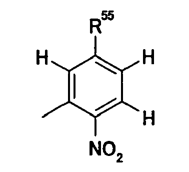 wherein
R⁵⁵ is an unsubstituted or substituted piperazinyl group optionally substituted by 1 to 5 substituents selected from Group E, Group F, a C₁₋₆ alkyl group (said C₁₋₆ alkyl group is optionally substituted by 1 to 3 substituents selected from Group F) and a C₁₋₆ alkoxy group (said C₁₋₆ alkoxy group is optionally substituted by 1 to 3 substituents selected from Group F); and
provision 1-3:
ring A is 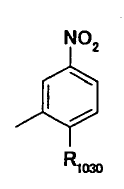 wherein R₁₀₃₀ is a chlorine atom, a pyrrolidinyl group, a morpholinyl group or a N-methylpiperazinyl group, and ring B is 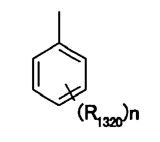 wherein n is 1 or 2, R₁₃₂₀ is a hydrogen atom, a chlorine atom or a methyl group;
provision 2:
when 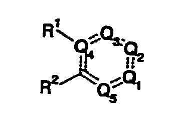 wherein R¹, R², Q₁, Q₂, Q₃, Q₄ and Q₅ are as defined above, is 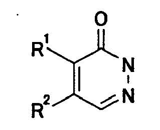 wherein R¹ and R² are as defined above, provided that the case where R¹ and R² form, together with Q₄ and the carbon atom to which they are respectively bonded, 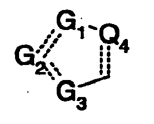 wherein G₁, G₂, G₃ and Q₄ are as defined above, is excluded, 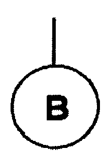 is 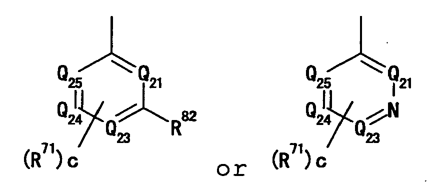 wherein Q₂₁, Q₂₃, Q₂₄ and Q₂₅ are the same or different and each is a carbon atom or a nitrogen atom, R⁷¹ is as defined above, R⁸² is as defined for R⁷¹ except that a hydrogen atom is excluded, c is 0, 1 or 2, or two adjacent R⁷¹s may form, together with the ring to which they are bonded, a fused ring (said fused ring is optionally further substituted by one substituent selected from Group E), and the following compounds are excluded:
6-(2,5-dimethylphenyl)-2-phenyl-3(2H)-pyridazinone,
6-(3,4-dimethylphenyl)-2-phenyl-3(2H)-pyridazinone,
6-(4-chloro-3-methylphenyl)-2-phenyl-3(2H)-pyridazinone,
2-(2-aminophenyl)-6-(3-chlorophenyl)-3(2H)-pyridazinone,
2-(2-aminophenyl)-6-[3-(trifluoromethyl)phenyl]-3(2H)-pyridazinone,
6-(3-chlorophenyl)-2-(2-nitrophenyl)-3(2H)-pyridazinone,
2-(2-nitrophenyl)-6-[3-(trifluoromethyl)phenyl]-3(2H)-pyridazinone,
6-(3,4-dichlorophenyl)-2-phenyl-4-(2,4,6-trimethylphenyl)-3(2H)-pyridazinone,
2-(2,4-dinitrophenyl)-6-(3-nitrophenyl)-3(2H)-pyridazinone,
2-(4-chlorophenyl)-6-(3-nitrophenyl)-3(2H)-pyridazinone,
2-(3-chlorophenyl)-6-(3-nitrophenyl)-3(2H)-pyridazinone,
2-(4-methylphenyl)-6-(3-nitrophenyl)-3(2H)-pyridazinone,
2-(3-methylphenyl)-6-(3-nitrophenyl)-3(2H)-pyridazinone,
2-(2-methylphenyl)-6-(3-nitrophenyl)-3(2H)-pyridazinone,
6-(3-nitrophenyl)-2-(4-nitrophenyl)-3(2H)-pyridazinone,
2,6-bis(3-nitrophenyl)-3(2H)-pyridazinone,
2-(2-nitrophenyl)-6-(3-nitrophenyl)-3(2H)-pyridazinone,
6-(3-nitrophenyl)-2-phenyl-3(2H)-pyridazinone,
6-(2-naphthyl)-2-phenyl-3(2H)-pyridazinone,
6-(1-naphthalenyl)-2-phenyl-3(2H)-pyridazinone,
2-(2-cyanophenyl)-4-(2-methoxyphenyl)-6-(3-pyridazinyl)-2H-pyridazin-3-one,
2-(2-cyanophenyl)-4-phenyl-6-(2-pyrazyl)-2H-pyridazin-3-one,
2-(2-aminophenyl)-6-(3-pyridazinyl)-3(2H)-pyridazinone,
2-(2-nitrophenyl)-6-(3-pyridazinyl)-3(2H)-pyridazinone,
6,6''-dichloro-[3,1'(6'H):3',3''-terpyridazine]-6'-one,
3-[1,6-dihydro-1-(4-methylphenyl)-6-oxo-3-pyridazinyl]-2(1H)-quinoxalinone,
3-[1,6-dihydro-1-(2-methylphenyl)-6-oxo-3-pyridazinyl]-2(1H)-quinoxalinone,
3-(1,6-dihydro-6-oxo-1-phenyl-3-pyridazinyl)-1-methyl-2(1H)-quinoxalinone,
3-[1-(4-chlorophenyl)-1,6-dihydro-6-oxo-3-pyridazinyl]-2(1H)-quinoxalinone, and
3-(1,6-dihydro-6-oxo-1-phenyl-3-pyridazinyl)-2(1H)-quinoxalinone;
provision 3:
when 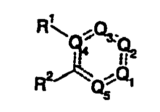 wherein R¹, R², Q₁, Q₂, Q₃, Q₄ and Q₅ are as defined above, is 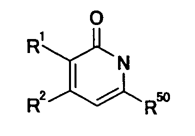 wherein R¹ and R² are as defined above, provided that the case where R¹ and R² form, together with Q₄ and the carbon atom to which they are respectively bonded, 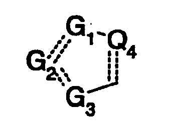 wherein G₁, G₂, G₃ and Q₄ are as defined above, is excluded, and R⁵⁰ is as defined above, 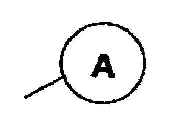 is 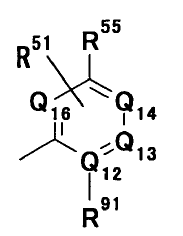 wherein Q₁₂, Q₁₃, Q₁₄, Q₁₆, R⁵¹ and R⁵⁵ are as defined above, R⁹¹ is as defined for R⁵¹ (provided that a hydrogen atom is excluded), 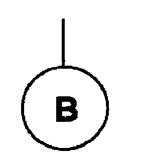 is 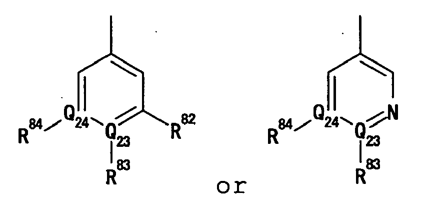 wherein
Q₂₃ and Q₂₄ are the same or different and each is a carbon atom or a nitrogen atom,
R⁸² is as defined for R⁷¹ (provided that a hydrogen atom is excluded),
R⁸³ and R⁸⁴ are the same or different and each is as defined for R⁷¹, or R⁸³ and R⁸⁴ may form, together with the ring to which they are bonded, a fused ring (said fused ring is optionally further substituted by one substituent selected from Group E);
provision 4:
when 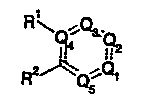 wherein R¹, R², Q₁, Q₂, Q₃, Q₄ and Q₅ are as defined above, is 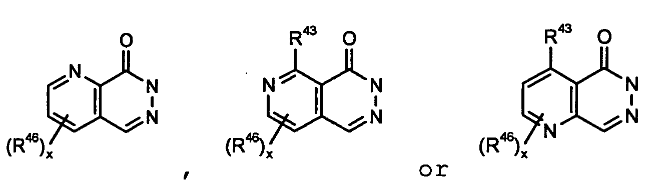 wherein R⁴³ and R⁴⁶ are the same or different and each is a hydrogen atom, a halogen atom, a hydroxyl group or a C₁₋₆ alkyl group, and x is as defined above,
the compounds satisfying the following provisions 4-1 and 4-2 are excluded:
provision 4-1: 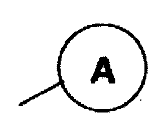 is 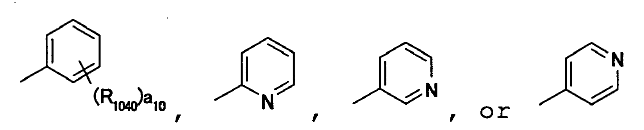 wherein when a₁₀ is 1, R₁₀₄₀ is an amino group, a hydrogen atom, a trifluoromethyloxy group, a bromine atom, a sulfamoyl group, a methanesulfonyl group, a hydroxyl group, a C₁₋₆ alkyl group, a C₁₋₆ alkoxy group, a chlorine atom, a fluorine atom, a trifluoromethyl group, a carbamoyl group, a nitro group or a cyano group, and a₁₀ is an integer of 2 or 3, R₁₀₄₀ are the same or different and each is a hydroxyl group, a C₁₋₆ alkyl group, a C₁₋₆ alkoxy group, a chlorine atom, a fluorine atom, a trifluoromethyl group, a carbamoyl group, a nitro group or a cyano group, and
ring B is a benzotriazole group, or 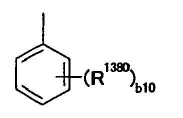 wherein b10 is an integer of 0 or 1 to 5, R¹³⁸⁰ are the same or different and each is a hydrogen atom, a halogen atom, a C₁₋₆ alkyl group, -COOH, a C₁₋₆ alkoxycarbonyl group, a carbamoyl group, a C₁₋₆ alkylcarbonyl group, a halocarbonyl group, a methylthio group, a trifluoromethyl group, a cyano group, a nitro group, a 2,2-difluorodioxolanyl group, a methanesulfonyl group or a trifluoromethyloxy group;
provision 4-2: 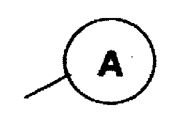 is 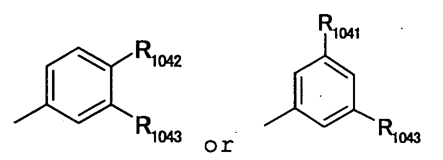 wherein R₁₀₄₁ is a fluorine atom or a trifluoromethyl group, R₁₀₄₂ is a fluorine atom or chlorine atom, R₁₀₄₃ is a fluorine atom, a chlorine atom, a cyano group or a trifluoromethyl group, or R₁₀₄₂ and R₁₀₄₃ form a ring represented by 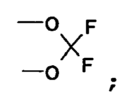 provision 5:
when 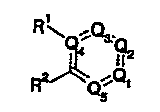 is 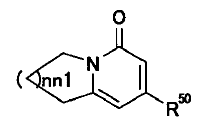 wherein R⁵⁰ is a hydrogen atom or a hydroxyl group, and nn1 is an integer of 1 to 3,
the compounds satisfying the following provisions 5-1 and 5-2 are excluded:
provision 5-1:
ring A is a phenyl group or a 3,4-dimethoxyphenyl group, and ring B is a phenyl group or a 4-chlorophenyl group, and nn1 is 1;
provision 5-2:
ring A is a phenyl group or a pyridyl group, which is optionally substituted by a halogen atom, a trifluoromethyl group, a nitro group, a C₁₋₆ alkyl group or a C₁₋₆ alkoxy group, and nn1 is 2 or 3;
provision 6:
when 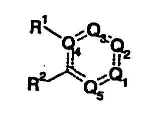 wherein R¹, R², Q₁, Q₂, Q₃, Q₄ and Q₅ are as defined above, is 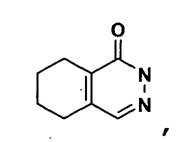 compounds in which ring A is a phenyl group and ring B is a 3,4-dimethoxyphenyl group are excluded;
provision 7:
when 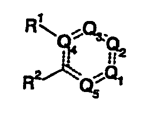 wherein R¹, R², Q₁, Q₂, Q₃, Q₄ and Q₅ are as defined above, is 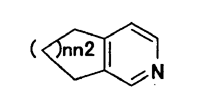 wherein nn2 is an integer of 1 to 3,
compounds in which ring A is a phenyl group or 4-chlorophenyl group, and nn2 is an integer of 1 to 3 are excluded;
provision 8:
when 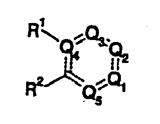 wherein R¹, R², Q₁, Q₂, Q₃, Q₄ and Q₅ are as defined above, is 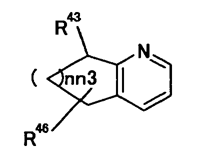 wherein R⁴³ is a hydrogen atom, a hydroxyl group, an acetyloxy group, a methyl group, a benzyl group, a 4-methylbenzyl group, -CH₂-NMe₂ or -CH₂-S-nBu, R⁴⁶ is a hydrogen atom or a methyl group, and nn3 is an integer of 1 to 3,
the following compounds are excluded:
compounds in which ring A is 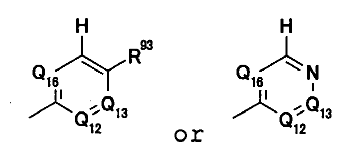 wherein Q₁₂, Q₁₃ and Q₁₆ are as described above, and R⁹³ is an amino group, a nitro group, a hydrogen atom, a trifluoromethyl group, a methoxy group, a methyl group,
a chlorine atom or an acetamide group, and
ring B is 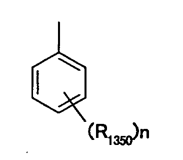 wherein R₁₃₅₀ is a hydrogen atom, -COOMe, a cyano group, - COOEt, t-Bu, a dimethylamino group, a methyl group, a chlorine atom, a dioxolanyl group, a 2-pyridyl group or a 2-pyrimidinyl group, and n is an integer of 1 to 3;
provision 9:
when 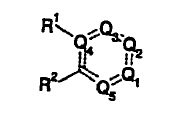 wherein R¹, R², Q₁, Q₂, Q₃, Q₄ and Q₅ are as defined above, is 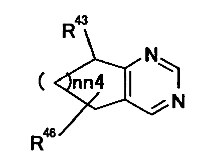 wherein R⁴³ and R⁴⁶ are as defined above, and nn4 is an integer of 1 to 3,
compounds in which ring A is a phenyl group, a 3,4-dimethoxyphenyl group, a 4-methylphenyl group, a 4-chlorophenyl group or a 4-fluorophenyl group, ring B is
a phenyl group, a 3,4-dimethoxyphenyl group, a 4-methylphenyl group, a 4-chlorophenyl group, a 4-fluorophenyl group or a 3-chlorophenyl group, R⁴³ and R⁴⁶ are each a hydrogen atom or a methyl group, and nn4 is an integer of 1 to 3 are excluded;
provision 10:
when 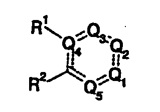 wherein R¹, R², Q₁, Q₂, Q₃, Q₄ and Q₅ are as defined above, is 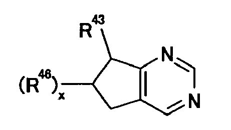 wherein R⁴³ and R⁴⁶ are the same or different and each is a hydrogen atom or a C₁₋₆ alkyl group,
compounds in which ring A is a phenyl group substituted, at the ortho- or meta- position, by a halogen atom, a C₁₋₆ alkyl group, a C₁₋₆ haloalkyl group, a C₁₋₆ alkoxy group or a C₁₋₆ haloalkyloxy group, and ring B is a phenyl group substituted by a hydrogen atom, a halogen atom, a C₁₋₆ alkyl group, a C₁₋₆ haloalkyl group, a C₁₋₆ alkoxy group or a C₁₋₆ haloalkyloxy group are excluded;
provision 11:
when 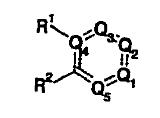 wherein R¹, R², Q₁, Q₂, Q₃, Q₄ and Q₅ are as defined above, is 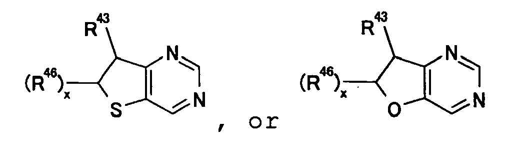 wherein R⁴³ and R⁴⁶ are the same or different and each is a hydrogen or a C₁₋₆ alkyl group,
compounds in which
ring A is a phenyl group substituted at the ortho- or meta- position by a halogen atom, a C₁₋₆ alkyl group, a C₁₋₆ haloalkyl group, a C₁₋₆ alkoxy group or a C₁₋₆ haloalkyloxy group, and ring B is a phenyl group substituted by a hydrogen atom, a halogen atom, a C₁₋₆ alkyl group, a C₁₋₆ haloalkyl group, a C₁₋₆ alkoxy group or a C₁₋₆ haloalkyloxy group are excluded; and
provision 12:
when 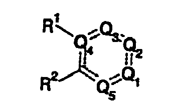 wherein R¹, R², Q₁, Q₂, Q₃, Q₄ and Q₅ are as defined above, is 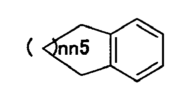 wherein nn5 is an integer of 1 to 3,
compounds in which ring A and ring B are each a 3-nitrophenyl group or a 4-nitrophenyl group, and nn5 is 2 are excluded.

7. The heterocyclic compound of claim 6, wherein, in the formula [1], 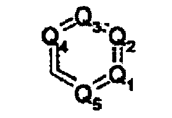 wherein Q₁, Q₂, Q₃, Q₄ and Q₅ are as defined above, is 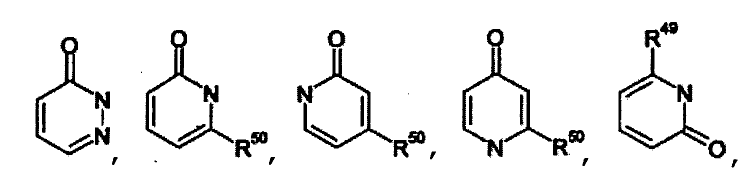 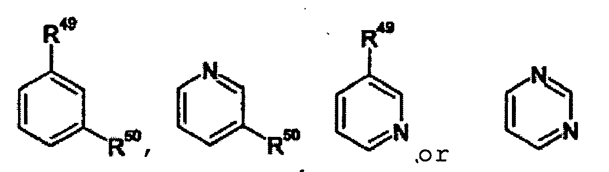 wherein R⁴⁹ and R⁵⁰ are as defined above, or a pharmaceutically acceptable salt thereof.

8. The heterocyclic compound of claim 6, wherein, in the formula [1], the ring formed by R¹ and R², together with Q₄ and the carbon atom to which they are bonded, is 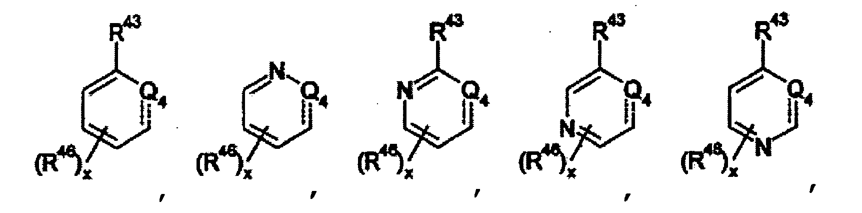 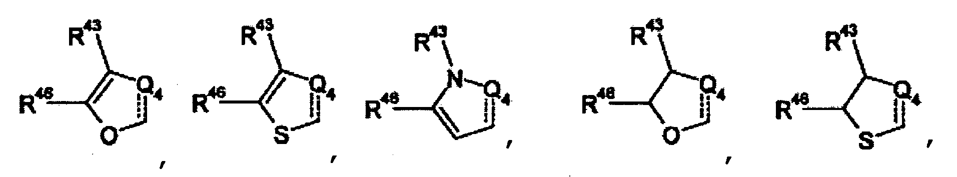 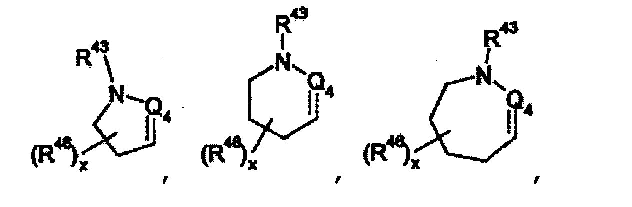 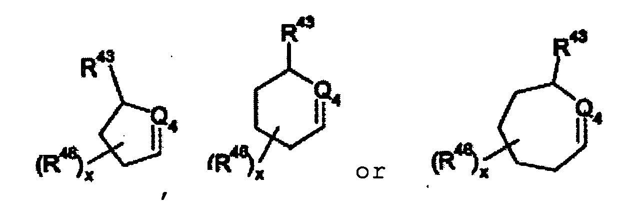 wherein Q₄, R⁴³, R⁴⁶ and x are as defined above, or a pharmaceutically acceptable salt thereof.

9. The heterocyclic compound of claim 6, wherein R¹ is
(1) a hydrogen atom,
(2) a C₁₋₆ alkyl group (said C₁₋₆ alkyl group is optionally substituted by 1 to 3 substituents selected from Group F),
(3) a C₂₋₆ alkenyl group (said C₂₋₆ alkenyl group is optionally substituted by 1 to 3 substituents selected from Group F),
(4) -J₁-R¹⁰¹ wherein J₁ and R¹⁰¹ are as defined in claim 6, or
(5) a C₆₋₁₄ aryl group (said C₆₋₁₄ aryl group is optionally substituted by 1 to 5 substituents selected from Group E), or a pharmaceutically acceptable salt thereof.

10. The heterocyclic compound of claim 6, wherein R² is a hydrogen atom, or a pharmaceutically acceptable salt thereof.

11. The heterocyclic compound of claim 6, wherein R⁴⁹ and R⁵⁰ are the same or different and each is a hydrogen atom, a hydroxyl group or a C₁₋₆ alkoxy group, or a pharmaceutically acceptable salt thereof.

12. The heterocyclic compound of claim 6, wherein R⁴³ is -L₃-J₃-R¹¹³ wherein L₂, J₃ and R¹¹³ are as defined in claim 6, or a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E), or a pharmaceutically acceptable salt thereof.

13. The heterocyclic compound of claim 6, wherein 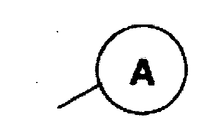 is 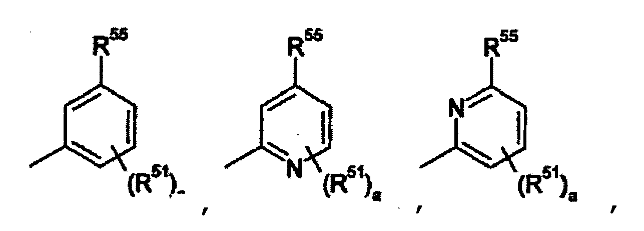 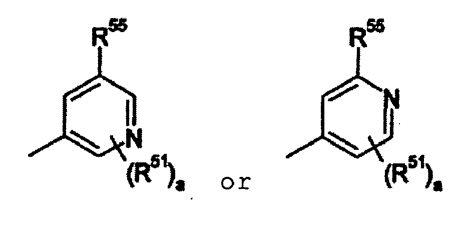 wherein
R⁵⁵ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkynyl group (said C₂₋₆ alkynyl group is optionally substituted by 1 to 3 substituents selected from Group F),
(5) -J₅-R¹²¹ wherein J₅ and R¹²¹ are as defined in claim 6,
(6) a monocyclic aryl group,
(7) a monocyclic heteroaryl group (said monocyclic heteroaryl group is optionally substituted by 1 to 5 substituents selected from Group E), or
(8) a heterocycloalkyl group,
a is 1 or 2,
R⁵¹ are the same or different and each is
(1) a hydrogen atom,
(2) a halogen atom,
(3) a cyano group,
(4) a nitro group,
(5) a hydroxyl group,
(6) a formyl group,
(7) a carboxyl group,
(8) a C₁₋₆ alkyl group (said C₁₋₆ alkyl group is optionally substituted by 1 to 3 substituents selected from Group G),
(9) a C₂₋₆ alkenyl group,
(10) a C₁₋₆ alkoxy group (said C₁₋₆ alkoxy group is optionally substituted by 1 to 3 substituents selected from Group G),
(11) a C₁₋₆ alkylthio group,
(12) a C₁₋₆ alkylsulfinyl group,
(13) a C₁₋₆ alkylsulfonyl group,
(14) a C₁₋₆ alkyl-carbonyl group (said C₁₋₆ alkyl-carbonyl group is optionally substituted by 1 to 3 substituents selected from Group G),
(15) a C₁₋₆ alkoxy-carbonyl group,
(16) -N(R⁵⁷)(R⁵⁸) wherein R⁵⁷ and R⁵⁸ are as defined in claim 6,
(17) -CO-N(R⁵⁷)(R⁵⁸) wherein R⁵⁷ and R⁵⁸ are as defined in claim 6, or
(18) -C(R⁵⁹)=N(R⁶⁰)
wherein
R⁵⁹ is
(1) a hydrogen atom,
R⁶⁰ is
(1) a hydroxyl group, or
(2) a C₁₋₆ alkyl-carbonyl-oxy group,
or
two adjacent R⁵¹s may form, together with the ring to which they are bonded, a fused ring (said fused ring is optionally further substituted by one substituent selected from Group E,
or a pharmaceutically acceptable salt thereof.

14. The heterocyclic compound of claim 6, wherein 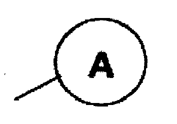 is 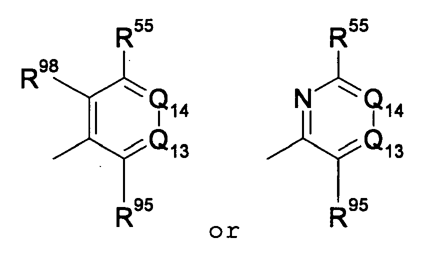 wherein
R⁹⁵ is as defined for R⁵¹ except that a hydrogen atom is excluded,
R⁹⁸ is as defined for R⁵¹, and
Q₁₃, Q₁₄, Q₁₆ and R⁵⁵ are as defined in claim 6,
or a pharmaceutically acceptable salt thereof.

15. The heterocyclic compound of claim 14, wherein
R⁹⁵ is
(1) a halogen atom,
(2) a cyano group,
(3) a nitro group,
(4) a formyl group,
(5) a C₁₋₆ alkyl group (said C₁₋₆ alkyl group is optionally substituted by 1 to 3 substituents selected from Group G),
(6) a C₁₋₆ alkylsulfinyl group (said C₁₋₆ alkylsulfinyl group is optionally substituted by 1 to 3 substituents selected from Group G), or
(7) a C₁₋₆ alkylsulfonyl group (said C₁₋₆ alkylsulfonyl group is optionally substituted by 1 to 3 substituents selected from Group G),
or a pharmaceutically acceptable salt thereof.

16. The heterocyclic compound of claim 6, wherein 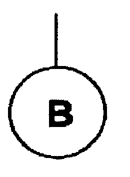 is 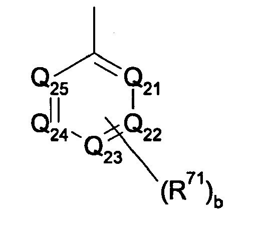 wherein
Q₂₁, Q₂₂, Q₂₃, Q₂₄, and Q₂₅ are the same or different and each is -N= or -CH=, and other symbols are as defined in claim 6, or two adjacent R⁷¹s may form, together with the ring to which they are bonded, a fused ring (said fused ring is optionally further substituted by one substituent selected from Group E),
or a pharmaceutically acceptable salt thereof.

17. The heterocyclic compound of claim 16, wherein 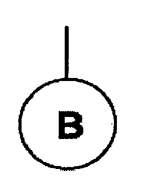 is 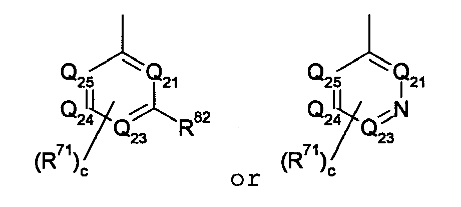 wherein .
R⁸² is as defined for R⁷¹ except that a hydrogen atom is excluded,
c is 0, 1 or 2,
R⁸² and the adjacent R⁷¹ may form, together with the ring to which they are bonded, a fused ring (said fused ring is optionally substituted by one substituent selected from Group E), and
other symbols are as defined in claim 16,
or a pharmaceutically acceptable salt thereof.

18. The heterocyclic compound of claim 16, wherein
R⁷¹ are the same or different and each is
(1) a hydrogen atom,
(2) a halogen atom,
(3) a cyano group,
(4) a hydroxyl group,
(5) a formyl group,
(6) a carboxyl group,
(7) a C₁₋₆ alkyl group (said C₁₋₆ alkyl group is optionally substituted by 1 to 3 substituents selected from Group G),
(8) a C₂₋₆ alkenyl group (said C₂₋₆ alkenyl group is optionally substituted by 1 to 3 substituents selected from Group G),
(9) a C₁₋₆ alkoxy group (said C₁₋₆ alkoxy group is optionally substituted by 1 to 3 substituents selected from Group G),
(10) a C₁₋₆ alkyl-carbonyl group,
(11) a C₁₋₆ alkoxy-carbonyl group,
(12) -N(R⁷²)(R⁷³) wherein R⁷² and R⁷³ are as defined in claim 6)
(13) -CO-N(R⁷²)(R⁷³) wherein R⁷² and R⁷³ are as defined in claim 6,
(14) -C(R⁷⁴)=N(R⁷⁵) wherein R⁷⁴ and R⁷⁵ are as defined in claim 6,
(15) a monocyclic aryl group,
(16) a monocyclic heteroaryl group (said monocyclic heteroaryl group is optionally substituted by 1 to 5 substituents selected from Group E), or
(17) a heterocycloalkyl group, or
two adjacent R⁷¹s may form, together with the ring to which they are bonded, a fused ring (said fused ring is optionally further substituted by one substituent selected from Group E),
or a pharmaceutically acceptable salt thereof.

19. The heterocyclic compound of claim 6 represented by the following formula [1], or a pharmaceutically acceptable salt thereof: 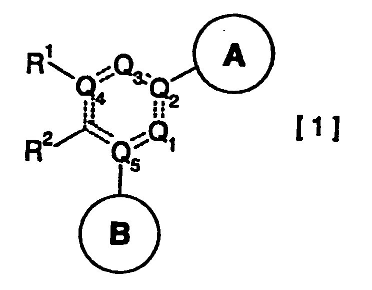 wherein 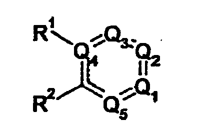 is 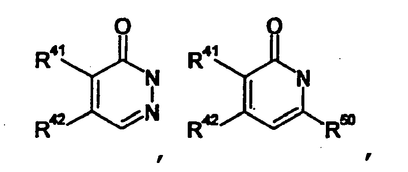 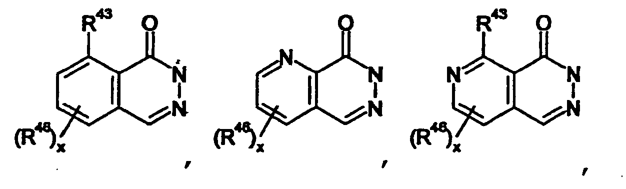 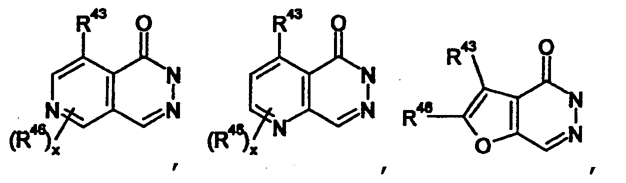 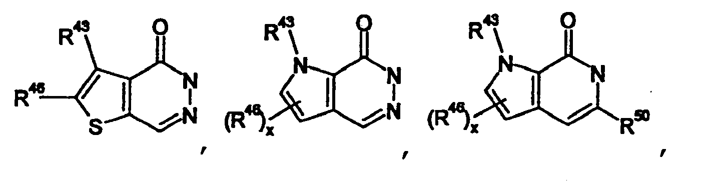 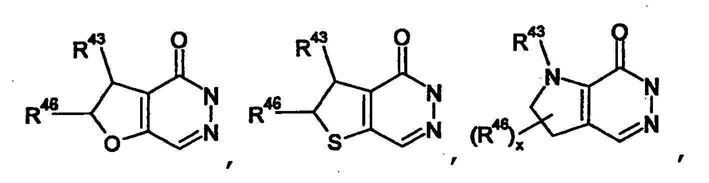 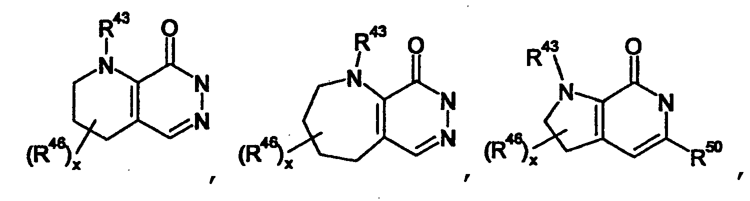 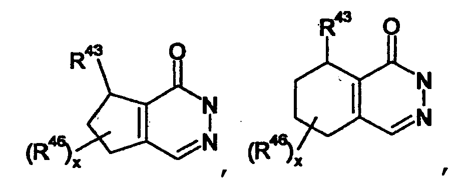 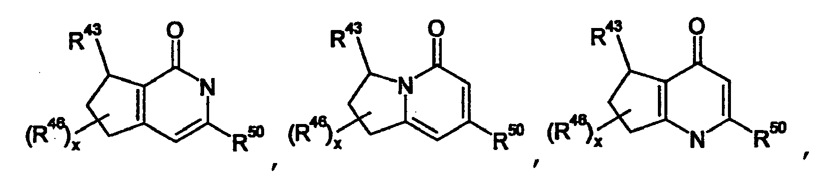 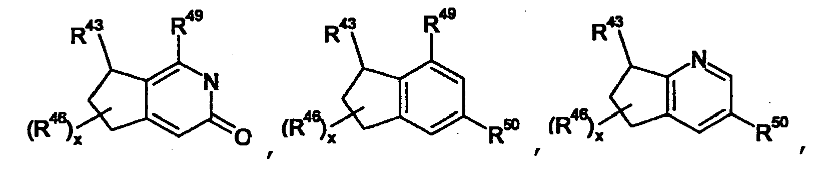 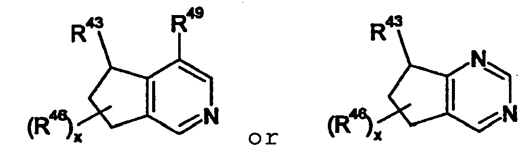 wherein
R⁴¹ is
(1) a hydrogen atom,
(2) a C₁₋₆ alkyl group (said C₁₋₆ alkyl group is optionally substituted by 1 to 3 substituents selected from Group F),
(3) a C₂₋₆ alkenyl group,
(4) -J₁-R¹⁰¹
wherein
J₁ is
(1) -N(R¹⁰²)- wherein R¹⁰² is a hydrogen atom or a C₁₋₆ alkyl group,
(2) -O-,
(3) -S-,
(4) -CO-,
(5) -CO-O-, or
(6) -CO-N(R¹⁰²)- wherein R¹⁰² is as defined above,
R¹⁰¹ is
(1) a hydrogen atom,
(2) -L₂-J₂-R¹⁰³
wherein
L₂ is
(1) a bond, or
(2) a C₁₋₆ alkylene group,
J₂ is
(1) -N(R¹⁰⁴)- wherein R¹⁰⁴ is a hydrogen atom or a C₁₋₆ alkyl group,
(2) -O-,
(3) -CO-,
(4) -SO₂-,
(5) -CO-O-,
(6) -CO-N(R¹⁰⁴)- wherein R¹⁰⁴ is as defined above, or
(7) -SO₂-N(R¹⁰⁴)- wherein R¹⁰⁴ is as defined above,
R¹⁰³ is
(1) a hydrogen atom,
(2) a C₁₋₆ alkyl group, or
(3) a C₁₋₆ alkyl-carbonyl group, or
(3) a C₁₋₆ alkyl group, or
R¹⁰¹ and R¹⁰² may form, together with the nitrogen atom to which they are bonded, a nitrogen-containing 4-to 7-membered heterocyclic group (said nitrogen-containing 4- to 7-membered heterocyclic group is optionally substituted by 1 to 5 substituents selected
from Group E), or
(5) a C₆₋₁₄ aryl group;
R⁴² is
(1) a hydrogen atom, or
(2) a C₁₋₆ alkyl group;
R⁴³ is
(1) a hydrogen atom,
(2) -L₃-J₃-R¹¹³
wherein
L₃ is
(1) a bond, or
(2) a C₁₋₆ alkylene group,
J₃ is
(1) -N(R¹¹⁴)- wherein R¹¹⁴ is a hydrogen atom or a C₁₋₆ alkyl group,
(2) -O-,
(3) -CO-,
(4) -SO₂-,
(5) -CO-O-, or
(6) -CO-N(R¹¹⁴)- wherein R¹¹⁴ is as defined above,
R¹¹³ is
(1) a hydrogen atom,
(2) -L₄-J₄-R¹¹⁵
wherein
L₄ is
(1) a bond, or
(2) a C₁₋₆ alkylene group,
J₄ is
(1) -N(R¹¹⁶)- wherein R¹¹⁶ is a hydrogen atom or a C₁₋₆ alkyl group,
(2) -O-, or
(3) -CO-,
R¹¹⁵ is
(1) a hydrogen atom,
(2) a C₁₋₆ alkyl group (said C₁₋₆ alkyl group is optionally substituted by 1 to 3 substituents selected from Group G), or
(3) a C₁₋₆ alkyl-carbonyl group, or
R¹¹⁵ and R¹¹⁶ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
(3) a C₁₋₆ alkyl group, or
R¹¹³ and R¹¹⁴ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E), or
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), a heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E);
x is 1 or 2;
R⁴⁶ are the same or different and each is
(1) a hydrogen atom,
(2) a hydroxyl group,
(3) a C₁₋₆ alkyl group, or
(4) a C₁₋₆ alkoxy group;
R⁴⁹ and R⁵⁰ are the same or different and each is
(1) a hydrogen atom,
(2) a hydroxyl group, or
(3) a C₁₋₆ alkoxy group; 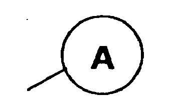 is 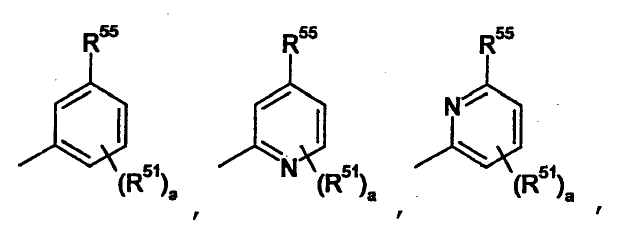 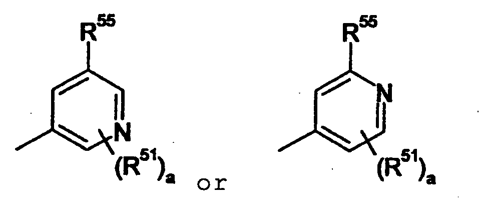
wherein
R⁵⁵ is
(1) a hydrogen atom,
(2) a substituent selected from a halogen atom, a cyano group and a nitro group,
(3) a C₁₋₆ alkyl group (said C₁₋₆ alkyl group is optionally substituted by 1 to 3 substituents selected from Group F),
(4) a C₂₋₆ alkynyl group (said C₂₋₆ alkynyl group is optionally substituted by 1 to 3 substituents selected from Group F),
(5) -J₅-R¹²¹
wherein
J₅ is
(1) -N(R¹²²)- wherein R¹²² is a hydrogen atom or a C₁₋₆ alkyl group,
(2) -O-,
(3) -CO-,
(4) -CO-O-,
(5) -CO-N(R¹²²)- wherein R¹²² is as defined above, or
(6) -N(R¹²³)-CO- wherein R¹²³ is a hydrogen atom or a C₁₋₆ alkyl group,
R¹²¹ is
(1) a hydrogen atom,
(2) -L₆-J₆-R¹²⁴
wherein
L₆ is
(1) a bond, or
(2) a C₁₋₆ alkylene group,
J₆ is
(1) -N(R¹²⁵)- wherein R¹²⁵ is a hydrogen atom or a C₁₋₆ alkyl group,
(2) -O-,
(3) -CO-,
(4) -CO-O-,
(5) -CO-N(R¹²⁵)- wherein R¹²⁵ is as defined above,
(6) -N(R¹²⁶)-CO- wherein R¹²⁶ is a hydrogen atom or a C₁₋₆ alkyl group,
(7) -N(R¹²⁶)-SO₂- wherein R¹²⁶ is as defined above,
(8) -N(R¹²⁶)-CO-O- wherein R¹²⁶ is as defined above, or
(9) -N(R¹²⁶)-CO-N(R¹²⁵)- wherein R¹²⁵ and R¹²⁶ are as defined above,
R¹²⁴ is
(1) a hydrogen atom, or
(2) a C₁₋₆ alkyl group (said C₁₋₆ alkyl group is optionally substituted by 1 to 3 substituents selected from Group G), or
R¹²⁴ and R¹²⁵ may form, together with the nitrogen atom to which they are bonded, a nitrogen-containing 4- to 7-membered heterocyclic group (said nitrogen-containing 4- to 7-membered heterocyclic group is optionally substituted by 1 to 5 substituents selected from Group E),
(3) a C₁₋₆ alkyl group [said C₁₋₆ alkyl group is optionally substituted by 1 to 3 substituents selected from a halogen atom, a cyano group, a nitro group, a C₆₋₁₄ aryl group (said C₆₋₁₄ aryl group is optionally substituted by 1 to 5 substituents selected from Group E), heteroaryl group (said heteroaryl group is optionally substituted by 1 to 5 substituents selected from Group E) and a C₃₋₈ cycloalkyl group (said C₃₋₈ cycloalkyl group is optionally substituted by 1 to 5 substituents selected from Group E),
(4) a substituent selected from a halogen atom, a cyano group and a nitro group,
(5) a C₆₋₁₄ aryl group (said C₆₋₁₄ aryl group is optionally substituted by 1 to 5 substituents selected from Group E),
(6) a heteroaryl group (said heteroaryl group is optionally substituted by 1 to 5 substituents selected from Group E), or
(7) a C₃₋₈ cycloalkyl group (said C₃₋₈ cycloalkyl group is optionally substituted by 1 to 5 substituents selected from Group E), or
R¹²¹ and R¹²² may form, together with the nitrogen atom to which they are bonded, a nitrogen-containing 4-to 7-membered heterocyclic group, wherein said nitrogen-containing 4- to 7-membered heterocyclic group is optionally substituted by 1 to 5 substituents selected from
(1) Group F,
(2) a C₁₋₆ alkyl group (said C₁₋₆ alkyl group is optionally substituted by 1 to 3 substituents selected from Group F),
(3) a C₁₋₆ alkoxy group (said C₁₋₆ alkoxy group is optionally substituted by 1 to 3 substituents selected from Group F), and
(4) an oxo group,
(6) a monocyclic aryl group,
(7) a monocyclic heteroaryl group (said monocyclic heteroaryl group is optionally substituted by 1 to 5 substituents selected from Group E), or
(8) a heterocycloalkyl group;
a is 1 or 2;
R⁵¹ are the same or different and each is
(1) a hydrogen atom,
(2) a halogen atom,
(3) a cyano group,
(4) a nitro group,
(5) a hydroxyl group,
(6) a formyl group,
(7) a carboxyl group,
(8) a C₁₋₆ alkyl group (said C₁₋₆ alkyl group is optionally substituted by 1 to 3 substituents selected from Group G),
(9) a C₂₋₆ alkenyl group,
(10) a C₁₋₆ alkoxy group (said C₁₋₆ alkoxy group is optionally substituted by 1 to 3 substituents selected from Group G),
(11) a C₁₋₆ alkylthio group,
(12) a C₁₋₆ alkylsulfinyl group,
(13) a C₁₋₆ alkylsulfonyl group,
(14) a C₁₋₆ alkyl-carbonyl group (said C₁₋₆ alkyl-carbonyl group is optionally substituted by 1 to 3 substituents selected from Group G),
(15) a C₁₋₆ alkoxy-carbonyl group,
(16) -N(R⁵⁷)(R⁵⁸)
wherein
R⁵⁷ and R⁵⁸ are the same or different and each is
(1) a hydrogen atom,
(2) a C₁₋₆ alkyl group,
(3) a C₁₋₆ alkylsulfonyl group,
(4) a C₁₋₆ alkyl-carbonyl group, or
(5) a C₁₋₆ alkoxy-carbonyl group, or
R⁵⁷ and R⁵⁸ may form, together with the nitrogen atom to which they are bonded, a nitrogen-containing 4-to 7-membered heterocyclic group,
(17) -CO-N(R⁵⁷)(R⁵⁸) wherein R⁵⁷ and R⁵⁸ are as defined above, or
(18) -C(R⁵⁹)=N(R⁶⁰)
wherein
R⁵⁹ is
(1) a hydrogen atom,
R⁶⁰ is
(1) a hydroxyl group, or
(2) a C₁₋₆ alkyl-carbonyl-oxy group, or two adjacent R⁵¹s may form, together with the ring to which they are bonded, a fused ring (said fused ring is optionally further substituted by one substituent selected from Group E); 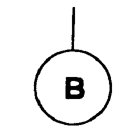
is 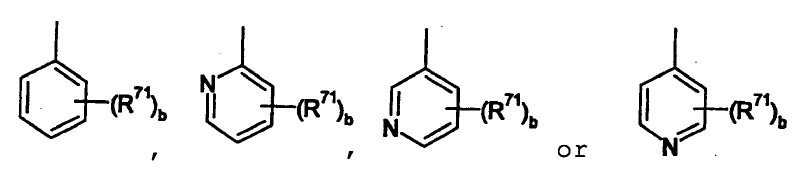
wherein
b is 1 or 2;
R⁷¹ are the same or different and each is
(1) a hydrogen atom,
(2) a halogen atom,
(3) a cyano group,
(4) a hydroxyl group,
(5) a formyl group,
(6) a carboxyl group,
(7) a C₁₋₆ alkyl group (said C₁₋₆ alkyl group is optionally substituted by 1 to 3 substituents selected from Group G),
(8) a C₂₋₆ alkenyl group (said C₂₋₆ alkenyl group is optionally substituted by 1 to 3 substituents selected from Group G),
(9) a C₁₋₆ alkoxy group (said C₁₋₆ alkoxy group is optionally substituted by 1 to 3 substituents selected from Group G),
(10) a C₁₋₆ alkyl-carbonyl group,
(11) a C₁₋₆ alkoxy-carbonyl group,
(12) -N(R⁷²)(R⁷³)
wherein
R⁷² and R⁷³ are the same or different and each is
(1) a hydrogen atom,
(2) a C₁₋₆ alkyl group (said C₁₋₆ alkyl group is optionally substituted by 1 to 3 substituents selected from Group G),
(3) a C₁₋₆ alkylsulfonyl group,
(4) a C₁₋₆ alkyl-carbonyl group,
(5) a C₁₋₆ alkoxy-carbonyl group,
(6) a carbamoyl group,
(7) a hydroxyl group,
(8) an amino group, or
(9) a C₁₋₆ alkyl-carbonyl-amino group, or
R⁷² and R⁷³ may form, together with the nitrogen atom to which they are bonded, a nitrogen-containing 4-to 7-membered heterocyclic group,
(13) -CO-N(R⁷²)(R⁷³) wherein R⁷² and R⁷³ are as defined above,
(14) -C(R⁷⁴)=N(R⁷⁵)
wherein
R⁷⁴ is
(1) a hydrogen atom,
(2) a C₁₋₆ alkyl group, or
(3) an amino group,
R⁷⁵ is
(1) a hydroxyl group,
(2) a C₁₋₆ alkoxy group (said C₁₋₆ alkoxy group is optionally substituted by 1 to 3 substituents selected from Group G), or
(3) -N(R⁷⁶)(R⁷⁷) wherein R⁷⁶ and R⁷⁷ are the same or different and each is a hydrogen atom, or a C₁₋₆ alkyl-carbonyl group,
(15) a monocyclic aryl group,
(16) a monocyclic heteroaryl group (said monocyclic heteroaryl group is optionally substituted by 1 to 5 substituents selected from Group E), or
(17) a heterocycloalkyl group, or
two adjacent R⁷¹s may form, together with the ring to which they are bonded, a fused ring (said fused ring is optionally further substituted by one substituent selected from Group E):
wherein
Group E consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkyl group (said C₁₋₆ alkyl group is optionally substituted by 1 to 3 substituents selected from Group G),
(12) a C₂₋₆ alkenyl group (said C₂₋₆alkenyl group is optionally substituted by 1 to 3 substituents selected from Group G),
(13) a C₂₋₆ alkynyl group (said C₂₋₆ alkynyl group is optionally substituted by 1 to 3 substituents selected from Group G),
(14) a C₁₋₆ alkoxy group (said C₁₋₆ alkoxy group is optionally substituted by 1 to 3 substituents selected from Group G),
(15) a C₁₋₆ alkylthio group,
(16) a C₁₋₆ alkylsulfinyl group,
(17) a C₁₋₆ alkylsulfonyl group,
(18) a C₁₋₆ alkyl-carbonyl group,
(19) a C₁₋₆ alkoxy-carbonyl group,
(20) a C₁₋₆ alkylamino group,
(21) a C₆₋₁₄aryl group,
(22) a heteroaryl group, and
(23) an oxo group;
Group F consists of
(1) a halogen atom,
(2) a cyano group,
(3) a nitro group,
(4) a hydroxyl group,
(5) a mercapto group,
(6) a formyl group,
(7) a carboxyl group,
(8) a C₁₋₆ alkoxy group,
(9) a C₁₋₆ alkylthio group,
(10) a C₁₋₆ alkylsulfinyl group,
(11) a C₁₋₆ alkylsulfonyl group,
(12) a C₁₋₆ alkyl-carbonyl group,
(13) a C₁₋₆ alkoxy-carbonyl group,
(14) -N(R⁹¹)(R⁹²) wherein R⁹¹ and R⁹² are the same or different and each is a hydrogen atom, a C₁₋₆ alkyl group, a C₁₋₆ alkyl-carbonyl group, a C₁₋₆ alkylsulfonyl group, a C₁₋₆ alkoxy-carbonyl group, a carbamoyl group, a C₁₋₆ alkylamino-carbonyl group, or a di(C₁₋₆ alkyl)amino-carbonyl group, or R⁹¹ and R⁹² may form, together with the nitrogen atom to which they are bonded, a nitrogen-containing 4- to 7-membered heterocyclic group,
(15) -CO-N(R⁹¹)(R⁹²) wherein R⁹¹ and R⁹² are as defined above,
(16) -SO₂-N(R⁹¹)(R⁹²) wherein R⁹¹ and R⁹² are as defined above,
(17) -O-CO-N(R⁹¹)(R⁹²) wherein R⁹¹ and R⁹² are as defined above,
(18) a C₆₋₁₄ aryl group,
(19) a C₆₋₁₄ aryl-oxy group,
(20) a C₆₋₁₄ aryl-carbonyl group, and
(21) a heteroaryl group;
Group G consists of
(1) a halogen atom,
(2) a cyano group,
(3) a nitro group,
(4) a hydroxyl group,
(5) a mercapto group,
(6) a formyl group,
(7) a carboxyl group,
(8) a carbamoyl group,
(9) a sulfamoyl group,
(10) an amino group,
(11) a C₁₋₆ alkoxy group,
(12) a C₁₋₆ alkylthio group,
(13) a C₁₋₆ alkylsulfinyl group,
(14) a C₁₋₆ alkylsulfonyl group,
(15) a C₁₋₆ alkyl-carbonyl group,
(16) a C₁₋₆ alkoxy-carbonyl group,
(17) a C₁₋₆ alkylamino group,
(18) a C₁₋₆ alkyl-carbonyl-amino group,
(19) a C₆₋₁₄ aryl group, and
(20) heteroaryl group,
provided that the compound of the formula [1] satisfies the following provisions 1 to 12:
provision 1:
when 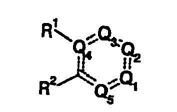 wherein R¹, R², Q₁, Q₂, Q₃, Q₄ and Q₅ are as defined above, is 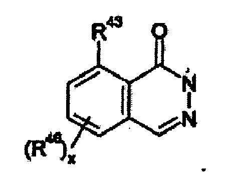 wherein R⁴³, R⁴⁶ and x are as defined above, 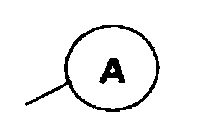 is 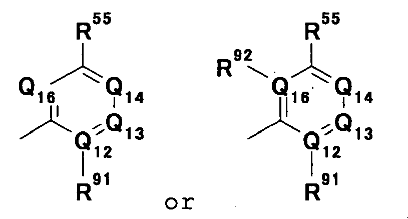 wherein
Q₁₂, Q₁₃, Q₁₄, Q₁₆ and R⁵⁵ are as defined above,
R⁹¹ is as defined for R⁵¹ (provided that a hydrogen atom is excluded), and
R⁹² is as defined for R⁵¹, and
the compounds satisfying the following provisions 1-1 to 1-3 are excluded:
provision 1-1:
ring A is 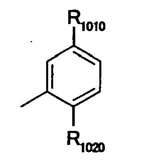 wherein R₁₀₁₀ is a chlorine atom, a piperidinyl group, a pyrrolidinyl group, an azepanylgroup or a morpholinyl group, and R₁₀₂₀ is a nitro group or an amino group, and ring B is 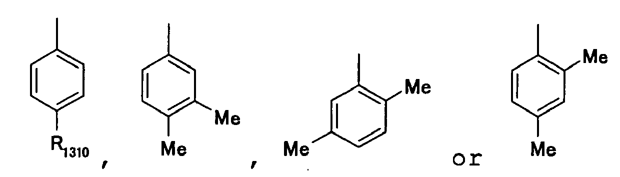 wherein R₁₃₁₀ is a hydrogen atom, a fluorine atom, a chlorine atom, a methyl group or an ethyl group;
provision 1-2:
ring A is 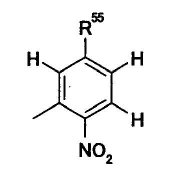 wherein
R⁵⁵ is an unsubstituted or substituted piperazinyl group optionally substituted by 1 to 5 substituents selected from Group E, Group F, a C₁₋₆ alkyl group (said C₁₋₆ alkyl group is optionally substituted by 1 to 3 substituents selected from Group F) and a C₁₋₆ alkoxy group (said C₁₋₆ alkoxy group is optionally substituted by 1 to 3 substituents selected from Group F); and
provision 1-3:
ring A is 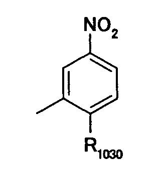 wherein R₁₀₃₀ is a chlorine atom, a pyrrolidinyl group, a morpholinyl group or a N-methylpiperazinyl group, and
ring B is 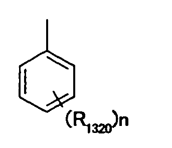 wherein n is 1 or 2, R₁₃₂₀ is a hydrogen atom, a chlorine atom or a methyl group;
provision 2:
when 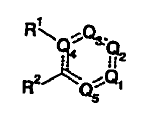 wherein R¹, R², Q₁, Q₂, Q₃, Q₄ and Q₅ are as defined above, is 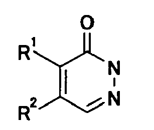 wherein R¹ and R² are as defined above, provided that the case where R¹ and R² form, together with Q₄ and the carbon atom to which they are respectively bonded, 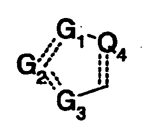 wherein G₁, G₂, G₃ and Q₄ are as defined above, is excluded, 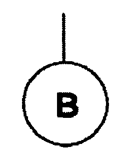 is 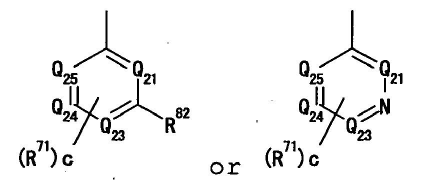 wherein Q₂₁, Q₂₃, Q₂₄ and Q₂₅ are the same or different and each is a carbon atom or a nitrogen atom, R⁷¹ is as defined above, R⁸² is as defined for R⁷¹ except that a hydrogen atom is excluded, c is 0, 1 or 2, or two adjacent R⁷¹ S may form, together with the ring to which they are bonded, a fused ring (said fused ring is optionally further substituted by one substituent selected from Group E), and the following compounds are excluded:
6-(2,5-dimethylphenyl)-2-phenyl-3(2H)-pyridazinone,
6-(3,4-dimethylphenyl)-2-phenyl-3(2H)-pyridazinone,
6-(4-chloro-3-methylphenyl)-2-phenyl-3(2H)-pyridazinone,
2-(2-aminophenyl)-6-(3-chlorophenyl)-3(2H)-pyridazinone,
2-(2-aminophenyl)-6-[3-(trifluoromethyl)phenyl]-3(2H)-pyridazinone,
6-(3-chlorophenyl)-2-(2-nitrophenyl)-3(2H)-pyridazinone,
2-(2-nitrophenyl)-6-[3-(trifluoromethyl)phenyl]-3(2H)-pyridazinone,
6-(3,4-dichlorophenyl)-2-phenyl-4-(2,4,6-trimethylphenyl)-3(2H)-pyridazinone,
2-(2,4-dinitrophenyl)-6-(3-nitrophenyl)-3(2H)-pyridazinone,
2-(4-chlorophenyl)-6-(3-nitrophenyl)-3(2H)-pyridazinone,
2-(3-chlorophenyl)-6-(3-nitrophenyl)-3(2H)-pyridazinone,
2-(4-methylphenyl)-6-(3-nitrophenyl)-3(2H)-pyridazinone,
2-(3-methylphenyl)-6-(3-nitrophenyl)-3(2H)-pyridazinone,
2-(2-methylphenyl)-6-(3-nitrophenyl)-3(2H)-pyridazinone,
6-(3-nitrophenyl)-2-(4-nitrophenyl)-3(2H)-pyridazinone,
2,6-bis(3-nitrophenyl)-3(2H)-pyridazinone,
2-(2-nitrophenyl)-6-(3-nitrophenyl)-3(2H)-pyridazinone,
6-(3-nitrophenyl)-2-phenyl-3(2H)-pyridazinone,
6-(2-naphthyl)-2-phenyl-3(2H)-pyridazinone,
6-(1-naphthalenyl)-2-phenyl-3(2H)-pyridazinone,
2-(2-cyanophenyl)-4-(2-methoxyphenyl)-6-(3-pyridazinyl)-2H-pyridazin-3-one,
2-(2-cyanophenyl)-4-phenyl-6-(2-pyrazyl)-2H-pyridazin-3-one,
2-(2-aminophenyl)-6-(3-pyridazinyl)-3(2H)-pyridazinone,
2-(2-nitrophenyl)-6-(3-pyridazinyl)-3(2H)-pyridazinone,
6,6"-dichloro-[3,1'(6'H):3',3"-terpyridazine]-6'-one,
3-[1,6-dihydro-1-(4-methylphenyl)-6-oxo-3-pyridazinyl]-2(1H)-quinoxalinone,
3-[1,6-dihydro-1-(2-methylphenyl)-6-oxo-3-pyridazinyl]-2(1H)-quinoxalinone,
3-(1,6-dihydro-6-oxo-1-phenyl-3-pyridazinyl)-1-methyl-2(1H)-quinoxalinone,
3-[1-(4-chlorophenyl)-1,6-dihydro-6-oxo-3-pyridazinyl]-2(1H)-quinoxalinone, and
3-(1,6-dihydro-6-oxo-1-phenyl-3-pyridazinyl)-2(1H)-quinoxalinone;
provision 3:
when 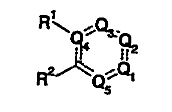 wherein R¹, R², Q₁, Q₂, Q₃, Q₄ and Q₅ are as defined above, is 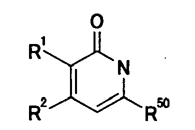 wherein R¹ and R² are as defined above, provided that the case where R¹ and R² form, together with Q₄ and the carbon atom to which they are respectively bonded, 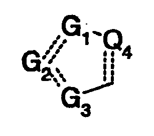 wherein G₁, G₂, G₃ and Q₄ are as defined above, is excluded, and R⁵⁰ is as defined above, 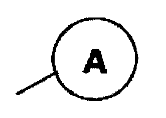 is 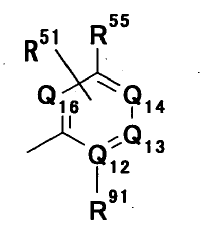 wherein Q₁₂, Q₁₃, Q₁₄, Q₁₆, R⁵¹ and R⁵⁵ are as defined above, R⁹¹ is as defined for R⁵¹ (provided that a hydrogen atom is excluded), 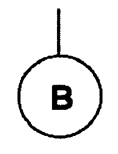 is 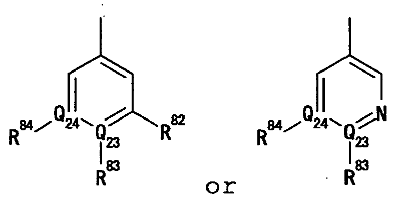 wherein
Q₂₃ and Q₂₄ are the same or different and each is a carbon atom or a nitrogen atom,
R⁸² is as defined for R⁷¹ (provided that a hydrogen atom is excluded),
R⁸³ and R⁸⁴ are the same or different and each is as defined for R⁷¹, or R⁸³ and R⁸⁴ may form, together with the ring to which they are bonded, a fused ring (said fused ring is optionally further substituted by one substituent selected from Group E);
provision 4:
when 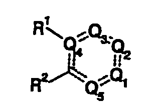 wherein R¹, R², Q₁, Q₂, Q₃, Q₄ and Q₅ are as defined above, is 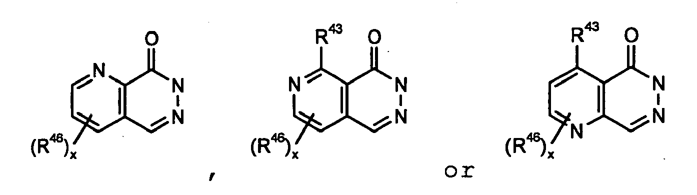 wherein R⁴³ and R⁴⁶ are the same or different and each is a hydrogen atom, a halogen atom, a hydroxyl group or a C₁₋₆ alkyl group, and x is as defined above,
the compounds satisfying the following provisions 4-1 and 4-2 are excluded:
provision 4-1: 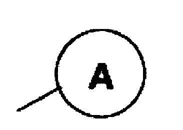 is 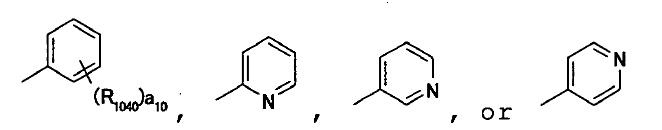 wherein when a₁₀ is 1, R₁₀₄₀ is an amino group, a hydrogen atom, a trifluoromethyloxy group, a bromine atom, a sulfamoyl group, a methanesulfonyl group, a hydroxyl group, a C₁₋₆ alkyl group, a C₁₋₆ alkoxy group, a chlorine atom, a fluorine atom, a trifluoromethyl group, a carbamoyl group, a nitro group or a cyano group, and a₁₀ is an integer of 2 or 3, R₁₀₄₀ are the same or different and each is a hydroxyl group, a C₁₋₆ alkyl group, a C₁₋₆ alkoxy group, a chlorine atom, a fluorine atom, a trifluoromethyl group, a carbamoyl group, a nitro group or a cyano group, and
ring B is a benzotriazole group, or 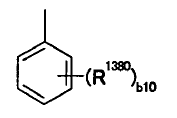 wherein b10 is an integer of 0 or 1 to 5, R¹³⁸⁰ are the same or different and each is a hydrogen atom, a halogen atom, a C₁₋₆ alkyl group, -COOH, a C₁₋₆ alkoxycarbonyl group, a carbamoyl group, a C₁₋₆ alkylcarbonyl group, a halocarbonyl group, a methylthio group, a trifluoromethyl group, a cyano group, a nitro group, a 2,2-difluorodioxolanyl group, a methanesulfonyl group or a trifluoromethyloxy group;
provision 4-2: 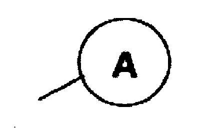 is 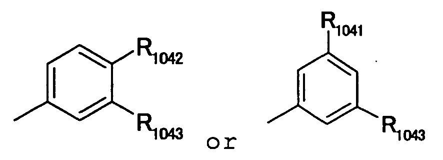 wherein R₁₀₄₁ is a fluorine atom or a trifluoromethyl group, R₁₀₄₂ is a fluorine atom or chlorine atom, R₁₀₄₃ is a fluorine atom, a chlorine atom, a cyano group or a trifluoromethyl group, or R₁₀₄₂ and R₁₀₄₃ form a ring represented by 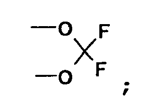 provision 5:
when 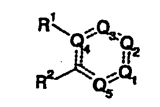 is 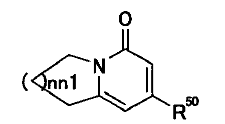 wherein R⁵⁰ is a hydrogen atom or a hydroxyl group, and nn1 is an integer of 1 to 3,
the compounds satisfying the following provisions 5-1 and 5-2 are excluded:
provision 5-1:
ring A is a phenyl group or a 3,4-dimethoxyphenyl group, and ring B is a phenyl group or a 4-chlorophenyl group, and nn1 is 1;
provision 5-2:
ring A is a phenyl group or a pyridyl group, which is optionally substituted by a halogen atom, a trifluoromethyl group, a nitro group, a C₁₋₆ alkyl group or a C₁₋₆ alkoxy group, and nn1 is 2 or 3;
provision 6:
when 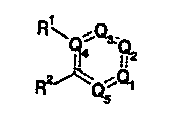 wherein R¹, R², Q₁, Q₂, Q₃, Q₄ and Q₅ are as defined above, is 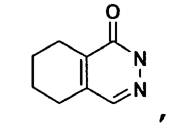 compounds in which ring A is a phenyl group and ring B is a 3,4-dimethoxyphenyl group are excluded;
provision 7:
when 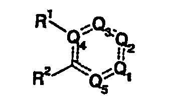 wherein R¹, R², Q₁, Q₂, Q₃, Q₄ and Q₅ are as defined above, is 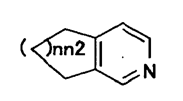 wherein nn2 is an integer of 1 to 3,
compounds in which ring A is a phenyl group or 4-chlorophenyl group, and nn2 is an integer of 1 to 3 are excluded;
provision 8:
when 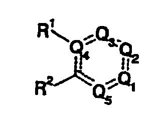 wherein R¹, R², Q₁, Q₂, Q₃, Q₄ and Q₅ are as defined above, is 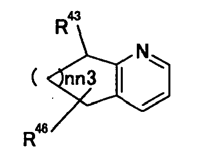 wherein R⁴³ is a hydrogen atom, a hydroxyl group, an acetyloxy group, a methyl group, a benzyl group, a 4-methylbenzyl group, -CH₂-NMe₂ or -CH₂-S-nBu, R⁴⁶ is a hydrogen atom or a methyl group, and nn3 is an integer of 1 to 3,
the following compounds are excluded:
compounds in which ring A is 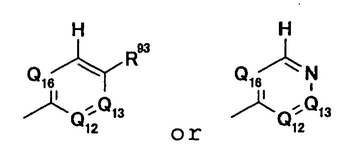 wherein Q₁₂, Q₁₃ and Q₁₆ are as described above, and R⁹³ is an amino group, a nitro group, a hydrogen atom, a trifluoromethyl group, a methoxy group, a methyl group,
a chlorine atom or an acetamide group, and
ring B is 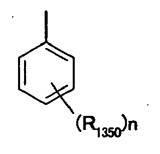 wherein R₁₃₅₀ is a hydrogen atom, -COOMe, a cyano group, - COOEt, t-Bu, a dimethylamino group, a methyl group, a chlorine atom, a dioxolanyl group, a 2-pyridyl group or a 2-pyrimidinyl group, and n is an integer of 1 to 3;
provision 9:
when 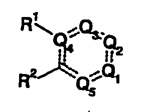 wherein R¹, R², Q₁, Q₂, Q₃, Q₄ and Q₅ are as defined above, is 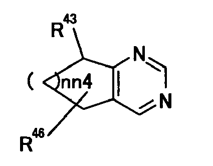 wherein R⁴³ and R⁴⁶ are as defined above, and nn4 is an integer of 1 to 3,
compounds in which ring A is a phenyl group, a 3,4-dimethoxyphenyl group, a 4-methylphenyl group, a 4-chlorophenyl group or a 4-fluorophenyl group, ring B is a phenyl group, a 3,4-dimethoxyphenyl group, a 4-methylphenyl group, a 4-chlorophenyl group, a 4-fluorophenyl group or a 3-chlorophenyl group, R⁴³ and R⁴⁶ are each a hydrogen atom or a methyl group, and nn4 is an integer of 1 to 3 are excluded;
provision 10:
when 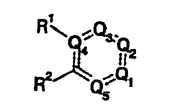 wherein R¹, R², Q₁, Q₂, Q₃, Q₄ and Q₅ are as defined above, is 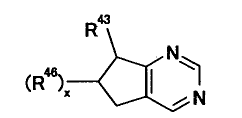 wherein R⁴³ and R⁴⁶ are the same or different and each is a hydrogen atom or a C₁₋₆ alkyl group,
compounds in which ring A is a phenyl group substituted, at the ortho- or meta- position, by a halogen atom, a C₁₋₆ alkyl group, a C₁₋₆ haloalkyl group, a C₁₋₆ alkoxy group or a C₁₋₆ haloalkyloxy group, and ring B is a phenyl group substituted by a hydrogen atom, a halogen atom, a
C₁₋₆ alkyl group, a C₁₋₆ haloalkyl group, a C₁₋₆ alkoxy group or a C₁₋₆ haloalkyloxy group are excluded;
provision 11:
when 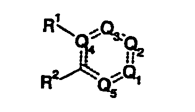 wherein R¹, R², Q₁, Q₂, Q₃, Q₄ and Q₅ are as defined above, is 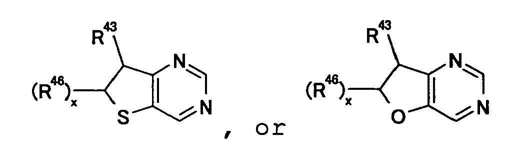 wherein R⁴³ and R⁴⁶ are the same or different and each is a hydrogen or a C₁₋₆ alkyl group,
compounds in which
ring A is a phenyl group substituted at the ortho- or meta- position by a halogen atom, a C₁₋₆ alkyl group, a C₁₋₆ haloalkyl group, a C₁₋₆ alkoxy group or a C₁₋₆ haloalkyloxy group, and ring B is a phenyl group substituted by a hydrogen atom, a halogen atom, a C₁₋₆ alkyl group, a C₁₋₆ haloalkyl group, a C₁₋₆ alkoxy group or a C₁₋₆ haloalkyloxy group are excluded; and
provision 12:
when 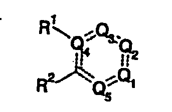 wherein R¹, R², Q₁, Q₂, Q₃, Q₄ and Q₅ are as defined above, is 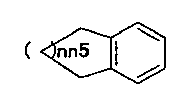 wherein nn5 is an integer of 1 to 3, compounds in which ring A and ring B are each a 3-nitrophenyl group or a 4-nitrophenyl group, and nn5 is 2 are excluded.

20. A heterocyclic compound represented by the following formula: 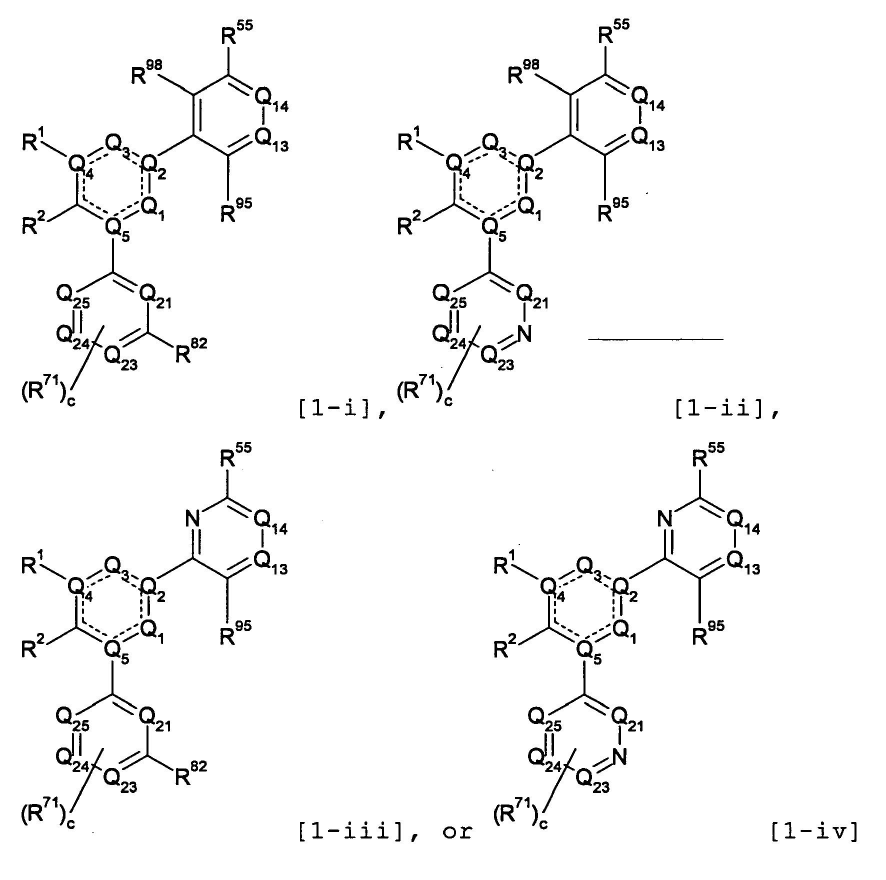 wherein Q₁, Q₂, Q₃, Q₄, Q₅, R¹, R², R⁵⁵ and R⁷¹ are as defined in claim 1, Q₁₃ and Q₁₄ are as defined in claim 6, R⁹⁵ and R⁹⁸ are as defined in claim 12, Q₂₁, Q₂₂, Q₂₄ and Q₂₅ are as defined in claim 14, and R⁸² is as defined in claim 17,
or a pharmaceutically acceptable salt thereof.

21. A pharmaceutical composition comprising the compound of any one of claims 6-20 or a pharmaceutically acceptable salt thereof and a pharmaceutically acceptable carrier.

22. An HCV entry inhibitor comprising the compound of any one of claims 6-20 or a pharmaceutically acceptable salt thereof as an active ingredient.

23. An anti-hepatitis virus agent comprising the compound of any one of claims 6-20 or a pharmaceutically acceptable salt thereof as an active ingredient.

24. A therapeutic agent for hepatitis C comprising the compound of any one of claims 6-20 or a pharmaceutically acceptable salt thereof as an active ingredient.

25. A therapeutic agent for hepatitis C comprising a combination of (a) the therapeutic agent for hepatitis C of claim 24, and (b) at least one pharmaceutical agent selected from other antiviral agent, anti-inflammatory agent and immunity enhancing agent.

26. A therapeutic agent for hepatitis C comprising a combination of (a) the therapeutic agent for hepatitis C of claim 24, and (b) an interferon.

27. An anti-hepatitis virus agent comprising a combination of (a) the anti-hepatitis virus agent of claim 23, and (b) at least one pharmaceutical agent selected from other antiviral agent, anti-inflammatory agent and immunity enhancing agent.

28. An anti-hepatitis virus agent comprising a combination of (a) the anti-hepatitis virus agent of claim 23, and (b) an interferon.

29. A pharmaceutical composition comprising a combination of (a) the compound of any one of claims 6-20 or a pharmaceutically acceptable salt thereof, and (b) at least one pharmaceutical agent selected from other antiviral agent, anti-inflammatory agent and immunity enhancing agent.

30. A pharmaceutical composition comprising a combination of (a) the compound of any one of claims 6-20 or a pharmaceutically acceptable salt thereof, and (b) an interferon.

31. Use of the compound of any one of claims 6-20 or a pharmaceutically acceptable salt thereof for the production of an HCV entry inhibitor.

32. Use of the compound of any one of claims 6-20 or a pharmaceutically acceptable salt thereof for the production of an anti-hepatitis C virus agent.

33. Use of the compound of any one of claims 6-20 or a pharmaceutically acceptable salt thereof for the production of a therapeutic agent for hepatitis C.

34. Use of a combination of (a) the therapeutic agent for hepatitis C of claim 24 and (b) at least one pharmaceutical agent selected from other antiviral agent, anti-inflammatory agent and immunity enhancing agent for the production of a therapeutic agent for hepatitis C.

35. Use of a combination of (a) the therapeutic agent for hepatitis C of claim 24 and (b) an interferon for the production of a therapeutic agent for hepatitis C.

36. Use of a combination of (a) the anti-hepatitis virus agent of claim 23 and (b) at least one pharmaceutical agent selected from other antiviral agent, anti-inflammatory agent and immunity enhancing agent for the production of an anti-hepatitis C virus agent.

37. Use of a combination of (a) the anti-hepatitis virus agent of claim 23 and (b) an interferon for the production of an anti-hepatitis C virus agent.
